(19) Europäisches Patentamt
European Patent Office
Office européen des brevets

(11) EP 3 432 934 B1

(12) **EUROPÄISCHE PATENTSCHRIFT**

(45) Veröffentlichungstag und Bekanntmachung des Hinweises auf die Patenterteilung:
**23.02.2022 Patentblatt 2022/08**

(21) Anmeldenummer: **17712140.7**

(22) Anmeldetag: **21.03.2017**

(51) Internationale Patentklassifikation (IPC):
***A61K 47/65*** *(2017.01)* ***A61K 47/68*** *(2017.01)*
***A61P 35/00*** *(2006.01)*

(52) Gemeinsame Patentklassifikation (CPC):
**C07D 207/335; A61K 47/65; A61K 47/6803; A61K 47/6845; A61K 47/6849; A61K 47/6889; A61P 35/00; C07D 401/12; C07D 401/14**

(86) Internationale Anmeldenummer:
**PCT/EP2017/056684**

(87) Internationale Veröffentlichungsnummer:
**WO 2017/162663 (28.09.2017 Gazette 2017/39)**

(54) **PRODRUGS VON CYTOTOXISCHEN WIRKSTOFFEN MIT ENZYMATISCH SPALTBAREN GRUPPEN**

PRODRUGS OF CYTOTOXIC AGENTS WITH ENZYMATICALLY CLEAVABLE GROUPS

PROMEDICAMENTS D'AGENTS ACTIFS CYTOTOXIQUES COMPRENANT DES GROUPES CLIVABLES PAR ENZYMES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA**

(30) Priorität: **24.03.2016 EP 16162400**
**21.12.2016 EP 16205988**

(43) Veröffentlichungstag der Anmeldung:
**30.01.2019 Patentblatt 2019/05**

(73) Patentinhaber: **Bayer Pharma Aktiengesellschaft**
**13353 Berlin (DE)**

(72) Erfinder:
- **LERCHEN, Hans-Georg**
  **51375 Leverkusen (DE)**
- **REBSTOCK, Anne-Sophie**
  **69410 Champagne au Mont d'Or (FR)**
- **MARX, Leo**
  **1920 Martigny (CH)**
- **JOHANNES, Sarah Anna Liesa**
  **40225 Düsseldorf (DE)**
- **STELTE-LUDWIG, Beatrix**
  **42489 Wülfrath (DE)**
- **DIETZ, Lisa**
  **42111 Wuppertal (DE)**
- **TERJUNG, Carsten**
  **44799 Bochum (DE)**
- **MAHLERT, Christoph**
  **42111 Wuppertal (DE)**
- **GREVEN, Simone**
  **41541 Dormagen (DE)**
- **SOMMER, Anette**
  **10405 Berlin (DE)**
- **BERNDT, Sandra**
  **16540 Hohen Neuendorf (DE)**

(74) Vertreter: **HGF**
**HGF Europe LLP**
**Neumarkter Straße 18**
**81673 München (DE)**

(56) Entgegenhaltungen:
**WO-A1-2015/096982 WO-A1-2015/096982**
**WO-A2-2014/062697 US-A1- 2015 343 083**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **MARY A. MATHIEU ET AL: "Substrate specificity of schistosome versus human legumain determined by P1-P3 peptide libraries", MOLECULAR AND BIOCHEMICAL PARASITOLOGY, Bd. 121, Nr. 1, 4. März 2002 (2002-03-04), Seiten 99-105, XP055298496, NL ISSN: 0166-6851, DOI: 10.1016/S0166-6851(02)00026-9**

## Beschreibung

### Einleitung und Stand der Technik

Die Erfindung betrifft neuartige Prodrugs, bei denen cytotoxische Wirkstoffe wie z.B. Kinesin Spindel Protein-Inhibitoren mit Gruppen konjugiert werden, die selektiv von Tumor-assoziierten Proteasen abgespalten werden und so den Wirkstoff freisetzen, sowie die Verwendung dieser Prodrugs bzw. Konjugate zur Behandlung und/oder Prävention von Krankheiten sowie die Verwendung dieser Prodrugs zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebszellen exprimieren häufig bestimmte Proteasen in stärkerem Maße als normale Zellen. Dies hat zu Ansätzen zur Erhöhung der Selektivität von cytotoxischen Wirkstoffen für Krebszellen geführt, bei denen die Wirkstoffe mit Gruppen verbunden werden, die von solchen Proteasen abgespalten werden, wodurch der Wirkstoff freigesetzt wird.

Eine solche Tumor-assoziierte Protease ist Legumain. Legumain ist eine Asparaginyl-Endopeptidase (S. Ishii, Methods Enzymol. 1994, 244, 604; J. M. Chen et al. J. Biol. Chem. 1997, 272, 8090) und wurde zur Prozessierung von Prodrugs von kleinen cytotoxischen Molekülen wie zum Beispiel unter anderem von Doxorubicin und Etoposid Derivaten genutzt (W. Wu et al. Cancer Res. 2006, 66, 970; L.Stern et al; Bioconjugate Chem. 2009, 20, 500; K.M. Bajjuri et al. ChemMedChem 2011, 6, 54).

In US 2015/0343083 A1 werden Legumain-spaltbare Peptid-Wirkstoff-Konjugate der Formel R-Y-Z-Asn-Linker-D beschrieben, wobei Linker p-Aminobenyzl carbamoyl oder p-Aminobenzyl carbonat darstellt, R ein aus unterschiedlichen chemischen Gruppen ausgewählter Rest ist, und D ein cytotoxischer Wirkstoff ist, Asn die Aminosäure Asparagin darstellt, und Y eine Aminosäure ausgewählt aus Ala, Thr, Ser, Leu, Arg, Pro, Val, Tyr und Phe darstellt, Z eine Aminosäure ausgewählt aus Ala, Thr, Asn und Pro darstellt wobei diese Aminosäuren stets in der natürlichen L-Konfiguration vorliegen.

In der WO 2014/062697 A2 werden Wirkstoff-Konjugate der Struktur $B-L(D)_x$ beschrieben, wobei B das Radikal eines Zelloberflächen-bindenden und/oder gerichteten Liganden ist, D ein Wirkstoffmolekül ist und L für einen polyvalenten Linker steht, der ein oder mehrere unnatürliche Aminosäuren enthält, wobei mindestens eine der unnatürliche Aminosäuren in der D-Form vorliegt. Die hier beschriebenen Konjugate unterscheiden sich aber in ihrer Struktur.

In der Arbeit von Mary A. Mathieu et al, (Molecular and Biochemical Parasitology, Bd. 121, Nr. 1,4 (2002-03-04), Seiten 99-105) wird die Substratspezifität von Schistosomen gegenüber humanem Legumain aus P1-P3 Peptid-Bibliotheken untersucht.

### Zusammenfassung der Erfindung

Die Erfindung betrifft die Aufgabe, die Tumorselektivität von cytotoxischen Wirkstoffen zu verbessern. Zur Lösung dieser Aufgabe stellt die Erfindung Prodrugs von cytotoxischen Wirkstoffmolekülen bereit. Hierbei ist an das Wirkstoffmolekül eine durch das Enyzm Legumain spaltbare Gruppe konjugiert, wobei der Wirkstoff und die durch Legumain spaltbare Gruppe entweder direkt über eine kovalente Bindung oder über einen *self-immolative linker* verbunden sind. Diese Prodrugs enthalten vorzugsweise einen Binder, der nach Bindung an ein Zielmolekül einer Tumorzelle von der Tumorzelle internalisiert und intrazellulär (vorzugsweise lysosomal) prozessiert wird. Dieser Binder kann entweder mit dem Wirkstoffmolekül gegebenenfalls über einen Linker verbunden sein, so daß zur Bildung eines aktiven Metaboliten beide Gruppen (Legumain spaltbare Gruppe und Binder) unabhängig voneinander prozessiert werden müssen, oder der Binder kann mit der durch das Enyzm Legumain spaltbaren Gruppe gegebenenfalls über einen Linker verbunden sein (so dass nach Spaltung der Legumain-spaltbaren Gruppe der Wirkstoff getrennt von dem Binder bzw. einem Derivat hiervon vorliegt). Als Wirkstoffmolekül wird ein Kinesin Spindel Protein-Inhibitor (KSP-Inhibitor) bevorzugt. Als Binder, der nach Bindung an ein Zielmolekül auf einer Tumorzelle von der Tumorzelle internalisiert und intrazellulär (vorzugsweise lysosomal) prozessiert wird, wird ein Antikörper bevorzugt. Besonders bevorzugt sind Antikörper-Wirkstoff-Konjugate (ADCs), wobei Antikörper und Wirkstoff über einen Linker, der eine Legumain spaltbare Gruppe aufweist, miteinander verbunden sind. Zudem sind bevorzugt Konjugate von Prodrugs mit Antikörpern (APDCs), wobei der Antikörper mit einem Prodrug des Wirkstoffs über einen Linker verbunden ist, wobei die Wirkung des Wirkstoffs durch eine Legumain spaltbare Gruppe maskiert ist. Die erfindungsgemäß verwendetete Legumain spaltbare Gruppe weist die Struktur X-L-Asn oder X-L-Asp auf, wobei X D-Ala, D-Pro, D- Val, D-Nva, D-Leu, D-Ile, D-Met, D-Phe, D-Tyr, D-Trp, D-Ser, D-Thr, D-Cys, D-Asn, D-Gln, D-Asp, D-Glu, D-Lys, D-Arg, D-Citrullin oder D-His bzw. die entsprechende N-alkylierte Aminosäure (C1-3 alkyliert, bevorzugt methyliert) darstellt und wobei bis zu zwei weitere Aminosäuren mit X N-verknüpft vorliegen können. Dabei bewirkt die Einführung der D-Aminosäure im Legumain-spaltbaren Linker eine Erhöhung der Stabilität in den Lysosomen von gesunden Organen (gezeigt in Kapitel C-1c). Wie anhand von repräsentativen Vergleichen mit geeigneten Referenzbeispielen gezeigt wurde (Kapitel C-1a), zeigen die erfindunsgemäßen ADCs und APDCs

mit einer D-Aminosäure im Linker eine hohe anti-Tumorwirkung, die den Epimeren mit all-L-Konfiguration im Linker (Referenzbeispielserien R3, 4, 5 und 9) überraschenderweise nicht oder kaum nachsteht (s. Kapitel C-1a).

Die erfindungsgemäßen Prodrugs eines Wirkstoffs D weisen die folgende allgemeine Formel Ia auf:

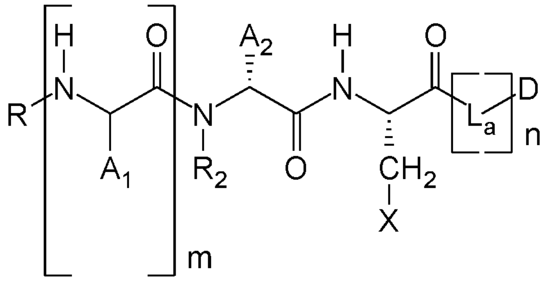

(Ia),

in der

m 0 bis 2 ist;

n 0 oder 1 ist;

X für $-C(=O)-NH_2$ oder -COOH steht,

$L_a$ für einen *self-immolative linker* steht,

$A_1$ für einen Rest einer der Aminosäuren Gly, Pro, Ala, Val, Nva, Leu, Ile, Met, Phe, Tyr, Trp, Ser, Thr, Cys, Asn, Gln, Asp, Glu, Lys, Arg, Citrullin oder His, oder einer der jeweiligen N-Alkyl-Aminosäuren steht,

$A_2$ für einen Rest einer der Aminosäuren D-Ala, D-Pro, D-Ser, D-Asn, D-Asp oder D-His steht,

$R_2$ für -H- oder $C_1$-$C_3$-Alkyl steht,

oder

$R_2$ für die Bindung zur Methylengruppe des Prolinringes steht, falls $A_2$ für einen Rest von D-Pro steht,

D für $-D_1-(L_b)_o-(LIG)_p$ steht,

$D_1$ für einen cytotoxischen Wirkstoff steht,

LIG für einen Binder steht, der nach Bindung an ein Zielmolekül einer Tumorzelle von der Tumorzelle internalisiert und intrazellulär, vorzugsweise lysosomal, prozessiert wird,

$L_b$ für einen Linker steht,

o und p jeweils unabhängig voneinander 0 oder 1 sind,

R für $Z_1$-(C=O)q- steht,

q 0 oder 1 ist,

$Z_1$ für eine $C_{1-10}$-Alkyl-, $C_{5-10}$-Aryl- oder $C_{6-10}$-Aralkyl-, $C_{5-10}$-Heteroalkyl-, $C_{1-10}$-Alkyl-O-$C_{6-10}$-Aryl-, $C_{5-10}$-Heterocycloalkyl-, Heteroaryl-, Heteroaryl-alkyl-, $C_{5-10}$-Heteroaryl-alkoxy-, $C_{1-10}$-Alkoxy-, $C_{6-10}$-Aryl-$C_{1-10}$-alkyloxy-, $C_{6-10}$-Aryloxy- oder $C_{6-10}$-Aralkoxy-, $C_{5-10}$-Heteroalkoxy-, $C_{1-10}$-Alkyl-O-$C_{6-10}$-Aryloxy- oder $C_{5-10}$-Heterocyclo-alkoxy-Gruppe steht, die ein- oder mehrfach mit $-NH_2$, -C(=O)-, -NH-Alkyl, $-N(Alkyl)_2$, -NH-C(=O)-Alkyl, -N(Alkyl)-C(=O)-Alkyl, $-S(=O)_3-H$, $-S(=O)_2-NH_2$, $-S(=O)_2-N(Alkyl)_2$, -COOH, $-C(=O)NH_2$, $-C(=O)-N(Alkyl)_2$ oder -OH substituiert sein kann, oder für -H oder eine Gruppe $-(CH_2)_{0-1}-O_X-(CH_2CH_2O)_V-R^1$ steht,

x 0 oder 1 ist

v eine Zahl von 1 bis 20 ist,

$R^1$ für -H, -Alkyl, $-CH_2-COOH$, $-CH_2-CH_2-COOH$ oder $-CH_2-CH_2-NH_2$ steht,

oder

R für LIG-$(L_c)_r$- steht,

LIG für einen Binder steht, der nach Bindung an ein Zielmolekül auf einer Tumorzelle von der Tumorzelle internalisiert und intrazellulär, vorzugsweise lysosomal, prozessiert wird,

$L_C$ für einen Linker steht und

r 0 oder 1 ist.

Hierbei steht $R^1$ in der Bedeutung von Alkyl vorzugsweise für $C_{1-12}$-Alkyl.

Vorzugsweise steht der unter $A_1$ und $A_2$ genannte Rest, der sich von einer der jeweiligen N-Alkyl Aminosäuren ableitet, für eine $C_1$-$C_3$ -alkylierte Aminosäure, besonders bevorzugt für eine methylierte Aminosäure.

Vorzugsweise ist der Binder (LIG) ein Antikörper oder eine Antigen-bindendes hiervon. Der Antikörper ist vorzugsweise ein humaner, humanisierter oder chimärer monoklonaler Antikörper oder ein Antigen-bindendes Fragment hiervon, insbesondere ein anti-TWEAKR-Antikörper, ein anti-EGFR-Antikörper, ein anti-B7H3-Antikörper oder ein anti-HER2-Antikörper oder ein Antigen-bindendes Fragment von diesen. Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

## Beschreibung der Abbildungen

Fig. 1 zeigt die Strategie der Transglutaminase-katalysierten konjugationsstellenspezifischen Funktionalisierung aglykosylierter Antikörper.

Fig. 2 zeigt Beispiele für nacheinander geschaltete enzymatische Schritte zur Wirkstofffreisetzung z.B. durch Histone Deacetylase und Cathepsin L gemäß Nat. Commun., 2013, 4, 2735.

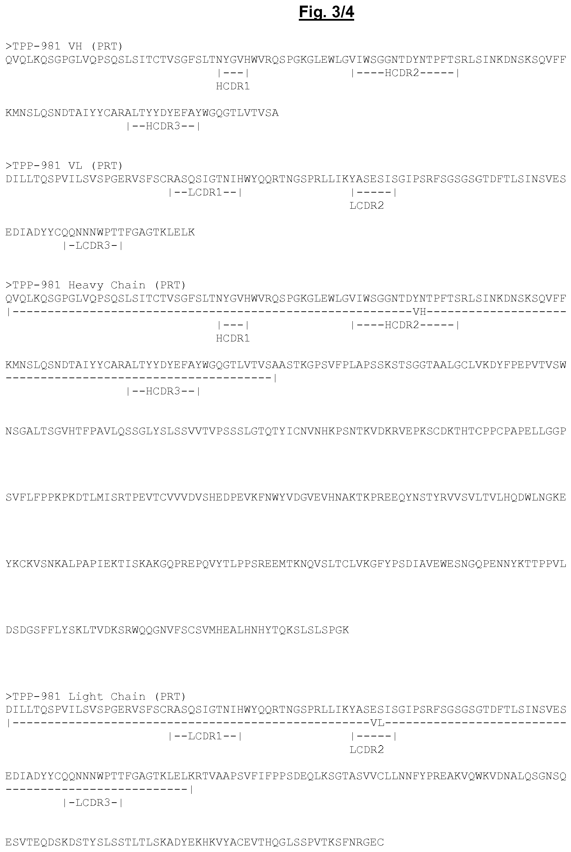

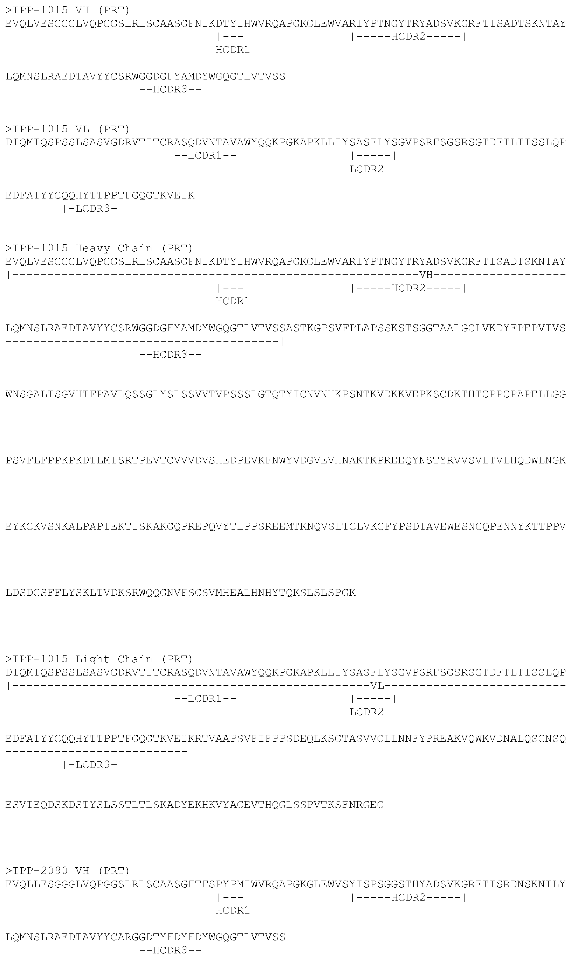

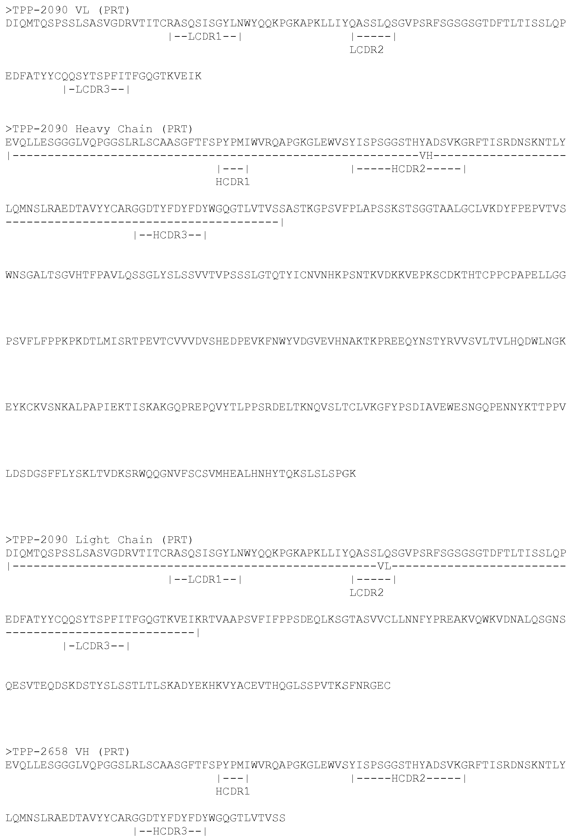

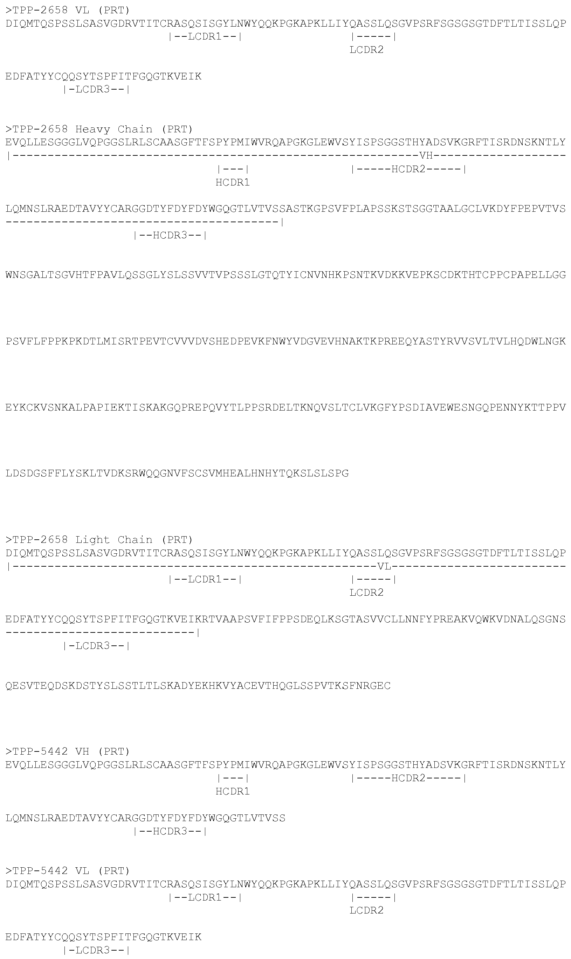

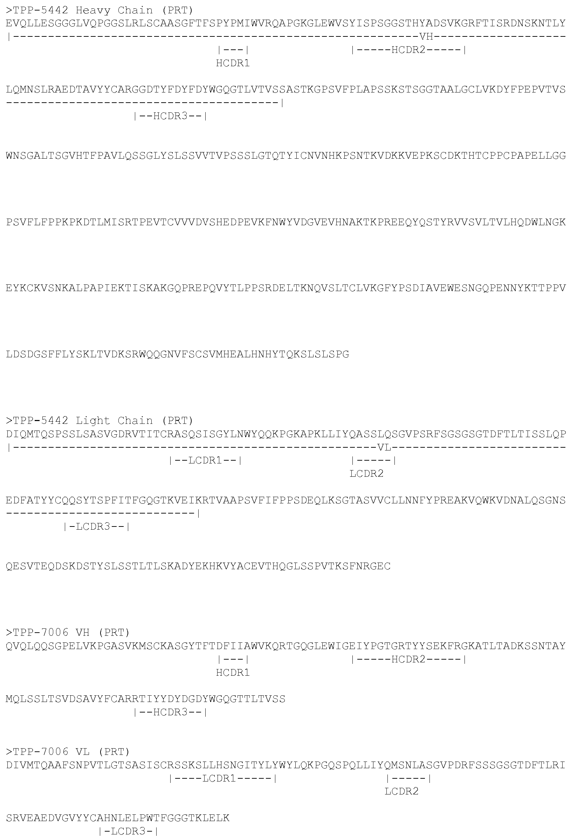

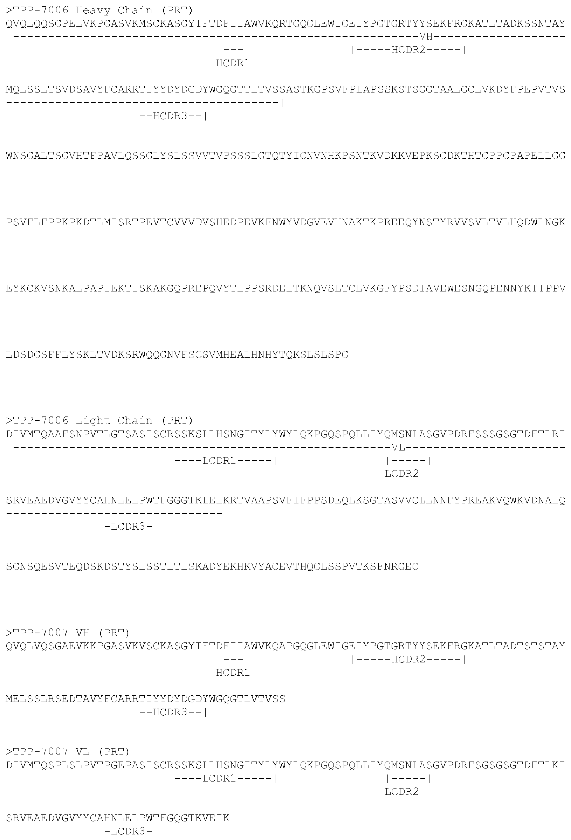

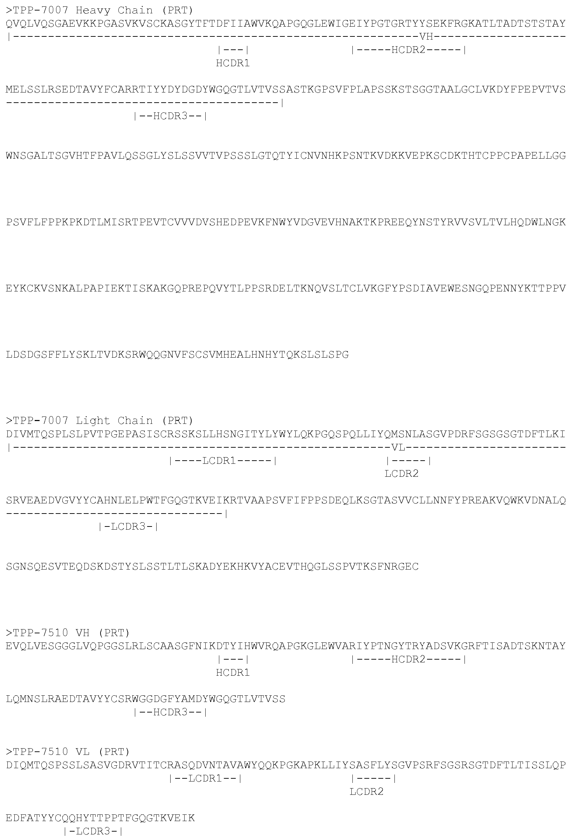

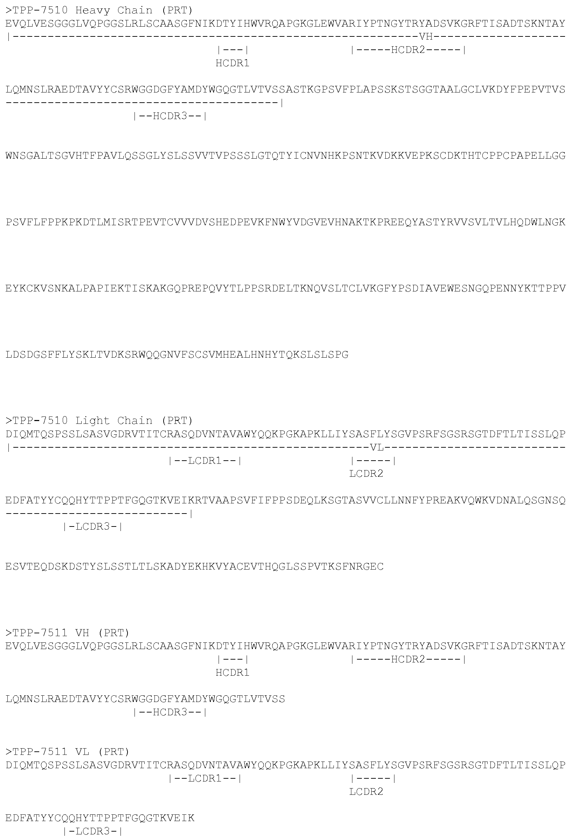

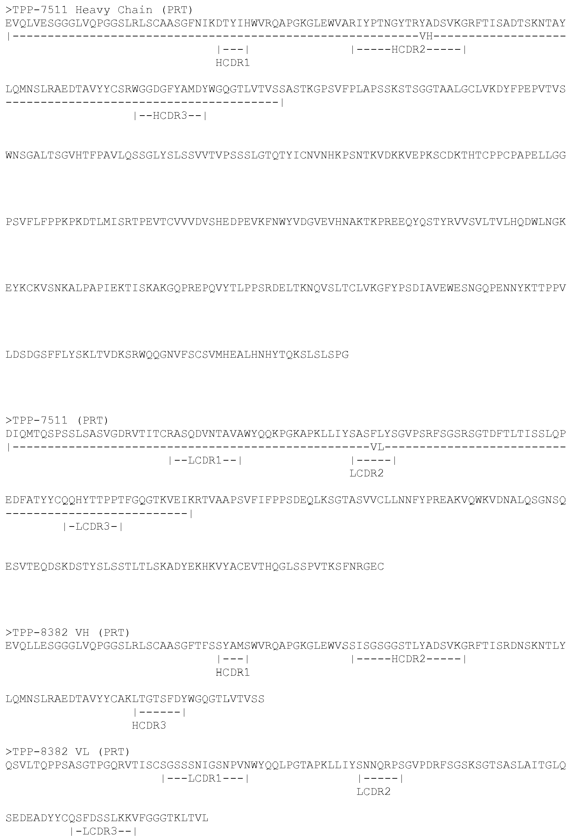

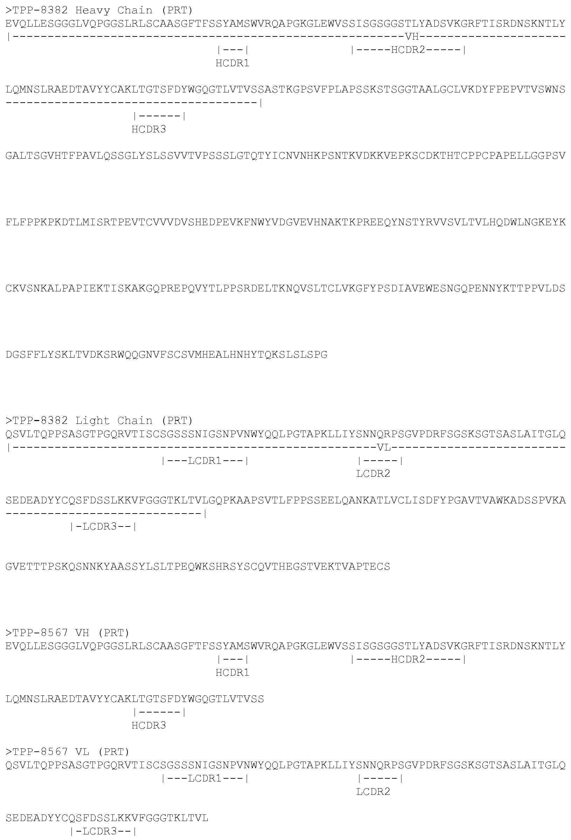

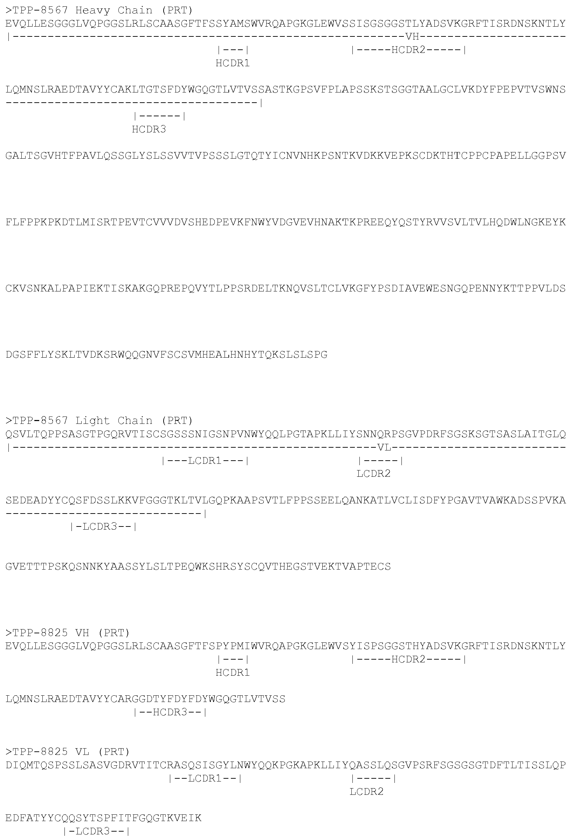

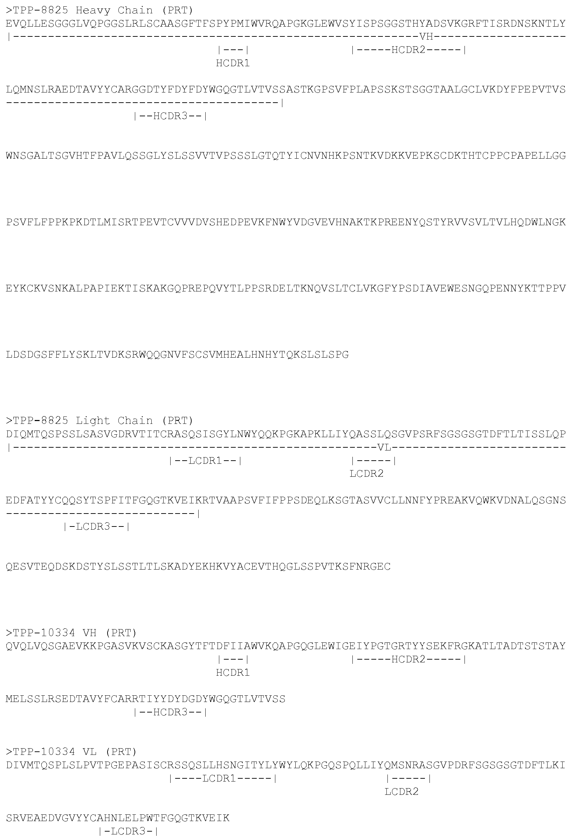

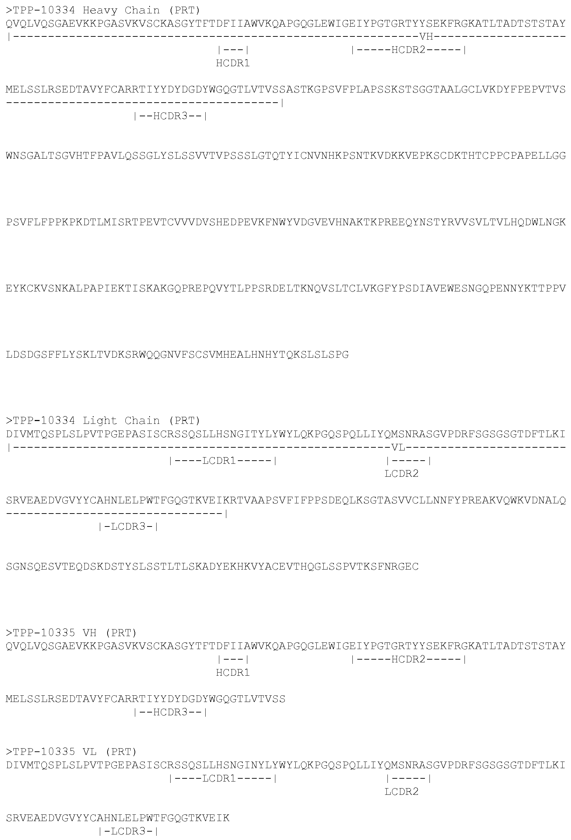

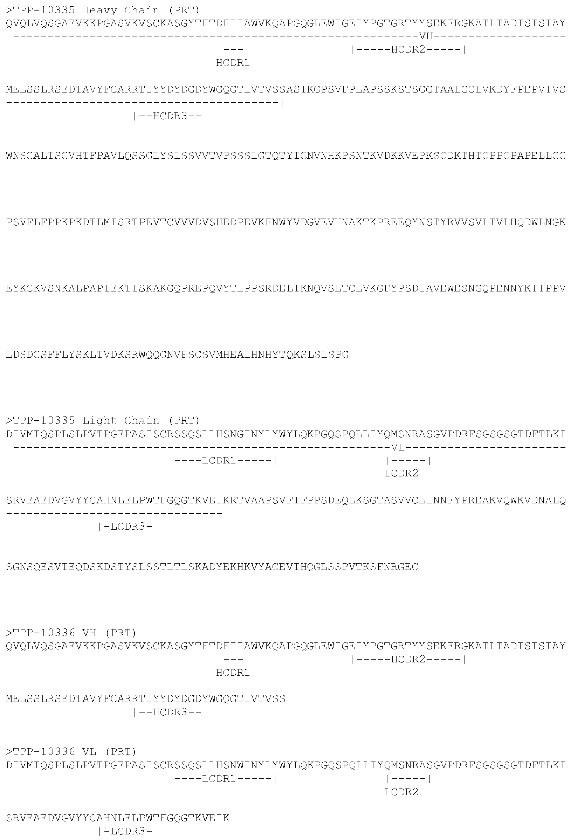

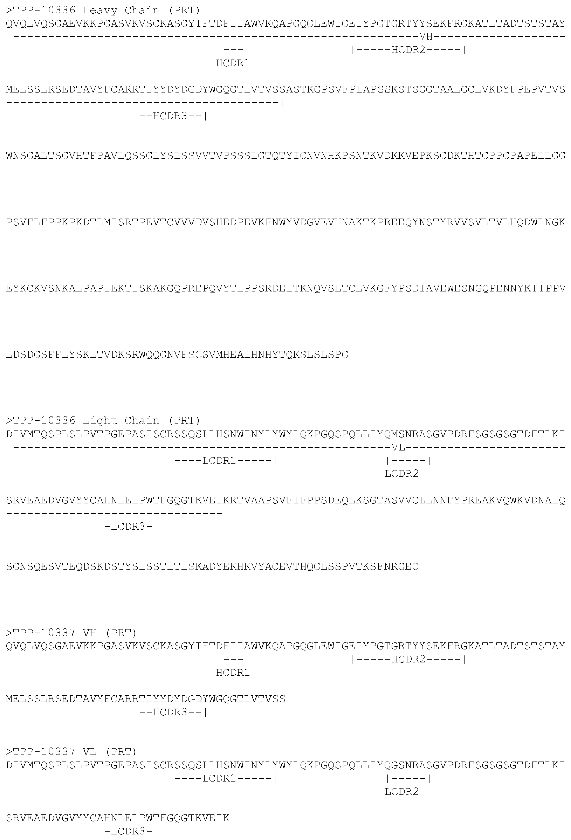

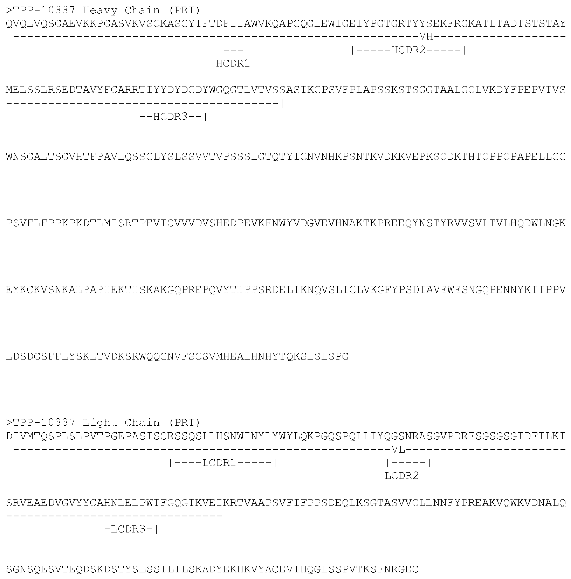

Fig. 3 zeigt annotierte Sequenzen von bevorzugten Antikörpern für Binder-WirkstoffKonjugate. Gezeigt sind die Proteinsequenzen der schweren und leichten Ketten der IgGs, sowie die VH und VL Regionen dieser Antikörper. Unterhalb der Sequenzen werden wichtige Regionen annotiert (VH und VL Regionen in IgGs, und die CDR Regionen (H-CDR1, H-CDR2, H-CDR3, L-CDR1, L-CDR2, L-CDR3)).

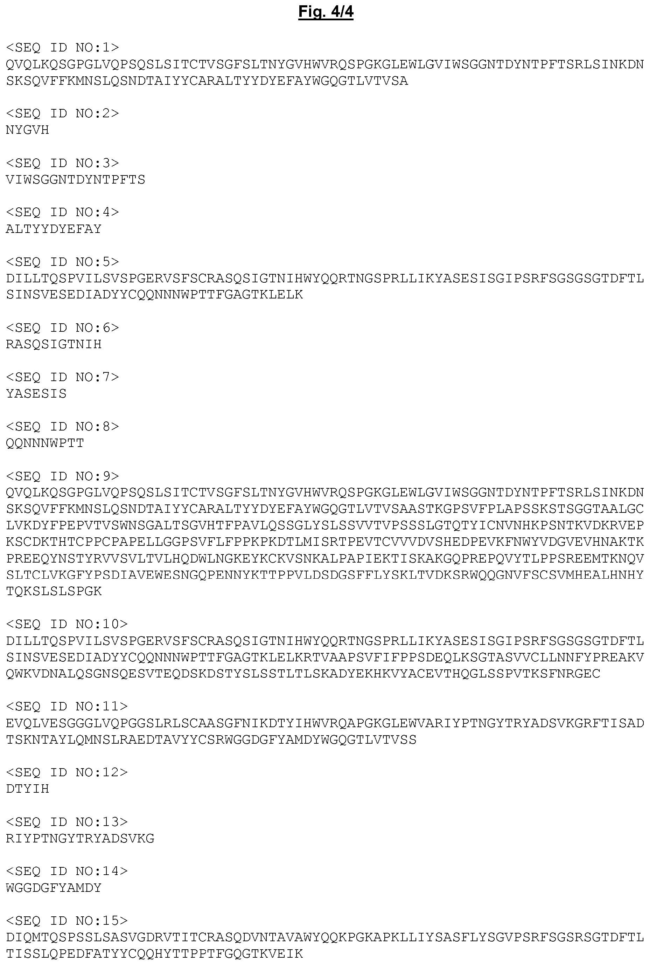

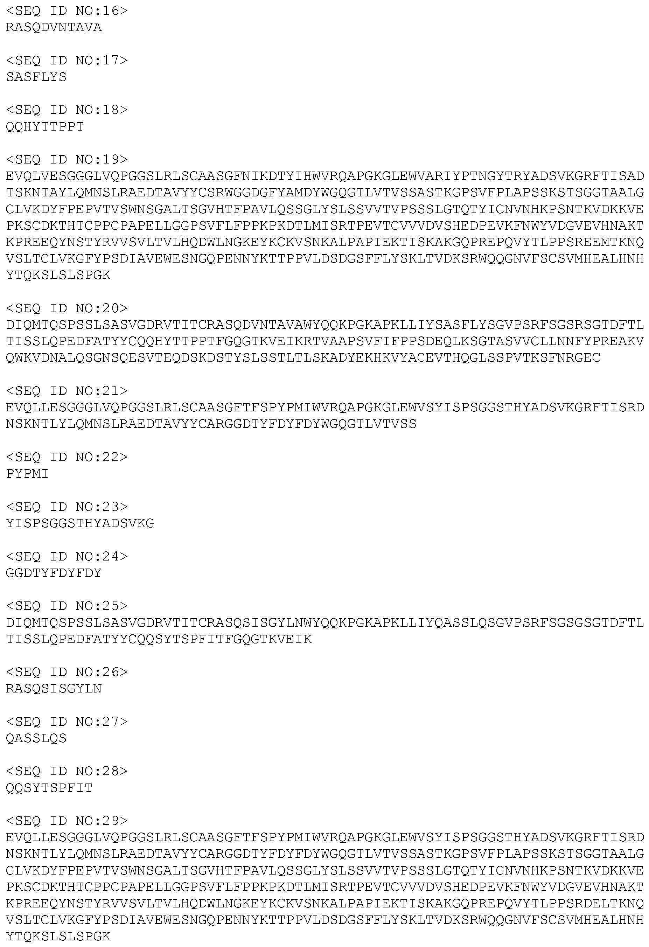

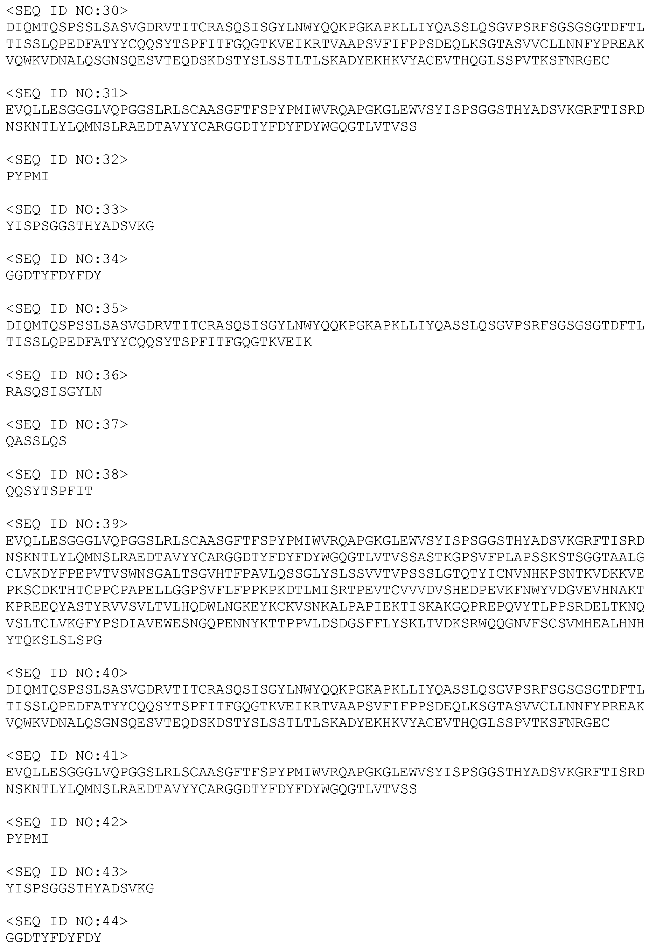

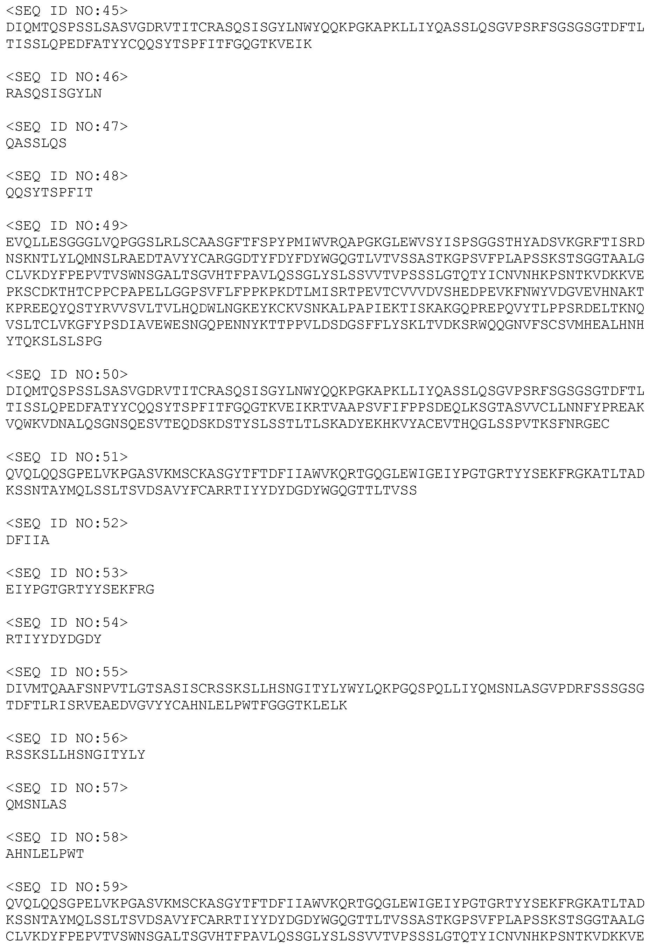

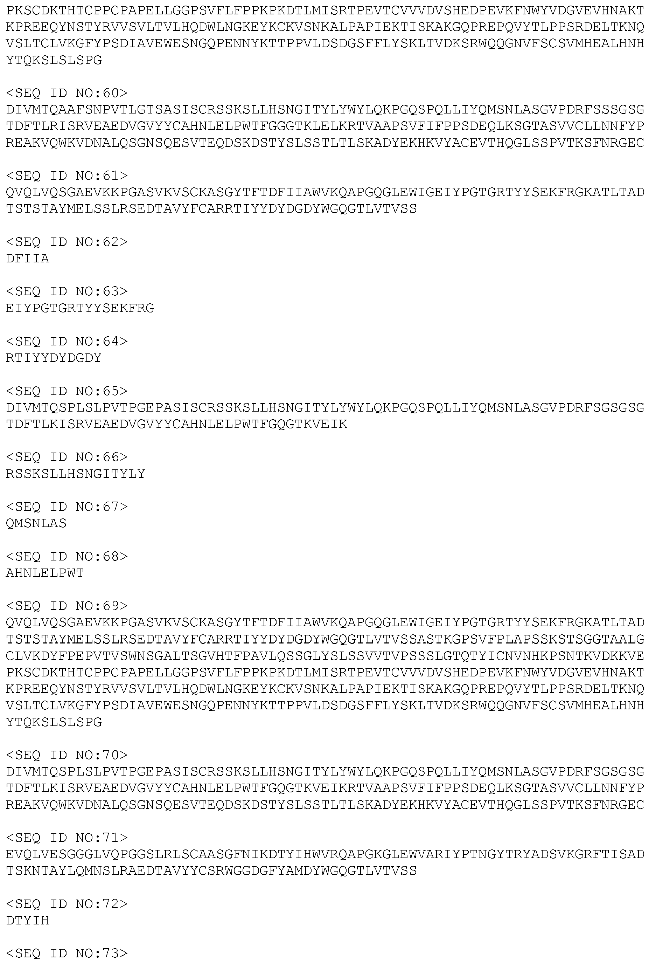

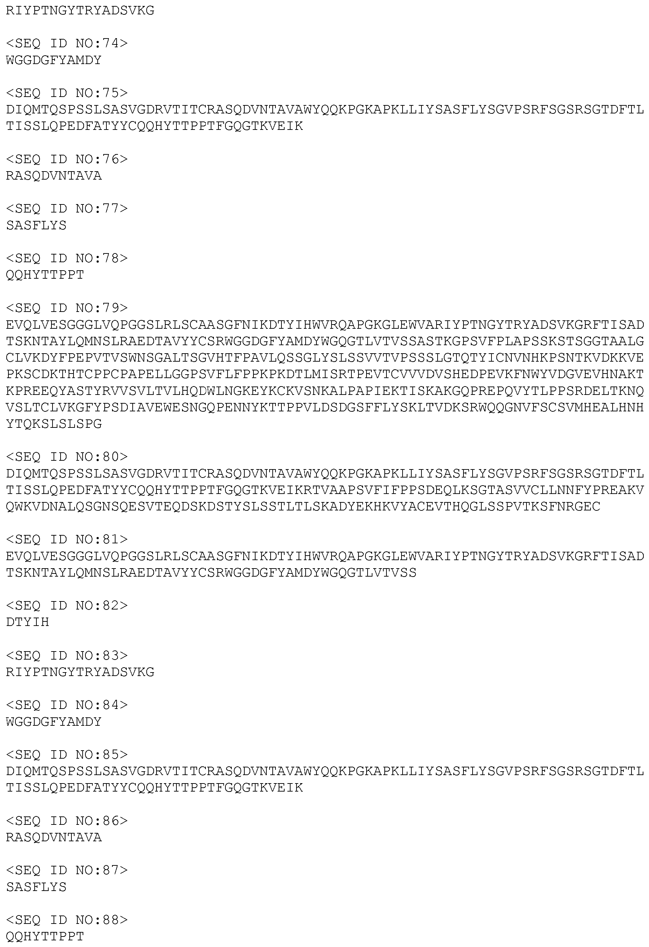

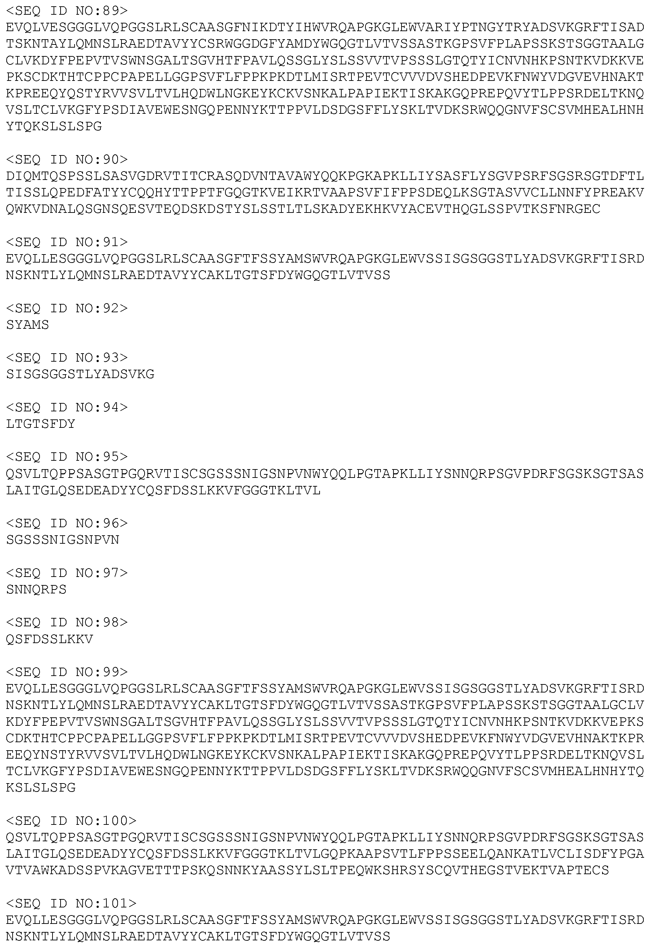

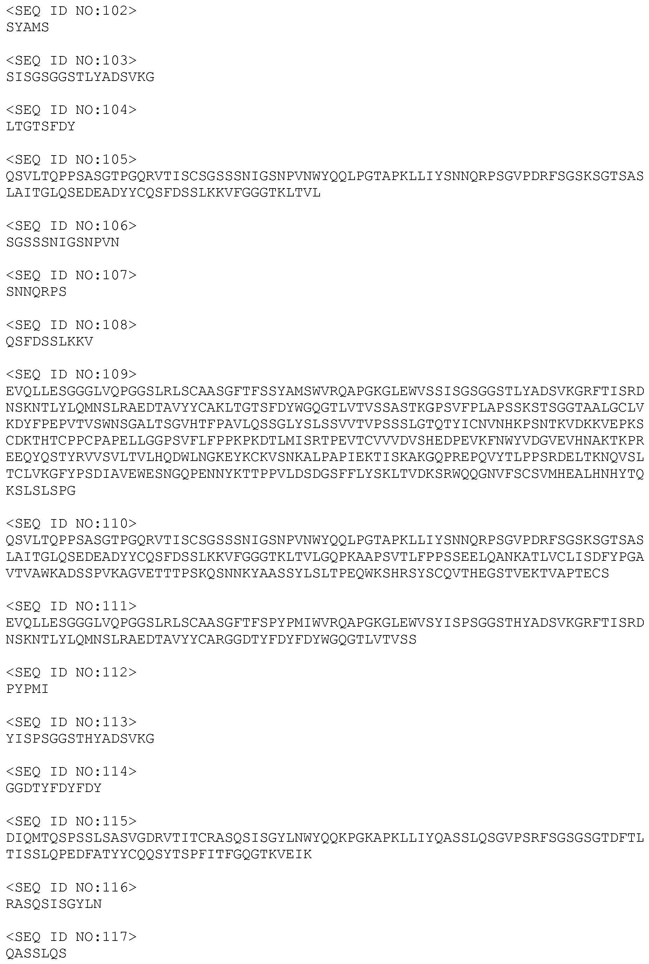

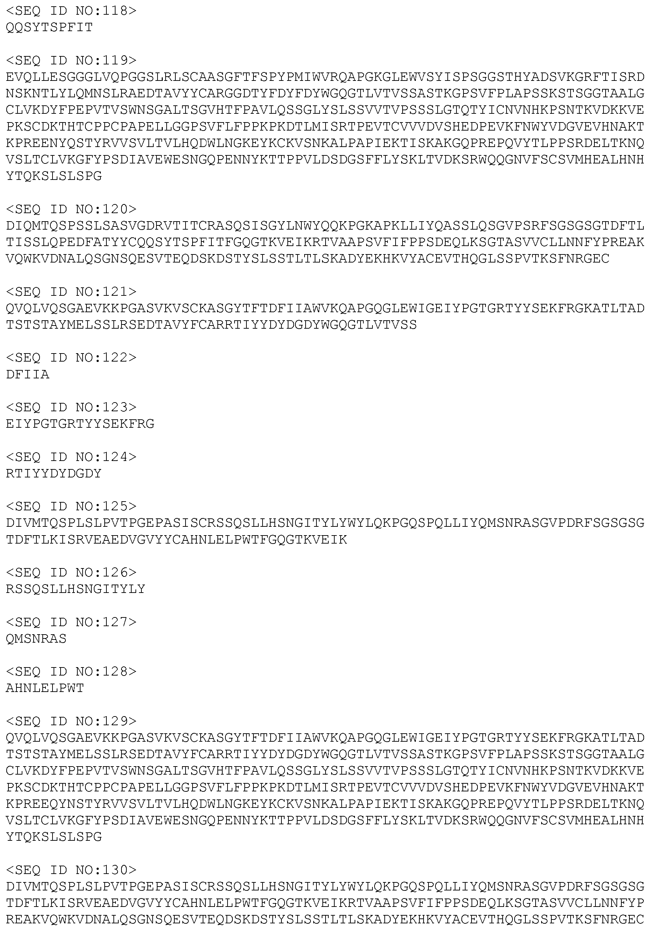

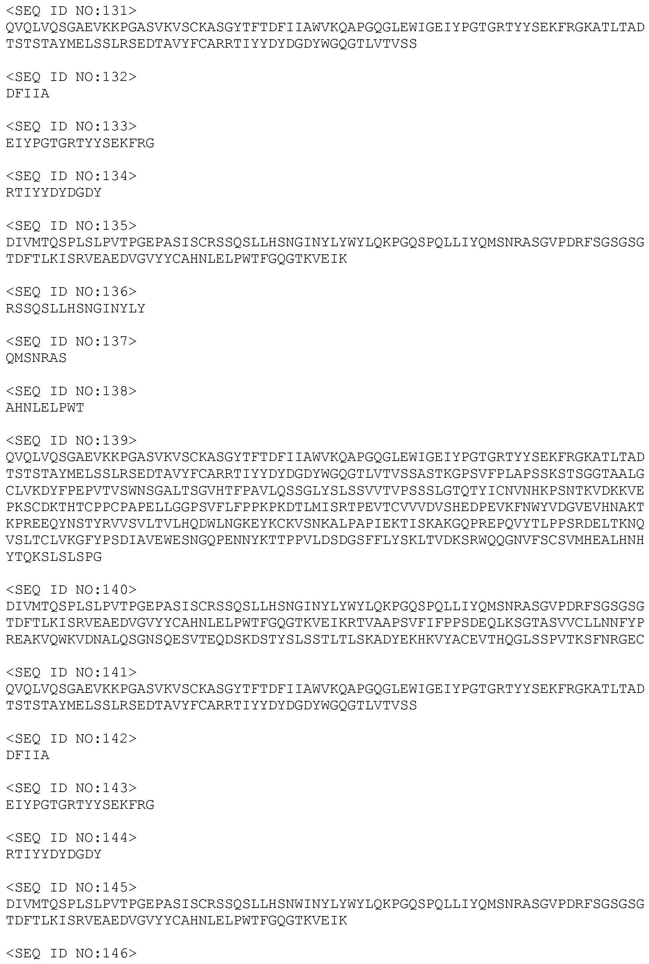

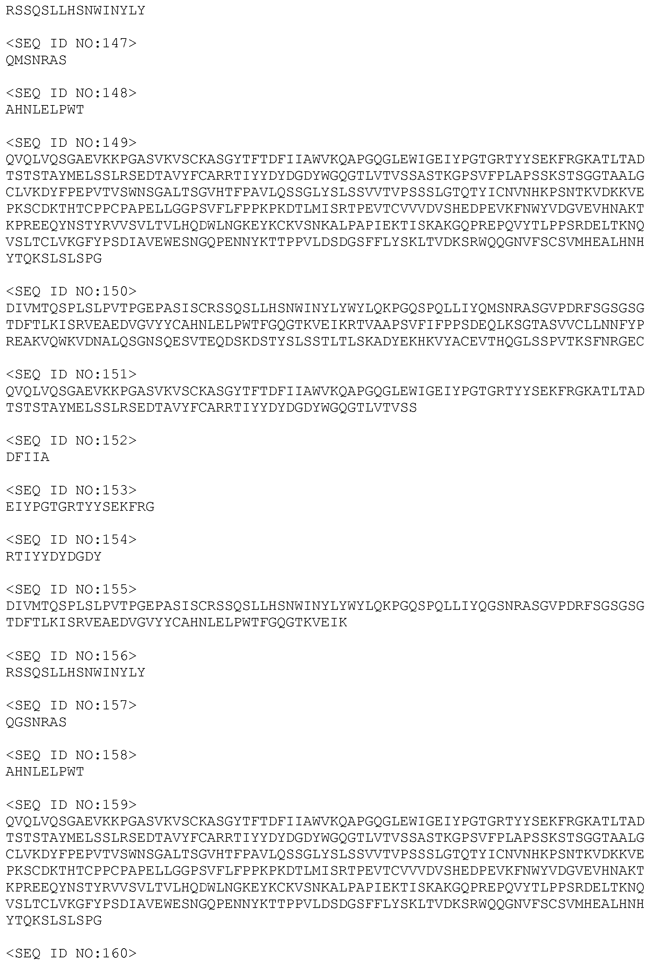

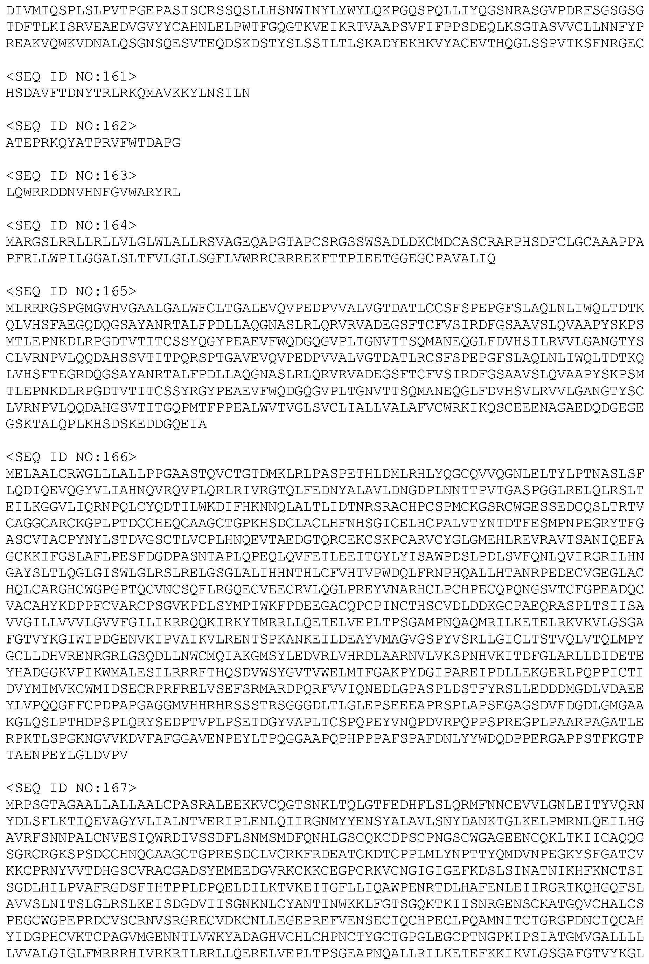

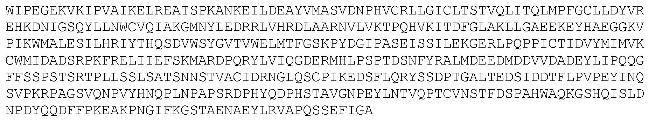

Fig. 4 zeigt das Sequenzlisting von Sequenzen der bevorzugten Antikörper für Binder Wirkstoff-Konjugate und von Sequenzen der Zielproteine.

## Detaillierte Beschreibung der Erfindung

Im Folgenden werden zunächst erfindungsgemäß verwendbare die Legumain spaltbaren Gruppen sowie die cytotoxische Wirkstoffe D, die gegebenenfalls über einen *self-immolative linker* miteinander verbunden sind, beschrieben. Anschließend wird der erfindungsgemäß bevorzugte Binder LIG, der nach Bindung an ein Zielmolekül einer Tumorzelle von der Tumorzelle internalisiert und intrazellulär (vorzugsweise lysosomal) prozessiert wird, beschrieben. Die verschiedenen Elemente der erfindungsgemäßen Verbindungen können ohne Einschränkung in beliebiger Kombination verwendet werden. Insbesondere können die jeweils als bevorzugt bzw. besonders bevorzugt dargestellten Wirkstoffe D in Kombination mit den jeweils als bevorzugt bzw. besonders bevorzugt dargestellten Binder LIG, ggf. in Kombination mit den jeweils als bevorzugt bzw. besonders bevorzugt dargestellten Linkern verwendet werden.

**Durch Legumain spaltbare Gruppe**

Die erfindungsgemäßen Verbindungen der allgemeinen Formel (Ia) weisen eine durch Legumain spaltbare Gruppe der Formel (Ia') auf

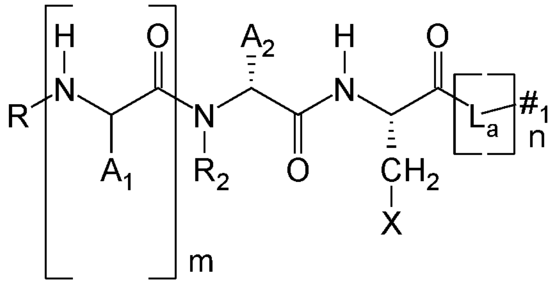

(Ia'),

in der

m eine Zahl von 0 bis 2 ist,
n 0 oder 1 ist,
X für -C(=O)-$NH_2$ oder -COOH steht,
$L_a$ für einen *self-immolative linker* steht,
$A_1$ für einen Rest einer der Aminosäuren Gly, Pro, Ala, Val, Nva, Leu, Ile, Met, Phe, Tyr, Trp, Ser, Thr, Cys, Asn, Gln, Asp, Glu, Lys, Arg, Citrullin oder His, oder einer der jeweiligen N-Alkyl-Aminosäuren steht,
$A_2$ für einen Rest einer der Aminosäuren D-Ala, D-Pro, D-Ser, D-Asn, D-Asp oder D-His steht,
$R_2$ für -H- oder $C_1$-$C_3$-Alkyl steht,

oder

$R_2$ für die Bindung zur Methylengruppe des Prolinringes steht, falls $A_2$ für einen Rest von D-Pro steht,
R für $Z_1$-(C=O)q- steht,
q 0 oder 1 ist,
$Z_1$ für eine $C_{1-10}$-Alkyl-, $C_{5-10}$-Aryl- oder $C_{6-10}$-Aralkyl-, $C_{5-10}$-Heteroalkyl-, $C_{1-10}$-Alkyl-O-$C_{6-10}$-Aryl-, $C_{5-10}$-Heterocycloalkyl-, Heteroaryl-, Heteroaryl-alkyl-, $C_{5-10}$-Heteroaryl-alkoxy-, $C_{1-10}$-Alkoxy-, $C_{6-10}$-Aryloxy-, $C_{6-10}$-Aryl-$C_{1-10}$-alkyloxy- oder $C_{6-10}$-Aralkoxy-, $C_{5-10}$-Heteroalkoxy-, $C_{1-10}$-Alkyl-O-$C_{6-10}$-Aryloxy- oder $C_{5-10}$-Heterocycloalkoxy-Gruppe steht, die ein- oder mehrfach mit -$NH_2$, -C(=O)-, -NH-Alkyl, -$N(Alkyl)_2$, -NH-C(=O)-Alkyl, -N(Alkyl)-C(=O)-Alkyl, -$S(=O)_3$-H, -$S(=O)_2$-$NH_2$, -$S(=O)_2$-$N(Alkyl)_2$, -COOH, -C(=O)-$NH_2$, -C(=O)-$N(Alkyl)_2$ oder -OH substituiert sein kann, oder für -H oder eine Gruppe -$(CH_2)_{0-1}$-$O_X$-$(CH_2CH_2O)_V$-$R^1$ steht,
x 0 oder 1 ist
v eine Zahl von 1 bis 20 ist,
$R^1$ für -H, -Alkyl, -$CH_2$-COOH, -$CH_2$-$CH_2$-COOH, oder -$CH_2$-$CH_2$-$NH_2$ steht,

oder

R für LIG-$(L_c)_r$- steht,
LIG für einen Binder steht, der nach Bindung an ein Zielmolekül einer Tumorzelle von der Tumorzelle internalisiert und intrazellulär, vorzugsweise lysosomal, prozessiert wird,
$L_C$ für einen Linker steht,
r 0 oder 1 ist und
#1 für die Bindung an den cytotoxischen Wirkstoff steht.

Hierbei steht $R^1$ in der Bedeutung von Alkyl vorzugsweise für $C_{1-12}$-Alkyl und m vorzugsweise für 1.
Wenn R für $Z_1$-(C(=O))q- steht, wird die durch Legumain spaltbare Gruppe der Formel Ia′ auch als durch Legumain spaltbare Kopfgruppe bezeichnet.
Wenn R für LIG-$(L_c)_r$- steht, wird die durch Legumain spaltbare Gruppe der Formel Ia′ auch als durch Legumain spaltbarer Linker bezeichnet.
Wenn die durch Legumain spaltbare Gruppe der Formel Ia′ eine durch Legumain spaltbare Kopfgruppe bezeichnet, ist q vorzugsweise 1.

Unter einer Legumain spaltbaren Kopfgruppe ist eine Gruppe zu verstehen, die eine für die Wirkung wichtige Aminogruppe des Wirkstoffes (vorzugsweise ein KSP- Inhibitor) bioreversibel blockiert.

X ist vorzugsweise -C(=O)-$NH_2$.

$A_2$ ist vorzugsweise ein Rest, der sich von einer der Aminosäuren D-Ala oder D-Pro ableitet.

Weiterhin sind Reste bevorzugt, die sich von D-Asp, D-Asn, D-His und D-Ser ableiten.

Wenn $A_2$ ein Rest ist, der sich von einer der Aminosäuren D-Ala oder D-Pro ableitet, dann weist die durch Legumain spaltbare Gruppe der allgemeinen Formel (Ia′)die folgenden allgemeinen Formeln (Ia″) und (Ia‴) auf:

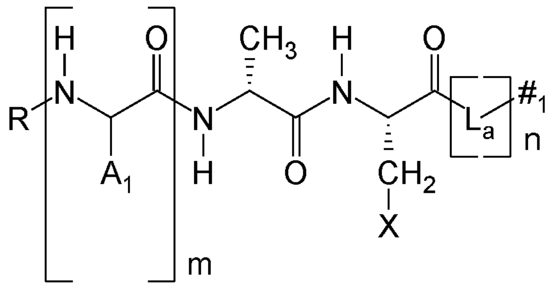

(Ia“)

und

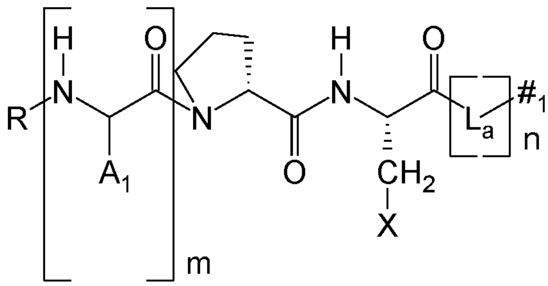

(Ia“‘)

Wenn $A_2$ in der allgemeinen Formel (Ia′) nicht D-Prolin ist, steht $R_2$ vorzugsweise für-H oder eine Methylgruppe, besonders bevorzugt ist-H.

In der durch Legumain spaltbaren Gruppe der Formel Ia′ können die Aminosäurereste $A_1$ sowohl in der L-Konfiguration als auch in der D-Konfiguration vorliegen. $A_1$ leitet sich vorzugsweise von Ala oder Val ab.

Bevorzugt ist L-Ala, D-Ala oder L-Val.

Besonders bevorzugt ist L-Ala.

In der durch Legumain spaltbaren Schutzgruppe ist q vorzugsweise 1.

$Z_1$ stellt vorzugsweise eine $C_{1-10}$-Alkyl-, $C_{6-10}$-Aralkyl-, $C_{5-10}$-Heteroaryl-alkyl-, $C_{6-10}$-Aralkoxy-, $C_{6-10}$-Aryl-$C_{1-10}$-alkyloxy- oder $C_{5-10}$-Heteroaryl-alkoxy-Gruppe oder eine Gruppe $-(CH_2)_{0-1}-O_X-(CH_2CH_2O)_V-R^1$ dar, in denen x, v und $R^1$ die oben angegeben Bedeutungen haben.

Als "Rest, der sich von einer der Aminosäuren ableitet", wird wie in der Chemie üblich als die Seitengruppen der Aminosäuren bezeichnet. Im Fall von Alanin also -$CH_3$ usw.

### *Self-immolative linker $L_a$*

Um eine effiziente Freisetzung des freien Wirkstoffs zu gewährleisten, können gegebenenfalls auch sogenannte self-immolative Linkerelemente ($L_a$) zwischen enzymatischer Spaltstelle und Wirkstoff eingebaut werden (Anticancer Agents in Medicinal Chemistry, 2008, 8, 618-637). Die Freisetzung des Wirkstoffs kann dabei nach verschiedenen Mechanismen erfolgen, beispielsweise nach initialer enzymatischer Freisetzung einer nukleophilen Gruppe durch anschließende Elimierung über eine elektronische Kaskade (Bioorg. Med. Chem., 1999, 7, 1597; J. Med. Chem., 2002, 45, 937; Bioorg. Med. Chem., 2002, 10, 71) oder durch Cyclisierung des entsprechenden Linkerelements (Bioorg. Med. Chem., 2003, 11, 2277; Bioorg. Med. Chem., 2007, 15, 4973; Bioorg. Med. Chem. Lett., 2007, 17, 2241) oder durch eine Kombination aus beidem (Angew. Chem. Inter. Ed., 2005, 44, 4378). Beispiele für solche Linkerelemente sind in der Abbildung dargestellt:

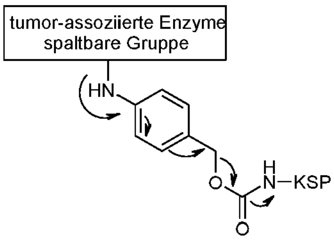


Elimination linker

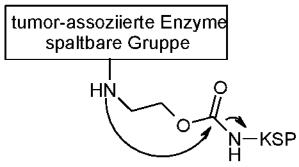


Cyclisation linker

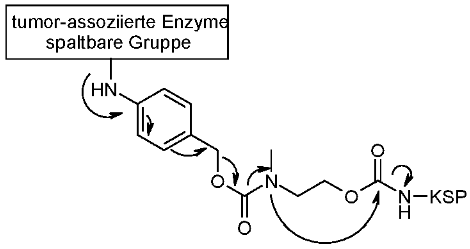


Elongated linker

Als $L_a$ werden erfindungsgemäß eine der folgenden Gruppen bevorzugt:

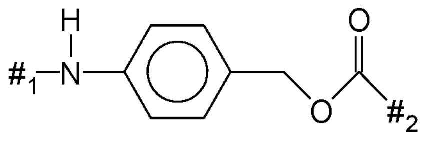

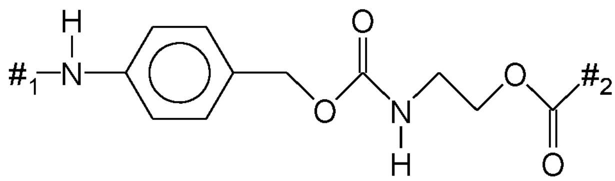

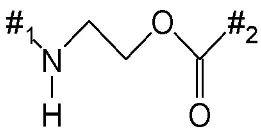

wobei $\#_1$ die Bindung zur Carbonylgruppe und $\#_2$ die Bindung zur Hydroxyl- bzw. Aminogruppe von D1 darstellt.

## Cytotoxische Wirkstoffe

In den erfindungsgemäßen Verbindungen der Formel Ia stellt D die Gruppe $-D_1-(L_b)_o-(LIG)_p$ dar, wobei

| | |
|---|---|
| $D_1$ | ein cytotoxischer Wirkstoff ist, |
| LIG | einen Binder darstellt, der nach Bindung an ein Zielmolekül einer Tumorzelle von der Tumorzelle internalisiert und intrazellulär und vorzugsweise lysosomal, prozessiert wird, |
| $L_b$ | einen Linker darstellt und |
| o und p | unabhängig voneinander 0 oder 1 sind. |

Als cytotoxischer Wirkstoff wird vorzugsweise Mitomycin, Doxorubicin, Aminopterin, Actinomycin, Bleomycin, 9-Amino-Camptothecin, n8-Acetyl-spermidin, 1-(2-Chloroethyl)-1,2-dimethansulfonyl-hydrazid, Tallysomycin, Cytarabin, Etoposid, Camptothecin, Taxol, Esperamicin, Podophyllotoxin, Anguidin, Vincristin, Vinblastin, Morpholin-doxorubicin, n-(5,5-diacetoxy-pentyl)-doxorubicin, Duocarmycin, Auristatin, Pyrrolobenzodiazepin-Derivate, Indolinobenzodiazepin (IGN)-Derivate und mono-Imin-IGN-Derivate, Calicheamicin, Daunorubicin, Camptophecin DX8951 (Exatecan) oder ein Kinesin Spindel Protein-Inhibitor (KSP-Inhibitor) verwendet, wobei der Wirkstoff über seine Hydroxyl- oder Aminogruppe an $L_a$ (bei n=1) oder die Carbonylgruppe (bei n=0) gemäß der allgemeinen Formel (Ia) gebunden ist. Eine entsprechende Derivatisierung dieser Wirkstoffe kann auf Grundlage bekannter Methoden erfolgen (siehe z.B. Synthesis, 1999, 1505 bzgl. Duocarmycin, Nat. Struct. Biol., 2002, 9, 337, Journal of Med. Chem., 2010, 53(3), 1043 bzgl. Camptothecin, ChemMedChem,. 2011, 6(1), 54 bzgl. Auristatin, Mol. Cancer. Ther., 2005, 4, 751 bzgl. Doxorubicin und J. Biol. Chem, 2008, 283, 9318 bzgl. Pyrrolobenzodiazepin-Derivate (PBD-Derivate); s.a. J.Med.Chem 2013, 56, 7564 und weitere

Referenzen in der Einleitung, J.Med.Chem. 2001, 44, 1341, Oncology Reports 2011, 26, 629)).

Besonders bevorzugt werden solche cytotoxischen Wirkstoffe, die eine für ihre Wirksamkeit essentielle freie Hydroxyl- oder Aminogruppe aufweisen, insbesondere solche, die eine für ihre Wirksamkeit essentielle freie Aminogruppe aufweisen. Durch die Kopplung der Legumain spaltbaren Gruppe an eine solche Gruppe kann die Wirksamkeit des cytotoxischen Wirkstoffs maskiert werden. Zu dieser Gruppe der Wirkstoffe gehört z.B. Doxorubicin, welches die folgende Formel aufweist:

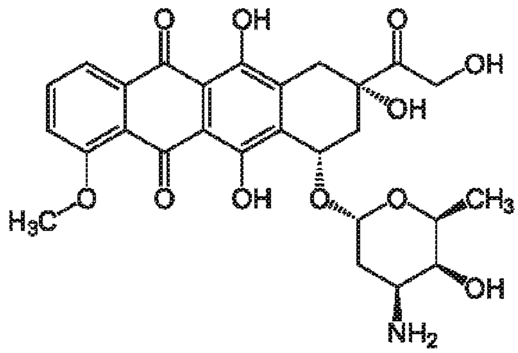

Durch Konjugation der Legumain spaltbaren Gruppe an die freie Aminogruppe des Doxorubicins kann dessen Wirksamkeit maskiert werden.

Weiterhin sind erfindungsgemäß Kinesin Spindel Protein-Inhibitoren als cytotoxische Wirkstoffe bevorzugt, wie sie in WO2015/096982 offenbart sind. Insbesondere bevorzugt sind solche Kinesin Spindel Protein-Inhibitoren der folgenden Formel II:

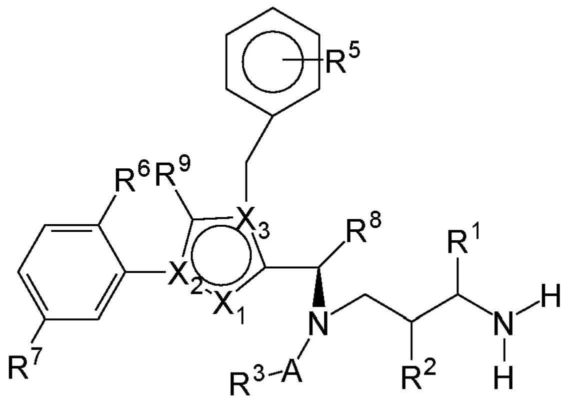

(II),

in der

$X_1$ für N,
$X_2$ für N und
$X_3$ für C steht;

oder

$X_1$ für N,
$X_2$ für C und
$X_3$ für N steht;

oder

$X_1$ für CH oder CF,
$X_2$ für C und

$X_3$ für N steht;

oder

$X_1$ für NH,
$X_2$ für C und
$X_3$ für C steht;

oder

$X_1$ für CH,
$X_2$ für N und
$X_3$ für C steht.

$R^1$ für -H, -L-#1, -MOD oder $-(CH_2)_{0-3}Z$ steht,
Z für -H, $-NHY^3$, $-OY^3$, $-SY^3$, Halogen, $-C(=O)-NY^1Y^2$, oder $-C(=O)-OY^3$ steht,
$Y^1$ und $Y^2$ unabhängig voneinander für -H, $-NH_2-(CH_2CH_2O)_{0-3}-(CH_2)_{0-3}Z'$ oder $-CH(CH_2W)Z'$ steht,
$Y^3$ für -H oder $-(CH_2)_{0-3}Z'$ steht,
Z′ für -H, $-NH_2$, $-S(=O)_3H$, -COOH, $-NH-C(=O)-CH_2-CH_2-CH(NH_2)-COOH$ oder $-(C(=O)-NH-CHY^4)_{1-3}-COOH$ steht;
W für -H oder -OH steht,
$Y^4$ für lineares oder verzweigtes $C_{1-6}$Alkyl- steht, welches gegebenenfalls mit $-NH-C(=O)-NH_2$ substituiert ist, oder für Aryl oder Benzyl steht, welches gegebenenfalls mit $-NH_2$ substituiert sein kann,

$R_2$ für -L-#1, -H, -MOD, $-C(=O)-CHY^4-NHY^5$ oder $-(CH_2)_{0-3}Z$ steht,
Z für -H, Halogen, $-OY^3$, $-SY^3$, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z′ für -H, $-S(=O)_3H$, $-NH_2$ oder -COOH steht;
$Y^4$ für lineares oder verzweigtes $C_{1-6}$Alkyl- steht, welches gegebenenfalls mit $-NH-C(=O)-NH_2$ substituiert ist oder für Aryl oder Benzyl steht, welches gegebenenfalls mit $-NH_2$ substituiert ist,
$Y^5$ für-H oder $-C(=O)-CHY^6-NH_2$ steht,
$Y^6$ für lineares oder verzweigtes $C_{1-6}$-Alkyl steht;

A für -C(=O)-, -S(=O)-, $-S(=O)_2-$, oder $-S(=O)_2-NH-$ steht,

$R^3$ für -L-#1, -MOD, oder eine gegebenenfalls substituierte Alkyl-, Cycloalkyl-, Aryl-, Heteroaryl-, Heteroalkyl-, Heterocycloalkyl-Gruppe steht, die mit ein bis drei OH-Gruppen, ein bis drei Halogenatomen, ein bis drei mono-, di- oder tri-halogenierten Alkylgruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei $-S(=O)_n$-Alkyl-Gruppen, ein bis drei $-S(=O)_2$-NH-Alkyl-Gruppen, 1-3 -NH-Alkyl-Gruppen, ein bis drei $-N(Alkyl)_2$-Gruppen, ein bis drei $NH_2$-Gruppen oder ein bis drei $-(CH_2)_{0-3}Z$-Gruppen substituiert sein können,
n 0, 1 oder 2 ist,
Z für -H, Halogen, $-OY^3$, $-SY^3$, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H, $-(CH_2)_{0-3}-CH-(NHC(=OCH_3)Z'$, $-(CH_2)_{0-3}-CH(NH_2)Z'$ oder $-(CH_2)_{0-3}Z'$ steht,
Z′ für -H, $-S(=O)_3H$, $-NH_2$ oder -COOH steht,
$R^5$ für -H, $-NH_2$, $-NO_2$, Halogen, -CN, $-CF_3$, $-OCF_3$, $-CH_2F$, $-CH_2F$, -SH oder $-(CH_2)_{0-3}Z$ steht,
Z für -H, $-OY^3$, $-SY^3$, Halogen, $-NHY^3$, $-C(=O)-NY^1Y^2$ oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ stehen,
Z’ für -H, $-S(=O)_3H$, $-NH_2$ oder -COOH steht,

$R^6$ und $R^7$ unabhängig voneinander für -H, -CN, $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, Hydroxy, $-NO_2$, $-NH_2$, -COOH oder Halogen stehen,
$R^8$ für $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, $C_{4-10}$-Cycloalkyl-, Fluor- $C_{4-10}$-Cycloalkyl- oder $-(CH_2)_{0-2}-(HZ^2)$ steht,

HZ$^2$ für einen 4 bis 7-gliedrigen Heterocyclus mit bis zu zwei Heteroatomen ausgewählt aus N, O und S steht, der mit -OH, -COOH, -$NH_2$ oder -L-#1 substituiert sein kann,

$R^9$ für -H, -F, -$CH_3$, -$CF_3$, -$CH_2F$ oder -$CHF_2$ steht,
-L-#1 für -$(L_b)_o$-$(LIG)_p$ steht,
LIG für einen Binder steht, der nach Bindung an ein Zielmolekül einer Tumorzelle von der Tumorzelle internalisiert und intrazellulär und vorzugsweise lysosomal, prozessiert wird,
$L_b$ für einen Linker steht,
o und p unabhängig voneinander für 0 oder 1 stehen,

-MOD für -$(NR^{10})_n$-$(G1)_o$-G2-G3 steht,
$R^{10}$ für -H oder $C_1$-$C_3$-Alkyl- steht,

G1 für -NH-C(=O)- , -C(=O)-NH- oder

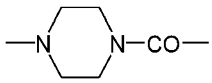

steht,

n 0 oder 1 ist,
o 0 oder 1 ist,
G2 für eine geradkettige oder verzweigte Kohlenwasserstoffkette mit 1 bis 20 Kohlenstoffatomen steht, die einfach oder mehrfach durch eine oder mehrere der Gruppen -O-, -S-, -S(=O)-, -$S(=O)_2$-, -NRY-, -$NR^yC(=O)$-, -C(=O)-$NR^y$-, -NRYNRY-, -$S(=O)_2$-$NR^yNR^y$-, -C(=O)-$NR^yNR^y$-C(=O)-, -$CR^x$=N-O unterbrochen sein kann, wobei die geradkettige oder verzweigte Kohlenwasserstoffkette mit -NH-C(=O)-$NH_2$, -COOH, -OH, -$NH_2$, Sulfonamid, Sulfon, Sulfoxid, oder Sulfonsäure substituiert sein kann,
G3 für -H oder -COOH steht,
$R^y$ für -H, Phenyl-, $C_1$-$C_{10}$-Alkyl-, $C_2$-$C_{10}$-Alkenyl-, oder $C_2$-$C_{10}$-Alkinyl- steht, die jeweils mit -NH-C(=O)-$NH_2$, -COOH, -OH, -$NH_2$, Sulfonamid, Sulfon, Sulfoxid, oder Sulfonsäure substituiert sein können,
$R^x$ für -H, $C_1$-$C_3$-Alkyl oder Phenyl steht,

sowie deren Salze, Solvate und Salze der Solvate.

Hierbei bevorzugt sond solche Verbindungen, in denen

$R^3$ für -L-#1 oder eine $C_{1-10}$-Alkyl-, $C_{6-10}$-Aryl- oder $C_{6-10}$-Aralkyl-, $C_{5-10}$-Heteroalkyl-, $C_{1-10}$-Alkyl-O-$C_{6-10}$-Aryl- oder $C_{5-10}$-Heterocycloalkyl-Gruppe steht, die mit ein bis drei OH-Gruppen, ein bis drei Halogenatomen, ein bis drei mono-, di- oder tri-halogenierten Alkylgruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei -$S(=O)_n$-Alkyl-Gruppen, ein bis drei -$S(=O)_2$-NH-Alkyl-Gruppen, 1-3 -NH-Alkyl-Gruppen, ein bis drei -$N(Alkyl)_2$-Gruppen, ein bis drei $NH_2$-Gruppen oder ein bis drei -$(CH_2)_{0-3}$Z-Gruppen substituiert sein können und

-MOD zumindest eine -COOH Gruppe aufweist.

Bevorzugt sind solche Verbindungen, in denen

$X_1$ für CH,
$X_2$ für C und
$X_3$ für N steht.

Durch Konjugation der Legumain spaltbaren Gruppe an die freie Aminogruppe der Verbindungen der Formel II kann dessen Wirksamkeit maskiert werden.

Diese erfindungsgemäß verwendeten Kinesin Spindel Protein-Inhibitoren besitzen eine für die Wirkung essentielle Aminogruppe. Durch Modifizierung dieser Aminogruppe mit Peptidderivaten wird die Wirkung gegenüber dem Kinesin-Spindelprotein blockiert und damit auch die Entfaltung einer cytotoxischen Wirkung inhibiert. Kann dieser Peptidrest jedoch durch Tumor-assoziierte Enzyme wie Legumain freigesetzt werden, so lässt sich die Wirkung gezielt im

Tumorgewebe wieder herstellen.

**Definitionen**

Der Begriff "substituiert" bedeutet, dass ein oder mehrere Wasserstoffe an dem bezeichneten Atom bzw. der bezeichneten Gruppe durch eine Auswahl aus der angegebenen Gruppe ersetzt ist/sind, mit der Maßgabe, dass die normale Wertigkeit des bezeichneten Atoms unter den vorliegenden Umständen nicht überschritten wird. Kombinationen von Substituenten und/oder Variablen sind zulässig.

Der Begriff "gegebenenfalls substituiert" bedeutet, dass die Anzahl an Substituenten gleich oder verschieden von Null sein kann. Wenn nicht anders angegeben, können gegebenenfalls substituierte Gruppen durch so viele fakultative Substituenten substituiert sein, wie sich durch Ersetzen eines Wasserstoffatoms durch einen Nicht-Wasserstoff-Substituenten an einem beliebigen verfügbaren Kohlenstoff- oder Stickstoff- oder Schwefelatom unterbringen lassen. Normalerweise kann die Anzahl an fakultativen Substituenten (falls vorhanden) 1, 2, 3, 4 oder 5, insbesondere von 1, 2 oder 3 sein.

So, wie hier verwendet, bedeutet der Ausdruck "ein- oder mehrfach", zum Beispiel in der Definition der Substituenten der Verbindungen der allgemeinen Formeln der vorliegenden Erfindung, "1, 2, 3, 4 oder 5, bevorzugt 1, 2, 3 oder 4, besonders bevorzugt 1, 2 oder 3, ganz besonders bevorzugt 1 oder 2".

Sind Reste in den erfindungsgemäßen Verbindungen substituiert, so können die Reste, wenn nicht anders angegeben, ein- oder mehrfach substituiert sein. Im Schutzbereich der vorliegenden Erfindung sind die Bedeutungen aller Reste, die mehrfach auftreten, unabhängig voneinander. Eine Substitution durch einen, zwei oder drei gleiche oder verschiedene Substituenten ist bevorzugt. Die Substitution durch einen Substituenten ist besonders bevorzugt.

Alkyl

Alkyl steht für einen linearen oder verzweigten, gesättigten, monovalenten Kohlenwasserstoffrest mit 1 bis 10 Kohlenstoffatomen ($C_1$-$C_{10}$-Alkyl), in der Regel 1 bis 6 ($C_1$-$C_6$-Alkyl), bevorzugt 1 bis 4 ($C_1$-$C_4$-Alkyl), und besonders bevorzugt 1 bis 3 Kohlenstoffatomen ($C_1$-$C_3$-Alkyl).

Beispielhaft und bevorzugt seien genannt:
Methyl-, Ethyl-, Propyl-, Butyl-, Pentyl-, Hexyl-, iso-Propyl-, iso-Butyl-, sec-Butyl, tert-Butyl-, iso-Pentyl-, 2-Methylbutyl-, 1-Methylbutyl-, 1-Ethylpropyl-, 1,2-Dimethylpropyl, neo-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- und 1,2-Dimethylbutyl-.

Besonders bevorzugt ist ein Methyl-, Ethyl-, Propyl-, Isopropyl- und tert-Butylrest.

Heteroalkyl

Heteroalkyl steht für eine geradkettige und/oder verzweigte Kohlenwasserstoffkette mit 1 bis 10 Kohlenstoffatomen, die einfach oder mehrfach durch eine oder mehrere der Gruppen -O-, -S-,

- C(=O)-, -S(=O)-, $-S(=O)_2-$, -NRY-, $-NR^yC(=O)-$, $-C(=O)-NR^y-$, -NRYNRY-, $-S(=O)_2-NR^yNR^y-$,
- $C(=O)-NR^yNR^y-$, $-CR^x=N-O-$ unterbrochen sein kann, und wobei die Kohlenwasserstoffkette einschließlich der Seitenketten, sofern vorhanden, mit $-NH-C(=O)-NH_2$, -COOH, -OH, $-NH_2$,
- $NH-C(=NNH_2)-$, Sulfonamid, Sulfon, Sulfoxid, oder Sulfonsäure substituiert sein kann,

Hierbei steht $R^y$ jeweils für -H, Phenyl-, $C_1$-$C_{10}$-Alkyl-, $C_2$-$C_{10}$-Alkenyl- oder $C_2$-$C_{10}$-Alkinyl-, die wiederum jeweils mit $-NH-C(=O)-NH_2$, -COOH, -OH, $-NH_2$, $-NH-C(=NNH_2)-$, Sulfonamid, Sulfon, Sulfoxid, oder Sulfonsäure substituiert sein können.

Hierbei steht $R^x$ für -H, $C_1$-$C_3$-Alkyl- oder Phenyl-.

Alkenyl

Alkenyl steht für eine geradkettige oder verzweigte einwertige Kohlenwasserstoffkette mit einer oder zwei Doppelbindungen und 2, 3, 4, 5, 6, 7, 8, 9 oder 10 Kohlenstoffatomen ($C_2$-$C_{10}$-Alkenyl), insbesondere 2 oder 3 Kohlenstoffatomen ($C_2$-$C_3$-Alkenyl), wobei es sich versteht, dass, wenn die Alkenylgruppe mehr als eine Doppelbindung enthält, die Doppelbindungen isoliert voneinander oder miteinander konjugiert sein können. Bei der Alkenylgruppe handelt es sich zum Beispiel um eine Ethenyl- (bzw. Vinyl-), Prop-2-en-1-yl- (bzw. "Allyl-"), Prop-1-en-1-yl-, But-3-enyl-, But-2-enyl-, But-1-enyl-, Pent-4-enyl-, Pent-3-enyl-, Pent-2-enyl-, Pent-1-enyl-, Hex-5-enyl-, Hex-4-enyl-, Hex-3-enyl-, Hex-2-enyl-, Hex-1-enyl-, Prop-1-en-2-yl- (bzw. "Isopropenyl-"), 2-Methylprop-2-enyl-, 1-Methylprop-2-enyl-, 2-Methylprop-1-enyl-,

1-Methylprop-1-enyl-, 3-Methylbut-3-enyl-, 2-Methylbut-3-enyl-, 1-Methylbut-3-enyl-, 3-Methylbut-2-enyl-, 2-Methylbut-2-enyl-, 1-Methylbut-2-enyl-, 3-Methylbut-1-enyl-, 2-Methylbut-1-enyl-, 1-Methylbut-1-enyl-, 1-,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, 3-Methylpent-3-enyl-, 2-Methylpent-3-enyl-, 1-Methylpent-3-enyl-, 4-Methylpent-2-enyl-, 3-Methylpent-2-enyl-, 2-Methylpent-2-enyl-, 1-Methylpent-2-enyl-, 4-Methylpent-1-enyl-, 3-Methylpent-1-enyl-, 2-Methylpent-1-enyl-, 1-Methylpent-1-enyl-, 3-Ethylbut-3-enyl-, 2-Ethylbut-3-enyl-, 1-Ethylbut-3-enyl-, 3-Ethylbut-2-enyl-, 2-Ethylbut-2-enyl-, 1-Ethylbut-2-enyl-, 3-Ethylbut-1-enyl-, 2-Ethylbut-1-enyl-, 1-Ethylbut-1-enyl-, 2-Propylprop-2-enyl-, 1-Propylprop-2-enyl-, 2-Isopropylprop-2-enyl-, 1-Isopropylprop-2-enyl-, 2-Propylprop-1-enyl-, 1-Propylprop-1-enyl-, 2-Isopropylprop-1-enyl-, 1-Isopropylprop-1-enyl-, 3,3-Dimethylprop-1-enyl-, 1-(1,1-Dimethylethyl)ethenyl-, Buta-1,3-dienyl-, Penta-1,4-dienyl- oder Hexa-1-5-dienylgruppe. Insbesondere handelt es sich bei der Gruppe um Vinyl oder Allyl.

Alkinyl

Alkinyl steht für eine geradkettige oder verzweigte einwertige Kohlenwasserstoffkette mit einer Dreifachbindung und mit 2, 3, 4, 5, 6, 7, 8, 9 oder 10 Kohlenstoffatomen ($C_2$-$C_{10}$-Alkinyl), insbesondere 2 oder 3 Kohlenstoffatomen ($C_2$-$C_3$-Alkinyl). Bei der $C_2$-$C_6$-Alkinylgruppe handelt es sich zum Beispiel um eine Ethinyl-, Prop-1-inyl-, Prop-2-inyl- (bzw. Propargyl-), But-1-inyl-, But-2-inyl-, But-3-inyl-, Pent-1-inyl-, Pent-2-inyl- , Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl-, 2-Methylpent-4-inyl-, 1-Methylpent-4-inyl-, 2-Methylpent-3-inyl-, 1-Methylpent-3-inyl-, 4-Methylpent-2-inyl-, 1-Methylpent-2-inyl-, 4-Methylpent-1-inyl-, 3-Methylpent-1-inyl-, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder 3,3-Dimethylbut-1-inylgruppe. Insbesondere handelt es sich bei der Alkinylgruppe um Ethinyl, Prop-1-inyl oder Prop-2-inyl.

Cycloalkyl

Cycloalkyl steht für einen gesättigten einwertigen mono- oder bicyclischen Kohlenwasserstoffrest mit 3-12 Kohlenstoffatomen ($C_3$-$C_{12}$-Cycloalkyl).

Hierbei steht ein monocyclischer Kohlenwasserstoffrest für einen monovalenten Kohlenwasserstoffrest mit in der Regel 3 bis 10 ($C_3$-$C_{10}$)-Cycloalkyl), bevorzugt 3 bis 8 ($C_3$-$C_8$-Cycloalkyl), und besonders bevorzugt 3 bis 7 ($C_3$-$C_7$-Cycloalkyl) Kohlenstoffatomen.

Beispielhaft und bevorzugt für einen monocyclischen Kohlenwasserstoffrest seien genannt: Cyclopropyl-, Cyclobutyl-, Cyclopentyl-, Cyclohexyl-, Cycloheptyl- und Cyclooctyl-. Besonders bevorzugt ist ein Cyclopropyl-, Cyclobutyl-, Cylopentyl-, Cyclohexyl- und Cycloheptyl-.

Hierbei steht ein bicyclischer Kohlenwasserstoffrest für einen Kohlenwasserstoffrest mit in der Regel 3 bis 12 Kohlenstoffatomen ($C_3$-$C_{12}$-Cycloalkyl), wobei hierbei eine Fusion aus zwei gesättigten Ringsystemen zu verstehen ist, die sich gemeinsam zwei direkt benachbarte Atome teilen. Beispielhaft und bevorzugt für einen bicyclischen Kohlenwasserstoffrest seien genannt: Bicyclo[2.2.0]hexyl-, Bicyclo[3.3.0]octyl-, Bicyclo[4.4.0]decyl-, Bicyclo[5.4.0]undecyl-, Bicyclo[3.2.0]heptyl-, Bicyclo[4.2.0]octyl-, Bicyclo[5.2.0]nonyl-, Bicyclo[6.2.0]decyl-, Bicyclo[4.3.0]nonyl-, Bicyclo[5.3.0]decyl-, Bicyclo[6.3.0]undecyl- und Bicyclo[5.4.0]undecyl-,

Heterocycloalkyl

Heterocycloalkyl steht für ein nicht aromatisches mono- oder bicyclisches Ringsystem mit ein, zwei, drei oder vier Heteroatomen, die gleich oder verschieden sein können. Als Heteroatome können Stickstoffatome, Sauerstoffatome oder Schwefelatome vorkommen.

Ein monocyclisches Ringsystem gemäß der vorliegenden Erfindung kann 3 bis 8, bevorzugt 4 bis 7, besonders bevorzugt 5 oder 6 Ringatome aufweisen.

Beispielhaft und bevorzugt für ein Heterocycloalkyl mit 3 Ringatomen seien genannt: Aziridinyl-.

Beispielhaft und bevorzugt für ein Heterocycloalkyl mit 4 Ringatomen seien genannt: Azetidinyl-, Oxetanyl-.

Beispielhaft und bevorzugt für ein Heterocycloalkyl mit 5 Ringatomen seien genannt: Pyrrolidinyl-, Imidazolidinyl- , Pyrazolidinyl-, Pyrrolinyl-, Dioxolanyl- und Tetrahydrofuranyl-.

Beispielhaft und bevorzugt für ein Heterocycloalkyl mit 6 Ringatomen seien genannt: Piperidinyl-, Piperazinyl-, Morpholinyl-, Dioxanyl-, Tetrahydropyranyl- und Thiomorpholinyl-.

Beispielhaft und bevorzugt für ein Heterocycloalkyl mit 7 Ringatomen seien genannt: Azepanyl-, Oxepanyl-, 1,3-Diazepanyl-, 1,4-Diazepanyl-.

Beispielhaft und bevorzugt für ein Heterocycloalkyl mit 8 Ringatomen seien genannt: Oxocanyl-, Azocanyl-.

Unter monocyclischem Heterocycloalkyl sind 4 bis 7-gliedrige, gesättigte Heterocyclylreste mit bis zu zwei Heteroatomen aus der Reihe O, N und S bevorzugt. Besonders bevorzugt sind Morpholinyl-, Piperidinyl-, Pyrrolidinyl- und Tetrahydrofuranyl-.

Ein bicyclisches Ringsystem mit ein, zwei, drei oder vier Heteroatomen, die gleich oder verschieden sein können, können gemäß der vorliegenden Erfindung 6 bis 12, bevorzugt 6 bis 10 Ringatome aufweisen, wobei ein, zwei, drei oder vier Kohlenstoffatome gegen gleiche oder verschiedene Heteroatome aus der Reihe O, N und S ausgetauscht sein können.

Beispielhaft seien genannt: Azabicyclo[3.3.0]octyl-, Azabicyclo[4.3.0]nonyl-, Diazabicyclo[4.3.0]nonyl-, Oxazabicyclo[4.3.0]nonyl-, Thiazabicyclo[4.3.0]nonyl- oder Azabicyclo[4.4.0]decyl- sowie aus weiteren möglichen Kombinationen gemäß Definition abgeleitete Reste.

Besonders bevorzugt ist Perhydrocyclopenta[c]pyrrolyl-, Perhydrofuro[3,2-c]pyridinyl-, Perhydropyrrolo[1,2-a]pyrazinyl-, Perhydropyrrolo[3,4-c]pyrrolyl- und 3,4-Methylenedioxyphenyl.

Heterocycloalkoxy

Heterocycloalkoxy steht für Heterocycloalkyl, welches über eine -O- Gruppe mit dem Rest des Moleküls verbunden ist.

Alkoxv

Alkoxy steht für einen linearen oder verzweigten, gesättigten Alkyletherrest der Formel -O-Alkyl mit in der Regel 1 bis 6 ($C_1$-$C_6$-Alkoxy), bevorzugt 1 bis 4 ($C_1$-$C_4$-Alkoxy), und besonders bevorzugt 1 bis 3 ($C_1$-$C_3$-Alkoxy) Kohlenstoffatomen.

Beispielhaft und bevorzugt seien genannt:
Methoxy-, Ethoxy-, n-Propoxy-, Isopropoxy-, *tert*-Butoxy-, n-Pentyloxy- und n-Hexyloxy-.

Aryl

Aryl bedeutet ein monovalentes, mono- oder bicyclisches, aus Kohlenstoffatomen bestehendes aromatisches Ringsystem. Beispiele sind Naphthyl- und Phenyl-; bevorzugt ist Phenyl- beziehungsweise ein Phenylrest.

$C_6$-$C_{10}$-Aralkyl

$C_{6-10}$-Aralkyl- steht im Rahmen der Erfindung für ein monocyclisches aromatisches Aryl, bespielhaft Phenyl, an das eine $C_1$-$C_4$-Alkylgruppe gebunden ist.

Eine beispielhafte $C_{6-10}$-Aralkyl-Gruppe ist Benzyl.

Heteroaryl

Heteroaryl bedeutet ein einwertiges monocyclisches, bicyclisches oder tricyclisches aromatisches Ringsystem mit 5, 6, 8, 9, 10, 11, 12, 13 oder 14 Ringatomen (eine "5- bis 14- gliedrige Heteroaryl"gruppe), insbesondere 5, 6, 9 oder 10 Ringatomen, zu verstehen, das mindestens ein Ringheteroatom und gegebenenfalls ein, zwei oder drei weitere Ringheteroatome aus der Gruppe N, O und S enthält und das über ein Ringkohlenstoffatom oder gegebenenfalls (wenn es die Wertigkeit erlaubt) über ein Ringstickstoffatom gebunden ist.

Bei der Heteroarylgruppe kann es sich um eine 5-gliedrige Heteroarylgruppe wie zum Beispiel Thienyl, Furyl, Pyrrolyl, Oxazolyl, Thiazolyl, Imidazolyl, Pyrazolyl, Isoxazolyl, Isothiazolyl, Oxadiazolyl, Triazolyl, Thiadiazolyl oder Tetrazolyl; oder eine 6-gliedrige Heteroarylgrupoe wie zum Beispiel Pyridinyl, Pyridazinyl, Pyrimidinyl, Pyrazinyl oder Triazinyl; oder eine tricyclische Heteroarylgruppe wie zum Beispiel Carbazolyl, Acridinyl oder Phenazinyl; oder eine 9-gliedrige Heteroarylgruppe wie zum Beispiel Benzofuranyl, Benzothienyl, Benzoxazolyl, Benzisoxazolyl, Benzimidazolyl, Benzothiazolyl, Benzotriazolyl, Indazolyl, Indolyl, Isoindolyl, Indolizinyl oder Purinyl; oder eine 10-gliedrige Heteroarylgruppe wie zum Beispiel Chinolinyl, Chinazolinyl, Isochinolinyl, Cinnolinyl, Phthalazinyl, Chinoxalinyl oder Pteridinyl handeln.

Im Allgemeinen, und wenn nicht anders erwähnt, schließen die Heteroarylreste alle möglichen isomeren Formen davon ein, z. B. Tautomere und Positionsisomere in Bezug auf den Anbindungspunkt zum Rest des Moleküls. Somit schließt, als erläuterndes, nicht einschließendes Beispiel, der Begriff Pyridinyl Pyridin-2-yl, Pyridin-3-yl und Pyridin-4-yl ein; oder der Begriff Thienyl schließt Thien-2-yl und Thien-3-yl ein.

$C_5$-$C_{10}$-Heteroaryl

$C_{5-10}$-Heteroaryl steht im Rahmen der Erfindung für ein mono-oder bicyclisches, aromatisches Ringsystem mit ein, zwei, drei oder vier Heteroatome, die gleich oder verschieden sein können. Als Heteroatome können vorkommen: N, O, S, S(=O) und/ oder $S(=O)_2$. Die Bindungsvalenz kann sich an einem beliebigen aromatischen Kohlenstoffatom oder an einem Stickstoffatom befinden.

Ein monocyclischer Heteroarylrest gemäß der vorliegenden Erfindung hat 5 oder 6 Ringatome.

Bevorzugt sind solche Heteroarylreste mit ein oder zwei Heteroatomen. Besonders bevorzugt sind hierbei ein oder zwei Stickstoffatome.

Heteroarylreste mit 5 Ringatomen umfassen beispielsweise die Ringe:
Thienyl, Thiazolyl, Furyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

Heteroarylreste mit 6 Ringatomen umfassen beispielsweise die Ringe:
Pyridinyl, Pyridazinyl, Pyrimidinyl, Pyrazinyl und Triazinyl.

Ein bicyclischer Heteroarylrest gemäß der vorliegenden Erfindung hat 9 oder 10 Ringatome.

Heteroarylreste mit 9 Ringatomen umfassen beispielsweise die Ringe:
Phthalidyl, Thiophthalidyl, Indolyl, Isoindolyl, Indazolyl, Benzothiazolyl, Benzofuryl, Benzothienyl, Benzimidazolyl, Benzoxazolyl, Azocinyl, Indolizinyl, Purinyl, Indolinyl.

Heteroarylreste mit 10 Ringatomen umfassen beispielsweise die Ringe:
Isochinolinyl, Chinolinyl, Chinolizinyl, Chinazolinyl, Chinoxalinyl, Cinnolinyl, Phthalazinyl, 1,7- und 1,8-Naphthyridinyl, Pteridinyl, Chromanyl.

Aryloxy

Aryloxy steht für einen Arylrest, der Formel Aryl-O-.

Beispielhaft und bevorzugt seien genannt: Phenoxy- und Naphthyloxy-

Heteroalkoxy

Heteroalkoxy steht für eine geradkettige und/oder verzweigte Kohlenwasserstoffkette mit 1 bis 10 Kohlenstoffatomen, die über -O- an den Rest des Moleküls gebunden ist und die weiterhin einfach oder mehrfach durch eine oder mehrere der Gruppen -O-, -S-, -C(=O)-, - S(=O)-, $-S(=O)_2-$, -NRY-,
$-NR^yC(=O)-$, $-C(=O)-NR^y-$, -NRYNRY-, $-S(=O)_2-NR^yNR^y-$, $-C(=O)-NR^yNR^y-$, $-CR^x=N-O-$unterbrochen sein kann, und wobei die Kohlenwasserstoffkette einschließlich der Seitenketten, sofern vorhanden, mit $-NH-C(=O)-NH_2$, -COOH, -OH, $-NH_2$, $-NH-C(=NNH_2)-$ , Sulfonamid, Sulfon, Sulfoxid, oder Sulfonsäure substituiert sein kann,

Hierbei steht $R^y$ jeweils für -H, Phenyl-, $C_1$-$C_{10}$-Alkyl-, $C_2$-$C_{10}$-Alkenyl- oder $C_2$-$C_{10}$-Alkinyl-, die wiederum jeweils mit $-NH-C(=O)-NH_2$, -COOH, -OH, $-NH_2$, $-NH-C(=NNH_2)-$, Sulfonamid, Sulfon, Sulfoxid, oder Sulfonsäure substituiert sein können.

Hierbei steht $R^x$ für -H, $C_1$-$C_3$-Alkyl- oder Phenyl-.

Halogen beziehungsweise Halogenatom steht im Rahmen der Erfindung für Fluor (-F), Chlor (-Cl), Brom (-Br), oder Iod (-I).

Bevorzugt ist Fluor (-F), Chlor (-Cl) und Brom (-Br).

Die erfindungsgemäßen Kinesin Spindel Protein-Inhibitor-Prodrugs weisen vorzugsweise die folgende Formel (IIa) auf:

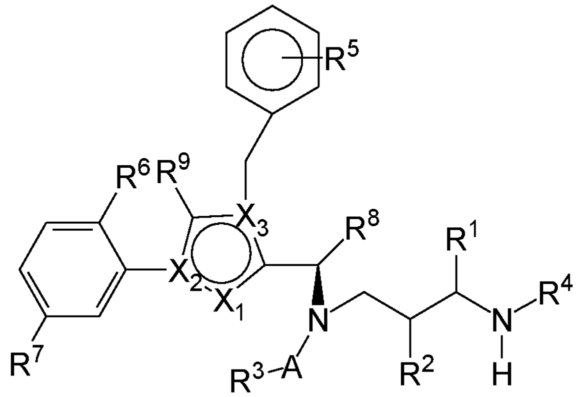

(IIa),

in der

$X_1$ für N,
$X_2$ für N und
$X_3$ für C steht,

oder

$X_1$ für N,
$X_2$ für C und
$X_3$ für N steht,

oder

$X_1$ für CH oder CF,
$X_2$ für C und
$X_3$ für N steht,

oder

$X_1$ für NH,
$X_2$ für C und
$X_3$ für C steht,

oder

$X_1$ für CH,
$X_2$ für N und
$X_3$ für C steht.

| | |
|---|---|
| A | für -C(=O)-, -S(=O)-, $-S(=O)_2-$, oder $-S(=O)_2-NH-$ steht |
| $R^1$ | für -H, -L-#1, -MOD oder $-(CH_2)_{0-3}Z$ steht, |
| Z | für -H, $-NHY^3$, $-OY^3$, $-SY^3$, Halogen, $-C(=O)-NY^1Y^2$, oder $-C(=O)-OY^3$ steht, |
| $Y^1$ und $Y^2$ | unabhängig voneinander für -H, $-NH_2$, $-(CH_2CH_2O)_{0-3}-(CH_2)_{0-3}Z'$ oder $-CH(CH_2W)Z'$ stehen, |
| $Y^3$ | für -H oder $-(CH_2)_{0-3}Z'$ steht, |
| Z′ | für -H, $-NH_2$, $-S(=O)_3H$, -COOH, $-NH-C(=O)-CH_2-CH_2-CH(NH_2)C(=O)-$ oder $-(C(=O)-NH-CHY^4)_{1-3}-COOH$ steht, |
| W | für -H oder -OH steht, |
| $Y^4$ | für lineares oder verzweigtes $C_{1-6}$Alkyl steht, das gegebenenfalls mit $-NH-C(=O)-NH_2$ substituiert ist, oder für Aryl oder Benzyl steht, das gegebenenfalls mit $-NH_2$ substituiert ist, |
| $R^2$ | für -H, -L-#1 , -MOD, $-C(=O)-CHY^4-NHY^5$ oder $-(CH_2)_{0-3}Z$ steht, |

Z für -H, Halogen, $-OY^3$, $-SY^3$, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z′ für -H, $-S(=O)_3H$, $-NH_2$ oder -COOH steht,
$Y^4$ für lineares oder verzweigtes $C_{1-6}$Alkyl- steht, das gegebenenfalls mit $-NHC(=O)-NH_2$ substituiert ist, oder für Aryl oder Benzyl steht, das gegebenenfalls mit $-NH_2$ substituiertes ist,
$Y^5$ für-H oder $-C(=O)-CHY^6-NH_2$ steht,
$Y^6$ für lineares oder verzweigtes $C_{1-6}$ Alkyl- steht,
$R^3$ für -MOD, -L-#1, oder eine gegebenenfalls substituierte Alkyl-, Cycloalkyl-, Aryl-, Heteroaryl-, Heteroalkyl-, Heterocycloalkyl- Gruppe steht, die mit ein bis drei OH-Gruppen, ein bis drei Halogenatomen, ein bis drei mono-, di- oder tri-halogenierten Alkylgruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei $-S(=O)_n$-Alkyl-Gruppen, ein bis drei $-S(=O)_2$-NH-Alkyl-Gruppen, 1-3 -NH-Alkyl-Gruppen, ein bis drei -N(Alkyl)$_2$-Gruppen, ein bis drei $NH_2$-Gruppen oder ein bis drei $-(CH_2)_{0-3}Z$-Gruppen substituiert sein können,

n 0, 1 oder 2 ist,
Z für -H, Halogen, $-OY^3$, $-SY^3$, $-NHY^3$, $-C(=O)-NY^1Y^2$ oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H, $-(CH_2)_{0-3}-CH(NHC(=O)CH_3)Z'$,$-(CH_2)_{0-3}-CH(NH_2)Z'$ oder $-(CH_2)_{0-3}Z'$ steht,
Z′ für -H, $-S(=O)_3H$, $-NH_2$ oder -COOH steht,

$R^4$ für die durch Legumain spaltbare Gruppe der Formeln Ia′, Ia″ und Ia‴ steht,
$R^5$ für -H, $-NH_2$, $-NO_2$, Halogen, -CN, $CF_3$, $-OCF_3$, $-CH_2F$, $-CH_2F$, -SH oder $-(CH_2)_{0-3}Z$ steht,
Z für -H, $-OY^3$, $-SY^3$, Halogen, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z′ für -H, $-S(=O)_3H$, $-NH_2$ oder -COOH steht,
$R^6$ und $R^7$ unabhängig voneinander für -H, -CN, $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, Hydroxy-, $-NO_2$, $-NH_2$, -COOH oder Halogen stehen,
$R^8$ für $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, $C_{4-10}$-Cycloalkyl-, Fluor-$C_{4-10}$-Cycloalkyl-oder $-(CH_2)_{0-2}-(HZ^2)$ steht,
$HZ^2$ für einen 4 bis 7-gliedrigen Heterocyclus mit bis zu zwei Heteroatome, ausgewählt aus N, O und S steht, der mit -OH, -COOH, $-NH_2$ oder -L-#1 substituiert sein kann,

$R^9$ für -H, -F, $-CH_3$, $-CF_3$, $-CH_2F$ oder $-CHF_2$ steht,

-L-#1 für $-(L_b)_o-(LIG)_p$ steht,
LIG für einen Binder steht, der nach Bindung an ein Zielmolekül einer Tumorzelle von der Tumorzelle internalisiert und intrazellulär und vorzugsweise lysosomal, prozessiert wird,
$L_b$ für einen Linker steht,

o und p unabhängig voneinander für 0 oder 1 stehen,

-MOD für $-(NR^{10})_n-(G1)_o$-G2-G3 darstellt,
$R^{10}$ für -H oder $C_1$-$C_3$-Alkyl- steht,

G1 für -NH-C-(=O)- , -C(=O)-NH- oder

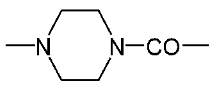

steht,
n 0 oder 1 ist;
o 0 oder 1 ist und
G2 für eine geradkettige oder verzweigte Kohlenwasserstoffkette mit 1 bis 20 Kohlenstoffatomen steht, die einfach oder mehrfach durch -O-, -S-, -S(=O)-, $-S(=O)_2$-, -NRY-, $-NR^yC(=O)$-, $-C(=O)NR^y$-, -NRYNRY-, $-S(=O)_2-NR^yNR^y$-,

-C(=O)-$NR^yNR^y$-, -C(=O)-, -$CR^x$=N-O- unterbrochen sein kann und die geradkettige oder verzweigte Kohlenwasserstoffkette mit -NH-C(=O)-$NH_2$, -COOH, -OH, -$NH_2$, Sulfonamid, Sulfon, Sulfoxid, oder Sulfonsäure substituiert sein kann,
$R^y$ für -H, Phenyl-, $C_1$-$C_{10}$-Alkyl-, $C_2$-$C_{10}$-Alkenyl- oder $C_2$-$C_{10}$-Alkinyl- steht, die jeweils mit -NH-C(=O)-$NH_2$, -COOH, -OH, -$NH_2$, Sulfonamid, Sulfon, Sulfoxid, oder Sulfonsäure substituiert sein können,
Rx für -H, $C_1$-$C_3$-Alkyl- oder Phenyl- steht,
G3 für -H oder -COOH steht und
-MOD mindestens eine COOH-Gruppe aufweist,

sowie ihre Salze, Solvate und Salze der Solvate.

Hierbei bevorzugt sind solche Verbindungen bei denen

$R^3$ für eine $C_{1-10}$-Alkyl-, $C_{6-10}$-Aryl- oder $C_{6-10}$-Aralkyl-, $C_{5-10}$-Heteroalkyl-,$C_{1-10}$-Alkyl-O-$C_{6-10}$-Aryl- oder $C_{5-10}$-Heterocycloalkyl-Gruppe steht, die mit ein bis drei OH-Gruppen, ein bis drei Halogenatomen, ein bis drei mono-, di- oder tri-halogenierten Alkylgruppe, ein bis drei -O-Alkyl-Gruppen, ein bis drei-SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei -$S(=O)_n$-Alkyl-Gruppen, ein bis drei -$S(=O)_2$-NH-Alkyl-Gruppen, 1-3 -NH-Alkyl- Gruppen, ein bis drei -$N(Alkyl)_2$-Gruppen, ein bis drei $NH_2$-Gruppen oder ein bis drei -$(CH_2)_{0-3}$Z-Gruppensubstituiert sein können, wobei n und Z die oben angegebenen Bedeutungen haben.

Der Rest -$(C(=O)-NH-CHY^4)_{1-3}$-COOH bedeutet, dass ein Rest -C(=O)-NH-$CHY^4$-COOH vorliegt, oder zwei Reste -(C(=O)-NH-$CHY^4$) aneinander gebunden sein können, gemäß

-C(=O)-NH-$CHY^4$-C(=O)-NH-$CHY^4$-COOH,

oder drei Reste aneinander gebunden sein können, gemäß

-C(=O)-NH-$CHY^4$-C(=O)-NH-$CHY^4$-C(=O)-NH-$CHY^4$-COOH.

Besonders bevorzugt sind die Verbindungen der allgemeinen Formel (IIa), in denen

$X_1$ für N steht,
$X_2$ für N steht und
$X_3$ für C steht,

oder

$X_1$ für CH oder CF steht,
$X_2$ für C steht und
$X_3$ für N steht,

oder

$X_1$ für NH steht,
$X_2$ für C steht und
$X_3$ für C steht,

oder

$X_1$ für H steht,
$X_2$ für N steht und
$X_3$ für C steht.

Insbesondere sind solche Verbindungen der allgemeinen Formel (IIa) bevorzugt, in denen

$X_1$ für N steht,
$X_2$ für N steht und

$X_3$ für C steht,

oder

$X_1$ für CH steht,
$X_2$ für C steht und
$X_3$ für N steht.

Ganz besonders bevorzugt sind solche Verbindungen der allgemeinen Formel (IIa), in denen

$X_1$ für CH steht,
$X_2$ für C steht und
$X_3$ für N steht.

Bevorzugt sind solche Verbindungen der allgemeinen Formel (IIa), in denen A für -C(=O)- steht.
Weiterhin bevorzugt sind solche Verbindungen der allgemeinen Formel (IIa), in denen

$R^1$ für -L-#1, -MOD, -H, -COOH, -C(=O)-NH-$NH_2$, -$(CH_2)_{1-3}NH_2$, -C(=O)-NZ″$(CH_2)_{1-3}$ $NH_2$ und -C(=O)-NZ″$CH_2$COOH steht und
Z″ für -H oder -$NH_2$ steht.

Falls in der allgemeinen Formel (IIa) $R^4$ für -L-#1 steht, dann steht $R^1$ vorzugsweise für -MOD.
Insbesondere steht in der allgemeinen Formel (IIa) $R^4$ für -L-#1 und $R^1$ für -MOD falls $R^3$ nicht für -MOD steht.
In der allgemeinen Formel (IIa) steht $R^2$ vorzugsweise für -H.
In der allgemeinen Formel (IIa) steht $R^3$ bevorzugt für

- L-#1 oder -MOD, oder für $C_{1-10}$-Alkyl-, das gegebenenfalls mit -OH, -O-Alkyl, -SH, -S-Alkyl, -O-C(=O)-Alkyl, -O-C(=O)-NH-Alkyl, -NH-C(=O)-Alkyl, -NH-C(=O)-NH-Alkyl,
- $S(=O)_n$-Alkyl, -$S(=O)_2$-NH-Alkyl, -NH-Alkyl, -$N(Alkyl)_2$, oder -$NH_2$ substituiert sein kann. Alkyl bedeutet hier vorzugsweise $C_{1-3}$Alkyl-.

In der allgemeinen Formel (IIa) steht $R^5$ ist bevorzugt für -H oder -F.
In der allgemeinen Formel (IIa) steht bevorzugt $R^6$ und $R^7$ unabhängig voneinander für -H, $C_{1-10}$-Alkyl, Fluor-$C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, Hydroxy- oder Halogen-.
In der allgemeinen Formel (IIa) steht bevorzugt $R^8$ für eine verzweigte $C_{1-5}$-Alkyl-Gruppe, insbesondere für eine Gruppe -$C(CH_3)_2$-$(CH_2)_{0-2}$-$R_y$, wobei $R_y$ für -H, -OH, -$C(=O)_2$H, oder -$NH_2$ steht.
Besonders bevorzugt steht $R^8$ für die Gruppe-$C(CH_3)_2$-$(CH_2)$-$R_y$, wobei $R_y$ für -H steht.
In der allgemeinen Formel (IIa) steht $R^9$ bevorzugt für -H oder -F.
In der allgemeinen Formel (IIa) steht -MOD bevorzugt für die Gruppe
HOOC-$(CHX)_X$-AM-$CH_2$-$CH_2$-NH-C(=O)-,
wobei

x eine Zahl von 2 bis 6 ist,
X für -H, -$NH_2$ oder -COOH steht, und
AM für -C(=O)-NH- oder -NH-C(=O)- steht.

In der allgemeinen Formel (IIa) steht -MOD besonders bevorzugt für die Gruppe

HOOC-$CH_2$-$CH_2$-CH(COOH)-NH-C(=O)-$CH_2$-$CH_2$-NH-C(=O)-,

HOOC-CH($NH_2$)-$CH_2$-$CH_2$-C(=O)-NH-$CH_2$-$CH_2$-NH-C(=O)-

und

HOOC-CH($NH_2$)-$(CH_2)_4$-NH-C(=O)-$CH_2$-$CH_2$-NH-C(=O)-.

Insbesondere bevorzugt sind Verbindungen der allgemeinen Formel (IIa), in der keiner oder einer der Substituenten $R^1$ und $R^3$ für -L-#1 steht, und

in denen

$X_1$ für N steht,
$X_2$ für N steht und
$X_3$ für C steht,

oder

$X_1$ für CH oder CF steht,
$X_2$ für C steht und
$X_3$ für N steht,

oder

$X_1$ für NH steht,
$X_2$ für C steht und
$X_3$ für C steht,

oder

$X_1$ für CH steht,
$X_2$ für N steht und
$X_3$ für C steht

und

A für -C(=O)- steht,

$R^1$ für -H, -COOH, -C(=O)-NH-$NH_2$, -$(CH_2)_{1-3}NH_2$, -C(=O)-NZ″$(CH_2)_{1-3}NH_2$ und -C(=O)-NZ″$CH_2$-COOH steht,

Z″ für -H oder -$NH_2$ steht,

$R^2$ für -H steht,

$R^3$ für eine Phenylgruppe steht, die ein oder mehrfach mit Halogen, $C_{1-3}$-Alkyl oder Fluor- $C_{1-3}$-Alkyl substituiert sein kann, oder für eine $C_{1-10}$-Alkylgruppe steht, die gegebenenfalls mit Fluor, -$OY^4$, -$SY^4$, -O-C(=O)-$Y^4$, -O-C(=O)-NH-$Y^4$, -NH-C(=O)-$Y^4$, -NH-C(=O)-NH-$Y^4$, -$S(=O)_n$-$Y^4$, -$S(=O)_2$-NH-$Y^4$, -NH-$Y^4$ oder -$N(Y^4)_2$, substituiert sein kann,

n 1 oder 2 ist und

$Y^4$ für -H oder für eine Phenylgruppe steht, die gegebenenfalls ein oder mehrfach mit Halogen, $C_{1-3}$-Alkyl oder Fluor-$C_{1-3}$-Alkyl substituiert sein kann, oder für Alkylsteht, das mit -OH, -COOH, und/oder -NH-C(=O)-$C_{1-3}$-Alkyl substituiert sein kann.

Hierbei steht $R^3$ und $Y^4$ vorzugsweise für eine Phenylgruppe, die ein oder mehrfach mit Fluor substituiert sein kann.

Hierbei steht $Y^4$ vorzugsweise für $C_{1-3}$-Alkyl-.

Auch insbesondere bevorzugt sind solche Verbindungen der allgemeinen Formel (IIa) bei denen

$R^3$ für eine Phenylgruppe steht, die ein oder mehrfach mit -OH, -O-Alkyl, -SH, -S-Alkyl, -O-C(=O)-Alkyl, -O-C(=O)-NH-Alkyl, -NH-C(=O)-Alkyl, -NH-C(=O)-NH-Alkyl, -$S(=O)_n$-Alkyl, -$S(=O)_2$-NH-Alkyl, -NH-Alkyl, -$N(Alkyl)_2$, oder -$NH_2$ substituiert sein kann,

n für 1 oder 2 steht.

$R^5$ für-H oder -F steht,

$R^6$ und $R^7$ unabhängig voneinander für -H, $C_{1-10}$-Alkyl, Fluor- $C_{1-10}$-Alkyl, $C_{2-10}$-Alkenyl-, Fluor- $C_{2-10}$-Alkenyl-,

$C_{2-10}$-Alkinyl-, Fluor- $C_{2-10}$-Alkinyl-, Hydroxy- oder Halogen- stehen,

$R^8$ für eine verzweigte $C_{1-5}$-Alkyl-Gruppe - steht und

$R^9$ für -H oder -F steht.

Hierbei ist $C_{1-3}$-Alkyl besonders bevorzugt.

Weiterhin sind die Verbindungen der allgemeinen Formel (IIa) bevorzugt bei denen

$R^1$ für -H, -L-#1, -COOH, HOOC-$CH_2$-$CH_2$-CH(COOH)-NH-C(=O)-$CH_2$-$CH_2$-NH-C(=O)-; HOOC-CH($NH_2$)-$CH_2$-$CH_2$-C(=O)-NH-$CH_2$-$CH_2$-NH-C(=O)- oder HOOC-CH($NH_2$)-$(CH_2)_4$-NH-C(=O)-$CH_2$-$CH_2$-NH-C(=O)- steht,

$R^2$ für -H steht,

A für -C(=O)- steht,

$R^3$ für -($CH_2$)OH, -CH($CH_3$)OH, -$CH_2$-S-$CH_2$CH-(COOH)-NH-C(=O)-$CH_3$, -CH($CH_3$)$OCH_3$, eine Phenylgruppe, die mit ein bis drei Halogenatomen, ein bis drei Aminogruppen, ein bis drei Alkylgruppen oder ein bis drei Halogenalkylgruppen, substituiert sein kann, HOOC-$CH_2$-$CH_2$-CH(COOH)-NH-C(=O)-$CH_2$-$CH_2$-NH-C(=O)-; HOOC-CH($NH_2$)-$CH_2$-$CH_2$-C(=O)-NH-$CH_2$-$CH_2$-NH-C(=O)-; HOOC-CH($NH_2$)-$(CH_2)_4$-NH-C(=O)-$CH_2$-$CH_2$-NH-C(=O)- oder -$CH_2$-$S_x$-$(CH_2)_{0-4}$-$CHY^5$-COOH steht,

x 0 oder 1 ist,

$Y^5$ für -H oder -$NHY^6$ steht,

$Y^6$ für -H, -C(=O)-$CH_3$ oder -L-#1 steht,

$R^5$ für -H steht,

$R^6$ und $R^7$ unabhängig voneinander für -H, $C_{1-3}$-Alkyl- oder Halogen- stehen,

$R^8$ für $C_{1-4}$-Alkyl- steht und

$R^9$ für -H steht.

Hierbei bevorzugt sind insbesondere dolche Verbindungen bei denen

$R^6$ und $R^7$ unabhängig voneinander für Wasserstoff oder Fluor stehen und

$R^8$ für tert-Butyl steht.

Weiterhin sind solche Verbindungen bevorzugt, bei denen

$R^1$ für -H, -COOH, HOOC-$CH_2$-$CH_2$-CH(COOH)-NH-C(=O)-$CH_2$-$CH_2$-NH-C(=O)-, HOOC-CH($NH_2$)-$CH_2$-$CH_2$-C(=O)-NH-$CH_2$-$CH_2$-NH-C(=O)- oder HOOC-CH($NH_2$)-$(CH_2)_4$-NH-C(=O)-$CH_2$-$CH_2$-NH-C(=O)- steht,

$R^2$ für -H steht,

A für -C(=O)- steht,

$R^3$ für -($CH_2$)OH, -CH($CH_3$)OH, -$CH_2$-S-$CH_2$CH(COOH)NH-C(=O)-$CH_3$, -CH($CH_3$)$OCH_3$, HOOC-$CH_2$-$CH_2$-CH(COOH)-NH-C(=O)-$CH_2$-$CH_2$-NH-C(=O)-, HOOC-CH($NH_2$)-$CH_2$-$CH_2$-C(=O)-NH-$CH_2$-$CH_2$-NH-C(=O)-, HOOC-CH($NH_2$)-$(CH_2)_4$-NH-C(=O)-$CH_2$-$CH_2$-NH-C(=O)-, -$CH_2$-$S_x$-$(CH_2)_{0-4}$-$CHY^5$-COOH oder für eine Phenylgruppe steht, die mit 1-3 Halogenatomen, ein bis drei Aminogruppen, ein bis drei Alkylgruppen oder ein bis drei Haloalkylgruppen substituert sein kann,

x 0 oder 1 ist,

$Y^5$ für -H oder -$NHY^6$ steht,

$Y^6$ für -H, -C(=O)-$CH_3$ oder -L-#1 steht,

$R^5$ für -H steht,

$R^6$ und $R^7$ unabhängig voneinander für -H, $C_{1-3}$-Alkyl- oder Halogen- stehen,

$R^8$ für $C_{1-4}$-Alkyl steht und

$R^9$ für -H steht,

wobei einer der Substituenten $R^1$ oder $R^3$ für -L-#1 steht.

Hierbei sind insbesondere solche Verbindungen bevorzugt bei denen

$R^6$ und $R^7$ für -F steht und

$R^8$ für tert-Butyl steht.

Weiterhin sind Verbindungen der allgemeinen Formel (IIb)

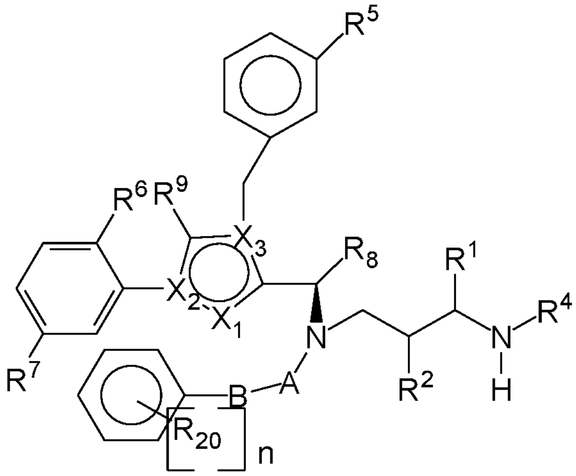

(IIb),

bevorzugt, in der

$X_1$, $X_2$,$X_3$, $R^1$,
$R^2$, $R^4$, $R^5$, $R^6$,

$R^7$, $R^8$ und $R^9$ die in der allgemeinen Formel (IIa) angegebenen Bedeutungen haben und

A für -C(=O)- steht,
B für eine Einfachbindung, -O-$CH_2$- oder -$CH_2$-O- steht,
$R^{20}$ für -$NH_2$, -F, -$CF_3$, oder -$CH_3$ steht und
n 0, 1, oder 2 ist.

Hierbei bevorzugt sind solche Verbindungen der allgemeinen Formel (IIb), in der

$X_1$ für CH steht,
$X_2$ für C steht und
$X_3$ für N steht.

Auch sind solche verbindungen der allgemeinen Formel (IIc)

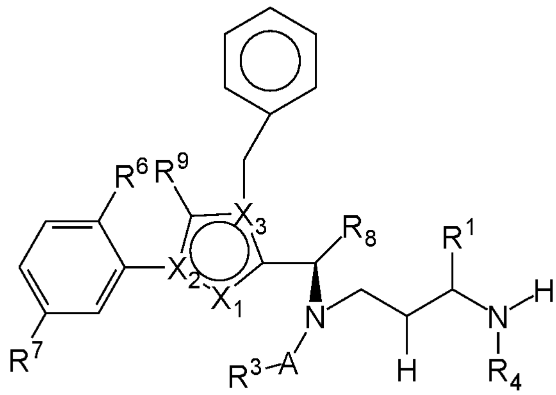

(IIc)

bevorzugt, in der
$X_1$, $X_2$, $X_3$ A, $R^1$, $R^3$, $R^4$, $R^6$, $R^7$, $R^8$ und $R^9$ die in der allgemeinen Formel (IIa) angegebenen Bedeutungen aufweisen.

Hierbei bevorzugt sind solche Verbindungen der allgemeinen Formel (IIc), in der

$X_1$ für CH steht,
$X_2$ für C steht,
$X_3$ für N steht,
A für -C(=O)- steht und
$R^3$ für $-CH_2OH$, $-CH_2OCH_3$, $-CH(CH_3)OH$ oder $-CH(CH_3)OCH_3$ steht.

Ferner sind auch solche Verbindungen der allgemeinen Formel (IId):

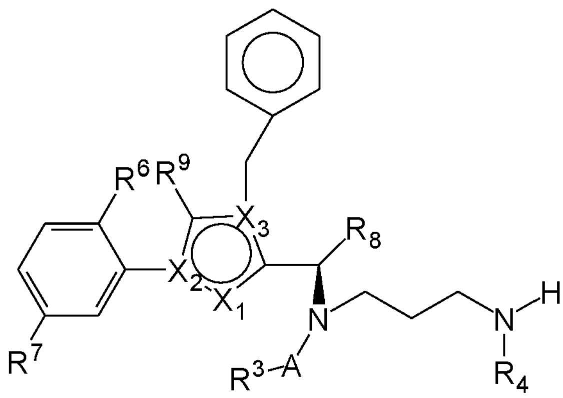

(IId)

bevorzugt, in der
$X_1$, $X_2$, $X_3$, A, $R^3$, $R^4$, $R^6$, $R^7$, $R^8$ und $R^9$ die in der allgemeinen Formel (IIa) angegebenen Bedeutungen haben.

Hierbei bevorzugt sind solche Verbindungen der allgemeinen Formel (IId), in der

$X_1$ für CH steht,
$X_2$ für C steht,
$X_3$ für N steht,
A für-C(=O)- steht,
$R^3$ für $-CH_2-S_x-(CH_2)_{0-4}-CHY^5-COOH$ steht,
x für 0 oder 1 steht,
$Y^5$ für -H oder $-NHY^6$ steht und
$Y^6$ für -H oder $-C(=O)CH_3$ steht.

Weiterhin bevorzugt sind solche Verbindungen der allgemeinen Formeln (IIa), (IIb), (IIc) und (IId), bei denen

- Z für -Cl oder -Br steht;
- $R^1$ für $-(CH_2)_{0-3}Z$ steht,
  Z für $-C(=O)-NY^1Y^2$ steht,
  $Y^1$ für -H, $-NH_2$, oder $-(CH_2CH_2O)_{0-3}-(CH_2)_{0-3}Z'$steht;
  $Y^2$ für $-(CH_2CH_2O)_{0-3}-(CH_2)_{0-3}Z'$ steht und
  Z' für -COOH steht;
- $Y^1$ für -H steht,
  $Y^2$ für $-(CH_2CH_2O)_3-CH_2CH_2Z'$ steht und
  Z' für -COOH steht;
- $Y^1$ für -H steht,
  $Y^2$ für $-CH_2CH_2Z'$ steht,
  Z' für $-(C(=O)NHCHY^4)_2-COOH$ steht und
  $Y^4$ die in der allgemeinen Formel (IIa) angegebene Bedeutung hat;
- $Y^1$ für -H steht,
  $Y^2$ für $-CH_2CH_2Z'$ steht,
  Z' für $-(C(=O)-NHCHY^4)_2-COOH$ steht und
  $Y^4$ für *i*-Propyl- oder $-(CH_2)_3-NH-C(=O)-NH_2$ steht;
- $Y^1$ für -H steht,
  $Y^2$ für $-CH_2CH_2Z'$ steht,
  Z' für $-(C(=O)-NHCHY^4)_2-COOH$ steht und
  $Y^4$ für $-CH_3$ oder $-(CH_2)_3-NH-C(=O)-NH_2$ steht;
- $Y^4$ für lineares oder verzweigtes, gegebenenfalls mit $-NH-C(=O)-NH_2$ substituiertes $C_{1-6}$ Alkyl steht;
- $Y^4$ für *i*-Propyl- oder $-CH_3$ steht;
- $Y^1$ für -H steht,
  $Y^2$ für $-CH_2CH_2Z'$ steht,
  Z' für $-C(=O)-NHCHY^4-COOH$ steht und
  $Y^4$ für gegebenenfalls mit $-NH_2$ substituiertes Aryl- oder Benzyl- steht;
- $Y^4$ für Aminobenzyl- steht;
- $R^2$ für $-(CH_2)_{0-3}Z$ steht,
  Z für $-SY^3$ steht und
  $Y^3$ die oben angegebene Bedeutung hat;
- $R^4$ für $-C(=O)-CHY^4-NHY^5$ steht,
  Y4 die oben angegebene Bedeutung hat und
  $Y^5$ für -H steht;
- $R^4$ für $-C(=O)-CHY^4-NHY^5$ steht,
  $Y^5$ für $-C(=O)-CHY^6-NH_2$ steht und
  $Y^4$ und$Y^6$ die oben angegebene Bedeutungen haben;
- $Y^4$ für ineares oder verzweigtes $C_{1-6}$ Alkyl steht, das gegebenenfalls mit $-NH-C(=O)-NH_2$ substituiert sein kann.

Weiterhin bevorzugt sind solche Verbindungen der allgemeinen Formel (IIa), in der $R^1$, $R^2$ oder $R^3$ für -MOD steht.

Besonders bevorzugt sind solche Verbindungen bei denen $R^3$ für -MOD und $R^1$ für -L-#1 steht, wobei

-MOD für $-(NR^{10})_n-(G1)_o-G2-G3$ steht,
$R^{10}$ für -H oder $C_1-C_3$-Alkyl- steht;

G1 für -NH-C(=O)- , -C(=O)-NH- oder

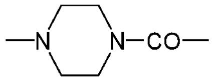

steht,

n 0 oder 1 ist,

o 0 oder 1 ist,

G2 für eine geradkettige oder verzweigte Kohlenwasserstoffkette mit 1 bis 20 Kohlenstoffatomen steht, die ein- oder mehrfach, gleich oder verschieden durch -O-, -S-, -S(=O)-, $-S(=O)_2$-, $-NR^y$-, $-NR^yC(=O)$-, $-C(=O)-NR^y$, $-NR^yNR^y$-, $-S(=O)_2-NR^yNR^y$-, $-C(=O)-NR^yNR^y$-, -C(=O)-, $-CR^x=N-O$- unterbrochen sein kann, wobei die geradkettige oder verzweigte Kohlenwasserstoffkette mit $-NH-C(=O)-NH_2$, -COOH, -OH, $-NH_2$, Sulfonamid, Sulfon, Sulfoxid, oder Sulfonsäure substituiert sein kann,

$R^y$ für-H, Phenyl-, $C_1$-$C_{10}$-Alkyl-, $C_2$-$C_{10}$-Alkenyl-, oder $C_2$-$C_{10}$-Alkinylsteht, die jeweils mit $-NH-C(=O)-NH_2$, -COOH, -OH, $-NH_2$, Sulfonamid, Sulfon, Sulfoxid, oder Sulfonsäure substituiert sein können,

Rx für -H, $C_1$-$C_3$-Alkyl oder Phenyl steht,

G3 für -H oder -COOH steht und

wobei die Gruppe -MOD vorzugsweise zumindest eine Gruppe -COOH aufweist.

Besonders bevorzugt weist die Gruppe -MOD eine COOH-Gruppe auf, z.B. in einer Betaingruppe.

Vorzugsweise ist diese COOH-Gruppe terminal ständig.

Weiterhin besonders bevorzugt weist die Gruppe -MOD die Gruppe

$$-CH_2-S_x-(CH_2)_{0-4}-CHY^5-COOH$$

auf,
in der

x 0 oder 1 ist,

$Y^5$ für -H oder $-NHY^6$ steht und

Y6 für -H oder $-C(=O)CH_3$ steht.

Weiterhin bevorzugt sind die Verbindungen der allgemeinen Formeln (IIa), (IIb), (IIc) und (IId), in denen

$X_1$ für N steht,
$X_2$ für N steht und
$X_3$ für C steht,

oder

$X_1$ für N steht,
$X_2$ für C steht und
$X_3$ für N steht,

oder

$X_1$ für CH oder CF steht,
$X_2$ für C steht und
$X_3$ für N steht,

oder

$X_1$ für NH steht,
$X_2$ für C steht und
$X_3$ für C steht,

oder

$X_1$ für CH oder CF steht,

$X_2$ für N steht und
$X_3$ für C steht,

$R^1$ für -H, -L-#1, -MOD oder $-(CH_2)_{0-3}Z$ steht,
Z für -H, $-NHY^3$, $-OY^3$, $-SY^3$, Halogen, $-C(=O)-NY^1Y^2$ oder $-C(=O)-OY^3$ steht,
$Y^1$ und $Y^2$ unabhängig voneinander für -H, $-NH_2$, $-(CH_2CH_2O)_{0-3}-(CH_2)_{0-3}Z'$ $Y^3$ oder $-CH(CH_2W)Z'$ stehen, für -H oder $-(CH_2)_{0-3}Z'$ steht,
Z' für-H, $-NH_2$, $-S(=O)_3H$, -COOH, $-NH-C(=O)-CH_2-CH_2-CH(NH_2)-COOH$ oder $-(C(=O)-NH-CHY^4)_{1-3}COOH$ steht,
W für -H oder -OH steht,
$Y^4$ für lineares oder verzweigtes $C_{1-6}$ Alkyl- steht, das gegebenenfalls mit $-NHC(=O)-NH_2$ substituiert ist, oder für Aryl- oder Benzyl- steht, die gegebenenfalls mit $-NH_2$ substituiert sind,

$R^2$ für -H, $-C(=O)-CHY^4-NHY^5$ oder $-(CH_2)_{0-3}Z$ steht,
Z für -H, Halogen, $-OY^3$, $-SY^3$, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z' für - H, $-S(=O)_3H$, $-NH_2$ oder -COOH steht;
$Y^4$ für lineares oder verzweigtes $C_{1-6}$ Alkyl- steht, das gegebenenfalls mit $-NH-C(=O)-NH_2$ substituiert ist oder für Aryl- oder Benzyl- steht, die gegebenenfalls mit $-NH_2$ substituiert sind,
$Y^5$ für -H oder $-C(=O)-CHY^6-NH_2$ steht,
$Y^6$ für lineares oder verzweigtes $C_{1-6}$ Alkyl steht,
A für -C(=O)-, -S(=O)-, $-S(=O)_2-$ oder $-S(=O)_2-NH-$ steht,
$R^3$ für -L-#1, -MOD oder eine Alkyl-, Cycloalkyl-, Aryl-, Heteroaryl-, Heteroalkyl-, Heterocycloalkyl- Gruppe steht, die gegebenenfalls mit ein bis drei OH-Gruppen, ein bis drei Halogenatomen, ein bis drei mono-, di- oder tri-halogenierten Alkyl-Gruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei $-S(O)_n$-Alkyl-Gruppen, ein bis drei $-S(=O)_2$-NH-Alkyl-Gruppen, ein bis drei -NH-Alkyl-Gruppen, ein bis drei -N(Alkyl)$_2$-Gruppen, ein bis drei $-NH((CH_2CH_2O)_{1-20}H)$-Gruppen, ein bis drei $NH_2$-Gruppen oder ein bis drei $-(CH_2)_{0-3}Z$-Gruppen substituiert sein können,
Z für -H, Halogen, $-OY^3$, $-SY^3$, $-NHY^3$, $-C(=O)-NY^1Y^2$ oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H, $-(CH_2)_{0-3}-CH(NH-C(=O)-CH_3)Z'$,$-(CH_2)_{0-3}-CH(NH_2)Z'$, oder $-(CH_2)_{0-3}Z'$ steht,
Z' für -H, $-S(=O)_3H$, $-NH_2$ oder -COOH steht,
$R^5$ für -H, -MOD, $-NH_2$, $-NO_2$, Halogen, -CN, $-CF_3$, $-OCF_3$, $-CH_2F$, $-CH_2F$, -SH
oder $-(CH_2)_{0-3}Z$ steht,
Z für -H, $-OY^3$, $-SY^3$, Halogen, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht und
Z' für -H, $-S(=O)_3H$, $-NH_2$ oder -C(=O)-OH steht,
$R^6$ und $R^7$ unabhängig voneinander für -H, -CN, $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, Hydroxy, $-NO_2$, $-NH_2$, -COOH oder Halogen stehen,
$R^8$ für $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, $C_{4-10}$-Cycloalkyl- oder Fluor- $C_{4-10}$-Cycloalkyl- steht,
$R^9$ für -H, -F, $-CH_3$, $-CF_3$, $-CH_2F$ oder $-CHF_2$ steht,
-MOD für die Gruppe $-(NR^{10})_n-(G1)_o$-G2-G3 steht,
$R^{10}$ für -H oder $C_1$-$C_3$-Alkyl- steht,
G1 für -NH-C(=O)- , -C(=O)-NH- oder

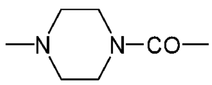

n 0 oder 1 ist,
o 0 oder 1 ist,
G2 für eine geradkettige oder verzweigte Kohlenwasserstoffkette mit 1 bis 20 Kohlenstoffatomen steht, die ein- oder mehrfach durch -O-, -S-, -S(=O)-, $-S(=O)_2-$, -NRY-, $-NR^yC(=O)-$, $-C(=O)-NR^y-$, -NRYNRY-,

$-S(=O)_2-NR^yNR^y-$, $-C(=O)-NR^yNR^y-$ unterbrochen sein kann, wobei die geradkettige oder verzweigte Kohlenwasserstoffkette mit $-NH-C(=O)-NH_2$, -COOH, -OH, $-NH_2$, Sulfonamid, Sulfon, Sulfoxid, oder Sulfonsäure substituiert sein kann,

$R^y$ für -H, -C(=O)-, $-CR^x=N-O-$ oder für gegebenenfalls mit $NH-C(=O)-NH_2$, -COOH, -OH, $-NH_2$, Sulfonamid, Sulfon, Sulfoxid oder Sulfonsäure substituiertes Phenyl-, $C_1$-$C_{10}$-Alkyl-, $C_2$-$C_{10}$-Alkenyl- oder $C_2$-$C_{10}$-Alkinyl- steht,

$R^x$ für -H, $C_1$-$C_3$-Alkyl- oder Phenyl- steht,

G3 für -H oder -COOH steht und

wobei die Gruppe -MOD vorzugsweise zumindest eine Gruppe -COOH aufweist und wobei $R^1$ und $R^3$ nicht gleichzeitig für -L-#1 stehen,
sowie ihre Salze, Solvate und Salze der Solvate.

Hierbei besonders bevorzugt sind die Verbindungen der allgemeinen Formeln (IIa), (IIb), (IIc) und (IId), in denen

$X_1$ für CH steht,
$X_2$ für C steht und
$X_3$ für N steht.

Hierbei weiterhin besonders bevorzugt sind die Verbindungen der allgemeinen Formeln (IIa), (IIb), (IIc) und (IId), in denen

R3 für eine $C_{1-10}$-Alkyl-, $C_{6-10}$-Aryl-, $C_{6-10}$-Aralkyl-, $C_{5-10}$-Heteroalkyl-, $C_{1-10}$-Alkyl-O-$C_{6-10}$-Aryl- oder $C_{5-10}$-Heterocycloalkyl-Gruppe steht, die gegebenenfalls mit ein bis drei OH-Gruppen, ein bis drei Halogenatomen, ein bis drei mono-, di- oder tri-halogenierten Alkyl-Gruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei $-S(O)_n$-Alkyl-Gruppen, ein bis drei $-S(=O)_2$-NH-Alkyl-Gruppen, ein bis drei -NH-Alkyl-Gruppen, ein bis drei $-N(Alkyl)_2$-Gruppen, ein bis drei $-NH((CH_2CH_2O)_{1-20}H)$-Gruppen, ein bis drei $NH_2$-Gruppen oder ein bis drei $-(CH_2)_{0-3}Z$-Gruppen substituiert sein können,

Z für -H, Halogen, $-OY^3$, $-SY^3$, $-NHY^3$, $-C(=O)-NY^1Y^2$ oder $-C(=O)-OY^3$ steht,

$Y^1$ und $Y^2$ unabhängig voneinander für -H, $-NH_2$, oder $-(CH_2)_{0-3}Z'$ stehen,

$Y^3$ für -H, $-(CH_2)_{0-3}-CH(NH-C(=O)-CH_3)Z'$,$-(CH_2)_{0-3}-CH(NH_2)Z'$, oder $-(CH_2)_{0-3}Z'$ steht und

Z' für -H, $-S(=O)_3H$, $-NH_2$ oder -COOH steht.

Weiterhin bevorzugte Verbindungen der allgemeinen Formeln (IIa), (IIb), (IIc) und (IId) sind die , in denen

$X_1$ für N steht,
$X_2$ für N steht und
$X_3$ für C steht,

oder

$X_1$ für N steht,
$X_2$ für C steht und
$X_3$ für N steht,

oder

$X_1$ für CH oder CF steht,
$X_2$ für C steht und
$X_3$ für N steht,

oder

$X_1$ für NH steht,
$X_2$ für C steht und
$X_3$ für C steht,

oder

$X_1$ für CH oder CF steht,
$X_2$ für N steht und
$X_3$ für C steht,

$R^1$ für -H, -L-#1, -MOD oder $-(CH_2)_{0-3}Z$ steht,
Z für -H, $-NHY^3$, $-OY^3$, $-SY^3$, Halogen, $-C(=O)-NY^1Y^2$ oder $-C(=O)-OY^3$ steht,
$Y^1$ und $Y^2$ unabhängig voneinander für -H, $-NH_2$, $-(CH_2CH_2O)_{0-3}-(CH_2)_{0-3}Z'$ oder $-CH(CH_2W)Z'$ steht,
$Y^3$ für -H oder $-(CH_2)_{0-3}Z'$ steht,
Z' für -H, $-NH_2$, $-S(=O)_3H$, -COOH, $-NH-C(=O)-CH_2-CH_2-CH(NH_2)COOH$ oder $-(C(=O)-NH-CHY^4)_{1-3}-COOH$ steht,
W für -H oder -OH steht,
$Y^4$ für lineares oder verzweigtes, gegebenenfalls mit $-NH-C(=O)-NH_2$ substituiertes $C_{1-6}$ Alkyl- steht oder für gegebenenfalls mit $-NH_2$ substituiertes Aryl- oder Benzyl- steht,
$R^2$ für -H, $-C(=O)-CHY^4-NHY^5$ oder $-(CH_2)_{0-3}Z$ steht,
Z für -H, Halogen, $-OY^3$, $-SY^3$, $-NHY^3$, $-C(=O)-NY^1Y^2$ oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z' für -H, $-S(=O)_3H$, $-NH_2$ oder -COOH steht,
$Y^4$ für lineares oder verzweigtes, gegebenenfalls mit $-NH-C(=O)-NH_2$ substituiertes $C_{1-6}$ Alkyl- steht oder für gegebenenfalls mit $-NH_2$ substituiertes Aryl- oder Benzyl- steht,
$Y^5$ für -H oder $-C(=O)-CHY^6-NH_2$ steht,
$Y^6$ für lineares oder verzweigtes $C_{1-6}$ Alkyl- steht,

A für -C(=O)-, -S(=O)-, $-S(=O)_2-$ oder $-S(=O)_2-NH-$ steht,

$R^3$ für -L-#1, -MOD oder eine Alkyl-, Cycloalkyl-, Aryl-, Heteroaryl-, Heteroalkyl-, Heterocycloalkyl- Gruppe steht, die gegebenenfalls mit ein bis drei OH-Gruppen, ein bis drei Halogenatomen, ein bis drei mono-, di- oder tri-halogenierten Alkyl-Gruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei $-S(O)_n$-Alkyl-Gruppen, ein bis drei $-S(=O)_2$-NH-Alkyl-Gruppen, ein bis drei -NH-Alkyl-Gruppen, ein bis drei -N(Alkyl)$_2$-Gruppen, ein bis drei $-NH((CH_2CH_2O)_{1-20}H)$-Gruppen, ein bis drei $NH_2$-Gruppen oder ein bis drei $-(CH_2)_{0-3}Z$-Gruppen substituiert sein können,
Z für -H, Halogen, $-OY^3$, $-SY^3$, $-NHY^3$, $-C(=O)-NY^1Y^2$ oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H, $-(CH_2)_{0-3}-CH(NH-C(=O)-CH_3)Z'$,$-(CH_2)_{0-3}-CH(NH_2)Z'$, oder $-(CH_2)_{0-3}Z'$ steht,
Z' für -H, $-S(=O)_3H$, $-NH_2$ oder -COOH steht,
$R^5$ für -H, -MOD, $-NH_2$, $-NO_2$, Halogen, -CN, $-CF_3$, $-OCF_3$, $-CH_2F$, $-CH_2F$, SH oder $-(CH_2)_{0-3}Z$ steht,
Z für -H, $-OY^3$, $-SY^3$, Halogen, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z' für -H, $-S(=O)_3H$, $-NH_2$ oder -COOH steht,
$R^6$ und $R^7$ unabhängig voneinander für -H oder Halogen stehen,
$R^8$ für $C_{1-10}$-Alkyl- oder Fluor- $C_{1-10}$-Alkyl- steht,
$R^9$ für -H, -F, $-CH_3$, $-CF_3$, $-CH_2F$ oder $-CHF_2$ steht,
-L-#1 für die Gruppe $-(L_b)_o-(LIG)_p$ steht,
LIG für einen Binder steht, der nach Bindung an ein Zielmolekül einer Tumorzelle von der Tumorzelle internalisiert und intrazellulär, vorzugsweise lysosomal, prozessiert wird,
$L_b$ für einen Linker steht,
o und p jeweils unabhängig voneinander 0 oder 1 sind,

-MOD für $-CH_2-S_x-(CH_2)_{0-4}-CHY^5-COOH$ steht,
x 0 oder 1 ist,
$Y^5$ für -H oder $-NHY^6$ steht,
$Y^6$ für -H oder $-C(=O)-CH_3$ steht und

wobei $R^1$ und $R^3$ nicht gleichzeitig für -L-#1 stehen,
sowie ihre Salze, Solvate und Salze der Solvate.

Hierbei bevorzugt sind solche Verbindungen der allgemeinen Formeln (IIa), (IIb), (IIc) und (IId), in denen

$X_1$ für CH steht,
$X_2$ für C steht und
$X_3$ für N steht.

Hierbei weiterhin bevorzugt sind solche Verbindungen der allgemeinen Formeln (IIa), (IIb), (IIc) und (IId) in denen

$R^3$ für eine $C_{1-10}$-Alkyl-, $C_{6-10}$-Aryl- oder $C_{6-10}$-Aralkyl-, $C_{5-10}$-Heteroalkyl-, $C_{1-10}$-Alkyl-O-$C_{6-10}$-Aryl- oder $C_{5-10}$-Heterocycloalkyl-Gruppe steht, die mit ein bis drei OH-Gruppen, ein bis drei Halogenatomen, ein bis drei mono-, di- oder tri-halogenierten Alkylgruppe, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei -$S(=O)_n$-Alkyl-Gruppen, ein bis drei -$S(=O)_2$-NH-Alkyl-Gruppen, 1-3 -NH-Alkyl-Gruppen, ein bis drei -N($Alkyl)_2$-Gruppen, ein bis drei $NH_2$-Gruppen oder ein bis drei -$(CH_2)_{0-3}$Z-Gruppen substituiert sein können,

Z für -H, Halogen, -$OY^3$, -$SY^3$, -$NHY^3$, -C(=O)-$NY^1Y^2$ oder -C(=O)-$OY^3$ steht,

$Y^1$ und $Y^2$ unabhängig voneinander für -H, -$NH_2$, oder -$(CH_2)_{0-3}$Z' stehen,

$Y^3$ für -H, -$(CH_2)_{0-3}$-CH(NH-C(=O)-$CH_3$)Z',-$(CH_2)_{0-3}$-CH($NH_2$)Z', oder -$(CH_2)_{0-3}$Z' steht und

Z' für -H, -$S(=O)_3H$, -$NH_2$ oder -COOH steht.

Sofern der Begriff "Alkyl" nicht weiter definiert ist, steht Alkyl vorzugsweise für $C_1$-$C_{10}$-Alkyl.

Sofern der Begriff "Halogen" nicht weiter definiert ist, steht Halogen für Fluor( -F), Chlor (-Cl) und Brom (-Br).

Besonders bevorzugt sind die folgenden Verbindungen der allgemeinen Formeln (V), (VI) und (VII), in denen $R^1$, $R^2$, $R^3$, $R^4$ und $R^5$ die in der allgemeinen Formel (IIa) angegebenen Bedeutungen haben:

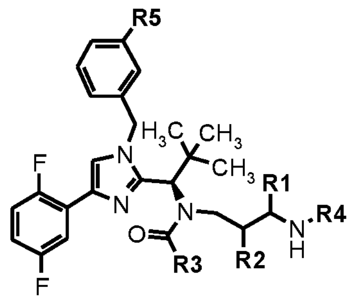

Formel (V),

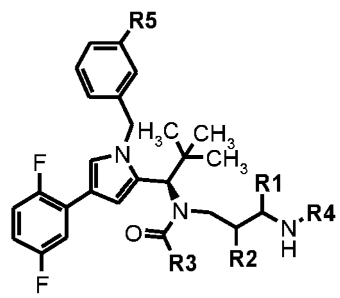

Formel (VI) und

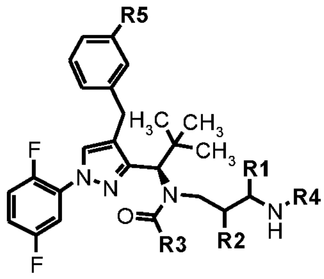

Formel (VII)

Besonders bevorzugt sind die Verbindungen der allgemeinen Formeln (V), (VI) und (VII), in denen $R^1$, $R^2$ und $R^5$ für -H stehen und $R^4$ die in der allgemeinen Formel (IIa) angegebenen Bedeutungen haben.

Insbesondere bevorzugt sind hierbei die Verbindungen der allgemeinen Formel (VI).

**Binder, der an ein Zielmolekül einer Tumorzelle bindet**

Der Begriff "Binder" wird im breitesten Sinne als ein Molekül verstanden, welches an ein Zielmolekül, das auf einer bestimmten, mit dem Binder-Wirkstoffkonjugat zu adressierenden Zielzellen-Population vorhanden ist, bindet. Der Begriff Binder ist in seiner breitesten Auslegung zu verstehen und umfasst z.B. auch Lektine, Proteine die bestimmte Zuckerketten binden können, oder Phospholipid-bindende Proteine. Solche Binder umfassen beispielsweise hochmolekulare Proteine (Bindeproteine), Polypeptide oder Peptide (Bindepeptide), nicht-peptidische (z.B. Aptamere (US5,270,163) Übersichtsartikel von Keefe AD., et al., Nat. Rev. Drug Discov. 2010; 9:537-550), oder Vitamine) und alle anderen zellbindenden Moleküle oder Substanzen. Bindeproteine sind z.B. Antikörper und Antikörperfragmente oder Antikörpermimetika wie z.B. Affibodies, Adnectins, Anticalins, DARPins, Avimers, Nanobodies (Übersichtsartikel von Gebauer M. et al., Curr. Opinion in Chem. Biol. 2009; 13:245-255; Nuttall S.D. et al., Curr. Opinion in Pharmacology 2008; 8:608-617). Bindepeptide sind z.B. Liganden eines Liganden-Rezeptorspaares, wie z.B. VEGF des Liganden-Rezeptorpaares VEGF/KDR, wie Transferrin des Liganden-Rezeptorpaares Transferrin/Transferrin-Rezeptor oder Cytokine/Cytokin-Rezeptor, wie TNFalpha des Liganden-Rezeptorpaares TNFalpha/TNFalpha Rezeptor.

Die erfindungsgemäßen Prodrugs enthalten vorzugsweise einen Binder, der an ein Zielmolekül einer Tumorzelle binden kann und in der Regel nach Bindung an das Zielmolekül von der Tumorzelle internalisiert und intrazellulär, vorzugsweise lysosomal, prozessiert wird. Dieser Binder kann entweder mit der durch das Enyzm Legumain spaltbaren Gruppe gegebenenfalls über einen Linker verbunden sein, so dass nach Spaltung der Legumain-spaltbaren Gruppe der Wirkstoff getrennt von dem Binder bzw. einem Derivat hiervon vorliegt. In diesem Fall stellt -D in der allgemeinen Formel (Ia) $-D_1$ dar und -R in der allgemeinen Formel (Ia) stellt $(L_c)_r$-LIG dar (Ausführungsform A). Ferner kann der Binder mit dem Wirkstoffmolekül gegebenenfalls über einen Linker verbunden sein, so dass nach Spaltung der Legumain-spaltbaren Gruppe der Wirkstoff zusammen mit dem Binder bzw. einem Derivat hiervon vorliegt. In diesem Fall stellt -D in der allgemeinen Formel (Ia) $-D_1-(L_b)_o$-LIG dar und R- in der allgemeinen Formel (Ia) stellt $Z_1$-(C(=O))q- dar (Ausführungsform B).

Die Verbindungen der Ausführungsform A weisen vorzugsweise die folgende allgemeine Formel III', besonders bevorzugt die folgenden allgemeinen Formeln III" und III'" auf:

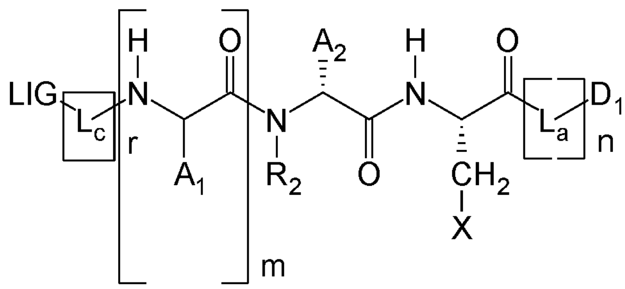

(III'),

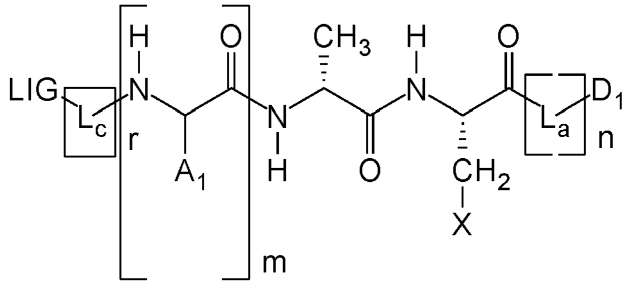

(III“) und

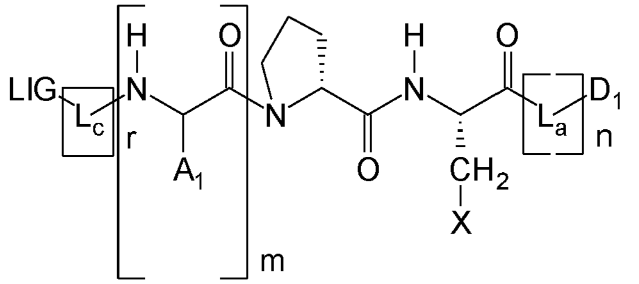

(III“‘),

wobei m, n, r, LIG, $L_a$, $L_c$, $D_1$, X, $A_1$ , $A_2$ und $R^2$ die in der allgemeinen Formel (Ia) angegebenen Bedeutungen haben.

Die Verbindungen der Ausführungsform B weisen vorzugsweise die folgende allgemeine Formel (IV’), besonders bevorzugt die allgemeinen Formeln (IV″) oder (IV‴) auf:

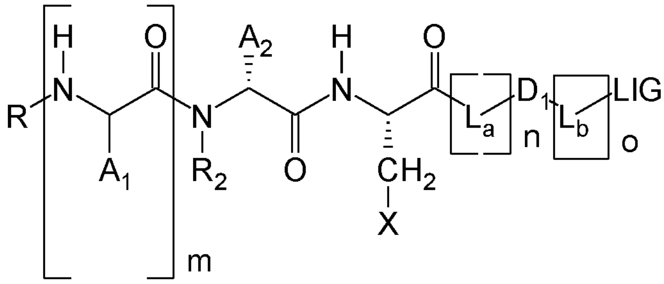

(IV‘),

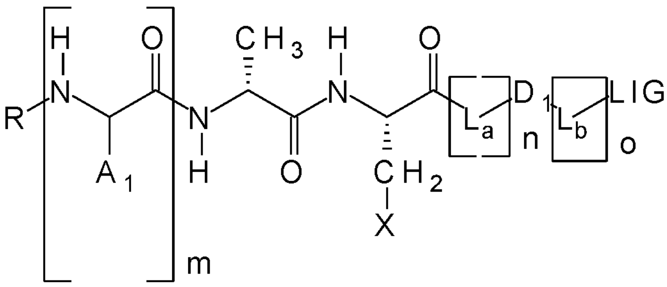

(IV“) und

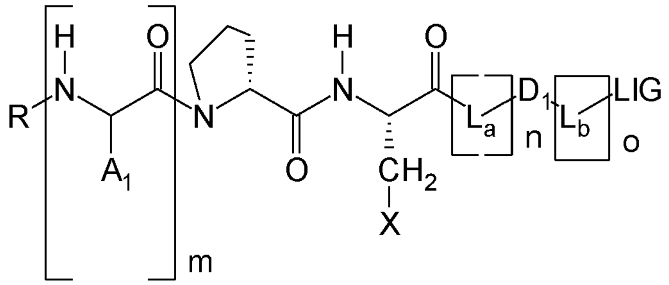

(IV‘‘‘),

wobei m, n, o, R, LIG, $L_a$, $L_b$, $D_1$, X, $A_1$, $A_2$ und $R_2$ die in der allgemeinen Formel (Ia) angegebenen Bedeutungen haben.

Der Binder LIG ist in der Regel ein Peptid, Protein (z.B. ein Antikörper) oder Derivat hiervon. Entsprechende Peptide sind aus der Literatur bekannt (einen Überblick geben D.Böhme und A. Beck-Sickinger, J.Pept.Sci. 2015-21.186; siehe auch B.Forner et al., Specialty Chemicals Magazine, May 2012; V. Ahrens et al., Future Med. Chem. 2012, 4, 1567; W.Tai et al., Mol. Pharmaceutics 2011, 8, 901; R.Soudy et al., J.Med.Chem.2013, 56, 7564 und weitere Referenzen in der Einleitung von R.Soudy et al., M.Langer et al., J.Med.Chem. 2001, 44, 1341; C.Gruendker et al., Oncology Reports 2011, 26, 629). Das Peptid bzw. Derivat hiervon wird vorzugsweise ausgewählt aus Octreotid, GnRH-III, [D-$Tyr^6$, β-$Ala^{11}$, $Phe^{13}$, $Nle^{14}$]BN(6-14), NT(8-13), c(RGDfK), HSDAVFTDNYTRLRKQMAVKKYLNSILN-$NH_2$ (SEQ ID NO: 161), NAPamide, [$Phe^7$, $Pro^{34}$]NPY, HER2-targeting peptide, ATEPRKQYATPRVFWTDAPG (SEQ ID NO: 162) oder LQWRRDDNVHNFGVWARYRL (SEQ ID NO: 163) [die Peptidsequenzen sind hier im geläufen 1-Buchstaben-Code für Aminosäuren angegeben]. Es ist möglich, weitere Peptidsequenzen mit Hilfe eines Screening-Verfahrens zu ermitteln, wie in Umlauf et al, Bioconj.Chem. 2014, Oct. 15; 25(10): 1829-37 beschrieben.

Im Falle der Ausführungsform A kann das Peptid direkt (z.B. mit seinem C-Terminus) an den N-Terminus der Legumain-spaltbaren Gruppe durch eine Peptidbindung gebunden sein. Es ist auch möglich, dass das Peptid über einen Linker $L_c$ an den N-Terminus der Legumain-spaltbaren Gruppe gebunden ist, wobei der Linker vorzugsweise an den C- oder N-Terminus des Peptids oder an eine Lysin- oder Cystein-Seitenkette des Peptids gebunden wird.

Im Falle der Ausführungsform B kann das Peptid direkt an das Wirkstoffmolekül gebunden sein. Es ist jedoch bevorzugt, dass das Peptid über einen Linker $L_b$ an das Wirkstoffmolekül gebunden ist, wobei der Linker vorzugsweise an den C- oder N-Terminus des Peptids oder an eine Lysin- oder Cystein-Seitenkette des Peptids gebunden wird. Die Bindung von $L_b$ bzw. des Peptids erfolgt in der Regel durch Substitution eines H-Atoms im Wirkstoffmolekül.

So können im Falle der Verbindungen der allgemeinen Formeln (IIa), (IIb), (IIc), (IId), (V), (VI) oder (VII) durch Substitution eines H-Atoms an $R^1$, $R^2$, $R^3$, $R^5$ oder $R^8$ in einer dem Durchschnittsfachmann bekannten Weise Konjugate erhalten werden, wobei einer der Substituenten $R^1$, $R^2$, $R^3$, $R^5$, oder $R^8$ -$(L_b)_o$-LIG darstellt. Besonders bevorzugt wird als Binder LIG ein Antikörper bzw. ein Antigen-bindendes Fragment oder Derivat hiervon, der bzw. das an ein extrazelluläres Zielmolekül einer Tumorzelle bindet. Besonders bevorzugt ist LIG ein Antikörper bzw. Fragment hiervon, an den bzw. an das ein oder mehrere cytotoxische Wirkstoffmoleküle gebunden sind. Im Falle der Ausführungsform A sind die erfindungsgemäßen Verbindungen also Antikörper-Drug-Konjugate (ADCs) der folgenden allgemeinen Formeln (IIIa') bzw. (IIIa") bzw. (IIIa"):

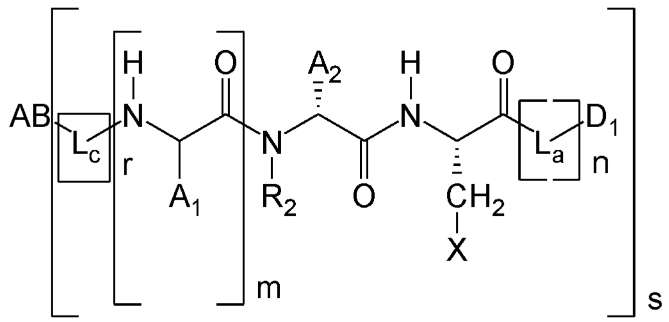

(IIIa‘),

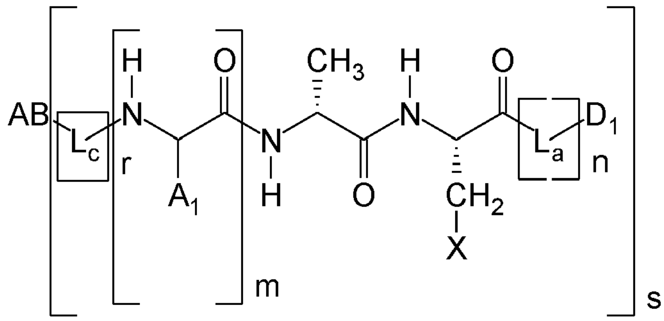

(IIIa“) und

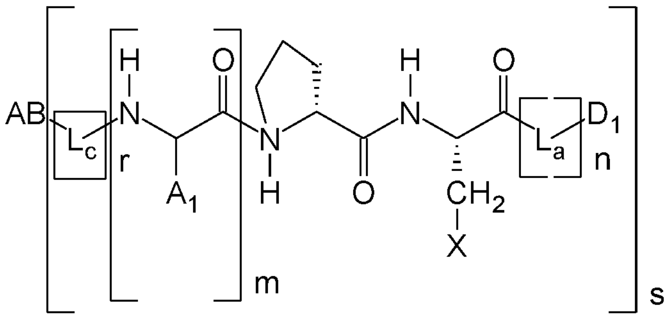

(IIIa“‘)

wobei m, n, r, $L_a$, $L_c$, $D_1$, X, $A_1$ , $A_2$ und $R^2$ die in der allgemeinen Formel (Ia) angegebenen Bedeutungen haben, AB einen Antikörper darstellt, und s eine Zahl von 1 bis 20, vorzugsgweise 2 bis 8, besonders bevorzugt 3 bis 5 wie z.B. 4 darstellt. Hierbei ist $D_1$ vorzugsweise eine Verbindung der allgemeinen Formel (IIa), (IIb), (IIc), (IId), (V), (VI) oder (VII), wobei ein Substituent ausgewählt aus $R^1$, $R^2$, $R^3$, $R^4$, $R^8$ nicht die oben unter den allgemeinen Formeln (IIa), (IIb), (llc), (IId), (V), (VI) und (VII) angegebene Bedeutung aufweist, sondern die Bindung an $L_a$ bzw. eine Carbonylgruppe darstellt.

Im Falle der Ausführungsform B sind die erfindungsgemäßen Verbindungen Antikörper-Prodrug-Konjugate (APDCs) der folgenden allgemeinen Formeln (IVa’), bzw. (IVa"), bzw. (IVa‴):

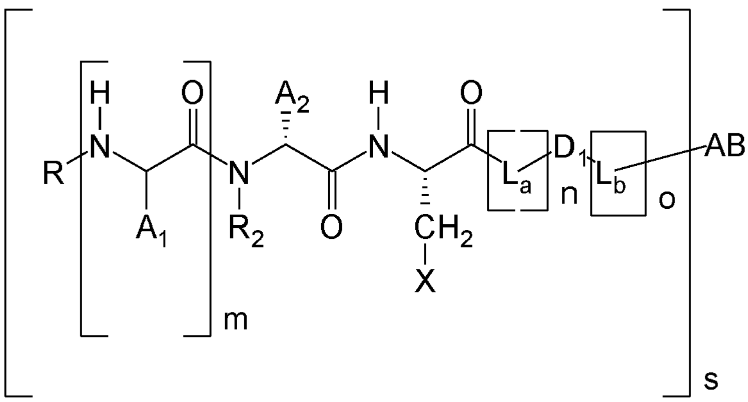

(IVa‘)

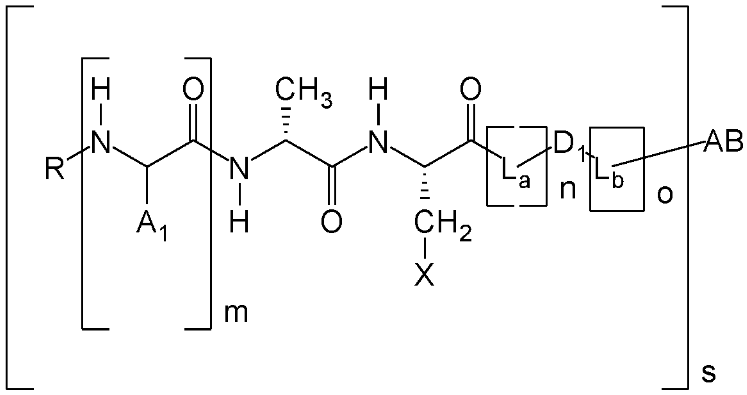

(IVa“) und

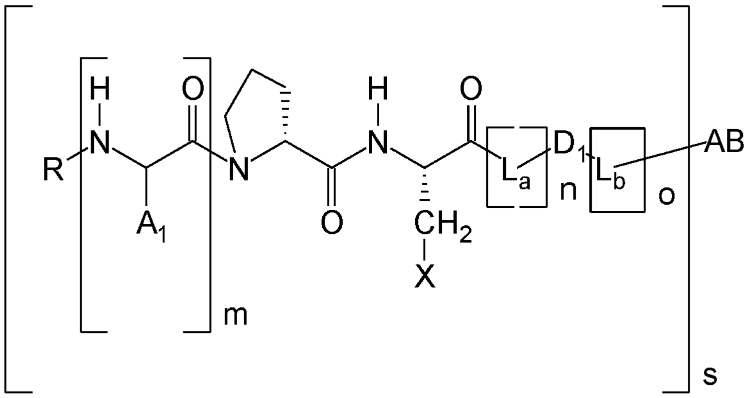

(IVa“‘),

in denen m, n, o, R, $L_a$, $L_b$, $D_1$, X, $A_1$ , $A_2$ und $R^2$ die in der allgemeinen Formel (Ia) angegebenen Bedeutungen haben und AB einen Antikörper darstellt, und s eine Zahl von 1 bis 20, vorzugsweise 2 bis 8, besonders bevorzugt 3 bis 5 wie z.B. 4 darstellt. Hierbei ist $D_1$ vorzugsweise eine Verbindung gemäß den allgemeinen Formeln (IIa), (IIb), (IIc), (IId), (V), (VI) oder (VII), wobei ein Substituent $R^4$ nicht die oben unter den allgemeinen Formenl (IIa), (IIb), (IIc), (IId), (V), (VI) oder (VII) angegebene Bedeutung aufweist, sondern die Bindung an $L_a$ bzw. eine Carbonylgruppe darstellt.

Der Antikörper (wie z.B. AB in obigen allgemeinen Formeln (IIIa) und (IVa) ist vorzugsweise ein humaner, humanisierter oder chimärer monoklonaler Antikörper oder ein Antigen-bindendes Fragment hiervon, insbesondere ein anti-TWEAKR-Antikörper, ein anti-EGFR-Antikörper, ein anti-B7H3-Antikörper oder ein anti-HER2-Antikörper oder eine Antigen-bindendes Fragment von diesen. Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Aus der Literatur sind verschiedene Möglichkeiten der kovalenten Kopplung (Konjugation) von organischen Molekülen an Antikörper bekannt. Erfindungsgemäß bevorzugt ist die Konjugation an den Antikörper über ein oder mehrere Schwefelatome von Cystein-Resten des Antikörpers und/oder über ein oder mehrere NH-Gruppen von Lysin-Resten des Antikörpers. Es ist jedoch auch möglich, das organische Molekül an den Antikörper über freie Carboxylgruppen oder über Zuckerreste des Antikörpers zu binden.

Der Antikörper bindet an ein extrazelluläres Zielmolekül der Tumorzelle. Ein "Zielmolekül" wird im breitesten Sinne als ein Molekül verstanden, welches in der Zielzellenpopulation vorhanden ist, und kann ein Protein (z.B. ein Rezeptor eines Wachstumsfaktors) oder ein nicht-peptidisches Molekül sein (z.B. ein Zucker oder Phospholipid). Bevorzugt ist es ein Rezeptor oder ein Antigen.

Der Begriff "extrazelluläres" Zielmolekül beschreibt ein an die Zelle gebundenes Zielmolekül, welches sich auf der Außenseite einer Zelle befindet oder den Teil eines Zielmoleküls, welcher sich auf der Außenseite einer Zelle befindet, d.h. ein Antikörper kann an einer intakten Zelle an sein extrazelluläres Zielmolekül binden. Ein extrazelluläres Zielmolekül kann in der Zellmembran verankert sein oder Bestandteil der Zellmembran sein. Der Fachmann kennt Verfahren, um

extrazelluläre Zielmoleküle zu identifizieren. Für Proteine kann dies über eine Bestimmung der Transmembrandomäne(n) und die Orientierung des Proteins in der Membran geschehen. Diese Angaben sind in der Regel in Proteindatenbanken (z.B. SwissProt) hinterlegt.

Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Under dem Begriff "Binder" wird gemäß der vorliegenden Erfindung ein Binderpeptid, ein Derivat eines Binderpeptids, ein Binderprotein oder ein Derivat eines Binderproteins verstanden. Der Binder ist über eine Bindung mit dem Linker verknüpft. Die Verknüpfung des Binders kann mittels eines Heteroatoms des Binders erfolgen. Erfindungsgemäße Heteroatome des Binders, die zur Verknüpfung verwendet werden können, sind:

Schwefel,über eine Sulfhydrylgruppe des Binders,

Sauerstoff, über eine Carboxylgruppe oder Hydroxylgruppe des Binders und

Stickstoff, über eine primäre oder sekundäre Amingruppe.

Insbesondere wird gemäß der vorliegenden Erfindung unter dem Begriff "Binder" ein Antikörper verstanden.

Die oben aufgeführten Heteroatome können im natürlichen Antikörper vorhanden sein oder durch chemische oder molekularbiologische Methoden eingeführt werden. Erfindungsgemäß hat die Verknüpfung des Antikörpers mit dem organischen Rest in Formel (I) nur einen geringen Einfluß auf die Bindeaktivität des Antikörpers zum Zielmolekül.

In einer bevorzugten Ausführungsform hat die Verknüpfung keinen Einfluß auf die Bindeaktivität des Binders zum Zielmolekül.

Der Begriff "Antikörper" wird gemäß der vorliegenden Erfindung in seinem breitesten Sinne verstanden und umfasst Immunglobulinmoleküle, beispielsweise intakte oder modifizierte monoklonale Antikörper, polyklonale Antikörper oder multispezifische Antikörper (z.B. bispezifische Antikörper). Ein Immunglobulinmolekül umfasst bevorzugt ein Molekül mit vier Polypeptidketten, zwei schwere Ketten (H Ketten) und zwei leichte Ketten (L Ketten), welche typischerweise durch Disulfidbrücken verknüpft sind. Jede schwere Kette umfasst eine variable Domäne der schweren Kette (abgekürzt VH) und konstante Domäne der schweren Kette. Die konstante Domäne der schweren Kette kann beispielsweise drei Domänen CH1, CH2 und CH3 umfassen. Jede leichte Kette umfasst eine variable Domäne (abgekürzt VL) und eine konstante Domäne. Die konstante Domäne der leichten Kette umfasst eine Domäne (abgekürzt CL). Die VH und VL Domänen können weiter unterteilt werden in Regionen mit Hypervariabilität, auch Komplementaritäts-bestimmende Regionen genannt ("complementarity determining region", abgekürzt CDR) und Regionen mit geringerer Sequenzvariabilität ("framework region", abgekürzt FR). Jede VH und VL Region setzt sich typischerweise aus drei CDRs und bis zu vier FRs zusammen. Beispielsweise vom Aminoterminus zum Carboxyterminus in der folgenden Reihenfolge FR1, CDR1, FR2, CDR2, FR3, CDR3, FR4. Ein Antikörper kann aus jeder dafür geeeigneten Spezies erhalten werden z.B. Kaninchen, Lama, Kamel, Maus, oder Ratte. In einer Ausführungsform ist der Antikörper humanen oder murinen Ursprungs. Ein Antikörper kann z.B. human, humanisiert oder chimär sein.

Der Begriff "monoklonaler" Antikörper bezeichnet Antikörper, die aus einer Population substantiell homogener Antikörper erhalten wurde, d.h. individuelle Antikörper der Population sind bis auf natürlich auftretende Mutationen, welche in geringfügiger Anzahl auftreten können, identisch. Monoklonale Antikörper erkennen mit hoher Spezifität eine einzige antigene Bindestelle. Der Begriff monoklonaler Antikörper bezieht sich nicht auf ein bestimmtes Herstellungsverfahren.

Der Begriff "intakter" Antikörper bezieht sich auf Antikörper, die sowohl eine Antigen-bindende Domäne als auch die konstante Domäne der leichten und schweren Kette umfassen. Die konstante Domäne kann eine natürlich vorkommende Domäne sein, oder eine Variante davon, bei der mehrere Aminosäurepositionen verändert wurden.

Der Begriff "modifizierter intakter" Antikörper bezieht sich auf intakte Antikörper, die mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert wurden. Des Weiteren können Antikörper dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug;26(8):925-32).

Der Begriff "humaner" Antikörper bezeichnet Antikörper, die aus einem Menschen erhalten werden können oder synthetische humane Antikörper sind. Ein "synthetischer" humaner Antikörper ist ein Antikörper, der in Teilen oder schweren als ganzes von synthetischen Sequenzen in silico erhältlich ist, die auf der Analyse humaner Antikörpersequenzen basieren. Ein humaner Antikörper kann z.B. durch eine Nukleinsäure kodiert sein, die aus einer Bibliothek von

Antikörpersequenzen, die humanen Ursprungs sind, isoliert wurde. Ein Beispiel solcher Antikörper ist in Söderlind et al., Nature Biotech. 2000, 18:853-856 zu finden.

Der Begriff "humanisierter" oder "chimärer" Antikörper beschreibt Antikörper, die aus einem nicht-humanen und einem humanen Sequenzanteil bestehen. Bei diesen Antikörpern wird ein Teil der Sequenzen des humanen Immunoglobulins (Rezipient) durch Sequenzanteile eines nicht-humanen Immunoglobulins (Donor) ersetzt. Der Donor ist in vielen Fällen ein murines Immunoglobulin. Bei humanisierten Antikörpern werden Aminosäuren der CDR des Rezipienten durch Aminosäuren des Donors ersetzt. Manchmal werden auch noch Aminosäuren des Frameworks durch korrespondierende Aminosäuren des Donors ersetzt. In machen Fällen enthält der humanisierte Antikörper Aminosäuren, die weder im Rezipient noch im Donor enthalten waren und die während der Optimierung des Antikörpers eingefügt wurden. Bei chimären Antikörpern werden die variablen Domänen des Donor-Immunoglobulins mit den konstanten Regionen eines humanen Antikörpers fusioniert.

Der Begriff Komplementaritäts-bestimmende Region (CDR) wie er hier verwendet wird, bezieht sich auf diejenigen Aminosäuren einer variablen Antikörperdomäne, die für die Bindung an das Antigen notwendig sind. Jede variable Region hat typischerweise drei CDR Regionen, welche als CDR1, CDR2 und CDR3 bezeichnet werden. Jede CDR Region kann Aminosäuren nach der Definition von Kabat und/oder Aminosäuren eines Hypervariablen Loops definiert nach Chotia umfassen. Die Definition nach Kabat umfasst zum Beispiel die Region von ungefähr Aminosäureposition 24 - 34 (CDR1), 50 - 56 (CDR2) und 89 - 97 (CDR3) der variablen leichten Kette und 31 - 35 (CDR1), 50 - 65 (CDR2) und 95 - 102 (CDR3) der variablen schweren Kette (Kabat et al., Sequences of Proteins of Immunological Interest, 5th Ed. Public Health Service, National Institutes of Health, Bethesda, MD. (1991)). Die Definition nach Chotia umfasst zum Beispiel die Region von ungefähr Aminosäureposition 26 - 32 (CDR1), 50 - 52 (CDR2) und 91 -96 (CDR3) der variablen leichetn Kette und 26 - 32 (CDR1), 53 - 55 (CDR2) und 96 - 101 (CDR3) der variablen schweren Kette Chothia and Lesk; J Mol Biol 196: 901-917 (1987)). In manchen Fällen kann eine CDR Aminosäuren aus einer CDR Region definiert nach Kabat und Chotia umfassen.

Abhängig von der Aminosäure-Sequenz der konstanten Domäne der schweren Kette können Antikörper in verschiedene Klassen eingeteilt werden. Es gibt fünf Hauptklassen von intakten Antikörpern: IgA, IgD, IgE, IgG und IgM, wobei mehrere davon in weitere Unterklassen aufgegliedert werden können. (Isotypen), z.B. IgG1, IgG2, IgG3, IgG4, IgA1 und IgA2. Die konstante Domäne der schweren Kette, die zu den unterschiedlichen Klassen korrespondieren, werden als [alpha/$\alpha$], [deita/$\delta$], [epsilon/$\varepsilon$], [gamma/$\gamma$] und [my/$\mu$] bezeichnet. Sowohl die dreidimensionale Struktur als auch die Untereinheitenstruktur von Antikörpern sind bekannt.

Der Begriff "funktionales Fragment" oder "antigen-bindendes Antikörperfragment" eines Antikörpers/Immunoglobulins ist definiert als ein Fragment eines Antikörpers/Immunoglobulins (z.B. die variable Domänen eines IgG), welches die Antigen-Bindedomänen des Antikörpers/Immunoglobulins noch umfasst. Die "Antigen-Bindedomäne" eines Antikörpers umfasst typischerweise eine oder mehrere Hypervariable Regionen eines Antikörpers, z.B. die CDR, CDR2 und/oder CDR3 Region. Allerdings kann auch die "Framework" oder die "Gerüst" Region eines Antikörpers zur Bindung des Antikörpers an das Antigen eine Rolle spielen. Die Framework Region bildet das Gerüst für die CDRs. Vorzugsweise umfasst die Antigen-Bindedomäne mindestens die Aminosäuren 4 bis 103 der variablen leichten Kette und Aminosäure 5 bis 109 der variablen schweren Kette, bevorzugter die Aminosäure 3 bis 107 der variablen leichten Kette und 4 bis 111 der variablen schweren Kette, besonders bevorzugt sind die kompletten variablen leichten und schweren Ketten , also Aminosäure 1 - 109 der VL und 1 bis 113 der VH (Nummerierung nach WO97/08320).

"Funktionale Fragmente" oder "antigen-bindende Antikörperfragmente" der Erfindung umfassen nicht abschliessend Fab, Fab', $F(ab')_2$ und Fv Fragmente, Diabodies, Single Domain Antibodies (DAbs), linerae Antikörper, Einzelketten Antikörper (single-chain Fv, abgekürzt scFv); und multispezifische, wie z.B. bi- und tri-spezifische, Antikörper, gebildet aus Antikörperfragmenten C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). Andere Antikörper als "multi-spezifische" oder "multi-funktionale" sind solche mit identischen Bindestellen. Multispezifische Antikörper können spezifisch für unterschiedliche Epitope eines Antigens sein oder spezifisch für Epitope von mehr als einem Antigen sein (siehe z.B. WO 93/17715; WO 92/08802; WO 91/00360; WO 92/05793; Tutt, et al., 1991, J. Immunol. 147:60 69; U. S. Pat. Nos. 4,474,893; 4,7 14,68 1 ; 4,925,648; 5,573,920; 5,601,8 19; oder Kostelny et al., 1992, J. Immunol. 148: 1547 1553). Ein $F(ab')_2$ oder Fab Molekül kann so konstruiert werden, dass die Zahl der intermolekularen Disulfidinteraktionen, die zwischen den Ch1 und den CL Domänen stattfindet, reduziert oder oder komplett verhindert werden kann.

"Epitope" bezeichnen Proteindeterminanten, die eine spezifische Bindung mit einem Immunglobulin oder T-Zell-Rezeptoren eingehen können. Epitopische Determinanten bestehen normalerweise aus chemisch aktiven Oberflächengruppen von Molekülen wie Aminosäuren oder Zuckerseitenketten, oder Kombinationen hiervon, und weisen normalerweise spezifische 3-dimensionale Struktureigenschaften wie auch spezifische Ladungseigenschaften auf.

"Funktionale Fragmente" oder "antigen-bindende Antikörperfragmente" können mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert werden. Des Weiteren können Antikörper und antigen-bindende Fragmente dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug; 26(8):925-32).

Polyklonale Antikörper können durch für den Durchschnittsfachmann bekannte Methoden hergestellt werden. Monoklonale Antiköper können durch für den Durchschnittsfachmann bekannte Methoden hergestellt werden (Köhler und Milstein, Nature, 256, 495-497, 1975). Humane bzw. humanisierte monoklonale Antiköper können durch für den Durchschnittsfachmann bekannte Methoden hergestellt werden (Olsson et al., Meth Enzymol. 92, 3-16 bzw. Cabilly et al US 4,816,567 oder Boss et al US 4,816,397).

Der Durchschnittsfachmann kennt vielfältige Methoden, um humane Antikörper und deren Fragmente herzustellen, wie beispielsweise mittels transgener Mäuse (N Lonberg und D Huszar, Int Rev Immunol. 1995; 13(1):65-93) oder Phage Display Technologien (Clackson et al., Nature. 1991 Aug 15;352(6336):624-8). Antikörper der Erfindung können aus rekombinanten Antikörperbibliotheken erhalten werden, die z.B. auf den Aminosäuresequenzen einer Vielzahl von Antikörpern besteht, die aus einer großen Anzahl gesunder Freiwilliger erstellt wurden. Antikörper können auch mittels bekannter rekombinanter DNS-Technologien hergestellt werden. Die Nukleinsäuresequenz eines Antikörpers kann durch Routinesequenzierung erhalten werden, oder ist aus öffentlich zugänglichen Datenbanken erhältlich.

Ein "isolierter" Antikörper oder Binder wurde von anderen Bestandteilen der Zelle gereinigt. Kontaminierende Bestandteile einer Zelle, welche mit einer diagnostischen oder therapeutischen Verwendung interferieren können, sind z.B. Enzyme, Hormone, oder andere peptidische- oder nicht-peptidische Bestandteile einer Zelle. Bevorzugt ist ein Antikörper oder Binder, der zu mehr als 95 Gew-% bezogen auf den Antikörper bzw. Binder aufgereinigt wurde (bestimmt durch z.B. Lowry Verfahren, UV-Vis Spektroskopie oder durch SDS-Kapillargelelektrophorese). Ausserdem ein Antikörper, der soweit aufgereinigt wurde, dass mindestens 15 Aminosäuren des Aminoterminus oder einer internen Aminosäuresequenz bestimmt werden können, oder zur Homogenität aufgereinigt wurde, wobei die Homogenität bestimmt wird durch SDS-PAGE unter reduzierenden oder nicht-reduzierenden Bedingungen (die Detektion kann mittels Coomassie Blau Anfärbung oder bevorzugt durch Silberfärbung bestimmt werden). Jedoch wird ein Antikörper normalerweise durch einen oder mehrere Reinigungsschritte hergestellt.

Der Begriff "spezifische Bindung" oder "bindet spezifisch" bezieht sich auf einen Antikörper oder Binder, der an ein vorbestimmtes Antigen/Zielmolekül bindet. Spezifische Bindung eines Antikörpers oder Binders beschreibt typischerweise einen Antikörper bzw. Binder mit einer Affinität von mindestens $10^{-7}$ M (als Kd-Wert; also vorzugsweise solche mit kleineren Kd-Werten als $10^{-7}$ M), wobei der Antikörper bzw. Binder eine mindestens zweifach höhere Affinität zum vorbestimmten Antigen/Zielmolekül als zu einem nichtspezifischen Antigen/Zielmolekül hat (z.B. Rinder Serumalbumin, oder Casein), welches nicht das vorbestimmte Antigen/Zielmolekül oder ein eng verwandtes Antigen/Zielmolekül ist. Die Antikörper weisen vorzugsweise eine Affinität von mindestens $10^{-7}$ M (als Kd-Wert; also vorzugsweise solche mit kleineren Kd-Werten als $10^{-7}$ M), vorzugsweise von mindestens $10^{-8}$ M, besonders bevorzugt in dem Bereich von $10^{-9}$ M bis $10^{-11}$ M auf. Die Kd-Werte können durch z.B. Oberflächenplasmonresonanzspektroskopie bestimmt werden.

Die erfindungsgemäßen Antikörper-Wirkstoff-Konjugate weisen ebenfalls Affinitäten in diesen Bereichen auf. Durch die Konjugation der Wirkstoffe wird die Affinität vorzugsweise nicht wesentlich beeinflusst (in der Regel wird die Affinität weniger als eine Größenordnung verringert, also z.B. maximal von $10^{-8}$ M auf $10^{-7}$ M).

Die erfindungsgemäßen verwendeten Antikörper zeichnen sich weiterhin vorzugsweise durch eine hohe Selektivität aus. Eine hohe Selektivität liegt vor, wenn der erfindungsgemäße Antikörper eine mindestens um den Faktor 2, bevorzugt Faktor 5 oder insbesondere bevorzugt Faktor 10 bessere Affinität am Zielprotein aufweisst als an einem unabhängigen anderen Antigen, z.B. humanem Serumalbumin (die Affinität kann z.B. durch Oberflächenplasmonenresonanzspektroskopie bestimmt werden).

Zudem sind die erfindungsgemäßen verwendeten Antikörper vorzugsweise kreuzreaktiv. Um präklinische Studien, z.B. toxikologische oder Wirksamkeitsstudien (z.B. in Xenograft-Mäusen), zu erleichtern und besser interpretieren zu können, ist es von Vorteil, wenn der erfindungsgemäß verwendete Antikörper nicht nur das humane Zielprotein bindet, sondern auch in der für die Studien verwendeten Spezies das Spezies-Zielprotein bindet. In einer Ausführungsform ist der erfindungsgemäß verwendete Antikörper zusätzlich zum humanen Zielprotein kreuzreaktiv zum Zielprotein mindestens einer weiteren Spezies. Für toxikologische und Wirksamkeitsstudien werden bevorzugt Spezien der Familien Nager, Hunde und nicht-humane Primaten, verwendet. Bevorzugte Nager Spezien sind Maus und Ratte. Bevorzugte nicht-humane Primaten sind Rhesusaffen, Schimpansen und Langschwanzmakaken.

In einer Ausführungsform ist der erfindungsgemäß verwendete Antikörper zusätzlich zum humanen Zielprotein kreuzreaktiv zum Zielprotein mindestens einer weiteren Spezies ausgewählt aus der Gruppe von Spezien bestehend aus Maus, Ratte und Langschwanzmakak (Macaca fascicularis). Insbesondere bevorzugt sind erfindungsgemäß verwendete Antikörper, die zusätzlich zum humanen Zielprotein mindestens kreuzreaktiv zum Maus-Zielprotein sind. Bevorzugt sind kreuzreaktive Antikörper, deren Affinität zum Zielprotein der weiteren nicht-humanen Spezies sich nicht um mehr als den Faktor 50, insbesondere nicht mehr als den Faktor zehn von der Affinität zum humanen Zielprotein unterscheidet.

*Gegen ein Krebs-Zielmolekül gerichtete Antikörper*

Bevorzugt ist das Zielmolekül, gegen das der Binder, z.B. ein Antikörper oder ein Antigen bindendes Fragment davon, gerichtet ist, ein Krebs-Zielmolekül. Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Antikörper, welche spezifisch gegen ein Antigen, wie z.B. ein Krebszell-Antigen, gerichtet sind, können vom Durchschnittsfachmann mittels ihm bekannter Verfahren hergestellt werden (wie z.B. rekombinante Expression) oder kommerziell erworben werden (wie z.B. von Merck KGaA, Deustchland). Beispiele bekannter kommerziell erhältlicher Antikörper in der Krebstherapie sind Erbitux® (Cetuximab, Merck KGaA), Avastin® (Bevacizumab, Roche) und Herceptin® (Trastuzumab, Genentech). Trastuzumab ist ein rekombinanter humanisierter monokloaler Antikörper vom IgG1kappa Typ, welcher mit hoher Affinität in einem Zell-basierten Assay (Kd = 5 nM) die extrazelluläre Domäne des humanen epidermalen Wachstumsrezeptors bindet. Der Antikörper wird rekombinant in CHO-Zellen hergestellt.

In einer bevorzugten Ausführungsform ist das Zielmolekül ein selektives Krebs-Zielmolekül.

In einer besonders bevorzugten Ausführungsform ist das Zielmolekül ein Protein.

In einer Ausführungsform ist das Zielmolekül ein extrazelluläres Zielmolekül. In einer bevorzugten Ausführungsform ist das extrazelluläre Zielmolekül ein Protein.

Krebs-Zielmoleküle sind dem Fachmann bekannt. Beispiele hierfür sind im Folgenden aufgeführt.

Beispiele für Krebs-Zielmoleküle sind:

(1) EGFR (EGF-Rezeptor, NCBI-Referenzsequenz NP_005219.2, NCBI-Gene ID: 1956)

(2) Mesothelin (SwissProt-Referenz Q13421-3), wobei Mesothelin von den Aminosäuren 296-598 kodiert wird. Aminosäuren 37-286 kodieren für "megakaryocyte-potentiating factor". Mesothelin ist durch einen GPI-Anker in der Zellmembran verankert und ist extrazellulär lokalisiert.

(3) Carboanhydrase IX (CA9, SwissProt-Referenz Q16790), NCBI-Gene ID: 768)

(4) C4.4a (NCBI -Referenzsequenz NP_055215.2; Synonym LYPD3, NCBI-Gene ID: 27076)

(5) CD52 (NCBI-Referenzsequenz NP_001794.2)

(6) HER2 (ERBB2; NCBI-Referenzsequenz NP_004439.2; NCBI-Gene ID: 2064)

(7) CD20 (NCBI-Referenzsequenz NP_068769.2)

(8) das Lymphozyten Aktivierungsantigen CD30 (SwissProt ID P28908)

(9) das Lymphozyten Adhesionsmolekül CD22 (SwissProt ID P20273; NCBI-Gene ID: 933)

(10) das Myloidzellen Oberflächenantigen CD33 (SwissProt ID P20138; NCBI-Gene ID: 945)

(11) das Transmembran Glykoprotein NMB (GPNMB, SwissProt ID Q14956, NCBI-Gene ID: 10457)

(12) das Adhesionsmolekül CD56 (SwissProt ID P13591)

(13) das Oberflächenmolekül CD70 (SwissProt ID P32970, NCBI-Gene ID: 970)

(14) das Oberflächenmolekül CD74 (SwissProt ID P04233, NCBI-Gene ID: 972)

(15) das B-Lymphozyten Antigen CD19 (SwissProt ID P15391, NCBI-Gene ID: 930)

(16) das Oberflächenprotein Mucin-1 (MUC1, SwissProt ID P15941, NCBI-Gene ID: 4582)

(17) das Oberflächenprotein CD138 (SwissProt ID P18827)

(18) das Integrin alphaV (NCBI -Referenzsequenz: NP_002201.1, NCBI-Gene ID: 3685)

(19) das teratocarcinoma-derived growth factor 1 Protein TDGF1 (NCBI - Referenzsequenz: NP_003203.1, NCBI-Gene ID: 6997)

(20) das Prostata spezifische Membranantigen PSMA (Swiss Prot ID: Q04609; NCBI-Gene ID: 2346)

(21) die Tyrosin-Proteinkinase EPHA2 (Swiss Prot ID: P29317, NCBI-Gene ID: 1969)

(22) das Oberflächenprotein SLC44A4 (NCBI -Referenzsequenz: NP_001171515.1, NCBI-Gene ID: 80736)

(23) das Oberflächenprotein BMPR1B (SwissProt: O00238)

(24) das Transportprotein SLC7A5 (SwissProt: Q01650)

(25) das epitheliale Psostataantigen STEAP1 (SwissProt: Q9UHE8, Gene ID: 26872)

(26) das Ovarkarzinomantigen MUC16 (SwissProt: Q8WXI7, Gene ID: 94025)

(27) das Transportprotein SLC34A2 (SwissProt: O95436, Gene ID: 10568)

(28) das Oberflächenprotein SEMA5b (SwissProt: Q9P283)

(29) das Oberflächenprotein LYPD1 (SwissProt: Q8N2G4)

(30) der Endothelin Rezeptor Typ B EDNRB (SwissProt: P24530, NCBI-Gene ID: 1910)

(31) das Ringfingerprotein RNF43 (SwissProt: Q68DV7)

(32) das Prostatakarzinom-assozierte Protein STEAP2 (SwissProt: Q8NFT2)

(33) der Kationenkanal TRPM4 (SwissProt: Q8TD43)

(34) der Komplementrezeptor CD21 (SwissProt: P20023)

(35) das B-Zell Antigen Rezeptorkomplex-assozierte Protein CD79b (SwissProt: P40259, NCBI-Gene ID: 974)

(36) das Zelladhäsionsantigen CEACAM6 (SwissProt: P40199)

(37) die Dipeptidase DPEP1 (SwissProt: P16444)

(38) der Interleukinrezeptor IL20Ralpha (SwissProt: Q9UHF4, NCBI-Gene ID: 3559)

(39) das Proteoglykan BCAN (SwissProt: Q96GW7)

(40) der Ephrin Rezeptor EPHB2 (SwissProt: P29323)

(41) das Prostatastammzellen-assozierte Protein PSCA (NCBI -Referenzsequenz: NP_005663.2 )

(42) das Oberflächenprotein LHFPL3 (SwissProt: Q86UP9)

(43) das Rezeptorprotein TNFRSF13C (SwissProt: Q96RJ3)

(44) das B-Zell Antigen Rezeptorkomplex-assozierte Protein CD79a (SwissProt: P11912)

(45) das Rezeptorprotein CXCR5 (CD185; SwissProt: P32302; NCBI-Gene ID 643, NCBI -Referenzsequenz: NP_001707.1)

(46) der Ionenkanal P2X5 (SwissProt: Q93086)

(47) das Lymphozytenantigen CD180 (SwissProt: Q99467)

(48) das Rezeptorprotein FCRL1 (SwissProt: Q96LA6)

(49) das Rezeptorprotein FCRL5 (SwissProt: Q96RD9)

(50) das MHC Klasse II Molekül Ia Antigen HLA-DOB (NCBI -Referenzsequenz: NP_002111.1)

(51) das T-Zell Protein VTCN1 (SwissProt: Q7Z7D3)

(52) TWEAKR (FN14, TNFRSF12A, NCBI -Referenzsequenz: NP_057723.1, NCBI-Gene ID: 51330)

(53) das Lymphozytenantigen CD37 (Swiss Prot: P11049, NCBI-Gene ID: 951)

(54) Der FGF Rezeptor 2; FGFR2 (NCBI-Gene ID: 2263; Official Symbol: FGFR2). Der FGFR2 Rezeptor kommt in verschiedenen Spleißvarianten vor (alpha, beta, IIIb, IIIc). Alle Spleißevarianten können als Zielmolekül fungieren.

(55) das transmembrane Glycoprotein B7H3 (CD276; NCBI-Gene ID: 80381 NCBI - Referenzsequenz: NP_001019907.1, Swiss Prot: Q5ZPR3-1)

(56) der B Zell Rezeptor BAFFR (CD268; NCBI-Gene ID: 115650)

(57) das Rezeptorprotein ROR 1 (NCBI-Gene ID: 4919)

(58) der Oberflächenrezeptor CD123 (IL3RA; NCBI-Gene ID: 3563; NCBI - Referenzsequenz: NP_002174.1; Swiss-Prot: P26951)

(59) das Rezeptorprotein Syncytin ( NCBI-Gene ID 30816)

(60) die Aspartat beta Hydroxylase (ASPH; NCBI-Gene ID 444)

(61) das Zelloberflächen Glycoprotein CD44 (NCBI-Gene ID: 960)

(62) CDH15 (Cadherin 15, NCBI-Gene ID: 1013)

(63) das Zelloberflächen Glycoprotein CEACAM5 (NCBI-Gene ID: 1048)

(64) das Zelladhäsionsmolekül L1-like (CHL1, NCBI-Gene ID: 10752)

(65) die Rezeptortyrosin-Kinase c-Met (NCBI-Gene ID: 4233)

(66) der Notch Ligand DLL3 (NCBI-Gene ID: 10683)

(67) das Ephrin A4 (EFNA4, NCBI-Gene ID: 1945)

(68) die Ectonucleotid-Pyrophosphatase/Phosphodiesterase 3 (ENPP3, NCBI-Gene ID: 5169)

(69) der Koagulationsfaktor III (F3, NCBI-Gene ID: 2152)

(70) der FGF Rezeptor 3 (FGFR3, NCBI-Gene ID: 2261)

(71) die Folatehydrolase FOLH1 (NCBI-Gene ID: 2346)

(72) der Folaterezeptor 1 (FOLR1; NCBI-Gene ID: 2348)

(73) die Guanylatzyklase 2C (GUCY2C, NCBI-Gene ID: 2984)

(74) die KIT proto-Oncogen Receptortyrosinkinase (NCBI-Gene ID: 3815)

(75) das lysosomal-assoziierte Membranprotein 1 (LAMP1, NCBI-Gene ID: 3916)

(76) der Lymphocytenantigen 6 Complex, locus E (LY6E, NCBI-Gene ID: 4061)

(77) das Protein NOTCH3 (NCBI-Gene ID: 4854)

(78) die Proteintyrosinkinase 7 (PTK7, NCBI-Gene ID: 5754)

(79) das Nectin Zelladhäsionsmolekül 4 (PVRL4, NECTIN4, NCBI-Gene ID: 81607)

(80) das Transmembranprotein Syndecan 1 (SDC1, NCBI-Gene ID: 6382)

(81) das SLAM Familienmitglied 7 (SLAMF7, NCBI-Gene ID: 57823)

(82) das Transportprotein SLC39A6 (NCBI-Gene ID: 25800)

(83) das SLIT und NTRK artige Familienmitglied 6 (SLITRK6, NCBI-Gene ID: 84189)

(84) der Zelloberflächenrezeptor TACSTD2 (NCBI-Gene ID: 4070)

(85) das Rezeptorprotein TNFRSF8 (NCBI-Gene ID: 943)

(86) das Rezeptorprotein TNFSF13B (NCBI-Gene ID: 10673)

(87) das Glykoprotein TPBG (NCBI-Gene ID: 7162)

(88) der Zelloberflächenrezeptor TROP2 (TACSTD2, NCBI-Gene ID: 4070)

(89) der Galanin-like G Protein-gekoppelte Rezeptor KISS1R (GPR54, NCBI-Gene ID: 84634)

(90) das Transportprotein SLAMF6 (NCBI-Gene ID: 114836)

In einem bevorzugten Gegenstand der Erfindung ist das Krebs-Zielmolekül ausgewählt aus der Gruppe bestehend aus den Krebs-Zielmolekülen (1) - (90), insbesondere TWEAKR, B7H3, EGFR und HER2.

In einem weiteren besonders bevorzugten Gegenstand der Erfindung bindet der Binder an ein extrazelluläres Krebs-Zielmolekül, welches ausgewählt wird aus der Gruppe bestehend aus aus den Krebs-Zielmolekülen (1) - (90), insbesondere TWEAKR, B7H3, EGFR und HER2.

In einem weiteren besonders bevorzugten Gegenstand der Erfindung bindet der Binder spezifisch an ein extrazelluläres Krebs-Zielmolekül, welches ausgewählt wird aus der Gruppe bestehend aus aus den Krebs-Zielmolekülen (1) - (90), insbesondere TWEAKR, B7H3, EGFR und HER2. In einer bevorzugten Ausführungsform wird der Binder nach Bindung an sein extrazelluläres Zielmolekül auf der Zielzelle durch die Bindung von der Zielzelle internalisiert. Dies bewirkt, dass das Binder-Wirkstoffkonjugat, welches ein Immunokonjugat oder ein ADC sein kann, von der Zielzelle aufgenommen wird. Anschliessend wird der Binder vorzugsweise intrazellulär, bevorzugt lysosomal, prozessiert.

In einer Ausführungsform ist der Binder ein Bindeprotein. In einer bevorzugten Ausführungsform ist der Binder ein Antikörper, ein antigen-bindendes Antikörperfragment, ein multispezifischer Antikörper oder ein Antikörpermimetikum.

Bevorzugte Antikörpermimetika sind Affibodies, Adnectins, Anticalins, DARPins, Avimers, oder Nanobodies. Bevorzugte multispezifischer Antikörper sind bispezifische und trispezifische Antikörper.

In einer bevorzugten Ausführungsform ist der Binder ein Antikörper oder ein antigen-bindendes Antikörperfragment, weiter bevorzugt ist ein isolierter Antikörper oder ein isoliertes antigen-bindendes Antikörperfragment.

Bevorzugte antigen-bindende Antikörperfragmente sind Fab, Fab', $F(ab')_2$ und Fv Fragmente, Diabodies, DAbs, lineare Antikörper und scFv. Besonders bevorzugt sind Fab, Diabodies und scFv.

In einer besonders bevorzugten Ausführungsform ist der Binder ein Antikörper. Besonders bevorzugt sind monoklonale Antikörper oder antigen-bindende Antikörperfragmente davon. Weiter besonders bevorzugt sind humane, humanisierte oder chimäre Antikörper oder antigen-bindende Antikörperfragmente davon.

Antikörper oder antigen-bindende Antikörperfragmente, die Krebs-Zielmoleküle binden, können vom Durchschnittsfachmann mit bekannten Verfahren hergestellt werden, wie z.B. chemische Synthese oder rekombinante Expression. Binder für Krebs-Zielmoleküle können kommerziell erworben werden oder können durch den Durchschnittsfachmann mit bekannten Verfahren hergestellt werden, wie z.B. chemische Synthese oder rekombinante Expression. Weitere Verfahren zur Herstellung von Antikörpern oder antigen-bindenden Antikörperfragmenten sind in WO 2007/070538 beschrieben (siehe Seite 22 "Antibodies"). Der Fachmann kennt Verfahren wie sogenannte Phage-Display Bibliotheken (z.B. Morphosys HuCAL Gold) erstellt und zur Auffindung von Antikörpern oder antigen-bindenden Antikörperfragmenten verwendet werden können (siehe WO 2007/070538, Seite 24 ff und AK-Beispiel 1 auf Seite 70, AK-Beispiel 2 auf Seite 72). Weitere Verfahren zur Herstellung von Antikörper, die DNA Bibliotheken aus B-Zellen verwenden, sind zum Beispiel auf Seite 26 (WO 2007/070538) beschrieben. Verfahren zur Humanisierung von Antikörpern sind auf Seite 30-32 von WO2007070538 und im Detail in Queen, et al.,.Pros. Natl. Acad. Sci. USA 86:10029-10033,1989 oder in WO 90/0786 beschrieben. Des Weiteren sind dem Fachmann Verfahren zur rekombinanten Expression von Proteinen im allgemeinen und im speziellen von Antikörpern bekannt (siehe z.B. in Berger and Kimrnel (Guide to Molecular Cloning Techniques, Methods in Enzymology, Vo1. 152, Academic Press, Inc.); Sambrook, et al., (Molecular Cloning: A Laboratory Manual, (Second Edition, Cold Spring Harbor Laboratory Press; Cold Spring Harbor, N.Y.; 1989) Vol. 1-3); Current Protocols in Molecular Biolony, (F. M. Ausabel et al. [Eds.], Current Protocols, Green Publishing Associates, Inc. / John Wiley & Sons, Inc.); Harlow et al., (Monoclonal Antibodies: A Laboratory Manual, Cold Spring Harbor Laboratory Press (19881, Paul [Ed.]); Fundamental Immunology, (Lippincott Williams & Wilkins (1998)); and Harlow, et al., (Using Antibodies: A Laboratory Manual, Cold Spring Harbor Laboratory Press (1998)). Der Fachmann kennt die entsprechenden Vektoren, Promotoren und Signalpeptide die zur Expression eines Proteins/Antikörpers notwendig sind. Gebräuchliche Verfahren sind auch in WO 2007/070538 auf den Seiten 41 - 45 beschrieben. Verfahren zur Herstellung eines IgG1-Antikörpers sind z.B. in WO 2007/070538 in Beispiel 6 auf Seite 74 ff beschrieben. Verfahren, mit denen die Internalisierung eines Antikörpers nach Bindung an sein Antigen bestimmt werden kann, sind dem Fachmann bekannt und sind z.B. in WO 2007/070538 auf Seite 80 beschrieben.

Der Fachmann kann die in WO 2007/070538 beschriebenen Verfahren, die zur Herstellung von Carboanhydrase IX (Mn)-Antikörpern verwendet wurden, anlog zur Herstellung für Antikörper mit anderer Zielmolekülspezifität verwenden.

## Bakterielle Expression

Dem Fachmann ist bekannt, auf welche Weise Antikörper, antigenbindenden Fragmente von diesen, oder Varianten von diesen mit Hilfe bakterieller Expression hergestellt werden können.

Geeignete Expressionsvektoren zur bakteriellen Expression gewünschter Proteine werden durch Insertion einer DNA Sequenz, welche das gewünschte Protein kodiert, im funktionellen Leserahmen zusammen mit geeigneten Translationsinitiations- und Translationsterminationssignalen und mit einem funktionellen Promotor konstruiert. Der Vektor umfasst ein oder mehrere phänotypisch selektierbare Marker und einen Replikationsursprung, um die Erhaltung des Vektors und, falls erwünscht, die Amplifikation desselben innerhalb des Wirtes zu ermöglichen. Geeignete prokaryotische Wirte zur Transformation umfassen, sind aber nicht limitiert auf, *E. coli, Bacillus subtilis, Salmonella typhimurium* und verschiedene Spezies aus dem Genus *Pseudomonas, Streptomyces,* und *Staphylococcus.* Bakterielle Vektoren können zum Beispiel basieren auf Bakteriophagen, Plasmiden, oder Phagemiden. Diese Vektoren können selektierbare Marker und einen bakteriellen Replikationsursprung enthalten, welche aus kommerziell erhältlichen Plasmiden abgeleitet sind. Viele kommerziell erhältlichen Plasmide enthalten typischerweise Elemente des gut bekannten Klonierungsvektors pBR322 (ATCC 37017). In bakteriellen Systemen können eine Anzahl von vorteilhaften Expressionsvektoren auf Basis der beabsichtigten Verwendung des zu exprimierenden Proteins ausgewählt werden.

Nach Transformation eines geeigneten Wirtsstammes und Wachstum des Wirtsstammes zu einer angemessenen Zelldichte wird der ausgewählte Promotor durch geeignete Mittel (z. B. Temperaturveränderung oder chemische Induktion) de-reprimiert / induziert, und die Zellen werden für eine zusätzliche Periode kultiviert. Die Zellen werden üblicherweise durch Zentrifugation geerntet, falls nötig auf physikalische Weise oder mit chemischen Mitteln aufgeschlossen, und der resultierende Rohextrakt wird zur weiteren Reinigung zurückgehalten.

Daher ist eine weitere Ausführungsform der vorliegenden Erfindung ein Expressionsvektor, welcher eine Nukleinsäure umfasst, welche einen neuen Antikörper der vorliegenden Erfindung kodiert.

Antikörper der vorliegenden Erfindung oder antigenbindende Fragmente von diesen beinhalten natürlich gereinigte Produkte, Produkte, die aus chemischen Synthesen stammen, und Produkte, die durch rekombinante Technologien in prokaryotischen Wirten, wie zum Beispiel *E. coli, Bacillus subtilis, Salmonella typhimurium* und verschiedene Spezies aus dem Genus *Pseudomonas, Streptomyces,* und *Staphylococcus,* bevorzugt *E. coli,* produziert werden.

## Säugerzellexpression

Dem Fachmann ist bekannt, auf welche Weise Antikörper, antigenbindenden Fragmente von diesen, oder Varianten von diesen mit Hilfe von Säugerzellexpression hergestellt werden können.

Bevorzugte regulatorische Sequenzen zur Expression in Säugerzellwirten umfassen virale Elemente, die zu einer hohen Expression in Säugerzellen führen, wie Promotoren und/oder Expressionsverstärker, welche vom Cytomegalovirus (CMV) (wie dem CMV Promotor/Enhancer), Simian Virus 40 (SV40) (wie dem SV40 Promotor/Enhancer), vom Adenovirus, (z.B. der Adenovirus major late promoter (AdMLP)) und vom Polyoma abgeleitet sind. Die Expression der Antikörper kann konstitutiv oder reguliert erfolgen (z.B. induziert durch Zugabe oder Entfernen von Kleinmolekül Induktoren wie Tetracyclin in Kombination mit dem Tet-System).

Zur weiteren Beschreibung viraler regulatorischer Elemente und Sequenzen von diesen sei verwiesen auf z.B. U.S. 5,168,062 von Stinski, U.S. 4,510,245 von Bell et al. und U.S. 4,968,615 von Schaffner et al.. Die rekombinanten Expressionsvektoren können ebenfalls einen Replikationsursprung und selektierbare Marker beinhalten (siehe z.B. U.S. 4,399,216, 4,634,665 und U.S. 5,179,017). Geeignete selektierbare Marker umfassen Gene, die Resistenz gegenüber Substanzen wie G418, Puromycin, Hygromycin, Blasticidin, Zeocin/Bleomycin, oder Methotrexate verleihen, oder selektierbare Marker, welche zur Auxotrophie einer Wirtszelle führen, wie Glutamine Synthetase (Bebbington et al., Biotechnology (N Y). 1992 Feb;10(2):169-75), wenn der Vektor in die Zelle eingebracht wurde.

Zum Beispiel vermittelt das Dihydrofolate-Reductase (DHFR) Gen Resistenz gegenüber Methotrexate, das neo Gen vermittelt Resistenz gegenüber G418, das bsd Gen aus *Aspergillus terreus* vermittelt Resistenz gegenüber Blasticidin, Puromycin N-acetyltransferase vermittelt Resistenz gegenüber Puromycin, das Sh ble Genprodukt vermittelt Resistenz gegenüber Zeocin, und Resistenz gegenüber Hygromycin wird vermittelt durch das *E. coli* Hygromycin-Resistenzgen (hyg or hph). Selektierbare Marker wie DHFR oder Glutamine-Synthetase sind auch hilfreich für Amplifikationstechniken in Verbindung mit MTX und MSX.

Die Transfektion eines Expressionsvektors in eine Wirtszelle kann mit Hilfe von Standardtechniken ausgeführt werden, unter anderem mit Elektroporation, Nucleofection, Calcium-Phosphate-Präzipitation, Lipofection, Polykation-basierter Transfektion wie Polyethlylenimine (PEI)-basierter Transfektion und DEAE-Dextran Transfection.

Geeignete Säugerwirtszellen für die Expression von Antikörpern, antigenbindenden Fragmenten von diesen, oder Varianten von diesen umfassen Chinese Hamster Ovary (CHO Zellen) wie CHO-K1, CHO-S, CHO-K1SV [inbegriffen DHFR-CHO Zellen, beschrieben in Urlaub and Chasin, (1980) Proc. Natl. Acad. Sci. USA 77:4216-4220 und Urlaub et al., Cell. 1983 Jun;33(2):405-12, verwendet mit einem DHFR selektierbaren Marker, wie beschrieben in R. J. Kaufman and P. A. Sharp (1982) Mol. Biol. 159:601-621, sowie andere Knockout-Zellen, wie ausgeführt in Fan et al., Biotechnol Bioeng. 2012 Apr;109(4):1007-15), NS0 myeloma Zellen, COS Zellen, HEK293 Zellen, HKB11 Zellen, BHK21 Zellen, CAP Zellen, EB66 Zellen, und SP2 Zellen.

Die Expression von Antikörpern, antigenbindenden Fragmenten von diesen, oder Varianten von diesen kann auch transient oder semi-stabil in Expressionssystemen erfolgen, wie HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 oder CAP-T Zellen (beispielsweise wie Durocher et al., Nucleic Acids Res. 2002 Jan 15;30(2):E9)

In einigen Ausführungsformen ist der Expressionsvektor in jener Weise konstruiert, dass das zu exprimierende Protein in das Zellkulturmedium, in welchem die Wirtszellen wachsen, sekretiert wird. Die Antikörper, die antigenbindenden Fragmente von diesen, oder die Varianten von diesen können aus dem Zellkulturmedium mit Hilfe dem Fachmann bekannten Proteinreinigungsmethoden gewonnen werden.

## Reinigung

Die Antikörper, die antigenbindenden Fragmente von diesen, oder die Varianten von diesen können aus rekombinanten Zellkulturen mit Hilfe gut bekannter Methoden gewonnen und gereinigt werden, umfassend beispielsweise Ammoniumsulfat- oder Ethanol- Präzipitation, Säureextraktion, Protein A Chromatographie, Protein G Chromatographie, Anion- oder Kationenaustauschchromatographie, Phospho-Cellulose Chromatographie, hydrophobe Interaktionschromatographie (HIC), Affinitätschromatographie, Hydroxylapatite Chromatographie and Lectin Chromatographie. High Performance Flüssigchromatographie ("HPLC") kann ebenfalls zur Reinigung angewendet werden. Siehe, beispielsweise, Colligan, Current Protocols in Immunology, or Current Protocols in Protein Science, John Wiley & Sons, NY, N.Y., (1997-2001), e.g., Chapters 1, 4, 6, 8, 9, 10.

Antikörper der vorliegenden Erfindung oder antigenbindenden Fragmente von diesen, oder die Varianten von diesen umfassen natürlich gereinigte Produkte, Produkte aus chemischen Syntheseverfahren und Produkte, welche mit Hilfe rekombinanter Techniken in prokaryotischen oder eukaryotischen Wirtszellen hergestellt werden. Eukaryotische Wirte umfassen beispielsweise Hefezellen, höhere Pflanzenzellen, Insektenzellen und Säugerzellen. Abhängig von der für die rekombinanten Expression gewählten Wirtszelle kann das exprimierte Protein glykosyliert oder nicht-glykosyliert vorliegen.

In einer bevorzugen Ausführungsform wird der Antikörper gereinigt (1) zu mehr als 95 Gew.-% gemessen beispielsweise mit der Lowry-Methode, mit UV-Vis Spektroskopie oder mit der SDS-Kapillargelelektrophorese (zum Beispiel mit einem Caliper LabChip GXII, GX 90 oder Biorad Bioanalyzer Gerät), und in mehr bevorzugten Ausführungsformen mehr als 99 Gew.-%, (2) zu einem Grade geeignet zur Bestimmung von mindestens 15 Resten der N-terminalen oder internen Aminosäuresequenz, oder (3) zur Homogenität bestimmt durch SDS-PAGE unter reduzierenden oder nicht-reduzierenden Bedingungen mit Hilfe von Coomassie-Blau oder bevorzugt SilberFärbung.

Gewöhnlich wird ein isolierter Antikörper mit Hilfe wenigstens eines Proteinreinigungsschrittes gewonnen.

Das antigenbindende Fragment gemäß einer der vorhergehenden Ausführungsformen oder ein antigenbindendes Fragment eines Antikörpers gemäß einer der vorhergehenden Ausführungsformen, das ein scFv-, Fab-, Fab'-Fragment oder ein F(ab')2-Fragment ist.

Der Antikörper oder das antigenbindende Fragment gemäß gemäß einer der vorhergehenden Ausführungsformen, der ein monoklonaler Antikörper oder ein antigenbindendes Fragment davon ist.

Der Antikörper oder das antigenbindende Fragment gemäß einem der vorhergehenden Ansprüche, der ein humaner, humanisierter oder chimärer Antikörper oder ein antigenbindendes Fragment ist.

## anti-TWEAKR-Antikörper

Erfindungsgemäß können anti-TWEAKR Antikörper verwendet werden.

Der Begriff "anti-TWEAKR Antikörper" oder "ein Antikörper, der an TWEAKR bindet" bezieht sich auf einen Antikörper, der das Krebs Zielmolekül TWEAKR (NCBI - Referenzsequenz: NP_057723.1; SEQ ID NO: 164) spezifisch bindet, vorzugsweise mit einer für eine diagnostische und/oder therapeutische Anwendung genügenden Affinität. In bestimmten Ausführungsformen bindet der Antikörper TWEAKR mit einer Dissotiationskonstante ($K_D$) von $\leq 1\mu M$, $\leq 100$ nM, $\leq 10$ nM, $\leq 1$ nM, $\leq 0.1$ nM, $\leq 0.01$ nM, oder $\leq 0.001$ nM.

Beispiele für Antikörper, die an TWEAKR binden, sind zum Beispiel in WO2009/020933(A2), WO2009/140177 (A2), WO 2014/198817 (A1) and WO 2015/189143 (A1) offenbart. Diese Antikörper und antigenbindende Fragmente können im Rahmen dieser Erfindung verwendet werden.

ITEM-4 ist ein anti-TWEAKR-Antikörper, der von Nakayama et al. beschrieben wurde (Nakayama, et al., 2003, Biochem Biophy Res Comm, 306:819-825). Humanisierte Varianten dieses Antikörpers basierend auf CDR-Umsetzung ("CDR grafting") werden von Zhou et al. (Zhou et al., 2013, J Invest Dermatol. 133(4):1052-62) sowie in WO 2009/020933 beschrieben. ). Humanisierte Varianten des ITEM-4 sind TPP-7006, TPP-7007, TPP-10334, TPP-10335, TPP-10336 und TPP-10337 Diese Antikörper und antigenbindende Fragmente können im Rahmen dieser Erfindung verwendet werden.

Bevorzugt sind im Rahmen dieser Erfindung die anti-TWEAKR Antikörper TPP-2090, TPP-2658, TPP-5442, TPP-8825, TPP-7006, TPP-7007, TPP-10334, TPP-10335, TPP-10336 und TPP-10337. Mehr bevorzugt sind die anti-TWEAKR Antikörper TPP-7006, TPP-7007, TPP-10334, TPP-10335, TPP-10336 und TPP-10337. Besonders bevorzugt sind die anti-TWEAKR Antikörper TPP-7006, TPP-7007, TPP-10336 und TPP-10337.

## anti-B7H3 Antikörper

Erfindungsgemäß können anti-B7H3 Antikörper verwendet werden.

Der Begriff "anti-B7H3 Antikörper" oder "ein Antikörper, der an B7H3 bindet" bezieht sich auf einen Antikörper, der das Krebs Zielmolekül B7H3 (NCBI -Referenzsequenz: NP_001019907.1; SEQ ID NO: 165) spezifisch bindet, vorzugsweise mit einer für eine diagnostische und/oder therapeutische Anwendung genügenden Affinität. In bestimmten Ausführungsformen bindet der Antikörper B7H3 mit einer Dissotiationskonstante ($K_D$) von $\leq 1\mu M$, $\leq 100$ nM, $\leq 10$ nM, $\leq 1$ nM, $\leq 0.1$ nM, $\leq 0.01$ nM, oder $\leq 0.001$ nM.

Beispiele für Antikörper und antigen-bindene Fragmente die an B7H3 binden, sind dem Fachmann bekannt und sind z.B in WO201109400, EP1773884 und WO2014061277 beschrieben. EP2121008 beschreibt den anti-B7H3 Antikörper 8H9 sowie seine CDR Sequenzen.

Diese Antikörper und antigenbindende Fragmente können im Rahmen dieser Erfindung verwendet werden.

Eine bevorzugte Ausführungsform der ant-B7H3 Antikörper wurden durch Screening einer Antikörper-Phagen-Display-Bibliothek auf rekombinant B7H3 aus der Maus (Maus CD276; Gene ID: 102657) und humanes B7H3 (human CD276; Gene ID: 80381) exprimierenden Zellen erhalten. Die gewonnenen Antikörper wurden in das humane IgG1 Format überführt. Der anti-B7H3 Antikörper TPP-8382 ist ein bevorzugtes Beispiel.

Bevorzugt sind im Rahmen dieser Erfindung die anti-B7H3 Antikörper TPP-8382 und TPP-8567.

## anti-HER2-Antikörper:

Erfindungsgemäß können anti-HER2 Antikörper verwendet werden.

Der Begriff "anti- HER2Antikörper" oder "ein Antikörper, der an HER2 bindet" bezieht sich auf einen Antikörper, der das Krebs Zielmolekül HER2 (NCBI -Referenzsequenz: NP_004439.2; SEQ ID NO: 166) spezifisch bindet, vorzugsweise mit einer für eine diagnostische und/oder therapeutische Anwendung genügenden Affinität. In bestimmten Ausführungsformen bindet der Antikörper HER2 mit einer Dissotiationskonstante ($K_D$) von ≤ 1μM, ≤ 100 nM, ≤ 10 nM, ≤ 1 nM, ≤ 0.1 nM, ≤ 0.01 nM, oder ≤ 0.001 nM.

Ein Beispiel für einen Antikörper, der das Krebs-Zielmoleküle HER2 bindet, ist Trastuzumab (Genentech). Trastuzumab ist ein humanisierter Antikörper, der zur unter anderem Behandlung von Brustkrebs eingesetzt wird. In einer besonders bevorzugten Ausführungsform ist der anti-Her2 Antikörper TPP-1015 (analog Trastuzumab).

Weitere Beispiele für Antikörper, die an HER2 binden, sind neben Trastuzumab (INN 7637, CAS NR: RN: 180288-69-1) und Pertuzumab (Cas NR: 380610-27-5), auch Antikörper, wie offenbart in WO 2009/123894-A2, WO 200/8140603-A2, oder in WO 2011/044368-A2. Beispiel für ein anti-HER2 Konjugat ist Trastuzumab-Emtansine (INN-Nr. 9295). Diese Antikörper und antigenbindende Fragmente können im Rahmen dieser Erfindung verwendet werden.

Besonders bevorzugt ist im Rahmen dieser Erfindung der anti-HER2Antikörper Trastuzumab und TPP-1015.

**anti-EGFR-Antikörper**

Erfindungsgemäß können anti-EGFR Antikörper verwendet werden.

Der Begriff "anti-EGFR Antikörper" oder "ein Antikörper, der an EGFR bindet" bezieht sich auf einen Antikörper, der das Krebs Zielmolekül EGFR (NCBI -Referenzsequenz: NP_005219.2; SEQ ID NO: 167) spezifisch bindet, vorzugsweise mit einer für eine diagnostische und/oder therapeutische Anwendung genügenden Affinität. In bestimmten Ausführungsformen bindet der Antikörper EGFR mit einer Dissotiationskonstante ($K_D$) von ≤ 1μM, ≤ 100 nM, ≤ 10 nM, ≤ 1 nM, ≤ 0.1 nM, ≤ 0.01 nM, oder ≤ 0.001 nM.

In einer bevorzugten Ausführungsform werden die anti-EGFR Antikörper ausgewählt aus der Gruppe bestehend aus TPP-981 (Cetuximab), Panitumumab, Nimotuzumab. In einer besonders bevorzugten Ausführungsform ist der anti-EGFR Antikörper TPP-981 (Cetuximab).

Weitere Ausführungsformen für EGFR-Antikörper sind:

- Zalutumumab / 2F8 / HuMax-EGFR, Firma Genmab A/S (WO 02/100348, WO 2004/056847, INN-Nummer 8605)
- Necitumumab / 11F8, ImClone / IMC-11F8, Firma ImClone Systems Inc [Eli Lilly & Co] (WO 2005/090407 (EP 01735348-A1, US 2007/0264253-A1, US 7,598,350, WO 2005/090407-A1), INN- Nummer 9083)
- Matuzumab / anti-EGFR MAb, Merck KGaA / anti-EGFR MAb, Takeda / EMD 72000 / EMD-6200 / EMD-72000 und EMD-55900 / MAb 425 / monoclonal antibody 425, Firma Merck KGaA / Takeda (WO 92/15683, INN-Nummer 8103 (Matuzumab))
- RG-7160 / GA-201 / GA201 / R-7160 / R7160 / RG7160 / RO-4858696 / RO-5083945 / RO4858696 / RO5083945, Firma Glycart Biotechnology AG (Roche Holding AG) (WO 2010/112413-A1, WO 2010/115554)
- GT-MAB 5.2-GEX / CetuGEX, Firma Glycotope GmbH (WO 2008/028686-A2 (EP 01900750-A1, EP 01911766-A1, EP 02073842-A2, US 2010/0028947-A1)
- ISU-101, Firma Isu Abxis Inc (ISU Chemical Co Ltd) / Scancell (WO 2008/004834-A1)
- ABT-806 / mAb-806 / ch-806 / anti-EGFR monoclonal antibody 806, Firma Ludwig Institute for Cancer Research / Abbott / Life Science Pharmaceuticals (WO 02/092771, WO 2005/081854 und WO 2009/023265)
- SYM-004 (consists of two chimeric IgG1 antibodies (992 and 1024)), Firma Symphogen A/S (WO 2010/022736-A2)
- MR1-1 /MR1-1KDEL, Firma IVAX Corp (Teva Pharmaceutical Industries Ltd) (Duke University), (Patent: WO2001/062931-A2)
- Antikörper gegen die Deletionsmutante, EGFRvIII, Firma Amgen/Abgenix (WO 2005/010151, US 7,628,986)
- SC-100, Firma Scancell Ltd (WO 01/088138-A1)
- MDX-447 / EMD 82633 / BAB-447 / H 447 / MAb, EGFR, Medarex/Merck KgaA, Firma Bristol-Myers Squibb (US) / Merck KGaA (DE) / Takeda (JP), (WO 91/05871, WO 92/15683)
- anti-EGFR-Mab, Firma Xencor (WO 2005/056606)

- DXL-1218 / anti-EGFR monoclonal antibody (cancer), InNexus, Firma InNexus Biotechnology Inc, Pharmaprojects PH048638

## anti-Carboanhvdrase IX Antikörper

Beispiele für Antikörper, die das Krebs-Zielmoleküle Carboanhydrase IX binden, sind in WO 2007/070538-A2 (z.B. Anspüche 1 - 16) beschrieben.

## anti-CD123 Antikörper

Der Begriff "anti- CD123 Antikörper" oder "ein Antikörper, der an CD123 bindet" bezieht sich auf einen Antikörper, der das Krebs Zielmolekül CD123 (NCBI -Referenzsequenz: NP_002174.1; Swiss-Prot: P26951) spezifisch bindet, vorzugsweise mit einer für eine diagnostische und/oder therapeutische Anwendung genügenden Affinität. In bestimmten Ausführungsformen bindet der Antikörper CD123 mit einer Dissotiationskonstante ($K_D$) von ≤ 1μM, ≤ 100 nM, ≤ 10 nM, ≤ 1 nM, ≤ 0.1 nM, ≤ 0.01 nM, oder ≤ 0.001 nM.

Die Generierung und Eigenschaften des monoklonalen Antikörpers 7G3, welcher die N-terminale Domäne von IL-3Rα, CD123, bindet, werden von Sun et al. beschrieben (Sun et al., 1996, Blood 87(1):83-92). US Patent Nummer 6,177,078 (Lopez) bezieht sich auf den anti-CD123-Antikörper 7G3. Eine chimäre Variante dieses Antikörpers (CSL360) ist in WO 2009/070844 sowie eine humanisierte Version (CSL362) in WO 2012/021934 beschrieben. Die Sequenz des 7G3 Antikörpers ist in EP2426148 offenbart worden. Diese Sequenz stellt den Ausgangspunkt für humanisierten Antikörper dar, die durch CDR-Umsetzung ("CDR grafting") erhalten werden.

Einen Antikörper, der besonders gut nach Zelloberflächenantigenbindung internalisiert, ist der anti-CD123 Antikörper 12F1, der von Kuo et al. offenbart wurde (Kuo et a., 2009, Bioconjug Chem. 20(10):1975-82). Der Antikörper 12F1 bindet mit einer höheren Affinität an CD123 als der Antikörper 7G3 und internalisiert nach Zelloberflächenantigenbindung deutlich schneller als 7G3. Bispezifische scFv Immunofusionsproteine basierend auf 12F1 werden in WO 2013/173820 offenbart. Antikörper.

Humanisierte Varianten der murinen 7G3 und 12F1 Antikörpern werden basierend auf CDR-Umsetzung ("CDR grafting") in germline Sequenzen und anschließender Optimierung generiert

## anti-CXCR5 Antikörper

Der Begriff "anti-CXCR5 Antikörper" oder "ein Antikörper, der an CXCR5 bindet" bezieht sich auf einen Antikörper, der das Krebs Zielmolekül CXCR5 (NCBI -Referenzsequenz: NP_001707.1) spezifisch bindet, vorzugsweise mit einer für eine diagnostische und/oder therapeutische Anwendung genügenden Affinität. In bestimmten Ausführungsformen bindet der Antikörper CXCR5 mit einer Dissotiationskonstante ($K_D$) von ≤ 1μM, ≤ 100 nM, ≤ 10 nM, ≤ 1 nM, ≤ 0.1 nM, ≤ 0.01 nM, oder ≤ 0.001 nM.

Beispiele für Antikörper und antigen-bindene Fragmente die an CXCR5 binden, sind dem Fachmann bekannt und z.B in EP2195023 beschrieben

Die Hybridomzellen für den Rattenantikörpers RF8B2 (ACC2153) wurden von DSMZ bezogen und die Sequenz des Antikörpers nach Standardmethoden identifiziert. Diese Sequenz stellt den Ausgangspunkt der humanisierten Antikörper dar, die durch CDR-Umsetzung ("CDR grafting") erhalten werden

Humanisierte Varianten dieses Antikörpers werden basierend auf CDR-Umsetzung ("CDR grafting") in germline Sequenzen generiert.

## anti-C4.4a Antikörper:

Beispiele für C4.4a Antikörper und antigen-bindende Fragmente sind in WO 2012/143499 A2 beschrieben. Die Sequenzen der Antikörper sind in Tabelle 1 der WO 2012/143499 A2 angegeben, wobei jede Zeile die jeweiligen CDR Aminosäuresequenzen der variablen leichten Kette bzw. der variablen Schweren Kette des in Spalte 1 aufgeführten Antikörpers wiedergibt.

## anti-CD20-Antikörper:

Ein Beispiel für einen Antikörper, der das Krebs-Zielmoleküle CD20 bindet, ist Rituximab (Genentech). Rituximab (CAS-Nummer: 174722-31-7) ist ein chimärer Antikörper, der zur Behandlung von Non-Hodgkin-Lymphom verwendet wird. Diese Antikörper und antigenbindende Fragmente davon können im Rahmen dieser Erfindung verwendet werden.

**anti-CD52-Antikörper:**

Ein Beispiel für einen Antikörper, der das Krebs-Zielmoleküle CD52 bindet, ist Alemtuzumab (Genzyme). Alemtuzumab (CAS-Nummer: 216503-57-0) ist ein humanisierter Antikörper, der zur Behandlung von chronischer lymphatischer Leukämie eingesetzt wird. Diese Antikörper und antigenbindende Fragmente davon können im Rahmen dieser Erfindung verwendet werden.

**anti-Mesothelin-Antikörper:**

Beispiele für anti-Mesothelin -Antikörper sind z.B. WO2009/068204 beschrieben. Alle WO2009/068204 offenbarten Antikörper und antigenbindende Fragmente können im Rahmen der hierin offenbarten Erfindung verwendet werden können. Besonders bevorzugt ist der in WO2009/068204 offenbarte Antikörper MF-T.

**anti-CD30-Antikörper**

Beispiele für Antikörper, die das Krebs-Zielmolekül CD30 binden und zur Behandlung von Krebs z.B. Hodgkin-Lymphoma verwendet werden können, sind Brentuximab, Iratumumab und Antikörper, wie in WO 2008/092117, WO 2008/036688 oder WO 2006/089232 offenbart. Beispiele für ein anti- CD30 Konjugat ist Brentuximab Vedotin (INN-Nr. 9144). Diese Antikörper und antigenbindende Fragmente davon können im Rahmen dieser Erfindung verwendet werden.

**anti-CD22-Antikörper**

Beispiele für Antikörper, die das Krebs-Zielmolekül CD22 binden und zur Behandlung von Krebs z.B. Lymphoma verwendet werden können, sind Inotuzumab oder Epratuzumab. Beispiele für anti- CD22 Konjugate sind Inotuzumab Ozagamycin (INN-Nr. 8574), oder anti-CD22-MMAE und anti-CD22-MC-MMAE (CAS RN: 139504-50-0 bzw. 474645-27-7).

Diese Antikörper und antigenbindende Fragmente davon können im Rahmen dieser Erfindung verwendet werden.

**anti-CD33-Antikörper**

Beispiele für Antikörper, die das Krebs-Zielmolekül CD33 binden und zur Behandlung von Krebs z.B. Leukämie verwendet werden können, sind Gemtuzumab oder Lintuzumab (INN 7580). Ein Beispiel für ein anti-CD33 Konjugat ist Gemtuzumab-Ozagamycin. Diese Antikörper und antigenbindende Fragmente können im Rahmen dieser Erfindung verwendet werden.

**anti-N MB-Antikörper**

Ein Beispiel für einen Antikörper, der das Krebs-Zielmolekül NMB bindet und zur Behandlung von Krebs z.B. Melanom oder Brustkrebs verwendet werden kann, ist Glembatumumab (INN 9199). Ein Beispiel für ein anti-NMB Konjugat ist Glembatumumab Vedotin (CAS RN: 474645-27-7). Diese Antikörper und antigenbindende Fragmente davon können im Rahmen dieser Erfindung verwendet werden.

**anti-CD56-Antikörper**

Ein Beispiel für einen Antikörper, der das Krebs-Zielmolekül CD56 bindet und zur Behandlung von Krebs z.B. Multiples Myelom, Kleinzelliges Lungenkarzinom, MCC oder Ovarialkarzinom verwendet werden kann, ist Lorvotuzumab. Ein Beispiel für ein anti-CD56 Konjugat ist Lorvotuzumab Mertansine (CAS RN: 139504-50-0). Diese Antikörper und antigenbindende Fragmente können im Rahmen dieser Erfindung verwendet werden.

**anti-CD70-Antikörper**

Beispiele für Antikörper, die das Krebs-Zielmolekül CD70 binden und zur Behandlung von Krebs z.B. Non-Hodgkin-Lymphom oder Nierenzellkrebs verwendet werden können, sind in WO 2007/038637-A2 oder WO 2008/070593-A2 offenbart. Ein Beispiel für ein anti-CD70 Konjugat ist SGN-75 (CD70 MMAF). Diese Antikörper und antigenbindende Fragmente können im Rahmen dieser Erfindung verwendet werden.

**anti-CD74-Antikörper**

Ein Beispiel für einen Antikörper, der das Krebs-Zielmolekül CD74 bindet und zur Behandlung von Krebs z.B. Multiplem Myelom verwendet werden kann, ist Milatuzumab. Ein Beispiel für ein anti-CD74 Konjugat ist Milatuzumab-Doxorubicin (CAS RN: 23214-92-8). Diese Antikörper und antigenbindende Fragmente können im Rahmen dieser Erfindung verwendet werden.

**anti-CD19-Antikörper**

Ein Beispiel für einen Antikörper, der das Krebs-Zielmolekül CD19 bindet und zur Behandlung von Krebs z.B. Non-Hodgkin-Lymphom verwendet werden kann, ist in WO 2008/031056-A2 offenbart. Weitere Antikörper und Beispiele für ein anti-CD19 Konjugat (SAR3419) sind in WO 2008/047242-A2 offenbart. Diese Antikörper und antigenbindende Fragmente davon können im Rahmen dieser Erfindung verwendet werden.

**anti-Mucin-Antikörper**

Beispiele für Antikörper, die das Krebs-Zielmolekül Mucin-1 binden und zur Behandlung von Krebs z.B. Non-Hodkin-Lymphom verwendet werden können, sind Clivatuzumab oder die in WO 2003/106495-A2, WO 2008/028686-A2 offenbarten Antikörper. Beispiele für anti-Mucin Konjugate sind in WO 2005/009369-A2 offenbart. Diese Antikörper und antigenbindende Fragmente davon können im Rahmen dieser Erfindung verwendet werden.

**anti-CD138-Antikörper**

Beispiele für Antikörper, die das Krebs-Zielmolekül CD138 binden und Konjugate davon, die zur Behandlung von Krebs z.B. Multiples Myelom verwendet werden können, sind WO 2009/080829-A1, WO 2009/080830-A1 offenbart. Diese Antikörper und antigenbindende Fragmente davon können im Rahmen dieser Erfindung verwendet werden.

**anti-Integrin-alphaV-Antikörper**

Beispiele für Antikörper, die das Krebs-Zielmolekül Integrin alphaV binden und zur Behandlung von Krebs z.B. Melanoma, Sarcoma oder Carcinoma verwendet werden können, sind Intetumumab (Cas RN: 725735-28-4), Abciximab (Cas-RN: 143653-53-6), Etaracizumab (Cas-RN: 892553-42-3) oder die in US 7,465,449, EP 719859-A1, WO 2002/012501-A1 oder WO2006/062779-A2 offenbarten Antikörper. Beispiele für anti-Integrin AlphaV Konjugate sind Intetumumab-DM4 und weitere in WO 2007/024536-A2 offenbarte ADCs. Diese Antikörper und antigenbindende Fragmente davon können im Rahmen dieser Erfindung verwendet werden.

**anti-TDGF1-Antikörper**

Beispiele für Antikörper, die das Krebs-Zielmolekül TDGF1 binden und zur Behandlung von Krebs verwendet werden können, sind die in WO 02/077033-A1, US 7,318,924, WO 2003/083041-A2 und WO 2002/088170-A2 offenbarten Antikörper. Beispiele für anti-TDGF1 Konjugate sind in WO 2002/088170-A2 offenbart. Diese Antikörper und antigenbindende Fragmente davon können im Rahmen dieser Erfindung verwendet werden.

**anti-PSMA-Antikörper**

Beispiele für Antikörper, die das Krebs-Zielmolekül PSMA binden und zur Behandlung von Krebs z.B. Prostatakarzinom verwendet werden können, sind die in WO 97/35616-A1, WO 99/47554-A1, WO 01/009192-A1 und WO2003/034903 offenbarten Antikörper. Beispiele für anti-PSMA Konjugate sind in WO 2009/026274-A1 und WO 2007/002222 offenbart. Diese Antikörper und antigenbindende Fragmente können im Rahmen dieser Erfindung verwendet werden.

**anti-EPHA2-Antikörper**

Beispiele für Antikörper, die das Krebs-Zielmolekül EPHA2 binden, zur Herstellung eines Konjugats und zur Behandlung von Krebs verwendet werden können, sind in WO 2004/091375-A2 offenbart. Diese Antikörper und antigenbindende Fragmente können im Rahmen dieser Erfindung verwendet werden.

## anti-SLC44A4-Antikörper

Beispiele für Antikörper, die das Krebs-Zielmolekül SLC44A4 binden, zur Herstellung eines Konjugats und zur Behandlung von Krebs, z.B. Pankreas- oder Prostatakarzinom verwendet werden können, sind in WO2009/033094-A2 und US2009/0175796-A1 offenbart. Diese Antikörper und antigenbindende Fragmente davon können im Rahmen dieser Erfindung verwendet werden.

## anti-HLA-DOB-Antikörper

Ein Beispiel für einen Antikörper, der das Krebs-Zielmolekül HLA-DOB bindet, ist der Antikörper Lym-1 (Cas-RN: 301344-99-0), der zur Behandlung von Krebs, z.B. Non-Hodgkin-Lymphom verwendet werden kann. Beispiele für anti-HLA-DOB Konjugate sind z.B. in WO 2005/081711-A2 offenbart. Diese Antikörper und antigenbindende Fragmente davon können im Rahmen dieser Erfindung verwendet werden.

## anti-VTCN1-Antikörper

Beispiele für Antikörper, die das Krebs-Zielmolekül VTCN1 binden, zur Herstellung eines Konjugats und zur Behandlung von Krebs, z.B. Ovarialkarzinom, Pankreas-, Lungen-, oder Brustkrebs verwendet werden können, sind in WO 2006/074418-A2 offenbart. Diese Antikörper und antigenbindende Fragmente davon können im Rahmen dieser Erfindung verwendet werden.

## anti-FGFR2-Antikörper

Beispiele für anti-FGFR2 Antikörper und antigen-bindende Fragmente sind in WO2013076186 beschrieben. Die Sequenzen der Antikörper sind in Tabelle 9 und Tabelle 10 der WO2013076186 angegeben. Bevorzugt sind Antikörper, Antigen-bindende Fragmente und Varianten der Antikörper, die sich von den als M048-D01 und M047-D08 bezeichneten Antikörpern ableiten.

## Bevorzugte Antikörper und Antigen-bindende Antikörperfragmente für erfindungsgemäße Binder-Wirkstoff-Konjugate

In dieser Anmeldung wird bei den Binder-Wirkstoff-Konjugaten auf die folgenden bevorzugten Antikörper Bezug genommen, wie in der nachstehenden Tabelle gezeigt: TPP-2090, TPP-2658, TPP-5442, TPP-8825, TPP-7006, TPP-7007, TPP-10334, TPP-10335, TPP-10336, TPP-10337, TPP-1015, TPP-7510, TPP-7511, TPP-8382 und TPP-8567.

**Tabelle:** Proteinsequenzen der Antikörper:

| Antikörper TPP-XXX | Antigen | SEQ ID NO: VH | SEQ ID NO: H-CDR1 | SEQ ID NO: H-CDR2 | SEQ ID NO: H-CDR3 | SEQ ID NO: VL | SEQ ID NO: L-CDR1 | SEQ ID NO: L-CDR2 | SEQ ID NO: L-CDR3 | SEQ ID NO: IgG Schwere Kette | SEQ ID NO: IgG Leichte Kette |
|---|---|---|---|---|---|---|---|---|---|---|---|
| TPP-981 | EGFR | 1 | 2 | 3 | 4 | 5 | 6 | 7 | 8 | 9 | 10 |
| TPP-1015 | HER2 | 11 | 12 | 13 | 14 | 15 | 16 | 17 | 18 | 19 | 20 |

| Antikörper TPP-XXX | Antigen | SEQ ID NO: VH | SEQ ID NO: H-CDR1 | SEQ ID NO: H-CDR2 | SEQ ID NO: H-CDR3 | SEQ ID NO: VL | SEQ ID NO: L-CDR1 | SEQ ID NO: L-CDR2 | SEQ ID NO: L-CDR3 | SEQ ID NO: IgG Schwere Kette | SEQ ID NO: IgG Leichte Kette |
|---|---|---|---|---|---|---|---|---|---|---|---|
| TPP-2090 | TWEAKR | 21 | 22 | 23 | 24 | 25 | 26 | 27 | 28 | 29 | 30 |
| TPP-2658 | TWEAKR | 31 | 32 | 33 | 34 | 35 | 36 | 37 | 38 | 39 | 40 |
| TPP-5442 | TWEAKR | 41 | 42 | 43 | 44 | 45 | 46 | 47 | 48 | 49 | 50 |
| TPP-7006 | TWEAKR | 51 | 52 | 53 | 54 | 55 | 56 | 57 | 58 | 59 | 60 |
| TPP-7007 | TWEAKR | 61 | 62 | 63 | 64 | 65 | 66 | 67 | 68 | 69 | 70 |
| TPP-7510 | HER2 | 71 | 72 | 73 | 74 | 75 | 76 | 77 | 78 | 79 | 80 |
| TPP-7511 | HER2 | 81 | 82 | 83 | 84 | 85 | 86 | 87 | 88 | 89 | 90 |
| TPP-8382 | B7H3 | 91 | 92 | 93 | 94 | 95 | 96 | 97 | 98 | 99 | 100 |
| TPP-8567 | B7H3 | 101 | 102 | 103 | 104 | 105 | 106 | 107 | 108 | 109 | 110 |
| TPP-8825 | TWEAKR | 111 | 112 | 113 | 114 | 115 | 116 | 117 | 118 | 119 | 120 |
| TPP-10334 | TWEAKR | 121 | 122 | 123 | 124 | 125 | 126 | 127 | 128 | 129 | 130 |
| TPP-10335 | TWEAKR | 131 | 132 | 133 | 134 | 135 | 136 | 137 | 138 | 139 | 140 |
| TPP-10336 | TWEAKR | 141 | 142 | 143 | 144 | 145 | 146 | 147 | 148 | 149 | 150 |
| TPP-10337 | TWEAKR | 151 | 152 | 153 | 154 | 155 | 156 | 157 | 158 | 159 | 160 |

TPP-2090, TPP-2658, TPP-5442, TPP-8825, TPP-7006, TPP-7007, TPP-10334, TPP-10335, TPP-10336, TPP-10337, TPP-1015, TPP-7510, TPP-7511, TPP-8382 und TPP-8567 sind Antikörper umfassend eine oder mehrere der in obiger Tabelle angegebenen CDR-Sequenzen (H-CDR1, H-CDR2, H-CDR3, L-CDR1, L-CDR2, L-CDR3) der variable Region der schwere Kette (VH) oder der variable Region der leichte Kette (VL). Vorzugsweise umfassen die Antikörper die angegebene variable Region der schwere Kette (VH) und/oder die variable Region der leichte Kette (VL). Vorzugsweise umfassen die Antikörper die angegebene Region der schweren Kette (IgG Schwere Kette) und/oder die angegebene Region der leichten Kette (IgG Leichte Kette).

TPP-981 ist ein anti-EGFR Antikörper umfassend eine variable Region der schwere Kette (VH) umfassend die variable CDR1-Sequenz der schweren Kette (H-CDR1), wie dargestellt durch SEQ ID NO: 2, die variable CDR2-Sequenz der schweren Kette (H-CDR2), wie dargestellt durch SEQ ID NO: 3 und die variable CDR3-Sequenz der schweren Kette (H-CDR3), wie dargestellt durch SEQ ID NO: 4, sowie eine variable Region der leichte Kette (VL) umfassend die variable CDR1-Sequenz der leichten Kette (L-CDR1), wie dargestellt durch SEQ ID NO: 6, die variable CDR2-Sequenz der

leichten Kette (L-CDR2), wie dargestellt durch SEQ ID NO: 7 und die variable CDR3-Sequenz der leichten Kette (L-CDR3), wie dargestellt durch SEQ ID NO: 8.

TPP-1015 ist ein anti-HER2 Antikörper umfassend eine variable Region der schwere Kette (VH) umfassend die variable CDR1-Sequenz der schweren Kette (H-CDR1), wie dargestellt durch SEQ ID NO: 12, die variable CDR2-Sequenz der schweren Kette (H-CDR2), wie dargestellt durch SEQ ID NO: 13 und die variable CDR3-Sequenz der schweren Kette (H-CDR3), wie dargestellt durch SEQ ID NO: 14, sowie eine variable Region der leichte Kette (VL) umfassend die variable CDR1-Sequenz der leichten Kette (L-CDR1), wie dargestellt durch SEQ ID NO: 16, die variable CDR2-Sequenz der leichten Kette (L-CDR2), wie dargestellt durch SEQ ID NO: 17 und die variable CDR3-Sequenz der leichten Kette (L-CDR3), wie dargestellt durch SEQ ID NO: 18.

TPP-2090 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 22, die variable CDR2-Sequenz der schweren Kette (H-CDR2), wie dargestellt durch SEQ ID NO: 23 und die variable CDR3-Sequenz der schweren Kette (H-CDR3), wie dargestellt durch SEQ ID NO: 24, sowie eine variable Region der leichte Kette (VL) umfassend die variable CDR1-Sequenz der leichten Kette (L-CDR1), wie dargestellt durch SEQ ID NO: 26, die variable CDR2-Sequenz der leichten Kette (L-CDR2), wie dargestellt durch SEQ ID NO: 27 und die variable CDR3-Sequenz der leichten Kette (L-CDR3), wie dargestellt durch SEQ ID NO: 28.

TPP-2658 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 32, die variable CDR2-Sequenz der schweren Kette (H-CDR2), wie dargestellt durch SEQ ID NO: 33 und die variable CDR3-Sequenz der schweren Kette (H-CDR3), wie dargestellt durch SEQ ID NO: 34, sowie eine variable Region der leichte Kette (VL) umfassend die variable CDR1-Sequenz der leichten Kette (L-CDR1), wie dargestellt durch SEQ ID NO: 36, die variable CDR2-Sequenz der leichten Kette (L-CDR2), wie dargestellt durch SEQ ID NO: 37 und die variable CDR3-Sequenz der leichten Kette (L-CDR3), wie dargestellt durch SEQ ID NO: 38.

TPP-5442 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 42, die variable CDR2-Sequenz der schweren Kette (H-CDR2), wie dargestellt durch SEQ ID NO: 43 und die variable CDR3-Sequenz der schweren Kette (H-CDR3), wie dargestellt durch SEQ ID NO: 44, sowie eine variable Region der leichte Kette (VL) umfassend die variable CDR1-Sequenz der leichten Kette (L-CDR1), wie dargestellt durch SEQ ID NO: 46, die variable CDR2-Sequenz der leichten Kette (L-CDR2), wie dargestellt durch SEQ ID NO: 47 und die variable CDR3-Sequenz der leichten Kette (L-CDR3), wie dargestellt durch SEQ ID NO: 48.

TPP-7006 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 52, die variable CDR2-Sequenz der schweren Kette (H-CDR2), wie dargestellt durch SEQ ID NO: 53 und die variable CDR3-Sequenz der schweren Kette (H-CDR3), wie dargestellt durch SEQ ID NO: 54, sowie eine variable Region der leichte Kette (VL) umfassend die variable CDR1-Sequenz der leichten Kette (L-CDR1), wie dargestellt durch SEQ ID NO: 56, die variable CDR2-Sequenz der leichten Kette (L-CDR2), wie dargestellt durch SEQ ID NO: 57 und die variable CDR3-Sequenz der leichten Kette (L-CDR3), wie dargestellt durch SEQ ID NO: 58.

TPP-7007 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 62, die variable CDR2-Sequenz der schweren Kette (H-CDR2), wie dargestellt durch SEQ ID NO: 63 und die variable CDR3-Sequenz der schweren Kette (H-CDR3), wie dargestellt durch SEQ ID NO: 64, sowie eine variable Region der leichte Kette (VL) umfassend die variable CDR1-Sequenz der leichten Kette (L-CDR1), wie dargestellt durch SEQ ID NO: 66, die variable CDR2-Sequenz der leichten Kette (L-CDR2), wie dargestellt durch SEQ ID NO: 67 und die variable CDR3-Sequenz der leichten Kette (L-CDR3), wie dargestellt durch SEQ ID NO: 68.

TPP-7510 ist ein anti-HER2 Antikörper umfassend eine variable Region der schwere Kette (VH) umfassend die variable CDR1-Sequenz der schweren Kette (H-CDR1), wie dargestellt durch SEQ ID NO: 72, die variable CDR2-Sequenz der schweren Kette (H-CDR2), wie dargestellt durch SEQ ID NO: 73 und die variable CDR3-Sequenz der schweren Kette (H-CDR3), wie dargestellt durch SEQ ID NO: 74, sowie eine variable Region der leichte Kette (VL) umfassend die variable CDR1-Sequenz der leichten Kette (L-CDR1), wie dargestellt durch SEQ ID NO: 76, die variable CDR2-Sequenz der leichten Kette (L-CDR2), wie dargestellt durch SEQ ID NO: 77 und die variable CDR3-Sequenz der leichten Kette (L-CDR3), wie dargestellt durch SEQ ID NO: 78.

TPP-7511 ist ein anti-HER2 Antikörper umfassend eine variable Region der schwere Kette (VH) umfassend die variable CDR1-Sequenz der schweren Kette (H-CDR1), wie dargestellt durch SEQ ID NO: 82, die variable CDR2-Sequenz der schweren Kette (H-CDR2), wie dargestellt durch SEQ ID NO: 83 und die variable CDR3-Sequenz der schweren Kette (H-CDR3), wie dargestellt durch SEQ ID NO: 84, sowie eine variable Region der leichte Kette (VL) umfassend die variable CDR1-Sequenz der leichten Kette (L-CDR1), wie dargestellt durch SEQ ID NO: 86, die variable CDR2-Sequenz der leichten Kette (L-CDR2), wie dargestellt durch SEQ ID NO: 87 und die variable CDR3-Sequenz der leichten Kette (L-CDR3), wie dargestellt durch SEQ ID NO: 88.

TPP-8382 ist ein anti-B7H3 Antikörper umfassend eine variable Region der schwere Kette (VH) umfassend die variable CDR1-Sequenz der schweren Kette (H-CDR1), wie dargestellt durch SEQ ID NO: 92, die variable CDR2-Sequenz der schweren Kette (H-CDR2), wie dargestellt durch SEQ ID NO: 93 und die variable CDR3-Sequenz der schweren Kette (H-CDR3), wie dargestellt durch SEQ ID NO: 94, sowie eine variable Region der leichte Kette (VL) umfassend die variable CDR1-Sequenz der leichten Kette (L-CDR1), wie dargestellt durch SEQ ID NO: 96, die variable CDR2-Sequenz der leichten Kette (L-CDR2), wie dargestellt durch SEQ ID NO: 97 und die variable CDR3-Sequenz der leichten Kette (L-CDR3), wie dargestellt durch SEQ ID NO: 98.

TPP-8567 ist ein anti-B7H3 Antikörper umfassend eine variable Region der schwere Kette (VH) umfassend die variable CDR1-Sequenz der schweren Kette (H-CDR1), wie dargestellt durch SEQ ID NO: 102, die variable CDR2-Sequenz der schweren Kette (H-CDR2), wie dargestellt durch SEQ ID NO: 103 und die variable CDR3-Sequenz der schweren Kette (H-CDR3), wie dargestellt durch SEQ ID NO: 104, sowie eine variable Region der leichte Kette (VL) umfassend die variable CDR1-Sequenz der leichten Kette (L-CDR1), wie dargestellt durch SEQ ID NO: 106, die variable CDR2-Sequenz der leichten Kette (L-CDR2), wie dargestellt durch SEQ ID NO: 107 und die variable CDR3-Sequenz der leichten Kette (L-CDR3), wie dargestellt durch SEQ ID NO: 108.

TPP-8825 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 112, die variable CDR2-Sequenz der schweren Kette (H-CDR2), wie dargestellt durch SEQ ID NO: 113 und die variable CDR3-Sequenz der schweren Kette (H-CDR3), wie dargestellt durch SEQ ID NO: 114, sowie eine variable Region der leichte Kette (VL) umfassend die variable CDR1-Sequenz der leichten Kette (L-CDR1), wie dargestellt durch SEQ ID NO: 116, die variable CDR2-Sequenz der leichten Kette (L-CDR2), wie dargestellt durch SEQ ID NO: 117 und die variable CDR3-Sequenz der leichten Kette (L-CDR3), wie dargestellt durch SEQ ID NO: 118.

TPP-10334 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 122, die variable CDR2-Sequenz der schweren Kette (H-CDR2), wie dargestellt durch SEQ ID NO: 123 und die variable CDR3-Sequenz der schweren Kette (H-CDR3), wie dargestellt durch SEQ ID NO: 124, sowie eine variable Region der leichte Kette (VL) umfassend die variable CDR1-Sequenz der leichten Kette (L-CDR1), wie dargestellt durch SEQ ID NO: 126, die variable CDR2-Sequenz der leichten Kette (L-CDR2), wie dargestellt durch SEQ ID NO: 127 und die variable CDR3-Sequenz der leichten Kette (L-CDR3), wie dargestellt durch SEQ ID NO: 128.

TPP-10335 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 132, die variable CDR2-Sequenz der schweren Kette (H-CDR2), wie dargestellt durch SEQ ID NO: 133 und die variable CDR3-Sequenz der schweren Kette (H-CDR3), wie dargestellt durch SEQ ID NO: 134, sowie eine variable Region der leichte Kette (VL) umfassend die variable CDR1-Sequenz der leichten Kette (L-CDR1), wie dargestellt durch SEQ ID NO: 136, die variable CDR2-Sequenz der leichten Kette (L-CDR2), wie dargestellt durch SEQ ID NO: 137 und die variable CDR3-Sequenz der leichten Kette (L-CDR3), wie dargestellt durch SEQ ID NO: 138.

TPP-10336 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 142, die variable CDR2-Sequenz der schweren Kette (H-CDR2), wie dargestellt durch SEQ ID NO: 143 und die variable CDR3-Sequenz der schweren Kette (H-CDR3), wie dargestellt durch SEQ ID NO: 144, sowie eine variable Region der leichte Kette (VL) umfassend die variable CDR1-Sequenz der leichten Kette (L-CDR1), wie dargestellt durch SEQ ID NO: 146, die variable CDR2-Sequenz der leichten Kette (L-CDR2), wie dargestellt durch SEQ ID NO: 147 und die variable CDR3-Sequenz der leichten Kette (L-CDR3), wie dargestellt durch SEQ ID NO: 148.

TPP-10337 ist ein anti-TWEAKR Antikörper umfassend eine variable Region der schwere Kette (VH) umfassend die variable CDR1-Sequenz der schweren Kette (H-CDR1), wie dargestellt durch SEQ ID NO: 152, die variable CDR2-Sequenz der schweren Kette (H-CDR2), wie dargestellt durch SEQ ID NO: 153 und die variable CDR3-Sequenz der schweren Kette (H-CDR3), wie dargestellt durch SEQ ID NO: 154, sowie eine variable Region der leichte Kette (VL) umfassend die variable CDR1-Sequenz der leichten Kette (L-CDR1), wie dargestellt durch SEQ ID NO: 156, die variable CDR2-Sequenz der leichten Kette (L-CDR2), wie dargestellt durch SEQ ID NO: 157 und die variable CDR3-Sequenz der leichten Kette (L-CDR3), wie dargestellt durch SEQ ID NO: 158.

TPP-981 ist ein anti-EGFR Antikörper umfassend vorzugsweise eine variable Region der schweren Kette (VH) entsprechend SEQ ID NO: 1 sowie eine variable Region der leichten Kette (VL) entsprechend SEQ ID NO: 5.

TPP-1015 ist ein anti-HER2 Antikörper umfassend vorzugsweise eine variable Region der schweren Kette (VH) entsprechend SEQ ID NO: 11 sowie eine variable Region der leichten Kette (VL) entsprechend SEQ ID NO: 15.

TPP-2090 ist ein anti-TWEAKR Antikörper umfassend vorzugsweise eine variable Region der schweren Kette (VH) entsprechend SEQ ID NO: 21 sowie eine variable Region der leichten Kette (VL) entsprechend SEQ ID NO: 25.

TPP-2658 ist ein anti-TWEAKR Antikörper umfassend vorzugsweise eine variable Region der schweren Kette (VH) entsprechend SEQ ID NO: 31 sowie eine variable Region der leichten Kette (VL) entsprechend SEQ ID NO: 35.

TPP-5442 ist ein anti-TWEAKR Antikörper umfassend vorzugsweise eine variable Region der schweren Kette

(VH) entsprechend SEQ ID NO: 41 sowie eine variable Region der leichten Kette (VL) entsprechend SEQ ID NO: 45.

TPP-7006 ist ein anti-TWEAKR Antikörper umfassend vorzugsweise eine variable Region der schweren Kette (VH) entsprechend SEQ ID NO: 51 sowie eine variable Region der leichten Kette (VL) entsprechend SEQ ID NO: 55.

TPP-7007 ist ein anti-TWEAKR Antikörper umfassend vorzugsweise eine variable Region der schweren Kette (VH) entsprechend SEQ ID NO: 61 sowie eine variable Region der leichten Kette (VL) entsprechend SEQ ID NO: 65.

TPP-7510 ist ein anti-HER2 Antikörper umfassend vorzugsweise eine variable Region der schweren Kette (VH) entsprechend SEQ ID NO: 71 sowie eine variable Region der leichten Kette (VL) entsprechend SEQ ID NO: 75.

TPP-7511 ist ein anti-HER2 Antikörper umfassend vorzugsweise eine variable Region der schweren Kette (VH) entsprechend SEQ ID NO: 81 sowie eine variable Region der leichten Kette (VL) entsprechend SEQ ID NO: 85.

TPP-8382 ist ein anti-B7H3 Antikörper umfassend vorzugsweise eine variable Region der schweren Kette (VH) entsprechend SEQ ID NO: 91 sowie eine variable Region der leichten Kette (VL) entsprechend SEQ ID NO: 95.

TPP-8567 ist ein anti-B7H3 Antikörper umfassend vorzugsweise eine variable Region der schweren Kette (VH) entsprechend SEQ ID NO: 101 sowie eine variable Region der leichten Kette (VL) entsprechend SEQ ID NO: 105.

TPP-8825 ist ein anti-TWEAKR Antikörper umfassend vorzugsweise eine variable Region der schweren Kette (VH) entsprechend SEQ ID NO: 111 sowie eine variable Region der leichten Kette (VL) entsprechend SEQ ID NO: 115.

TPP-10334 ist ein anti-TWEAKR Antikörper umfassend vorzugsweise eine variable Region der schweren Kette (VH) entsprechend SEQ ID NO: 121 sowie eine variable Region der leichten Kette (VL) entsprechend SEQ ID NO: 125.

TPP-10335 ist ein anti-TWEAKR Antikörper umfassend vorzugsweise eine variable Region der schweren Kette (VH) entsprechend SEQ ID NO: 131 sowie eine variable Region der leichten Kette (VL) entsprechend SEQ ID NO: 135.

TPP-10336 ist ein anti-TWEAKR Antikörper umfassend vorzugsweise eine variable Region der schweren Kette (VH) entsprechend SEQ ID NO: 141 sowie eine variable Region der leichten Kette (VL) entsprechend SEQ ID NO: 145.

TPP-10337 ist ein anti-TWEAKR Antikörper umfassend vorzugsweise eine variable Region der schweren Kette (VH) entsprechend SEQ ID NO: 151 sowie eine variable Region der leichten Kette (VL) entsprechend SEQ ID NO: 155.

TPP-981 ist ein anti-EGFR Antikörper umfassend vorzugsweise eine Region der schweren Kette entsprechend SEQ ID NO: 9 sowie eine Region der leichten Kette entsprechend SEQ ID NO: 10.

TPP-1015 ist ein anti-HER2 Antikörper umfassend vorzugsweise eine Region der schweren Kette entsprechend SEQ ID NO: 19 sowie eine Region der leichten Kette entsprechend SEQ ID NO: 20.

TPP-2090 ist ein anti-TWEAKR Antikörper umfassend vorzugsweise eine Region der schweren Kette entsprechend SEQ ID NO: 29 sowie eine Region der leichten Kette entsprechend SEQ ID NO: 30.

TPP-2658 ist ein anti-TWEAKR Antikörper umfassend vorzugsweise eine Region der schweren Kette entsprechend SEQ ID NO: 39 sowie eine Region der leichten Kette entsprechend SEQ ID NO: 40.

TPP-5442 ist ein anti-TWEAKR Antikörper umfassend vorzugsweise eine Region der schweren Kette entsprechend SEQ ID NO: 49 sowie eine Region der leichten Kette entsprechend SEQ ID NO: 50.

TPP-7006 ist ein anti-TWEAKR Antikörper umfassend vorzugsweise eine Region der schweren Kette entsprechend SEQ ID NO: 59 sowie eine Region der leichten Kette entsprechend SEQ ID NO: 60.

TPP-7007 ist ein anti-TWEAKR Antikörper umfassend vorzugsweise eine Region der schweren Kette entsprechend SEQ ID NO: 69 sowie eine Region der leichten Kette entsprechend SEQ ID NO: 70.

TPP-7510 ist ein anti-HER2 Antikörper umfassend vorzugsweise eine Region der schweren Kette entsprechend SEQ ID NO: 79 sowie eine Region der leichten Kette entsprechend SEQ ID NO: 80.

TPP-7511 ist ein anti-HER2 Antikörper umfassend vorzugsweise eine Region der schweren Kette entsprechend SEQ ID NO: 89 sowie eine Region der leichten Kette entsprechend SEQ ID NO: 90.

TPP-8382 ist ein anti-B7H3 Antikörper umfassend vorzugsweise eine Region der schweren Kette entsprechend SEQ ID NO: 99 sowie eine Region der leichten Kette entsprechend SEQ ID NO: 100.

TPP-8567 ist ein anti-B7H3 Antikörper umfassend vorzugsweise eine Region der schweren Kette entsprechend SEQ ID NO: 109 sowie eine Region der leichten Kette entsprechend SEQ ID NO: 110.

TPP-8825 ist ein anti-TWEAKR Antikörper umfassend vorzugsweise eine Region der schweren Kette entsprechend SEQ ID NO: 119 sowie eine Region der leichten Kette entsprechend SEQ ID NO: 120.

TPP-10334 ist ein anti-TWEAKR Antikörper umfassend vorzugsweise eine Region der schweren Kette entsprechend SEQ ID NO: 129 sowie eine Region der leichten Kette entsprechend SEQ ID NO: 130.

TPP-10335 ist ein anti-TWEAKR Antikörper umfassend vorzugsweise eine Region der schweren Kette entsprechend SEQ ID NO: 139 sowie eine Region der leichten Kette entsprechend SEQ ID NO: 140.

TPP-10336 ist ein anti-TWEAKR Antikörper umfassend vorzugsweise eine Region der schweren Kette entsprechend SEQ ID NO: 149 sowie eine Region der leichten Kette entsprechend SEQ ID NO: 150.

TPP-10337 ist ein anti-TWEAKR Antikörper umfassend vorzugsweise eine Region der schweren Kette entsprechend SEQ ID NO: 159 sowie eine Region der leichten Kette entsprechend SEQ ID NO: 160.

**Linker für den Binder LIG ($L_b$ und $L_c$)**

Aus der Literatur sind verschiedene Möglichkeiten der kovalenten Kopplung (Konjugation) von organischen Molekülen an Peptide oder Proteine wie Antikörper bekannt (siehe z.B. K. Lang and J. W. Chin. Chem. Rev. 2014, 114, 4764-4806, M. Rashidian et al. Bioconjugate Chem. 2013, 24, 1277-1294). Erfindungsgemäß bevorzugt ist die Konjugation des organischen Rests an einen Antikörper über ein oder mehrere Schwefelatome von Cystein-Resten des Antikörpers, die entweder als freie Thiole bereits vorliegen oder durch Reduktion von Disulfidbrücken generiert werden, und/oder über eine oder mehrere NH-Gruppen von Lysin-Resten des Antikörpers. Es ist jedoch auch möglich, den KSP-Inhibitor bzw. Prodrug an den Antikörper über Tyrosinreste, über Glutaminreste, über Reste unnatürlicher Aminosäuren, über freie Carboxylgruppen oder über Zuckerreste des Antikörpers zu binden.

Es ist erfindungsgemäß auch möglich, die Wirkstoffmoleküle an spezifische Konjugationsstellen des Binders zu konjugieren, wodurch die Produkthomogenität verbessert wird. Die Literatur beschreibt verschiedene Methoden zur konjugationsstellenspezifischen Konjugation (Agarwal et al., Bioconjug. Chem. 26, 176-192 (2015); Cal et al., Angew. Chem. Int. Ed. Engl.53, 10585-10587 (2014); Behrens et al., MAbs 6, 46-53 (2014); Panowski et al., MAbs 6, 34-45 (2014)). Diese Methoden enthalten insbesondere auch enzymatische Konjugationsmethoden, die beispielsweise Transglutaminasen (TGases), Glykosyltransferasen oder das Formylglycin generierende Enzym ((Sochaj et al., Biotechnology Advances 33, 775-784, (2015)) verwenden.

Erfindungsgemäß können konjugationsstellenspezifische Binder-Konjugate des Kinesin-Spindel-Protein-Inhibitors, in welchen die Kinesin-Spindel-Protein-Inhibitoren an Glutamin-Seitenketten der Binder konjugiert sind, bereitgestellt werden.

Wenn der Binder ein Antikörper ist, beinhaltet er ein Akzeptor-Glutamin, bevorzugt in der konstanten Region. Solche Akzeptor-Glutamine können durch Mutation von geeigneten Positionen zu Glutamin eingeführt werden (zum Beispiel die Mutation N297Q der schweren Kette, Kabat EU Nummerierung) oder durch Generierung von deglycosylierten oder aglycosylierten Antikörpern (zum Beispiel durch enzymatische Deglykosylierung durch PNGase F oder durch Mutation N297X der schweren Kette, Kabat EU Nummerierung (X kann hier jede Aminosäure außer N sein)). Im letzteren Fall eines deglykosylierten oder aglykosylierten Antikörpers wird der Glutaminrest Q295 (Kabat EU Nummerierung) der schweren Kette ein Akzeptor-Glutamin. Besonders bevorzugt ist ein Antikörper, der die Mutation N297A oder N297Q (Kabat EU Nummerierung) enthält.

Daher beinhalten alle in dieser Erfindung beschriebenen Antikörper ebenso aglykosylierte Varianten dieser Antikörper, die entweder durch Deglykosylierung durch PNGase F oder durch Mutation von N297 (Kabat EU Nummerierung) (Kabat numbering system of antibodies, see Kabat et al. , Sequences of Proteins of Immulological Interest, 5th Ed. Public Health Service, National Institutes of Health, Bethesda, MD. (1991)) der schweren Kette zu jeder anderen Aminosäure außer N hergestellt werden. Desweiteren enthalten alle hier beschriebenen Antikörper ebenso Varianten der beschriebenen Antikörper, die durch ein Engineering ein oder mehrere Akzeptor-Glutamin-Reste für Transglutaminasekatalysierte Reaktionen enthalten.

Ein Weg für solche konjugationsstellenspezifischen Konjugationen sind literaturbeschriebene Ansätze, die sich mit konjugationsstellenspezifischer Konjugation von Bindern mittels Transglutaminase befassen. Transglutaminasen (TGases), die auch die bakterielle Transglutaminase (BTG) (EC 2.3.2.13) beinhalten, sind eine Familie von Enzymen, die die Bildung einer kovalenten Bindung zwischen der $\gamma$-Carbonyl-AmidGruppe von Glutaminen und der primären Amin-Gruppe von Lysinen katalysieren. Da solche Transglutaminasen auch andere Substrate als Lysin als Amin-Donor akzeptieren, wurden sie verwendet, um Proteine einschließlich Antikörper an geeigneten Akzeptor-Glutaminen zu modifizieren (Jeger et al., Angewandte Chemie Int. Ed. Engl 49, 9995-9997 (2010); Josten et al., J. Immunol. Methods 240, 47-54 (2000); Mindt et al., Bioconjugate Chem. 19, 271-278 (2008); Dennler et al., in Antibody Drug Conjuagtes (Ducry, L., Ed.), pp 205-215, Humana Press. (2013)). Auf der einen Seite wurden Transglutaminasen für die Konjugation von Wirkstoffen an Antikörper verwendet, welche artifizielle Glutamin-Tags enthalten, welche Akzeptorglutamin-Reste sind, welche durch genetisches Engineering in den Antikörper eingefügt wurden ((Strop et al., Chem. Biol. 20, 161-167 (2013)). Auf der anderen Seite wurde beschrieben, dass der konservierte Glutamin-Rest Q295 (Kabat EU Nummerierung) der konstanten Region der schweren Kette von Antikörpern der einzige $\gamma$-Carbonyl-Amid-Donor für die bakterielle Transglutaminase (EC 2.3.2.13) im Rückgrat von aglykosylierten IgG1-Molekülen ist, und somit ein Akzeptor-Glutamin darstellt, während kein Akzeptor-Glutamin im Rückgrat des IgG1 vorhanden ist, wenn der Antikörper an Position N297 (Kabat EU Nummerierung) der schweren Kette glykosyliert ist (Jeger et al., Angewandte Chemie Int. Ed. Engl 49, 9995-9997 (2010)). Zusammenfassend kann die bakterielle Transglutaminase für die Konjugation eines Amin-Donor-Substrats, zum Beispiel eines Wirkstoff-Linker-Konstrukts, an einen Akzeptor-Glutamin-Rest eines Antikörpers verwendet werden. Solche Akzeptor-Glutamine können durch Engineering des Antikörpers durch Mutationen oder durch die Generierung aglykosylierter Antikörper eingeführt werden. Solche aglykosylierten Antikörper können durch Deglykosylierung unter Verwendung von N-glycosidase F (PNGase F) oder durch Mutation von N297 der Glykosylierungsstelle der schweren Kette (Kabat EU Nummerierung) zu jeder anderen Aminosäure außer N eingeführt werden. Die enzymatische Konjugation solcher aglykosylierter Antikörper unter Verwendung von bakterieller Transglutaminase wurde für aglykosylierte

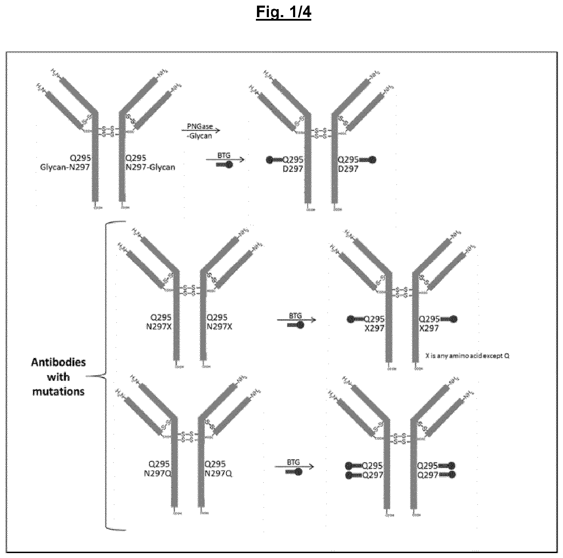

Antikörpervarianten beschrieben, die die Mutationen N297D, N297Q (Jeger et al., Angewandte Chemie Int. Ed. Engl 49, 9995-9997 (2010)) oder N297S (siehe Patentanmeldungen WO2013092998A1 und WO2013092983A2) enthalten. Die enzymatische Konjugation solcher aglykosylierter Antikörperm mittels Transglutaminase liefert generell ADCs mit einer DAR von 2, in welchen beide schweren Ketten spezifisch an Position Q295 (Kabat EU Nummerierung) funktionalisiert sind. Nur die Mutation N297Q der schweren Kette liefert eine zusätzliche Konjugationsstelle pro schwerer Kette. Die Konjugation solcher Varianten führt zu ADCs mit einer DAR von 4, in welchen beide schweren spezifisch an den Positionen Q295 und Q297 funktionalisiert sind. Antikörpervarianten, in welchen die schweren Ketten die Mutationen Q295N und N297Q tragen, weisen pro schwerer Kette nur einen Akzeptor-Glutamin-Rest an Position Q297 (Kabat Nummerierung) auf (Simone Jeger, Site specific conjugation of tumour targeting antibodies using transglutaminase, Dissertation at ETH Zürich (2009)). In der Literatur existieren mehrere Beispiele, die die konjugationsstellenspezifische Konjugation von aglykosylierten Antikörpern unter Verwendung von bakterieller Transglutaminase beschreiben (zum Beispiel Dennler et al., Bioconjugate Chemistry 19, 569-578 (2014); Lhospice et al., Molecular Pharmaceutics 12, 1863-1871 (2015)). Die Strategie der Transglutaminase-katalysierten konjugationsstellenspezifischen Funktionalsisierung aglykosylierter Antikörper ist in Abbildung 1 zusammengefasst.

Zur Kopplung - sowohl konjugationsstellenspezifisch als auch konjugationsstellenunspezifisch - werden sogenannte Linker verwendet. Linker lassen sich in die Gruppe der *in vivo* spaltbaren Linker und in die Gruppe der *in vivo* stabilen Linker unterteilen (siehe L. Ducry und B. Stump, Bioconjugate Chem. 21, 5-13 (2010)). Die *in vivo* spaltbaren Linker weisen eine *in vivo* spaltbare Gruppe auf, wobei wiederum zwischen chemisch *in vivo* spaltbaren und enzymatisch *in vivo* spaltbaren Gruppen unterschieden werden kann. "Chemisch *in vivo* spaltbar" bzw. "enzymatisch *in vivo* spaltbar" bedeutet, dass die Linker bzw. Gruppen im Blutkreislauf stabil sind und erst an bzw. in der Zielzelle durch die dort veränderte chemische bzw. enzymatische Umgebung (niedrigerer pH-Wert; erhöhte Glutathion-Konzentration; Vorliegen lysosomaler Enzyme wie Cathepsin oder Plasmin, oder Glyosidasen wie beispielsweise β-Glucuronidasen) sich spalten, um so den niedermolekularen KSP-Inhibitor oder ein Derivat davon freizusetzen. Die chemisch *in vivo* spaltbaren Gruppen sind insbesondere Disulfid, Hydrazon, Acetal und Aminal; die enzymatisch *in vivo* spaltbaren Gruppen sind insbesondere 2-8-Oligopeptidgruppe, insbesondere eine Dipeptidgruppe oder Glycoside. Peptidspaltstellen sind in Bioconjugate Chem. 2002, 13, 855-869, sowie Bioorganic & Medicinal Chemistry Letters 8 (1998) 3341-3346 *sowie* Bioconjugate Chem. 1998, 9, 618-626 offenbart. Hierzu gehören z.B. Alanin-Alanin-Asparagin, Valin-Alanin, Valin-Lysin, Valin-Citrullin, Alanin-Lysin und Phenylalanin-Lysin (ggf. mit zusätzlicher Amidgruppe).

Um eine effiziente Freisetzung des freien Wirkstoffs zu gewährleisten, können gegebenenfalls auch sogenannte self-immolative Linkerelemente (SIG) zwischen enzymatischer Spaltstelle und Wirkstoff eingebaut werden (Anticancer Agents in Medicinal Chemistry, 2008, 8, 618-637). Die Freisetzung des Wirkstoffs kann dabei nach verschiedenen Mechanismen erfolgen, beispielsweise nach initialer enzymatischer Freisetzung einer nukleophilen Gruppe durch anschließende Elimierung über eine elektronische Kaskade (Bioorg. Med. Chem., 1999, 7, 1597; J. Med. Chem., 2002, 45, 937; Bioorg. Med. Chem., 2002, 10, 71) oder durch Cyklisierung des entsprechenden Linkerelements (Bioorg. Med. Chem., 2003, 11, 2277; Bioorg. Med. Chem., 2007, 15, 4973; Bioorg. Med. Chem. Lett., 2007, 17, 2241) oder durch eine Kombination aus beidem (Angew. Chem. Inter. Ed., 2005, 44, 4378). Beispiele für solche Linkerelemente sind in der Abbildung dargestellt:

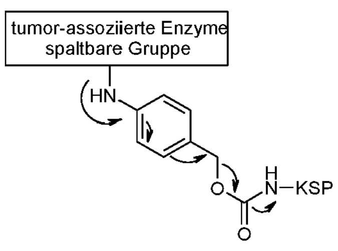


Elimination linker

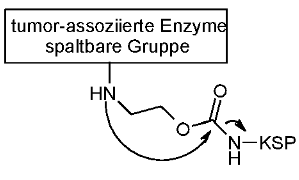


Cyclisation linker

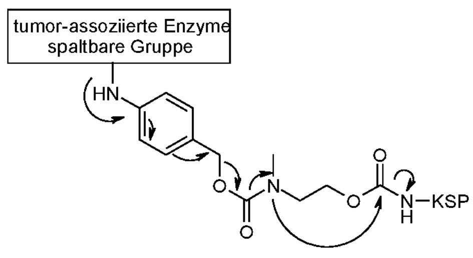


Elongated linker

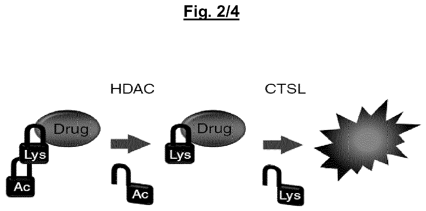

Beispiele für nacheinander geschaltete enzymatische Schritte zur Wirkstofffreisetzung z.B. durch Histone Deacetylase und Cathepsin L sind in Nat. Commun., 2013, 4, 2735 beschrieben und sind in Abbildung 2 veranschaulicht.

Die *in vivo* stabilen Linker zeichnen sich durch eine hohe Stabilität aus (weniger als 5 % Metabolite nach 24 Stunden in Plasma) und weisen die oben genannten chemisch oder enzymatisch *in vivo* spaltbaren Gruppen nicht auf.

Der Linker-$L_b$- bzw. -$L_c$- weist vorzugsweise eine der folgenden Grundstrukturen (i) bis (iv) auf:

(i) $-(C=O)_m-$SG1-L1-L2-

(ii) $-(C=O)_m-$L1-SG-L1-L2-

(iii) $-(C=O)_m-$L1-L2-

(iv) $-(C{=}O)_m$-L1-SG-L2

wobei m 0 oder 1 ist; SG eine (chemisch oder enzymatisch) *in vivo* spaltbare Gruppe (insbesondere Disulfid, Hydrazon, Acetal und Aminal; oder eine mit Legumain, Cathepsin oder Plasmin spaltbare 2-8-Oligopeptidgruppe) ist, SG1 eine Oligopeptidgruppe oder vorzugsweise eine Dipeptidgruppe ist, L1 unabhängig voneinander für *in vivo* stabile organische Gruppen stehen, und L2 für eine Kopplungsgruppe an den Binder oder eine Einfachbindung steht. Die Kopplung erfolgt hierbei vorzugsweise an einen Cysteinrest oder einen Lysinrest des Antikörpers. Alternativ kann die Kopplung an einen Tyrosinrest, Glutaminrest oder an eine unnatürliche Aminosäure des Antikörpers erfolgen. Die unnatürlichen Aminosäuren können beispielsweise Aldehyd- oder Ketogruppen (wie z.B. Formylglycin) oder Azid- oder Alkingruppen enthalten (siehe hierzu Lan & Chin, Cellular Incorporation of Unnatural Amino Acids and Bioorthogonal Labeling of Proteins, Chem.Rev. 2014, 114, 4764-4806).

Erfindungsgemäß bevorzugt ist insbesondere die Linker-Grundstruktur (iii). Die Verabreichung eines erfindungsgemäßen Konjugats mit einer Linker-Grundstruktur (iii) und Kopplung des Linkers an einen Cystein- oder Lysinrest des Antikörpers führt durch Metabolisierung zu Cystein- bzw. Lysinderivaten der folgenden Formeln:

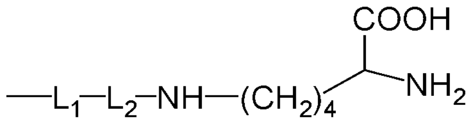
;
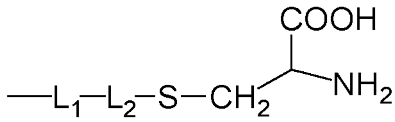

wobei L1 jeweils an den cytotoxischen Wirkstoff wie z.B. den niedermolekularen KSP-Inhibitor gebunden ist, z.B. einer Verbindung der Formel (IIa), (IIb), (IIc), (IId), (V), (VI) oder (VII).

Erfindungsgemäß bevorzugt sind auch die Linker-Grundstrukturen (ii) and (iv), insbesondere bei Anbindung an die Position $R^1$ in einer Verbindung der Formel (IIa), (IIb), (IIc), (IId), oder (V), insbesondere wenn die Gruppe $L_1$ eine der folgenden Strukturen aufweist:

(a) $-NH-(CH_2)_{0-4}-(CHCH_3)_{0-4}-CHY^5-C({=}O)-Y^7$, in der

$Y^5$ für -H oder $-NHY^6$ steht,
$Y^6$ für -H oder $-C({=}O)-CH_3$ steht und
$Y^7$ für eine Einfachbindung oder $-NH-(CH_2)_{0-4}-CHNH_2-C({=}O)-$ steht,

so dass nach der Spaltung die entsprechende Struktur

- $NH-(CH_2)_{0-4}-(CHCH_3)_{0-4}-CHY^5-COOH$ bzw.
- $NH-(CH_2)_{0-4}-(CHCH_3)_{0-4}-CHY^5-C({=}O)-NH-(CH_2)_{0-4}-CHNH_2-COOH$ erhalten wird.

(b) $-CH_2-S_x-(CH_2)_{0-4}-CHY^5-C({=}O)-$, in der

x 0 oder 1 ist,
$Y^5$ für -H oder $-NHY^6$ steht und
$Y^6$ für -H oder $-C({=}O)-CH_3$ steht,

so dass nach der Spaltung die entsprechende Struktur

- $CH_2-S_x-(CH_2)_{0-4}-CHY^5-COOH$

erhalten wird.

Wenn der Linker an eine Cystein-Seitenkette bzw. einen Cysteinrest gebunden ist, leitet sich L2 vorzugsweise von einer mit der Sulfhydryl-Gruppe des Cysteins reaktiven Gruppe ab. Hierzu zählen Haloacetyle, Maleimide, Aziridine, Acryloyle, Arylierende Verbindungen, Vinylsulfone, Pyridyldisulfide, TNB-thiole and Disulfid-reduzierende Mittel. Diese Gruppen reagieren in der Regel elektrophil mit der Sulfhydryl-Bindung unter Bildung einer Sulfid (z.B. Thioether)- oder Disulfid-Brücke. Bevorzugt sind stabile Sulfidbrücken.

L2 weist vorzugsweise folgende Strukturen auf:

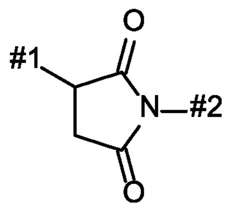 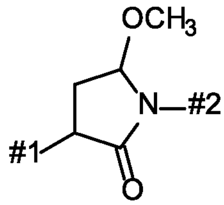 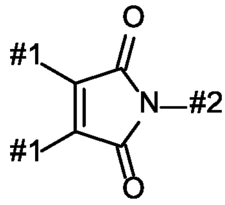 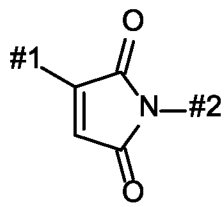

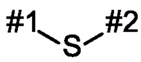 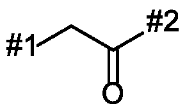 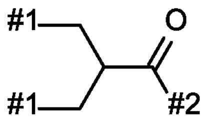

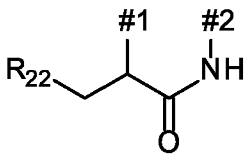 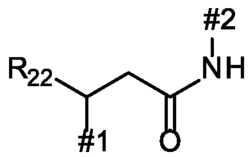 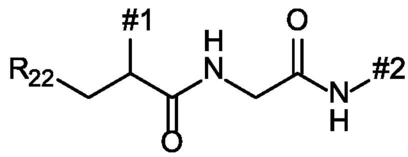

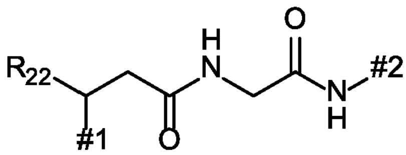

in denen

- $\#^1$ die Verknüpfungsstelle mit dem Schwefelatom des Antikörpers kennzeichnet,
- $\#^2$ die Verknüpfungsstelle mit der Gruppe $L_1$ kennzeichnet, und
- $R_{22}$ für -COOH, -C(=O)-OR, -C(=O)R, -C(=O)-NHR oder $-C(=O)N(R)_2$ steht und
- R für $C_{1-3}$-Alkyl-, $-C(=O)-NH_2$ oder -COOH steht.

Hierbei bevorzugt ist, wenn R für -COOH steht.

Besonders bevorzugt sind die Verbindungen der vorliegenden Erfindung in denen L2 die folgenden Formeln A3 und A4 aufweist:

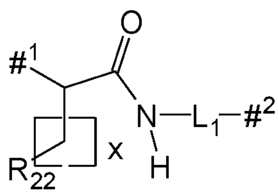

Formel A3,

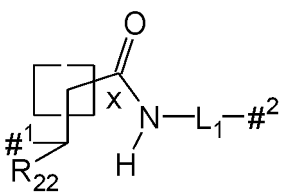

Formel A4,

in denen

- $\#^1$ für die Verknüpfungsstelle mit dem Schwefelatom des Antikörpers steht,
- $\#^2$ für die Verknüpfungsstelle mit dem Wirkstoffmolekül steht,
- x 1 oder 2 ist und
- $R_{22}$ für -COOH, -C(=O)-OR, -C(=O)-R, -C(=O)-$NR_2$, -C(=O)-NHR oder -C(=O)-$NH_2$ steht und
- R für $C_{1-3}$-Alkyl- steht.

Vorzugsweise steht hierbei $R^{22}$ für -COOH und insbesondere steht hierbei $R^{22}$ für -COOH, falls x für 1 steht.

In einem erfindungsgemäßen Konjugat bzw. in einem Gemisch der erfindungsgemäßen Konjugate liegen die Bindungen an einen Cysteinrest des Antikörpers vorzugsweise zu über 80 %, besonders bevorzugt zu über 90 % (jeweils bezogen auf die Gesamtanzahl von Bindungen des Linkers an den Antikörper), besonders bevorzugt in einer der beiden Strukturen der Formel A3 oder A4 vor. Hierbei liegen die Strukturen der Formel A3 oder A4 in der Regel gemeinsam vor, vorzugsweise in einem Verhältnis von 60:40 bis 40:60 bezogen auf die Anzahl der Bindungen an den Antikörper. Die restlichen Bindungen liegen dann in der Struktur

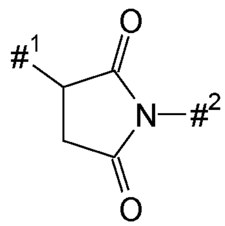

vor, in der

- $\#^1$ für die Verknüpfungsstelle mit dem Schwefelatom des Antikörpers steht und
- $\#^2$ für die Verknüpfungsstelle mit dem Wirkstoffmolekül steht,

Erfindungsgemäß wird L1 vorzugsweise durch die Formel

$$\#^1\text{-}(NR^{10})_n\text{-}(G1)_o\text{-}G2\text{-}\#^2$$

dargestellt, in der

- $\#^1$ für die Verknüpfungsstelle mit dem Schwefelatom des Antikörpers steht und
- $\#^2$ für die Verknüpfungsstelle mit dem Wirkstoffmolekül steht,
- $R^{10}$ für -H, -$NH_2$ oder $C_1$-$C_3$-Alkyl- steht,
- n 0 oder 1 ist,
- o 0 oder 1 ist,

G1 für -NH-C(=O)-, -C(=O)-NH- oder

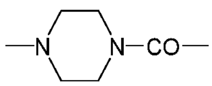

steht und

G2 für eine geradkettige oder verzweigte Kohlenwasserstoffkette mit 1 bis 100 Kohlenstoffatomen steht, die aus Arylengruppen und/oder geradkettigen und/oder verzweigten und/oder cyclischen Alkylengruppen besteht, die ein- oder mehrfach durch eine oder mehrere der Gruppen -O-, -S-, -S(=O)-, -$S(=O)_2$, -NRY-, -$NR^yC$(=O)-, -C(NH)NRY-, -C(=O)-$NR^y$-, -$NR^yNR^y$-, -$S(=O)_2NR^yNR^y$-, -C(=O)-$NR^yNR^y$-, -C(=O)-, -$CR^x$=N-O- und/oder einen 3 bis 10-gliedriger, aromatischen oder nicht aromatischen Heterozyklus mit bis zu 4 Heteroatomen ausgewählt aus N, O und S, -S(=O)- oder -$S(=O)_2$- unterbrochen sein kann und wobei die geradkettige oder verzweigte Kohlenwasserstoffkette zusätzlich mit -NH-C(=O)-$NH_2$, -COOH, -OH, -$NH_2$, Sulfonamid, Sulfon, Sulfoxid, oder Sulfonsäure substituiert sein kann,

Ry für-H, Phenyl-, $C_1$-$C_{10}$-Alkyl-, $C_2$-$C_{10}$-Alkenyl oder $C_2$-$C_{10}$-Alkinyl- steht, die jeweils mit -NH-C(=O)-$NH_2$, -COOH, -OH, -$NH_2$, Sulfonamid, Sulfon, Sulfoxid, oder Sulfonsäure substituiert sein können und

Rx für -H, $C_1$-$C_3$-Alkyl- oder Phenyl- darstellt steht.

Hierbei steht G1 vorzugsweise für

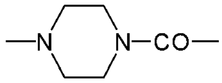

und $R^{10}$ steht vorzugsweie nicht für -$NH_2$, falls G1 für -NH-C(=O)- oder

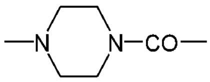

steht.

Bevorzugt steht G2 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen, die ein- oder mehrfach durch eine oder mehrere der Gruppen -O-, -S-, -S(=O)-, -S(=O)$_2$, -NH-, -C(=O)-, -NH-C(=O)-, -C(=O)-NH-, -NMe-, -NHNH-, -S(=O)$_2$-NHNH-, -C(=O)-NHNH- und einen 5 bis 10gliedriger, aromatischen oder nicht aromatischen Heterozyklus mit bis zu 4 Heteroatomen ausgewählt aus N, O und S, oder -S(=O)- unterbrochen sein kann

Besonders bevorzugt steht G2 für

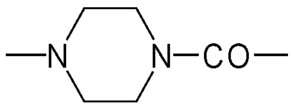

, und wobei die geradkettige oder verzweigte Kohlenwasserstoffkette zusätzlich mit -NH-C(=O)-$NH_2$ substituiert sein kann.

G2 steht ferner vorzugsweise für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen, die einfach oder mehrfach durch eine oder mehrere der Gruppen -O-, -S-, -S(=O)-, -S(=O)$_2$-, -NH-, -C(=O)-, -NH-C(=O)-, -C(=O)-NH-, -NMe-, -NHNH-, -S(=O)$_2$-NHNH-, -C(=O)-NHNH-, -CR$^x$=N-O- und einen 3 bis 10-gliedrigen, aromatischen oder nicht aromatischen Heterozyklus mit bis zu 4 Heteroatomen ausgewählt aus =N-, -O- und -S-, -S(=O)- oder -S(=O)$_2$- unterbrochen sein kann, wobei die geradkettige oder verzweigte Kohlenwasserstoffkette zusätzlich mit -NH-C(=O)-$NH_2$, -COOH, -OH, -$NH_2$, Sulfonamid, Sulfon, Sulfoxid, oder Sulfonsäure substituiert sein kann und

Rx für -H, $C_1$-$C_3$-Alkyl- oder Phenyl- steht.

Hierbei steht G2 vorzugsweise für

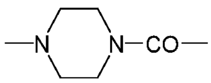

.

Vorzugsweise steht G2 für die unterbrechenden Gruppen der Strukturen

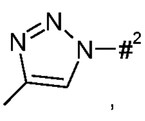 , 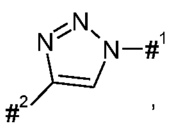 , 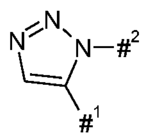 , 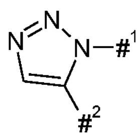 ,

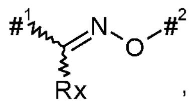 , 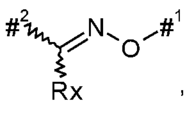 , 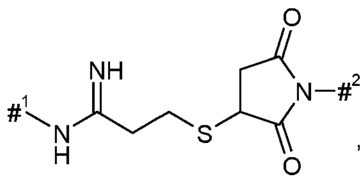 ,

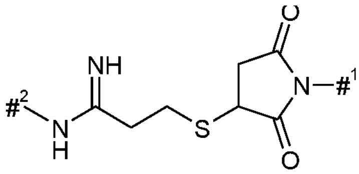

in denen

Rx für -H, $C_1$-$C_3$-Alkyl- oder Phenyl- steht,
#[1] für die Bindung zum KSP-Inhibitor bzw. Prodrug steht und
#[2] für die Bindung zur Kopplungsgruppe zum Antikörper (z.B. L2).

Eine geradkettige oder verzweigte Kohlenwasserstoffkette aus Arylengruppen und/oder geradkettigen und/oder verzweigten und/oder cyclischen Alkylengruppen umfasst in der Regel einen $\alpha,\omega$-divalenten Alkylrest mit der jeweils angegebenen Zahl von Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen).

Die Alkylengruppen in der

Eine verzweigte Kohlenwasserstoffkette bedeutet, dass ein oder mehrere H-Atome der geradkettigen Kohlenwasserstoffkette bzw. der geradkettigen Alkylengruppen durch $C_{1-10}$-Alkylgruppen substituiert sind und so verzweigte Kohlenwasserstoff- bzw. Seitenketten bilden.
Die Kohlenwasserstoffkette kann weiterhin cyclische Alkylengruppen (Cycloalkandiyl) enthalten, z.B. 1,4-Cyclohexandiyl oder 1,3-Cyclopentandiyl. Diese cyclischen Gruppen können ungesättigt sein. Insbesondere können in der Kohlenwasserstoffkette aromatische Gruppen (Arylengruppen) vorliegen, z.B. Phenylen. Auch in den cyclischen Alkylengruppen und den Arylengruppen können wiederum in oder mehrere H-Atome ggf. durch $C_{1-10}$-Alkylgruppen substituiert sein. Es wird so eine Kohlenwasserstoffkette gebildet, die ggf. verzweigt ist. Diese Kohlenwasserstoffkette weist insgesamt 0 bis 100 Kohlenstoffatomen, vorzugsweise 1 bis 50, besonders bevorzugt 2 bis 25 Kohlenstoffatome auf.
Die verzweigten Kohlenwasserstoff- bzw. Seitenketten können mit -NH-C(=O)-$NH_2$, - COOH, -OH, -$NH_2$, Sulfonamid, Sulfon, Sulfoxid, oder Sulfonsäure substituiert sein.
Die Kohlenwasserstoffketten können einfach oder mehrfach durch eine oder mehrere der Gruppen

- O-, -S-, -S(=O)-, -S(=O)$_2$-, -NH-, -C(=O)-, -NH-C(=O)-, -C(=O)-NH-, -NMe-, -NHNH-,
- S(=O)$_2$-NHNH-, -C(=O)-NHNH- und einen 5 bis 10gliedriger, aromatischen oder nicht aromatischen Heterozyklus mit bis zu 4 Heteroatomen ausgewählt aus =N-, -O- und -S-,
- S(=O)- oder -S(=O)$_2$- unterbrochen sein.

Bevorzugt ist hier eine Gruppe

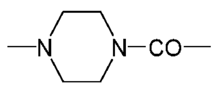 .

Weitere unterbrechende Gruppen in G2 sind vorzugsweise

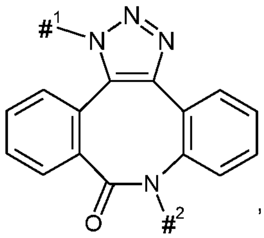 , 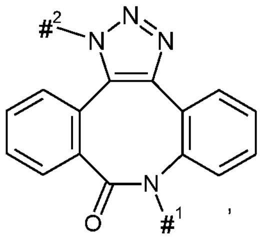 , 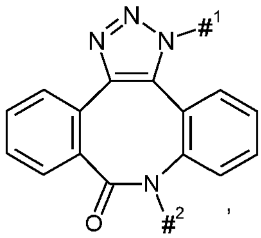 , 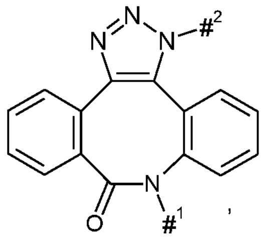 ,

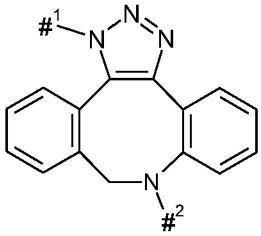 , 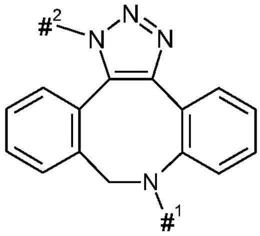 , 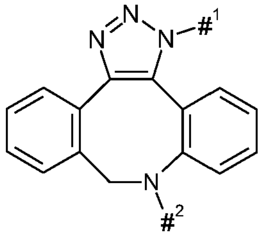 , 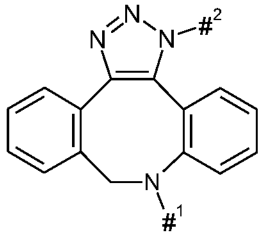 ,

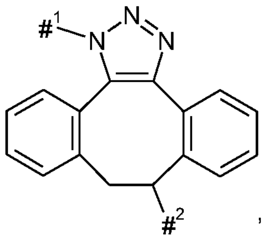 , 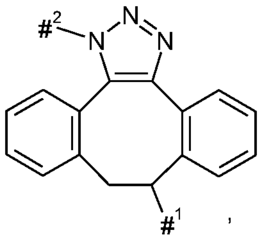 , 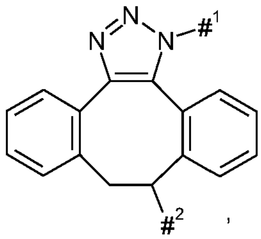 , 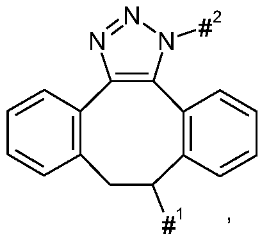 ,

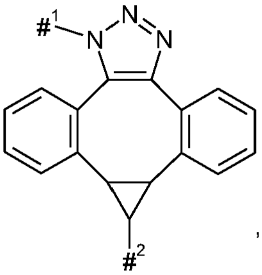 , 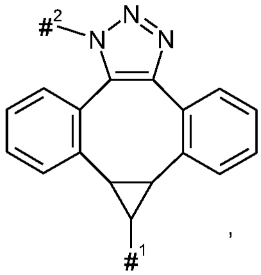 , 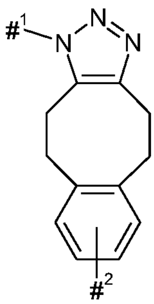 , 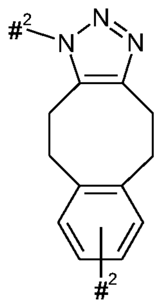

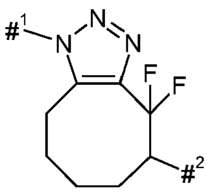 , 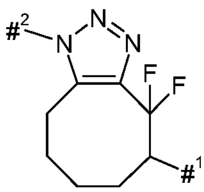 , 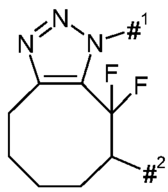 , 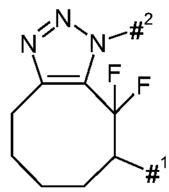

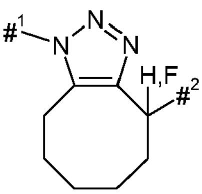 , 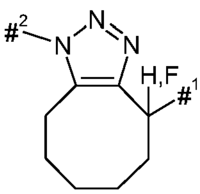 , 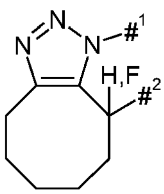 , 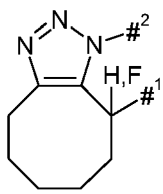

Vorzugsweise entspricht der Linker L der folgenden Formel

§-(C(=O))m-L1-L2-§§,

in der

| | |
|---|---|
| m | 0 oder 1 ist, |
| § | für die Bindung an das Wirkstoffmolekül bzw. Prodrug steht, |
| §§ | für die Bindung an das Binderpeptid oder -protein steht, und |
| L1 und L2 | die oben genannten Bedeutungen aufweisen. |

Besonders bevorzugt und mit Bezug auf die die obigen Definitionen entspricht L1 der folgenden vereinfachten Formel

$-NR^{11}B-$,

in der

| | |
|---|---|
| $R^{11}$ | für -H oder $-NH_2$ steht, |
| B | für die Gruppe $-[(CH_2)_x-(X^4)_y]_w-(CH_2)_z-$ steht, |
| w | 0 bis 20 ist, |
| x | 0 bis 5 ist, |
| y | 0 oder 1 ist, |
| z | 0 bis 5 ist und |

$X^4$ für -O-, -C(=O)-NH-,-NH-C(=O)- oder

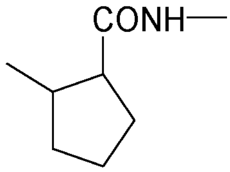

steht.

Bevorzugt weist der Linker L die Formel

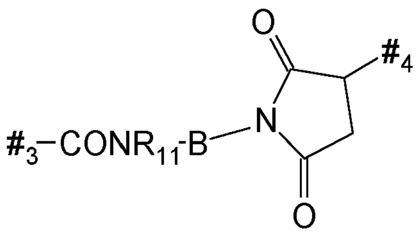

| | |
|---|---|
| auf, in der #3 | für die Bindung an das Wirkstoffmolekül bzw. Prodrug steht, |
| #4 | für die Bindung an das Binderpeptid oder -protein steht, |
| $R^{11}$ | für -H oder $-NH_2$ steht, |
| B | für die Gruppe$-[(CH_2)_x-(X^4)_y]_w-(CH_2)_z-$ steht, |
| w | 0 bis 20 ist, |
| x | 0 bis 5 ist, |
| y | 0 oder 1 ist, |
| z | 1 bis 5 ist und |
| $X^4$ | für -O-, -C(=O)-NH-, -NH-C(=O)- oder |

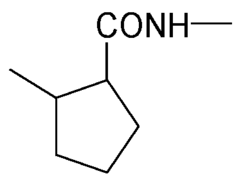

steht.

Die oben genannten Linker werden insbesondere bevorzugt in Konjugaten der Formel (IIa), in denen der Linker durch Substitution eines H-Atoms an $R^1$ oder in Verbindung mit einem spaltbaren Linker SG1 an $R^4$ ankoppelt, d.h. $R^1$ für -L-#1 steht oder $R^4$ für -SG1-L-#1 steht, wobei #1 für die Bindung an den Antikörper steht.

In einem erfindungsgemäßen Konjugat bzw. in einem Gemisch der erfindungsgemäßen Konjugate liegen die Bindungen an einen Cysteinrest des Antikörpers vorzugsweise zu über 80 %, besonders bevorzugt zu über 90 % vor, jeweils bezogen auf die Gesamtanzahl von Bindungen des Linkers an den Antikörper.

Hierbei besonders bevorzugt sind die beiden Strukturen der allgemeinen Formeln (A5) und (A6)

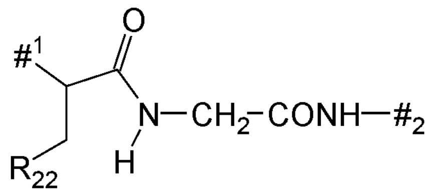

(A5) und

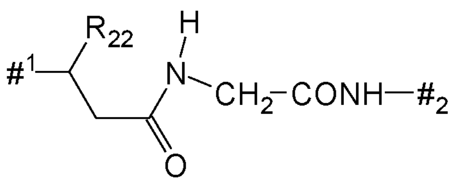

(A6),

| | |
|---|---|
| in denen $\#^1$ | für die Verknüpfungsstelle mit dem Schwefelatom des Antikörpers steht, |
| $\#^2$ | für die Verknüpfungsstelle mit der Gruppe $L^1$ steht, |
| $R^{22}$ | für -COOH, -C(=O)-OR, -C(=O)-R, -C(=O)-$NH_2$, -C(=O)-$NR_2$ oder -C(=O)-NHR steht und |
| R | für $C_{1-3}$-Alkyl- steht. |

Besonders bevorzugt steht $R^{22}$ für -COOH.

Hierbei liegen die Strukturen der allgemeinen Formeln A5 und A6 in der Regel gemeinsam vor, vorzugsweise in einem Verhältnis von 60:40 bis 40:60 bezogen auf die Anzahl der Bindungen an den Antikörper. Die restlichen Bindungen liegen dann in der Struktur

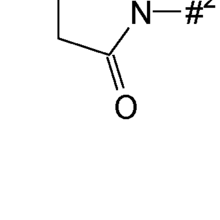

vor,
in der

#1 und #2 die oben angegebenen Bedeutungen haben.

Die Linker $-L_b-$ und $-L_c-$, die an eine Cystein-Seitenkette beziehungsweise einen Cysteinrest gebunden sind, weisen die allgemeine Formel

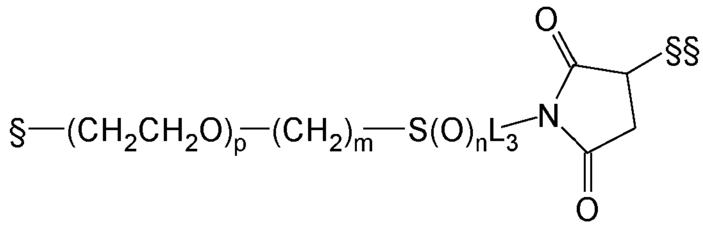

auf,
in der

§ für die Bindung an das Wirkstoffmolekül beziehungsweise Prodrug steht,
§§ für die Bindung an das Binderpeptid oder -protein steht,
m 0, 1, 2, oder 3 ist,
n 0, 1 oder 2 ist,
p 0 bis 20 ist,
L3 für die Gruppe

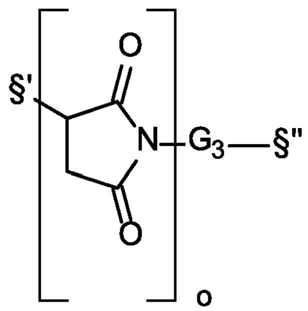

steht, in der
o 0 oder 1 ist,
$G_3$ für eine geradkettige oder verzweigte Kohlenwasserstoffkette mit 1 bis 100 Kohlenstoffatomen aus Arylengruppen und/oder geradkettigen und/oder cyclischen Alkylengruppen steht, die ein- oder mehrfach durch -O-, -S-, -S(=O)-, $-S(=O)_2$-, -NH-, -C(=O)-, -NHC(=O)-, -C(=O)-NH-, -NMe-, -NHNH-, $-S(=O)_2$-NHNH-, -C(=O)-NHNH- und einen 3 bis 10gliedriger aromatischen oder nicht aromatischen Heterozyklus mit bis zu 4 Heteroatomen ausgewählt aus =N-, -O- und -S-, -S(=O)-oder $-S(=O)_2$- unterbrochen sein kann wobei die geradkettige oder verzweigte Kohlenwasserstoffkette zusätzlich mit $-NH-C(=O)-NH_2$, -COOH, -OH, $-NH_2$, Sulfonamid, Sulfon, Sulfoxid oder Sulfonsäure substituiert sein kann,
§' für die Bindung an die -S(O)n-Gruppe und
§'' für die Bindung an das N-Atom im Ring steht.

Vorzugsweise ist der aromatische oder nicht aromatischen Heterozyklus 5 bis 10 gliedrig.

Bevorzugt steht $G_3$ für

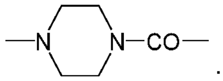

Bevorzugt sind solche Verbindungen der Formel

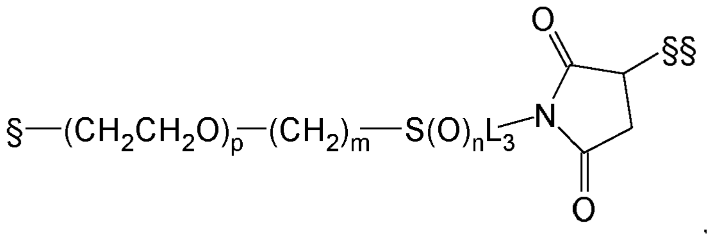

in der

m 1 ist,
p 0 ist,
n 0 ist,
L3 für die Gruppe

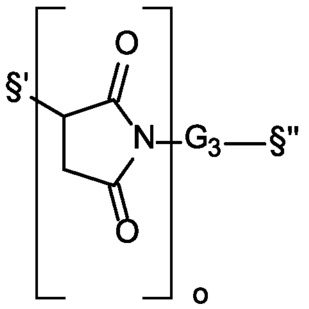

steht,
in der
o 0 oder 1 ist,
$G_3$ für $-(CH_2CH_2O)_s - (CH_2)_t - (C(=O)-NH)_u - CH_2CH_2O)_v - (CH_2)_w-$ steht,
s, t, v und w unabhängig voneinander für 0 bis 20 steht,
u 0 oder 1 ist,
§' für die Bindung an die -S(O)n-Gruppe und
§'' für die Bindung an das N-Atom im Ring steht.

Bevorzugte Gruppen für L1 in der obigen Formel §-(C(=O))m-L1-L2-§§ sind die, die in der folgenden Tabelle aufgeführt sind, wobei r eine Zahl von 0 bis 20, vorzugsweise von 0 bis 15, besonders bevorzugt von 1 bis 20, insbesondere bevorzugt von 2 bis 10 aufweist.

| L1 |
|---|
| 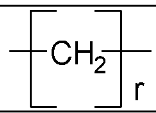 |
| 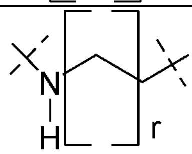 |

| L1 |
|---|
| 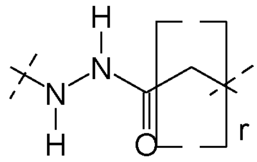 |
| 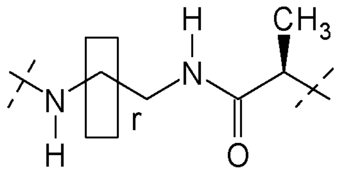 |
| 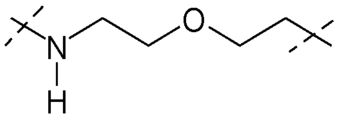 |
| 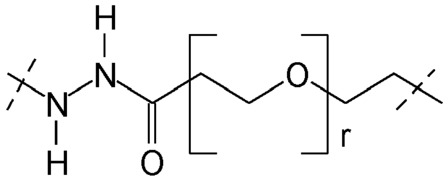 |
| 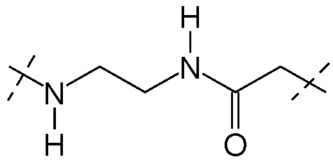 |
| 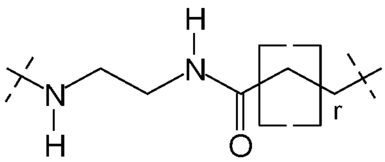 |
| 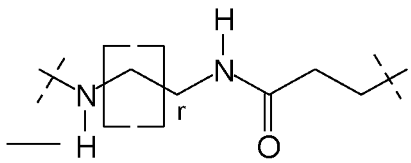 |
| 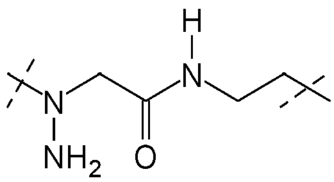 |
| 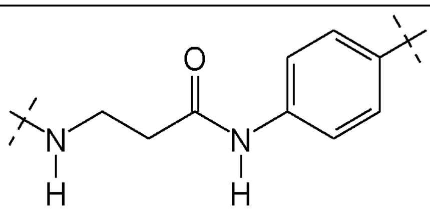 |

| L1 |
|---|
| 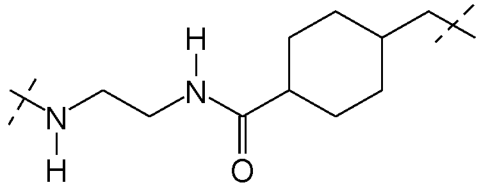 |
| 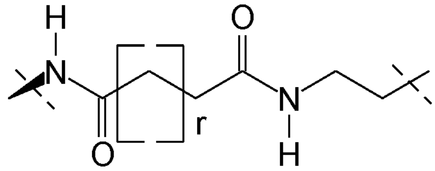 |
| 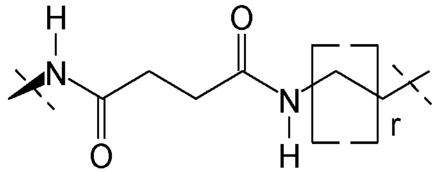 |
| 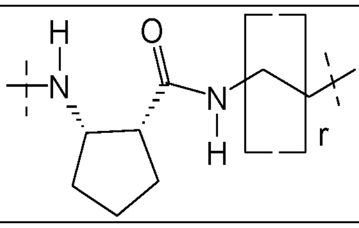 |
| 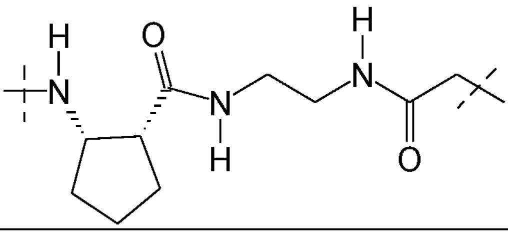 |
| 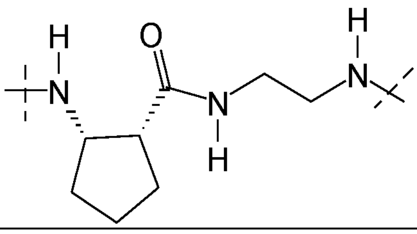 |
| 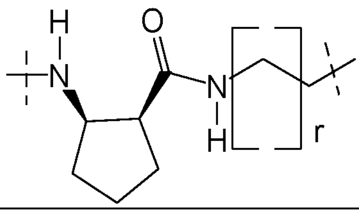 |

| L1 |
| --- |
| 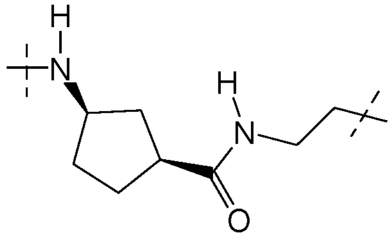 |
| 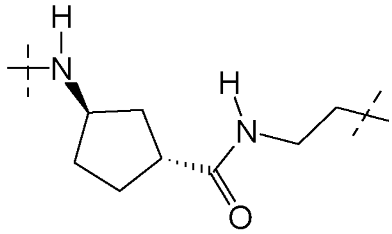 |
| 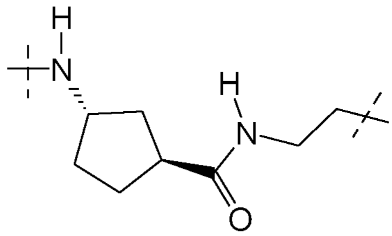 |
| 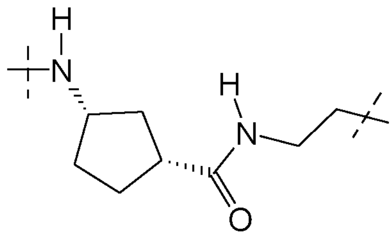 |
| 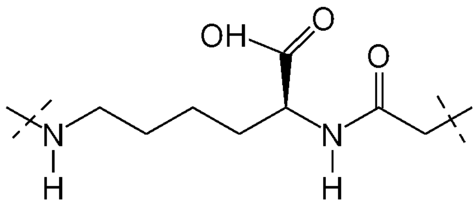 |
| 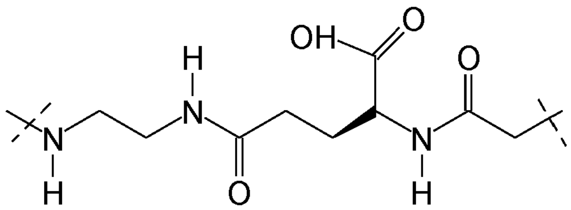 |
| 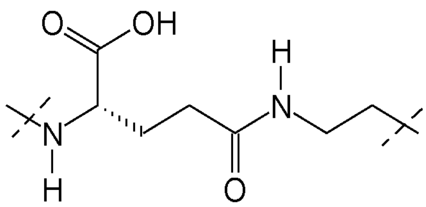 |

| L1 |
| --- |
| 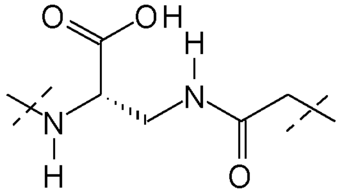 |
| 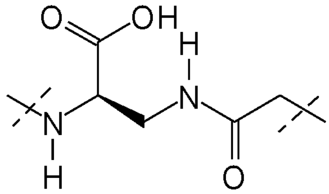 |
| 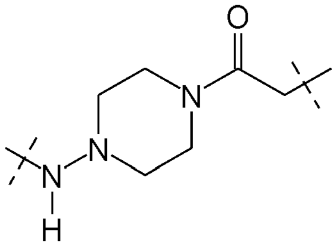 |
| 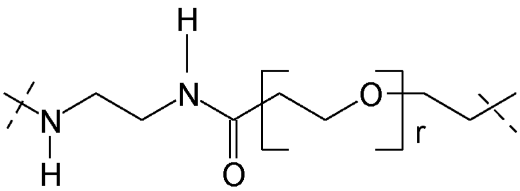 |
| 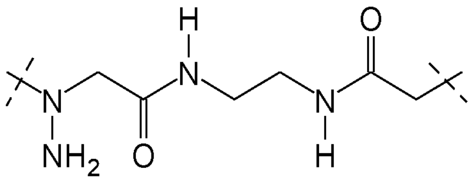 |
| 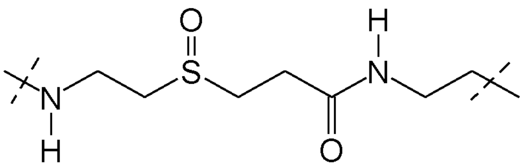 |
| 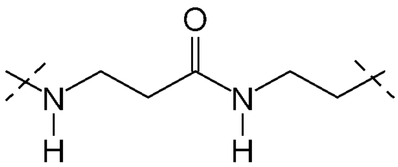 |
| 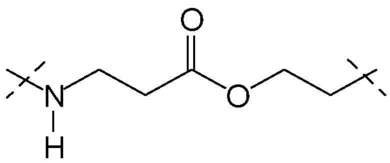 |

| L1 |
| --- |
| 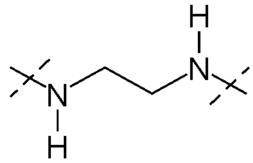 |
| 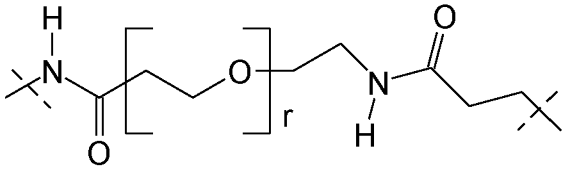 |
| 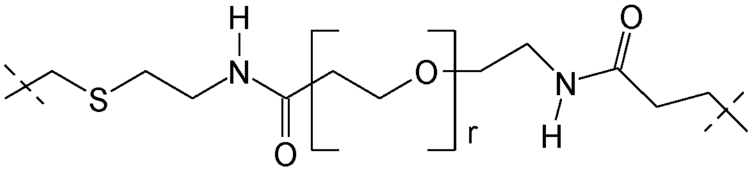 |
| 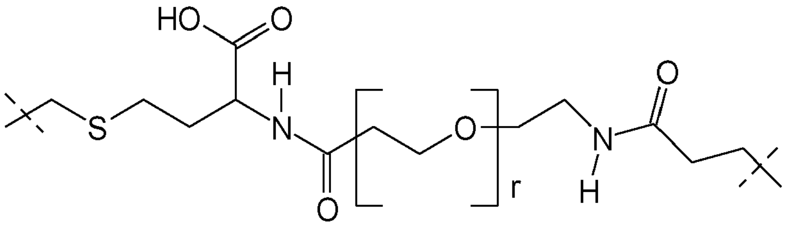 |
| 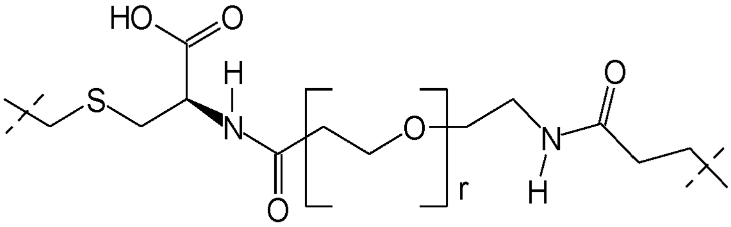 |
| 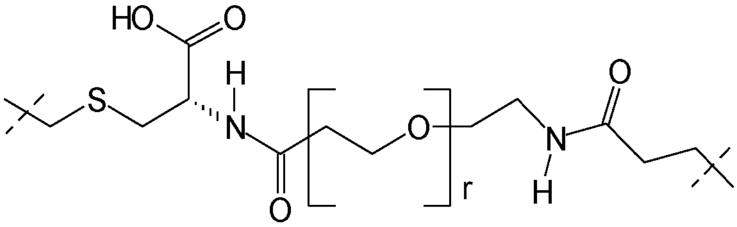 |
| 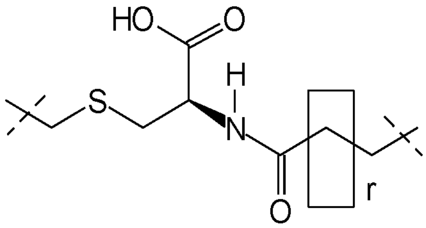 |
| 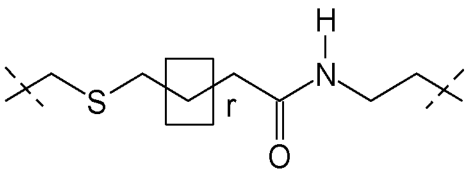 |

| L1 |
|---|
| 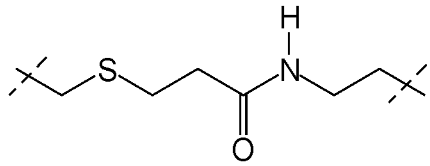 |
| 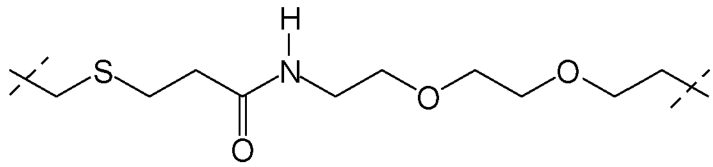 |
| 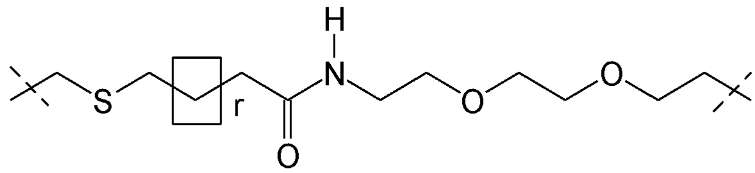 |
| 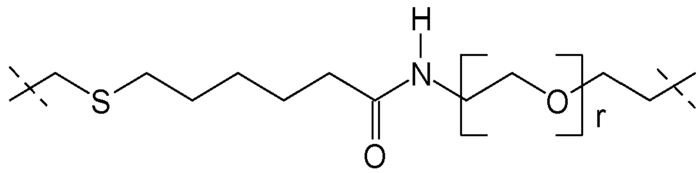 |
| 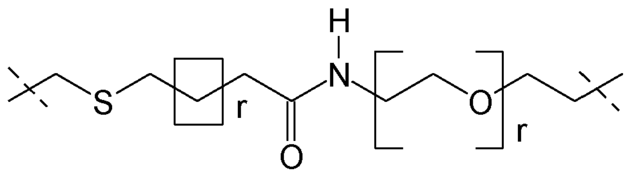 |
| 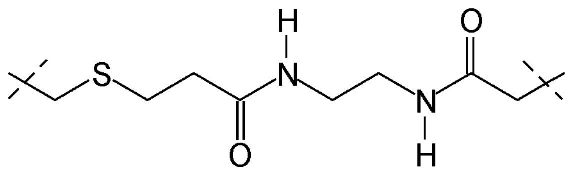 |
| 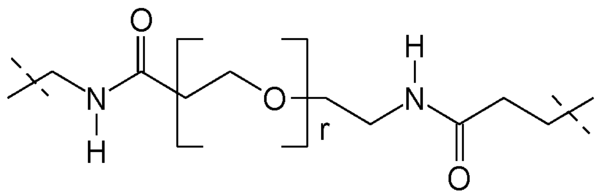 |
| 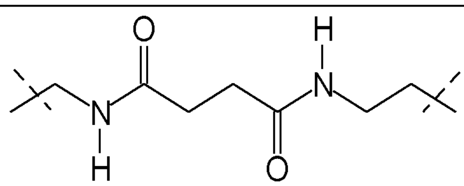 |
| 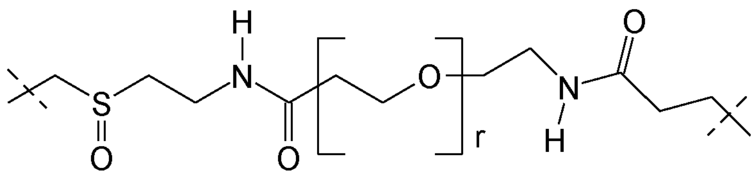 |

| L1 |
|---|
| 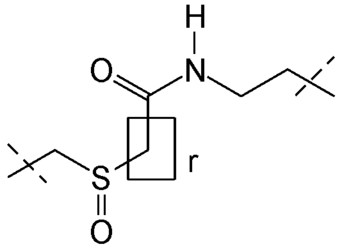 |
| 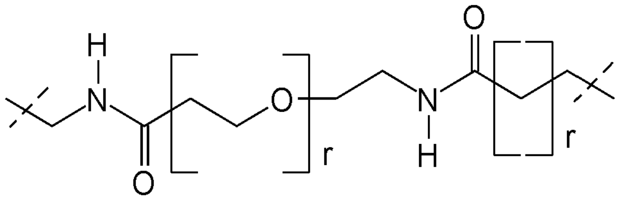 |
| 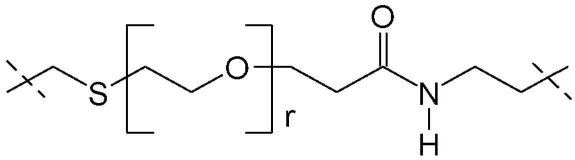 |
| 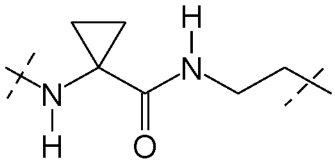 |
| 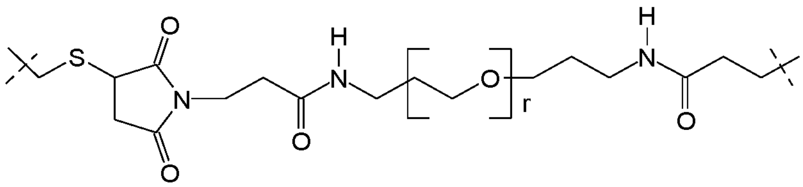 |
| 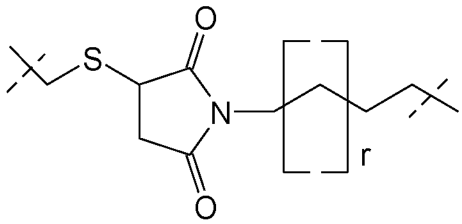 |
| 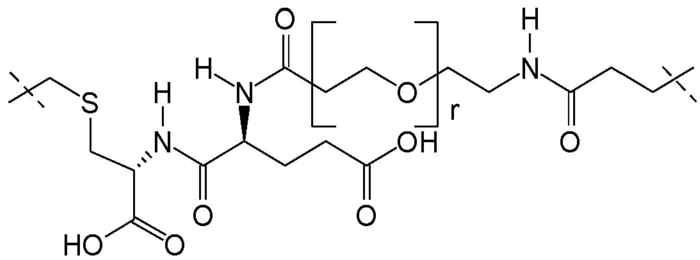 |

| L1 |
| --- |
| 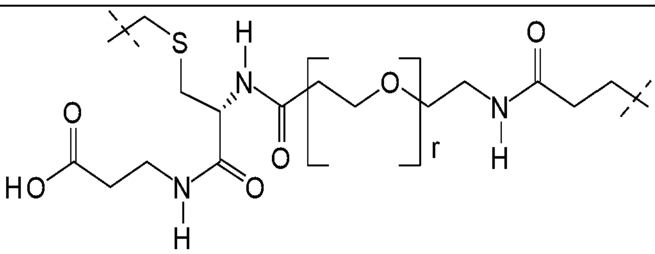 |
| 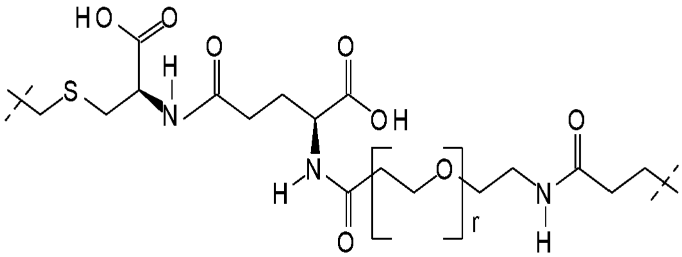 |
| 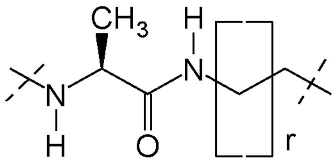 |
| 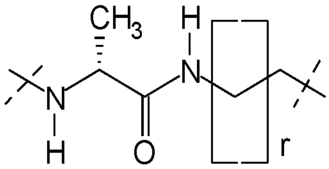 |
| 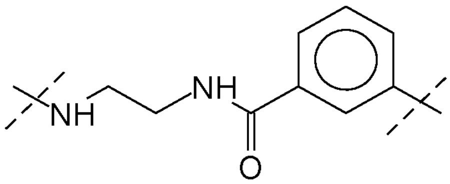 |
| 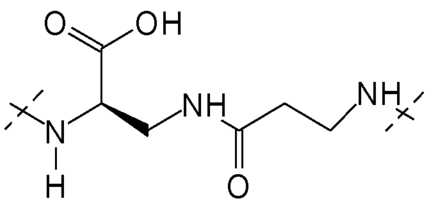 |

| L1 |
| --- |
| 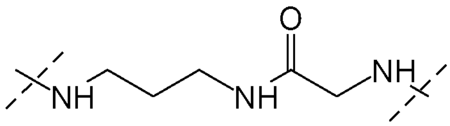 |
| 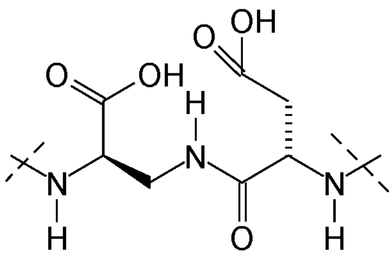 |
| 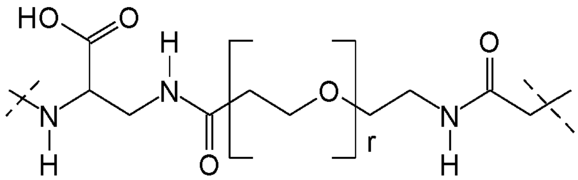 |
| 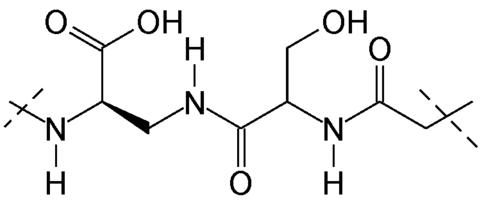 |
| 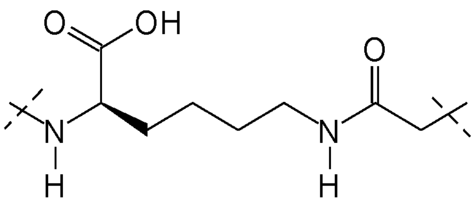 |

| L1 |
|---|
| 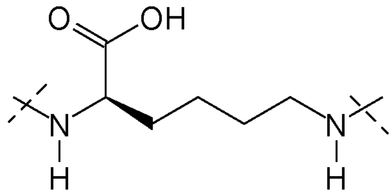 |
| 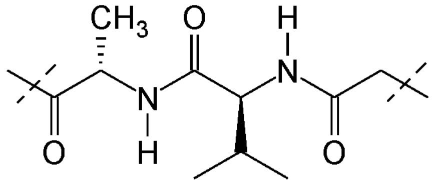 |
| 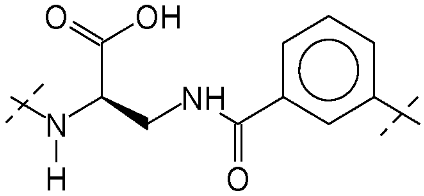 |
| 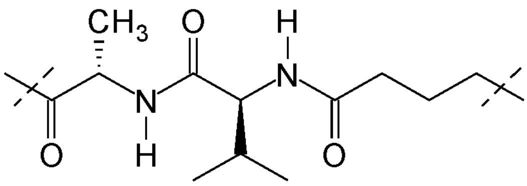 |
| 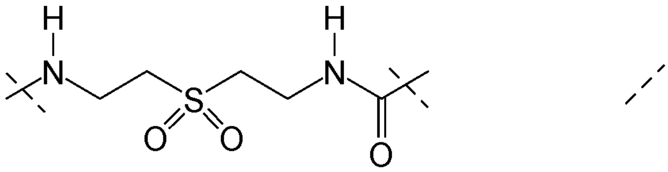 |
| 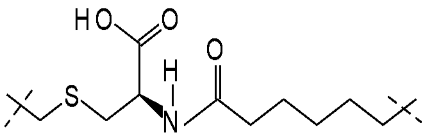 |

| L1 |
|---|
| 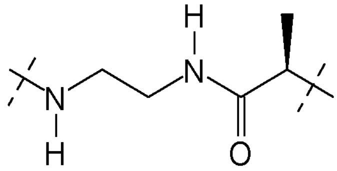 |
| 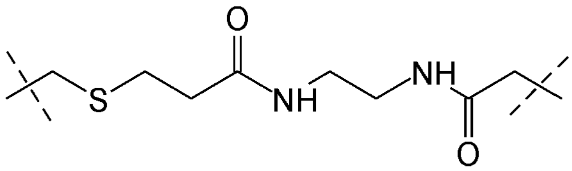 |
| 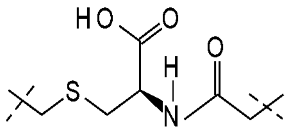 |
| 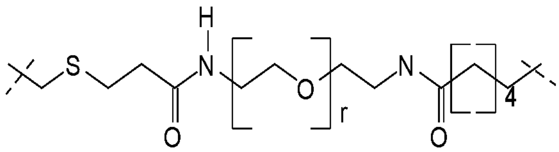 |
| 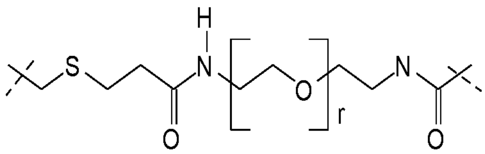 |
| 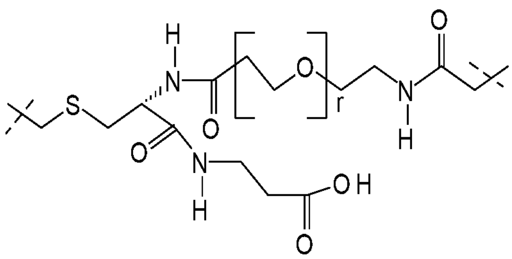 |

| L1 |
| --- |
| 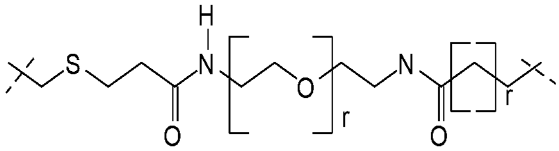 |
| 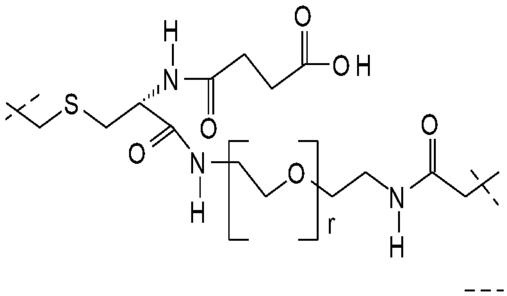 |
| 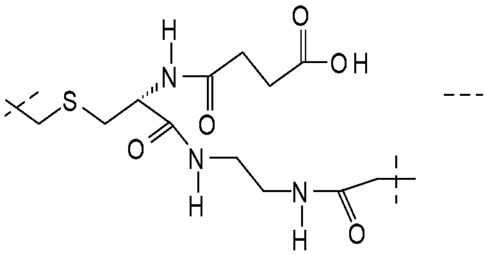 |
| 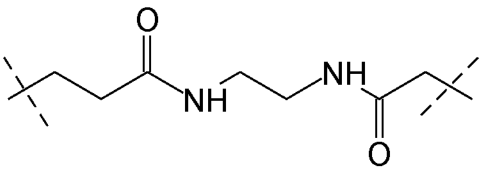 |
| 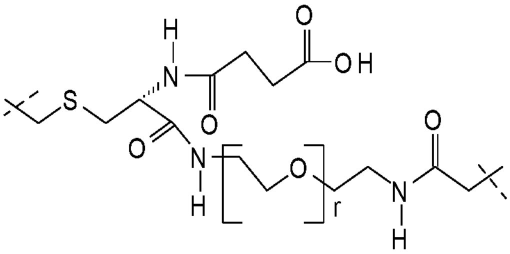 |

| L1 |
|---|
| 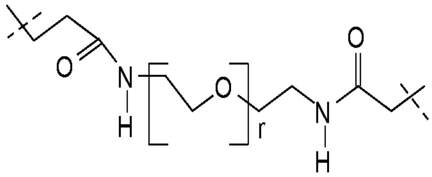 |
| 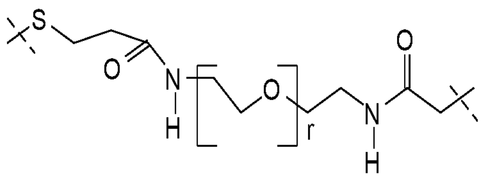 |
| 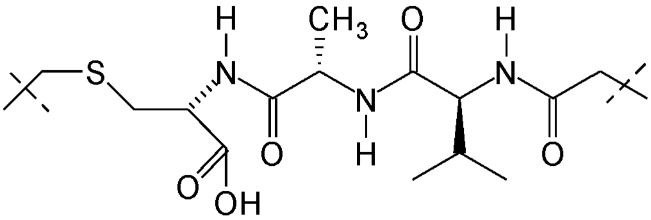 |
| 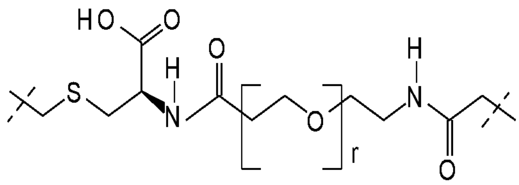 |
| 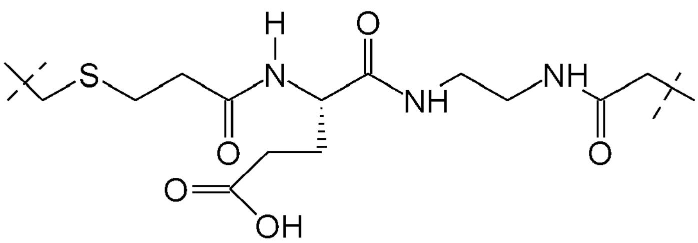 |

| L1 |
|---|
| 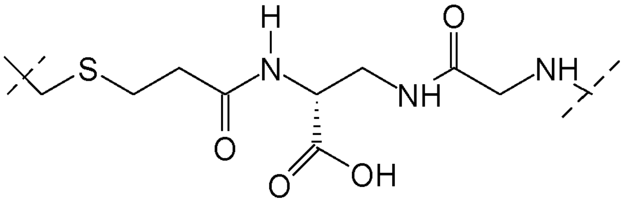 |
| 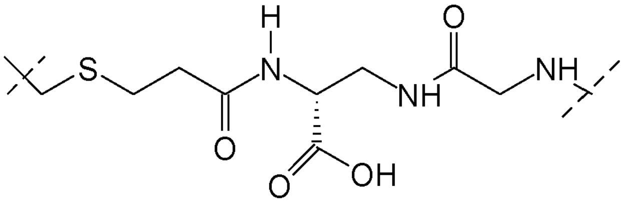 |

Weitere Beispiele für L1 sind in Tabelle C angegeben, in denen diese Gruppe in einem Kasten hervorgehoben ist.

In den folgenden Tabellen A und A' sind Beispiele für einen Linkerteil L1 angegeben. In den Tabellen ist weiterhin angegeben, mit welcher Gruppe L2 diese Beispiele für L1 vorzugsweise kombiniert werden, sowie die bevorzugte Ankopplungsstelle ($R^1$ oder $R^3$ in einer Verbindung der Formeln (IIa), (IIb), (IIc), (IId), (V) und (VI)) sowie der bevorzugte Wert für m, also ob vor L1 eine Carbonylgruppe vorliegt oder nicht (vgl. §-(C(=O))m-L1-L2-§§). Diese Linker werden vorzugsweise an einen Cysteinrest gekoppelt. Wenn L2 ein Bernsteinsäureimid ist bzw. sich hiervon ableitet, kann dieses Imid ganz oder teilweise auch in Form des hydrolysierten offenkettigen Bernsteinsäureamids vorliegen, wie oben beschrieben. In Abhängigkeit von L1 kann diese Hydrolyse zu offenkettigen Bernsteinsäureamiden mehr oder weniger stark oder gar nicht ausgeprägt sein.

Tabelle A (Subst. = Substituent)

| Su bst | m | L1 | L2 |
|---|---|---|---|
| $R^1$ | 1 | 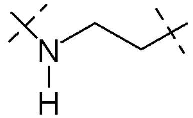 | 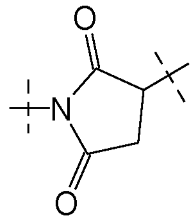 |
| $R^1$ | 1 | 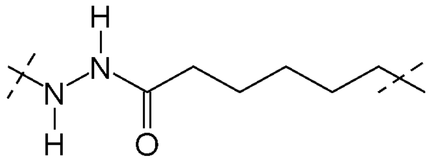 | 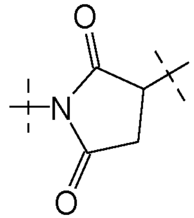 |
| $R^1$ | 1 | 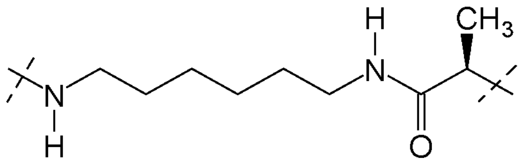 | 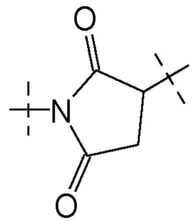 |
| $R^1$ | 1 | 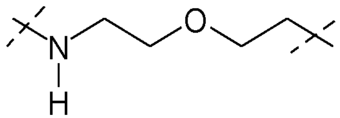 | 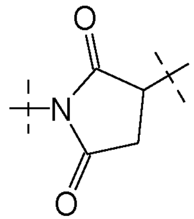 |

(fortgesetzt)

| Su bst | m | L1 | L2 |
|---|---|---|---|
| $R^1$ | 1 | 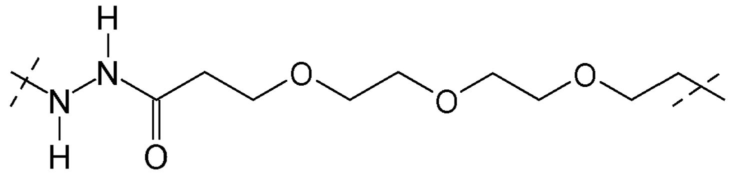 | 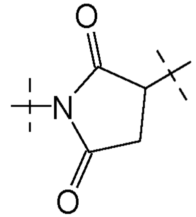 |
| $R^1$ | 1 | 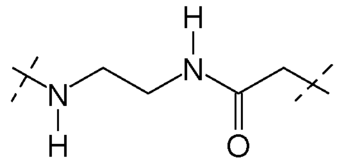 | 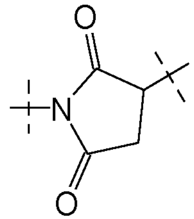 Siehe Anmerkung ** |
| $R^1$ | 1 | 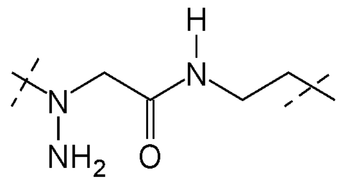 | 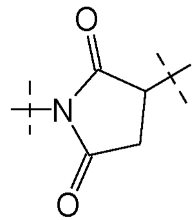 |
| $R^1$ | 1 | 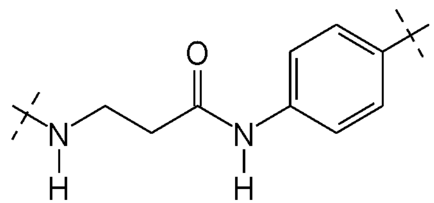 | 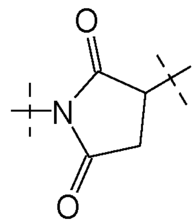 |

(fortgesetzt)

| Su bst | m | L1 | L2 |
|---|---|---|---|
| $R^1$ | 1 | 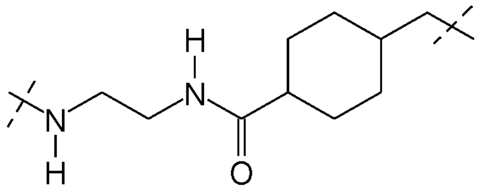 | 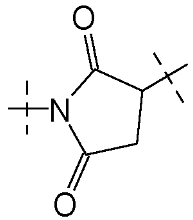 |
| $R^1$ | 1 | 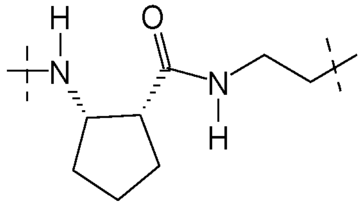 | 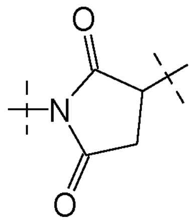 |
| $R^1$ | 1 | 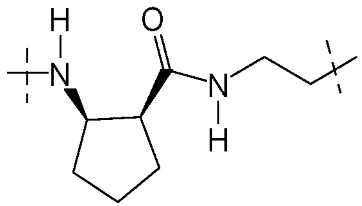 | 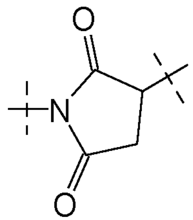 |
| $R^1$ | 1 | 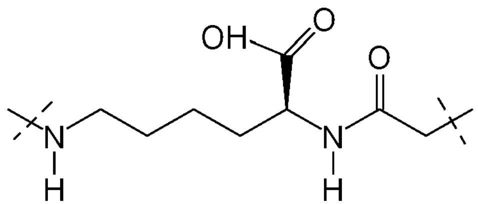 | 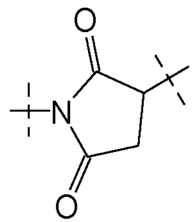 Siehe Anmerkung ** |

(fortgesetzt)

| Su bst | m | L1 | L2 |
|---|---|---|---|
| $R^1$ | 1 | 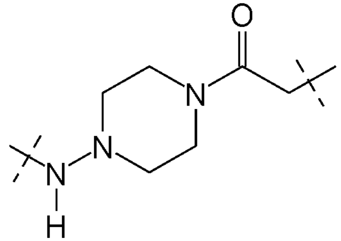 | 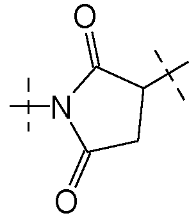 Siehe Anmerkung ** |
| $R^1$ | 1 | 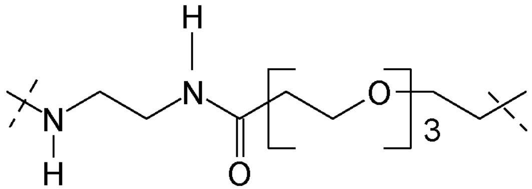 | 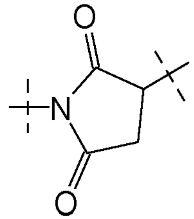 |
| $R^1$ | 1 | 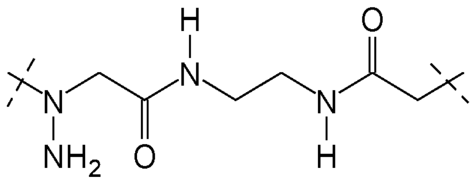 | 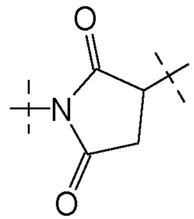 Siehe Anmerkung ** |
| $R^1$ | 1 | 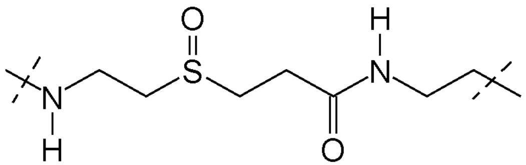 | 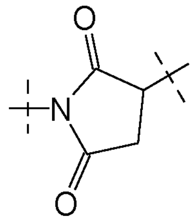 |

(fortgesetzt)

| Su bst | m | L1 | L2 |
|---|---|---|---|
| $R^1$ | 1 | 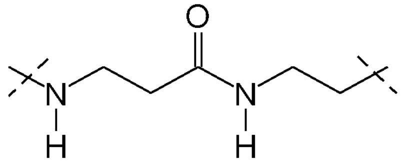 | 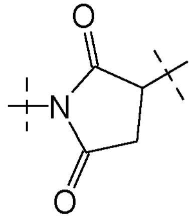 |
| $R^1$ | 1 | 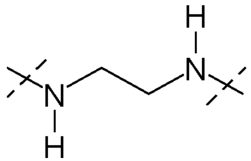 | 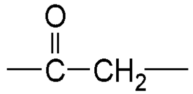 |
| $R^1$ | 1 | 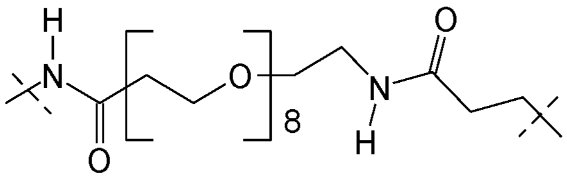 | 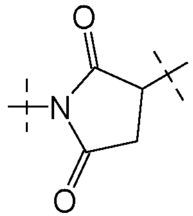 |
| $R^3$ | 0 | 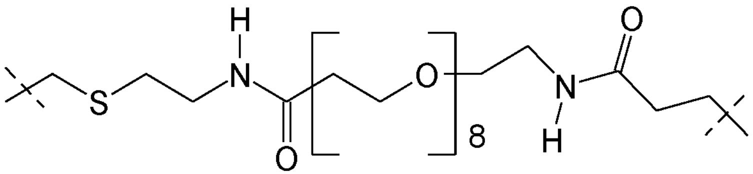 | 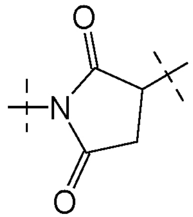 |

(fortgesetzt)

| Su bst | m | L1 | L2 |
|---|---|---|---|
| $R^1$ | 1 | 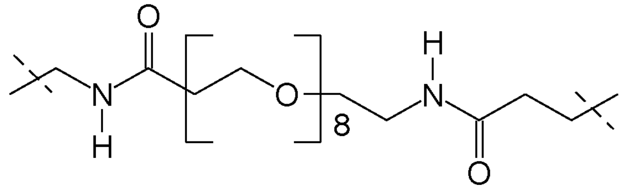 | 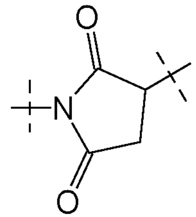 |
| $R^3$ | 0 | 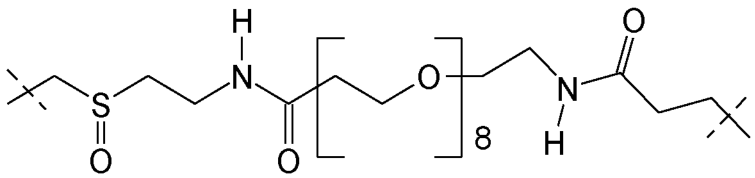 | 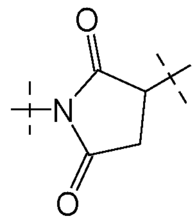 |
| $R^1$ | 1 | 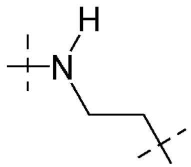 | 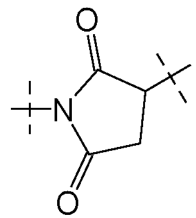 |
| $R^1$ | 0 | 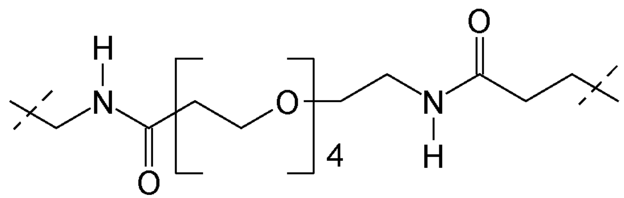 | 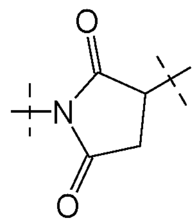 |

(fortgesetzt)

| Su bst | m | L1 | L2 |
|---|---|---|---|
| $R^3$ | 0 | 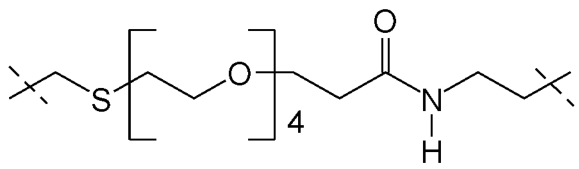 | 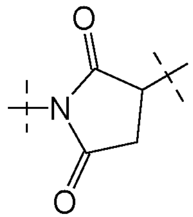 |
| $R^3$ | 0 | 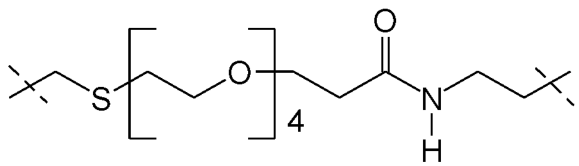 | 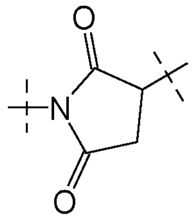 |
| $R^1$ | 1 | 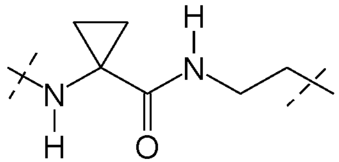 | 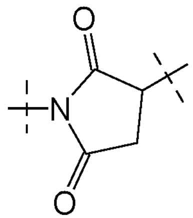 |
| $R^3$ | 0 | 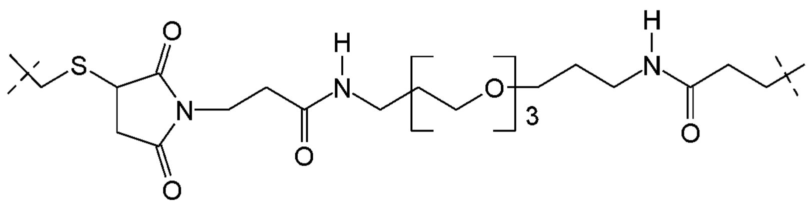 | 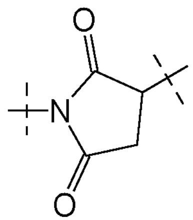 |

(fortgesetzt)

| Su bst | m | L1 | L2 |
|---|---|---|---|
| $R^3$ | 0 | 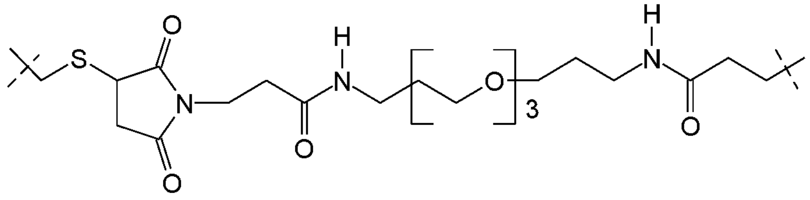 | 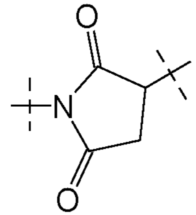 |
| $R^3$ | 0 | 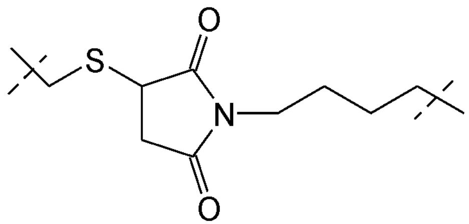 | 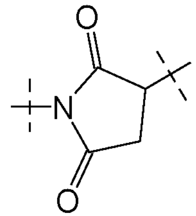 |
| $R^3$ | 0 | 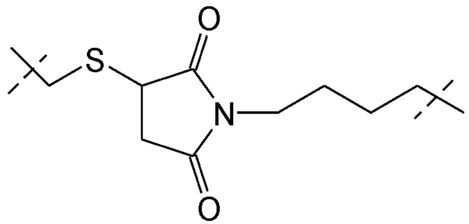 | 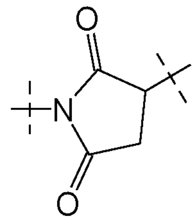 |
| $R^1$ | 1 | 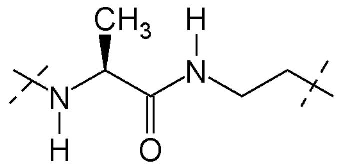 | 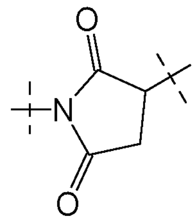 |

(fortgesetzt)

| Su bst | m | L1 | L2 |
|---|---|---|---|
| $R^1$ | 1 | 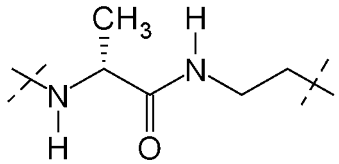 | 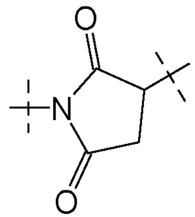 |

**Besonders bevorzugt werden die in diesen Zeilen angegebenen Linker L1 mit einem Linker L2 verknüpft, die ausgewählt sind aus den allgemeinen Formeln (A7) und (A8):

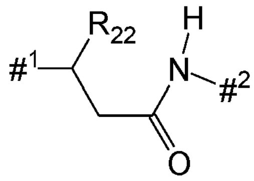

(A7),

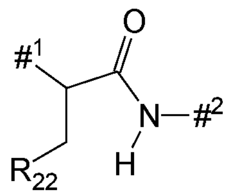

(A8)

in denen

$\#^1$ für die Verknüpfungsstelle mit dem Schwefelatom des Binders steht,
$\#^2$ für die Verknüpfungsstelle mit der Gruppe $L^1$ steht und
$R^{22}$ vorzugsweise für -COOH steht.

In einem erfindungsgemäßen Konjugat beziehungsweise in einem Gemisch der erfindungsgemäßen Konjugate liegen die Bindungen an einen Cysteinrest des Binders vorzugsweise zu über 80 %, besonders bevorzugt zu über 90 %, jeweils bezogen auf die Gesamtanzahl von Bindungen des Linkers an den Binder und besonders bevorzugt in einer der beiden Strukturen der Formeln (A7) und (A8) vor.

Hierbei liegen die Strukturen der Formeln (A7) und (A8) in der Regel gemeinsam vor, vorzugsweise in einem Verhältnis von 60:40 bis 40:60, bezogen auf die Anzahl der Bindungen an den Binder. Die restlichen Bindungen liegen dann in der Struktur

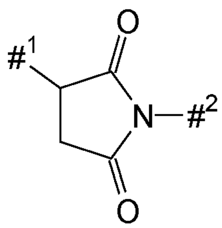

vor, in der $\#^1$ und $\#^2$ die oben angegebenen Bedeutungen haben.

Tabelle A' (Subst. = Substituent)

| Subs t. | m | L1 | L2 |
|---|---|---|---|
| $R^1$ | 1 | 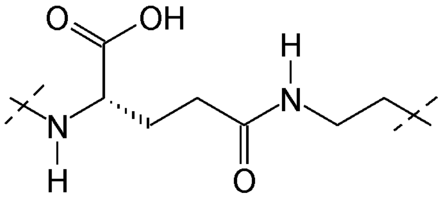 | 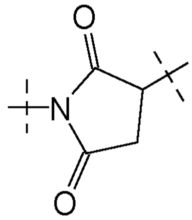 |
| $R^1$ | 1 | 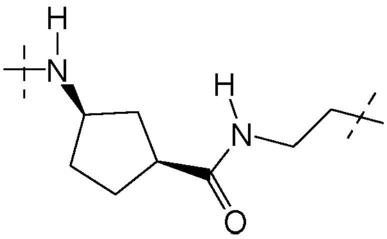 | 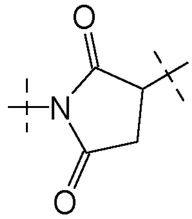 |
| $R^1$ | 1 | 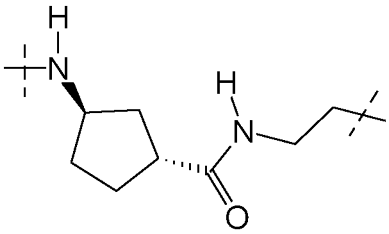 | 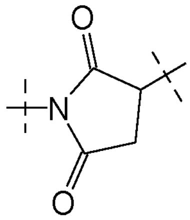 |
| $R^1$ | 1 | 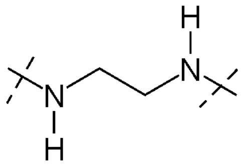 | 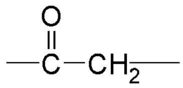 |
| $R^1$ | 1 | 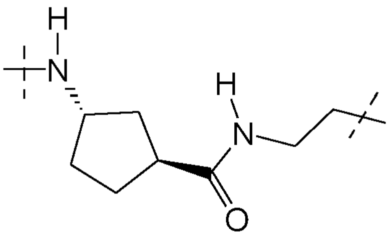 | 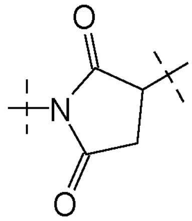 |
| $R^1$ | 1 | 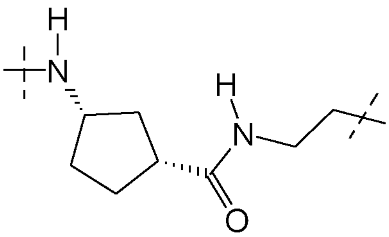 | 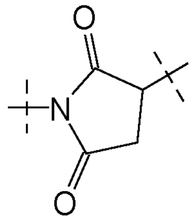 |

(fortgesetzt)

| Subs t. | m | L1 | L2 |
|---|---|---|---|
| $R^3$ | 0 | 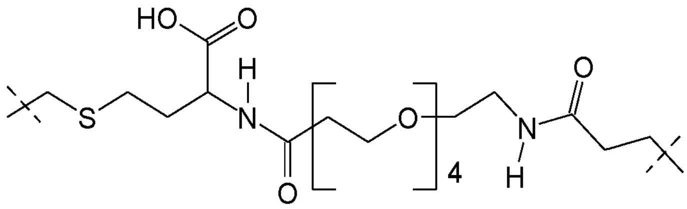 | 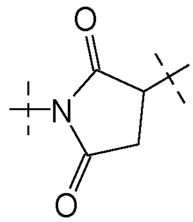 |
| $R^3$ | 0 | 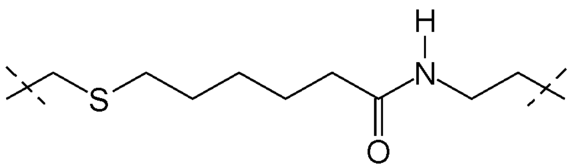 | 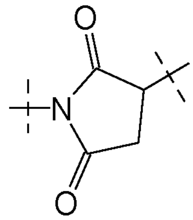 |
| $R^3$ | 0 | 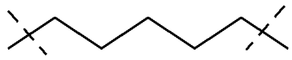 | 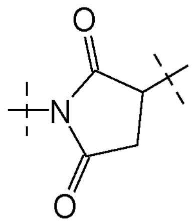 |
| $R^3$ | 0 | 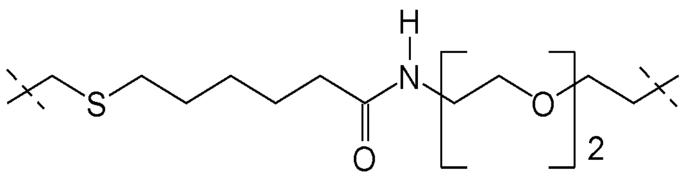 | 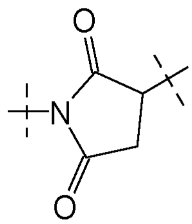 |
| $R^3$ | 0 | 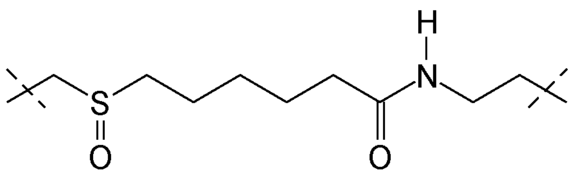 | 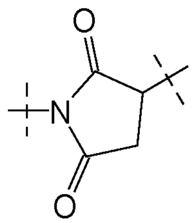 |
| $R^1$ | 1 | 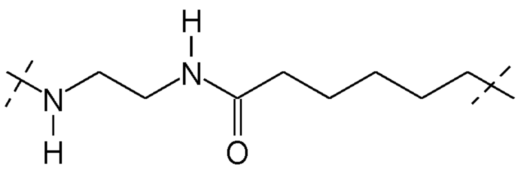 | 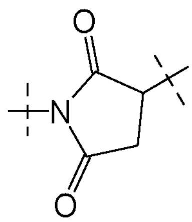 |

(fortgesetzt)

| Subs t. | m | L1 | L2 |
|---|---|---|---|
| $R^1$ | 1 | 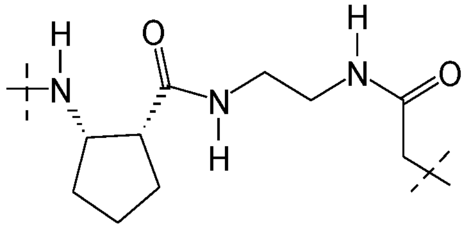 | 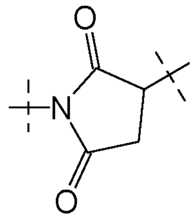 Siehe Anmerkung ** |
| $R^1$ | 1 | 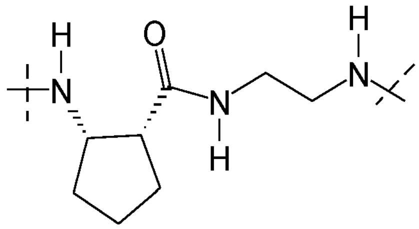 | 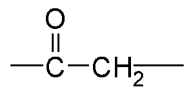 |
| $R^1$ | 1 | 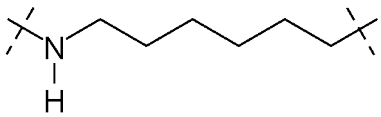 | 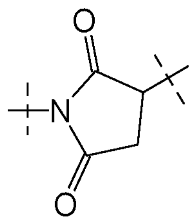 |
| $R^1$ | 1 | 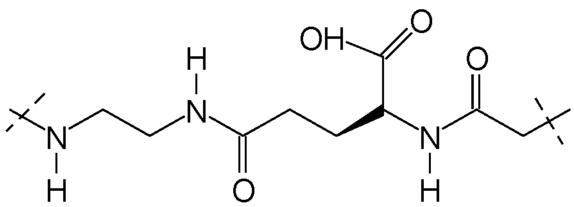 | 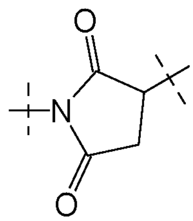 Siehe Anmerkung ** |
| $R^1$ | 1 | 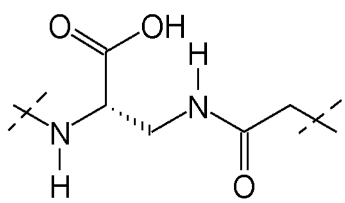 | 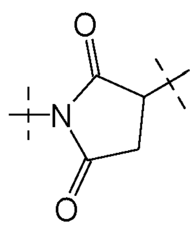 Siehe Anmerkung ** |

(fortgesetzt)

| Subs t. | m | L1 | L2 |
|---|---|---|---|
| $R^1$ | 1 | 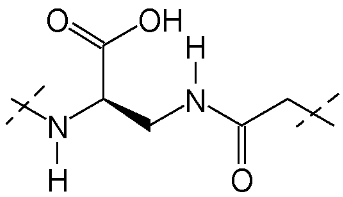 | 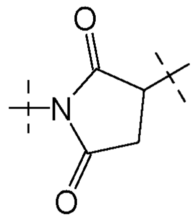 Siehe Anmerkung ** |
| $R^1$ | 0 | 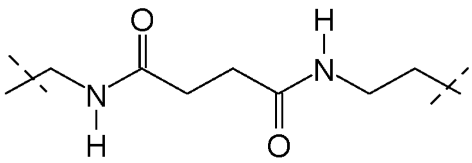 | 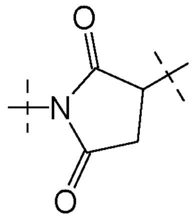 |
| $R^1$ | 1 | 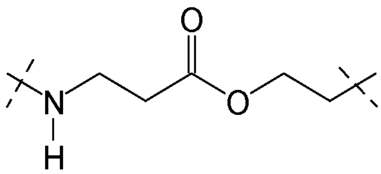 | 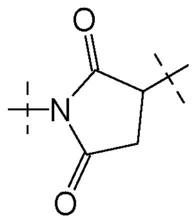 |
| $R^1$ | 1 | 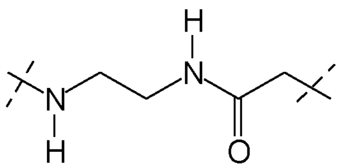 | 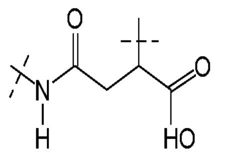 und 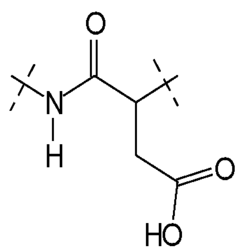 Siehe Anmerkung *** |

(fortgesetzt)

| Subs t. | m | L1 | L2 |
|---|---|---|---|
| R1 | 1 | 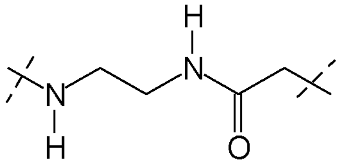 | 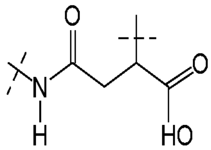 |
| R1 | 1 | 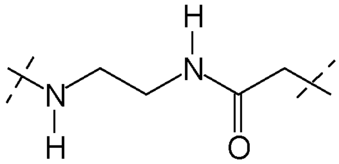 | |
| | | | 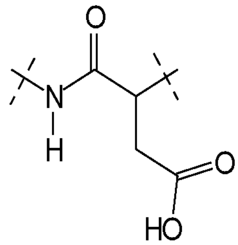 Identisch zu den beiden vorher |
| R1 | 1 | 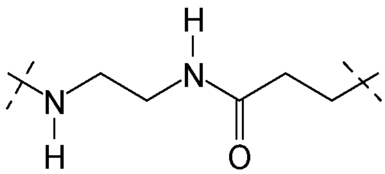 | 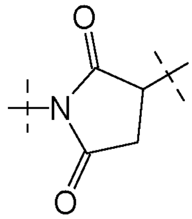 |
| R3 | 0 | 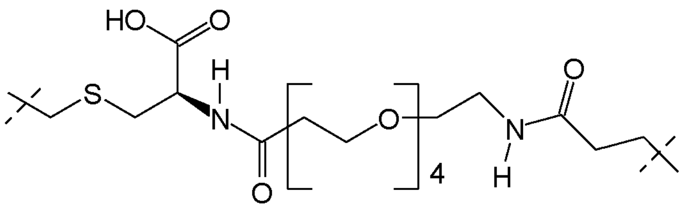 | 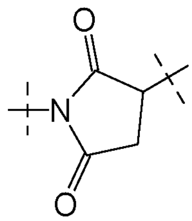 |
| R3 | 0 | 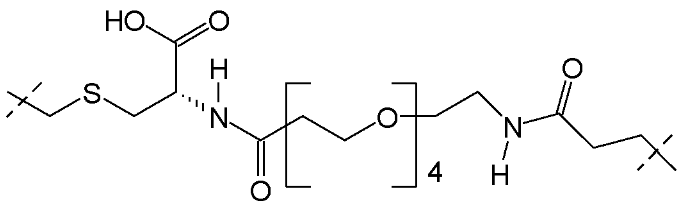 | 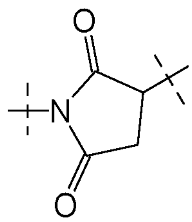 |

(fortgesetzt)

| Subs t. | m | L1 | L2 |
|---|---|---|---|
| $R^3$ | 0 | 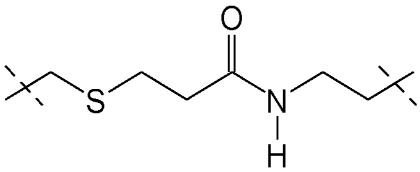 | 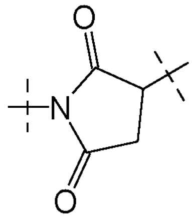 |
| $R^3$ | 0 | 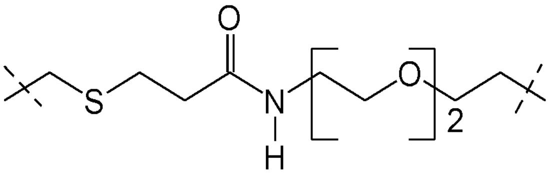 | 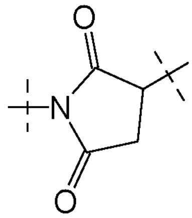 |
| $R^3$ | 0 | 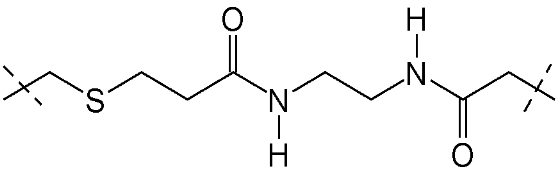 | 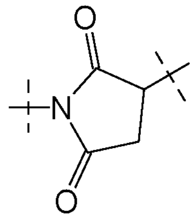 Siehe Anmerkung ** |
| $R^3$ | 0 | 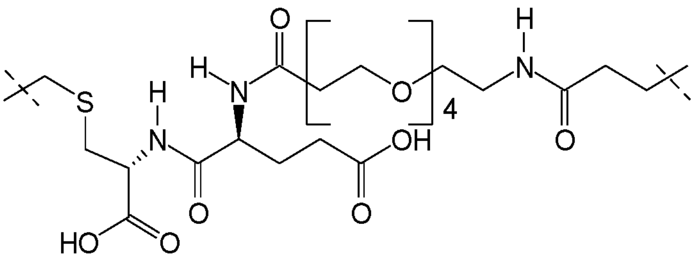 | 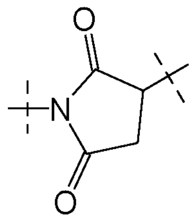 |
| $R^3$ | 0 | 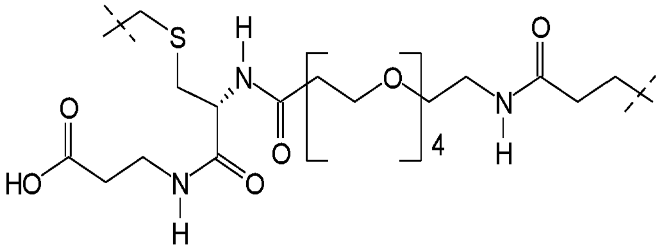 | 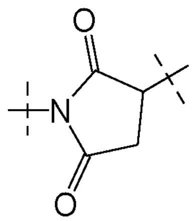 |

(fortgesetzt)

| Subs t. | m | L1 | L2 |
|---|---|---|---|
| $R^3$ | 0 | 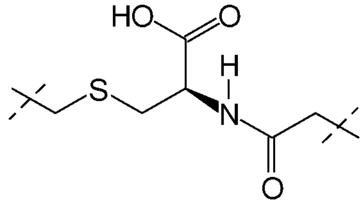 | 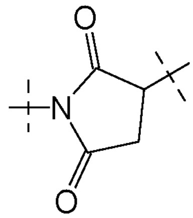 Siehe Anmerkung ** |
| $R^3$ | 0 | 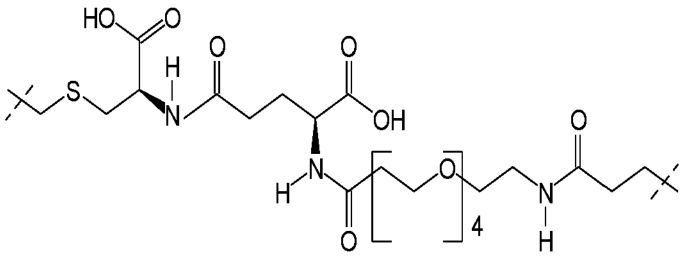 | 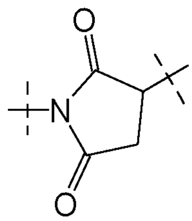 |
| $R^2$ | 0 | 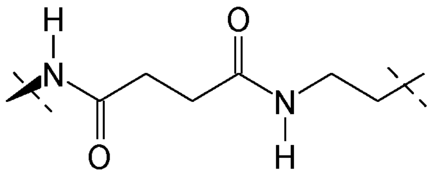 | 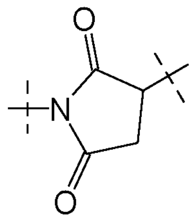 |
| $R^1$ | 1 | 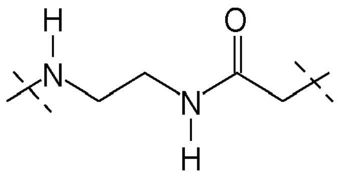 | 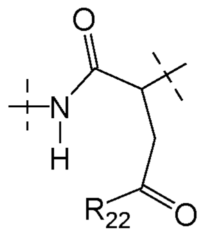 Mit $R_{22}$ = -OH oder _$NH_2$ |
| $R^1$ | 1 | 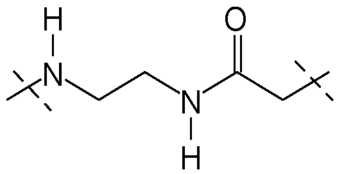 | 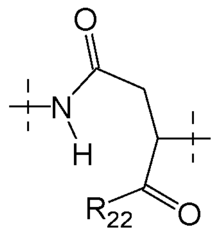 Mit $R_{22}$ = -OH oder _$NH_2$ |

(fortgesetzt)

| Subs t. | m | L1 | L2 |
|---|---|---|---|
| $R^1$ | 1 | 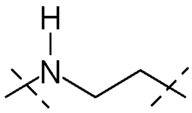 | 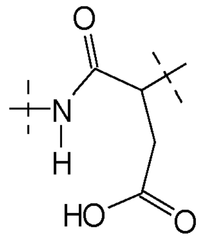 und 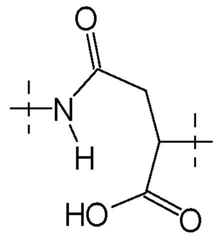 Siehe Anmerkung *** |
| $R^1$ | 1 | 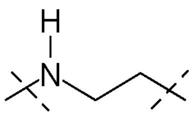 | 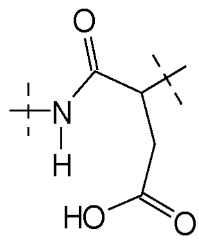 |
| $R^1$ | 1 | 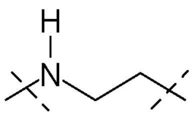 | 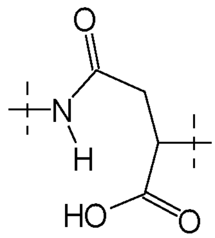 |

(fortgesetzt)

| Subs t. | m | L1 | L2 |
|---|---|---|---|
| $R^1$ | 1 | 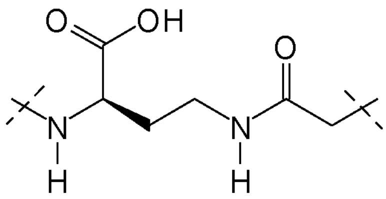 | 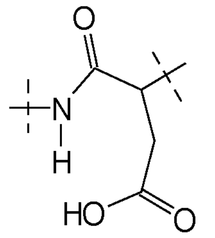 und 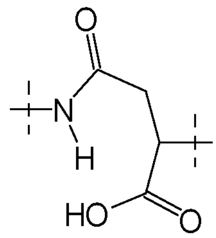 Siehe Anmerkung *** |
| $R^1$ | 1 | 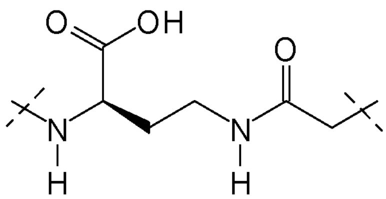 | 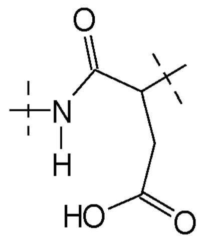 |
| $R^1$ | 1 | 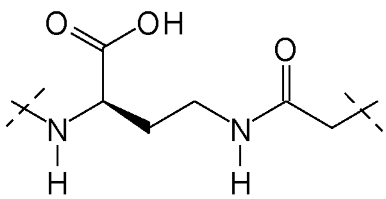 | 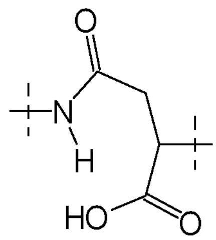 |

(fortgesetzt)

| Subs t. | m | L1 | L2 |
|---|---|---|---|
| $R^3$ | 0 | 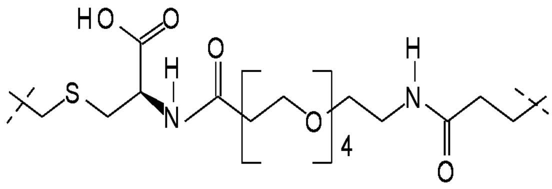 | 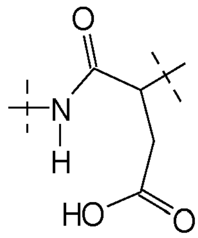 und 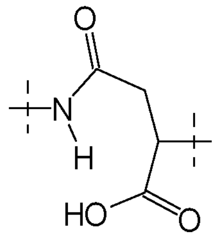 Siehe Anmerkung *** |
| $R^3$ | 0 | 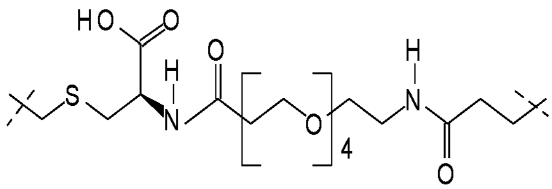 | 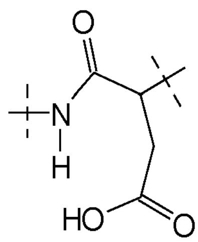 |
| $R^3$ | 0 | 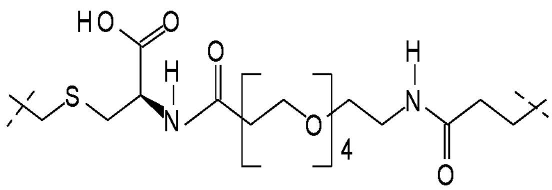 | 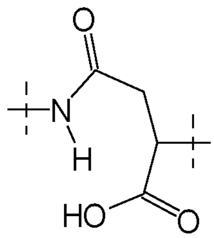 |

(fortgesetzt)

| Subs t. | m | L1 | L2 |
|---|---|---|---|
| $R^3$ | 0 | 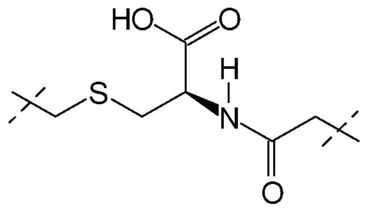 | 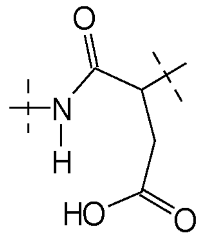 und 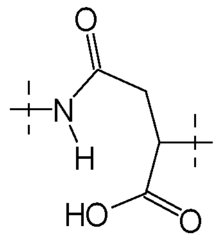 Siehe Anmerkung *** |
| $R^3$ | 0 | 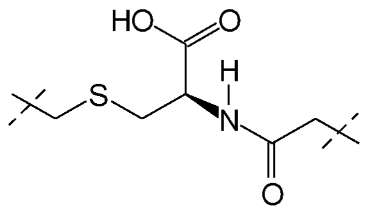 | 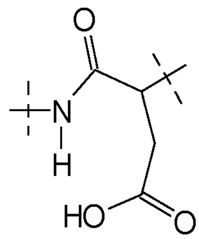 |
| $R^3$ | 0 | 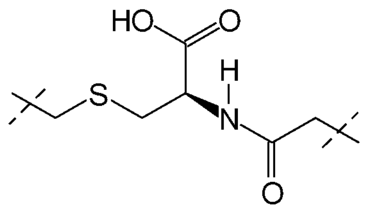 | 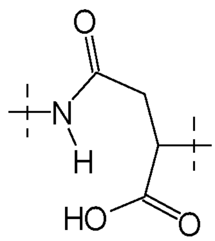 |

(fortgesetzt)

| Subs t. | m | L1 | L2 |
|---|---|---|---|
| $R^3$ | 0 | 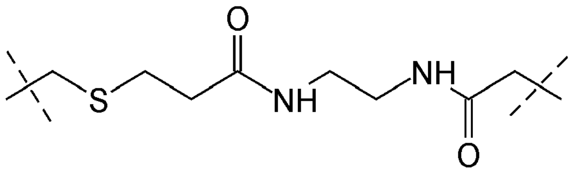 | 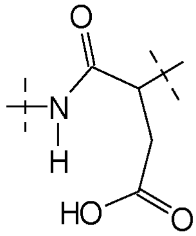 und 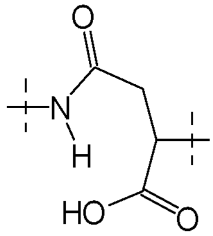 Siehe Anmerkung *** |
| $R^3$ | 0 | 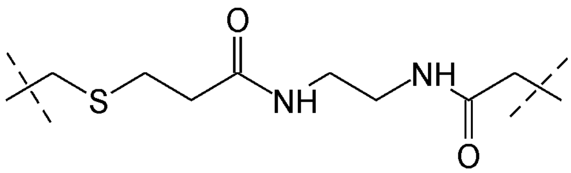 | 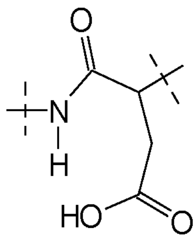 |
| $R^3$ | 0 | 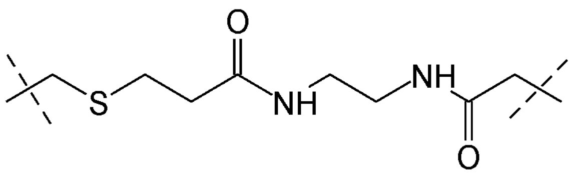 | 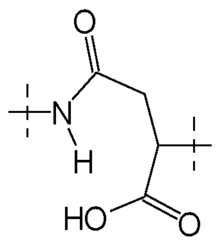 |
| $R^1$ | 1 | 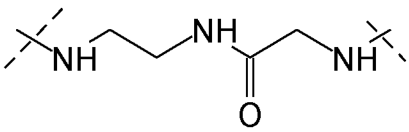 | 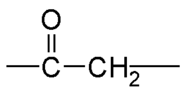 |

(fortgesetzt)

| Subs t. | m | L1 | L2 |
|---|---|---|---|
| $R^1$ | 1 | 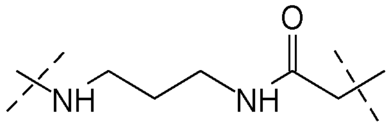 | 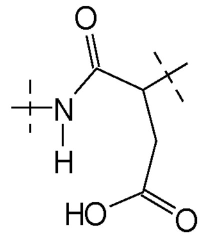 und 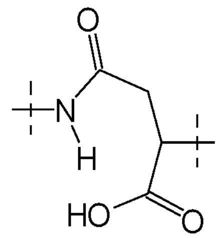 Siehe Anmerkung *** |
| $R^1$ | 1 | 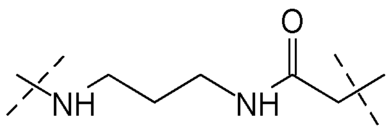 | 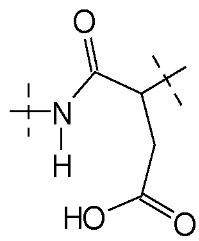 |
| $R^1$ | 1 | 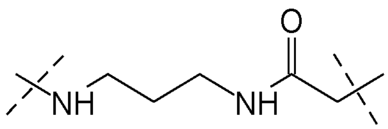 | 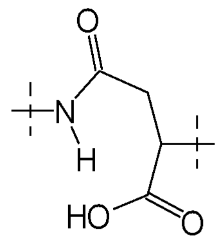 |

(fortgesetzt)

| Subs t. | m | L1 | L2 |
|---|---|---|---|
| $R^1$ | 1 | 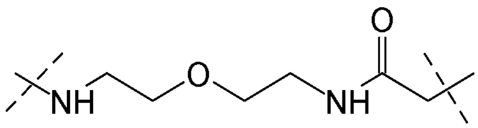 | 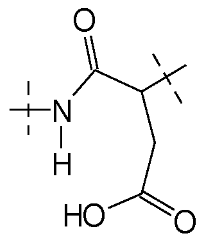 und 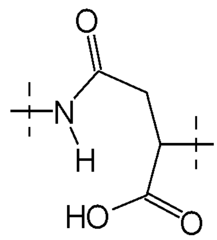 Siehe Anmerkung *** |
| $R^1$ | 1 | 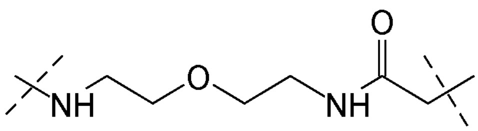 | 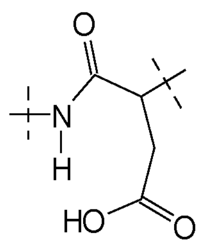 |
| $R^1$ | 1 | 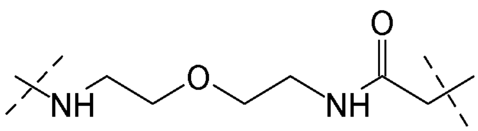 | 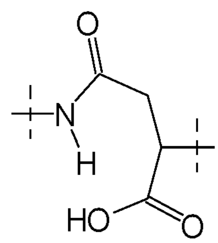 |
| R3 | 0 | 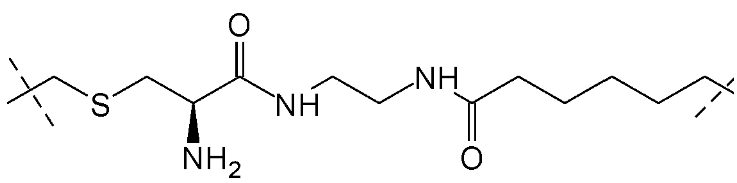 | 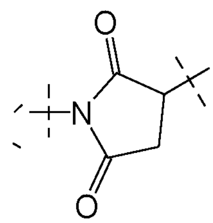 |

(fortgesetzt)

| Subs t. | m | L1 | L2 |
|---|---|---|---|
| R1 | 0 | 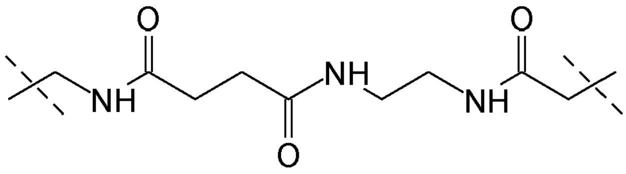 | 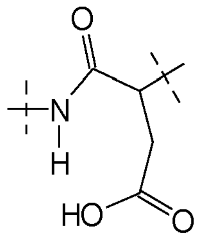 |
| | | | und 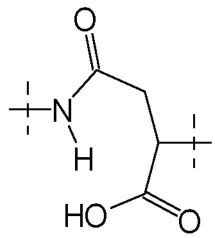 Siehe Anmerkung *** |
| R1 | 0 | 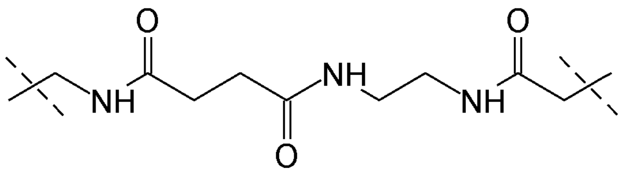 | 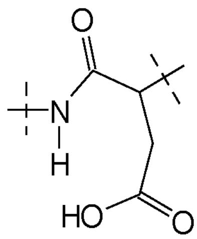 |
| R1 | 0 | 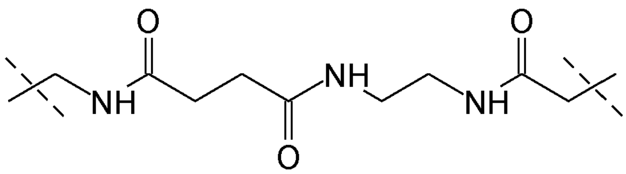 | 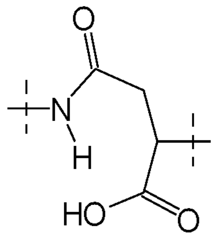 |
| R1 | 1 | 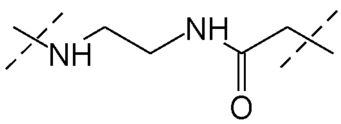 | 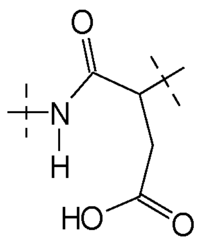 |

(fortgesetzt)

| Subs t. | m | L1 | L2 |
|---|---|---|---|
| | | | Und<br>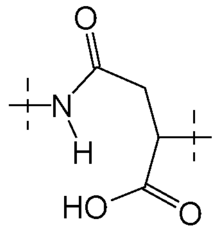 |
| R1 | 1 | 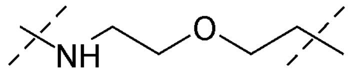 | 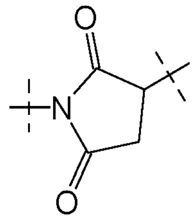 |
| R1 | 1 | 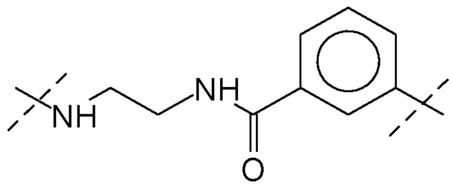 | 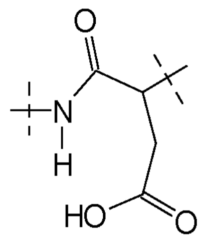<br>Und<br>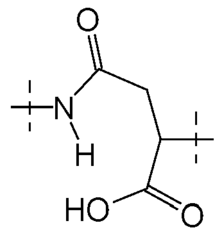 |
| $R^1$ | 1 | 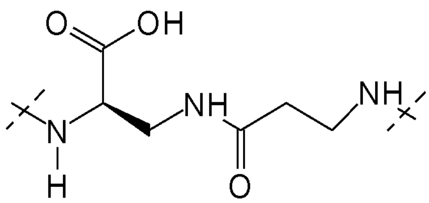 | 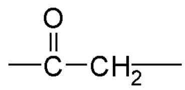 |
| $R^1$ | 1 | 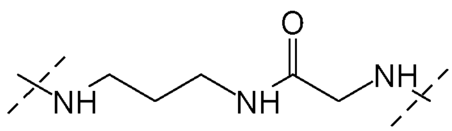 | 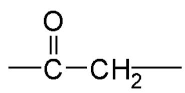 |

(fortgesetzt)

| Subs t. | m | L1 | L2 |
|---|---|---|---|
| $R^1$ | 1 | 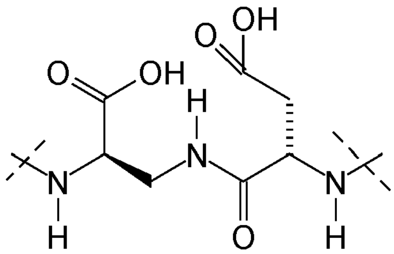 | 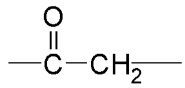 |
| $R^1$ | 1 | 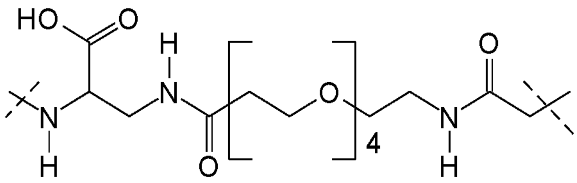 | 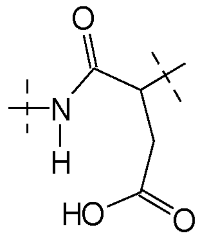 und 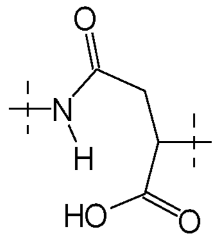 Siehe Anmerkung *** |
| $R^1$ | 1 | 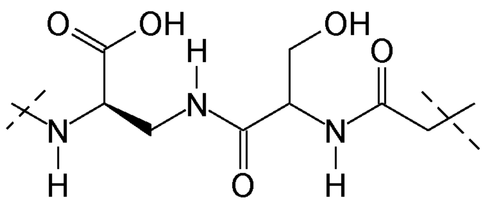 | 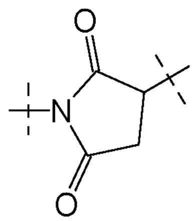 Siehe Anmerkung ** |

(fortgesetzt)

| Subs t. | m | L1 | L2 |
|---|---|---|---|
| $R^1$ | 1 | 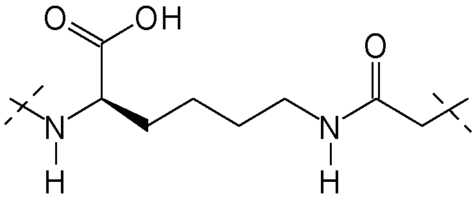 | 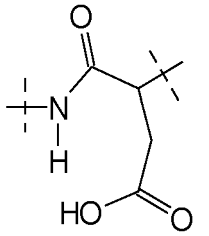 und 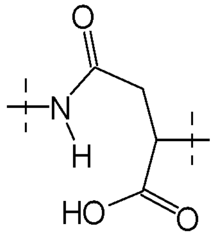 Siehe Anmerkung *** |
| $R^1$ | 1 | 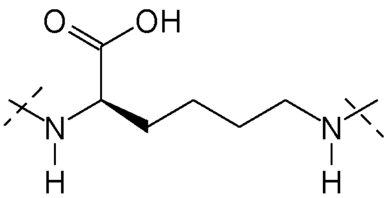 | 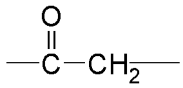 |

(fortgesetzt)

| Subs t. | m | L1 | L2 |
|---|---|---|---|
| $R^4$ | 0 | 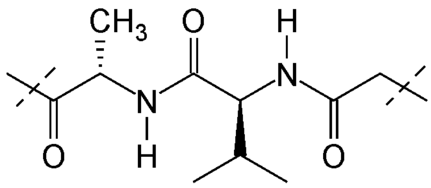 | 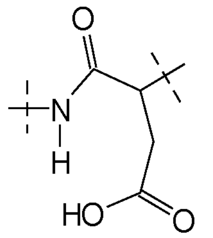<br>und<br>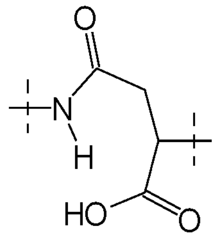<br>Siehe Anmerkung ***<br>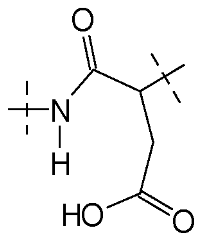<br>und<br>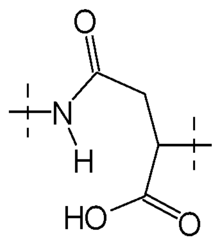<br>Siehe Anmerkung *** |

(fortgesetzt)

| Subs t. | m | L1 | L2 |
|---|---|---|---|
| $R^1$ | 1 | 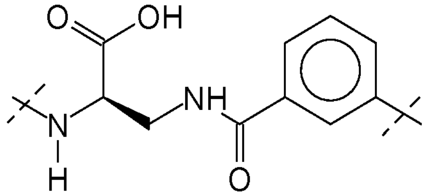 | 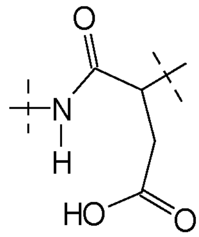 und 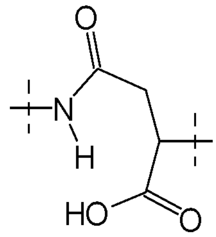 Siehe Anmerkung *** |
| $R^4$ | 0 | 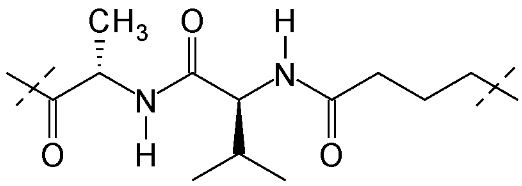 | 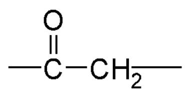 |

(fortgesetzt)

| Subs t. | m | L1 | L2 |
|---|---|---|---|
| $R^1$ | 1 | 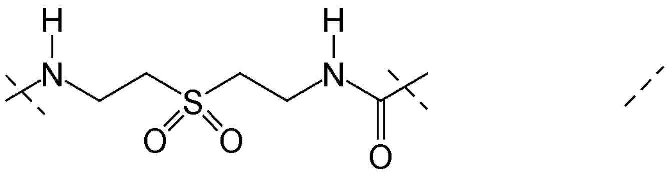 | 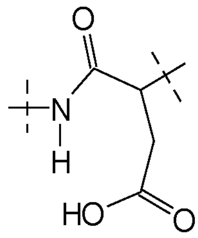 und 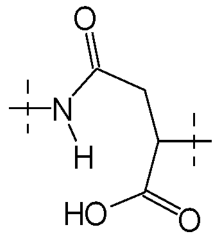 Siehe Anmerkung** |
| $R^3$ | 0 | 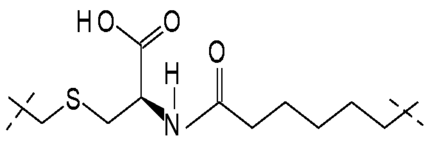 | 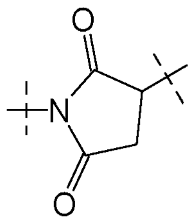 Siehe Anmerkung ** |

(fortgesetzt)

| Subs t. | m | L1 | L2 |
|---|---|---|---|
| $R^1$ | 1 | 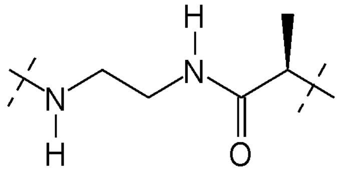 | 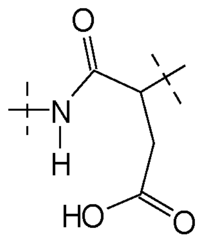<br>und<br>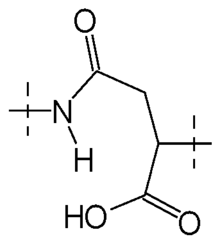<br>Siehe Anmerkung** |
| $R^3$ | 0 | 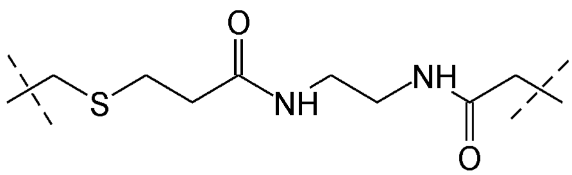 | 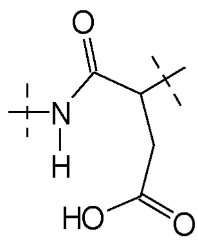<br>und<br>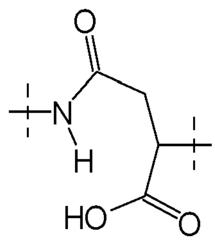<br>Siehe Anmerkung*** |

(fortgesetzt)

| Subs t. | m | L1 | L2 |
|---|---|---|---|
| $R^3$ | 0 | 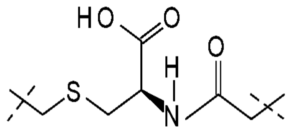 | 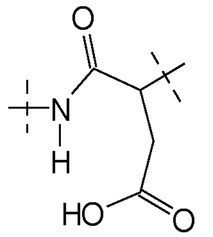<br>und<br>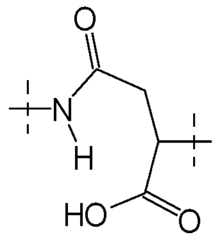<br>Siehe<br>Anmerkung *** |
| $R^3$ | 0 | 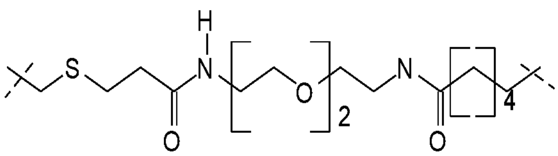 | 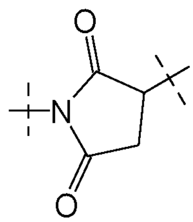<br>Siehe<br>Anmerkung ** |
| $R^3$ | 0 | 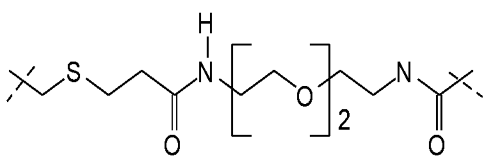 | 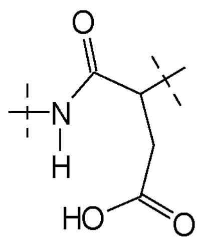 |

(fortgesetzt)

| Subs t. | m | L1 | L2 |
|---|---|---|---|
| | | | und 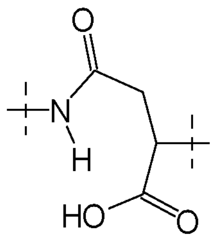 Siehe Anmerkung *** |
| R3 | 0 | 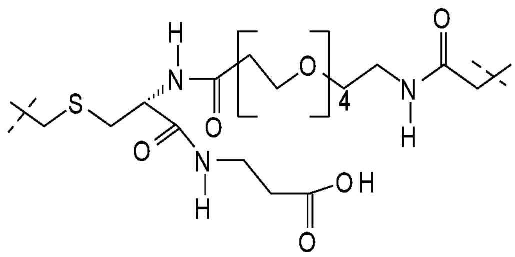 | 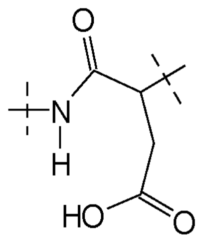 und 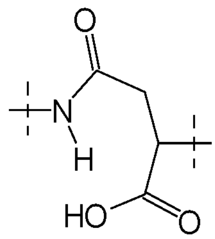 Siehe Anmerkung *** |
| R3 | 0 | 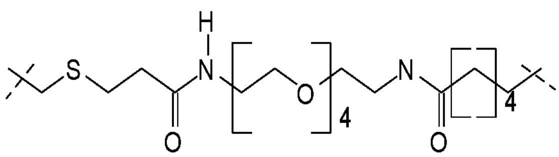 | 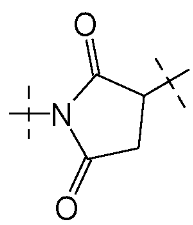 Siehe Anmerkung ** |

(fortgesetzt)

| Subs t. | m | L1 | L2 |
|---|---|---|---|
| $R^3$ | 0 | 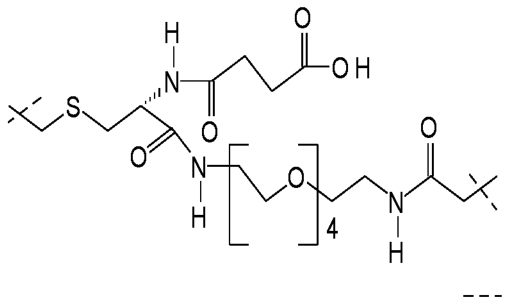 | 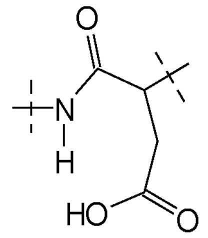<br>und<br>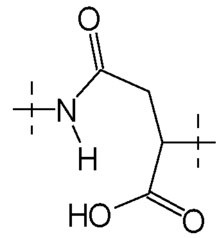<br>Siehe<br>Anmerkung *** |
| $R^3$ | 0 | 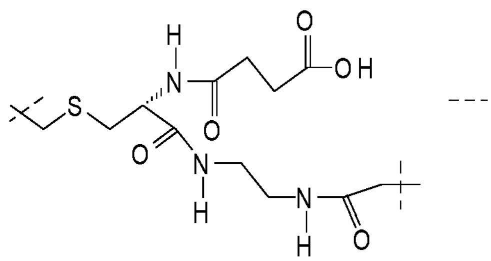 | 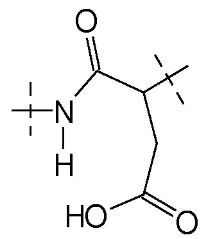<br>und<br>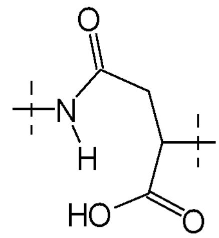<br>Siehe<br>Anmerkung *** |

(fortgesetzt)

| Subs t. | m | L1 | L2 |
|---|---|---|---|
| $R^3$ | 0 | 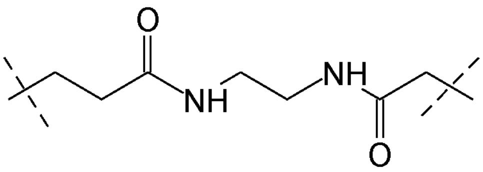 | 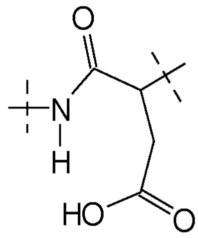 und 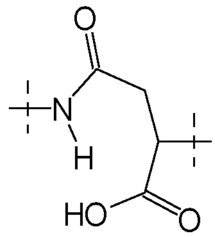 Siehe Anmerkung*** |
| $R^3$ | 0 | 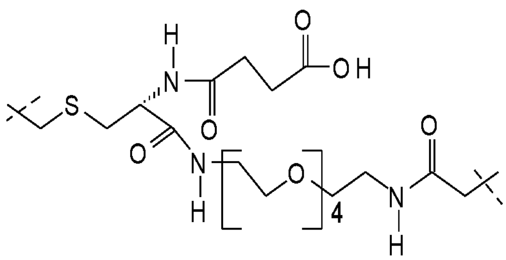 | 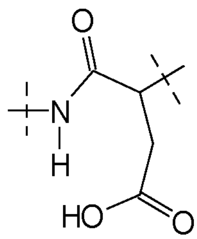 und 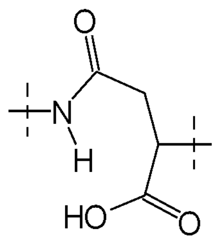 Siehe Anmerkung *** |

(fortgesetzt)

| Subs t. | m | L1 | L2 |
|---|---|---|---|
| $R^3$ | 0 | 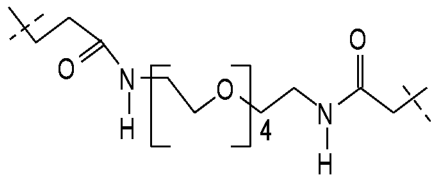 | 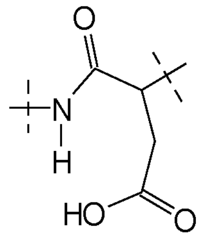 und 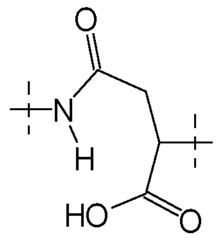 Siehe Anmerkung *** |
| $R^3$ | 0 | 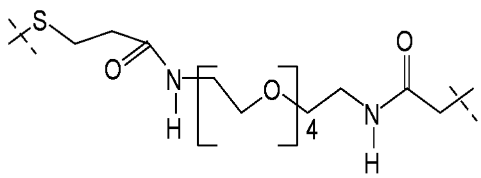 | 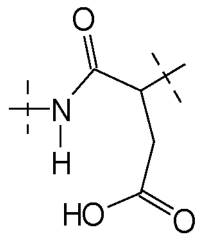 und 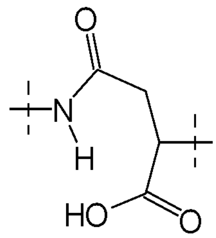 Siehe Anmerkung *** |

(fortgesetzt)

| Subs t. | m | L1 | L2 |
|---|---|---|---|
| $R^3$ | 0 | 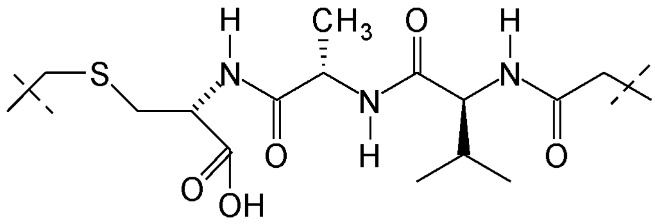 | 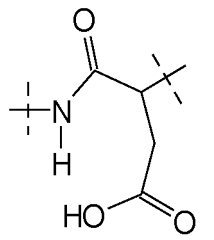 und 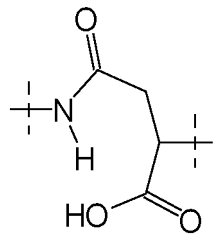 Siehe Anmerkung *** |
| $R^3$ | 0 | 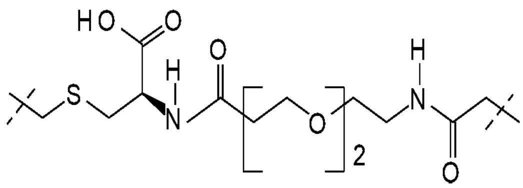 | 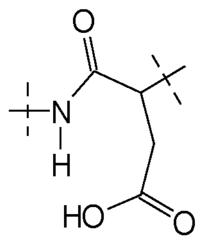 und 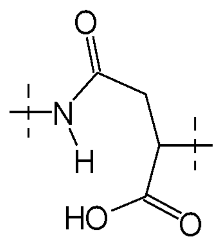 Siehe Anmerkung *** |

(fortgesetzt)

| Subs t. | m | L1 | L2 |
|---|---|---|---|
| $R^3$ | 0 | 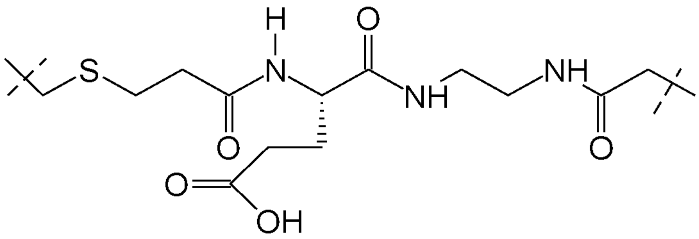 | 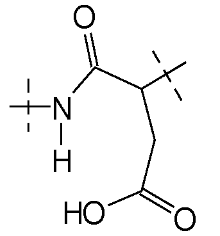 und 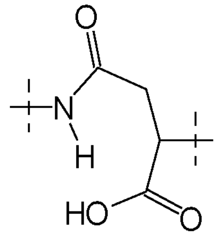 Siehe Anmerkung*** |
| $R^3$ | 0 | 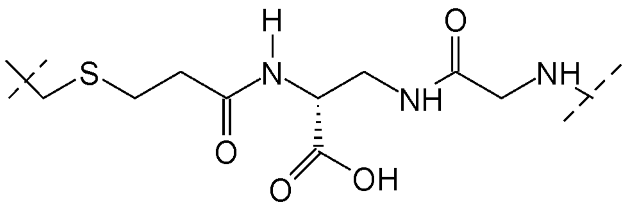 | 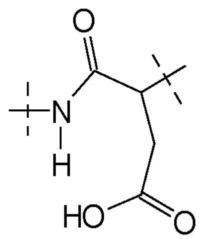 und 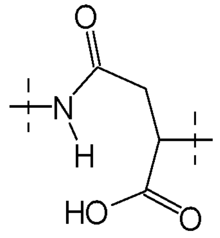 Siehe Anmerkung*** |
| $R^3$ | 0 | 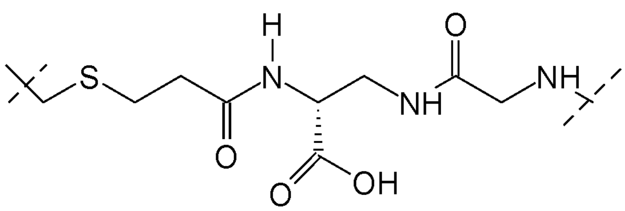 | 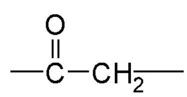 |

**: Siehe Anmerkung ** zu Tabelle A.

***: Bei Vorhandensein dieser Struktur L2 kann zugleich eine Struktur L2 der folgenden Formel vorliegen:

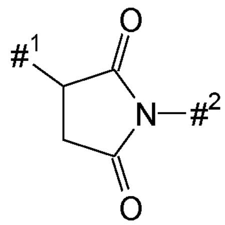

Beispiele für Konjugate mit entsprechenden Linkern weisen folgende Strukturen auf, wobei X1 CH, X2 C, und X3 N darstellt, und L1 die oben angegebenen Bedeutung aufweist, L2 und L3 dieselbe Bedeutung wie L1 hat, AK1 für einen -Antikörper steht, der über einen Cysteinrest gebunden ist, und n eine Zahl von 1 bis 10 ist.

Besonders bevorzugt ist AK1 ein Antikörper oder eine Antigen-bindendes hiervon. Der Antikörper ist vorzugsweise ein humaner, humanisierter oder chimärer monoklonaler Antikörper oder ein Antigen-bindendes Fragment hiervon, insbesondere ein anti-TWEAKR-Antikörper, ein anti-EGFR-Antikörper, ein anti-B7H3-Antikörper oder ein anti-HER2-Antikörper oder ein Antigen-bindendes Fragment von diesen. Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

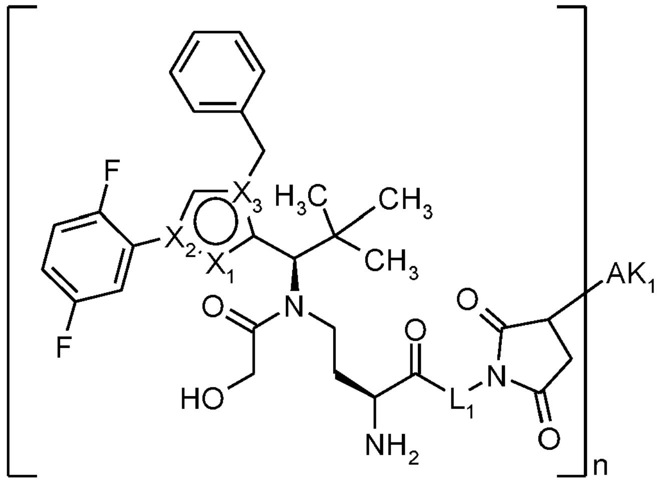

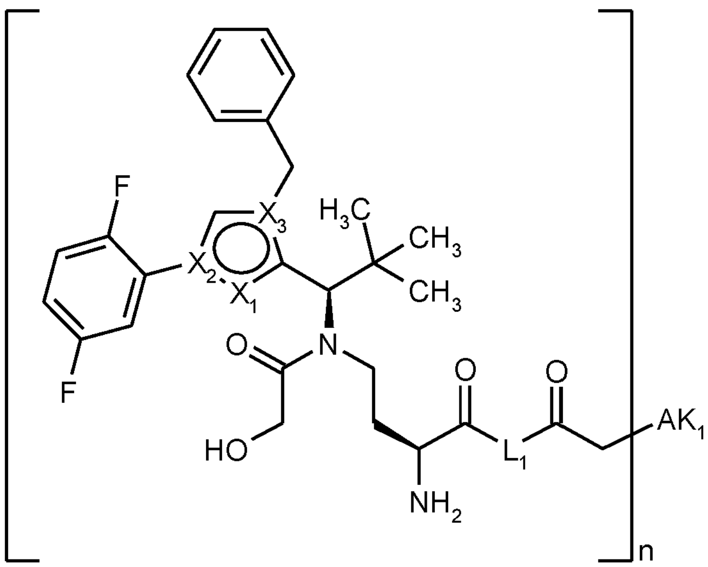

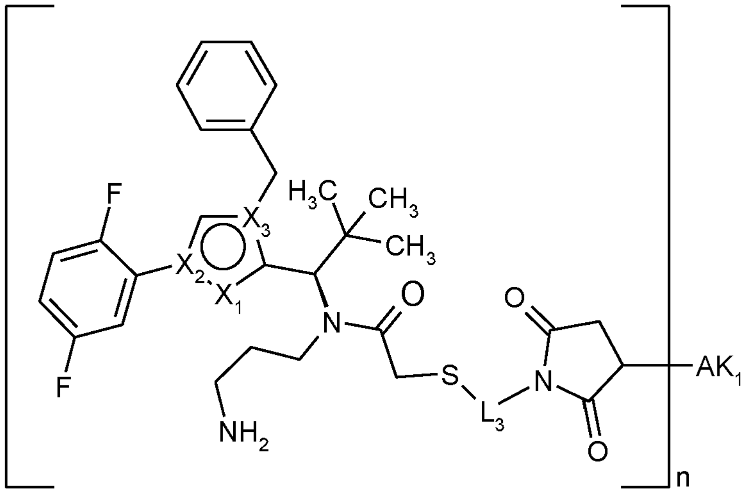

Hierbei steht

AK1 für einen anti-TWEAKR-Antikörper, einen anti-EGFR-Antikörper, einen anti-B7H3-Antikörper oder einen anti-HER2-Antikörper oder ein Antigen-bindendes Fragment von diesen und

n für eine Zahl von 1 bis 20.

Weiterhin bevorzugt ist die Grundstruktur (i), (ii) und (iv), der Linker

(i) $-(C{=}O)_m$-SG1-L1-L2-
(ii) $-(C{=}O)_m$ -L1-SG-L1-L2-
(iii) $-(C{=}O)_m$ -L1-SG-L2,

wobei SG1 bzw. SG eine durch Cathepsin spaltbare Gruppe darstellt, und L1 und L2 die in der Tabelle A' aufgeführten Bedeutungen aufweisen. Besonders bevorzugt sind die folgenden Gruppen:

- Val-Ala-C(=O)-NH- (hierdurch Spaltung der Amid-Bindung am C-terminalen Amid von Alanin)
- NH-Val-Lys-C(=O)-NH- (Spaltung der Amid-Bindung am C-terminalen Amid von Lysin)
- NH-Val-Cit-C(=O)-NH- (Spaltung der Amid-Bindung am C-terminalen Amid von Citrullin)
- NH-Phe-Lys-C(=O)-NH- (Spaltung der Amid-Bindung am C-terminalen Amid von Lysin)
- NH-Ala-Lys-C(=O)-NH- (Spaltung der Amid-Bindung am C-terminalen Amid von Lysin)
- NH-Ala-Cit-C(=O)-NH- (Spaltung der Amid-Bindung am C-terminalen Amid von Citrullin)

Hierbei sind SG1 bzw. SG gemäß den allgemeinen Formeln besonders bevorzugt

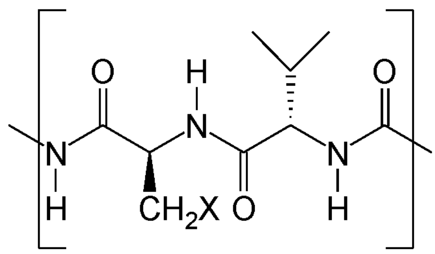

,

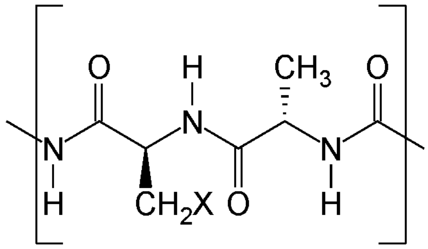

und

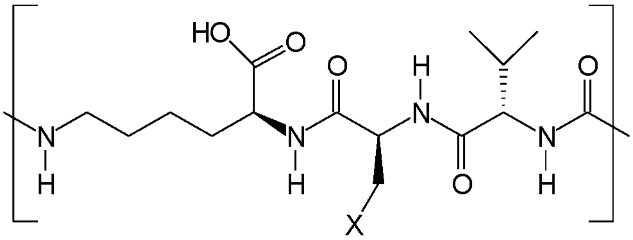

,

in denen

X für -H oder eine $C_{1-10}$-Alkylgruppe, die gegebenenfalls mit -NH-C(=O)-$NH_2$, -COOH, -OH, -$NH_2$, oder Sulfonsäure substituiert sein kann, steht.

In der folgenden Tabelle C sind Beispiele für einen Linkerteil -SG1-L1- bzw. -L1-SG-L1-angegeben, wobei SG1 bzw. SG eine durch Cathepsin spaltbare Gruppe ist. In der Tabelle C ist weiterhin angegeben, mit welcher Gruppe L2 diese Beispiele für -SG1-L1-bzw. -L1-SG-L1- vorzugsweise kombiniert werden, sowie die bevorzugte Ankopplungsstelle ($R^1$-$R^5$) sowie der bevorzugte Wert für m, also ob vor L1 eine Carbonylgruppe vorliegt oder nicht (vgl. §-(C(=O))m-L1-L2-§§). Diese Linker werden vorzugsweise an einen Cysteinrest gekoppelt. Die L1-Gruppe ist in einem Rahmen hervorgehoben. Diese Gruppen L1 können jedoch durch eine der Gruppe L1, die für die obige Formel §-(C(=O))m-L1-L2-§§ angegeben ist, ausgetauscht werden. Wenn L2 ein Bernsteinsäureamid ist bzw. sich hiervon ableitet, kann dieses Amid ganz oder teilweise auch in Form des hydrolysierten offenkettigen Bernsteinsäureamids vorliegen, wie oben beschrieben.

Tabelle C (Subst. = Substituent)

| Su bst | m | -SG1-L1- bzw. -L1-SG-L1- | L2 |
|---|---|---|---|
| $R^1$ | 1 | 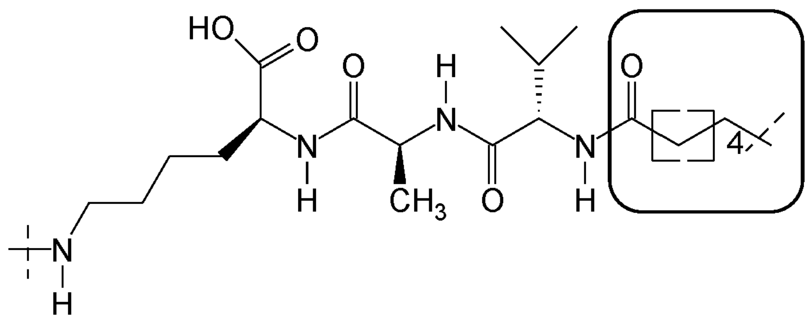 | 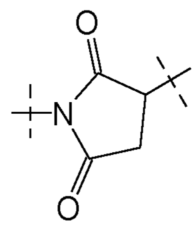 |
| $R^1$ | 1 | 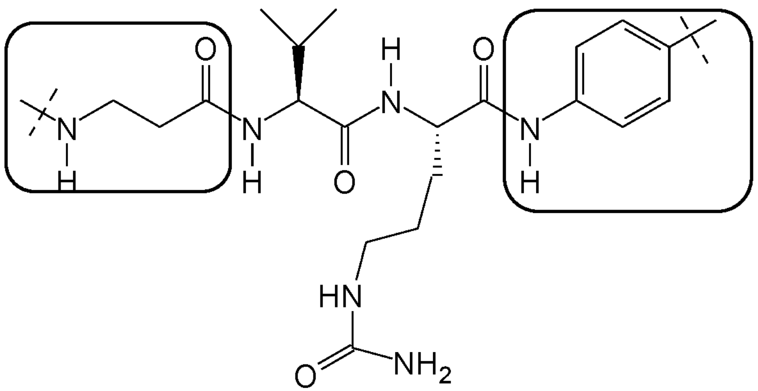 | 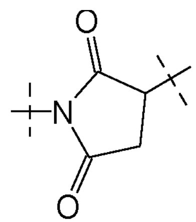 |

(fortgesetzt)

| Su bst | m | -SG1-L1- bzw. -L1-SG-L1- | L2 |
|---|---|---|---|
| $R^1$ | 1 | 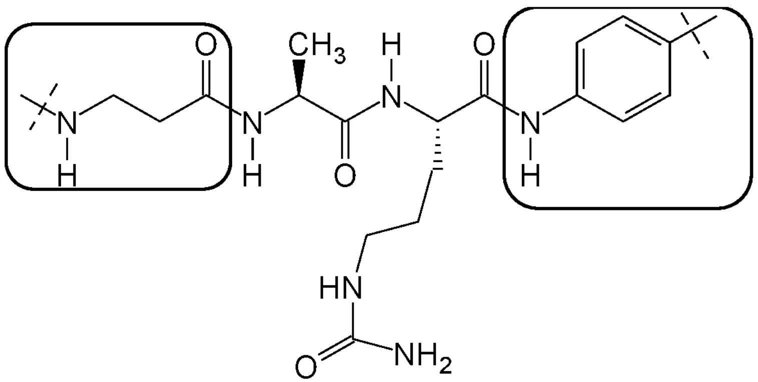 | 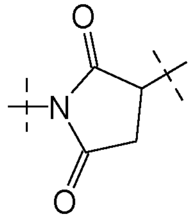 |
| $R^1$ | 1 | 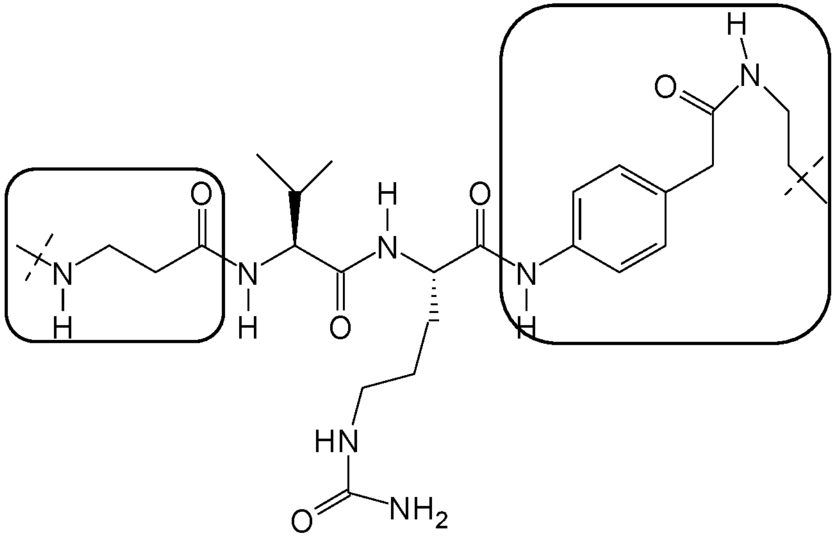 | 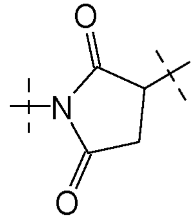 |

(fortgesetzt)

| Su bst | m | -SG1-L1- bzw. -L1-SG-L1- | L2 |
|---|---|---|---|
| $R^1$ | 1 | 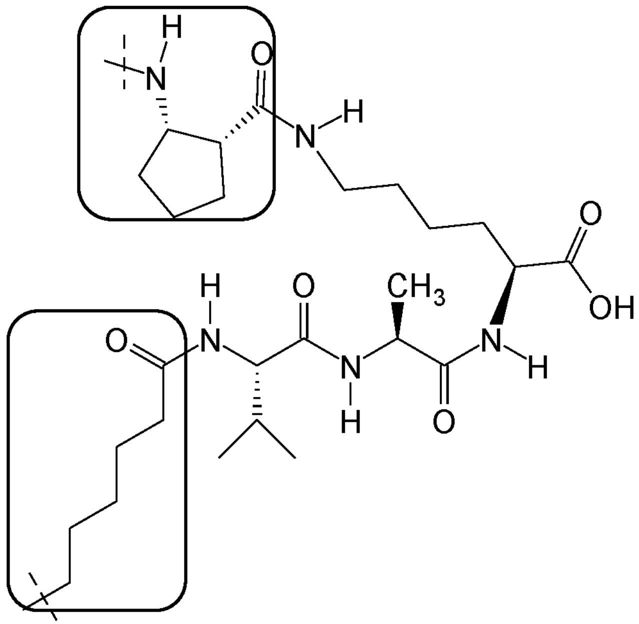 | 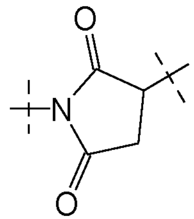 |

(fortgesetzt)

| Su bst | m | -SG1-L1- bzw. -L1-SG-L1- | L2 |
|---|---|---|---|
| $R^1$ | 1 | 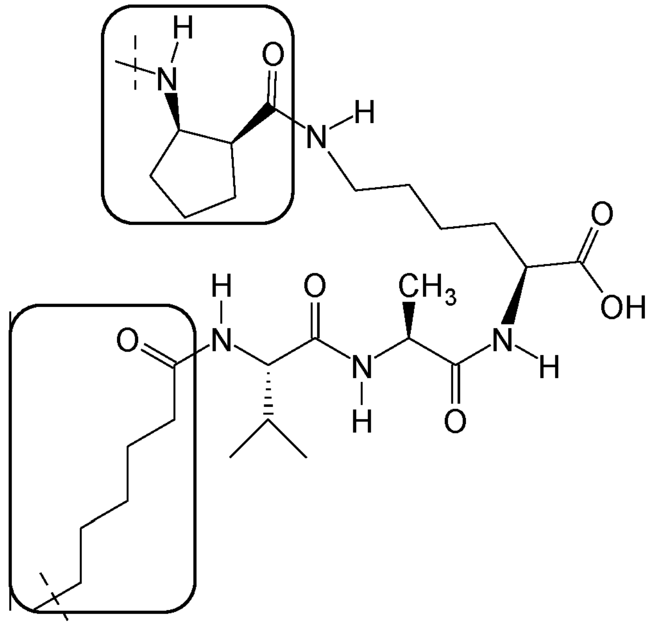 | 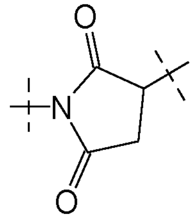 |
| $R^1$ | 1 | 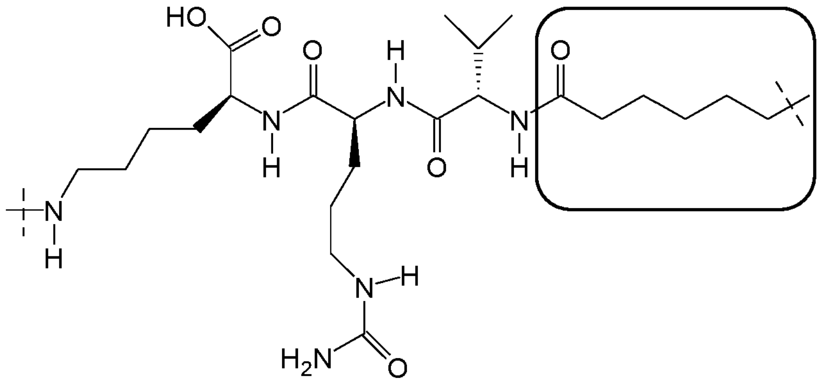 | 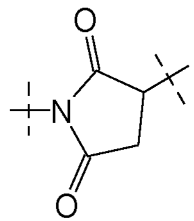 |

(fortgesetzt)

| Su bst | m | -SG1-L1- bzw. -L1-SG-L1- | L2 |
| --- | --- | --- | --- |
| $R^1$ | 1 | 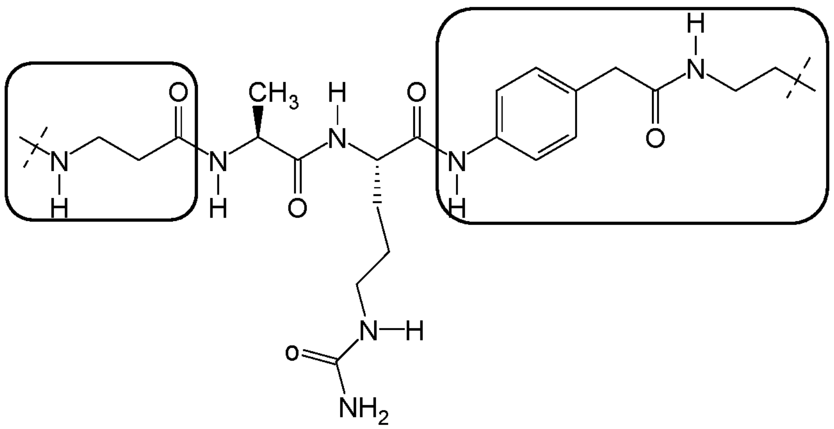 | 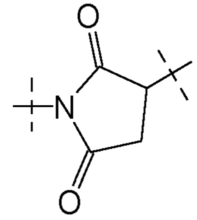 |
| $R^1$ | 1 | 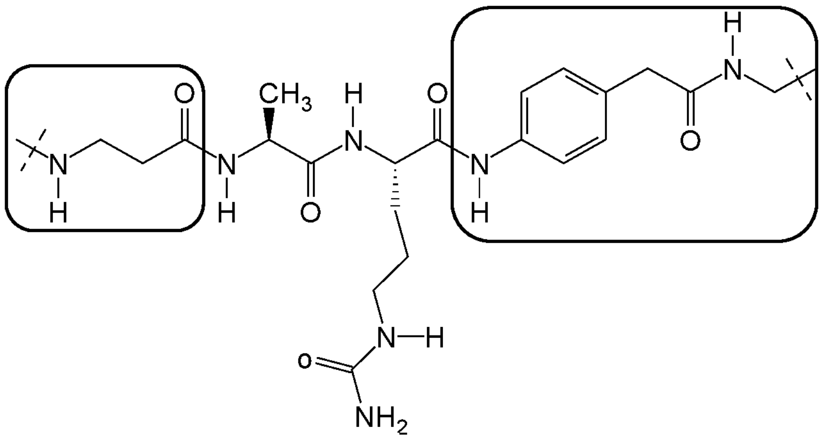 | 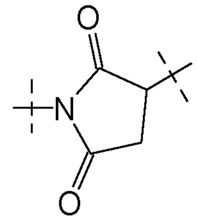 |

(fortgesetzt)

| Su bst | m | -SG1-L1- bzw. -L1-SG-L1- | L2 |
|---|---|---|---|
| $R^1$ | 1 | 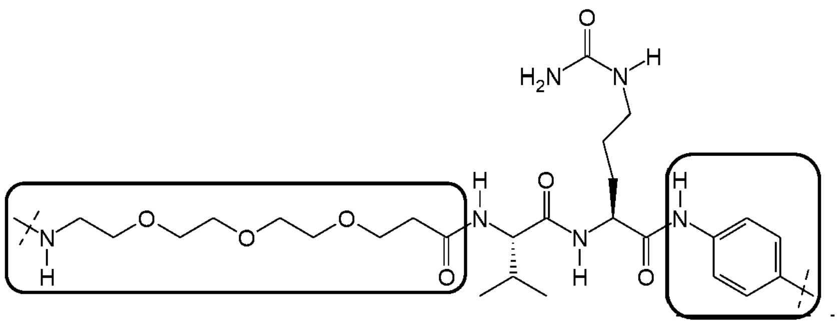 | 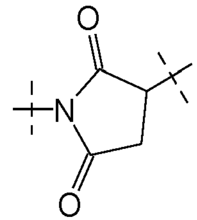 |
| $R^1$ | 1 | 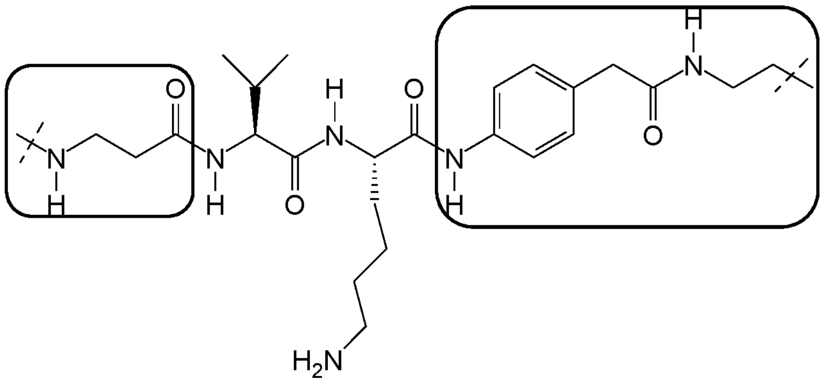 | 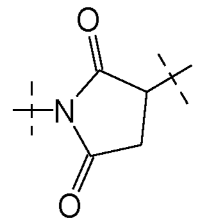 |

(fortgesetzt)

| Su bst | m | -SG1-L1- bzw. -L1-SG-L1- | L2 |
|---|---|---|---|
| $R^1$ | 1 | 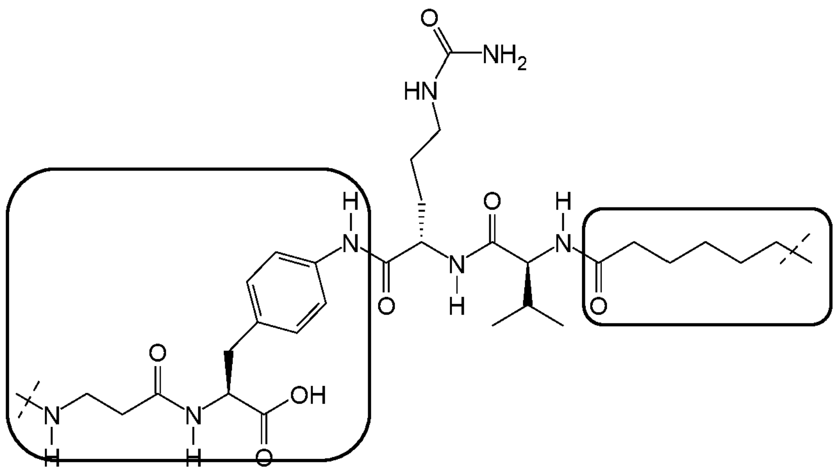 | 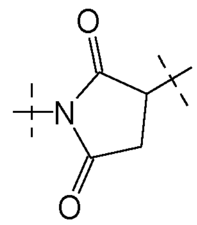 |
| $R^1$ | 1 | 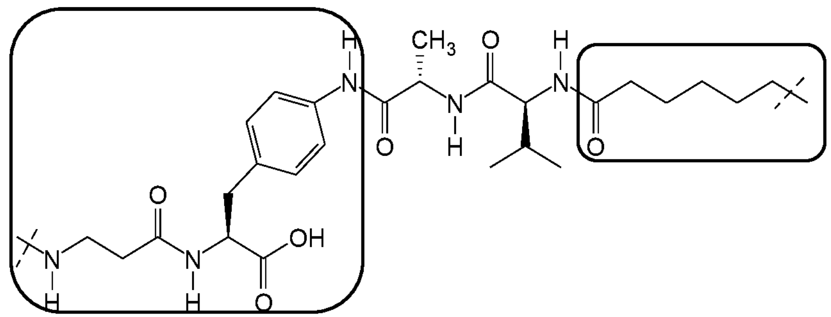 | 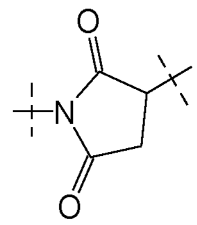 |
| $R^1$ | 0 | 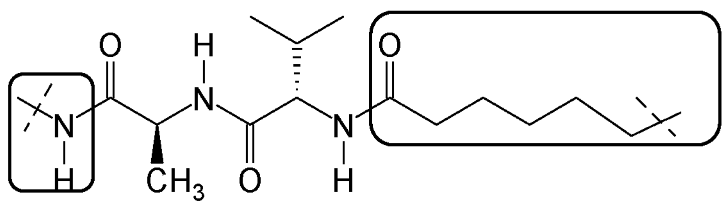 | 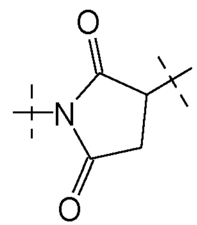 |

(fortgesetzt)

| Su bst | m | -SG1-L1- bzw. -L1-SG-L1- | L2 |
|---|---|---|---|
| $R^1$ | 1 | 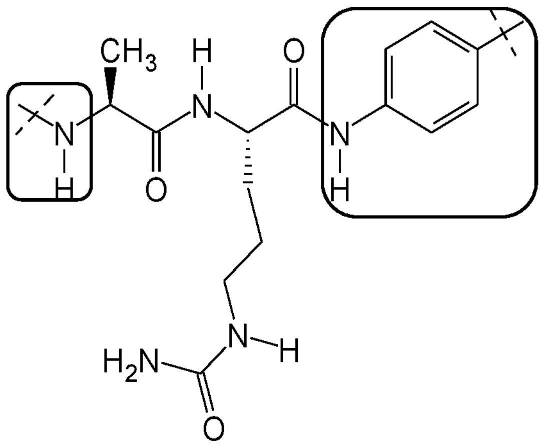 | 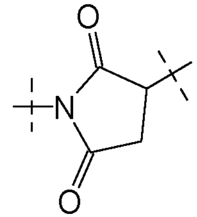 |
| $R^1$ | 0 | 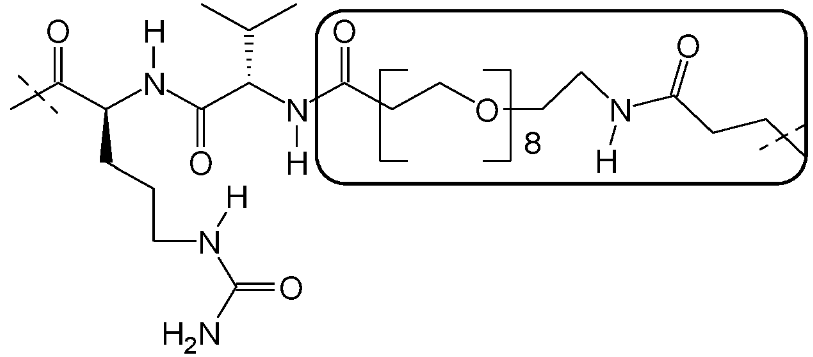 | 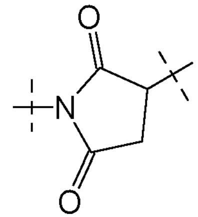 |
| $R^1$ | 0 | 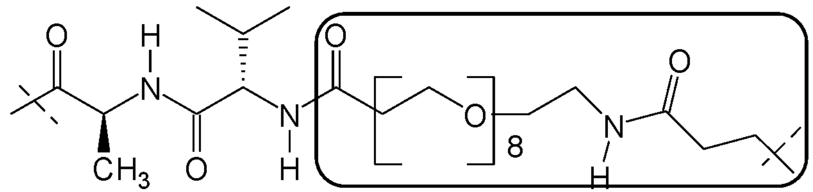 | 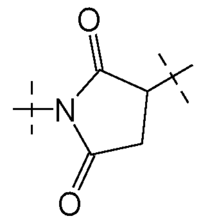 |

(fortgesetzt)

| Su bst | m | -SG1-L1- bzw. -L1-SG-L1- | L2 |
|---|---|---|---|
| $R^1$ | 0 | 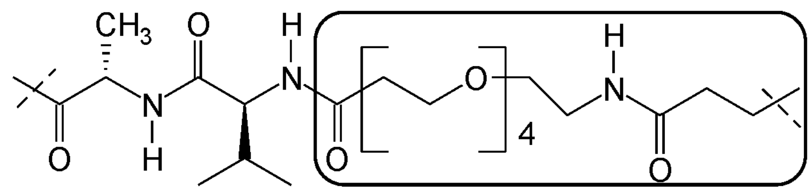 | 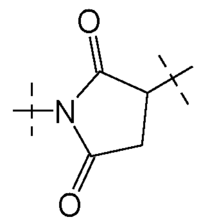 |
| $R^1$ | 0 | 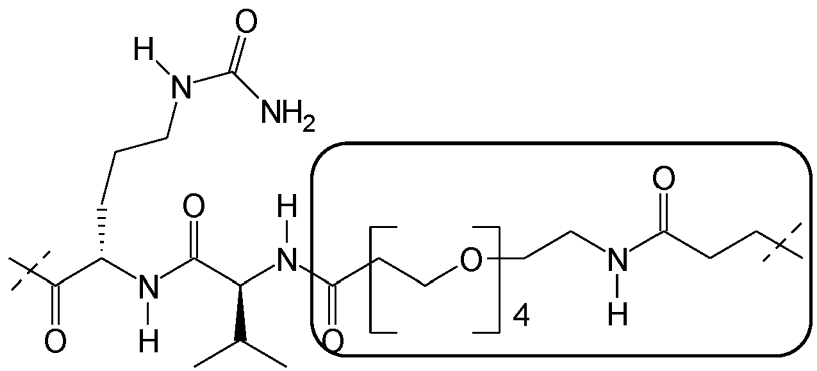 | 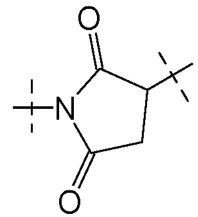 |
| $R^1$ | 0 | 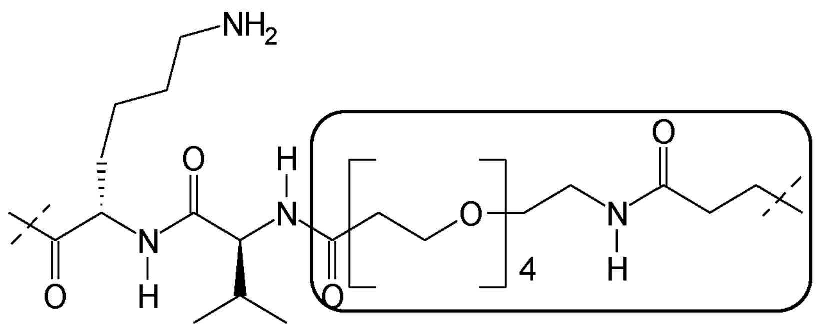 | 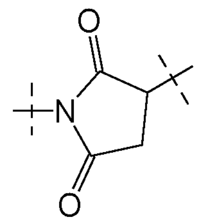 |

(fortgesetzt)

| Su bst | m | -SG1-L1- bzw. -L1-SG-L1- | L2 |
|---|---|---|---|
| $R^1$ | 0 | 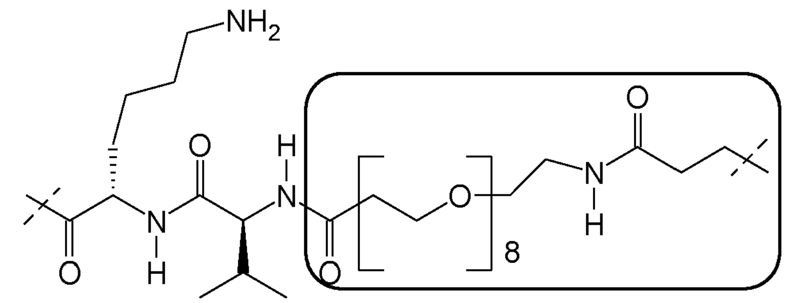 | 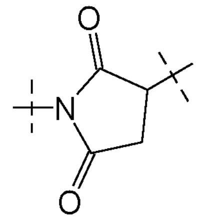 |
| $R^1$ | 0 | 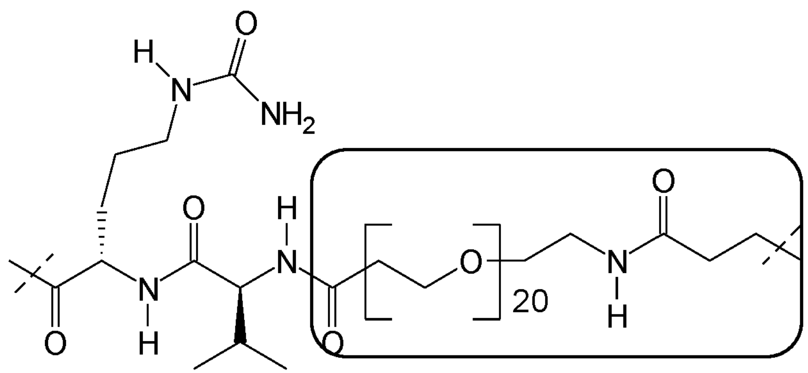 | 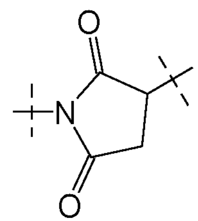 |
| $R^3$ | 0 | 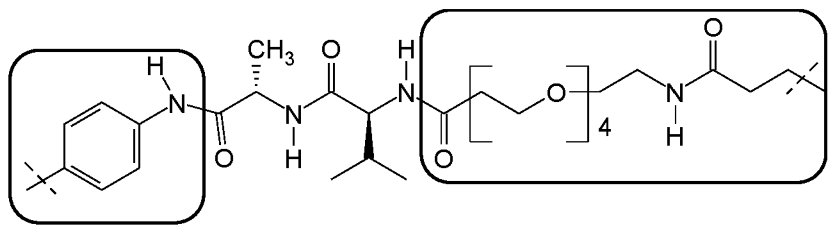 | 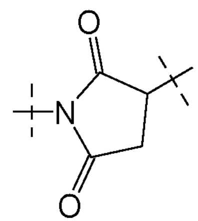 |

(fortgesetzt)

| Su bst | m | -SG1-L1- bzw. -L1-SG-L1- | L2 |
|---|---|---|---|
| $R^3$ | 0 | 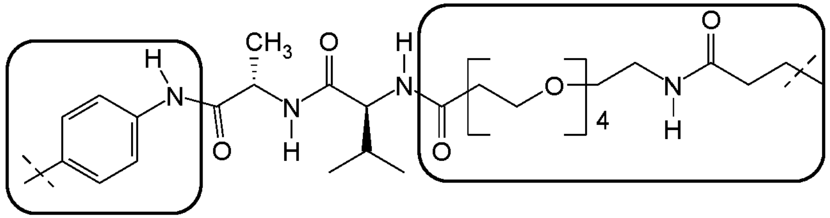 | 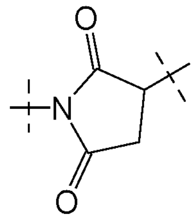 |
| $R^1$ | 1 | 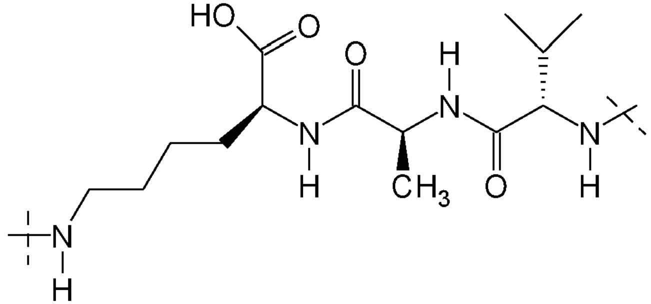 | 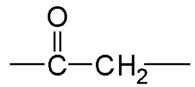 |
| $R^1$ | 1 | 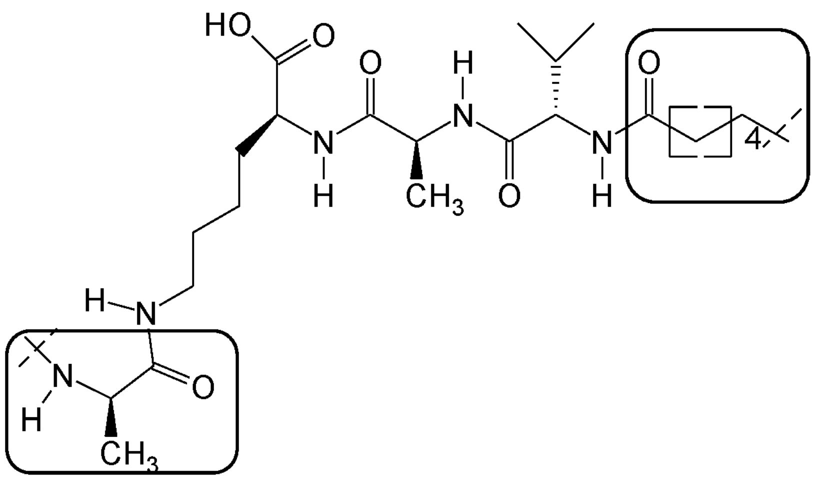 | 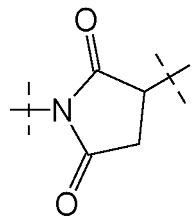 |

(fortgesetzt)

| Su bst | m | -SG1-L1- bzw. -L1-SG-L1- | L2 |
|---|---|---|---|
| $R^1$ | 1 | 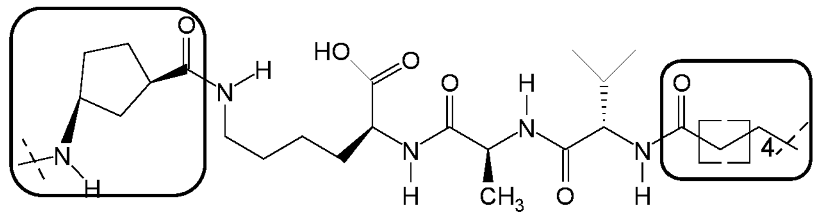 | 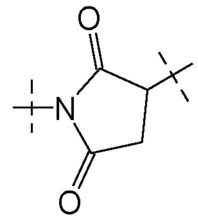 |
| $R^3$ | 0 | 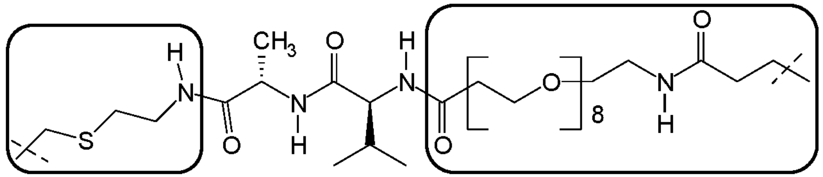 | 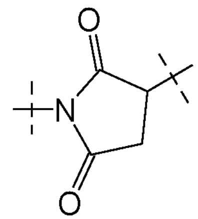 |
| $R^1$ | 1 | 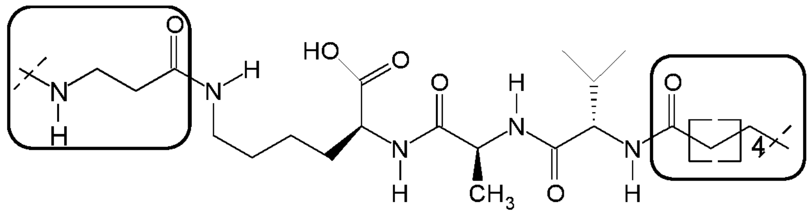 | 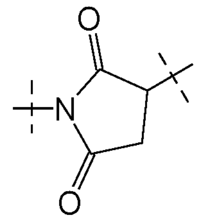 |

(fortgesetzt)

| Su bst | m | -SG1-L1- bzw. -L1-SG-L1- | L2 |
|---|---|---|---|
| $R^1$ | 1 | 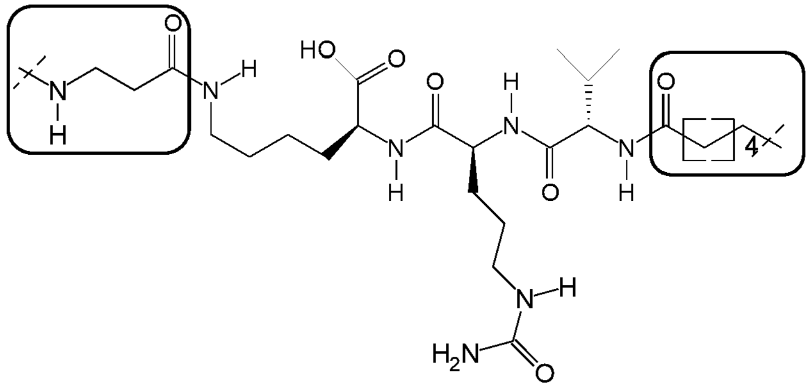 | 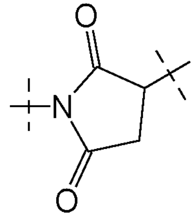 |
| $R^1$ | 1 | 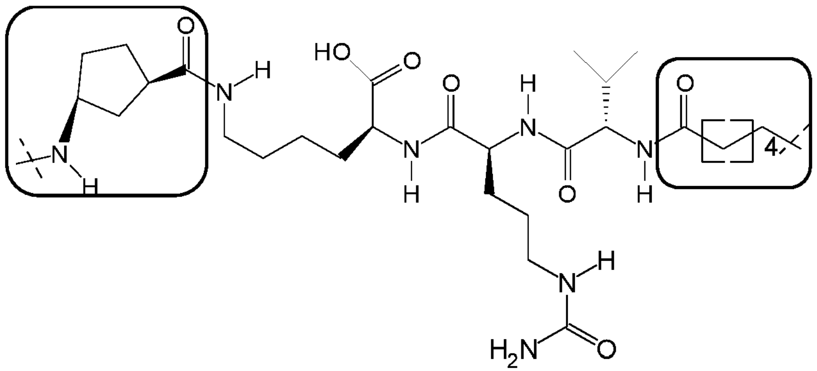 | 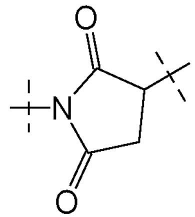 |

(fortgesetzt)

| Su bst | m | -SG1-L1- bzw. -L1-SG-L1- | L2 |
| --- | --- | --- | --- |
| $R^1$ | 1 | 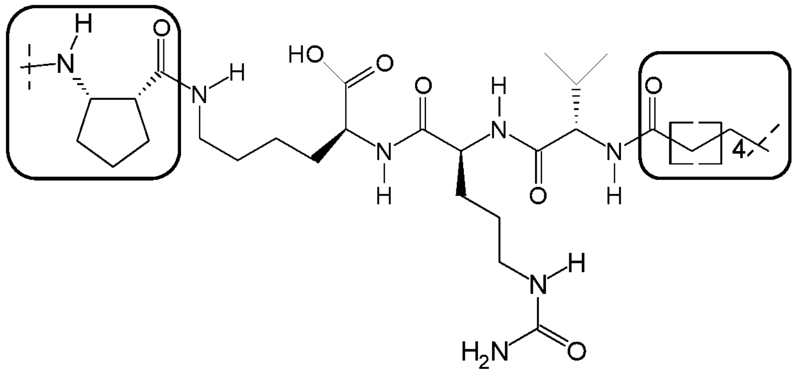 | 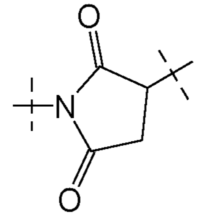 |
| $R^1$ | 1 | 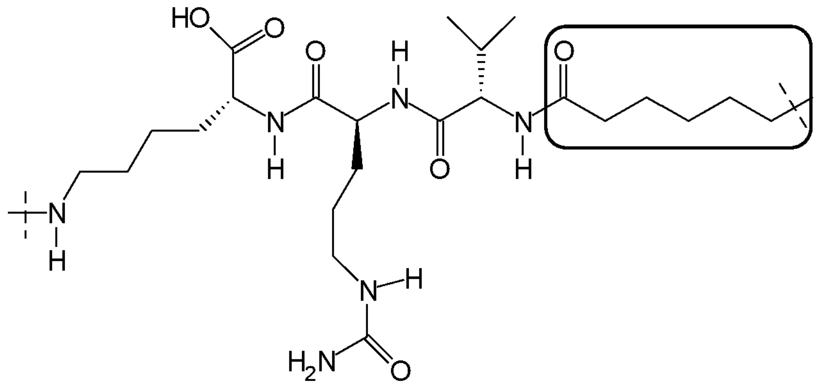 | 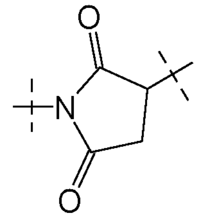 |

(fortgesetzt)

| Su bst | m | -SG1-L1- bzw. -L1-SG-L1- | L2 |
|---|---|---|---|
| $R^1$ | 1 | 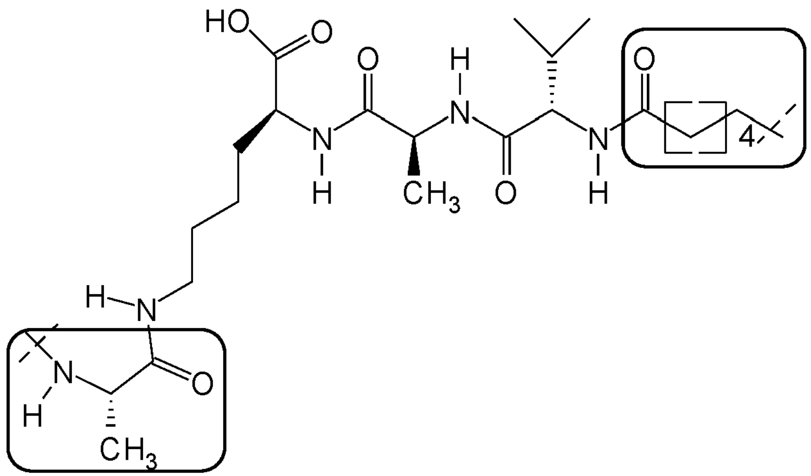 | 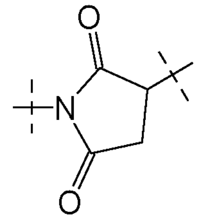 |
| $R^1$ | 1 | 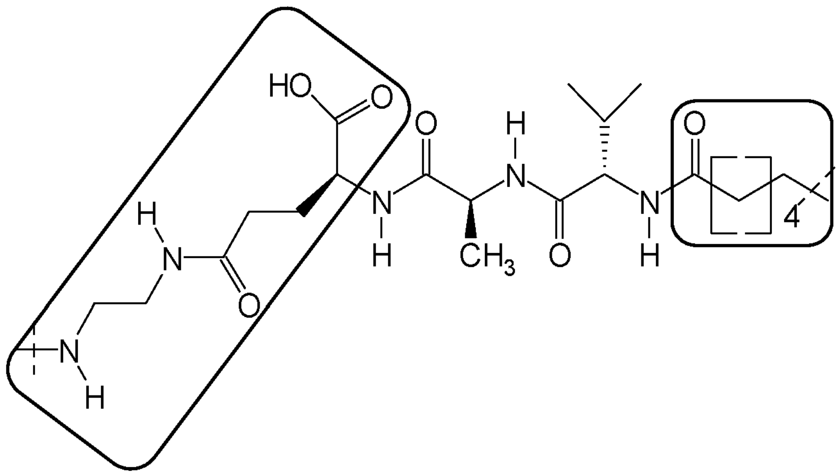 | 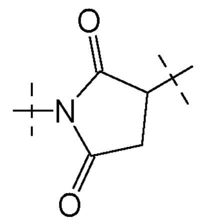 |

(fortgesetzt)

| Su bst | m | -SG1-L1- bzw. -L1-SG-L1- | L2 |
|---|---|---|---|
| $R^1$ | 1 | 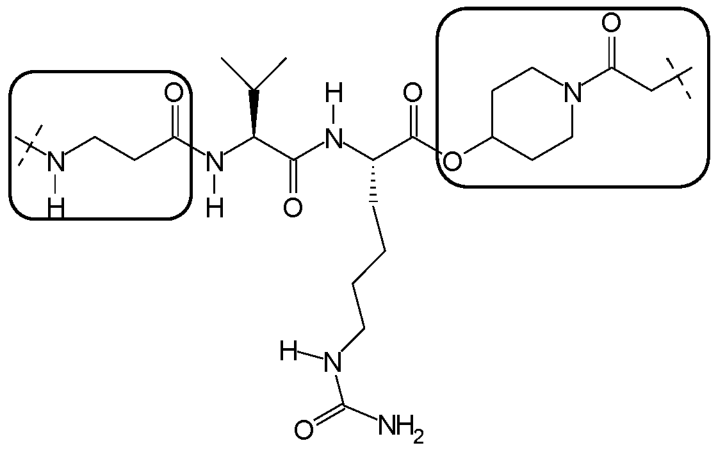 | 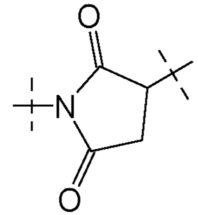 |
| $R^1$ | 1 | 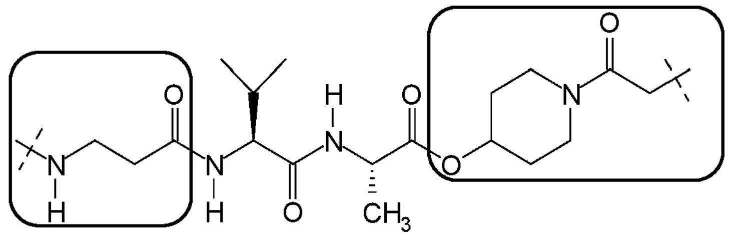 | 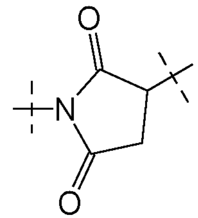 |
| $R^1$ | 1 | 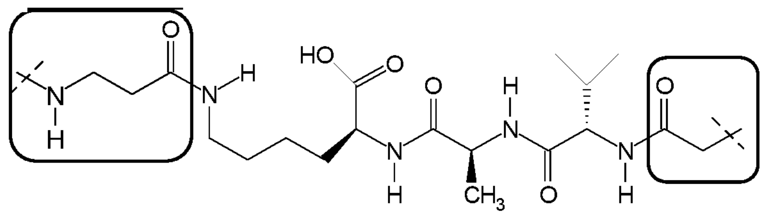 | 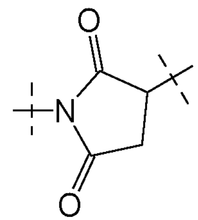 |

(fortgesetzt)

| Su bst | m | -SG1-L1- bzw. -L1-SG-L1- | L2 |
|---|---|---|---|
| $R^3$ | 0 | 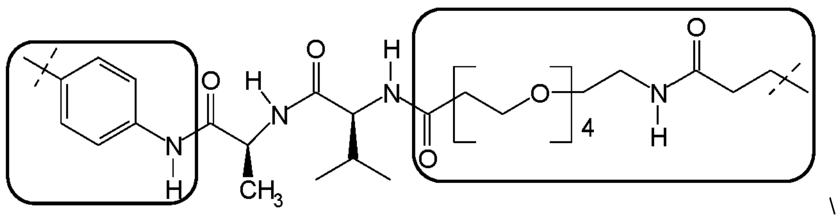 | 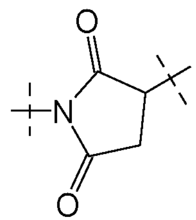 |

Beispiele für Konjugate mit der Grundstruktur (i) des Linkers

(i) $-(C{=}O)_m$-SG1-L1-L2-,

in der m, SG1, L1 und L2 die in der Tabelle C angegebenen Bedeutungen haben, weisen eine der folgenden Strukturen auf

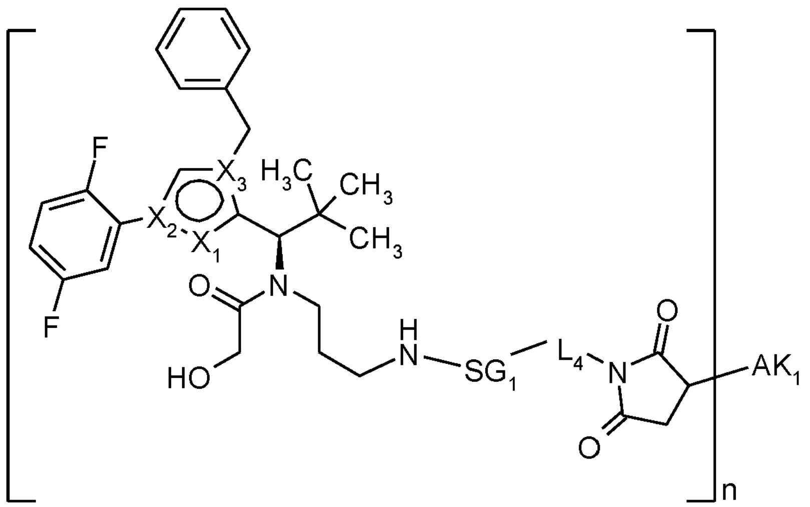

in denen

X1 für CH steht,
X2 für C steht,
X3 für N steht,
L4 dieselbe Bedeutung wie oben in der Tabelle C für L1 angegeben hat,
AK1 für einen anti-TWEAKR-Antikörper, einen anti-EGFR-Antikörper, einen anti-B7H3-Antikörper oder einen anti-HER2-Antikörper oder ein Antigen-bindendes Fragment von diesen steht, der über einen Cysteinrest gebunden ist,

und n eine Zahl von 1 bis 20 ist, und das Wasserstoffatom in Position $R^4$ gemäß Formel IIa (d.h. in der Gruppe $-NH_2$) durch die erfindungsgemäß verwendete, durch Legumain spaltbare Gruppe der Formel la ersetzt ist.

Besonders bevorzugt ist AK1 ein Antikörper oder eine Antigen-bindendes hiervon. Der Antikörper ist vorzugsweise ein humaner, humanisierter oder chimärer monoklonaler Antikörper oder ein Antigen-bindendes Fragment hiervon, insbesondere ein anti-TWEAKR-Antikörper, ein anti-EGFR-Antikörper, ein anti-B7H3-Antikörper oder ein anti-HER2-Antikörper oder ein Antigen-bindendes Fragment von diesen. Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Im Falle der Transglutaminase-katalysierten Konjugation offenbart die Literatur verschiedene Möglichkeiten der kovalenten Kopplung (Konjugation) von organischen Molekülen an Binder wie z.B. Antikörper in einer konjugationsstellenspezifischen Art und Weise, (siehe zum Beispiel (Sochaj et al., Biotechnology Advances, 33, 775-784, (2015), Panowski et al., MAbs 6, 34-45 (2014)). Erfindungsgemäß bevorzugt ist die Konjugation der KSP-Inhibitoren bzw. Prodrugs an einen Antikörper über Akzeptor-Glutaminreste des Antikörpers unter Verwendung von Transglutaminase. Solche Akzeptorglutaminreste können über Engineering des Antikörpers oder durch Mutationen generiert werden, die aglykosylierte Antikörper erzeugen. Die Anzahl dieser Akzeptorglutamine im Antikörper ist bevorzugt 2 oder 4. Für die Kopplung (Konjugation) werden geeignete Linker verwendet. Geeignete Linkerstrukturen sind solche, die über eine über eine freie Amin-Donor-Funktionalität verfügen, die ein geeignetes Substrat für die Transglutaminase darstellt. Die Verknüpfung des Linkers an den Antikörper kann dabei auf vielfältige Weise erfolgen.

Vorzugsweise weist bei einer Transglutaminase-katalysierten Konjugation der Linker eine der bereits oben genannten Grundstrukturen (i) bis (iv)

(i) $-(C{=}O)_m$-SG1-L1-L2-
(ii) $-(C{=}O)_m$ -L1-SG-L1-L2-

(iii) -$(C=O)_m$ -L1-L2-
(iv) -$(C=O)_m$ -L1-SG-L2

auf, in denen
L1, SG, SG1 und m die oben genannten Bedeutungen haben,
**[0386]** L2 jedoch vorzugsweise eine der folgenden Gruppen darstellt:

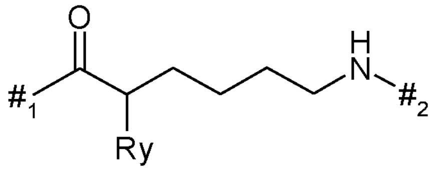

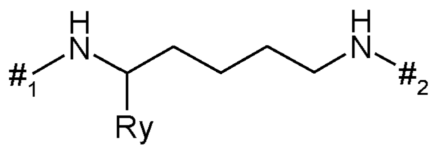

in denen

Ry für -H, -C(=O)-NH-alkyl, -NH-C(=O)-alkyl, -C(=O)-$NH_2$ oder -$NH_2$ steht,

$\#^1$ für den Verknüpfungspunkt mit $L^1$ steht und

$\#^2$ für den Verknüpfungspunkt mit dem Glutaminrest des Binders steht.

Vorzugsweise steht hierbei Ry für -H oder -NH-C(=O)-$CH_3$.
Beispiele entsprechender Konjugate weisen die folgenden Strukturen auf, wobei X1, X2, X3, Ry und L1 die oben angegebenen Bedeutungen haben, AK für einen Binder steht, der vorzugsweise ein Antikörper ist und n vorzugsweise 2 oder 4 ist.

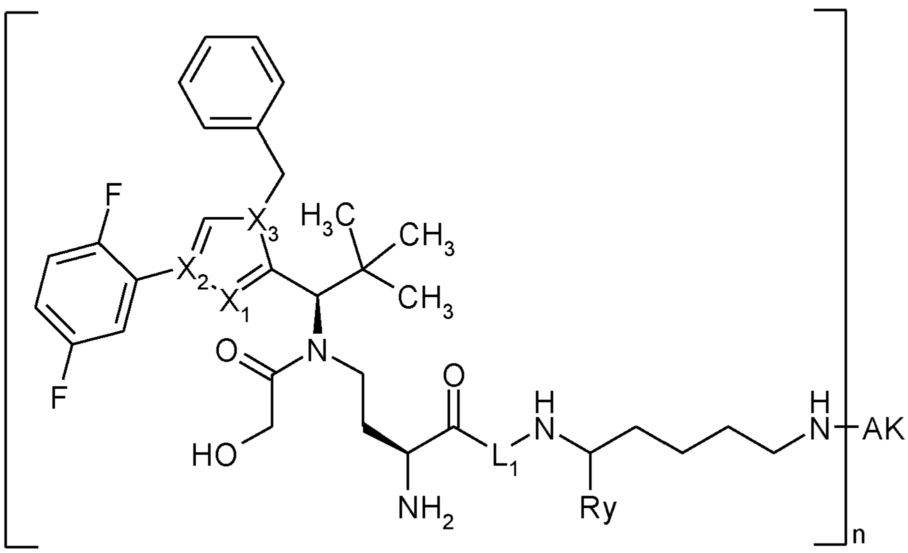

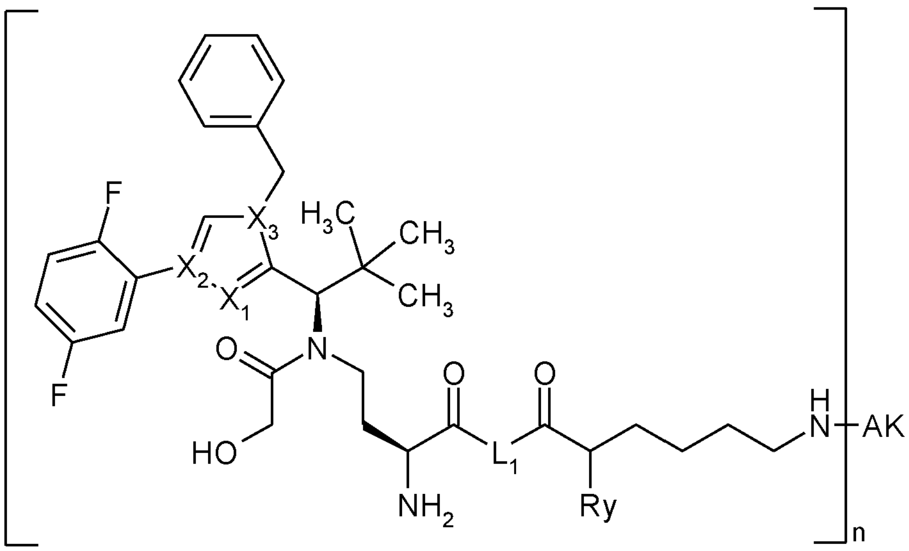

## Besonders bevorzugte KSP-Inhibitor-Konjugate

Als Binder oder Antikörper werden hierbei vorzugswiese die in der Beschreibung als bevorzugt beschriebenen Binder bzw. Antikörper verwendet.

Insbesonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Besonders bevorzugte Konjugate sind:

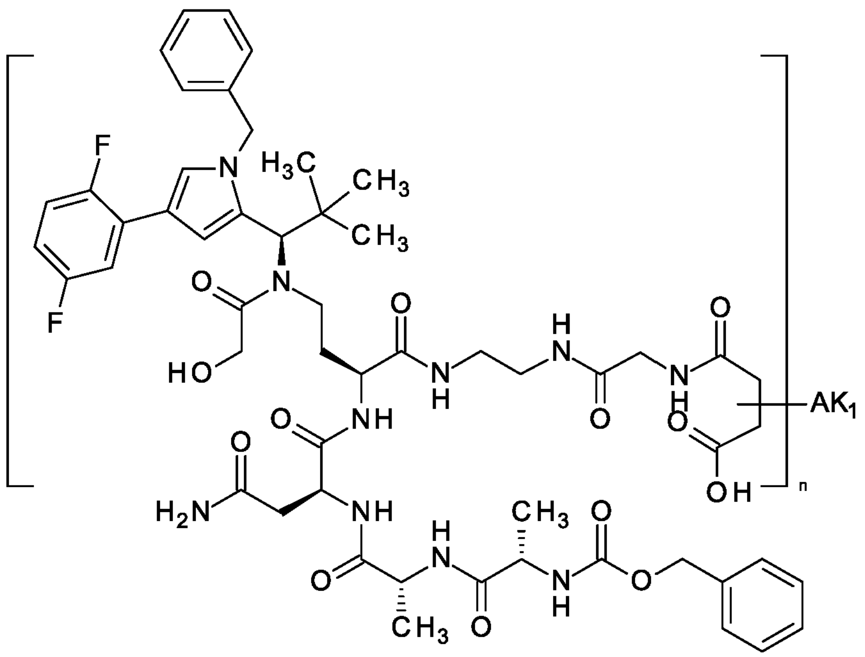

,

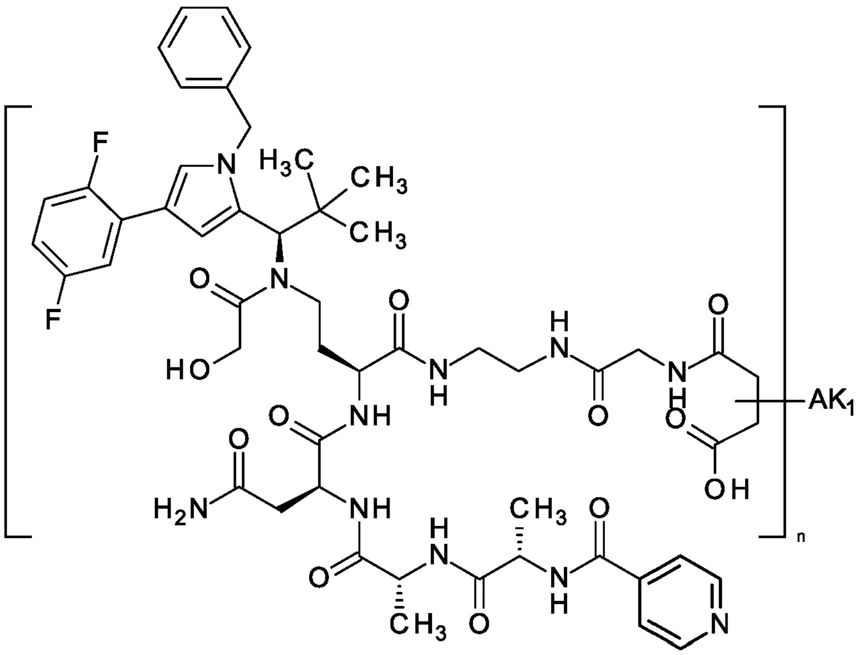

,

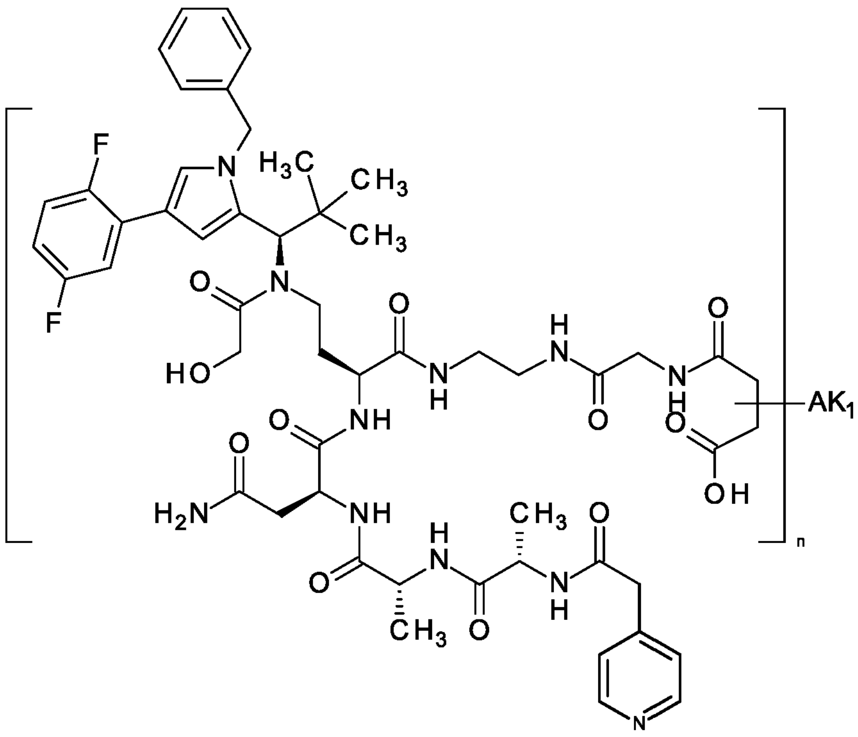

,

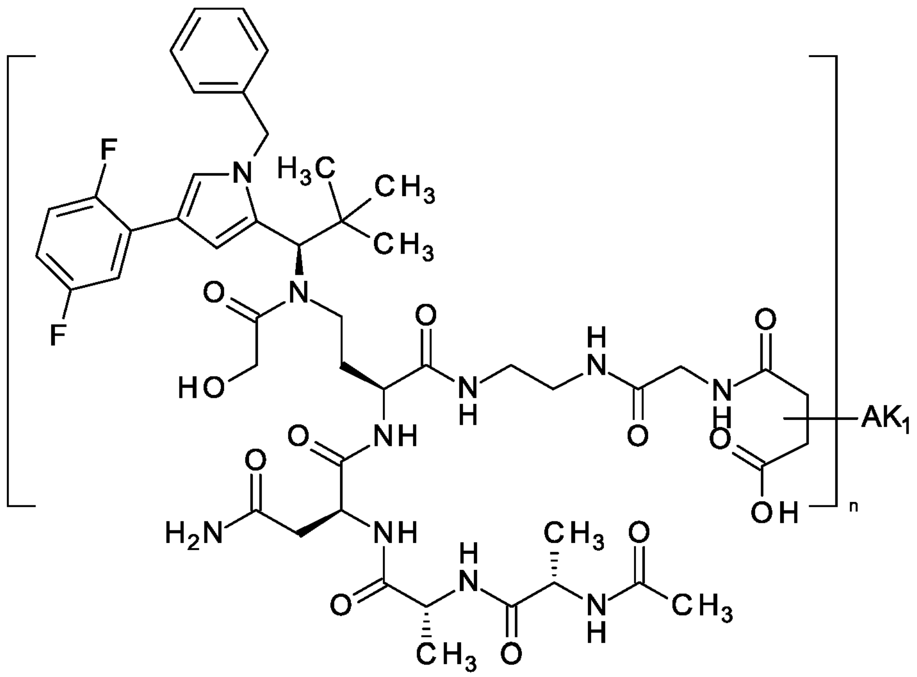

,

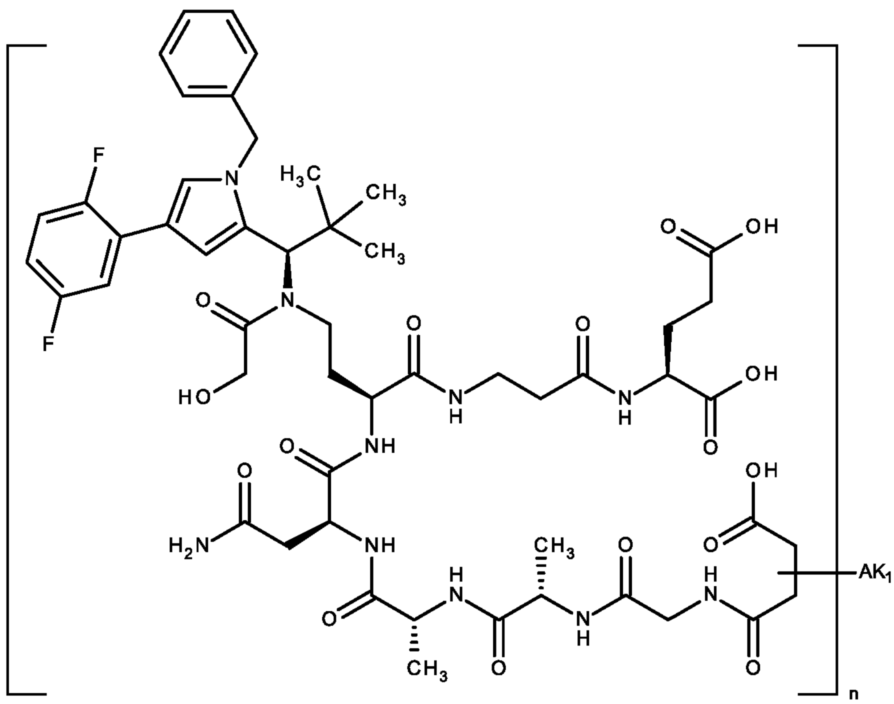

,

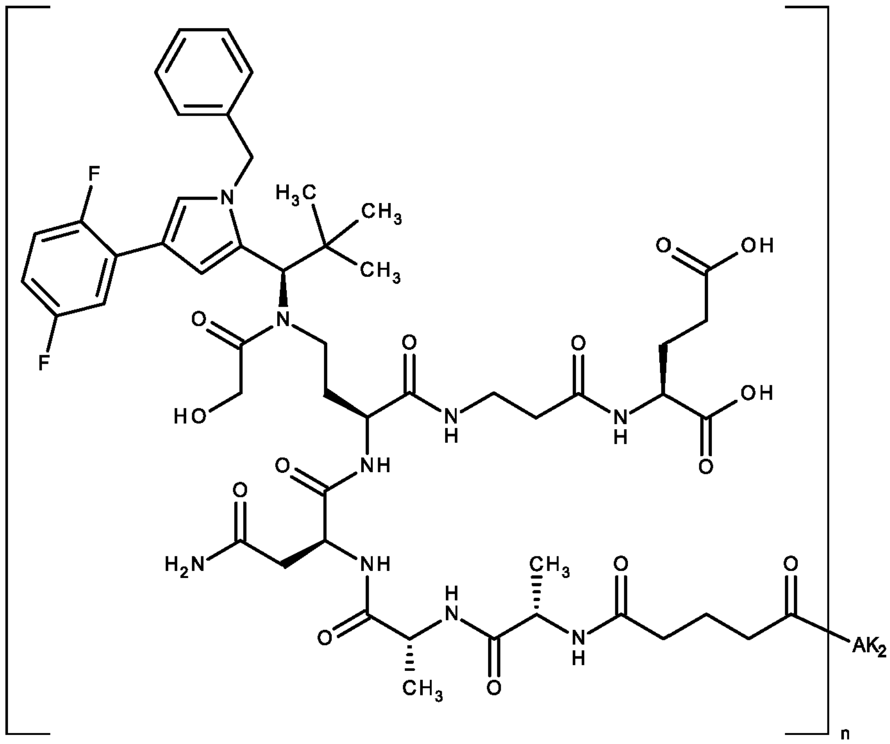

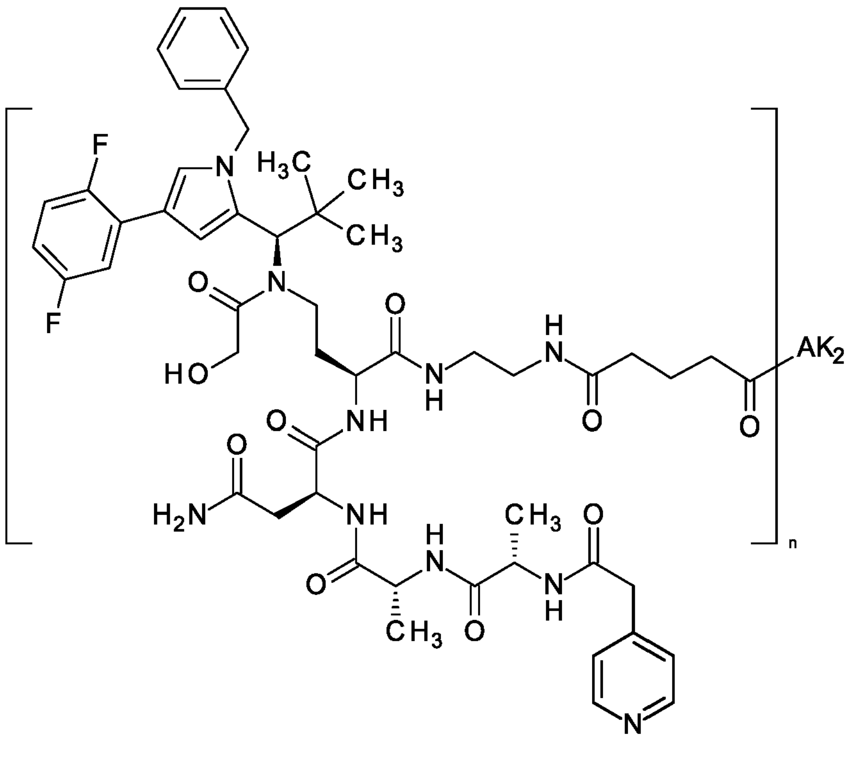

,

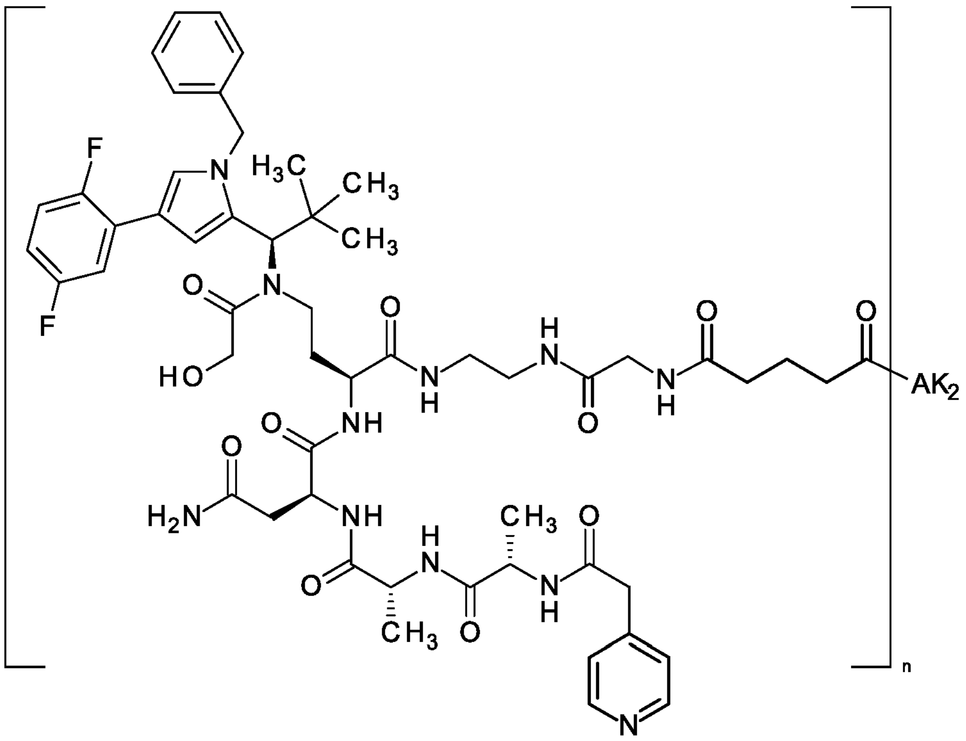

,

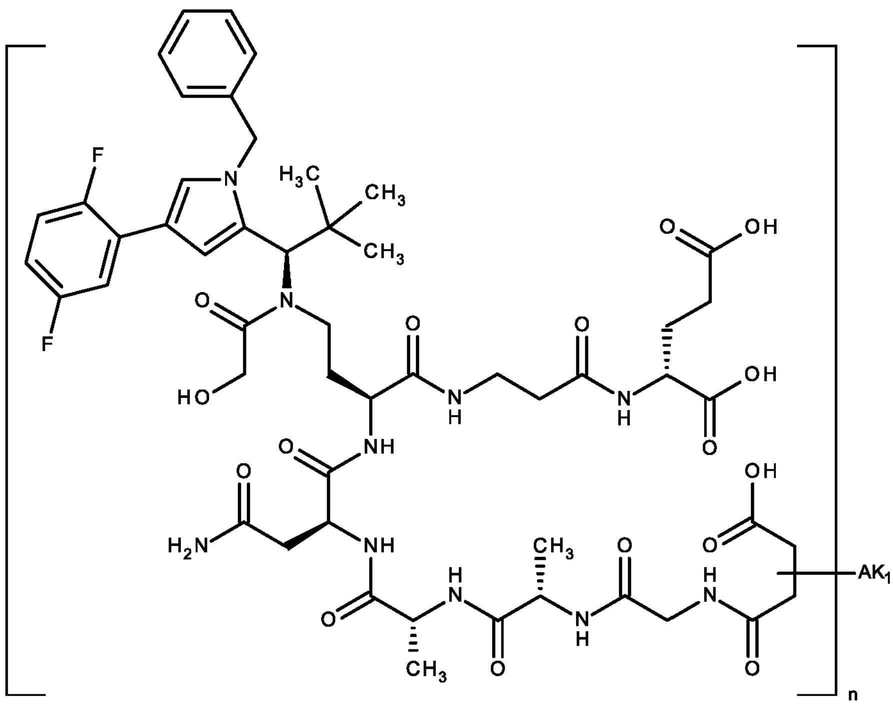

,

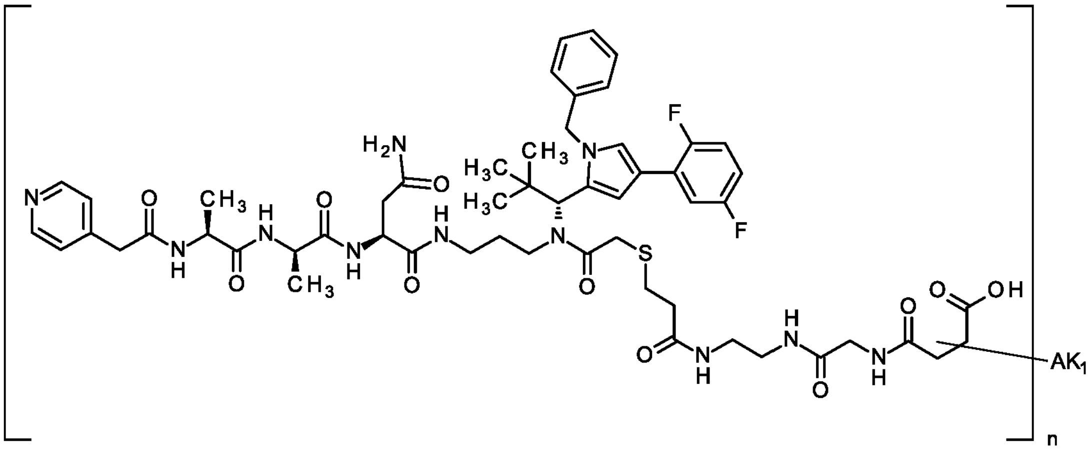

,

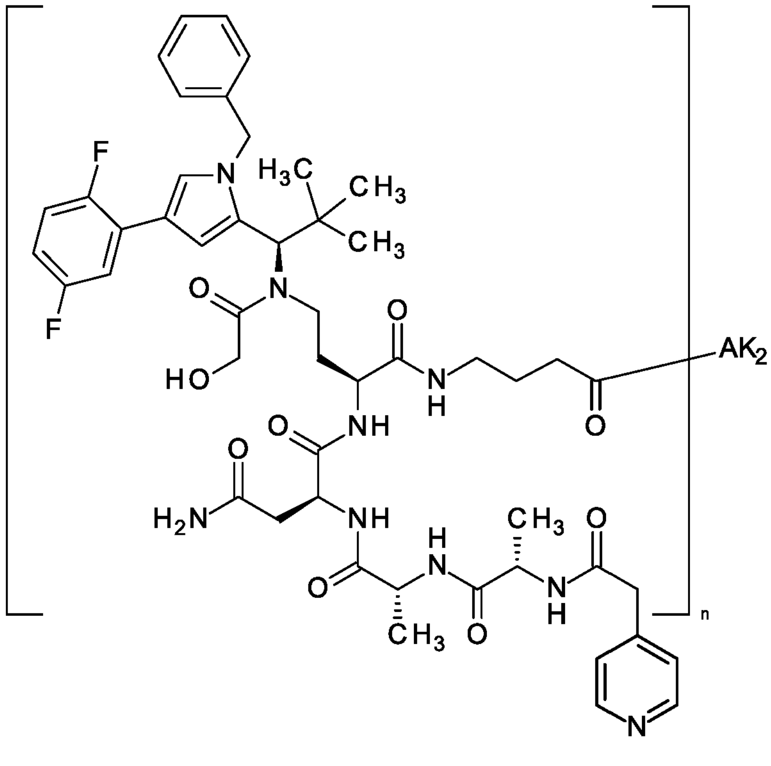

,

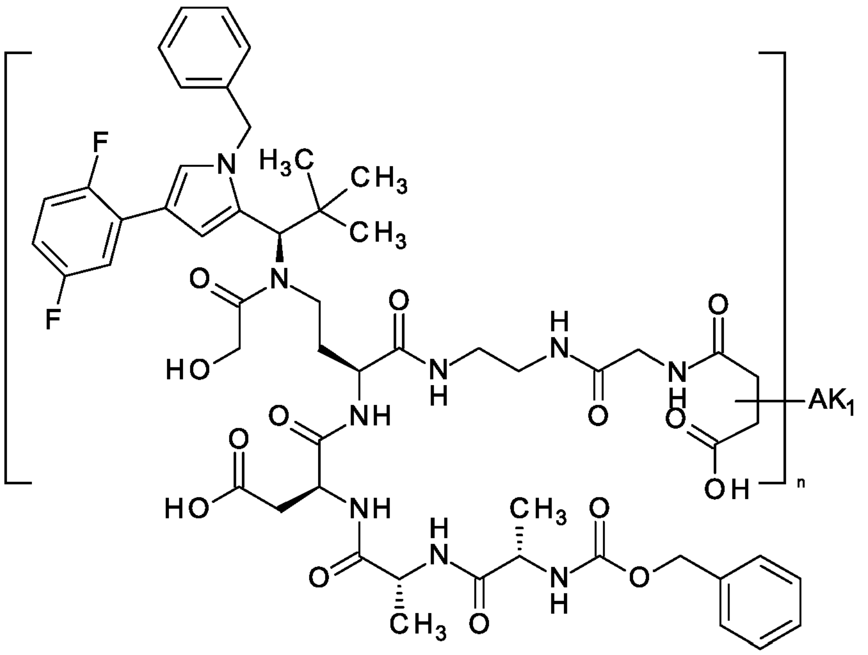

,

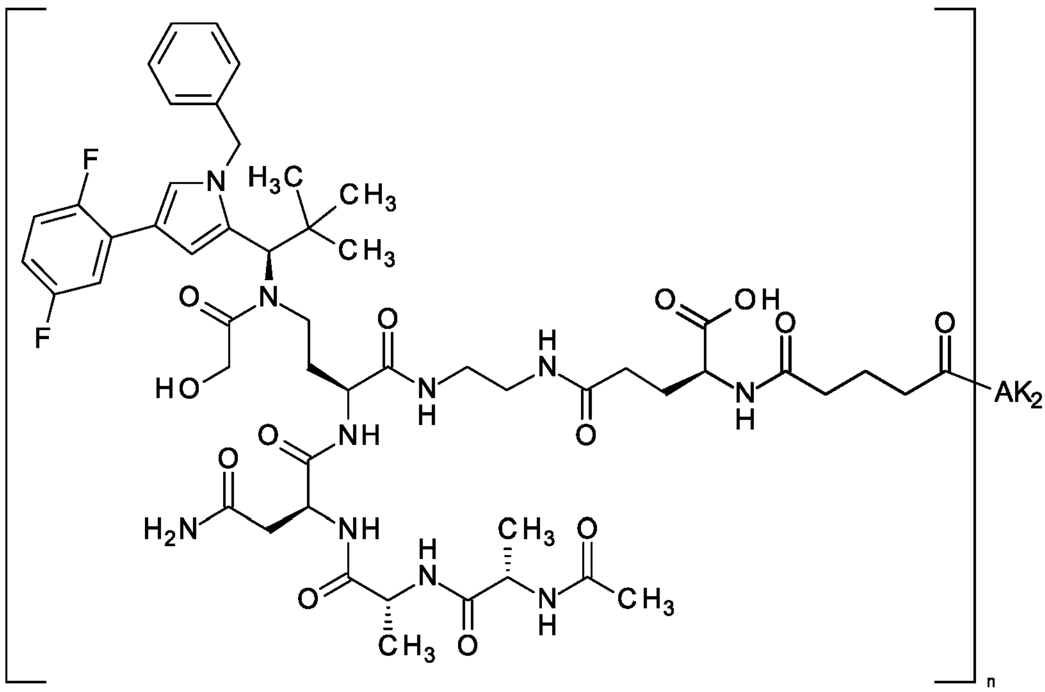

,

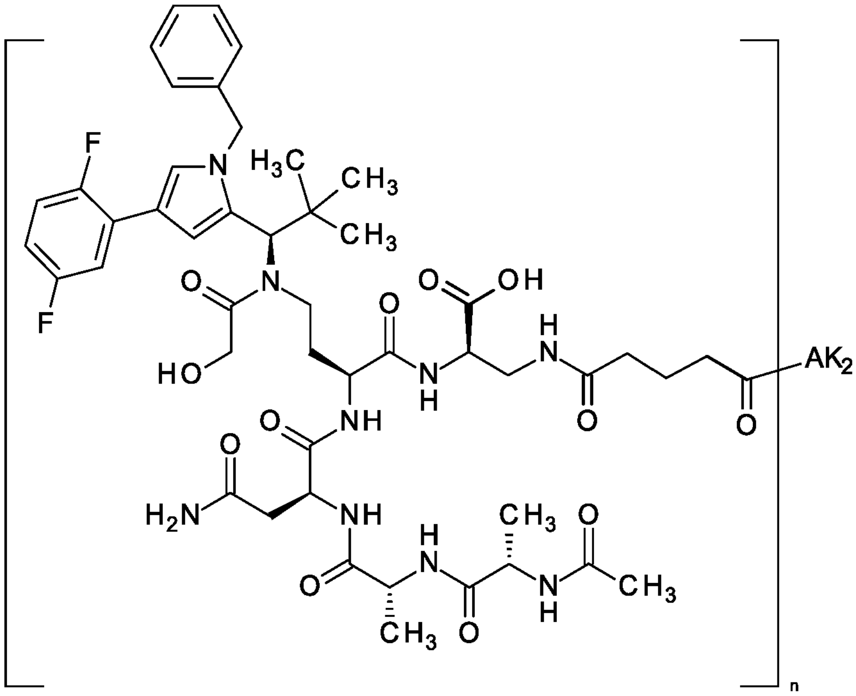

,

,

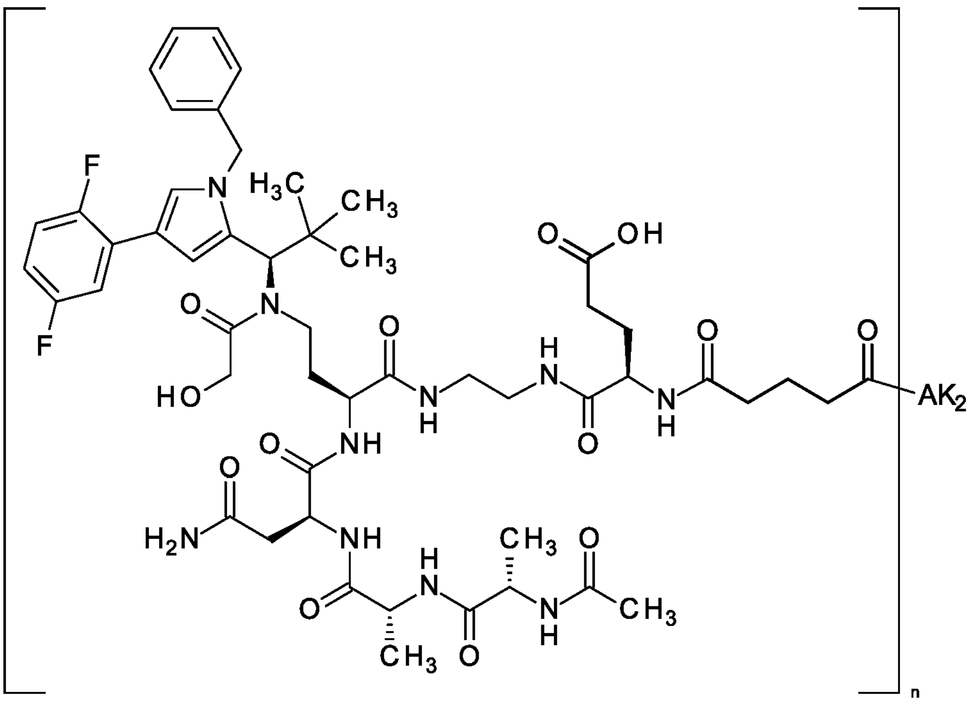

,

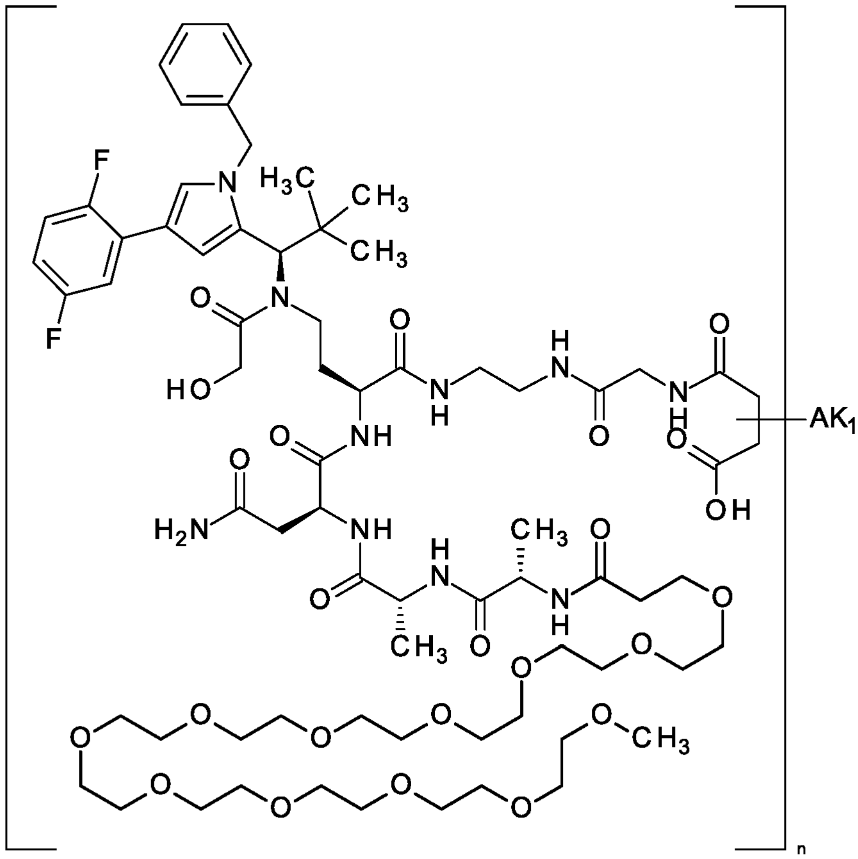

,

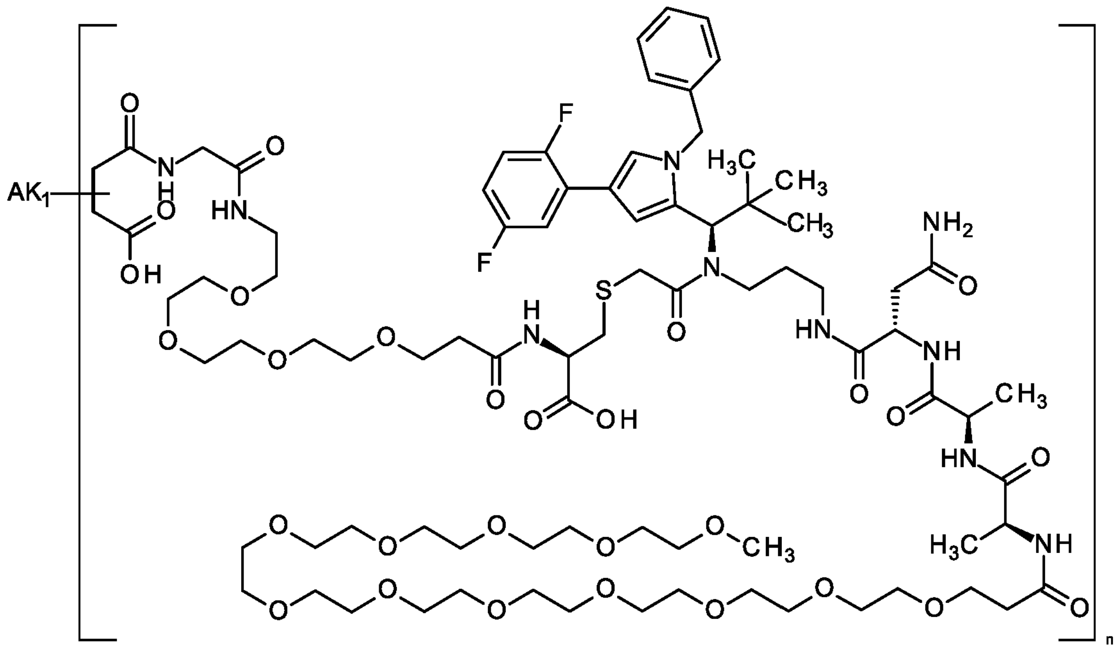

,

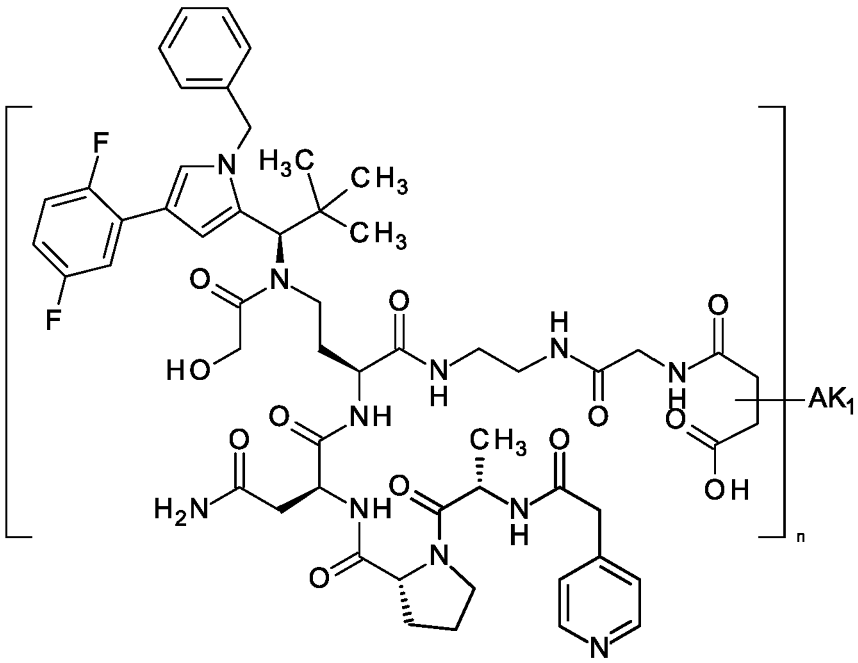

,

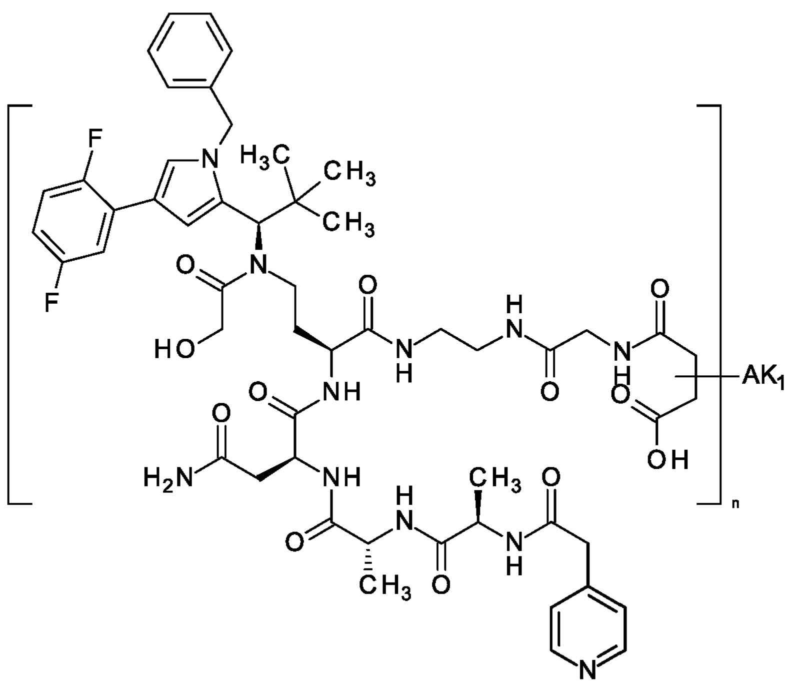

,

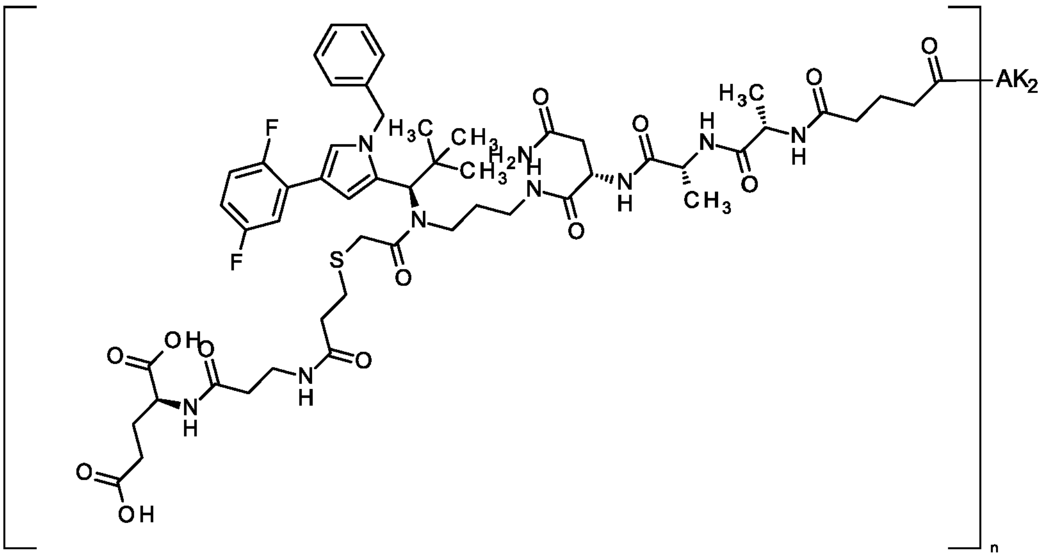

,

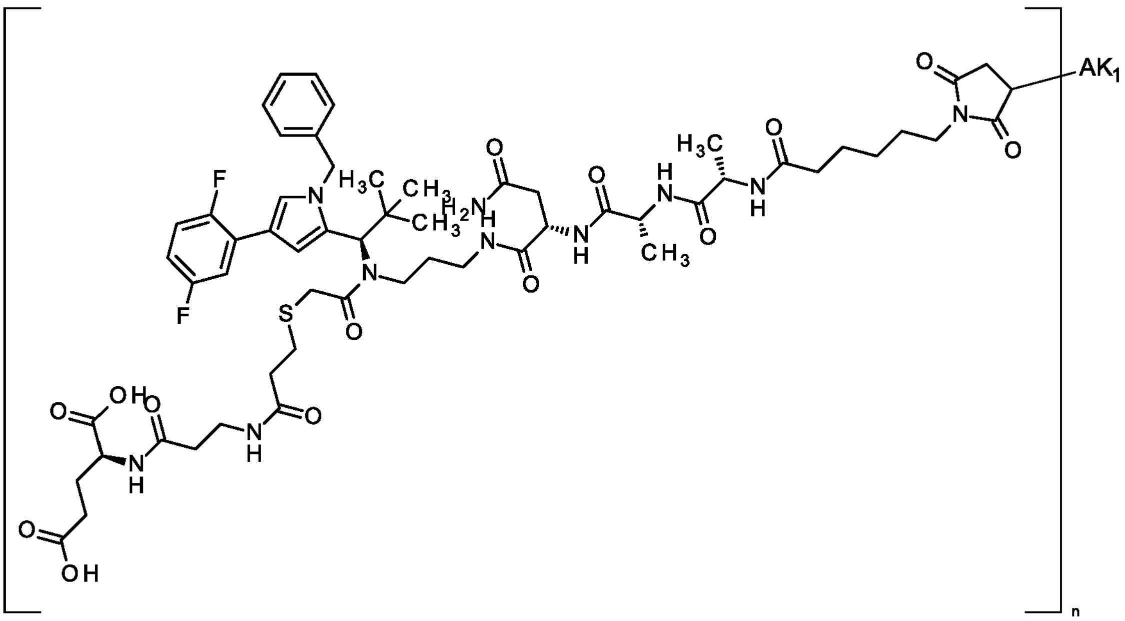

,

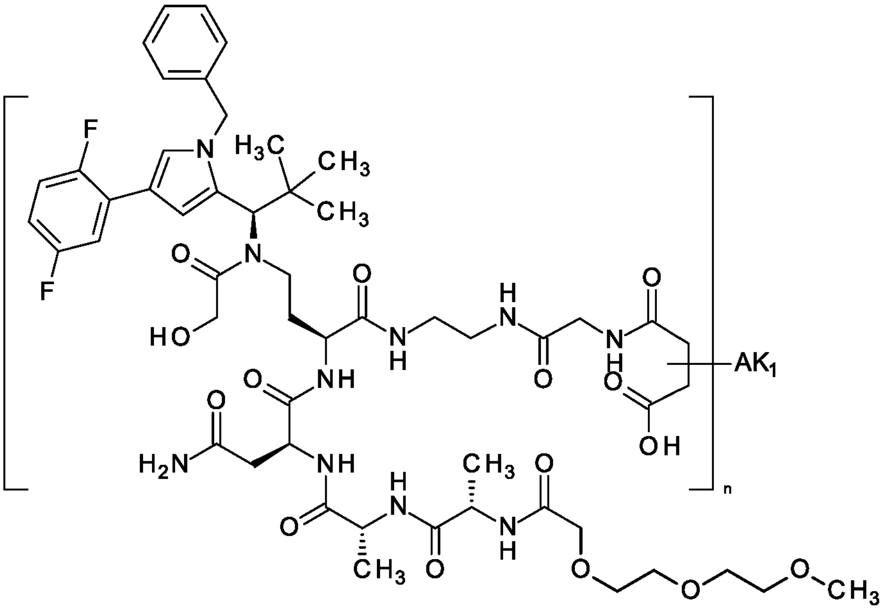

,

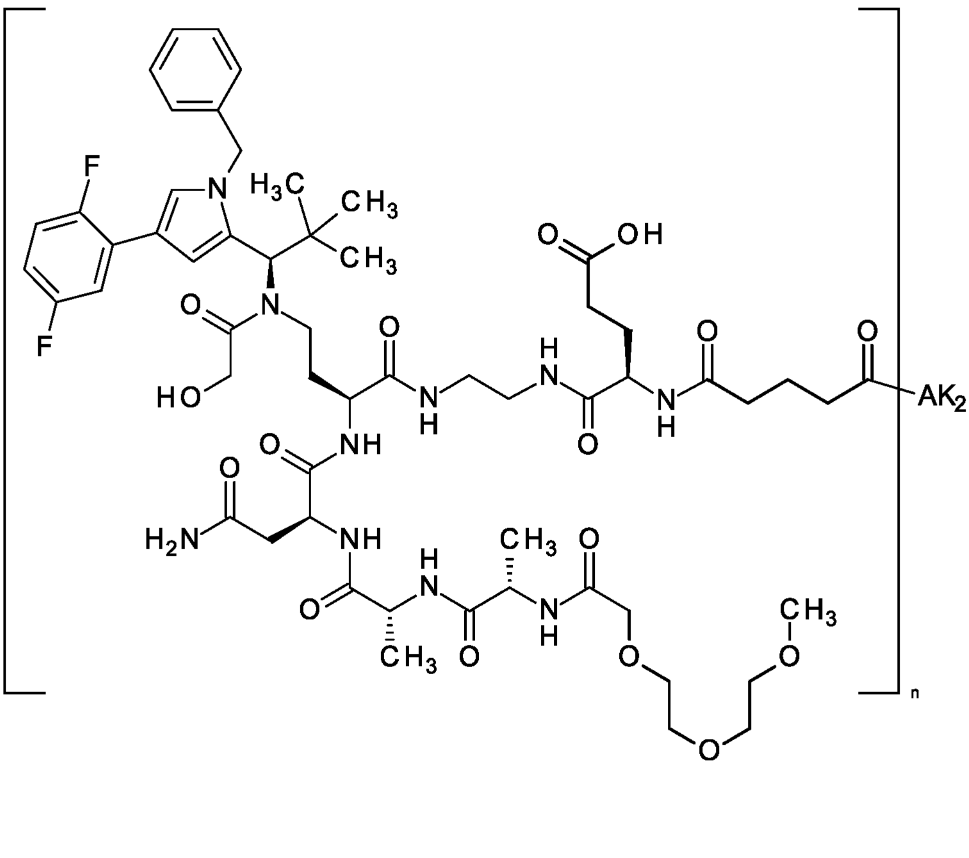

,

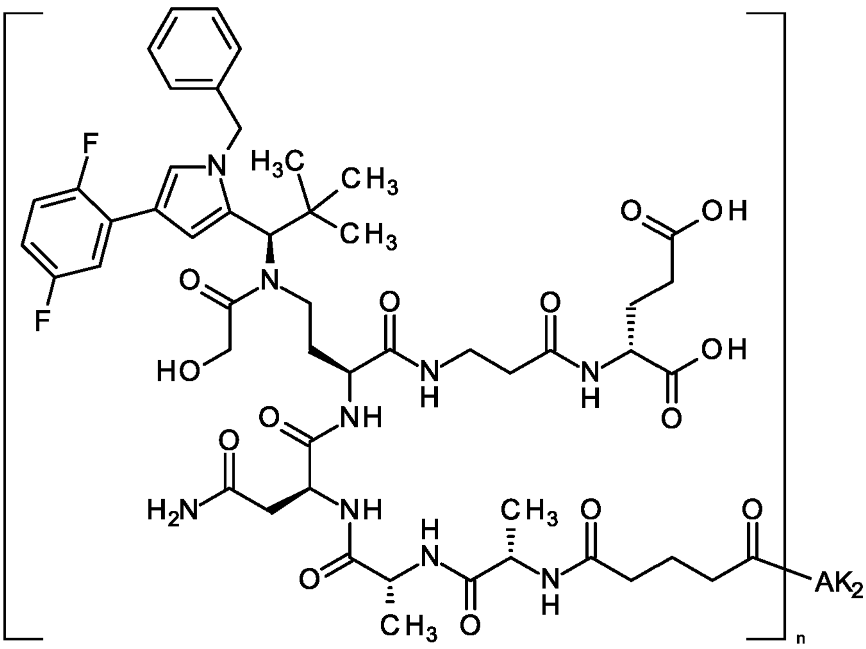

,

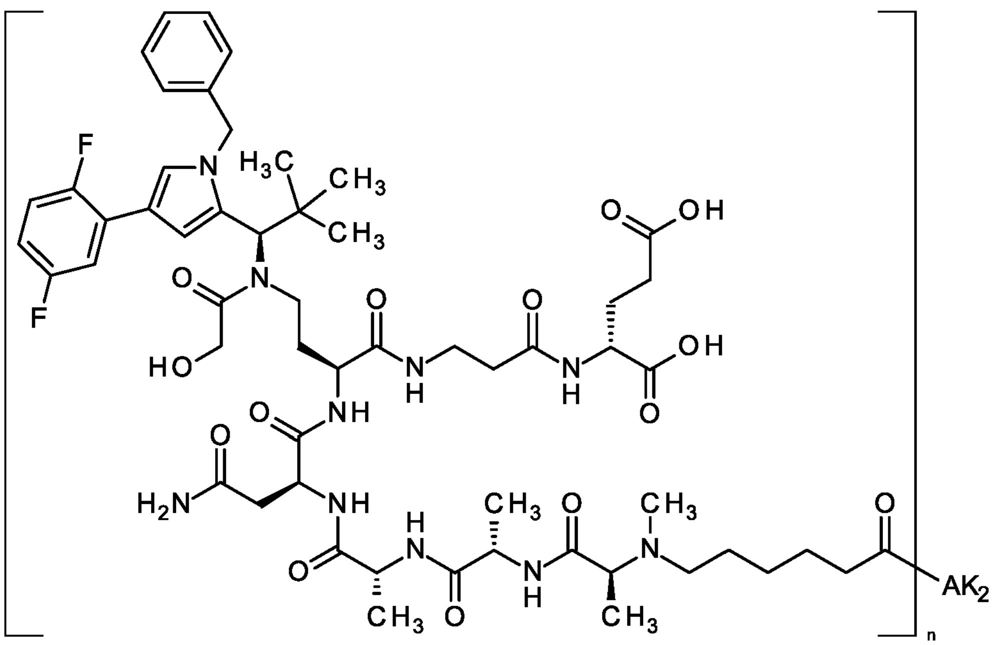

,

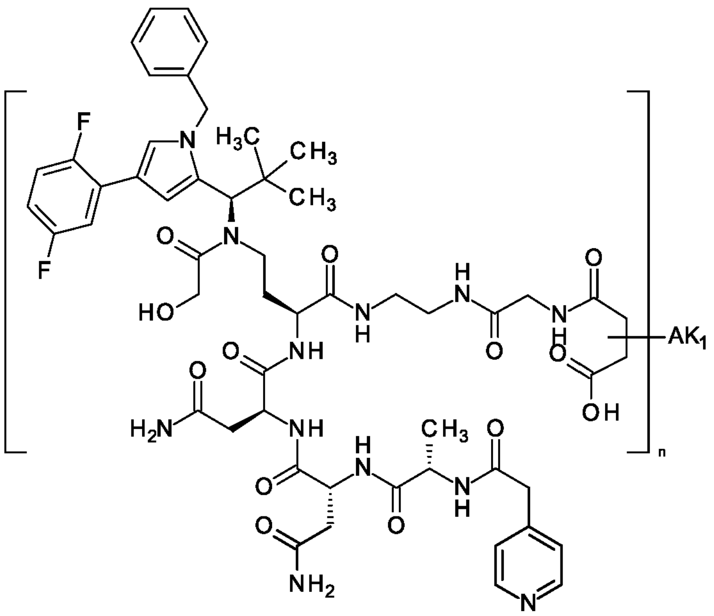

,

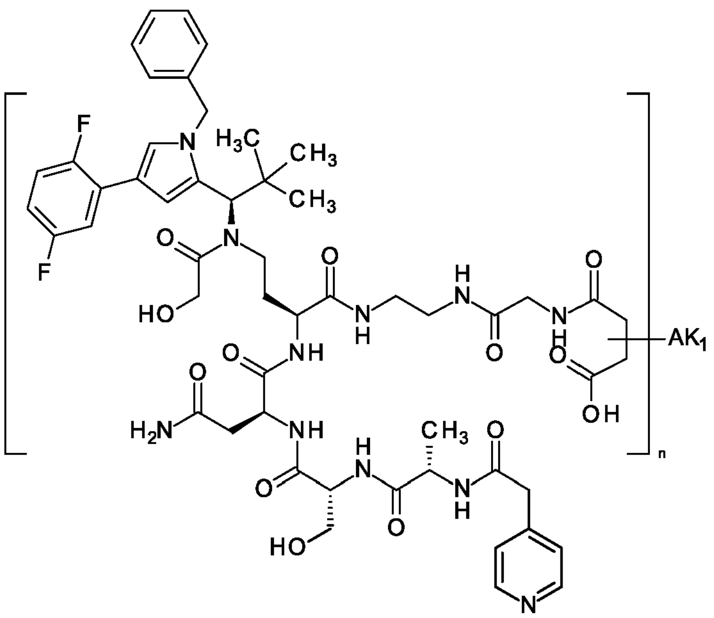

,

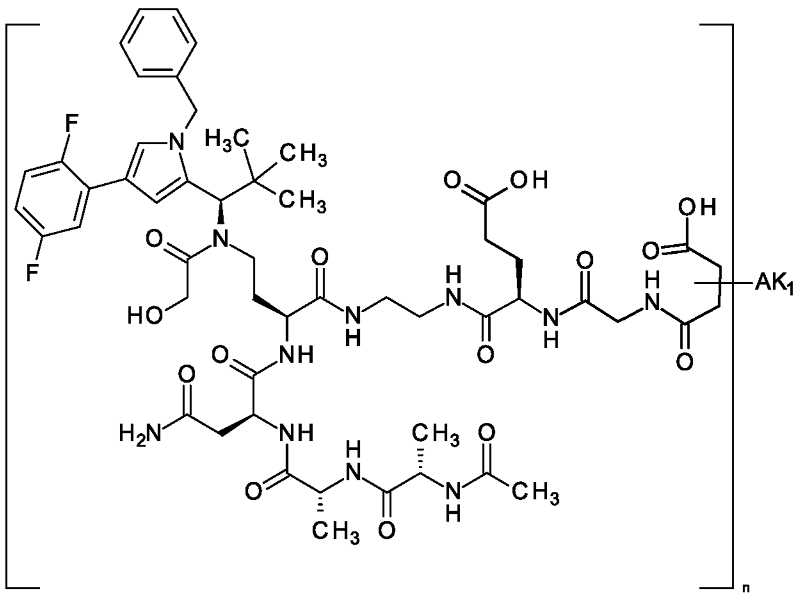

,

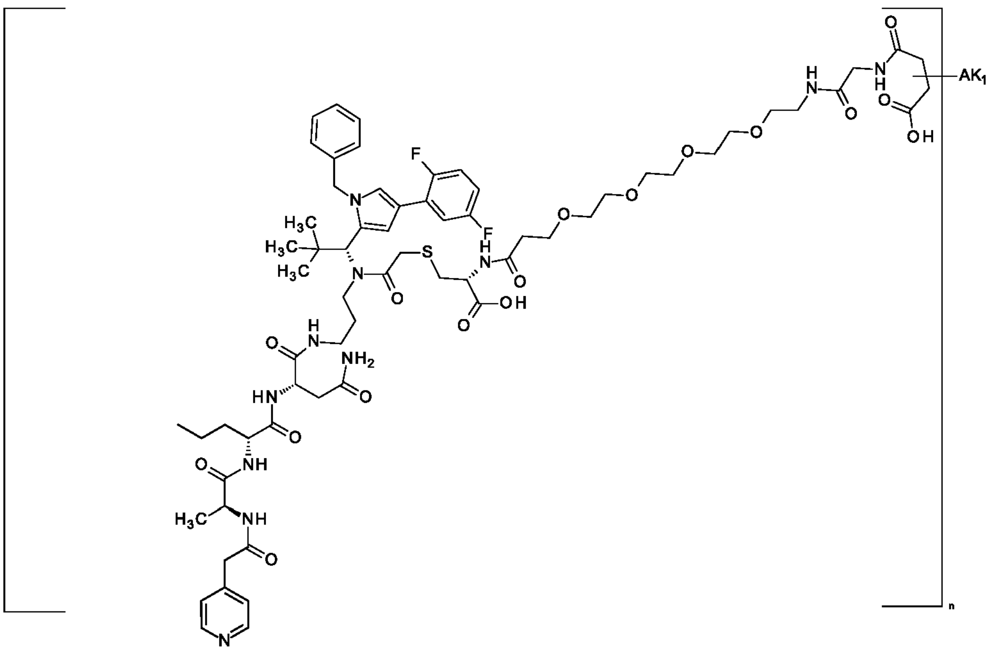

,

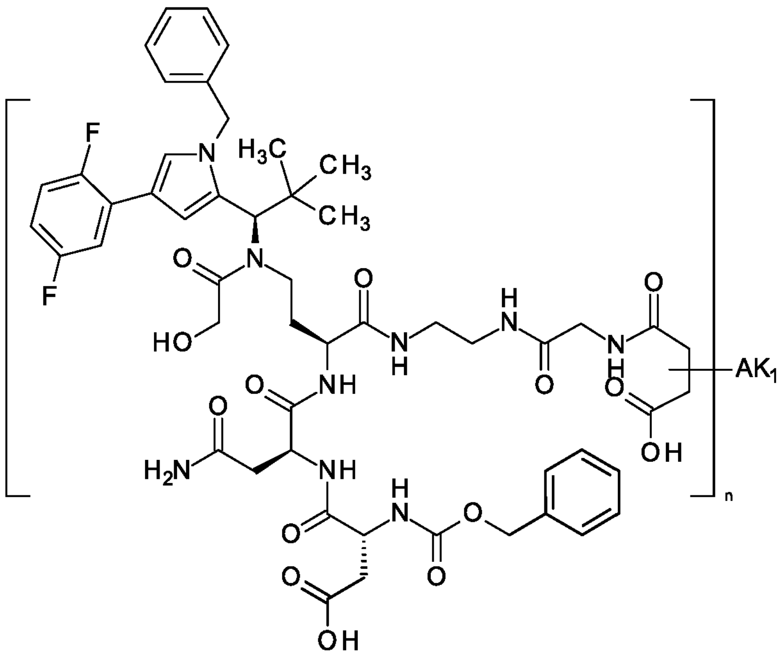

,

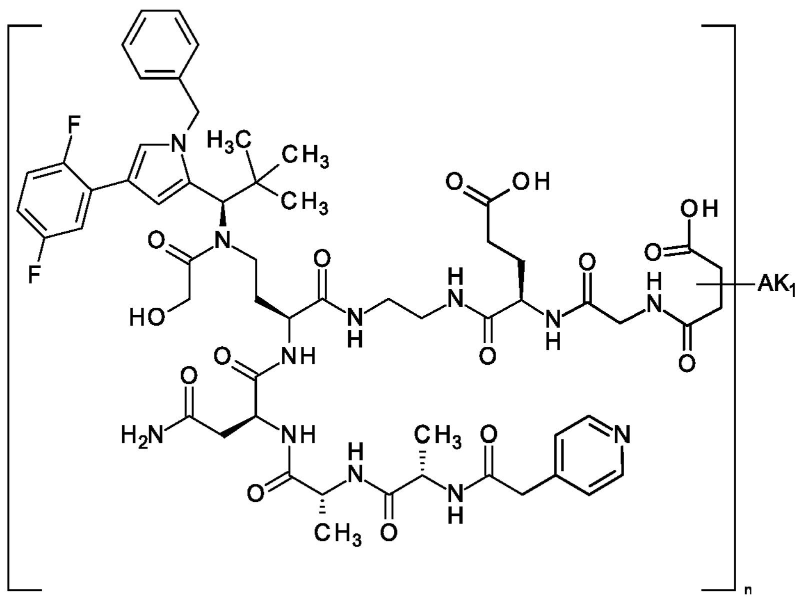

,

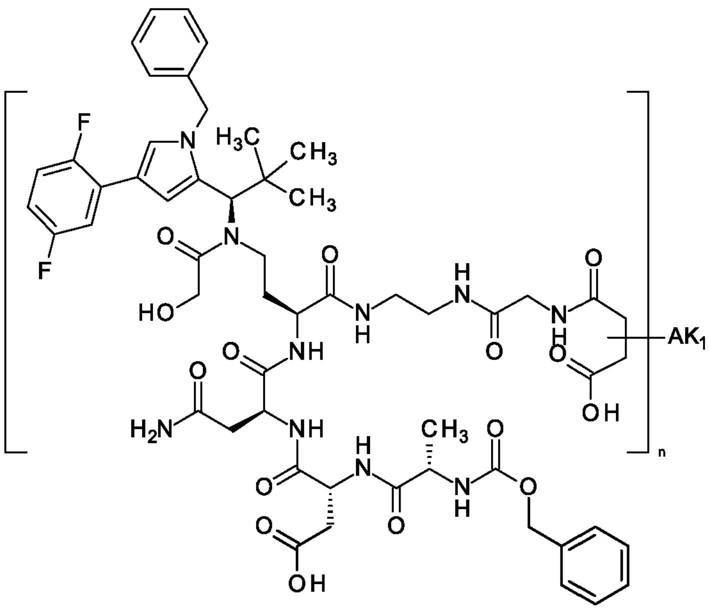

,

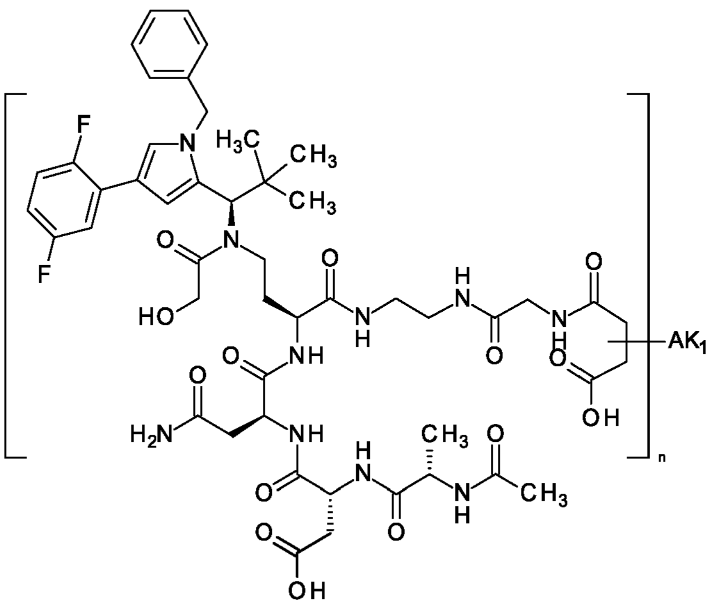

,

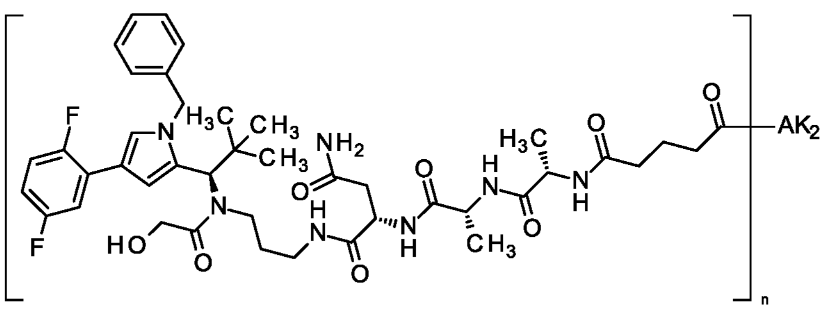

,

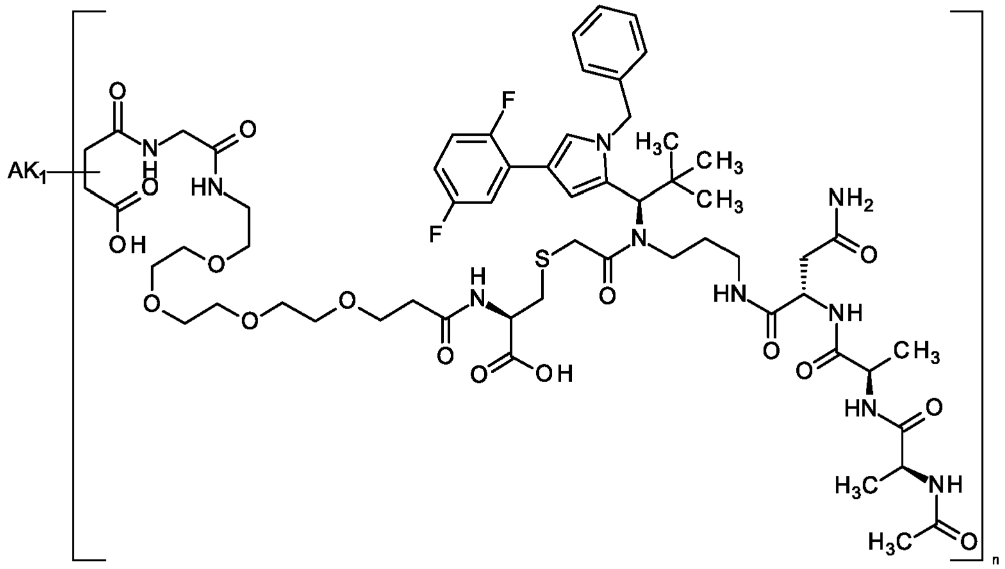

,

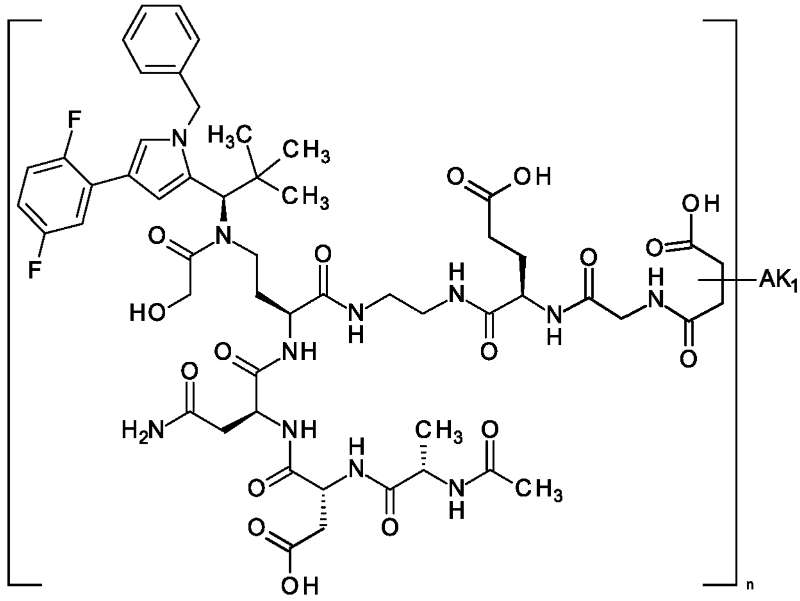

,

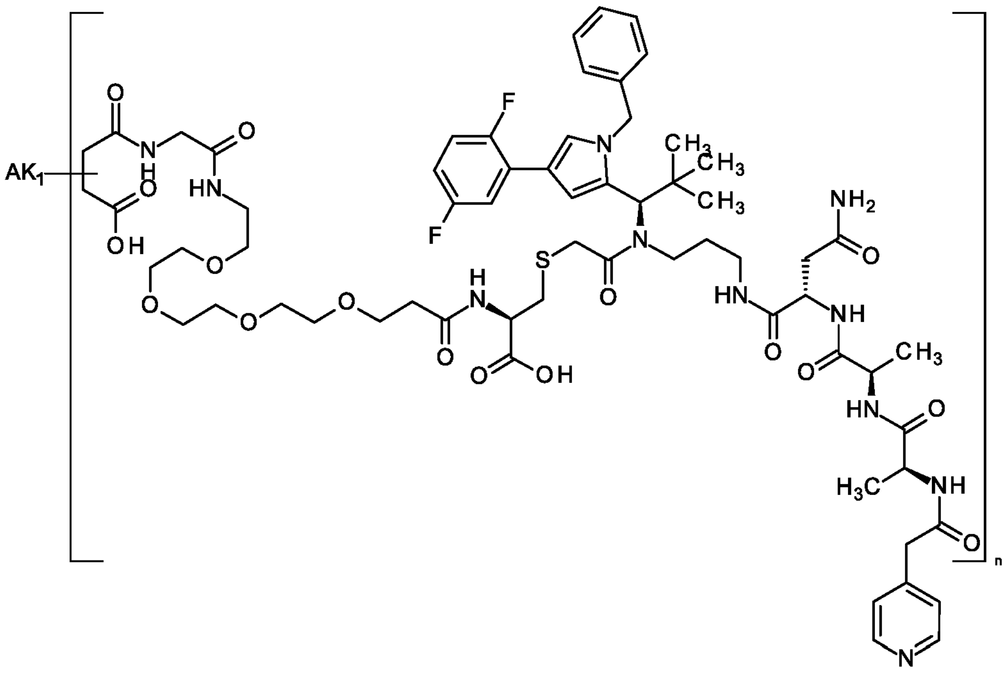

,

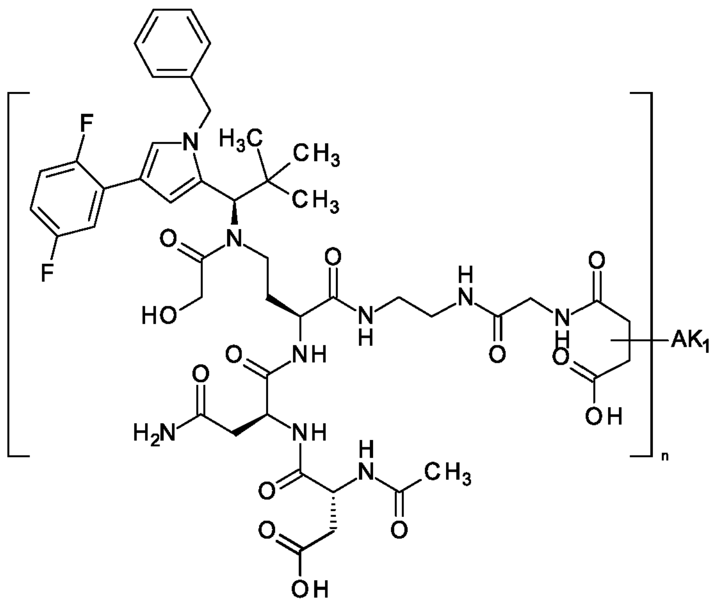

,

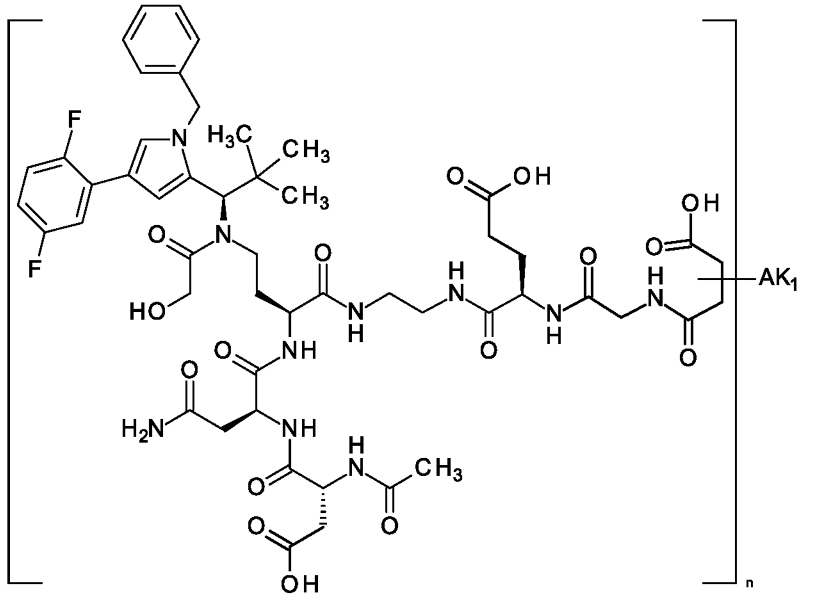

,

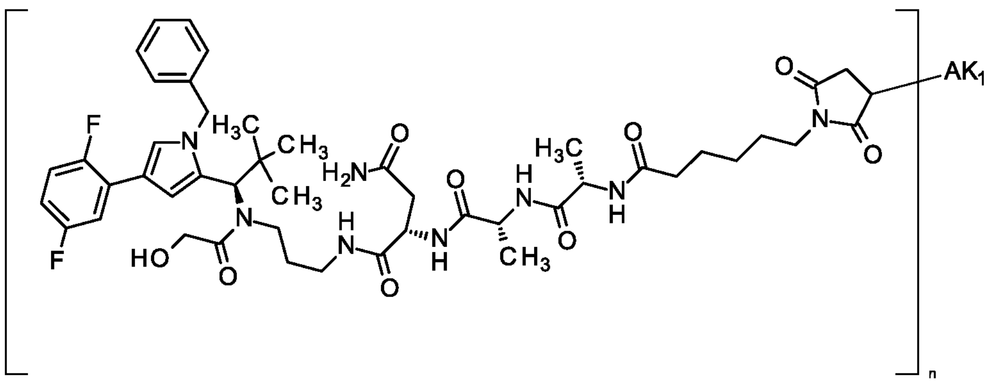

.

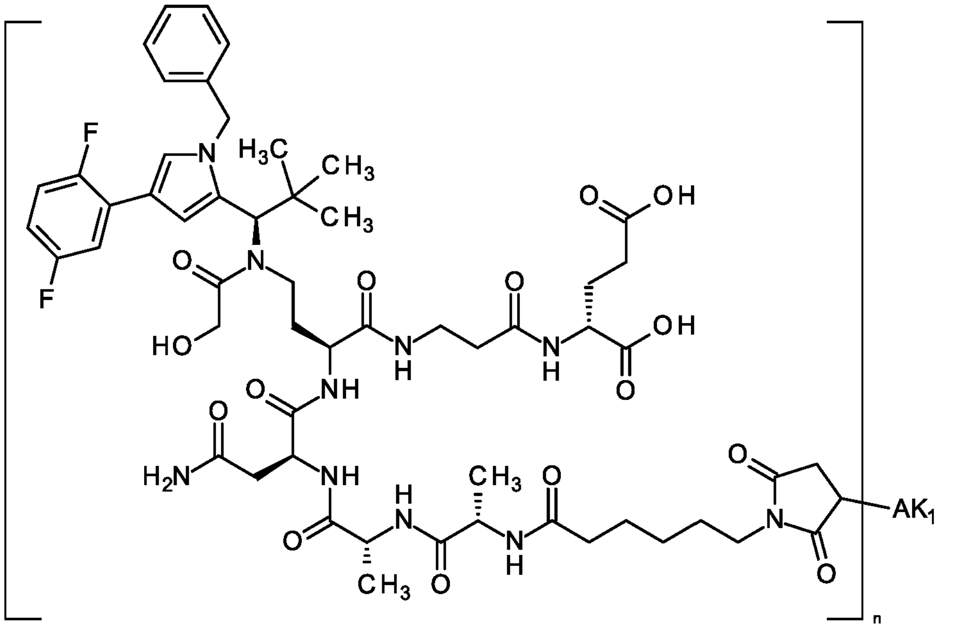

,

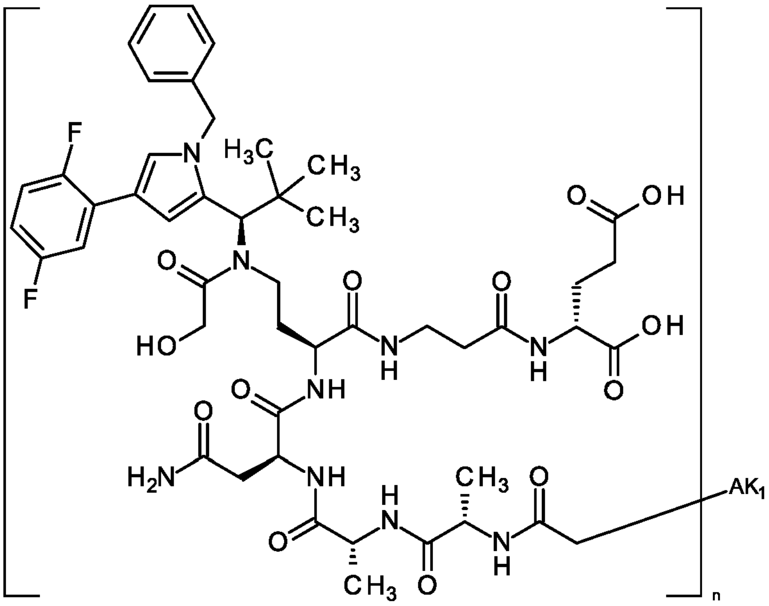

,

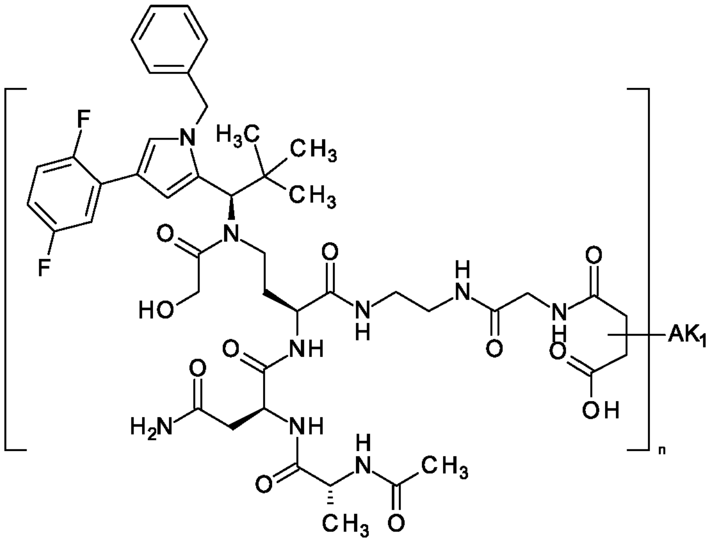

,

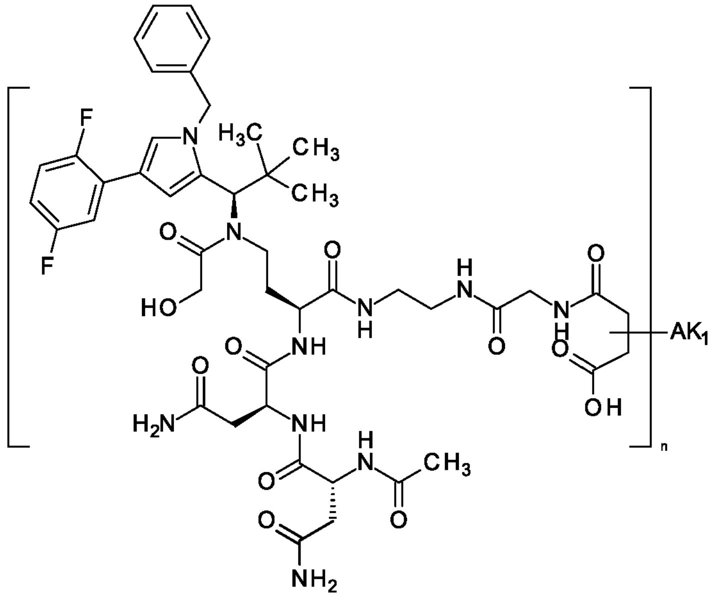

,

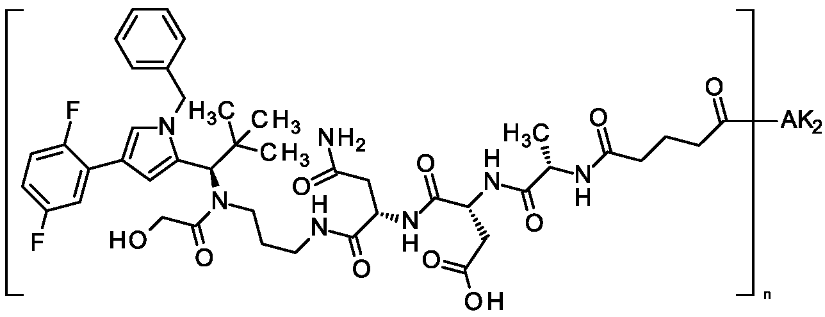

,

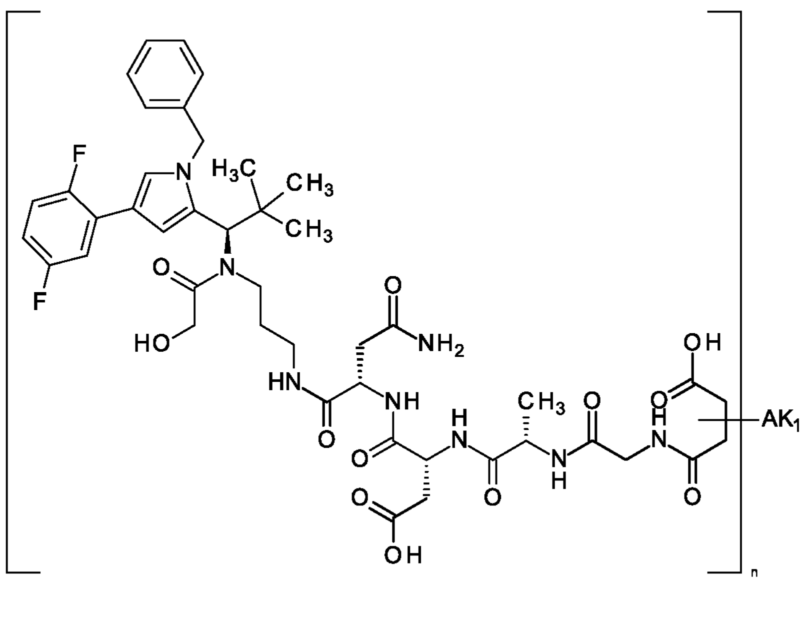

,

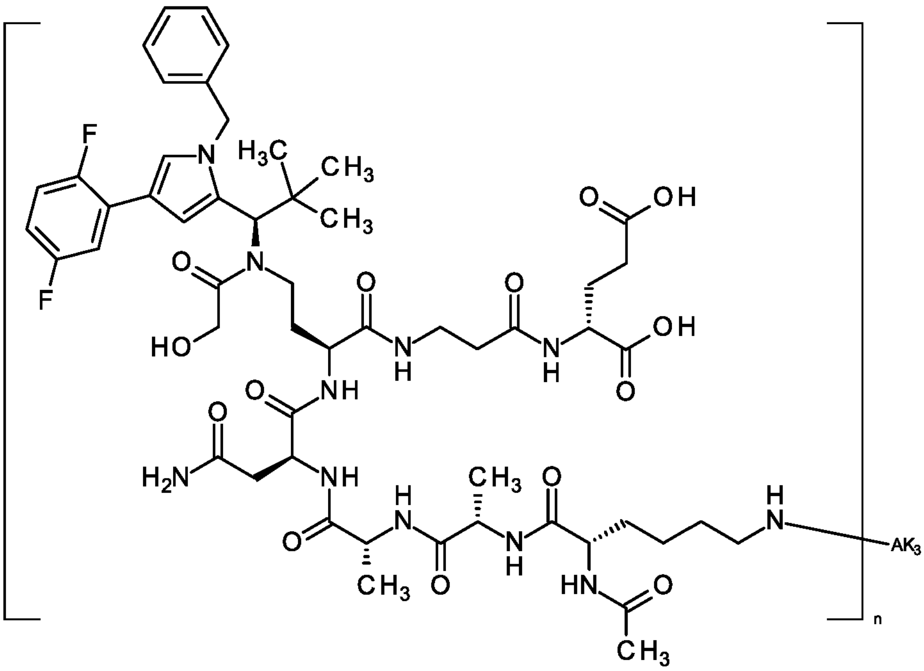

,

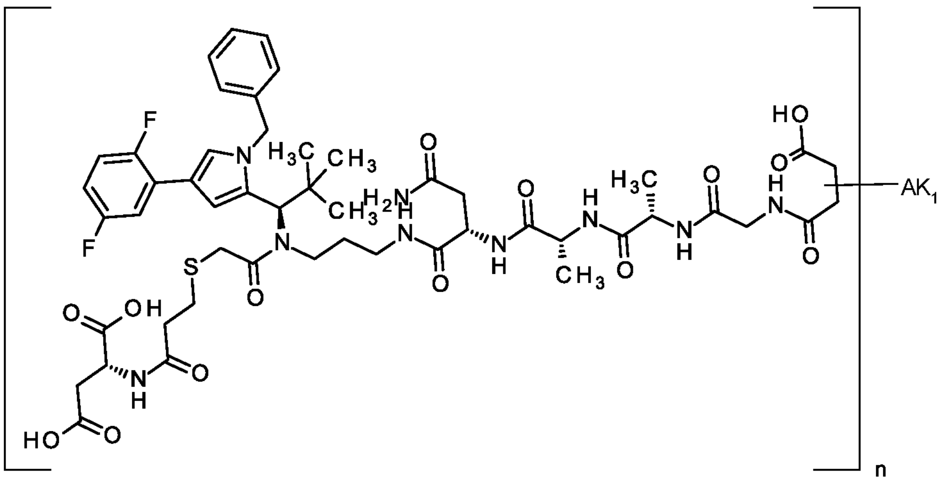

,

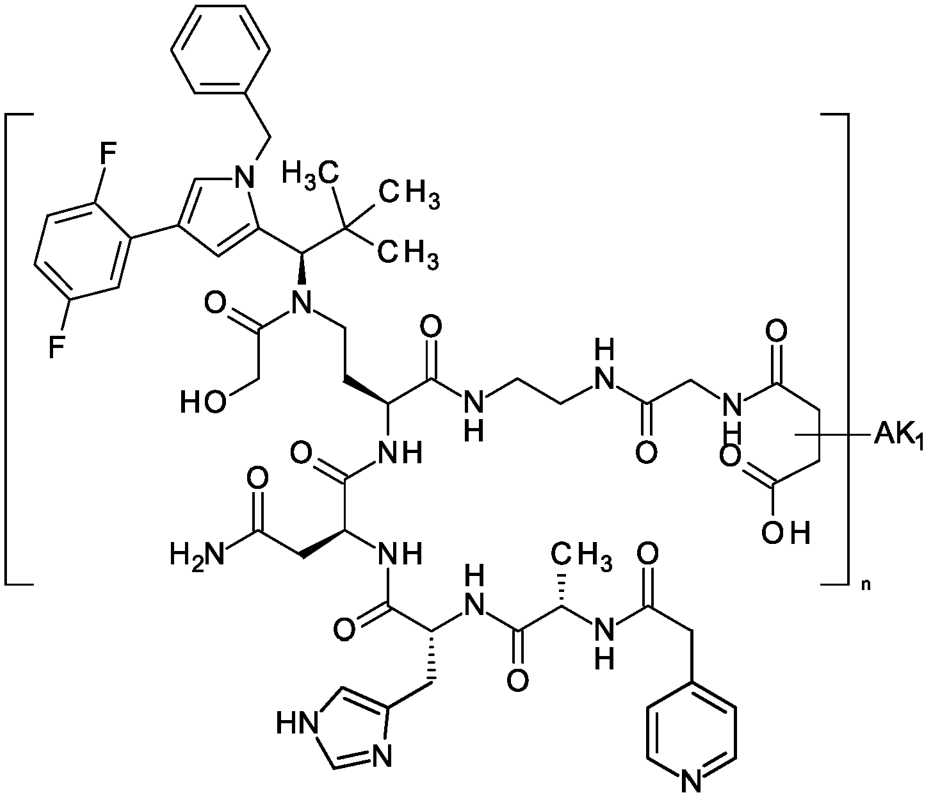

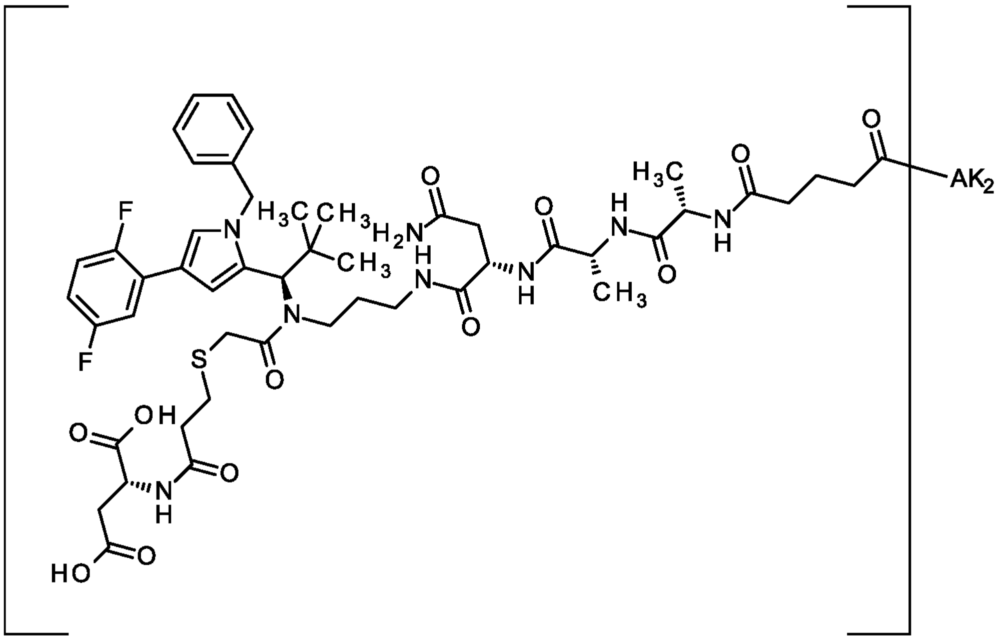

und

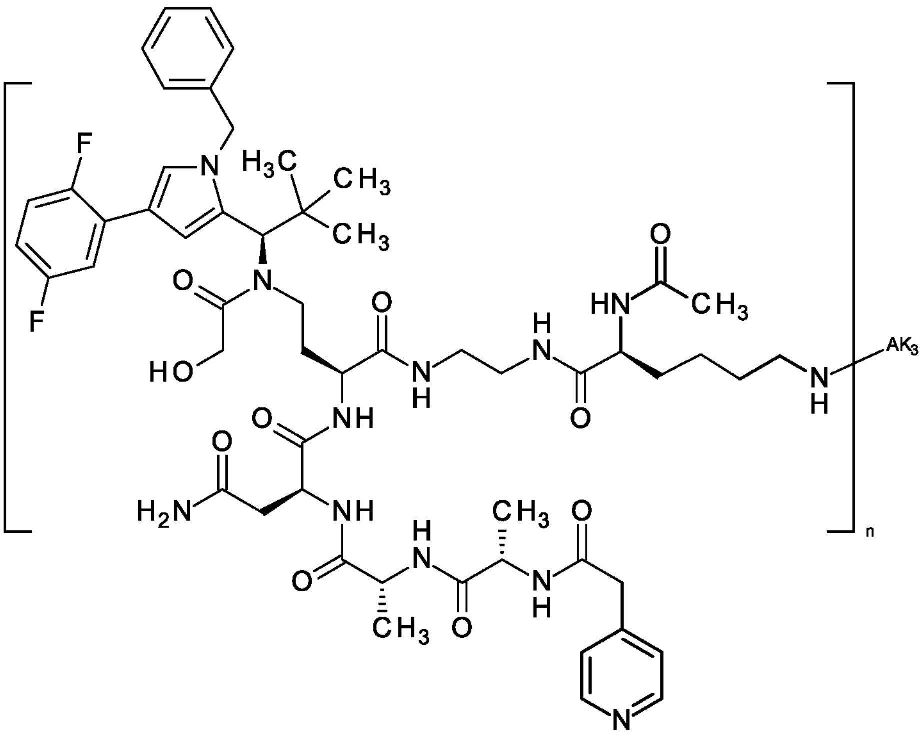

.

**KSP-Inhibitor - Linker-Intermediate bzw. Prodrug-Linker-Intermediate und Herstellung der Konjugate**

Die erfindungsgemäßen Konjugate werden hergestellt, in dem zunächst der niedermolekulare KSP-Inhibitor bzw. das Prodrug hiervon mit einem Linker versehen wird. Da so erhaltene Intermediat wird dann mit dem Binder (vorzugsweise Antikörper) umgesetzt.

Für die Kopplung an einen Cysteinrest wird vorzugsweise eine der folgenden Verbindungen mit dem Cystein-haltigen Binder wie z.B. einem Antikörper, der dazu ggf. partiell reduziert wurde, umgesetzt:

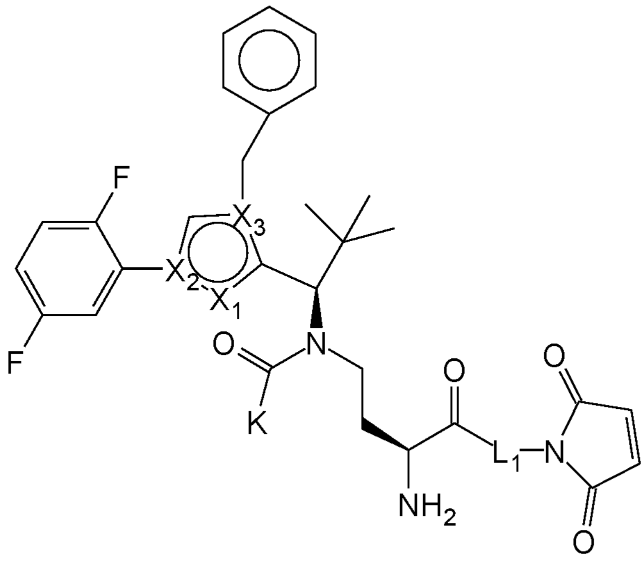

TFA

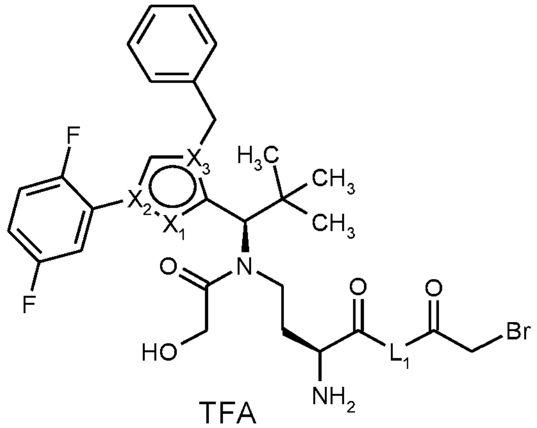

TFA

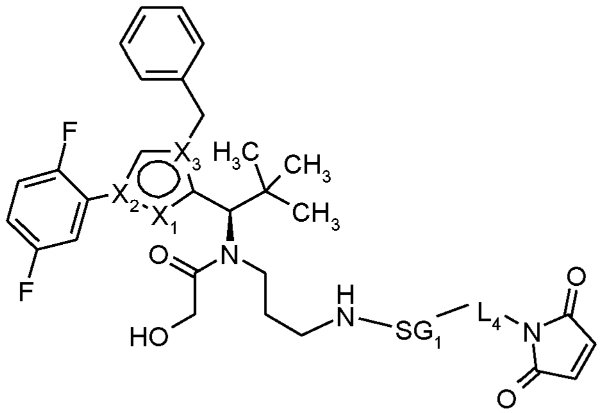

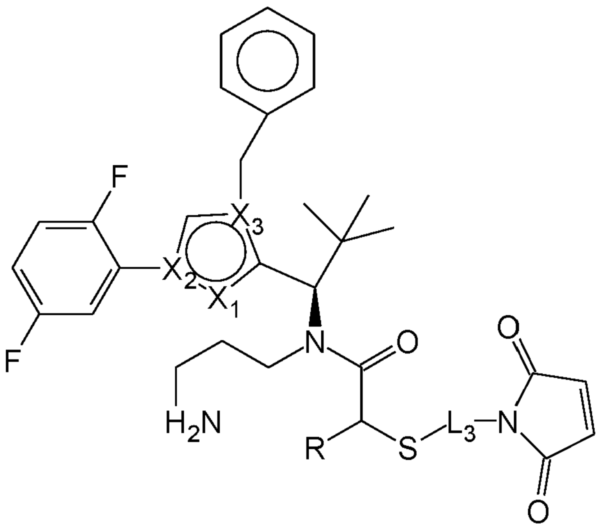

TFA

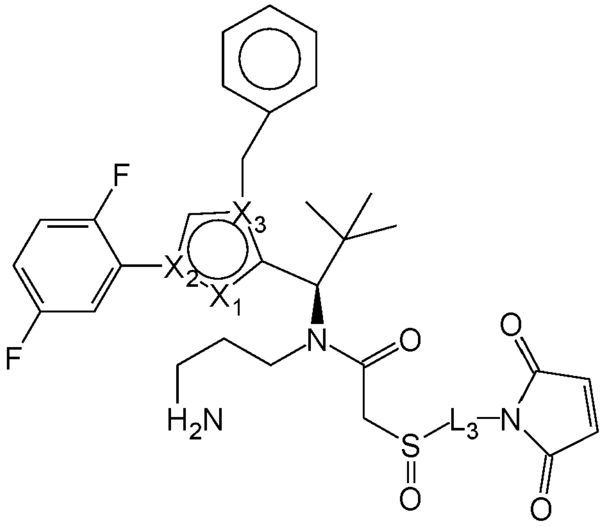

TFA

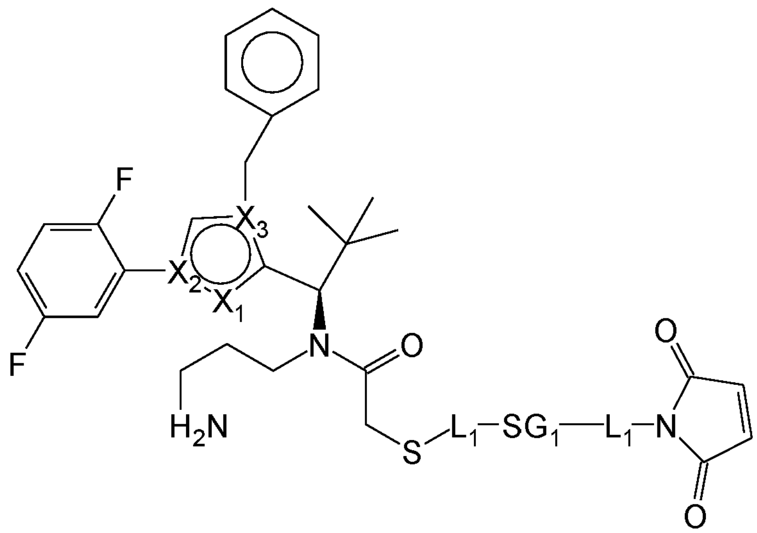

TFA

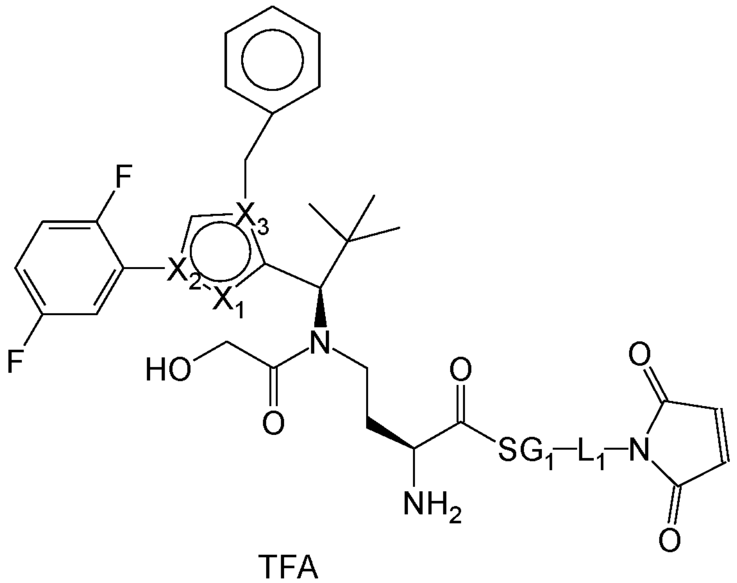

TFA

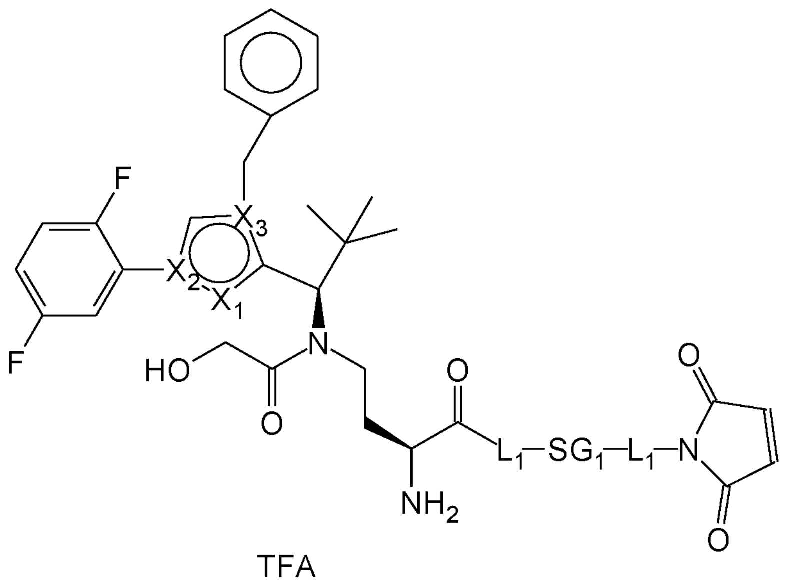

TFA

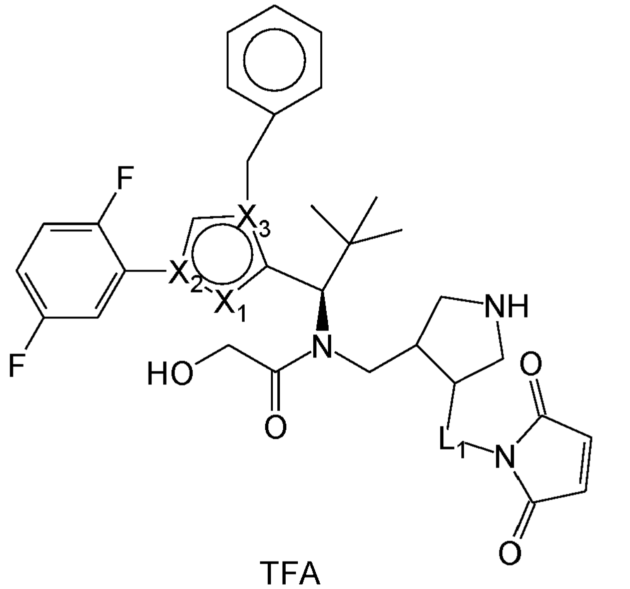

TFA

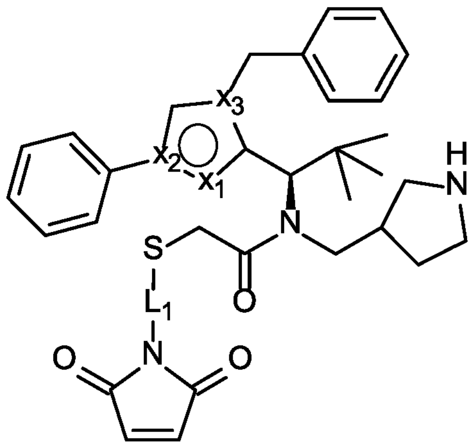

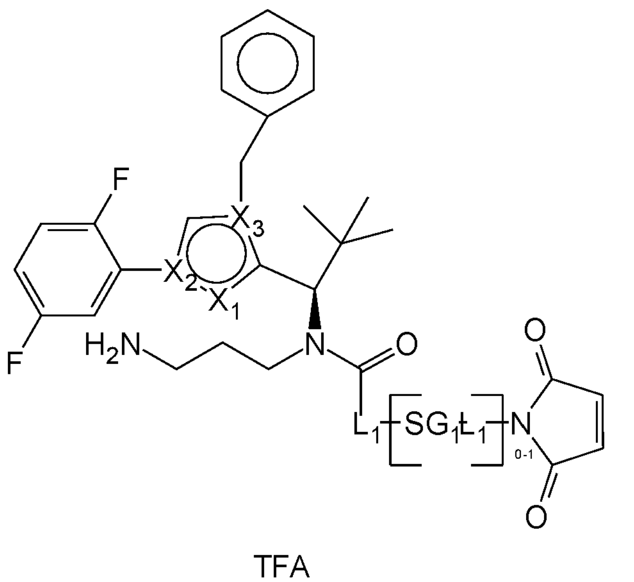

TFA

in denen

| | |
|---|---|
| $X_1$ | für CH steht, |
| $X_2$ | für C steht, |
| $X_3$ | für N steht, |
| R | für -H oder -COOH steht, |
| K | für lineares oder verzweigtes, gegebenenfalls mit $C_1$-$C_6$ Alkoxy- oder -OH substituiertes $C_1$-$C_6$ Alkyl- steht und |
| $SG_1$, $L_1$, $L_2$, $L_3$ und $L_4$ | die oben angegebenen Bedeutungen haben. |

In den zuvor beschriebenen Formeln wie auch in den folgenden Reaktionsschemata bzw. Strukturformeln kann das Wasserstoffatom in Position $R^4$ gemäß Formel IIa, d.h. in der Gruppe -$NH_2$, durch die durch die erfindungsgemäß verwendete, durch Legumain spaltbare Gruppe der Formel Ia ersetzt sein.

In jeder der obigen Verbindungen wie auch den folgenden Verbindungen kann die tert.-butyl-Gruppe durch Cyclohexyl ersetzt sein.

Die Verbindung kann z.B in Form ihres Trifluoressigsäuresalzes verwendet werden. Zur Reaktion mit dem Binder wie z.B. dem Antikörper wird die Verbindung vorzugsweise in 2 bis 12fachem molaren Überschuss gegenüber dem Binder verwendet.

Für die Kopplung an einen Lysinrest wird vorzugsweise eine der folgenden Verbindungen mit dem Lysin-haltigen Binder wie z.B. einem Antikörper umgesetzt:

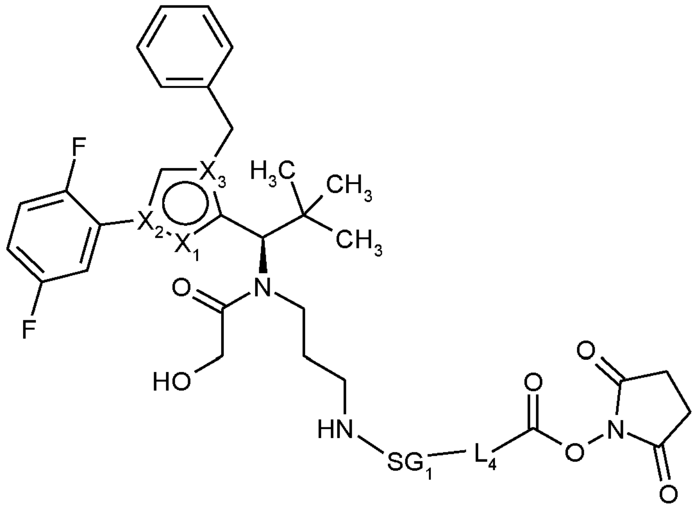

In der Formel steht

$X_1$ für CH steht,
$X_2$ für C steht,
$X_3$ für N steht und
$L_4$ die gleiche Bedeutung wie $L_1$ hat, wobei $L_1$ die gleiche Bedeutung wie oben beschrieben hat.

Für ein an einen Cysteinrest ankoppelndes Intermediat können die Reaktionen wie folgt veranschaulicht werden:

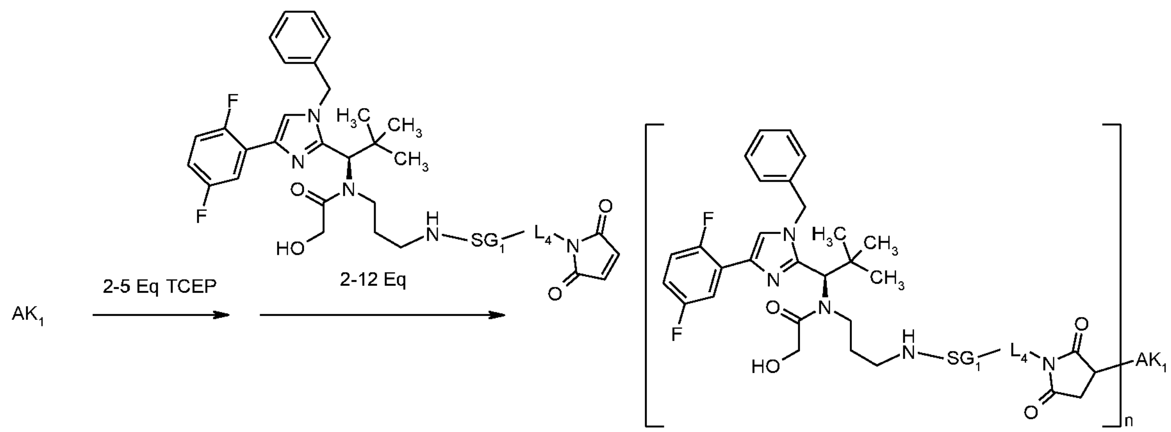


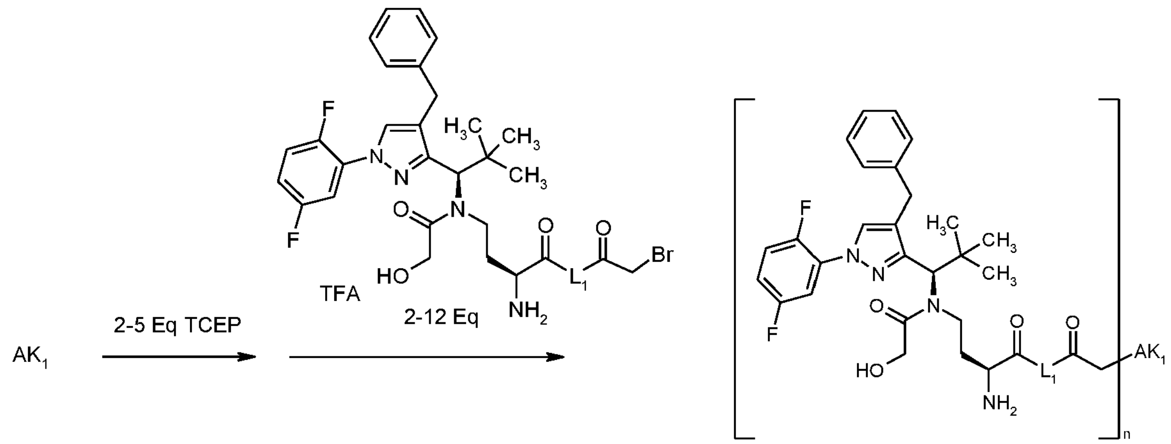


Die anderen Intermediate und anderen Antikörper können entsprechend umgesetzt werden.

Für ein an einen Lysinrest ankoppelndes Intermediat kann die Reaktion wie folgt veranschaulicht werden:

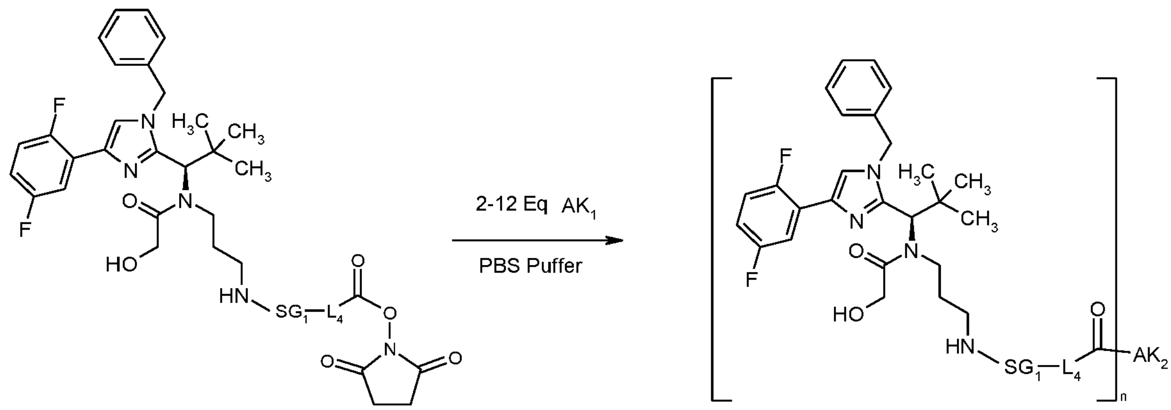


Erfindungsgemäß werden so die folgenden Konjugate erhalten:

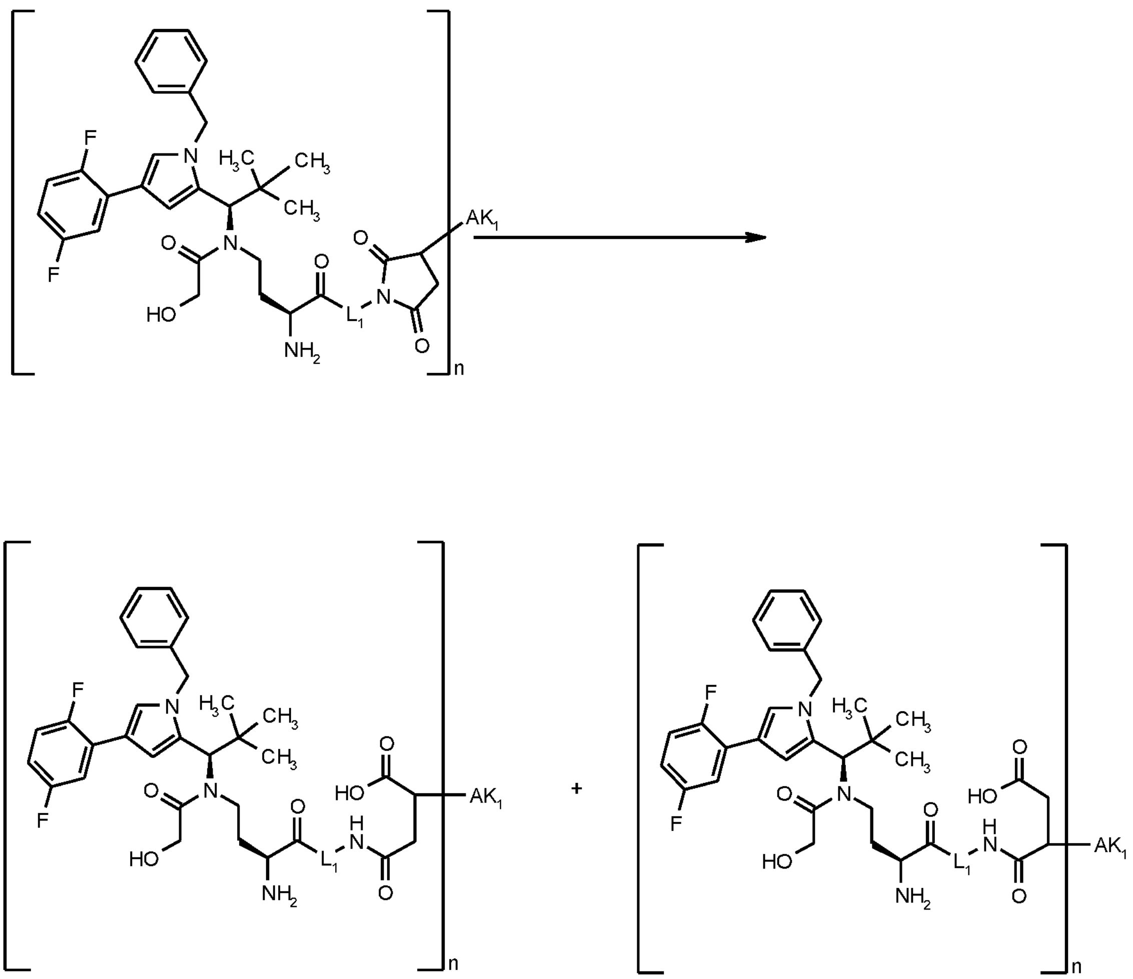

Diese Umsetzung (Ringöffnung) kann bei pH 7.5 bis 9, vorzugsweise bei pH 8, bei einer Temperatur von 25 ° C bis 37 °C erfolgen, z.B. durch Rühren. Die bevorzugte Rührzeit beträgt 8 bis 30 Stunden.

In den obigen Formeln stellen X1 CH, X2 C, und X3 N dar; haben SG1 und L1 die gleiche Bedeutung wie oben beschrieben, und L2, L3 und L4 die gleiche Bedeutung wie L1; und R und K haben die gleiche Bedeutung wie oben beschrieben.

AK1 ist ein über einen Cysteinrest gekoppelter anti-TWEAKR-Antikörper, ein anti-EGFR-Antikörper, ein anti-B7H3-Antikörper oder ein anti-HER2-Antikörper oder ein Antigen-bindendes Fragment von diesen, und AK2 ist ein über einen Lysinrest gekoppelter anti-TWEAKR-Antikörper, ein anti-EGFR-Antikörper, ein anti-B7H3-Antikörper oder ein anti-HER2-Antikörper oder ein Antigen-bindendes Fragment von diesen. Besonders bevorzugt sind AK1 und AK2 die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

***Weitere Definitionen***

Der Ausdruck "Transglutaminase", austauschbar auch verwendet als "TGase" oder "TG", wird verstanden als ein Enzym, welches die Fähigkeit besitzt, Proteine durch eine Acyl-Transfer-Reaktion zwischen der γ-Carboxamid-Gruppe von peptidgebundenem Glutamin und der ε-Aminogruppe von Lysin oder einem strukturell verwandten primären Amin, so wie zum Beispiel einer Aminopentylgruppe oder zum Beispiel einem peptidgebundenen Lysin, zu verknüpfen, was in einer 8-(γ-glutamyl)-Lysin-Isopeptidbindung resultiert. TGasen schließen unter anderem bakterielle Transglutaminase (BTG) wie zum Beispiel das Enzym mit der EC Referenznummer 2.3.2.13 (Protein-Glutamin-γ-Glutamyltransferase) ein.

Der Ausdruck Akzeptorglutamin meint, wenn er bezogen ist auf einen Aminosäurerest eines Antikörpers, einen Glutaminrest, der, unter geeigneten Bedingungen, durch eine Transglutaminase erkannt wird und transglutaminasekatalysiert durch eine Reaktion zwischen ebendiesem Glutamin und einem Lysin oder einem strukturell verwandten primären Amin, wie zum Beispiel einer Aminopentylgruppe, mit diesem verknüpft werden kann. Gegebenenfalls ist das Akzeptor-Glutamin ein oberflächen-exponiertes Glutamin.

Mit "Aminosäuremodifikation" oder "Mutation" ist hier eine Aminosäuresubstitution, - insertion, und/oder eine -deletion in einer Polypeptidsequenz gemeint. Die bevorzugte Aminosäuremodifikation ist hier eine Substitution. Mit "Aminosäuresubstitution" oder "Substitution" ist hier ein Austausch einer Aminosäure an einer gegebenen Position in einer Proteinsequenz durch eine andere Aminosäure gemeint. Zum Beispiel beschreibt die Substitution Y50W eine Variante eines parentalen Polypeptids, in welcher das Tyrosin an Position 50 durch ein Tryptophan ausgetauscht ist. Eine "Variante" von einem Polypeptid beschreibt ein Polypeptid, welches eine Aminosäuresequenz hat, die substanziell identisch zu einem Referenzpolypeptid ist, typischerweise einem nativen oder "parentalen" Polypeptid. Die Polypeptidvariante kann eine oder mehrere Aminosäureaustauche, -deletionen, und/oder -insertionen an bestimmten Positionen in der nativen Aminosäuresequenz aufweisen.

Der Ausdruck "konjugationsstellenspezifisches Konjugat" beschreibt ein Konjugat eines Binders, vorzugsweise eines Antikörpers, und einem Rest, vorzugsweise einem Linker-Wirkstoff-Rest, wobei der Binder an einer oder mehreren definierten Positionen, vorzugsweise Glutamin-Resten, funktionalisiert ist. Transglutaminasen (TGasen, TGases), beinhaltend bakterieller Transglutaminase (BTG) (EC 2.3.2.13), zeigen starke Spezifität in der Erkennung von Glutamin-Protein-Substraten und können eine "konjugationsstellenspezifische Konjugation" katalysieren.

Der Ausdruck "homogenes Konjugat" oder "homogenes ADC" beschreibt ein Gemisch von konjugationsstellenspezifischen Konjugaten wobei mindestens 60, 70, 80, oder 90 % der Binder dieselbe Anzahl von konjugierten Resten pro Binder haben. Im Fall eines Antikörpers sollte diese Anzahl eine gerade Zahl sein, vorzugsweise 2 oder 4.

***Isotope, Salze, Solvate, isotopische Varianten***

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden.

Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

**Besondere Ausführungsformen**

Die folgenden Ausführungsformen sind besonders bevorzugt:

Ausführungsform A:

Ein APDC der Formeln IVa' bzw. IVa" bzw. IVa''', wobei $D_1$ in den Formeln IVa' bzw. IVa" bzw. IVa''' eine Verbindung der Formel (IIe)

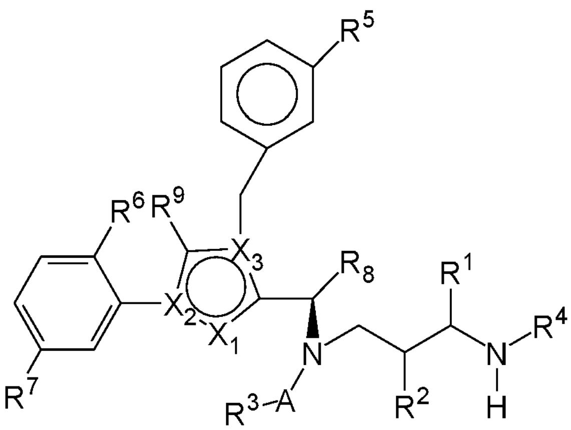

(IIe)

ist, in der

$X_1$ für N,
$X_2$ für N und
$X_3$ für C steht

oder

$X_1$ für CH steht,
$X_2$ für C steht und
$X_3$ für N steht

oder

$X_1$ für NH steht,
$X_2$ für C steht und
$X_3$ für C steht

oder

$X_1$ für CH steht,
$X_2$ für N steht und
$X_3$ für C steht,

A für -C(=O)- steht,

$R^1$ für -L-#1, -H, -COOH, -C(=O)-NHNH$_2$, -(CH$_2$)$_{1-3}$NH$_2$, -C(=O)-NZ"(CH$_2$)$_{1-3}$NH$_2$ und -C(=O)-NZ"CH$_2$-COOH steht,

Z" für -H oder -NH$_2$ steht,

$R^2$ für -H steht,

$R^4$ für eine Gruppe der Formel (Ia) steht,

$R^3$ für -L-#1 oder eine C$_{1-10}$-Alkyl-Gruppe steht, die gegebenenfalls mit -OH, -O-Alkyl, -SH, -S-Alkyl, -O-C(=O)-Alkyl, -O-C(=O)-NH-Alkyl, -NH-C(=O)-Alkyl, -NH-C(=O)-NH-Alkyl, -S(=O)$_n$-Alkyl, -S(=O)$_2$-NH-Alkyl, -NH-Alkyl, -N(Alkyl)$_2$, oder -NH$_2$ substituiert sein kann,

$R^5$ für -H oder -F steht,

$R^6$ und $R^7$ unabhängig voneinander für -H, C$_{1-3}$-Alkyl, Fluor- C$_{1-3}$-Alkyl, C$_{2-4}$-Alkenyl-, Fluor- C$_{2-4}$-Alkenyl-, C$_{2-4}$-Alkinyl-, Fluor- C$_{2-4}$-Alkinyl-, Hydroxy- oder Halogen- stehen,

$R^8$ für eine verzweigte C$_{1-5}$-Alkyl-Gruppe oder Cyclohexyl-Gruppe steht,

$R^9$ für -H oder -F steht,

-L-#1 für die Linker-Gruppe

§-(C(=O))$_m$-L1-L2-§§

steht, in der
m 0 oder 1 ist,
§ die Bindung an den KSP-Inhibitor darstellt,
§§ die Bindung an den Antikörper darstellt,
L2 für eine der Gruppen

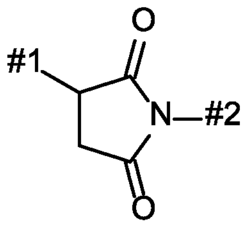 , 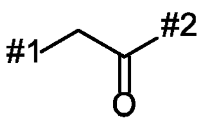 , 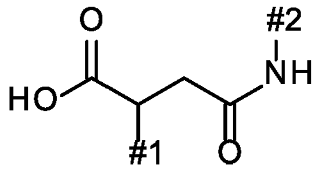 ,

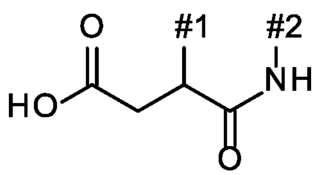 , 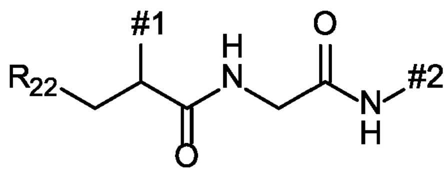

oder

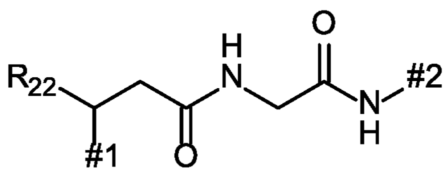

steht,
#[1] für die Verknüpfungsstelle mit dem Schwefelatom des Antikörpers steht,
#[2] für die Verknüpfungsstelle mit der Gruppe L1 steht,
L1 für die Gruppe

#$^{1-}$(NR$^{10}$)$_n$-(G1)$_o$-G2-#$^2$

steht, in der

$R^{10}$ für -H, $-NH_2$ oder $C_{1-3}$-Alkyl- steht,

G1 für-NHC(=O)- oder steht,

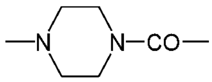

steht,

n 0 oder 1 ist,

o 0 oder 1 ist und

G2 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen steht, die einfach oder mehrfach, gleich oder verschieden durch -O-, -S-, -S(=O)-, $-S(=O)_2$, -NH-, -C(=O)-, -NH-C(=O)-, -C(=O)-NH-, -NMe-, -NHNH-, $-S(=O)_2$-NHNH-, -C(=O)-NHNH- und einen 3 bis 10gliedrigen, aromatischen oder nicht aromatischen Heterozyklus mit bis zu 4 Heteroatomen ausgewählt aus =N-, -O- und -S-, oder -S(=O)- unterbrochen sein kann, wobei die geradkettige oder verzweigte Kohlenwasserstoffkette mit $-NH-C(=O)-NH_2$, -COOH, -OH, $-NH_2$, Sulfonamid, Sulfon, Sulfoxid, oder Sulfonsäure substituiert sein kann,

$\#^1$ für die Bindung zum KSP-Inhibitor steht,

$\#^2$ für die Bindung zu L2 zum Antikörper steht,

wobei einer der Substituenten $R^1$ und $R^3$ für die Linker-gruppe -L-#1 steht, sowie deren Salze, Solvate und Salze der Solvate und wobei die in den Formeln IVa′ bzw. IVa″ bzw. IVa‴genannten Antikörper humane, humanisierte oder chimäre monoklonale Antikörper oder ein Antigen-bindendes Fragment hiervon sind und n in den Formeln IVa′ bzw. IVa″ bzw. IVa‴ eine Zahl von 1 bis 10 ist.

Vorzugsweise ist hierbei der Antikörper ein anti-TWEAKR-Antikörper, ein anti-EGFR-Antikörper ein anti-B7H3-Antikörper oder ein anti-HER2-Antikörper oder ein Antigen-bindendes Fragment von diesen.

Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Hierbei bevorzugt sind solche Verbindungen der Formel (IIe) in denen$R_3$ in der Bedeutung von Alkyl vorzugsweise für $C_{1-3}$ Alkyl- steht.

Hierbei steht G2 vorzugsweise für

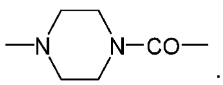

Alternativ kann der Linker -L-#1 an eine Lysin-Seitenkette bzw. einen Lysinrest gebunden sein. Dann, weist er vorzugsweise die folgende Formel

$$-\S-(SG)_x-L4-C(=O)-\S\S,$$

auf, in der

§ die Bindung an den KSP-Inhibitor darstellt,

§§ die Bindung an den Antikörper darstellt,

x 0 oder 1 ist,

SG für eine spaltbare Gruppe steht,

L4 für eine Einfachbindung oder eine Gruppe

$$-(C(=O))_y-G4-$$

steht,

y 0 oder 1 ist und

G4 für eine geradkettige oder verzweigte Kohlenwasserstoffkette mit 1 bis 100 Kohlenstoffatomen aus Arylengruppen

und/oder geradkettigen und/oder verzweigte und/oder cyclischen Alkylengruppen steht, die einfach oder mehrfach gleich oder verschieden durch -O-, -S-, -S(=O)-, $-S(=O)_2-$, -NH-, -C(=O)-, -NH-C(=O)-, -C(=O)-NH-, -NMe-, -NHNH-, $-S(=O)_2$-NHNH-,-C(=O)-NHNH- und einen 5 bis 10-gliedriger, aromatischen oder nicht aromatischen Heterozyklus mit bis zu 4 Heteroatomen ausgewählt aus =N-, -O- und -S-, -S(=O)- oder $-S(=O)_2$-unterbrochen sein kann , wobei die geradkettige oder verzweigte Kohlenwasserstoffkette mit-NH-C(=O)-$NH_2$, -COOH, -OH, $-NH_2$, Sulfonamid, Sulfon, Sulfoxid oder Sulfonsäure substituiert sein kann.

Hierbei steht SG vorzugsweise für ein 2-8 Oligopeptid, besonders bevorzugt für ein Dipeptid.

Vorzugsweise kann die geradkettige oder verzweigte Kohlenstoffkette von G4 durch

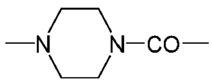

unterbrochen sein.

<u>Ausführungsform B:</u>

Ein APDC der Formeln IVa′ bzw. IVa″ bzw. IVa‴, wobei $D_1$ in den Formeln IVa′ bzw. IVa″ bzw. IVa‴ eine Verbindung der Formel (IIf)

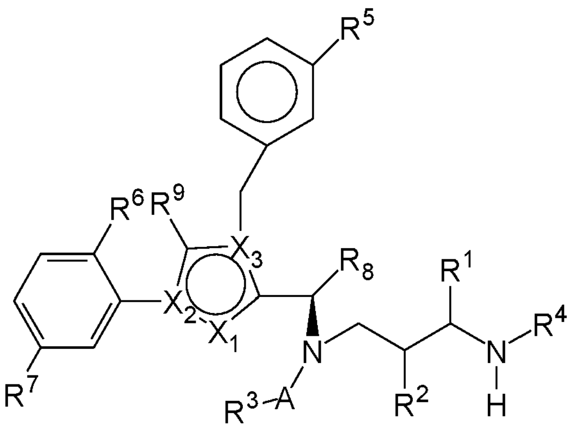

(IIf)

ist, in der

$X_1$ für N steht,
$X_2$ für N steht und
$X_3$ für C steht,

oder

$X_1$ für CH steht,
$X_2$ für C steht und
$X_3$ für N steht,

oder

$X_1$ für NH steht,
$X_2$ für C steht und
$X_3$ für C steht,

oder

$X_1$ für CH steht,

$X_2$ für N steht und
$X_3$ für C steht,

A für -C(=O)- steht,

$R^1$ für -L-#1, -H, -COOH, $-C(=O)-NHNH_2$, $-(CH_2)_{1-3}NH_2$, $-C(=O)-NZ''(CH_2)_{1-3}NH_2$ und $-C(=O)-NZ''CH_2C(=O)-OH$ steht,
Z" für -H oder $-NH_2$ steht,
$R^2$ für -H steht,
$R^4$ für eine Gruppe der Formel (Ia) steht,
$R^3$ für -L-#1 oder eine $C_{1-10}$-Alkyl-Gruppe steht, die gegebenenfalls mit -OH, -O-Alkyl, -SH, -S-Alkyl, -O-C(=O)-Alkyl, -O-C(=O)-NH-Alkyl, -NH-C(=O)-Alkyl, -NH-C(=O)-NH-Alkyl, $-S(=O)_n$-Alkyl, $-S(=O)_2$-NH-Alkyl, -NH-Alkyl, $-N(Alkyl)_2$ und $-NH_2$ substituiert sein kann,
$R^5$ für -H oder -F steht,
$R^6$ und $R^7$ unabhängig voneinander für-H, $C_{1-3}$-Alkyl, Fluor- $C_{1-3}$-Alkyl, $C_{2-4}$-Alkenyl-, Fluor- $C_{2-4}$-Alkenyl-, $C_{2-4}$-Alkinyl-, Fluor- $C_{2-4}$-Alkinyl-, Hydroxy- oder Halogen- stehen,
$R^8$ für eine verzweigte $C_{1-5}$-Alkyl-Gruppe steht,
$R^9$ für -H oder -F steht,
-L-#1 für die Gruppe

$$§-(C(=O))_m-L1-L2-§§$$

steht, in der
m 0 oder 1 ist;
§ die Bindung an den KSP-Inhibitor darstellt,
§§ die Bindung an den Antikörper darstellt,
L2 für die Gruppe

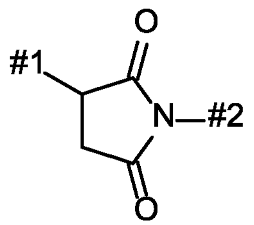
,
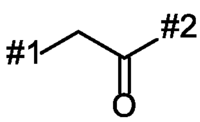
,
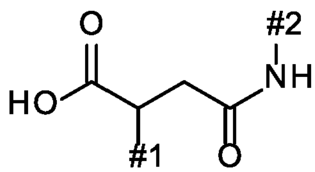
,

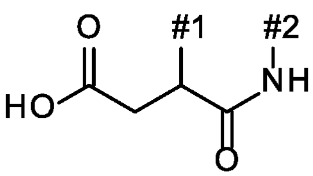
,
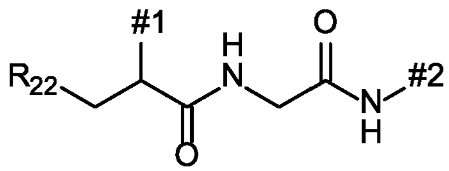

oder

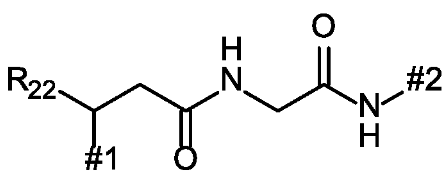

steht,
$\#^1$ für die Verknüpfungsstelle mit dem Schwefelatom des Antikörpers steht,
$\#^2$ für die Verknüpfungsstelle mit der Gruppe L1 steht,
L1 für die Gruppe

$$\#^1\text{-}(NR^{10})_n\text{-}(G1)_o\text{-}G2\text{-}\#^2$$

steht, in der

$R^{10}$ für -H, $-NH_2$ oder $C_{1\text{-}C3}$-Alkyl- steht,

G1 für -NH-C(=O)- oder

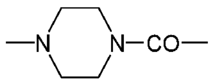

steht,

n 0 oder 1 ist,

o 0 oder 1 ist,

G2 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen steht, die einfach oder mehrfach, gleich oder verschieden durch -O-, -S-, -S(=O)-, $-S(=O)_2$.-, -NH-, -C(=O)-, -NH-C(=O)-, -C(=O)-NH-, -NMe-, -NHNH-, $-S(=O)_2$-NHNH-, -C(=O)-NHNH- und einen 3 bis 10gliedrigen, aromatischen oder nicht aromatischen Heterozyklus mit bis zu 4 Heteroatomen, ausgewählt aus =N-, -O- und -S-, oder -S(=O)- unterbrochen sein kann, wobei die geradkettige oder verzweigte Kohlenwasserstoffkette mit -NH-C(=O)-$NH_2$, -COOH, -OH, $-NH_2$, Sulfonamid, Sulfon, Sulfoxid, oder Sulfonsäure substituiert sein kann,

$\#^1$ für die Bindung zum KSP-Inhibitor steht,

$\#^2$ für die Bindung zu L2 zum Antikörper steht,

wobei einer der Substituenten $R^1$ und $R^3$ für die Linker-Gruppe-L-#1 steht, sowie deren Salze, Solvate und Salze der Solvate und wobei die in den Formeln IVa′ bzw. IVa″ bzw. IVa‴ genannten Antikörper humane, humanisierte oder chimäre monoklonale Antikörper oder ein Antigen-bindendes Fragment hiervon sind und n in den Formeln IVa′ bzw. IVa″ bzw. IVa‴ eine Zahl von 1 bis 10 ist.

Vorzugsweise ist hierbei der Antikörper ein anti-TWEAKR-Antikörper, ein anti-EGFR-Antikörper ein anti-B7H3-Antikörper oder ein anti-HER2-Antikörper oder ein Antigen-bindendes Fragment von diesen.

Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Hierbei bevorzugt sind solche Verbindungen der Formel (IIf) in der $R_3$ in den in der Bedeutung von Alkyl vorzugsweise für $C_{1\text{-}3}$ Alkyl- steht.

Hierbei steht G2 vorzugsweise für

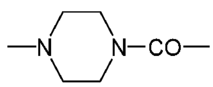

.

Alternativ kann der Linker -L-#1 an eine Lysin-Seitenkette bzw. einen Lysinrest gebunden sein. Dann, weist er vorzugsweise die folgende Formel

$$\text{-§-}(SG)_x\text{-L4-C(=O)-§§}$$

auf, in der

§ die Bindung an den KSP-Inhibitor darstellt,

§§ die Bindung an den Antikörper darstellt,

x 0 oder 1 ist,

SG für eine spaltbare Gruppe steht,

L4 für eine Einfachbindung oder für eine Gruppe

$$-(C{=}O)_y\text{-G4-}$$

-steht,

y 0 oder 1 ist und

G4 für eine geradkettige oder verzweigte Kohlenwasserstoffkette mit 1 bis 100 Kohlenstoffatomen aus Arylengruppen und/oder geradkettigen und/oder verzweigte und/oder cyclischen Alkylengruppen steht, die einfach oder mehrfach gleich oder verschieden durch -O-, -S-, -S(=O)-, -S(=O)$_2$, -NH-, -C(=O)-, -NH-C(=O)-, -C(=O)-NH-, -NMe-, -NHNH-, -S(=O)$_2$-NHNH-, -C(=O)-NHNH- und einen 5 bis 10-gliedrigen, aromatischen oder nicht aromatischen Heterozyklus mit bis zu 4 Heteroatomen, ausgewählt aus =N-, -O- und -S-, -S(=O)- oder -S(=O)$_2$- unterbrochen sein kann, wobei die geradkettige oder verzweigte Kohlenwasserstoffkette mit -NH-C(=O)-NH$_2$, -COOH, -OH, -NH$_2$, Sulfonamid, Sulfon, Sulfoxid oder Sulfonsäure substituiert sein kann.

Hierbei steht SG vorzugsweise für ein 2-8 Oligopeptid, besonders bevorzugt für ein Dipeptid.

Vorzugsweise kann die geradkettige oder verzweigte Kohlenstoffkette von G4 durch

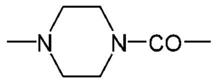

unterbrochen sein.

Ausführungsform C:

Ein APDC der Formel IVa′ bzw. IVa″ bzw. IVa‴, wobei $D_1$ in den Formeln IVa′ bzw. IVa″ bzw. IVa‴ eine Verbindung der Formel (IIg)

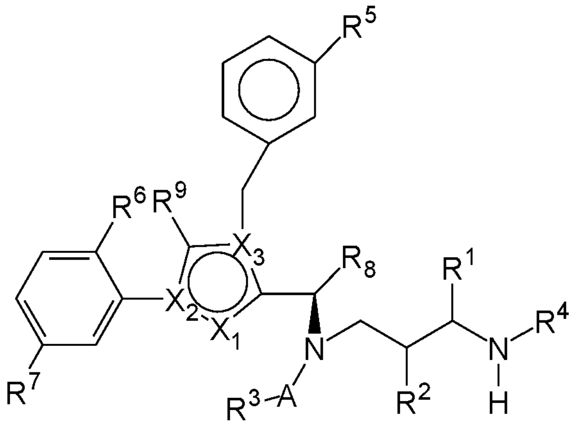

(IIg)

ist, in der

$X_1$ für N,
$X_2$ für N und
$X_3$ für C steht

oder

$X_1$ für CH steht,
$X_2$ für C steht und
$X_3$ für N steht

oder

$X_1$ für NH steht,
$X_2$ für C steht und
$X_3$ für C steht

oder

$X_1$ für CH steht,
$X_2$ für N steht und
$X_3$ für C steht,

A für -C(=O)- steht,

$R^1$ für -L-#1 steht,

$R^2$ für -H steht,
$R^4$ für eine Gruppe der Formel (Ia) steht,
$R^3$ für eine $C_{1-10}$-Alkyl-Gruppe steht, die gegebenenfalls mit -OH, -O-Alkyl, -SH, -S-Alkyl, -O-C(=O)-Alkyl, -O-C(=O)-NH-Alkyl, -NH-C(=O)-Alkyl, -NH-C(=O)-NH-Alkyl, $-S(=O)_n$-Alkyl, $-S(=O)_2$-NH-Alkyl, -NH-Alkyl, $-N(Alkyl)_2$ oder $-NH_2$ substituiert sein kann oder für -MOD steht,
-MOD für die Gruppe

$$-(NR^{10})_n-(G1)_o-G2-H$$

steht,
$R^{10}$ für -H oder $C_1$-$C_3$-Alkyl- steht,
G1 für -NH-C(=O)- , -C(=O)-NH- oder

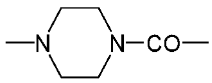

steht
n 0 oder 1 ist,
o 0 oder 1 ist,
G2 für eine geradkettige und/oder verzweigte Kohlenwasserstoffkette mit 1 bis 20 Kohlenstoffatomen steht, die einfach oder mehrfach, gleich oder verschieden durch -O-, -S-, -SO-, $-S(=O)_2$, $-NR^y$-, $-NR^yC(=O)$-, $-C(=O)NR^y$-, $-NR^yNR^y$-, $-S(=O)_2-NR^yNR^y$-, $-C(=O)-NR^yNR^y-C(=O)$- oder $-CR^x=N-O$- unterbrochen sein kann, wobei die geradkettige oder verzweigte Kohlenwasserstoffkette mit $-NH-C(=O)-NH_2$,-COOH, -OH, $-NH_2$, Sulfonamid, Sulfon, Sulfoxid, oder Sulfonsäure substituiert sein kann,
Rx für -H, $C_1$-$C_3$-Alkyl- oder Phenyl- steht,
$R^y$ für-H, Phenyl-, $C_1$-$C_{10}$-Alkyl-, $C_2$-$C_{10}$-Alkenyl- oder $C_2$-$C_{10}$-Alkinyl- steht, die jeweils mit $-NH-C(=O)-NH_2$, -COOH, -OH, $-NH_2$, Sulfonamid, Sulfon, Sulfoxid, oder Sulfonsäure substituiert sein können,
$R^5$ für -H oder -F steht,
$R^6$ und $R^7$ unabhängig voneinander für-H, $C_{1-3}$-Alkyl, Fluor- $C_{1-3}$-Alkyl, $C_{2-4}$-Alkenyl-, Fluor- $C_{2-4}$-Alkenyl-, $C_{2-4}$-Alkinyl-, Fluor- $C_{2-4}$-Alkinyl-, Hydroxy- oder Halogen- stehen,
$R^8$ für eine verzweigte $C_{1-5}$-Alkyl-Gruppe steht,
$R^9$ für -H oder -F steht,
-L-#1 für die Linker-Gruppe

$$§-(C(=O))_m-L1-L2-§§$$

steht, in der
m 0 oder 1 ist,
§ die Bindung an den KSP-Inhibitor darstellt,
§§ die Bindung an den Antikörper darstellt,
L2 für eine der Gruppen

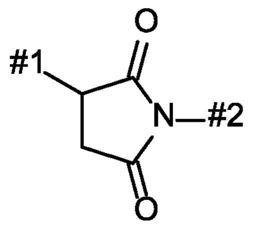 , 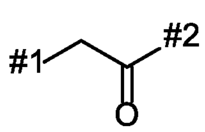 , 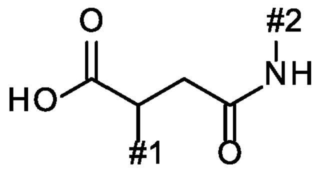 ,

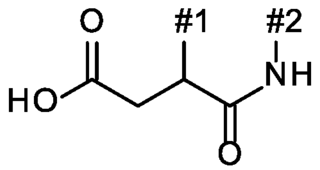 , 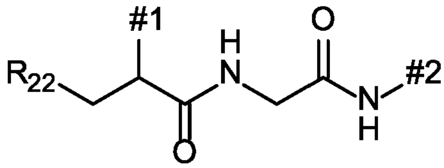

oder

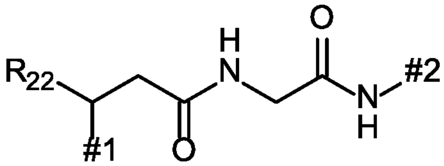

steht,

#[1] für die Verknüpfungsstelle mit dem Schwefelatom des Antikörpers steht,

#[2] für die Verknüpfungsstelle mit der Gruppe L1 steht,

L1 für die Gruppe

$$\#^1\text{-}(NR^{10})_n\text{-}(G1)_o\text{-}G2\text{-}\#^2$$

steht, in der

$R^{10}$ für -H, $-NH_2$ oder $C_{1-3}$-Alkyl- steht,

G1 für-NHC(=O)- oder

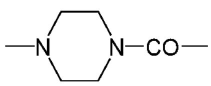

steht,

n 0 oder 1 ist,

o 0 oder 1 ist und

G2 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen steht, die einfach oder mehrfach, gleich oder verschieden durch -O-, -S-, -S(=O)-, $-S(=O)_2$, -NH-, -C(=O)-, -NH-C(=O)-, -C(=O)-NH-, -NMe-, -NHNH-, $-S(=O)_2$-NHNH-, -C(=O)-NHNH- und einen 3 bis 10gliedrigen, aromatischen oder nicht aromatischen Heterozyklus mit bis zu 4 Heteroatomen ausgewählt aus =N-, -O- und -S-, oder -S(=O)- unterbrochen sein kann, wobei die geradkettige oder verzweigte Kohlenwasserstoffkette mit $-NH-C(=O)-NH_2$, -COOH, -OH, $-NH_2$, Sulfonamid, Sulfon, Sulfoxid, oder Sulfonsäure substituiert sein kann,

#[1] für die Bindung zum KSP-Inhibitor steht,

#[2] für die Bindung zu L2 zum Antikörper steht,

sowie deren Salze, Solvate und Salze der Solvate und wobei die in den Formeln IVa′ bzw. IVa″ bzw. IVa‴ genannten Antikörper humane, humanisierte oder chimäre monoklonale Antikörper oder ein Antigen-bindendes Fragment hiervon sind und n in den Formeln IVa′ bzw. IVa″ bzw. IVa‴ eine Zahl von 1 bis 10 ist.

Vorzugsweise ist hierbei der Antikörper ein anti-TWEAKR-Antikörper, ein anti-EGFR-Antikörper ein anti-B7H3-Antikörper oder ein anti-HER2-Antikörper oder ein Antigen-bindendes Fragment von diesen.

Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Hierbei bevorzugt sind solche Verbindungen der Formel (IIg), in der $R^3$ in den in der Bedeutung von Alkyl vorzugsweise für $C_{1-3}$ Alkyl- steht.

Hierbei weist -MOD vorzugsweise zumindest eine COOH-Gruppe auf.

Ausführungsform D:

Ein APDC der Formeln IVa′ bzw. IVa″ bzw. IVa‴, wobei $D_1$ in den Formeln IVa′ bzw. IVa″ bzw. IVa‴ eine Verbindung Formel (IIh)

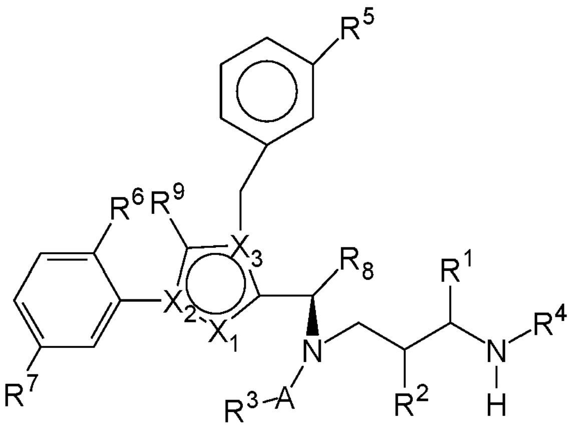

(IIh)

ist, in der

$X_1$ für N,
$X_2$ für N und
$X_3$ für C steht

oder

$X_1$ für CH steht,
$X_2$ für C steht und
$X_3$ für N steht

oder

$X_1$ für NH steht,
$X_2$ für C steht und
$X_3$ für C steht

oder

$X_1$ für CH steht,
$X_2$ für N steht und
$X_3$ für C steht,
A für -C(=O)- steht,
$R^1$ für -H oder -COOH steht,

| | |
|---|---|
| $R^2$ | für -H steht, |
| $R^4$ | für eine Gruppe der Formel Ia steht, |
| $R^3$ | für -L-#1 steht, |
| $R^5$ | für -H oder -F steht, |
| $R^6$ und $R^7$ | unabhängig voneinander für -H, $C_{1-3}$-Alkyl, Fluor- $C_{1-3}$-Alkyl, $C_{2-4}$-Alkenyl-, Fluor- $C_{2-4}$-Alkenyl-, $C_{2-4}$-Alkinyl-, Fluor- $C_{2-4}$-Alkinyl-, Hydroxy- oder Halogen- stehen, |
| $R^8$ | für eine verzweigte $C_{1-5}$-Alkyl-Gruppe steht, |
| $R^9$ | für -H oder -F steht, |
| -L-#1 | für die Linker-Gruppe |

$$§\text{-}(C(=O))_m\text{-}L1\text{-}L2\text{-}§§$$

steht, in der

| | |
|---|---|
| m | 0 oder 1 ist, |
| § | die Bindung an den KSP-Inhibitor darstellt, |
| §§ | die Bindung an den Antikörper darstellt, |
| L2 | für eine der Gruppen |

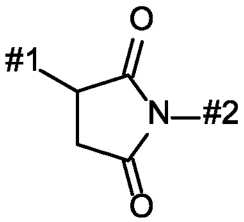
,
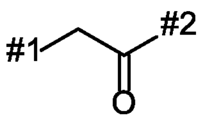
,
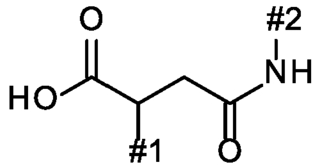
,

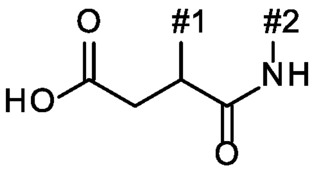
,
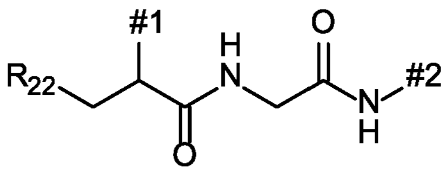

oder

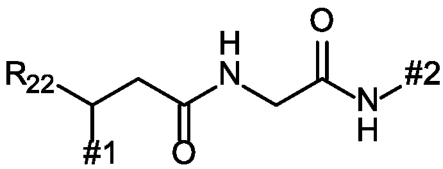

steht,

| | |
|---|---|
| $\#^1$ | für die Verknüpfungsstelle mit dem Schwefelatom des Antikörpers steht, |
| $\#^2$ | für die Verknüpfungsstelle mit der Gruppe L1 steht, |
| L1 | für die Gruppe |

$$\#^1\text{-}(NR^{10})_n\text{-}(G1)_o\text{-}G2\text{-}\#^2$$

steht, in der

| | |
|---|---|
| $R^{10}$ | für -H, $-NH_2$ oder $C_{1-3}$-Alkyl- steht, |
| G1 | für -NHC(=O)- oder |

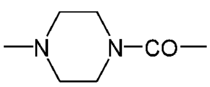

steht,

n 0 oder 1 ist,

o 0 oder 1 ist und

G2 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen steht, die einfach oder mehrfach, gleich oder verschieden durch -O-, -S-, -S(=O)-, $-S(=O)_2-$, -NH-, -C(=O)-, -NH-C(=O)-, -C(=O)-NH-, -NMe-, -NHNH-, $-S(=O)_2-NHNH-$, -C(=O)-NHNH- und einen 3 bis 10gliedrigen, aromatischen oder nicht aromatischen Heterozyklus mit bis zu 4 Heteroatomen ausgewählt aus =N-, -O- und -S-, oder -S(=O)- unterbrochen sein kann, wobei die geradkettige oder verzweigte Kohlenwasserstoffkette mit $-NH-C(=O)-NH_2$, -COOH, -OH, $-NH_2$, Sulfonamid, Sulfon, Sulfoxid, oder Sulfonsäure substituiert sein kann,

$\#^1$ für die Bindung zum KSP-Inhibitor steht,

$\#^2$ für die Bindung zu L2 zum Antikörper steht, sowie deren Salze, Solvate und

Salze der Solvate und wobei die in den Formeln IVa′ bzw. IVa″ bzw. IVa‴genannten Antikörper humane, humanisierte oder chimäre monoklonale Antikörper oder ein Antigen-bindendes Fragment hiervon sind und n in den Formeln IVa′ bzw. IVa″ bzw. IVa‴ eine Zahl von 1 bis 10 ist.

Vorzugsweise ist hierbei der Antikörper ein anti-TWEAKR-Antikörper, ein anti-EGFR-Antikörper ein anti-B7H3-Antikörper oder ein anti-HER2-Antikörper oder ein Antigen-bindendes Fragment von diesen.

Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

Hierbei steht G2 vorzugsweise für

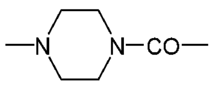

.

Ausführungsform E:

Ein APDC der Formeln IVa′ bzw. IVa" bzw. IVa"', wobei $D_1$ in den Formeln IVa′ bzw. IVa″ bzw. IVa‴ eine Verbindung Formel (IIi)

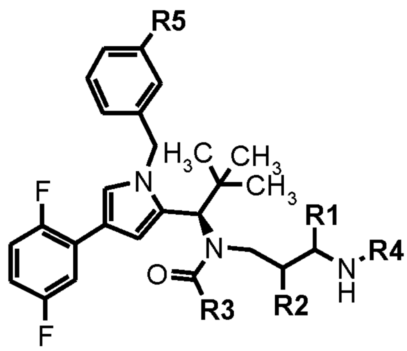

(IIi)

ist, in der

$R^1$ für -L-#1 steht,

-L-#1 für die Linker-Gruppe

$$§-(C(=O))_m-L1-L2-§§$$

steht, in der

m 0 oder 1 ist,

§ die Bindung an den KSP-Inhibitor darstellt,

§§ die Bindung an den Antikörper darstellt,

L2 für die Gruppe

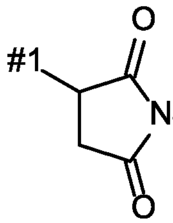,
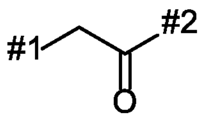,
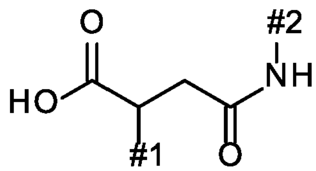,

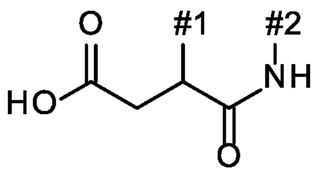,
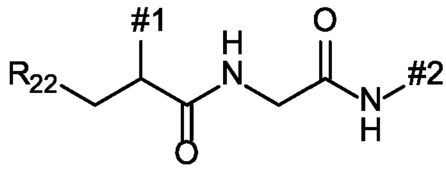

oder

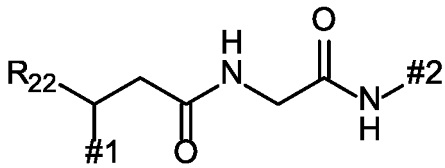

steht,

$R^{22}$ für -COOH, -C(=O)-O-$C_{1-3}$-Alkyl, -C(=O)-$C_{1-3}$-Alkyl, -C(=O)-NH-$C_{1-3}$-Alkyl oder -C(=O)-$NH_2$ steht,

$\#^1$ für die Verknüpfungsstelle mit dem Schwefelatom des Antikörpers steht,

$\#^2$ für die Bindung zu L1 steht,

L1 für die Gruppe

$$\#^1\text{-}(NR^{10})_n\text{-}(G1)_o\text{-}G2\text{-}\#^2$$

steht, in der

$R^{10}$ für-H steht,

G1 für -NHC(=O)- oder

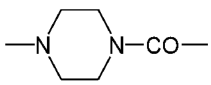

steht,

n 0 oder 1 ist,

o 0 oder 1 ist,

G2 für $C_{1-3}$-Alkyl steht,

$\#^1$ für die Bindung zum KSP-Inhibitor steht,

$\#^2$ für die Bindung zu L2 zum Antikörper steht,

$R^2$und $R^5$ für -H stehen,

$R^3$ für -$CH_2OH$ steht und

$R^4$ für eine Gruppe der Formel (Ia) steht,

sowie deren Salze, Solvate und Salze der Solvate und wobei die in den Formeln IVa′ bzw. IVa″ bzw. IVa‴genannten Antikörper humane, humanisierte oder chimäre monoklonale Antikörper oder ein Antigen-bindendes Fragment hiervon sind und n in den Formeln IVa′ bzw. IVa″ bzw. IVa‴ eine Zahl von 1 bis 10 ist.

Bevorzugt sind solche Verbindungen der Formel (IIi), in der $R^{22}$ für-COOH steht.

In einem erfindungsgemäßen Konjugat bzw. in einem Gemisch der erfindungsgemäßen Konjugate liegen die Bindungen an einen Cysteinrest des Antikörpers, jeweils bezogen auf die Gesamtanzahl von Bindungen des Linkers an den Antikörper, vorzugsweise zu über 80 %, besonders bevorzugt zu über 90 % vor.

Hierbei besonders bevorzugt sind erfindungsgemäß Konjugate, die als L2 die Gruppe

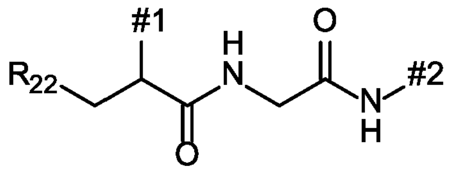

oder

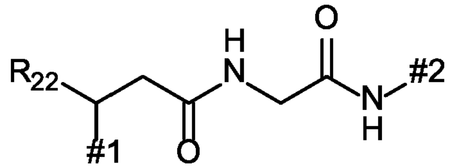

aufweisen, in denen $R^{22}$ die oben angegebenen Bedeutungen hat.

In der Regel liegen Konjugate mit beiderlei L2 Gruppen vor, vorzugsweise in einem Verhältnis von 60:40 bis 40:60 bezogen auf die Anzahl der Bindungen an den Antikörper.

Die restlichen Bindungen liegen dann mit der Struktur

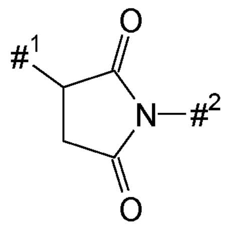

vor, in der#1 und #2 die oben angegebenen Bedeutungen haben

Vorzugsweise ist hierbei der Antikörper ein anti-TWEAKR-Antikörper, ein anti-EGFR-Antikörper ein anti-B7H3-Antikörper oder ein anti-HER2-Antikörper oder ein Antigen-bindendes Fragment von diesen.

Besonders bevorzugt sind die anti-TWEAKR-Antikörper TPP-7006, TPP-7007, TPP-10336 und TPP-10337, die anti-B7H3-Antikörper TPP-8382 und TPP-8567, der anti-EGFR-Antikörper Cetuximab (TPP-981) und die anti-HER2-Antikörper Trastuzumab und TPP-1015 oder eine Antigen-bindendes Fragment von diesen.

## Therapeutische Verwendungen

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (Mammakarzinome einschließlich ductaler und lobulärer Formen, auch *in situ*), Atemwegstumore (kleinzelliges und nicht-kleinzelliges Karzinom, Bronchialkarzinome), Hirntumore (z.B. des Hirnstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischthepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome

und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Die Behandlung der zuvor genannten Krebserkrankungen mittels der erfindungsgemäßen Verbindungen umfasst sowohl eine Behandlung der soliden Tumore als auch eine Behandlung metastasierter oder zirkulierender Formen hiervon.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten antihyperproliferativen, zytostatischen oder zytotoxischen Substanzen zur Behandlung von Krebserkrankungen kombiniert werden. Als geeignete Kombinationswirkstoffe seien beispielhaft genannt:

1311-chTNT, Abarelix, Abirateron, Aclarubicin, Adalimumab, Ado-Trastuzumab Emtansin, Afatinib, Aflibercept, Aldesleukin, Alemtuzumab, Alendronsäure, Alitretinoin, Altretamin, Amifostine, Aminoglutethimid, Hexyl-5-Aminolevulinat, Amrubicin, Amsacrin, Anastrozol, Ancestim, Anethole dithiolethione, Anetumab Ravtansin, Angiotensin II, Antithrombin III, Aprepitant, Arcitumomab, Arglabin, Arsentrioxid, Asparaginase, Atezolizumab, Axitinib, Azacitidin, Belotecan, Bendamustin, Besilesomab, Belinostat, Bevacizumab, Bexaroten, Bicalutamid, Bisantren, Bleomycin, Blinatumomab, Bortezomib, Buserelin, Bosutinib, Brentuximab Vedotin, Busulfan, Cabazitaxel, Cabozantinib, Calcitonin, Calciumfolinat, Calciumlevofolinat, Capecitabin, Capromab, Carbamazepine, Carboplatin, Carboquon, Carfilzomib, Carmofur, Carmustin, Catumaxomab, Celecoxib, Celmoleukin, Ceritinib, Cetuximab, Chlorambucil, Chlormadinon, Chlormethin, Cidofovir, Cinacalcet, Cisplatin, Cladribin, Clodronsäure, Clofarabin, Cobimetinib, Copanlisib, Crisantaspase, Crizotinib, Cyclophosphamid, Cyproteron, Cytarabin, Dacarbazin, Dactinomycin, Daratumumab, Dabrafenib, Dasatinib, Daunorubicin, Decitabin, Degarelix, Denileukin-Diftitox, Denosumab, Depreotid, Deslorelin, Dexrazoxane, Dibrospidiumchlorid, Dianhydrogalactitol, Diclofenac, Docetaxel, Dolasetron, Doxifluridin, Doxorubicin, Doxorubicin + Estron, Dronabinol, Edrecolomab, Elliptiniumacetat, Endostatin, Enocitabin, Enzalutamid, Epirubicin, Epitiostanol, Epoetin-alfa, Epoetin-beta, Epoetin-zeta, Eptaplatin, Eribulin, Erlotinib, Esomeprazol, Estramustin, Etoposid, Ethinylestradiol, Everolimus, Exemestan, Fadrozol, Fentanyl, Fluoxymesteron, Floxuridin, Fludarabin, Fluoruracil, Flutamid, Folinsäure, Formestan, Fosaprepitant, Fotemustin, Fulvestrant, Gadobutrol, Gadoteridol, Gadotersäure-Megluminsalz, Gadoversetamid, Gadoxetsäure Dinatriumsalz (Gd-EOB-DTPA Dinatriumsalz), Galliumnitrat, Ganirelix, Gefitinib, Gemcitabin, Gemtuzumab, Glucarpidase, Glutoxim, Goserelin, Granisetron, Granulocyten Kolonie stimulierender Factor (G-CSF), Granulocyten Macrophagen Kolonie stimulierender Factor (GM-CSF), Histamindihydrochlorid, Histrelin, Hydroxycarbamid, 1-125-Seeds, Ibandronsäure, Ibritumomab-Tiuxetan, Ibrutinib, Idarubicin, Ifosfamid, Imatinib, Imiquimod, Improsulfan, Indisetron, Incadronic acid, Ingenolmebutat, Interferon-alfa, Interferon-beta, Interferon-gamma, Iobitridol, Iobenguane (123I), Iomeprol, Ipilimumab, Irinotecan, Itraconazole, Ixabepilon, Ixazomib,Lanreotid, Lansoprazole, Lansoprazol, Lapatinib, Lasocholine, Lenalidomid, Lenvatinib, Lenograstim, Lentinan, Letrozol, Leuprorelin, Levamisol, Levonorgestrel, Levothyroxin-Natrium, Lipegfilgrastim, Lisurid, Lobaplatin, Lomustin, Lonidamin, Masoprocol, Medroxyprogesteron, Megestrol, Melarsoprol, Melphalan, Mepitiostan, Mercaptopurin, Mesna, Methadon, Methotrexat, Methoxsalen, Methylaminolevulinat, Methylprednisolon, Methyltestosteron, Metirosin, Mifamurtid, Miltefosin, Miriplatin, Mitobronitol, Mitoguazon, Mitolactol, Mitomycin, Mitotan, Mitoxantron, Mogamulizumab, Molgramostim, Mopidamol, Morphinhydrochlorid, Morphinsulfat, Nabilon, Nabiximols, Nafarelin, Naloxon + Pentazocin, Naltrexon, Nartograstim, Necitumumab, Nedaplatin, Nelarabin, Neridronsäure, Netupitant/palonosetron, Nivolumabpentetreotid, Nilotinib, Nilutamid, Nimorazol, Nimotuzumab, Nimustin, Nintedanib, Nitracrin, Nivolumab, Obinutuzumab, Octreotid, Ofatumumab, Olaparib, Olaratumab, Omacetaxin-Mepesuccinat, Omeprazol, Ondansetron, Orgotein, Orilotimod, Osimertinib, Oxaliplatin, Oxycodon, Oxymetholon, Ozogamicin, p53-Gentherapie, Paclitaxel, Palbociclib, Palifermin, Palladium-103-Seed, Palonosetron, Pamidronsäure, Panitumumab, Panobinostat, Pantoprazol, Pazopanib, Pegaspargase, Pembrolizumab, Peg-interferon-alfa-2b, Pembrolizumab, Pemetrexed, Pentostatin, Peplomycin, Perflubutane, Perfosfamid, Pertuzumab, Picibanil, Pilocarpin, Pirarubicin, Pixantron, Plerixafor, Plicamycin, Poliglusam, Polyestradiolphosphat, Polyvinylpyrrolidone + Natriumhyaluronat, Polysaccharid-K, Pomalidomid, Ponatinib, Porfimer-Natrium, Pralatrexat, Prednimustin, Prednison, Procarbazin, Procodazole, Propranolol, Quinagolid, Rabeprazol, Racotumomab, Radium-223-chlorid, Radotinib, Raloxifen, Raltitrexed, Ramosetron, Ramucirumab, Ranimustin, Rasburicase, Razoxan, Refametinib, Regorafenib, Risedronsäure, Rhenium-186 Etidronat, Rituximab, Rolapitant, Romidepsin, Romurtid, Roniciclib, Samarium (153Sm) lexidronam, Satumomab, Secretin, Siltuximab, Sipuleucel-T, Sizofiran, Sobuzoxan, Natriumglycididazol, Sonidegib, Sorafenib, Stanozolol, Streptozocin, Sunitinib, Talaporfin, Talimogen Laherparepvec, Tamibaroten, Tamoxifen, Tapentadol, Tasonermin, Teceleukin, Technetium (99mTc) Nofetumomab Merpentan, 99mTc-HYNIC-[Tyr3]-octreotid, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Thyrotropin alfa, Tioguanin, Tocilizumab, Topotecan, Toremifen, Tositumomab, Trabectedin, Trametinib, Tramadol, Trastuzumab, Treosulfan, Tretinoin, Trifluridine + Tipiracil, Trametinib, Trilostan, Triptorelin, Trofosfamid, Thrombopoietin, Ubenimex, Valrubicin, Vandetanib, Vapreotid, Valatinib, Vemurafenib, Vinblastin, Vincristin, Vindesin, Vinflunin, Vinorelbin, Vismodegib, Vorinostat, Yttrium-90-Glasmikrokugeln, Zinostatin, Zinostatin-Stimalamer, Zoledronsäure, Zorubicin.

Zudem können die Antikörper ausgewählt sein aus der Klasse der MPS1-Inhibitoren oder Antikörper gegen die Targets OX-40, CD137 / 4-1BB, DR3, IDO1 / IDO2, LAG-3, und CD40.

Darüber hinaus können die erfindungsgemäßen Verbindungen auch in Kombination mit einer Strahlentherapie und/oder einer chirurgischen Intervention eingesetzt werden.

Generell können mit der Kombination von Verbindungen der vorliegenden Erfindung mit anderen, zytostatisch oder zytotoxisch wirksamen Agenzien folgende Ziele verfolgt werden:

- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise, parenteral, möglicherweise inhalativ oder als Implantat bzw. Stent.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, vaginal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B.. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen,Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Augentropfen, Augensalben, Augenbäder, okulare Inserte, Ohrentropfen, -sprays, -pulver, -spülungen, -tampons, , Vaginalkapseln, wässrige Suspensionen (Lotionen, Schüttelmixturen), lipophile Suspensionen, Emulsionen, Mikroemulsionen, Salben, Cremes, transdermale therapeutische Systeme (wie beispielsweise Pflaster), Milch, Pasten, Schäume, Streupuder, Implantate oder Stents.

Bevorzugt ist die parenterale Applikation, insbesondere die intravenöse Applikation.

Die erfindungsgemäßen Verbindungen können in die angeführten Applikationsformen überführt werden. Dies kann in an sich bekannter Weise durch Mischen mit pharmazeutisch geeigneten Hilfsstoffen geschehen. Zu diesen Hilfsstoffen zählen u.a.

- Füll- und Trägerstoffe (beispielsweise Cellulose, mikrokristalline Cellulose wie z.B. Avicel®, Laktose, Mannitol, Stärke, Calciumphosphate wie z.B. Di-Cafos®),

- Salbengrundlagen (beispielsweise Vaselin, Paraffine, Triglyceride, Wachse, Wollwachs, Wollwachsalkohole, Lanolin, hydrophile Salbe, Polyethylenglycole),

- Suppositoriengrundlagen (zum Beispiel Polyethylenglycole, Kakaobutter, Hartfett),

- Lösungsmittel (z.B. Wasser, Ethanol, Isopropanol, Glycerol, Propylenglycol, mittelkettige Triglyceride fette Öle, flüssige Polyethylenglycole, Paraffine),

- Tenside, Emulgatoren, Dispergier- oder Netzmittel (beispielsweise Natriumdodecylsulfat, Lecithin, Phospholipide, Fettalkohole wie z.B. Lanette®, Sorbitanfettsäureester wie z.B. Span®, Polyoxy-ethylen-Sorbitanfettsäureester wie z.B. Tween®, Polyoxyethylen-Fettsäureglyceride wie z.B. Cremophor®, Polyoxethlyen-Fettsäureester, Polyoxethlyen-Fettalkoholether, Glycerolfettsäureester, Poloxamere wie z.B. Pluronic®),

- Puffersubstanzen sowie Säuren und Basen (beispielsweise Phosphate, Carbonate, Citronensäure, Essigsäure, Salzsäure, Natronlauge, Ammoniumcarbonat, Trometamol, Triethanolamin),

- Isotonisierungsmittel (beispielsweise Glucose, Natriumchlorid),

- Adsorptionsmittel (beispielsweise hochdisperse Siliciumdioxide),

- Viskositätserhöhende Mittel, Gelbildner, Verdickungs- bzw. Bindemittel (beispielsweise Polyvinylpyrrolidon, Methylcellulose, Hydroxypropylmethylcellulose, Hydroxypropylcellulose, Carboxymethylcellulose-Natrium, Stärke, Carbomere, Polyacrylsäuren wie z.B. Carbopol®, Alginate, Gelatine),

- Sprengmittel (beispielsweise modifizierte Stärke, Carboxymethylcellulose-Natrium, Natriumstärkeglycolat wie z.B. Explotab®, quervernetztes Polyvinylpyrrolidon, Croscarmellose-Natrium wie z.B. AcDiSol®),

- Fließregulier-, Schmier-, Gleit- und Formtrennmittel (beispielsweise Magnesiumstearat, Stearinsäure, Talkum, hochdisperse Siliciumdioxide wie z.B. Aerosil®),

- Überzugsmittel (beispielsweise Zucker, Schellac) sowie Filmbildemittel für sich schnell oder modifiziert auflösende Filme bzw. Diffusionsmembranen (beispielsweise Polyvinylpyrrolidone wie z.B. Kollidon®, Polyvinylalkohol, Hydroxypropylmethylcellulose, Hydroxypropylcellulose, Ethylcellulose, Hydroxypropylmethylcellulosephthalat, Celluloseacetat, Celluloseacetatphthtalat, Polyacrylate, Polymethacrylate wie z.B. Eudragit®),
- Kapselmaterialien (z.B. Gelatine, Hydroxypropylmethylcellulose),
- Synthetische Polymere (beispielsweise Polylactide, Polyglycolide, Polyacrylate, Polymethacrylate wie z.B Eudragit®, Polyvinylpyrrolidone wie z.B. Kollidon®, Polyvinylalcohole, Polyvinylacetate, Polyethylenoxide, Polyethylenglycole und deren Copolymere und Blockcopolymere),
- Weichmacher (beispielsweise Polyethylenglycole, Propylenglykol, Glycerol, Triacetin, Triacetylcitrat, Dibutylphthalat),
- Penetrationsenhancer,
- Stabilisatoren (z.B. Antioxidantien wie beispielsweise Ascorbinsäure, Ascorbylpalmitat, Natriumascorbat, Butylhydroxyanisol, Butylhydroxytoluol, Propylgallat),
- Konservierungsmittel (beispielsweise Parabene, Sorbinsäure, Thiomersal, Benzalkoniumchlorid, Chlorhexidinacetat, Natriumbenzoat),
- Farbstoffe (z.B. anorganische Pigmente wie beispielsweise Eisenoxide, Titandioxid),
- Aromen, Süßungsmittel, Geschmacks- und / oder Geruchskorrigentien.

Ein weiterer Gegenstand der vorliegenden Erfindung sind pharmazeutische Zusammensetzungen, die mindestens eine erfindungsgemäße Verbindung, üblicherweise zusammen mit einem oder mehreren pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 0.1 bis 20 mg/kg, vorzugsweise etwa 0.3 bis 7 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die erfindungsgemäßen Verbindungen können als isotopische Varianten vorliegen. Die Erfindung umfasst daher eine oder mehrere isotopische Varianten der erfindungsgemäßen Verbindungen, insbesondere deuteriumhaltige Verbindungen.

Der Begriff "isotopische Variante" einer Verbindung oder eines Reagenzes ist definiert als eine Verbindung mit einem unnatürlichen Anteil eines oder mehrerer der Isotope, aus denen eine derartige Verbindung aufgebaut ist.

Der Begriff "isotopische Variante der erfindungsgemäßen Verbindungen" ist definiert als eine erfindungsgemäße Verbindung mit einem unnatürlichen Anteil eines oder mehrerer der Isotope, aus denen eine derartige Verbindung aufgebaut ist.

Unter dem Ausdruck "unnatürlicher Anteil" ist ein Anteil eines derartigen Isotops zu verstehen, der höher als dessen natürliche Häufigkeit ist. Die in diesem Zusammenhang anzuwendenden natürlichen Häufigkeiten von Isotopen finden sich in "Isotopic Compositions of the Elements 1997", Pure Appl. Chem., 70(1), 217-235, 1998.

Beispiele für derartige Isotope sind stabile und radioaktive Isotope von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{11}C$, $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$ bzw. $^{131}I$.

Im Hinblick auf die Behandlung und/oder Prophylaxe der hier angegebenen Störungen enthalten die isotopischen Variante(n) der erfindungsgemäßen Verbindungen vorzugsweise Deuterium ("deuteriumhaltige erfindungsgemäße Verbindungen"). Isotopische Varianten der erfindungsggemäßen Verbindungen, in die ein oder mehrere radioaktive Isotope, wie $^{3}H$ oder $^{14}C$, eingebaut sind, sind beispielsweise bei Arzneistoff- und/oder Substratsgewebeverteilungsstudien von Nutzen. Diese Isotope sind wegen ihrer leichten Einbaubarkeit und Nachweisbarkeit besonders bevorzugt. In eine erfindungsgemäße Verbindung können Positronen emittierende Isotope wie $^{18}F$ oder $^{11}C$ eingebaut werden. Diese isotopischen Varianten der erfindungsgemäßen Verbindungen eignen sich zur Verwendung bei in-vivo-Bildge-

bungsanwendungen. Deuteriumhaltige und $^{13}C$-haltige erfindungsgemäße Verbindungen können im Rahmen präklinischer oder klinischer Studien bei Massenspektrometrie-Analysen verwendet werden.

Isotopische Varianten der erfindungsgemäßen Verbindungen können im Allgemeinen nach dem Fachmann bekannten Verfahren wie den in den hier beschriebenen Schemata und/oder Beispielen beschrieben hergestellt werden, indem man ein Reagenz durch eine isotopische Variante des Reagenzes, vorzugsweise ein deuteriumhaltiges Reagenz, ersetzt. Je nach den gewünschten Deuterierungsstellen kann in einigen Fällen Deuterium aus $D_2O$ entweder direkt in die Verbindungen oder in Reagenzien, die für die Synthese derartiger Verbindungen verwendet werden können, eingebaut werden. Ein nützliches Reagenz zum Einbau von Deuterium in Moleküle ist auch Deuteriumgas. Eine schnelle Route zum Einbau von Deuterium ist die katalytische Deuterierung von olefinischen Bindungen und acetylenischen Bindungen. Zum direkten Austausch von Wasserstoff gegen Deuterium in funktionelle Gruppen enthaltenden Kohlenwasserstoffen können auch Metallkatalysatoren (d.h. Pd, Pt und Rh) in Gegenwart von Deuteriumgas verwendet werden. Verschiedene deuterierte Reagenzien und Synthesebausteine sind im Handel von Firmen wie beispielsweise C/D/N Isotopes, Quebec, Kanada; Cambridge Isotope Laboratories Inc., Andover, MA, USA; und CombiPhos Catalysts, Inc., Princeton, NJ, USA, erhältlich.

Der Begriff "deuteriumhaltige Verbindung" ist definiert als eine erfindungsgemäße Verbindung, in der ein oder mehrere Wasserstoffatome durch ein oder mehrere Deuteriumatome ersetzt sind und in der die Häufigkeit von Deuterium in jeder deuterierten Position der Verbindung der allgemeinen Formel (I) höher ist als die natürliche Häufigkeit von Deuterium, die etwa 0,015% beträgt. Insbesondere ist in einer deuteriumhaltigen erfindungsgemäßen Verbindung die Häufigkeit von Deuterium in jeder deuterierten Position der Verbindung der allgemeinen Formel (I) höher als 10%, 20%, 30%, 40%, 50%, 60%, 70% oder 80%, vorzugsweise höher als 90%, 95%, 96% oder 97%, noch weiter bevorzugt höher als 98% oder 99% in dieser Position bzw. diesen Positionen. Es versteht sich, dass die Häufigkeit von Deuterium in jeder deuterierten Position von der Häufigkeit von Deuterium in anderen deuterierten Positionen unabhängig ist.

Durch den selektiven Einbau eines oder mehrerer Deuteriumatome in eine erfindungsgemäße Verbindung können die physikalisch-chemischen Eigenschaften (wie beispielsweise Acidität [C. L. Perrin, et al., J. Am. Chem. Soc., 2007, 129, 4490], Basizität [C. L. Perrin et al., J. Am. Chem. Soc., 2005, 127, 9641], Lipophilie [B. Testa et al., Int. J. Pharm., 1984, 19(3), 271]) und/oder das Stoffwechselprofil des Moleküls verändert und Veränderungen des Verhältnisses von Stammverbindung zu Metaboliten oder der gebildeten Mengen von Metaboliten verursacht werden. Derartige Veränderungen können zu bestimmten therapeutischen Vorteilen führen und daher unter bestimmten Umständen bevorzugt sein. Es ist über verringerte Stoffwechselraten und Stoffwechselumschaltung, bei der sich das Verhältnis von Metaboliten ändert, berichtet worden (A. E. Mutlib et al., Toxicol. Appl. Pharmacol., 2000, 169, 102). Diese Veränderungen der Exposition gegenüber Stammverbindung und Metaboliten können wichtige Konsequenzen hinsichtlich Pharmakodynamik, Verträglichkeit und Wirksamkeit einer deuteriumhaltigen erfindungsgemäßen Verbindung haben. In einigen Fällen wird durch den Deuteriumersatz die Bildung eines unerwünschten oder toxischen Metaboliten verringert oder eliminiert und die Bildung eines gewünschten Metaboliten verstärkt (z.B. Nevirapin: A. M. Sharma et al., Chem. Res. Toxicol., 2013, 26, 410; Efavirenz: A. E. Mutlib et al., Toxicol. Appl. Pharmacol., 2000, 169, 102). In anderen Fällen besteht der Haupteffekt der Deuterierung darin, die Geschwindigkeit der systemischen Clearance zu verringern. Dadurch wird die biologische Halbwertszeit der Verbindung erhöht. Zu den potentiellen klinischen Vorteilen gehören die Fähigkeit zur Aufrechterhaltung einer ähnlichen systemischen Exposition mit verringerten Spitzenspiegeln und erhöhten Talspiegeln. Dies könnte je nach der Pharmakokinetik/Pharmakodynamik-Beziehung der jeweiligen Verbindung zu geringeren Nebenwirkungen und erhöhter Wirksamkeit führen. Beispiele für diesen Deuterium-Effekt sind ML-337 (C. J. Wenthur et al., J. Med. Chem., 2013, 56, 5208) und Odanacatib (K. Kassahun et al., WO2012/112363). Es ist auch noch über weitere Fälle berichtet worden, bei denen verringerte Verstoffwechslungsraten zu einer Erhöhung der Arzneistoffexposition ohne Änderung der Rate der systemischen Clearance führen (z.B. Rofecoxib: F. Schneider et al., Arzneim. Forsch. / Drug. Res., 2006, 56, 295; Telaprevir: F. Maltais et al., J. Med. Chem., 2009, 52, 7993). Deuterierte Arzneistoffe, die diesen Effekt zeigen, können verringerte Dosierungsanforderungen haben (z.B. geringere Zahl von Dosen oder niedrigere Dosierung zur Erzielung der gewünschten Wirkung) und/oder zu geringeren Metabolitenbelastungen führen.

Eine erfindungsgemäßen Verbindungen können mehrere potenzielle Angriffsstellen für die Verstoffwechslung aufweisen. Zur Optimierung der oben beschriebenen Effekte auf die physikalisch-chemischen Eigenschaften und das Stoffwechselprofil können deuteriumhaltige erfindungsgemäße Verbindungen mit einem bestimmten Muster eines oder mehrerer Deuterium-Wasserstoff-Austausche gewählt werden. Insbesondere ist/sind das Deuteriumatom/die Deuteriumatome deuteriumhaltiger erfindungsgemäßer Verbindung(en) an ein Kohlenstoffatom gebunden und/oder steht/stehen in den Positionen der erfindungsgemäßen Verbindungen, bei denen es sich um Angriffsstellen für Verstoffwechslungsenzyme wie z.B. Cytochrom $P_{450}$ handelt.

## Beispiele

Die nachfolgenden Beispiele erläutern die Ausführbarkeit der vorliegenden Erfindung, ohne die Erfindung nur alleine auf diese Beispiele zu beschränken.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

**Synthesewege:**

Exemplarisch zu den Ausführungsbeispielen sind in folgenden Schemata beispielhafte Synthesewege dargestellt.

In diesen Schemata kann gemäß Formel IIa der an der Amino-Gruppe $-NHR^4$ stehende Substituent $R^4$ die Gruppe $Z_1$-(C=O)$_{(0-1)}$-(P3)$_{(0-2)}$-P2-NH-CH($CH_2$C(=O)$NH_2$)-C(=O)- sein.

Hierbei steht

P2 für eine D-Aminosäure ausgewählt aus der Gruppe D-Gly, D-Pro, D-Ala, D-Val, D-Nva, D-Leu, D-Ile, D-Met, D-Phe, D-Tyr, D-Trp, D-Ser, D-Thr, D-Cys, D-Asn, D-Gln, D-Asp, D-Glu, D-Lys, D-Arg, D-Citrullin und D-His,

P3 für eine L- oder D-Aminosäure, ausgewählt aus der Gruppe Gly, Pro, Ala, Val, Nva, Leu, Ile, Met, Phe, Tyr, Trp, Ser, Thr, Cys, Asn, Gln, Asp, Glu, Lys, Arg, Citrullin und His,

$Z_1$ für eine $C_{1-10}$-Alkyl-, $C_{5-10}$-Aryl- oder $C_{6-10}$-Aralkyl-, $C_{5-10}$-Heteroalkyl-, $C_{1-10}$-Alkyl-O-$Cr_{6-10}$-Aryl-, $C_{5-10}$-Heterocycloalkyl-, Heteroaryl-, Heteroaryl-alkyl-, $C_{5-10}$-Heteroaryl-alkoxy-, $C_{1-10}$-Alkoxy-, $C_{6-10}$-Aryloxy-, $Cr_{6-10}$-Aryl-$C_{1-10}$-alkyloxy- oder $C_{6-10}$-Aralkoxy-, $C_{5-10}$-Heteroalkoxy-, $C_{1-10}$-Alkyl-O-$C_{6-10}$-Aryloxy- oder $C_{5-10}$-Heterocycloalkoxy-Gruppe steht, die ein- oder mehrfach mit $-NH_2$, -C(=O)-, -NH-Alkyl, -N(Alkyl)$_2$, -NH-C(=O)-Alkyl, -N(Alkyl)-C(=O)-Alkyl, -S(=O)$_3$-H, -S(=O)$_2$-$NH_2$, -S(=O)$_2$-N(Alkyl)$_2$, -COOH, -C(=O)$NH_2$, -C(=O)-N(Alkyl)$_2$ oder -OH substituiert sein kann, oder für -H oder eine Gruppe $-(CH_2)_{0-1}-O_x-(CH_2CH_2O)_v-R^1$ steht,

x für 0 oder 1,

v für eine Zahl von 1 bis 20 und

$R^1$ die in der Formel (II) angegebenen Bedeutungen hat.

Schema 1:

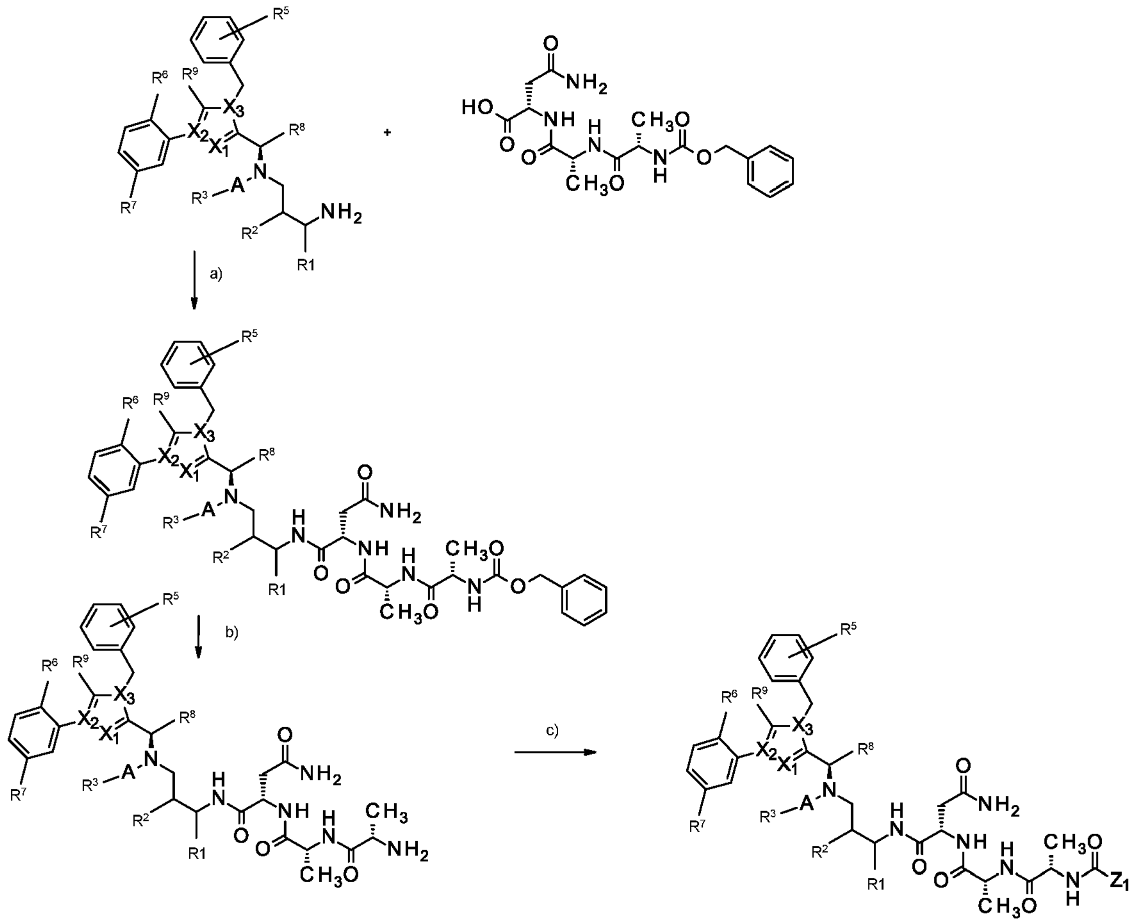


[a): HATU, DMF, N,N-Diisopropylethylamin, RT oder EDCI, HOBT, N,N-Diisopropylethylamin, DMF, RT b) $H_2$, 10% Pd-C, MeOH, RT; c) $Z_1$-COOH, EDCI, HOBT, N,N-Diisopropylethylamin, DMF, RT oder $Z_1$-COOH, HATU, N,N-Diisopropylethylamin, DMF, RT oder $Z_1$-COOSu, N,N-Diisopropylethylamin, DMF, RT]

Weiterhin können andere Intermediate gemäß Schema 2 und 3 in Legumain-spaltbare ADC-Precursor überführt werden:

Schema 2:

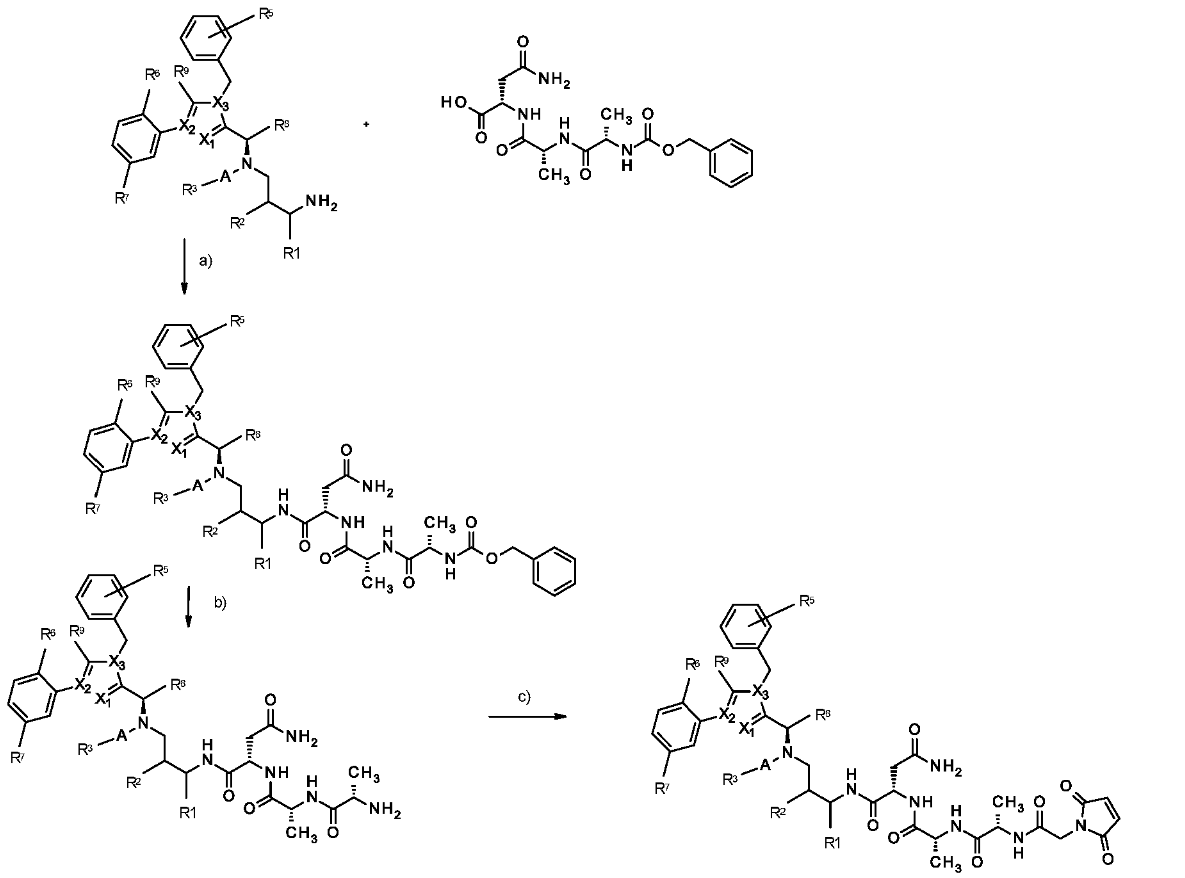


[a): HATU, DMF, N,N-Diisopropylethylamin, RT oder EDCI, HOBT, N,N-Diisopropylethylamin, DMF, RT b) $H_2$, 10% Pd-C, MeOH, RT; c) 1,1'-[(1,5-Dioxopentan-1,5-diyl)bis(oxy)]dipyrrolidin-2,5-dion, N,N-Diisopropylethylamin, DMF, RT]

Schema 3:

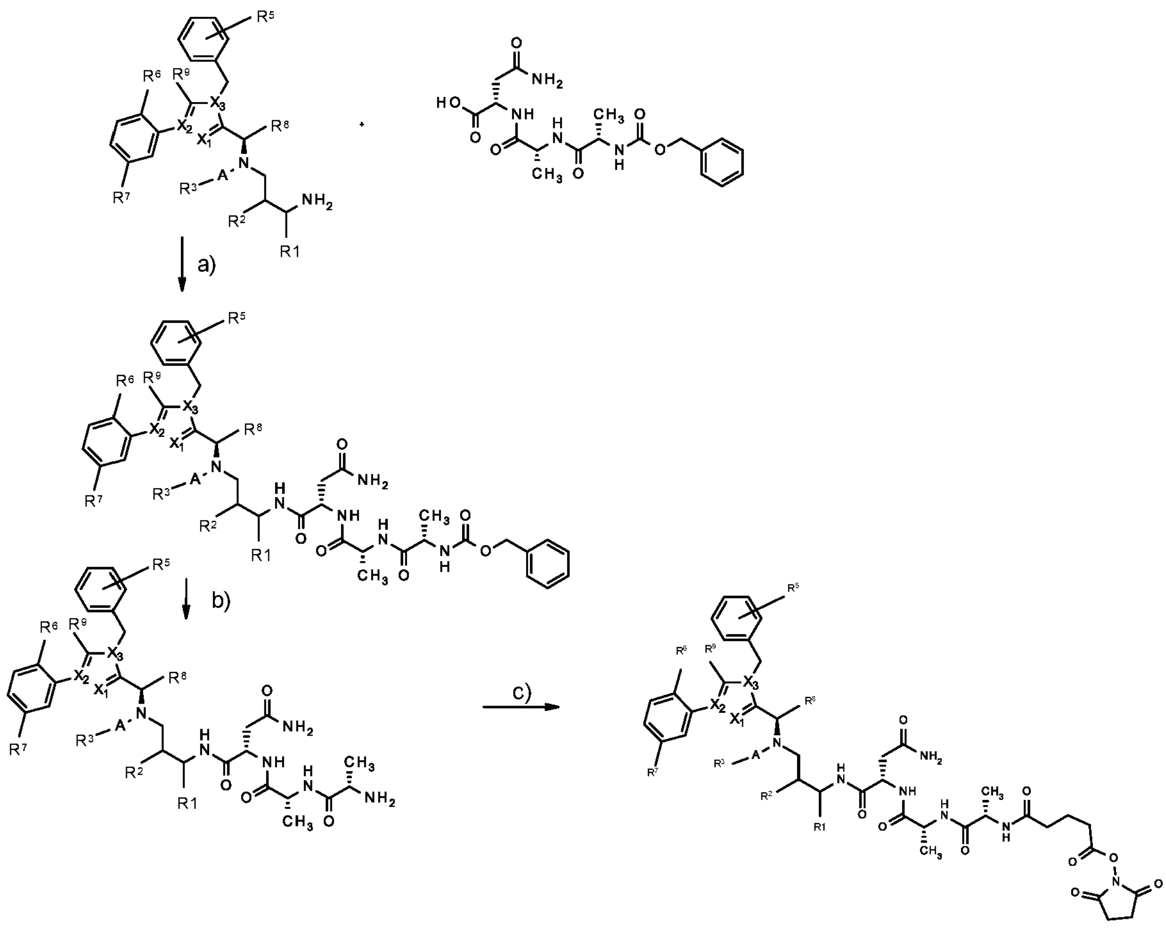


[a): HATU, DMF, N,N-Diisopropylethylamin, RT oder EDCI, HOBT, N,N-Diisopropylethylamin, DMF, RT b) $H_2$, 10% Pd-C, MeOH, RT; c)) 1,1'-[(1,5-Dioxopentan-1,5-diyl)bis(oxy)]dipyrrolidin-2,5-dion, N,N-Diisopropylethylamin, DMF, RT]

Alternativ zu der in Schemata 1-3 dargestellten Benzyloxycarbonyl-Gruppe können andere in der Peptidchemie etablierte Schutzgruppen eingesetzt werden und nach entsprechenden ebenso bekannten Methoden abgespalten werden. Die Auswahl der Schutzgruppenstrategie erfolgt nach entsprechenden dem Fachmann bekannten Anforderungen zur Kompatibilität mit anderen im Molekül vorkommenden Strukturelementen. Falls noch vorhanden können weitere Schutzgruppen im Molekül in einem letzten Schritt entfernt werden.

Die Synthesen können auch ggf. in ihrer Sequenz umgestellt werden.

Weiterhin kann die proteinreaktive Gruppe im Sinne des Linkerstrukturen L1-L2 im Rahmen der Ansprüche variiert werden.

Schema 4: Synthese von ADC-Precurser Molekülen der Intermediatserie F, die gemäß Schema 1 in APDCs überführt werden können

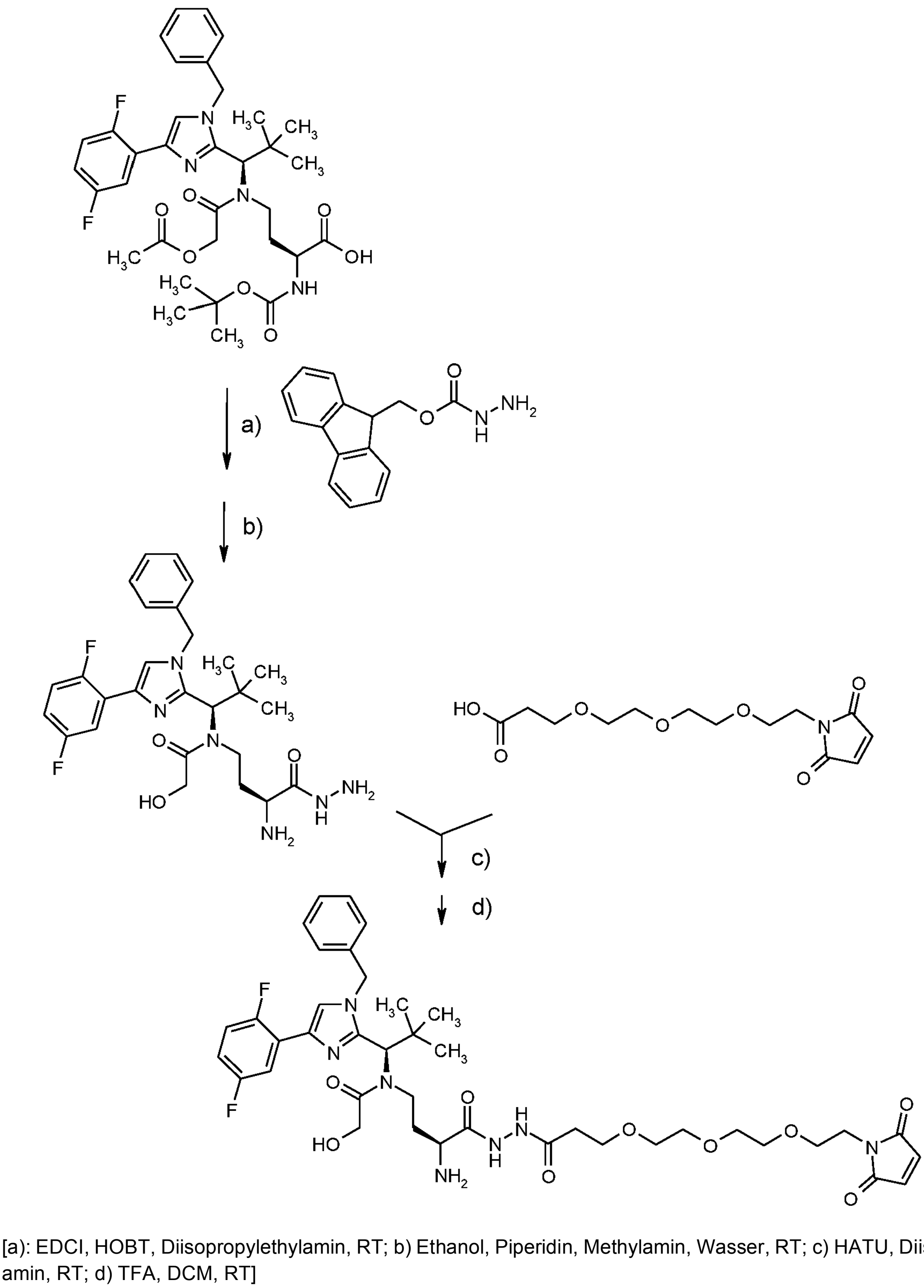


[a): EDCI, HOBT, Diisopropylethylamin, RT; b) Ethanol, Piperidin, Methylamin, Wasser, RT; c) HATU, Diisopropylethylamin, RT; d) TFA, DCM, RT]

Schema 5: Synthese von ADC-Precurser Molekülen der Intermediatserie F, die gemäß Schema 1 in APDCs überführt werden können

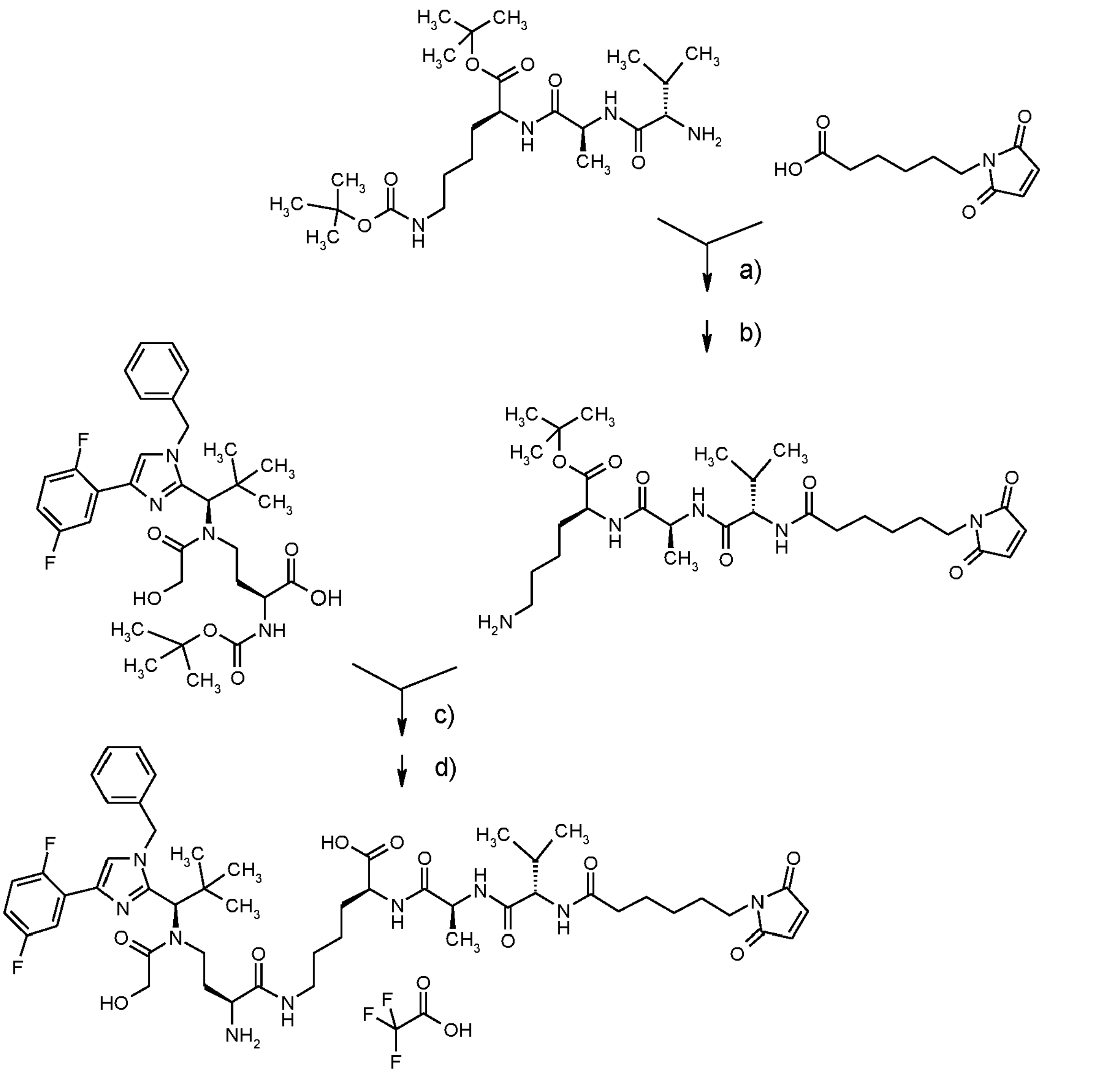


[a): beispielsweise EDCI, HOBT, Diisopropylethylamin, DMF, RT; b) beispielsweise DCM/TFA 20:1, RT; c) beispielsweise HATU, Diisopropylethylamin, DMF, RT; d) beispielsweise TFA, DCM, RT]

Schema 6: Synthese von ADC-Precurser Molekülen der Intermediatserie F, die gemäß Schema 1 in APDCs überführt werden können

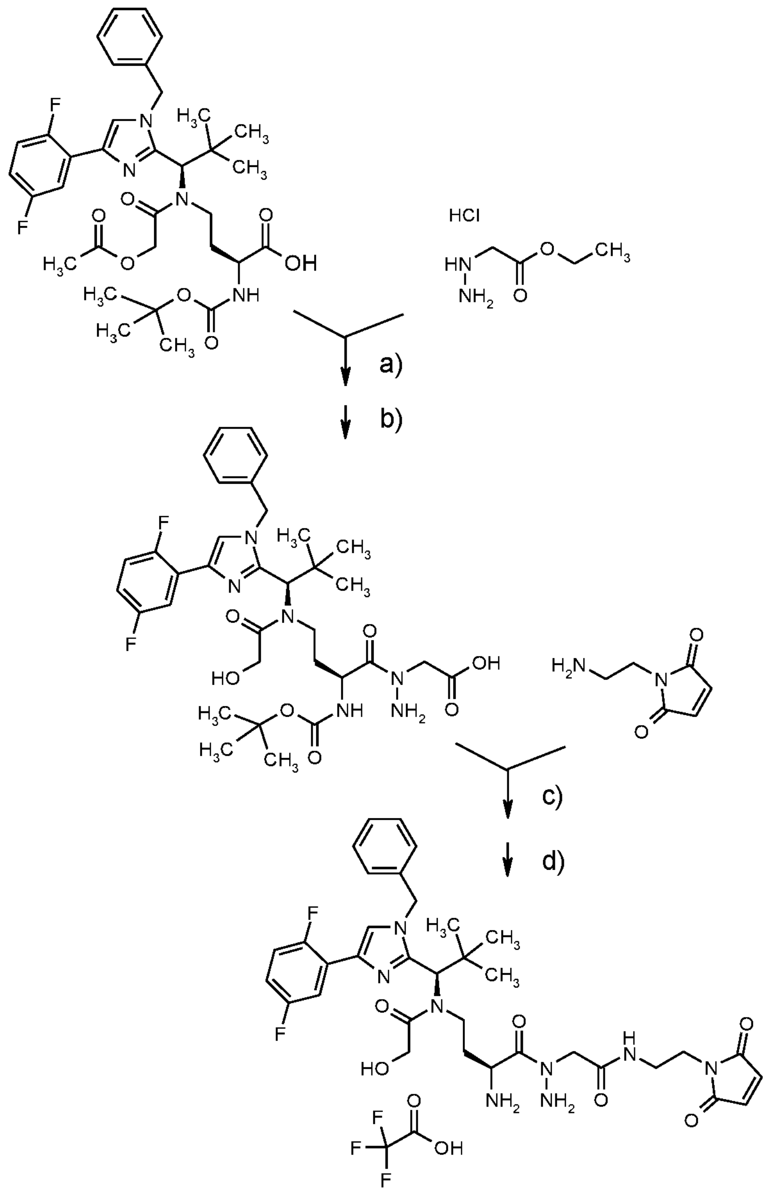


[a): beispielsweise 2-Bromo-1-ethylpyridinium tetrafluoroborate, Diisopropylethylamin, DCM, RT; b) beispielsweise 2M LiOH-Lösung, THF, Wasser, RT, HPLC-Trennung der Regiosisomeren; c) beispielsweise EDCI, HOBT, Diisopropylethylamin, DMF, RT; d) beispielsweise TFA, DCM, RT]

Schema 7: Synthese von ADC-Precurser Molekülen der Intermediatserie F, die gemäß Schema 1 in APDCs überführt werden können

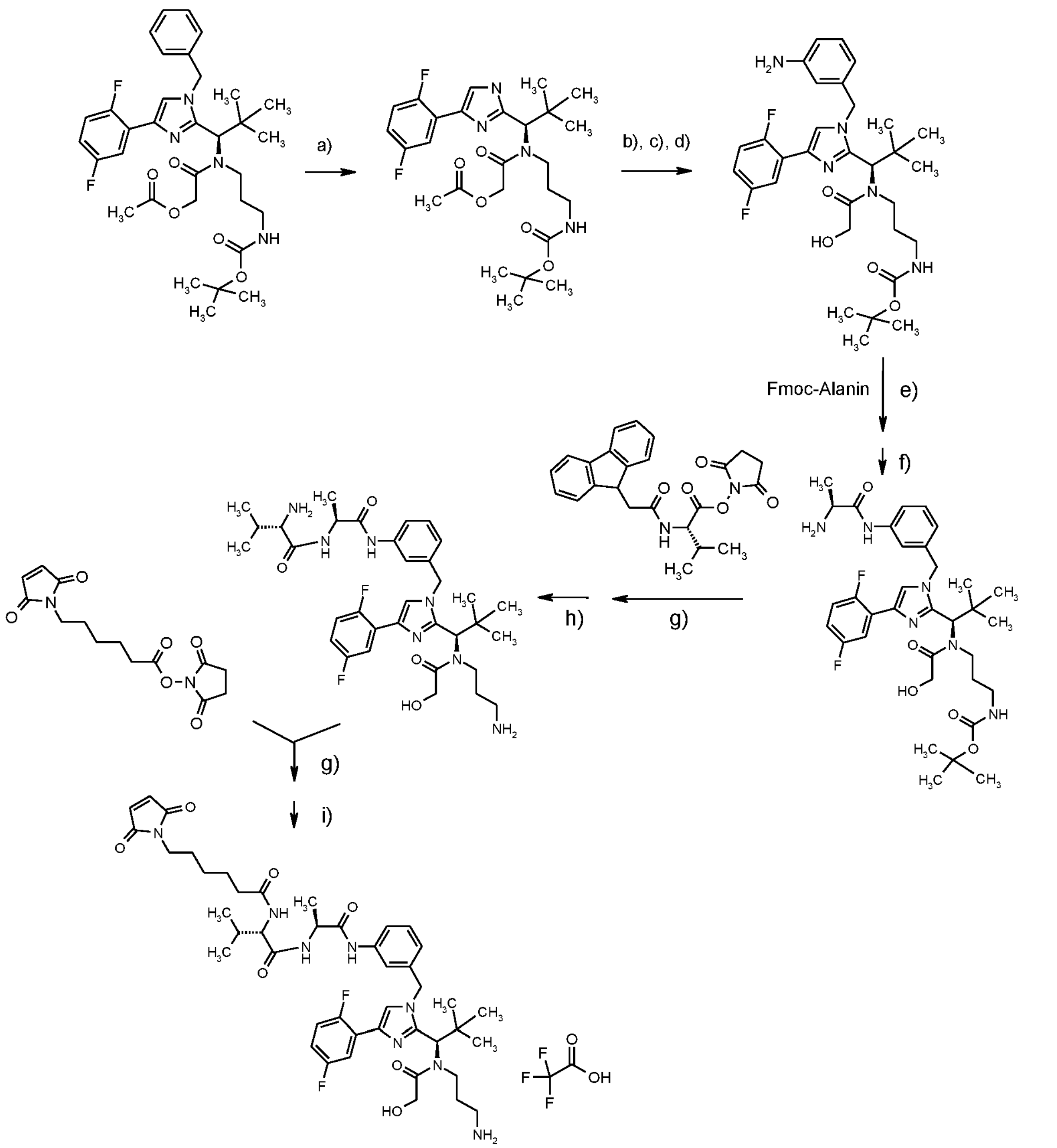


[a): beispielsweise $H_2$, Pd-C, EtOH, RT; b) beispielsweise p-Nitrobenzylbromid, $K_2CO_3$, DMF; c) beispielsweise Ethanol, 40%-ige Methylaminlösung in Wasser, 50 °C; d) beispielsweise Dinatriumdithionit, THF, Wasser, 50 °C; e) beispielsweise HATU, Diisopropylethylamin, DMF, RT; f) beispielsweise Piperidin, 40%-ige Methylaminlösung in

Wasser, Ethanol, 50 °C; g) beispielsweise Diisopropylethylamin, DMF, RT; h) beispielsweise Piperidin, DMF, RT; i) TFA, DCM, RT]

Schema 8: Synthese von ADC-Precurser Molekülen der Intermediatserie F, die gemäß Schema 1 in APDCs überführt werden können

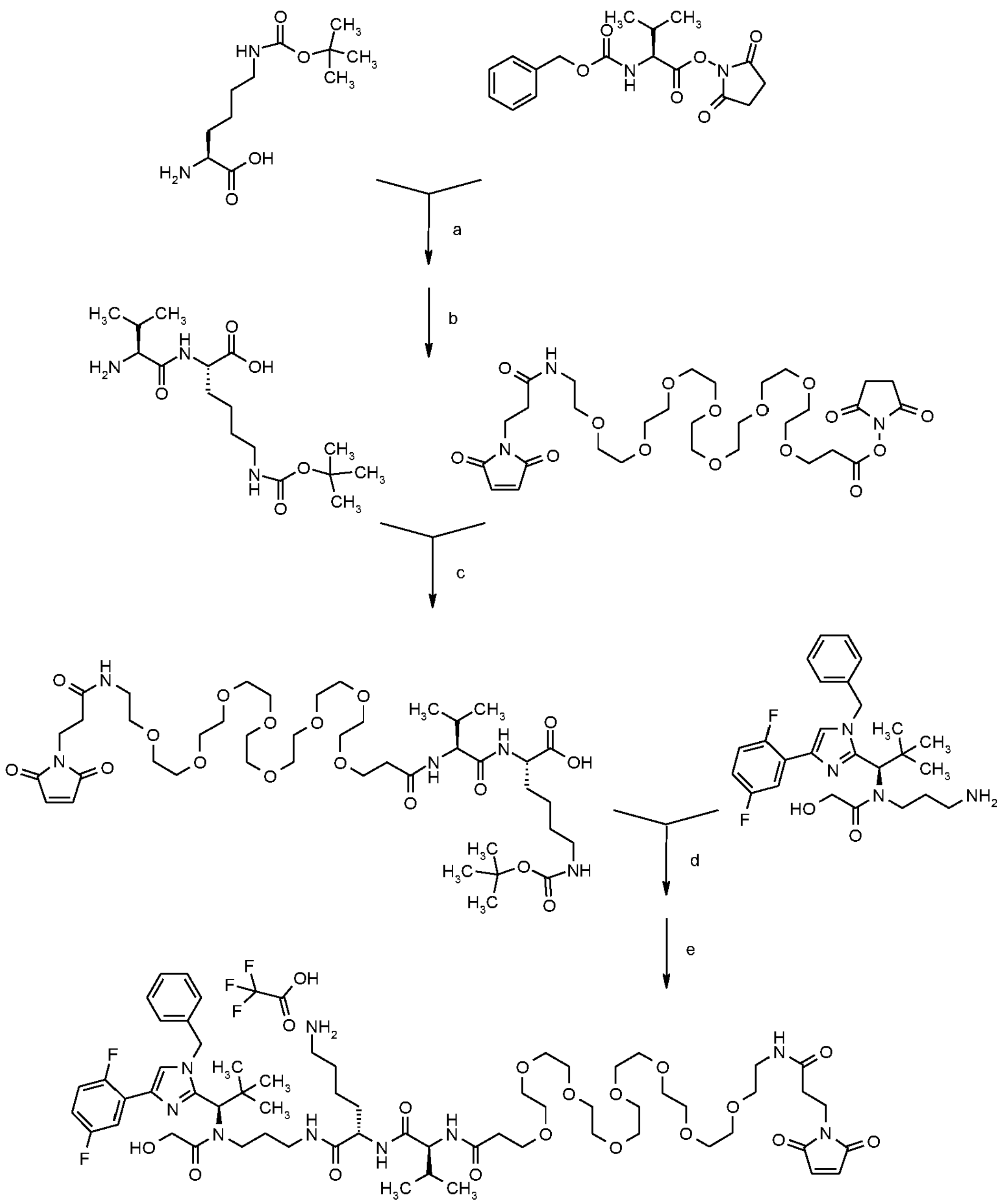


[a): beispielsweise $Et_3N$, DMF, RT; b) beispielsweise $H_2$, Pd-C, EtOH/Ethylacetat/THF (1:1:1), RT; c) beispielsweise 4-Methylmorpholin, DMF, RT; d) beispielsweise HATU, HOAt, Diisopropylethylamin, DMF, RT; e) beispielsweise TFA, RT]

Schema 9: Synthese von ADC-Precurser Molekülen der Intermediatserie F, die gemäß Schema 1 in APDCs überführt werden können

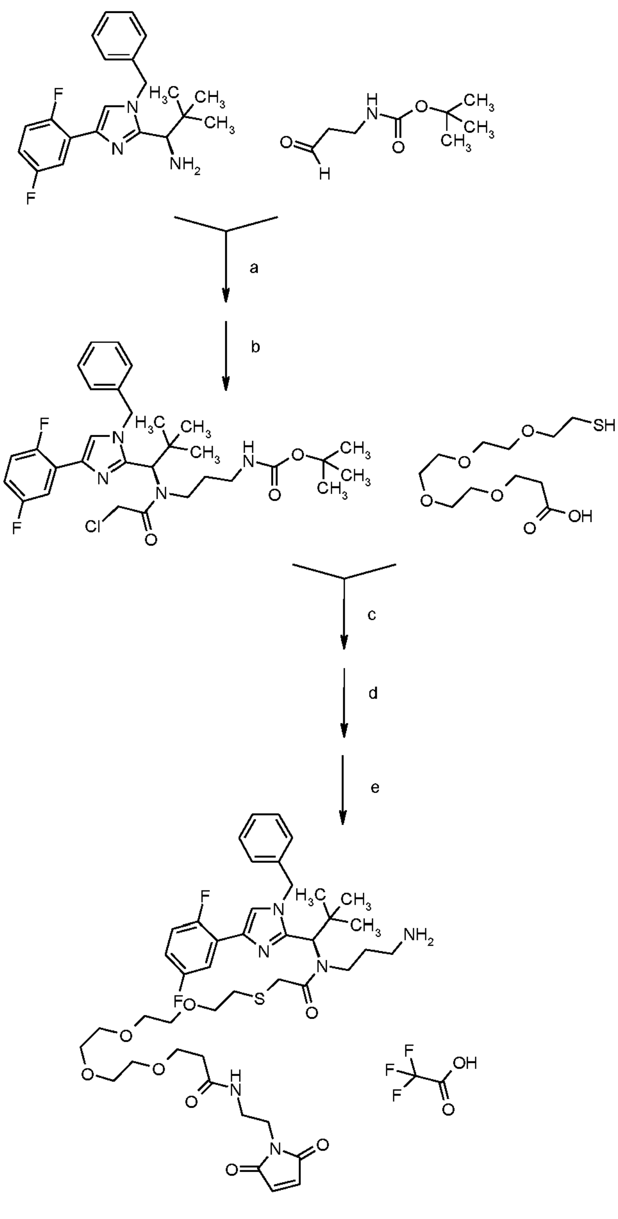


[a): beispielsweise $NaBH(OAc)_3$, HOAc, Dichlormethan, RT; b) beispielsweise Chloracetylchlorid, $NEt_3$, DCM, RT; c) beispielsweise $CS_2CO_3$, DMF, 50 °C; d) beispielsweise 1-(2-Aminoethyl)-1H-pyrrol-2,5-dionhydrochlorid(1:1), T3P[(R)], Diisopropylethylamin, MeCN, RT; e) beispielsweise TFA, RT]

Schema 10: Synthese von ADC-Precurser Molekülen der Intermediatserie F, die gemäß Schema 1 in APDCs überführt werden können

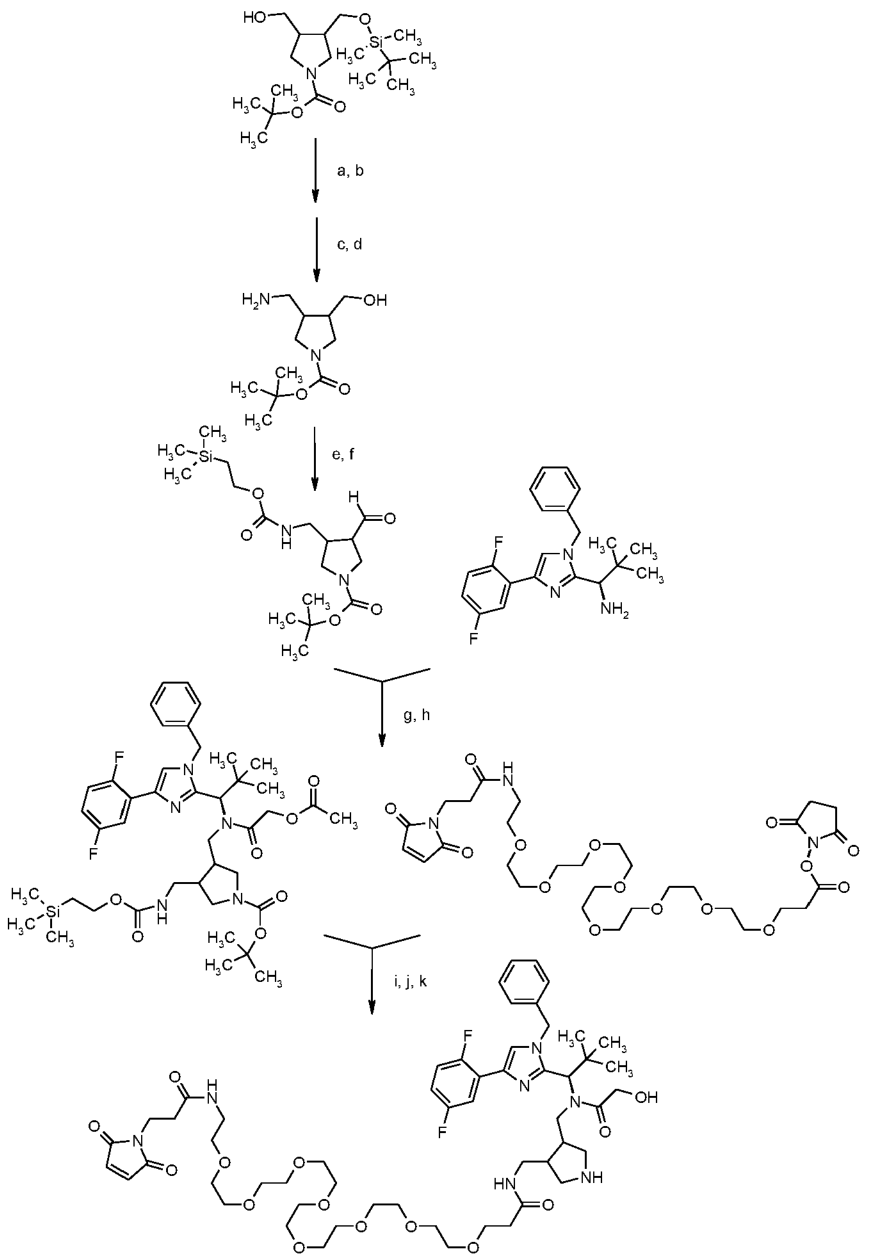


[a): beispielsweise Methansulfonsäurechlorid, $NEt_3$, Dichlormethan, 0 °C; b) beispielsweise $NaN_3$, DMF, 40 °C; c) beispielsweise $H_2$, Pd-C, EtOH/Ethylacetat (1:1), RT; d) beispielsweise TBAF, THF, RT; e) beispielsweise 1-({[2-(Trimethylsilyl)ethoxy]carbonyl}oxy)pyrrolidin-2,5-dion, $NEt_3$, $CaCO_3$, 1,4-Dioxan, RT; f) beispielsweise N-Chlorsuccinimid, TEMPO, Tetra-n-butylammoniumchlorid, Chloroform, 0.05 N Kaliumcarbonat/0.05 N Natriumhydrogencarbonat-Lösung (1:1); g) beispielsweise $NaBH(OAc)_3$, HOAc, Dichlormethan, RT; h) beispielsweise Chloracetylchlorid, $NEt_3$, DCM, RT; i) beispielsweise TBAF, THF, Wasser, RT; j) beispielsweise 4-Methylmorpholin, DMF, RT; k) beispielsweise TFA, RT]

Schema 11: Synthese von ADC-Precurser Molekülen der Intermediatserie F, die gemäß Schema 1 in APDCs überführt werden können

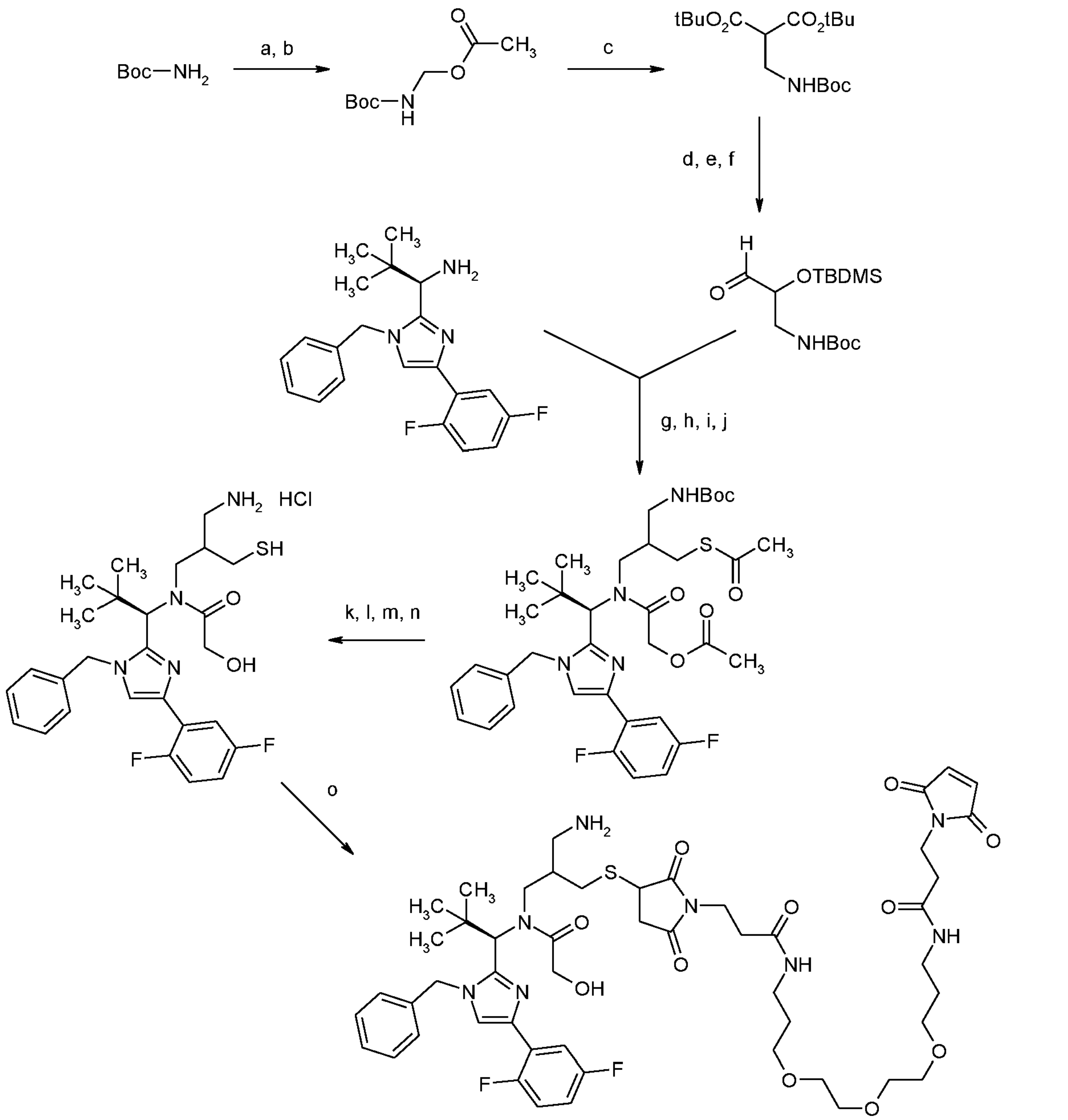


[a): beispielsweise Formaldehyd, $Na_2CO_3$, Wasser, RT; b) beispielsweise $AC_2O$, Pyridin, THF, RT; c) beispielsweise Di-tert-butylmalonat, KOtBu, THF, RT; d) beispielsweise $LiBH_4$, THF, RT; e) beispielsweise TBDMSCl, Imidazol, DCM, RT; f) beispielsweise Dess-Martin-Periodinan, DCM; g) beispielsweise Natriumtriacetoxyborhydrid, AcOH, DCM, RT; h) beispielsweise $nBu_4NF$, THF, RT; i) beispielsweise $SOCl_2$, THF, RT; j) beispielsweise AcSK, $nBu_4NI$, DMF, 90°C; k) beispielsweise NaOH, MeOH, THF, RT; l) beispielsweise

TCEP, Dioxan, RT; m) beispielsweise Trennung der Epimere; n) beispielsweise 6N Salzsäure, THF, RT o) beispielsweise Mal-dPEG(3)-Mal, PBS-Puffer, ACN, RT]

Schema 12: Synthese von ADC-Precurser Molekülen der Intermediatserie F, die gemäß Schema 1 in APDCs überführt werden können

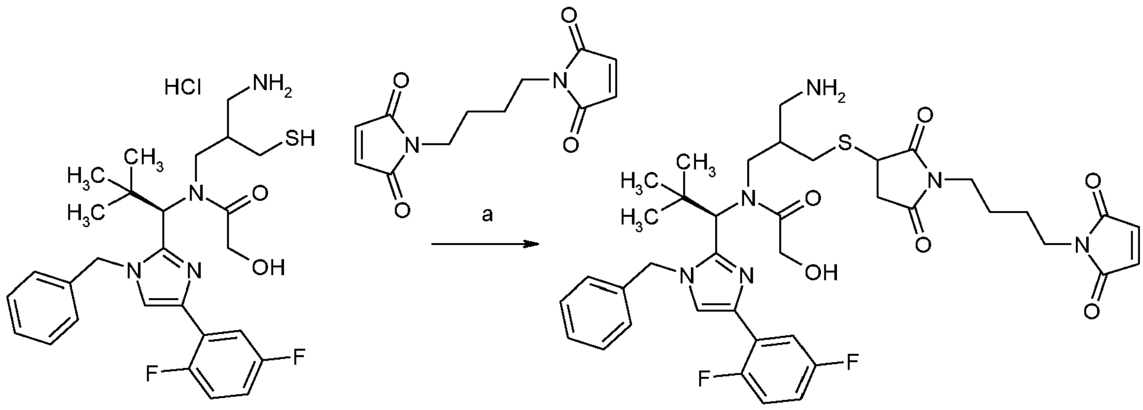


[a): beispielsweise Mal-dPEG(3)-Mal, PBS-Puffer, ACN, RT]

Schema 13: Synthese von ADC-Precurser Molekülen der Intermediatserie F, die gemäß Schema 1 in APDCs überführt werden können

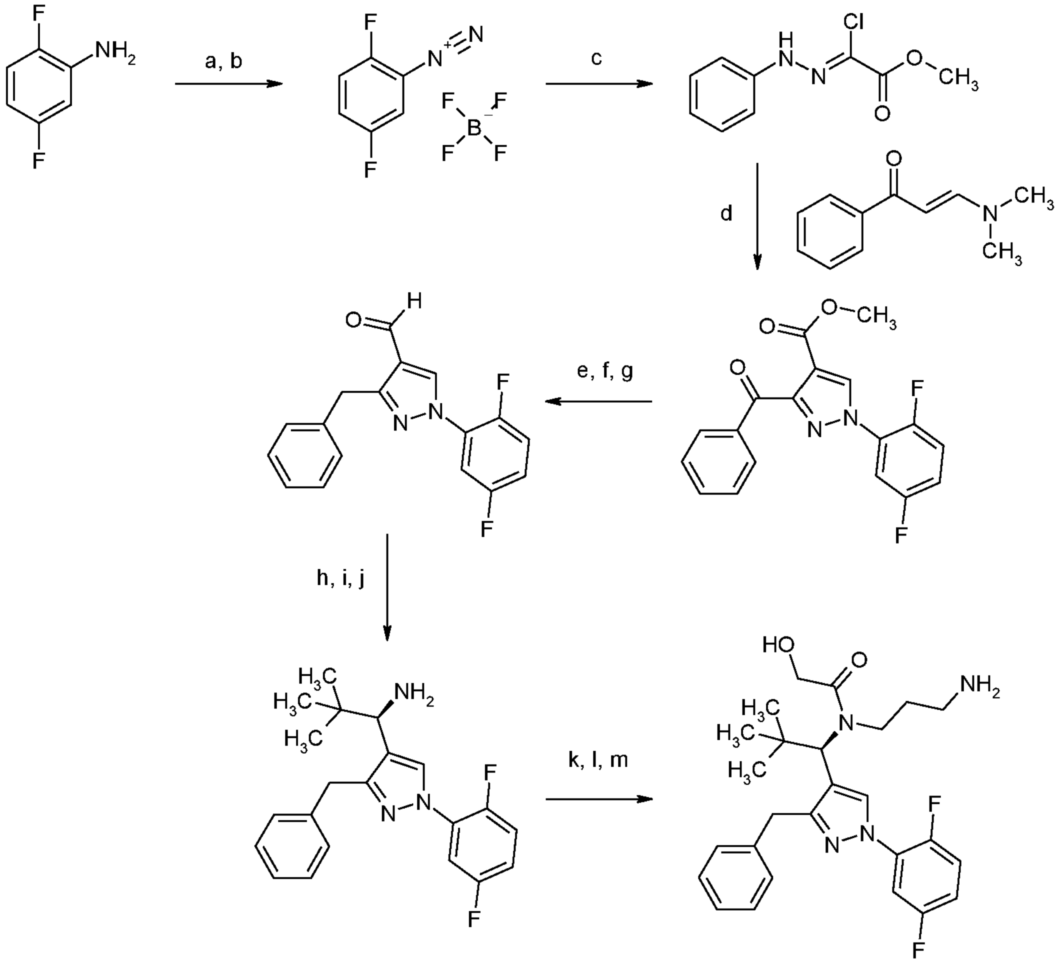

[a): beispielsweise $BF_3OEt_2$, THF, 0°C; b): beispielsweise Isoamylnitrit, -10°C, 0.5 h; c): beispielsweise Methyl-2-chlor-3-oxobutanoat, Pyridin, Wasser, -5°C; d): beispielsweise $NEt_3$, Toluol, RT; e): beispielsweise $Et_3SiH$, TFA, RT; f): beispielsweise $LiBH_4$, THF, 60°C; g): beispielsweise Dess-Martin-Periodinan, DCM, RT; h): beispielsweise (R)-(+)-Methyl-2-propansulfinsäureamid, Titan(IV)isopropylat, THF, RT; i): beispielsweise tert-BuLi, Pentan, THF, -78°C; j): beispielsweise HCl in Dioxan, THF, MeOH, RT; k): beispielsweise 3-(1,3-Dioxo-1,3-dihydro-2H-isoindol-2-yl)propanal, $NaB(OAc)_3H$, AcOH, DCM, RT; l): beispielsweise 2-Chlor-2-oxoethylacetat, $NEt_3$, DCM, RT; m): beispielsweise Methylamin, Wasser, EtOH, 50°C]

Schema 14: Synthese von Vorstufen von ADC-Precurser Molekülen

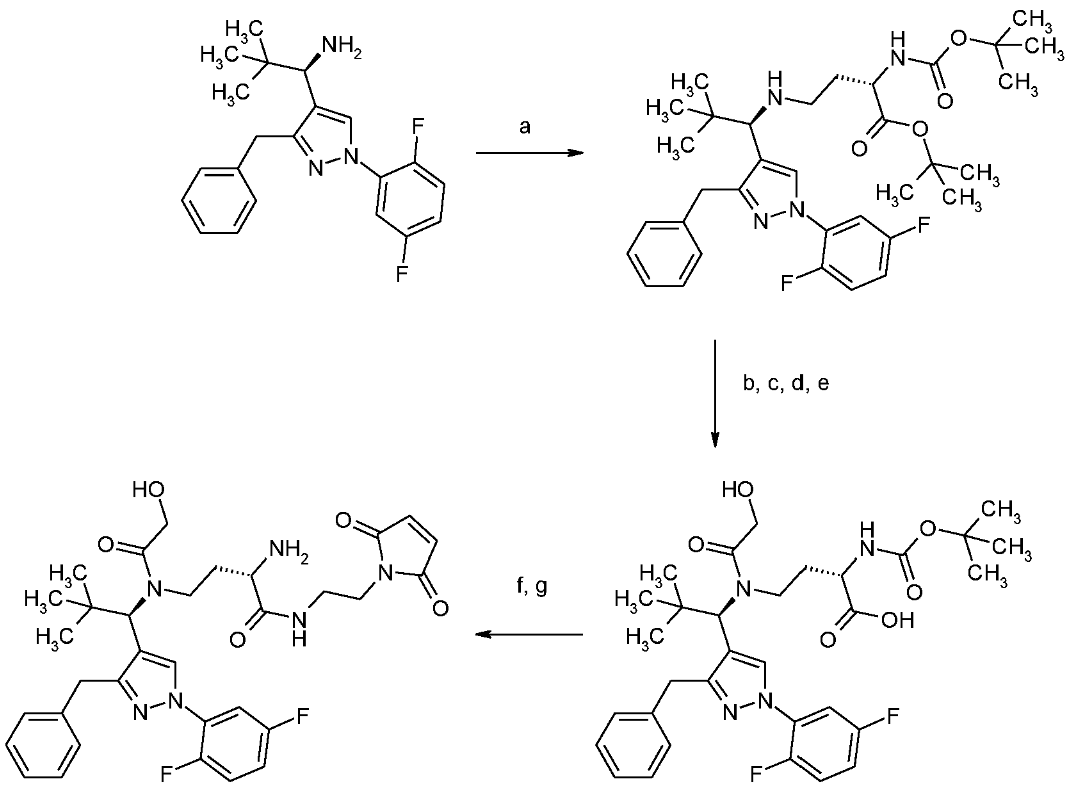


[a): beispielsweise tert-Butyl-N-(tert-butoxycarbonyl)-5-oxo-L-norvalinat, $NaB(OAc)_3H$, AcOH, DCM, RT; b): beispielsweise 2-Chlor-2-oxoethylacetat, $NEt_3$, DCM, RT; c): beispielsweise Methylamin, Wasser, EtOH, 60°C; d): beispielsweise THF, DCM, 50°C; e): beispielsweise $Boc_2O$, $NEt_3$, DCM, RT; f): beispielsweise Trifluoressigsäure-1-(2-aminoethyl)-1H-pyrrol-2,5-dion(1:1), HATU, Diisopropylethylamin, DMF, RT; f): beispielsweise TFA, DCM, RT]

Schema 15: Synthese von Intermediaten

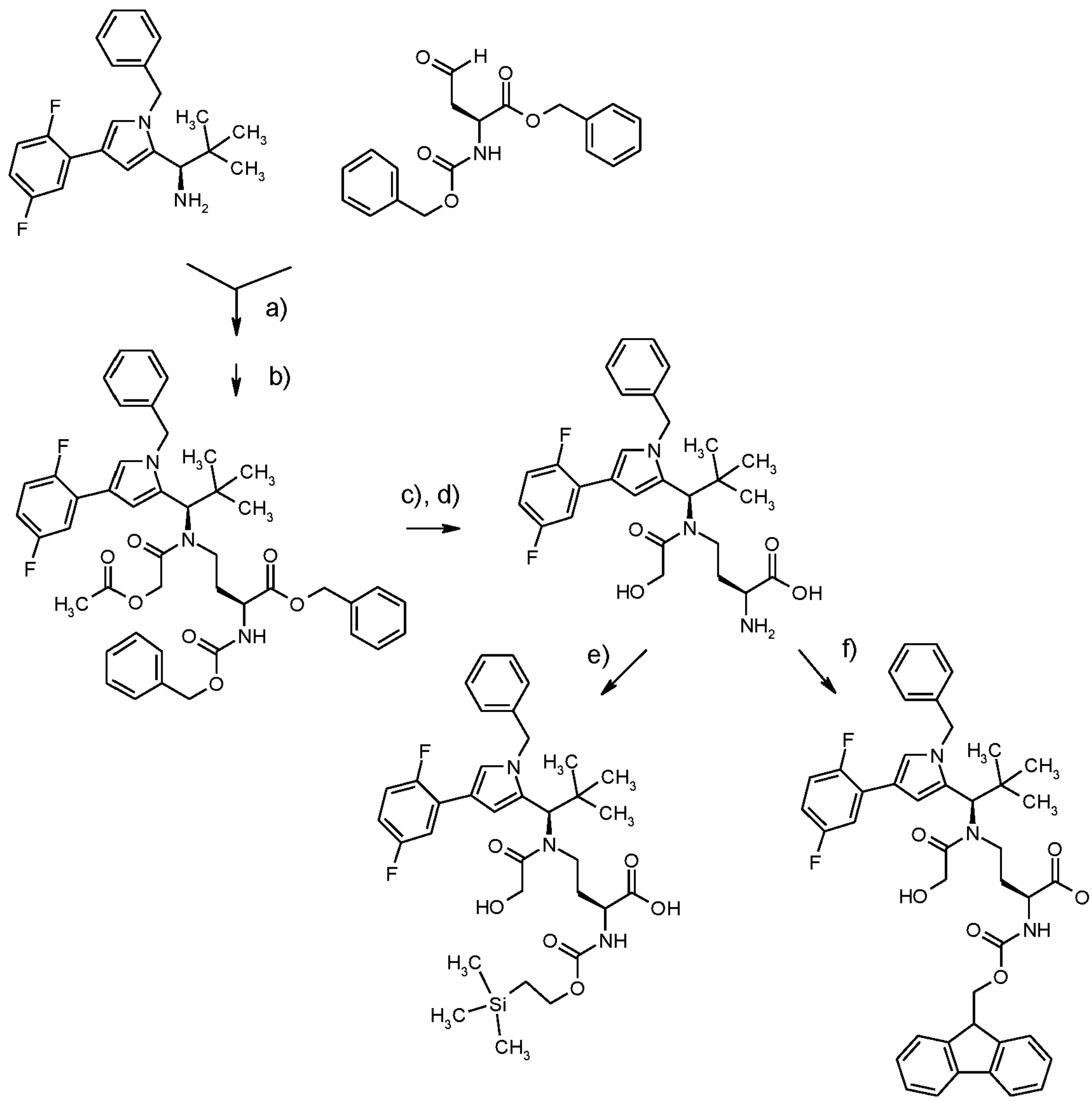


[a): beispielsweise Natriumtriacetoxyborhydrid, Essigsäure, DCM, RT; b) beispielsweise Acetoxyacetylchlorid, NEt3, DCM, RT; c) beispielsweise LiOH, THF/Wasser, RT; d) beispielsweise $H_2$, Pd-C, EtOH, RT; e) beispielsweise Teoc-OSu, NEt3, Dioxan, RT; f) beispielsweise Fmoc-Cl, Diisopropylethylamin, Dioxan/Wasser 2:1, RT]

Schema 16: Synthese von Intermediaten

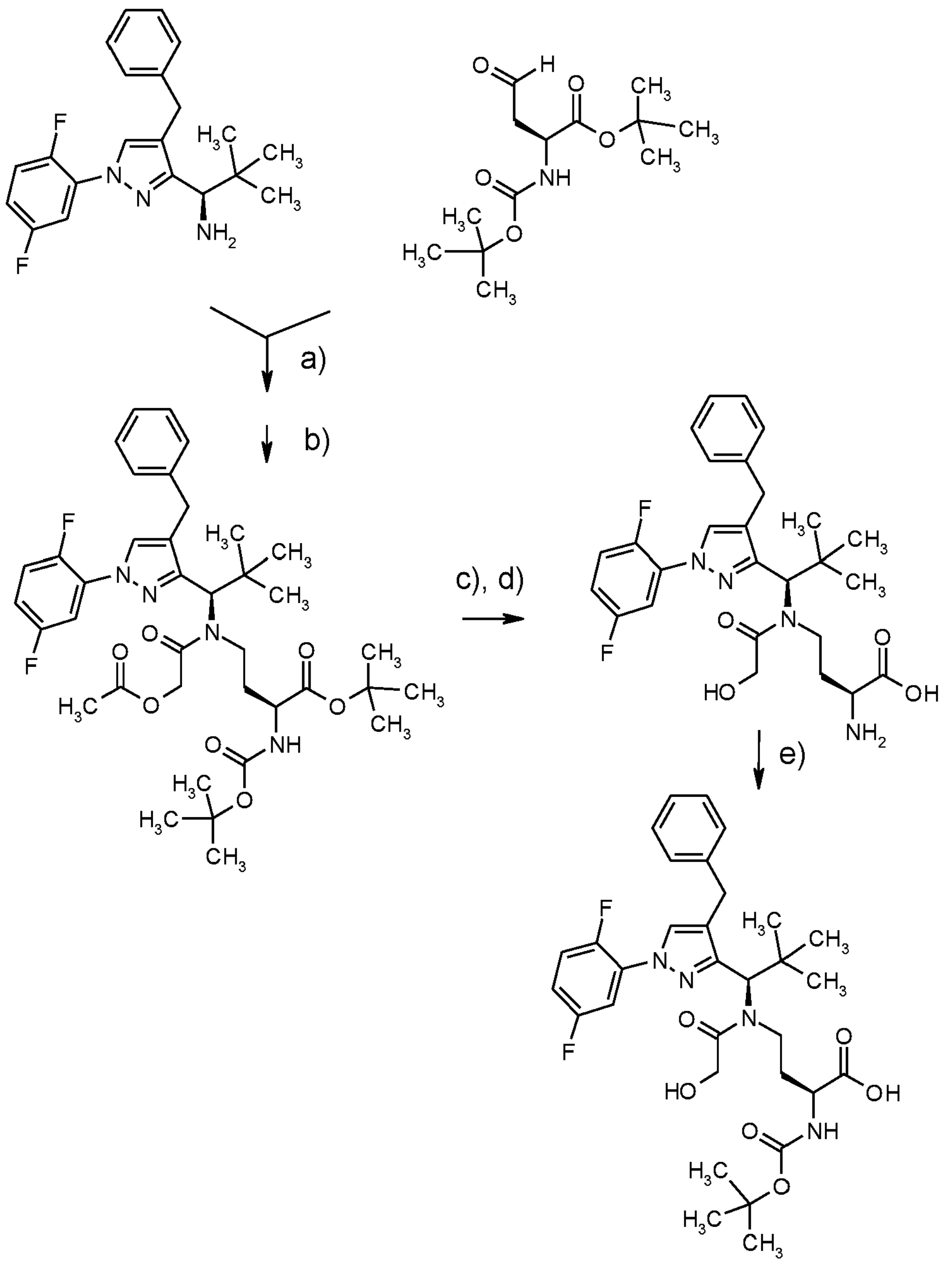


[a): beispielsweise Natriumtriacetoxyborhydrid, Essigsäure, DCM, RT; b) beispielsweise Acetoxyacetylchlorid, NEt3, DCM, RT;c) beispielsweise LiOH, Methanol, RT; d) beispielsweise TFA, DCM, RT; e) beispielsweise $Boc_2O$, Diisopropylethylamin, DCM, RT]

Schema 17: Synthese von Intermediaten

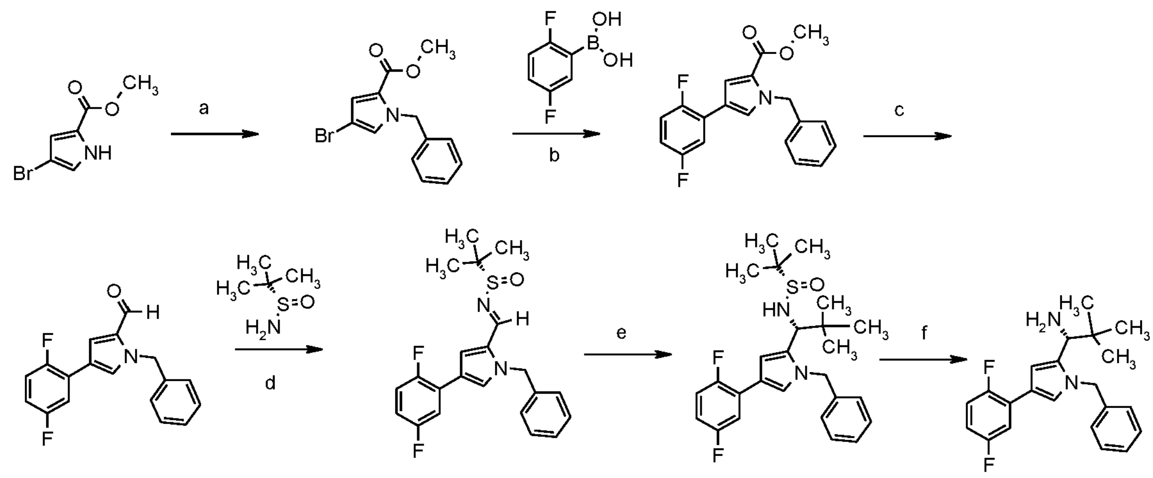


[a): beispielsweise Benzylbromid, $CS_2CO_3$, DMF, RT; b) beispielsweise $Pd(dppf)_2Cl_2$, DMF, $Na_2CO_3$, 85 °C; c) beispielsweise $LiAlH_4$, THF, 0 °C; $MnO_2$, DCM, RT; d) beispielsweise $Ti(iOPr)_4$, THF, RT; e) beispielsweise tBuLi, THF, -78 °C; MeOH, $NH_4Cl$; f) beispielsweise HCl/1,4-Dioxan]

Schema 18: Synthese von ADC-Precurser Molekülen der Intermediatserie F, die gemäß Schema 1 in APDCs überführt werden können

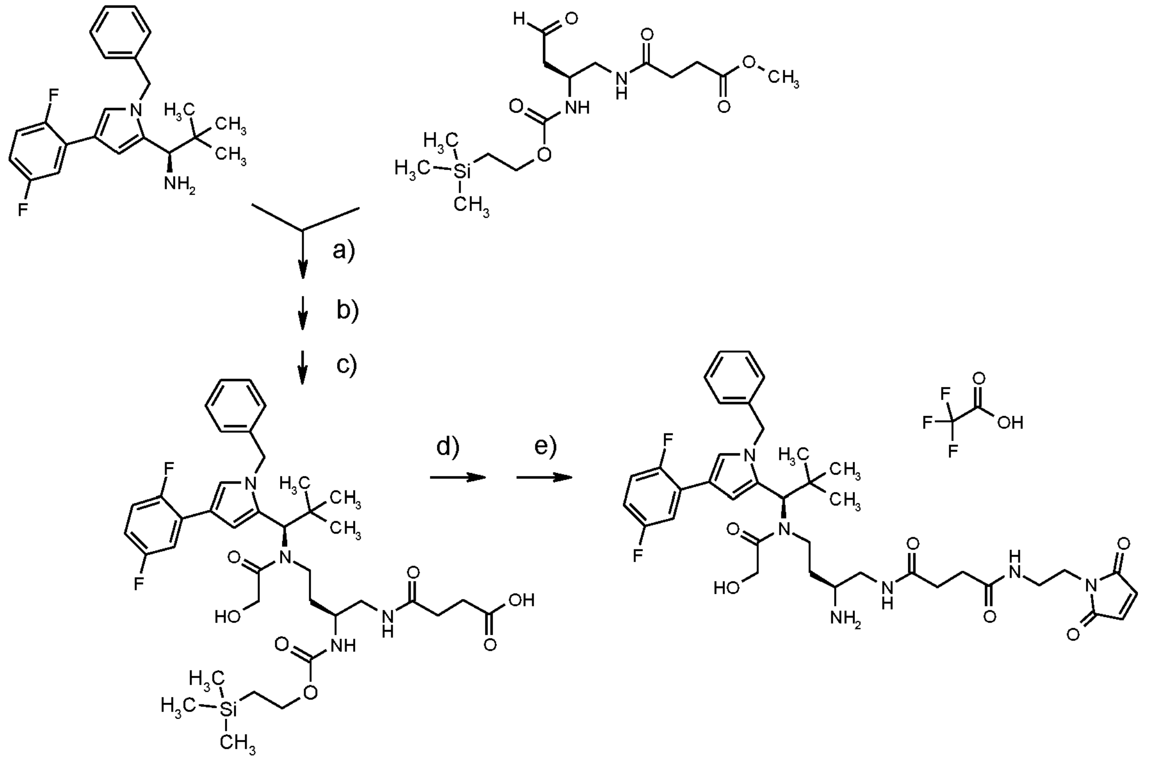


[a): Natriumtriacetoxyborhydrid, Essigsäure, DCM, RT; b) Acetoxyacetylchlorid, Diisopropylethylamin, DCM, RT; c) LiOH, MeOH, RT; d) Trifluoressigsäure--1-(2-aminoethyl)-1H-pyrrol-2,5-dion (1:1) HATU, DMF, Diisopropylethylamin, RT; e)

Zinkchlorid, Trifluorethanol, 50°C, EDTA.]

Schema 19: Synthese von ADC-Precurser Molekülen der Intermediatserie F, die gemäß Schema 1 in APDCs überführt werden können

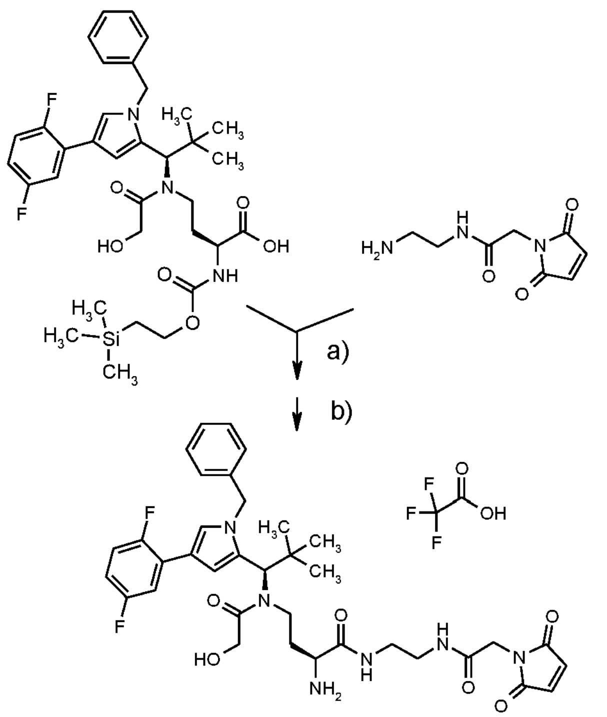


[a): HATU, DMF, Diisopropylethylamin, RT; b) Zinkchlorid, Trifluorethanol, 50°C, EDTA.]

Schema 20: Synthese von ADC-Precurser Molekülen der Intermediatserie F, die gemäß Schema 1 in APDCs überführt werden können

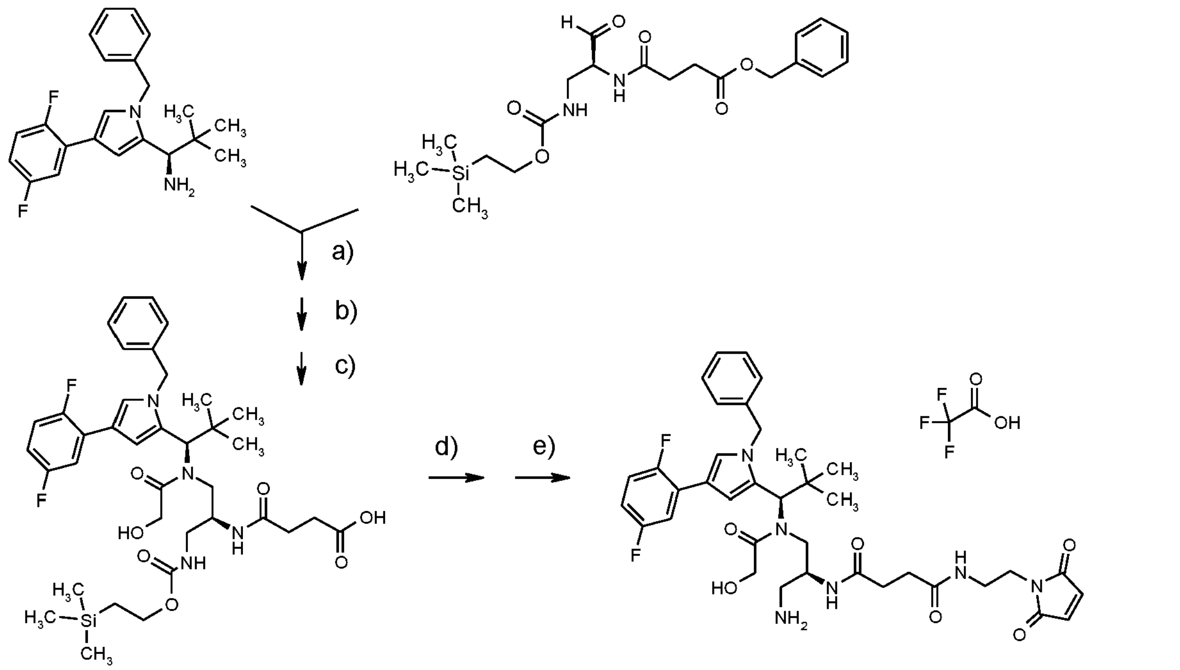


[a): Natriumtriacetoxyborhydrid, Essigsäure, DCM, RT; b) Acetoxyacetylchlorid, Triethylamin, DCM, RT; c) LiOH, MeOH, RT; d) Trifluoressigsäure--1-(2-aminoethyl)-1H-pyrrol-2,5-dion (1:1) HATU, DMF, Diisopropylethylamin, RT; e) Zinkchlorid, Trifluorethanol, 50°C, EDTA.]

Schema 21: Allgemeines Verfahren zur Synthese von Intermediaten und ADC-Precursern

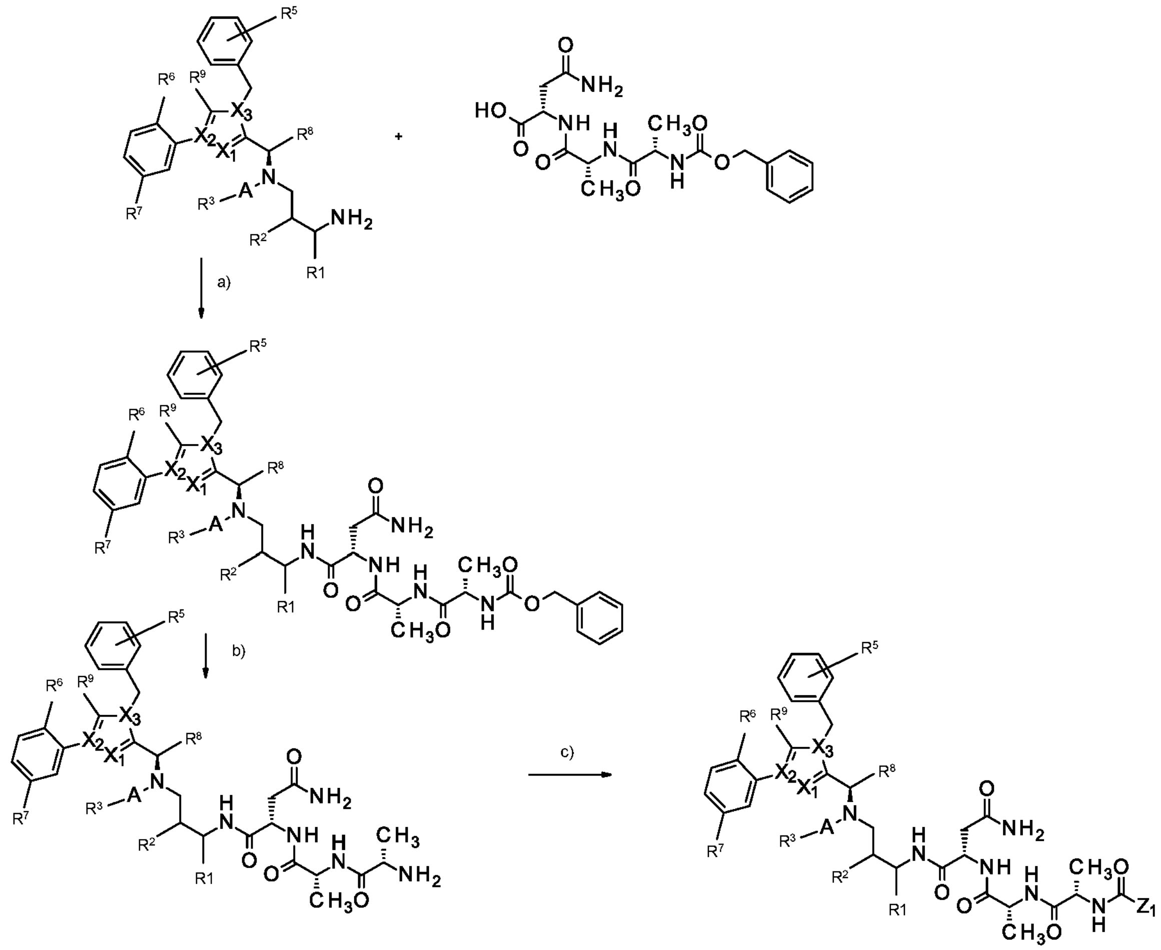


[a): HATU, DMF, N,N-Diisopropylethylamin, RT oder EDCI, HOBT, N,N-Diisopropylethylamin, DMF, RT b) $H_2$, 10% Pd-C, MeOH, RT; c) $Z_1$-COOH, EDCI, HOBT, N,N-Diisopropylethylamin, DMF, RT oder $Z_1$-COOH, HATU, N,N-Diisopropylethylamin, DMF, RT oder $Z^1$-COOSu, N,N-Diisopropylethylamin, DMF, RT]

Schema 22: Synthese von ADC-Precurser Molekülen mit Legumain-spaltbaren Linkern

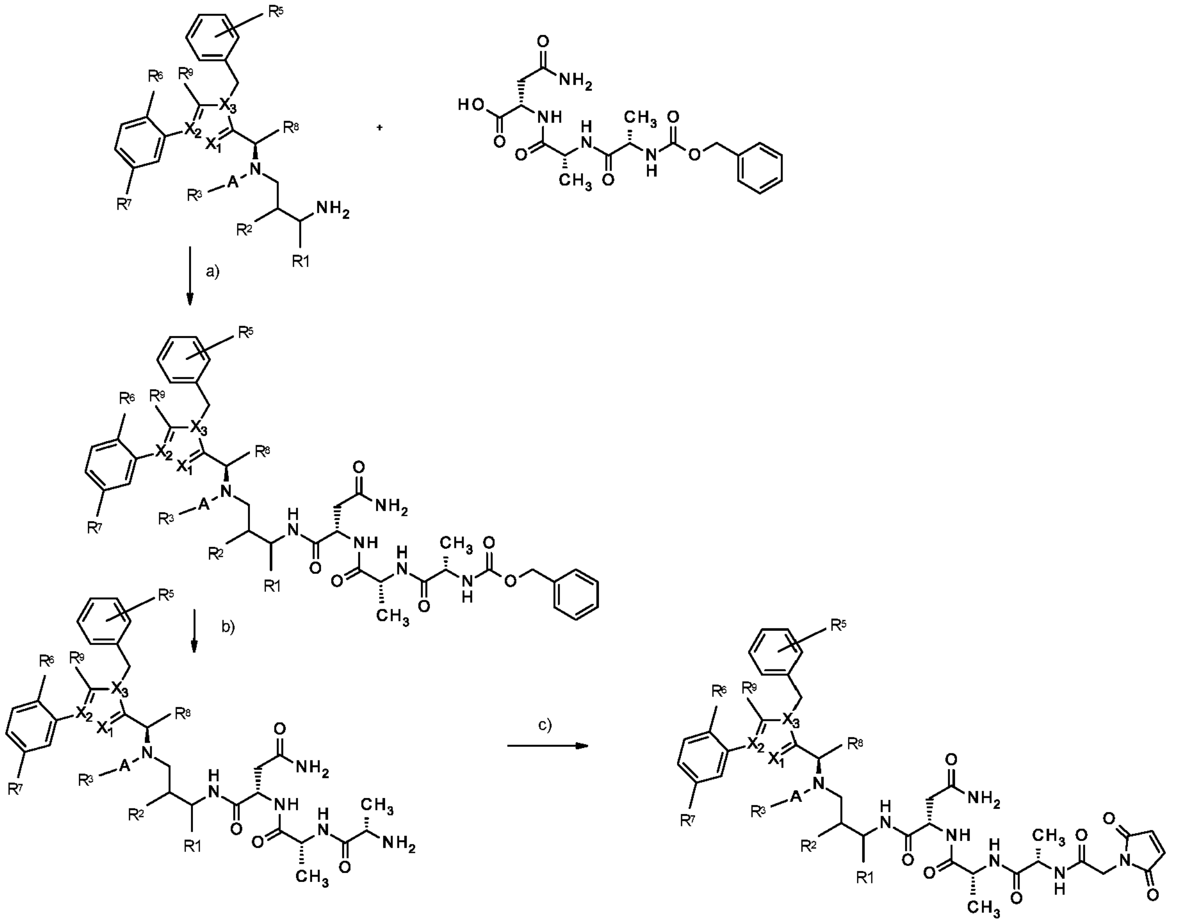


[a): HATU, DMF, N,N-Diisopropylethylamin, RT oder EDCI, HOBT, N,N-Diisopropylethylamin, DMF, RT b) $H_2$, 10% Pd-C, MeOH, RT; c) 1,1'-[(1,5-Dioxopentan-1,5-diyl)bis(oxy)]dipyrrolidin-2,5-dion, N,N-Diisopropylethylamin, DMF, RT]

Schema 23: Synthese von ADC-Precurser Molekülen mit Legumain-spaltbaren Linkern

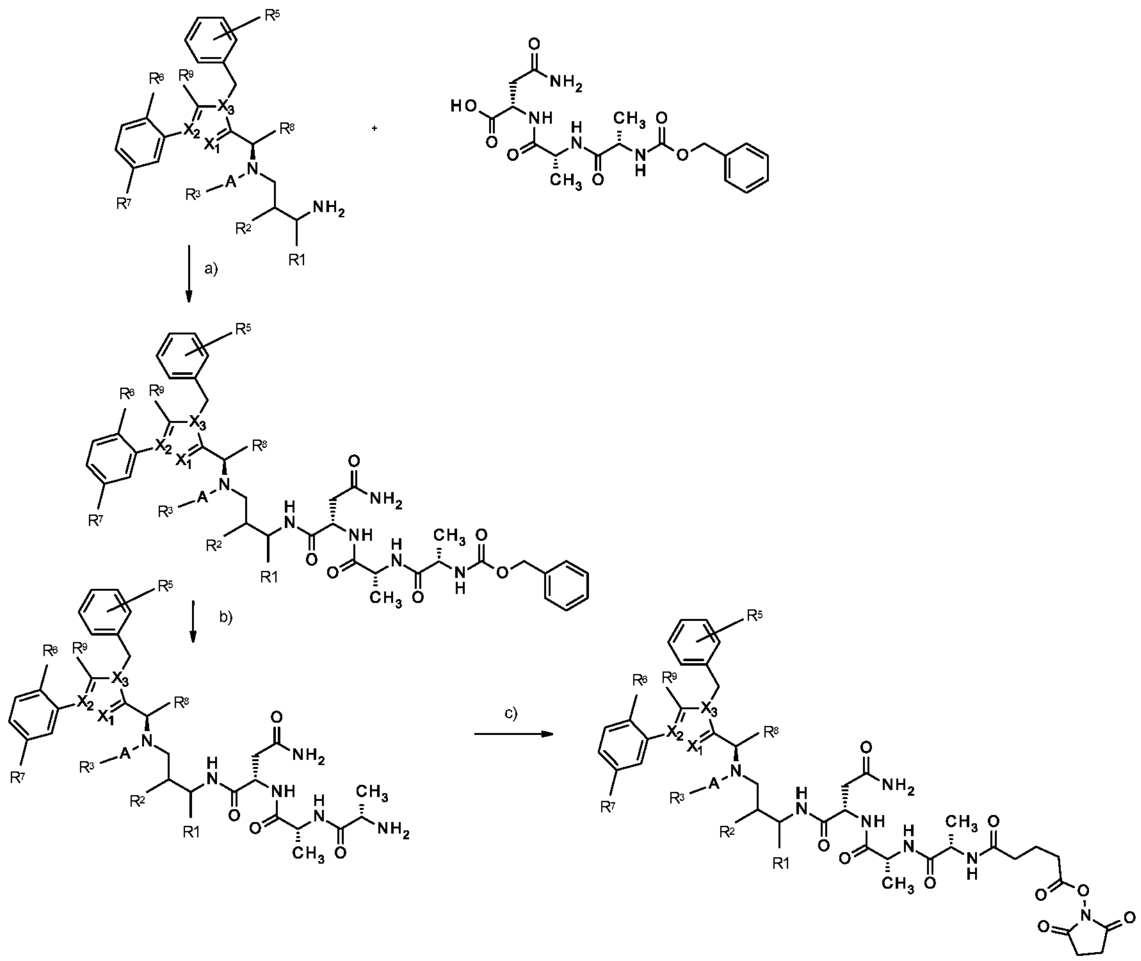


[a): HATU, DMF, N,N-Diisopropylethylamin, RT oder EDCI, HOBT, N,N-Diisopropylethylamin, DMF, RT b) $H_2$, 10% Pd-C, MeOH, RT; c)) 1,1'-[(1,5-Dioxopentan-1,5-diyl)bis(oxy)]dipyrrolidin-2,5-dion, N,N-Diisopropylethylamin, DMF, RT]

Weiterhin können andere Intermediate gemäß Schema 21, 22 und 23 in Legumainspaltbare ADC-und APDC Precursor überführt werden.

Alternativ zu der in Schemata 21-23 dargestellten Benzyloxycarbonyl-Gruppe können andere in der Peptidchemie etablierte Schutzgruppen eingesetzt werden und nach entsprechenden ebenso bekannten Methoden abgespalten werden. Die Auswahl der Schutzgruppenstrategie erfolgt nach entsprechenden dem Fachmann bekannten Anforderungen zur Kompatibilität mit anderen im Molekül vorkommenden Strukturelementen. Falls noch vorhanden können weitere Schutzgruppen im Molekül in einem letzten Schritt entfernt werden.

Die Synthesen können auch ggf. in ihrer Sequenz umgestellt werden.

Schema 24: Synthese von Cystein-verknüpften ADCs mit Legumain-spaltbarer Kopfgruppe

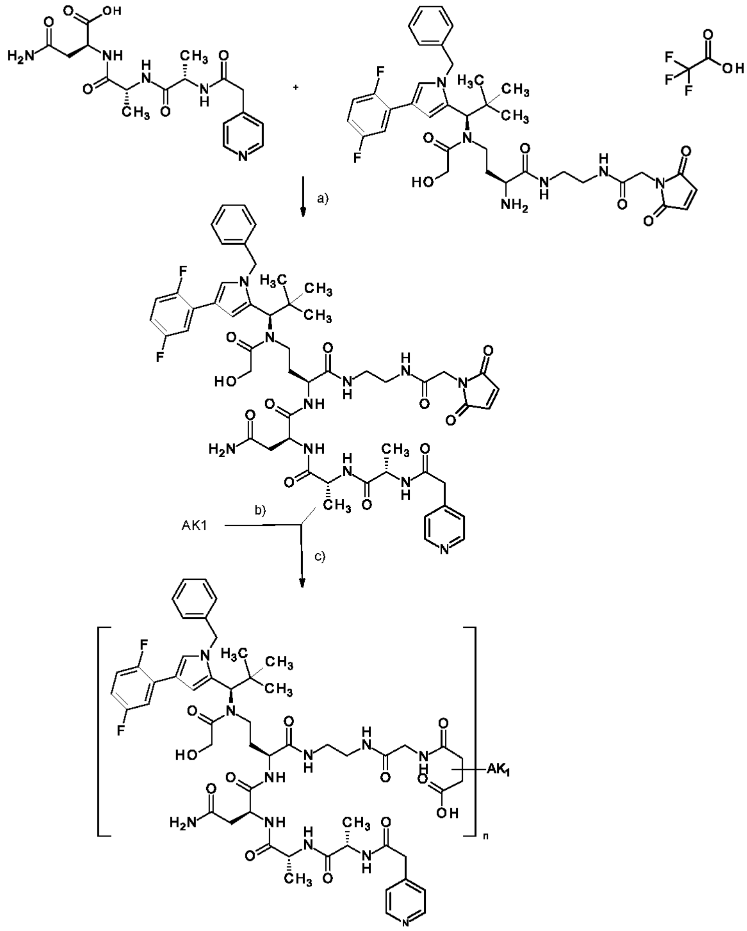


[a): HATU, DMF, N,N-Diisopropylethylamin, RT; b) 2-5 Eq TCEP, PBS pH7.2, 30 min Rühren bei RT; c) 90 min Rühren bei RT unter Argon, dann Umpuffern auf pH 8 mittels PD 10-Säulen (Sephadex® G-25, GE Healthcare) und Rühren über Nacht unter Argon bei RT und anschließendes Aufkonzentrieren mittels Ultrazentrifugation und Einstellen der angestrebten Konzentration mit PBS bei pH 7.2)]

Schema 25: Synthese von Lysin-verknüpften ADCs mit Legumain-spaltbarem Linker

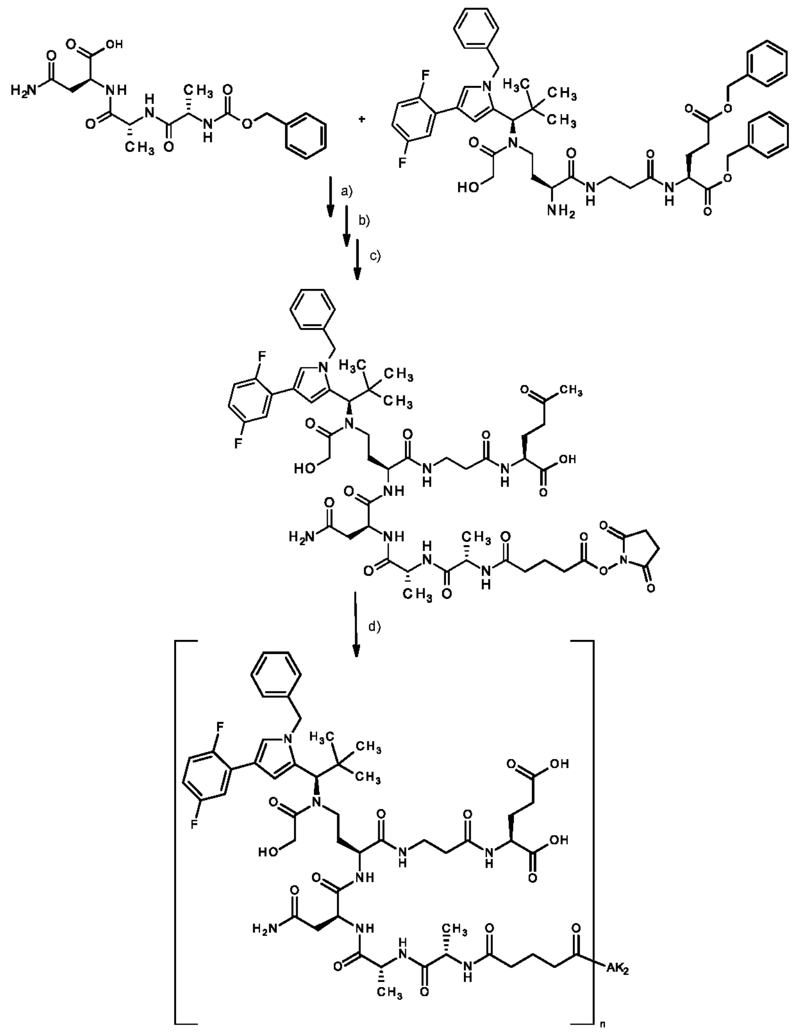


[a): HATU, DMF, N,N-Diisopropylethylamin, RT; b) $H_2$, 10% Pd-C, Methanol 1.5 h, RT; c) 1,1'-[(1,5-Dioxopentan-1,5-diyl)bis(oxy)]dipyrrolidin-2,5-dion, N,N-Diisopropylethylamin, DMF, Rühren über Nacht bei RT; d) AK2 in PBS, 5 Equiv. Aktivester gelöst in DMSO zugeben, 60 min Rühren bei RT unter Argon, erneut 5 Equiv. Aktivester gelöst in DMSO nachsetzen, 60 min Rühren bei RT unter Argon, dann Reinigung über mit PBS Puffer (pH 7.2) equillibrierte PD 10-Säulen (Sephadex® G-25, GE Healthcare) und anschließendes Aufkonzentrieren mittels Ultrazentrifugation und Einstellen der angestrebten Konzentration mit PBS Puffer (pH 7.2)].

Schema 26: Synthese von ADC-Precursern mit Legumain-spaltbarer Kopfgruppe

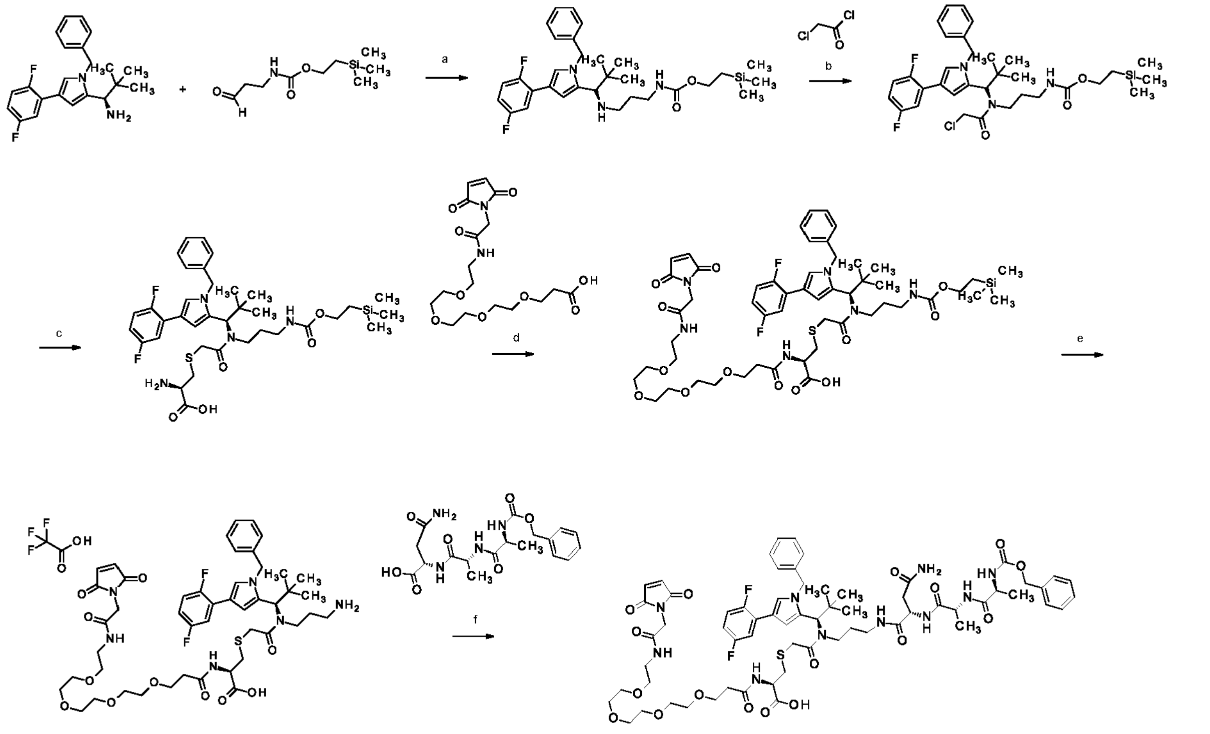


[a): $NaBH(OAc)_3$, HOAc, Dichlormethan, RT; b) Chloracetylchlorid, $NEt_3$, DCM, RT; c) L-Cystein, $NaHCO_3$, DBU, Isopropanol/Wasser, 50 °C; d) HATU, DMF, Diisopropylethylamin, RT; e) Zinkchlorid, Trifluorethanol, 50°C; f) d) HATU, DMF, Diisopropylethylamin, RT]

Schema 27: Synthese von ADCs über Transglutaminase-Kupplung

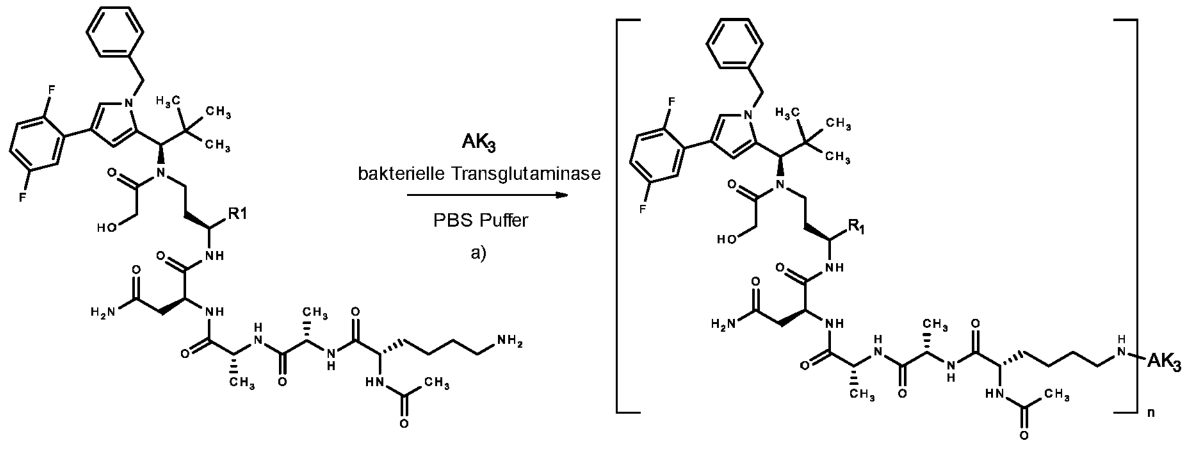


[a: 5 mg AK3 in DPBS pH 7.4 (c~10mg/mL), 6 Equivalente eines Toxophor-Linker-Precursors (e.g. Intermediat Q31-Q34), 50 μL einer Lösung aus 12.5 μL (1.25 U) rekombinanter bakterieller Transglutaminase Lösung in Wasser (100 U/mL) und 37.5 μL DPBS pH 7.4 hinzugegeben, 24h bei 37°C inkubieren]

## A. Beispiele

**Abkürzungen und Akronyme:**

| | |
|---|---|
| A498 | humane Tumorzelllinie |
| ABCB1 | ATP-binding cassette sub-family B member 1 (Synonym für P-gp und MDR1) |
| abs. | Absolut |
| Ac | Acetyl |
| ACN | Acetonitril |
| aq. | wässrig, wässrige Lösung |
| ATP | Adenosintriphosphat |
| BCRP | Brustkrebs-Resistenz-Protein, ein Efflux-Transporter |
| BEP | 2-Brom-1-ethylpyridinium-Tetrafluoroborat |
| Boc | *tert.*-Butoxycarbonyl |
| br. | breit (bei NMR) |
| Bsp. | Beispiel |
| BxPC3 | humane Tumorzelllinie |
| *ca.* | *circa,* ungefähr |
| CI | chemische Ionisation (bei MS) |
| D | Dublett (bei NMR) |
| D | Tag(e) |
| DC | Dünnschichtchromatographie |
| DCI | direkte chemische Ionisation (bei MS) |
| DCM | Dichlormethan |
| Dd | Dublett von Dublett (bei NMR) |
| DMAP | 4-*N*,*N*-Dimethylaminopyridin |
| DME | 1,2-Dimethoxyethan |
| DMEM | Dulbecco's Modified Eagle Medium (standardisiertes Nährmedium für die Zellkultur) |
| DMF | *N*,*N*-Dimethylformamid |
| DMSO | Dimethylsulfoxid |
| D/P | Dye (Fluoreszentfarbstoff)/Protein Verhältnis |
| DPBS, D-PBS, PBS | Dulbecco's Phosphat-gepufferte Salz-Lösung<br>PBS = DPBS = D-PBS, pH7,4, Fa. Sigma, No D8537 Zusammensetzung:<br>0,2 g KCl<br>0,2 g $KH_2PO_4$ (anhyd)<br>8,0 g NaCl<br>1,15 g $Na_2HPO_4$ (anhyd)<br>ad 1 l mit $H_2O$ auffüllen |
| Dt | Dublett von Triplett (bei NMR) |
| DTT | DL-Dithiothreitol |
| d. Th. | der Theorie (bei chemischer Ausbeute) |
| EDC | *N'*-(3-Dimethylaminopropyl)-*N*-ethylcarbodiimid-Hydrochlorid |
| EGFR | Epidermal growth factor receptor = Epidermaler Wachstumsfaktor Rezeptor |
| EI | Elektronenstoß-Ionisation (bei MS) |
| ELISA | Enzyme-linked Immunosorbent Assay |
| eq. | Äquivalent(e) |
| ESI | Elektrospray-Ionisation (bei MS) |
| ESI-MicroTofq | ESI- MicroTofq (Name des Massenspektrometer mit Tof = Time Of Flight und q = Quadrupol) |
| FCS | fötales Kälberserum |
| Fmoc | (9*H*-Fluoren-9-ylmethoxy)carbonyl |

| | |
|---|---|
| ges. | Gesättigt |
| GTP | Guanosin-5'-triphosphat |
| H | Stunde(n) |
| HATU | *O*-(7-Azabenzotriazol-1-yl)-*N*,*N*,*N'*,*N'*-tetramethyluroniumhexafluorophosphat |
| HEPES | 4-(2-Hydroxyethyl)piperazin-1-ethansulfonsäure |
| HOAc | Essigsäure |
| HOAt | 1-Hydroxy-7-azabenzotriazol |
| HOBt | 1-Hydroxy-1*H*-benzotriazol-Hydrat |
| HOSu | *N*-Hydroxysuccinimid |
| HPLC | Hochdruck-, Hochleistungsflüssigchromatographie |
| $IC_{50}$ | halbmaximale Inhibitionskonzentration |
| i.m. | intramuskulär, Applikation in den Muskel |
| i.v. | intravenös, Applikation in die Vene |
| konz. | konzentriert |
| KPL-4 | humane Tumorzelllinien |
| KU-19-19 | humane Tumorzelllinie |
| LC-MS | Flüssigchromatographie-gekoppelte Massenspektrometrie |
| LLC-PK1-Zellen | Lewis lung carcinoma pork kidney cell line |
| L-MDR | Human MDR1 transfizierte LLC-PK1 Zellen |
| LoVo | humane Tumorzelllinie |
| m | Multiplett (bei NMR) |
| MDR1 | Multidrug resistence protein 1 |
| MeCN | Acetonitril |
| min | Minute(n) |
| MS | Massenspektrometrie |
| MTT | 3-(4,5-Dimethylthiazol-2-yl)-2,5-diphenyl-2H-tetrazoliumbromid |
| NCI-H292 | humane Tumorzelllinie |
| -Nme- | Eine am Stickstoffatom gebundene Methyl-Gruppe bedeutet |
| NMM | *N*-Methylmorpholin |
| NMP | *N*-Methyl-2-pyrrolidinon |
| NMR | Kernresonanzspektrometrie |
| NMRI | Mausstamm, Herkunft aus dem Naval Medical Research Institute (NMRI) |
| Nude Mäuse | Nacktmäuse(Versuchstiere) |
| NSCLC | Non small cell lung cancer (Nicht-kleinzelliges Bronchialkarzinom) |
| PBS | Phosphat-gepufferte Salz-Lösung |
| Pd/C | Palladium auf Aktivkohle |
| P-gp | P-Glycoprotein, ein Transporterprotein |
| PNGaseF | Enzym zur Zuckerabspaltung |
| quant. | quantitativ (bei Ausbeute) |
| Quart | Quartett (bei NMR) |
| Quint | Quintett (bei NMR) |
| $R_f$ | Retentionsindex (bei DC) |
| RT | Raumtemperatur |
| $R_t$ | Retentionszeit (bei HPLC) |
| S | Singulett (bei NMR) |
| s.c. | subcutan, Applikation unter die Haut |
| SCC-4 | humane Tumorzelllinie |
| SCID Mäuse | Versuchsmäuse mit einem schweren kombinierten Immundefekt (severe combined immunodeficiency) |
| SK-HEP-1 | humane Tumorzelllinie |
| t | Triplett (bei NMR) |
| TBAF | Tetra-n-butylammoniumfluorid |
| TCEP | Tris(2-carboxyethyl)phosphin |
| TEMPO | (2,2,6,6-Tetramethyl-piperidin-1-yl)oxyl |
| Teoc | Trimethylsilylethoxycarbonyl |
| *tert.* | Tertiär |
| TFA | Trifluoressigsäure |
| THF | Tetrahydrofuran |

| | |
|---|---|
| T3P® | 2,4,6-Tripropyl-1,3,5,2,4,6-trioxatriphosphinan-2,4,6-trioxid |
| U251 | humane Tumorzelllinie |
| UV | Ultraviolett-Spektrometrie |
| v/v | Volumen zu Volumen-Verhältnis (einer Lösung) |
| Z | Benzyloxycarbonyl |

**Aminosäuren Abkürzungen**

Ala = Alanin

Arg = Arginin

Asn = Asparagin

Asp =Asparaginsäure

Cys =Cystein

Glu = Glutaminsäure

Gln = Glutamin

Gly = Glycin

His = Histidin

Ile = Isoleucin

Leu = Leucin

Lys = Lysin

Met = Methionin

Nva = Norvalin

Phe = Phenylalanin

Pro = Prolin

Ser = Serin

Thr = Threonin

Trp = Tryptophan

Tyr = Tyrosin

Val = Valin

**HPLC- und LC-MS-Methoden:**

Methode 1 (LC-MS):

Instrument: Waters ACQUITY SQD UPLC System; Säule: Waters Acquity UPLC HSS T3 1.8 μ 50 × 1 mm; Eluent A: 1 l Wasser + 0.25 mL 99%ige Ameisensäure, Eluent B: 1 l Acetonitril + 0.25 mL 99%ige Ameisensäure;

Gradient: 0.0 min 90% A → 1.2 min 5% A → 2.0 min 5% A Ofen: 50°C; Fluss: 0.40 mL/min; UV-Detektion: 208 - 400 nm.

Methode 2 (LC-MS):

Gerätetyp MS: Waters Synapt G2S; Gerätetyp UPLC: Waters Acquity I-CLASS; Säule: Waters, BEH300, 2.1 × 150 mm, C18 1.7 μm; Eluent A: 1 l Wasser + 0.01% Ameisensäure; Eluent B: 1 l Acetonitril + 0.01% Ameisensäure; Gradient: 0.0 min 2% B → 1.5 min 2% B → 8.5 min 95% B → 10.0 min 95% B; Ofen: 50°C; Fluss: 0.50 mL/min; UV-Detektion: 220 nm

Methode 3 (LC-MS):

Instrument MS: Waters (Micromass) QM; Instrument HPLC: Agilent 1100 Serie; Säule : Agient ZORBAX Extend-C18 3.0x50mm 3.5-Micron; Eluent A: 1 l Wasser + 0.01 mol Ammoniumcarbonat, Eluent B: 1 l Acetonitril; Gradient: 0.0 min 98% A → 0.2min 98% A → 3.0 min 5% A→ 4.5 min 5% A ; Ofen: 40°C; Fluss: 1.75 mL/min; UV-Detektion: 210 nm

Methode 4 (LC-MS):

Gerätetyp MS: Waters Synapt G2S; Gerätetyp UPLC: Waters Acquity I-CLASS; Säule: Waters, HSST3, 2.1 × 50 mm, C18 1.8 μm; Eluent A: 1 l Wasser + 0.01% Ameisensäure; Eluent B: 1 l Acetonitril + 0.01% Ameisensäure; Gradient: 0.0 min 10% B → 0.3 min 10% B → 1.7 min 95% B → 2.5 min 95% B; Ofen: 50°C; Fluss: 1.20 mL/min; UV-Detektion: 210 nm

Methode 5 (LC-MS):

Instrument: Waters ACQUITY SQD UPLC System; Säule: Waters Acquity UPLC HSS T3 1.8 μ 50 × 1 mm; Eluent A: 1 l Wasser + 0.25 mL 99%ige Ameisensäure , Eluent B: 1 l Acetonitril + 0.25 mL 99%ige Ameisensäure; Gradient: 0.0 min 95% A → 6.0 min 5% A → 7.5 min 5% A Ofen: 50°C; Fluss: 0.35 mL/min; UV-Detektion: 210 - 400 nm.

Methode 6 (LC-MS):

Instrument: Micromass Quattro Premier mit Waters UPLC Acquity; Säule: Thermo Hypersil GOLD 1.9 μ 50 × 1 mm; Eluent A: 1 l Wasser + 0.5 mL 50%ige Ameisensäure, Eluent B: 1 l Acetonitril + 0.5 mL 50%ige Ameisensäure; Gradient: 0.0 min 97% A → 0.5 min 97% A → 3.2 min 5% A → 4.0 min 5% A Ofen: 50°C; Fluss: 0.3 mL/min; UV-Detektion: 210 nm.

Methode 7 (LC-MS):

Instrument: Agilent MS Quad 6150;HPLC: Agilent 1290; Säule: Waters Acquity UPLC HSS T3 1.8 μ 50 × 2.1 mm; Eluent A: 1 l Wasser + 0.25 mL 99%ige Ameisensäure , Eluent B: 1 l Acetonitril + 0.25 mL 99%ige Ameisensäure; Gradient: 0.0 min 90% A → 0.3 min 90% A → 1.7 min 5% A → 3.0 min 5% A Ofen: 50°C; Fluss: 1,20 mL/min; UV-Detektion: 205 - 305 nm.

Methode 8 (LC-MS):

Gerätetyp MS: Waters Synapt G2S; Gerätetyp UPLC: Waters Acquity I-CLASS; Säule: Waters, HSST3, 2.1 × 50 mm, C18 1.8 μm; Eluent A: 1 l Wasser + 0.01% Ameisensäure; Eluent B: 1 l Acetonitril + 0.01% Ameisensäure; Gradient: 0.0 min 2% B → 2.0 min 2% B → 13.0 min 90% B → 15.0 min 90% B; Ofen: 50°C; Fluss: 1.20 ml/min; UV-Detektion: 210 nm

Methode 9: LC-MS-Prep Reinigungsmethode für die Beispiele 181-191 (Methode LIND-LC-MS-Prep)

Instrument MS: Waters, Instrument HPLC: Waters (Säule Waters X-Bridge C18, 19 mm × 50 mm, 5 μm, Eluent A: Wasser + 0.05% Ammoniak, Eluent B: Acetonitril (ULC) mit Gradient; Fluss: 40 ml/min; UV-Detektion: DAD; 210 - 400 nm).

bzw.

Instrument MS: Waters, Instrument HPLC: Waters (Säule Phenomenex Luna 5μ C18(2) 100A, AXIA Tech. 50

× 21.2 mm, Eluent A: Wasser + 0.05% Ameisensäure, Eluent B: Acetonitril (ULC) mit Gradient; Fluss: 40 ml/min; UV-Detektion: DAD; 210 - 400 nm).

Methode 10: LC-MS-Analytik-Methode für die Beispiele 181-191 (LIND_SQD_SB_AQ)

Instrument MS: Waters SQD; Instrument HPLC: Waters UPLC; Säule: Zorbax SB-Aq (Agilent), 50 mm × 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

Methode 11 (HPLC):

| | |
|---|---|
| Gerät: | HP1100 Serie |
| Säule: | Merck Chromolith SpeedROD RP-18e, 50-4,6mm, Best.-Nr.1.51450.0001, Vorsäule Chromolith Guard Cartridge Kit, RP-18e, 5-4,6mm, Best.-Nr. 1.51470.0001 |
| Gradient: | Flow 5mL/ Min |
| | Injection Volume 5µl |
| | Solvent A: HCLO4 (70%ig) in Wasser (4mL/ l) |
| | Solvent B: Acetonitril |
| | Start 20% B |
| | 0.50 Min 20% B |
| | 3.00 Min 90% B |
| | 3.50 Min 90% B |
| | 3.51 Min 20% B |
| | 4.00 Min 20% B |
| Säulentemperatur: | 40°C |
| Wellenlänge: | 210nm |

Methode 12: (LC-MS):

Gerätetyp MS: Thermo Scientific FT-MS; Gerätetyp UHPLC+: Thermo Scientific UltiMate 3000; Säule: Waters, HSST3, 2.1 × 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm

Methode 13: (LC-MS):

Instrument MS: Waters (Micromass) Quattro Micro; Instrument Waters UPLC Acquity; Säule : Waters BEH C18 1.7 µ 50 × 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A→ 2.51 min 10% A → 3.0 min 10% A; Ofen: 40°C; Fluss: 0.5 ml/min; UV-Detektion: 210 nm

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien,

deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

Methode 14: (LC-MS) (MCW-LTQ-POROSHELL-TFA98-10min)

Gerätetyp MS: ThermoFisherScientific LTQ-Orbitrap-XL; Gerätetyp HPLC: Agilent 1200SL; Säule: Agilent, POROSHELL 120, 3 × 150 mm, SB - C18 2.7 μm; Eluent A: 1 l Wasser + 0.1% Trifluoressigsäure; Eluent B: 1 l Acetonitril + 0.1% Trifluoressigsäure; Gradient: 0.0 min 2% B → 0.3 min 2% B → 5.0 min 95% B → 10.0 min 95% B; Ofen: 40°C; Fluss: 0.75 ml/min; UV-Detektion: 210 nm

**Ausgangsverbindungen und Intermediate:**

Für die Herstellung der erfindungsgemäßen Verbindungen geeignete Ausgangsverbindungen sowie die Herstellung geeigneter Intermediate sind bereits in der WO2015/96982 A1 beschrieben.

Die Intermediate C1 bis C73, L1 bis L73, F1 bis F58 sowie F82 bis F91, F103 bis F129, F142 bis F156, F163 bis F180, F192 bis F196, F204 bis F207, F209 bis F218, F235, F236, F238, F241 bis F245, F247, F248 und F254 gemäß WO2015/96982 A1 gehören zur Offenbarung der vorliegenden Anmeldung. Sofern im Folgenden auf Verbindungen mit bestimmten Nummern verwiesen wird (z.B. Intermediat C1, L1 oder F1), handelt es sich um die Verbindungen mit diesen Nummern gemäß WO2015/96982 A1. Weitere Ausgangsverbindungen und Intermediate werden im Folgenden beschrieben.

**Intermediat C74**

Trifluoressigsäure -2-(trimethylsilyl)ethyl-3-amino-N-[(2S)-4-[{(1R)-1-[1-benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-D-alaninat (1:1)

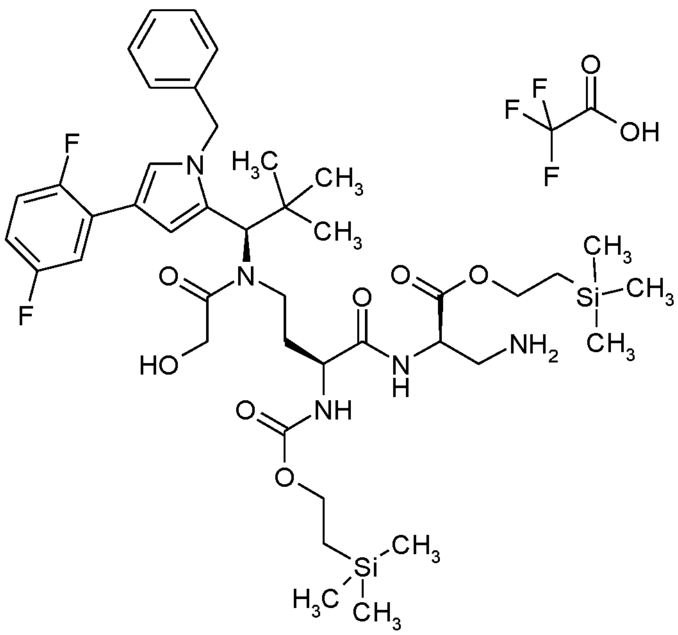

75mg (0.114 mmol) von Intermediat C58 wurden in 12.5 ml DMF aufgenommen und mit 78 mg (0.171 mmol) von Intermediat L75 in Gegenwart von 65 mg (0.11 mmol) HATU und 79 μL *N,N*-Diisopropylethylamin gekuppelt. Nach Reinigung durch präparative HPLC wurde das Intermediat in 20 ml Ethanol aufgenommen und über 10%-igem Palladium auf Aktivkohle 1 h bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1:1 wurden 63 mg (64% d.Th. über 2 Stufen) der Titelverbindung erhalten.

LC-MS (Methode 1): $R_t$ = 1.16 min; MS (Elpos): m/z = 844 $[M+H]^+$.

## Intermediat C75

Methyl-(2S)-4-[(acetoxyacetyl){(1R)-1-[1-benzyl-4-(2,5-difluorphenyl)-1H-pyrrol-2-yl]-2,2-dimethylpropyl}amino]-2-({[2-(trimethylsilyl)ethoxy]carbonyl}amino)butanoat

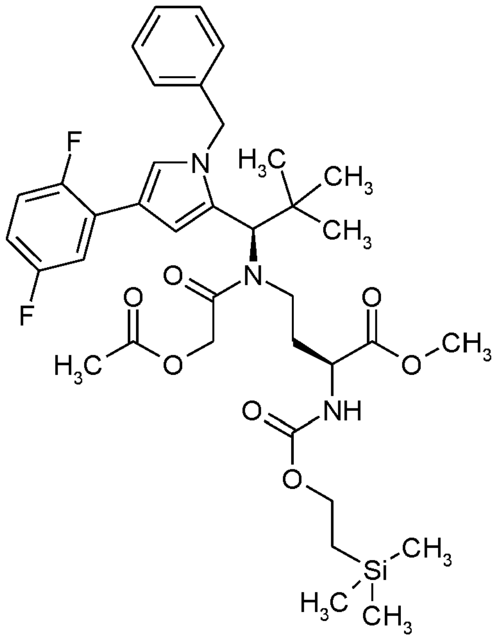

4.3 g (12.2 mmol) von Intermediat C52 wurden in 525 mL DCM gelöst und mit 3.63 g (17.12 mmol) Natriumtriacetoxyborhydrid sowie mit 8.4 mL Essigsäure versetzt. Nach 5 min Rühren bei RT wurden 3.23 g (11.85 mmol) Methyl-(2S)-4-oxo-2-({[2-(trimethylsilyl)ethoxy]carbonyl} amino)butanoat (hergestellt aus (3S)-3-Amino-4-methoxy-4-oxobutansäure nach klassischen Methoden) gelöst in 175 mL DCM zugegeben und der Ansatz weitere 45 min bei RT gerührt. Der Ansatz wurde dann mit DCM verdünnt und zweimal mit 100 mL gesättigter Natriumhydrogencarbonatlösung und dann mit gesättigter Natriumchloridlösung ausgeschüttelt. Die organische Phase wurde über Magnesiumsulfat getrocknet, filtriert und anschließend eingeengt. Der Rückstand wurde mittels präparativer HPLC aufgereinigt. Nach Vereinigung der entsprechenden Fraktionen, Einengen und

Trocknen des Rückstandes im Hochvakuum erhielt man 4.6 g (61% d. Th.) des Intermediats.

LC-MS (Methode 12): $R_t$ = 1.97 min; MS (ESIpos): m/z = 614.32 $(M+H)^+$.

200 mg (0.33 mmol) dieses Intermediats wurden in 10 mL DCM gelöst und dann mit 105 µL Triethylamin sowie mit 77 µL (0.717 mmol) Acetoxyacetylchlorid versetzt. Der Ansatz wurde über Nacht bei RT gerührt und dann im Vakuum eingeengt. Der Rückstand wurde in Ethylacetat aufgenommen und zweimal mit gesättigter Natriumhydrogencarbonatlösung und dann mit gesättigter Natriumchloridlösung ausgeschüttelt. Die organische Phase wurde über Magnesiumsulfat getrocknet und anschließend eingeengt. Es wurden 213 mg (75%) der Titelverbindung als beiger Schaum erhalten.

LC-MS (Methode 1): $R_t$ = 1.46 min; MS (ESIpos): m/z = 714 $(M+H)^+$.

## Intermediat C76

N-[(Benzyloxy)carbonyl]-L-valyl-N-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-alaninamid

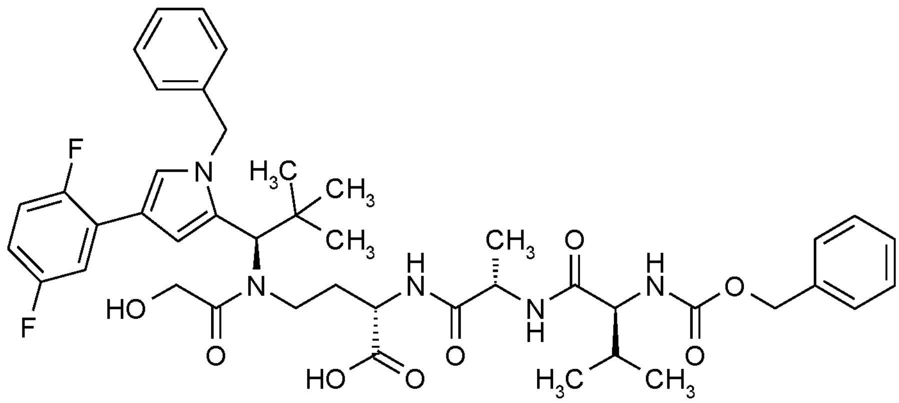

Die Titelverbindung wurde ausgehend von Intermediat C75 nach klassischen Methoden der Peptidchemie hergestellt (Spaltung der Teoc-Schutzgruppe mit Zinkchlorid, Acylierung mit N-[(Benzyloxy)carbonyl]-L-valyl-L-alanin in Gegenwart von HATU und Esterspaltung mit Lithiumhydroxid in THF/Wasser).

LC-MS (Methode 1): $R_t$ = 1.23 min; MS (ESIpos): m/z = 818 $(M+H)^+$.

## Intermediat C77

S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein

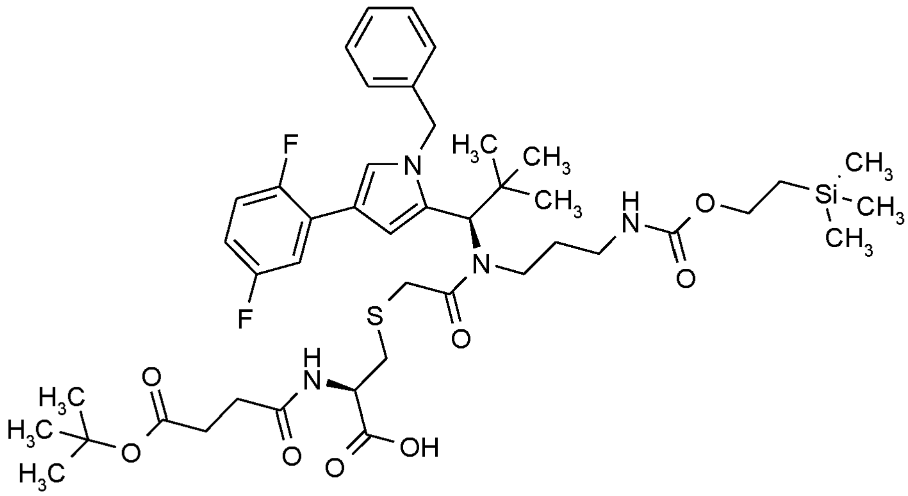

4-tert-Butoxy-4-oxobutansäure (8.39 mg, 48.1 μmol) wurde in 1.0 mL DMF vorgelegt, mit 7.37 mg (48.1 μmol) 1-Hydroxy-1H-benzotriazol Hydrat, 15.5 mg ((48.1 μmol) (Benzotriazol-1-yloxy)bisdimethylaminomethyliumfluorobora und 8.60 μl (48.1 μmol) N,N-Diisopropyl-ethylamin versetzt und 10 Minuten bei RT gerührt. 40.0 mg (0.048 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (Intermediat C71) wurden in 1.0 mL DMF vorgelegt, mit 25.4 μl (141.9 μmol) N,N-Diisopropylethylamin versetzt, zum Ansatz gegeben und 4 h bei RT gerührt. Die Reaktionsmischung wurde direkt mittles präp. RP-HPLC (Säule: Reprosil 125x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 35.0 mg (83 % d. Th.) der Titelverbindung.

LC-MS (Methode 12): $R_t$ = 2.76 min; MS (ESIpos): m/z = 873 $[M+H]^+$

**Intermediat C78**

11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-säure

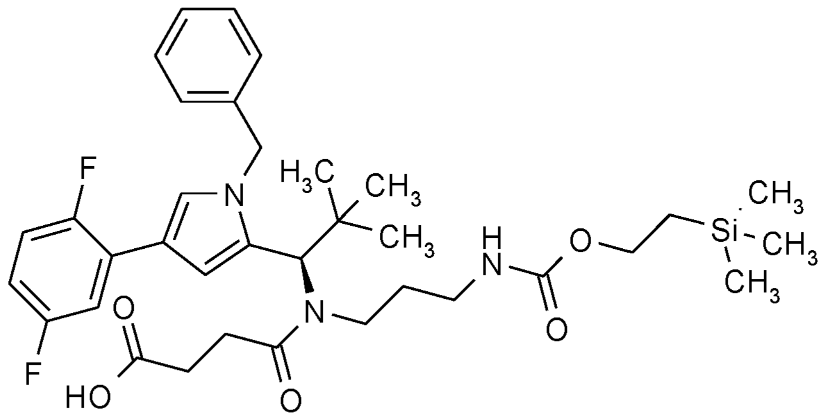

197 mg (0.354 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese Intermediat C11) wurden in 5.0 mL Dichlormethan vorgelegt und auf 40°C erhitzt. Bei dieser Temperatur wurden 240 µl (3.0 mmol) Pyridin und 220 µl (1.8 mmol) Methyl-4-chlor-4-oxobutanoat zugegeben und 1 h bei RT gerührt. Dann wurden 240 µl (3.0 mmol) Pyridin und 220 µl (1.8 mmol) Methyl-4-chlor-4-oxobutanoat zugegeben und 1 hbei RT gerührt. Dann wurden 240 µl (3.0 mmol) Pyridin und 220 µl (1.8 mmol) Methyl-4-chlor-4-oxobutanoat zugegeben und 1 h bei RT gerührt. Die Reaktionsmischung wurde mit Ethylacetat verdünnt und die organische Phase wurde je dreimal mit 5%iger $KHSO_4$-Lösung extrahiert. Die organische Phase wurde mit ges. NaCl-Lösung gewaschen und über Magnesiumsulfat getrocknet. Die Lösemittel wurden im Vakuum verdampft. Der Rückstand wurde mittels präp. RP-HPLC (Säule: Reprosil 250×30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 74.1 mg (31 % d. Th.) Methyl-11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-oat.

LC-MS (Methode 1): $R_t$ = 1.49 min; MS (ESIpos): m/z = 670 $[M+H]^+$

78.3 mg (117 µmol) Methyl-11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-oat wurden in 4.0 mL THF vorgelegt, mit 800 µl Methanol, 160 µl Wasser und 230 µl (230 µmol) wässriger LiOH-Lösung (1M) versetzt. Die Reaktionsmischung wurde 3 h bei RT gerührt, mit Essigsäure gequencht und direkt mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 64.8 mg (85 % d. Th.) der Titel Verbindung.

LC-MS (Methode 12): $R_t$ = 2.61 min; MS (ESIneg): m/z = 654 $[M-H]^-$

**Intermediat C79**

Trifluoressigsäure --2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1)

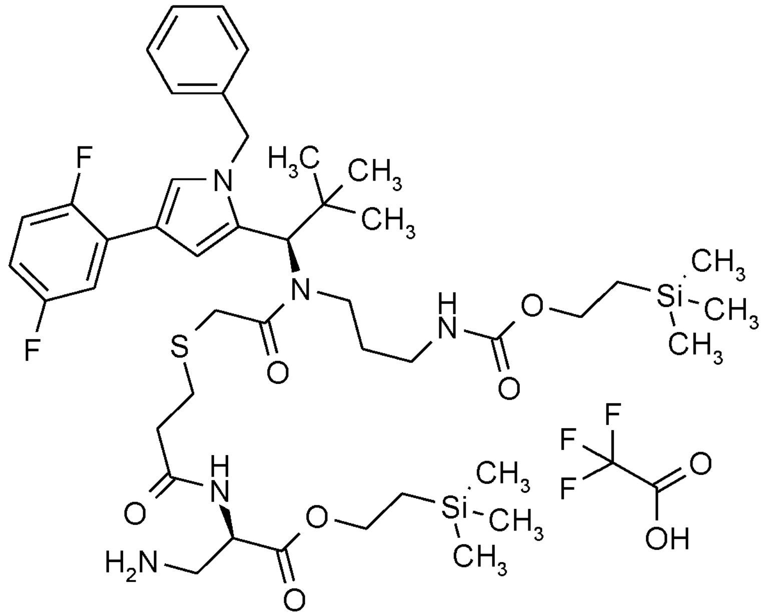

57.4 mg (81.8 μmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden in 5.7 mL DMF vorgelegt, mit 74.0 mg (164 μmol) Trifluoressigsäure 2-(trimethylsilyl)ethyl-3-{[(benzyloxy)carbonyl]amino}-D-alaninat (1:1) (Intermediat L75), 43 μl (250 μmol) N,N-Diisopropylethylamin und 62.2 mg (164 μmol) HATU versetzt und 1 h bei RT nachgerührt. Die Reaktionsmischung wurde 1 h bei RT gerührt, mit Essigsäure gequencht und direkt mittels präp. RP-HPLC (Säule: Reprosil 125x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 52.4 mg (63 % d. Th.) der Verbindung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{[(benzyloxy)carbonyl]amino}-D-alaninat. LC-MS (Methode 1): $R_t$ = 1.64 min; MS (ESIpos): m/z = 1022 $[M]^+$

Unter Argon wurden 6.23 mg (27.7 μmol) Palladium(II)acetat: in 3.0 ml Dichloromethan vorgelegt, mit 12 μL (83 μmol) Triethylamin und 89 μL (550 μmol) Triethylsilan versetzt und 5 Minuten. gerührt. Dann wurden 56.7 mg (55.5 μmol) 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{[(benzyloxy)carbonyl]amino}-D-alaninat in 3.0 mL Dichloromethan zugegeben und über Nacht bei RT gerührt. Der Ansatz wurde bis fast zur Trockene eingeengt, mit Acetonitril/Wasser versetzt, filtriert und mittels präp. RP-HPLC (Säule: Reprosil 125x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 37.4 mg (67 % d. Th.) der Titel Verbindung.

LC-MS (Methode 12): ): $R_t$ = 2.15 min; MS (ESIpos): m/z = 888 $[M+H]^+$

**Intermediat C80**

S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-(glycylamino)-4,7,10,13-tetraoxapentadecan-1-oyl]-L-cystein -trifluoressigsäure (1:1)

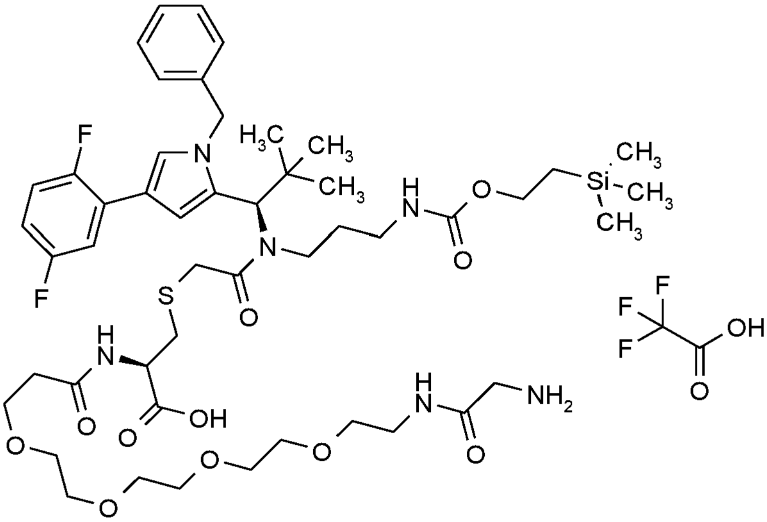

Unter Argon, wurden 43.4 mg (95.1 μmol) 1-({N-[(Benzyloxy)carbonyl]glycyl}amino)-3,6,9,12-tetraoxapentadecan-15-säure (Intermediat L90) in 2.5 ml DMF vorgelegt, mit 14.6 mg (95.1 μmol) 1-Hydroxy-1H-benzotriazol Hydrat, 30.5 mg (95.1 μmol) (Benzotriazol-1-yloxy)bisdimethyl-aminomethyliumfluoroborat und 16.5 μl (95.1 μmol) N,N-Diisopropylethylamin versetzt und 10 min gerührt. 79.0 mg (95.1 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (Intermediat C71) wurden in 2.5 ml DMF gelöst mit 49.5μl (285.3 μmol) N,N-Diisopropylethylamin versetzt und zum Ansatz gegeben. Die Reactionmischung wurde 2 h bei RT gerührt und direkt mittels präp. RP-HPLC (Säule: Reprosil 125x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 44.2 mg (40 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(benzyloxy)carbonyl]glycyl}amino)-4,7,10,13-tetraoxapentadecan-1-oyl]-L-cystein. LC-MS (Methode 12): $R_t$ = 2.57 min; MS (ESIpos): m/z = 1156 $[M+H]^+$

60.2 mg (52.1 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(benzyloxy)carbonyl]glycyl}amino)-4,7,10,13-tetraoxapentadecan-1-oyl]-L-cystein wurden in 3.0 mL Ethanol suspendiert und mit 6.0 mg Palladium auf Aktivkohle (10%ig) versetzt und bei RT 1 h lang mit Wasserstoff unter Normaldruck hydriert. Zweimal wurden 6.0 mg Palladium auf Aktivkohle (10%ig) versetzt und bei RT 1 h lang mit Wasserstoff unter Normaldruck hydriert. Der Katalysator wurde abfiltriert und die Reaktionsmischung im Vakuum vom Lösemittel befreit und im Hochvakuum getrocknet. Der Rückstand wurde mittels präp. RP-HPLC (Säule: Reprosil 125x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 29.4 mg (50 % d. Th.) der Titel Verbindung. LC-MS (Methode 5): $R_t$ = 3.77 min; MS (ESIpos): m/z = 1021 $[M+H]^+$

**Intermediat C81**

(R)-1-[1-Benzyl-4-(2,5-difluorphenyl)-1H-pyrrol-2-yl]-1-cyclohexylmethanamin

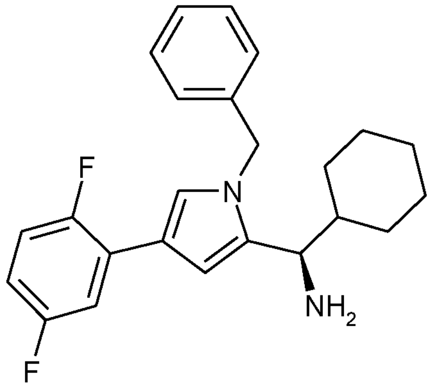

Eine Lösung von 3.12 ml (6.24 mmol) Dimethylzink in Toluol (2.0 M) unter Argon bei -78 °C wurde mit 18.7 ml (37.45 mmol) Cyclohexylmagnesiumchlorid in Diethylether (2M) versetzt und die Mischung wurde 30 Minuten bei -78 °C gerührt. Anschließend wurde eine Lösung von 5.0 g (12.48 mmol) (R)-N-{(E/Z)-[1-Benzyl-4-(2,5-difluorphenyl)-1H-pyrrol-2-yl]methylen}-2-methylpropan-2-sulfinamid in THF bei -78 °C zugegeben und das Reaktionsgemisch wurde 1 h bei dieser Temperatur gerührt und dann 4 h bei RT. Dann wurden bei -78 °C mL gesättigte Ammoniumchlorid-Lösung zugegeben und die Reaktionsmischung wurde auf RT kommen lassen. Es wurde mit Ethylacetat verdünnt und mit Wasser gewaschen. Die organische Phase wurde über Magnesiumsulfat getrocknet und das Lösemittel wurde im Vakuum verdampft. Der Rückstand wurde mittels Biotage Isolera gereinigt (Kieselgel, Ethylacetat/Cyclohexan 25:75). Man erhielt 1.59 g (26% d. Th.) der Zwischenstufe erhalten.

LC-MS (Methode 12): $R_t$ = 2.76 min; MS (ESIneg): m/z = 483 $[M-H]^-$

264.0 mg (0.54 mmol) dieser Zwischenstufe wurden in 0.5 mL 1,4-Dioxan unter Argon vorgelegt und dann mit 1.36 mL HCl in 1,4-Dioxan-Lösung (4.0 M) versetzt. Das Reaktionsgemisch wurde 1 h bei RT gerührt. Die Reaktionsmischung wurde mit Dichlormethan versetzt und mit einer wäs. 1M Natriumhydroxid-Lösung gewaschen. Die organische Phase wurde mit Magnesiumsulfat getrocknet und das Lösemittel wurde im Vakuum verdampft. Der Rückstand wurde mittels Biotage Isolera gereinigt (Kieselgel, Methanol/Dichlornethan 98:2). Das Lösemittel wurde im Vakuum verdampft und der Rückstand wurde in Dichlormethan gelöst und mit einer Natriumhydrogencarbonat-Lösung gewaschen und über Natriumsulfat getrocknet. Das Lösemittel wurde im Vakuum verdampft und der Rückstand wurde im Hochvakuum getrocknet. Man erhielt 148 mg (72% d. Th.) der Titelverbindung.

LC-MS (Methode 13): $R_t$ = 2.07 min; MS (ESIpos): m/z = 364 $[M-NH_2]^+$

## Intermediat C82

2-(Trimethylsilyl)ethyl-(3-{[(R)-[1-benzyl-4-(2,5-difluorphenyl)-1H-pyrrol-2-yl](cyclohexyl)methyl]amino}propyl)carbamat

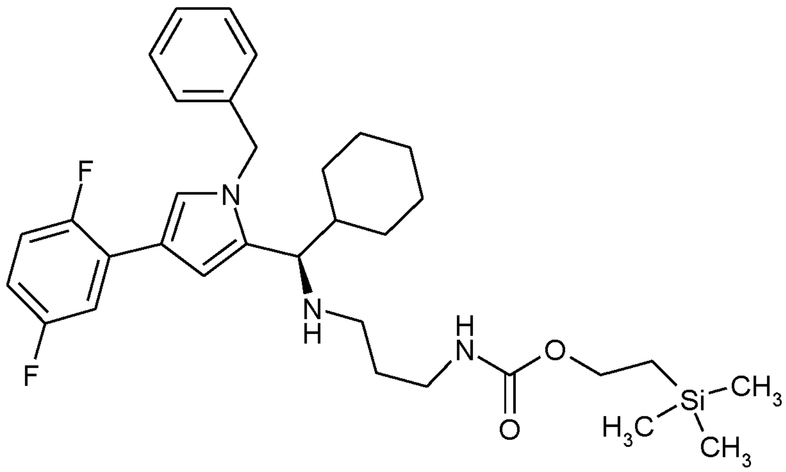

Eine Lösung von 503.0 mg (1.32 mmol) 1-[1-Benzyl-4-(2,5-difluorphenyl)-1H-pyrrol-2-yl]-1-cyclohexylmethanamin (Intermediat C81) in 1.4 ml Dichlormethan unter Argon wurde mit 392.2 mg (1.85 mmol) Natriumtriacetoxyborhydrid und 91.29 mg (1.52 mmol) Essigsaüre versetzt und das Reaktionsgemisch wurde 10 Minuten bei RT gerührt. Anschließend wurde eine Lösung von 574.6 (2.38 mmol) 2-(Trimethylsilyl)ethyl-(3-oxopropyl)carbamat in Dichlormethan hinzugegeben und das Gemisch wurde über Nacht bei RT gerührt. Nach Zugabe von 143 mg (0.66 mmol) 2-(Trimethylsilyl)ethyl-(3-oxopropyl)carbamat wurde die Mischung noch 2 h weiter gerührt. Das Reaktionsmgemisch wurde mit Dichlormethan verdünnt und die organische Phase wurde je zweimal mit ges. Natriumcarbonat-Lösung und mit ges. NaCl-Lösung gewaschen und über Natriumsulfat getrocknet und eingeengt. Der Rückstand wurde mittels präparative HPLC gereinigt. Die Lösemittel wurden im Vakuum verdampft und der Rückstand im Hochvakuum getrocknet. Man erhielt g (50% d. Th.) der Titelverbindung. Man erhielt 488 g (63 % d. Th.) der Titelverbindung.

LC-MS (Methode 12): $R_t$ = 1.89 min; MS (ESIpos): m/z = 582 $(M+H)^+$.

**Intermediat C83**

2-(Trimethylsilyl)ethyl-(3-{[(R)-[1-benzyl-4-(2,5-difluorphenyl)-1H-pyrrol-2-yl](cyclohexyl)methyl](chloracetyl)amino}propyl)carbamat

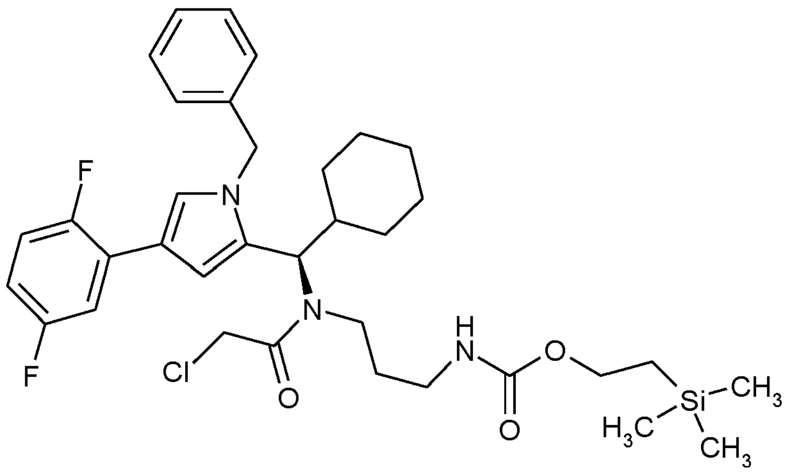

Zur eine Lösung von 487.9 mg (0.84 mmol) 2-(Trimethylsilyl)ethyl-(3-{[(R)-[1-benzyl-4-(2,5-difluorphenyl)-1H-pyrrol-2-yl](cyclohexyl)methyl]amino}propyl)carbamat (Intermediat C82) in 8.40 ml Dichlormethan mit Mol Sieb 4 Å wurden 280.0 mg (2.77 mmol) Triethylamin und 397.8 mg (3.52 mmol) Chloracetylchlorid zugegeben und das Reakionsgemisch wurde 6 h bei RT gerührt. Die Reaktionsmischung wurde mit Dichlormethan verdünnt und die organische Phase wurde mit ges. Natriumhydrogencarbonat-Lösung und ges. Ammoniumchlorid-Lösung gewaschen. Die organische Phase wurde über Natriumsulfat getrocknet und eingeengt. Der Rückstand wurde ohne Reinigung weiter eingesetzt. Man erhielt 470 mg (85 % d. Th.) der Titelverbindung.

LC-MS (Methode 12): $R_t$ = 2.88 min; MS (ESIpos): m/z = 680 $(M+Na)^+$.

**Intermediat C84**

S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-L-cystein

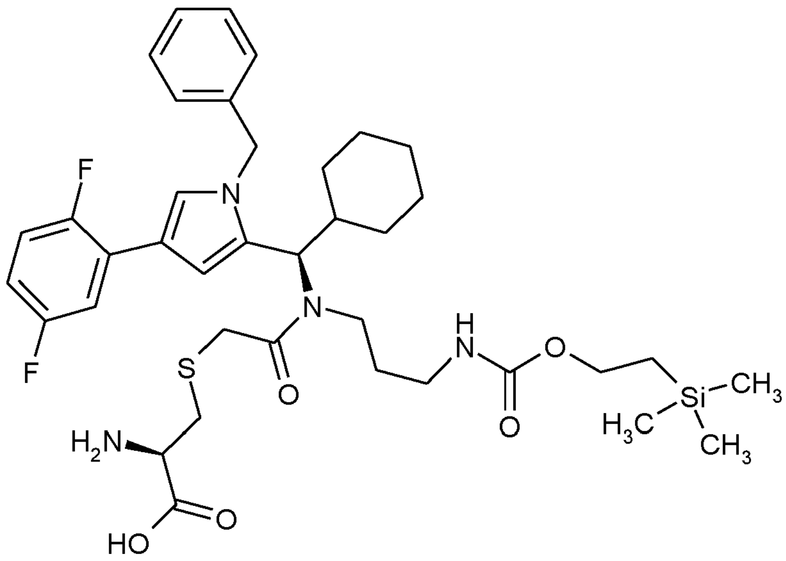

322.1 mg (2.66 mmol) L-Cystein wurden in 0.19 mL Wasser zusammen mit 319.0 mg (3.80 mmol) Natriumhydrogencarbonat suspendiert. Dazu wurden 250.0 mg (0.38 mmol) 2-(Trimethylsilyl)-ethyl-(3-{[(R)-[1-benzyl-4-(2,5-difluorphenyl)-1H-pyrrol-2-yl](cyclohexyl)methyl](chloracetyl)-amino}propyl)carbamat (Intermediat C83) gelöst in 1.90 mL *iso*-Propanol und 693.8 mg (4.56 mmol) 1,8-Diazabicyclo(5.4.0)undec-7-en gegeben. Die Reaktionsmischung wurde 3.5 h bei 50 °C gerührt. Die Reaktionsmischung wurde mit Ethylacetat versetzt und die org. Phase wurde mehrmals mit ges.

Natriumhydrogencarbonat-Lösung und mit ges. NaCl-Lösung gewaschen. Die organische Phase wurde über Natriumsulfat getrocknet und das Lösemittel im Vakuum verdampft. Der Rückstand wurde ohne weitere Reinigung weiter eingesetzt. Man erhielt 276 mg (97 % d. Th.) der Titelverbindung.

LC-MS (Methode 12): $R_t$ = 2.34 min; MS (ESIpos): m/z = 744 $(M+H)^+$.

## Intermediat C85

S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-N-[6-(2,5-dioxo-2,5-dihydro-1H-pyrrol-1-yl)hexanoyl]-L-cystein

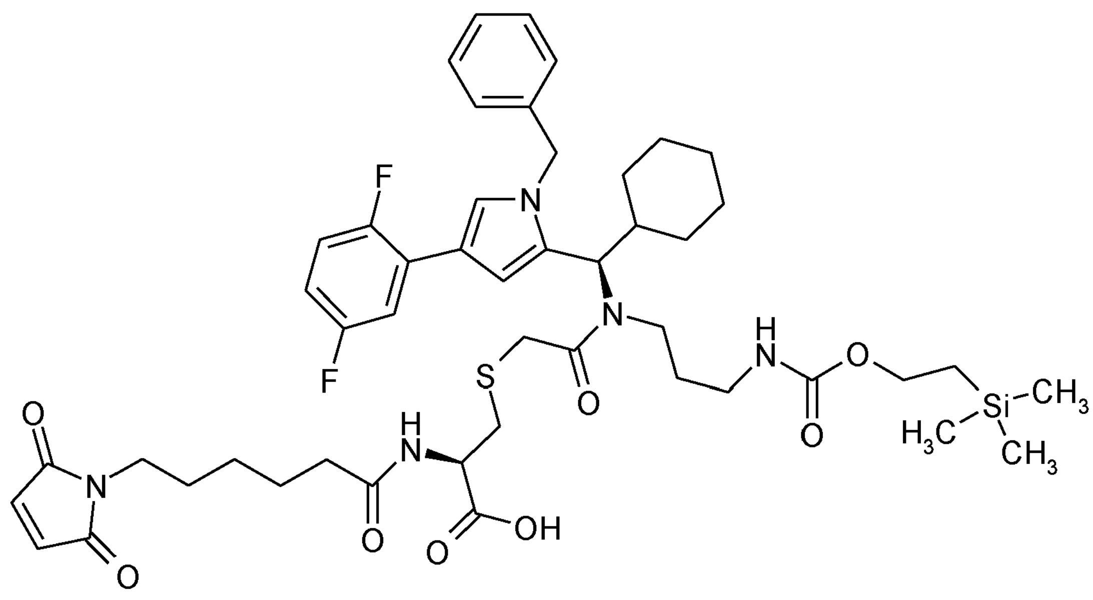

Zu einer Mischung von 100 mg (0.13 mmol) S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-L-cystein (1:1) (Intermediat C84) und 41.5 mg (0.13 mmol) 1-{6-[(2,5-Dioxopyrrolidin-1-yl)oxy]-6-oxohexyl}-1H-pyrrol-2,5-dion in 4.0 ml DMF wurden 34.8 mg ( 0.27 mmol) N,N-diisopropylethylamin hinzugegeben und das Reaktionsgemisch wurde 3 h bei RT gerührt. Die Mischung wurde ohne Aufarbeitung mittels präparative HPLC gereinigt. Man erhielt 88 mg (70% d. Th.) der Titelverbindung.

LC-MS (Methode 12): $R_t$ = 2.71 min; MS (ESIpos): m/z = 936 $(M+H)^+$.

**Intermediat C86**

11-[(R)-[1-Benzyl-4-(2,5-difluorphenyl)-1H-pyrrol-2-yl](cyclohexyl)methyl]-2,2-dimethyl-6,12-dioxo-5-oxa-14-thia-7,11-diaza-2-silaheptadecan-17-säure

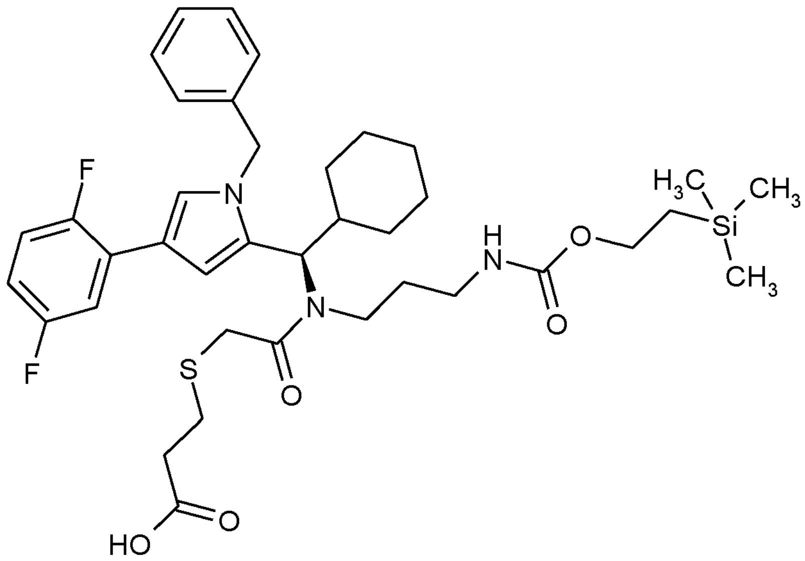

Eine Mischung von 220.0 mg (0.33 mmol) 2-(Trimethylsilyl)ethyl-(3-{[(R)-[1-benzyl-4-(2,5-difluorphenyl)-1H-pyrrol-2-yl](cyclohexyl)methyl](chloracetyl)amino}propyl)carbamat (Intermediat C83) und 39.02 mg (0.37 mmol) 3-Sulfanylpropansäure in 7.45 ml Methanol und einige Tropfen Wasser wurde mit 161.65 mg (1.17 mmol) Kaliumcarbonat versetzt. Das Reaktionsgemisch wurde 4 h bei 50 °C gerührt. Die Reaktionsmischung wurde mit Ethylacetat versetzt und die org. Phase wurde mehrmals mit Wasser und mit ges. NaCl-Lösung gewaschen. Die organische Phase wurde über Natriummsulfat getrocknet und das Lösemittel im Vakuum verdampft. Der Rückstand wurde ohne Aufarbeitung weiter eingesetzt. Man erhielt 201 mg (83 % d. Th.) der Titelverbindung.

LC-MS (Methode 12): $R_t$ = 2.72 min; MS (ESIneg): m/z = 726 $(M-H)^-$.

**Intermediat C87**

2-(Trimethylsilyl)ethyl-{13-[(R)-[1-benzyl-4-(2,5-difluorphenyl)-1H-pyrrol-2-yl](cyclohexyl)methyl]-1-(2,5-dioxo-2,5-dihydro-1H-pyrrol-1-yl)-2,7,12-trioxo-10-thia-3,6,13-triazahexadecan-16-yl}carbamat

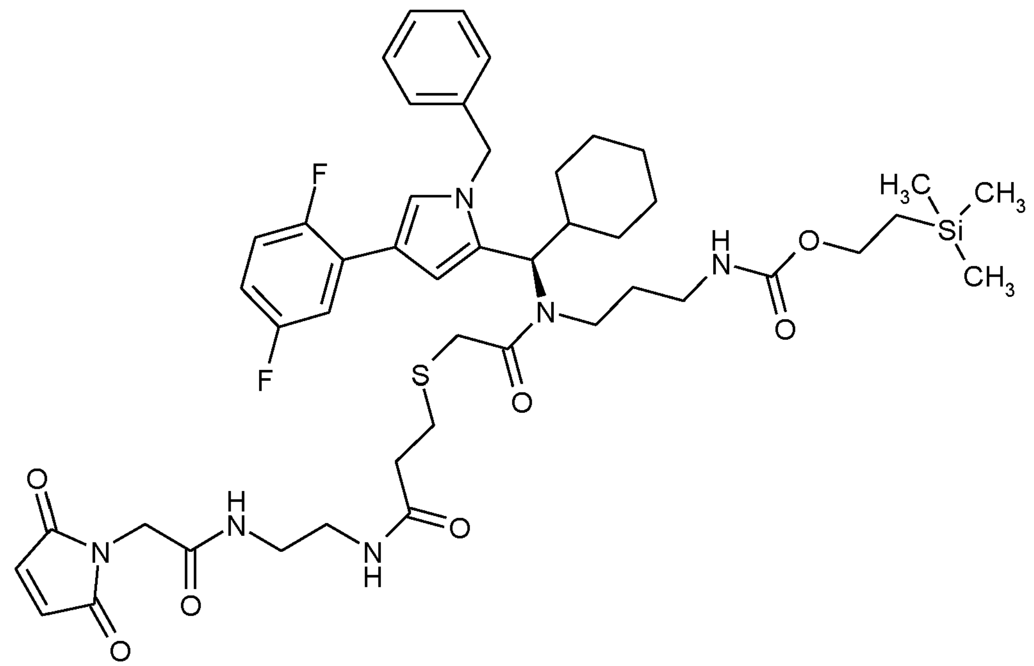

Zu einer Lösung von 100 mg (0.14 mmol) 11-[(R)-[1-Benzyl-4-(2,5-difluorphenyl)-1H-pyrrol-2-yl](cyclohexyl)methyl]-2,2-dimethyl-6,12-dioxo-5-oxa-14-thia-7,11-diaza-2-silaheptadecan-17-säure (Intermediat C86) in 1.37 ml DMF wurden 54.18 mg (0.28 mmol) N-(2-Aminoethyl)-2-(2,5-dioxo-2,5-dihydro-1H-pyrrol-1-yl)acetamid (Intermediat L1), 71.01 mg (0.50 mmol) N,N-diisopropylethylamin, 104.46 mg (0.27 mmol) HATU und 0.23 ml (0.14 mmol) 1-Hydoxy-7-Azabenzotriazole 0.5 M in DMF hinzugegeben. Das Reaktionsgemisch wurde 5 h bei RT gerührt. Die Mischung wurde ohne weitere Aufarbeitung mittels wurde mittels präparative HPLC gereinigt. Man erhielt 41 mg (33 % d. Th ) der Titelverbindung.

LC-MS (Methode 12): $R_t$ = 2.61 min; MS (ESIpos): m/z = 907 $(M+H)^+$.

## Intermediat C88

*tert*-Butyl-3-[({(1R)-1-[1-benzyl-4-(2,5-difluorphenyl)-1H-pyrrol-2-yl]-2,2-dimethylpropyl}amino)methyl]pyrrolidin-1-carboxylat-trifluoressigsäure (1:1)

Mischung aus Stereoisomeren

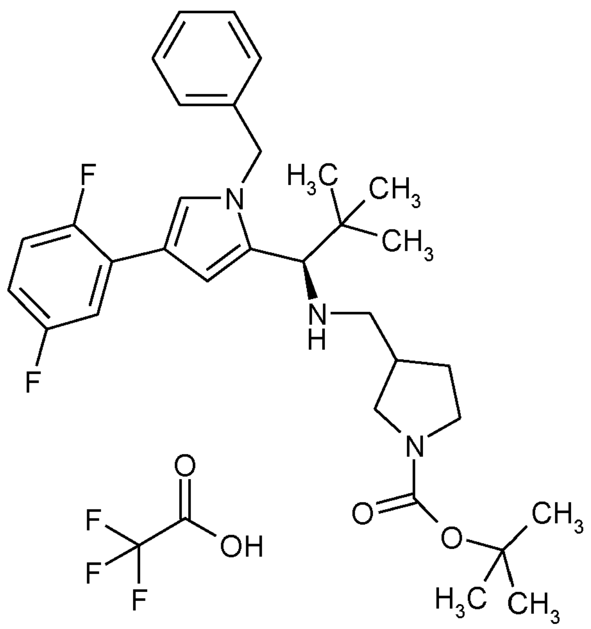

Eine Lösung von 2.04 g (5.75 mmol) (1R)-1-[1-Benzyl-4-(2,5-difluorphenyl)-1H-pyrrol-2-yl]-2,2-dimethylpropan-1-amin (Intermediat C52) in 51 ml Dichlormethan wurde mit 1.71 g (8.05 mmol) Natriumtriacetoxyborhydrid und 0.40 g (6.61 mmol) Essigsaüre versetzt und die Reaktionsgemisch wurde 5 Minuten bei RT gerührt. Anschließend wurde eine Lösung von 1.32 g (6.61 mmol) tert-Butyl-3-formylpyrrolidin-1-carboxylat in 20 ml Dichlormethan hinzugegeben und die Mischung wurde über Nacht bei RT gerührt. Die Reaktionsmischung wurde mit Ethylacetat verdünnt und die organische Phase wurde je zweimal mit ges. Natriumcarbonat-Lösung und mit ges. NaCl-Lösung gewaschen und über Magnesiumsulfat getrocknet und eingeengt. Der Rückstand wurde mittels präparative HPLC gereinigt. Die Lösemittel wurden im Vakuum verdampft und der Rückstand im Hochvakuum getrocknet. Man erhielt 1.86 g (50% d. Th.) der Titelverbindung.

LC-MS (Methode 1): $R_t$ = 0.99 min; MS (ESIpos): m/z = 538 $(M+H-CF_3CO_2H)^+$.

**Intermediat C89**

*tert*-Butyl-3-{[{(1R)-1-[1-benzyl-4-(2,5-difluorphenyl)-1H-pyrrol-2-yl]-2,2-dimethylpropyl}(chloracetyl)amino]methyl}pyrrolidin-1-carboxylat

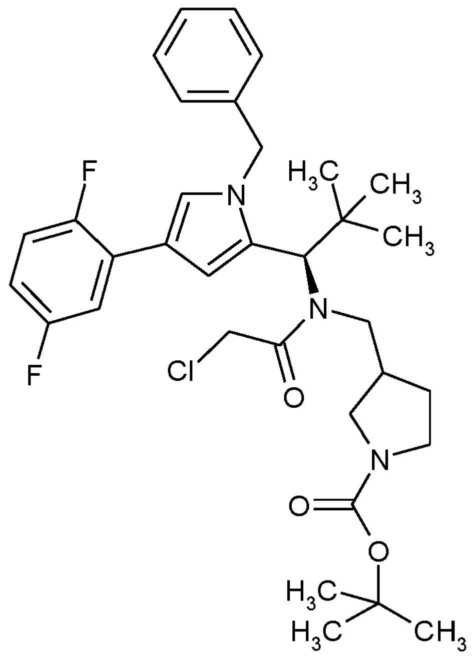

Eine Lösung von 2.89 g (4.19 mmol, 80 Reinheit) von tert-Butyl-3-[({(1R)-1-[1-benzyl-4-(2,5-difluorphenyl)-1H-pyrrol-2-yl]-2,2-dimethylpropyl}amino)methyl]pyrrolidin-1-carboxylat (Intermediat C88) in 42 ml Dichlormethan mit Molsieb 4 Å wurde mit 1.36 g (13.42 mmol) Triethylamin und 2.13 g (18.87 mmol) Chloracetylchlorid versetzt. Das Reaktionsgemisch wurde bei RT 5 h gerührt. Die Mischung wurde einrotiert und der Rückstand wurde mittels präparative HPLC gereinigt. Man erhielt 449 mg (17 % d. Th.) von Isomere 1 und 442 mg (17 % d. Th.) von Isomere 2 der Titelverbindung.

Isomer 1 LC-MS (Methode 1): $R_t$ = 2.74 min; MS (ESIpos): m/z = 614 $(M+H)^+$.

Isomer 2 LC-MS (Methode 1): $R_t$ = 2.78 min; MS (ESIpos): m/z = 614 $(M+H)^+$.

**Intermediat C90**

S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-L-cystein (Isomer 1)

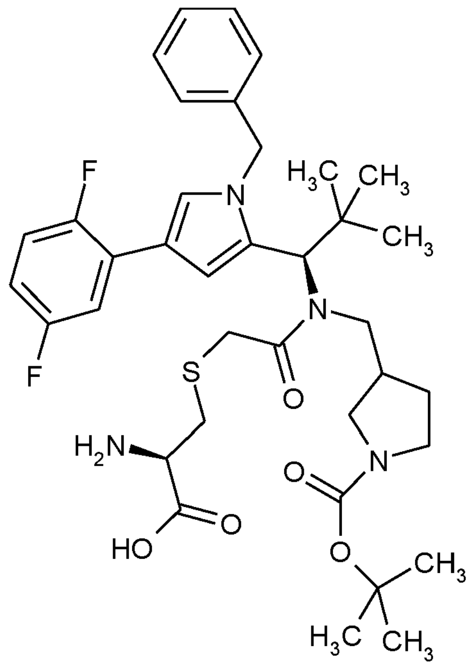

357.3 mg (0.58 mmol) L-Cystein wurden in 2.3 mL Wasser zusammen mit 488.7 mg (4.07 mmol) Natriumhydrogencarbonat suspendiert. Dazu wurden 357.0 mg (0.58 mmol tert-Butyl-3-{[{(1R)-1-[1-benzyl-4-(2,5-difluorphenyl)-1H-pyrrol-2-yl]-2,2-dimethylpropyl}(chloracetyl)amino]methyl}-pyrrolidin-1-carboxylat (Isomer 1) (Intermediat C89, Isomer 1) in 23.0 mL *iso*-Propanol gelöst und 1.06 g (6.98 mmol) 1,8-Diazabicyclo(5.4.0)undec-7-en gegeben. Die Reaktionsmischung wurde 3 h bei 50 °C gerührt. Die Reaktionsmischung wurde mit Ethylacetat versetzt und die org. Phase wurde mehrmals mit ges. Natriumhydrogencarbonat-Lösung und einmal mit ges. NaCl-Lösung gewaschen. Die organische Phase wurde über Magnesiumsulfat getrocknet und das Lösemittel im Vakuum verdampft. Der Rückstand wurde ohne Reinigung weiter eingesetzt. Man erhielt 255.0 mg (62 % d. Th.) der Titelverbindung.

LC-MS (Methode 1): $R_t$ = 1.09 min; MS (ESIpos): m/z = 699 $(M+H)^+$.

## Intermediat C91

S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-L-cystein (Isomer 2)

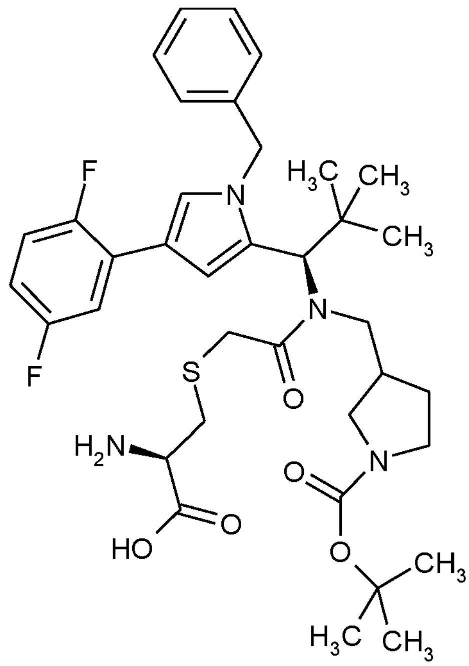

453.5 mg (3.74 mmol) L-Cystein wurden in 2.1 mL Wasser zusammen mit 449.2 mg (5.35 mmol) Natriumhydrogencarbonat suspendiert. Dazu wurden 3287.4 mg (0.54) mmol tert-Butyl-3-{[{(1R)-1-[1-benzyl-4-(2,5-difluorphenyl)-1H-pyrrol-2-yl]-2,2-dimethylpropyl}(chloracetyl)amino]-methyl}pyrrolidin-1-carboxylat (Intermediat C89, Isomer 2) in 21.1 mL *iso*-Propanol gelöst und 0.98 g (6.42 mmol) 1,8-Diazabicyclo(5.4.0)undec-7-en gegeben. Die Reaktionsmischung wurde 3 h bei 50 °C gerührt. Die Reaktionsmischung wurde mit Ethylacetat versetzt und die org. Phase wurde mehrmals mit ges. Natriumhydrogencarbonat-Lösung und einmal mit ges. NaCl-Lösung gewaschen. Die organische Phase wurde über Magnesiumsulfat getrocknet und das Lösemittel im Vakuum verdampft. Der Rückstand wurde ohne Reinigung weiter eingesetzt. Man erhielt 221.0 mg (59 % d. Th.) der Titelverbindung.

LC-MS (Methode 1): $R_t$ = 1.12 min; MS (ESIpos): m/z = 699 $(M+H)^+$.

**Intermediat C92**

S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Isomer 1)

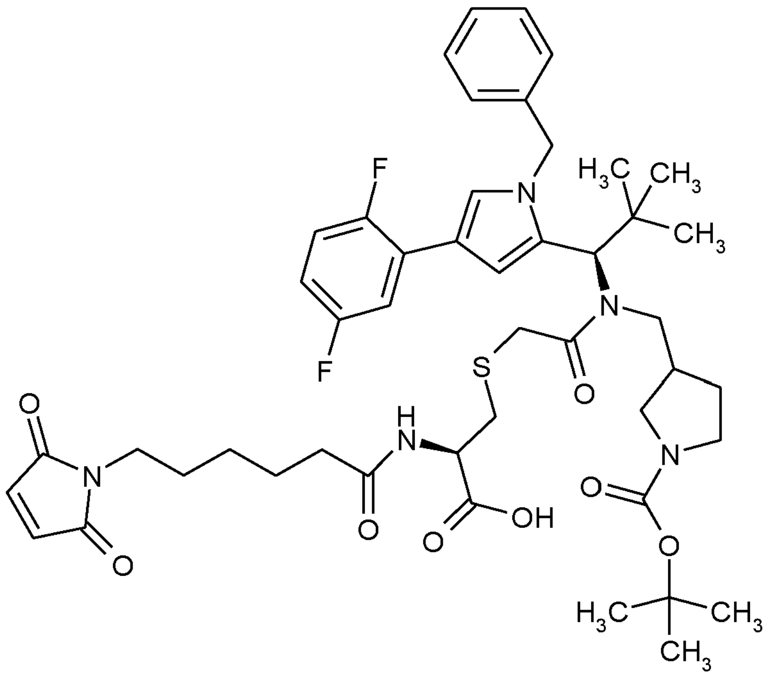

Eine Mischung von 50 mg (0.07 mmol) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-L-cystein (Intermediat C90) und 22.06 mg (0.07 mmol) 1-{6-[(2,5-Dioxopyrrolidin-1-yl)oxy]-6-oxohexyl}-1H-pyrrol-2,5-dion in 3.3 ml DMF wurde mit 18.49 mg (0.14 mmol) N,N-Diisopropylethylamin versetzt und das Reaktionsgemisch wurde bei RT 45 Minuten gerührt. Die Mischung wurde ohne Aufarbeitung mittels mittels präparative HPLC gereinigt. Man erhielt 65 mg (100 % d. Th., 71 % Reinheit) der Titelverbindung.

LC-MS (Methode 1): $R_t$ = 1.31 min; MS (ESIpos): m/z = 892 $(M+H)^+$.

## Intermediat C93

S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Isomer 2)

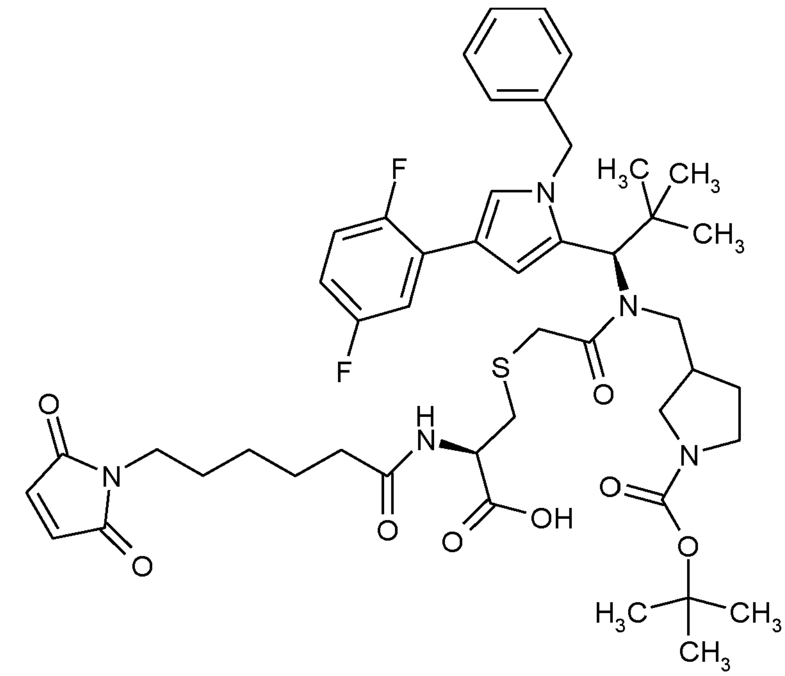

Eine Mischung von 50.0 mg (0.07 mmol) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-L-cystein (Intermediat C91) und 22.06 mg (0.07 mmol) 1-{6-[(2,5-Dioxopyrrolidin-1-yl)oxy]-6-oxohexyl}-1H-pyrrol-2,5-dion in 3.0 ml DMF wurde mit 18.49 mg (0.14 mmol) N,N-Diisopropylethylamin versetzt und das Reaktionsgemisch wurde bei RT 90 Minuten gerührt. Die Mischung wurde ohne Aufarbeitung mittels präparative HPLC gereinigt. Man erhielt 63 mg (98 % d. Th, 73 % Reinheit)der Titelverbindung.

LC-MS (Methode 1): $R_t$ = 1.34 min; MS (ESIpos): m/z = 892 $(M+H)^+$.

__Intermediat C94__

S-[2-({(1R)-1-[1-Benzyl-4-(2,5-difluorphenyl)-1H-pyrrol-2-yl]-2,2-dimethylpropyl}{[(1-(tert-butoxycarbonyl)pyrrolidin-3-yl]methyl}amino)-2-oxoethyl]-N-[(2,5-dioxo-2,5-dihydro-1H-pyrrol-1-yl)acetyl]-L-cystein (Isomer 1)

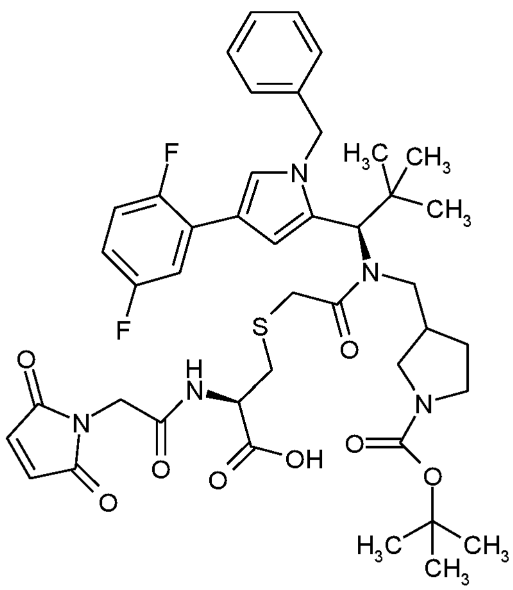

Eine Mischung von (50.0 mg, 0.07 mmol) S-[2-({(1R)-1-[1-Benzyl-4-(2,5-difluorphenyl)-1H-pyrrol-2-yl]-2,2-dimethylpropyl}{[(3R)-1-(tert-butoxycarbonyl)pyrrolidin-3-yl]methyl}amino)-2-oxoethyl]-L-cystein (Intermediat C90) und 18.0 mg (0.07 mmol) -{2-[(2,5-Dioxopyrrolidin-1-yl)oxy]-2-oxoethyl}-1H-pyrrol-2,5-dion in 3.3 ml DMF wurde mit 18.5 mg (0.14 mmol) N,N-Diisopropylethylamin versetzt und das Reaktionsgemisch wurde 30 Minuten bei RT gerührt. Die Reaktionsmischung wurde mit Ethylacetat versetzt und die organische Phase wurde mehrmals mit ges. $NH_4Cl$-Lösung und mit ges. NaCl-Lösung gewaschen. Die organische Phase wurde über Magnesiumsulfat getrocknet und das Lösemittel im Vakuum verdampft. Der Rückstand wurde ohne weitere Reinigung eingesetzt. Man erhielt 57 mg (81 % d. Th, 85 % Reinheit) der Titelverbindung.

LC-MS (Methode 1): $R_t$ = 0.96 min; MS (ESIpos): m/z = 836 $(M+H)^+$.

## Intermediat C95

3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Isomer 1)

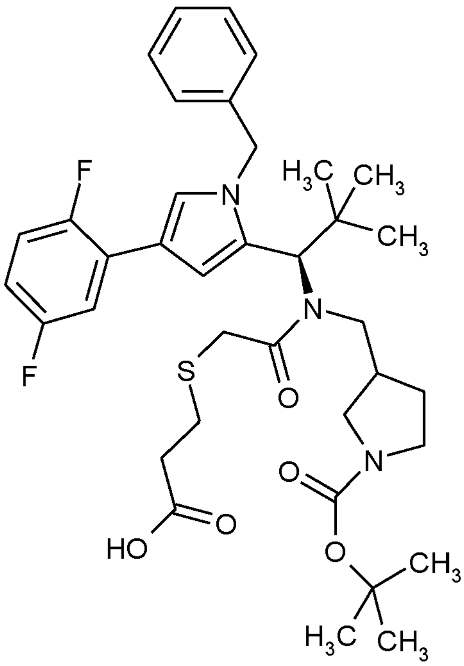

Eine Mischung von 384.0 mg (0.62 mmol) tert-Butyl-3-{[{(1R)-1-[1-benzyl-4-(2,5-difluorphenyl)-1H-pyrrol-2-yl]-2,2-dimethylpropyl}(chloracetyl)amino]methyl}pyrrolidin-1-carboxylat (Intermediat C89, Isomer 1) und 73.0 mg (0.69 mmol) 3-Sulfanylpropansäure in 14 ml Methanol und einige Tropfen Wasser wurde mit 302.5 mg (2.19 mmol) Kaliumcarbonat versetzt. Das Reaktionsgemisch wurde 2.5 h bei 50 °C gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne Aufarbeitung weiter eingesetzt. Man erhielt 358.0 mg (84 % d. Th.) der Titelverbindung.

LC-MS (Methode 1): $R_t$ = 1.33 min; MS (ESIpos): m/z = 684 $(M+H)^+$.

## Intermediat C96

3-{[2-({(1R)-1-[1-Benzyl-4-(2,5-difluorphenyl)-1 H-pyrrol-2-yl]-2,2-dimethylpropyl}{[1-(tert-butoxycarbonyl)pyrrolidin-3-yl]methyl}amino)-2-oxoethyl]sulfanyl}propansäure (Isomer 2)

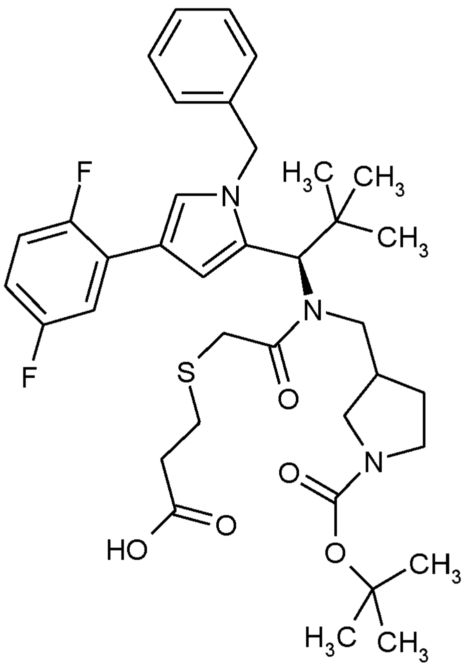

Eine Mischung von 287.0 mg (0.45 mmol) tert-Butyl-3-{[{(1R)-1-[1-benzyl-4-(2,5-difluorphenyl)-1H-pyrrol-2-yl]-2,2-dimethylpropyl}(chloracetyl)amino]methyl}pyrrolidin-1-carboxylat (Intermediat C89, Isomer 2) und 54.6 mg (0.51 mmol) 3-Sulfanylpropansäure in 14 ml Methanol und einige Tropfen Wasser wurde mit 226.0 mg (1.64 mmol) Kaliumcarbonat versetzt. Das Reaktionsgemisch wurde 2.5 h bei 50 °C gerührt. Die Reaktionsmischung wurde mit Ethylacetat versetzt und die org. Phase wurde mehrmals mit Wasser und mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne Aufarbeitung weiter eingesetzt. Man erhielt 318.7 mg (88 % d. Th., 88 % Reinheit) der Titelverbindung.

LC-MS (Methode 1): $R_t$ = 1.36 min; MS (ESIpos): m/z = 684 $(M+H)^+$.

## Intermediat C97

tert-Butyl-3-[2-{(1R)-1-[1-benzyl-4-(2,5-difluorphenyl)-1H-pyrrol-2-yl]-2,2-dimethylpropyl}-14-(2,5-dioxo-2,5-dihydro-1H-pyrrol-1-yl)-3,8,13-trioxo-5-thia-2,9,12-triazatetradec-1-yl]pyrrolidin-1-carboxylat (Isomer 2)

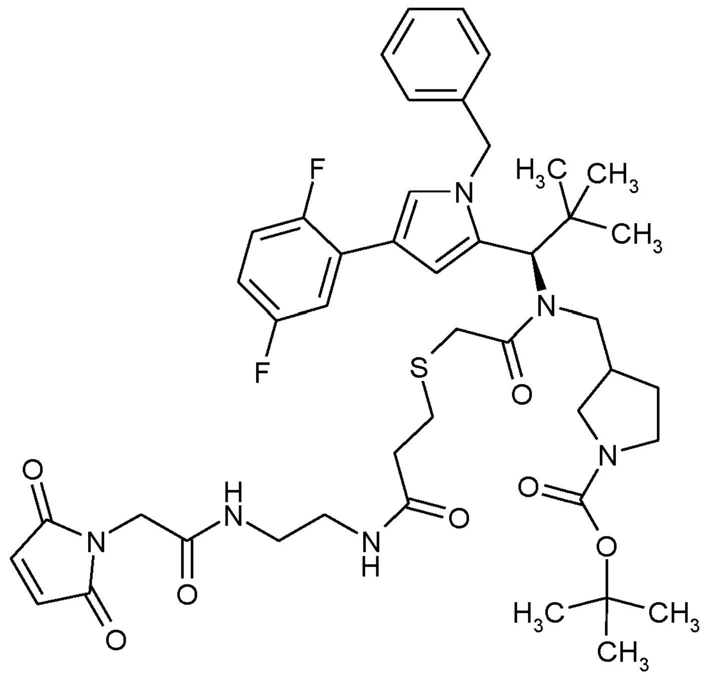

Zu einer Lösung von 25.0 mg (0.04 mmol) von 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Intermediat C96) in 2.81 ml DMF unter Argon wurden 14.17 mg (0.11 mmol) N,N-diisoprylethylamin und 27.80 mg (0.07 mmol) HATU hinzugegeben. Das Reaktionsgemisch wurde 10 Minuten bei RT gerührt. Anschließend wurde eine Lösung von 22.75 mg (0.07 mmol) N-(2-Aminoethyl)-2-(2,5-dioxo-2,5-dihydro-1H-pyrrol-1-yl)acetamid -ethan (1:1) Trifluoressigsäure (Intermediat L1) in 1.4 ml DMF und 5 mg (0.04 mmol) N,N-diisopropylethylamin hinzugegeben und das Gemisch wurde ü.N bei RT gerührt. Die Mischung wurde

LC-MS (Methode 5): $R_t$ = 4.39 min; MS (ESIpos): m/z = 86 mit Wasser versetzt und mit Dichlormethan extrahiert. Die organische Phase wurde über Magnesiumsulfat getrocknet und das Lösemittel im Vakuum verdampft. Der Rückstand wurde ohne Aufarbeitung weiter eingesetzt. Man erhielt 26 mg (84 % d. Th.) der Titelverbindung.3 $(M+H)^+$.

## Intermediat C98

tert-Butyl-3-[2-{(1R)-1-[1-benzyl-4-(2,5-difluorphenyl)-1H-pyrrol-2-yl]-2,2-dimethylpropyl}-18-(2,5-dioxo-2,5-dihydro-1H-pyrrol-1-yl)-3,8,13-trioxo-5-thia-2,9,12-triazaoctadec-1-yl]pyrrolidin-1-carboxylat (Isomer 2)

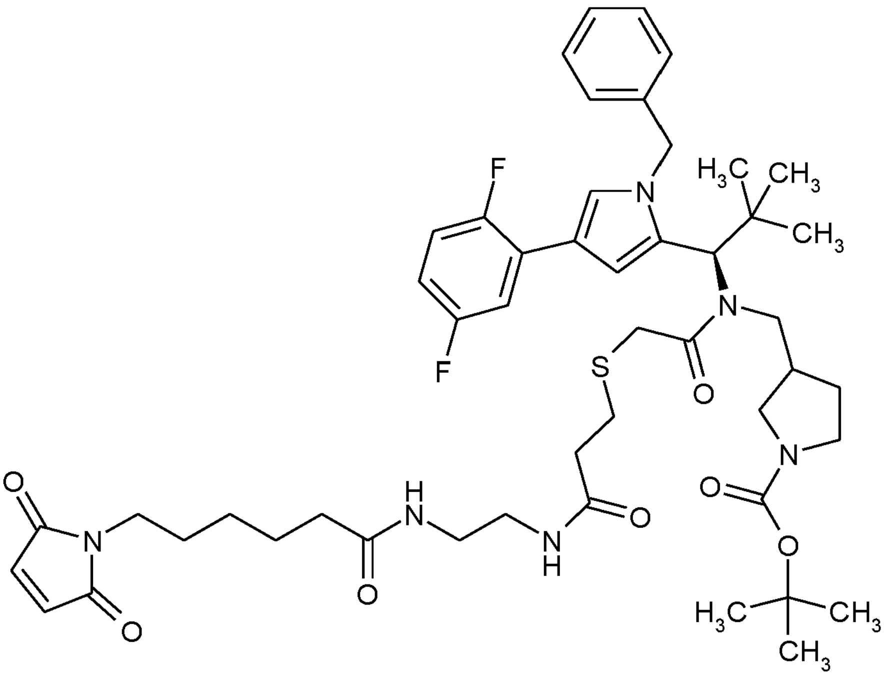

Zu einer Lösung von 25.0 mg (0.04 mmol) von 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Intermediat C96) in 2.81 ml DMF unter Argon wurden 14.17 mg (0.11 mmol) N,N-diisoprylethylamin und 27.80 mg (0.07 mmol) HATU hinzugegeben. Das Reaktionsgemisch wurde 10 Minuten bei RT gerührt. Anschließend wurde eine Lösung von 37.30 mg (0.07 mmol) N-(2-Aminoethyl)-6-(2,5-dioxo-2,5-dihydro-1H-pyrrol-1-yl)hexanamid -ethan (1:1) Trifluoressigsäure in 1.4 ml DMF und 5 mg (0.04 mmol) N,N-diisopropylethylamin hinzugegeben und das Gemisch wurde üN bei RT gerührt. Die Mischung wurde mit Wasser versetzt und mit Dichlormethan extrahiert. Die organische Phase wurde über Magnesiumsulfat getrocknet und das Lösemittel im Vakuum verdampft. Der Rückstand wurde ohne weitere Reinigung eingesetzt. Man erhielt 22 mg (63 % d. Th.) der Titelverbindung.

LC-MS (Methode 5): $R_t$ = 4.54 min; MS (ESIpos): m/z = 919 $(M+H)^+$.

## Intermediat C99

tert-Butyl-3-[2-{(1R)-1-[1-benzyl-4-(2,5-difluorphenyl)-1H-pyrrol-2-yl]-2,2-dimethylpropyl}-24-(2,5-dioxo-2,5-dihydro-1H-pyrrol-1-yl)-3,8,19-trioxo-12,15-dioxa-5-thia-2,9,18-triazatetracos-1-yl]pyrrolidin-1-carboxylat (Isomer 2)

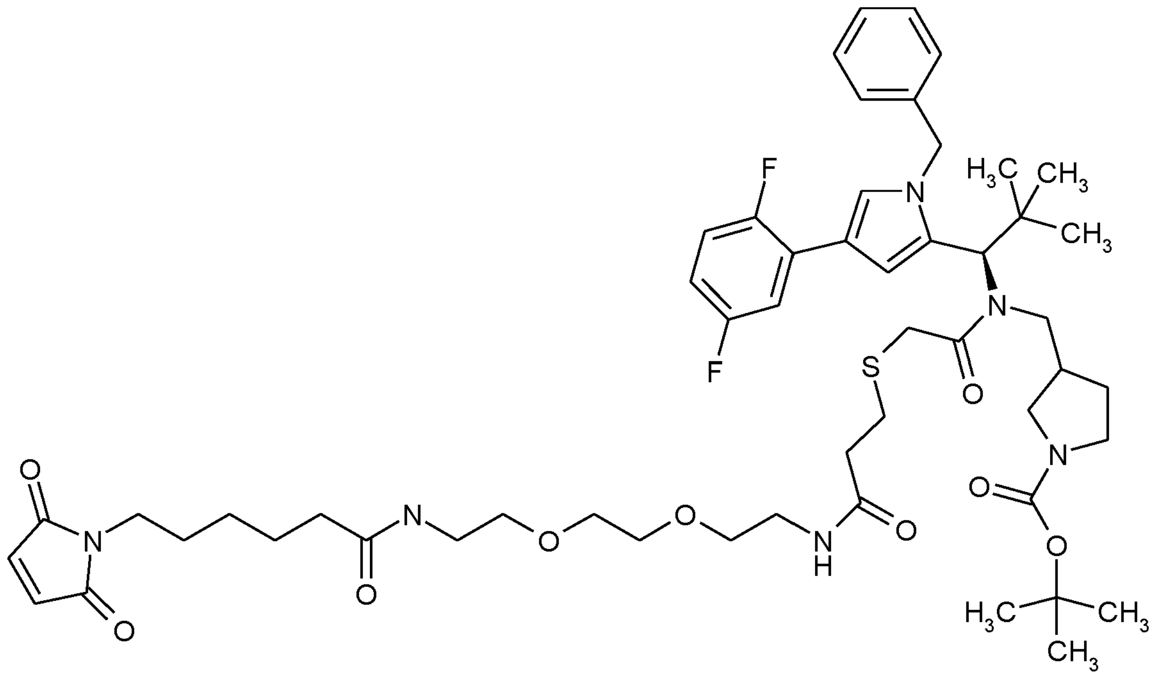

Zu einer Lösung von 25.0 mg (0.04 mmol) von 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Intermediat C96) in 2.81 ml DMF unter Argon wurden 14.17 mg (0.11 mmol) N,N-diisoprylethylamin und 27.80 mg (0.07 mmol) HATU hinzugegeben. Das Reaktionsgemisch wurde 10 Minuten bei RT gerührt. Anschließend wurde eine Lösung von 35.05 mg (0.07 mmol) N-{2-[2-(2-Aminoethoxy)ethoxy]ethyl}-6-(2,5-dioxo-2,5-dihydro-1H-pyrrol-1-yl)hexanamid -ethan (1:1)Trifluoressigsäure (Intermediat L82) in 1.4 ml DMF und 5 mg (0.04 mmol) N,N-diisopropylethylamin hinzugegeben und das Gemisch wurde üN bei RT gerührt. Die Mischung wurde mit Wasser versetzt und mit Dichlormethan extrahiert. Die organische Phase wurde über Magnesiumsulfat getrocknet und das Lösemittel im Vakuum verdampft und der Rückstand im Hochvakuum getrocknet. Der Rückstand wurdemittels präp. HPLC gereinigt Man erhielt 25 mg (60 % d. Th.) der Titelverbindung.

LC-MS (Methode 1): $R_t$ = 4.52 min; MS (ESIpos): m/z = 1007 $(M+H)^+$.

**Intermediat C100**

2-(Trimethylsilyl)ethyl-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2R)-2-(2,5-dioxo-2,5-dihydro-1H-pyrrol-1-yl)propanoyl]amino}ethyl)amino]-1-oxobutan-2-yl}carbamat

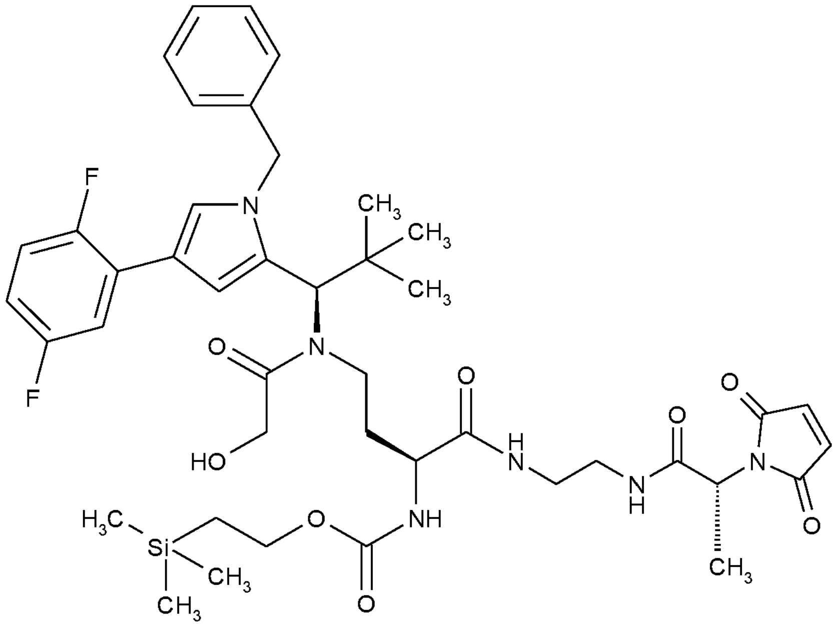

Zu einer Lösung von 45 mg (0.068 mmol) (2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butansäure (Intermediat C58) in 5.8 ml DMF wurden 22.2 mg (0.068 mmol) (2R)-N-(2-aminoethyl)-2-(2,5-dioxo-2,5-dihydro-1H-pyrrol-1-yl)propanamid (1:1) Trifluoressigsäure zugegeben. Nach 30 Minuten Rühren bei RT die Mischung wurde mit 39 mg (0.10 mmol) HATU und 36 mg (0.27 mmol) N,N-Diisopropylethylamin versetzt. Das Reaktionsgemisch wurde 1 h bei RT gerührt. Das Gemisch wurde ohne Aufarbeitung mittels präparative HPLC gereinigt. Man erhielt 7 mg (12 % d. Th) der Titelverbindung.
LC-MS (Methode 1): $R_t$ = 1.41 min; MS (ESIpos): m/z 851 $(M+H)^+$.

## Intermediat C101

Trifluoressigsäure-methyl-(2S)-4-[(acetoxyacetyl){(1R)-1-[1-benzyl-4-(2,5-difluorphenyl)-1H-pyrrol-2-yl]-2,2-dimethylpropyl}amino]-2-aminobutanoat (1:1)

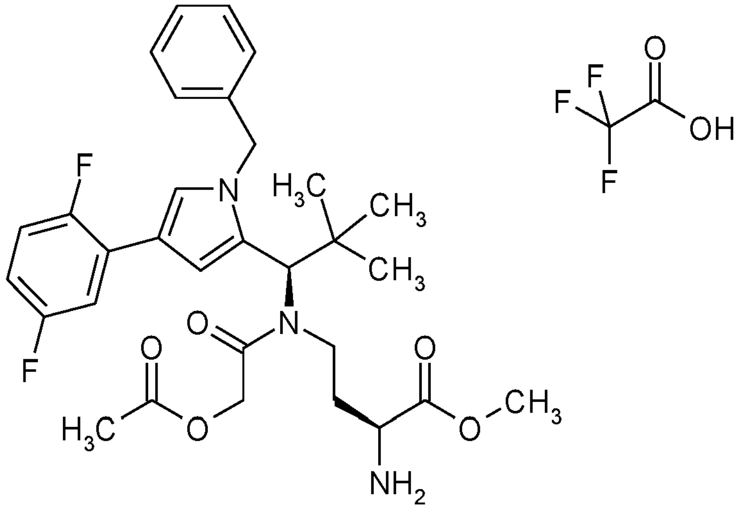

4.3 g (12.2 mmol) von Intermediat C52 wurden in 525 mL DCM gelöst und mit 3.63 g (17.12 mmol) Natriumtriacetoxyborhydrid sowie mit 8.4 mL Essigsäure versetzt. Nach 5 min Rühren bei RT wurden 3.23 g (11.85 mmol) Methyl-(2S)-4-oxo-2-({[2-(trimethylsilyl)ethoxy]carbonyl} amino)butanoat (hergestellt aus (3S)-3-Amino-4-methoxy-4-oxobutansäure nach klassischen Methoden) gelöst in 175 mL DCM zugegeben und der Ansatz weitere 45 min bei RT

gerührt. Der Ansatz wurde dann mit DCM verdünnt und zweimal mit 100 mL gesättigter Natriumhydrogencarbonatlösung und dann mit gesättigter Natriumchloridlösung ausgeschüttelt. Die organische Phase wurde über Magnesiumsulfat getrocknet, filtriert und anschließend eingeengt. Der Rückstand wurde mittels präparativer HPLC aufgereinigt. Nach Vereinigung der entsprechenden Fraktionen, Einengen und Trocknen des Rückstandes im Hochvakuum erhielt man 4.6 g (61% d. Th.) des Intermediats.

LC-MS (Methode 12): $R_t$ = 1.97 min; MS (ESIpos): m/z = 614.32 $(M+H)^+$.

2.06 g (3.36 mmol) von diesem Intermediat wurden in 76 mL DCM vorgelegt und mit 0.81 mL (7.17 mmol) 2-Chlor-2-oxoethylacetat in Gegenwart von 2.1 ml Triethylamin acyliert. Nach 20 h Rühren bei RT wurden weitere 0.36 mL 2-Chlor-2-oxoethylacetat und 0.94 ml Triethylamin zugegeben und der Ansatz weitere 15 min bei RT gerührt. Anschließend wurde mit 500 mL Ethylacetat verdünnt und nacheinander zweimal mit 300 mL 5%-iger Zitronensäure, zweimal mit 300 mL gesättigter Natriumhydrogencarbonatlösung und einmal mit 100 mL gesättigter Natriumchloridlösung ausgeschüttelt, dann über Magnesiumsulfat getrocknet und eingeengt. Nach Trocknung im Hochvakuum wurden 2.17 g (79% d. Th.) des geschützten Intermediats erhalten.

LC-MS (Methode 1): $R_t$ = 1.48 min; MS (ESIpos): m/z = 714 $(M+H)^+$.

321 mg (0.342 mmol) von diesem Intermediat wurde in 7 mL 2,2,2-Trifluorethanol gelöst. Es wurden 279.5 mg (2.05 mmol) Zinkchlorid zugegeben und der Ansatz 2 h bei 50°C gerührt. Anschließend wurden 599 mg (2.05 mmol) Ethylendiamin-N,N,N',N'-tetraessigsäure und 2 mL einer 0.1%igen Trifluoressigsäurelösung in Wasser hinzugegeben und der Ansatz anschließend im Vakuum eingeengt. Der Rückstand wurde mittels präparativer HPLC gereinigt. Nach Einengen der entsprechenden Fraktionen und Lyophilisation des Rückstands aus Acetonitril/Wasser wurden 60 mg (26% d.Th.) der Titelverbindung erhalten, die zum Teil noch einen Teil der de-acetylierten Verbindung enthielt.

LC-MS (Methode 1): $R_t$ = 0.91 min und 0.95 min; MS (ESIpos): m/z = 528 und 570 $(M+H)^+$.

## Intermediat C102

(2S)-4-[{(1R)-1-[1-Benzyl-4-(2,5-difluorphenyl)-1H-pyrrol-2-yl]-2,2-dimethylpropyl} (glycoloyl)amino]-2-{[(benzyloxy)carbonyl]amino}butansäure

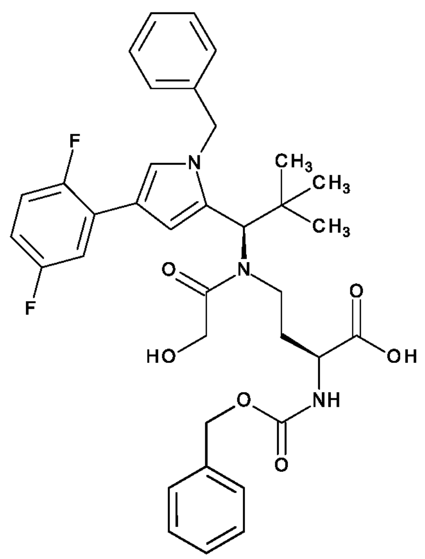

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat acyliert und schließlich mit 2M Lithiumhydroxidlösung in Methanol die Hydrolyse der beiden Estergruppen durchgeführt. LC-MS (Methode 1): $R_t$ = 1.31 min; MS (ESIpos): m/z = 646 $(M-H)^-$.

## Intermediat C103

2-(Trimethylsilyl)ethyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-N2-{[2-(trimethylsilyl) ethoxy]carbonyl}-L-glutaminat

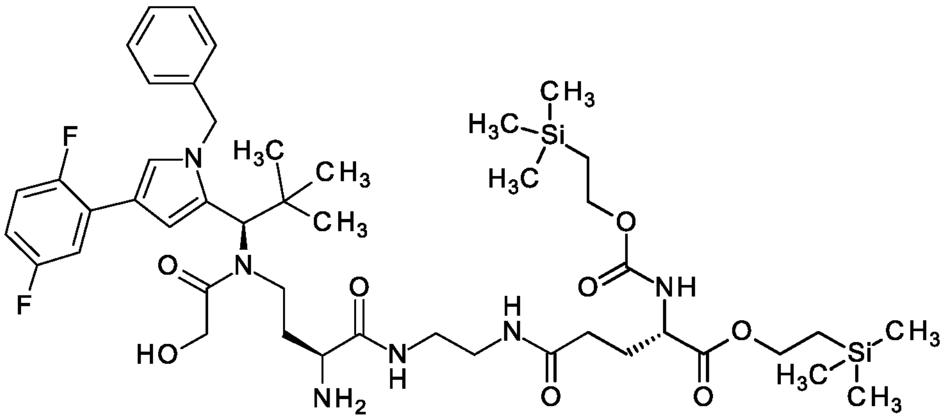

Die Titelverbindung wurde zunächst durch Kupplung von 151 mg (0.23 mmol) Intermediat C102 mit 128 mg (0.234 mmol) Intermediat L98 in DMF in Gegenwart von HATU und N,N-Diisopropylethylamin hergestellt. Anschließend wurde die Z-Schutzgruppe durch 30-minütige Hydrierung über 10%-igem Palladium auf Aktivkohle bei RT unter Wasserstoff-Normaldruck entfernt, wobei die Titelverbindung erhalten wurde.

Ausb.: 30% d. Th. über 2 Stufen

LC-MS (Methode 1): $R_t$ = 1.14 min; MS (ESIpos): m/z = 929 $(M+H)^+$.

## Intermediat C104

2-(Trimethylsilyl)ethyl-(3R,4R)-3-[({(1R)-1-[1-benzyl-4-(2,5-difluorphenyl)-1H-pyrrol-2-yl]-2,2-dimethylpropyl}amino)methyl]-4-fluorpyrrolidin-1-carboxylat

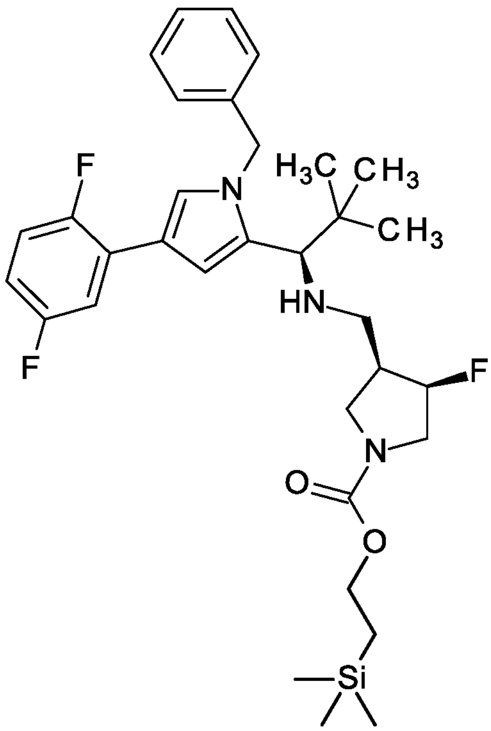

Eine Lösung von 2.24 g (6.31 mmol) (1R)-1-[1-Benzyl-4-(2,5-difluorphenyl)-1H-pyrrol-2-yl]-2,2-dimethylpropan-1-amin in 56.0 ml Dichlormethan mit Mol Sieb 4 Å wurde mit 1.87 g (8.84 mmol) Natriumtriacetoxyborhydrid versetz, und das Gemisch wurde 15 Minuten bei Raumtemperatur gerührt. Anschließend wurden 2.20 g (7.58 mmol) 2-(Trimethylsilyl)ethyl-(3R,4S)-3-fluor-4-formylpyrrolidin-1-carboxylat (WO 2014/151030A1) zugegeben, und das Reaktionsgemisch wurde 3.5 h bei Raumtemperatur gerührt. Das Gemisch wurde mit Dichlormethan verdünnt und die organische Phase wurde mit ges. Natriumhydrogencarbonat-Lösung und Wasser gewaschen. Die organische Phase wurde über Natriumsulfat getrocknet und eingeengt. Der Rückstand wurdemittels präp. HPLC gereinigt. Man erhielt 1.39 g (24 % d. Th.) der Titelverbindung.

LC-MS (Methode 1): $R_t$ = 1.15 min; MS (ESIpos): m/z = 600 $(M+H)^+$.

## Intermediat C105

2-(Trimethylsilyl)ethyl-(3R,4R)-3-{[{(1R)-1-[1-benzyl-4-(2,5-difluorphenyl)-1H-pyrrol-2-yl]-2,2-dimethylpropyl}(chloracetyl)amino]methyl}-4-fluorpyrrolidin-1-carboxylat

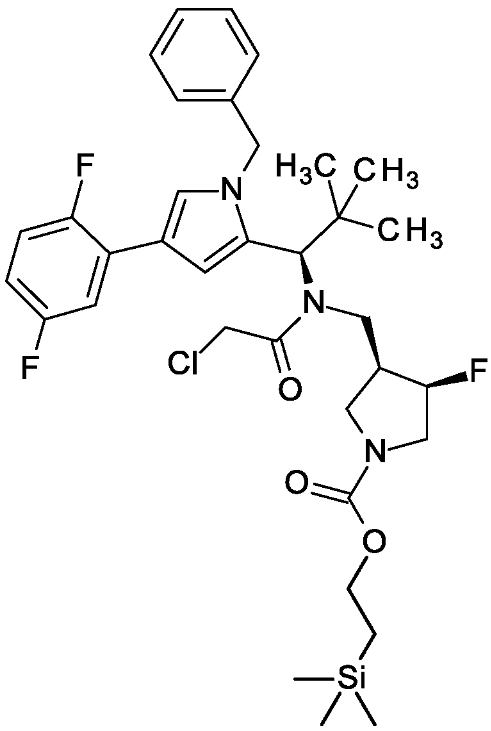

Zur eine Lösung von 692.8 mg (0.88 mmol) 2-(Trimethylsilyl)ethyl-(3R,4R)-3-[({(1R)-1-[1-benzyl-4-(2,5-difluorphenyl)-1H-pyrrol-2-yl]-2,2-dimethylpropyl}amino)methyl]-4-fluorpyrrolidin-1-carboxylat (Intermediat C104) in 8.7 ml Dichlormethan mit Mol Sieb 4Å wurden 295.0 mg (2.91 mmol) Triethylamin und 418.9 mg (3.71 mmol) Chloracetylchlorid zugegeben und das Reakionsgemisch wurde 2.5 h bei RT gerührt. Die Reaktionsmischung wurde mit Dichlormethan verdünnt und die organische Phase wurde mit ges. Natriumhydrogencarbonat-Lösung und ges. Ammoniumchlorid-Lösung gewaschen. Die organische Phase wurde über Natriumsulfat getrocknet und eingeengt. Der Rückstand wurde nochmal in 8.7 ml Dichlormethan mit Mol Sieb 4Å wurden 295.0 mg (2.91 mmol) Triethylamin und 418.9 mg (3.71 mmol) Chloracetylchlorid zugegeben und das Reakionsgemisch wurde 3 h bei RT gerührt. Die Reaktionsmischung wurde mit Dichlormethan verdünnt und die organische Phase wurde mit ges. Natriumhydrogencarbonat-Lösung und ges. Ammoniumchlorid-Lösung gewaschen. Die organische Phase wurde über Natriumsulfat getrocknet und eingeengt. Die organische Phase wurde über Natriumsulfat getrocknet, eingeengt und ohne Reinigung weiter eingesetzt. Man erhielt 691 mg (74 % d. Th., 64% Reinheit) der Titelverbindung.

LC-MS (Methode 1): $R_t$ = 1.78 min; MS (ESIpos): m/z = 676 $(M+H)^+$.

## Intermediat C106

3-{[2-({(1R)-1-[1-Benzyl-4-(2,5-difluorphenyl)-1H-pyrrol-2-yl]-2,2-dimethylpropyl}{[(3R,4R)-4-fluor-1-{[2-(trimethylsilyl)ethoxy]carbonyl}pyrrolidin-3-yl]methyl}amino)-2-oxoethyl]sulfanyl}propansäure

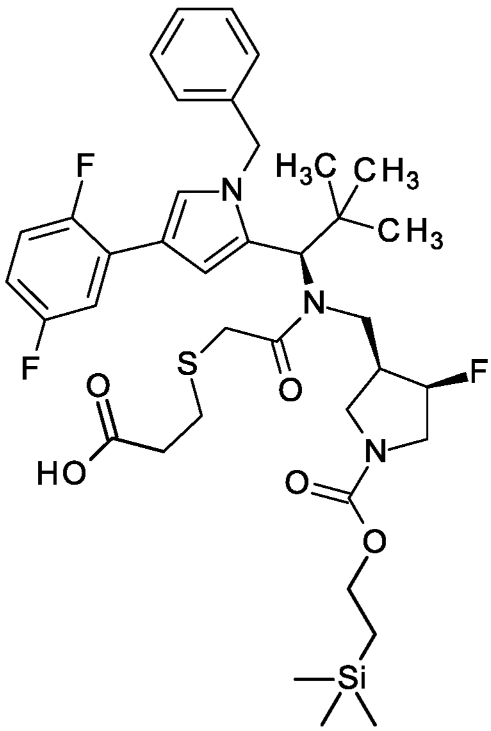

Eine Mischung von 691.0 mg (0.65 mmol) 2-(Trimethylsilyl)ethyl-(3R,4R)-3-{[{(1R)-1-[1-benzyl-4-(2,5-difluorphenyl)-1H-pyrrol-2-yl]-2,2-dimethylpropyl}(chloracetyl)-amino]methyl}-4-fluorpyrrolidin-1-carboxylat (Intermediat C105) und 76.3 mg (0.72 mmol) 3-Sulfanylpropansäure in 15 ml Methanol und einige Tropfen Wasser wurde mit 316 mg (2.29 mmol) Kaliumcarbonat versetzt. Das Reaktionsgemisch wurde 1.5 h bei 50 °C gerührt. Die Reaktionsmischung wurde mit Ethylacetat versetzt und die org. Phase wurde mehrmals mit Wasser und mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne Aufarbeitung weiter eingesetzt. Man erhielt 502 mg (67 % d. Th., 65% Reinheit) der Titelverbindung.

LC-MS (Methode 1): $R_t$ = 1.48 min; MS (ESIneg): m/z = 744 $(M-H)^-$.

**Intermediat C107**

S-[2-({(1R)-1-[1-Benzyl-4-(2,5-difluorphenyl)-1H-pyrrol-2-yl]-2,2-dimethylpropyl}{[(3R,4R)-4-fluor-1-{[2-(trimethylsilyl)ethoxy]carbonyl}pyrrolidin-3-yl]methyl}amino)-2-oxoethyl]-L-cystein

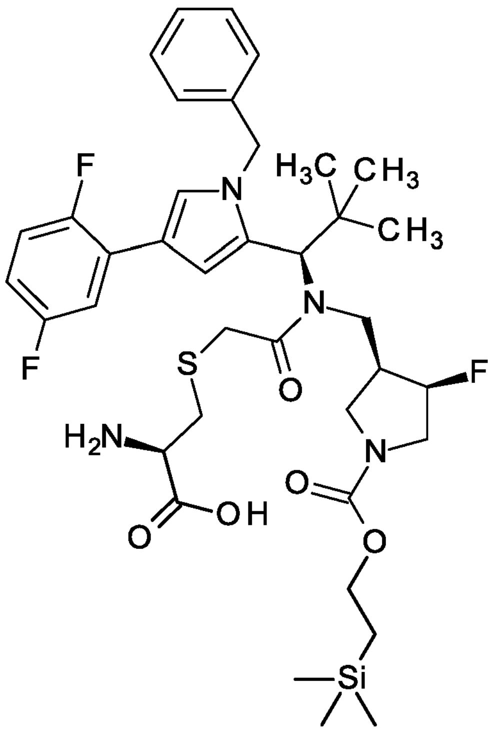

203.6 mg (1.68 mmol) L-Cystein wurden in 0.95 mL Wasser zusammen mit 201.7 mg (2.40 mmol) Natriumhydrogencarbonat suspendiert. Dazu wurden 170.0 mg (0.24 mmol 2-(Trimethylsilyl)-ethyl-(3R,4R)-3-{[{(1R)-1-[1-benzyl-4-(2,5-difluorphenyl)-1H-pyrrol-2-yl]-2,2-dimethyl-propyl}(chloracetyl)amino]methyl}-4-fluorpyrrolidin-1-carboxylat (Intermediat 105) gelöst in 9.5 mL *iso*-Propanol und 438.5 mg (2.40 mmol) und 1,8-Diazabicyclo(5.4.0)undec-7-en gegeben. Die Reaktionsmischung wurde 3 h bei 50 °C gerührt. Das Gemisch wurde mit Ethylacetat versetzt und die org. Phase wurde mehrmals mit ges. Natriumhydrogencarbonat-Lösung und mit ges. NaCl-Lösung gewaschen. Die organische Phase wurde über Natriumsulfat getrocknet und das Lösemittel im Vakuum verdampft. Der Rückstand wurde ohne weitere Reinigung weiter eingesetzt. Man erhielt 152 mg (83 % d. Th.) der Titelverbindung.

LC-MS (Methode 1): $R_t$ = 1.26 min; MS (ESIpos): m/z = 762 $(M+H)^+$.

## Intermediat C108

2-(Trimethylsilyl)ethyl $N^6$-(N-{(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}-beta-alanyl)-$N^2$-{[2-(trimethylsilyl)ethoxy]carbonyl}-L-lysinat

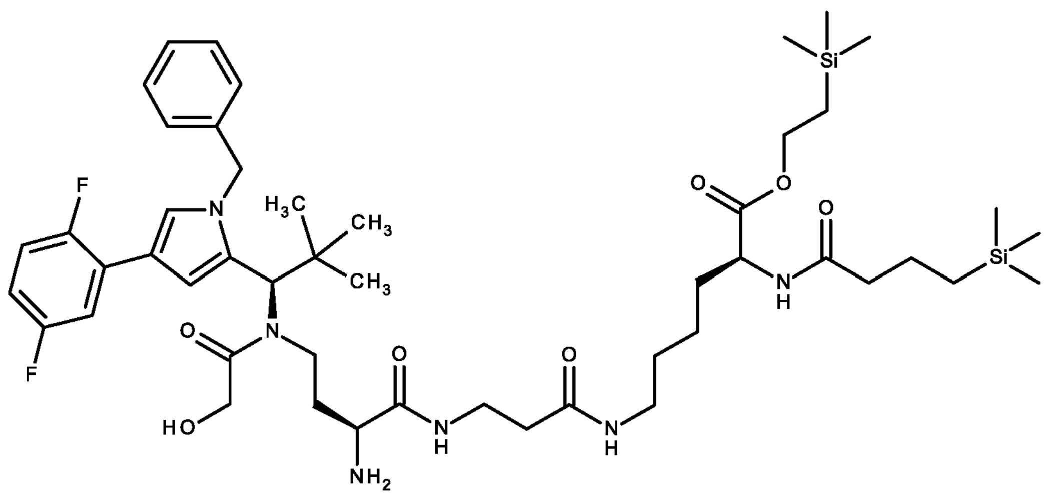

Die Titelverbindung wurde durch Kupplung von 103 mg (0.16 mmol) Intermediat C102 mit 110 mg (0.175 mmol) 2-(Trimethylsilyl)ethyl $N^6$-beta-alanyl-$N^2$-{[2-(trimethylsilyl)ethoxy]carbonyl}-L-lysinat in DMF in Gegenwart von EDCI, HOBT und N,N-Diisopropylethylamin hergestellt. Anschließend wurde die Z-Schutzgruppe durch 1-stündige Hydrierung über 10%-igem Palladium auf Aktivkohle in Dichlormethan/Methanol 1:1 bei RT unter Wasserstoff-Normaldruck entfernt, wobei die Titelverbindung in einer Ausbeute von 113 mg (75% d. Th. über 2 Stufen) erhalten wurde.

LC-MS (Methode 1): $R_t$ = 1.17 min; MS (ESIpos): m/z = 957 $(M+H)^+$.

Die hier eingesetzte Zwischenstufe wurde nach klassischen Methoden der Peptidchemie durch Kupplung von kommerziell erhältlichem N-(tert-Butoxycarbonyl)-beta-alanin und 2-(Trimethylsilyl)ethyl $N^2$-[(benzyloxy)carbonyl]-L-lysinat in Gegenwart von HATU, hydrogenolytischer Abspaltung der Z-Schutzgruppe, Einführung der Trimethylsilylethyloxy carbonyl(Teoc)-Schutzgruppe mit 1-({[2-(trimethylsilyl)ethoxy]carbonyl}oxy)pyrrolidine-2,5-dion und schließlich schonender Abspaltung der Boc-Schutzgruppe durch 45-minütiges Rühren in einer 7.5%-igen Trifluoressigsäurelösung in Dichlormethan hergestellt.

LC-MS (Methode 1): $R_t$ = 0.83 min; MS (ESIpos): m/z = 462 $(M+H)^+$.

## Intermediat C109

Di-tert-butyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-L-glutamat

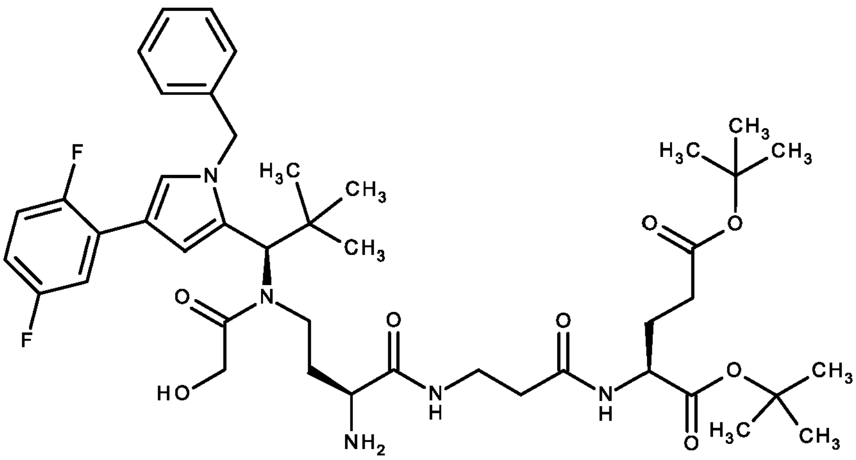

Zunächst wurde das Dipeptidderivat Di-tert-butyl-beta-alanyl-L-glutamat nach klassischen Methoden der Peptidchemie durch Kupplung von kommerziell erhältlichen N-[(Benzyloxy)-carbonyl]-beta-alanin und Di-tert-butyl L-glutamat Hydrochlorid (1:1) in Gegenwart von HATU und anschließender hydrogenolytischer Abspaltung der Z-Schutzgruppe hergestellt. Die Titelverbindung wurde dann durch Kupplung dieses Intermediats mit Intermediat C102 in Gegenwart von HATU und N,N-Diisopropylethylamin und anschließender Abspaltung der Z-Schutzgruppe durch 45-minütige Hydrierung über 10%-igem Palladium auf Aktivkohle in Methanol bei RT unter Wasserstoff-Normaldruck hergestellt.

LC-MS (Methode 1): $R_t$ = 0.99 min; MS (ESIpos): m/z = 826 $[M+H]^+$.

## Intermediat C110

Dibenzyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-L-glutamat

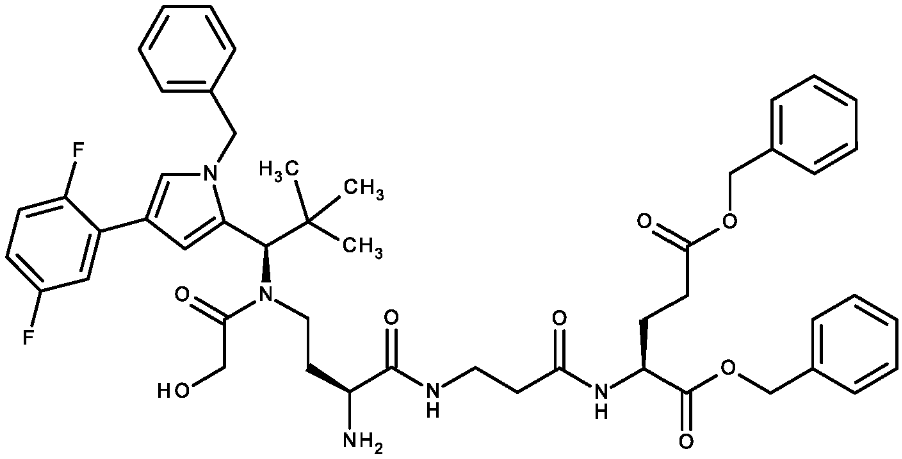

Die Titelverbindung wurde durch Kupplung von Dibenzyl-L-glutamat, das zuvor durch Verteilung zwischen Ethylacetat und 5%-iger Natriumhydrogencarbonatlösung aus seinem p-Toluolsulfon säure-Salz freigesetzt wurde mit Intermediat C61 in Gegenwart von HATU und N,N-Diisopropylethylamin und anschließender Abspaltung der Teoc-Schutzgruppe mittels Zinkchlorid in Trifluorethanol hergestellt.

LC-MS (Methode 1): $R_t$ = 1.09 min; MS (ESIpos): m/z = 894 $[M+H]^+$.

## Intermediat C110(D)

Dibenzyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-D-glutamat

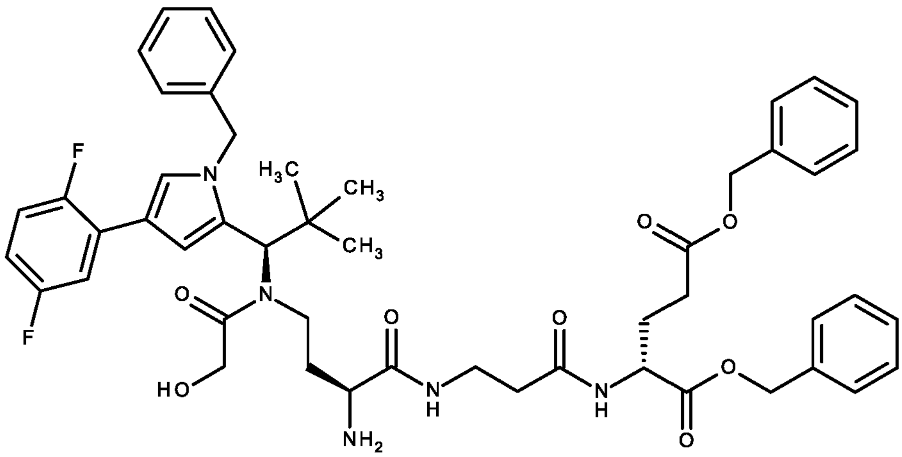

Die Titelverbindung wurde durch Kupplung von Dibenzyl-D-glutamat, das zuvor durch Verteilung zwischen Ethylacetat und 5%-iger Natriumhydrogencarbonatlösung aus seinem p-Toluolsulfon säure-Salz freigesetzt wurde mit Intermediat C61 in Gegenwart von HATU und N,N-Diisopropylethylamin und anschließender Abspaltung der Teoc-Schutzgruppe mittels Zinkchlorid in Trifluorethanol hergestellt.

LC-MS (Methode 1): $R_t$ = 1.08 min; MS (ESIpos): m/z = 894 $[M+H]^+$.

## Intermediat C111

Di-tert-butyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-D-glutamat

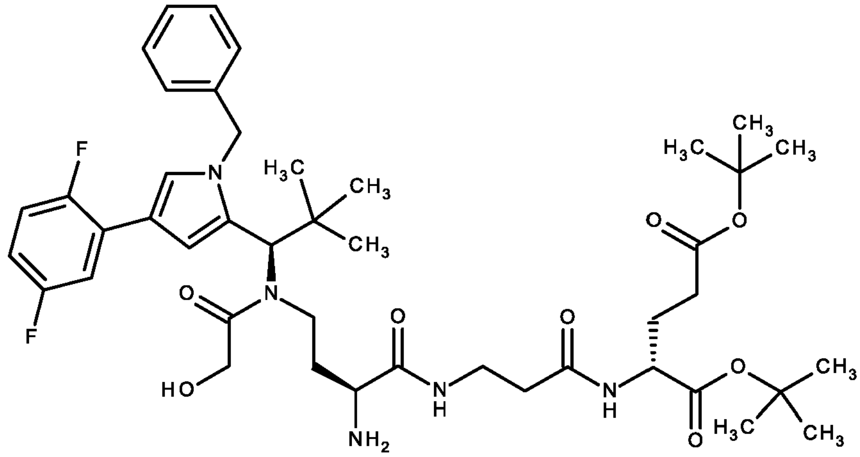

Die Synthese der Titelverbindung erfolgte in Analogie zu Intermediat C109.

LC-MS (Methode 1): $R_t$ = 1.06 min; MS (ESIpos): m/z = 826 $[M+H]^+$.

## Intermediat C112

N-Acetyl-L-alanyl-D-alanyl-N1-{(2S)-1-[(2-aminoethyl)amino]-4-[{(1R)-1-[1-benzyl-4-(2,5-difluorphenyl)-1H-pyrrol-2-yl]-2,2-dimethylpropyl}(glycoloyl)amino]-1-oxobutan-2-yl}-L-aspartamid

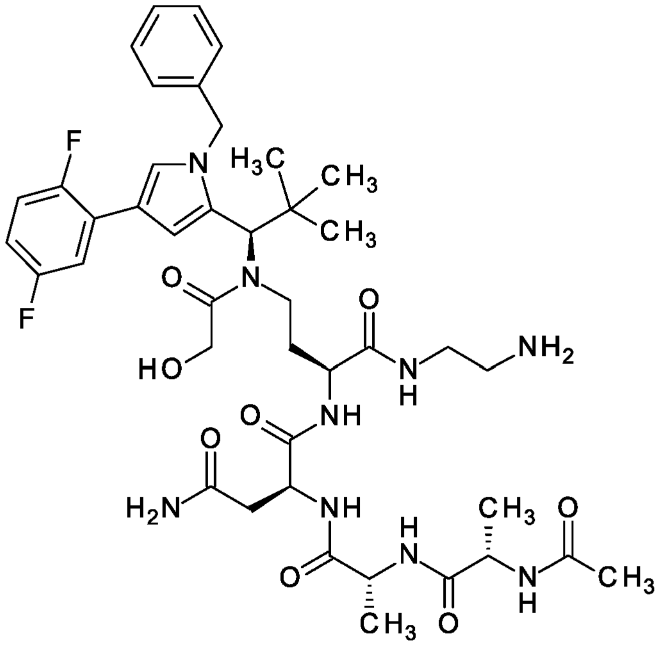

Zunächst wurde (2S)-4-[{(1R)-1-[1-Benzyl-4-(2,5-difluorphenyl)-1H-pyrrol-2-yl]-2,2-dimethyl propyl}(glycoloyl)amino]-2-[(tert-butoxycarbonyl)amino]butansäure mit Benzyl-(2-aminoethyl) carbamathydrochlorid (1:1) in Gegenwart von HATU und N,N-Diisopropylethylamin gekuppelt. Alternativ kann als Edukt auch Intermediat C58 eingesetzt werden. Anschließend erfolgte die Abspaltung der Boc-Schutzgruppe durch 5-stündiges Rühren bei 50°C in Trifluorethanol mit 4 Equivalenten Zinkchlorid. Das erhaltene Intermediat wurde dann mit Intermediat L111 in Gegenwart von HATU und N,N-Diisopropylethylamin gekuppelt. Im letzten Schritt wurde durch 1-stündige Hydrierung über 10%-igem Palladium auf Aktivkohle in DCM-Methanol 1:1 bei RT unter Wasserstoff-Normaldruck die Titelverbindung erhalten.

LC-MS (Methode 1): $R_t$ = 0.8 min; MS (ESIpos): m/z = 854 $[M+H]^+$.

**Intermediat C113**

Trifluoressigsäure-benzyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-3-{[(benzyloxy)carbonyl]amino}-D-alaninat (1:1)

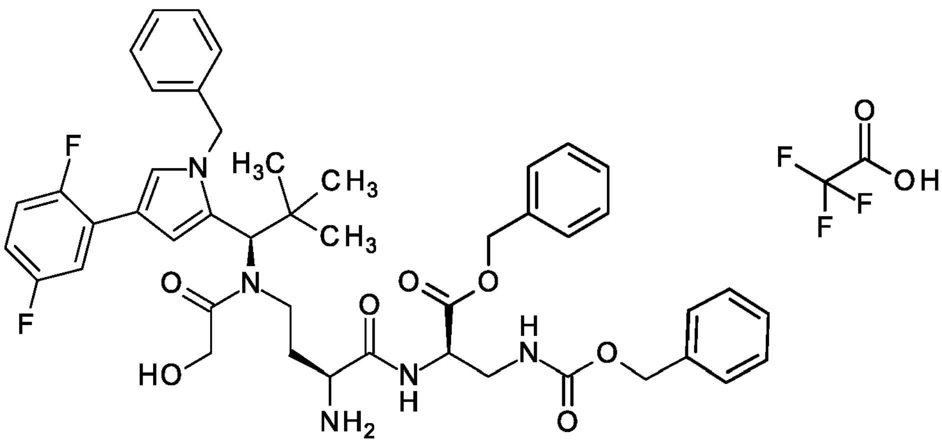

Zunächst wurde Trifluoressigsäure -benzyl-3-{[(benzyloxy)carbonyl]amino}-D-alaninat (1:1) ausgehend von kommerziell erhältlichem 3-{[(Benzyloxy)carbonyl]amino}-N-(tert-butoxycarbonyl)-D-alanin durch Veresterung mit Benzylalkohol in Gegenwart von EDC/DMAP und anschließender Abspaltung der Boc-Schutzgruppe mit Trifluoressigsäure hergestellt. Dieser Aminosäurebaustein wurde dann mit Intermediat C58 in Gegenwart von HATU und N,N-Diiso-propylethylamin in DMF gekuppelt. Im letzten Schritt wurde durch 2-stündiges Rühren bei 50°C in Trifluorethanol mit 6 Equivalenten Zinkchlorid und Reinigung durch präparative HPLC die Titelverbindung erhalten.

LC-MS (Methode 1): $R_t$ = 1.05 min; MS (ESIpos): m/z = 824 $[M+H]^+$.

**Intermediat C114**

Trifluoressigsäure--tert-butyl-4-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)butanoat (1:1)

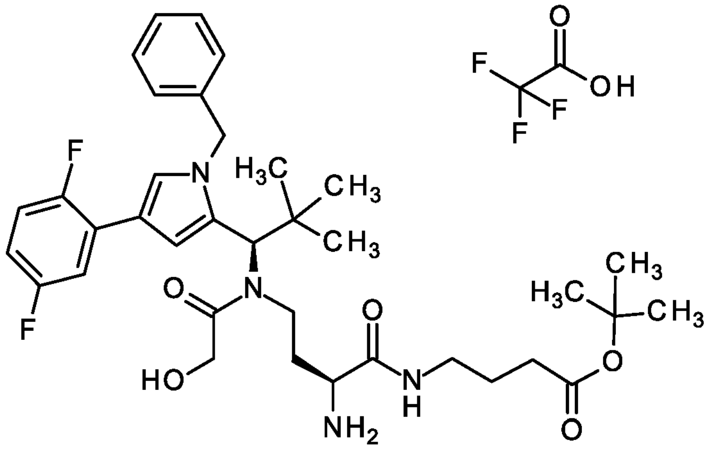

Zunächst wurde Intermediat C102 mit tert-Butyl-4-aminobutanoathydrochlorid (1:1) in Gegenwart von HATU und N,N-Diisopropylethylamin gekuppelt. Anschließend wurde durch 1-stündige Hydrierung über 10%-igem Palladium auf Aktivkohle in DCM-Methanol 1:1 bei RT unter Wasserstoff-Normaldruck die Titelverbindung erhalten.

LC-MS (Methode 1): $R_t$ = 1.0 min; MS (ESIpos): m/z = 655 $[M+H]^+$.

## Intermediat C116

Trifluoressigsäure N1-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid (1:1)

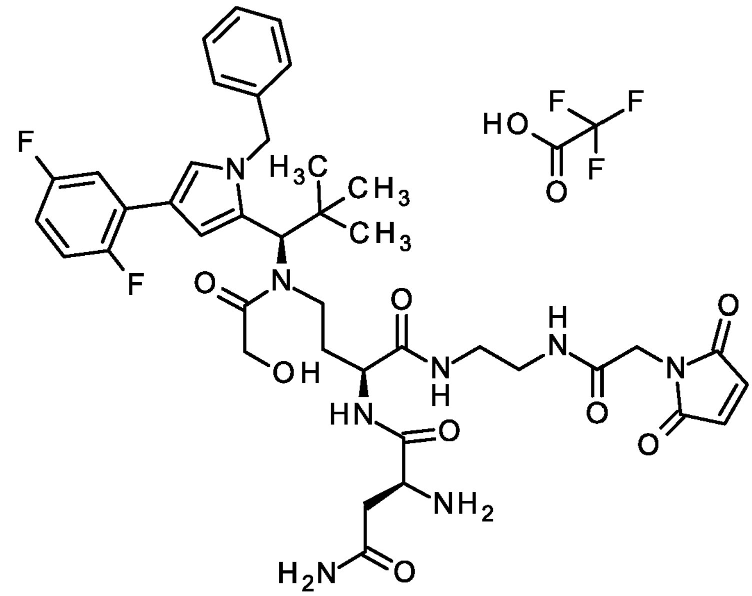

Trifluoressigsäure (2S)-2-amino-4-[{(1R)-1-[1-benzyl-4-(2,5-difluorphenyl)-1 H-pyrrol-2-yl]-2,2-dimethylpropyl}(glycoloyl)amino]-N-(2-{[(2,5-dioxo-2,5-dihydro-1H-pyrrol-1-yl)acetyl]amino}ethyl)butanamid (1:1) (81.0 mg, 100 µmol) (Intermediat F104) und 2,5-Dioxopyrrolidin-1-yl-N2-(tert-butoxycarbonyl)-L-asparaginat (43.0 mg, 131 µmol) wurden in 5.0 ml DMF gelöst. Die Reaktionsmischung wurde mit N,N-Diisopropylethylamin (61 µl, 350 µmol), 1h bei RT nachgerührt, und dann direkt mittels präp. RP-HPLC (Säule: Chromatorex 125x30; 10µ, Fluss: 75 mL/min, MeCN/Wasser, 0.1 % TFA) gereinigt. Die Lösemittel wurden im Vakuum verdampft und der Rückstand lyophilisiert. Man erhielt 84 mg (88 % d. Th.) der Verbindung tert-Butyl-[(2S)-4-amino-1-({(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}amino)-1,4-dioxobutan-2-yl]carbamat.

LC-MS (Methode 1): $R_t$ = 1.09 min; MS (ESIpos): m/z = 907 $[M+H]^+$

Tert-Butyl-[(2S)-4-amino-1-({(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}amino)-1,4-dioxobutan-2-yl]carbamat (83.0 mg, 91.5 µmol) wurde in 5.0 ml Trifluorethanol gelöst. Die Reaktionsmischung wurde mit Zinkchlorid (74.8 mg, 549 µmol) versetzt und 15 min bei 50°C nachgerührt. Der Ansatz wurde mit Ethylendiamin-N,N,N',N'-tetraessigsaeure (160 mg, 549 µmol) versetzt, mit 5.0 ml Acetonitril/Wasser verdünnt, mit TFA (20 µl) versetzt und 10 min nachgerührt. Der Ansatz wurde durch einen Spritzenfilter filtriert und mittels präp. RP-HPLC (Säule: Chromatorex 125x30; 10µ, Fluss: 75 mL/min, MeCN/Wasser, 0.1% TFA) gereinigt. Die Lösemittel wurden im Vakuum verdampft und der Rückstand im Hochvakuum getrocknet. Man erhielt 50 mg (58 % d. Th.) der Titelverbindung.

LC-MS (Methode 1): $R_t$ = 0.81 min; MS (ESIpos): m/z = 807 $[M+H]^+$

## Intermediat C117

Trifluoressigsäure --dibenzyl-N-[3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-beta-alanyl-L-glutamat (1:1)

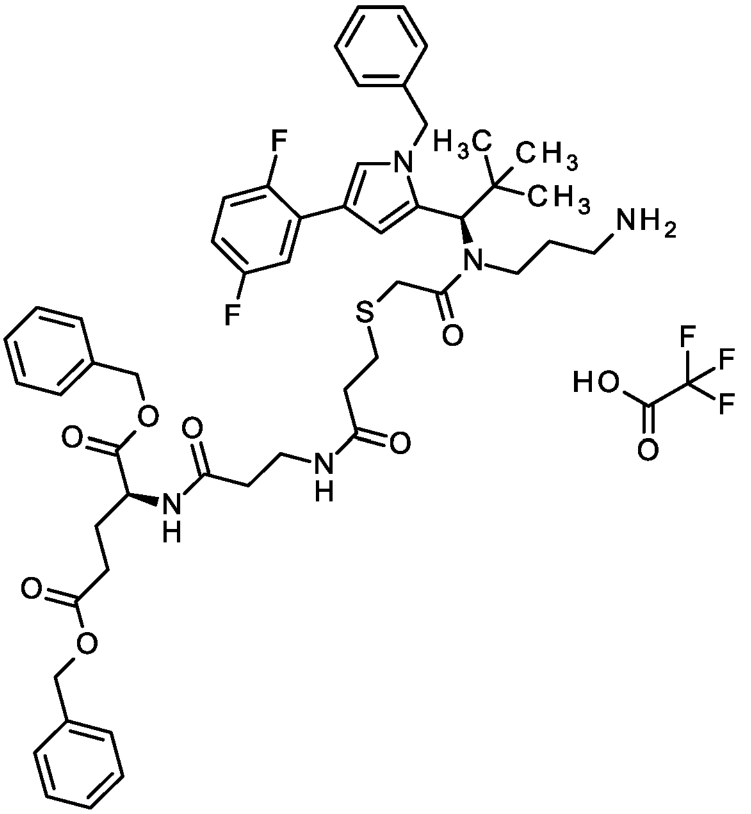

Die Titelverbindung wurde durch Kupplung von Trifluoressigsäure --dibenzyl-beta-alanyl-L-glutamat (1:1) (Intermediat L127) mit 11-{(1R)-1-[1-Benzyl-4-(2,5-difluorphenyl)-1H-pyrrol-2-yl]-2,2-dimethylpropyl}-2,2-dimethyl-6, 12-dioxo-5-oxa-14-thia-7, 11-diaza-2-silaheptadecan-17-säure in Gegenwart von HATU und N,N-Diisopropylethylamin und anschließender Abspaltung der Teoc-Schutzgruppe mittels Zinkchlorid in Trifluorethanol hergestellt.

LC-MS (Methode 1): $R_t$ = 1.07 min; MS (ESIpos): m/z = 938 $[M+H]^+$.

## Intermediat C118

9H-Fluoren-9-ylmethyl-{3-[{(1R)-1-[1-benzyl-4-(2,5-difluorphenyl)-1H-pyrrol-2-yl]-2,2-dimethylpropyl}(chloracetyl)amino]propyl}carbamat

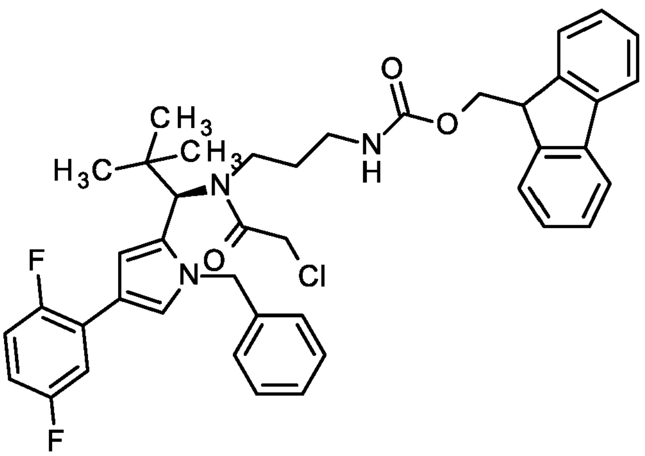

9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (2.50 g, 3.94 mmol) und Triethylamin (1.6 ml, 12 mmol) wurden in 200 mL Dichlormethan vorgelegt

und auf 0 °C abgekühlt. Bei dieser Temperatur wurde Chloracetylchlorid (2.23 g, 19.7 mmol) zugegeben. Die Reaktionsmischung wurde 5h bei RT gerührt und mit Ethylacetat verdünnt. Die organische Phase wurde dreimal mit gesättigter Natriumhydrogencarbonat-Lösung und gesättigter Ammoniumchlorid-Lösung gewaschen. Die organische Phase wurde mit gesättigter Natriumchloridlösung gewaschen und über Magnesiumsulfat getrocknet. Der Rückstand wurde ohne weitere Reinigung in der nächsten Synthesestufe eingesetzt. Man erhielt 1.7 g (63 % d. Th.) der Titelverbindung.

LC-MS (Methode 1): $R_t$ = 1.52 min; MS (ESIpos): m/z = 710 $(M+H^+)^+$.

## Intermediat C119

Trifluoressigsäure --2-(trimethylsilyl)ethyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-N-(tert-butoxycarbonyl)-L-cysteinat (1:1)

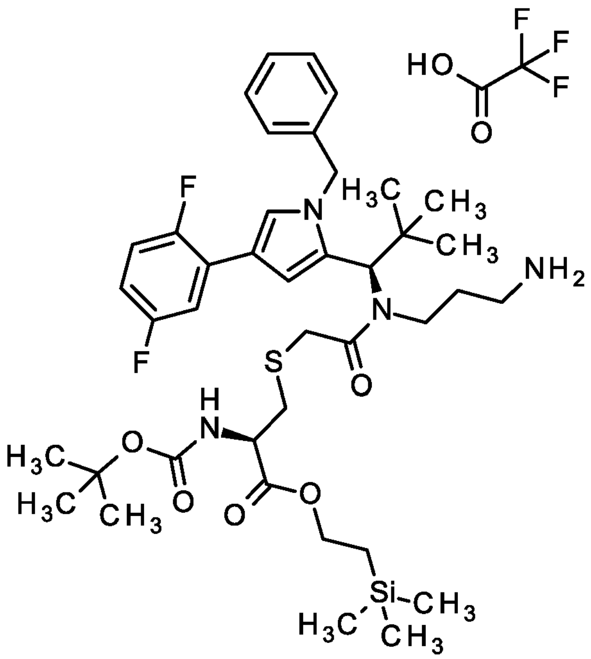

9H-Fluoren-9-ylmethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C118) (208 mg, 294 µmol) und 2-(Trimethylsilyl)ethyl-N-(tert-butoxycarbonyl)-L-cysteinat (99.3 mg, 309 µmol)

(Intermediat L128) wurden in 5.0 ml DMF vorgelegt, mit 1 Tropfen Triethylamin versetzt und übernacht bei RT nachgerührt. Die Reaktionsmischung wurde mit 1.0 ml Wasser (0,1% TFA) versetzt und mittels präp. RP-HPLC (Säule: Reprosil 250×30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 252 mg (86 % d. Th.) 2-(Trimethylsilyl)ethyl-S-{2-[{(1R)-1-[1-benzyl-4-(2,5-difluorphenyl)-1H-pyrrol-2-yl]-2,2-dimethylpropyl}(3-{[(9H-fluoren-9-ylmethoxy)carbonyl]amino}propyl)amino]-2-oxoethyl}-N-(tert-butoxycarbonyl)-L-cysteinat.

LC-MS (Methode 1): $R_t$ = 1.76 min; MS (ESIpos): m/z = 995 $[M+H]^+$

2-(Trimethylsilyl)ethyl-S-{2-[{(1R)-1-[1-benzyl-4-(2,5-difluorphenyl)-1H-pyrrol-2-yl]-2,2-dimethylpropyl}(3-{[(9H-fluoren-9-ylmethoxy)carbonyl]amino}propyl)amino]-2-oxoethyl}-N-(tert-butoxycarbonyl)-L-cysteinat (63.1 mg, 63.4 µmol) wurde in 2.0 ml DMF vorgelegt, mit 200 µl Morpholin versetzt und 2h30 bei RT nachgerührt. Die Reaktionsmischung wurde mit 1.0 ml Wasser (0,1% TFA) versetzt und mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 51 mg (91 % d. Th.) der titel Verbindung.

LC-MS (Methode 1): $R_t$ = 1.36 min; MS (ESIpos): m/z = 773 $[M+H]^+$

**Intermediat C120**

N-{[2-(2-Methoxyethoxy)ethoxy]acetyl}-L-alanyl-D-alanyl-$N^1$-{(2S)-1-[(2-aminoethyl)amino]-4-[{(1R)-1-[1-benzyl-4-(2,5-difluorphenyl)-1H-pyrrol-2-yl]-2,2-dimethylpropyl}(glycoloyl)amino]-1-oxobutan-2-yl}-L-aspartamid

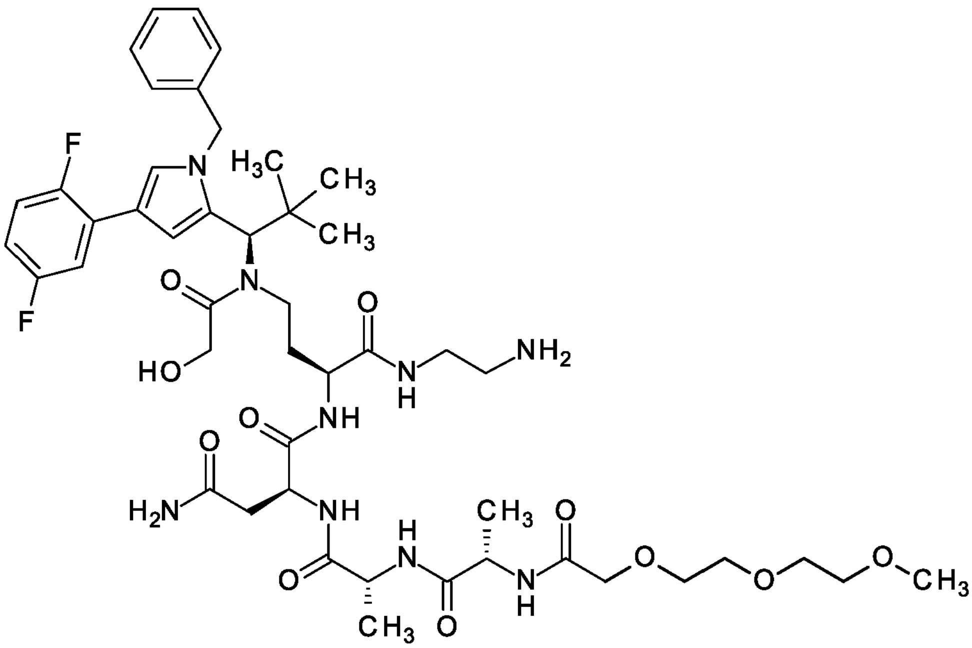

Dieses Intermediat wurde in Analogie zu Intermediat C112 hergestellt, wobei zur Kupplung Intermediat L129 verwendet wurde.

LC-MS (Methode 1): $R_t$ = 0.77 min; MS (ESIpos): m/z = 972 $[M+H]^+$

**Intermediat C121**

Dibenzyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-D-glutamat

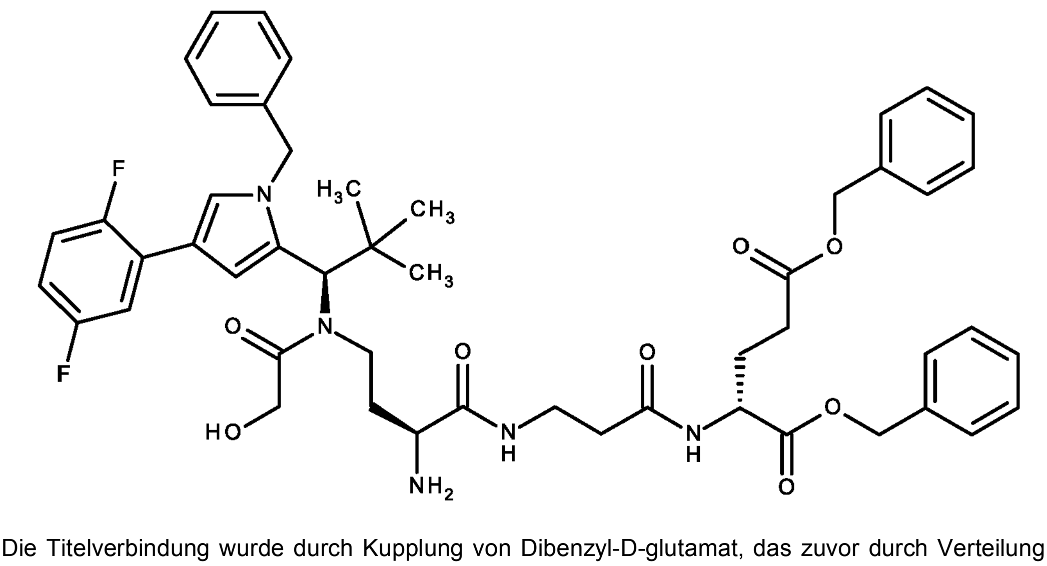

Die Titelverbindung wurde durch Kupplung von Dibenzyl-D-glutamat, das zuvor durch Verteilung zwischen

Ethylacetat und 5%-iger Natriumhydrogencarbonatlösung aus seinem p-Toluolsulfonsäure-Salz freigesetzt wurde mit Intermediat C61 in Gegenwart von HATU und N,N-Diisopropylethylamin und anschließender Abspaltung der Teoc-Schutzgruppe mittels Zinkchlorid in Trifluorethanol hergestellt.

LC-MS (Methode 1): $R_t$ = 1.05 min; MS (ESIpos): m/z = 894 $[M+H]^+$.

**Intermediat C122**

tert-Butyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-$N^2$-(tert-butoxycarbonyl)-D-alpha-glutaminat

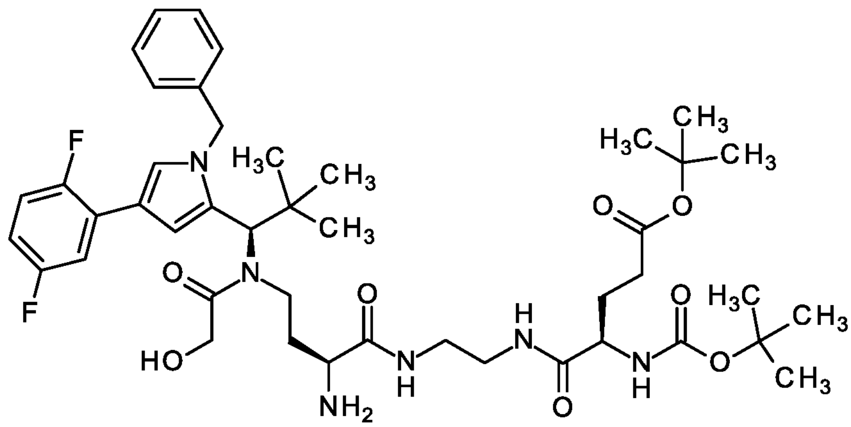

Die Titelverbindung wurde durch Kupplung von Dibenzyl-D-glutamat, das zuvor durch Verteilung zwischen Ethylacetat und 5%-iger Natriumhydrogencarbonatlösung aus seinem p-Toluolsulfonsäure-Salz freigesetzt wurde mit Intermediat C61 in Gegenwart von HATU und N,N-Diisopropylethylamin und anschließender Abspaltung der Teoc-Schutzgruppe mittels Zinkchlorid in Trifluorethanol hergestellt.

LC-MS (Methode 1): $R_t$ = 1.05 min; MS (ESIpos): m/z = 894 $[M+H]^+$.

**Intermediat C123**

tert-Butyl-N-[2-({(2S)-2-(L-asparaginylamino)-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-$N^2$-(tert-butoxycarbonyl)-D-alpha-glutaminat

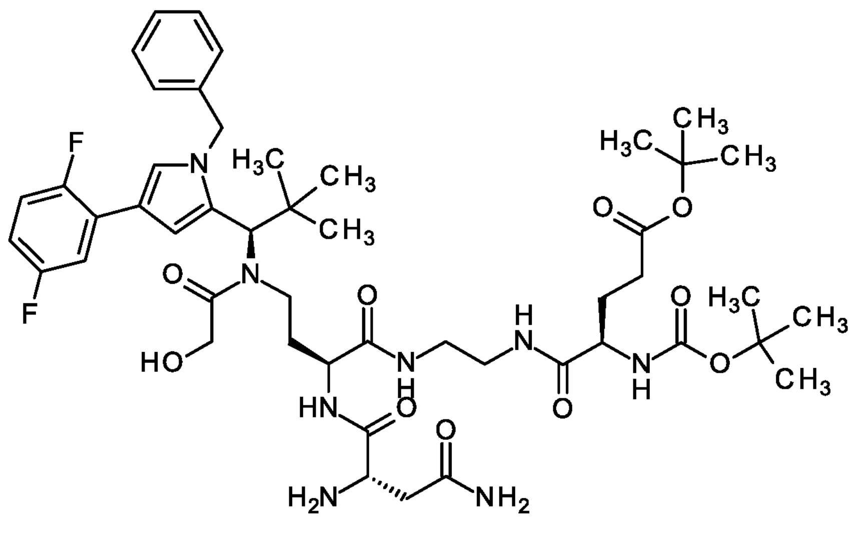

Die Titelverbindung wurde durch Kupplung von 4-Nitrophenyl-$N^2$-[(benzyloxy)carbonyl]-L-asparaginat mit Intermediat C122 in DMF in Gegenwart N,N-Diisopropylethylamin und anschließender Abspaltung der Z-Schutzgruppe durch Hydrierung über 10%-igem Palladium auf Aktivkohle in DCM/Methanol 1:1 unter Wasserstoff-Normaldruck bei RT hergestellt.

LC-MS (Methode 1): $R_t$ = 0.98 min; MS (ESIpos): m/z = 955 $[M+H]^+$.

## Intermediat C124

8-{(1R)-1-[1-Benzyl-4-(2,5-difluorphenyl)-1H-pyrrol-2-yl]-2,2-dimethylpropyl}-1-(9H-fluoren-9-yl)-3,9-dioxo-2-oxa-11-thia-4, 8-diazatetradecan-14-säure

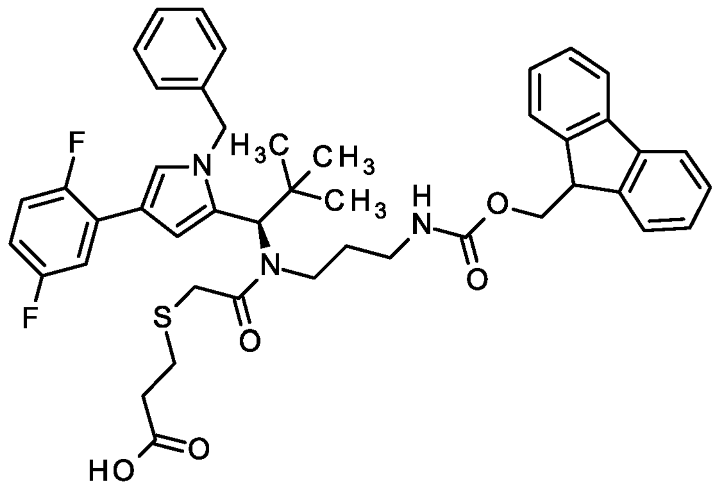

9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (3.50 g, 5.52 mmol) (Intermediat C67) und Triethylamin (2.3 ml, 17 mmol) wurden in Dichlormethan (700 ml, 11 mol) vorgelegt. Chloracetylchlorid (3.12 g, 27.6 mmol) wurde zugegeben und die Mischung wurde 5 h bei RT gerührt. Die Reaktionsmischung wurde mit Ethylacetat verdünnt und die organische Phase wurde mit zuerst mit einer 10%igen Zitronensäurelösung und anschließend mit ges. Natriumhydrogencarbonat-Lösung und ges. Natriumchlorid-Lösung gewaschen. Die organische Phase wurde mit ges. NaCl-Lösung gewaschen und über Magnesiumsulfat getrocknet. Die Lösemittel wurden im Vakuum verdampft.

Das so erhaltende Intermediat 9H-Fluoren-9-ylmethyl-{3-[{(1R)-1-[1-benzyl-4-(2,5-difluorphenyl)-1H-pyrrol-2-yl]-2,2-dimethylpropyl}(chloracetyl)amino]propyl}carbamat (350 mg, 493 µmol) wurde zusammen mit 3-Sulfanylpropansäure (93 µl, 990 µmol) in DMF (7.0 ml) gelöst und mit Triethylamin versetzt. Die Reaktion wurde für 5 h bei Raumtemperatur gerührt anschließend wurde die Reaktion durch Zugabe von 3 mL Wasser + 0.1 % TFA gestoppt. Die Mischung wurde im Vakuum aufkonzentriert und der Rückstand wurde mittels präperativer HPLC gereinigt. Es wurden 307 mg (80%) der Titelverbindung erhalten.

LC-MS (Methode 1): $R_t$ = 1.40 min; MS (ESIpos): m/z = 780 $[M+H]^+$

## Intermediat C125

Di-tert-butyl-N-[3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-D-aspartat-Trifluoressigsäure Salz

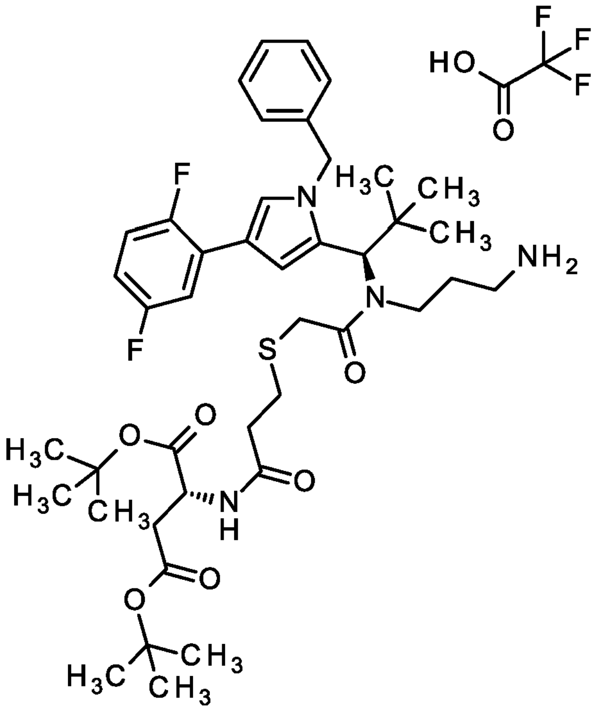

Zu einer Mischung aus 8-{(1R)-1-[1-Benzyl-4-(2,5-difluorphenyl)-1H-pyrrol-2-yl]-2,2-dimethylpropyl}-1-(9H-fluoren-9-yl)-3,9-dioxo-2-oxa-11-thia-4,8-diazatetradecan-14-säure (200 mg, 256 μmol, Intermediat C124) und Di-tert-butyl-D-aspartathydrochlorid Salz (86.7 mg, 308 μmol) in DMF (3.0 ml) wurde HATU (117 mg, 308 μmol) und N,N-Diisopropylethylamin (130 μl, 770 μmol) gegben und die Reaktion wurde für 10 min bei Raumtemperatur gerührt. Die Reaktionsmischung wurde mit Wasser+ 01% TFA gequencht und direkt mittels präp. RP-HPLC gereinigt. Die Lösemittel wurden im Vakuum verdampft und der Rückstand im Hochvakuum getrocknet.

LC-MS (Methode 1): $R_t$ = 1.61 min; MS (ESIpos): m/z = 1007 $[M+H]^+$

Das erhaltene Intermediat wurde in DMF (3.0 ml) gelöst und mit Morpholin (200 μl, 2.3 mmol) versetzt. Die Mischung wurde 5 h bei Raumtemperatur gerührt und anschließend mit Wasser+ 01% TFA gequencht und direkt mittels präp. RP-HPLC gereinigt. Die Lösemittel wurden im Vakuum verdampft und der Rückstand im Hochvakuum getrocknet. Es wurden 182 m der Titelverbinsung erhalten.

LC-MS (Methode 5): $R_t$ = 3.84 min; MS (ESIpos): m/z = 785 $[M+H]^+$

<u>**Intermediat C126**</u>

Di-tert-butyl-(2R)-2-{[(2S,5R,8S)-2-amino-8-(2-amino-2-oxoethyl)-14-{(1R)-1-[1-benzyl-4-(2,5-difluorphenyl)-1H-pyrrol-2-yl]-2,2-dimethylpropyl}-5-methyl-3,6,9,15,20-pentaoxo-17-thia-4,7, 10, 14-tetraazaicosan-20-yl]amino}succinat

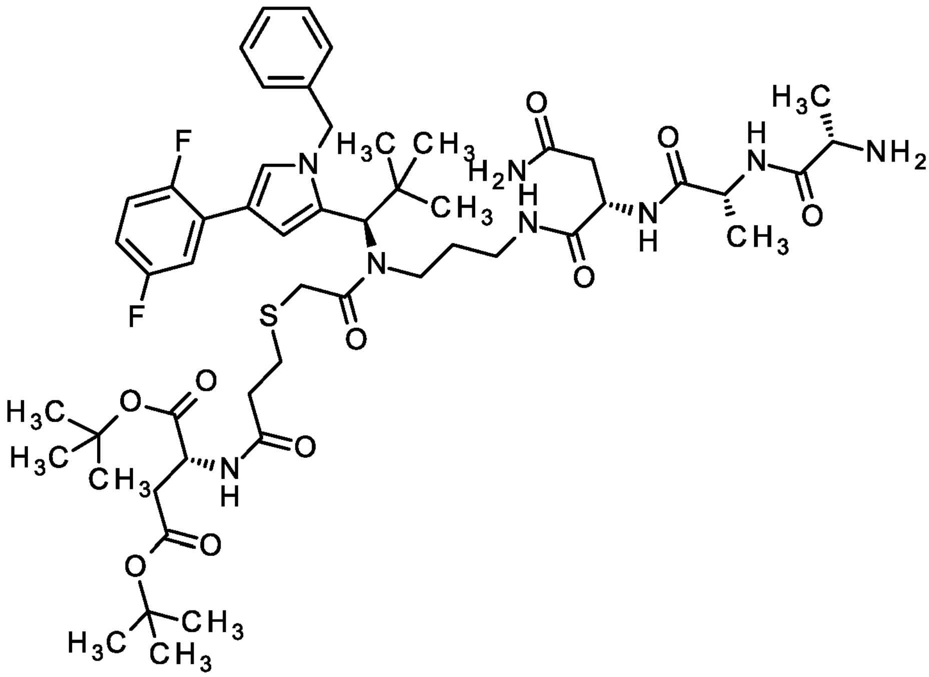

Die Titelverbindung wurde durch Kupplung von N-[(Benzyloxy)carbonyl]-L-alanyl-D-alanyl-L-asparagin (Intermediat L108) mit Intermediat C125 in DMF in Gegenwart N,N-Diisopropylethylamin und HATU und anschließender Abspaltung der Z-Schutzgruppe durch Hydrierung über 10%-igem Palladium auf Aktivkohle Essigsäureethylester/Ethanol 1:1 unter Wasserstoff-Normaldruck bei RT hergestellt.

LC-MS (Methode 1): $R_t$ = 1.02 min; MS (ESIpos): m/z = 1041 $[M+H]^+$

## Intermediat C127

Trifluoressigsäure -di-tert-butyl-(2R)-2-{[(4S,7R,10S)-10-(2-amino-2-oxoethyl)-16-{(1R)-1-[1-benzyl-4-(2,5-difluorphenyl)-1H-pyrrol-2-yl]-2 ,2-dimethylpropyl}-1-(2, 5-dioxo-2, 5-dihydro-1H-pyrrol-1-yl)-4,7-dimethyl-2,5,8,11,17,22-hexaoxo-19-thia-3,6,9,12,16-pentaazadocosan-22-yl]amino}succinat Salz

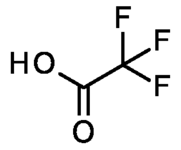

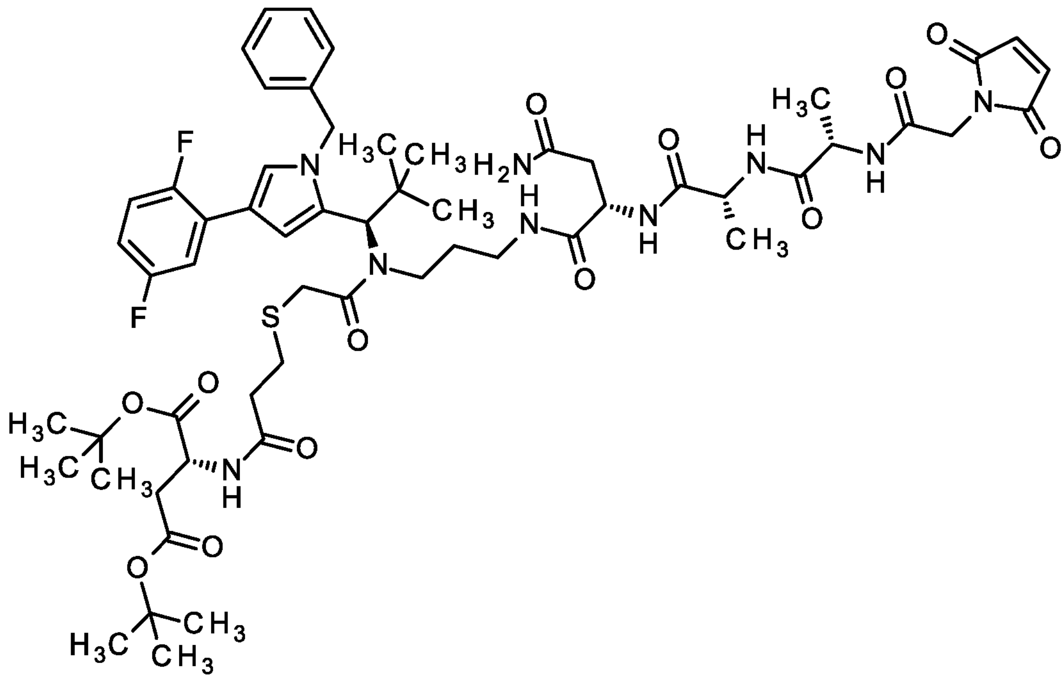

Di-tert-butyl-(2R)-2-{[(2S,5R,8S)-2-amino-8-(2-amino-2-oxoethyl)-14-{(1R)-1-[1-benzyl-4-(2,5-difluorphenyl)-1H-pyrrol-2-yl]-2,2-dimethylpropyl}-5-methyl-3,6,9,15,20-pentaoxo-17-thia-4,7,10,14-tetraazaicosan-20-yl]amino}succinat (12.0 mg, 11.5 μmol, Intermediat C126) und 1-{2-[(2,5-Dioxopyrrolidin-1-yl)oxy]-2-oxoethyl}-1H-pyrrol-2,5-dion (3.20 mg, 12.7 μmol) wurden in DMF (1.0 ml) gelöst und mit N,N-Diisopropylethylamin (4.0 μl, 23 μmol) versetzt. Die Mischung wurde 1 h bei Raumtemperatur gerührt. Anschließend wurde mit Wasser+ 01% TFA gequencht und die Mischung wurde direkt mittels präp. RP-HPLC gereinigt. Die Lösemittel wurden im Vakuum verdampft und der Rückstand im Hochvakuum getrocknet.

LC-MS (Methode 1): $R_t$ = 1.25 min; MS (ESIpos): m/z = 1178 $[M+H]^+$

## Intermediat C128

$N^2$-Acetyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-$N^6$-(tert-butoxycarbonyl)-L-lysinamid

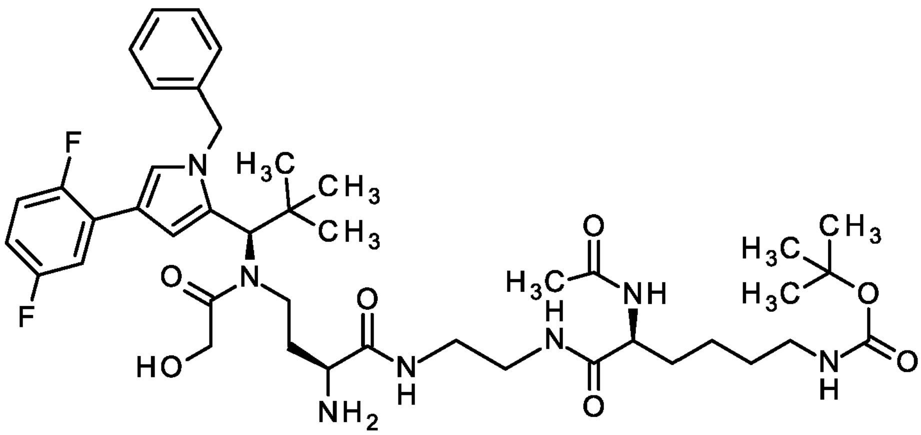

Zu einer Lösung aus (2S)-4-[{(1R)-1-[1-Benzyl-4-(2,5-difluorphenyl)-1H-pyrrol-2-yl]-2,2-dimethylpropyl}(glycoloyl)amino]-2-{[(benzyloxy)carbonyl]amino}butansäure (17.0 mg, 26.2 μmol, Intermediat C102) in DMF (2.8 ml) wurde N2-Acetyl-N-(2-aminoethyl)-N6-(tert-butoxycarbonyl)-L-lysinamid (9.54 mg, 28.9 μmol, Intermediat L135), HATU (18.0 mg, 47.2 μmol) und N,N-Diisopropylethylamin (9.1 μl, 52 μmol) gegeben. Die Reaktionsmischung wurde 1 h bei Raumtemperatur gerührt. Anschließend wurde das Lösungsmittel im Hochvakuum entfernt und der Rückstand wurde mittels präp. HPLC gereinigt.

LC-MS (Methode 1): $R_t$ = 1.27 min; MS (ESIpos): m/z = 960 $[M+H]^+$

Das wurde in DCM/EtOH gelöst und die Z-Schutzgruppe wurde durch Hydrierung über 10%-igem Palladium auf Aktivkohle unter Wasserstoff-Normaldruck bei RT abgespalten.

LC-MS (Methode 1): $R_t$ = 0.97 min; MS (ESIpos): m/z = 826 $[M+H]^+$

## Intermediat C129

L-Alanyl-D-alanyl-N1-[3-({(1R)-1-[1-benzyl-4-(2,5-difluorphenyl)-1H-pyrrol-2-yl]-2,2-dimethylpropyl}{[(3-{[(1R)-1,2-dicarboxyethyl]amino}-3-oxopropyl)sulfanyl]acetyl}amino)propyl]-L-aspartamid -trifluoressigsäure Salz

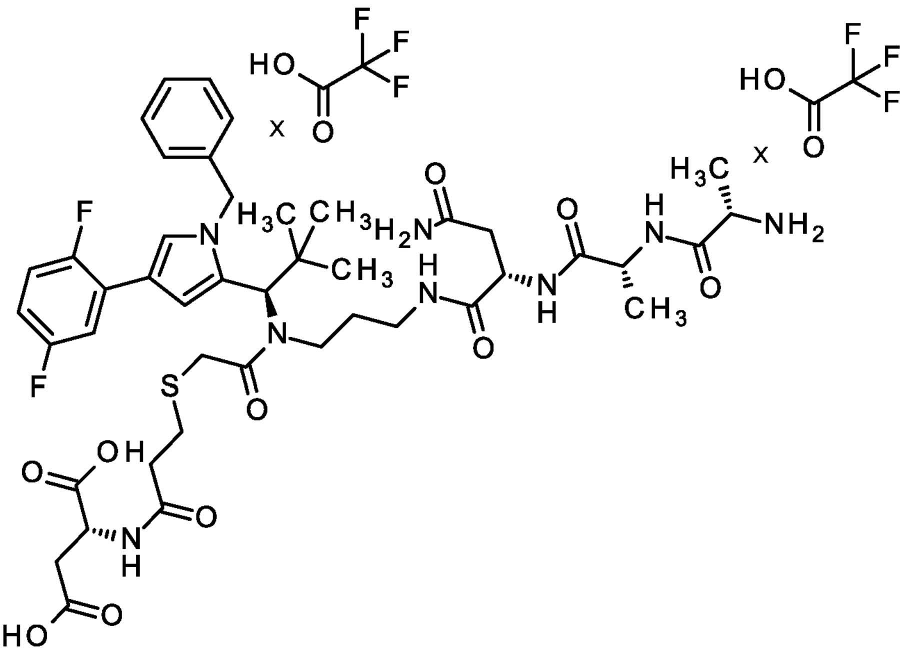

Intermediat C126 wurden in Trifluorethanol (2.0 ml) gelöst und mit Zinkdichlorid (9.19 mg, 67.4 μmol) versetzt. Nach einer Stunde rühren bei 50 °C wurde Zinkchlrid (9.19 mg, 67.4 μmol) zugegeben und die Reaktionsmischung wurde eine weitere Stunde bei 50 °C gerührt. Die Reaktionsmischung wurde mit Ethylendiamin-N,N,N',N'-tetraessigsäure

(19.7 mg, 67.4 μmol) versetzt, kurz gerührt und dann mit Wasser (0.1% TFA) versetzt. Die Reinigung erfolgte direkt mittles präp. RP-HPLC. Die Lösemittel wurden im Vakuum verdampft und der Rückstand wurde lyophilisiert. Man erhielt 7.6 mg (52 % d. Th.) der Titelverbindung.

LC-MS (Methode 3): $R_t$ = 0.84 min; MS (ESIneg): m/z = 927 $[M-H]^-$

## Intermediat L74

3-[2-[2-[2-[2-[[2-(2,5-dioxopyrrol-1-yl)acetyl]amino]ethoxy]ethoxy]ethoxy]ethoxy]propansäure

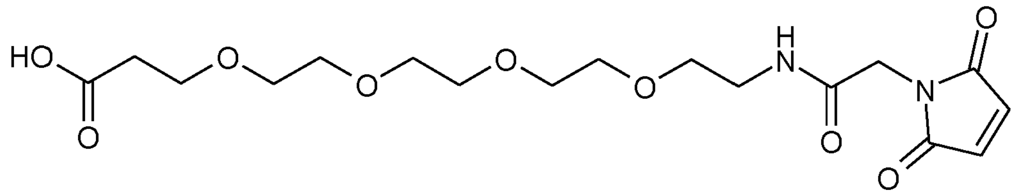

107 mg (0.335 mmol) *tert*-butyl 3-[2-[2-[2-(2-aminoethoxy)ethoxy]ethoxy]ethoxy]propanoat und 93 mg (0.369 mmol) (2,5-dioxopyrrolidin-1-yl) 2-(2,5-dioxopyrrol-1-yl)acetat wurden in 5 ml Dimethylformamid gelöst und mit 0.074 mL (0.671 mmol) N-Methylmorpholin versetzt. Die Reaktionsmischung wurde über Nacht bei RT gerührt. Es wurde 0.048 mL (0.838 mmol) Essigsäure zugegeben und die Reaktionsmischung wurde direkt mittles präparativer RP-HPLC gereinigt (Säule: Reprosil 125×30; 10μ, Fluss: 50 mL/min, MeCN/Wasser/0.1%TFA). Die Lösemittel wurden im Vakuum verdampft und der Rückstand im Hochvakuum getrocknet. Man erhielt 133 mg (86%, Reinheit 100%) *tert*-butyl 3-[2-[2-[2-[2-[[2-(2,5-dioxopyrrol-1-yl)acetyl]amino]ethoxy]ethoxy]ethoxy]ethoxy]propanoat.

LC-MS (Methode 1): $R_t$ = 0.82 min; MS (ESIpos): m/z = 459 $(M+H)^+$.

Zu einer Lösung von *tert*-butyl 3-[2-[2-[2-[2-[[2-(2,5-dioxopyrrol-1-yl)acetyl]amino]ethoxy]ethoxy]ethoxy]ethoxy]propanoat (130 mg, 0.284 mmol) in 5 ml dichlormethan wurde 0.5 ml TFA zugegeben. Das Reaktionsgemisch wird über Nacht bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 102 mg (90%, Reinheit 100%) der Titelverbindung.

LC-MS (Methode 1): $R_t$ = 0.52 min; MS (ESIpos): m/z = 402 $(M+H)^+$.

## Intermediat L75

Trifluoressigsäure--2-(trimethylsilyl)ethyl-3-{[(benzyloxy)carbonyl]amino}-D-alaninat (1:1)

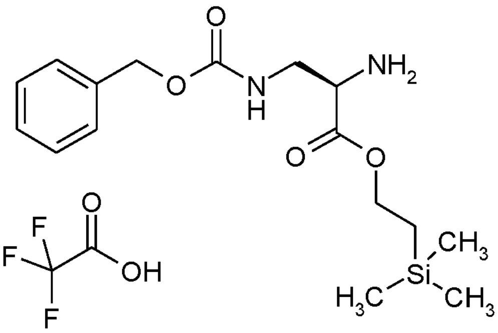

Die Titelverbindung wurde ausgehend von 3-{[(Benzyloxy)carbonyl]amino}-N-(tert-butoxycarbonyl)-D-alanin nach klassischen Methoden der Peptidchemie (Veresterung mit 2-(Trimethyl-silylethanol mittels EDCI/DMAP und Abspaltung der Boc-Schutzgruppe mit Trifluoressigsäure hergestellt. Man erhielt 405 mg (58 % d. Th. über 2 Stufen) der Titelverbindung.

LC-MS (Methode 1): $R_t$ = 0.75 min; MS (ESIpos): m/z = 339 $(M+H)^+$.

## Intermediat L76

(2S)-2-Brom-4-oxo-4-[2-(trimethylsilyl)ethoxy]butansäure

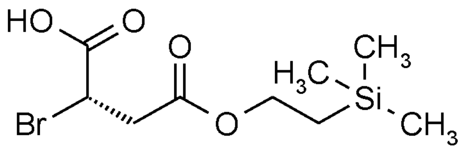

Zunächst wurde ein geeignet geschütztes Asparaginsäurederivat ausgehend von (3S)-4-(Benzyloxy)-3-{[(benzyloxy)carbonyl]amino}-4-oxobutansäure nach klassischen Methoden der Peptidchemie (Veresterung mit 2-(Trimethylsilylethanol mittels EDCI/DMAP und hydrogenolytische Abspaltung der Z-Schutzgruppe und des Benzylesters hergestellt. 470 mg (1.8 mmol) der so erhaltenen (2S)-2-Amino-4-oxo-4-[2-(trimethylsilyl)ethoxy]-butansäure wurden in 10 ml Wasser suspendiert und mit 1.8 mL einer 1 molaren Salzsäure sowie 0.5 ml konzentrierter Schwefelsäure und anschließend mit 863 mg (7.25 mmol Kaliumbromid versetzt. Dann wurden bei 10°C über einen Zeitraum von 30 min eine Lösung aus 150 mg (2.175 mmol) Natriumnitrit in 1 ml Wasser zugetropft und der Ansatz 2 h bei 10-15°C gerührt. Anschließend wurde der Ansatz mit 50 mL Ethylacetat ausgeschüttelt. Die organische Phase wurde mit gesättigter Natriumchloridlösung gewaschen und über Magnesiumsulfat getrocknet. Nach Abdampfen des Lösungsmittels und Reinigung durch präparative HPLC wurden 260 mg (48% d.Th.) der Titelverbindung erhalten.

LC-MS (Methode 1): $R_t$ = 1.03 min; MS (ESIneg): m/z = 295 und 297 $(M-H)^-$.

$^1$H-NMR (400 MHz, $CDCl_3$): $\delta$ [ppm] = 0.03 (s, 9H), 0.95 (t, 2H), 2.94 und 3.2 (2dd, 2H), 4.18 (t, 2H), 4.57 (t, 1H).

## Intermediat L77

Trifluoressigsäure --N-[2-(2-aminoethoxy)ethyl]-2-bromacetamid (1:1)

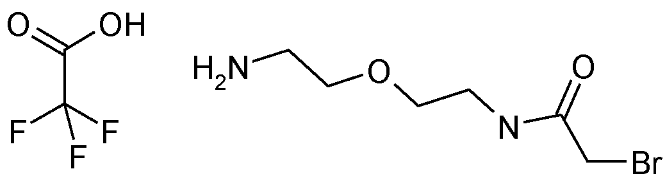

Zunächst wurden 418 mg (2.05 mmol) tert-Butyl-[2-(2-aminoethoxy)ethyl]carbamat mit 638 mg (2.46 mmol) Bromessigsäureanhydrid umgesetzt und anschließend die Boc-Schutzgruppe mit Trifluoressigsäure entfernt. Man erhielt 551 mg (63 % d. Th. über 2 Stufen) der Titelverbindung.

LC-MS (Methode): $R_t$ = 0.32 min; MS (ESIpos): m/z = 227 und 225 $(M+H)^+$.

## Intermediat L78

N-[(2,5-Dioxo-2,5-dihydro-1H-pyrrol-1-yl)acetyl]-beta-alanin

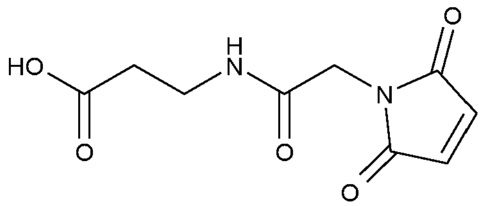

Die Titelverbindung wurde ausgehend von kommerziell erhältlichem (2,5-Dioxo-2,5-dihydro-1H-pyrrol-1-yl)essigsäure durch Kupplung mit *tert*-Butyl-beta-alaninathydrochlorid (1:1) in Gegenwart von EDCI/HOBt und N,N-Diisopropylethylamin und anschließende Entschützung mit Trifluoressigsäure hergestellt.

LC-MS (Methode 1): $R_t$ = 0.32 min; MS (ESIpos): m/z = 227 $(M+H)^+$.

## Intermediat L79

N-[6-(2,5-Dioxo-2,5-dihydro-1H-pyrrol-1-yl)hexanoyl]-beta-alanin

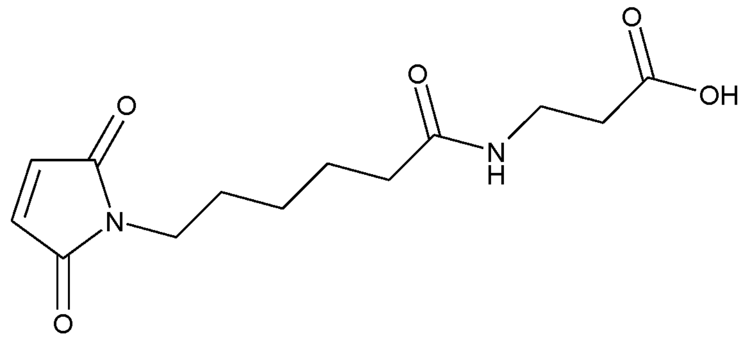

64.8 mg (0.357 mmol) *tert*-Butyl-beta-alaninathydrochlorid (1:1) und 100 mg (0.324 mmol) 1-{6-[(2,5-Dioxopyrrolidin-1-yl)oxy]-6-oxohexyl}-1H-pyrrol-2,5-dion wurden in 4 ml Dimethylformamid gelöst und mit 65.6 mg (0.649 mmol) N-Methylmorpholin versetzt. Die Reaktionsmischung wurde über Nacht bei RT gerührt. Es wurde 0.048 mL (0.838 mmol) Essigsäure zugegeben und die Reaktionsmischung wurde direkt mittles präp. RP-HPLC gereinigt (Säule: Reprosil 250x30; 10$\mu$, Fluss: 50 mL/min, MeCN/Wasser/0.1%TFA). Die Lösemittel wurden im Vakuum verdampft und der Rückstand im Hochvakuum getrocknet. Man erhielt 84.5 mg (77%, Reinheit 100%) *tert*-Butyl-N-[6-(2,5-dioxo-2,5-dihydro-1H-pyrrol-1-yl)hexanoyl]-beta-alaninat.

LC-MS (Methode 1): $R_t$ = 0.78 min; MS (ESIpos): m/z = 339 $(M+H)^+$.

Zu einer Lösung von *tert*-Butyl-N-[6-(2,5-dioxo-2,5-dihydro-1H-pyrrol-1-yl)hexanoyl]-beta-alaninat (82.8 mg, 0.244 mmol) in 8 ml dichlormethan wurde 1.62 ml TFA zugegeben. Das Reaktionsgemisch wird 2 Stunden bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 62.7 mg (87%, Reinheit 95%) der Titelverbindung.

LC-MS (Methode 1): $R_t$ = 0.75 min; MS (ESIpos): m/z = 283 $(M+H)^+$.

## Intermediat L80

2-(Trimethylsilyl)ethyl-3-[(15-amino-4,7,10,13-tetraoxapentadecan-1-oyl)amino]-N-(tert-butoxycarbonyl)-D-alaninat

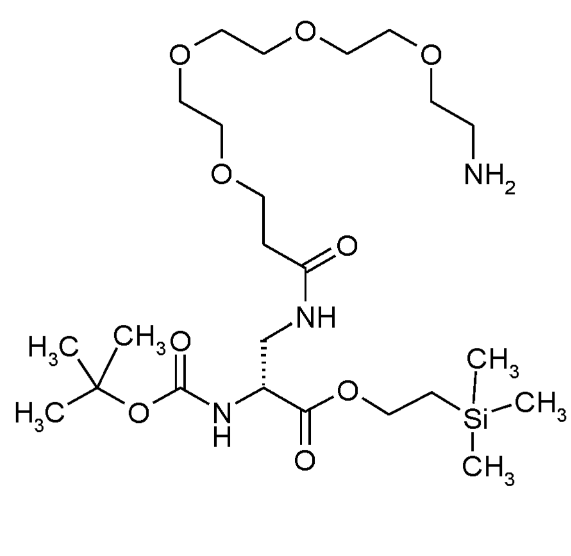

Die Titelverbindung wurde ausgehend von kommerziell erhältlichem 3-{[(Benzyloxy)carbonyl] amino}-N-(tert-butoxycarbonyl)-D-alanin --N-cyclohexylcyclohexanamin (1:1) nach klassischen Methoden der Peptidchemie (Freisetzung aus dem Salz und Veresterung mit 2-(Trimethylsilyl-ethanol mittels EDCI/DMAP, hydrogenolytische Abspaltung der Z-Schutzgruppe, Kupplung mit kommerziell erhältlicher 3-Oxo-1-phenyl-2,7,10,13,16-pentaoxa-4-azanonadecan-19-säure in Gegenwart von HATU und N, N-Diisopropylethylamin und erneute hydrogenolytische Abspaltung der Z-Schutzgruppe) hergestellt.

LC-MS (Methode 1): $R_t$ = 0.70 min; MS (ESIpos): m/z = 552 $(M+H)^+$.

## Intermediat L81

Trifluoressigsäure -benzyl-{2-[(2-aminoethyl)sulfonyl]ethyl}carbamat (1:1)

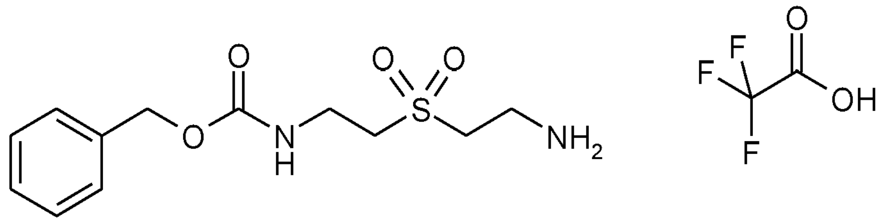

250 mg (1.11 mmol) 2,2'-Sulfonyldiethanamin wurden mit 92.3 mg (0.37 mmol) 1-{[(Benzyloxy)-carbonyl] oxy}pyrrolidin-2,5-dion in Gegenwart von N, N-Diisopropylethylamin in DMF gekuppelt. Nach anschließender HPLC Reinigung wurden 70 mg (47% d. Th.) der Titelverbindung erhalten.

C-MS (Methode 12): $R_t$ = 0.64 min; MS (ESIpos): m/z = 257.11 $(M+H)^+$.

## Intermediat L82

Trifluoressigsäure --N-{2-[2-(2-aminoethoxy)ethoxy]ethyl}-6-(2,5-dioxo-2,5-dihydro-1H-pyrrol-1-yl)hexanamid (1:1)

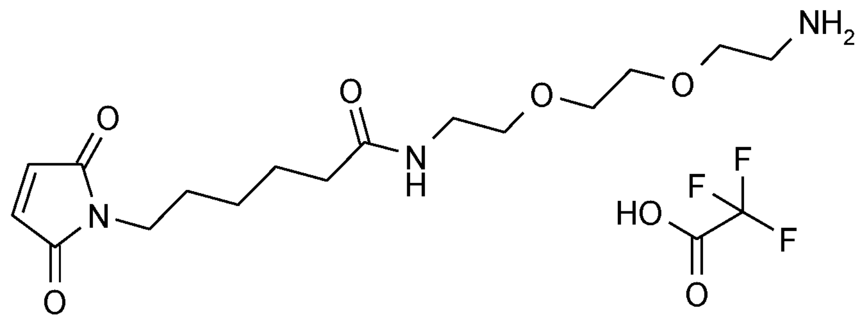

88.6 mg (0.357 mmol) N-Boc-2,2'-(ethylenedioxy)diethylamin und 100 mg (0.324 mmol) N-Succinimidyl 6-maleimidohexanoat wurden in 4.0 ml Dimethylformamid gelöst und mit 0.071 mL (0.650 mmol) N-Methylmorpholin versetzt. Die Reaktionsmischung wurde über Nacht bei RT gerührt. Es wurde 0.048 mL (0.838 mmol) Essigsäure zugegeben und die Reaktionsmischung wurde direkt mittles präp. RP-HPLC gereinigt (Säule: Reprosil 125x30; 10µ, Fluss: 75 mL/min, MeCN/Wasser/0.1%TFA). Die Lösemittel wurden im Vakuum verdampft und der Rückstand im Hochvakuum getrocknet. Man erhielt 127 mg (81% d. Th) tert-Butyl-{2-[2-(2-{[6-(2,5-dioxo-2,5-dihydro-1H-pyrrol-1-yl)hexanoyl]amino}ethoxy)ethoxy]ethyl}carbamat.

LC-MS (Methode 1): $R_t$ = 0.78 min; MS (ESIpos): m/z = 442 $(M+H)^+$.

Zu einer Lösung von 123 mg (225 µmol) tert-Butyl-{2-[2-(2-{[6-(2,5-dioxo-2,5-dihydro-1H-pyrrol-1-yl)hexanoyl]amino}ethoxy)ethoxy]ethyl}carbamat in 7.5 ml dichlormethan wurde 2.0 ml TFA zugegeben. Das Reaktionsgemisch wird 2 h bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 111 mg (100% d. Th) der Titelverbindung.

LC-MS (Methode 1): $R_t$ = 0.31 min; MS (ESIpos): m/z = 342 $(M+H)^+$.

$^1$H-NMR (400 MHz, DMSO-$d_6$): δ [ppm] = 1.17 (m, 2H), 1.47 (m, 4H), 2.04 (m, 2H), 2.98 (m, 2H), 3.19 (m, 2H), 3.39 (m, 4H), 3,56 (m, 6H), 7.01 (s, 2H), 7.72 (bs, 3H), 7.80 (m, 1H).

## Intermediat L83

Trifluoressigsäure--N-{2-[2-(2-aminoethoxy)ethoxy]ethyl}-2-(2,5-dioxo-2,5-dihydro-1H-pyrrol-1-yl)acetamid (1:1)

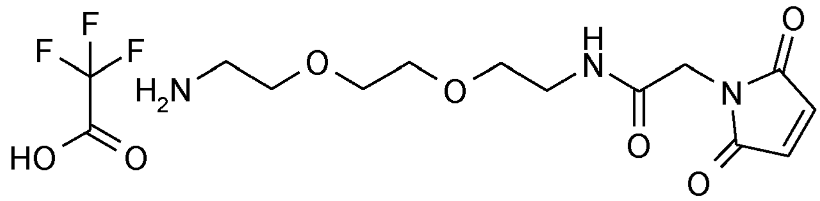

200 mg (0.805 mmol) tert-Butyl-{2-[2-(2-aminoethoxy)ethoxy]ethyl}carbamat, 150 mg (0.966 mmol) (2,5-Dioxo-2,5-dihydro-1H-pyrrol-1-yl)essigsäure und 560 μl (3.2 mmol) N,N-Diiso-propylethylamin wurden in 10 ml Dimethylformamid gelöst und mit 459 mg (1,21 mmol) HATU versetzt. Die Reaktionsmischung wurde 30 Minuten bei RT gerührt. Die Lösemittel wurden im Vakuum verdampft und der Rückstand in Dichloromethan gelöst. Die organische Phase wurde zweimal mit 5%iger Citronensäure-Lösung gewaschen, über Magnesiumsulfat getrocknet und das Lösemittel im Vakuum verdampft. Der Rückstand wurde mittels Biotage Isolera gereinigt (Kieselgel, Säule 25 g SNAP, Dischloromethan:Methanol 98:2). Man erhielt 276 mg (89 % d. Th) tert-Butyl-{2-[2-(2-{[(2,5-dioxo-2,5-dihydro-1H-pyrrol-1-yl)acetyl]amino}ethoxy)ethoxy]ethyl}carbamat.

LC-MS (Methode 1): $R_t$ = 0.67 min; MS (ESIpos): m/z = 386 $(M+H)^+$.

Zu einer Lösung von tert-Butyl-{2-[2-(2-{[(2,5-dioxo-2,5-dihydro-1H-pyrrol-1-yl)acetyl]amino}ethoxy)ethoxy]ethyl}carbamat (275 mg, 714 μmol) in 15 ml dichlormethan wurde 4 ml TFA zugegeben. Das Reaktionsgemisch wird 30 Minuten bei RT gerührt. Die Reaktionsmischung wurde im Vakuum verdampft und der Rückstand wurde in Wasser aufgenommen und lyophilisiert. Man erhielt 281 mg (99% d. Th) der Titelverbindung. LC-MS (Methode 1): $R_t$ = 0.17 min; MS (ESIpos): m/z = 286 $(M+H)^+$.

## Intermediat L84

Trifluoressigsäure N-(14-amino-3,6,9,12-tetraoxatetradec-1-yl)-6-(2,5-dioxo-2,5-dihydro-1H-pyrrol-1-yl)hexanamid (1:1)

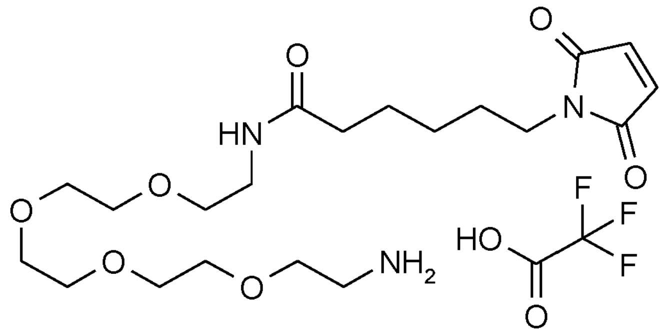

200 mg (0.594 mmol) tert-Butyl-(14-amino-3,6,9,12-tetraoxatetradec-1-yl)carbamat und 202 mg (0.654 mmol) 1-{6-[(2,5-Dioxopyrrolidin-1-yl)oxy]-6-oxohexyl}-1H-pyrrol-2,5-dion wurden in 4.0 ml Dimethylformamid gelöst und mit 0.130 mL (1.2 mmol) N-Methylmorpholin versetzt. Die Reaktionsmischung wurde über Nacht bei RT gerührt. Es wurde 0.085 ml (1.5 mmol) Essigsäure zugegeben und die Reaktionsmischung wurde direkt mittles präp. RP-HPLC gereinigt (Säule: Reprosil 125×30; 10μ, Fluss: 50 mL/min, MeCN/Wasser/0.1%TFA). Die Lösemittel wurden im Vakuum verdampft und der Rückstand im Hochvakuum getrocknet. Man erhielt 275 mg (73% d. Th) tert-Butyl-[21-(2,5-dioxo-2,5-dihydro-1H-pyrrol-1-yl)-16-oxo-3,6,9,12-tetraoxa-15-azahenicos-1-yl]carbamat.

LC-MS (Methode 1): $R_t$ = 0.81 min; MS (ESIpos): m/z = 530 $(M+H)^+$.

Zu einer Lösung von tert-Butyl-[21-(2,5-dioxo-2,5-dihydro-1H-pyrrol-1-yl)-16-oxo-3,6,9,12-tetraoxa-15-azahenicos-1-yl]carbamat (268 mg, 505 µmol) in 5.0 ml dichlormethan wurde 780 µl (10 mmol) TFA zugegeben. Das Reaktionsgemisch wurde über Nacht bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 266 mg (97% d. Th) der Titelverbindung.

LC-MS (Methode 1): $R_t$ = 0.46 min; MS (ESIpos): m/z = 430 $(M+H)^+$.

$^1$H-NMR (400 MHz, DMSO-$d_6$): δ [ppm] = 1.17 (m, 2H), 1.47 (m, 4H), 2.03 (m, 2H), 2.99 (m, 2H), 3.18 (m, 2H), 3.38 (m, 4H), 3,52 (m, 8H), 3,58 (m, 6H), 7.01 (s, 2H), 7.73 (bs, 3H), 7.80 (m, 1H).

**Intermediat L85**

Trifluoressigsäure --N-(14-amino-3,6,9,12-tetraoxatetradec-1-yl)-2-(2,5-dioxo-2,5-dihydro-1H-pyrrol-1-yl)acetamid (1:1)

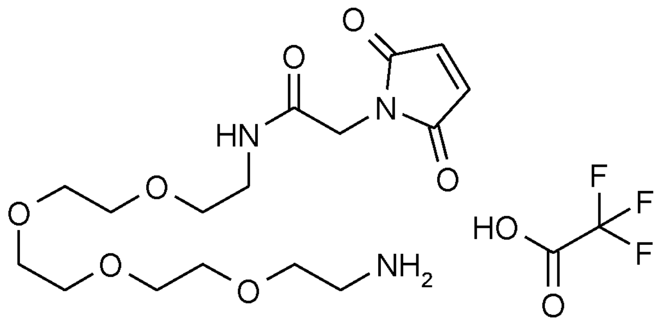

200 mg (0.594 mmol) tert-Butyl-(14-amino-3,6,9,12-tetraoxatetradec-1-yl)carbamat, 111 mg (0.713 mmol) (2,5-Dioxo-2,5-dihydro-1H-pyrrol-1-yl)essigsäure und 410 µl (2.4 mmol) N,N-Diisopropylethylamin wurden in 6 ml Dimethylformamid gelöst und mit 339 mg (0.892 mmol) HATU versetzt. Die Reaktionsmischung wurde 1 h bei RT gerührt und direkt mittles präp. RP-HPLC gereinigt (Säule: Reprosil 250×30; 10µ, Fluss: 50 mL/min, MeCN/Wasser/0.1%TFA). Die Lösemittel wurden im Vakuum verdampft und der Rückstand im Hochvakuum getrocknet. Man erhielt 130 mg (43% d: Th) tert-Butyl-[17-(2,5-dioxo-2,5-dihydro-1H-pyrrol-1-yl)-16-oxo-3,6,9,12-tetraoxa-15-azaheptadec-1-yl]carbamat.

LC-MS (Methode 1): $R_t$ = 0.71 min; MS (ESIpos): m/z = 474 $(M+H)^+$.

Zu einer Lösung tert-Butyl-[17-(2,5-dioxo-2,5-dihydro-1H-pyrrol-1-yl)-16-oxo-3,6,9,12-tetraoxa-15-azaheptadec-1-yl]carbamat (126 mg, 267 µmol) in 4.0 ml Dichlormethan wurde 410 µl (5.3 mmol) TFA zugegeben. Das Reaktionsgemisch wurde über Nacht bei RT gerührt. Die Reaktionsmischung wurde im Vakuum verdampft und der Rückstand am Hochvakuum getrocknet. Man erhielt 124 mg (95% d: Th) der Titelverbindung.

LC-MS (Methode 13): $R_t$ = 0.74 min; MS (ESIpos): m/z = 374 $(M+H)^+$.

$^1$H-NMR (400 MHz, DMSO-$d_6$): δ [ppm] = 2.99 (m, 2H), 3.22 (m, 2H), 3.41 (m, 2H), 3,53 (m, 8H), 3,58 (m, 6H), 4.02 (s, 2H), 7.09 (s, 2H), 7.73 (bs, 3H), 8.21 (m, 1H).

**Intermediat L86**

N-[(2,5-Dioxo-2,5-dihydro-1H-pyrrol-1-yl)acetyl]-L-valyl-L-alanin

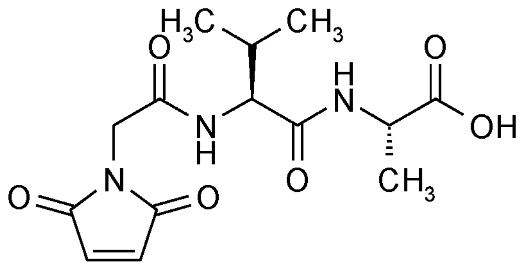

100 mg (0.531 mmol) L-Valyl-L-alanin und 134 mg (0.531 mmol) 1-{2-[(2,5-Dioxopyrrolidin-1-yl)oxy]-2-oxoethyl}-1H-pyrrol-2,5-dion wurden in 3 ml Dimethylformamid gelöst und mit 0.150 mL (1.1 mmol) Triethylamin versetzt. Die Reaktionsmischung wurde 8 h bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 71.5 mg (41 % d. Th.) der Titelverbindung.
LC-MS (Methode 1): $R_t$ = 0.42 min; MS (ESIpos): m/z = 326 $(M+H)^+$.

**Intermediat L87**

3-[2-(2-{[(2,5-Dioxo-2,5-dihydro-1H-pyrrol-1-yl)acetyl]amino}ethoxy)ethoxy]propansäure

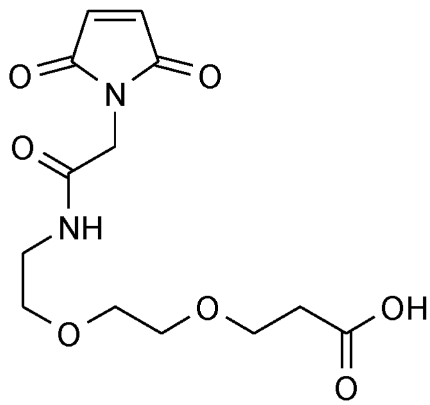

250 mg (1.07 mmol) tert-Butyl-3-[2-(2-aminoethoxy)ethoxy]propanoat, 151 mg (0.974 mmol) 2-(2,5-Dioxo-2,5-dihydro-1H-pyrrol-1-yl)acetic acid, 224 mg (1.46 mmol) 1-Hydroxy-1H-benzotriazol Hydrat und 224 mg (1.17 mmol) 1-(3-Dimethylaminopropyl)-3-ethylcarbodiimidhydrochlorid wurden in 5.0 ml Dimethylformamid gelöst. Die Reaktionsmischung wurde 1 h bei RT gerührt. Der Ansatz wurde mit Ethylacetat versetzt, zweimal mit 5%iger Citronensäure-Lösung und mit ges. Natriumhydrogencarbonat-Lösung extrartiert. Die organische Phase wurde zweimal mit ges. Natriumchlorid-Lösung gewaschen,. über Magnesiumsulfat getrocknet und das Lösemittel im Vakuum abgedampft. Der Rückstand wurde über die präp. RP-HPLC gereinigt (Säule: Reprosil 250x40; 10µ, Fluss: 50 mL/min, MeCN/Wasser/0.1%TFA). Die Lösemittel wurden im Vakuum verdampft und der Rückstand im Hochvakuum getrocknet. Man erhielt 267 mg (64% d: Th) tert-Butyl-3-[2-(2-{[(2,5-dioxo-2,5-dihydro-1H-pyrrol-1-yl)acetyl]-amino}ethoxy)ethoxy]propanoat.
LC-MS (Methode 1): Rt = 0.73 min; MS (ESIpos): m/z = 371 $(M+H)^+$.
Zu einer Lösung von tert-Butyl-3-[2-(2-{[(2,5-dioxo-2,5-dihydro-1H-pyrrol-1-yl)acetyl]amino}-ethoxy)ethoxy]propanoat (263 mg, 710 µmol) in 10 ml dichlormethan wurde 1.1 ml (14 mmol) TFA zugegeben. Das Reaktionsgemisch wurde über Nacht bei RT gerührt. Die Reaktionsmischung wurde im Vakuum verdampft und der Rückstand am Hochvakuum getrocknet. Man erhielt 240 mg (94% d: Th) der Titelverbindung.
LC-MS (Methode 12): $R_t$ = 0.57 min; MS (ESIpos): m/z = 315 $(M+H)^+$.

**Intermediat L88**

2,5-Dioxopyrrolidin-1-yl-N-[6-(2,5-dioxo-2,5-dihydro-1H-pyrrol-1-yl)hexanoyl]-L-valyl-L-alaninat

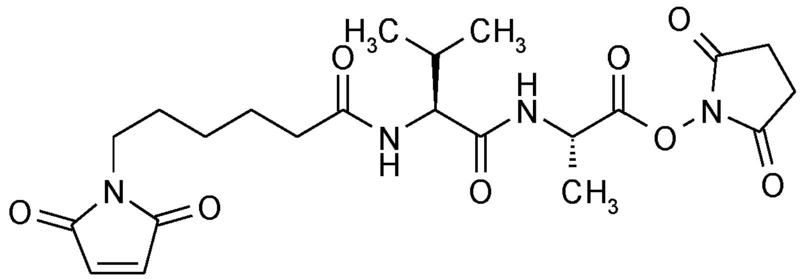

150 mg (0.797 mmol) L-Valyl-L-alanin und 246 mg (0.797 mmol) 1-{6-[(2,5-Dioxopyrrolidin-1-yl)oxy]-6-oxohexyl}-1H-pyrrol-2,5-dion wurden in 4.0 ml Dimethylformamid gelöst und mit 0.220 mL (1.6 mmol) Triethylamin versetzt. Die Reaktionsmischung wurde über Nacht bei RT gerührt. Die Reaktionsmischung wurde direkt mittles präp. RP-HPLC gereinigt (Säule: Reprosil 250×30; 10μ, Fluss: 50 mL/min, MeCN/Wasser). Die Lösemittel wurden im Vakuum verdampft und der Rückstand im Hochvakuum getrocknet. Man erhielt 302 mg (97 % d. Th.) N-[6-(2,5-Dioxo-2,5-dihydro-1H-pyrrol-1-yl)hexanoyl]-L-valyl-L-alanin.

LC-MS (Methode 12): $R_t$ = 1.02 min; MS (ESIpos): m/z = 382 $(M+H)^+$.

$^1$H-NMR (400 MHz, DMSO-$d_6$): δ [ppm] = 0.82 (dd, 6H), 1.17 (m, 2H), 1.27 (d, 3H), 1.48 (m, 4H), 1.94 (m, 1H), 2.13 (m, 2H), 3.38 (t, 2H), 4.17 (m, 2H), 7.00 (s, 2H), 7.75 (d, 1H), 8.19 (d, 1H).

130 mg (0.531 mmol) N-[6-(2,5-Dioxo-2,5-dihydro-1H-pyrrol-1-yl)hexanoyl]-L-valyl-L-alanin wurden in 6.5 ml Dichloromethan gelöst und mit 58.8 mg (0.511 mmol) 1-Hydroxypyrrolidin-2,5-dion und 78.4 mg (0.409 mmol) 1-(3-Dimethylaminopropyl)-3-ethylcarbodiimidhydrochlorid versetzt. 58.8 mg (0.511 mmol) 1-Hydroxypyrrolidin-2,5-dion und 78.4 mg (0.409 mmol) 1-(3-Dimethylaminopropyl)-3-ethylcarbodiimidhydrochlorid wurden nocheinmal zugegeben. Der Ansatz wurde mit Dichloromethan versetzt und dreimal mit Wasser gewaschen. Die organische Phase wurde über Magnesiumsulfat getrocknet, das Lösemittel im Vakuum abgedampft und der Rückstand im Hochvakuum getrocknet. Man erhielt 172 mg (87 % d. Th.) der Titelverbindung.

LC-MS (Methode 12): $R_t$ = 1.28 min; MS (ESIpos): m/z = 479 $(M+H)^+$.

**Intermediat L89**

1-Benzyl-5-[2-(trimethylsilyl)ethyl]-L-glutamathydrochlorid (1:1)

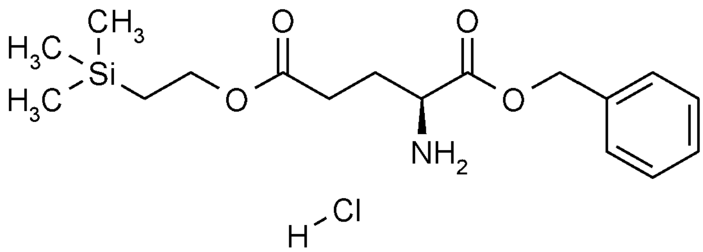

1.00 g (2.96 mmol) (4S)-5-(Benzyloxy)-4-[(tert-butoxycarbonyl)amino]-5-oxopentansäure wurden in 13.0 ml THF vorgelegt und mit 510 μl (3.6 mmol) 2-(Trimethylsilyl)ethanol und 109 mg (889 μmol) 4-Dimethylaminopyridin versetzt. Die Reactionmischung wurde auf 0°C gekühlt und mit 682 mg (3.56 mmol) N-Ethyl-N'-3-(dimethylaminopropyl)carbodiimid hydrochlorid versetzt. Die Reactionmischung wurde über Nacht bei RT gerührt. Die Lösemittel wurden im Vakuum verdampft und der Rückstand in Ethylacetat gelöst. Die organische Phase wurde zweimal mit 0.1 HCl-Lösung und ges. Natriumchlorid-Lösunggewaschen, über Magnesiumsulfat getrocknet und das Lösemittel im Vakuum verdampft. Der Rückstand wurde mittels Biotage Isolera gereinigt (Kieselgel, Säule 25 g SNAP, Cyclohexan:Ethylacetat 80:20). Man erhielt 649 mg (50 % d. Th) der Verbindung 1-Benzyl-5-[2-(trimethylsilyl)ethyl]-N-(tert-butoxycarbonyl)-L-glutamat.

LC-MS (Methode 1): $R_t$ = 4.6 min; MS (ESIpos): m/z = 438 $(M+H)^+$.

649 mg (1.48 mmol) 1-Benzyl-5-[2-(trimethylsilyl)ethyl]-N-(tert-butoxycarbonyl)-L-glutamat wurden in 7.0 ml Dioxan gelöst und unter Eisbadkühlung wurden 14 ml (59 mmol) 4N HCl in Dioxan dazugegeben. Die Reactionmischung wurde über Nacht bei RT gerührt. Die Reaktionsmischung wurde im Vakuum verdampft und der Rückstand am Hochvakuum getrocknet und mittels Biotage Isolera gereinigt (Kieselgel, Säule 25 g SNAP, Dischloromethan:Methanol 90:10). Man erhielt 320 mg (57 % d. Th) der Titel Verbindung. LC-MS (Methode 1): $R_t$ = 0.79 min; MS (ESIpos): m/z = 338 $(M+H)^+$.

**Intermediat L90**

1-({N-[(Benzyloxy)carbonyl]glycyl}amino)-3,6,9,12-tetraoxapentadecan-15-säure

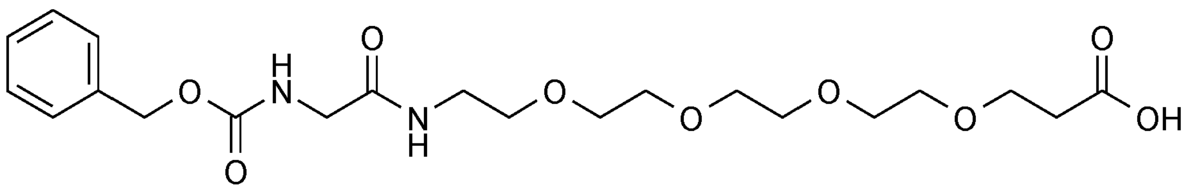

118 mg (566 μmol) N-[(Benzyloxy)carbonyl]glycin wurden in 5.0 ml DMF vorgelegt, mit 200 mg (622 μmol) tert-Butyl-1-amino-3,6,9,12-tetraoxapentadecan-15-oat, 130 mg (849 μmol) 1-Hydroxy-1H-benzotriazol Hydrat und 130 mg (679 μmol) 1-(3-Dimethylaminopropyl)-3-ethylcarbodiimidhydrochlorid versetzt und 1 h bei RT gerührt. Der Ansatz wurde mit Ethylacetat versetzt, zweimal mit 5%iger Citronensäure-Lösung und mit gesättigter Natriumhydrogen-carbonat-Lösung extrartiert. Die organische Phase wurde zweimal mit gesättigter Natriumchlorid-Lösung gewaschen und. über Magnesiumsulfat getrocknet. Die Lösemittel wurden im Vakuum verdampft und der Rückstand im Hochvakuum getrocknet. Man erhielt 274 mg (95% d: Th.) tert-Butyl-1-({N-[(benzyloxy)carbonyl]glycyl}amino)-3,6,9,12-tetraoxapentadecan-15-oat.

LC-MS (Methode 12): Rt = 1.69 min; MS (ESIpos): m/z = 513 $(M+H)^+$.

Zu einer Lösung von 274 mg (535 μmol)tert-Butyl-1-({N-[(benzyloxy)carbonyl]glycyl}amino)-3,6,9,12-tetraoxapentadecan-15-oat in 5.0 ml dichlormethan wurde 820 μl (11 mmol) TFA zugegeben. Das Reaktionsgemisch wurde 3 h bei RT gerührt. Die Reaktionsmischung wurde im Vakuum verdampft und der Rückstand in Wasser aufgenommen und lyophilisiert. Man erhielt 262 mg (100% d: Th.) der Titelverbindung.

LC-MS (Methode 12): $R_t$ = 1.12 min; MS (ESIpos): m/z = 457 $(M+H)^+$.

**Intermediat L91**

Trifluoressigsäure--2-(trimethylsilyl)ethyl-1-{[3-amino-N-(tert-butoxycarbonyl)-D-alanyl]amino}-3,6,9,12-tetraoxapentadecan-15-oat (1:1)

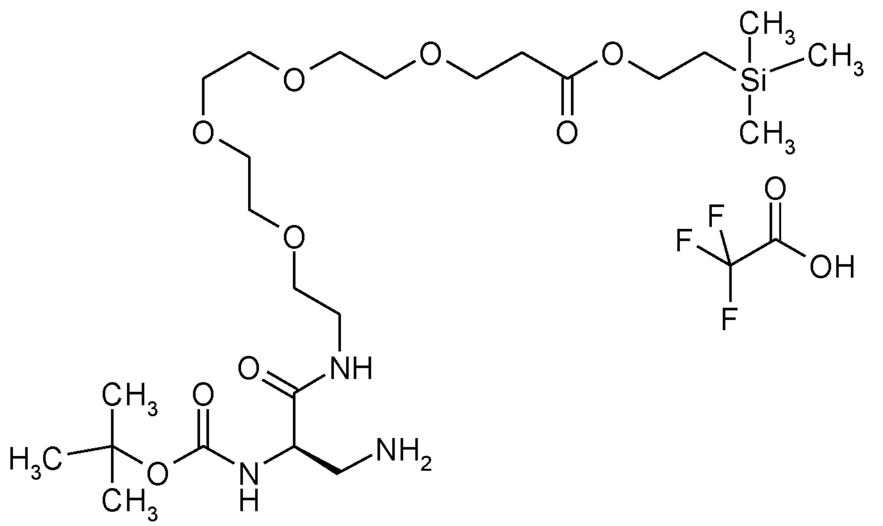

Die Titelverbindung wurde ausgehend von kommerziell erhältlicher 3-Oxo-1-phenyl-2,7,10,13,16-pentaoxa-4-azanonadecan-19-säure nach klassischen Methoden der Peptidchemie (Veresterung mit 2-Trimethylsilylethanol mittels EDCI/DMAP, hydrogenolytische Abspaltung der Z-Schutzgruppe, Kupplung mit kommerziell erhältlichem N-(tert-Butoxycarbonyl)-3-{[(9H-fluoren-9-ylmethoxy) carbonyl]amino}-D-alanin und Abspaltung der Fmoc-Schutzgruppe) hergestellt.

LC-MS (Methode 1): $R_t$ = 0.74 min; MS (ESIpos): m/z = 552 $(M+H)^+$.

## Intermediat L92

N-[(Benzyloxy)carbonyl]-L-alanyl-L-alanyl-L-asparagin

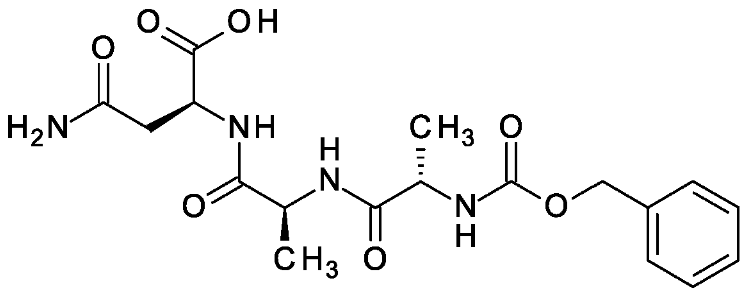

Die Titelverbindung wurde nach klassischen Methoden der Peptidchemie durch HATU-Kupplung in Gegenwart von N,N-Diisopropylethylamin von kommerziell erhältlichen N-[(Benzyloxy) carbonyl]-L-alanyl-L-alanin mit tert-Butyl-L-asparaginat und anschließender Entschützung der Carboxygruppe mit Trifluoressigsäure hergestellt.
LC-MS (Methode 1): $R_t$ = 0.5 min; MS (ESIpos): m/z = 409 $(M+H)^+$.

## Intermediat L93

N-Acetyl-L-alanyl-L-alanyl-L-asparagin

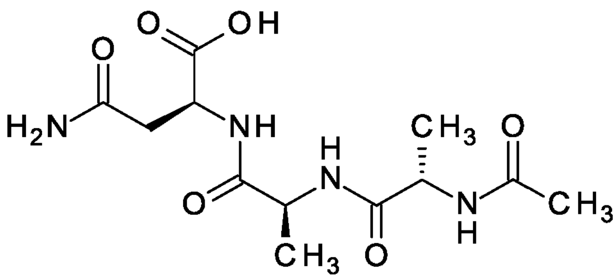

Die Titelverbindung wurde nach klassischen Methoden der Peptidchemie durch HATU-Kupplung in Gegenwart von N,N-Diisopropylethylamin von kommerziell erhältlichen N-[(Benzyloxy) carbonyl]-L-alanyl-L-alanin mit tert-Butyl-L-asparaginat, anschließender Abspaltung der Z-Schutzgruppe durch Hydrierung in DCM-Methanol über 10% Palladium auf Aktivkohle, dann folgender Acetylierung mit Essigsäure in DMF in Gegenwart von HATU und N,N-Diisopropylethylamin und schließlich Entschützung der Carboxygruppe mit Trifluoressigsäure hergestellt.
LC-MS (Methode 1): $R_t$ = 0.16 min; MS (ESIpos): m/z = 317 $(M+H)^+$.
$^1H$-NMR (400 MHz, DMSO-$d_6$): $\delta$ [ppm] = 1.19 (2d, 6H), 1.82 (s, 3H), 2.5 (m, 2H), 4.26 (m, 2H), 4.48 (q, 1H), 6.9 (s, 1H), 7.36 (s, 1H), 8.0 (m, 3H), 12.54 (s, 1H).

## Intermediat L94

N-{4-Oxo-4-[2-(trimethylsilyl)ethoxy]butanoyl}-L-alanyl-L-alanyl-L-asparagin

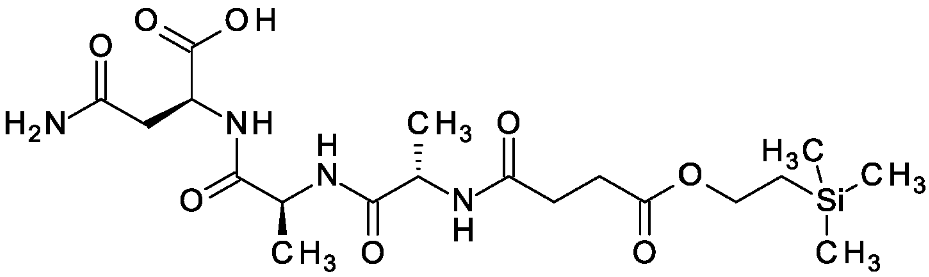

Zunächst wurde 4-Oxo-4-[2-(trimethylsilyl)ethoxy]butansäure durch Umsetzung von 4-(Benzyl-oxy)-4-oxobutansäure mit 2-(Trimethylsilyl)ethanol in Gegenwart von EDCI/DMAP in DCM und anschließender hydrogenolytischen Spaltung des Benzylesters hergestellt. LC-MS (Methode 1): $R_t$ = 0.89 min; MS (ESIpos): m/z = 217 $(M-H)^-$.

Weiterhin wurde Trifluoressigsäure --4-nitrobenzyl-L-alanyl-L-alanyl-L-asparaginat (1:1) durch Kupplung von N-(tert-Butoxycarbonyl)-L-alanyl-L-alanin mit 4-Nitrobenzyl-L-asparaginat hydrobromid (1:1) in DMF in Gegenwart von HATU und N,N-Diisopropylethylamin und anschließender Entschützung der Aminogruppe mit Trifluoressigsäure in DCM hergestellt.

LC-MS (Methode 1): $R_t$ = 0.43 min; MS (ESIpos): m/z = 410 $(M+H)^+$.

Die Titelverbindung wurde dann durch Kupplung dieser beiden Intermediate in DMF in Gegenwart von HATU und N,N-Diisopropylethylamin und anschließender Abspaltung des p-Nitro-benzylesters durch Hydrierung in DCM-Methanol 1:9 über 10% Palladium auf Aktivkohle hergestellt.

LC-MS (Methode 1): $R_t$ = 0.79 min; MS (ESIpos): m/z = 475 $(M+H)^+$.

## Intermediat L95

N-[(Benzyloxy)carbonyl]-L-valyl-L-alanin

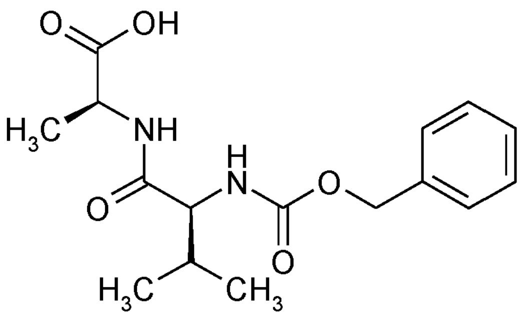

Dieses Intermediat wurde ausgehend von N-[(Benzyloxy)carbonyl]-L-valin und tert-Butyl-L-alaninathydrochlorid (1:1) mit klassischen Methoden der Peptidchemie hergestellt.

LC-MS (Methode 12): $R_t$ = 1.34 min; MS (ESIpos): m/z = 323.16 $(M+H)^+$.

## Intermediat L96

N-Acetyl-L-valyl-$N^5$-carbamoyl-L-ornithinamid

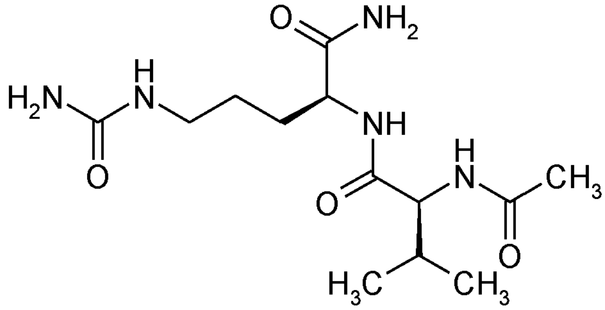

Dieses Intermediat wurde nach klassischen Methoden der Peptidchemie hergestellt beginnend mit der Kupplung von 2,5-Dioxopyrrolidin-1-yl-N-[(benzyloxy)carbonyl]-L-valinat mit $N^5$-Carbamoyl-L-ornithin, dann folgender hydrogenolytischer Abspaltlung der Z-Schutzgruppe über 10% Palladium/Aktivkohle in Ethanol und schließlich durch Umsetzung des erhaltenen Dipeptids mit 1-Acetoxypyrrolidin-2,5-dion.

LC-MS (Methode 1): $R_t$ = 0.25 min; MS (ESIpos): m/z = 317 $(M+H)^+$.

## Intermediat L97

1-(2,5-Dioxo-2,5-dihydro-1H-pyrrol-1-yl)-2-oxo-6,9,12,15,18,21,24,27-octaoxa-3-azatriacontan-30-säure

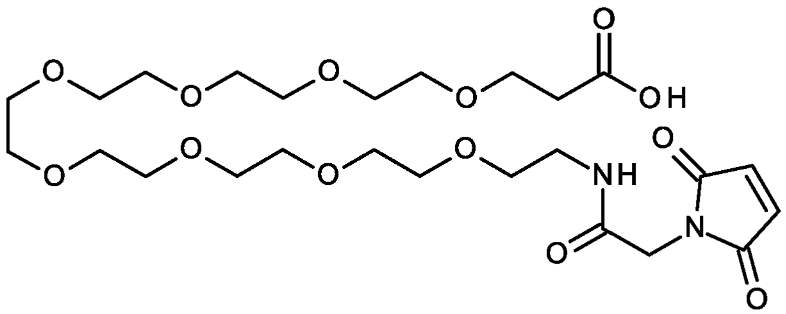

Tert-Butyl-1-amino-3,6,9,12,15,18,21,24-octaoxaheptacosan-27-oat (100 mg, 201 μmol) wurde in 1.0 ml DMF vorgelegt und mit (2,5-Dioxo-2,5-dihydro-1H-pyrrol-1-yl)essigsäure (46.8 mg, 301 μmol), 1-Hydroxy-1H-benzotriazol Hydrat (76.9 mg, 502 μmol) und 1-(3-Dimethylaminopropyl)-3-ethylcarbodiimidhydrochlorid (77.0 mg, 402 μmol) versetzt. Die Reaktionsmischung wurde über Nacht bei RT gerührt und dann mit Ethylacetat versetzt. Die organische Phase wurde zweimal mit 5%iger Citronensäure-Lösung, mit ges. Natriumhydrogencarbonat-Lösung und dann mit ges. Natriumchlorid-Lösung gewaschen. Die organische Phase wurde über Magnesiumsulfat getrocknet. Die Lösemittel wurden im Vakuum verdampft und der Rückstand mittles präp. RP-HPLC gereinigt (Säule: Reprosil 125x30; 10μ, Fluss: 50 mL/min, MeCN/Wasser/0.1%TFA). Die Lösemittel wurden im Vakuum verdampft und der Rückstand im Hochvakuum getrocknet. Man erhielt 19.1 mg (13% d. Th.) der Verbindung tert-Butyl-1-(2,5-dioxo-2,5-dihydro-1H-pyrrol-1-yl)-2-oxo-6,9,12,15,18,21,24,27-octaoxa-3-azatriacontan-30-oat.

LC-MS (Methode 1): $R_t$ = 0.87 min; MS (ESIpos): m/z = 635 $[M+H]^+$

Zu einer Lösung von tert-Butyl-1-(2,5-dioxo-2,5-dihydro-1H-pyrrol-1-yl)-2-oxo-6,9,12,15,18,21, 24,27-octaoxa-3-azatriacontan-30-oat (19.1 mg, 30.1 μmol) in 1.0 ml DCM wurde TFA (62 μl, 600 μmol) zugegeben. Das Reaktionsgemisch wurde 3h bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 10.8 mg (46 % d. Th.) der Titelverbindung.

LC-MS (Methode 1): $R_t$ = 0.55 min; MS (ESIneg): m/z = 577 $[M-H]^-$.

## Intermediat L98

2,2-Dimethylpropansäure--2-(trimethylsilyl)ethyl-N-(2-aminoethyl)-$N^2$-{[2-(trimethylsilyl) ethoxy]carbonyl}-L-glutaminat (1:1)

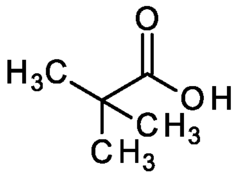

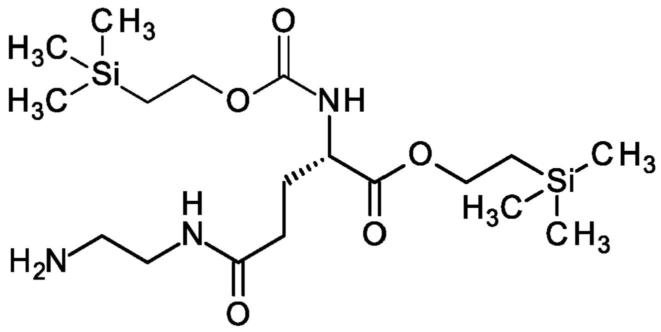

Zunächst wurde (4S)-5-tert-Butoxy-4-[(tert-butoxycarbonyl)amino]-5-oxopentansäure in Gegenwart von HATU und N,N-Diisopropylethylamin mit Benzyl-(2-aminoethyl)carbamat gekuppelt. Anschließend wurden mittels Trifluoressigsäure in DCM die Boc-Schutzgruppe sowie der tert.-Butylester abgespalten. Dann wurde zunächst die Aminogruppe durch Umsetzung mit 1-({[2-(Trimethylsilyl)ethoxy]carbonyl}oxy)pyrrolidin-2,5-dion in DMF/Wasser in Gegenwart von N,N-Diisopropylethylamin und anschließend die Carboxygruppe durch Umsetzung mit 2-(Trimethylsilyl)ethanol in DCM in Gegenwart von EDCI /DMAP erneut geschützt. Im letzten Schritt erfolgte die Entschützung der terminalen Aminogruppe mittels Hydrogenolyse über 10%-igem Palladium auf Aktivkohle in Ethanol unter Normaldruck. Nach Abfiltrieren des Katalysators, Einengen, Reinigung durch präparative HPLC und Gefriertrocknung des Rückstandes aus Acetonitril/Wasser wurde die Titelverbindung erhalten.

LC-MS (Methode 1): $R_t$ = 0.82 min; MS (ESIpos): m/z = 434 $(M+H)^+$.

## Intermediat L99

Trifluoressigsäure --2-(trimethylsilyl)ethyl-N-[(2,5-dioxo-2,5-dihydro-1H-pyrrol-1-yl)acetyl]-L-valyl-L-alanyl-beta-alanyl-L-lysinat (1:1)

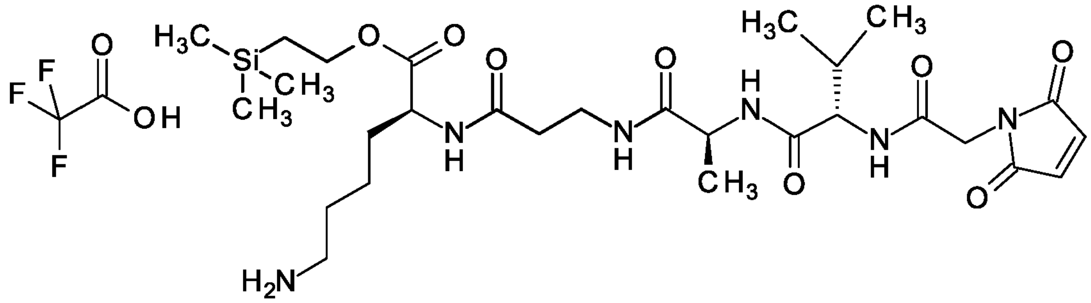

Zunächst wurde ausgehend von N2-[(Benzyloxy)carbonyl]-N6-(tert-butoxycarbonyl)-L-lysin nach klassischen Methoden der Peptidchemie 2-(Trimethylsilyl)ethyl-N6-(tert-butoxycarbonyl)-L-lysinat hergestellt. Dieses Intermediat wurde dann in Gegenwart von HATU und N,N-Diisopropylethylamin mit dem nach Standardmethoden hergestellten Tripeptidbaustein N-[(Benzyloxy) carbonyl]-L-valyl-L-alanyl-beta-alanin gekuppelt. Die Z-Schutzgruppe wurde anschließend durch Hydrogenolyse in Methanol entfernt und das erhaltene Intermediat mit (2,5-Dioxo-2,5-dihydro-1H-pyrrol-1-yl)essigsäure in Gegenwart von HATU und N, N Diisopropylethylamin gekuppelt. Im letzten Schritt erfolgte die Entschützung der Seitenkettenaminogruppe unter schonenden Bedingungen durch 1h Rühren in 10%iger Trifluoressigsäure in DCM bei RT. Nach Einengen und Gefriertrocknung aus Acetonitril/Wasser wurde die Titelverbindung erhalten.

LC-MS (Methode 1): $R_t$ = 0.64 min; MS (ESIpos): m/z = 625 $(M+H)^+$

## Intermediat L100

3-[5-(2-{[(2,5-Dioxo-2,5-dihydro-1H-pyrrol-1-yl)acetyl]amino}ethyl)-1,2,4-oxadiazol-3-yl]propansäure

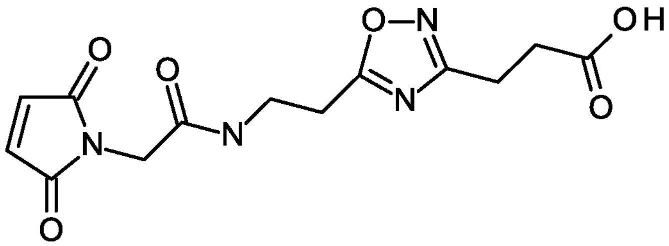

Zu einer Lösung von Methyl-3-cyanpropanoat (4 g, 35.4 mmol) in 120 ml Ethanol wurden 3.69 g (53 mmol) Hydroxylaminehydrochlorid und 15 ml (110 mmol) Triethylamin zugegeben. Das Reaktionsgemisch wurde bei 50 °C 3h gerührt. Das Gemischt wurde eingeengt und der Rückstand wurde in Ethylacetat gelöst und anschließend mit Wasser und Brine gewaschen. Die organische Phase wurde über Magnesiumsulfat getrocknet und eingeengt. Der Rückstand wurde ohne weitere Reinigung eingesetzt. Man erhielt 5 g (97% d. Th.) Methyl-(4Z)-4-amino-4-(hydroxyimino)-butanoat.

Zu einer Lösung von Methyl-(4Z)-4-amino-4-(hydroxyimino)butanoat (4.85 g, 33.19 mmol) in 120.0 ml ml Dioxan wurden 6.91 g (36.50 mmol) N-(tert-Butoxycarbonyl)-beta-alanin und 8.22 g (39.82 mmol) 1,3-Dicycloxexylcarbodiimid zugegeben. Das Reaktionsgemisch wurde bei Raumtemperatur 3h gerührt. Das Gemisch wurde eingeengt und der Rückstand wurde in Wasser gelöst und mit Ethylacetat extrahiert. Die organisch Phase wurde über Natriumsulfat getrocknet und eingeent. Der Rückstand wurde mittels FlashChromatographie. Man erhielt 6.0 g (57% d.Th.) Methyl-(4E)-4-{[N-(tert-butoxycarbonyl)-beta-alanyl]amino}-4-(hydroxyimino)butanoat.

Eine Lösung von Methyl-(4E)-4-{[N-(tert-butoxycarbonyl)-beta-alanyl]amino}-4-(hydroxyimino)butanoat ( 6.0 g, 18.9 mmol) in 100 ml DMF wurde 5h bei 120 °C gerührt. Das Gemisch wurde mit Wasser versetzt und mit Ethylacetat extrahiert. Die organische Phase wurde über Natriumsulfat getrocknet und eingeengt. Der Rückstand wurde mittels präp. HPLC gereinigt. Man erhielt 4 g (71% d. Th.) Methyl-3-(5-{2-[(tert-butoxycarbonyl)amino]ethyl}-1,2,4-oxadiazol-3-yl)propanoat.

Zu einer Lösung von Methyl-(4E)-4-{[N-(tert-butoxycarbonyl)-beta-alanyl]amino}-4-(hydroxyimino)butanoat (4.00 g, 13.4 mmol) in 60 ml THF wurde eine Lösung von LiOH (1.60 g, 66.8 mmol) in 10 ml Wasser zugegeben. Das Reaktionsgemisch wurde über Nacht bei 60 °C gerührt. Das Gemisch wurde mit Wasser versetzt und mit Ethylacetat extrahiert. Die organische Phase wurde über Natriumsulfat getrocknet und eingeengt. Der Rückstand wurde ohne weitere Reinigung eingesetzt. Man erhielt 3.60 g (87% d. Th.) 3-(5-{2-[(tert-Butoxycarbonyl)amino]ethyl}-1,2,4-oxadiazol-3-yl)propansäure.

Zur eine Lösung von 3-(5-{2-[(tert-Butoxycarbonyl)amino]ethyl}-1,2,4-oxadiazol-3-yl)propansäure (2.0 g, 7.01 mmol) in 30 ml Dichlormethan wurden 2.0 ml (26 mmol) Trifluoressigsäure zugegeben. Das Reaktionsgemisch wurde 1h bei Raumtemperatur gerührt. Das Gemisch wurde mit Wasser versetzt und extrahiert mit Dichlormethan. Die organische Phase wurde über Natriumsulfat getrocknet und eingeengt. Der Rückstand wurde ohne weitere reinigung eingesetzt. Man erhielt 1.50 g (72% d. Th.) 3-[5-(2-Aminoethyl)-1,2,4-oxadiazol-3-yl]propansäure -trifluoressigsäure (1:1).

Zur eine Lösung von 3-[5-(2-Aminoethyl)-1,2,4-oxadiazol-3-yl]propansäure (1.5 g, 5.01 mmol) in 25 ml DMF wurden 1.30 g (5.52 mmol) 1-[2-(2,5-Dioxopyrrolidin-1-yl)-2-oxoethyl]-1H-pyrrol-2,5-dion und 1.52 g (15.04 mmol) Triethylmin zugegeben. Das Reaktionsgemisch wurde 1h bei Raumtemperatur gerührt. Das Gemisch wurde mit Wasser versetzt und extrahiert mit Dichloromethan. Die organische Phase wurde über Natriumsulfat getrocknet und eingeengt. Der Rückstand wurde präp. HPLC gereinigt. Man erhielt 774 m g (47% d. Th.) der Titel Verbindung.

$^{1}$H-NMR (300 MHz, DMSO-$d_6$): $\delta$ [ppm] = 2.67 (t, 2H), 2.91 (t, 2H), 3.03 (t, 2H), 3.46 (q, 2H), 4.28 (s, 2H), 7.01 (s, 2H), 8.37 (t, 1H), 12.28 (bs, 1H).

## Intermediat L101

tert-butyl-L-alanyl-L-alanyl-L-asparaginat

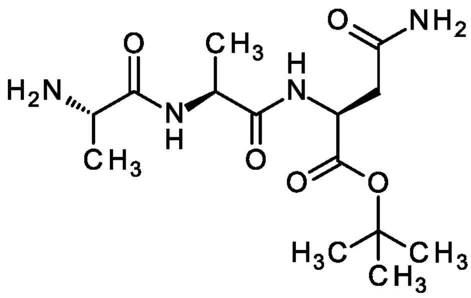

Die Titelverbindung wurde nach klassischen Methoden der Peptidchemie durch HATU-Kupplung in Gegenwart von N,N-Diisopropylethylamin von kommerziell erhältlichen N-[(Benzyloxy)-carbonyl]-L-alanyl-L-alanin mit tert-Butyl-L-asparaginathydrochlorid, dann folgender hydrogenolytischer Abspaltlung der Z-Schutzgruppe über 10% Palladium/Aktivkohle in Methanol.

LC-MS (Methode 7): $R_t$ = 0.23 min; MS (ESIneg): m/z = 329 $(M-H)^-$.

## Intermediat L102

N-(38-Oxo-2,5,8,11,14, 17,20,23,26,29,32,35-dodecaoxaoctatriacontan-38-yl)-L-alanyl-L-alanyl-L-asparagin

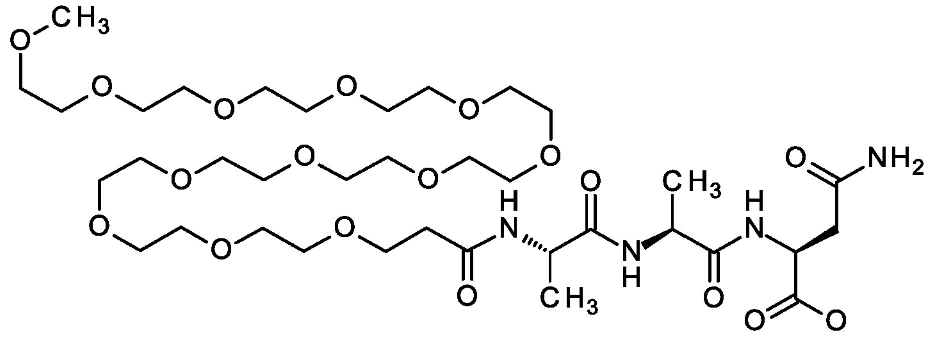

215 mg 2,5,8,11,14,17,20,23,26,29,32,35-Dodecaoxaoctatriacontan-38-säure (365 µmol) und 133 mg intermediat L101 (402 µmol) wurden in 1.4 ml DMF vorgelegt, mit 146 mg HATU (384 µmol) und 160 µl N,N-Diisopropylethylamin (910 µmol) versetzt und 3 h bei RT gerührt. Es wurde Wasser (1,5 mL) und ACN (0,5 mL) addiert. Die Reaktionslösung wurde mittels präparativer HPLC gereinigt (Eluent: ACN/Wasser + 0.1% TFA, Gradient = 1:9 → 3:2) und anschließender Abspaltung der Butoxycarbonylschutzgruppe mit 2 mL TFA in 2 mL DCM (3 h bei RT gerührt).

LC-MS (Methode 1): $R_t$ = 0.56 min; MS (ESIneg): m/z = 844.5 $(M+H)^+$.

## Intermediat L103

N-(pyridin-4-ylacetyl)-L-alanyl-L-alanyl-L-asparagine trifluoroacetate (1:1)

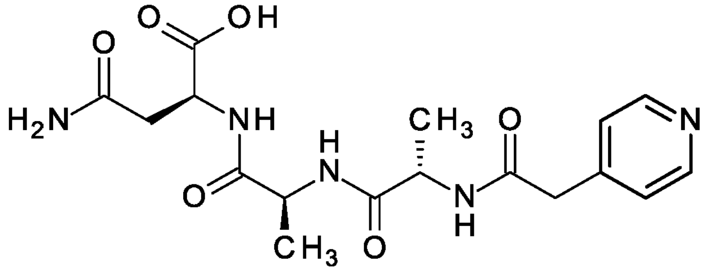

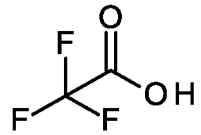

Die Titelverbindung wurde nach klassischen Methoden der Peptidchemie beginnend mit der Kupplung von 4-Pyridinessigsäure mit kommerziell erhältlichem tert-Butyl-L-alanyl-L-alaninat in Gegenwart von HATU und N,N-Diisopropylethylamin, dann folgender Entschützung mit Trifluoressigsäure, Kupplung mit tert-Butyl-L-asparaginat und anschließender Entschützung der Carboxygruppe mit Trifluoressigsäure hergestellt. LC-MS (Methode 1): $R_t$ = 0.15 min; MS (ESIpos): m/z = 394 $(M+H)^+$.

## Intermediat L104

N-Isonicotinoyl-L-alanyl-L-alanyl-L-asparagin trifluoracetat (1:1)

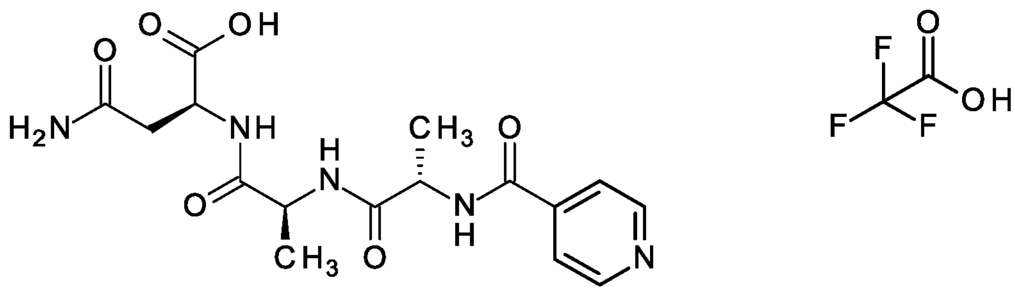

Die Titelverbindung wurde in Analogie zu Intermediat L103 beginnend mit der Kupplung von Isonicotinsäure mit kommerziell erhältlichem tert-Butyl-L-alanyl-L-alaninat hergestellt. LC-MS (Methode 1): $R_t$ = 0.17 min; MS (ESIpos): m/z = 380 $(M+H)^+$.

## Intermediat L105

tert-butyl N-{[2-(2-methoxyethoxy)ethoxy]acetyl}-L-alanyl-L-alanyl-L-asparaginat trifluor acetate (1:1)

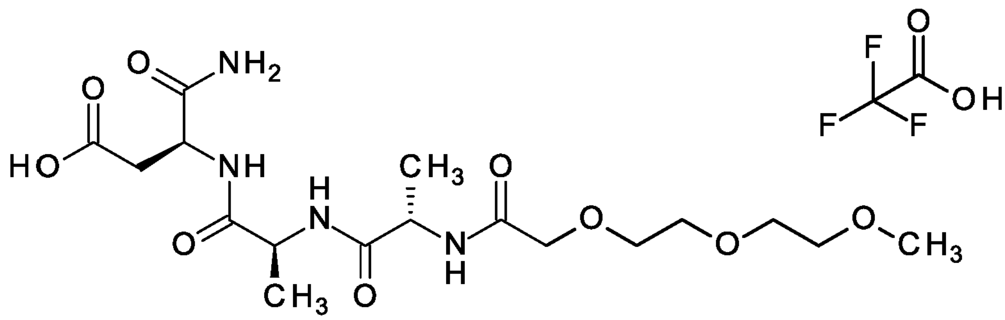

Die Titelverbindung wurde in Analogie zu Intermediat L103 beginnend mit der Kupplung von [2-(2-methoxyethoxy)ethoxy]essigsäure mit kommerziell erhältlichem tert-Butyl-L-alanyl-L-alaninat hergestellt.

LC-MS (Methode 1): $R_t$ = 0.17 min; MS (ESIpos): m/z = 380 $(M+H)^+$.

## Intermediat L106

N-[(Benzyloxy)carbonyl]-L-alanyl-L-alanyl-L-asparagin

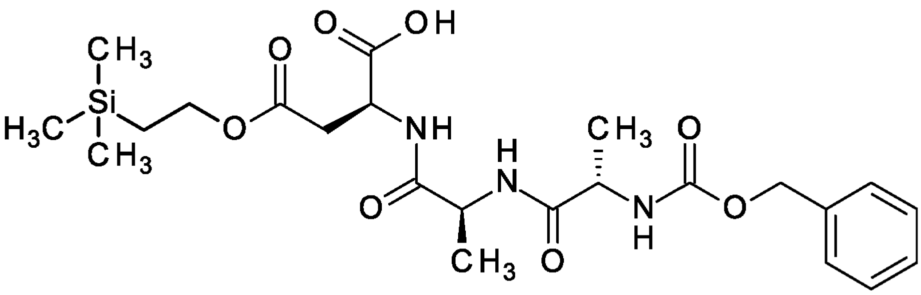

Die Titelverbindung wurde nach klassischen Methoden der Peptidchemie durch Kupplung von kommerziell erhältlichen N-[(Benzyloxy) carbonyl]-L-alanyl-L-alanin mit 1-tert-Butyl 4-[2-(trimethylsilyl)ethyl] L-aspartat in Gegenwart von HATU und N,N-Diisopropylethylamin hergestellt. Dieser Aminosäurebaustein wurde aus (3S)-4-tert-Butoxy-3-[(tert-butoxycarbonyl)amino]-4-oxobutanoic acid durch Veresterung mit 2-(Trimethylsilyl)ethanol in Gegenwart von EDCI und DMAP und anschließender schonender Entferung der tert.-Butoxycarbonylschutzgruppe mittels 5 %-iger Trifluoressigsäure in DCM hergestellt. Anschließend wurden 745 mg (1.317 mmol) des voll geschützten Intermediats in 43.5 ml DCM gelöst und durch Zugabe von 3.5 ml Trifluoressigsäure und 5-stündigem Rühren bei RT der tert-Butylester schonend gespalten. Aus dem entstandenen Produktgemisch wurden nach Reinigung durch präparative HPLC 168 mg (25 % d. Th.) der Titelverbindung isoliert.

LC-MS (Methode 1): $R_t$ = 0.95 min; MS (ESIpos): m/z = 510 $(M+H)^+$.

## Intermediat L107

N-[(2,5-Dioxo-2,5-dihydro-1H-pyrrol-1-yl)acetyl]-L-alanyl-D-alanyl-L-asparagin

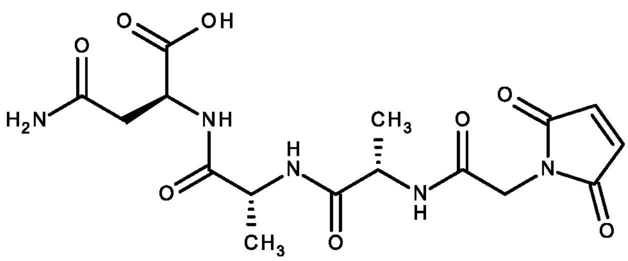

Die Titelverbindung wurde nach klassischen Methoden der Peptidchemie durch HATU-Kupplung von N-[(Benzyloxy) carbonyl]-L-alanyl-D-alanin mit tert-Butyl-L-asparaginat in Gegenwart von N,N-Diisopropylethylamin, anschließender Abspaltung der Z-Schutzgruppe durch Hydrierung in Methanol über 10% Palladium auf Aktivkohle, dann folgender Acylierung mit 1-{2-[(2,5-dioxopyrrolidin-1-yl)oxy]-2-oxoethyl}-1H-pyrrole-2,5-dion in DMF in Gegenwart von N,N-Diisopropylethylamin und abschließender Entschützung der Carboxygruppe mittels Trifluoressigsäure hergestellt.

LC-MS (Methode 1): $R_t$ = 0.18 min; MS (ESIpos): m/z = 412 $(M+H)^+$.

## Intermediat L108

N-[(Benzyloxy)carbonyl]-L-alanyl-D-alanyl-L-asparagin

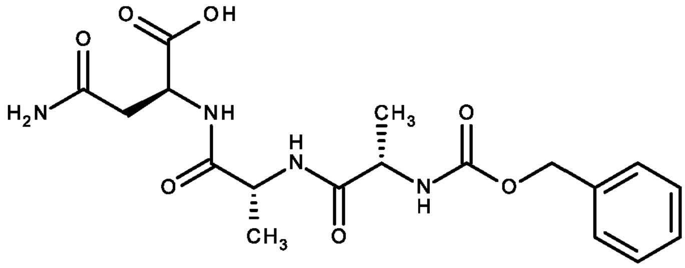

Die Titelverbindung wurde in Analogie zu Beispiel L92 nach klassischen Methoden der Peptidchemie durch HATU-Kupplung von N-[(Benzyloxy) carbonyl]-L-alanyl-D-alanin mit tert-Butyl-L-asparaginat in Gegenwart von N,N-Diisopropylethylamin und anschließender Entschützung der Carboxygruppe mit Trifluoressigsäure hergestellt.

LC-MS (Methode 12): $R_t$ = 0.88 min; MS (ESIpos): m/z = 409 $(M+H)^+$.

## Intermediat L109

N-Isonicotinoyl-L-alanyl-D-alanyl-L-asparagine trifluoroacetate (1:1)

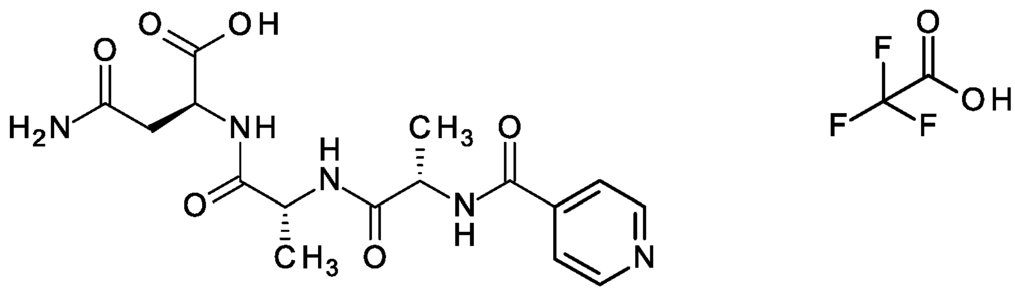

Die Titelverbindung wurde nach klassischen Methoden der Peptidchemie durch HATU-Kupplung von N-[(Benzyloxy) carbonyl]-L-alanyl-D-alanin mit tert-Butyl-L-asparaginat in Gegenwart von N,N-Diisopropylethylamin, anschließender hydrogenolytischer Abspaltung der Z-Schutzgruppe, Kupplung mit Isonicotinsäure in Gegenwart von HATU und N,N-Diisopropylethylamin und schließlich Entschützung der Carboxygruppe mit Trifluoressigsäure hergestellt.

LC-MS (Methode 13): $R_t$ = 0.54 min; MS (ESIpos): m/z = 380 $(M+H)^+$.

## Intermediat L110

N-(pyridin-4-ylacetyl)-L-alanyl-D-alanyl-L-asparagine trifluoroacetate (1:1)

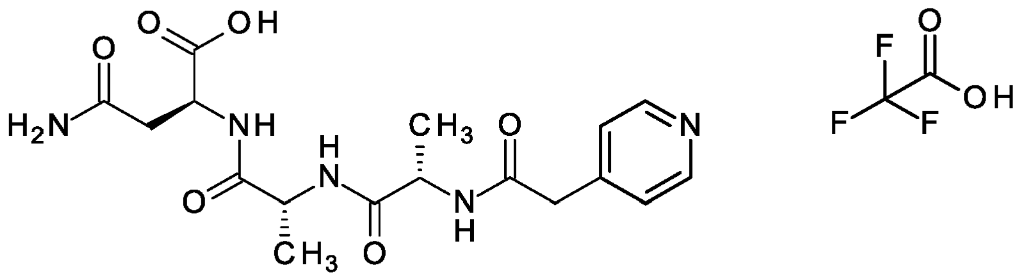

Die Titelverbindung wurde nach klassischen Methoden der Peptidchemie durch HATU-Kupplung von N-[(Benzyloxy) carbonyl]-L-alanyl-D-alanin mit tert-Butyl-L-asparaginat in Gegenwart von N,N-Diisopropylethylamin, anschließender hydrogenolytischer Abspaltung der Z-Schutzgruppe, Kupplung mit Pyridin-4-yl essigsäure Hydrochlorid (1:1) in Gegenwart von HATU und N,N-Diisopropylethylamin und schließlich Entschützung der Carboxygruppe mit Trifluoressigsäure hergestellt.

LC-MS (Methode 13): $R_t$ = 0.5 min; MS (ESIpos): m/z = 394 $(M+H)^+$.

**Intermediat L111**

N-Acetyl-L-alanyl-D-alanyl-L-asparagin

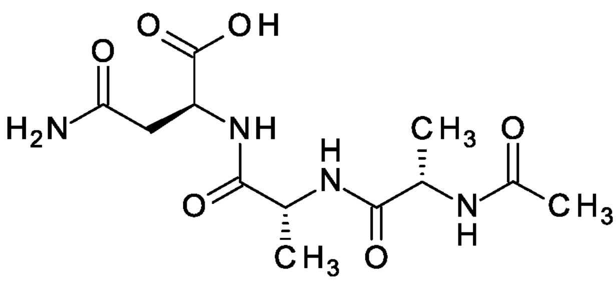

Die Titelverbindung wurde nach klassischen Methoden der Peptidchemie durch HATU-Kupplung von N-[(Benzyloxy) carbonyl]-L-alanyl-D-alanin mit tert-Butyl-L-asparaginat in Gegenwart von N,N-Diisopropylethylamin, anschließender Entschützung der Carboxygruppe mit Trifluoressigsäure, dann folgender hydrogenolytischer Abspaltung der Z-Schutzgruppe und schließlich Kupplung mit 1-Acetoxypyrrolidin-2,5-dion in Gegenwart von N,N-Diisopropyl ethylamin hergestellt.

LC-MS (Methode 1): $R_t$ = 0.17 min; MS (ESIpos): m/z = 317 $(M+H)^+$.

**Intermediat L112**

Trifluoressigsäure --N-(2-aminoethyl)-$N^2$-{[2-(trimethylsilyl)ethoxy]carbonyl}glycinamid (1:1)

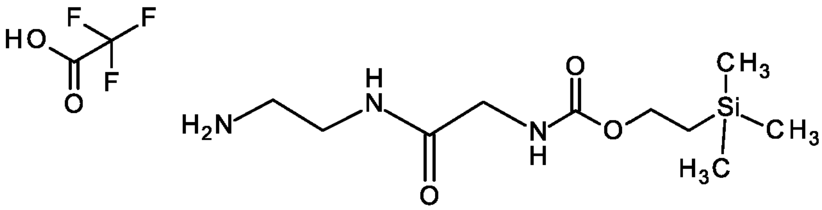

Die Titelverbindung wurde nach klassischen Methoden der Peptidchemie durch Umsetzung von Glycin mit 1-({[2-(Trimethylsilyl)ethoxy]carbonyl}oxy)pyrrolidin-2,5-dion in Gegenwart von N,N-Diisopropylethylamin, anschließender HATU-Kupplung mit Benzyl (2-aminoethyl)carbamat Hydrochlorid (1:1) in Gegenwart von N,N-Diisopropylethylamin und schließlich durch hydrogenolytische Absapltung der Z-Gruppe hergestellt.

LC-MS (Methode 1): $R_t$ = 0.46 min; MS (ESIpos): m/z = 262 $(M+H)^+$.

**Intermediat L113**

(5S,8R,11S)-5,8-Dimethyl-3,6,9-trioxo-11-{2-oxo-2-[2-(trimethylsilyl)ethoxy]ethyl}-1-phenyl-2-oxa-4,7,10-triazadodecan-12-säure

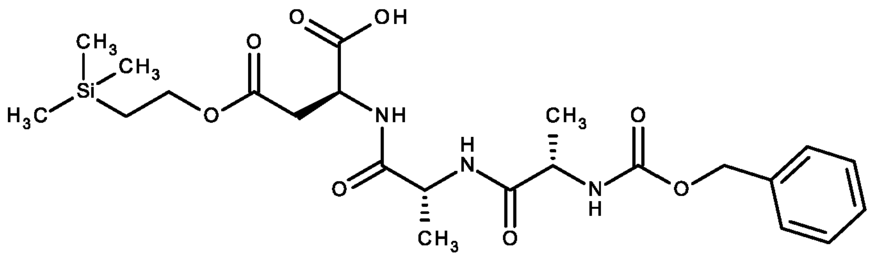

Die Titelverbindung wurde nach klassischen Methoden der Peptidchemie durch HATU-Kupplung von N-[(Benzyloxy)carbonyl]-L-alanyl-D-alanin mit 1-tert-Butyl-4-[2-(trimethylsilyl)ethyl]-L-aspartat in DMF in Gegenwart von N,N-Diisopropylethylamin und anschließender schonender Abspaltung des tert-Butylesters durch Rühren in 7.5%iger Trifluoressigsäure in DCM hergestellt. mittels hergestellt. Aus der erhaltenen Produktmischung wurde die Titelverbindung mittels präparativer HPLC isoliert. LC-MS (Methode 12): $R_t$ = 1.77 min; MS (ESI neg): m/z = 508 $(M-H)^-$.

**Intermediat L114**

N-[(Benzyloxy)carbonyl]-L-alanyl-D-alanin

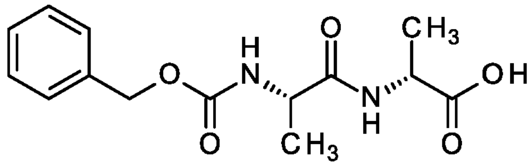

Die Titelverbindung wurde nach klassischen Methoden der Peptidchemie durch HATU-Kupplung von N-carbobenzyloxy-L-alanin mit D-Alanin tert-butylester hydrochlorid in Gegenwart von N,N-Diisopropylethylamin, und schließlich Abspaltung der Butoxycarbonylschutzgruppe mit TFA und DCM.

LC-MS (Methode 1): $R_t$ = 0.61 min; MS (ESIpos): m/z = 295 $(M+H)^+$.

**Intermediat L115**

tert-butyl-L-alanyl-D-alanyl-L-asparaginat

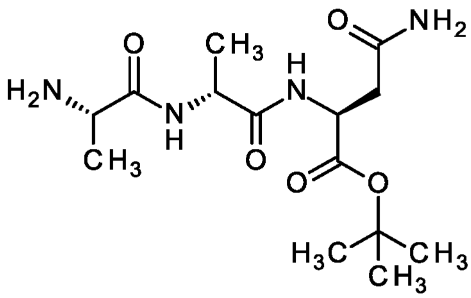

Die Titelverbindung wurde nach klassischen Methoden der Peptidchemie durch HATU-Kupplung von tert-butyl-L-asparaginathydrochlorid mit N-[(Benzyloxy)carbonyl]-L-alanyl-D-alanin (Intermediat L114) in Gegenwart von N,N-Diisopropylethylamin, und schließlich Abspaltung der Z-Schutzgruppe.

LC-MS (Methode 3): $R_t$ = 0.91 min; MS (ESIpos): m/z = 331 $(M+H)^+$.

**Intermediat L116**

N-(38-Oxo-2,5,8,11,14,17,20,23,26,29,32,35-dodecaoxaoctatriacontan-38-yl)-L-alanyl-D-alanyl-L-asparagin

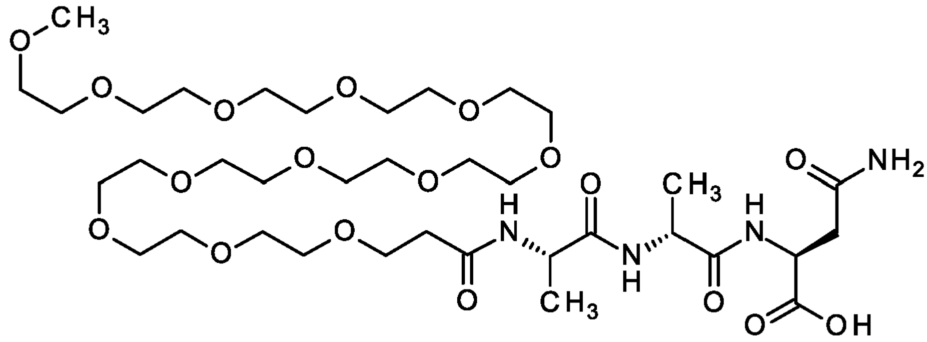

500 mg 2,5,8,11,14,17,20,23,26,29,32,35-Dodecaoxaoctatriacontan-38-säure (849 μmol) und 309 mg tert-butyl-L-alanyl-D-alanyl-L-asparaginat (intermediat L115, 934 μmol) wurden in 5,8 ml DMF vorgelegt, mit 420 mg HATU (1,104 mmol) und 518 μl *N,N*-Diisopropylethylamin (2,97 mmol) versetzt und 40 min bei 0 °C gerührt. Es wurde Wasser (1 mL) und ACN (2 mL) addiert. Die Reaktionslösung wurde mittels präparativer HPLC gereinigt (Eluent: ACN/Wasser + 0.1% TFA, Gradient = 1:9 → 3:2) und anschließender Abspaltung der Butoxycarbonylschutzgruppe mit 3 mL TFA in 3 mL DCM (3 h bei RT gerührt).

LC-MS (Methode 1): $R_t$ = 0.56 min; MS (ESIneg): m/z = 843 $(M-H)^-$.

**Intermediat L117**

tert-Butyl-L-alanyl-D-alaninat

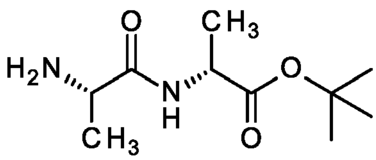

Die Titelverbindung wurde nach klassischen Methoden der Peptidchemie durch HATU-Kupplung von N-carbobenzyloxy-L-alanine mit D-Alanine tert-butylester hydrochloride in Gegenwart von N,N-Diisopropylethylamin, und schließlich Abspaltung der Z-Schutzgruppe.

LC-MS (Methode 7): $R_t$ = 0.29 min; MS (ESIpos): m/z = 217 $(M+H)^+$.

**Intermediat L118**

N-(38-Oxo-2,5,8,11,14,17,20,23,26,29,32,35-dodecaoxaoctatriacontan-38-yl)-L-alanyl-D-alanin

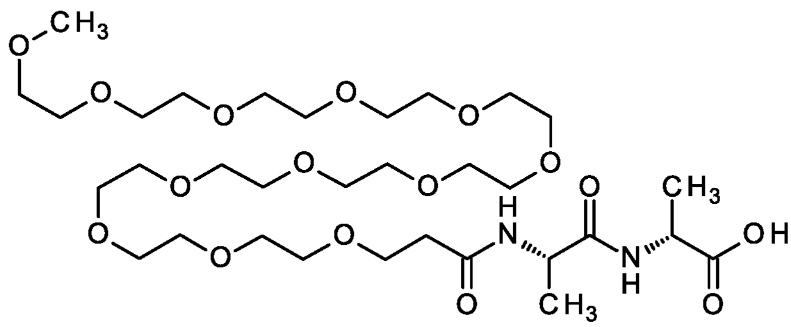

223 mg 2,5,8,11,14,17,20,23,26,29,32,35-Dodecaoxaoctatriacontan-38-säure (379 µmol) und 90,4 mg tert-Butyl-L-alanyl-D-alaninat (intermediat L117, 417 µmol) wurden in 1,2 ml DMF vorgelegt, mit 144 mg HATU (379 mmol) und 198 µl *N,N*-Diisopropylethylamin (1,14 mmol) versetzt und 30 min bei 0 °C gerührt. Es wurde Wasser (1 mL) und ACN (2 mL) addiert. Die Reaktionslösung wurde mittels präparativer HPLC gereinigt (Eluent: ACN/Wasser + 0.1% TFA, Gradient = 1:9 → 3:2) und anschließender Abspaltung der Butoxycarbonylschutzgruppe mit 1,5 mL TFA in 1,5 mL DCM (90 min bei RT gerührt).

LC-MS (Methode 1): $R_t$ = 0.59 min; MS (ESIneg): m/z = 729 $(M-H)^-$.

**Intermediat L119**

N-(Pyridin-4-ylacetyl)-L-alanyl-D-prolyl-L-alpha-asparagin -trifluoressigsäure (1:1)

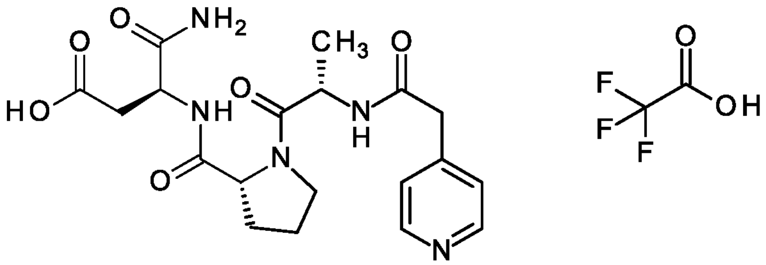

Die Synthese der Titelverbindung erfolgte nach klassischen Methoden der Peptidchemie beginnend mit der HATU-Kupplung von N-[(Benzyloxy)carbonyl]-L-alanin mit tert-Butyl-D-prolinat in Gegenwart von N,N-Diisopropylethylamin und anschließender Entschützung der Carboxygruppe mit Trifluoressigsäure in DCM. Anschließend erfolgte die Kupplung mit tert-Butyl-L-asparaginat in Gegenwart von HATU und N,N-Diisopropylethylamin und dann die hydrogenolytische Abspaltung der Z-Schutzgruppe in DCM/Methanol 1:1 über 10%-igem Palladium auf Aktivkohle bei RT unter Wasserstoff-Normaldruck. Schließlich wurde das erhaltene Intermediat durch Kupplung mit 4-Pyridinessigsäure in Gegenwart von N,N-Diisopropylethylamin und anschließender Entschützung der Carboxygruppe mit Trifluoressigsäure in DCM in die Titelverbindung überführt.

LC-MS (Methode 1): $R_t$ = 0.16 min; MS (ESIpos): m/z = 420 $(M+H)^+$.

**Intermediat L120**

Trifluoressigsäure --N-(pyridin-4-ylacetyl)-D-alanyl-D-alanyl-L-aspartamid (1:1)

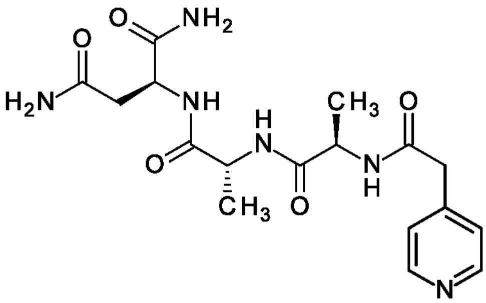

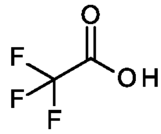

Die Synthese der Titelverbindung erfolgte nach klassischen Methoden der Peptidchemie beginnend mit der HATU-Kupplung von N-[(Benzyloxy)carbonyl]-D-alanin mit tert-Butyl-D-alaninat hydrochlorid (1:1) in Gegenwart von N,N-Diisopropylethylamin und anschließender Entschützung der Carboxygruppe mit Trifluoressigsäure in DCM. Anschließend erfolgte die Kupplung mit tert-Butyl-L-asparaginat in Gegenwart von HATU und N,N-Diisopropylethylamin und dann die hydrogenolytische Abspaltung der Z-Schutzgruppe in DCM/Methanol 1:1 über 10%-igem Palladium auf Aktivkohle bei RT unter Wasserstoff-Normaldruck. Schließlich wurde das erhaltene Intermediat durch Kupplung mit 4-Pyridinessigsäure in Gegenwart von HATU und N,N-Diisopropylethylamin und anschließender Entschützung der Carboxygruppe mit Trifluoressigsäure in DCM in die Titelverbindung überführt.

LC-MS (Methode 1): $R_t$ = 0.16 min; MS (ESIpos): m/z = 394 $(M+H)^+$.

## Intermediat L126

N-(Pyridin-4-ylacetyl)-L-alanyl-D-norvalyl-L-asparagin -trifluoressigsäure Salz

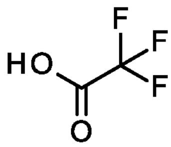

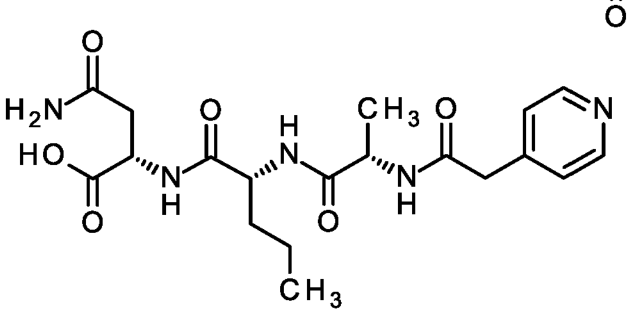

Die Synthese der Titelverbindung erfolgte nach klassischen Methoden der Peptidchemie beginnend mit der HATU-Kupplung von Pyridin-4-ylessigsäurehydrochlorid mit tert-Butyl-L-alaninathydrochlorid (1:1) in Gegenwart von N,N-Diisopropylethylamin und anschließender Entschützung der Carboxygruppe mit Trifluoressigsäure in DCM. Anschließend erfolgte die Kupplung mit 4-Methylbenzolsulfonsäure-benzyl-D-norvalinat in Gegenwart von HATU und N,N-Diisopropylethylamin und dann die Abspaltung der Benzyl-Schutzgruppe mit Lithiumhydroxidmonohydrat in THF/Wasser bei RT unter Wasserstoff-Normaldruck. Schließlich wurde das erhaltene Intermediat durch Kupplung mit tert-Butyl-L-asparaginat in Gegenwart von HATU und N,N-Diisopropylethylamin und anschließender Entschützung der Carboxygruppe mit Trifluoressigsäure in DCM in die Titelverbindung überführt.

LC-MS (Methode 1): $R_t$ = 0.18 min; MS (ESIpos): m/z = 422 $[M+H]^+$.

## Intermediat L127

Trifluoressigsäure -dibenzyl-beta-alanyl-L-glutamat Salz

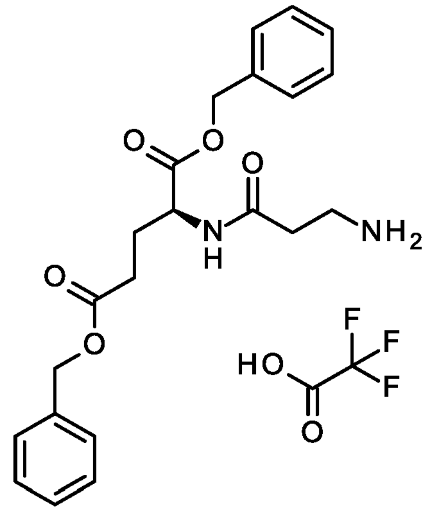

Die Titelverbindung wurde durch Kupplung von 4-Methylbenzolsulfonsäure-dibenzyl-L-glutamat (1:1) mit N-(tert-Butoxycarbonyl)-beta-alanin in Gegenwart von HATU und N,N-Diisopropylethylamin und anschließender Abspaltung der *t*-Butyl-Schutzgruppe mittels Trifluoressigsaeure in Dichloromethan hergestellt.
LC-MS (Methode 1): $R_t$ = 0.72 min; MS (ESIpos): m/z = 399 $[M+H]^+$

<u>**Intermediat L128**</u>

2-(Trimethylsilyl)ethyl-N-(tert-butoxycarbonyl)-L-cysteinat

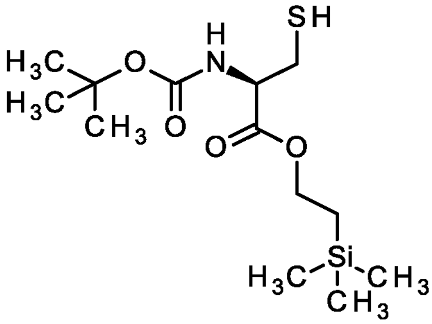

N,N'-Bis(tert-butoxycarbonyl)-L-cystin (7.00 g, 15.9 mmol) wurde in 80 mL Acetonitril gelöst und auf 0°C abgekühlt. Bei dieser Temperatur wurden Pyridin (2.6 ml, 32 mmol), 2-(Trimethylsilyl)ethanol (2.5 ml, 17 mmol) und 1,3-Dicyclohexylcarbodiimid (3.61 g, 17.5 mmol) zugegeben. Die Reaktionsmischung wurde 1h bei 0°C und dann übernacht bei RT gerührt. Die Reaktionsmischung wurde filtriert und der Filterkuchen mit Acetonitril nachgewaschen. Das Lösemittel wurde im Vakuum verdampft. Der Rückstand wurde mittles Biotage Isolera gereinigt (Kieselgel, Ethylacetat/Cyclohexan 1:2) gereinigt. Die Lösemittel wurden im Vakuum verdampft und der Rückstand im Hochvakuum getrocknet. Man erhielt 4.14 g (41 %) der Verbindung Bis[2-(trimethylsilyl)ethyl]-N,N'-bis(tert-butoxycarbonyl)-L-cystinat.
Unter Argon wurde Bis[2-(trimethylsilyl)ethyl]-N,N'-bis(tert-butoxycarbonyl)-L-cystinat (300 mg, 468 μmol) in 1.0 ml Wasser und 3.0 ml DMF vorgelegt. Die Reaktionsmischung wurde mit TCEP (335 mg, 1.17 mmol) versetzt und 1h bei RT nachgerührt. Der Ansatz wurde mit Ethyl Acetat verdünnt und mehrmals mit Wasser und mit gesättigter Natriumchloridlösung gewaschen. Die organische Phase wurde über Magnesiumsulfat getrocknet und anschließend eingeengt. Der Rückstand wurde durch Säulenchromatographie über Biotage/Isolera (SNAP 25g) mit Cyclohexan/Ethylacetat 9:1 als Eluent gereinigt. Die Lösemittel wurden im Vakuum verdampft und der Rückstand im Hochvakuum getrocknet. Man erhielt 106 mg (70 % d. Th.) der titel Verbindung.
LC-MS (Methode 1): $R_t$ = 1.28 min; MS (ESIneg): m/z = 320 $[M-H]^-$

**Intermediat L129**

N-{[2-(2-Methoxyethoxy)ethoxy]acetyl}-L-alanyl-D-alanyl-L-asparagin

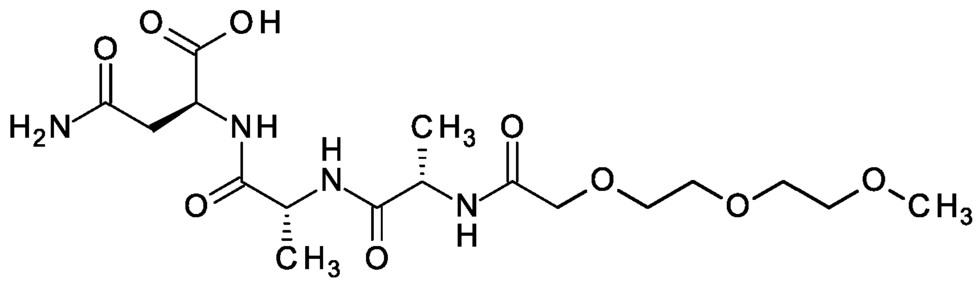

Die Titelverbindung wurde nach klassischen Methoden der Peptidchemie durch HATU-Kupplung in Gegenwart von N,N-Diisopropylethylamin von N-[(Benzyloxy)-carbonyl]-L-alanyl-D-alanin mit tert-Butyl-L-asparaginathydrochlorid, dann folgender hydrogenolytischer Abspaltlung der Z-Schutzgruppe über 10% Palladium/Aktivkohle in Methanol, dann erneuter HATU Kupplung mit [2-(2-Methoxyethoxy)ethoxy]essigsäure und abschließender Spaltung des tert-Butylesters mit TFA hergestellt.
LC-MS (Methode 1): $R_t$ = 0.6 min; MS (ESIpos): m/z = 491 $(M+H)^+$.

**Intermediat L130**

N-(tert-Butoxycarbonyl)-L-alanyl-D-asparaginyl-L-asparagin

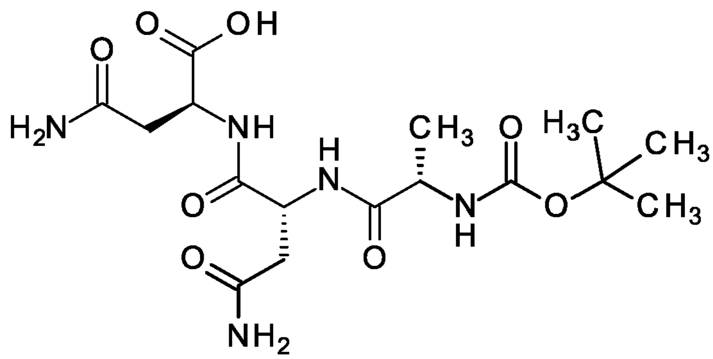

Die Titelverbindung wurde nach klassischen Methoden der Peptidchemie durch HATU-Kupplung in Gegenwart von N,N-Diisopropylethylamin von $N^2$-(tert-Butoxycarbonyl)-D-asparagin mit 4-Nitrobenzyl-L-asparaginathydrobromid (1:1), dann folgender Spaltung der Boc-Schutzgruppe mit TFA, anschließender Kupplung von tert-Butoxycarbonyl)-L-alanin ebenfalls mittels HATU und zum Schluß hydrogenolytischer Abspaltlung des p-Nitrobenzylesters über 10% Palladium/Aktivkohle in Dichlormethan/Methanol 1:1.
LC-MS (Methode 1): $R_t$ = 0.38 min; MS (ESIpos): m/z = 418 $(M+H)^+$.

**Intermediat L131**

Trifluoressigsäure --tert-butyl-N-(2-aminoethyl)-$N^2$-(tert-butoxycarbonyl)-D-alpha-glutaminat (1:1)

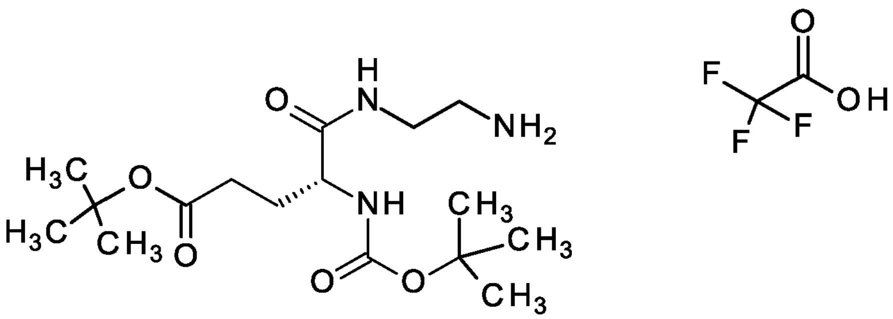

Die Titelverbindung wurde nach klassischen Methoden der Peptidchemie durch HATU-Kupplung in Gegenwart von N,N-Diisopropylethylamin von (2R)-5-tert-Butoxy-2-[(tert-butoxycarbonyl)amino]-5-oxopentansäure mit Benzyl-(2-aminoethyl)carbamathydrochlorid (1:1) und dann folgender hydrogenolytischer Abspaltlung des Benzylesters über 10% Palladium/Aktivkohle in Ethanol hergestellt.
LC-MS (Methode 1): $R_t$ = 0.6 min; MS (ESIpos): m/z = 346 $(M+H)^+$.

**Intermediat L132**

$N^2$-Acetyl-D-asparagin

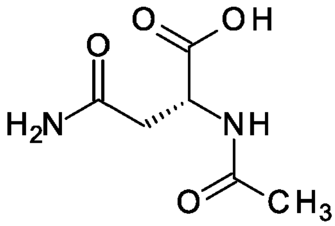

Die Titelverbindung wurde nach klassischen Methoden der Peptidchemie zunächst durch Versterung von $N^2$-[(Benzyloxy)carbonyl]-D-asparagin mit (2-Trimethylsilyl)ethanol mit EDCI/DMAP in DCM, dann folgender hydrogenolytischer Abspaltlung des Benzylesters über 10% Palladium/Aktivkohle in DCM/Methanol 1:1, dann durch Umsetzung mit 1-Acetoxypyrrolidin-2,5-dion in Gegenwart von N,N-Diisopropylethylamin und schließlich durch Abspaltung der Teoc-Schutzgruppe durch 1 h Rühren bei 50°C mit 6 Equivalenten Zinkchlorid in Trifluorethanol hergestellt.
LC-MS (Methode 1): $R_t$ = 0.15 min; MS (ESIneg): m/z = 173 $(M-H)^-$.

**Intermediat L133**

$N^2$-Acetyl-$N^6$-[(benzyloxy)carbonyl]-L-lysin

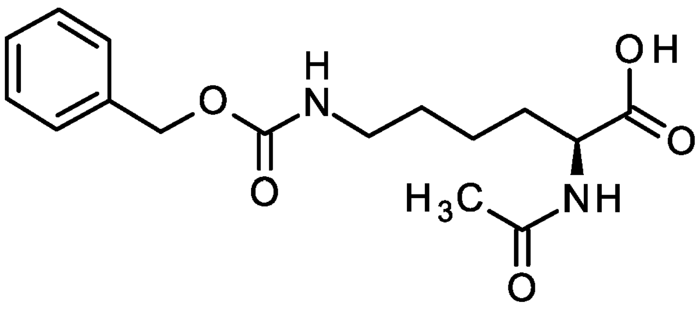

Zu einer Lösung aus N2-Acetyl-L-lysin (947 mg, 5.03 mmol) in THF/Wasser/NaHCO3-Lsg (15 mL/6 mL/12 mL)

wurde Benzylcarbonochloridat (944 mg, 5.53 mmol) gelöst in 5 ml THF zugeben. Der Ansatz wird 2h bei RT gerührt. Und anschließend mit Wasser und Ethylacetate verdünnt. Ein pH von 4 wird mit verdünnter HCl eingestellt. Die organische Phase wurde über MgSO4 getrocknet, abgesaugt und eingeengt. Der Rückstand sowie die gefriergetrocknete wässrige Phase wurden zusammen über präparative HPLC gereinigt.

LC-MS (Methode 1): $R_t$ = 0.65 min; MS (ESIpos): m/z = 323 $[M+H]^+$

**Intermediat L134**

$N^2$-Acetyl-$N^6$-[(benzyloxy)carbonyl]-L-lysyl-L-alanyl-D-alanyl-L-asparagin

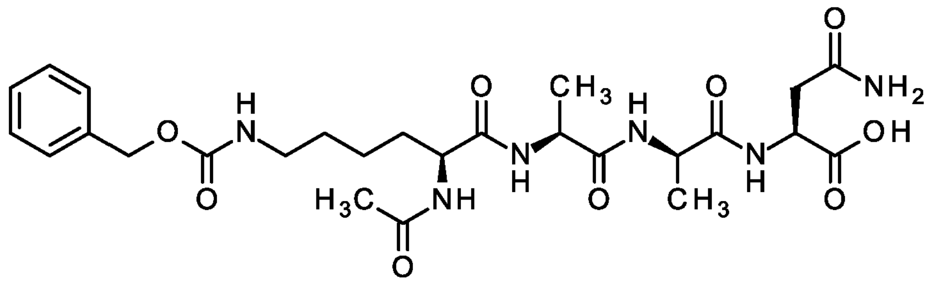

Die Synthese der Titelverbindung erfolgte nach klassischen Methoden der Peptidchemie beginnend mit der HATU-Kupplung von N2-Acetyl-N6-[(benzyloxy)carbonyl]-L-lysin (97.6 mg, 303 μmol) (Intermediat L133) mit tert-Butyl-L-alanyl-D-alanyl-L-asparaginat (Intermediat L115) (100 mg, 303 μmol) in Gegenwart von N,N-Diisopropylethylamin und anschließender Entschützung der Carboxygruppe mit Trifluoressigsäure in DCM.

LC-MS (Methode 1): $R_t$ = 0.58 min; MS (ESIpos): m/z = 579 $[M+H]^+$

**Intermediat L135**

$N^2$-Acetyl-N-(2-aminoethyl)-$N^6$-(tert-butoxycarbonyl)-L-lysinamid

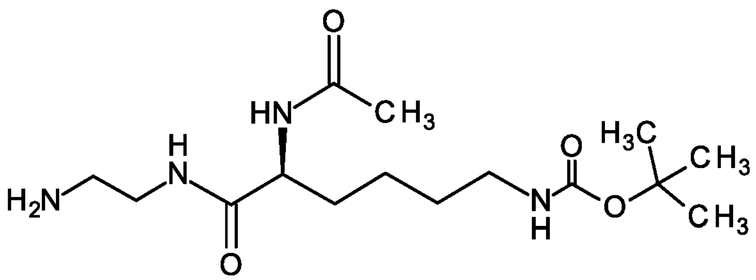

Die Titelverbindung wurde nach klassischen Methoden der Peptidchemie durch HATU-Kupplung von kommerziell erhältlichen of $N^2$-Acetyl-$N^6$-(tert-butoxycarbonyl)-L-lysin mit Benzyl (2-aminoethyl)carbamat hydrochloride (1:1) in Gegenwart von N,N-Diisopropylethylamin und anschließender Abspaltung der Z-Schutzgruppe durch Hydrierung in DCM/Methanol 1:1 über 10% Palladium auf Aktivkohle.

LC-MS (Methode 1): $R_t$ = 0.43 min; MS (ESIpos): m/z = 331 $(M+H)^+$.

**Intermediat F239**

S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[(2,5-dioxo-2,5-dihydro-1H-pyrrol-1-yl)acetyl]-L-cystein -trifluoressigsäure (1:1)

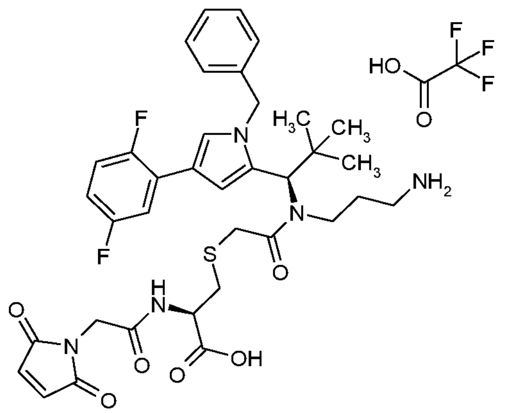

Unter Argon wurden 7.5 mg (0.05 mmol) (2,5-Dioxo-2,5-dihydro-1H-pyrrol-1-yl)essigsäure in 1.5 mL DMF vorgelegt und mit 7.5 mg (0.05 mmol) HOBt, 15.5 mg (0.05 mmol) TBTU und 6.2 mg (0.05 mmol) N,N-Diisopropylethylamin versetzt. Die Reaktionsmischung wurde 10 min bei RT gerührt. Es wurden dann 40.0 mg (0.05 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) gelöst in 1.5 mL DMF und 18.7 mg (0.14 mmol) N,N-Diisopropylethylamin zugegeben und die Reaktionsmischung wurde über Nacht bei RT gerührt. Die Reaktionsmischung wurde direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 11.2 mg (25 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein.

LC-MS (Methode 1): $R_t$ = 1.37 min; MS (ESIpos): m/z = 854 $(M+H)^+$.

10.9 mg (12.8 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein wurden in 2.0 mL Trifluorethanol gelöst und mit 10.4 mg (76.6 µmol) Zinkdichlorid versetzt. Die Reaktionsmischung rührte 4 h bei 50 °C. Die Reaktionsmischung wurde mit 22.4 mg (0.08 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand wurde lyophilisiert. Man erhielt 7.5 mg (65 % d. Th.) der Titelverbindung. LC-MS (Methode 1): $R_t$ = 0.92 min; MS (ESIpos): m/z = 710 $(M+H)^+$.

## Intermediat F255

R/S-(N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl})- homocysteintrifluoressigsäure (1:1)

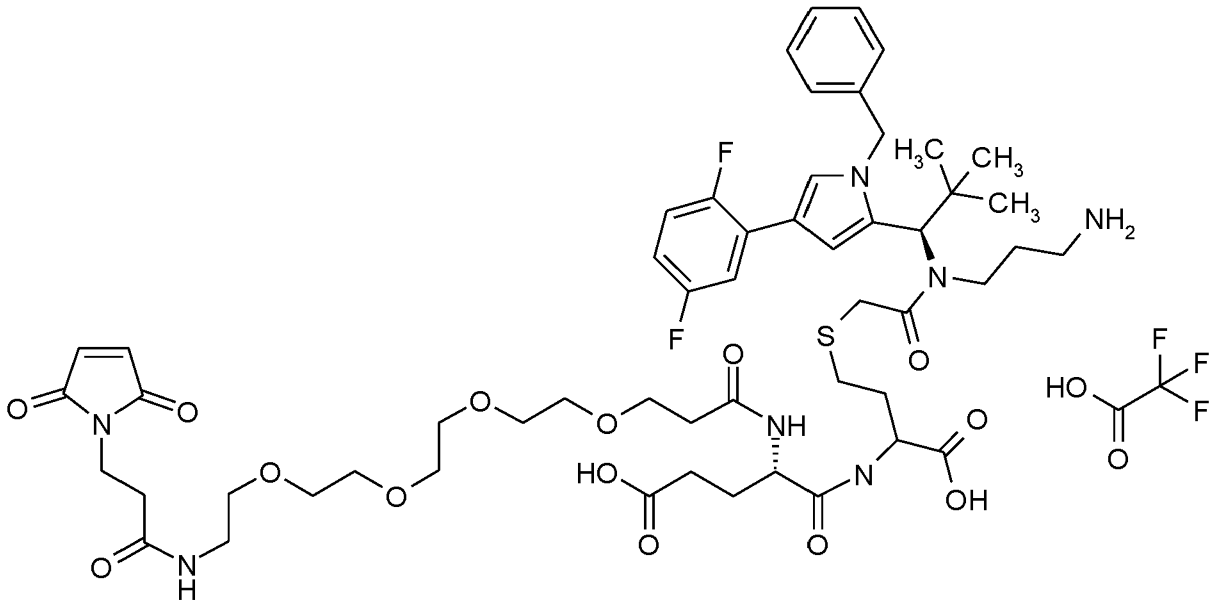

13.1 mg (0.04 mmol) (2S)-5-(Benzyloxy)-2-{[(benzyloxy)carbonyl]amino}-5-oxopentansäure wurden in 1.0 mL DMF vorgelegt und mit 5.4 mg (0.04 mmol) HOBt, 11.4 mg (0.04 mmol) TBTU und 4.6 mg (0.04 mmol) N,N-Diisopropylethylamin versetzt. Die Reaktionsmischung wurde 10 min bei RT gerührt. 30.0 mg (0.04 mmol) R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-homocystein-trifluoressigsäure(1:1) (Intermediat C11) gelöst in 12.9 mg (0.1 mmol) N,N-Diisopropylethylamin und 1 mL DMF wurden dann zugegeben. Die Reaktionsmischung wurde über Nacht bei RT gerührt. Die Reaktionsmischung wurde direkt mittles präp. RP-HPLC (Säule: Reprosil 250x30; 10μ, Fluss: 50 mL/min, MeCN/Wasser/0.1%TFA). Die Lösemittel wurden im Vakuum verdampft und der Rückstand im Hochvakuum getrocknet. Man erhielt 32 mg (73 %) der Verbindung 4-[2-[[(1R)-1-[1-benzyl-4-(2,5-difluorophenyl)pyrrol-2-yl]-2,2-dimethyl-propyl]-[3-(2-trimethylsilylethoxycarbonylamino)propyl]amino]-2-oxo-ethyl]sulfanyl-2-[[(2S)-5-benzyloxy-2-(benzyloxycarbonylamino)-5-oxo-pentanoyl]amino]butansäure.

LC-MS (Methode 1): $R_t$ = 1.53 min; MS (ESIpos): m/z = 1084 $(M+H)^+$.

41.4 mg (0.038 mmol) 4-[2-[[(1R)-1-[1-benzyl-4-(2,5-difluorophenyl)pyrrol-2-yl]-2,2-dimethyl-propyl]-[3-(2-trimethylsilylethoxycarbonylamino)propyl]amino]-2-oxo-ethyl]sulfanyl-2-[[(2S)-5-benzyloxy-2-(benzyloxycarbonylamino)-5-oxo-pentanoyl]amino]butansäure wird in 10 mL Ethanol, mit 4.2 mg Pd/C versetzt und mit Normaldruck hydriert. Die Reaktionsmischung wurde über einen Pappfilter filtriert und der Filterkuchen mit Ethanol nachgewaschen. Das Lösemittel wurde im Vakuum ohne Erwärmen verdampft. Der Rückstand wurde mittles präp. RP-HPLC (Säule: Reprosil 250x40; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 21.1 mg (56 %) der Verbindung R/S-(L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein -trifluoressigsäure (1:1).

LC-MS (Methode 1): $R_t$ = 1.11 min; MS (ESIpos): m/z = 860 $(M+H)^+$.

20.4 mg (20.94 μmol) R/S-(L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein -trifluoressigsäure (1:1) wurden zusammen mit 11.8 mg (23.04 μmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxa-pentadec-1-yl}propanamid in 1.0 mL DMF vorgelegt und mit 4.2 mg (41.88 μmol) 4-Methyl-morpholin versetzt. Die Reaktionsmischung wurde über Nacht bei RT gerührt und dann mit 3.1 mg (0.05 mmol) Essigsäure versetzt. Die Reaktionsmischung wurde direkt mittles präp. RP-HPLC (Säule: Reprosil 250x30; 10μ, Fluss: 50 mL/min, MeCN/Wasser/0.1% TFA) gereinigt. Die Lösemittel wurden im Vakuum verdampft und der Rückstand im Hochvakuum getrocknet. Man erhielt 9.5 mg (36 %) der Verbindung R/S-(N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein.

LC-MS (Methode 1): $R_t$ = 1.66 min; MS (ESIpos): m/z = 1259 $(M+H)^+$.

9.4 mg (7.47 μmol) R/S-(N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein wurden in 1.5 mL Trifluorethanol gelöst und mit 6.1 mg (44.81 μmol) Zinkdichlorid versetzt. Die Reaktionsmischung rührte 3 h bei 50 °C. Die Reaktionsmischung wurde

mit 13.1 mg (0.05 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 6.9 mg (75 %) der Titelverbindung LC-MS (Methode 1): $R_t$ = 0.87 min; MS (ESIpos): m/z = 1114 $(M+H)^+$.

## Intermediat F256

Trifluoressigsäure --N-{(2S)-2-amino-4-[{(1R)-1-[1-benzyl-4-(2,5-difluorphenyl)-1H-pyrrol-2-yl]-2,2-dimethylpropyl}(glycoloyl)amino]butyl}-N'-[2-(2-{[(2,5-dioxo-2,5-dihydro-1H-pyrrol-1-yl)acetyl]amino}ethoxy)ethyl]succinamid (1:1)

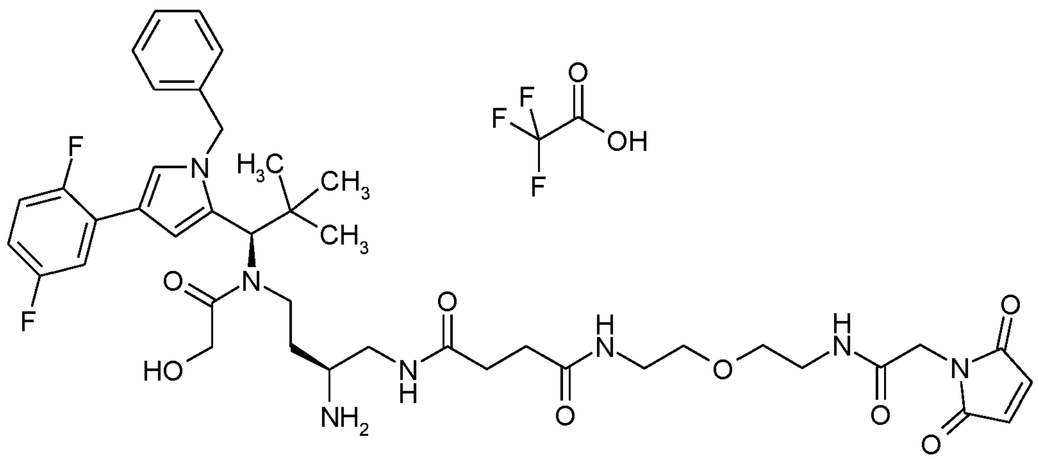

Die Titelverbindung wurde durch Kupplung von 10 mg (0.014 mmol) von Intermediat C65 und 9.6 mg (0.027 mmol) Trifluoressigsäure--N-[2-(2-aminoethoxy)ethyl]-2-(2,5-dioxo-2,5-dihydro-1H-pyrrol-1-yl)acet amid (1:1) in Gegenwart von HATU und N,N-Diisopropylethylamin und anschließender Entschützung mit Zinkchlorid in Trifluorethanol wie in Intermediat F119 beschrieben hergestellt. Nach Reinigung durch präparative HPLC wurden 8 mg (64% d. Th. über 2 Stufen) der Titelverbindung erhalten.

LC-MS (Methode 1): $R_t$ = 0.84 min; MS (ESIpos): m/z = 822 $(M+H)^+$.

## Intermediat F257

R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[18-(2,5-dioxo-2,5-dihydro-1H-pyrrol-1-yl)-17-oxo-4,7,10,13-tetraoxa-16-azaoctadecan-1-oyl]-L-cystein-trifluoressigsäure (1:1)

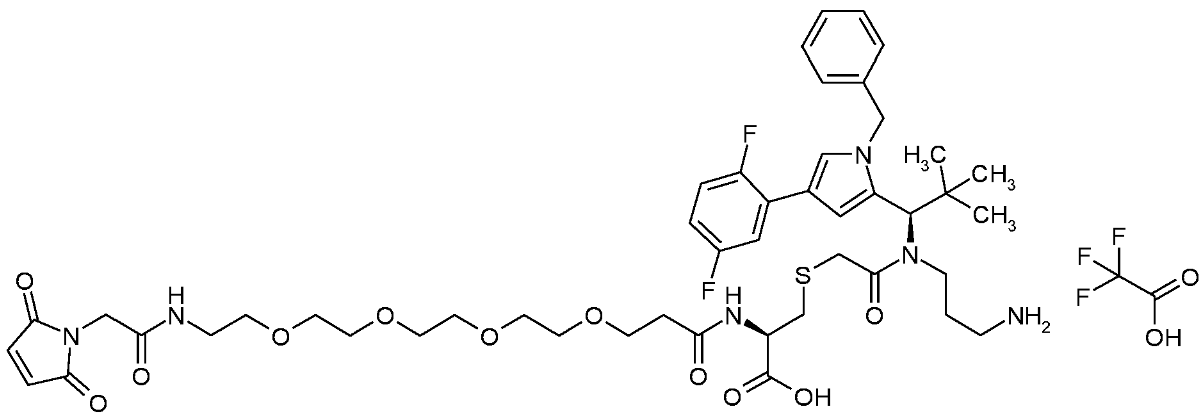

50.0 mg (0.06 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteintrifluoressigsäure (1:1) (Intermediat C71) und 29 mg (0.07 mmol) 3-[2-[2-[2-[2-[[2-(2,5-dioxopyrrol-1-yl)acetyl]amino]ethoxy]ethoxy]ethoxy]ethoxy]propansäure (Intermediat L74) wurden in 3.0 mL DMF gelöst und mit 27.3 mg (0.07 mmol) HATU und 23.3 mg (0.18 mmol) N,N-Diisopropylethylamin versetzt. Die Reaktionsmischung wurde 2 Stunde bei RT gerührt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 17.4 mg (26 %) der Verbindung R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[18-(2,5-dioxo-2,5-dihydro-1H-pyrrol-1-yl)-17-oxo-4,7,10,13-tetraoxa-16-azaoctadecan-1-oyl]-L-cystein.

LC-MS (Methode 6): $R_t$ = 1.34 min; MS (ESIpos): m/z = 1101 $(M+H)^+$. 17 mg (0.02 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[18-(2,5-dioxo-2,5-dihydro-1H-pyrrol-1-yl)-17-oxo-4,7,10,13-tetraoxa-16-azaoctadecan-1-oyl]-L-cystein wurden in 1.0 mL Trifluorethanol gelöst und mit 6.3 mg (0.05 mmol) Zinkdichlorid versetzt. Die Reaktionsmischung rührte über Nacht bei 50 °C. Die Reaktionsmischung wurde mit 13.5 mg (0.05 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.6 mg (46 %) der Titelverbindung.

LC-MS (Methode 1): $R_t$ = 0.91 min; MS (ESIpos): m/z = 957 $(M+H)^+$.

**Intermediat F258**

Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-[3-({2-[(bromacetyl)amino]ethyl}amino)-3-oxopropyl]butanamid (1:1)

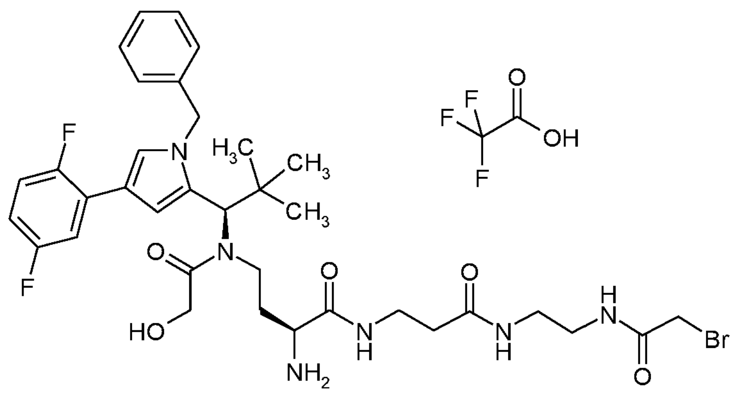

Die Titelverbindung wurde durch Kupplung von Intermediat C58 mit Trifluoressigsäurebenzyl-[2-(beta-alanylamino)ethyl]carbamat (1:1) mittels HATU, anschließender Hydrogenolyse, dann folgender Kupplung mit 1-(2-Bromacetoxy)pyrrolidin-2,5-dion und schließlich durch Entschützung mit Zinkchlorid hergestellt.

LC-MS (Methode 1): $R_t$ = 0.86 min; MS (ESIpos): m/z = 747 und 749$(M+H)^+$.

**Intermediat F259**

N-{(2S)-2-Amino-4-[{(1R)-1-[1-benzyl-4-(2,5-difluorphenyl)-1H-pyrrol-2-yl]-2,2-dimethyl propyl}(glycoloyl)amino]butanoyl}-3-{[N-(bromacetyl)glycyl]amino}-D-alanin-trifluoressigsäure (1:1)

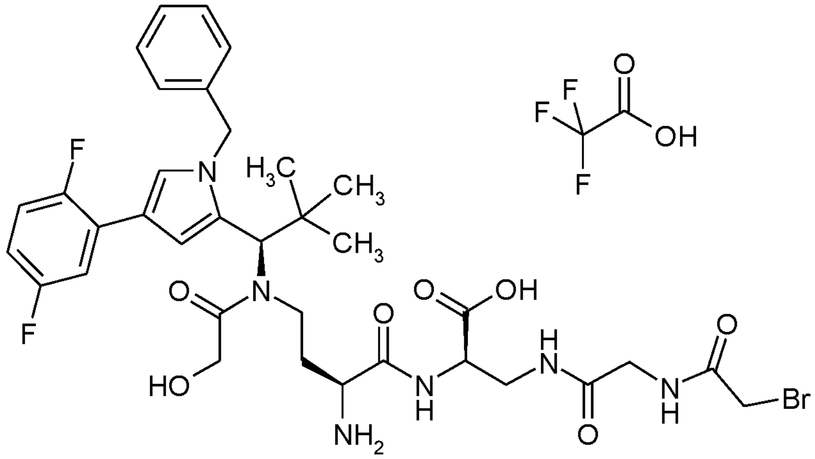

75mg (0.114 mmol) von Intermediat C58 wurden in 12.5 ml DMF aufgenommen und mit 78 mg (0.171 mmol) von Intermediat L75 in Gegenwart von 65 mg (0.11 mmol) HATU und 79 μL *N,N*-Diisopropylethylamin gekuppelt. Nach Reinigung durch präparative HPLC wurde das Intermediat in 20 ml Ethanol aufgenommen und über 10%-igem Palladium auf Aktivkohle 1 h bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1:1 wurden 63 mg (64% d.Th. über 2 Stufen) von 2-(Trimethylsilyl)ethyl-3-amino-N-[(2S)-4-[{(1R)-1-[1-benzyl-4-(2,5-difluorphenyl)-1H-pyrrol-2-yl]-2,2-dimethyl-propyl}(glycoloyl)amino]-2-({[2-(trimethylsilyl)ethoxy]carbonyl}amino) butanoyl]-D-alaninat erhalten.

LC-MS (Methode 1): $R_t$ = 1.16 min; MS (EIpos): m/z = 844 $[M+H]^+$.

40 mg (0.047 mmol) von diesem Intermediat wurden dann wie oben beschrieben mit N-[(Benzyloxy)carbonyl]glycin in Gegenwart von HATU gekuppelt und anschließend erneut hydrogenolytisch entschützt.

Die Titelverbindung wurde dann durch Kupplung von 10 mg (0.012 mmol) dieses Intermediats mit 7.7 mg (0.032 mmol) von kommerziell erhältlichem 1-(2-Bromacetoxy)pyrrolidin-2,5-dion in Gegenwart von 4 μl N,N-Diisopropylethylamin und anschließender Entschützung mit Zinkchlorid in Trifluorethanol wie in Intermediat F119 beschrieben hergestellt. Nach Reinigung durch präparative HPLC wurden 1.3 mg der Titelverbindung erhalten.

LC-MS (Methode 1): $R_t$ = 0.83 min; MS (ESIpos): m/z = 777 und 779 $(M+H)^+$.

## Intermediat F260

$N^6$-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-$N^2$-{N-[(2,5-dioxo-2,5-dihydro-1H-pyrrol-1-yl)acetyl]-L-valyl-L-alanyl}-L-lysin -trifluoressigsäure (1:1)

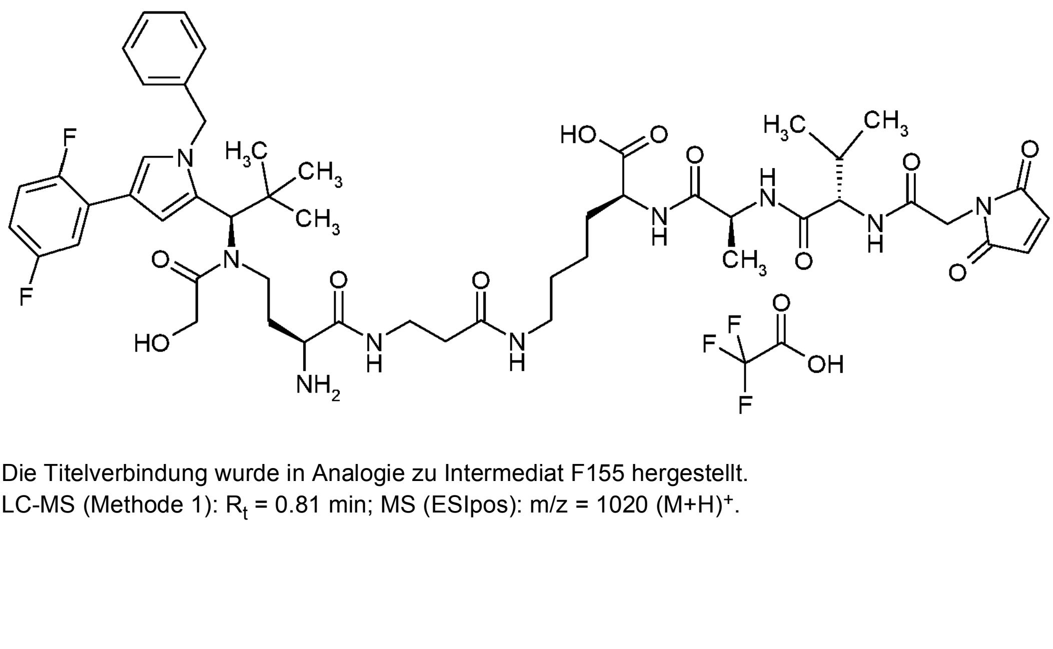

Die Titelverbindung wurde in Analogie zu Intermediat F155 hergestellt.

LC-MS (Methode 1): $R_t$ = 0.81 min; MS (ESIpos): m/z = 1020 $(M+H)^+$.

**Intermediat F261**

Trifluoressigsäure -(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{2-[(bromacetyl)amino]ethoxy}ethyl)-butanamid (1:1)

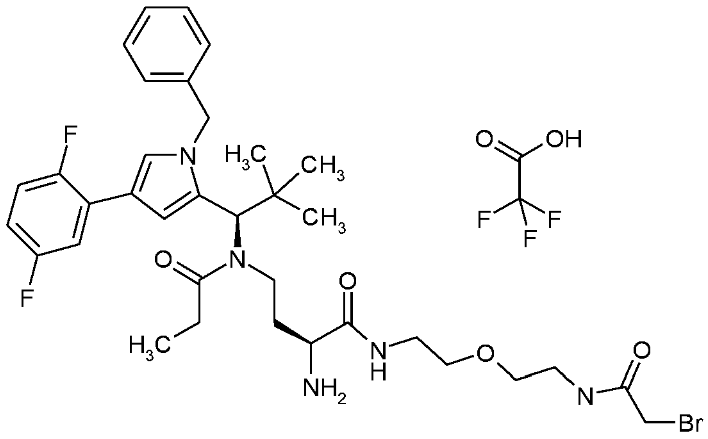

Die Titelverbindung wurde durch Kupplung von 20 mg (0.03 mmol) Intermediat C58 mit 25.8 mg (0.061 mmol) Intermediat L77 in Gegenwart von HATU und anschließende Entschützung mit Zinkchlorid hergestellt. Es wurden 11.9 mg (47% d.Th. über 2 Stufen) der Titelverbindung erhalten.

LC-MS (Methode 1): $R_t$ = 0.84 min; MS (ESIpos): m/z = 722 und 720 $(M+H)^+$.

**Intermediat F262**

S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-{3-[2-(2-{[3-(2,5-dioxo-2,5-dihydro-1H-pyrrol-1-yl)propanoyl]amino}ethoxy)ethoxy]propanoyl}-L-cystein -trifluoressigsäure (1:1)

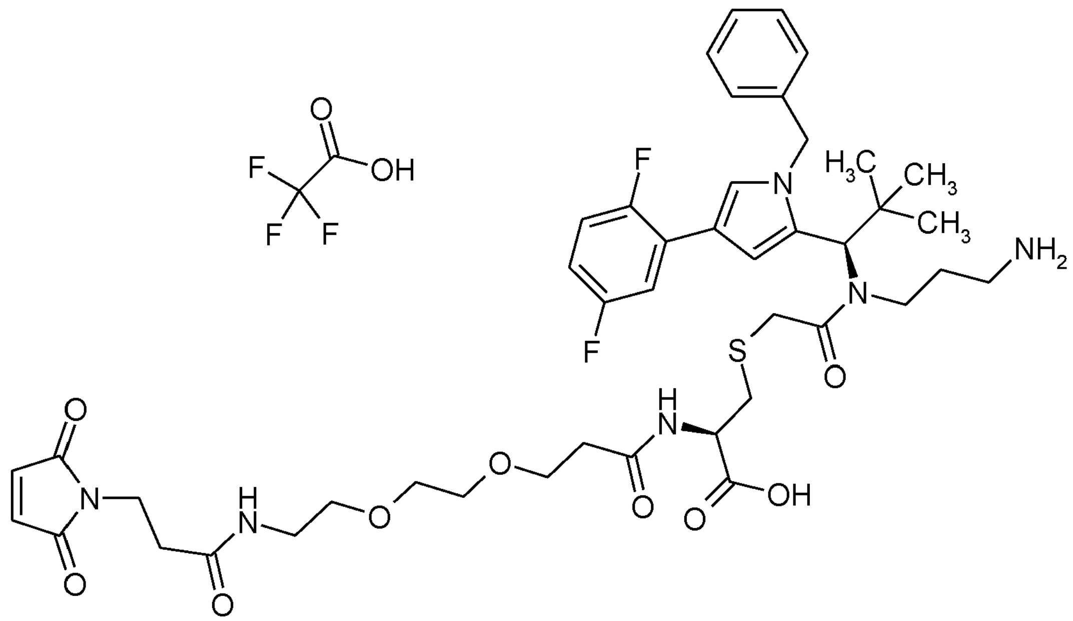

30 mg (36 µmol) S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1) (Intermediat C71) wurden zusammen mit 16,9 mg (40 µmol)

3-(2,5-Dioxo-2,5-dihydro-1H-pyrrol-1-yl)-N-[2-(2-{3-[(2,5-dioxopyrrolidin-1-yl)oxy]-3-oxopropoxy}ethoxy)ethyl]propanamid in 1.5 mL DMF vorgelegt und mit 10,9 mg (108 μmol) 4-Methylmorpholin versetzt. Die Reaktionsmischung wurde über Nacht bei RT gerührt und dann mit 7.58 mg (0.13 mmol) Essigsäure versetzt. Die Reaktionsmischung wurde direkt mittles präp. RP-HPLC (Säule: Reprosil 250x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 33,4 mg (80 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{[3-(2,5-dioxo-2,5-dihydro-1H-pyrrol-1-yl)propanoyl]amino}ethoxy)ethoxy]propanoyl}-L-cystein.

LC-MS (Methode 1): $R_t$ = 1.34 min; MS (ESIpos): m/z = 1027 $(M+H)^+$.

32.8 mg (32 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{[3-(2,5-dioxo-2,5-dihydro-1H-pyrrol-1-yl)propanoyl]amino}ethoxy)ethoxy]propanoyl}-L-cystein wurden in 3.0 mL Trifluorethanol gelöst und mit 26.1 mg (192 μmol) Zinkdichlorid versetzt. Die Reaktionsmischung rührte 2 h bei 50 °C Die Reaktionsmischung wurde mit 56.0 mg (0.192 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand wurde lyophilisiert. Man erhielt 22.9 mg (71 % d. Th.) der Titelverbindung.

LC-MS (Methode 1): $R_t$ = 0.88 min; MS (ESIpos): m/z = 883 $(M+H)^+$.

## Intermediat F263

N-[(2,5-Dioxo-2,5-dihydro-1H-pyrrol-1-yl)acetyl]-beta-alanyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

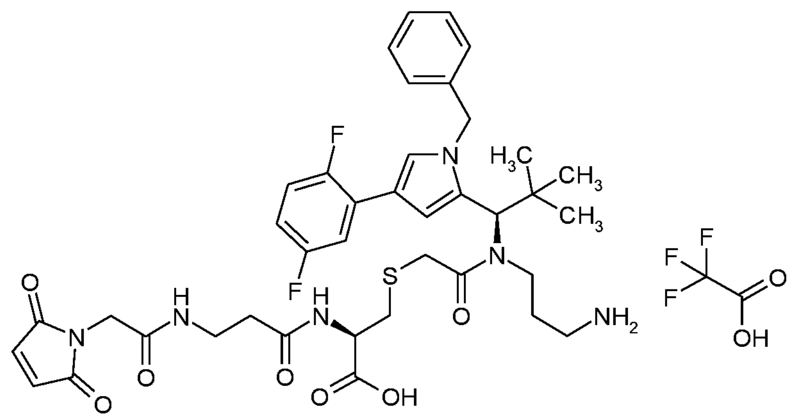

30.0 mg (0.036 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteintrifluoressigsäure (1:1) (Intermediat C71) und 9.8 mg (0.04 mmol) N-[(2,5-Dioxo-2,5-dihydro-1H-pyrrol-1-yl)acetyl]-beta-alanin (Intermediat L78) wurden in 1.0 mL DMF gelöst und mit 16.4 mg (0.04 mmol) HATU und 14.0 mg (0.11 mmol) N,N-Diisopropylethylamin versetzt. Die Reaktionsmischung wurde 2 Stunde bei RT gerührt. Die Reaktionsmischung wurde direkt mittels präp. RP-HPLC gereinigt (Säule: Reprosil 125x30; 10μ, Fluss: 50 mL/min, MeCN/Wasser; 0.1 % TFA). Die Lösemittel wurden im Vakuum verdampft und der Rückstand im Hochvakuum getrocknet. Man erhielt 4.2 mg (13 %) der Verbindung N-[(2,5-Dioxo-2,5-dihydro-1H-pyrrol-1-yl)acetyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein.

LC-MS (Methode 6): $R_t$ = 1.31 min; MS (ESIpos): m/z = 925 $(M+H)^+$.

11.3 mg (0.011 mmol) N-[(2,5-Dioxo-2,5-dihydro-1H-pyrrol-1-yl)acetyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 5.0 mg (0.04 mmol) Zinkdichlorid versetzt. Die Reaktionsmischung rührte 2 Stunde bei 50 °C. Die Reaktionsmischung wurde mit 10.7 mg (0.04 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 4.4 mg (40 %) der Titelverbindung.

LC-MS (Methode 1): $R_t$ = 0.91 min; MS (ESIpos): m/z = 781 $(M+H)^+$.

## Intermediat F264

N-[6-(2,5-Dioxo-2,5-dihydro-1H-pyrrol-1-yl)hexanoyl]-beta-alanyl-S-{2-[(3-aminopropyl) {(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

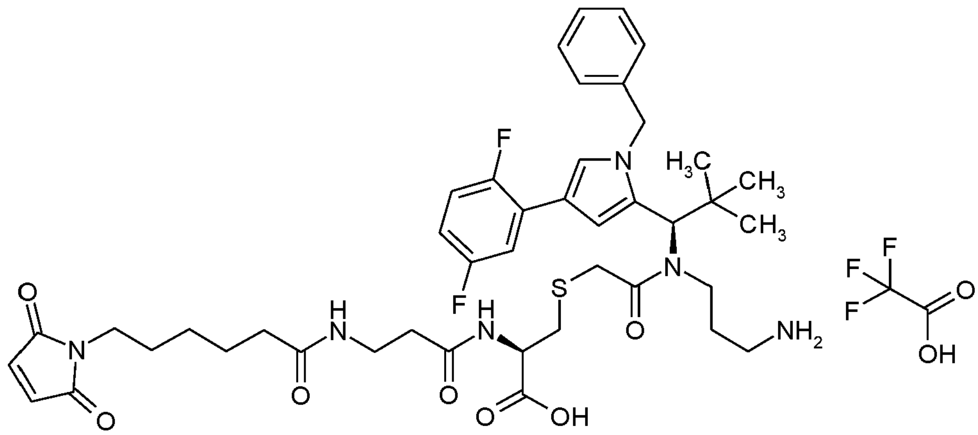

30.0 mg (0.036 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteintrifluoressigsäure (1:1) (Intermediat C71) und 12.2 mg (0.04 mmol) N-[6-(2,5-Dioxo-2,5-dihydro-1H-pyrrol-1-yl)hexanoyl]-beta-alanin (Intermediat L79) wurden in 1.0 mL DMF gelöst und mit 16.4 mg (0.04 mmol) HATU und 14.0 mg (0.11 mmol) N,N-Diisopropylethylamin versetzt. Die Reaktionsmischung wurde 2 Stunde bei RT gerührt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 8.9 mg (24 %) der Verbindung N-[6-(2,5-Dioxo-2,5-dihydro-1H-pyrrol-1-yl)hexanoyl]-beta-alanyl-S-(11-{(1R)-1-[1-benzyl-4-(2,5-difluorphenyl)-1H-pyrrol-2-yl]-2,2-dimethylpropyl}-2,2-dimethyl-6, 12-dioxo-5-oxa-7,11-diaza-2-silatridecan-13-yl)-L-cystein.

LC-MS (Methode 6): $R_t$ = 1.38 min; MS (ESIpos): m/z = 981 $(M+H)^+$.

15.3 mg (0.015 mmol) N-[6-(2,5-Dioxo-2,5-dihydro-1H-pyrrol-1-yl)hexanoyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 6.3 mg (0.045 mmol) Zinkdichlorid versetzt. Die Reaktionsmischung rührte 2 Stunde bei 50 °C. Die Reaktionsmischung wurde mit 13.5 mg (0.045 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 9.1 mg (62 %) der Titelverbindung.

LC-MS (Methode 1): $R_t$ = 0.92 min; MS (ESIpos): m/z = 837 $(M+H)^+$.

## Intermediat F265

Trifluoressigsäure --N-(3-aminopropyl)-N-{(1R)-1-[1-benzyl-4-(2,5-difluorphenyl)-1H-pyrrol-2-yl]-2,2-dimethylpropyl}-22-(2,5-dioxo-2,5-dihydro-1H-pyrrol-1-yl)-6,17-dioxo-10,13-dioxa-3-thia-7,16-diazadocosan-1-amid (1:1)

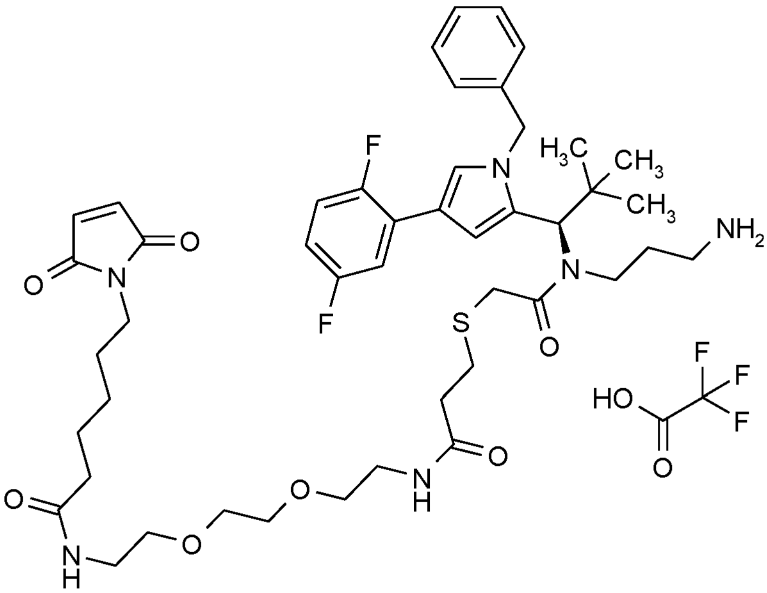

30.0 mg (42.7 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) und 25.3 mg (55.6 µmol) Trifluoressigsäure N-{2-[2-(2-aminoethoxy)ethoxy]ethyl}-6-(2,5-dioxo-2,5-dihydro-1H-pyrrol-1-yl)hexanamid (1:1) (Intermediat L82) wurden in 1.9 mL Acetonitril vorgelegt und mit 60 µl (340 µmol) N,N-Diisopropylethylamin und 33 µl (56 µmol) 2,4,6-Tripropyl-1,3,5,2,4,6-trioxatriphosphinan-2,4,6-trioxid 50 % in Ethylacetat versetzt. Die Reaktionsmischung wurde über Nacht bei RT nachgerührt. Es wurde Wasser (2.0 mL) zugegeben und die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 26.7 mg (60 % d. Th.) der Verbindung 2-(Trimethylsilyl)ethyl-[4-{(1R)-1-[1-benzyl-4-(2,5-difluorphenyl)-1H-pyrrol-2-yl]-2,2-dimethylpropyl}-26-(2,5-dioxo-2,5-dihydro-1H-pyrrol-1-yl)-5,10,21-trioxo-14,17-dioxa-7-thia-4,11,20-triazahexacos-1-yl]carbamat.

LC-MS (Methode 1): $R_t$ = 1.40 min; MS (ESIpos): m/z = 1025 $(M+H)^+$.

25.3 mg (24.7 µmol) 2-(Trimethylsilyl)ethyl-[4-{(1R)-1-[1-benzyl-4-(2,5-difluorphenyl)-1H-pyrrol-2-yl]-2,2-dimethylpropyl}-26-(2,5-dioxo-2,5-dihydro-1H-pyrrol-1-yl)-5,10,21-trioxo-14,17-dioxa-7-thia-4,11,20-triazahexacos-1-yl]carbamat wurden in 2.0 mL Trifluorethanol gelöst und mit 20.2 mg (148 µmol) Zinkdichlorid versetzt. Die Reaktionsmischung rührte 1 h bei 50 °C. Die Reaktionsmischung wurde mit 43.3 mg (148 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 23.4 mg (95 % d. Th.) der Titel Verbindung.

LC-MS (Methode 1): $R_t$ = 0.89 min; MS (ESIpos): m/z = 881 $(M+H)^+$.

## Intermediat F266

Trifluoressigsäure N-(3-aminopropyl)-N-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,13-dioxo-6,9-dioxa-16-thia-3,12-diazaoctadecan-18-amid (1:1)

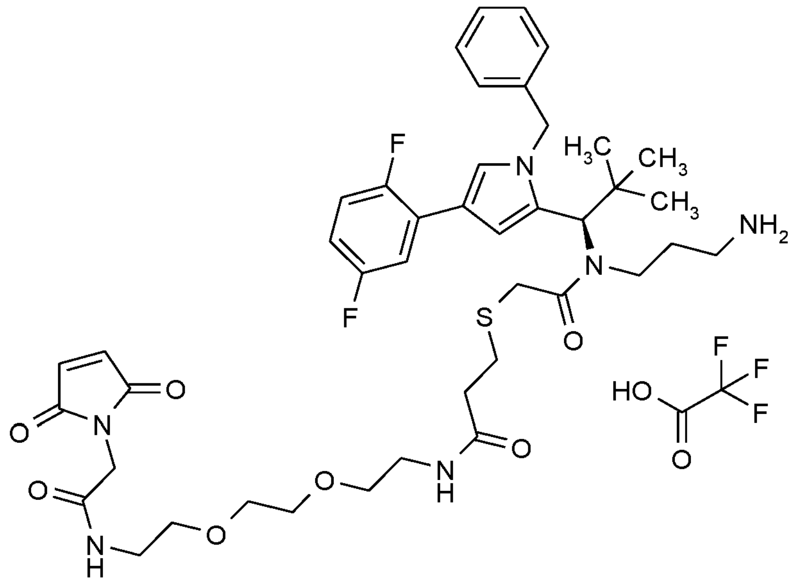

30.0 mg (0.043 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden zusammen mit 22.2 mg (0.056 mmol) Trifluoressigsäure N-{2-[2-(2-aminoethoxy)ethoxy]ethyl}-2-(2,5-dioxo-2,5-dihydro-1H-pyrrol-1-yl)acetamid (1:1) (Intermediat L83) in 1.9 mL Acetonitril vorgelegt. Dann wurden 60 µl (0.34 mmol) N,N-Diisopropylethylamin zugegeben und 33 µl (0.056 mmol) T3P (50 % in Ethylacetat) zugetropft. Die Reaktionsmischung wurde über Nacht bei RT gerührt. Es wurde Wasser (2.0 mL) zugegeben. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 20.5 mg (49 % d. Th.) der Verbindung 2-(Trimethylsilyl)ethyl-[19-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,13,18-trioxo-6,9-dioxa-16-thia-3,12,19-triazadocosan-22-yl]carbamat. LC-MS (Methode 1): $R_t$ = 1.38 min; MS (ESIpos): m/z = 969 $(M+H)^+$.

19.1 mg (19.7 µmol) 2-(Trimethylsilyl)ethyl-[19-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,13,18-trioxo-6,9-dioxa-16-thia-3,12,19-triazadocosan-22-yl]carbamat wurden in 2.0 mL Trifluorethanol gelöst und mit 16.1 mg (118 µmol) Zinkdichlorid versetzt. Die Reaktionsmischung rührte 1 h bei 50 °C. Die Reaktionsmischung wurde mit 34.6 mg (118 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 13.9 mg (75 % d. Th.) der Titel Verbindung.

LC-MS (Methode 1): $R_t$ = 0.86 min; MS (ESIpos): m/z = 825 $(M+H)^+$.

## Intermediat F267

S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[1-(2,5-dioxo-2,5-dihydro-1H-pyrrol-1-yl)-2,18-dioxo-6,9,12,15-tetraoxa-3-azaoctadecan-18-yl]-L-cysteinyl-beta-alanin -trifluoressigsäure (1:1)

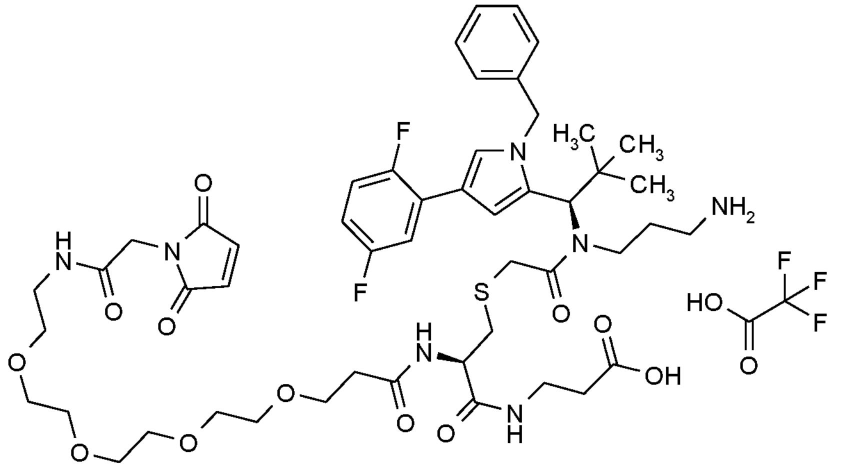

Unter Argon wurden 13.4 mg (33.3 μmol) 1-(2,5-Dioxo-2,5-dihydro-1H-pyrrol-1-yl)-2-oxo-6,9,12,15-tetraoxa-3-azaoctadecan-18-säure (Intermediat L74) in 1.0 ml DMF vorgelegt, mit 9.3 μl (54.4 μmol) N,N-Diisopropylethylamin und 12.6 mg (33.3 μmol) HATU versetzt. Die Reaktionsmischung wurde 10 min bei RT gerührt. 25.0 mg (27.7 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin -trifluoressigsäure (1:1) (siehe Synthese Intermediat F216) gelöst in 4,7 μl (27.7 μmol) N,N-Diisopropylethylamin und 1.0 mL DMF wurden dann zugegeben. Die Reaktionsmischung wurde 90 Minuten bei RT gerührt. Die Reaktionsmischung wurde direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 6.90 mg (19 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[1-(2,5-dioxo-2,5-dihydro-1H-pyrrol-1-yl)-2,18-dioxo-6,9,12,15-tetraoxa-3-azaoctadecan-18-yl]-L-cysteinyl-beta-alanin.

LC-MS (Methode 5): $R_t$ = 4.44 min; MS (ESIpos): m/z = 1172 $(M+H)^+$.

6.70 mg (5.71 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[1-(2,5-dioxo-2,5-dihydro-1H-pyrrol-1-yl)-2,18-dioxo-6,9,12,15-tetraoxa-3-azaoctadecan-18-yl]-L-cysteinyl-beta-alanin wurden in 1.0 mL Trifluorethanol gelöst und mit 4.67 mg (34.3 μmol) Zinkdichlorid versetzt. Die Reaktionsmischung rührte 1 h bei 50 °C. Die Reaktionsmischung wurde mit 10 mg (34.3 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 4.4 mg (67 % d. Th.) der Titel Verbindung.

LC-MS (Methode 1): $R_t$ = 0.85 min; MS (ESIpos): m/z = 1028 $(M+H)^+$.

## Intermediat F268

Trifluoressigsäure N-(3-aminopropyl)-N-{(1R)-1-[1-benzyl-4-(2,5-difluorphenyl)-1H-pyrrol-2-yl]-2,2-dimethylpropyl}-28-(2,5-dioxo-2,5-dihydro-1H-pyrrol-1-yl)-6,23-dioxo-10,13,16,19-tetraoxa-3-thia-7,22-diazaoctacosan-1-amid (1:1)

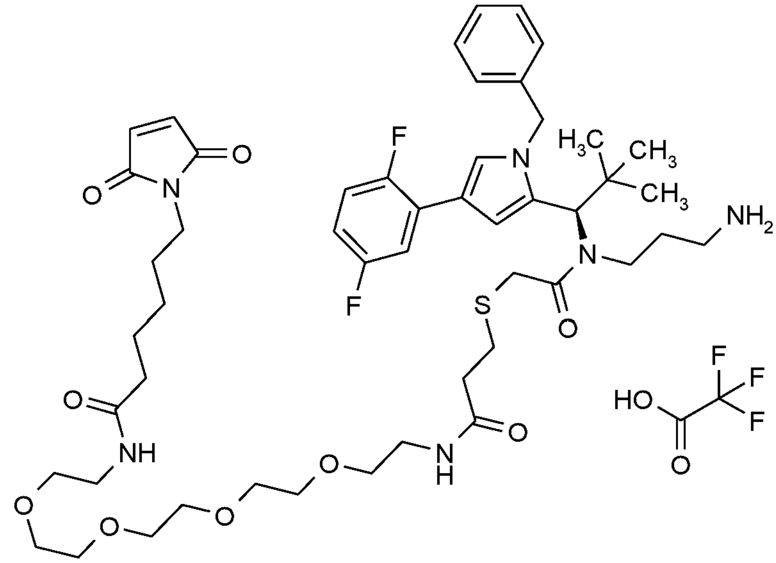

30.0 mg (0.043 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden zusammen mit 30.2 mg (0.056 mmol) Trifluoressigsäure N-(14-amino-3,6,9,12-tetraoxatetradec-1-yl)-6-(2,5-dioxo-2,5-dihydro-1H-pyrrol-1-yl)-hexanamid (1:1) (Intermediat L84) in 2.0 mL Acetonitril vorgelegt. Dann wurden 60 µl (0.34 mmol) N,N-Diisopropylethylamin zugegeben und 33 µl (0.056 mmol) T3P (50 % in Ethylacetat) zugetropft. Die Reaktionsmischung wurde über Nacht bei RT gerührt. Es wurde Wasser (2.0 mL) zugegeben. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 27.9 mg (59 % d. Th.) der Verbindung 2-(Trimethylsilyl)ethyl-[4-{(1R)-1-[1-benzyl-4-(2,5-difluorphenyl)-1H-pyrrol-2-yl]-2,2-dimethylpropyl}-32-(2,5-dioxo-2,5-dihydro-1H-pyrrol-1-yl)-5,10,27-trioxo-14,17,20,23-tetraoxa-7-thia-4,11,26-triazadotriacont-1-yl]carbamat.

LC-MS (Methode 1): $R_t$ = 1.41 min; MS (ESIpos): m/z = 1114 $(M+H)^+$.

25.6 mg (23.0 µmol) 2-(Trimethylsilyl)ethyl-[4-{(1R)-1-[1-benzyl-4-(2,5-difluorphenyl)-1H-pyrrol-2-yl]-2,2-dimethylpropyl}-32-(2,5-dioxo-2,5-dihydro-1H-pyrrol-1-yl)-5,10,27-trioxo-14,17,20,23-tetraoxa-7-thia-4,11,26-triazadotriacont-1-yl]carbamat wurden in 2.5 mL Trifluorethanol gelöst und mit 18.8 mg (138 µmol) Zinkdichlorid versetzt. Die Reaktionsmischung rührte 1 h bei 50 °C. Die Reaktionsmischung wurde mit 40.3 mg (138 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 22.2 mg (88 % d. Th.) der Titel Verbindung.

LC-MS (Methode 1): $R_t$ = 0.94 min; MS (ESIpos): m/z = 969 $(M+H)^+$.

## Intermediat F269

4-{[(8R,14R)-13-(3-Aminopropyl)-14-[1-benzyl-4-(2,5-difluorphenyl)-1H-pyrrol-2-yl]-1-(2,5-dioxo-2,5-dihydro-1H-pyrrol-1-yl)-15,15-dimethyl-2,7,12-trioxo-10-thia-3,6,13-triazahexadecan-8-yl]amino}-4-oxobutansäure -trifluoressigsäure (1:1)

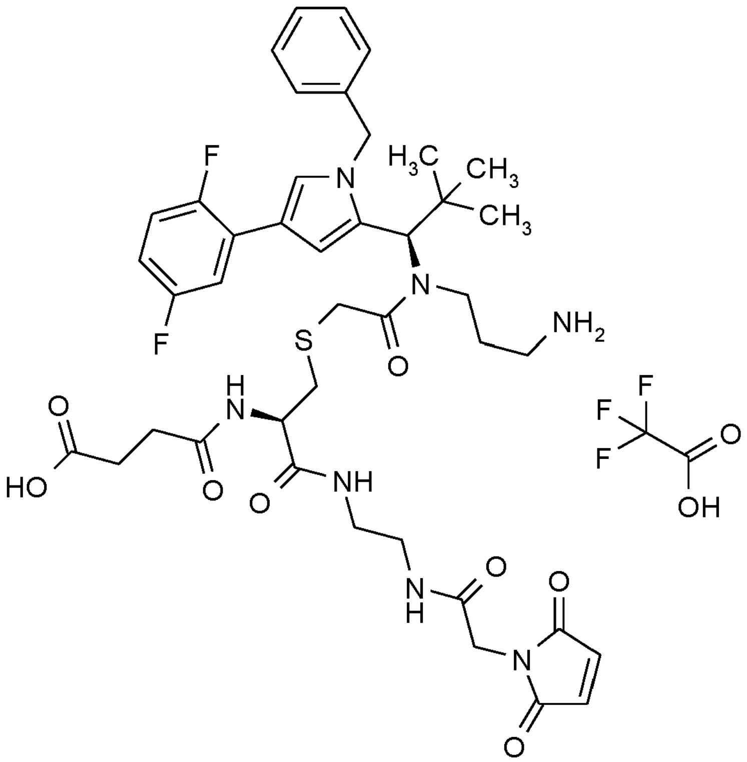

17.0 mg (0.0195 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) wurden zusammen mit 4.99 mg (0.0253 mmol) N-(2-Aminoethyl)-2-(2,5-dioxo-2,5-dihydro-1H-pyrrol-1-yl)acetamid (Intermediat L1) in 1.0 mL Acetonitril vorgelegt. Dann wurden 27 µl (0.16 mmol) N,N-Diisopropylethylamin zugegeben und 15 µl (0.025 mmol) T3P (50 % in Ethylacetat) zugetropft. Die Reaktionsmischung wurde über Nacht bei RT gerührt. Es wurde Wasser (2.0 mL) zugegeben. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 9.5 mg (46 % d. Th.) der Verbindung tert-Butyl-4-{[(16R)-11-{(1R)-1-[1-benzyl-4-(2,5-difluorphenyl)-1H-pyrrol-2-yl]-2,2-dimethylpropyl}-23-(2,5-dioxo-2,5-dihydro-1H-pyrrol-1-yl)-2,2-dimethyl-6,12,17,22-tetraoxo-5-oxa-14-thia-7,11,18,21-tetraaza-2-silatricosan-16-yl]amino}-4-oxobutanoat.

LC-MS (Methode 1): $R_t$ = 1.47 min; MS (ESIpos): m/z = 1052 $(M+H)^+$.

8.3 mg (7.89 µmol) tert-Butyl-4-{[(16R)-11-{(1R)-1-[1-benzyl-4-(2,5-difluorphenyl)-1H-pyrrol-2-yl]-2,2-dimethylpropyl}-23-(2,5-dioxo-2,5-dihydro-1H-pyrrol-1-yl)-2,2-dimethyl-6,12,17,22-tetraoxo-5-oxa-14-thia-7,11,18,21-tetraaza-2-silatricosan-16-yl]amino}-4-oxobutanoat wurden in 1.0 mL Trifluorethanol gelöst und mit 6.45 mg (47.3 µmol) Zinkdichlorid versetzt. Die Reaktionsmischung rührte 6 h bei 50 °C. 6.45 mg (47.3 µmol) Zinkdichlorid wurden zugegeben und die Reaktionsmischung rührte über Nacht bei 50 °C Die Reaktionsmischung wurde mit 27.7 mg (94.6 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 1.10 mg (14 % d. Th.) der Titel Verbindung.

LC-MS (Methode 1): $R_t$ = 0.89 min; MS (ESIpos): m/z = 852 $(M+H)^+$.

## Intermediat F270

Trifluoressigsäure --N-(3-aminopropyl)-N-{(1R)-1-[1-benzyl-4-(2,5-difluorphenyl)-1H-pyrrol-2-yl]-2,2-dimethylpropyl}-N'-(2-{[(2,5-dioxo-2,5-dihydro-1H-pyrrol-1-yl)acetyl]amino}ethyl)succinamid (1:1)

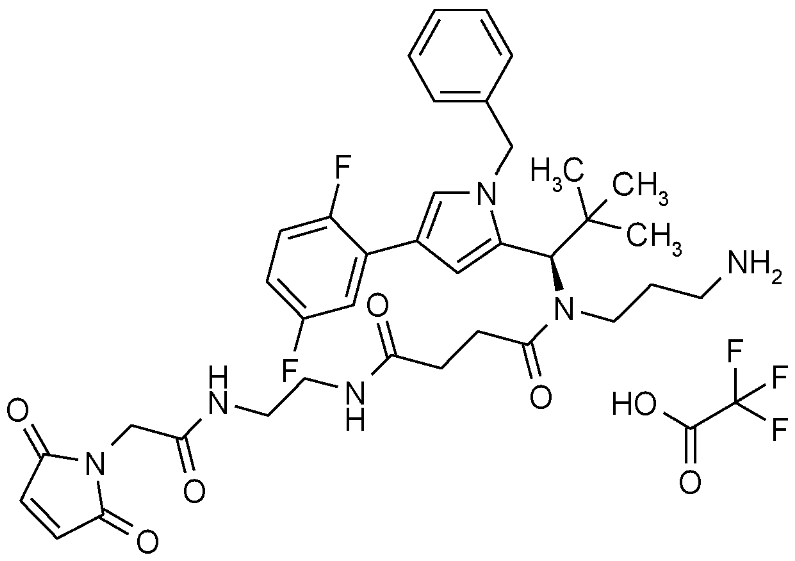

Unter Argon wurden 15.0 mg (22.9 μmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-säure (Intermediat C78) in 1.0 ml DMF vorgelegt, mit 8.0 μl (45.8 μmol) N,N-Diisopropylethylamin und 10.4 mg (27.4 μmol) HATU versetzt. Die Reaktionsmischung wurde 10 min bei RT gerührt. 8.54 mg (27.4 μmol) Trifluoressigsäure N-(2-aminoethyl)-2-(2,5-dioxo-2,5-dihydro-1H-pyrrol-1-yl)acetamid (1:1) (Intermediat L1) gelöst in 4.0 μl (22.9 μmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 14.7 mg (77 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl}{4-[(2-{[(2,5-dioxo-2,5-dihydro-1H-pyrrol-1-yl)acetyl]amino}ethyl)amino]-4-oxobutanoyl}amino)propyl]carbamat.

LC-MS (Methode 5): $R_t$ = 1.33 min; MS (ESIpos): m/z = 835 $(M+H)^+$.

13.2 mg (15.8 μmol) 2-(Trimethylsilyl)ethyl-[3-({(1R)-1-[1-benzyl-4-(2,5-difluorphenyl)-1H-pyrrol-2-yl]-2,2-dimethylpropyl}{4-[(2-{[(2,5-dioxo-2,5-dihydro-1H-pyrrol-1-yl)acetyl]amino}-ethyl)amino]-4-oxobutanoyl}amino)propyl]carbamat wurden in 2.0 mL Trifluorethanol gelöst und mit 12.9 mg (94.8 μmol) Zinkdichlorid versetzt. Die Reaktionsmischung rührte 1 h bei 50 °C. Die Reaktionsmischung wurde mit 27.7 mg (94.6 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 10.9 mg (83 % d. Th.) der Titel Verbindung.

LC-MS (Methode 1): $R_t$ = 0.83 min; MS (ESIpos): m/z = 691 $(M+H)^+$.

## Intermediat F271

4-{[(20R,26R)-25-(3-Aminopropyl)-26-[1-benzyl-4-(2,5-difluorphenyl)-1H-pyrrol-2-yl]-1-(2,5-dioxo-2,5-dihydro-1H-pyrrol-1-yl)-27,27-dimethyl-2,19,24-trioxo-6,9,12,15-tetraoxa-22-thia-3,18,25-triazaoctacosan-20-yl]amino}-4-oxobutan-säure -trifluoressigsäure (1:1)

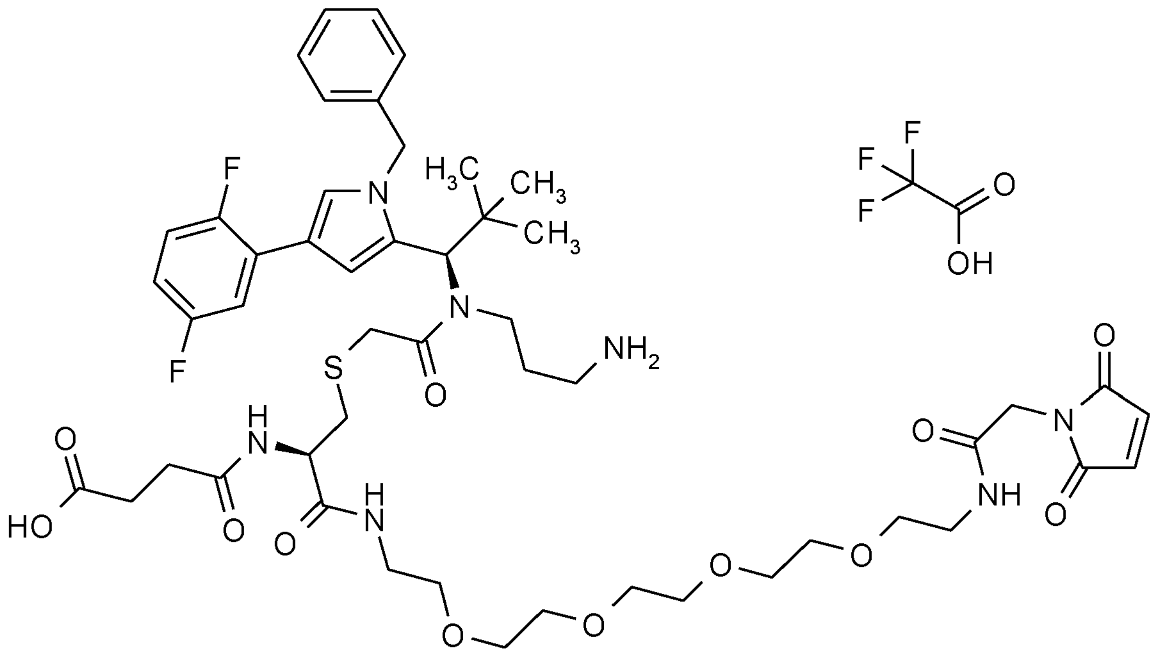

Unter Argon wurden 19.4 mg (22.2 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) in 2.0 ml DMF vorgelegt und mit 21.7 mg (44.4 μmol) Trifluoressigsäure --N-(14-amino-3,6,9,12-tetraoxatetradec-1-yl)-2-(2,5-dioxo-2,5-dihydro-1H-pyrrol-1-yl)acetamid (1:1) (Intermediat L74), 12 μl (67 μmol) N,N-Diisopropylethylamin und 16.9 mg (44.4 μmol) HATU versetzt. Die Reaktionsmischung wurde 1 h bei RT gerührt. Die Reaktionsmischung wurde direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 18.1 mg (66 % d. Th.) der Verbindung tert-Butyl-4-{[(16R)-11-{(1R)-1-[1-benzyl-4-(2,5-difluorphenyl)-1H-pyrrol-2-yl]-2,2-dimethylpropyl}-35-(2,5-dioxo-2,5-dihydro-1H-pyrrol-1-yl)-2,2-dimethyl-6,12,17,34-tetraoxo-5,21,24,27,30-pentaoxa-14-thia-7,11,18,33-tetraaza-2-silapentatriacontan-16-yl]amino}-4-oxobutanoat. LC-MS (Methode 4): $R_t$ = 1.79 min; MS (ESIpos): m/z = 1250 $(M+Na)^+$.

18.1 mg (14.7 μmol) tert-Butyl-4-{[(16R)-11-{(1R)-1-[1-benzyl-4-(2,5-difluorphenyl)-1H-pyrrol-2-yl]-2,2-dimethylpropyl}-35-(2,5-dioxo-2,5-dihydro-1H-pyrrol-1-yl)-2,2-dimethyl-6,12,17,34-tetraoxo-5,21,24,27,30-pentaoxa-14-thia-7,11,18,33-tetraaza-2-silapentatriacontan-16-yl]amino}-4-oxobutanoat wurden in 2.0 mL Trifluorethanol gelöst und mit 12.0 mg (88.4 μmol) Zinkdichlorid versetzt. Die Reaktionsmischung rührte 4 h bei 50 °C. Die Reaktionsmischung wurde mit 25.8 mg (88.4 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 12.3 mg (73 % d. Th.) der Titel Verbindung.

LC-MS (Methode 1): $R_t$ = 0.87 min; MS (ESIpos): m/z = 1028 $(M+H)^+$.

**Intermediat F272**

Trifluoressigsäure --N-(3-aminopropyl)-N-{(1R)-1-[1-benzyl-4-(2,5-difluorphenyl)-1H-pyrrol-2-yl]-2,2-dimethylpropyl}-N'-[17-(2,5-dioxo-2,5-dihydro-1H-pyrrol-1-yl)-16-oxo-3,6,9,12-tetraoxa-15-azaheptadec-1-yl]succinamid (1:1)

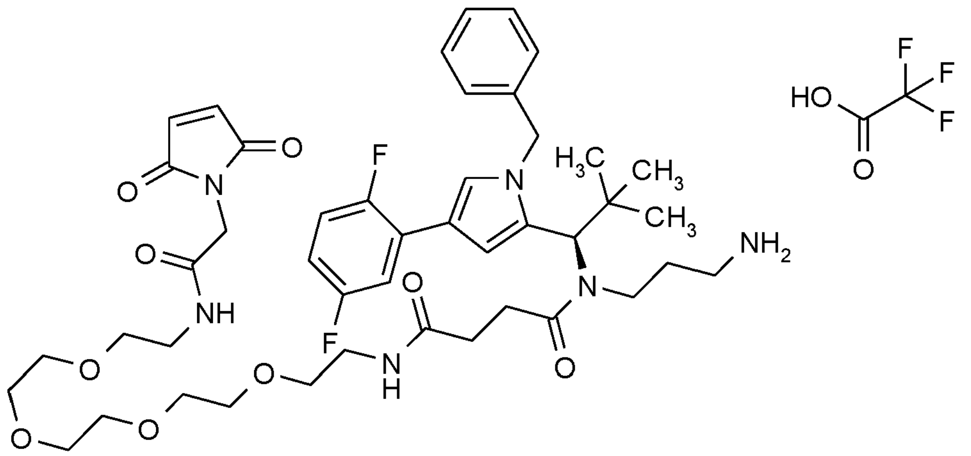

Unter Argon wurden 15.0 mg (22.9 μmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-säure (Intermediat C78) in 1.0 ml DMF vorgelegt, mit 8.0 μl (45.8 μmol) N,N-Diisopropylethylamin und 10.4 mg (27.4 μmol) HATU versetzt. Die Reaktionsmischung wurde 10 min bei RT gerührt. 13.4 mg (27.4 μmol) Trifluoressigsäure --N-(14-amino-3,6,9,12-tetraoxatetradec-1-yl)-2-(2,5-dioxo-2,5-dihydro-1H-pyrrol-1-yl)acetamid (1:1) (Intermediat L85) gelöst in 4.0 μl (22.9 μmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 15.8 mg (68 % d. Th.) der Verbindung 2-(Trimethylsilyl)ethyl-[23-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,22-trioxo-6,9,12,15-tetraoxa-3,18,23-triazahexacosan-26-yl]carbamat.

LC-MS (Methode 1): $R_t$ = 1.35 min; MS (ESIpos): m/z = 1011 $(M+H)^+$.

15.1 mg (14.9 μmol) 2-(Trimethylsilyl)ethyl-[23-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,22-trioxo-6,9,12,15-tetraoxa-3,18,23-triazahexacosan-26-yl]carbamat wurden in 2.0 mL Trifluorethanol gelöst und mit 12.2 mg (89.6 μmol) Zinkdichlorid versetzt. Die Reaktionsmischung rührte 1 h bei 50 °C. Die Reaktionsmischung wurde mit 26.2 mg (89.6 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 10.3 mg (70 % d. Th.) der Titel Verbindung.

LC-MS (Methode 1): $R_t$ = 0.88 min; MS (ESIpos): m/z = 867 $(M+H)^+$.

## Intermediat F273

Trifluoressigsäure N-(3-aminopropyl)-N-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19-dioxo-6,9,12,15-tetraoxa-22-thia-3,18-diazatetracosan-24-amid (1:1)

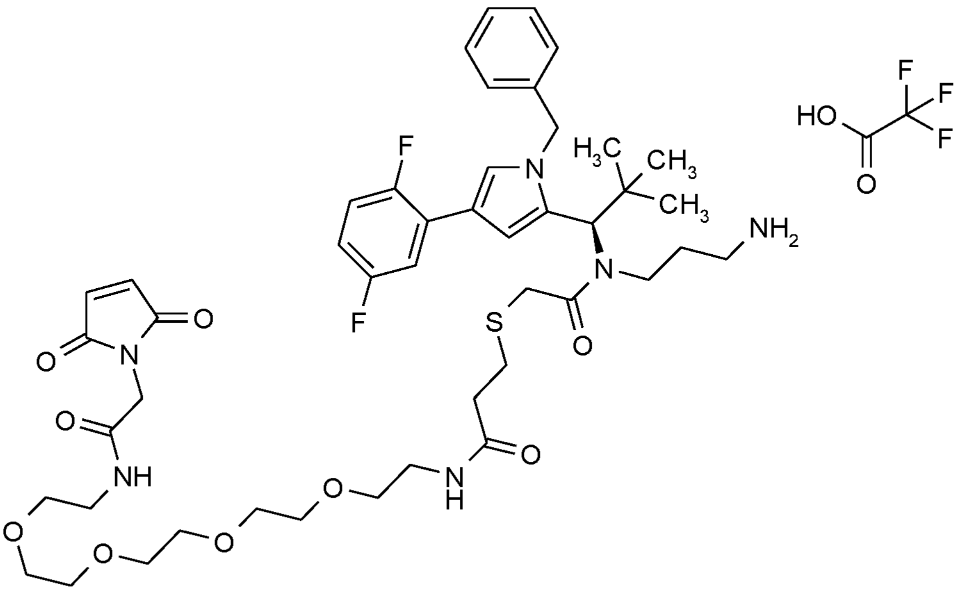

Unter Argon wurden 20.0 mg (28.5 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) in 1.0 ml DMF vorgelegt, mit 10.0 µl (57.0 µmol) N,N-Diisopropylethyl-amin und 13.0 mg (34.2 µmol) HATU versetzt. Die Reaktionsmischung wurde 10 min bei RT gerührt. 16.7 mg (34.2 µmol) Trifluoressigsäure --N-(14-amino-3,6,9,12-tetraoxatetradec-1-yl)-2-(2,5-dioxo-2,5-dihydro-1H-pyrrol-1-yl)acetamid (1:1) (Intermediat L85) gelöst in 5.0 µl (28.5 µmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 18.6 mg (62 % d. Th.) der Verbindung 2-(Trimethylsilyl)ethyl-[25-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,24-trioxo-6,9,12,15-tetraoxa-22-thia-3,18,25-triazaoctacosan-28-yl]carbamat. LC-MS (Methode 1): $R_t$ = 1.37 min; MS (ESIpos): m/z = 1057 $(M+H)^+$.

17.1 mg (16.2 µmol) 2-(Trimethylsilyl)ethyl-[25-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,24-trioxo-6,9,12,15-tetraoxa-22-thia-3,18,25-triazaoctacosan-28-yl]carbamat wurden in 2.0 mL Trifluorethanol gelöst und mit 13.2 mg (97.0 µmol) Zinkdichlorid versetzt. Die Reaktionsmischung rührte 1 h bei 50 °C. Die Reaktionsmischung wurde mit 28.4 mg (97.0 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 9.80 mg (59 % d. Th.) der Titel Verbindung.

LC-MS (Methode 1): $R_t$ = 0.88 min; MS (ESIpos): m/z = 913 $(M+H)^+$.

## Intermediat F274

N-[(2,5-Dioxo-2,5-dihydro-1H-pyrrol-1-yl)acetyl]-L-valyl-L-alanyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

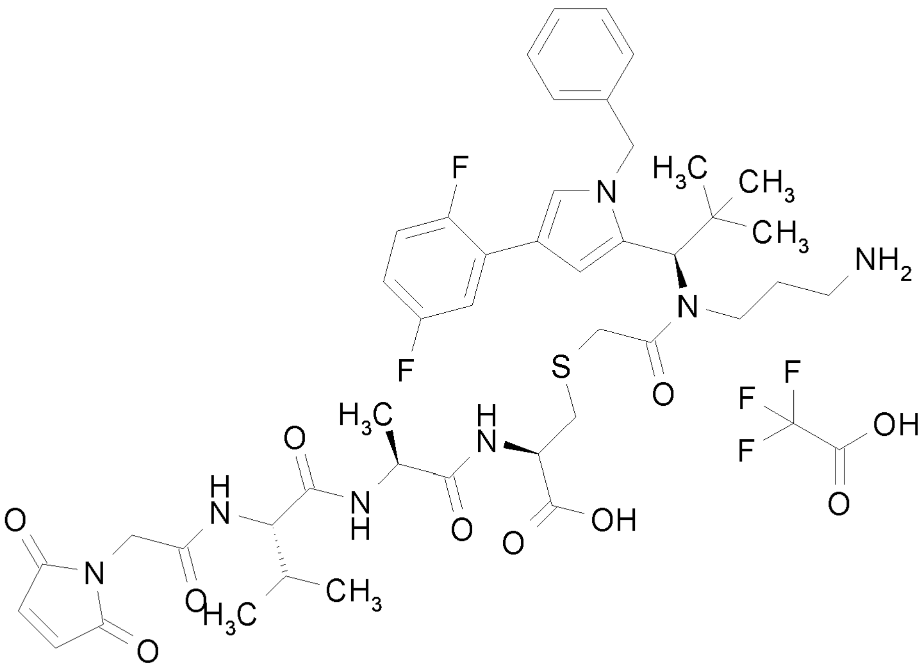

13.9 mg (0.0167 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein - trifluoressigsäure (1:1) (Intermediat C71) wurden zusammen mit 7.07 mg (0.0217 mmol) N-[(2,5-Dioxo-2,5-dihydro-1H-pyrrol-1-yl)acetyl]-L-valyl-L-alanin (Intermediat L86) in 2.0 mL Acetonitril vorgelegt. Dann wurden 23 µl (0.13 mmol) N,N-Diisopropylethylamin zugegeben und 13 µl (0.022 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 3.70 mg (19 % d. Th.) der Verbindung N-[(2,5-Dioxo-2,5-dihydro-1H-pyrrol-1-yl)acetyl]-L-valyl-L-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein.

LC-MS (Methode 1): $R_t$ = 1.34 min; MS (ESIpos): m/z = 1024 $(M+H)^+$.

10.6 mg (10.3 µmol) N-[(2,5-Dioxo-2,5-dihydro-1H-pyrrol-1-yl)acetyl]-L-valyl-L-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 8.46 mg (62.1 µmol) Zinkdichlorid versetzt. Die Reaktionsmischung rührte 1 h bei 50 °C. Die Reaktionsmischung wurde mit 18.1 mg (62.1 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.60 mg (54 % d. Th.) der Titel Verbindung.

LC-MS (Methode 12): $R_t$ = 1.69 min; MS (ESIpos): m/z = 880 $(M+H)^+$.

## Intermediat F275

N-[3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-N-(2-{[(2,5-dioxo-2,5-dihydro-1H-pyrrol-1-yl)acetyl]amino}ethyl)-L-alpha-glutamin -trifluoressigsäure (1:1)

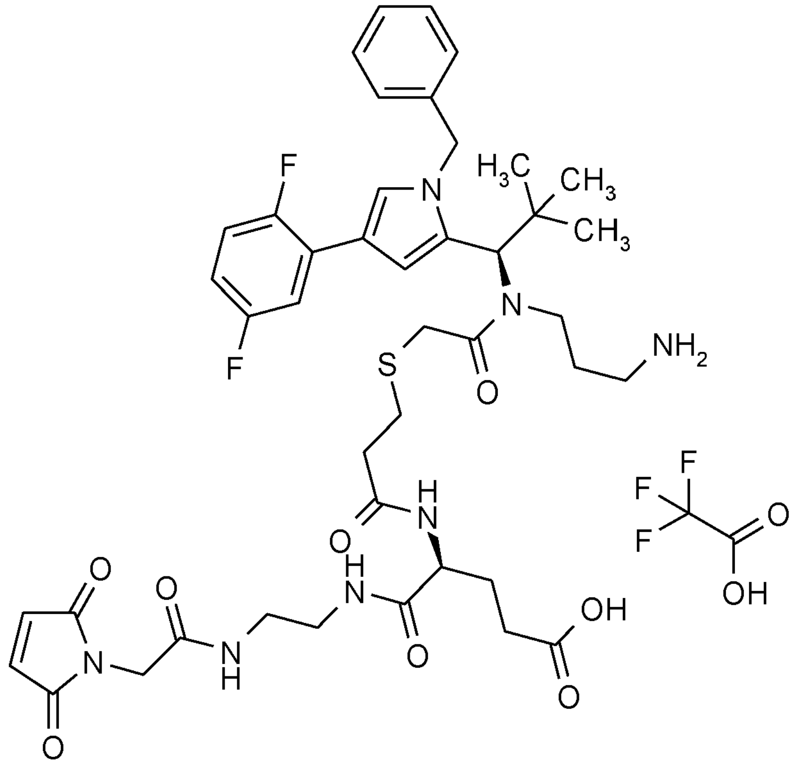

39.0 mg (55.6 μmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden in 4.0 mL DMF vorgelegt, mit 41.6 mg (111 μmol) 1-Benzyl-5-[2-(trimethylsilyl)ethyl]-L-glutamathydrochlorid (1:1) (Intermediat L89), 29 μl (170 μmol) N,N-Diisopropylethylamin und 42.3 mg (111 μmol) HATU versetzt und 1 Stunde bei RT nachgerührt. Die Reaktionsmischung wurde 1 Stunde bei RT gerührt, mit Essigsäure gequencht und direkt mittels präp. RP-HPLC (Säule: Reprosil 250x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 53.1 mg (93 % d. Th.) der Verbindung 1-Benzyl-5-[2-(trimethylsilyl)-ethyl]-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-L-glutamat.

LC-MS (Methode 1): $R_t$ = 1.71 min; MS (ESIpos): m/z = 1021 $[M+H]^+$

Unter Argon wurden 7.60 mg (33,9 μmol) Palladium(II)acetat in 3.0 ml Dichloromethan vorgelegt und mit 14 μl (100 μmol) Triethylamin und 110 μl (680 μmol) Triethylsilan versetzt. Die Reaktionsmischung wurde 5 min bei RT gerührt und mit 69.2 mg (67.7 μmol) 1-Benzyl-5-[2-(trimethylsilyl)ethyl]-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-L-glutamat gelöst in 3.0 mL Dichlormethan versetzt. Die Reaktionsmischung wurde über Nacht bei RT gerührt. Die Reaktionsmischung wurde über einen Pappfilter filtriert und der Filterkuchen mit Dichlormethan nachgewaschen. Das Lösemittel wurde im Vakuum verdampft. Der Rückstand wurde mittles präp. RP-HPLC (Säule: Reprosil 250x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 38.4 mg (61 % d. Th.) der Verbindung 19S)-11-{(1R)-1-[1-Benzyl-4-(2,5-difluorphenyl)-1H-pyrrol-2-yl]-2,2-dimethylpropyl}-2,2-dimethyl-6,12,17-trioxo-19-{3-oxo-3-[2-(trimethylsilyl)ethoxy]propyl}-5-oxa-14-thia-7,11,18-triaza-2-silaicosan-20-säure. LC-MS (Methode 1): $R_t$ = 1.53 min; MS (ESIpos): m/z = 931 $(M+H)^+$.

10.0 mg (10.7 μmol) (19S)-11-{(1R)-1-[1-Benzyl-4-(2,5-difluorphenyl)-1H-pyrrol-2-yl]-2,2-dimethylpropyl}-2,2-dimethyl-6,12,17-trioxo-19-{3-oxo-3-[2-(trimethylsilyl)ethoxy]propyl}-5-oxa-14-thia-7,11,18-triaza-2-silaicosan-20-säure wurden in 1.0 mL DMF vorgelegt, mit 6.73 mg (21.5 μmol) N-(2-Aminoethyl)-2-(2,5-dioxo-2,5-dihydro-1H-pyrrol-1-yl)acetamid 2,2,2-trifluorethan-1,1-diol (1:1) (Intermediat L1), 5.6 μl (32 μmol) N,N-Diisopropylethylamin und 8.17 mg (21.5 μmol) HATU versetzt und 1 Stunde bei RT nachgerührt. Die Reaktionsmischung wurde 3 Stunde bei RT gerührt, mit Essigsäure gequencht und direkt mittels präp. RP-HPLC (Säule: Reprosil 125x30; 10μ, Fluss: 50 mL/min, MeCN/Wasser, 0.1% TFA) gereinigt. Die Lösemittel wurden im Vakuum verdampft und der Rückstand im Hochvakuum getrocknet. Man erhielt 6.90 mg (58 % d. Th.) der Verbindung 2-(Trimethylsilyl)ethyl-N2-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-N-(2-{[(2,5-dioxo-2,5-dihydro-1H-pyrrol-1-yl)acetyl]amino}-ethyl)-L-alpha-glutaminat.

LC-MS (Methode 1): $R_t$ = 1.57 min; MS (ESIpos): m/z = 1110 $[M+H]^+$

6.90 mg (6.21 μmol) 2-(Trimethylsilyl)ethyl-$N^2$-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-N-(2-{[(2,5-dioxo-2,5-dihydro-1H-pyrrol-1-yl)acetyl]amino}ethyl)-L-alpha-glutaminat wurden in 2.0 mL Trifluorethanol gelöst und mit 5.1 mg (37.2 μmol) Zinkdichlorid versetzt. Die Reaktionsmischung rührte 3 h bei 50 °C. 5.1 mg (37.2 μmol) Zinkdichlorid wurden zugegeben und die Reaktionsmischung rührte 3 h bei 50 °C. 5.1 mg (37.2 μmol) Zinkdichlorid wurden zugegeben und die Reaktionsmischung rührte 3 h bei 50 °C. 10.1 mg (74.4 μmol) Zinkdichlorid wurden zugegeben und die Reaktionsmischung rührte über Nacht bei 50 °C und 72 h bei RT. Die Reaktionsmischung wurde mit 54.5 mg (186 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 2.4 mg (39 % d. Th.) der Titel Verbindung.

LC-MS (Methode 1): $R_t$ = 0.86 min; MS (ESIpos): m/z = 866 $(M+H)^+$.

## Intermediat F276

S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-{3-[2-(2-{[(2,5-dioxo-2,5-dihydro-1H-pyrrol-1-yl)acetyl]amino}ethoxy)ethoxy]propanoyl}-L-cystein -trifluoressigsäure (1:1)

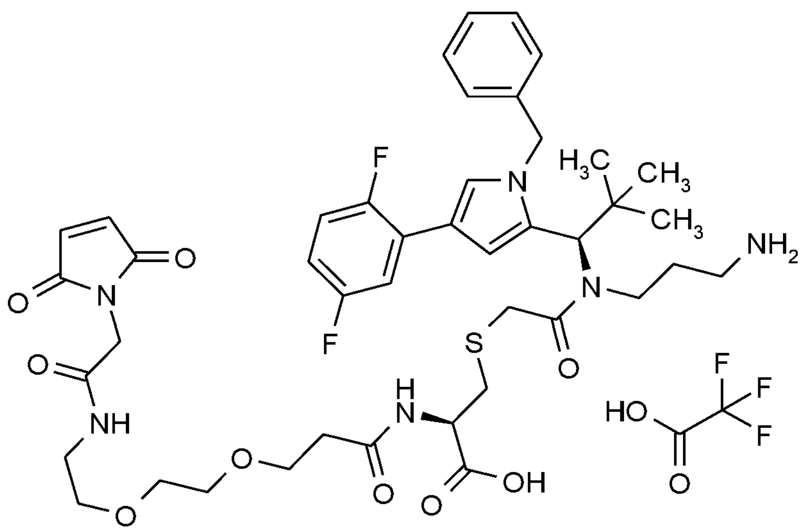

Unter Argon wurden 9.08 mg (28.9 μmol) 3-[2-(2-{[(2,5-Dioxo-2,5-dihydro-1H-pyrrol-1-yl)acetyl]amino}ethoxy)ethoxy]propansäure (Intermediat L87) in 1.0 ml DMF vorgelegt, mit 8.33 μl (48.2 μmol) N,N-Diisopropylethylamin und 11.0 mg (28.9 μmol) HATU versetzt.

Die Reaktionsmischung wurde 10 min bei RT gerührt. 20.0 mg (27.7 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (Intermediat C71) gelöst in 4,67 μl (24.1 μmol) N,N-Diisopropylethylamin und 1.0 mL DMF wurden dann zugegeben. Die Reaktionsmischung wurde 1 h bei RT gerührt. Die Reaktionsmischung wurde direkt mittels präparativer RP-HPLC (Säule: Reprosil 250x30; 10μ, Fluss: 50 mL/min, MeCN/Wasser, 0.1% TFA). Die Lösemittel wurden im Vakuum verdampft und der Rückstand im Hochvakuum getrocknet. Man erhielt 4.70 mg (19 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{[(2,5-dioxo-2,5-dihydro-1H-pyrrol-1-yl)acetyl]amino}ethoxy)-ethoxy]propanoyl}-L-cystein. LC-MS (Methode 12): $R_t$ = 2.47 min; MS (ESIpos): m/z = 1013 $(M+H)^+$.

13.9 mg (13.7 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{[(2,5-dioxo-2,5-dihydro-1H-pyrrol-1-yl)acetyl]amino}ethoxy)ethoxy]propanoyl}-L-cystein wurden in 2.0 mL Trifluorethanol gelöst und mit 5.6 mg (41.2 μmol) Zinkdichlorid versetzt. Die Reaktionsmischung rührte 1 h bei 50 °C. 5.6 mg (41.2 μmol) Zinkdichlorid wurden zugegeben und die Reaktionsmischung rührte 30 Minuten bei 50 °C. Die Reaktionsmischung wurde mit 24.1 mg (82.4 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10μ, Fluss: 50 mL/min, MeCN/Wasser, 0.1% TFA). Die Lösemittel

wurden im Vakuum verdampft und der Rückstand im Hochvakuum getrocknet. Man erhielt 10.8 mg (80 % d. Th.) der Titel Verbindung.

LC-MS (Methode 12): $R_t$ = 1.58 min; MS (ESIpos): m/z = 869 $(M+H)^+$.

## Intermediat F277

N-[3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-3-[(bromacetyl)amino]-D-alanin - trifluoressigsäure (1:1)

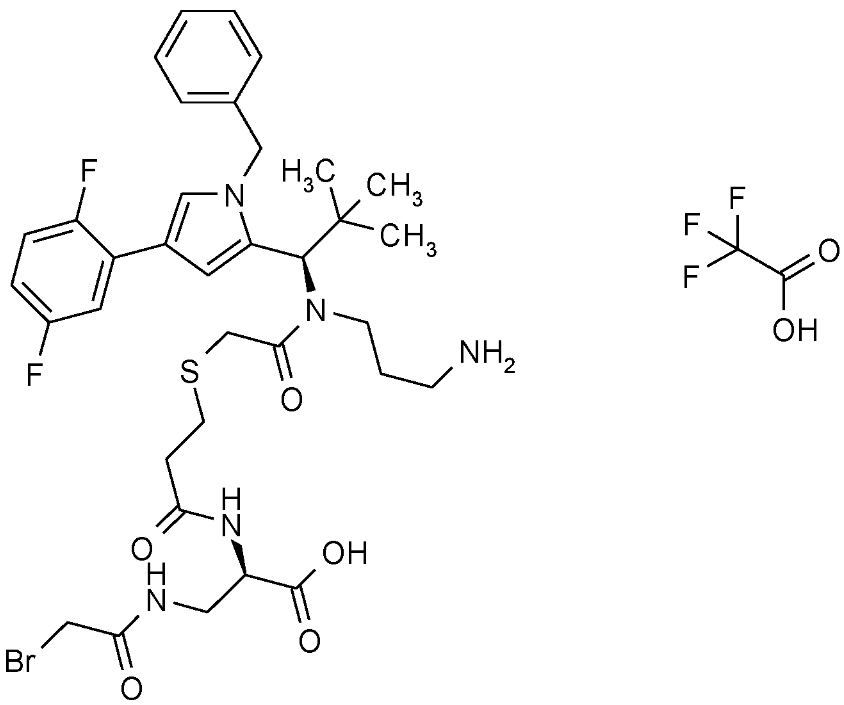

8.90 mg (8.88 μmol) Trifluoressigsäure --2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1) (Intermediat C80) und 2.31 mg (9.77 μmol) 1-(2-Bromacetoxy)pyrrolidin-2,5-dion wurden in 1 ml Dimethylformamid gelöst und mit 2.9 μl (27 μmol) N-Methylmorpholin versetzt. Die Reaktionsmischung wurde 1 h bei RT gerührt. Die Reaktionsmischung wurde direkt mittles präp. RP-HPLC gereinigt (Säule: Reprosil 125x30; 10μ, Fluss: 50 mL/min, MeCN/Wasser/0.1%TFA). Die Lösemittel wurden im Vakuum verdampft und der Rückstand im Hochvakuum getrocknet. Man erhielt 5.80 mg (65% d. Th.) der Verbinfung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-[(bromacetyl)amino]-D-alaninat.

LC-MS (Methode 1): $R_t$ = 1.57 min; MS (ESIpos): m/z = 1008 $(M+H)^+$.

5.80 mg (5.75 μmol) 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-[(bromacetyl)amino]-D-alaninat wurden in 2.0 mL Trifluorethanol gelöst und mit 4.70 mg (34.5 μmol) Zinkdichlorid versetzt. Die Reaktionsmischung rührte 3 h bei 50 °C. 4.70 mg (34.5 μmol) Zinkdichlorid wurden zugegeben und die Reaktionsmischung rührte 5 h bei 50 °C. Die Reaktionsmischung wurde mit 20.2 mg (69.0 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 1.70 mg (34 % d. Th.) der Titel Verbindung.

LC-MS (Methode 1): $R_t$ = 0.90 min; MS (ESIpos): m/z = 764 $(M+H)^+$.

**Intermediat F278**

N-[3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-3-{[(2,5-dioxo-2,5-dihydro-1H-pyrrol-1-yl)acetyl]amino}-D-alanin -trifluoressigsäure (1:1)

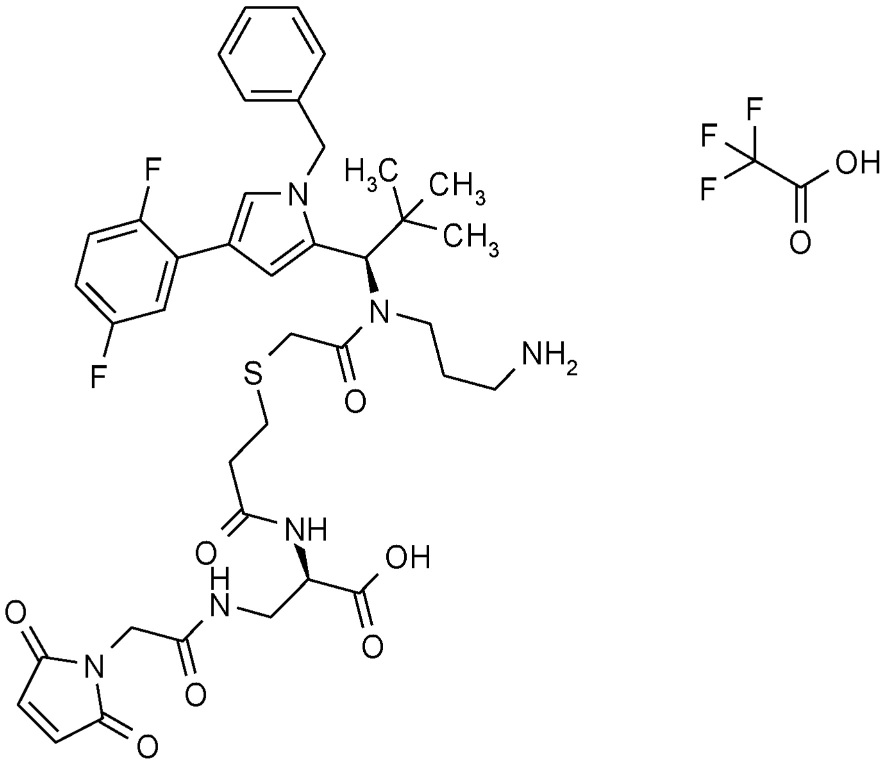

10.0 mg (9.98 μmol) Trifluoressigsäure -2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1) (Intermediat C80) und 2.77 mg (11.0 μmol) 1-{2-[(2,5-Dioxopyrrolidin-1-yl)oxy]-2-oxoethyl}-1H-pyrrol-2,5-dion wurden in 1 ml Dimethylformamid gelöst und mit 3.3 μl (30 μmol) N-Methylmorpholin versetzt. Die Reaktionsmischung wurde über Nacht bei RT gerührt. Die Reaktionsmischung wurde mit 2.0 μl (35 μmol) Essigsaeure versetzt und direkt mittles präp. RP-HPLC gereinigt (Säule: Reprosil 125x30; 10μ, Fluss: 50 mL/min, MeCN/Wasser/0.1%TFA). Die Lösemittel wurden im Vakuum verdampft und der Rückstand im Hochvakuum getrocknet. Man erhielt 5.50 mg (54% d. Th.) der Verbindung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{[(2,5-dioxo-2,5-dihydro-1H-pyrrol-1-yl)acetyl]amino}-D-alaninat.

LC-MS (Methode 1): $R_t$ = 1.51 min; MS (ESIpos): m/z = 1024 $(M+H)^+$.

5.50 mg (5.36 μmol) 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{[(2,5-dioxo-2,5-dihydro-1H-pyrrol-1-yl)acetyl]amino}-D-alaninat wurden in 1.0 mL Trifluorethanol gelöst und mit 4.39 mg (32.2 μmol) Zinkdichlorid versetzt. Die Reaktionsmischung rührte 1 h bei 50 °C. 4.39 mg (32.2 μmol) Zinkdichlorid wurden zugegeben und die Reaktionsmischung rührte 1 h bei 50 °C.. 4.39 mg (32.2 μmol) Zinkdichlorid wurden zugegeben und die Reaktionsmischung rührte 4 h bei 50 °C. Die Reaktionsmischung wurde mit 28.2 mg (96.5 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 2.70 mg (56 % d. Th.) der Titel Verbindung.

LC-MS (Methode 1): $R_t$ = 0.89 min; MS (ESIpos): m/z = 781 $(M+H)^+$.

**Intermediat F279**

N-[6-(2,5-Dioxo-2,5-dihydro-1H-pyrrol-1-yl)hexanoyl]-L-valyl-N-[3-({(1R)-1-[1-benzyl-4-(2,5-difluorphenyl)-1H-pyrrol-2-yl]-2,2-dimethylpropyl}[({(2R)-2-carboxy-2-[(3-carboxypropanoyl)amino]ethyl}sulfanyl)acetyl]amino)propyl]-L-alaninamid

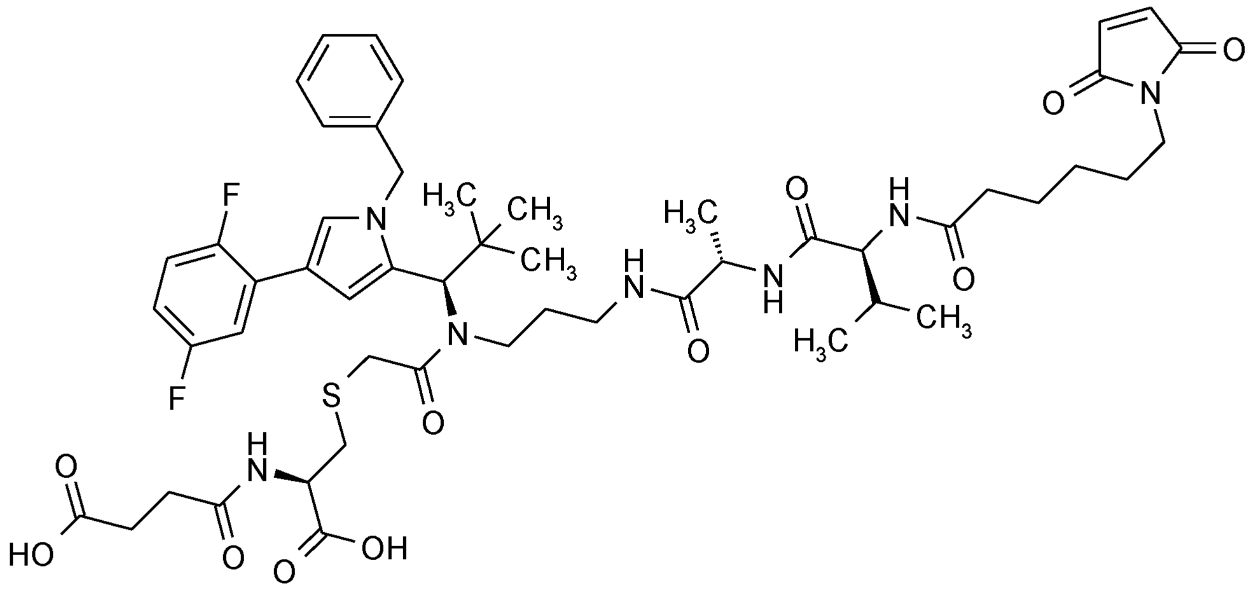

12.2 mg (14 μmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) wurden in 2.0 mL Trifluorethanol gelöst und mit 11.4 mg (83.8 μmol) Zinkdichlorid versetzt. Die Reaktionsmischung rührte 3 h bei 50 °C. Die Reaktionsmischung wurde mit 24.5 mg (83.8 μmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 4.60 mg (42 % d. Th.) der Verbindung 4-{[(1R)-2-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)-1-carboxyethyl]amino}-4-oxobutansäure - trifluoressigsäure (1:1).

LC-MS (Methode 1): $R_t$ = 0.88 min; MS (ESIpos): m/z = 673 $(M+H)^+$.

10.0 mg (12.7 μmol) 4-{[(1R)-2-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)-1-carboxyethyl]amino}-4-oxobutansäure -trifluoressigsäure (1:1) und 7.41 mg (12.7 μmol) 2,5-Dioxopyrrolidin-1-yl-N-[6-(2,5-dioxo-2,5-dihydro-1H-pyrrol-1-yl)hexanoyl]-L-valyl-L-alaninat (Intermediat L88) wurden in 1.5 ml Dimethylformamid gelöst und mit 4.4 μl (25 μmol) N,N-Diisopropylethylamin versetzt. Die Reaktionsmischung wurde 2 h bei RT gerührt. Die Reaktionsmischung wurde mit 2.0 μl (35 μmol) Essigsaeure versetzt und direkt mittles präp. RP-HPLC gereinigt (Säule: Reprosil 250x30; 10μ, Fluss: 50 mL/min, MeCN/Wasser/0.1%TFA). Die Lösemittel wurden im Vakuum verdampft und der Rückstand im Hochvakuum getrocknet. Man erhielt 5.20 mg (39% d. Th.) der Titel Verbinfung.

LC-MS (Methode 1): $R_t$ = 1.11 min; MS (ESIpos): m/z = 1036 $(M+H)^+$.

**Intermediat F280**

Trifluoressigsäure --N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-3-(2,5-dioxo-2,5-dihydro-1H-pyrrol-1-yl)benzamid (1:1)

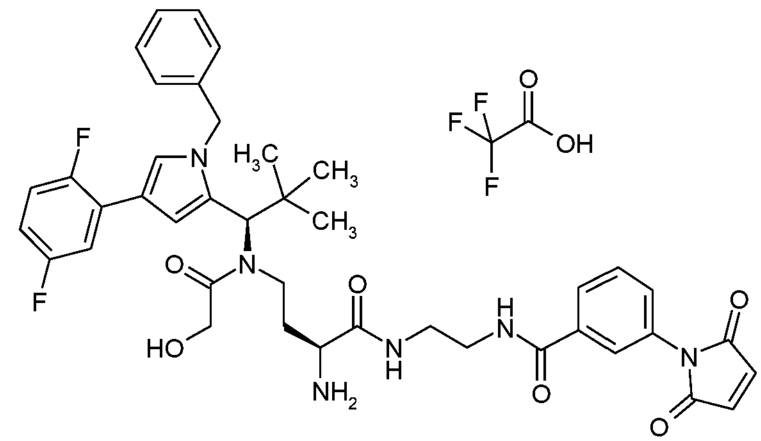

Die Titelverbindung wurde ausgehend von Intermediat C64 durch Kupplung mit kommerziell erhältlichem 1-(3-{[(2,5-Dioxopyrrolidin-1-yl)oxy]carbonyl}phenyl)-1 H-pyrrol-2,5-dion und anschließender Entschützung mit Zinkchlorid hergestellt.

LC-MS (Methode 1): $R_t$ = 0.88 min; MS (ESIpos): m/z = 755 $(M+H)^+$.

**Intermediat** F281

N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[N-(bromacetyl)-beta-alanyl]amino}-D-alanin -trifluoressigsäure (1:1)

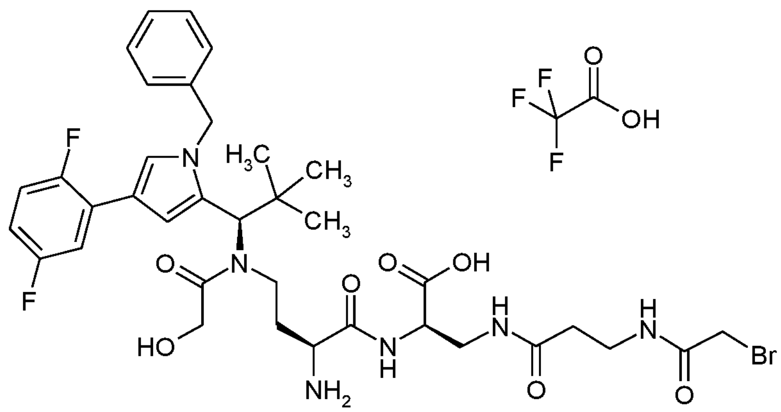

Zunächst wurden die modifizierten Aminosäurebausteine N-(Bromacetyl)-beta-alanin sowie 2-(Trimethylsilyl)ethyl-3-amino-N-(tert-butoxycarbonyl)-D-alaninat nach klassischen Methoden der Peptidchemie hergestellt. Anschließend wurden diese in Gegenwart von HATU und Morpholin miteinander gekuppelt. Dann wurde mittels 10%-iger Trifluoressigsäure in Dichlormethan die tert. Butoxycarbonyl-Schutzgruppe abgelöst und so das Intermediat 2-(Trimethylsilyl) ethyl-3-{[N-(bromacetyl)-beta-alanyl]amino}-D-alaninat erhalten.

Schließlich wurde die Titelverbindung durch Kupplung dieses Intermediats mit Intermediat C58 in Gegenwart von HATU und 4-Methylmorpholin und anschließender Entschützung mit Zinkchlorid hergestellt.

LC-MS (Methode 1): $R_t$ = 0.87 min; MS (ESIpos): m/z = 791 und 793 $(M+H)^+$.

**Intermediat** F282

Trifluoressigsäure --(2S)-2-amino-4-[{(1R)-1-[1-benzyl-4-(2,5-difluorphenyl)-1 H-pyrrol-2-yl]-2,2-dimethylpropyl}(glycoloyl)amino]-N-(3-{[N-(bromacetyl)glycyl]amino}propyl)-butanamid (1:1)

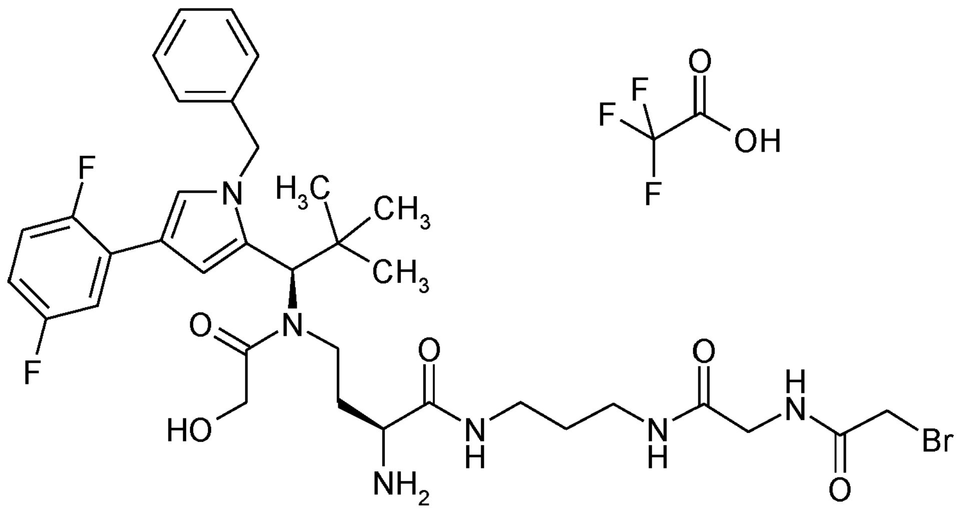

Zunächst wurde nach klassischen Methoden der Peptidchemie ausgehend von tert-Butylglycinat und Bromessigsäureanhydrid das Intermediat Trifluoressigsäure --N-(3-aminopropyl)-N2-(bromacetyl)glycinamid (1:1) hergestellt.

Schließlich wurde die Titelverbindung durch Kupplung dieses Intermediats mit Intermediat C58 in Gegenwart von HATU und 4-Methylmorpholin und anschließender Entschützung mit Zinkchlorid hergestellt.

LC-MS (Methode 1): $R_t$ = 0.83 min; MS (ESIpos): m/z = 747 und 749 $(M+H)^+$.

## Intermediat F283

N-[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]-$N^2$-(bromacetyl)-L-alpha-asparagin -trifluoressigsäure (1:1)

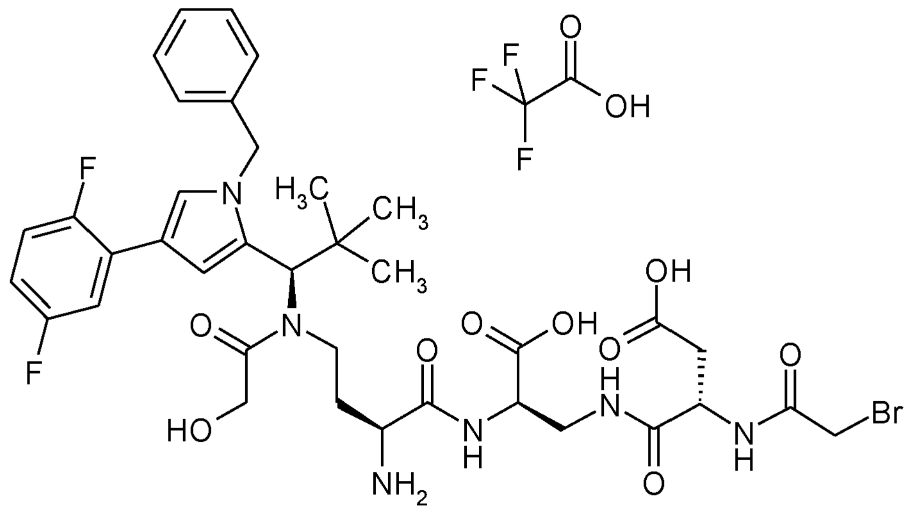

Zunächst wurde ausgehend von (2S)-2-Amino-4-oxo-4-[2-(trimethylsilyl)ethoxy]butansäure und Bromessigsäureanhydrid der modifizierten Aminosäurebaustein (2S)-2-[(Bromacetyl)amino]-4-oxo-4-[2-(trimethylsilyl)ethoxy]butansäure sowie ausgehend von kommerziell erhältlichem 3-{[(Benzyloxy)carbonyl]amino}-N-(tert-butoxycarbonyl)-D-alanin--N-cyclohexylcyclohexan-amin (1:1) der Aminosäurebaustein 2-(Trimethylsilyl)ethyl-3-amino-N-(tert-butoxycarbonyl)-D-alaninat hergestellt. Beide Bausteine wurden in Gegenwart von HATU und Morpholin miteinander gekuppelt und anschließend wurde mittels 5%-iger Trifluoressigsäure in Dichlormethan die tert. Butoxycarbonyl-Schutzgruppe unter Erhalt der Silylethylester-Schutzgruppen abgelöst und so das Intermediat Trifluoressigsäure--2-(trimethylsilyl)ethyl-N-{(2R)-2-amino-3-oxo-3-[2-(trimethyl-silyl)ethoxy] propyl}-N2-(bromacetyl)-L-alpha-asparaginat (1:1) erhalten.

Schließlich wurde die Titelverbindung durch Kupplung dieses Intermediats mit Intermediat C58 in Gegenwart

von HATU und 4-Methylmorpholin und anschließender Entschützung mit Zinkchlorid hergestellt.

LC-MS (Methode 1): $R_t$ = 0.84 min; MS (ESIpos): m/z = 835 und 837 $(M+H)^+$.

**Intermediat F284**

N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[1-(2,5-dioxo-2,5-dihydro-1 H-pyrrol-1-yl)-2,18-dioxo-6,9,12,15-tetraoxa-3-azaoctadecan-18-yl]amino}-D-alanin -trifluoressigsäure (1:1)

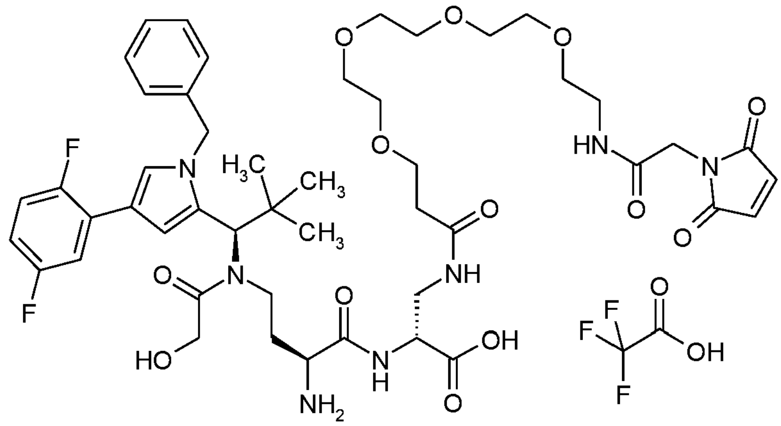

Zunächst wurde Intermediat L80 mit kommerziell erhältlicher (2,5-Dioxo-2,5-dihydro-1H-pyrrol-1-yl)essigsäure in Gegenwart von HATU und N,N-Diisopropylethylamin gekuppelt und anschließend wurde mittels 16%-iger Trifluoressigsäure in Dichlormethan die tert. Butoxycarbonyl-Schutzgruppe unter Erhalt der Silylethylester-Schutzgruppe abgelöst. Schließlich wurde die Titelverbindung durch Kupplung dieses Intermediats mit Intermediat C58 in Gegenwart von HATU und N, N-Diisopropylethylamin und anschließender Entschützung mit Zinkchlorid hergestellt.

LC-MS (Methode 12): $R_t$ = 1.46 min; MS (ESIpos): m/z = 984.45 $(M+H)^+$.

**Intermediat F285**

N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-[(18-brom-17-oxo-4,7,10, 13-tetraoxa-16-azaoctadecan-1-oyl)amino]-D-alanin -trifluoressigsäure (1:1)

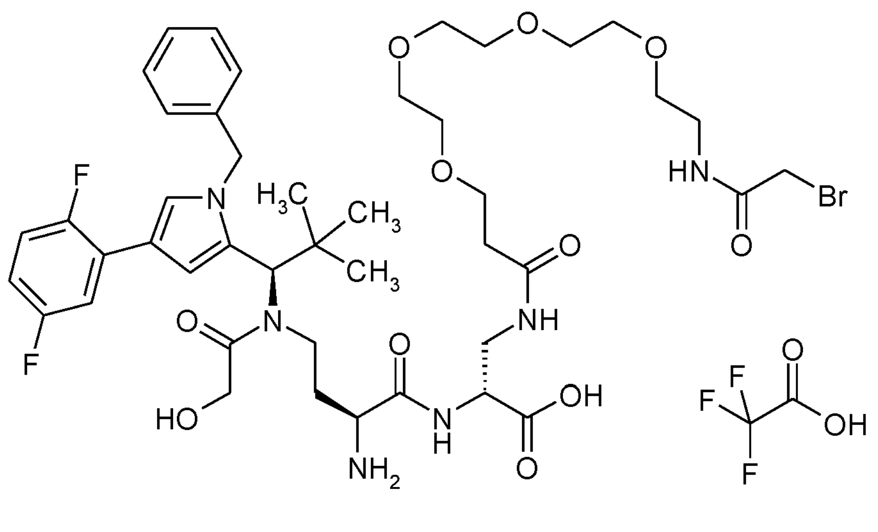

Zunächst wurde Intermediat L80 mit kommerziell erhältlichem Bromessigsäureanhydrid acyliert und anschließend wurde mittels 20%-iger Trifluoressigsäure in Dichlormethan die tert. Butoxycarbonyl-Schutzgruppe unter Erhalt der Silylethylester-Schutzgruppe abgelöst. Schließlich wurde die Titelverbindung durch Kupplung dieses Intermediats mit Intermediat C58 in Gegenwart von HATU und N, N-Diisopropylethylamin und anschließender Entschützung mit Zinkchlorid hergestellt.

LC-MS (Methode 1): $R_t$ = 0.85 min; MS (ESIpos): m/z = 967 und 969 $(M+H)^+$.

**Intermediat F286**

1-[(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[(2,5-dioxo-2,5-dihydro-1 H-pyrrol-1-yl) acetyl]amino}-D-alanyl)amino]-3,6,9,12-tetraoxapentadecan-15-säure -trifluoressigsäure (1:1)

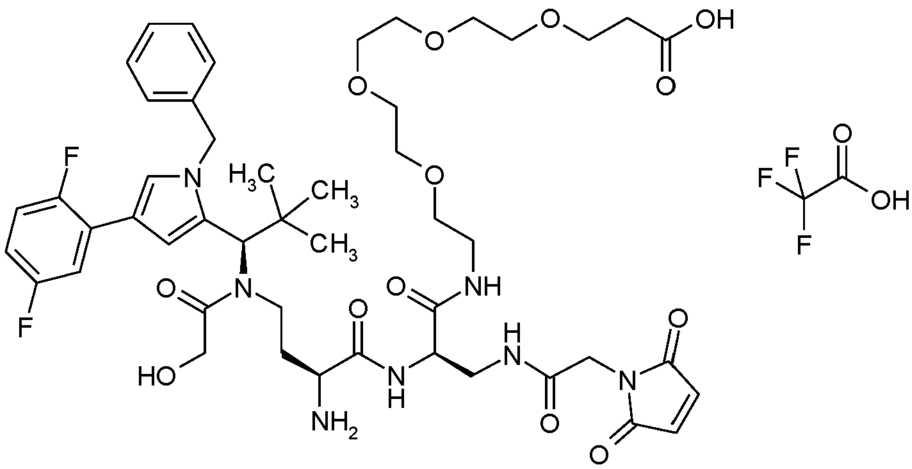

Zunächst wurde Intermediat L91 mit (2,5-Dioxo-2,5-dihydro-1H-pyrrol-1-yl)essigsäure in Gegenwart von HATU und N, N Diisopropylethylamin gekuppelt und anschließend wurde mit 12.5%-iger TFA in DCM die Boc-Schutzgruppe abgelöst. Das so erhaltene Intermediat wurde mit Intermediat C58 in Gegenwart von HATU und N, N Diisopropylethylamin gekuppelt und anschließend durch Entschützung mit Zinkchlorid in die Titelverbindung überführt.

LC-MS (Methode 1): $R_t$ = 0.84 min; MS (ESIpos): m/z = 984 $(M+H)^+$.

**Intermediat F288**

N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-({N-[(2,5-dioxo-2,5-dihydro-1H-pyrrol-1-yl)acetyl]-L-seryl}amino)-D-alanin -trifluoressigsäure (1:1)

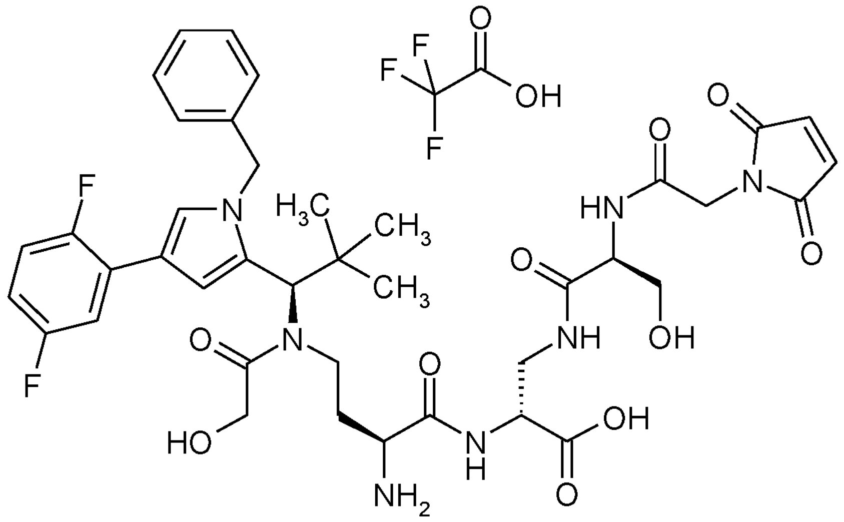

35 mg (39 µmol) von Intermediat C74 wurden in Gegenwart von HATU und N, N-Diisopropyethylamin mit N-[(2,5-Dioxo-2,5-dihydro-1H-pyrrol-1-yl)acetyl]-L-serin gekuppelt, das zuvor ausgehend von tert-Butyl-O-tert-butyl-L-serinat und (2,5-Dioxo-2,5-dihydro-1H-pyrrol-1-yl)essigsäure hergestellt wurde. Nach Entschützung mit Zinkchlorid und HPLC Reinigung wurden 14 mg (38% d. Th.) der Titelverbindung erhalten.

LC-MS (Methode 12): $R_t$ = 1.43 min; MS (ESIpos): m/z = 824.34 $(M+H)^+$.

## Intermediat F289

$N^2$-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-$N^6$-[(2,5-dioxo-2,5-dihydro-1H-pyrrol-1-yl)acetyl]-D-lysin -trifluoressigsäure (1:1)

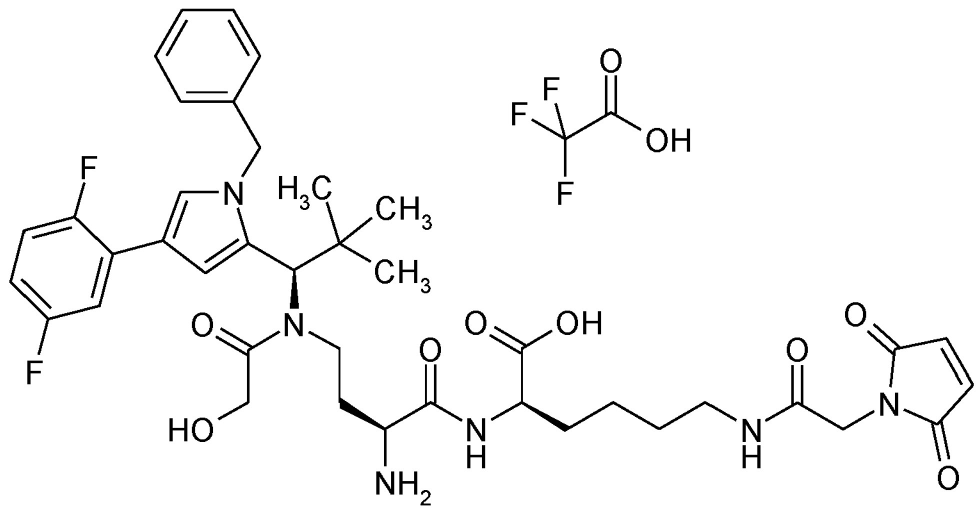

Zunächst wurde Trifluoressigsäure --2-(trimethylsilyl)ethyl-$N^6$-[(2,5-dioxo-2,5-dihydro-1H-pyrrol-1-yl)acetyl]-D-lysinat (1:1) nach klassischen Methoden der Peptidchemie ausgehend von $N^6$-[(Benzyloxy)carbonyl]-$N^2$-(tert-butoxycarbonyl)-D-lysin hergestellt.

12.5 mg (25 µmol) von diesem Intermediat wurden in Gegenwart von HATU und 4-Methylmorpholin mit 15 mg (23 µmol) von Intermediat C58 gekuppelt. Nach Entschützung mit Zinkchlorid und HPLC Reinigung wurden 14 mg (53% d. Th.) der Titelverbindung erhalten.

LC-MS (Methode 1): $R_t$ = 0.83 min; MS (ESIpos): m/z = 779 $(M+H)^+$.

**Intermediat F290**

$N^2$-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-$N^6$-(bromacetyl)-D-lysin-trifluoressigsäure (1:1)

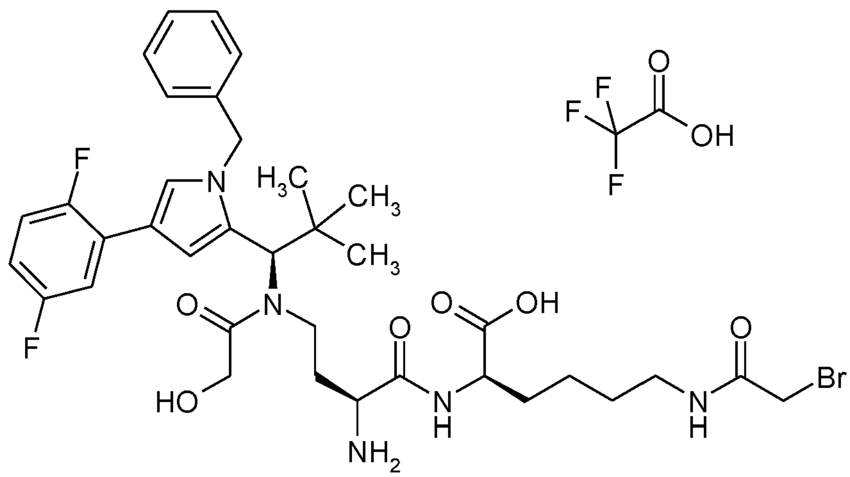

Zunächst wurde Trifluoressigsäure --2-(trimethylsilyl)ethyl-N6-(bromacetyl)-D-lysinat (1:1) nach klassischen Methoden der Peptidchemie ausgehend von $N^6$-[(Benzyloxy)carbonyl]-$N^2$-(tert-butoxycarbonyl)-D-lysin hergestellt.
12 mg (25 μmol) von diesem Intermediat wurden in Gegenwart von HATU und 4-Methylmorpholin mit 15 mg (23 μmol) von Intermediat C58 gekuppelt. Nach Entschützung mit Zinkchlorid und HPLC Reinigung wurden 7 mg (36% d. Th.) der Titelverbindung erhalten.
LC-MS (Methode 1): $R_t$ = 0.86 min; MS (ESIpos): m/z = 762 und 764 $(M+H)^+$.

**Intermediat F291**

N-[(2,5-Dioxo-2,5-dihydro-1H-pyrrol-1-yl)acetyl]-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid

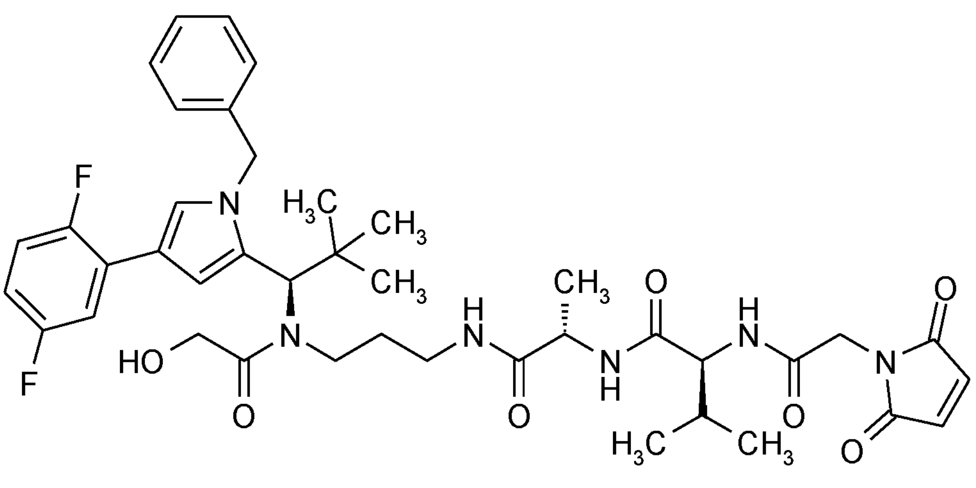

Die Titelverbindung wurde ausgehend von Beispiel M9 zunächst durch Kupplung mit N-[(Benzyloxy)carbonyl]-L-valyl-L-alanin in Gegenwart von HATU und N,N-Diisopropylethylamin hergestellt. Im nächsten Schritt wurde die Z-Schutzgruppe durch 1-stündige Hydrierung über 10%-igem Palladium auf Aktivkohle bei RT unter Wasserstoff-Normaldruck entfernt und das entschützte Intermediat anschließend durch Kupplung mit (2,5-Dioxo-2,5-dihydro-1H-pyrrol-1-yl)essigsäure in Gegenwart von HATU und N, N Diisopropylethylamin in die Titelverbindung überführt.

LC-MS (Methode 1): $R_t$ = 1.21 min; MS (ESIpos): m/z = 777 $(M+H)^+$.

## Intermediat F293

N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[3-(2,5-dioxo-2,5-dihydro-1 H-pyrrol-1-yl)benzoyl]amino}-D-alanin -trifluoressigsäure (1:1)

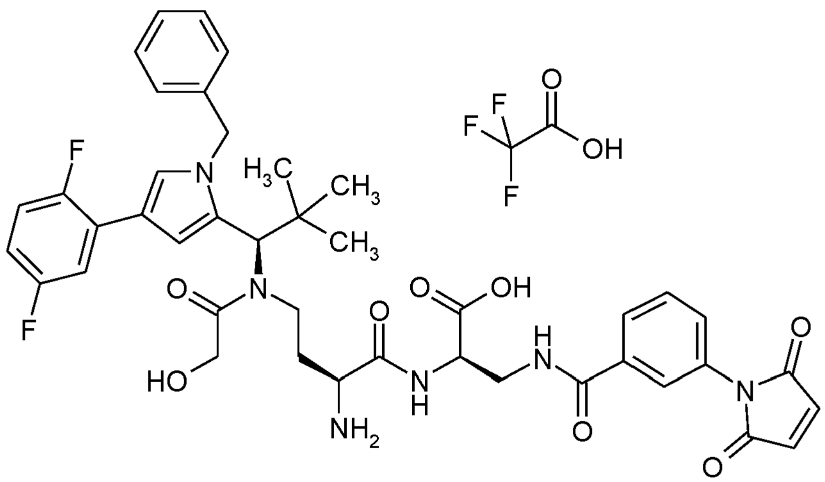

35 mg (39 µmol) von Intermediat C74 wurden in 4 ml DMF gelöst und in Gegenwart von N, N-Diisopropylethylamin mit 13.5 mg (43 µmol) kommerziell erhältlichem 1-(3-{[(2,5-Dioxo-pyrrolidin-1-yl)oxy] carbonyl}phenyl)-1H-pyrrol-2,5-dion gekuppelt. Nach Entschützung mit Zinkchlorid und HPLC Reinigung wurden 12 mg (34% d. Th.) der Titelverbindung erhalten.

LC-MS (Methode 12): $R_t$ = 0.93 min; MS (ESIpos): m/z = 799 $(M+H)^+$.

## Intermediat F294

N-{5-[(2,5-Dioxopyrrolidin-1-yl)oxy]-5-oxopentanoyl}-L-valyl-N-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-alaninamid

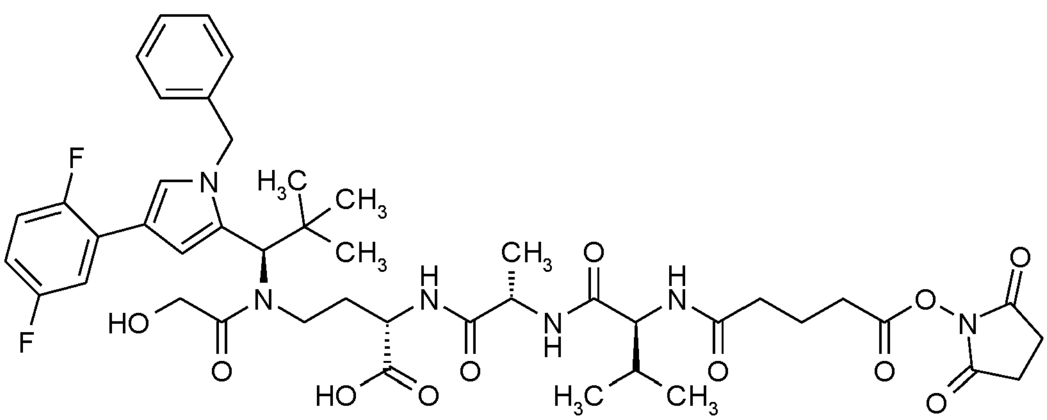

41 mg (0.05 mmol) von Intermediat C76 gelöst in 12 mL Methanol wurden über 10 mg 10%-igem Palladium auf Aktivkohle 1 h lang bei RT unter Wasserstoff-Normaldruck hydriert. Der Katalysator wurde danach abfiltriert und das Lösungsmittel im Vakuum entfernt. Man erhielt 32 mg (92% d.Th.) des entschützten Intermediats.

15 mg (0.022 mmol) von diesem Intermediat wurden in DMF gelöst und mit 13 mg (0.039 mmol) 1,1'-[(1,5-Dioxopentan-1,5-diyl)bis(oxy)]dipyrrolidin-2,5-dion und 7 μL N, N-Diisopropylethyl amin versetzt. Nach 1 h Rühren bei RT wurde der Ansatz eingeengt und der Rückstand mittels HPLC gereinigt. So wurden 9 mg (45% d. Th.) der Titelverbindung erhalten.

LC-MS (Methode 1): $R_t$ = 1.08 min; MS (ESIpos): m/z = 895 $(M+H)^+$.

## Intermediat F295

N-[(2,5-Dioxo-2,5-dihydro-1H-pyrrol-1-yl)acetyl]-L-valyl-N-{(1S)-3-[{(1R)-1-[1-benzyl-4-(2,5-difluorphenyl)-1H-pyrrol-2-yl]-2,2-dimethylpropyl}(glycoloyl)amino]]-1-carboxypropyl}-L-alaninamid

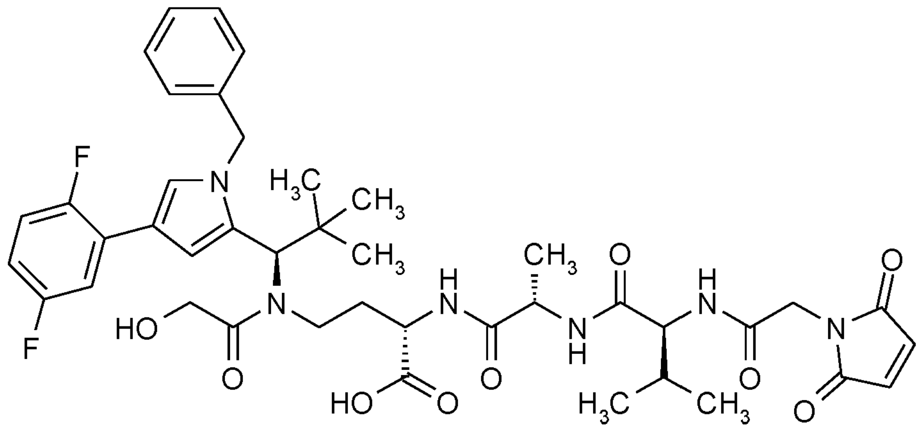

41 mg (0.05 mmol) von Intermediat C76 gelöst in 12 mL Methanol wurden über 10 mg 10%-igem Palladium auf Aktivkohle 1 h lang bei RT unter Wasserstoff-Normaldruck hydriert. Der Katalysator wurde danach abfiltriert und das Lösungsmittel im Vakuum entfernt. Man erhielt 32 mg (92% d.Th.) des entschützten Intermediats.

15 mg (0.022 mmol) von diesem Intermediat wurden in 4 mL DMF gelöst und mit 10 mg (0.039 mmol) 1-{2-[(2,5-Dioxopyrrolidin-1-yl)oxy]-2-oxoethyl}-1H-pyrrol-2,5-dion und 7 μL N, N-Diisopropylethyl amin versetzt. Nach 2 h Rühren bei RT wurde der Ansatz eingeengt und der Rückstand mittels HPLC gereinigt. So wurden 10 mg (56% d. Th.) der Titelverbindung erhalten.

LC-MS (Methode 1): $R_t$ = 1.08 min; MS (ESIpos): m/z = 821 $(M+H)^+$.

## Intermediat F296

Trifluoressigsäure --(2S)-2-amino-4-[{(1R)-1-[1-benzyl-4-(2,5-difluorphenyl)-1 H-pyrrol-2-yl]-2,2-dimethylpropyl}(glycoloyl)amino]-N-{2-[(2-{[(2,5-dioxo-2,5-dihydro-1H-pyrrol-1-yl)acetyl]amino}ethyl)sulfonyl]ethyl}butanamid (1:1)

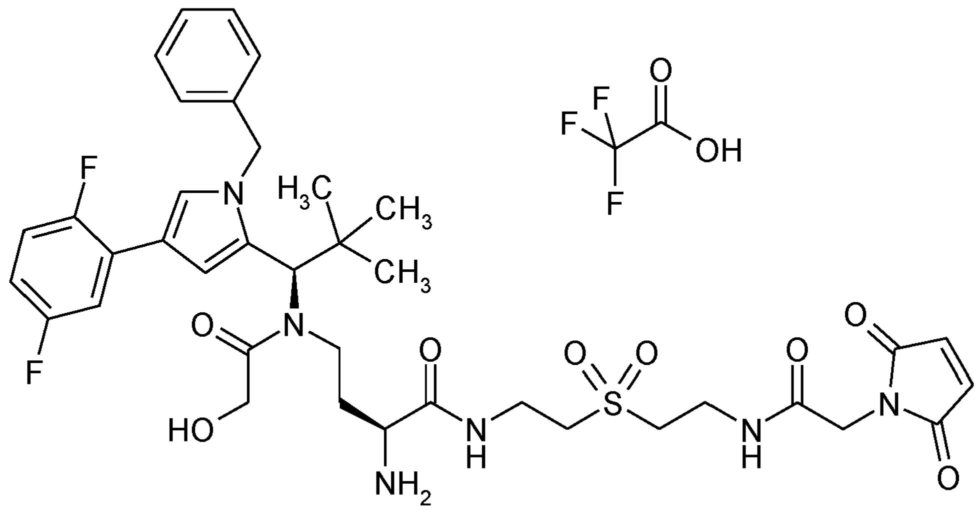

Die Titelverbindung wurde ausgehend von Intermediat L81 durch Kupplung mit Intermediat C58 in Gegenwart von HATU und N,N-Diisopropylethylamin hergestellt. Im nächsten Schritt wurde die Z-Schutzgruppe durch 30-minütige Hydrierung über 10%-igem Palladium auf Aktivkohle in DCM/Methanol 1:1 bei RT unter Wasserstoff-Normaldruck entfernt. Das entschützte Intermediat wurde anschließend durch Kupplung mit (2,5-Dioxo-2,5-dihydro-1H-pyrrol-1-yl)essigsäure in Gegenwart von HATU und N, N Diisopropylethylamin und schließlich durch Entschützung mit Zinkchlorid in die Titelverbindung überführt.

LC-MS (Methode 1): $R_t$ = 0.83 min; MS (ESIpos): m/z = 785 $(M+H)^+$.

## Intermediat F297

S-{2-[{(1R)-1-[1-Benzyl-4-(2,5-difluorphenyl)-1H-pyrrol-2-yl]-2,2-dimethylpropyl}(pyrrolidin-3-ylmethyl)amino]-2-oxoethyl}-N-[6-(2,5-dioxo-2,5-dihydro-1H-pyrrol-1-yl)hexanoyl]-L-cystein-trifluoressigsäure (1:1) (Isomer 1)

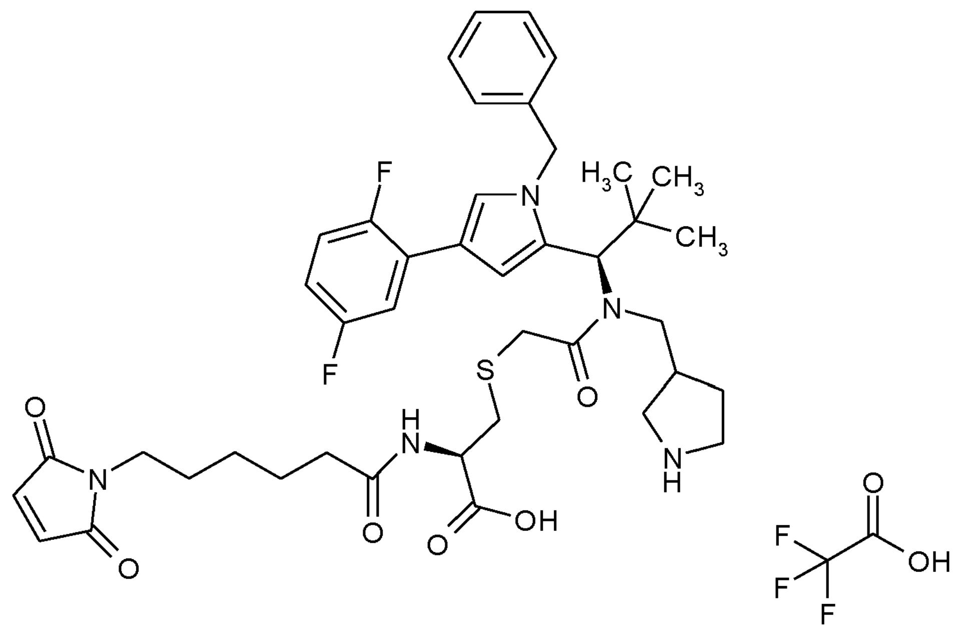

Eine Lösung von 36 mg (0.03 mmol, 68% Reinheit) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Intermediat C92) in 0.74 ml 2,2,2-Trifluoroethanol unter Argon wurde mit 15 mg (0.11 mmol) Zinkchlorid versetzt und das Reaktionsgemisch wurde 7 h bei 50 °C gerührt. Anschließend wurden 32 mg (0.11

mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 6,4 mg (25 % d. Th) der Titelverbindung.

LC-MS (Methode 1): Rt = 0.95 min; MS (ESIpos): m/z = 792 $(M+H-CF_3CO_2H)^+$.

**Intermediat F298**

S-{2-[{(1R)-1-[1-Benzyl-4-(2,5-difluorphenyl)-1H-pyrrol-2-yl]-2,2-dimethylpropyl}(pyrrolidin-3-ylmethyl)amino]-2-oxoethyl}-N-[6-(2,5-dioxo-2,5-dihydro-1H-pyrrol-1-yl)hexanoyl]-L-cystein-trifluoressigsäure (1:1) (Isomer 2)

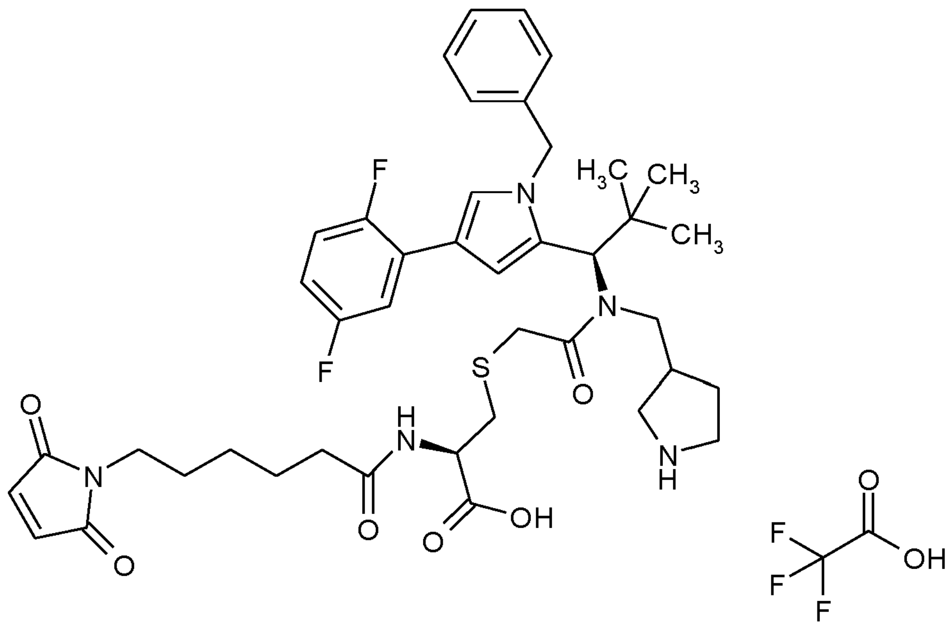

Eine Lösung von 45 mg (0.04 mmol, 71% Reinheit) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Intermediat C91) in 0.94 ml 2,2,2-Trifluoroethanol unter Argon wurde mit 19 mg (0.14 mmol) Zinkchlorid versetzt und das Reaktionsgemisch wurde 3 h bei 50 °C gerührt. Anschließend wurden 42 mg (0.14 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 5,7 mg (18 % d. Th.) der Titelverbindung.

LC-MS (Methode 1): Rt = 0.96 min; MS (ESIpos): m/z = 791 $(M+H-CF_3CO_2H)^+$.

**Intermediat F299**

S-(2-{(3-Aminopropyl)[(R)-[1-benzyl-4-(2,5-difluorphenyl)-1H-pyrrol-2-yl](cyclohexyl)-methyl]amino}-2-oxoethyl)-N-[6-(2,5-dioxo-2,5-dihydro-1H-pyrrol-1-yl)hexanoyl]-L-cystein-trifluoressigsäure (1:1)

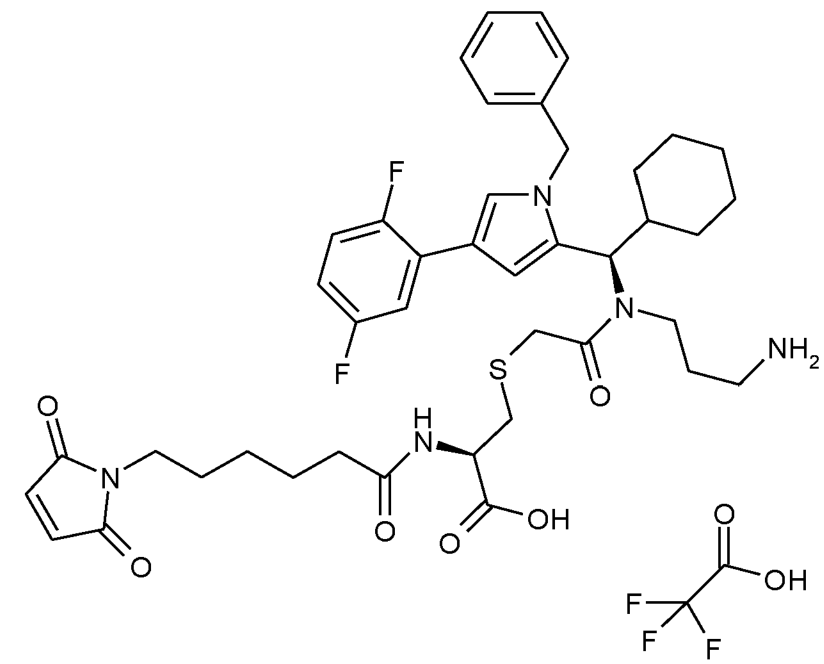

Eine Lösung von 88.0 mg (0.09 mmol) S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-N-[6-(2,5-dioxo-2,5-dihydro-1H-pyrrol-1-yl)hexanoyl]-L-cystein (Intermediat C84) in 1.88 ml 2,2,2-Trifluoroethanol wurde mit 76.8 mg (0.57 mmol) Zinkchlorid versetzt und das Reaktionsgemisch wurde 3 h bei 50 °C gerührt. Anschließend wurden 164.6 mg (0.57 mmol) EDTA hinzugegeben und die Mischung wurde 15 Minuten gerührt. Die Reaktionsmischung wurde mit Ethylacetat versetzt und die org. Phase wurde mehrmals mit Wasser und mit ges. NaCl-Lösung gewaschen. Die organische Phase wurde über Natriumsulfat getrocknet und das Lösemittel im Vakuum verdampft. Der Rückstand wurde mittels präparative HPLC gereinigt. Man erhielt 31 mg (35 % d. Th. ) der Titelverbindung.

LC-MS (Methode 12): $R_t$ = 1.82 min; MS (ESIpos): m/z = 792 $(M+H)^+$.

## Intermediat F300

(2S)-2-Amino-4-[{(1R)-1-[1-benzyl-4-(2,5-difluorphenyl)-1H-pyrrol-2-yl]-2,2-dimethylpropyl}(glycoloyl)amino]-N-(2-{[(2R)-2-(2,5-dioxo-2,5-dihydro-1H-pyrrol-1-yl)propanoyl]amino}ethyl)butanamid

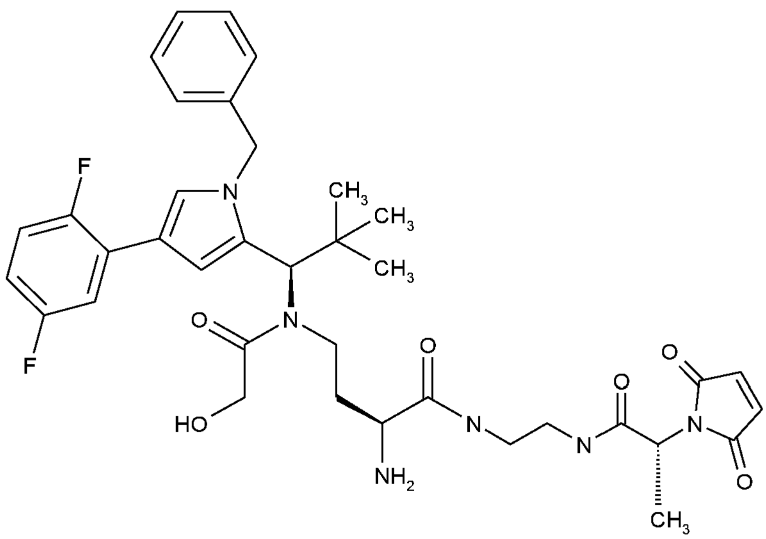

Eine Lösung von 7 mg (0.08 mmol)) 2-(Trimethylsilyl)ethyl-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2R)-2-(2,5-dioxo-2,5-dihydro-1H-pyrrol-1-yl)propanoyl]amino}ethyl)amino]-1-oxobutan-2-yl}carbamat (Intermediat C100) in 0.2 ml 2,2,2-Trifluoroethanol unter Argon wurde mit 11 mg (0.08 mmol) Zinkchlorid versetzt und das Reaktionsgemisch wurde 8 h bei 50 °C gerührt. Anschließend wurden 14 mg (0.05 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 1,6 mg (27 % d. Th.) der Titelverbindung.

LC-MS (Methode 1): Rt = 0.88 min; MS (ESIpos): m/z = 707 $(M+H-CF_3CO_2H)^+$.

## Intermediat F302

S-{2-[{(1R)-1-[1-benzyl-4-(2,5-difluorophenyl)-1H-pyrrol-2-yl]-2,2-dimethylpropyl}(pyrrolidin-3-ylmethyl)amino]-2-oxoethyl}-N-[(2,5-dioxo-2,5-dihydro-1H-pyrrol-1-yl)acetyl]-L-cysteine trifluoroacetate (1:1) (Isomer 1

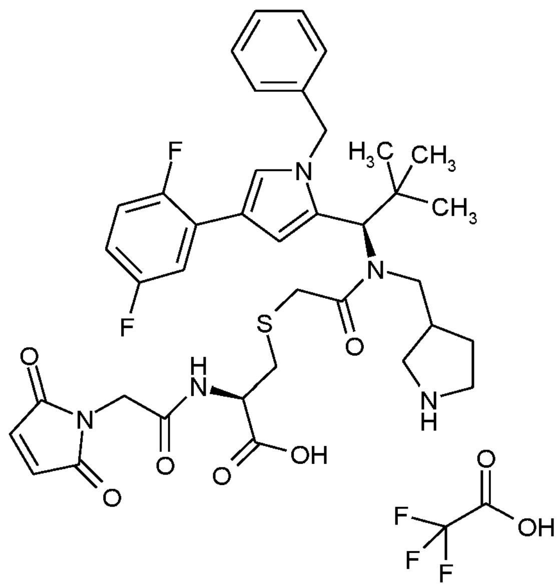

Eine Mischung von 56.9 mg (58.2 mmol, 85 % Reinheit) S-[2-({(1R)-1-[1-Benzyl-4-(2,5-difluorphenyl)-1H-pyrrol-2-yl]-2,2-dimethylpropyl}{[(1-(tert-butoxycarbonyl)pyrrolidin-3-yl]methyl}amino)-2-oxoethyl]-N-[(2,5-dioxo-2,5-dihydro-1H-pyrrol-1-yl)acetyl]-L-cystein (Intermediat C94) in 1.4 ml 2,2,2-Trifluoroethanol unter Argon wurde mit 31.7 mg (0.23 mmol) Zinkchlorid versetzt und das Reaktionsgemisch wurde 3 h bei 50 °C gerührt. Anschließend wurden 68.0 mg (0.23 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 7 mg (13 % d. Th) der Titelverbindung.

LC-MS (Methode 1): $R_t$ = 0.91 min; MS (ESIpos): m/z = 736 $(M+H-CF_3CO_2H)^+$.

## Intermediat F305

N-{(1R)-1-[1-Benzyl-4-(2,5-difluorphenyl)-1H-pyrrol-2-yl]-2,2-dimethylpropyl}-22-(2,5-dioxo-2,5-dihydro-1H-pyrrol-1-yl)-6,17-dioxo-N-(pyrrolidin-3-ylmethyl)-10,13-dioxa-3-thia-7,16-diazadocosan-1-amid (1:1) Trifluoressigsäure (Isomer 2)

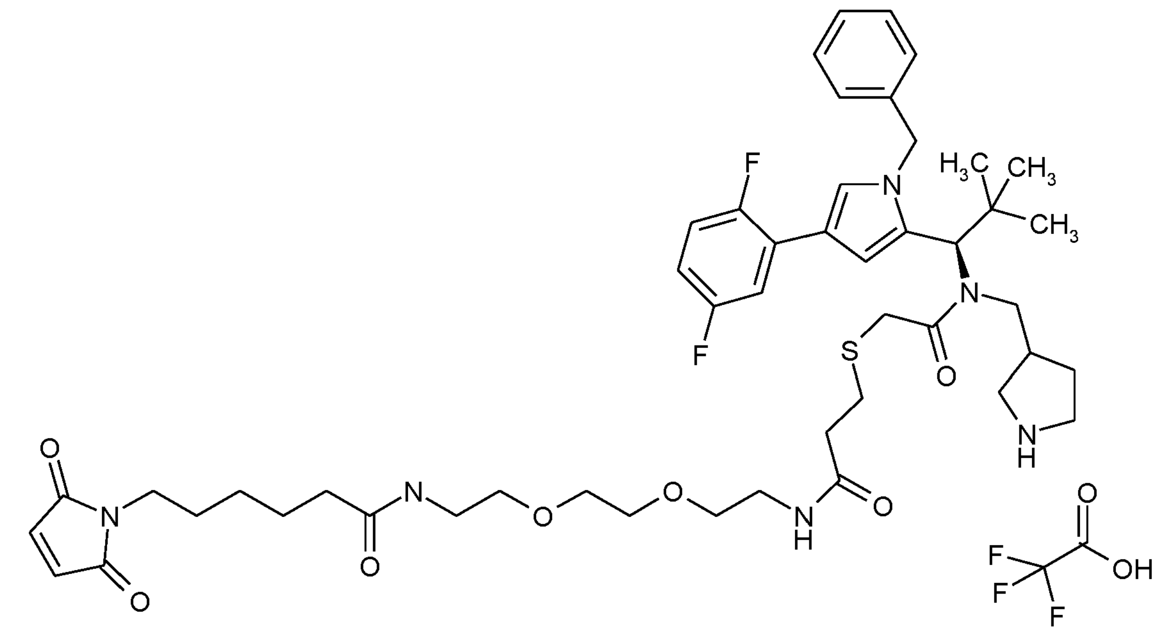

Eine Lösung von 24.80 mg (0.02 mmol) tert-Butyl-3-[2-{(1R)-1-[1-benzyl-4-(2,5-difluorphenyl)-1H-pyrrol-2-yl]-2,2-dimethylpropyl}-24-(2,5-dioxo-2,5-dihydro-1H-pyrrol-1-yl)-3,8,19-trioxo-12,15-dioxa-5-thia-2,9,18-triazatetracos-1-yl]pyrrolidin-1-carboxylat (Intermediat C99) in 0.65 ml 2,2,2-Trifluoroethanol wurde mit 13.42 mg (0.10 mmol) Zinkchlorid versetzt und das Reaktionsgemisch wurde 8 h bei 50 °C gerührt. Anschließend wurden 28.78 mg (0.10 mmol) EDTA hinzugegeben und die Mischung wurde 15 Minuten gerührt. Die Reaktionsmischung wurde mit Ethylacetat versetzt und die org. Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 10 mg (44 % d. Th.) der Titelverbindung.

LC-MS (Methode 5): Rt = 3.11 min; MS (ESIpos): m/z = 907 $(M+H-CF_3CO_2H)^+$.

**Intermediat F306**

S-{2-[{(1R)-1-[1-Benzyl-4-(2,5-difluorphenyl)-1H-pyrrol-2-yl]-2,2-dimethylpropyl}(3-{[N2-(tert-butoxycarbonyl)-L-asparaginyl]amino}propyl)amino]-2-oxoethyl}-N-[1-(2,5-dioxo-2,5-dihydro-1H-pyrrol-1-yl)-2,18-dioxo-6,9,12,15-tetraoxa-3-azaoctadecan-18-yl]-L-cystein

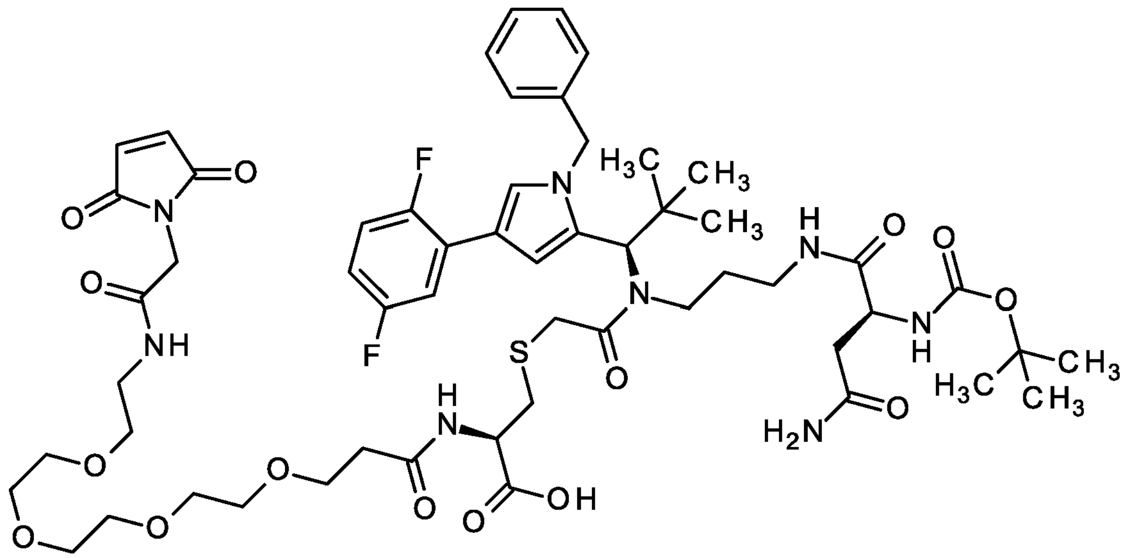

Eine Lösung von 22 mg von Imtermediat F257 (0.02 mmol) in 0,22 mL DMF bei RT, wurde mit 10,7 µl N,N-Diisopropylethylamin (0,062 mmol) und 10 mg N-alpha-Boc-L-asparagine N-hydroxysuccinimide ester versetzt. Die Mischung wurde 15 Minuten gerührt. Es wurde Wasser (2 mL) und ACN (4 mL) addiert. Die Reaktionslösung wurde mittels präparativer HPLC gereinigt (Eluent: ACN/Wasser + 0.1% TFA, Gradient =1:9 →3:2). Man erhielt 21 mg (87% d. Th.) der Titelverbindung.

LC-MS (Methode 1): $R_t$ = 1,07 min; MS (ESIpos): m/z = 1171 $(M+H)^+$.

**Intermediat F307**

S-[2-([3-(L-Asparaginylamino)propyl]{(1R)-1-[1-benzyl-4-(2,5-difluorphenyl)-1H-pyrrol-2-yl]-2,2-dimethylpropyl}amino)-2-oxoethyl]-N-[1-(2,5-dioxo-2,5-dihydro-1H-pyrrol-1-yl)-2,18-dioxo-6,9,12,15-tetraoxa-3-azaoctadecan-18-yl]-L-cystein -trifluoressigsäure (1:1)

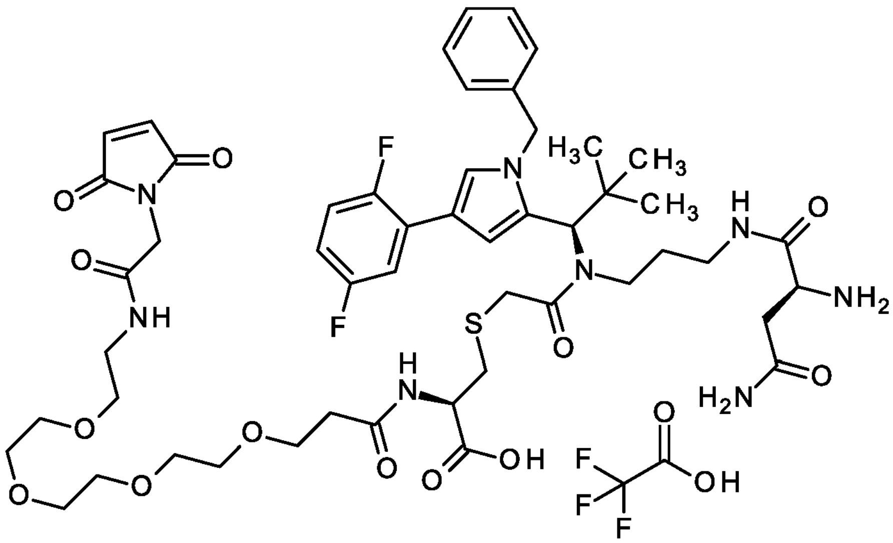

Eine Lösung von 21 mg Imtermediat F306 (0.017 mmol) in 1,5 mL 2,2,2-trifluoroethanol, wurde mit 24,3 mg (0.178 mmol) Zinkchlorid versetzt und das Reaktionsgemisch wurde 60 min bei 60 °C gerührt. Anschließend wurden 52,1 mg (0.178 mmol) EDTA hinzugegeben und die Mischung wurde 15 Minuten gerührt. Es wurde Wasser (2 mL) und ACN (4 mL) addiert. Die Reaktionslösung wurde filtriert und mittels präparativer HPLC gereinigt (Eluent: ACN/Wasser + 0.1% TFA, Gradient = 1:9 → 3:2). Man erhielt 18 mg (85% d. Th.) der Titelverbindung.

LC-MS (Methode 1): $R_t$ = 0,87 min; MS (ESIpos): m/z = 1071 $(M+H)^+$.

**Allgemeine Vorschrift zur Synthese der APDC oder ADC Precursor (Intermediatserie Q)**

**APDC-Precursor:**

Die zuvor beschriebenen Intermediate der F-Serie (F1-F305) oder gegebenenfalls geschützte Vorstufen können gemäß Schema 1 gegebenenfalls auch nach abschließender Entschützung in die APDC-Precurser Q überführt werden. Nach Freisetzung der N-terminalen Aminogruppe der Legumain-spaltbaren Kopfgruppe und nachfolgender Modifizierung der APDC Percursormoleküle mit strukturdiversen Substituenten $Z_1$ (Schema 1) kann auch eine Verbesserung des Eigenschaftsprofils der APDCs erreicht werden. Die proteinreaktive Gruppe zur Anbindung des APDC-Percursor Moleküls an den Antikörper kann sich dabei in den Positionen R1, R2 oder R3 befinden.

Eine examplarische Vorschrift ist hier dargestellt:

0.037 mmol eines Intermediats F1-Fx werden in 1-20 mL bevorzugt 5-10 mL eines geeigneten Lösungsmittels wie beispielsweise DMF, DMSO, DCM, Chloroform, Toluol, THF, Methanol oder einer Mischung derselben aufgenommen und mit 0.039 mmol eines N-terminal modifizierten Tripeptid-Derivats wie z.B. Intermediat L92 sowie mit 0.041 mmol eines gängigen Kupplungsreagenzes wie z. B. HATU, EDCI/HOBT, BEP etc. und 0.11 mmol einer gängigen Base wie

z.B. N,N-Diisopropylethylamin, Triethylamin, 4-Methylmorpholin etc. versetzt. Nach 5 min Rühren bei RT wird der Ansatz mit 2 Tropfen Trifluoressigsäure angesäuert und eingeengt. Der Rückstand wird durch präparative HPLC gereinigt. Die entsprechenden Fraktionen werden im Vakuum eingeengt und der Rückstand aus Acetonitril/Wasser lyophilisiert.

Wenn es sich bei der besagten N-terminalen Modifizierung des angeknüpften Tripeptid-Derivats um eine Schutzgruppe handelt, kann diese anschließend nach bekannten Methoden abgespalten werden, beispielsweise eine Z-Schutzgruppe bevorzugt mittels Hydrogenolyse, eine Boc-Schutzgruppe mittels Säurespaltung oder mittels Zinkchlorid , eine Fmoc-Schutzgruppe durch basische Spaltung oder eine Teoc-Gruppe mittels Fluoriden oder mit Zinkchorid.

Schließlich kann die so freigesetzte Aminogruppe zur Verbesserung des Eigenschaftsprofils acyliert oder alkyliert werden, beispielsweise mit aminreaktiven Gruppen wie Aktivestern, Säurechloriden, Isocyanaten etc. oder durch Kupplung mit Carbonsäurederivaten in

Gegenwart eines gängigen Kupplungsreagenzes wie z. B. HATU, EDCI/HOBT, BEP etc. sowie einer gängigen Base wie z.B. N,N-Diisopropylethylamin, Triethylamin, 4-Methylmorpholin etc. Falls noch vorhanden können weitere Schutzgruppen im Molekül in einem letzten Schritt entfernt werden.

Schema 1:

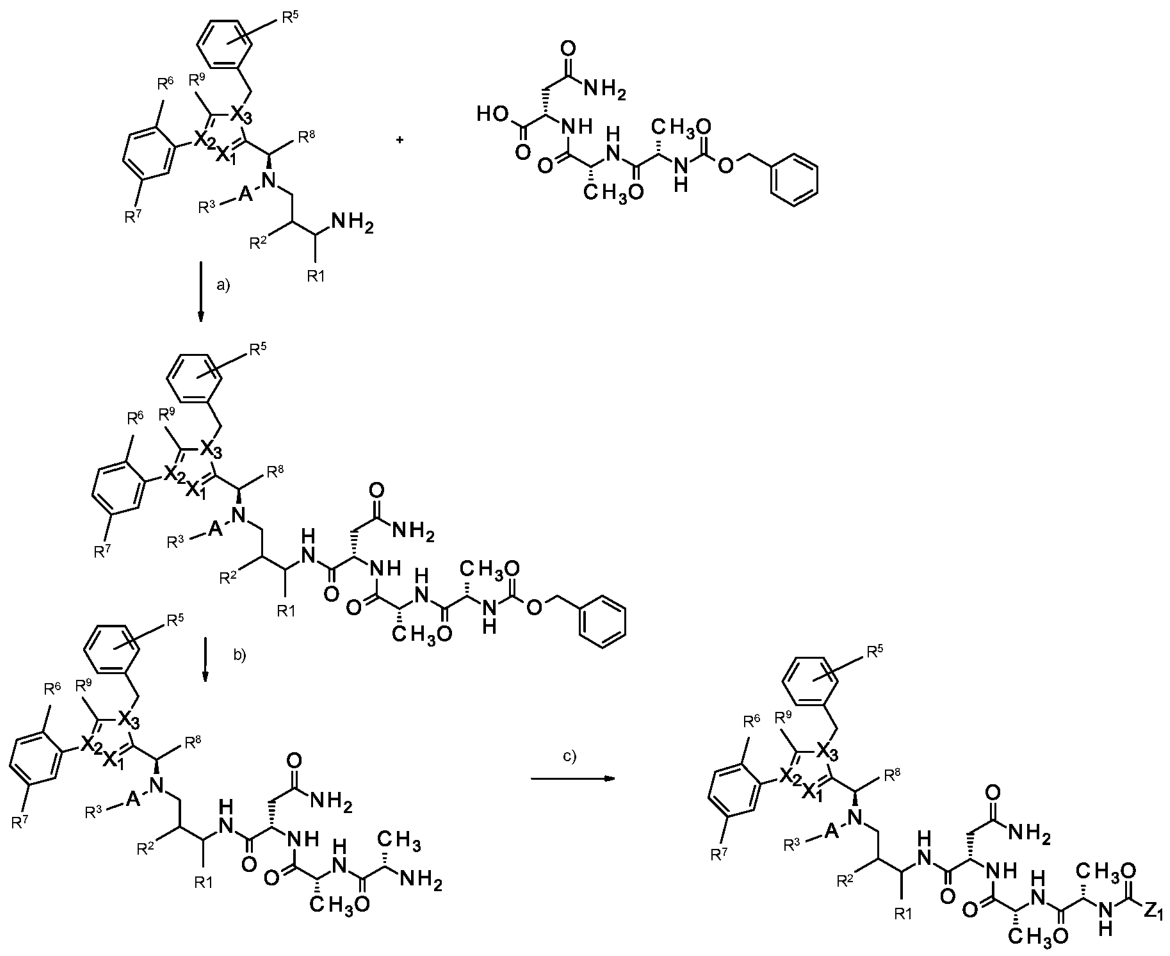


[a): HATU, DMF, N,N-Diisopropylethylamin, RT oder EDCI, HOBT, N,N-Diisopropylethylamin, DMF, RT b) $H_2$, 10% Pd-C, MeOH, RT; c) $Z_1$-COOH, EDCI, HOBT, N,N-Diisopropylethylamin, DMF, RT oder $Z_1$-COOH, HATU, N,N-Diisopropylethylamin, DMF, RT oder $Z_1$-COOSu, N,N-Diisopropylethylamin, DMF, RT]

**ADC Percursor:**

Weiterhin können andere Intermediate, die noch keine proteinreaktiven Gruppen enthalten, gemäß Schema 2 und 3 in Legumain-spaltbare ADC-Precursor überführt werden:

Schema 2:

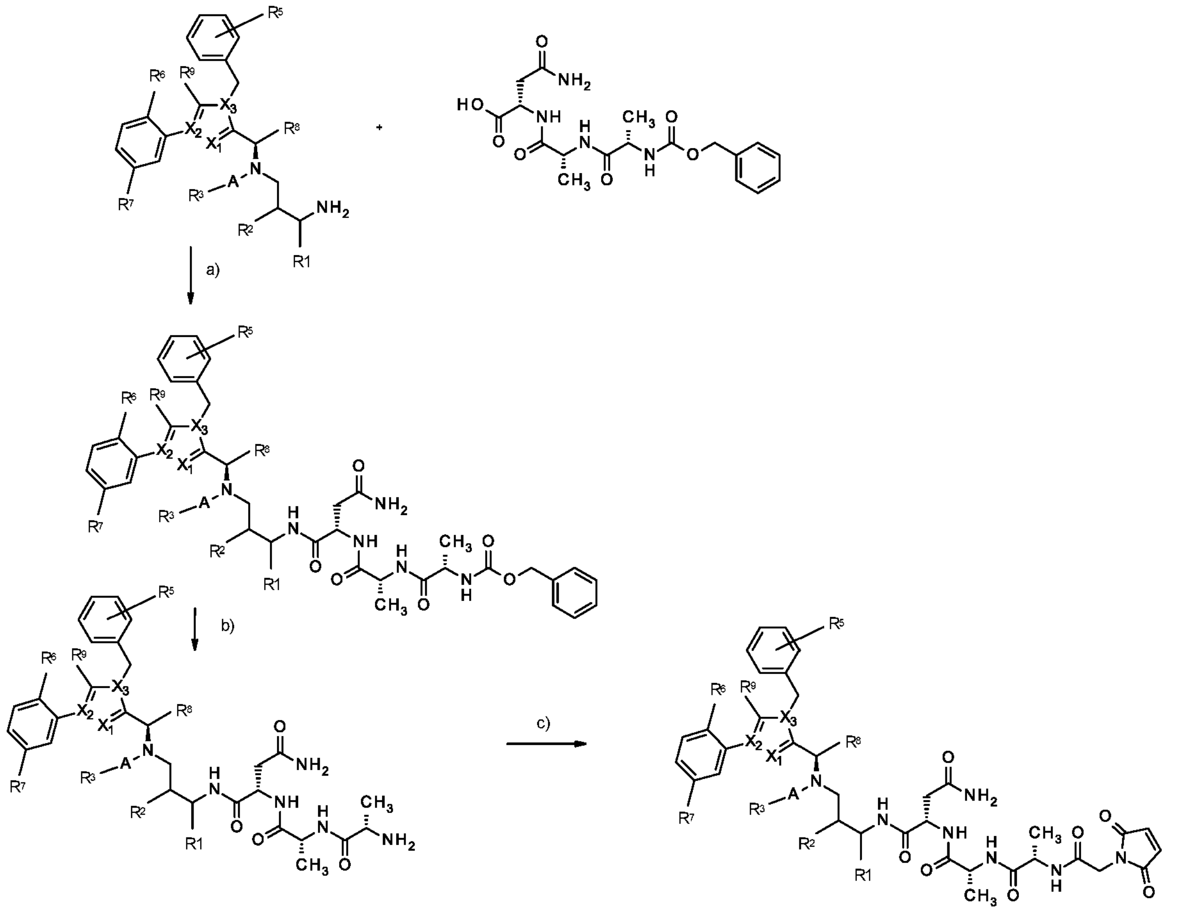


[a): HATU, DMF, N,N-Diisopropylethylamin, RT oder EDCI, HOBT, N,N-Diisopropylethylamin, DMF, RT b) $H_2$, 10% Pd-C, MeOH, RT; c) 1,1'-[(1,5-Dioxopentan-1,5-diyl)bis(oxy)]dipyrrolidin-2,5-dion, N,N-Diisopropylethylamin, DMF, RT]

Schema 3:

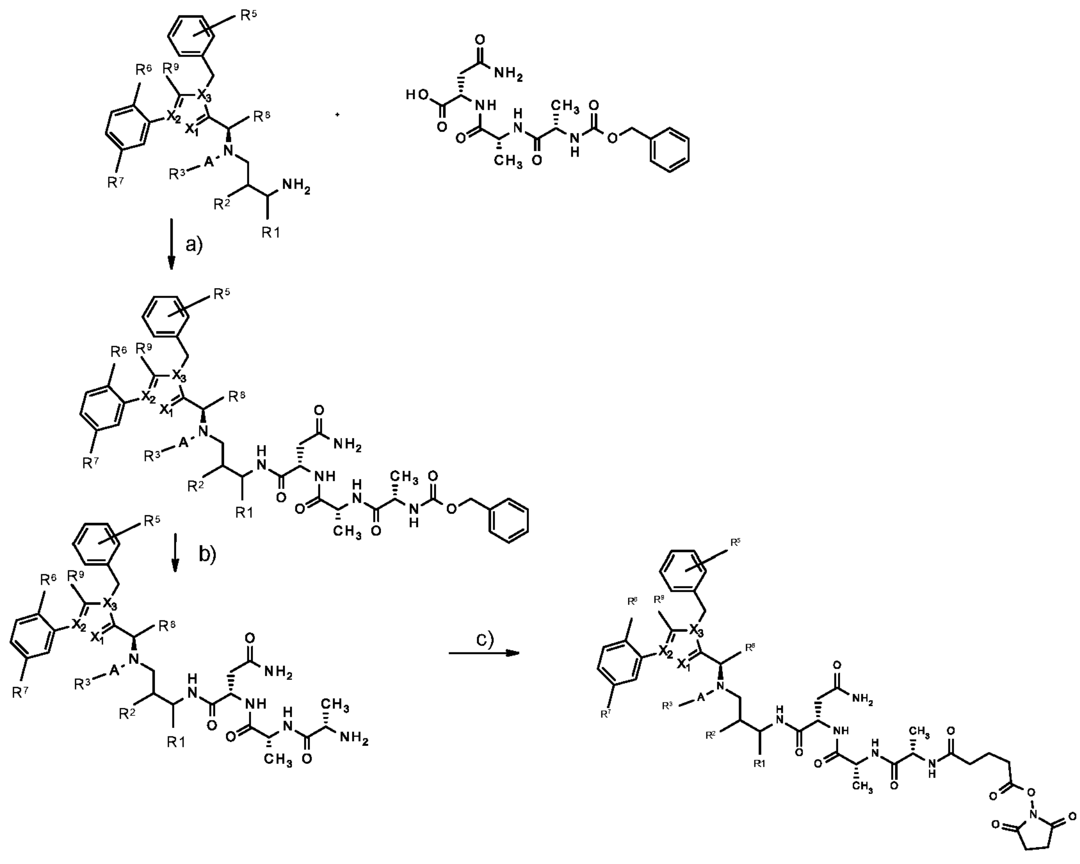


[a): HATU, DMF, N,N-Diisopropylethylamin, RT oder EDCI, HOBT, N,N-Diisopropylethylamin, DMF, RT b) $H_2$, 10% Pd-C, MeOH, RT; c) ) 1,1'-[(1,5-Dioxopentan-1,5-diyl)bis(oxy)]dipyrrolidin-2,5-dion, N,N-Diisopropylethylamin, DMF, RT]

Alternativ zu der in Schemata 1-3 dargestellten Benzyloxycarbonyl-Gruppe können andere in der Peptidchemie etablierte Schutzgruppen eingesetzt werden und nach entsprechenden ebenso bekannten Methoden abgespalten werden. Die Auswahl der Schutzgruppenstrategie erfolgt nach entsprechenden dem Fachmann bekannten Anforderungen zur Kompatibilität mit anderen im Molekül vorkommenden Strukturelementen. Falls noch vorhanden können weitere Schutzgruppen im Molekül in einem letzten Schritt entfernt werden.

Die Synthesen können auch ggf. in ihrer Sequenz umgestellt werden.

Weiterhin kann die proteinreaktive Gruppe im Sinne der Linkerstrukturen L1-L2 im Rahmen der Ansprüche variiert werden.

**Intermediat R1**

N-[(Benzyloxy)carbonyl]-L-alanyl-D-alanyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

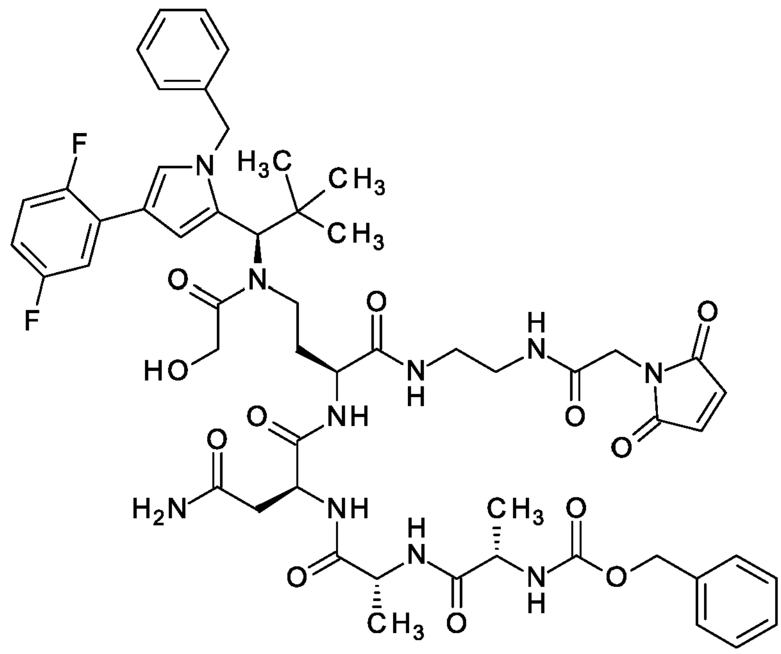

7.5 mg (0.009 mmol) von Intermediat F104 wurden in 2 ml DMF gelöst und nach Zugabe von 5.3 mg (0.014 mmol) HATU, 5 μL N,N-Diisopropylethylamin und 5.8 mg (0.011 mmol) von Intermediat L108 15 min bei RT gerührt. Anschließend wurde der Ansatz im Vakuum eingeengt und der Rückstand durch präparative HPLC gereinigt. Es wurden 3.2 mg (31% d. Th.) der Titelverbindung erhalten.

LC-MS (Methode 1): $R_t$ = 1.10 min; MS (ESIpos): m/z = 1083 $(M+H)^+$.

## Intermediat R2

N-isonicotinyl-L-alanyl-D-alanyl-N[1]-{(2S)-4-[{(1R)-1-[1-benzyl-4-(2,5-difluorphenyl)-1H-pyrrol-2-yl]-2,2-dimethylpropyl}(glycolyl)amino]-1-[(2-{[(2,5-dioxo-2,5-dihydro-1H-pyrrol-1-yl)acetyl]amino}ethyl)amino]-1-oxobutan-2-yl}-L-aspartamid trifluoracetat (1:1)

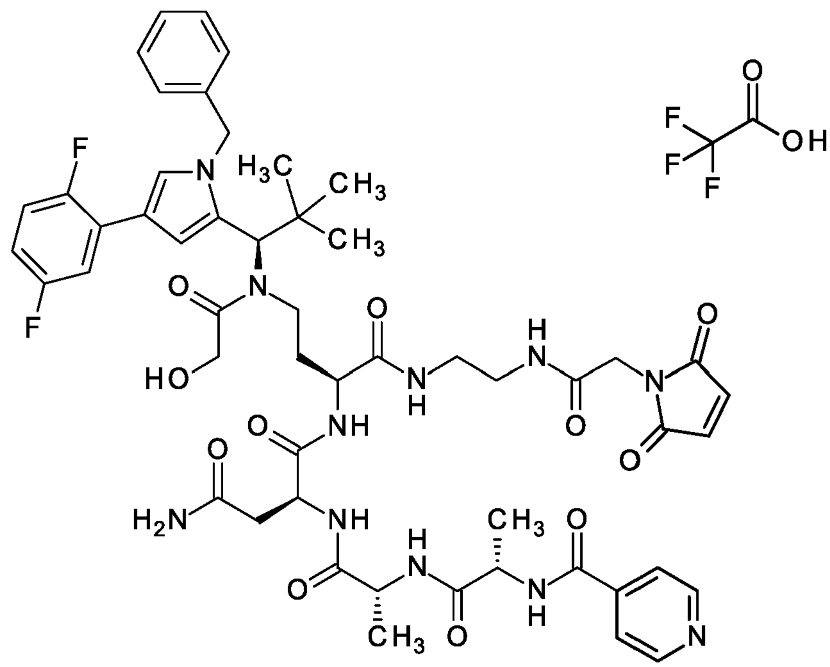

12.7 mg (0.016 mmol) von Intermediat F104 wurden in Analogie zu Intermediat R1mit 7.8 mg (0.016 mmol) Intermediat L109 gekuppelt. Es wurden 4.5 mg (24% d. Th.) der Titelverbindung erhalten.

LC-MS (Methode 12): $R_t$ = 1.74 min; MS (ESIpos): m/z = 1054 $(M+H)^+$.

**Intermediat** R3

N-(pyridin-4-ylacetyl)-L-alanyl-D-alanyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid trifluoracetat (1:1)

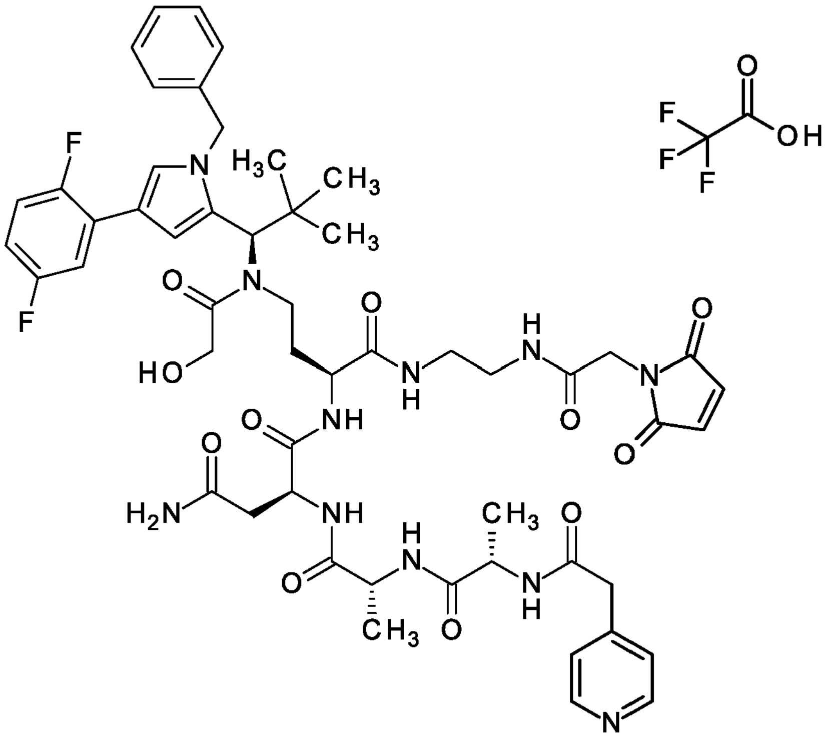

100 mg (0.124 mmol) von Intermediat F104 wurden in Analogie zu Intermediat R1 mit 75 mg (0.15 mmol) Intermediat L110 gekuppelt. Nach Reinigung mittels präparativer HPLC wurden 58 mg (39% d.Th.) der Titelverbindung erhalten.

LC-MS (Methode 1): $R_t$ = 0.88 min; MS (ESIpos): m/z = 1068 $(M+H)^+$.

**Intermediat R4**

N-Acetyl-L-alanyl-D-alanyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

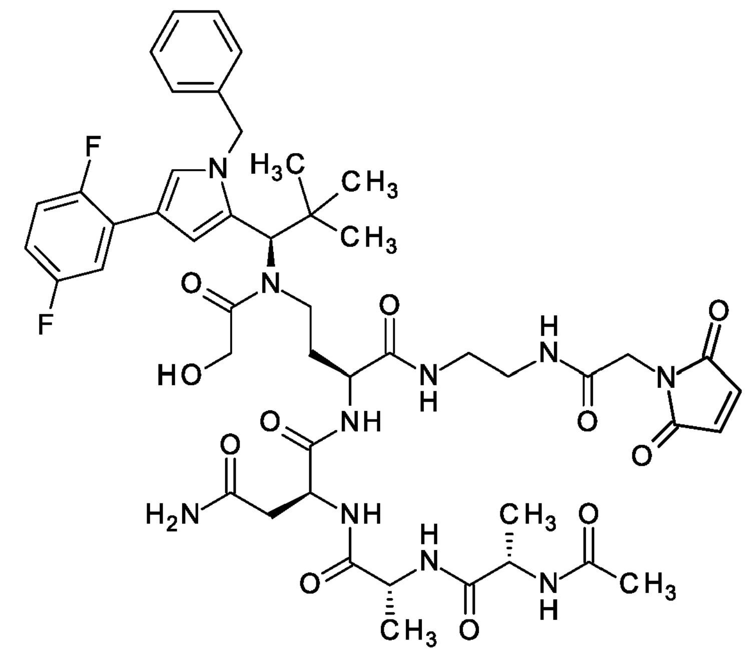

20 mg (0.025 mmol) von Intermediat F104 wurden in Analogie zu Intermediat R1 mit 30.6 mg (0.062 mmol) von Intermediat L111 gekuppelt. Es wurden 2.3 mg (9% d. Th.) der Titelverbindung erhalten.

LC-MS (Methode 1): $R_t$ = 0.97 min; MS (ESIpos): m/z = 991 $(M+H)^+$.

**Intermediat R5**

N-[(2,5-Dioxo-2,5-dihydro-1H-pyrrol-1-yl)acetyl]-L-alanyl-D-alanyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid

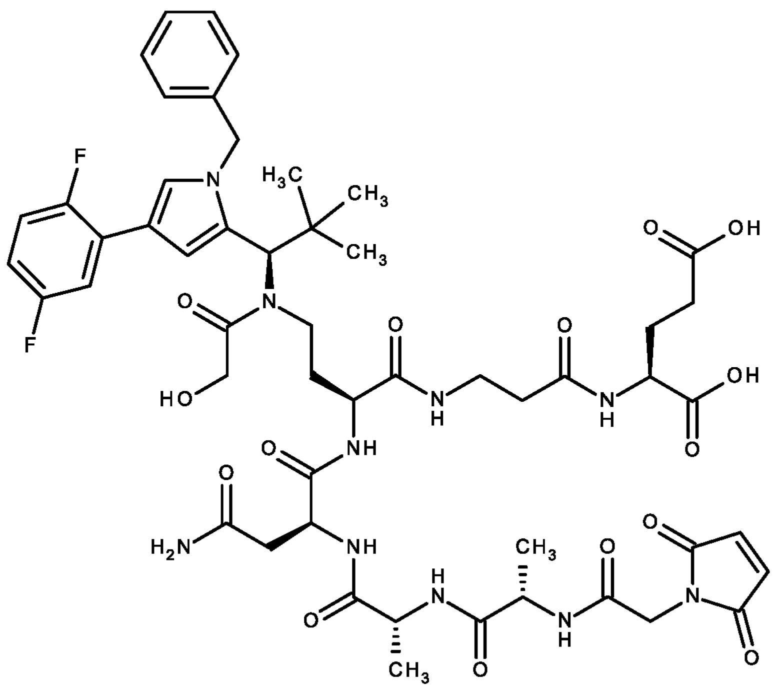

Die Titelverbindung wurde ausgehend von Verbindung C110 zunächst durch Kupplung mit Intermediat L108

in Gegenwart von HATU und N,N-Diisopropylethylamin hergestellt. Im nächsten Schritt wurden alle Schutzgruppen durch 1.5-stündige Hydrierung über 10%-igem Palladium auf Aktivkohle in Methanol unter Wasserstoff-Normaldruck bei RT entfernt und das entschützte Intermediat anschließend durch Umsetzung mit 1-{2-[(2,5-Dioxopyrrolidin-1-yl)oxy]-2-oxoethyl}-1H-pyrrol-2,5-dion in Gegenwart von N,N-Diisopropylethylamin in die Titelverbindung überführt.

LC-MS (Methode 1): $R_t$ = 0.94 min; MS (ESIpos): m/z = 1107 [M+H]+.

## Intermediat R6

N-{5-[(2,5-Dioxopyrrolidin-1-yl)oxy]-5-oxopentanoyl}-L-alanyl-D-alanyl-N[1]-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid

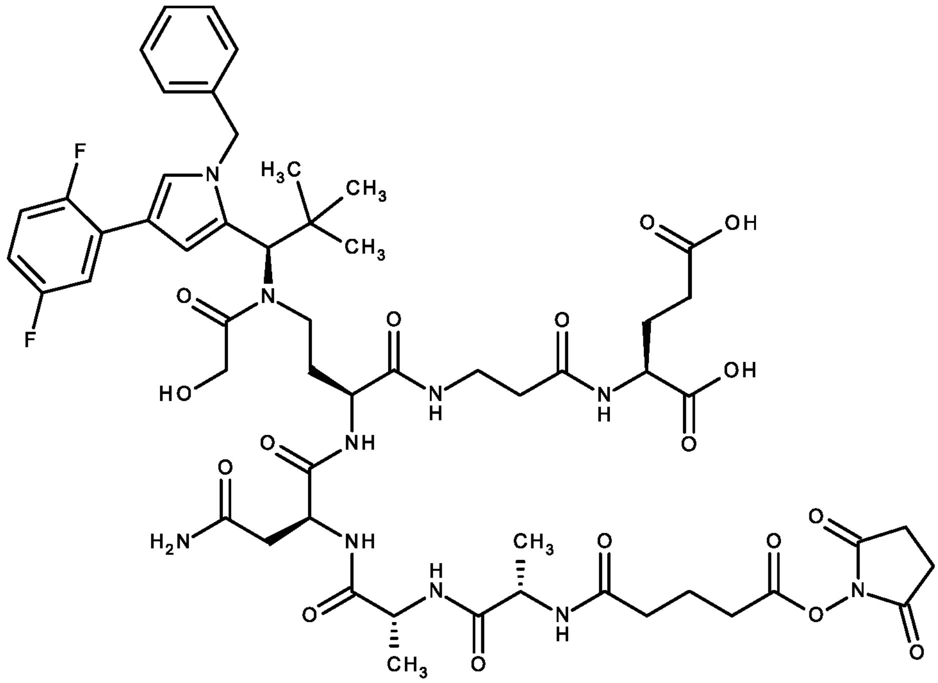

Die Titelverbindung wurde ausgehend von Verbindung C110 zunächst durch Kupplung mit Intermediat L108 in Gegenwart von HATU und N,N-Diisopropylethylamin hergestellt. Im nächsten Schritt wurden alle Schutzgruppen durch 1.5-stündige Hydrierung über 10%-igem Palladium auf Aktivkohle in Methanol unter Wasserstoff-Normaldruck bei RT entfernt und das entschützte Intermediat anschließend durch Umsetzung mit 1,1'-[(1,5-Dioxopentan-1,5-diyl)bis(oxy)]dipyrrolidin-2,5-dion in Gegenwart von N,N-Diisopropylethylamin in DMF in die Titelverbindung überführt.

LC-MS (Methode 1): $R_t$ = 0.95 min; MS (ESIpos): m/z = 1181 [M+H]+.

## Intermediat R7

N-(Pyridin-4-ylacetyl)-L-alanyl-D-alanyl-N[1]-[(2S)-4-[{(1R)-1-[1-benzyl-4-(2,5-difluorphenyl)-1H-pyrrol-2-yl]-2,2-dimethylpropyl}(glycoloyl)amino]-1-{[2-({5-[(2,5-dioxopyrrolidin-1-yl)oxy]-5-oxopentanoyl}amino)ethyl]amino}-1-oxobutan-2-yl]-L-aspartamid

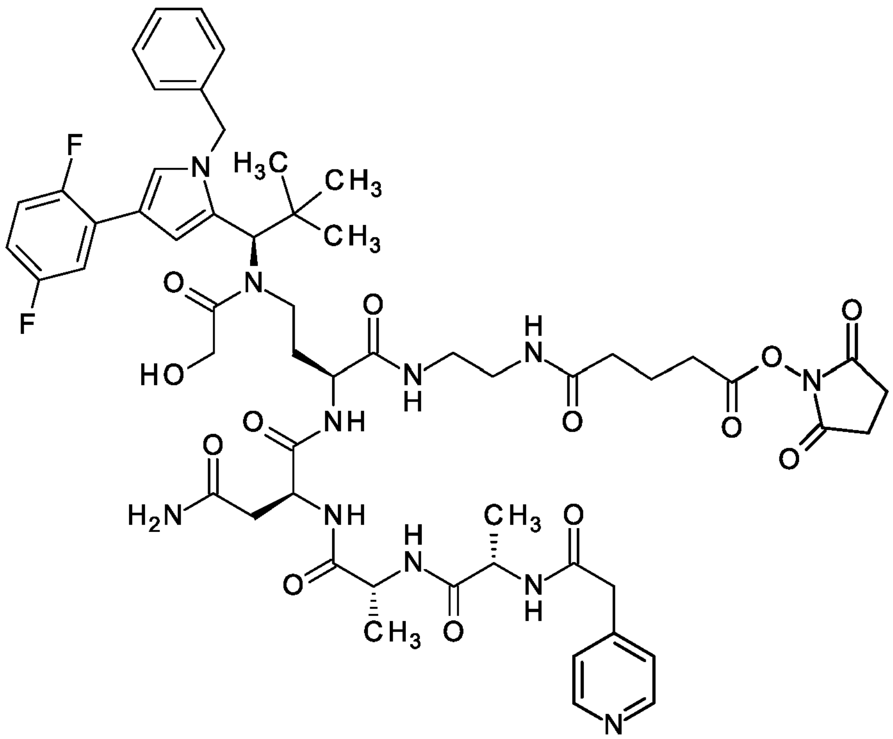

Die Titelverbindung wurde hergestellt durch Kupplung von Intermediat C102 mit tert-Butyl (2-aminoethyl)carbamat in Gegenwart von HATU und N,N-Diisopropylethylamin, anschließender Abspaltung der Z-Schutzgruppe durch 1-stündige Hydrierung über 10%-igem Palladium auf Aktivkohle in DCM-Methanol 1:1 bei RT unter Wasserstoff-Normaldruck, dann Kupplung mit Intermediat L110 in Gegenwart von HATU und N,N-Diisopropylethylamin und folgender Abspaltung der Boc-Gruppe durch 2-stündiges Rühren bei 50°C in Trifluorethanol mit 6 Equiv. Zinkchlorid. Im letzten Schritt wurde das erhaltene Intermediat in DMF aufgenommen und mit 1,1'-[(1,5-Dioxopentan-1,5-diyl)bis(oxy)]dipyrrolidin-2,5-dion in Gegenwart von N,N-Diisopropyl ethylamin in die Titelverbindung überführt.

LC-MS (Methode 1): $R_t$ = 0.84 min; MS (ESIpos): m/z = 1142 [M+H]+.

## Intermediat R8

N-(Pyridin-4-ylacetyl)-L-alanyl-D-alanyl-N$^1$-[(2S)-4-[{(1R)-1-[1-benzyl-4-(2,5-difluorphenyl)-1H-pyrrol-2-yl]-2,2-dimethylpropyl}(glycoloyl)amino]-1-({2-[(N-{5-[(2,5-dioxopyrrolidin-1-yl)oxy]-5-oxopentanoyl}glycyl)amino]ethyl}amino)-1-oxobutan-2-yl]-L-aspartamid

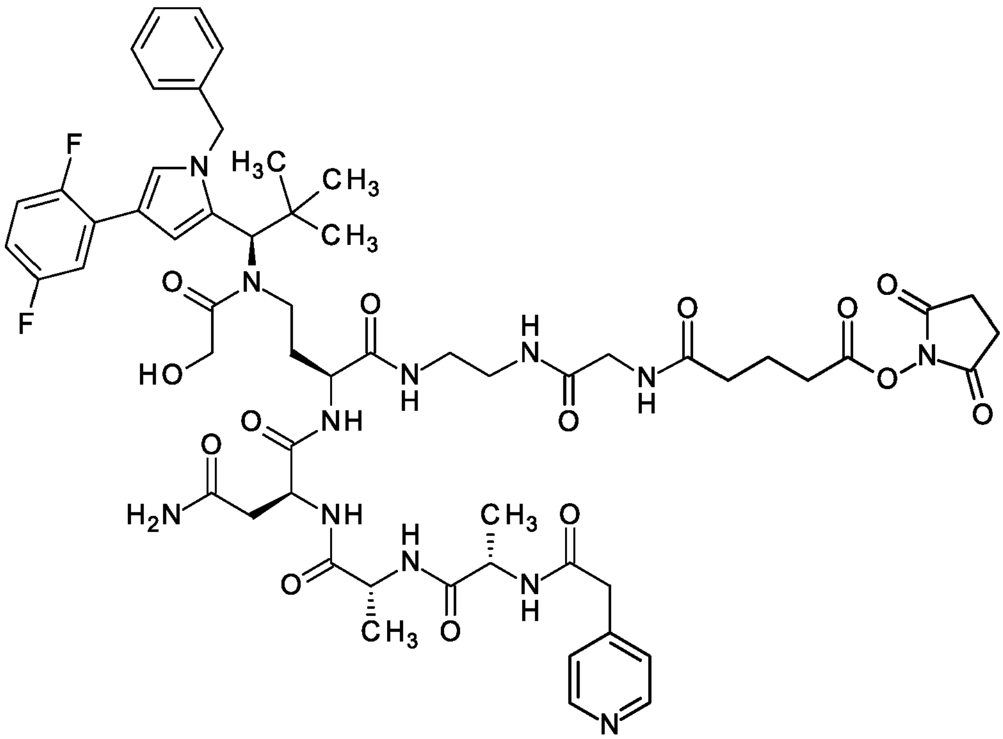

Die Titelverbindung wurde in Analogie zu Intermediat R7 ausgehend von Intermediat C102 und Intermediat L112 hergestellt.

LC-MS (Methode 1): $R_t$ = 0.88 min; MS (ESIpos): m/z = 1199 $[M+H]^+$.

## Intermediat R9

N-[(2,5-Dioxo-2,5-dihydro-1H-pyrrol-1-yl)acetyl]-L-alanyl-D-alanyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1R)-1,3-dicarboxypropyl]amino}-3-oxopropyl)amino]-1-oxobutan-2-yl}-L-aspartamid

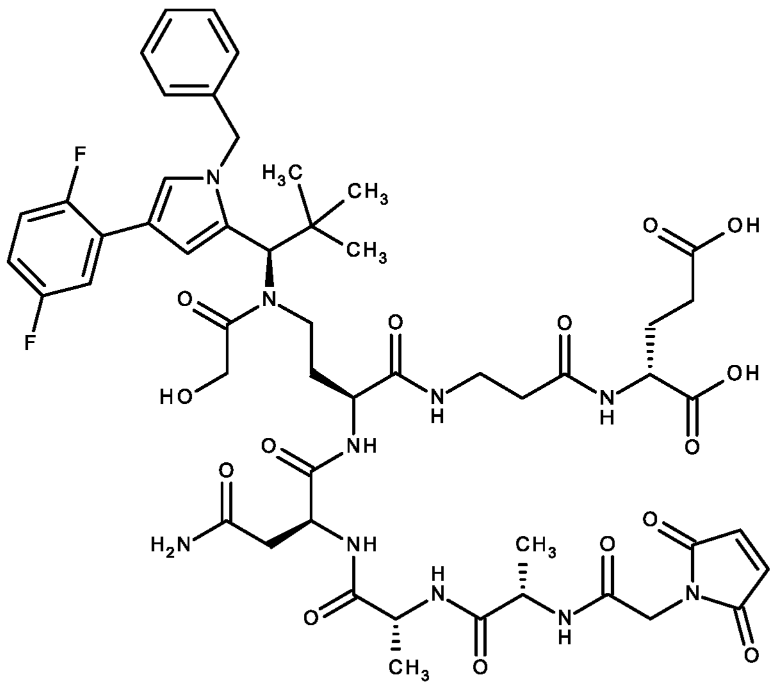

Die Titelverbindung wurde ausgehend von Verbindung C111 zunächst durch Kupplung mit Intermediat L107 in Gegenwart von HATU und N,N-Diisopropylethylamin und anschließende Entschützung mittels Zinkchlorid hergestellt.

LC-MS (Methode 1): $R_t$ = 0.9 min; MS (ESIpos): m/z = 1107 $[M+H]^+$.

__Intermediat R10__

Trifluoressigsäure --N-(pyridin-4-ylacetyl)-L-alanyl-D-alanyl-N1-[13-{(1 R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]-L-aspartamid (1:1)

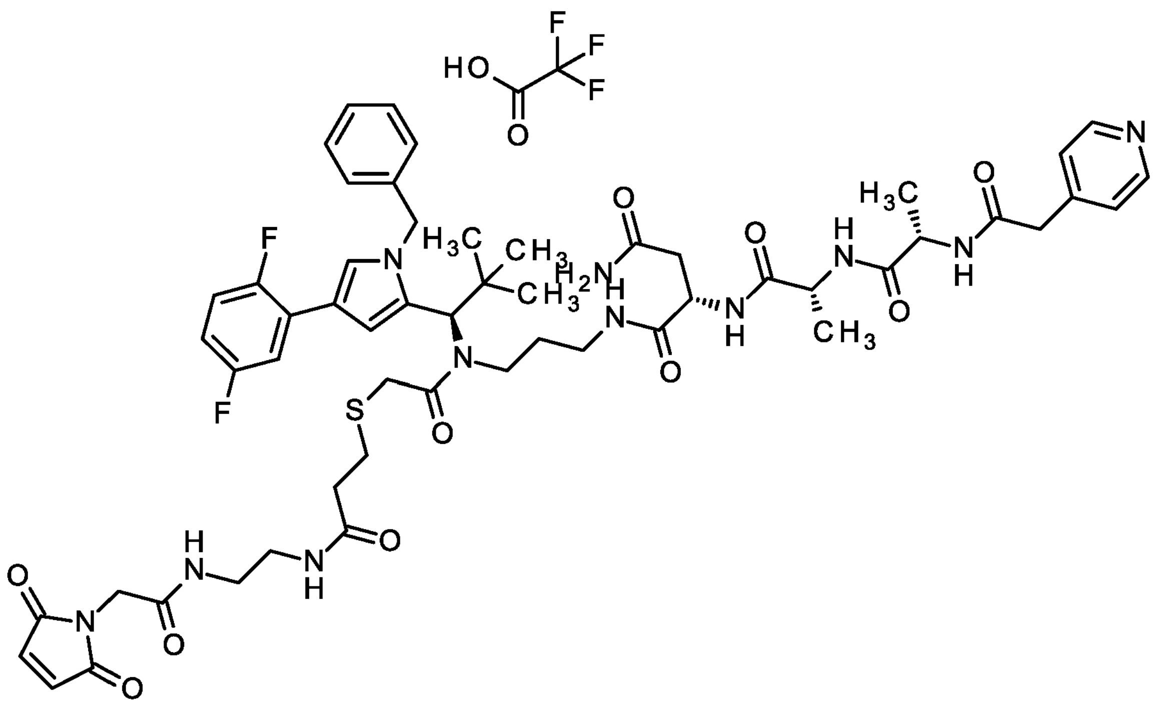

15.0 mg (17.6 µmol) Trifluoressigsäure --3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-(2-{[(2,5-dioxo-2,5-dihydro-1H-pyrrol-1-yl)acetyl]amino}ethyl)propanamid (1:1) (Intermediat F240) wurden zusammen mit 11.6 mg (22.9 µmol) N-(Pyridin-4-ylacetyl)-L-alanyl-D-alanyl-L-asparagin -trifluoressigsäure (1:1) (Intermediat L110) in 2.0 mL Acetonitril vorgelegt. Dann wurden 25 µl (0.14 mmol) N,N-Diisopropylethylamin zugegeben und 14 µl (23 µmol) T3P (50 % in Ethylacetat) zugetropft. Die Reaktionsmischung wurde 30 Minuten bei RT gerührt. Die Reaktionsmischung wurde direkt mittles präparativer RP-HPLC (Säule: Reprosil 250x30; 10µ, Fluss: 50 mL/min, MeCN/Wasser, 0.1%TFA). Die Lösemittel wurden im Vakuum verdampft und der Rückstand im Hochvakuum getrocknet. Man erhielt 5.70 mg (26 % d. Th.) der Titel Verbindung.

LC-MS (Methode 1): $R_t$ = 0.86 min; MS (ESIpos): m/z = 1112 $(M+H)^+$.

## Intermediat R11

N-(Pyridin-4-ylacetyl)-L-alanyl-D-alanyl-$N^1$-[(2S)-4-[{(1R)-1-[1-benzyl-4-(2,5-difluorphenyl)-1H-pyrrol-2-yl]-2,2-dimethylpropyl}(glycoloyl)amino]-1-({4-[(2,5-dioxopyrrolidin-1-yl)oxy]-4-oxo butyl}amino)-1-oxobutan-2-yl]-L-aspartamid

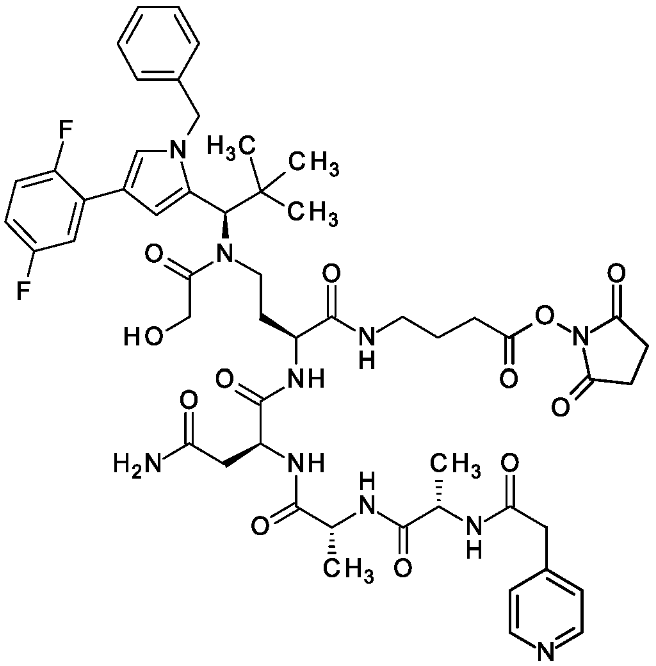

20 mg (31 µmol) von Intermediat C114 wurden zusammen mit 11.6 mg (22.9 µmol) N-(Pyridin-4-ylacetyl)-L-alanyl-D-alanyl-L-asparagin -trifluoressigsäure (1:1) (Intermediat L110) in 5.0 mL DMF vorgelegt. Dann wurden 11 µl N,N-Diisopropylethylamin und 21 mg (55 µmol) HATU zugegeben. Die Reaktionsmischung wurde 60 Minuten bei RT gerührt. Die Reaktionsmischung wurde direkt mittles präp. RP-HPLC (Säule: Reprosil 250x30; 10µ, Fluss: 50 mL/min, MeCN/Wasser, 0.1%TFA) gereinigt. Die Lösemittel wurden im Vakuum verdampft und der Rückstand im Hochvakuum getrocknet. Es folgte die Abspaltung des tert-Butylestergruppe durch 2-stündiges Rühren bei 50°C in Trifluorethanol mit 6 Equiv. Zinkchlorid. Nach Zugabe von 6 Equiv. EDTA erfolte die Reinigung durch präparative HPLC. Im letzten Schritt wurde das erhaltene Intermediat in DMF aufgenommen und mit 15 Equivalenten 1-Hydroxypyrrolidin-2,5-dion versetzt und durch 60 minütiges Rühren in Gegenwart von 5 Equiv. HATU und 5 Equiv. N,N-Diisopropyl ethylamin in die Titelverbindung überführt. Diese wurde durch präparative HPLC aufgereinigt.

LC-MS (Methode 1): $R_t$ = 0.87 min; MS (ESIpos): m/z = 1071 $(M+H)^+$.

**Intermediat R12**

N-[(Benzyloxy)carbonyl]-L-alanyl-D-alanyl-N-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-alpha-asparagin

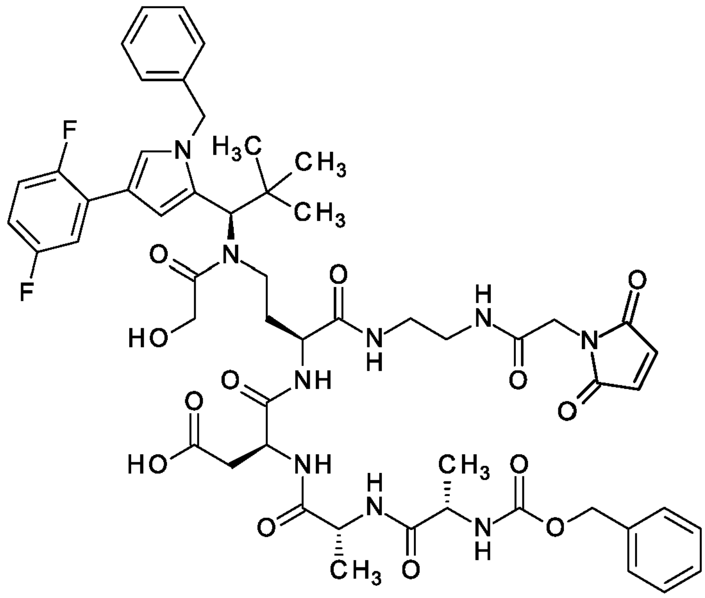

Die Titelverbindung wurde ausgehend von Verbindung F104 zunächst durch Kupplung mit Intermediat L113 in DMF in Gegenwart von HATU und N,N-Diisopropylethylamin und anschließende Entschützung mittels Zinkchlorid hergestellt.
LC-MS (Methode 1): $R_t$ = 1.1 min; MS (ESIpos): m/z = 1084 $[M+H]^+$.

**Intermediat R13**

N-Acetyl-L-alanyl-D-alanyl-$N^1$-[(2S)-4-[{(1R)-1-[1-benzyl-4-(2,5-difluorphenyl)-1H-pyrrol-2-yl]-2,2-dimethylpropyl}(glycoloyl)amino]-1-({2-[(N-{5-[(2,5-dioxopyrrolidin-1-yl)oxy]-5-oxopentanoyl}-L-gamma-glutamyl)amino]ethyl}amino)-1-oxobutan-2-yl]-L-aspartamid

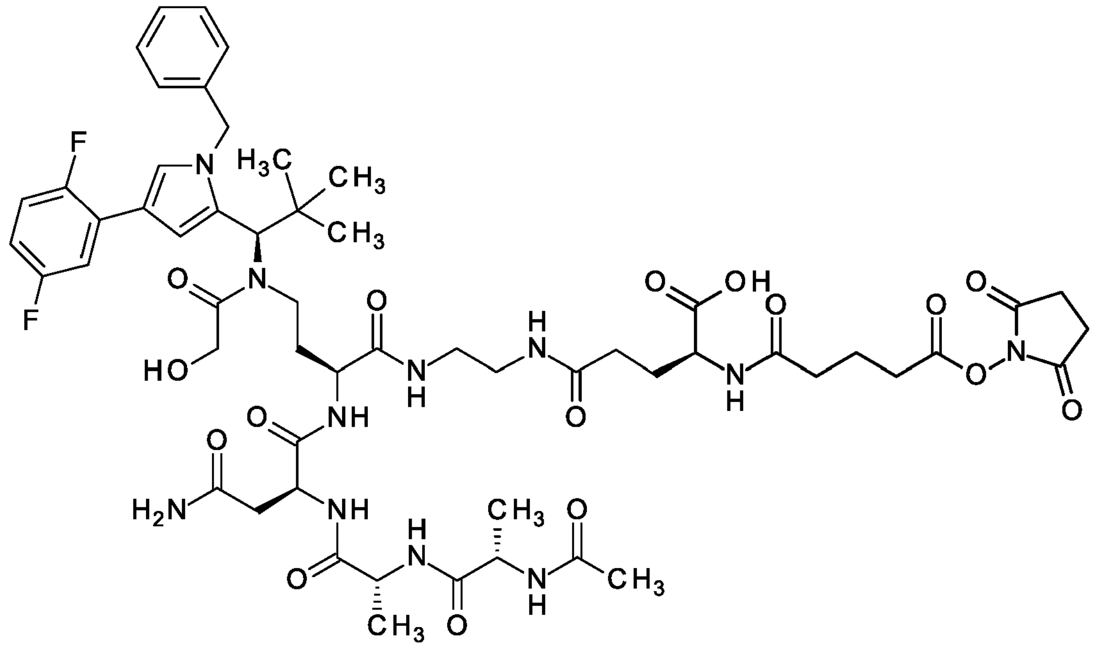

Zunächst wurde Intermediat C112 in Gegenwart von HATU und N,N-Diisopropylethylamin mit (4S)-5-(Benzyloxy)-4-{[(benzyloxy)carbonyl]amino}-5-oxopentansäure gekuppelt. Anschließend erfolgte die vollständige Entschützung durch 1-stündige Hydrierung über 10%-igem Palladium auf Aktivkohle in DCM-Methanol 1:1 bei RT unter Wasserstoff-Normaldruck. Schließlich wurde durch Umsetzung mit 5 Equivalenten 1,1'-[(1,5-Dioxopentan-1,5-diyl)bis(oxy)]dipyrrolidin-2,5-dion in DMF in Gegenwart von 3 Equivalenten N,N-Diisopropylethylamin die Titelverbindung erhalten. LC-MS (Methode 1): $R_t$ = 0.91 min; MS (ESIpos): m/z = 1194 $[M+H]^+$.

## Intermediat R14

N-Acetyl-L-alanyl-D-alanyl-$N^1$-[(2S)-4-[{(1R)-1-[1-benzyl-4-(2,5-difluorphenyl)-1H-pyrrol-2-yl]-2,2-dimethylpropyl}(glycoloyl)amino]-1-{[(1R)-1-carboxy-2-({5-[(2,5-dioxopyrrolidin-1-yl)oxy]-5-oxopentanoyl}amino)ethyl]amino}-1-oxobutan-2-yl]-L-aspartamid

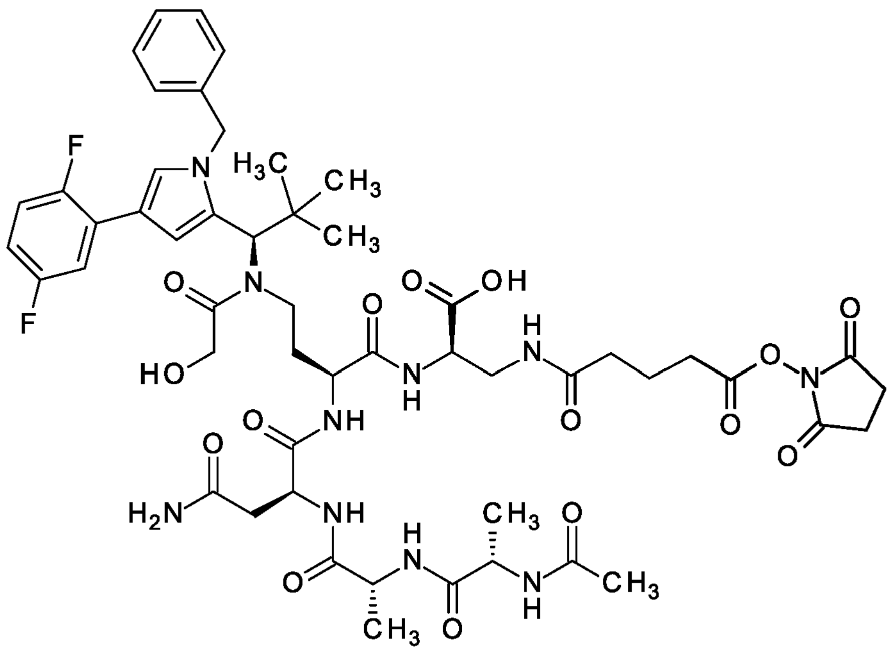

Zunächst wurde Intermediat C113 in Gegenwart von HATU und N,N-Diisopropylethylamin mit Intermediat L111 gekuppelt. Anschließend erfolgte die vollständige Entschützung durch 1-stündige Hydrierung über 10%-igem Palladium auf Aktivkohle in DCM-Methanol 1:1 bei RT unter Wasserstoff-Normaldruck. Schließlich wurde durch Umsetzung mit 2.5 Equivalenten 1,1'-[(1,5-Dioxopentan-1,5-diyl)bis(oxy)]dipyrrolidin-2,5-dion in DMF in Gegenwart von 3.5 Equivalenten N,N-Diisopropylethylamin die Titelverbindung erhalten.

LC-MS (Methode 1): $R_t$ = 0.92 min; MS (ESIpos): m/z = 1109 [M+H]+.

## Intermediat R15

N-Acetyl-L-alanyl-D-alanyl-N[1]-[(2S)-4-[{(1R)-1-[1-benzyl-4-(2,5-difluorphenyl)-1H-pyrrol-2-yl]-2,2-dimethylpropyl}(glycoloyl)amino]-1-({2-[(N-{5-[(2,5-dioxopyrrolidin-1-yl)oxy]-5-oxo pentanoyl}-D-alpha-glutamyl)amino]ethyl}amino)-1-oxobutan-2-yl]-L-aspartamid

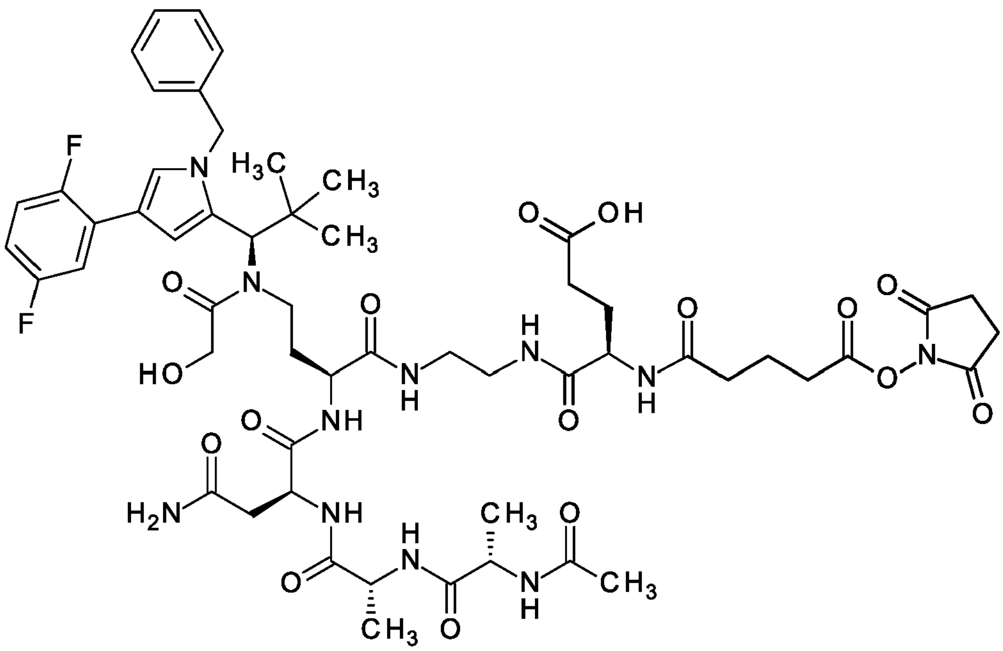

Zunächst wurde Intermediat C112 in DMF in Gegenwart von HATU und N,N-Diisopropylethyl-amin mit kommerziell erhältlicher (2R)-5-(Benzyloxy)-2-{[(benzyloxy) carbonyl]amino}-5-oxopentansäure gekuppelt. Anschließend erfolgte die vollständige Entschützung durch 1-stündige Hydrierung über 10%-igem Palladium auf Aktivkohle in Methanol bei RT unter Wasserstoff-Normaldruck. Schließlich wurde durch Umsetzung mit 2.5 Equivalenten 1,1'-[(1,5-Dioxopentan-1,5-diyl)bis(oxy)]dipyrrolidin-2,5-dion in DMF in Gegenwart von 3 Equivalenten N,N-Diiso-propylethylamin die Titelverbindung erhalten.

LC-MS (Methode 1): $R_t$ = 0.92 min; MS (ESIpos): m/z = 1194 $[M+H]^+$.

## Intermediat R16

N-(38-Oxo-2,5,8,11,14,17,20,23,26,29,32,35-dodecaoxaoctatriacontan-38-yl)-L-alanyl-D-alanyl-N1-{(2S)-4-[{(1R)-1-[1-benzyl-4-(2,5-difluorphenyl)-1H-pyrrol-2-yl]-2,2-dimethylpropyl}-(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

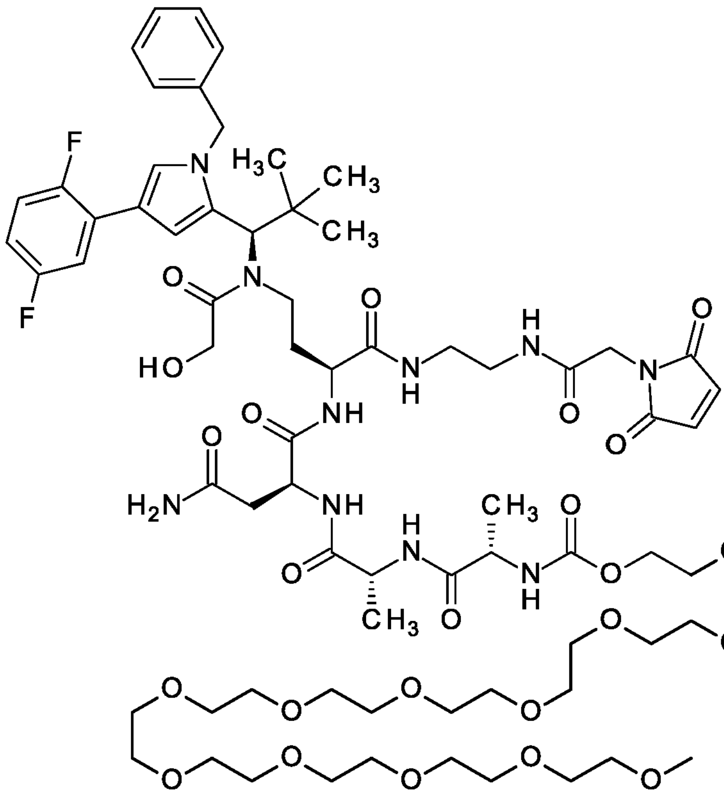

20 mg (0.025 mmol) von Intermediat F104 und 23 mg Intermediat L116 (0,027 mmol) wurden in 0,2 ml DMF gelöst und nach Zugabe von 11.3 mg (0.029 mmol) HATU, 13 µL N,N-Diisopropylethylamin (0.074 mmol) 45 min bei RT gerührt. Es wurde Wasser (1 mL) und ACN (1 mL) addiert. Die Reaktionslösung wurde mittels präparativer HPLC gereinigt (Eluent: ACN/Wasser + 0.1% TFA, Gradient = 3:7 → 7:3). Es wurden 19 mg (50% d. Th.) der Titelverbindung erhalten.

LC-MS (Methode 4): $R_t$ = 1.19 min; MS (ESIpos): m/z = 1519,8055 $(M+H)^+$.

## Intermediat R17

N-(38-Oxo-2,5,8,11,14,17,20,23,26,29,32,35-dodecaoxaoctatriacontan-38-yl)-L-alanyl-D-alanyl-N1-[(20R)-25-{(1R)-1-[1-benzyl-4-(2,5-difluorphenyl)-1H-pyrrol-2-yl]-2,2-dimethylpropyl}-20-carboxy-1-(2,5-dioxo-2,5-dihydro-1H-pyrrol-1-yl)-2,18,24-trioxo-6,9,12,15-tetraoxa-22-thia-3,19,25-triazaoctacosan-28-yl]-L-aspartamid

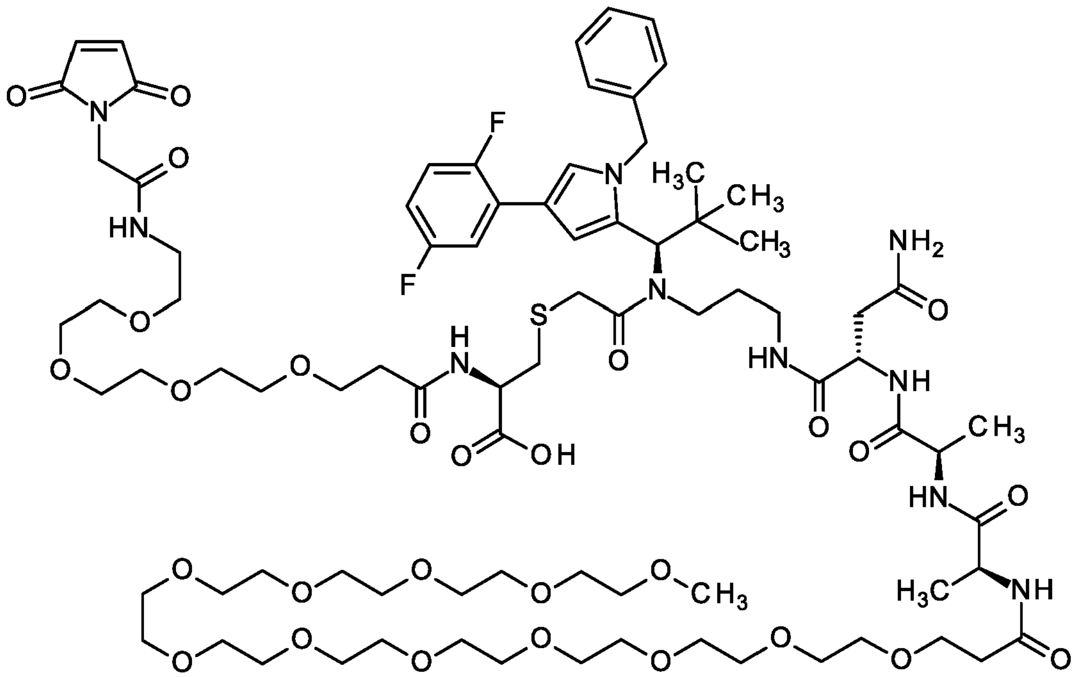

Eine Lösung von 14,4 mg von Intermediat L118 (0.020 mmol) in 0,25 ml DMF wurde mit 9 µl 4-ethylmorpholin (0,08 mmoL) und 5,78 mg HATU (0,015 mmol) versetzt und nach Zugabe von 18 mg Internediat F307 (0.015 mmol), und die Mischung wurde 30 Minuten bei RT gerührt. Es wurde Wasser (1,5 mL) und ACN (1,5 mL) addiert. Die Reaktionslösung wurde mittels präparativer HPLC gereinigt (Eluent: ACN/Wasser + 0.1 % TFA, Gradient = 35% → 65%). Es wurden 10 mg (36% d. Th.) der Titelverbindung erhalten.

LC-MS (Methode 14): $R_t$ = 5.44 min; MS (ESIpos): m/z = 892,4320 $(M+2H)^{2+}$.

## Intermediat R18

N-(Pyridin-4-ylacetyl)-L-alanyl-D-prolyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1 H-pyrrol-1-yl)acetyl]amino}ethyl)amino]-1-oxobutan-2-yl}-L-aspartamid

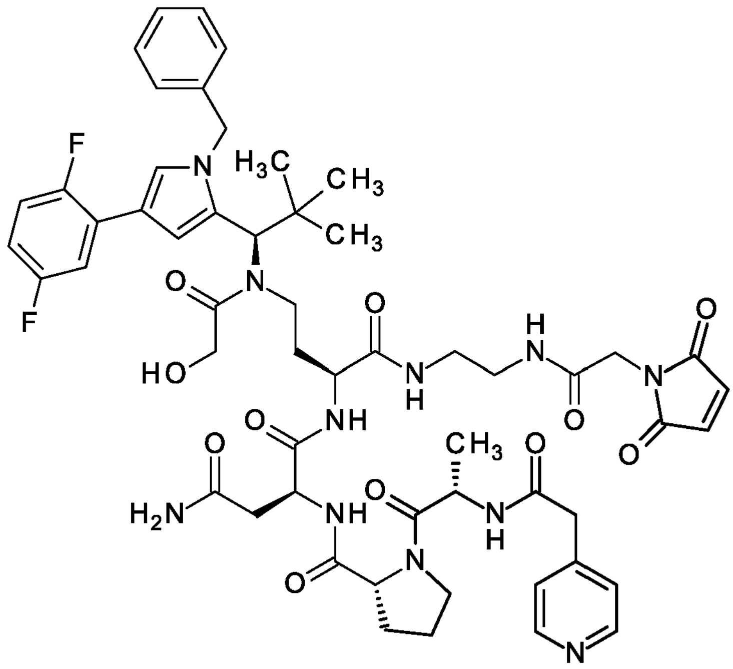

15 mg (0.019 mmol) von Intermediat F104 wurden in Analogie zu Intermediat R1 mit 12 mg (0.022 mmol) Intermediat L119 gekuppelt. Nach Reinigung mittels präparativer HPLC wurden 4.7 mg (23% d.Th.) der Titelverbindung erhalten.

LC-MS (Methode 1): $R_t$ = 0.85 min; MS (ESIpos): m/z = 1094 $(M+H)^+$.

## Intermediat R19

N-(Pyridin-4-ylacetyl)-D-alanyl-D-alanyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

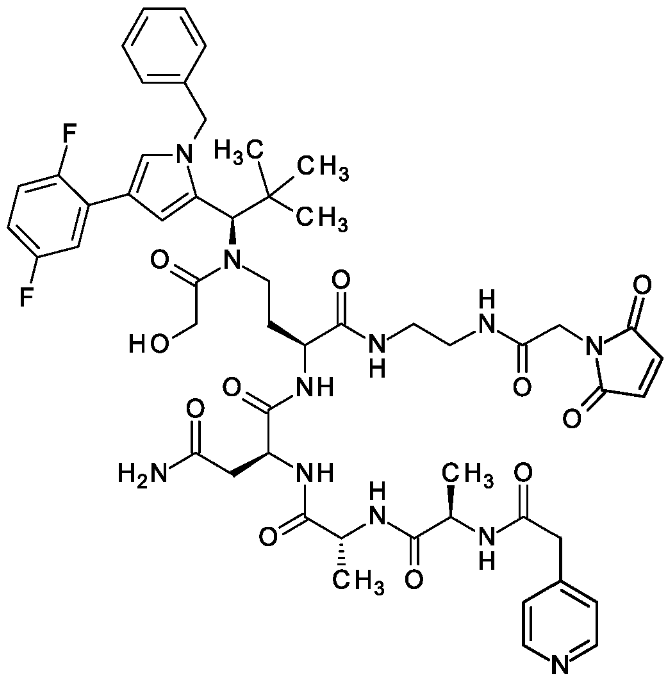

15 mg (0.019 mmol) von Intermediat F104 wurden in Analogie zu Intermediat R1 mit 10.4 mg (0.02 mmol) Intermediat L120 gekuppelt. Nach Reinigung mittels präparativer HPLC wurden 5.7 mg (29% d.Th.) der Titelverbindung erhalten.

LC-MS (Methode 1): $R_t$ = 0.83 min; MS (ESIpos): m/z = 1068 $(M+H)^+$.

## Intermediat R20

N-{5-[(2,5-Dioxopyrrolidin-1-yl)oxy]-5-oxopentanoyl}-L-alanyl-D-alanyl-$N^1$-[(16S)-4-{(1R)-1-[1-benzyl-4-(2,5-difluorphenyl)-1H-pyrrol-2-yl]-2,2-dimethylpropyl}-16,18-dicarboxy-5,10,14-trioxo-7-thia-4,11,15-triazaoctadec-1-yl]-L-asparta-mid - trifluoressigsäure Salz

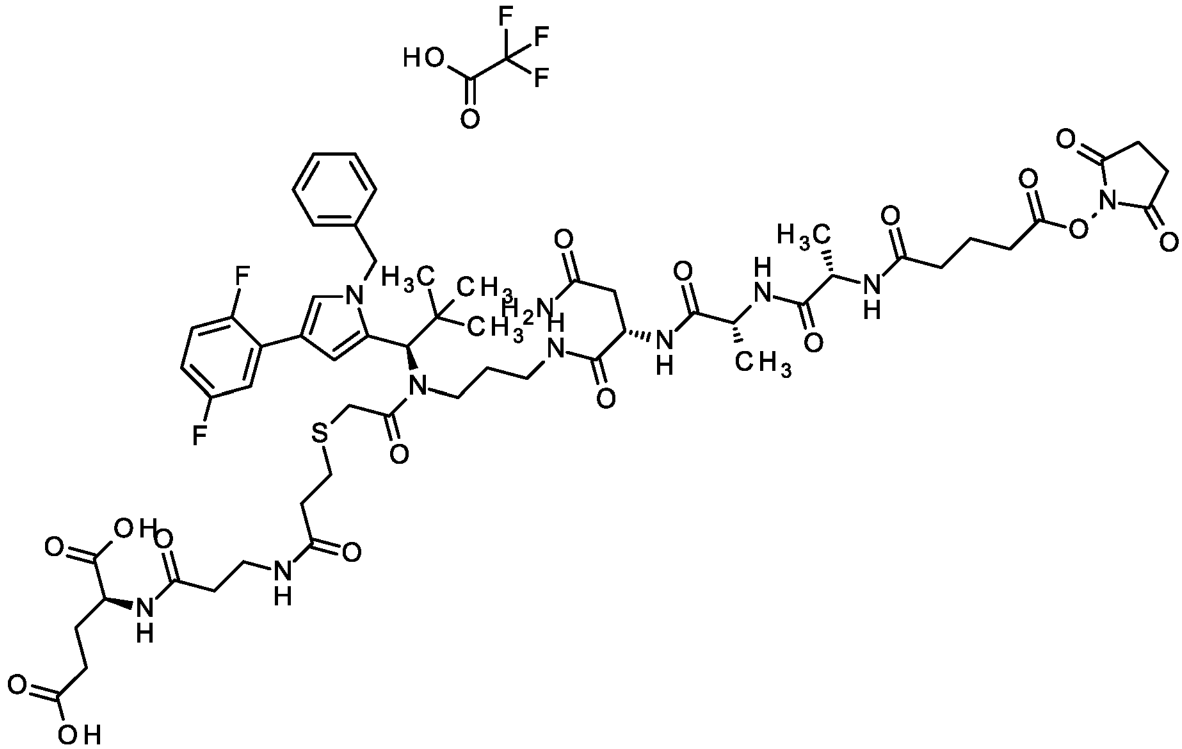

Die Titelverbindung wurde ausgehend von Verbindung C117 zunächst durch Kupplung mit Intermediat L108 in Gegenwart von HATU und N,N-Diisopropylethylamin hergestellt. Im nächsten Schritt wurden alle Schutzgruppen durch übernacht Hydrierung über 10%-igem Palladium auf Aktivkohle in Ethanol:Ethyl Acetat unter Wasserstoff-Normaldruck bei RT entfernt und das entschützte Intermediat anschließend durch Umsetzung mit 1,1'-[(1,5-Dioxopentan-1,5-diyl)bis(oxy)]dipyrrolidin-2,5-dion in Gegenwart von N,N-Diisopropylethylamin in DMF in die Titelverbindung überführt.

LC-MS (Methode 1): $R_t$ = 0.94 min; MS (ESIneg): m/z = 1223 $[M-H]^-$.

## Intermediat R21

N-[6-(2,5-Dioxo-2,5-dihydro-1H-pyrrol-1-yl)hexanoyl]-L-alanyl-D-alanyl-$N^1$-[(16S)-4-{(1R)-1-[1-benzyl-4-(2,5-difluorphenyl)-1H-pyrrol-2-yl]-2,2-dimethylpropyl}-16,18-dicarboxy-5,10,14-trioxo-7-thia-4,11,15-triazaoctadec-1-yl]-L-aspartamid -trifluoressigsäure (1:1)

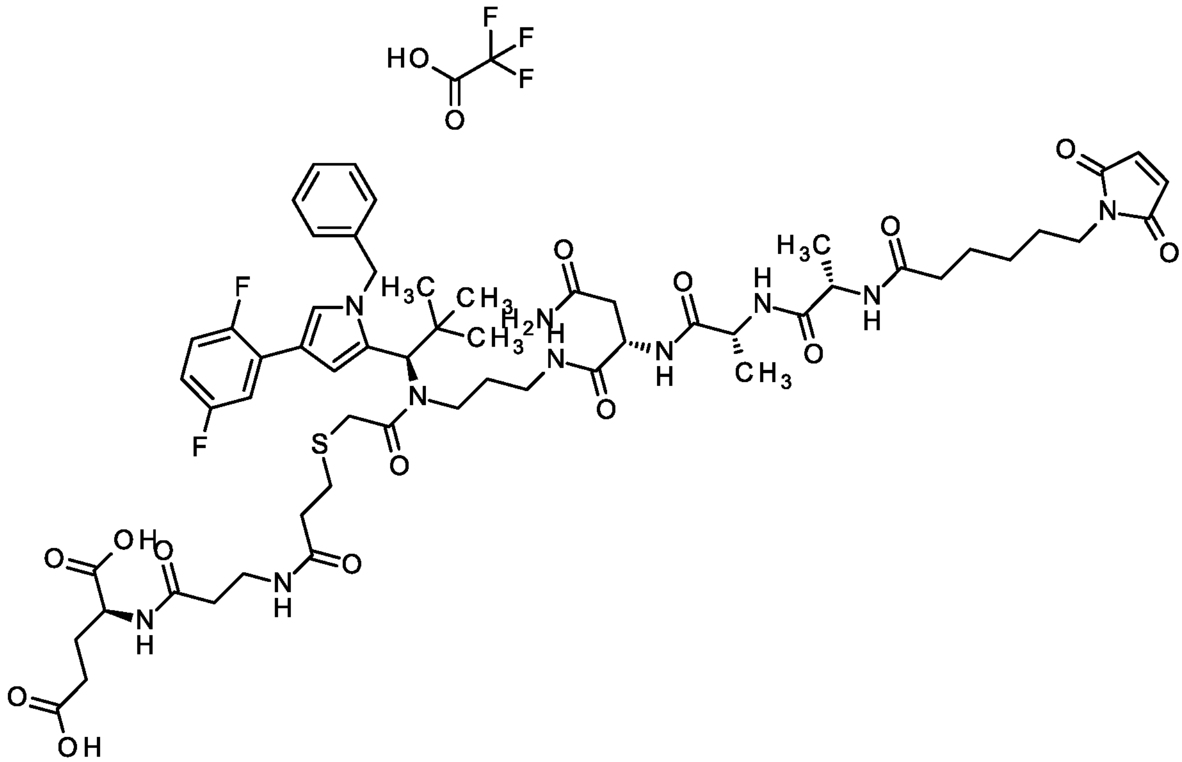

Die Titelverbindung wurde ausgehend von Verbindung C117 zunächst durch Kupplung mit Intermediat L108 in Gegenwart von HATU und N,N-Diisopropylethylamin hergestellt. Im nächsten Schritt wurden alle Schutzgruppen durch übernacht Hydrierung über 10%-igem Palladium auf Aktivkohle in Ethanol:Ethyl Acetat unter Wasserstoff-Normaldruck bei RT entfernt und das entschützte Intermediat anschließend durch Umsetzung mit 1-{6-[(2,5-Dioxopyrrolidin-1-yl)oxy]-6-oxohexyl}-1H-pyrrol-2,5-dion in Gegenwart von N, N-Diisopropylethylamin in DMF in die Titelverbindung überführt.

LC-MS (Methode 1): $R_t$ = $R_t$ = 0.97 min; MS (ESIneg): m/z = 1205 $[M-H]^-$.

## Intermediat R22

N-{[2-(2-Methoxyethoxy)ethoxy]acetyl}-L-alanyl-D-alanyl-N[1]-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

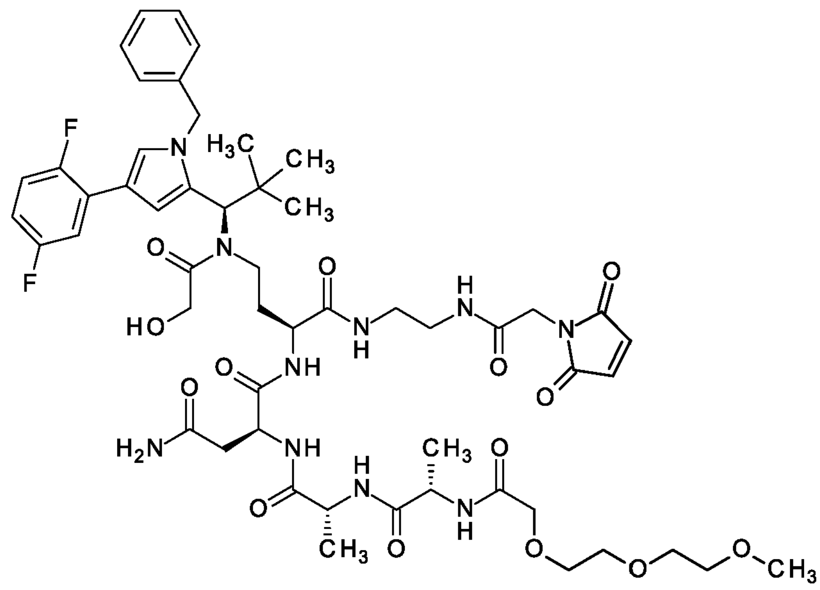

20 mg (0.025 mmol) von Intermediat F104 wurden in Analogie zu Intermediat R1 mit 11.9 mg (0.027 mmol) Intermediat L129 gekuppelt. Nach Reinigung mittels präparativer HPLC wurden 13.4 mg (49% d.Th.) der Titelverbindung erhalten.

LC-MS (Methode 1): $R_t$ = 0.98 min; MS (ESIpos): m/z = 1109 $(M+H)^+$.

## Intermediat R23

N-{[2-(2-Methoxyethoxy)ethoxy]acetyl}-L-alanyl-D-alanyl-N1-[(2S)-4-[{(1R)-1-[1-benzyl-4-(2,5-difluorphenyl)-1H-pyrrol-2-yl]-2,2-dimethylpropyl}(glycoloyl)amino]-1-({2-[(N-{5-[(2,5-dioxo pyrrolidin-1-yl)oxy]-5-oxopentanoyl}-D-alpha-glutamyl)amino]ethyl}amino)-1-oxobutan-2-yl]-L-aspartamid

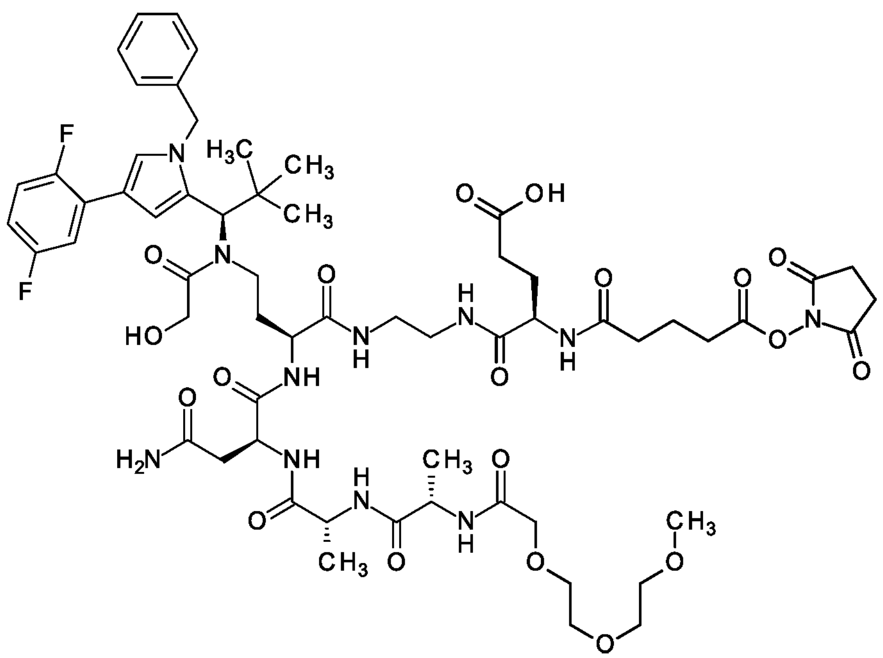

Zunächst wurde Intermediat C120 mit (2R)-5-(Benzyloxy)-2-{[(benzyloxy)carbonyl]amino}-5-oxopentansäure in Gegenwart von HATU gekuppelt. Dann wurden hydrogenolytisch die Benzyloxycarbonyl-Schutzgruppe sowie der Benzylester über 10% Palladium/Aktivkohle entfernt. Das erhaltene Intermediat wurde mit 1,1'-[(1,5-Dioxopentan-1,5-diyl)bis(oxy)] dipyrrolidin-2,5-dion in Gegenwart von N,N-Diisopropylethylamin in DMF in die Titelverbindung überführt.
LC-MS (Methode 1): $R_t = R_t = 0.94$ min; MS (ESIpos): m/z = 1312 $[M+H]^+$.

## Intermediat R24

N-{5-[(2,5-Dioxopyrrolidin-1-yl)oxy]-5-oxopentanoyl}-L-alanyl-D-alanyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1R)-1,3-dicarboxypropyl]amino}-3-oxopropyl)amino]-1-oxobutan-2-yl}-L-aspartamid

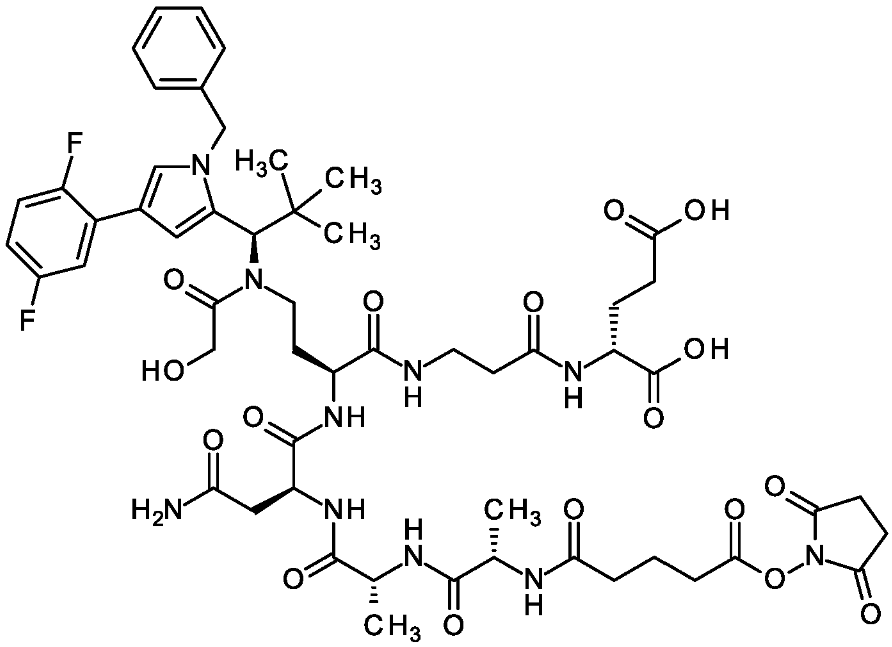

Die Titelverbindung wurde ausgehend von Verbindung C121 zunächst durch Kupplung mit Intermediat L108 in Gegenwart von HATU und N,N-Diisopropylethylamin hergestellt. Im nächsten Schritt wurden alle Schutzgruppen durch Hydrierung über 10%-igem Palladium auf Aktivkohle in Ethanol unter Wasserstoff-Normaldruck bei RT entfernt und das entschützte Intermediat anschließend durch Umsetzung mit 1,1'-[(1,5-Dioxopentan-1,5-diyl)bis(oxy)]dipyrrolidin-2,5-dion in Gegenwart von N,N-Diisopropylethylamin in DMF in die Titelverbindung überführt.
LC-MS (Methode 1): $R_t = R_t = 0.92$ min; MS (ESIpos): m/z = 1181 $[M+H]^+$.

## Intermediar R25

N-{6-[(2,5-Dioxopyrrolidin-1-yl)oxy]-6-oxohexyl}-N-methyl-L-alanyl-L-alanyl-D-alanyl-N1-{(2S)-4-[{(1R)-1-[1-benzyl-4-(2,5-difluorphenyl)-1H-pyrrol-2-yl]-2,2-dimethylpropyl} (glycoloyl)amino]-1-[(3-{[(1R)-1,3-dicarboxypropyl]amino}-3-oxopropyl)amino]-1-oxobutan-2-yl}-L-aspartamid

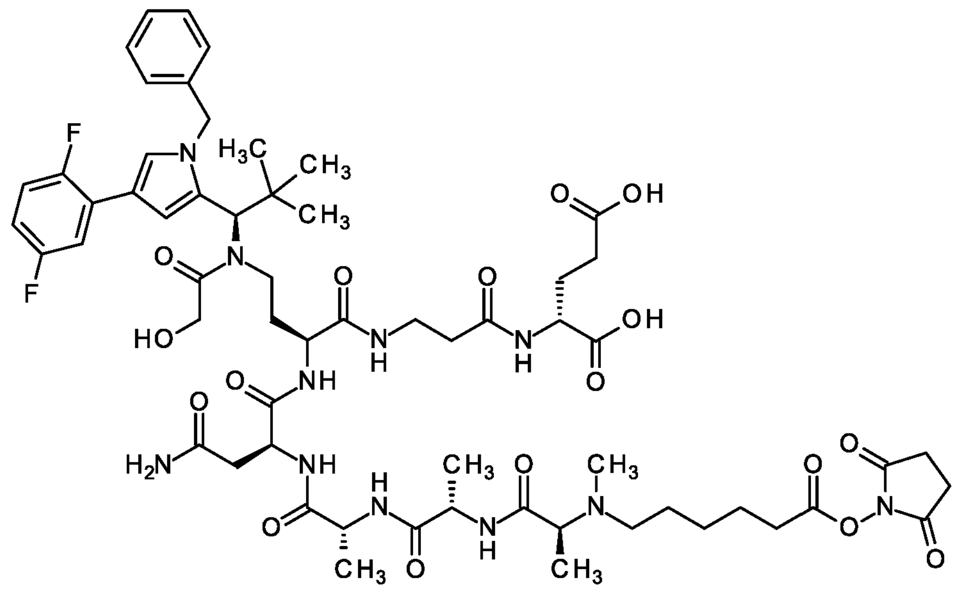

Zunächst wurde Trifluoressigsäure --4-nitrobenzyl-L-alanyl-D-alanyl-L-asparaginat (1:1) durch Kupplung von N-(tert-Butoxycarbonyl)-L-alanyl-D-alanin mit 4-Nitrobenzyl-L-asparaginat hydrobromid (1:1) in DMF in Gegenwart von HATU und N,N-Diisopropylethylamin und anschließender Entschützung der Aminogruppe mit Trifluoressigsäure in DCM hergestellt.

Dieses Intermediat wurde in DMF in Gegenwart von HATU und N,N-Diisopropylethylamin mit N-(tert-butoxycarbonyl)-N-methyl-L-alanin gekuppelt. Anschließend wurde der p-Nitrobenzylester durch Hydrierung in DCM-Methanol 1:1 über 10% Palladium auf Aktivkohle abgespalten.

Das so erhaltene Intermediat wurde in DMF in Gegenwart von HATU und N,N-Diisopropylethylamin mit Intermediat C121 gekuppelt. Anschließend wurde die Boc-Schutzgruppe durch 1h Rühren bei 50°C mit 4 Equivalenten Zinkchlorid in Trifluorethanol abgespalten.

LC-MS (Methode 1): $R_t$ = 0.99 min; MS (ESIpos): m/z = 1235 $(M+H)^+$.

45 mg (33 μmol) von diesem Intermediat wurden mit 26 mg (200 μmol) 6-Oxohexansäure, die zuvor nach Litereaturvorschrift hergestellt wurde (J.Org.Chem. 1993, 58, 2196), in 20.5 mL Methanol zusammengegeben und mit 7.6 μL Essigsäure sowie 30 mg (320 μmol) Boran-Pyridin-Komplex versetzt. Der Ansatz wurde 4.5 h bei RT gerührt und anschließend im Vakuum eingeengt und durch präparative HPLC gereinigt. Es wurden 38 mg (84% d. Th.) des Intermediats erhalten.

38 mg (0.028 mmol) von diesem Intermediat wurden in 10 ml DMF aufgenommen und mit 53 mg (0.42 mmol) 1-Hydroxypyrrolidin-2,5-dion und 24.5 μL N,N-Diisopropylethylamin sowie portionsweise mit insgesamt 77 mg (0.2 mmol) HATU versetzt. Nach 2 h Rühren bei RT wurde die Reaktionslösung mit TFA auf einen pH-Wert von 3-4 eingestellt und anschließend eingeengt und durch präparative HPLC gereinigt. Es wurden 39 mg (96%) des geschützten Intermediats erhalten, welches anschließend in 15 ml Ethanol aufgenommen wurde. Nach Zugabe von 10% Palladium auf Aktivkohle wurden die Benzylestergruppen hydrogenolytisch unter Wasserstoff-Normaldruck entfernt und nach Abfiltrieren des Katalysators, Einengen der verbliebenen Lösung, und anschließender Lyophilisation des Rückstandes aus Acetonitril/Wasser 9:1 wurden 34 mg (94% d. Th.) der Titelverbindung erhalten.

LC-MS (Methode 1): $R_t$ = 0.8 min; MS (ESIpos): m/z = 1266 $(M+H)^+$.

**Intermediat R26**

N-(Pyridin-4-ylacetyl)-L-alanyl-D-asparaginyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

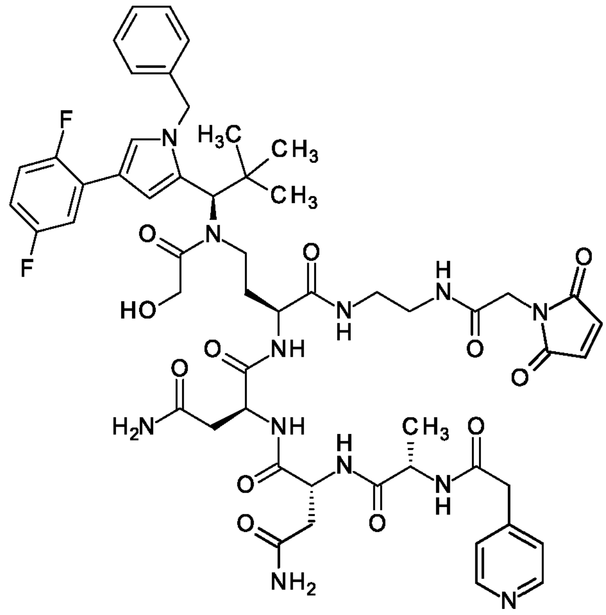

15 mg (0.019 mmol) von Intermediat F104 wurden in Analogie zu Intermediat R1 mit 10.3 mg (0.022 mmol) Intermediat L130 gekuppelt. Anschließend wurde die Boc-Schutzgruppe durch 2h Rühren bei 50°C mit 6 Equivalenten Zinkchlorid in Trifluorethanol abgespalten. Im letzten Schritt wurde das Intermediat mit 4-Pyridinessigsäure mittels HATU gekuppelt. LC-MS (Methode 1): $R_t$ = 0.84 min; MS (ESIpos): m/z = 1111 $(M+H)^+$.

<u>**Intermediat R27**</u>

N-(Pyridin-4-ylacetyl)-L-alanyl-D-seryl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

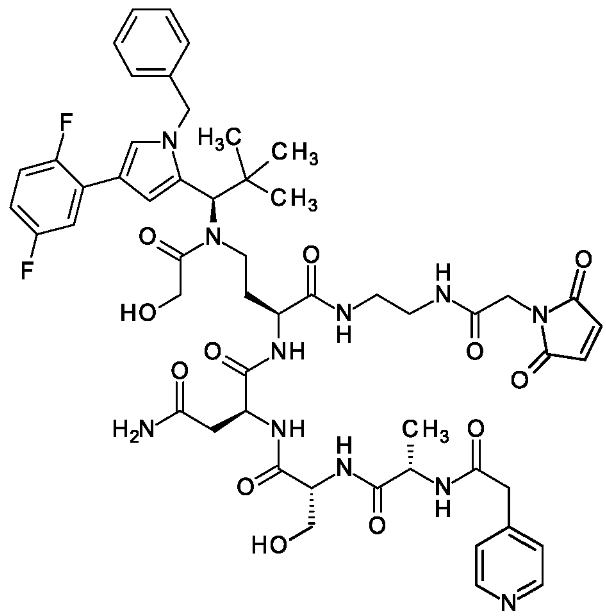

Zunächst wurde N-(Pyridin-4-ylacetyl)-L-alanyl-D-serin durch Kupplung der Bausteine Pyridin-4-ylessigsäurehydrochlorid (1:1) und tert-Butyl-L-alaninathydrochlorid (1:1) mittels HATU, anschließender tert.Butylester Spaltung mit TFA in DCM, Kupplung mit Benzyl-D-serinathydrochlorid (1:1) und schließlich durch hydrogenolytische Spaltung des

Benzylesters über 10% Palladium/Aktivkohle hergestellt.

8.4 mg (25 μmol) von diesem Intermediat wurden dann durch Kupplung mit 20 mg (21.2 μmol) von Intermediat C116 in DMF in Gegenwart von 9.7 mg (25.5 μmol) HATU und 18.5 μL N,N-Diisopropylethylamin in die Titelverbindung überführt.

LC-MS (Methode 5): $R_t$ = 2.71 min; MS (ESIpos): m/z = 1084 $(M+H)^+$.

## Intermediat R28

N-Acetyl-L-alanyl-D-alanyl-N1-[(2S)-4-[{(1R)-1-[1-benzyl-4-(2,5-difluorphenyl)-1H-pyrrol-2-yl]-2,2-dimethylpropyl}(glycoloyl)amino]-1-{[2-({N-[(2,5-dioxo-2,5-dihydro-1H-pyrrol-1-yl)acetyl]-D-alpha-glutamyl}amino)ethyl]amino}-1-oxobutan-2-yl]-L-aspartamid

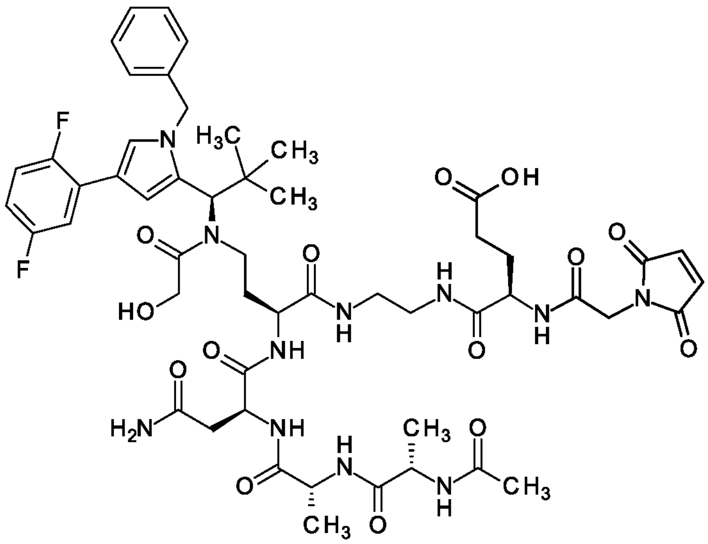

Die Titelverbindung wurde in Analogie zu Intermediat R15 hergestellt. Im letzten Schritt wurde an Stelle von 1,1'-[(1,5-Dioxopentan-1,5-diyl)bis(oxy)]dipyrrolidin-2,5-dion das Maleinimid-Derivat 1-{2-[(2,5-Dioxopyrrolidin-1-yl)oxy]-2-oxoethyl}-1H-pyrrol-2,5-dion in die Kupplung eingesetzt.

LC-MS (Methode 1): $R_t$ = 0.91 min; MS (ESIpos): m/z = 1121 $(M+H)^+$.

## Intermediat R29

N-(Pyridin-4-ylacetyl)-L-alanyl-D-norvalyl-N1-[(20R)-25-{(1R)-1-[1-benzyl-4-(2,5-difluorphenyl)-1H-pyrrol-2-yl]-2,2-dimethylpropyl}-20-carboxy-1-(2,5-dioxo-2,5-dihydro-1H-pyrrol-1-yl)-2,18,24-trioxo-6,9,12,15-tetraoxa-22-thia-3,19,25-triazaoctacosan-28-yl]-L-aspartamid -trifluoressigsäure (1:1)

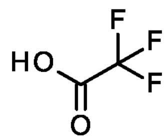

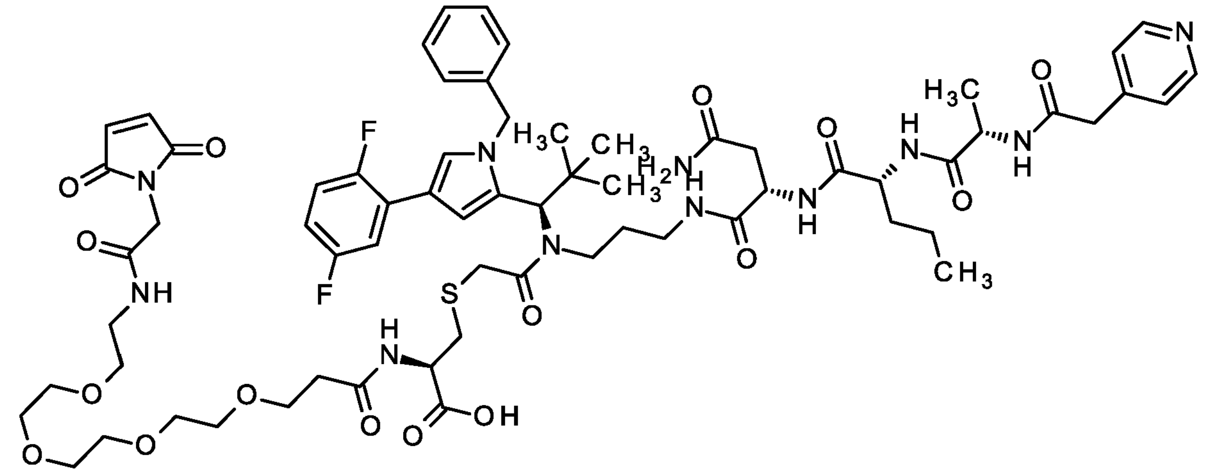

Die Titelverbindung wurde ausgehend von Verbindung C119 zunächst durch Kupplung mit Intermediat L126 in Gegenwart von HATU und N,N-Diisopropylethylamin hergestellt. Im nächsten Schritt wurde der Teoc-Schutzgruppe mittels Zinkchlorid in Trifluorethanol entfernt und das entschützte Intermediat anschließend durch Umsetzung mit 1-(2,5-Dioxo-2,5-dihydro-1H-pyrrol-1-yl)-2-oxo-6,9,12,15-tetraoxa-3-azaoctadecan-18-säure in Gegenwart von HATU und N,N-Diisopropylethylamin in die Titelverbindung überführt. LC-MS (Methode 14): $R_t$ = 5.28 min; MS (ESIpos): m/z = 1360 $[M+H]^+$.

## Intermediat R30

N-[(Benzyloxy)carbonyl]-D-alpha-asparagyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

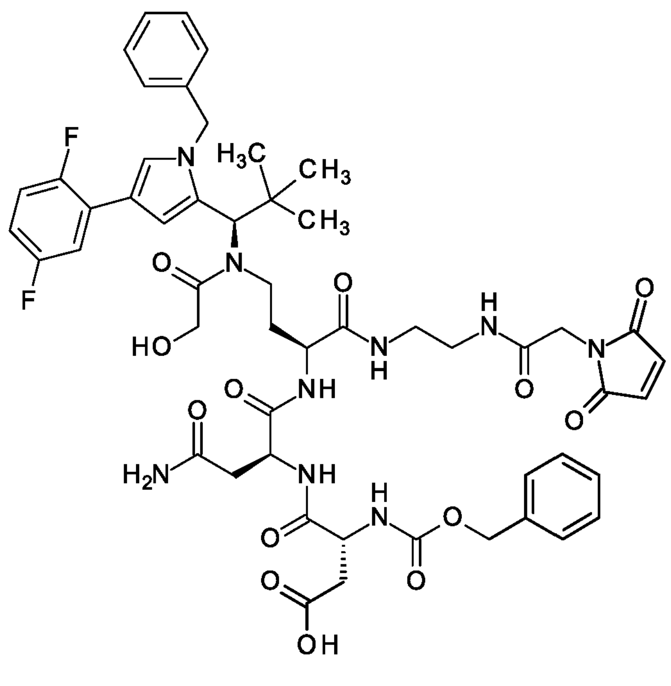

Die Titelverbindung wurde ausgehend von Verbindung C116 zunächst durch Kupplung mit (2R)-2-{[(Benzyloxy)carbonyl]amino}-4-tert-butoxy-4-oxobutansäure in Gegenwart von HATU und N,N-Diisopropylethylamin hergestellt. Anschließend wurde der tert.-Butylester durch 2h Rühren bei 50°C mit 6 Equivalenten Zinkchlorid in Trifluorethanol abgespalten und nach Reinigung mittels präparativer HPLC die Titelverbindung erhalten.
LC-MS (Methode 1): $R_t$ = $R_t$ = 1.06 min; MS (ESIpos): m/z = 1056 $[M+H]^+$.

## Intermediat R31

N-(Pyridin-4-ylacetyl)-L-alanyl-D-alanyl-N1-[(2S)-4-[{(1R)-1-[1-benzyl-4-(2,5-difluorphenyl)-1H-pyrrol-2-yl]-2,2-dimethylpropyl}(glycoloyl)amino]-1-{[2-({N-[(2,5-dioxo-2,5-dihydro-1H-pyrrol-1-yl)acetyl]-D-alpha-glutamyl}amino)ethyl]amino}-1-oxobutan-2-yl]-L-aspartamid

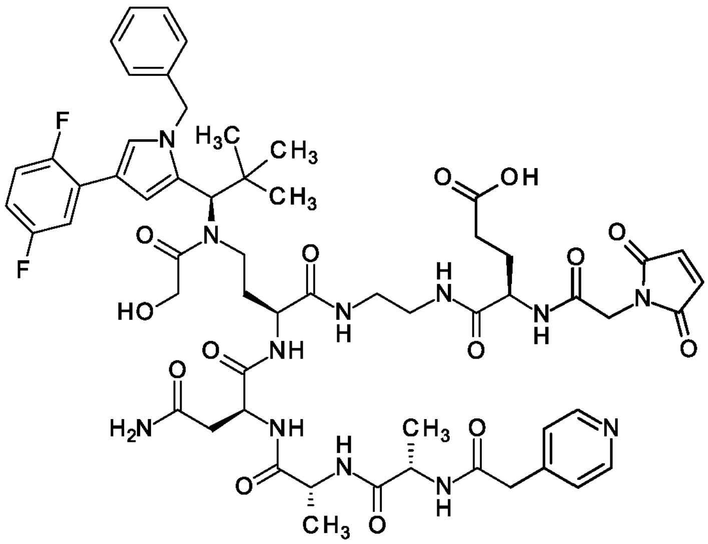

Die Synthese der Titelverbindung begann mit der Kupplung von Intermediat C102 mit Intermediat L131 in Gegenwart von HATU und N,N-Diisopropylethylamin. Anschließend wurde hydrogenolytisch mit Wasserstoff unter Normaldruck über 10% Palladium/ Aktivkohle in Ethanol die Z-Gruppe abgespalten. Anschließend erfolgte die Kupplung mit Intermediat L110 in DMF in Gegenwart von HATU und N,N-Diisopropylethylamin. Dann wurden die Boc-Schutzgruppe sowie der tert.-Butylester durch 6h Rühren bei 50°C mit 6 Equivalenten Zinkchlorid in Trifluorethanol abgespalten. Im letzten Schritt wurde das erhaltene Intermediat mit 1-{2-[(2,5-Dioxopyrrolidin-1-yl)oxy]-2-oxoethyl}-1H-pyrrol-2,5-dion in DMF in Gegenwart von N,N-Diisopropylethylamin gekuppelt und nach Reinigung mittels präparativer HPLC die Titelverbindung erhalten.
LC-MS (Methode 12): $R_t$ = $R_t$ = 1.49 min; MS (ESIpos): m/z = 1197 $[M+H]^+$.

## Intermediat R32

N-[(Benzyloxy)carbonyl]-L-alanyl-D-alpha-asparagyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid - trifluoressig säure (1:1)

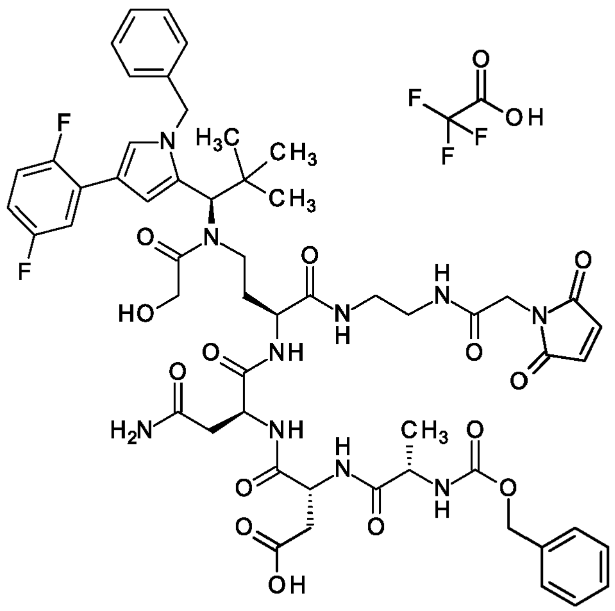

Die Synthese der Titelverbindung begann mit der HATU-Kupplung in Gegenwart von N,N-Diisopropylethylamin von 2,5-Dioxopyrrolidin-1-yl-N-[(benzyloxy)carbonyl]-L-alaninat mit (2R)-2-Amino-4-tert-butoxy-4-oxobutansäure. Das erhaltene Intermediat wurde dann mit Intermediat C116 ebenfalls mittels HATU-Kupplung in Gegenwart von N,N-Diisopropylethylamin umgesetzt. Im letzten Schritt erfolgte die Abspaltung des tert.-Butylesters durch 2h Rühren bei 50°C mit 6 Equivalenten Zinkchlorid in Trifluorethanol.

LC-MS (Methode 1): $R_t$ = 1.05 min; MS (ESIpos): m/z = 1127 $(M+H)^+$.

## Intermediat R33

N-Acetyl-L-alanyl-D-alpha-asparagyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid -trifluoressigsäure (1:1)

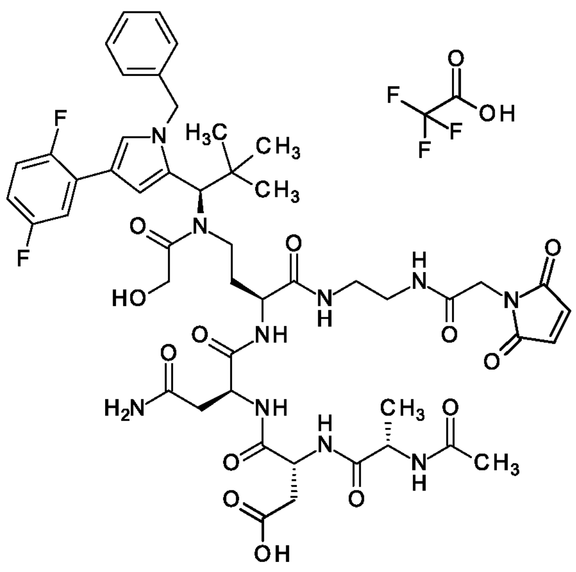

Die Synthese der Titelverbindung begann mit der HATU-Kupplung in Gegenwart von N,N-Diisopropylethylamin von 2,5-Dioxopyrrolidin-1-yl-N-[(benzyloxy)carbonyl]-L-alaninat mit (2R)-2-Amino-4-tert-butoxy-4-oxobutansäure. Anschließend wurde hydrogenolytisch mit Wasserstoff unter Normaldruck über 10% Palladium/ Aktivkohle in Ethanol die

Z-Gruppe abgespalten. Dann erfolgte eine Umsetzung mit 1-Acetoxypyrrolidin-2,5-dion in DMF in Gegenwart von N,N-Diisopropylethylamin. Das erhaltene Intermediat wurde dann mit Intermediat C116 mittels HATU-Kupplung in Gegenwart von N,N-Diisopropylethylamin umgesetzt. Im letzten Schritt erfolgte die Abspaltung des tert.-Butylesters durch 2h Rühren bei 50°C mit 6 Equivalenten Zinkchlorid in Trifluorethanol.

LC-MS (Methode 1): $R_t$ = 0.95 min; MS (ESIpos): m/z = 1035 $(M+H)^+$.

**Intermediat R34**

N-{5-[(2,5-Dioxopyrrolidin-1-yl)oxy]-5-oxopentanoyl}-L-alanyl-D-alanyl-$N^1$-{3-[{(1R)-1-[1-benzyl-4-(2,5-difluorphenyl)-1H-pyrrol-2-yl]-2,2-dimethylpropyl}(glycoloyl)amino]propyl}-L-aspartamid

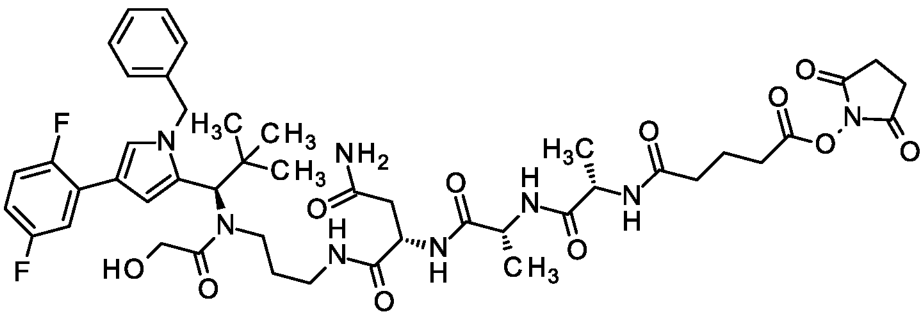

Die Titelverbindung wurde ausgehend von Verbindung M9 zunächst durch Kupplung mit Intermediat L108 in Gegenwart von HATU und N,N-Diisopropylethylamin hergestellt. Im nächsten Schritt wurde die Z-Schutzgruppe durch Hydrierung über 10%-igem Palladium auf Aktivkohle in DCM/Methanol unter Wasserstoff-Normaldruck bei RT entfernt und das entschützte Intermediat anschließend durch Umsetzung mit 1,1'-[(1,5-Dioxopentan-1,5-diyl)bis(oxy)]dipyrrolidin-2,5-dion in Gegenwart von N,N-Diisopropylethylamin in DMF in die Titelverbindung überführt.

LC-MS (Methode 1): $R_t$ = $R_t$ = 1.01 min; MS (ESIpos): m/z = 937 $[M+H]^+$.

**Intermediat R35**

N-Acetyl-L-alanyl-D-alanyl-$N^1$-[(20R)-25-{(1R)-1-[1-benzyl-4-(2,5-difluorphenyl)-1H-pyrrol-2-yl]-2,2-dimethylpropyl}-20-carboxy-1-(2,5-dioxo-2,5-dihydro-1H-pyrrol-1-yl)-2,18,24-trioxo-6,9,12,15-tetraoxa-22-thia-3,19,25-triazaoctacosan-28-yl]-L-aspartamid

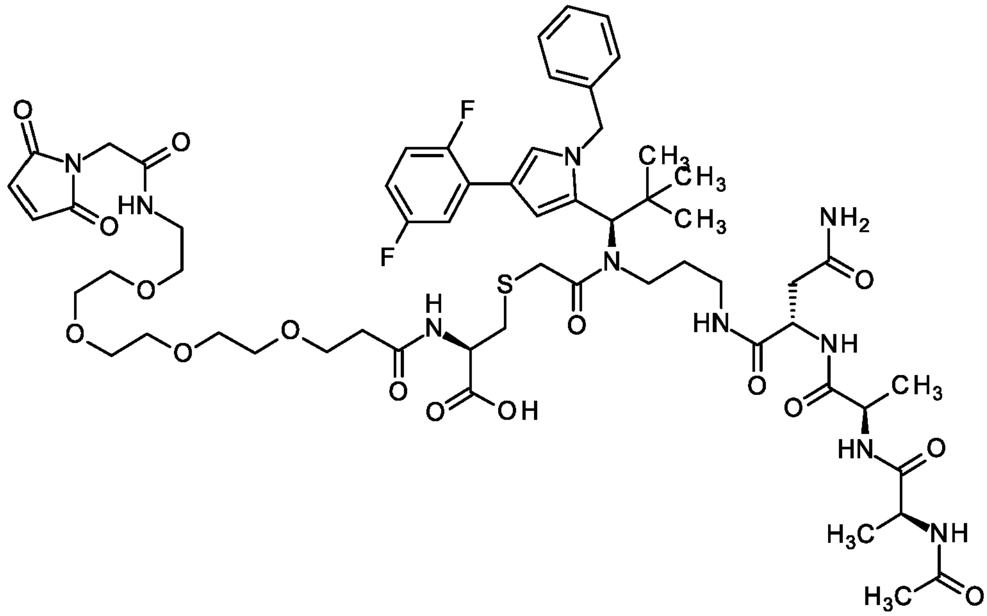

Die Titelverbindung wurde ausgehend von Verbindung C119 zunächst durch Kupplung mit Intermediat L111 in Gegenwart von HATU und N,N-Diisopropylethylamin hergestellt. Im nächsten Schritt wurde der Boc-Schutzgruppe sowie der Trimethylsilylethylester mittels Zinkchlorid in Trifluorethanol entfernt und das entschützte Intermediat anschließend durch Umsetzung mit 1-(2,5-Dioxo-2,5-dihydro-1H-pyrrol-1-yl)-2-oxo-6,9,12,15-tetraoxa-3-azaoctadecan-18-säure in Gegenwart von HATU und N,N-Diisopropylethylamin in die Titelverbindung überführt.

LC-MS (Methode 12): $R_t$ = 1.78 min; MS (ESIneg): m/z = 1253 $[M-H]^-$.

## Intermediat R36

N-Acetyl-L-alanyl-D-alpha-asparagyl-N1-[(2S)-4-[{(1R)-1-[1-benzyl-4-(2,5-difluorphenyl)-1H-pyrrol-2-yl]-2,2-dimethylpropyl}(glycoloyl)amino]-1-{[2-({N-[(2,5-dioxo-2,5-dihydro-1H-pyrrol-1-yl)acetyl]-D-alpha-glutamyl}amino)ethyl]amino}-1-oxobutan-2-yl]-L-aspartamid

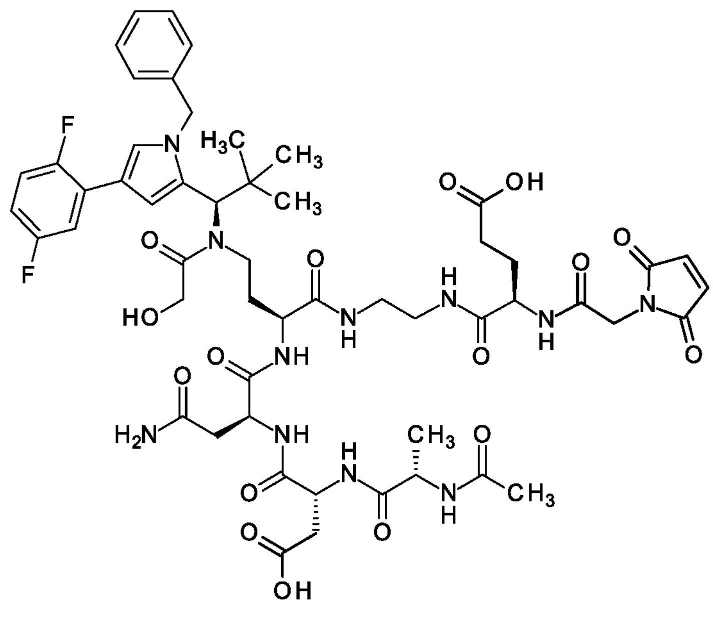

Die Synthese der Titelverbindung begann mit der HATU-Kupplung in Gegenwart von N,N-Diisopropylethylamin

von 2,5-Dioxopyrrolidin-1-yl-N-[(benzyloxy)carbonyl]-L-alaninat mit (2R)-2-Amino-4-tert-butoxy-4-oxobutansäure. Anschließend wurde hydrogenolytisch mit Wasserstoff unter Normaldruck über 10% Palladium/ Aktivkohle in Ethanol die Z-Gruppe abgespalten. Dann erfolgte eine Umsetzung mit 1-Acetoxypyrrolidin-2,5-dion in DMF in Gegenwart von N,N-Diisopropylethylamin. Das erhaltene Intermediat wurde dann mit Intermediat C123 mittels HATU-Kupplung in DMF in Gegenwart von N,N-Diisopropyl ethylamin umgesetzt. Dann erfolgte die Abspaltung der tert.-Butylester sowie der Boc-Schutzgruppe durch 2h Rühren bei 50°C mit 6 Equivalenten Zinkchlorid in Trifluorethanol. Im letzten Schritt wurde durch Kupplung mit 1-{2-[(2,5-Dioxopyrrolidin-1-yl)oxy]-2-oxoethyl}-1H-pyrrol-2,5-dion in DMF in Gegenwart von N,N-Diisopropylethylamin die Titelverbindung hergestellt.

LC-MS (Methode 1): $R_t$ = 0.89 min; MS (ESIpos): m/z = 1164 $(M+H)^+$.

## Intermediat R37

N-(Pyridin-4-ylacetyl)-L-alanyl-D-alanyl-N1-[(20R)-25-{(1R)-1-[1-benzyl-4-(2,5-difluorphenyl)-1H-pyrrol-2-yl]-2,2-dimethylpropyl}-20-carboxy-1-(2,5-dioxo-2,5-dihydro-1H-pyrrol-1-yl)-2,18,24-trioxo-6,9,12,15-tetraoxa-22-thia-3,19,25-triazaoctacosan-28-yl]-L-aspartamid

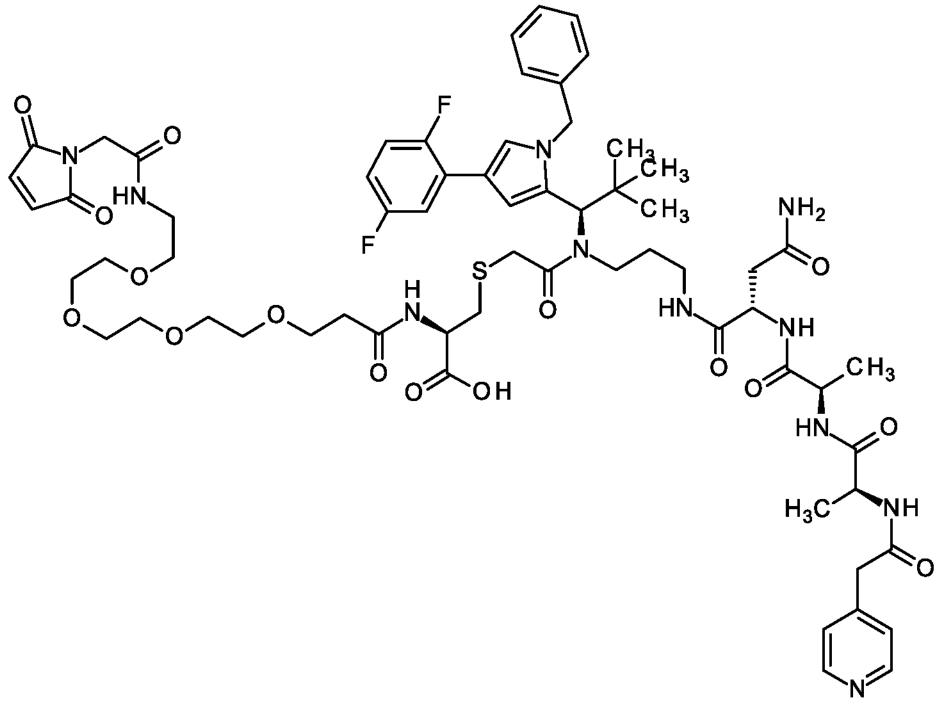

Die Titelverbindung wurde ausgehend von Verbindung C119 zunächst durch Kupplung mit Intermediat L110 in Gegenwart von HATU und N,N-Diisopropylethylamin hergestellt. Im nächsten Schritt wurde die Boc-Schutzgruppe sowie der Trimethylsilylethylester mittels Zinkchlorid in Trifluorethanol entfernt und das entschützte Intermediat anschließend durch Umsetzung mit 1-(2,5-Dioxo-2,5-dihydro-1H-pyrrol-1-yl)-2-oxo-6,9,12,15-tetraoxa-3-azaoctadecan-18-säure in Gegenwart von HATU und N,N-Diisopropylethylamin in die Titelverbindung überführt.

LC-MS (Methode 1): $R_t$ = 0.86 min; MS (ESIpos): m/z = 1332 $[M+H]^+$.

## Intermediat R38

N-Acetyl-D-alpha-asparagyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

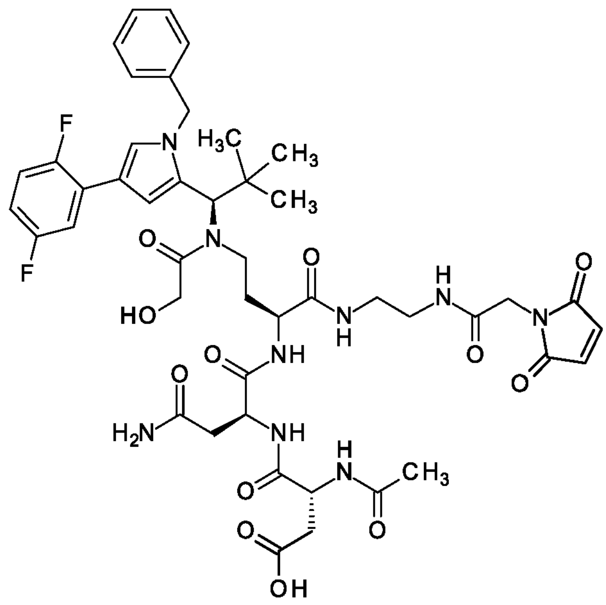

Die Synthese der Titelverbindung begann mit der Umsetzung von 1-Acetoxypyrrolidin-2,5-dion in DMF mit (2R)-2-Amino-4-tert-butoxy-4-oxobutansäure in Gegenwart von N,N-Diisopropylethylamin. Das erhaltene Intermediat wurde dann in DMF mit Intermediat C116 mittels HATU-Kupplung in Gegenwart von N,N-Diisopropylethylamin umgesetzt. Im letzten Schritt erfolgte die Abspaltung des tert.-Butylesters durch 40min Rühren bei 50°C mit 6 Equivalenten Zinkchlorid in Trifluorethanol.

LC-MS (Methode 1): $R_t$ = 0.92 min; MS (ESIpos): m/z = 964 $(M+H)^+$.

**Intermediat R39**

N-Acetyl-D-alpha-asparagyl-$N^1$-[(2S)-4-[{(1R)-1-[1-benzyl-4-(2,5-difluorphenyl)-1H-pyrrol-2-yl]-2,2-dimethylpropyl}(glycoloyl)amino]-1-{[2-({N-[(2,5-dioxo-2,5-dihydro-1H-pyrrol-1-yl)acetyl]-D-alpha-glutamyl}amino)ethyl]amino}-1-oxobutan-2-yl]-L-aspartamid

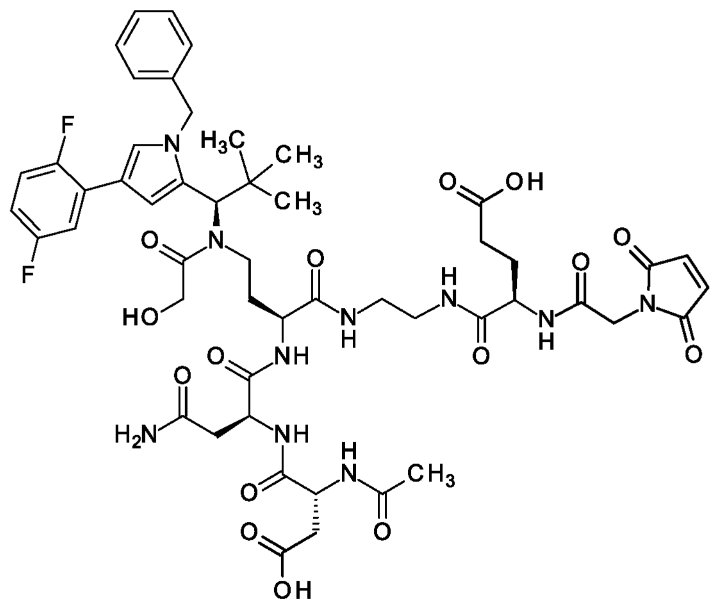

Die Synthese der Titelverbindung begann mit der Umsetzung von 1-Acetoxypyrrolidin-2,5-dion in DMF mit

(2R)-2-Amino-4-tert-butoxy-4-oxobutansäure in Gegenwart von N,N-Diisopropylethylamin. Das erhaltene Intermediat wurde dann in DMF mit Intermediat C123 mittels HATU-Kupplung in Gegenwart von N,N-Diisopropylethylamin umgesetzt. Dann erfolgte die Abspaltung der tert.-Butylester sowie der Boc-Schutzgruppe durch 8h Rühren bei 50°C mit 8 Equivalenten Zinkchlorid in Trifluorethanol. Im letzten Schritt wurde durch Kupplung mit 1-{2-[(2,5-Dioxopyrrolidin-1-yl)oxy]-2-oxoethyl}-1H-pyrrol-2,5-dion in DMF in Gegenwart von N,N-Diisopropylethylamin die Titelverbindung hergestellt.

LC-MS (Methode 1): $R_t$ = 0.89 min; MS (ESIpos): m/z = 1093 $(M+H)^+$.

## Intermediat R40

N-[6-(2,5-Dioxo-2,5-dihydro-1H-pyrrol-1-yl)hexanoyl]-L-alanyl-D-alanyl-$N^1$-{3-[{(1R)-1-[1-benzyl-4-(2,5-difluorphenyl)-1H-pyrrol-2-yl]-2,2-dimethylpropyl}(glycoloyl)amino]propyl}-L-aspartamid

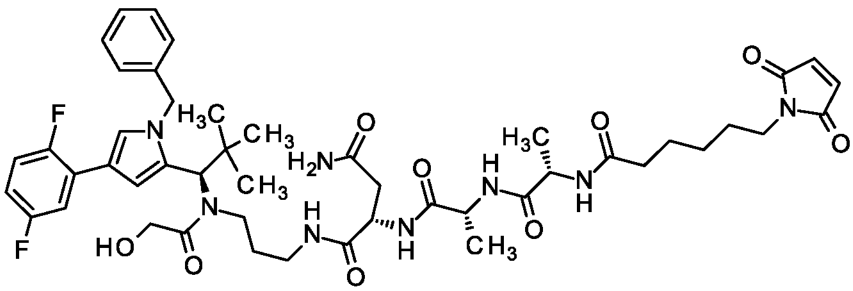

Die Titelverbindung wurde ausgehend von Verbindung M9 zunächst durch Kupplung mit Intermediat L108 in Gegenwart von HATU und N,N-Diisopropylethylamin hergestellt. Im nächsten Schritt wurde die Z-Schutzgruppe durch Hydrierung über 10%-igem Palladium auf Aktivkohle in DCM/Methanol unter Wasserstoff-Normaldruck bei RT entfernt und das entschützte Intermediat anschließend durch Umsetzung mit 1-{6-[(2,5-Dioxopyrrolidin-1-yl)oxy]-6-oxohexyl}-1H-pyrrol-2,5-dion in Gegenwart von N,N-Diisopropylethylamin in DMF in die Titelverbindung überführt.

LC-MS (Methode 1): $R_t$ = $R_t$ = 1.05 min; MS (ESIpos): m/z = 919 $[M+H]^+$.

## Intermediat R41

N-[6-(2,5-Dioxo-2,5-dihydro-1H-pyrrol-1-yl)hexanoyl]-L-alanyl-D-alanyl-$N^1$-{3-[{(1R)-1-[1-benzyl-4-(2,5-difluorphenyl)-1H-pyrrol-2-yl]-2,2-dimethylpropyl}(glycoloyl)amino]propyl}-L-aspartamid

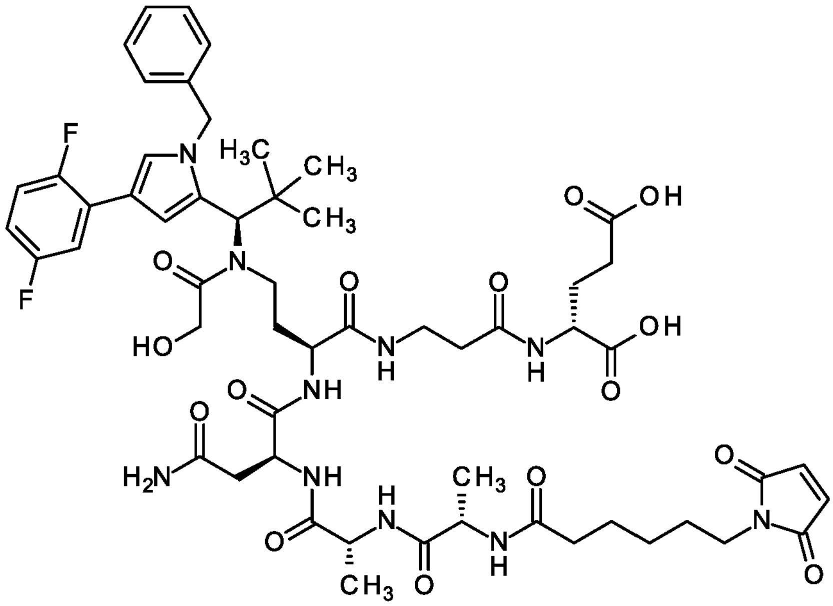

Die Titelverbindung wurde ausgehend von Verbindung C121 zunächst durch Kupplung mit Intermediat L108 in Gegenwart von HATU und N,N-Diisopropylethylamin hergestellt. Im nächsten Schritt wurden alle Schutzgruppen durch Hydrierung über 10%-igem Palladium auf Aktivkohle in Ethanol unter Wasserstoff-Normaldruck bei RT entfernt und das entschützte Intermediat anschließend durch Umsetzung mit 1-{6-[(2,5-Dioxopyrrolidin-1-yl)oxy]-6-oxohexyl}-1H-pyrrol-2,5-dion in Gegenwart von N,N-Diisopropylethylamin in DMF in die Titelverbindung überführt.

LC-MS (Methode 1): $R_t$ = $R_t$ = 0.96 min; MS (ESIpos): m/z = 1163 $[M+H]^+$.

## Intermediat R42

N-(Bromacetyl)-L-alanyl-D-alanyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1R)-1,3-dicarboxypropyl]amino}-3-oxopropyl)amino]-1-oxobutan-2-yl}-L-aspartamid

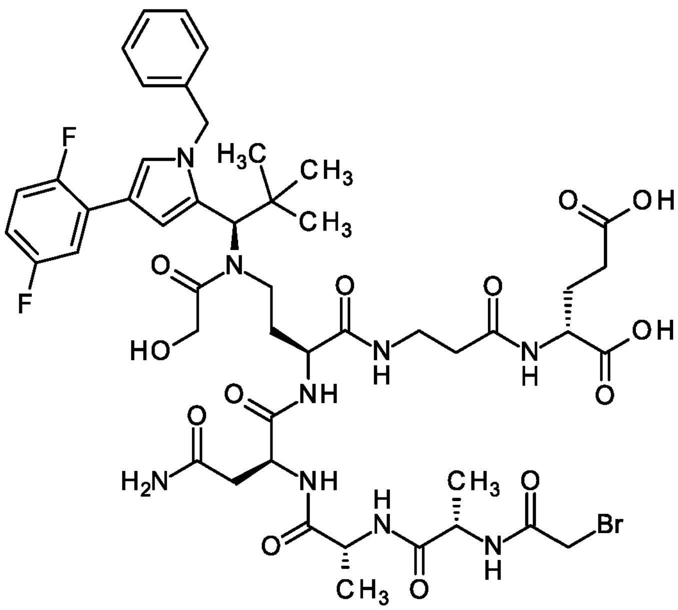

Die Titelverbindung wurde ausgehend von Verbindung C121 zunächst durch Kupplung mit Intermediat L108 in Gegenwart von HATU und N,N-Diisopropylethylamin hergestellt. Im nächsten Schritt wurden alle Schutzgruppen

durch Hydrierung über 10%-igem Palladium auf Aktivkohle in Ethanol unter Wasserstoff-Normaldruck bei RT entfernt und das entschützte Intermediat anschließend durch Umsetzung mit 1-(2-Bromacetoxy) pyrrolidin-2,5-dionin Gegenwart von N,N-Diisopropylethylamin in DMF in die Titelverbindung überführt.

LC-MS (Methode 1): $R_t$ = $R_t$ = 0.93 min; MS (ESIpos): m/z = 1090 und 1092 $[M+H]^+$.

**Intermediat R43**

N-Acetyl-D-alanyl-N[1]-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]-amino} ethyl)amino]-1-oxobutan-2-yl}-L-aspartamid

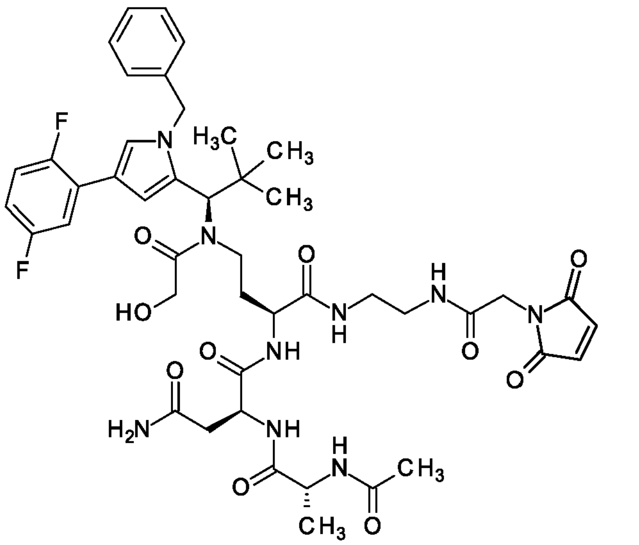

81 mg (0.1 mmol) von Intermediat F104 wurden in Analogie zu Intermediat R1mit 43 mg (0.13 mmol) 2,5-Dioxopyrrolidin-1-yl-N[2]-(tert-butoxycarbonyl)-L-asparaginat gekuppelt. Dann erfolgte die Abspaltung der Boc-Schutzgruppe durch 20 min Rühren bei 50°C mit 6 Equivalenten Zinkchlorid in Trifluorethanol. Im letzten Schritt wurde durch Kupplung mit N-Acetyl-D-alanin in DMF mittels HATU in Gegenwart von N,N-Diisopropylethylamin die Titelverbindung hergestellt.

LC-MS (Methode 12): $R_t$ = 1.77 min; MS (ESIpos): m/z = 920 $(M+H)^+$.

**Intermediat R44**

N[2]-Acetyl-D-asparaginyl-N[1]-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]-amino}ethyl)amino]-1-oxobutan-2-yl}-L-aspartamid

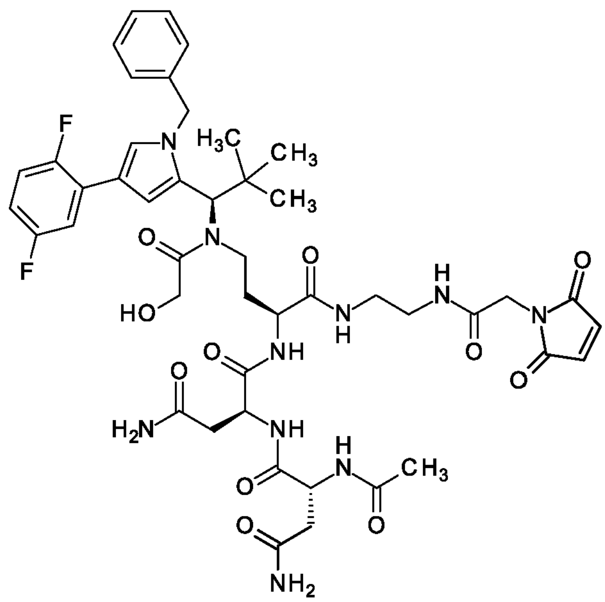

Die Titelverbindung wurde ausgehend von Verbindung C116 durch Kupplung mit Intermediat L132 in Gegenwart von HATU und N,N-Diisopropylethylamin hergestellt. LC-MS (Methode 1): $R_t$ = $R_t$ = 0.91 min; MS (ESIpos): m/z = 963 $[M+H]^+$.

## Intermediat R45

N-{5-[(2,5-Dioxopyrrolidin-1-yl)oxy]-5-oxopentanoyl}-L-alanyl-D-alpha-asparagyl-$N^1$-{3-[{(1R)-1-[1-benzyl-4-(2,5-difluorphenyl)-1H-pyrrol-2-yl]-2,2-dimethylpropyl}(glycoloyl)-amino]propyl}-L-aspartamid

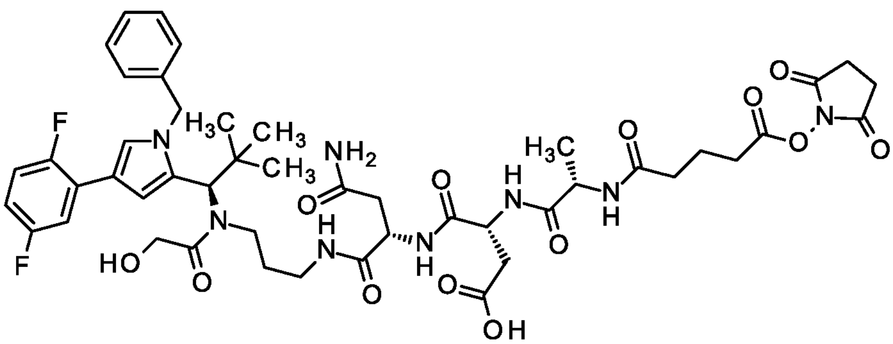

Die Titelverbindung wurde ausgehend von Verbindung M9 mit dem Fachmann bekannten Methoden der peptidchemie über mehrere Stufen hergestellt:
Zunächst durch Kupplung der Verbindung M9 mit 4-Nitrophenyl-$N^2$-[(benzyloxy)carbonyl]-L-asparaginat in DMF in Gegenwart von N,N-Diisopropylethylamin; dann folgende Abspaltung der Z-Schutzgruppe durch Hydrierung über 10%-igem Palladium auf Aktivkohle in Ethanol unter Wasserstoff-Normaldruck bei RT; dann folgende Kupplung mit (2R)-2-{[(Benzyloxy)carbonyl]amino}-4-tert-butoxy-4-oxobutansäure in Gegenwart von HATU und N,N-Diisopropylethylamin; dann folgende Abspaltung der Z-Schutzgruppe durch Hydrierung über 10%-igem Palladium auf Aktivkohle in DCM/Methanol 1:1 unter Wasserstoff-Normaldruck bei RT; dann folgende Kupplung mit N-[(Benzyloxy)carbonyl]-L-alanin in Gegenwart von HATU und N,N-Diisopropylethylamin und erneute hydrogenolytische Abspaltung der Z-Schutzgruppe; anschließende Spaltung des tert.-Butylesters durch 3 h Rühren bei 50°C mit 6 Equivalenten Zinkchlorid in Trifluorethanol und schließlich durch durch Umsetzung mit 1,1'-[(1,5-Dioxopentan-1,5-diyl)bis(oxy)] dipyrrolidin-2,5-dion in Gegenwart von N,N-Diisopropylethylamin in DMF.

LC-MS (Methode 1): $R_t$ = 0.98 min; MS (ESIpos): m/z = 981 $[M+H]^+$.

**Intermediat R46**

N-[(2,5-Dioxo-2,5-dihydro-1H-pyrrol-1-yl)acetyl]-L-alanyl-D-alpha-asparagyl-$N^1$-{3-[{(1R)-1-[1-benzyl-4-(2,5-difluorphenyl)-1H-pyrrol-2-yl]-2,2-dimethylpropyl}(glycoloyl)amino]-propyl}-L-aspartamid

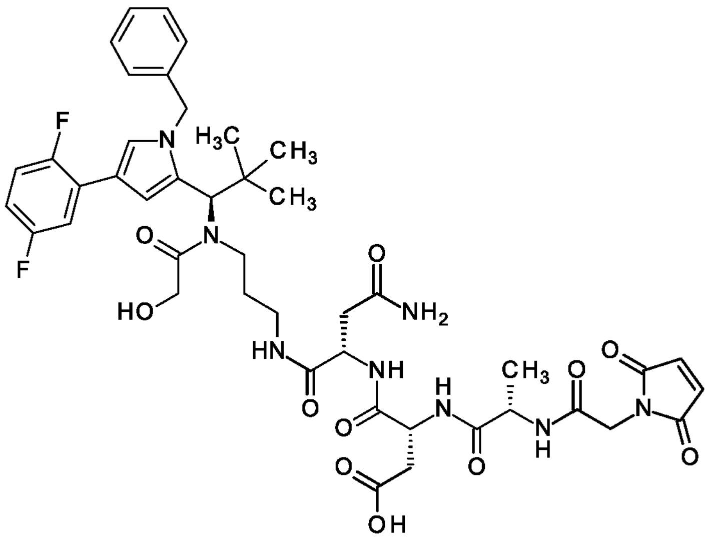

Die Titelverbindung wurde in Analogie zu Intermediat R45 hergestellt. Im letzten Schritt wurde in die Kupplung an Stelle von 1,1'-[(1,5-Dioxopentan-1,5-diyl)bis(oxy)] dipyrrolidin-2,5-dion jedoch 1-{2-[(2,5-Dioxopyrrolidin-1-yl)oxy]-2-oxoethyl}-1H-pyrrol-2,5-dion eingesetzt.

LC-MS (Methode 1): $R_t$ = $R_t$ = 0.99 min; MS (ESIpos): m/z = 907 $[M+H]^+$

**Intermediat R47**

N-[(2,5-Dioxo-2,5-dihydro-1H-pyrrol-1-yl)acetyl]-L-alanyl-D-alanyl-$N^1$-[3-({(1R)-1-[1-benzyl-4-(2,5-difluorphenyl)-1H-pyrrol-2-yl]-2,2-dimethylpropyl}{[(3-{[(1R)-1,2-dicarboxyethyl]-amino}-3-oxopropyl)sulfanyl]acetyl}amino)propyl]-L-aspartamid -trifluoressigsäure Salz

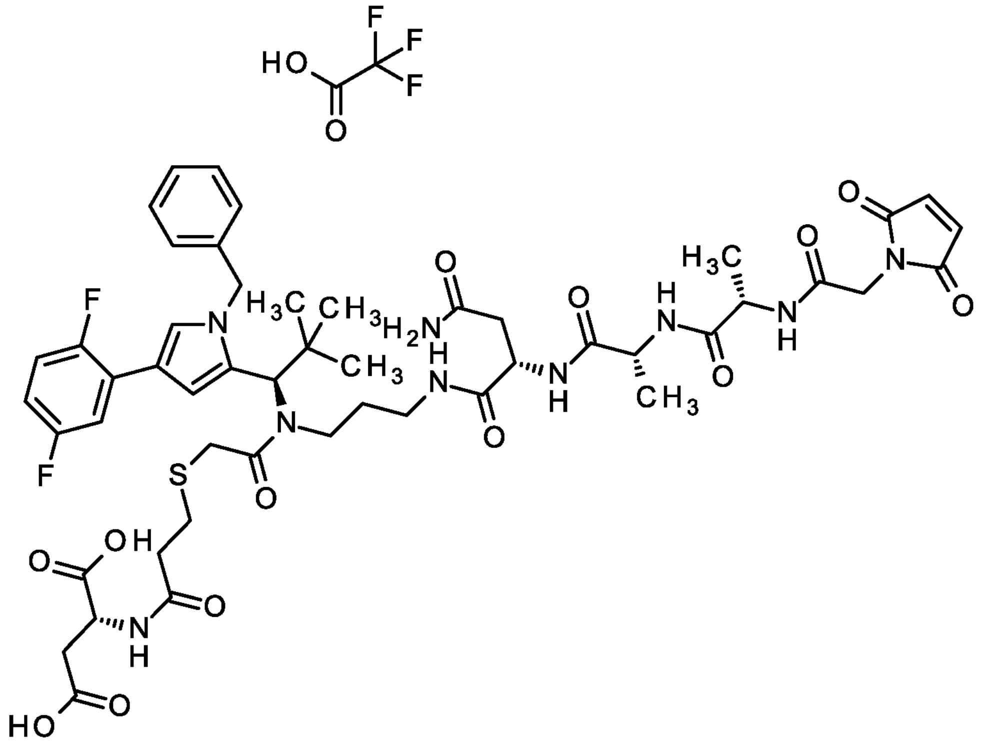

Intermediat C127 wurden in Trifluorethanol (2.0 ml) gelöst und mit Zinkdichlorid (4.18 mg, 30.6 µmol) versetzt. Nach einer Stunde rühren bei 50 °C wurde Zinkchlrid (4.18 mg, 30.6 µmol) zugegeben und die Reaktionsmischung wurde eine weitere Stunde bei 50 °C gerührt. Es wurde erneut Zinkchlorid (4.18 mg, 30.6 µmol) zugegeben und die Reaktionsmischung rührte 1 h bei 50 °C. Die Reaktionsmischung wurde mit Ethylendiamin-N,N,N',N'-tetraessigsäure (8.95 mg, 30.6 µmol) versetzt, 10 min gerührt und dann mit Wasser (0.1% TFA) versetzt. Die Reinigung erfolgte direkt mittles präp. RP-HPLC. Die Lösemittel wurden im Vakuum verdampft und der Rückstand wurde lyophilisiert. Man erhielt 4.3 mg (71 % d. Th.) der Titelverbindung.

LC-MS (Methode 1): $R_t$ = 0.95 min; MS (ESIneg): m/z = 1064 $[M-H]^-$

## Intermediat R48

N-(Pyridin-4-ylacetyl)-L-alanyl-D-histidyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

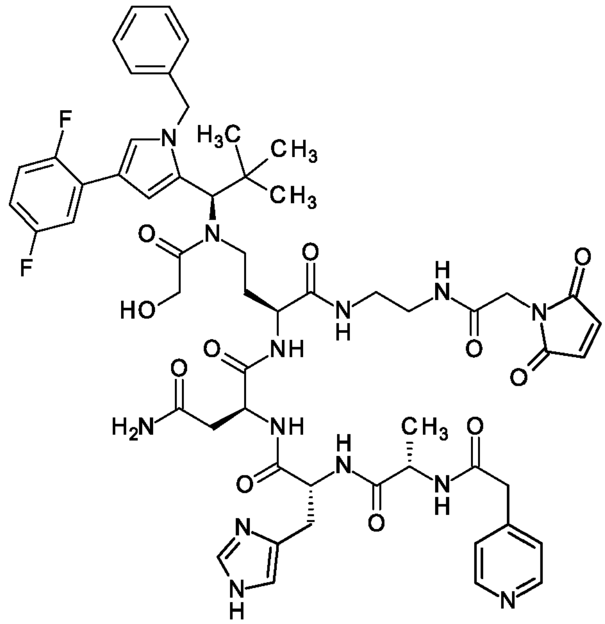

Die Titelverbindung wurde hergestellt durch Kupplung von Intermediat C102 mit tert-Butyl (2-aminoethyl)carbamat in Gegenwart von HATU und N,N-Diisopropylethylamin, anschließender Abspaltung der Z-Schutzgruppe durch 1-stündige Hydrierung über 10%-igem Palladium auf Aktivkohle in DCM-Methanol 1:1 bei RT unter Wasserstoff-Normaldruck, dann folgender Kupplung mit 4-Nitrophenyl-N2-[(benzyloxy)carbonyl]-L-asparaginat in DMF und anschließender Abspaltung der Z-Schutzgruppe durch 1-stündige Hydrierung über 10%-igem Palladium auf Aktivkohle in Ethanol bei RT unter Wasserstoff-Normaldruck.

Dieses Intermediat wurde anschließend mit N-[(9H-Fluoren-9-ylmethoxy)carbonyl]-1-{[2-(trimethylsilyl)ethoxy]carbonyl}-D-histidin, welches zuvor durch Umsetzung von N-[(9H-Fluoren-9-ylmethoxy)carbonyl]-D-histidin mit 1-({[2-(Trimethylsilyl)ethoxy]carbonyl}oxy) pyrrolidin-2,5-dion in Gegenwart von N,N-Diisopropylethylamin in DMF hergestellt wurde, in Gegenwart von HATU und N,N-Diisopropylethylamin in DMF gekuppelt. Dann wurde die Fmoc-Schutzgruppe mit Piperidin in DMF abgespalten. Die entschützte Verbindung wurde in Gegenwart von HATU und N,N-Diisopropylethylamin mit N-(Pyridin-4-ylacetyl)-L-alaninhydrochlorid (1:1) gekuppelt, das zuvor durch Umsetzung von Pyridin-4-ylessigsäurehydrochlorid (1:1) mit tert.-Butyl-L-alaninathydrochlorid (1:1) mittels HATU, und anschließender tert.-Butylester Spaltung mit TFA in DCM hergestellt wurde.

Von dem erhaltenen Intermediat wurde durch 1-stündiges Rühren bei 50°C in Trifluorethanol mit 6 Equiv. Zinkchlorid die Boc-Gruppe abgespalten. Im letzten Schritt wurde durch Umsetzung mit 1-{2-[(2,5-Dioxopyrrolidin-1-yl)oxy]-2-oxoethyl}-1H-pyrrol-2,5-dion in DMF in Gegenwart von N,N-Diisopropylethylamin die Titelverbindung hergestellt.

LC-MS (Methode 1): $R_t$ = 0.75 min; MS (ESIpos): m/z = 1134 $[M+H]^+$.

## Intermediat R49

N-{5-[(2,5-Dioxopyrrolidin-1-yl)oxy]-5-oxopentanoyl}-L-alanyl-D-alanyl-N1-[3-({(1R)-1-[1-benzyl-4-(2,5-difluorphenyl)-1H-pyrrol-2-yl]-2,2-dimethylpropyl}{[(3-{[(1R)-1,2-dicarboxy-ethyl]amino}-3-oxopropyl)sulfanyl]acetyl}amino)propyl]-L-aspartamid-trifluoressigsäure Salz

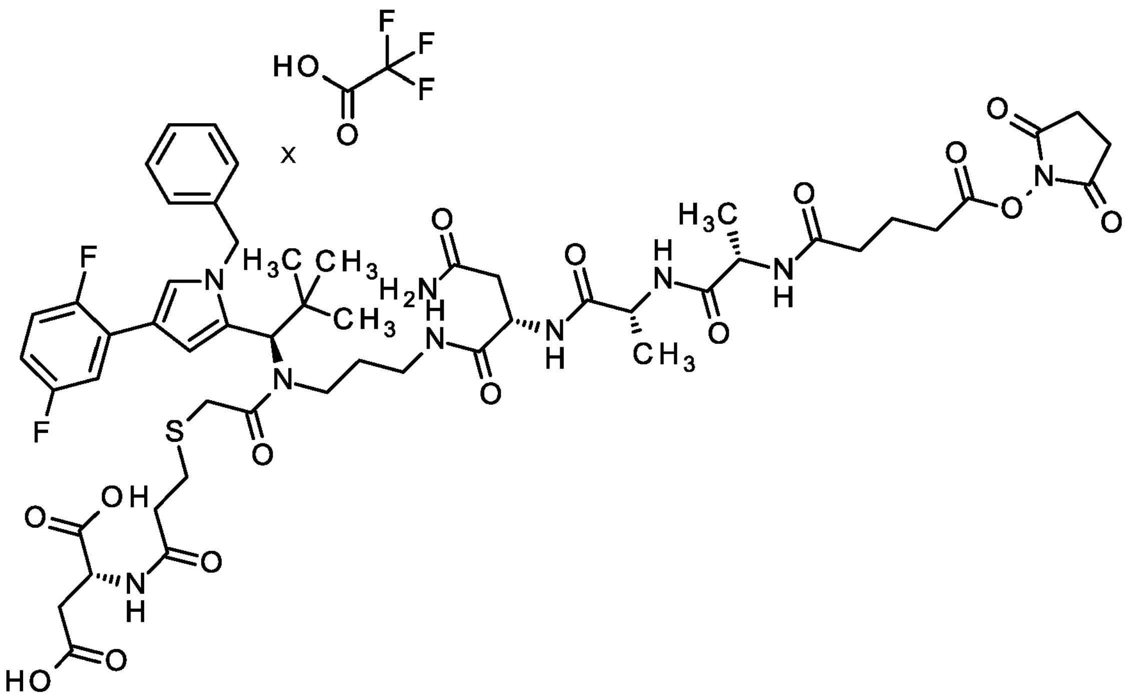

L-Alanyl-D-alanyl-N1-[3-({(1R)-1-[1-benzyl-4-(2,5-difluorphenyl)-1H-pyrrol-2-yl]-2,2-dimethylpropyl}{[(3-{[(1R)-1,2-dicarboxyethyl]amino}-3-oxopropyl)sulfanyl]acetyl}amino)propyl]-L-aspartamid -trifluoressigsäure Salz (7.60 mg, 6.57 µmol, Intermediat C129) und 1,1'-[(1,5-Dioxopentan-1,5-diyl)bis(oxy)]dipyrrolidin-2,5-dion (5.36 mg, 16.4 µmol) wurden in DMF (1.0 ml) gelöst und mit N,N-Diisopropylethylamin (4.6 µl, 26 µmol) versetzt. Die Mischung wurde 3.5 h bei Raumtemperatur gerührt. Anschließend wurde mit Wasser+ 01% TFA gequencht und die Mischung wurde direkt mittels präp. RP-HPLC gereinigt. Die Lösemittel wurden im Vakuum verdampft und der Rückstand wurde lyophilisiert.

LC-MS (Methode 1): $R_t$ = 0.97 min; MS (ESIneg): m/z = 1138 $[M-H]^-$

## Intermediat R50

$N^2$-Acetyl-L-lysyl-L-alanyl-D-alanyl-$N^1$-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1R)-1,3-dicarboxypropyl]amino}-3-oxopropyl)amino]-1-oxobutan-2-yl}-L-aspartamid -trifluoressigsäure Salz

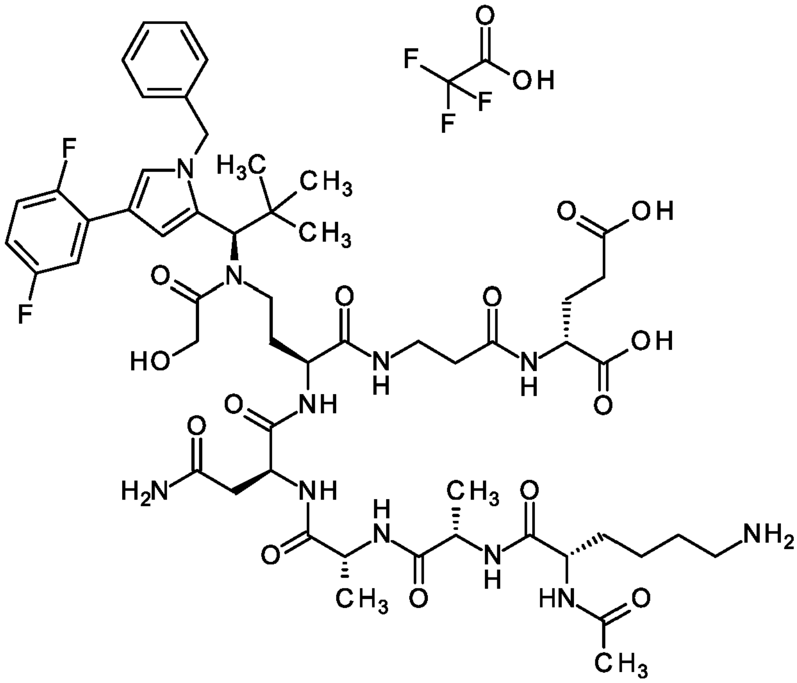

Die Titelverbindung wurde ausgehend von Verbindung C110D mit dem Fachmann bekannten Methoden der Peptidchemie über mehrere Stufen hergestellt:
Zunächst wurde das Intermediates C110D mit Intermediat L134 in DMF in Gegenwart von N,N-Diisopropylethylamin und HATU gekuppelt.

LC-MS (Methode 1): $R_t$ = 1.27 min; MS (ESIpos): m/z = 1454 $[M+H]^+$

Anschließend wurde die Z-Schutzgruppe durch Hydrierung über 10%-igem Palladium auf Aktivkohle in Dichlormethan/Methanl unter Wasserstoff-Normaldruck bei RT abgespalten.

LC-MS (Methode 1): $R_t$ = 0.76 min; MS (ESIpos): m/z = 1140 $[M+H]^+$

<u>**Intermediat R51**</u>

Trifluoressigsäure --N-(pyridin-4-ylacetyl)-L-alanyl-D-alanyl-$N^1$-{(2S)-1-({2-[($N^{2-}$acetyl-L-lysyl)amino]ethyl}amino)-4-[{(1R)-1-[1-benzyl-4-(2,5-difluorphenyl)-1H-pyrrol-2-yl]-2,2-dimethylpropyl}(glycoloyl)amino]-1-oxobutan-2-yl}-L-aspartamid Salz

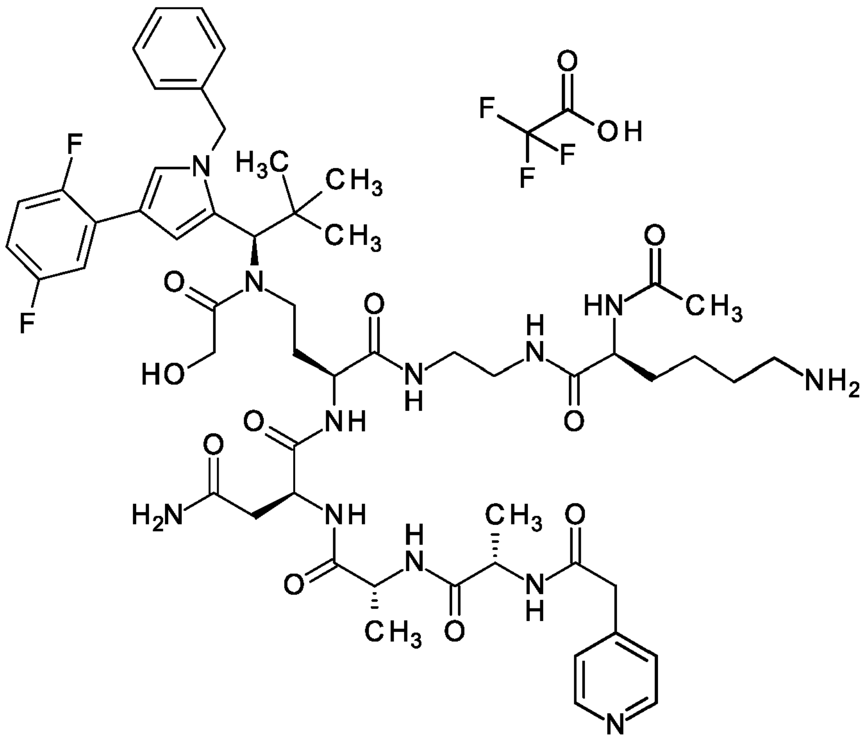

Die Titelverbindung wurde ausgehend von Verbindung C128 mit dem Fachmann bekannten Methoden der Peptidchemie über mehrere Stufen hergestellt:
Zunächst wurde das Intermediates C128 mit Intermediat L110 in DMF in Gegenwart von N,N-Diisopropylethylamin und HATU gekuppelt.

LC-MS (Methode 1): $R_t$ = 0.97 min; MS (ESIpos): m/z = 1201 $[M+H]^+$

Anschließende Spaltung des tert.-Butylesters durch 30 min Rühren bei 50°C mit 6 Equivalenten Zinkchlorid in Trifluorethanol lieferte die Titelverbindung.

LC-MS (Methode 1): $R_t$ = 0.72 min; MS (ESIpos): m/z = 1101 $[M+H]^+$

## B: Herstellung von Antikörper-Wirkstoff-Konjugaten (ADC)

### B-1. Allgemeines Verfahren zur Generierung von Antikörpern

Die Proteinsequenz (Aminosäuresequenz) der verwendeten Antikörper, zum Beispiel TPP-2090, TPP-2658, TPP-5442, TPP-8825, TPP-7006, TPP-7007, TPP-10334, TPP-10335, TPP-10336, TPP-10337, TPP-1015, TPP-7510, TPP-7511, TPP-8382 und TPP-8567, wurde in eine für das Protein kodierende DNA Sequenz nach einem dem Fachmann gut bekannten Verfahren überführt und in einen für die transiente Säugerzellkultur geeigneten Expressionsvektor inseriert (wie von Tom et al., Kapitel 12 in Methods Express: Expression Systems herausgegeben von Micheal R. Dyson and Yves Durocher, Scion Publishing Ltd, 2007 beschrieben).

Der kommerziell erhältliche Antiköper Cetuximab (TPP-981; Handelsname: Erbitux), wurde für die hier beschriebenen Ausführungsbeispiele verwendet.

### B-2. Allgemeines Verfahren zur Expression von Antikörpern in Säugerzellen

Die Antikörper, zum Beispiel TPP-2090, TPP-2658, TPP-5442, TPP-8825, TPP-7006, TPP-7007, TPP-10334, TPP-10335, TPP-10336, TPP-10337, TPP-1015, TPP-7510, TPP-7511, TPP-8382 und TPP-8567, wurden in transienten Säugerzellkulturen produziert, wie von Tom et al., Kapitel 12 in Methods Express: Expression Systems herausgegeben von Micheal R. Dyson and Yves Durocher, Scion Publishing Ltd, 2007 beschrieben.

### B-3. Allgemeines Verfahren zur Aufreinigung von Antikörpern aus Zellüberständen.

Die Antikörper, zum Beispiel TPP-2090, TPP-2658, TPP-5442, TPP-8825, TPP-7006, TPP-7007, TPP-10334, TPP-10335, TPP-10336, TPP-10337, TPP-1015, TPP-7510, TPP-7511, TPP-8382 und TPP-8567, wurden aus den

Zellkulturüberständen gewonnen. Die Zellüberstände wurden durch Zentrifugation von Zellen geklärt. Anschließend wurde der Zellüberstand durch Affinitätschromatographie auf einer MabSelect Sure (GE Healthcare) Chromatographiesäule gereinigt. Dazu wurde die Säule in DPBS pH 7.4 (Sigma/Aldrich) equillibriert, der Zellüberstand aufgetragen und die Säule mit ca 10 Säulenvolumina DPBS pH 7.4 + 500 mM Natriumchlorid gewaschen. Die Antikörper wurden in 50 mM Natriumacetat pH 3.5 + 500 mM Natriumchlorid eluiert und anschliessend durch Gelfiltrationschromatographie auf einer Superdex 200 Säule (GE Healthcare) in DPBS pH 7.4 weiter gereinigt.

Die kommerziell erhältlichen Antikörper wurden mit Standard- Chromatographiemethoden aufgereinigt (Protein A Chromatopgraphie, präparative Gelfiltrationschomatographie (SEC - size exclusion Chromatographie)).

## B-4. Allgemeines Verfahren zur Kupplung an Cystein-Seitenketten

In die Kupplungsreaktionen wurde folgender Antikörper eingesetzt:

Beispiele a: Cetuximab (Anti EGFR AK)

Beispiele e: TPP-1015 (Anti-Her2 AK)

Beispiele h1: Anti-B7H3 AK (TPP-8382)

Beispiele h2: Anti-B7H3 AK (TPP-8567)

Beispiele k: Anti-TWEAKR AK (TPP-2658)

Beispiele I1: Anti-TWEAKR AK (TPP-7007)

Beispiele I2: Anti-TWEAKR AK (TPP-7006)

Beispiele I3: Anti-TWEAKR AK (TPP-10336)

Beispiele I4: Anti-TWEAKR AK (TPP-10337)

Die Kupplungsreaktionen wurden üblicherweise unter Argon durchgeführt.

Zu einer Lösung des entsprechenden Antikörpers in PBS-Puffer im Konzentrationsbereich zwischen 1 mg/mL und 20 mg/mL, bevorzugt im Bereich von ungefähr 10mg/ml bis 15 mg/ml wurden zwischen 2 und 5 Äquivalenten Tris(2-carboxyethyl)phosphin-Hydrochlorid (TCEP), gelöst in PBS-Puffer, gegeben und 30 min bis 1h bei RT gerührt. Dazu kann die Lösung des jeweils eingesetzten Antikörpers in der in den Ausführungsbeispielen angegebenen Konzentration eingesetzt werden oder gegebenenfalls auch mit PBS Puffer bis etwa auf die Hälfte der angegebenen Startkonzentration verdünnt werden, um in den bevorzugten Konzentrationsbereich zu kommen. Anschließend wurden, je nach angestrebter Beladung, zwischen 2 und 12 Äquivalenten, bevorzugt etwa 5-10 Äquivalente der zu kuppelnden Maleinimid-Vorläufer-Verbindung oder Halogenid-Vorläufer-Verbindung als Lösung in DMSO hinzugefügt. Dabei sollte die Menge an DMSO 10% des Gesamtvolumens nicht überschreiten. Der Ansatz wurde bei Maleinimid-Precursern 60-240 min bei RT und bei Halogenid-Precursern zwischen 8 und 24 h bei RT gerührt und anschließend über in PBS equillibierte PD 10-Säulen (Sephadex® G-25, GE Healthcare) gegeben und mit PBS-Puffer eluiert. Üblicherweise wurden, wenn nicht anders angegeben, 5 mg des entsprechenden Antikörpers in PBS-Puffer zur Reduktion und der nachfolgenden Kupplung eingesetzt. Nach Aufreinigung über die PD10 Säule wurden so jeweils Lösungen des entsprechenden ADCs in 3.5 mL PBS-Puffer erhalten. Anschließend wurde eine Aufkonzentration mittels Ultrazentrifugation durchgeführt und die Probe ggf. mit PBS-Puffer zurückverdünnt. Falls notwendig wurde zur besseren Abtrennung niedermolekularer Bestandteile die Aufkonzentration mittels Ultrafiltration nach Rückverdünnung mit PBS Puffer wiederholt. Für biologische Testungen wurden je nach Bedarf in den finalen ADC Proben ggf. durch Rückverdünnung Konzentrationen im Bereich von 0.5-15 mg/mL eingestellt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

Die in den Beispielen dargestellten ADCs können in Abhängigkeit vom Linker gegebenenfalls auch mehr oder weniger stark ausgeprägt in Form der hydrolysierten offenkettigen Bernsteinsäureamide mit den Antikörpern verknüpft vorliegen.

Insbesondere die KSP-I-ADCs, die über die Linker-Substruktur

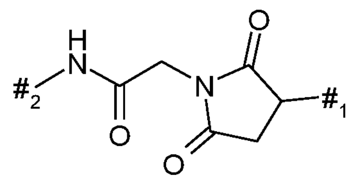

mit Thiolgruppen der Antikörper verknüpft sind, können auch ggf. durch Umpuffern im Anschluss an die Kupplung und ca. 20-24-stündiges Rühren bei pH 8 gemäß Schema 28 gezielt in die die über offenkettige Bersteinsäureamide verknüpften ADCs hergestellt werden.

#1 stellt die Schwefelbrücke zum Antikörper dar und #2 die Verknüpfungsstelle zum modifizierten KSP-Inhibitor

Solche ADCs, bei denen der Linker über hydrolysierte offenkettige Bernsteinsäureamide mit den Antikörpern verknüpft vorliegt, können gegebenenfalls auch gezielt nach exemplarischen Vorschrift wie folgt dargestellt werden:

**Small Scale Kupplung:**

Zu einer Lösung von 2-5 mg des entsprechenden Antikörpers in PBS-Puffer im Konzentrationsbereich zwischen 1 mg/mL und 20 mg/mL, bevorzugt im Bereich von ungefähr 5 mg/ml bis 15 mg/ml wurden zwischen 2 und 5 Äquivalenten Tris(2-carboxyethyl)phosphin-Hydrochlorid (TCEP), gelöst in PBS-Puffer, gegeben und 30 min bis 1h bei RT gerührt. Anschließend wurden, je nach angestrebter Beladung, zwischen 2 und 12 Äquivalenten, bevorzugt etwa 5-10 Äquivalente der zu kuppelnden Maleinimid-Vorläufer-Verbindung als Lösung in DMSO hinzugefügt. Dabei sollte die Menge an DMSO 10% des Gesamtvolumens nicht überschreiten. Der Ansatz wurde 60-240 min bei RT gerührt und anschließend mit PBS-Puffer, der zuvor auf pH 8 eingestellt wurde, auf ein Volumen von 2.5-7.5 ml verdünnt und dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt. Anschließend wurde die Lösung durch Ultrazentrifugation aufkonzentriert und rückverdünnt mit PBS-Puffer (pH 7.2).

**Medium scale Kupplung:**

20-200 mg des betreffenden Antikörpers in PBS-Puffer (c~5-15 mg/mL) wurden unter Argon mit einer Lösung aus 2-5 Equivalenten, bevorzugt 3 Equivalenten TCEP in PBS-Puffer (c~0.2-0.8 mg/ml bevorzugt 0.5 mg/ml) versetzt. Der Ansatz wurde 30min bei RT gerührt und anschließend wurden 2-12, bevorzugt 5-10 Equivalente einer Maleinimid-Vorläuferverbindung gelöst in DMSO zugegeben. Nach weiteren 1.5h-2h Rühren bei RT wurde der Ansatz mit PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf eine Konzentration von 1-7 mg/mL verdünnt. Diese Lösung wurde über Nacht bei RT unter Argon gerührt. Dann erfolgte ggf. wieder ein Umpuffern auf pH 7.2. Die ADC-Lösung wurde durch Ultrazentrifugation aufkonzentriert, rückverdünnt mit PBS-Puffer (pH7.2) und dann ggf. erneut bis auf eine Konzentration von etwa 10mg/mL aufkonzentriert.

Weitere potentiell hydrolyse-sensitive Thianylsuccinimid-Brücken zum Antikörper in den Ausführungsbeispielen enthalten folgende Linker-Substrukturen, wobei #1 die Thioetherverknüpfung zum Antikörper und #1 die Verknüpfungsstelle zum modifizierten KSP-Inhibitor darstellt:

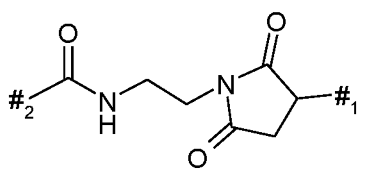

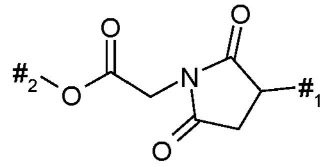

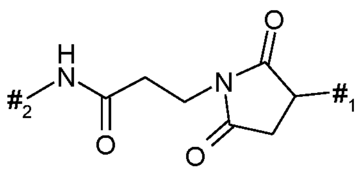

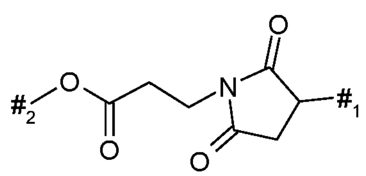

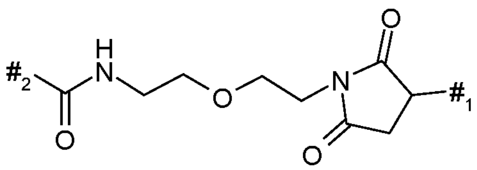 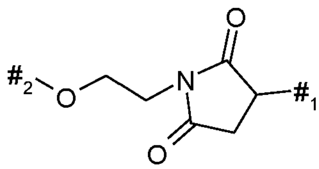

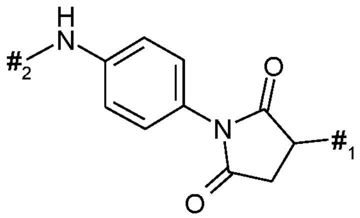 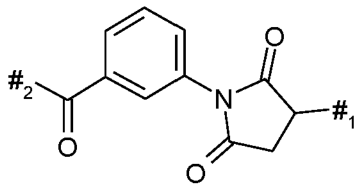

Diese Linker-Substrukturen stellen die Verknüpfungseinheit zum Antikörper dar und haben (neben der weiteren Linkerzusammensetzung) einen signifikanten Einfluß auf die Struktur und das Profil der in Tumorzellen gebildeten Metabolite.

In den dargestellten Strukturformeln kann dabei $AK_1$ die Bedeutung haben

Beispiele a: Cetuximab (partiell reduziert)- S§[1]

Beispiele e: TPP-1015 (partiell reduziert)- S§[1]

Beispiele h1: Anti-B7H3 AK (TPP-8382 partiell reduziert) - S§[1]

Beispiele h2: Anti-B7H3 AK (TPP-8567 partiell reduziert) - S§[1]

Beispiele k: Anti-TWEAKR AK (TPP-2658 partiell reduziert) - S§[1]

Beispiele I1: Anti-TWEAKR AK (TPP-7007 partiell reduziert) - S§[1]

Beispiele I2: Anti-TWEAKR AK (TPP-7006 partiell reduziert) - S§[1]

Beispiele I3: Anti-TWEAKR AK (TPP-10336 partiell reduziert) - S§[1]

Beispiele I4: Anti-TWEAKR AK (TPP-10337 partiell reduziert) - S§[1]

wobei

§[1] die Verknüpfung mit der Succinimid-Gruppe oder mit ggf. daraus entstandenen isomeren hydrolysierten offenkettigen Bernsteinsäureamiden bzw. des Alkylenrestes bedeutet,

und

S für das Schwefelatom eines Cystein-Restes des partiell reduzierten Antikörpers steht.

**B-5. Allgemeines Verfahren zur Kupplung an Lysin-Seitenketten**

In die Kupplungsreaktionen wurden folgende Antikörper eingesetzt:

Beispiele a: Cetuximab (Anti EGFR AK)

Beispiele e: TPP-1015 (Anti-Her2 AK)

Beispiele h1: Anti-B7H3 AK (TPP-8382)

Beispiele h2: Anti-B7H3 AK (TPP-8567)

Beispiele k: Anti-TWEAKR Antikörper (TPP-2658)

Beispiele I1: Anti-TWEAKR AK (TPP-7007)

Beispiele I2: Anti-TWEAKR AK (TPP-7006)

Beispiele I3: Anti-TWEAKR AK (TPP-10336)

Beispiele I4: Anti-TWEAKR AK (TPP-10337)

Die Kupplungsreaktionen wurden üblicherweise unter Argon durchgeführt.

Zu einer Lösung des entsprechenden Antikörpers in PBS-Puffer im Konzentrationsbereich zwischen 1 mg/mL und 20 mg/mL, bevorzugt etwa 10 mg/mL, wurden, je nach angestrebter Beladung, zwischen 2 und 8 Äquivalente der zu kuppelnden Vorläufer-Verbindung als Lösung in DMSO gegeben. Nach 30 min bis 6 h Rühren bei RT wurde nochmals die gleiche Menge an Vorläufer-Verbindung in DMSO hinzugefügt. Dabei sollte die Menge an DMSO 10% des Gesamtvolumens nicht überschreiten. Nach weiteren 30 min bis 6 h Rühren bei RT wurde der Ansatz über in PBS equillibierte PD 10-Säulen (Sephadex® G-25, GE Healthcare) gegeben und mit PBS-Puffer eluiert. Üblicherweise wurden, wenn nicht anders angegeben, 5 mg des entsprechenden Antikörpers in PBS-Puffer zur Kupplung eingesetzt. Nach Aufreinigung über die PD10 Säule wurden so jeweils Lösungen des entsprechenden ADCs in 3.5 mL PBS-Puffer erhalten. Anschließend wurde eine Aufkonzentration mittels Ultrazentrifugation durchgeführt und die Probe ggf. mit PBS-Puffer zurückverdünnt. Falls notwendig wurde zur besseren Abtrennung niedermolekularer Bestandteile die Aufkonzentration mittels Ultrafiltration nach Rückverdünnung mit PBS Puffer wiederholt. Für biologische Testungen wurden je nach Bedarf in den finalen ADC Proben ggf. durch Rückverdünnung Konzentrationen im Bereich von 0.5-15 mg/mL eingestellt.

Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

In den dargestellten Strukturformeln hat dabei $AK_2$ die Bedeutung

Beispiele a: Cetuximab - NH§$^2$

Beispiele e: TPP-1015 - NH§$^2$

Beispiele h1: Anti-B7H3 AK (TPP-8382) - NH§$^2$

Beispiele h2: Anti-B7H3 AK (TPP-8567) - NH§$^2$

Beispiele k: Anti-TWEAKR Antikörper (TPP-2658) - NH§$^2$

Beispiele I1: Anti-TWEAKR AK (TPP-7007) - NH§$^2$

Beispiele I2: Anti-TWEAKR AK (TPP-7006) - NH§$^2$

Beispiele I3: Anti-TWEAKR AK (TPP-10336) - NH§$^2$

Beispiele I4: Anti-TWEAKR AK (TPP-10337) - NH§$^2$

wobei

§$^2$ die Verknüpfung mit der Carbonylgruppe bedeutet,

und

NH für die Seitenketten-Aminogruppe eines Lysin-Restes des Antikörpers steht.

## B-5a. Allgemeines Verfahren zur ADC Synthese mittels bakterieller Transglutaminase

In die Kupplungsreaktionen in der Beispielserie t wurden folgende Antikörper eingesetzt (die nachfolgende Nomenklatur Antikörper-HC-N297Z meint den Antikörper, bei dem an beiden schweren Ketten die Aminosäure N297 (Kabat-Nummerierung) durch die Aminosäure Z ausgetauscht wurde, die Nomenklatur TPP-xxxx-HC-Q295N-HC-N297Q meint den Antikörper mit der TPP-XXXX, bei dem an beiden schweren Ketten die Aminosäure Q295 (Kabat-Nummerierung) durch die Aminosäure N und die Aminosäure N297 (Kabat-Nummerierung) durch die Aminosäure Q ausgetauscht wurde. Der Antikörpername des Ursprungsantikörpers kann dabei entweder als Name (Beispiel Trastuzumab) oder als TPP-XXXX angegeben sein (Antikörper mit der TPP-Nummer XXXX)):

$AK_{3a}$: Anti-TWEAKR Antikörper (TPP-2658) (entspricht TPP-2090-HC-N297A)

$AK_{3b}$: Anti-TWEAKR Antikörper (TPP-5442) (entspricht TPP-2090-HC-N297Q)

$AK_{3c}$: Anti-TWEAKR Antikörper (TPP-8825) (entspricht TPP-2090-HC-Q295N-HC-N297Q)

$AK_{3d}$: anti-HER2 Antikörper (TPP-7510) (entspricht TPP-1015-HC-N297A)

$AK_{3e}$: anti-HER2 Antikörper (TPP-7511) (entspricht TPP-1015-HC-N297Q)

Allgemeine Vorschrift um eine maximale DAR von 2 zu erzielen:

Zu einer Lösung von 5 mg der entsprechenden aglyco Antikörper Variante (HC-N297A) in DPBS pH 7.4 (c~5-15 mg/mL) wurden 20 μL (6 Equivalente) einer Lösung eines geeigneten Toxophor-Linker-Precursors (e.g. Intermediate R50 und R51; 10 mM Lösung in DMSO) hinzugegeben. Nach 5 min Inkubation bei 37°C wurden 50 μL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Gesamtvolumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.00625 μmol des b-Transglutaminase Blockers Zedira C100 in 12.5 μL DPBS zu der Lösung hinzugefügt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

**Allgemeine Vorschrift um eine maximale DAR von 4 zu erzielen:**

Zu einer Lösung von 5 mg der entsprechenden aglyco Antikörper Variante (HC-N297Q) in DPBS pH 7.4 (c~5-15mg/mL) wurden 16-24 Euivalente einer Lösung eines geeigneten Toxophor-Linker-Precursors (e.g. Intermediat R50 und R51; 10 mM Lösung in DMSO) hinzugegeben. Nach 5 min Inkubation bei 37°C wurden 400 μL (10 U) einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Gesamtvolumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.1 μmol des b-Transglutaminase Blockers Zedira C100 in 200 μL DPBS zu der Lösung hinzugefügt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

**Allgemeine Vorschrift für Transglutaminase vermittelte Kupplung im größeren Maßstab um eine maximale DAR von 2 zu erhalten:**

Zu einer Lösung von 30 mg der aglycosylierten Variante (HC-N297A) des jeweiligen Antikörpers in DPBS pH 7.4 (c~5-15 mg/mL) wurden 6 Equivalente einer Lösung des entsprechenden Toxophor-Linker-Precursors (10 mM in DMSO) hinzugegeben. Nach 5 min Inkubation bei 37°C wurden 200 μL (7.5 U) einer Lösung von rekombinanter bakterieller Transglutaminase in Wasser (Produkt Nummer T001 von Zedira GmbH, Darmstadt, Germany) (25 U/mL) hinzugegeben und weitere 24h bei 37°C inkubiert. Die Reaktionsmischung wurde über Gelfiltrationschromatographie auf

einer Superdex 200 Säule (GE Healthcare) in DPBS pH 7.4 gereinigt, um kleine Moleküle und die Transglutaminase vom ADC abzutrennen. Anschließend wurde die ADC-Lösung über Amicon Ultracel-30K Zentrifugationsröhrchen (Millipore) auf finale Konzentrationen von 5-25 mg/mL ankonzentriert. Die Lösung wurde anschließend sterilfiltriert.

Die jeweiligen in den Ausführungsbeispielen angegebenen Konzentrationen der ADC-Lösungen wurden bestimmt. Die Beladung wurde nach den in Kapitel B7 beschriebenen Methoden bestimmt. Die ADC batches wurden wie in den Ausführungsbeispielen angezeigt charakterisiert.

**Allgemeine Vorschrift für Transglutyminase vermittelte Kupplung im größeren Maßstab um eine maximale DAR von 4 zu erhalten:**

Zu einer Lösung von 30 mg der aglycosylierten Variante (HC-N297Q) des jeweiligen Antikörpers in DPBS pH 7.4 (c~5-15 mg/mL) wurden 16-24 Equivalente einer Lösung des entsprechenden Toxophor-Linker-Precursors (10 mM in DMSO) hinzugegeben. Nach 5 min Inkubation bei 37°C wurden 2400 μL (60 U) einer Lösung von rekombinanter bakterieller Transglutaminase in Wasser (Produkt Nummer T001 von Zedira GmbH, Darmstadt, Germany) (25 U/mL) hinzugegeben und weitere 24h bei 37°C inkubiert. Die Reaktionsmischung wurde über Gelfiltrationschromatographie auf einer Superdex 200 Säule (GE Healthcare) in DPBS pH 7.4 gereinigt, um kleine Moleküle und die Transglutaminase vom ADC abzutrennen. Anschließend wurde die ADC-Lösung über Amicon Ultracel-30K Zentrifugationsröhrchen (Millipore) auf finale Konzentrationen von 5-25 mg/mL ankonzentriert. Die Lösung wurde anschleießend sterilfiltriert.

Die jeweiligen in den Ausführungsbeispielen angegebenen Konzentrationen der ADC-Lösungen wurden bestimmt. Die Beladung wurde nach den in Kapitel B7 beschriebenen Methoden bestimmt. Die ADC batches wurden wie in den Ausführungsbeispielen angezeigt charakterisiert.

In den dargestellten Strukturformeln der Beispielserie t hat $AK_3$ jeweils die Bedeutung

$AK_{3a}$: Anti-TWEAKR Antikörper (TPP-2658) (entspricht TPP-2090-HC-N297A) - CO-$\S_2$

$AK_{3b}$: Anti-TWEAKR Antikörper (TPP-5442) (entspricht TPP-2090-HC-N297Q) - CO-$\S_2$

$AK_{3c}$: Anti-TWEAKR Antikörper (TPP-8825) (entspricht TPP-2090-HC-Q295N-HC-N297Q) - CO-$\S_2$

$AK_{3d}$: anti-HER2 Antikörper (TPP-7510) (entspricht TPP-1015-HC-N297A) - CO-$\S_2$

$AK_{3e}$: anti-HER2 Antikörper (TPP-7511) (entspricht TPP-1015-HC-N297Q) - CO-$\S_2$

wobei

$\S^2$ die Verknüpfung mit der Aminogruppe eines Toxophor-Linker-Precursors bedeutet,

und

CO für die Seitenketten-Carbonylgruppe eines Glutamin-Restes des Antikörpers steht.

**B-6a. Allgemeines Verfahren zur Herstellung von geschlossenen Succinimid-Cystein-Addukten:**

In einer beispielhaften Ausführung wurden 10 μmol der oben beschriebenen Maleinimid-Vorläuferverbindungen wurden in 3-5 mL DMF aufgenommen und mit 2.1 mg (20 μmol) L-Cystein versetzt. Das Reaktionsgemisch wurde zwischen 2h und 24 h bei RT gerührt, anschließend im Vakuum eingeengt und dann mittels präparativer HPLC gereinigt.

**B-6aa. Allgemeines Verfahren zur Herstellung von isomeren geöffneten Bernsteinsäureamid-Cystein-Addukten:**

In einer beispielhaften Ausführung wurden 68 μmol der oben beschriebenen Maleinimid-Vorläuferverbindungen in 15 mL DMF aufgenommen und mit 36 mg (136 μmol) N-{[2-(Trimethylsilyl)ethoxy]carbonyl}-L-cystein versetzt. Das Reaktionsgemisch wurde ~20h bei RT gerührt, anschließend im Vakuum eingeengt und dann mittels präparativer HPLC gereinigt. Nach Vereinigung der entsprechenden Fraktionen und Abdampfen der Lösemittel im Vakuum wurde der Rückstand in 15 mL THF/Wasser 1:1 gelöst. Es wurden 131 μL einer 2M wässrigen Lithiumhydroxidlösung zugegeben und der Ansatz 1 h bei RT gerührt. Anschließend wurde der Ansatz mit einer 1M Salzsäure neutral gestellt, das Lösemittel im Vakuum abgedampft und der Rückstand durch präparative HPLC gereinigt. Es wurden ~50% d. Th. der regioisomeren geschützten Intermediate als farbloser Schaum erhalten.

Im letzten Schritt wurden 0.023 mmol von diesen regiosisomeren Hydrolyseprodukten in 3 mL 2,2,2-Trifluorethanol gelöst. Es wurden 12.5 mg (0.092 mmol) Zinkchlorid zugegeben und der Ansatz 4 h bei 50°C gerührt. Anschließend wurden 27 mg (0.092 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden die hydrolysierten offenen Sulfanylbernsteinsäure-amide als Regioisomerengemisch erhalten.

Weitere Aufreinigung und Charakterisierung der erfindungsgemäßen Konjugate

Nach erfolgter Umsetzung wurde in einigen Fällen das Reaktionsgemisch beispielsweise durch Ultrafiltration aufkonzentriert und anschließend mittels Chromatographie, beispielsweise mittels einer Sephadex® G-25, entsalzt und gereinigt. Die Elution erfolgte beispielsweise mit Phosphat-gepufferter Salzlösung (PBS). Anschließend wurde die Lösung sterilfiltriert und eingefroren. Alternativ kann das Konjugat lyophylisiert werden.

**B-7. Bestimmung des Antikörpers, der Toxophorbeladung und des Anteils geöffneter Cvstein-Addukte**

Zur Protein-Identifizierung wurde neben der Molekulargewichtsbestimmung nach Deglykosylierung und/oder Denaturierung ein tryptischer Verdau durchgeführt, der nach Denaturierung, Reduktion und Derivatisierung die Identität des Proteins anhand der nachgewiesenen tryptischen Peptide bestätigt.

Von den erhaltenen Lösungen der in den Ausführungsbeispielen beschriebenen Konjugate in PBS Puffer wurde die Toxophorbeladung wie folgt bestimmt:

Die Bestimmung der Toxophor Beladung von Lysin-verknüpften ADCs erfolgte nach massenspektrometrischer Bestimmung der Molekulargewichte der einzelnen Konjugatspezies. Hierbei wurden vorab die Antikörperkonjugate mittels PNGaseF deglycosyliert, die Probe angesäuert und nach HPLC-Trennung/Entsalzung massenspektrometrisch mittels ESI-MicroTof$_Q$ (Bruker Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution berechnet. Nach Signal Integration der verschiedenen Spezies wurde dann die DAR (= Drug/Antibody Ratio) berechnet. Hierzu wurde die Summe der Toxophor-Anzahl gewichteten Integrationsergebnisse aller Spezies geteilt durch die Summe der einfach gewichteten Integrationsergebnisse aller Spezies.

Die Bestimmung der Toxophorbeladung von Cystein-verknüpften Konjugaten wurde über Reversed-Phase-Chromatographie des reduzierten und denaturierten ADCs bestimmt. Zur ADC-Lösung (1mg/mL, 50μL) wurde Guanidinium-Hydrochlorid (GuHCl) (28.6 mg) und eine Lösung von DL-Dithiothreitol (DTT) (500mM, 3 μL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymeric Reversed Phase Säule (Katalognummer PL1912-3802) (2.1 ×150 mm, 8 μm particle size, 1000 Å) bei einer Flussrate von 1 mL/min mit folgendem Gradienten verwendet: 0 min, 25 %B; 3 min, 25 %B; 28 min, 50 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden der leichten Kette mit einem Toxophor (L1) und den schweren Ketten mit einem, zwei und drei Toxophoren (H1, H2, H3) zugeordnet.

Die durchschnittliche Beladung des Antikörpers mit Toxophoren wurde aus den durch Integration bestimmten Peakflächen als die zweifache Summe der HC-Beladung und der LC-Beladung bestimmt, wobei sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks berechnete, und wobei sich die HC-Beladung aus der Summe der Toxophor-Anzahl gewichteten Integrationsergebnisse aller HC-Peaks geteilt durch die Summe der einfach gewichteten Integrationsergebnisse aller HC- Peaks berechnete. In vereinzelten Fällen konnte es vorkommen, dass die Toxophorbeladung aufgrund von Ko-Elutionen einiger Peaks nicht exakt möglich war.

In den Fällen, in denen keine ausreichende HPLC-Trennung von leichter und schwerer Kette möglich war, erfolgte die Bestimmung der Toxophorbeladung von Cystein verknüpften Konjugaten mittels massenspektrometrischer Bestimmung der Molekulargewichte der einzelnen Konjugatspezies an leichter und schwerer Kette.

Dazu wurde zur ADC-Lösung (1mg/mL, 50μL) Guanidinium-Hydrochlorid (GuHCl) (28.6 mg) und eine Lösung von DL-Dithiothreitol (DTT) (500mM, 3 μL) gegeben. Die Mischung wurde für eine Stunde bei 55 °C inkubiert und massenspektrometrisch nach online Entsaltzung mittels ESI-MicroTof$_Q$ (Bruker Daltonik) analysiert.

Zur DAR-Bestimmung wurden alle Spektren über das Signal im TIC (Total Ion Chromatogramm) addiert und das Molekulargewicht der verschiedenen Konjugatspezies an leichter und schwerer Kette auf Basis von MaxEnt Deconvolution berechnet. Die durchschnittliche Beladung des Antikörpers mit Toxophoren wurde aus den durch Integration bestimmten Peakflächen als die zweifache Summe der HC-Beladung und der LC-Beladung bestimmt. Hierbei berechnete

sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks und die HC-Beladung aus der Summe der Toxophor-Anzahl gewichteten Integrationsergebnisse aller HC-Peaks geteilt durch die Summe der einfach gewichteten Integrationsergebnisse aller HC- Peaks.

Bei den geöffneten Konstukten wurde zur Bestimmung des Anteils des geöffneten Cystein-Addukts das Molekulargewichtsflächenverhältnis vom geschlossenen und offenen Cystein-Addukt (Molekulargewichtsdelta 18Dalton) aller einfach konjugierten leichten und schweren Kettenvarianten bestimmt. Der Mittelwert über alle Varianten ergab den Anteil des geöffneten Cystein-Addukts.

Die Bestimmung der Toxophorbeladung von Glutamin-verknüpften Konjugaten wurde über Reversed-Phase-Chromatographie des reduzierten und denaturierten ADCs bestimmt. Zur ADC-Lösung (1mg/mL, 50μL) wurde Guanidinium-Hydrochlorid (GuHCl) (28.6 mg) und eine Lösung von DL-Dithiothreitol (DTT) (500mM, 3 μL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymeric Reversed Phase Säule (Katalognummer PL1912-3802) (2.1 ×150 mm, 8 μm particle size, 1000 Å) bei einer Flussrate von 1 mL/min mit folgendem Gradienten verwendet: 0 min, 31 %B; 1 min, 31 %B; 14 min, 38 %B, 16 min, 95 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden den schweren Ketten mit einem und zwei Toxophoren (H1, H2) zugeordnet.

Die durchschnittliche Beladung des Antikörpers mit Toxophoren wurde aus den durch Integration bestimmten Peakflächen als die zweifache Summe der HC-Beladung und der LC-Beladung bestimmt, wobei sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks berechnete, und wobei sich die HC-Beladung aus der Summe der Toxophor-Anzahl gewichteten Integrationsergebnisse aller HC-Peaks geteilt durch die Summe der einfach gewichteten Integrationsergebnisse aller HC- Peaks berechnete.

Alternativ erfolgte die Bestimmung der Toxophor Beladung von Glutamin-verknüpften ADCs nach massenspektrometrischer Bestimmung der Molekulargewichte der einzelnen Konjugatspezies. Hierbei wurde die Probe angesäuert und nach HPLC-Trennung/Entsalzung massenspektrometrisch mittels ESI-MicroTof$_Q$ (Bruker Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution berechnet. Nach Signal Integration der verschiedenen Spezies wurde dann die DAR (= Drug/Antibody Ratio) berechnet. Hierzu wurde die Summe der Toxophor-Anzahl gewichteten Integrationsergebnisse aller Spezies geteilt durch die Summe der einfach gewichteten Integrationsergebnisse aller Spezies.

## B-8. Überprüfung der Antigen-Bindung des ADCs

Die Bindefähigkeit des Binders an das Zielmolekül wurde nach erfolgter Kopplung überprüft. Dazu sind dem Fachmann vielfältige Methoden bekannt, beispielsweise kann die Affinität des Konjugats mittels ELISA-Technologie oder Oberflächenplasmonresonanzanalyse (BIAcore™ Messungen) überprüft werden. Die Konjugatkonzentration kann der Fachmann mit gängigen Methoden messen, beispielsweise für Antikörper-Konjugate mittels Proteinbestimmung. (siehe auch Doronina et al.; Nature Biotechnol. 2003; 21:778-784 und Polson et al., Blood 2007; 1102:616-623).

## Ausführungsbeispiele Metabolite

### Beispiel M1

S-[1-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-2,5-dioxopyrrolidin-3-yl]-L-cystein-trifluoressigsäure (1:1)

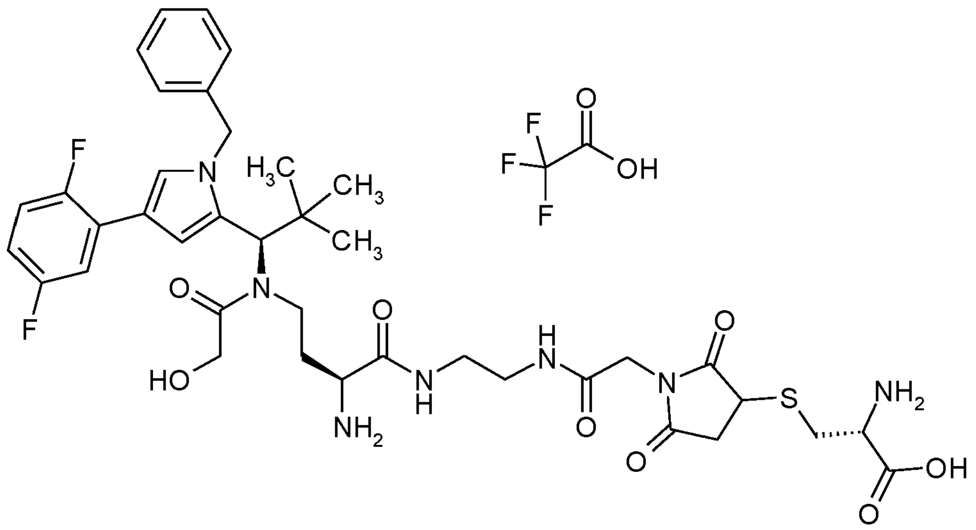

1.8 mg (2 μmol) von Intermediat F104 wurden in 1 ml DMF aufgenommen und mit 2.7 mg (22 μmol) L-Cystein versetzt. Das Reaktionsgemisch wurde 20 h bei RT gerührt, anschließend im Vakuum eingeengt und dann mittels präparativer HPLC gereinigt. Es verblieben 0.6 mg (26% d. Th.) der Titelverbindung als farbloser Schaum.
LC-MS (Methode 1): $R_t$ = 0.80 min; MS (Elpos): m/z = 814 $[M+H]^+$.

<u>**Beispiel M2**</u>

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure -trifluoressigsäure (1:1)
und
4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure -trifluoressigsäure (1:1)

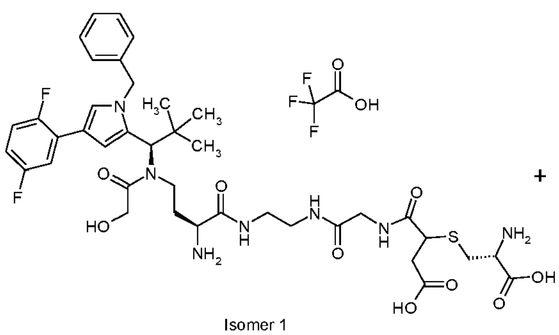
Isomer 1

+

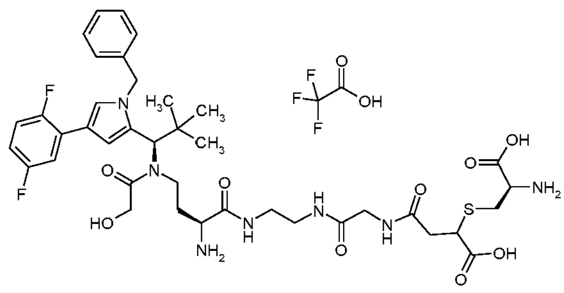
Isomer 2

LC-MS (Methode 1): $R_t$ = 0.80 min; MS (Elpos): m/z = 814 $[M+H]^+$.
Zunächst wurde L-Cystein mit 1-({[2-(Trimethylsilyl)ethoxy]carbonyl}oxy)pyrrolidine-2,5-dion in DMF in Gegenwart von N,N-Diisopropylethylamin in N-{[2-(Trimethylsilyl)ethoxy]carbonyl}-L-cystein überführt.
406 mg (1.53 mmol) N-{[2-(Trimethylsilyl)ethoxy]carbonyl}-L-cystein wurden in 10 ml DMF gelöst und mit 157.5 mg (1.606 mmol) Maleinsäureanhydrid versetzt und der Ansatz 1 Stunde bei RT gerührt. 130 μL dieser Lösung wurden mit 7.5 mg (0.01 mmol) von Intermediat C66 versetzt und der Ansatz 5 min bei RT gerührt. Anschließend wurde im Vakuum eingeengt und der Rückstand durch präparative HPLC gereinigt. Das Lösemeittel wurde im Vakuum abgedampft und der Rückstand im Hochvakuum getrocknet. Es wurden 10 mg (89%) des geschützten Intermediats erhalten, wobei weder im HPLC noch im LC-MS die Regioisomere aufgetrennt werden konnten.
LC-MS (Methode 1): $R_t$ = 1.38 min; MS (Elpos): m/z = 1120 $[M+H]^+$.
Im letzten Schritt wurden die 10 mg von diesem Intermediat in 2 mL 2,2,2-Trifluorethanol gelöst. Es wurden 12 mg (0.088 mmol) Zinkchlorid zugegeben und der Ansatz 30 min bei 50°C gerührt. Anschließend wurden 26 mg (0.088 mmol) Ethylendiamin-N,N,N',N'-tetraessigsäure zugesetzt und das Lösemittel im Vakuum verdampft. Der Rückstand

wurde mittels präparativer HPLC gereinigt. Nach Einengen der entsprechenden Fraktionen und Lyophilisation des Rückstands aus Acetonitril/Wasser wurden 8.3 mg (99% d. Th.) der Titelverbindung als Regioisomerengemisch imVerhältnis 87:13 erhalten.

LC-MS (Methode 5): $R_t$ = 2.3 min und 2.43 min; MS (ESIpos): m/z = 832 $(M+H)^+$.

$^1$H-NMR Hauptregioisomer: (500 MHz, DMSO-$d_6$): $\delta$ = 8.7 (m, 1H), 8.5 (m, 2H), 8.1 (m, 1H), 7.6 (m, 1H), 7.5 (s, 1H) 7.4-7.15 (m, 6H), 6.9-7.0 (m, 1H), 6.85 (s, 1H), 5.61 (s, 1H), 4.9 und 5.2 (2d, 2H), 4.26 und 4.06 (2d, 2H), 3.5-3.8 (m, 5H), 3.0-3.4 (m, 5H), 2.75-3.0 (m, 3H), 2.58 und 2.57 (dd, 1H), 0.77 und 1,5 (2m, 2H), 0.81 (s, 9H).

Alternativ wurden die regioisomeren Titelverbindungen wie folgt hergestellt:
Zunächst wurde dazu L-Cystein mit 1-({[2-(Trimethylsilyl)ethoxy]carbonyl}oxy)pyrrolidin-2,5-dion in DMF in Gegenwart von N,N-Diisopropylethylamin in N-{[2-(Trimethylsilyl)ethoxy]carbonyl}-L-cystein überführt.

55 mg (0.068 mmol) von Intermediat F104 und 36 mg (0.136 mmol) N-{[2-(Trimethylsilyl) ethoxy]carbonyl}-L-cystein 15 ml DMF gelöst und der Ansatz 20 h bei RT gerührt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 15 mL THF/Wasser 1:1 gelöst. Es wurden 131 $\mu$L einer 2M wässrigen Lithiumhydroxidlösung zugegeben und der Ansatz 1 h bei RT gerührt. Anschließend wurde der Ansatz mit einer 1M Salzsäure neutral gestellt, das Lösemittel im Vakuum abgedampft und der Rückstand durch präparative HPLC gereinigt. Es wurden 37 mg (50% d. Th.) der regioisomeren geschützten Intermediate als farbloser Schaum erhalten.

LC-MS (Methode 5): $R_t$ = 3.33 min und 3.36 min; MS (ESIpos): m/z = 976 $(M+H)^+$.

Im letzten Schritt wurden 25 mg (0.023 mmol) von diesem Intermediat in 3 mL 2,2,2-Trifluorethanol gelöst. Es wurden 12.5 mg (0.092 mmol) Zinkchlorid zugegeben und der Ansatz 4 h bei 50°C gerührt. Anschließend wurden 27 mg (0.092 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 18.5 mg (85% d. Th.) der Titelverbindung als Regioisomerengemisch imVerhältnis 21:79 erhalten.

LC-MS (Methode 5): $R_t$ = 2.37 min und 3.44 min; MS (ESIpos): m/z = 832 $(M+H)^+$.

Die gezielte Herstellung der einzelnen Regioisomere der Titelverbindungen erfolgte folgendermaßen:

## Beispiel M3

4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)-amino]-3-{[(2R)-2-amino-2-carboxyethyl]sulfanyl}-4-oxobutansäure-trifuoressigsäure (1:1)
und
4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)-amino]-2-{[(2R)-2-amino-2-carboxyethyl]sulfanyl}-4-oxobutansäure-trifluoressigsäure (1:1)

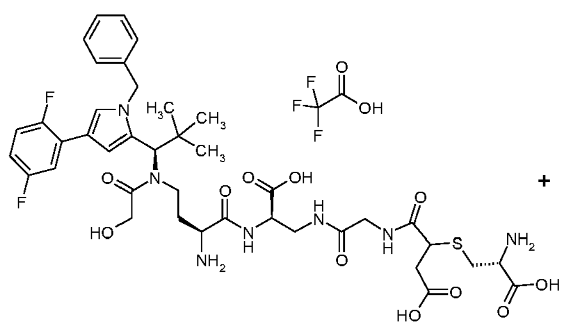
+
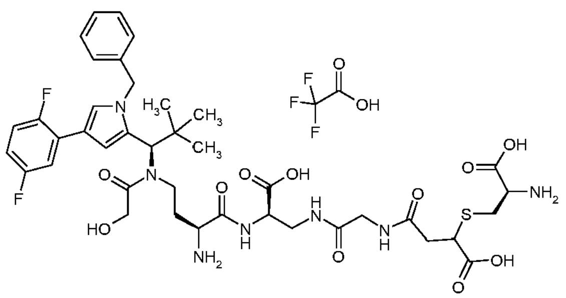

Zunächst wurde L-Cystein mit 1-({[2-(Trimethylsilyl)ethoxy]carbonyl}oxy)pyrrolidin-2,5-dion in DMF in Gegenwart von *N,N*-Diisopropylethylamin in N-{[2-(Trimethylsilyl)ethoxy]carbonyl}-L-cystein überführt. 11 mg (0.013 mmol) von Intermediat F193 und 8 mg (0.016 mmol) N-{[2-(Trimethylsilyl) ethoxy]carbonyl}-L-cystein wurden 3 ml DMF gelöst und der Ansatz 20 h bei RT gerührt. Anschließend wurde eingeengt und der Rückstand durch präparative HPLC gereinigt.

Nach Vereinigung der entsprechenden Fraktionen und Abdampfen der Lösemittel im Vakuum wurde der Rückstand in 2 mL THF/Wasser 1:1 gelöst. Es wurden 19 $\mu$L einer 2M wässrigen Lithiumhydroxidlösung zugegeben und der Ansatz 1 h bei RT gerührt. Dann wurden nochmals 19 $\mu$L der 2M wässrigen Lithiumhydroxidlösung zugegeben und der Ansatz über Nacht bei RT gerührt. Anschließend wurde mit einer 1M Salzsäure neutral gestellt, das Lösemittel im Vakuum abgedampft und der Rückstand durch präparative HPLC gereinigt. Es wurden 4.1 mg (38% d. Th.) der regioisomeren geschützten Intermediate als farbloser Schaum erhalten.

LC-MS (Methode 1): $R_t$ = 1.03 min (breit); MS (ESIpos): m/z = 1020 $(M+H)^+$.

Im letzten Schritt wurden 4.1 mg (0.004 mmol) von diesem Intermediat in 3 mL 2,2,2-Trifluorethanol gelöst. Es wurden 3 mg (0.022 mmol) Zinkchlorid zugegeben und der Ansatz 1 h bei 50°C gerührt. Anschließend wurden 6 mg (0.022 mmol) Ethylendiamin-N,N,N',N'-tetraessigsäure und 2 ml einer 0.1%-igen wässrigen Trifluoressigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 5 mg (quant.) der Titelverbindung als Regioisomerengemisch imVerhältnis 20:80 erhalten.

LC-MS (Methode 1): $R_t$ = 0.78 min (breit); MS (ESIpos): m/z = 876 $(M+H)^+$.

LC-MS (Methode 5): $R_t$ = 2.36 min und 2.39 min; MS (ESIpos): m/z = 876 $(M+H)^+$.

**Beispiel M4**

S-(1-{2-[2-({(2S)-2-Amino-4-[{(1R)-1-[1-benzyl-4-(2,5-difluorpheny1)-1H-pyrrol-2-yl]-2,2-dimethylpropyl}(glycoloyl)amino]butanoyl}amino)ethoxy]ethyl}-2,5-dioxopyrrolidin-3-yl)-L-cystein -trifluoressigsäure (1:1)

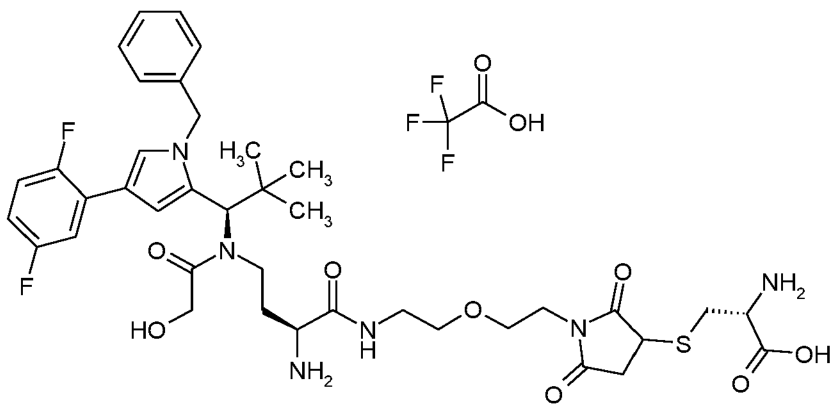

3 mg (4 μmol) von Intermediat F248 wurden in 2 ml DMF aufgenommen und mit 0.9 mg (8 μmol) L-Cystein versetzt. Das Reaktionsgemisch wurde 18 h bei RT gerührt und dann im Vakuum eingeengt. Der Rückstand wurde mittels präparativer HPLC gereinigt. Die entsprechenden Fraktionen wurden eingeengt und nach Lyophilisation des Rückstands aus Acetonitril/Wasser verblieben 1.1 mg (32% d. Th.) der Titelverbindung als weißer Feststoff.

LC-MS (Methode 1): $R_t$ = 0.78 min; MS (Elpos): m/z = 801 $[M+H]^+$.

**Beispiel M5**

(3R,7S)-7-Amino-17-{[(2R)-2-amino-2-carboxyethyl]sulfanyl}-3-[1-benzyl-4-(2,5-difluorphenyl)-1H-pyrrol-2-yl]-4-glycoloyl-2,2-dimethyl-8,16-dioxo-12-oxa-4,9,15-triazanonadecan-19-säure -trifluoressigsäure (1:1)
und
(3R,7S)-7-Amino-18-{[(2R)-2-amino-2-carboxyethyl]sulfanyl}-3-[1-benzyl-4-(2,5-difluorphenyl)-1H-pyrrol-2-yl]-4-glycoloyl-2,2-dimethyl-8,16-dioxo-12-oxa-4,9,15-triazanonadecan-19-säure -trifluoressigsäure (1:1)

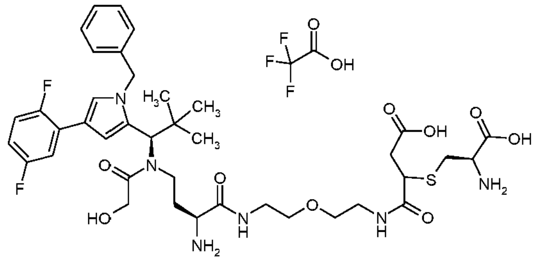

+

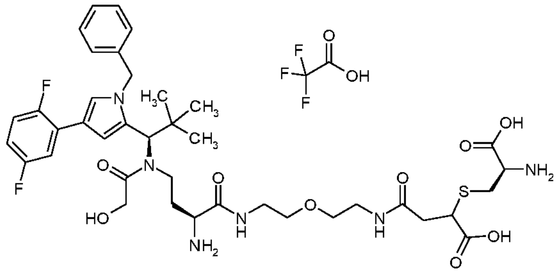

8 mg (0.010 mmol) von der geschützten Zwischenstufe von Intermediat F248 und 5.1 mg (0.02 mmol) N-{[2-(Trimethylsilyl) ethoxy]carbonyl}-L-cystein 3 ml DMF gelöst und der Ansatz 18 h bei RT gerührt und anschließend 2 h im

Ultraschallbad behandelt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 2 mL THF/Wasser 1:1 gelöst. Es wurden 15 µL einer 2M wässrigen Lithiumhydroxidlösung zugegeben und der Ansatz 15 min bei RT gerührt. Anschließend wurde der Ansatz mit einer 1M Salzsäure auf einen pH-Wert von ~3 eingestellt, mit 20 ml Kochsalzlösung verdünnt und zweimal mit 20 ml Ethylacetat extrahiert. Die organische Phase wurde über Magnesiumsulfat getrocknet, eingeengt und der Rückstand aus Acetonitril/Wasser lyophilisiert. Es wurden 8.4 mg (78% d. Th. über 2 Stufen) der regioisomeren geschützten Intermediate als farbloser Schaum erhalten.

LC-MS (Methode 1): $R_t$ = 1.44 min und 3.43 min; MS (ESIpos): m/z = 1107 $(M+H)^+$.

Im letzten Schritt wurden 8 mg (0.007 mmol) von diesem Intermediat in 5 mL 2,2,2-Trifluor-ethanol gelöst. Es wurden 9.8 mg (0.072 mmol) Zinkchlorid zugegeben und der Ansatz 1.5 h bei 50°C gerührt. Anschließend wurde Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 4 mg (59% d. Th.) der Titelverbindung als Regioisomerengemisch imVerhältnis 31:67 erhalten.

LC-MS (Methode 1): $R_t$ = 0.79 min und 0.81 min; MS (ESIpos): m/z = 819 $(M+H)^+$.

**Beispiel M6**

2-{[(2R)-2-Amino-2-carboxyethyl]sulfanyl}-4-({(14R)-13-(3-aminopropyl)-14-[1-benzyl-4-(2,5-difluorphenyl)-1H-pyrrol-2-yl]-15,15-dimethyl-2,7,12-trioxo-10-thia-3,6,13-triazahexadec-1-yl}amino)-4-oxobutansäure -trifluoressigsäure (1:2) und 3-{[(2R)-2-Amino-2-carboxyethyl]sulfanyl}-4-({(14R)-13-(3-aminopropyl)-14-[1-benzyl-4-(2,5-difluorphenyl)-1H-pyrrol-2-yl]-15,15-dimethyl-2,7,12-trioxo-10-thia-3,6,13-triazahexadec-1-yl}amino)-4-oxobutansäure -trifluoressigsäure (1:2)

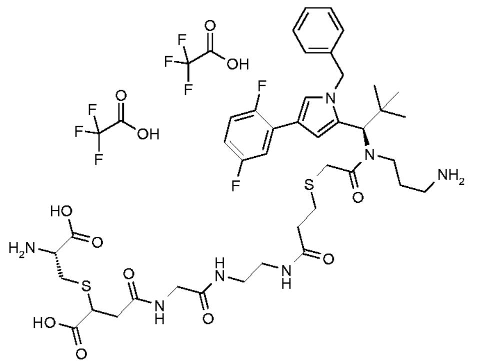

+

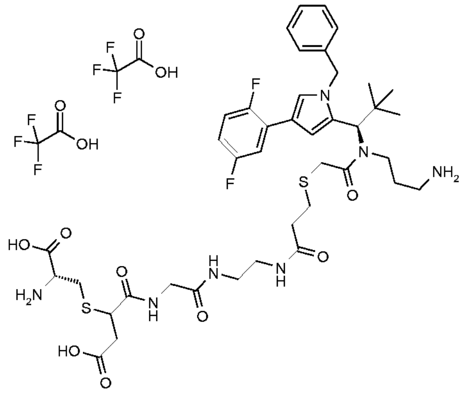

18 mg (0.021 mmol) von Intermediat F213 und 11.2 mg (0.04 mmol) N-{[2-(Trimethylsilyl) ethoxy]carbonyl}-L-cystein 2 ml DMF gelöst und der Ansatz 18 h bei RT gerührt. Die Reaktionsmischung wurde im Vakuum verdampft. Der Rückstand (21.2 mg) wurde in 3 mL THF/Wasser 1:1 gelöst. Es wurden 0.04 mL einer 2M wässrigen Lithiumhydroxidlösung zugegeben und der Ansatz 3 Stunden bei RT gerührt. Es wurden 0.02 mL einer 2M wässrigen Lithiumhydroxidlösung zugegeben und der Ansatz 1 Stunde bei RT gerührt. Anschließend wurde der Ansatz mit 7.2 mg (0.12 mmol) Essigsäure auf einen pH-Wert von ~7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 13 mg (57% über 2 Stufen) der regioisomeren geschützten Intermediate erhalten.

LC-MS (Methode 1): $R_t$ = 1.03 min; MS (ESIpos): m/z = 1020 $(M+H)^+$.

Im letzten Schritt wurden 13 mg (0.01 mmol) von diesem Intermediat in 2 mL 2,2,2-Trifluorethanol gelöst. Es wurden 6.2 mg (0.05 mmol) Zinkchlorid zugegeben und der Ansatz 7 h bei 50°C gerührt. Anschließend wurde 13.3 mg (0.05 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 10.3 mg (81.4%) der Titelverbindung als Regioisomerengemisch erhalten.

LC-MS (Methode 1): $R_t$ = 1.03 min; MS (ESIpos): m/z = 875 $(M+H)^+$.

**Beispiel** M7

S-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-L-cystein - trifluoressigsäure (1:1)

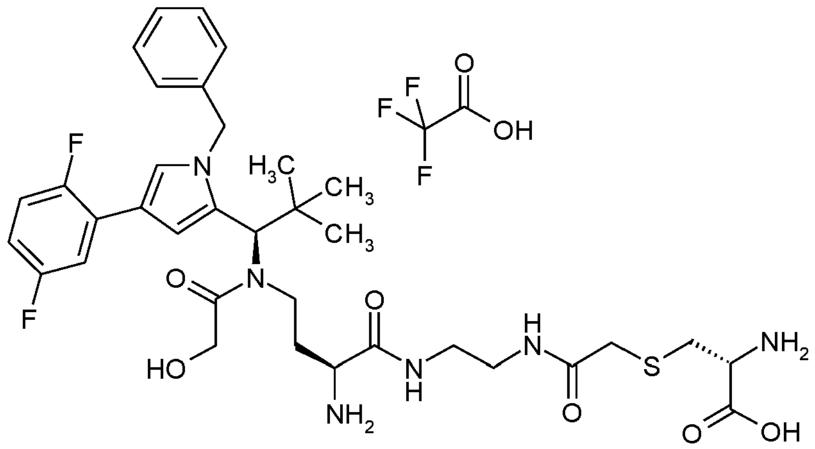

6 mg (8 μmol) von Intermediat F119 wurden in 3 mL DMF aufgenommen und mit 1.8 mg (15 μmol) L-Cystein versetzt. Das Reaktionsgemisch wurde 6 h bei RT gerührt und dann 3 Tage bei RT stehen gelassen. Anschließend wurde der Ansatz im Vakuum eingeengt und das Produkt mittels präparativer HPLC gereinigt.
LC-MS (Methode 1): $R_t$ = 0.81 min; MS (ESIpos): m/z = 717 $(M+H)^+$.

**Beispiel M8**

(3R)-6-{(11S,15R)-11-Amino-15-[1-benzyl-4-(2,5-difluorphenyl)-1H-pyrrol-2-yl]-14-glycoloyl-16,16-dimethyl-2,5,10-trioxo-3,6,9,14-tetraazaheptadec-1-yl}-5-oxothiomorpholin-3-carbonsäure -trifluoressigsäure (1:1)

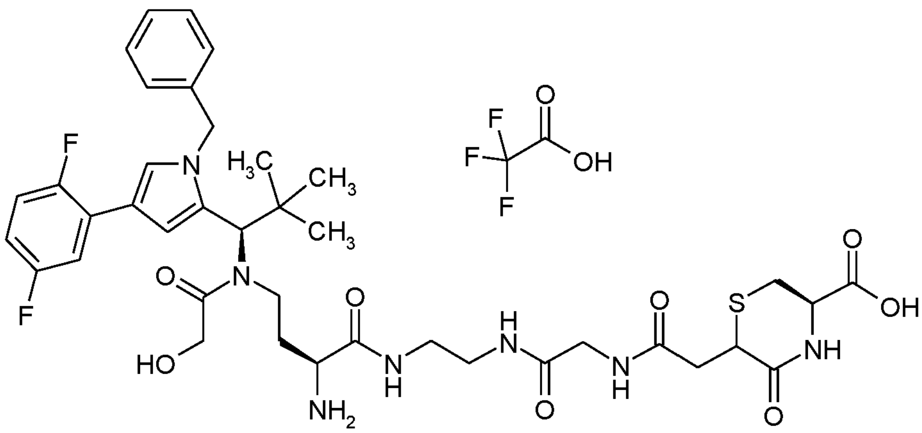

4 mg (0.004 mmol) der Verbindung aus Beispiel 135 wurden in 4 mL THF/Wasser gelöst und mit 48 μL einer 2molaren wässrigen Lithiumhydroxidlösung versetzt. Der Ansatz wurde 1 h bei RT gerührt und anschließend eingeengt und mittels präparativer HPLC gereinigt. Nach Vereinigung, Einengen und Lyophilisation aus Acetonitril/Wasser der entsprechenden Fraktionen wurden 2.4 mg (60% d. Th.) der Titelverbindung erhalten.
LC-MS (Methode 1): $R_t$ = 0.86 min; MS (Elpos): m/z = 814 $[M+H]^+$.

**Beispiel M9**

N-(3-Aminopropyl)-N-{(1R)-1-[1-benzyl-4-(2,5-difluorphenyl)-1H-pyrrol-2-yl]-2,2-dimethylpropyl}-2-hydroxyacetamid

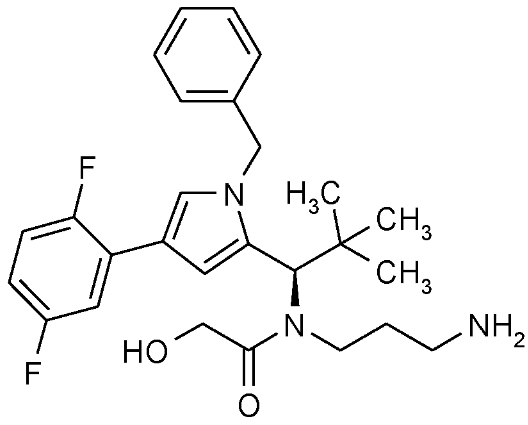

150.0 mg (0.42 mmol) (1R)-1-[1-Benzyl-4-(2,5-difluorphenyl)-1H-pyrrol-2-yl]-2,2-dimethylpropan-1-amin (Intermediat C52) wurden in 2.0 mL Dichlormethan vorgelegt und mit 29.2 mg (0.49 mmol) HOAc und 125.6 mg (0.59 mmol) Natriumtriacetoxyborhydrid versetzt und 5 min bei RT gerührt. Es wurden 98.9 mg (0.49 mmol) 3-(1,3-Dioxo-1,3-dihydro-2H-isoindol-2-yl)propanal zugegeben. Die Reaktionsmischung wurde über Nacht bei RT gerührt. Die Reaktionsmischung wurde mit Ethylacetat verdünnt und die organische Phase zweimal mit ges. Natriumcarbonat-Lösung und einmal mit ges. NaCl-Lösung gewaschen. Nach der Trocknung über Magnesiumsulfat wurde das Lösemittel im Vakuum verdampft und der Rückstand mittels Kieselgel (Laufmittel: Dichlormethan/Methanol 100:1) gereinigt. Die Lösemittel wurden im Vakuum verdampft und der Rückstand im Hochvakuum getrocknet. Man erhielt 188.6 mg (74 %) der Verbindung 2-[3-({(1R)-1-[1-Benzyl-4-(2,5-difluorphenyl)-1H-pyrrol-2-yl]-2,2-dimethylpropyl}amino)propyl]-1H-isoindol-1,3(2H)-dion.

LC-MS (Methode 1): $R_t$ = 1.00 min; MS (ESIpos): m/z = 541 $[M+H]^+$.

171.2 mg (0.32 mmol) 2-[3-({(1R)-1-[1-Benzyl-4-(2,5-difluorphenyl)-1H-pyrrol-2-yl]-2,2-dimethylpropyl}amino)propyl]-1H-isoindol-1,3(2H)-dion wurden in 5.0 mL Dichlormethan vorgelegt und mit 73.6 mg (0.73 mmol) Triethylamin versetzt. Bei 0 °C wurden 94.9 mg (0.70 mmol) Acetoxyessigsäurechlorid zugegeben und die Reaktionsmischung wurde über Nacht bei RT gerührt Die Reaktionsmischung wurde mit Ethylacetat verdünnt und die organische Phase zweimal mit ges. Natriumhydrogencarbonat-Lösung und einmal mit ges. NaCl-Lösung gewaschen. Nach der Trocknung über Magnesiumsulfat wurde das Lösemittel im Vakuum verdampft und der Rückstand wurde mittels Biotage Isolera gereinigt (Kieselgel, Säule 10 g SNAP, Fluss 12 mL/min, Ethylacetat/Cyclohexan 1:3) gereinigt. Die Lösemittel wurden im Vakuum verdampft und der Rückstand im Hochvakuum getrocknet. Man erhielt 159.0 mg (77 %) der Verbindung 2-({(1R)-1-[1-Benzyl-4-(2,5-difluorphenyl)-1H-pyrrol-2-yl]-2,2-dimethylpropyl}[3-(1,3-dioxo-1,3-dihydro-2H-isoindol-2-yl)propyl]amino)-2-oxoethylacetat.

LC-MS (Methode 1): $R_t$ = 1.35 min; MS (ESIpos): m/z = 642 $[M+H]^+$.

147.2 mg (0.23 mmol) 2-({(1R)-1-[1-Benzyl-4-(2,5-difluorphenyl)-1H-pyrrol-2-yl]-2,2-dimethylpropyl}[3-(1,3-dioxo-1,3-dihydro-2H-isoindol-2-yl)propyl]amino)-2-oxoethylacetat wurden in 4.0 mL Ethanol vorgelegt und mit 356.2 mg (4.59 mmol) Methanamin (40 % in Wasser) versetzt. Die Reaktionsmischung wurde über Nacht bei 50 °C gerührt. Das Lösemittel wurde im Vakuum verdampft und der Rückstand dreimal mit Toluol kodestilliert. Der Rückstand wurde mittels Kieselgel (Laufmittel: Dichlormethan/Methanol 10:1) gereinigt. Die Lösemittel wurden im Vakuum verdampft und der Rückstand im Hochvakuum getrocknet. Man erhielt 67.4 mg (63 %) der Titelverbindung.

LC-MS (Methode 1): $R_t$ = 0.91 min; MS (ESIpos): m/z = 470 $[M+H]^+$.

**Beispiel M10**

(2R,28R)-28-Amino-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-25-(carboxymethyl)-4,20,24-trioxo-7,10,13,16-tetraoxa-26-thia-3,19,23-triazanonacosan-1,29-disäure -trifluoressigsäure (1:2) und
(1R,28R,34R)-1-Amino-33-(3-aminopropyl)-34-[1-benzyl-4-(2,5-difluorphenyl)-1H-pyrrol-2-yl]-35,35-dimethyl-6,10,26,32-tetraoxo-14,17,20,23-tetraoxa-3,30-dithia-7,11,27,33-tetraazahexatriacontan-1,4,28-tricarbonsäure -trifluo-

ressigsäure (1:2)

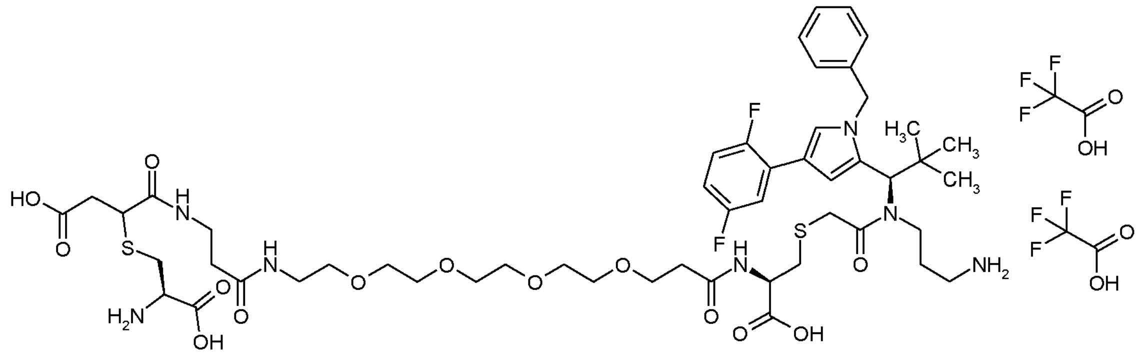

+

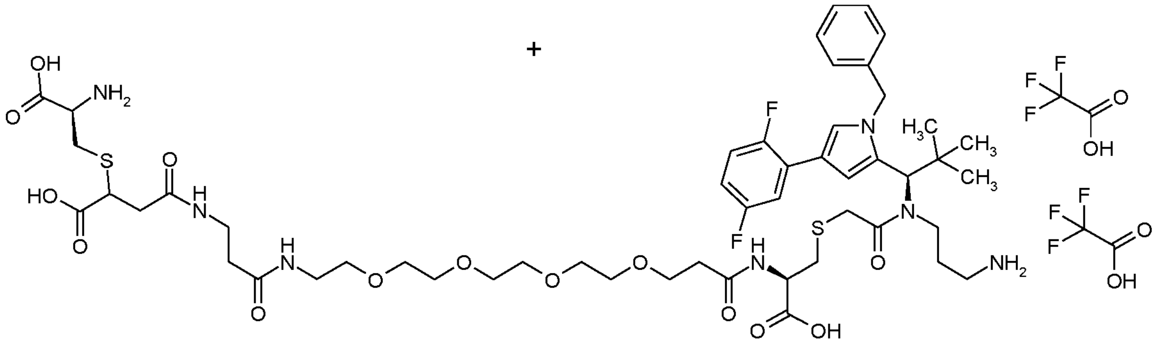

20 mg (0.018 mmol) R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cystein-trifluoressigsäure (1:1) (Intermediat F209) und 9.78 mg (0.036 mmol) N-{[2-(Trimethylsilyl) ethoxy]carbonyl}-L-cystein wurden in 2 ml DMF gelöst und der Ansatz 18 h bei RT gerührt. Die Reaktionsmischung wurde im Vakuum verdampft. Der Rückstand (47.7 mg) wurde in 3 mL THF/Wasser 1:1 gelöst. Es wurden 0.08 mL einer 2M wässrigen Lithiumhydroxidlösung zugegeben und der Ansatz 1 Stunde bei RT gerührt. Anschließend wurde der Ansatz mit 9.26 mg (0.15 mmol) Essigsäure auf einen pH-Wert von ~7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 15.3 mg (29% über 2 Stufen) der regioisomeren geschützten Intermediate erhalten.

LC-MS (Methode 6): $R_t$ = 12.26 min und 12.30min; MS (ESIpos): m/z = 1254 $(M+H)^+$.

Im letzten Schritt wurden 15.3 mg (0.01 mmol) von diesem Intermediat in 2 mL 2,2,2-Trifluorethanol gelöst. Es wurden 6.1 mg (0.05 mmol) Zinkchlorid zugegeben und der Ansatz 2 h bei 50°C gerührt. Anschließend wurde 13.1 mg (0.05 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 11.9 mg (79.5%) der Titelverbindung als Regioisomerengemisch erhalten.

LC-MS (Methode 1): $R_t$ = 0.85 min; MS (ESIpos): m/z = 1110 $(M+H)^+$.

## Beispiel M11

S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:2)

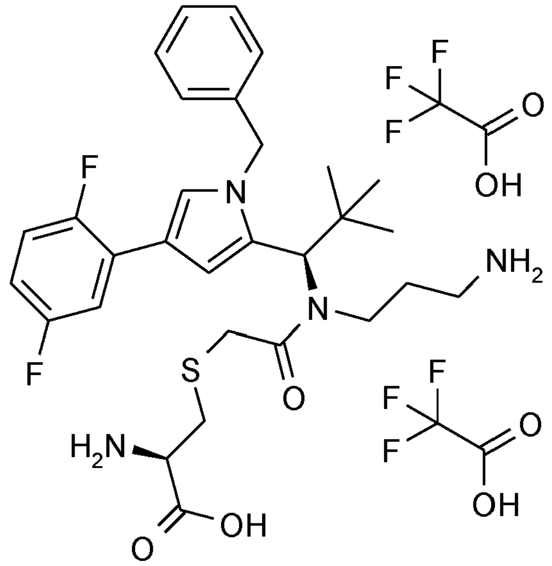

15.0 mg (0.018 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein - trifluoressigsäure (1:1) (Intermediat C71) wurden in 1.0 mL Trifluorethanol gelöst und mit 7.4 mg (0.054 mmol) Zinkdichlorid versetzt. Die Reaktionsmischung rührte über Nacht bei 50 °C. Die Reaktionsmischung wurde mit 15.8 mg (0.054 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 11.1 mg (77 %) der Titelverbindung. LC-MS (Methode 1): $R_t$ = 0.83 min; MS (ESIpos): m/z = 573 $(M+H)^+$.

## Beispiel M12

4-{[(1R)-2-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)-1-carboxyethyl]amino}-4-oxobutansäure - trifluoressigsäure (1:1)

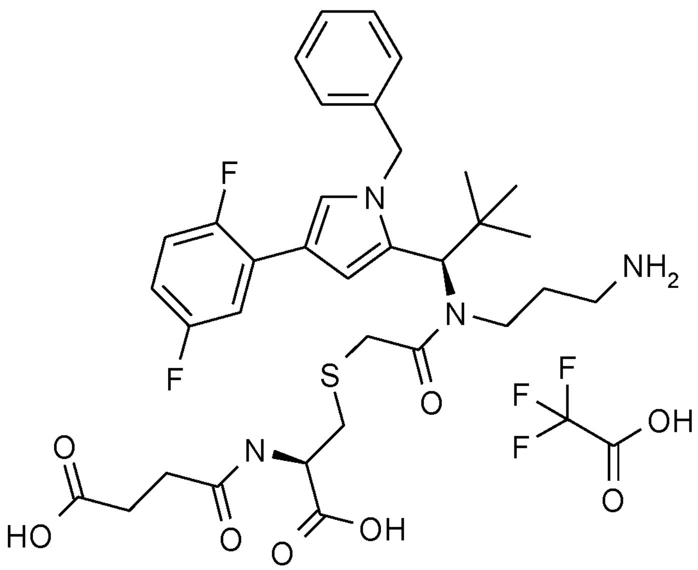

12.2 mg (0.014 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) wurden in 2.0 mL Trifluorethanol gelöst und mit 11.4 mg (0.084 mmol) Zinkdichlorid versetzt. Die Reaktionsmischung

rührte 3 h bei 50 °C. Die Reaktionsmischung wurde mit 24.5 mg (0.084 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10μ, Fluss: 50 mL/min, MeCN/Wasser, 0.1% TFA). Die Lösemittel wurden im Vakuum verdampft und der Rückstand im Hochvakuum getrocknet. Man erhielt 4.6 mg (42 %) der Titelverbindung.

LC-MS (Methode 1): $R_t$ = 0.88 min; MS (ESIpos): m/z = 673 $(M+H)^+$.

**Beispiel M13**

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure -trifluoressigsäure (1:1) Regioisomer 1, Epimer 1 (2R) oder (2S)

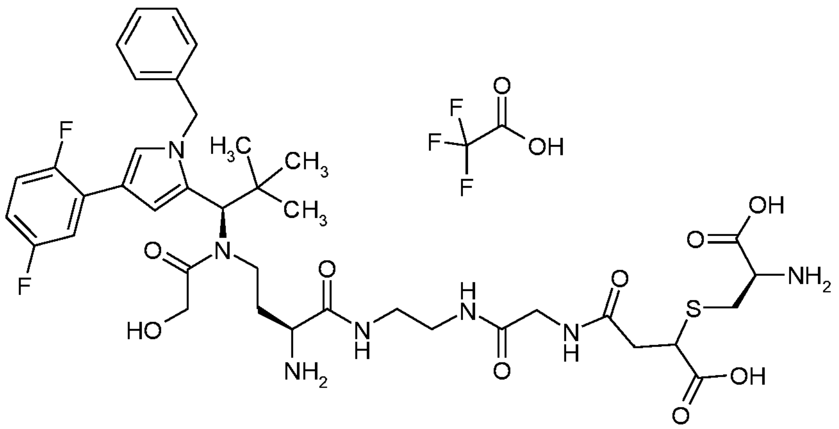

LC-MS (Methode 5): $R_t$ = 2.44 min; MS (ESIpos): m/z = 832 $[M+H]^+$.

Zunächst wurde Methyl-L-cysteinathydrochlorid (1:1) mit 1-({[2-(Trimethylsilyl)ethoxy] carbonyl}oxy)pyrrolidine-2,5-dion in DMF in Gegenwart von *N,N*-Diisopropylethylamin in Methyl-N-{[2-(trimethylsilyl)ethoxy]carbonyl}-L-cysteinat überführt.

408 mg (1.93 mmol) von kommerziell erhältlicher 3-Brom-4-methoxy-4-oxobutansäure und 180 mg (0.644 mmol) Methyl-N-{[2-(trimethylsilyl)ethoxy]carbonyl}-L-cysteinat wurden in 8 ml DMF gelöst und mit 147 mg (0.97 mmol) 1,8-Diazabicyclo(5.4.0)undec-7-en versetzt. Nach 18 h Rühren bei RT wurden nochmals 136 mg (0.64 mmol) 3-Brom-4-methoxy-4-oxobutansäure sowie 147 mg (0.97 mmol) 1,8-Diazabicyclo(5.4.0)undec-7-en hinzugefügt und der Ansatz weitere 12 h bei RT gerührt und anschließend im Vakuum eingeengt. Der Rückstand wurde durch präparative HPLC gereinigt. Nach Vereinigung der entsprechenden Fraktionen und Abdampfen der Lösemittel im Vakuum wurden 151 mg (57% d. Th.) 4-Methoxy-3-{[(2R)-3-methoxy-3-oxo-2-({[2-(trimethylsilyl)ethoxy]carbonyl}amino)propyl] sulfanyl}-4-oxobutansäure erhalten.

LC-MS (Methode 12): $R_t$ = 1.74 min; MS (ESIneg): m/z = 408 $(M-H)^-$.

Von diesem Intermediat wurden 145 mg mittels überkritischer Fluidchromatographie über chirale Säulen in die Einzeldiastereomere aufgetrennt (SFC; Säule: DAICEL, AD-H 5u 250x20 mm; Fluss: 80 mL/min; Methode: AD-25%ETOH-80ml; Druck: 100 bar; Wellenlänge: 210 nM) und dabei wurden von Epimer 1 63 mg (43%) und von Epimer 2 58 mg (40%) erhalten.

Epimer 1 wurde wie folgt charakterisiert:
LC-MS (Methode 5): $R_t$ = 2.94 min; MS (ESIneg): m/z = 408 $(M-H)^-$.

$^1$H-NMR: (400 MHz, DMSO-$d_6$): δ = 7.57 (d, 1H), 4.24 (m, 1H), 4.05 (t, 2H), 3.67 (t, 1H), 3.65 (s, 3H), 3.62 (s, 3H), 3.05 (dd, 1H), 2.70-2.88 (m, 2H), 2.59 (dd, 1H), 0.93 (t, 2H), 0.02 (s, 9H).

Epimer 2 wurde wie folgt charakterisiert:
LC-MS (Methode 5): $R_t$ = 2.95 min; MS (ESIneg): m/z = 408 $(M-H)^-$.

$^1$H-NMR: (400 MHz, DMSO-$d_6$): δ = 7.58 (d, 1H), 4.16-4.23 (m, 1H), 4.05 (t, 2H), 3.67 (dd, 1H), 3.65 (s, 3H), 3.64 (s, 3H), 3.04 (dd, 1H), 2.88 (dd, 1H), 2.77 (dd, 1H), 2.61 (dd, 1H), 0.92 (t, 2H), 0.02 (s, 9H).

32.5 mg (0.079 mmol) von Epimer 1 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 43 mg (57% d. Th.) vom voll geschützten Intermediat Methyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-2-{[(2R)-3-methoxy-3-oxo-2-({[2-(trimethylsilyl)ethoxy]carbonyl}amino)propyl] sulfanyl}-4-oxobutanoat erhalten wurden. Anschließend wurden 40 mg (0.035 mmol) dieses Intermediats mit 0.9 mL einer 2 molaren Lithiumhydroxidlösung in 11 ml Methanol 20 min lang bei RT gerührt, wobei beide Methylestergruppen abgespalten wurden. Nach HPLC-Reinigung wurden 12 mg (31% d. Th.) des Dicarbonsäurederivats erhalten.

LC-MS (Methode 5): $R_t$ = 4.74 min; MS (ESIpos): m/z = 1120 $[M+H]^+$.

10 mg (0.009 mmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 2.6 mg (30% d. Th.) der Titelverbindung erhalten.

LC-MS (Methode 5): $R_t$ = 2.44 min; MS (ESIpos): m/z = 832 $[M+H]^+$.

**Beispiel M14**

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure -trifluoressigsäure (1:1) Regioisomer 1, Epimer 2 (2R oder 2S)

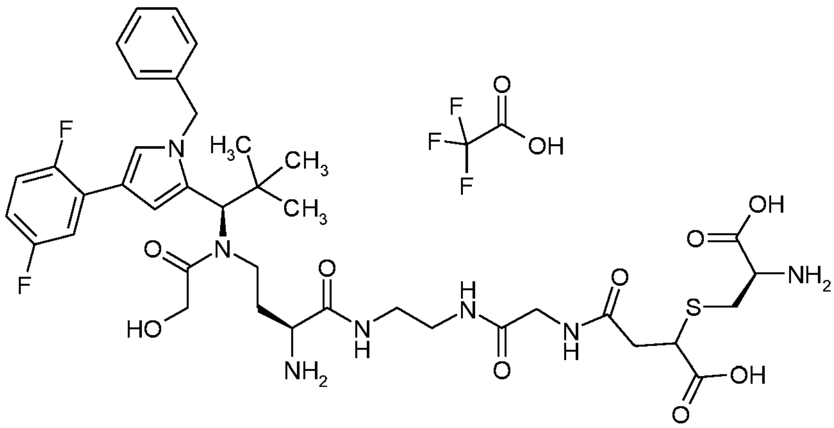

LC-MS (Methode 5): $R_t$ = 2.44 min; MS (Elpos): m/z = 832 $[M+H]^+$.

Das in Beispiel M13 beschriebene Intermediat Epimer 2 wurde in Analogie zu der Beschreibung in Beispiel M13 umgesetzt:

32.5 mg (0.079 mmol) von Epimer 2 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 43 mg (57% d. Th.) vom voll geschützten Intermediat Methyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-2-{[(2R)-3-methoxy-3-oxo-2-({[2-(trimethylsilyl)ethoxy]carbonyl}amino)propyl] sulfanyl}-4-oxobutanoat erhalten wurden. Anschließend wurden 40 mg (0.035 mmol) dieses Intermediats mit 0.9 mL einer 2 molaren Lithiumhydroxidlösung in 11 ml Methanol 20 min lang bei RT gerührt, wobei beide Methylestergruppen abgespalten wurden. Nach HPLC-Reinigung wurden 11 mg (28% d. Th.) des Dicarbonsäurederivats erhalten.

LC-MS (Methode 5): $R_t$ = 4.74 min; MS (ESIpos): m/z = 1120 $[M+H]^+$.

10 mg (0.009 mmol) von diesem Intermediat wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 4.4 mg (52% d. Th.) der Titelverbindung erhalten.

LC-MS (Methode 5): $R_t$ = 2.44 min; MS (ESIpos): m/z = 832 $[M+H]^+$.

**Beispiel M15**

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure -trifluoressigsäure (1:1) Regioisomer 2, Epimer 1 (3R oder 3S)

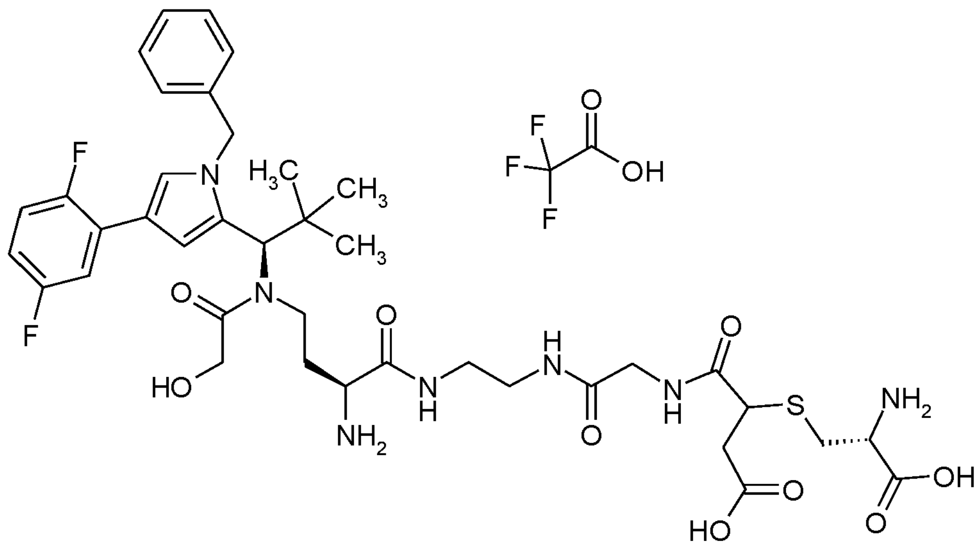

LC-MS (Methode 5): $R_t$ = 2.45 min; MS (Elpos): m/z = 832 $[M+H]^+$.

742.8 mg (3.3 mmol) von kommerziell erhältlicher 2-Brom-4-ethoxy-4-oxobutansäure und 802 mg (2.87 mmol) Methyl-N-{[2-(trimethylsilyl)ethoxy]carbonyl}-L-cysteinat wurden in 32 ml DMF gelöst und mit 655.4 mg (4.31 mmol) 1,8-Diazabicyclo(5.4.0)undec-7-en versetzt. Nach 20 h Rühren bei RT wurde der Ansatz im Vakuum eingeengt und der Rückstand durch präparative HPLC gereinigt. Nach Vereinigung der entsprechenden Fraktionen und Abdampfen der Lösemittel im Vakuum wurden 521 mg (43% d. Th.) 4-Ethoxy-2-{[(2R)-3-methoxy-3-oxo-2-({[2-(trimethylsilyl)ethoxy]carbonyl}amino)propyl]sulfanyl}-4-oxobutansäure erhalten.

LC-MS (Methode 5): $R_t$ = 3.13 min; MS (ESIpos): m/z = 424 $(M+H)^+$.

Von diesem Intermediat wurden 510 mg mittels überkritischer Fluidchromatographie über chirale Säulen in die Einzeldiastereomere aufgetrennt (SFC; Säule: DAICEL, AD-H 5u 250x20 mm; Fluss: 80 mL/min; Methode: AD-10%ETOH-80ml; Druck: 100 bar; Wellenlänge: 210 nM) und dabei wurden von Epimer 1 100 mg (20%) und von Epimer 2 141 mg (28%) erhalten.

Epimer 1 wurde wie folgt charakterisiert:
LC-MS (Methode 1): $R_t$ = 0.99 min; MS (ESIneg): m/z = 422 $(M-H)^-$.

$^1$H-NMR: (400 MHz, DMSO-$d_6$): $\delta$ = 7.60 (d, 1H), 4.18-4.26 (m, 1H), 4.01-4.08 (m, 4H), 3.63 (s, 3H), 3.59 (dd, 1H), 3.04 (dd, 1H), 2.92 (dd, 1H), 2.80 (dd, 1H), 2.63 (dd, 1H), 1.17 (t, 3H), 0.92 (t, 2H), 0.02 (s, 9H).

Epimer 2 wurde wie folgt charakterisiert:
LC-MS (Methode 5): $R_t$ = 2.95 min; MS (ESIneg): m/z = 408 $(M-H)^-$.

$^1$H-NMR: (400 MHz, DMSO-$d_6$): $\delta$ = 7.56 (d, 1H), 4.21-4.29 (m, 1H), 4.01-4.1 (m, 4H), 3.64 (s, 3H), 3.58 (dd, 1H), 3.08 (dd, 1H), 2.85 (dd, 1H), 2.78 (dd, 1H), 2.60 (dd, 1H), 1.17 (t, 3H), 0.93 (t, 2H), 0.02 (s, 9H).

33.6 mg (0.079 mmol) von Epimer 1 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 51 mg (63% d. Th.) vom voll geschützten Intermediat

Ethyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-3-{[(2R)-3-methoxy-3-oxo-2-({[2-(trimethylsilyl)ethoxy] carbonyl}amino)propyl] sulfanyl}-4-oxobutanoat erhalten wurden.

Anschließend wurden 49 mg (0.042 mmol) dieses Intermediats mit 0.5 mL einer 2 molaren Lithiumhydroxidlösung in 12 ml THF/Wasser 1:1 30 min lang bei RT gerührt, wobei beide Methylestergruppen abgespalten wurden. Nach Ansäuern und HPLC-Reinigung wurden 11 mg (24% d. Th.) des Dicarbonsäurederivats erhalten.

LC-MS (Methode 5): $R_t$ = 4.68 min; MS (ESIpos): m/z = 1120 $[M+H]^+$.

11 mg (0.01 mmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 3.7 mg (39% d. Th.) der Titelverbindung erhalten.

LC-MS (Methode 5): $R_t$ = 2.45 min; MS (ESIpos): m/z = 832 $[M+H]^+$.

## Beispiel M16

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure -trifluoressigsäure (1:1) Regioisomer 2, Epimer 2 (3R oder 3S)

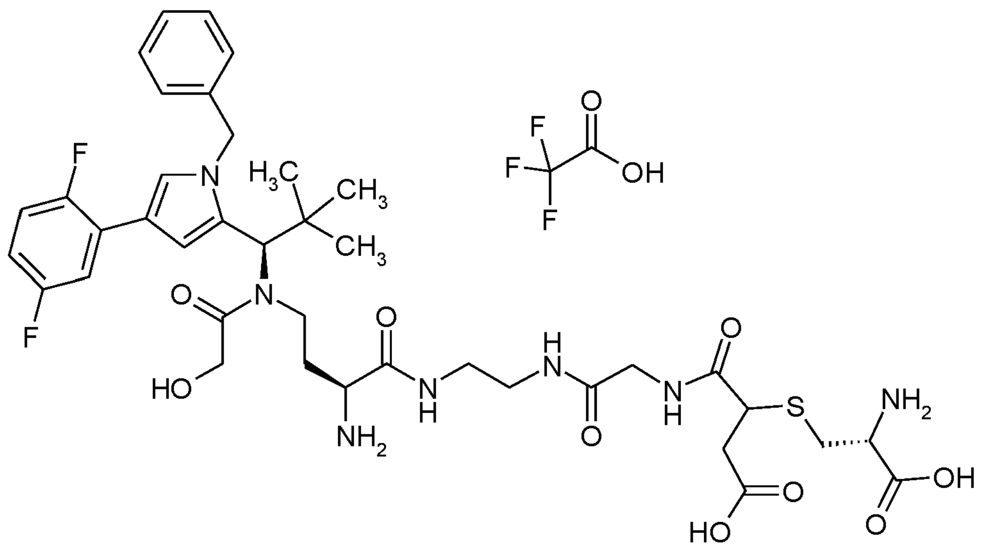

LC-MS (Methode 5): $R_t$ = 2.44 min; MS (Elpos): m/z = 832 $[M+H]^+$.

Das in Beispiel M15 beschriebene Intermediat Epimer 2 wurde in Analogie zu der Beschreibung in Beispiel M15 umgesetzt:

33.6 mg (0.079 mmol) von Epimer 2 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 51 mg (63% d. Th.) vom voll geschützten Intermediat

Ethyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-3-{[(2R)-3-methoxy-3-oxo-2-({[2-(trimethylsilyl)ethoxy] carbonyl}amino)propyl] sulfanyl}-4-oxobutanoat erhalten wurden.

Anschließend wurden 49 mg (0.042 mmol) dieses Intermediats mit 0.5 mL einer 2 molaren Lithiumhydroxidlösung in 12 ml THF/Wasser 1:1 30 min lang bei RT gerührt, wobei beide Methylestergruppen abgespalten wurden. Nach Ansäuern und HPLC-Reinigung wurden 13.4 mg (28% d. Th.) des Dicarbonsäurederivats erhalten.

LC-MS (Methode 5): $R_t$ = 4.66 min; MS (ESIpos): m/z = 1120 $[M+H]^+$.

13.4 mg (0.012 mmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 7.5 mg (66% d. Th.) der Titelverbindung erhalten.

LC-MS (Methode 5): $R_t$ = 2.44 min; MS (ESIpos): m/z = 832 $[M+H]^+$.

## Beispiel M17

(2S)-2-Amino-4-[{(1R)-1-[1-benzyl-4-(2,5-difluorphenyl)-1H-pyrrol-2-yl]-2,2-dimethylpropyl} (glycoloyl)amino]butansäurehydrochlorid (1:1)

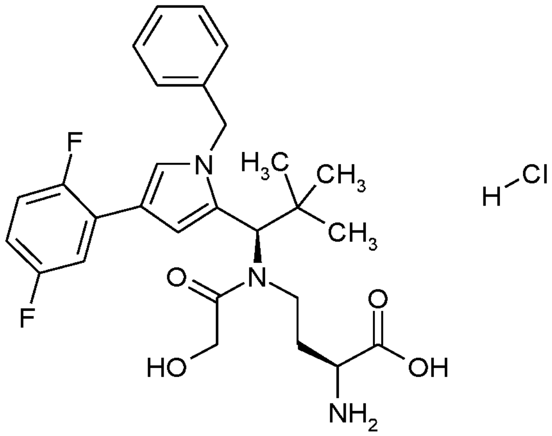

150 mg (0.2 mmol) von Intermediat C53 wurden in 15 mL DMF gelöst und mit 2.29 g (20.39 mmol) DABCO versetzt. Der Ansatz wurde 30 min im Ultraschallbad behandelt. Durch Zugabe von 1.17 ml Essigsäure wurde der Ansatz dann auf pH 3-4 gebracht und im Vakuum eingeengt. Der Rückstand wurde durch präparative HPLC gereinigt und die entsprechenden Fraktionen wurden bei RT im Vakuum eingeengt. Der Rückstand wurde in Acetonitril/Wasser 1:1 aufgenommen, mit 5 mL einer 4N Salzsäure versetzt und anschließend lyophilisiert. So wurden 81 mg (68% d. Th.) der Titelverbindung erhalten.

LC-MS (Methode 5): $R_t$ = 2.69 min; MS (Elpos): m/z = 514 $[M+H]^+$.

## Beispiel M18

N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-L-glutamin -trifluoressigsäure (1:1)

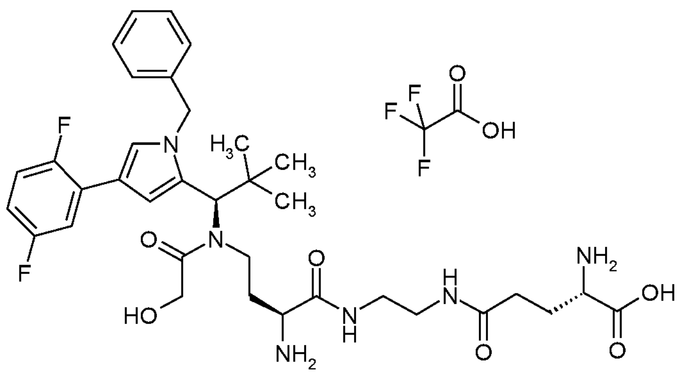

Zunächst wurde nach dem Fachmann allgemein bekannten Methoden der Peptidchemie Trifluoressigsäure-benzyl-N-(2-aminoethyl)-$N^2$-[(benzyloxy)carbonyl]-L-glutaminat (1:1) hergestellt. Diese Intermediat wurde dann in Gegenwart von HATU mit Intermediat C58 gekuppelt. Anschließend wurde zunächst durch hydrogenolytische Spaltung die Benzyloxycarbonyl-Schutzgruppe sowie der Benzylester entfernt und anschließend mit Zinkchlorid die 2-(Trimethylsilyl)ethoxycarbonyl-Schutzgrupe.

LC-MS (Methode 6): $R_t$ = 1.91 min; MS (Elpos): m/z = 685 $[M+H]^+$.

## Beispiel M19

$N^6$-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-L-lysin -trifluoressigsäure (1:1)

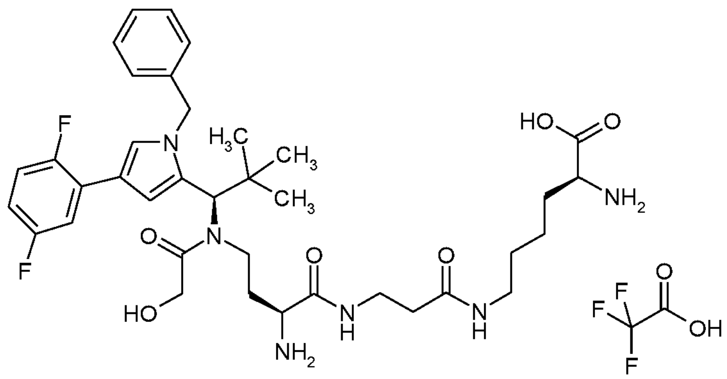

Zunächst wurde nach in der Peptidchemie bekannten klassischen Schutzgruppenoperationen Trifluoressigsäure --2-(trimethylsilyl)ethyl-N2-[(benzyloxy)carbonyl]-L-lysinat (1:1) hergestellt. Dieses Intermediat wurde dann in Gegenwart von HATU mit Intermediat C61 gekuppelt. Anschließend wurde zunächst mit Zinkchlorid die 2-(Trimethylsilyl)ethoxycarbonyl-Schutzgrupe sowie der 2-(Trimethylsilyl)ethylester gespalten. Schließlich wurde die Titelverbindung durch hydrogenolytische Spaltung der Benzyloxycarbonyl-Schutzgruppe und Reinigung durch präparative HPLC erhalten.

HPLC (Methode 11): $R_t$ = 1.65 min;

**Beispiel M20**

(1R,4R,27R,33R)-1-Amino-32-(3-aminopropyl)-33-[1-benzyl-4-(2,5-difluorphenyl)-1H-pyrrol-2-yl]-34,34-dimethyl-6,9,25,31-tetraoxo-13,16,19,22-tetraoxa-3,29-dithia-7,10,26,32-tetraazapentatriacontan-1,4,27-tricarbonsäure -trifluoressigsäure (1:2)

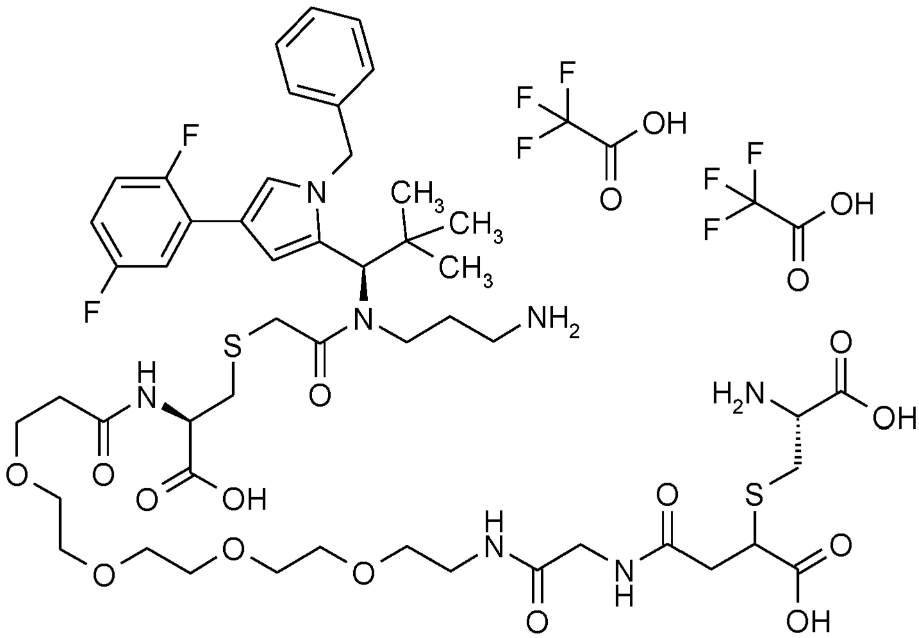

Zunächst wurde Methyl-L-cysteinathydrochlorid (1:1) mit 1-({[2-(Trimethylsilyl)ethoxy] carbonyl}oxy)pyrrolidine-2,5-dion in DMF in Gegenwart von *N,N*-Diisopropylethylamin in Methyl-N-{[2-(trimethylsilyl)ethoxy]carbonyl}-L-cysteinat überführt.

408 mg (1.93 mmol) von kommerziell erhältlicher 3-Brom-4-methoxy-4-oxobutansäure und 180 mg (0.644 mmol) Methyl-N-{[2-(trimethylsilyl)ethoxy]carbonyl}-L-cysteinat wurden in 8 ml DMF gelöst und mit 147 mg (0.97 mmol) 1,8-Diazabicyclo(5.4.0)undec-7-en versetzt. Nach 18 h Rühren bei RT wurden nochmals 136 mg (0.64 mmol) 3-Brom-4-methoxy-4-oxobutansäure sowie 147 mg (0.97 mmol) 1,8-Diazabicyclo(5.4.0)undec-7-en hinzugefügt und der Ansatz

weitere 12 h bei RT gerührt und anschließend im Vakuum eingeengt. Der Rückstand wurde durch präparative HPLC gereinigt. Nach Vereinigung der entsprechenden Fraktionen und Abdampfen der Lösemittel im Vakuum wurden 151 mg (57% d. Th.) 4-Methoxy-3-{[(2R)-3-methoxy-3-oxo-2-({[2-(trimethylsilyl)ethoxy]carbonyl}amino)propyl] sulfanyl}-4-oxobutansäure erhalten.

LC-MS (Methode 12): $R_t$ = 1.74 min; MS (ESIneg): m/z = 408 $(M-H)^-$.

3.66 mg (8.93 μmol) von 4-Methoxy-3-{[(2R)-3-methoxy-3-oxo-2-({[2-(trimethylsilyl)ethoxy]-carbonyl}amino)propyl] sulfanyl}-4-oxobutansäure wurden in Gegenwart von 3.66 mg (8.93 μmol) HATU und 1.6 μl (15 μmol) 4-Methylmorpholin mit 13.0 mg (7.44 μmol) von S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-(glycylamino)-4,7,10,13-tetraoxapentadecan-1-oyl]-L-cystein -trifluoressigsäure (1:1) (Intermediat C80) gekuppelt, wobei nach HPLC Reinigung 3.9 mg (37% d. Th.) vom voll geschützten Intermediat S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(8R,11R)-8,11-bis(methoxycarbonyl)-2,2-dimethyl-6,13-dioxo-5-oxa-10-thia-7-aza-2-silatridecan-13-yl]glycyl}amino)-4,7,10,13-tetraoxapentadecan-1-oyl]-L-cystein erhalten wurden.

Anschließend wurden 3.90 mg (2.76 μmol) dieses Intermediats mit 35 μl einer 2 molaren Lithiumhydroxidlösung in 1.0 ml THF/Wasser 3:1 15 min lang bei RT gerührt, wobei beide Methylestergruppen abgespalten wurden. Nach HPLC-Reinigung wurden 3.60 mg (94% d. Th.) des Dicarbonsäurederivats erhalten.

LC-MS (Methode 5): $R_t$ = 4.83 min; MS (ESIpos): m/z = 1385 $[M+H]^+$.

3.6 mg (2.6 μmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 1.92 mg (55% d. Th.) der Titelverbindung erhalten.

LC-MS (Methode 5): $R_t$ = 2.72 min; MS (ESIneg): m/z = 1094 $[M-H]^-$.

## Beispiel M21

(2R,24S,27R)-27-Amino-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-24-(carboxymethyl)-4,20,23-trioxo-7,10,13,16-tetraoxa-25-thia-3,19,22-triazaoctacosan-1,28-disäure -trifluoressigsäure (1:2)

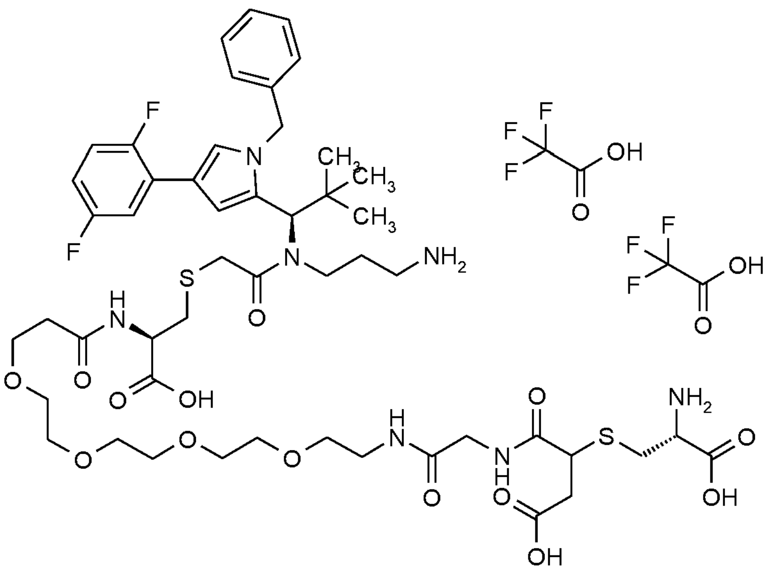

742.8 mg (3.3 mmol) von kommerziell erhältlicher 2-Brom-4-ethoxy-4-oxobutansäure und 802 mg (2.87 mmol) Methyl-N-{[2-(trimethylsilyl)ethoxy]carbonyl}-L-cysteinat wurden in 32 ml DMF gelöst und mit 655.4 mg (4.31 mmol) 1,8-Diazabicyclo(5.4.0)undec-7-en versetzt. Nach 20 h Rühren bei RT wurde der Ansatz im Vakuum eingeengt und der Rückstand durch präparative HPLC gereinigt. Nach Vereinigung der entsprechenden Fraktionen und Abdampfen der Lösemittel im Vakuum wurden 521 mg (43% d. Th.) 4-Ethoxy-2-{[(2R)-3-methoxy-3-oxo-2-({[2-(trimethylsilyl)ethoxy]carbonyl}amino)propyl]sulfanyl}-4-oxobutansäure erhalten.

LC-MS (Methode 5): $R_t$ = 3.13 min; MS (ESIpos): m/z = 424 $(M+H)^+$.

4.36 mg (10.3 μmol) von 4-Ethoxy-2-{[(2R)-3-methoxy-3-oxo-2-({[2-(trimethylsilyl)ethoxy]-carbonyl}amino)propyl]sulfanyl}-4-oxobutansäure wurden in Gegenwart von 3.92 mg (10.3 μmol) HATU und 1.9 μl (17 μmol) 4-Methylmorpholin mit 15.0 mg (8.59 μmol) von S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-(glycylamino)-4,7,10,13-tetraoxapentadecan-1-oyl]-L-cystein -trifluoressigsäure (1:1) (Intermediat C80) gekuppelt, wobei nach HPLC Reinigung 3.6 mg (26% d. Th.) vom voll geschützten Intermediat S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(8R,11S)-11-(2-ethoxy-2-oxoethyl)-8-(methoxycarbonyl)-2,2-dimethyl-6,12-dioxo-5-oxa-10-thia-7-aza-2-siladodecan-12-yl]glycyl}amino)-4,7,10,13-tetraoxapentadecan-1-oyl]-L-cystein erhalten wurden.

Anschließend wurden 6.20 mg (2.82 μmol) dieses Intermediats mit 35 μl einer 2 molaren Lithiumhydroxidlösung in 1.0 ml THF/Wasser 1:1 15 min lang bei RT gerührt, wobei beide Estergruppen abgespalten wurden. Nach Ansäuern und HPLC-Reinigung wurden 3.60 mg (92% d. Th.) des Dicarbonsäurederivats erhalten.

LC-MS (Methode 5): $R_t$ = 4.71 min; MS (ESIpos): m/z = 1385 $[M+H]^+$.

3.60 mg (1.69 μmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 0.88 mg (39% d. Th.) der Titelverbindung erhalten.

LC-MS (Methode 5): $R_t$ = 2.72 min; MS (ESIneg): m/z = 1094 $[M-H]^-$.

**Beispiel M22**

(2R,27R)-27-Amino-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-24-(carboxymethyl)-4,20,23-trioxo-7,10,13,16-tetraoxa-25-thia-3,19,22-triazaoctacosan-1,28-disäure-trifluoressigsäure (1:2) und
(1R,27R,33R)-1-Amino-32-(3-aminopropyl)-33-[1-benzyl-4-(2,5-difluorphenyl)-1H-pyrrol-2-yl]-34,34-dimethyl-6,9,25,31-tetraoxo-13,16,19,22-tetraoxa-3,29-dithia-7,10,26,32-tetraazapentatriacontan-1,4,27-tricarbonsäure-trifluoressigsäure (1:2)

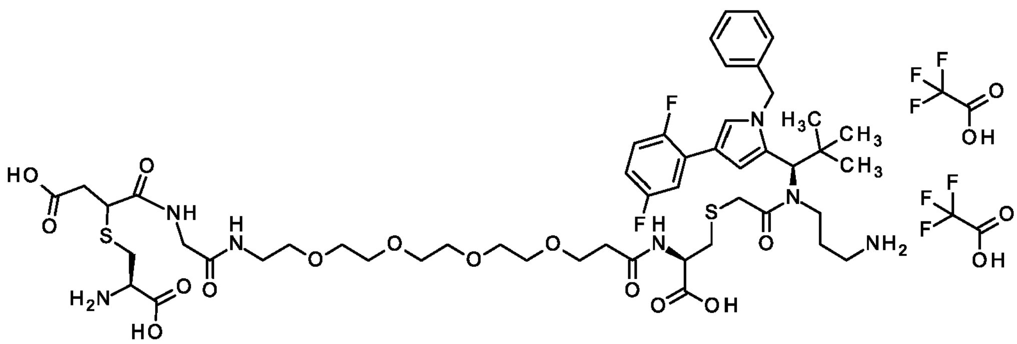

+

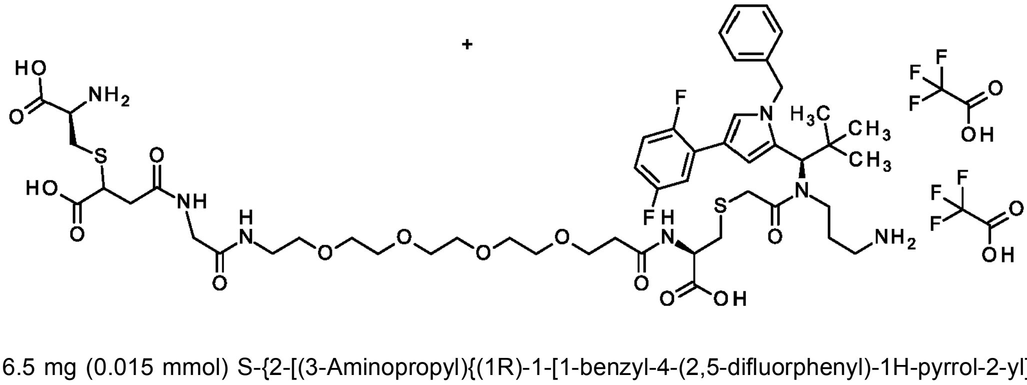

16.5 mg (0.015 mmol) S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[1-(2,5-dioxo-2,5-dihydro-1H-pyrrol-1-yl)-2,18-dioxo-6,9,12,15-tetraoxa-3-azaoctadecan-18-yl]-L-cystein -trifluoressigsäure (1:1) (Intermediat F257) und 8.18 mg (0.031 mmol) N-{[2-(Trimethylsilyl) ethoxy]carbonyl}-L-cystein wurden in 2 ml DMF gelöst und der Ansatz 18 h bei RT gerührt. Die Reaktionsmischung wurde im Vakuum verdampft. Der Rückstand (28.9 mg) wurde in 3 mL THF/Wasser 1:1 gelöst. Es wurden 0.046 mL einer 2M wässrigen Lithiumhydroxidlösung zugegeben und der Ansatz 3 Stunde bei RT gerührt. Anschließend wurde der Ansatz

mit 5.2 µl (0.092 mmol) Essigsäure auf einen pH-Wert von ~7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 12.1 mg (58% über 2 Stufen) der regioisomeren geschützten Intermediate erhalten. LC-MS (Methode 12): $R_t$ = 1.82 min; MS (ESIpos): m/z = 1240 $(M+H)^+$.

Im letzten Schritt wurden 12,1 mg (0.009 mmol) von diesem Intermediat in 2 mL 2,2,2-Trifluor-ethanol gelöst. Es wurden 7.3 mg (0.054 mmol) Zinkchlorid zugegeben und der Ansatz 2 h bei 50°C gerührt. Anschließend wurde 15.7 mg (0.054 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 6.4 mg (59%) der Titelverbindung als Regioisomerengemisch erhalten.

LC-MS (Methode 1): $R_t$ = 0.86 min; MS (ESIpos): m/z = 1096 $(M+H)^+$.

**Beispiel M23**

N-{(2S)-2-Amino-4-[{(1R)-1-[1-benzyl-4-(2,5-difluorphenyl)-1H-pyrrol-2-yl]-2,2-dimethyl propyl}(glycoloyl)amino]butanoyl}-beta-alanyl-L-glutaminsäure -trifluoressigsäure (1:1)

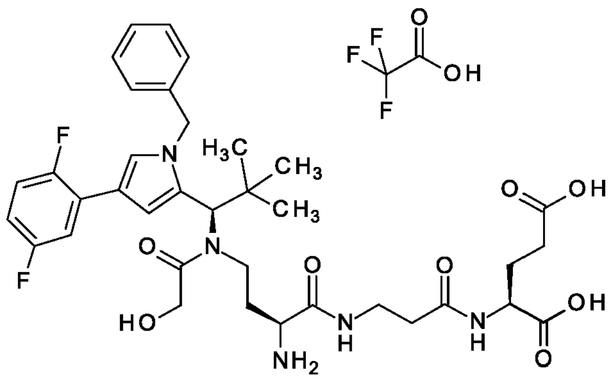

Zunächst wurde Di-tert-butyl L-glutamate hydrochloride (1:1) in Gegenwart von HATU und *N,N*-Diisopropylethylamin mit Intermediat C61 gekuppelt. Anschließend wurde das geschützte Intermediat in Trifluorethanol aufgenommen und durch Rühren über Nacht bei 50°C in Gegenwart von Zinkchlorid vollständig entschützt. Die Aufarbeitung erfolgte nach Zugabe von EDTA durch Reinigung mittels präparativer HPLC.

LC-MS (Methode 12): $R_t$ = 1.45 min; MS (ESIpos): m/z = 714 $[M+H]^+$.

**Beispiel M24**

N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-D-glutaminsäure-trifluoressigsäure (1:1)

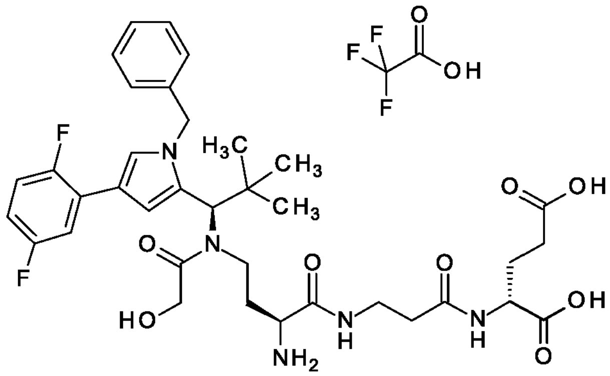

Zunächst wurde Di-tert-butyl D-glutamate hydrochloride (1:1) in Gegenwart von HATU und *N,N*-Diisopropylethylamin mit Intermediat C61 gekuppelt. Anschließend wurde das geschützte Intermediat in Trifluorethanol aufgenommen und durch Rühren bei 50°C in Gegenwart von Zinkchlorid vollständig entschützt. Die Aufarbeitung erfolgte nach Zugabe von EDTA durch Reinigung mittels präparativer HPLC.

LC-MS (Methode 12): $R_t$ = 1.41 min; MS (ESIpos): m/z = 714 $[M+H]^+$.

**Beispiel M25**

N-{(2S)-2-Amino-4-[{(1R)-1-[1-benzyl-4-(2,5-difluorphenyl)-1H-pyrrol-2-yl]-2,2-dimethyl propyl}(glycoloyl)amino]butanoyl}-L-glutaminsäure -trifluoressigsäure (1:1)

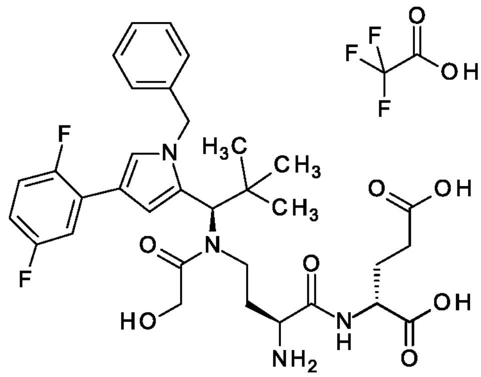

Zunächst wurde Di-tert-butyl L-Glutamat hydrochlorid (1:1) in Gegenwart von HATU und *N,N*-Diisopropylethylamin mit Intermediat C61 gekuppelt. Im nächsten Schritt wurde die Z-Schutzgruppe durch 45-minütige Hydrierung über 10%-igem Palladium auf Aktivkohle in Methanol bei RT unter Wasserstoff-Normaldruck entfernt. Anschließend wurde das partiell geschützte Intermediat in Trifluorethanol aufgenommen und durch 7 stündiges Rühren bei 50°C in Gegenwart von Zinkchlorid vollständig entschützt. Die Aufarbeitung erfolgte nach Zugabe von EDTA durch Reinigung mittels präparativer HPLC.

LC-MS (Methode 12): $R_t$ = 1.44 min; MS (ESIpos): m/z = 643 $[M+H]^+$.

**Beispiel M26**

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure -trifluoressigsäure (1:1) Regioisomer 1, Epimerengemisch

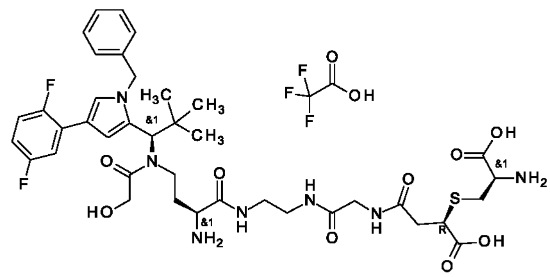

und

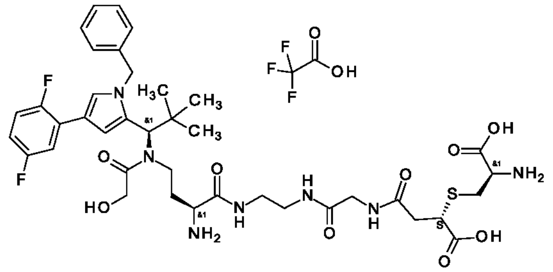

Dieses Beipiel beschreibt das Epimerengemisch der Verbindungen aus Beispiel 13 und Beispiel 14. Die Synthese erfolgte in Analogie zu Beispiel 13, wobei auf die Auftrennung der beiden Epimere durch überkritische Fluid-Chromatograpie verzichtet wurde und die Titelverbindung als Epimerengemisch hergestellt wurde.

LC-MS (Methode 5): $R_t$ = 2.43 min; MS (ESIpos): m/z = 832 $[M+H]^+$.

## Beispiel M27

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure -trifluoressigsäure (1:1) Regioisomer 2, Epimerengemisch

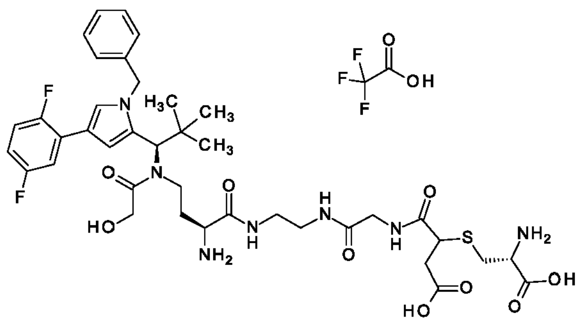

und

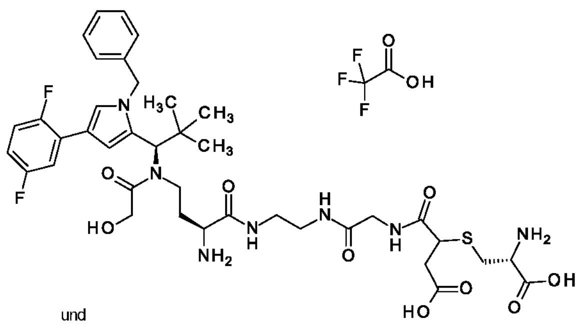

Dieses Beipiel beschreibt das Epimerengemisch der Verbindungen aus Beispiel 15 und Beispiel 16. Die Synthese erfolgte in Analogie zu Beispiel 15, wobei auf die Auftrennung der beiden Epimere durch überkritische Fluid-Chromatograpie verzichtet wurde und die Titelverbindung als Epimerengemisch hergestellt wurde.

LC-MS (Methode 5): $R_t$ = 2.45 min; MS (Elpos): m/z = 832 $[M+H]^+$.

## Beispiel M28

$N^6$-{(3R,7S)-7-Amino-3-[1-benzyl-4-(2,5-difluorphenyl)-1H-pyrrol-2-yl]-4-glycoloyl-2,2-dimethyl-8,13,16,20-tetraoxo-4,9,12,15-tetraazaicosan-20-yl}-L-lysin-trifluoressigsäure (1:1)

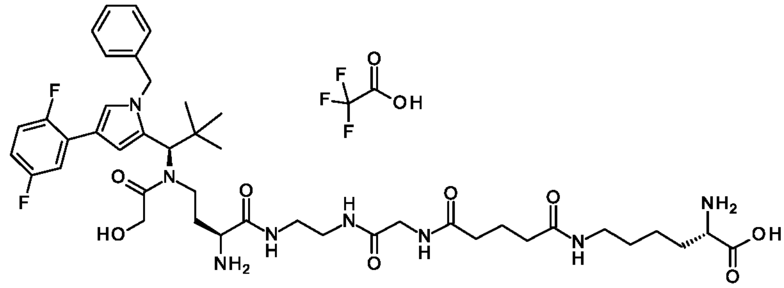

Die Titelverbindung wurde ausgehend von Intermediat C66 nach klassischen Methoden der Peptidchemie zunächst durch Kupplung mit 1,1'-[(1,5-Dioxopentan-1,5-diyl)bis(oxy)] dipyrrolidin-2,5-dion zu 2-(Trimethylsilyl)ethyl-[(2S)-4-[{(1R)-1-[1-benzyl-4-(2,5-difluorphenyl)-1H-pyrrol-2-yl]-2,2-dimethylpropyl}(glycoloyl)amino]-1-({2-[(N-{5-[(2,5-dioxopyrrolidin-1-yl)oxy]-5-oxopentanoyl}glycyl)amino]ethyl}amino)-1-oxobutan-2-yl]carbamat in DMF in Gegenwart von N,N-Diisopropylethylamin hergestellt. Im nächsten Schritt erfolgte ebenfalls in DMF in Gegenwart von N,N-Diisopropylethylamin die Kupplung mit tert-Butyl-$N^2$-(tert-butoxycarbonyl)-L-lysinathydrochlorid (1:1), bevor dann im letzten Schritt alle Schutzgruppen mit 6 Equivalenten Zinkchlorid in Trifluorethanol durch 3 stündiges Rühren bei 50°C abgespalten wurden.

LC-MS (Methode 1): $R_t$ = 0.74 min; MS (ESIpos): m/z = 855 $[M+H]^+$.

**Beispiel M29**

$N^6$-(5-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}amino)ethyl]amino}-5-oxopentanoyl)-L-lysin - trifluoressigsäure (1:1)

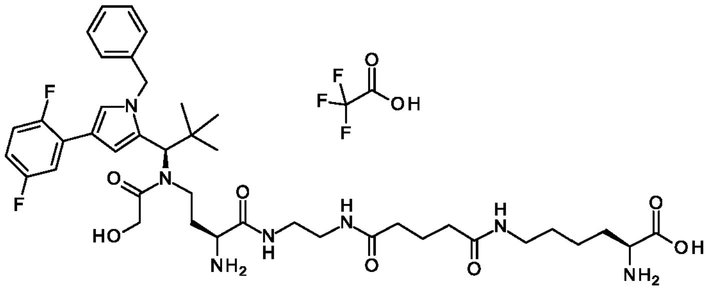

Zunächst wurde (2S)-4-[{(1R)-1-[1-Benzyl-4-(2,5-difluorphenyl)-1H-pyrrol-2-yl]-2,2-dimethyl propyl}(glycoloyl)amino]-2-[(tert-butoxycarbonyl)amino]butansäure mit Benzyl-(2-aminoethyl) carbamathydrochlorid (1:1) in Gegenwart von HATU und N,N-Diisopropylethylamin gekuppelt. Anschließend erfolgte die Abspaltung der Z-Schutzgruppe durch 45-minütige Hydrierung über 10%-igem Palladium auf Aktivkohle in Methanol bei RT unter Wasserstoff-Normaldruck. Dann erfolgte in Analogie zu Beispiel M28 die Kupplung mit 1,1'-[(1,5-Dioxopentan-1,5-diyl)bis(oxy)] dipyrrolidin-2,5-dion. Im nächsten Schritt wurde das Intermediat mit tert-Butyl-$N^2$-(tert-butoxycarbonyl)-L-lysinathydrochlorid (1:1) in DMF in Gegenwart von N,N-Diisopropylethylamin gekuppelt, bevor dann im letzten Schritt alle Schutzgruppen mit 8 Equivalenten Zinkchlorid in Trifluorethanol durch 5.5 stündiges Rühren bei 50°C abgespalten wurden. Nach Reinigung mittels präparativer HPLC wurde die Titelverbindung erhalten.

LC-MS (Methode 12): $R_t$ = 1.28 min; MS (ESIneg): m/z = 796 $[M-H]^+$.

**Ausführungsbeispiele APDCs und ADCs**

Die in den Strukturformen der Ausführungsbeispiele dargestellten APDCs und ADCs, die über Maleinimid-Reste an Cysteinseitenketten der Antikörper gekuppelt wurden, liegen in Abhängigkeit vom Linker und von dem Kupplungsprotokoll zum überwiegenden Teil in den jeweils dargestellten ring-geöffneten bzw. ring-geschlossenen Formen vor. Zu einem kleinen Anteil kann jedoch die jeweils andere Form in der Präparation enthalten sein. Die Kupplungsreaktionen wurden unter Argon durchgeführt.

**Beispiel 1a**

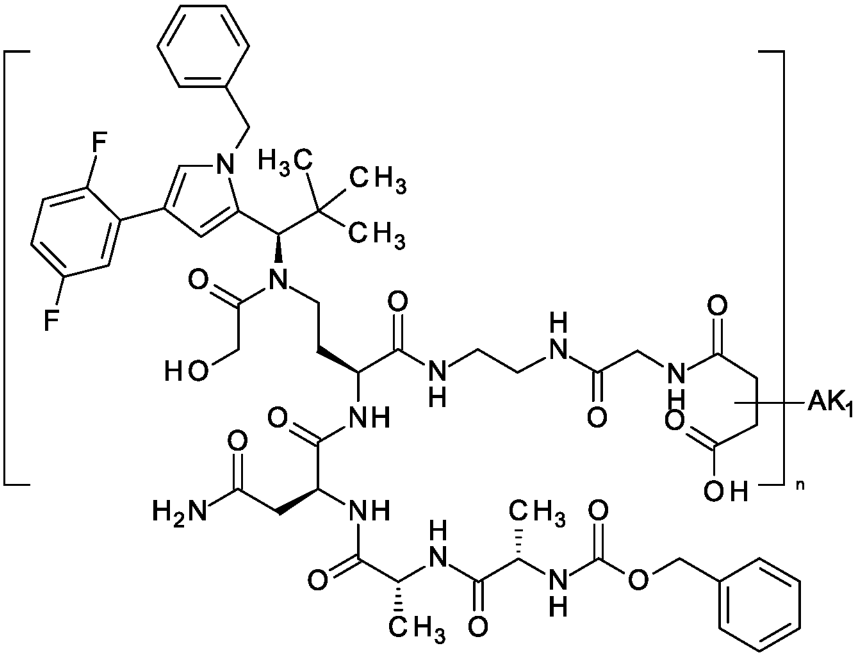

5 mg Cetuximab in 0.5 ml PBS (c=10 mg/mL) wurden unter Argon mit einer Lösung aus 0.028 mg TCEP in 0.05 ml PBS-Puffer versetzt. Der Ansatz wurde 30min bei RT gerührt und anschließend wurden 0.25 mg (0.00023 mmol) von Intermediat R1 gelöst in 50 μl DMSO zugegeben. Nach weiteren 90 min Rühren bei RT wurde der Ansatz mit PBS-Puffer, der zuvor auf pH 8 eingestellt wurde, auf ein Volumen von 2.5 ml verdünnt und dann über eine mit PBS-Puffer pH8 equillibrierte PD 10-Säule (Sephadex® G-25, GE Healthcare) gegeben und mit PBS-Puffer pH8 eluiert. Das Eluat wurde anschließend über Nacht bei RT unter Argon gerührt.

Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 1.85 mg/mL
Drug/mAb Ratio: 2.6

## Beispiel 1e

In analoger Weise wurde Intermediat R1 mit 5 mg anti-HER2 Antikörper TPP-1015 gekuppelt. Der erhaltene ADC Batch wurde folgendermaßen charakterisiert:

Protein Konzentration: 1.85 mg/mL
Drug/mAb Ratio: 3.4

## Beispiel 1k

In analoger Weise wurde Intermediat R1 mit 5 mg anti-TWEAKR Antikörper TPP-2658 gekuppelt. Der erhaltene ADC Batch wurde folgendermaßen charakterisiert:

Protein Konzentration: 1.42 mg/mL
Drug/mAb Ratio: 2.9

## Beispiel 2a

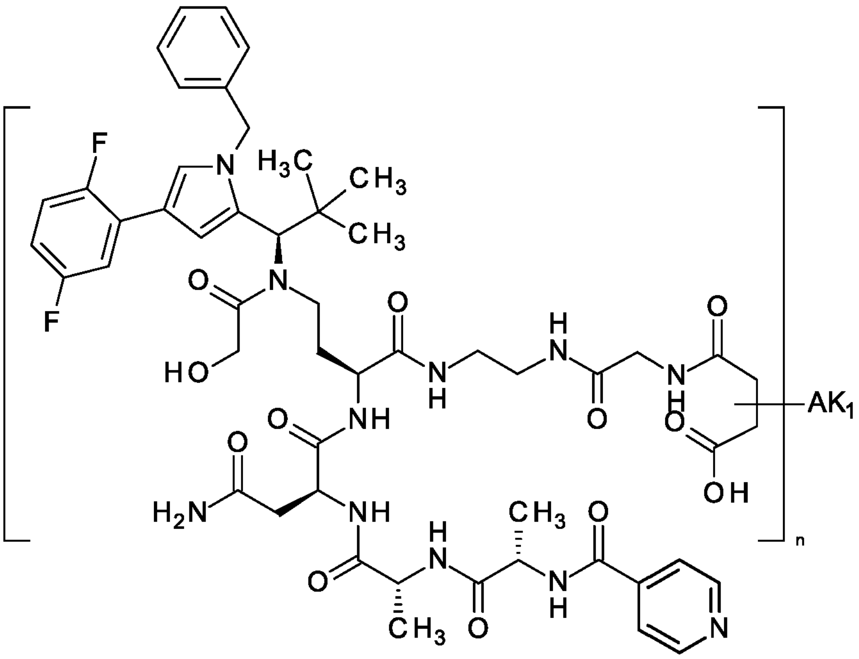

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.27 mg (0.00023 mmol) von Intermediat R2 gelöst in 50 µl DMSO zugegeben. Nach weiteren 90 min Rühren bei RT wurde der Ansatz mit 1.942 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 1.99 mg/mL
Drug/mAb Ratio: 2.6

**Beispiel 2e**

In analoger Weise wurde Intermediat R2 mit 5 mg anti-HER2 Antikörper TPP-1015 gekuppelt. Der erhaltene ADC Batch wurde folgendermaßen charakterisiert:

Protein Konzentration: 1.6 mg/mL
Drug/mAb Ratio: 3.5

**Beispiel 2k**

In analoger Weise wurde Intermediat R2 mit 5 mg anti-TWEAKR Antikörper TPP-2658 gekuppelt. Der erhaltene ADC Batch wurde folgendermaßen charakterisiert:

Protein Konzentration: 2.05 mg/mL
Drug/mAb Ratio: 3.4

**Beispiel 3a**

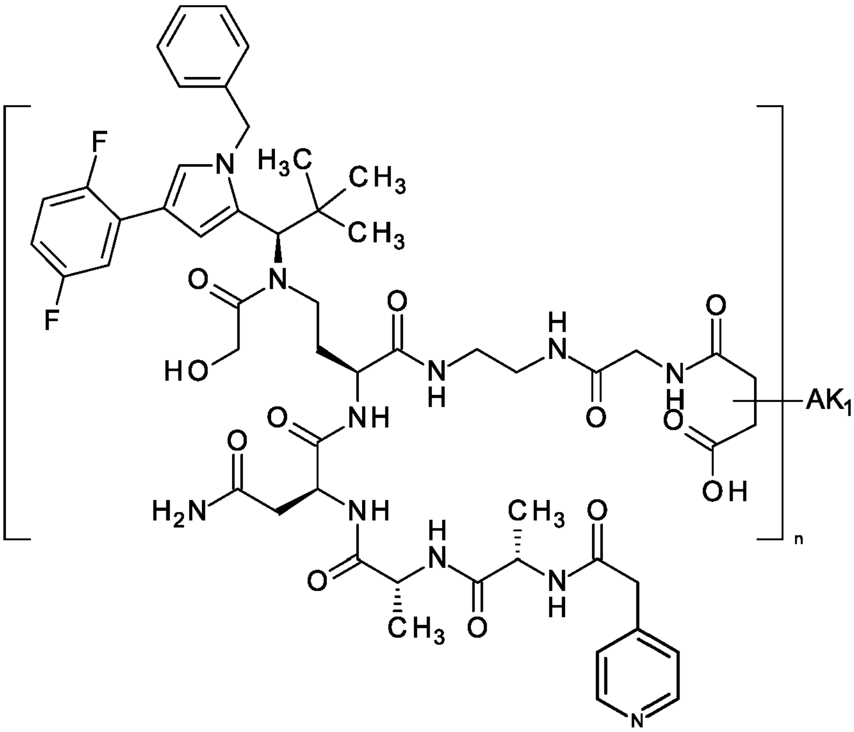

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.28 mg (0.00023 mmol) von Intermediat R3 gelöst in 50 µl DMSO zugegeben. Nach weiteren 90 min Rühren bei RT wurde der Ansatz mit 1.942 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 2.07 mg/mL
Drug/mAb Ratio: 2.2

**Beispiel 3e**

In analoger Weise wurde Intermediat R3 mit 5 mg anti-HER2 AntikörperTPP-1015 gekuppelt. Der erhaltene ADC Batch wurde folgendermaßen charakterisiert:

Protein Konzentration: 1.85 mg/mL
Drug/mAb Ratio: 3.6

**Beispiel 3h1**

80 mg vom anti-B7H3 Antikörper TPP-8382 in 5.1 ml PBS (c=15.7 mg/mL) wurden unter Argon mit einer Lösung aus 0.46 mg TCEP in 0.75 ml PBS-Puffer (pH 7.2) versetzt. Der Ansatz wurde 30min bei RT gerührt und anschließend wurden 4.41 mg (0.0037 mmol) von Intermediat R3 gelöst in 585 µl DMSO zugegeben. Nach weiteren 90 min Rühren bei RT wurde der Ansatz mit PBS-Puffer, der zuvor auf pH 8 eingestellt wurde, auf 7.5 ml verdünnt und portionsweise über mit PBS-Puffer pH 8 equillibrierte PD 10-Säule (Sephadex® G-25, GE Healthcare) gegeben und mit PBS-Puffer pH 8 eluiert. Die Eluate wurden wieder vereinigt, mit PBS-Puffer pH 8 auf 12.5 ml verdünnt und über Nacht bei RT unter Argon gerührt. Diese Lösung wurde dann über mit PBS-Puffer pH7.2 equillibrierte PD 10-Säulen (Sephadex® G-25, GE Healthcare) gegeben und mit PBS-Puffer pH7.2 eluiert. Anschließend wurde durch Cross-flow aufkonzentriert. Der erhaltene ADC Batch wurde folgendermaßen charakterisiert:

Protein Konzentration: 13.86 mg/mL

Drug/mAb Ratio: 4.8

**Beispiel 3h2**

In analoger Weise wurde Intermediat R3 mit 50 mg anti-B7H3 Antikörper TPP-8567 gekuppelt. Der erhaltene ADC Batch wurde folgendermaßen charakterisiert:

Protein Konzentration: 9.64 mg/mL
Drug/mAb Ratio: 4.3

**Beispiel 3k**

In analoger Weise wurde Intermediat R3 mit 5 mg anti-TWEAKR Antikörper TPP-2658 gekuppelt. Der erhaltene ADC Batch wurde folgendermaßen charakterisiert:

Protein Konzentration: 1.47 mg/mL
Drug/mAb Ratio: 3.4

**Beispiel 3l1**

30 mg vom anti-TWEAKR Antikörper TPP-7007 in 3.4 ml PBS (c=8.8 mg/mL) wurden unter Argon mit einer Lösung aus 0.172 mg TCEP in 0.3 ml PBS-Puffer (pH 7.2) versetzt. Der Ansatz wurde 30min bei RT gerührt und anschließend wurden 1.66 mg (0.0014 mmol) von Intermediat R3 gelöst in 300 µl DMSO zugegeben. Nach weiteren 90 min Rühren bei RT wurde der Ansatz mit PBS-Puffer, der zuvor auf pH 8 eingestellt wurde, auf 5 ml verdünnt und portionsweise über mit PBS-Puffer pH 8 equillibrierte PD 10-Säule (Sephadex® G-25, GE Healthcare) gegeben und mit PBS-Puffer pH 8 eluiert. Die Eluate wurden wieder vereinigt, mit PBS-Puffer pH 8 auf 7.5 ml verdünnt und über Nacht bei RT unter Argon gerührt. Diese Lösung wurde dann über mit PBS-Puffer pH7.2 equillibrierte PD 10-Säulen (Sephadex® G-25, GE Healthcare) gegeben und mit PBS-Puffer pH7.2 eluiert. Anschließend wurde durch Ultrazentrifugation aufkonzentriert, rückverdünnt mit PBS-Puffer (pH 7.2) und erneut aufkonzentriert und steril filtriert. Der erhaltene ADC Batch wurde folgendermaßen charakterisiert:

Protein Konzentration: 8.86 mg/mL
Drug/mAb Ratio: 4.4

**Beispiel 3l2**

In analoger Weise wurde Intermediat R3 mit 48.5 mg anti-TWEAKR Antikörper TPP-7006 in 4.16 ml PBS (c=11.6 mg/mL) gekuppelt. Der erhaltene ADC Batch wurde folgendermaßen charakterisiert:

Protein Konzentration: 11.4 mg/mL
Drug/mAb Ratio: 2.5

**Beispiel 3l3**

In analoger Weise wurde Intermediat R3 mit 30 mg anti-TWEAKR Antikörper TPP-10336 in 2.61 ml PBS (c=11.5 mg/mL) gekuppelt. Der erhaltene ADC Batch wurde folgendermaßen charakterisiert:

Protein Konzentration: 7.72 mg/mL
Drug/mAb Ratio: 2.5

**Beispiel 3l4**

In analoger Weise wurde Intermediat R3 mit 30 mg anti-TWEAKR Antikörper TPP-10337 in 2.78 ml PBS (c=10.8 mg/mL) gekuppelt. Der erhaltene ADC Batch wurde folgendermaßen charakterisiert:

Protein Konzentration: 9.33 mg/mL
Drug/mAb Ratio: 2.7

**Beispiel 4a**

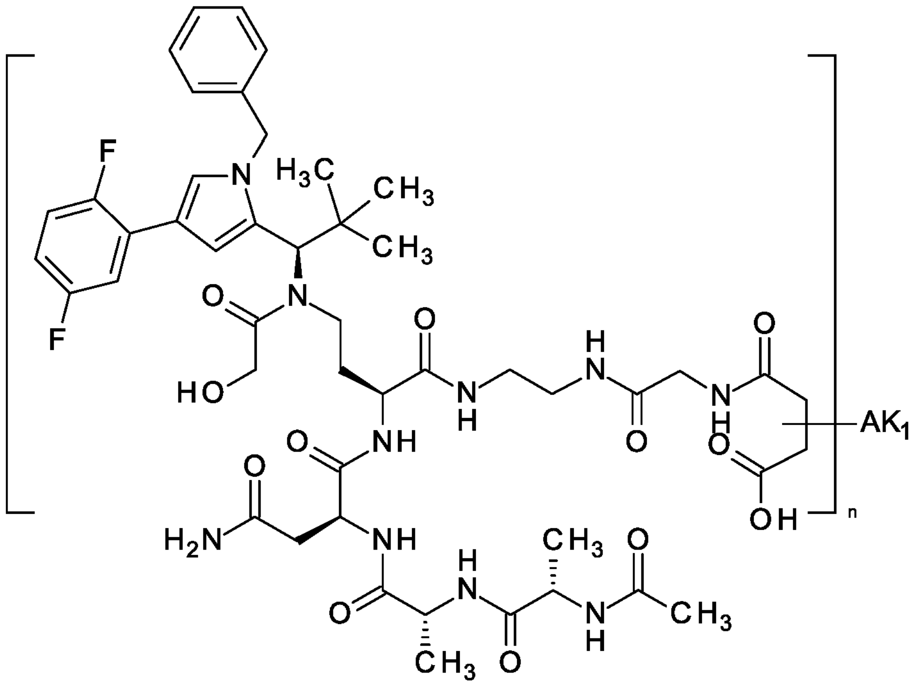

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat R4 gelöst in 50 µl DMSO zugegeben. Nach weiteren 90 min Rühren bei RT wurde der Ansatz mit 1.9 ml PBS-Puffer, der zuvor auf pH 8 eingestellt wurde, auf ein Volumen von 2.5 ml verdünnt und dann über eine mit PBS-Puffer pH8 equillibrierte PD 10-Säule (Sephadex® G-25, GE Healthcare) gegeben und mit PBS-Puffer pH8 eluiert. Das Eluat wurde anschließend über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 2.29 mg/mL
Drug/mAb Ratio: 2.5

**Beispiel 4e**

In analoger Weise wurde Intermediat R4 mit 5 mg anti-HER2 Antikörper TPP-1015 gekuppelt. Der erhaltene ADC Batch wurde folgendermaßen charakterisiert:

Protein Konzentration: 1.75 mg/mL
Drug/mAb Ratio: 3.2

**Beispiel 4k**

In analoger Weise wurde Intermediat R4 mit 5 mg anti-TWEAKR Antikörper TPP-2658 gekuppelt. Der erhaltene ADC Batch wurde folgendermaßen charakterisiert:

Protein Konzentration: 1.73 mg/mL
Drug/mAb Ratio: 3.3

**Beispiel 5a**

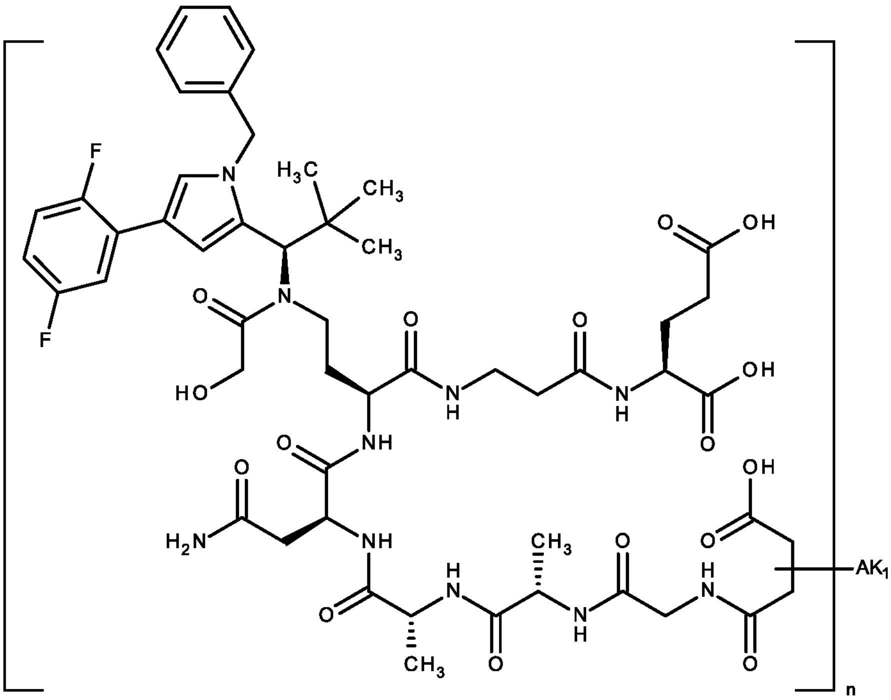

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R5 gelöst in 50 µl DMSO zugegeben. Nach weiteren 90 min Rühren bei RT wurde der Ansatz mit 1.942 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 1.91 mg/mL
Drug/mAb Ratio: 2.7

**Beispiel 5e**

In analoger Weise wurde Intermediat R5 mit 5 mg anti-HER2 Antikörper TPP-1015 gekuppelt. Der erhaltene ADC Batch wurde folgendermaßen charakterisiert:

Protein Konzentration: 1.40 mg/mL
Drug/mAb Ratio: 3.6

**Beispiel 5k**

In analoger Weise wurde Intermediat R5 mit 5 mg anti-TWEAKR Antikörper TPP-2658 gekuppelt. Der erhaltene ADC Batch wurde folgendermaßen charakterisiert:

Protein Konzentration: 1.85 mg/mL
Drug/mAb Ratio: 3.0

**Beispiel 6a**

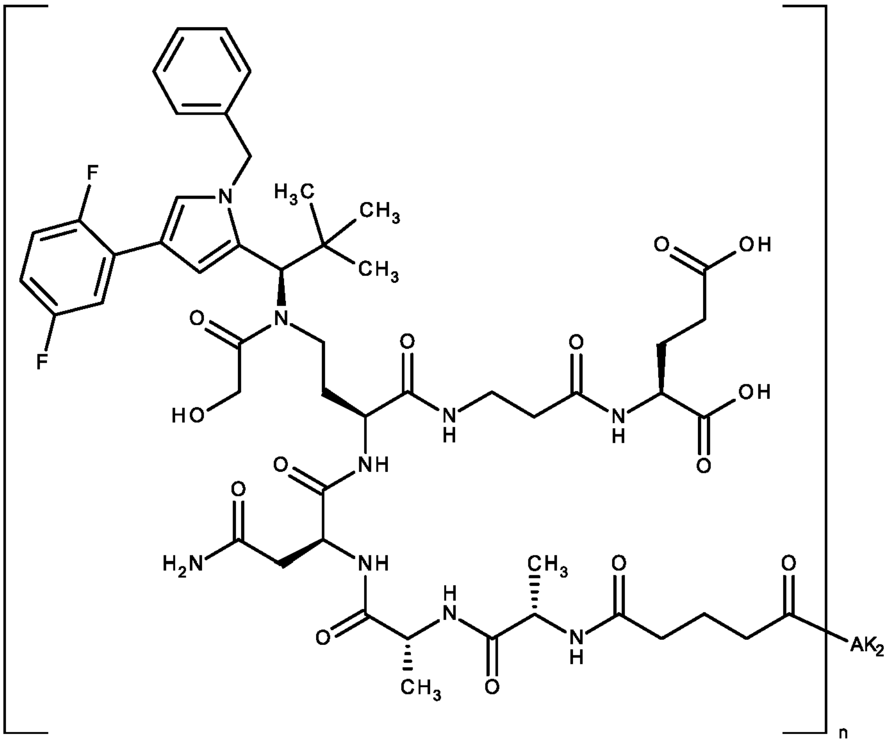

Eingesetzt wurden hier zur Kupplung mit Intermediat R6 5 mg Cetuximab in 459 µl PBS (c = 10.92 mg/mL). Zunächst wurden 5 Eq (0.2mg) von Intermediat R6 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.04 mg/mL

Drug/mAb Ratio: 2.1

**Beispiel 6e**

In analoger Weise wurde Intermediat R6 mit 5 mg anti-HER2 Antikörper TPP-1015 gekuppelt. Der erhaltene ADC Batch wurde folgendermaßen charakterisiert:

Protein Konzentration: 1.74 mg/mL
Drug/mAb Ratio: 2.0

**Beispiel 6k**

In analoger Weise wurde Intermediat R6 mit 5 mg anti-TWEAKR Antikörper TPP-2658 gekuppelt. Der erhaltene ADC Batch wurde folgendermaßen charakterisiert:

Protein Konzentration: 2.0 mg/mL
Drug/mAb Ratio: 2.4

**Beispiel 7a**

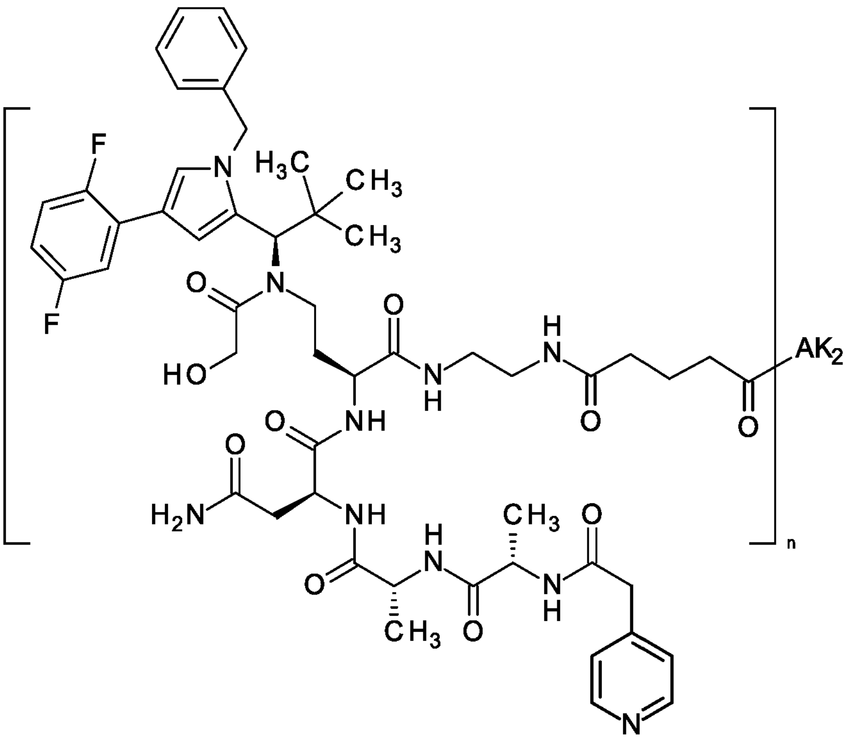

5 mg Cetuximab in 0.5 ml PBS (c=10 mg/mL) wurden unter Argon mit einer Lösung aus 5 Eq (0.19 mg) von Intermediat R7 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.29 mg/mL
Drug/mAb Ratio: 2.9

**Beispiel 7e**

In analoger Weise wurde Intermediat R7 mit 5 mg anti-HER2 Antikörper TPP-1015 gekuppelt. Der erhaltene ADC Batch wurde folgendermaßen charakterisiert:

Protein Konzentration: 1.95 mg/mL
Drug/mAb Ratio: 3.5

**Beispiel 7k**

In analoger Weise wurde Intermediat R7 mit 5 mg anti-TWEAKR Antikörper TPP-2658 gekuppelt. Der erhaltene ADC Batch wurde folgendermaßen charakterisiert:

Protein Konzentration: 1.60 mg/mL
Drug/mAb Ratio: 5.3

**Beispiel 8a**

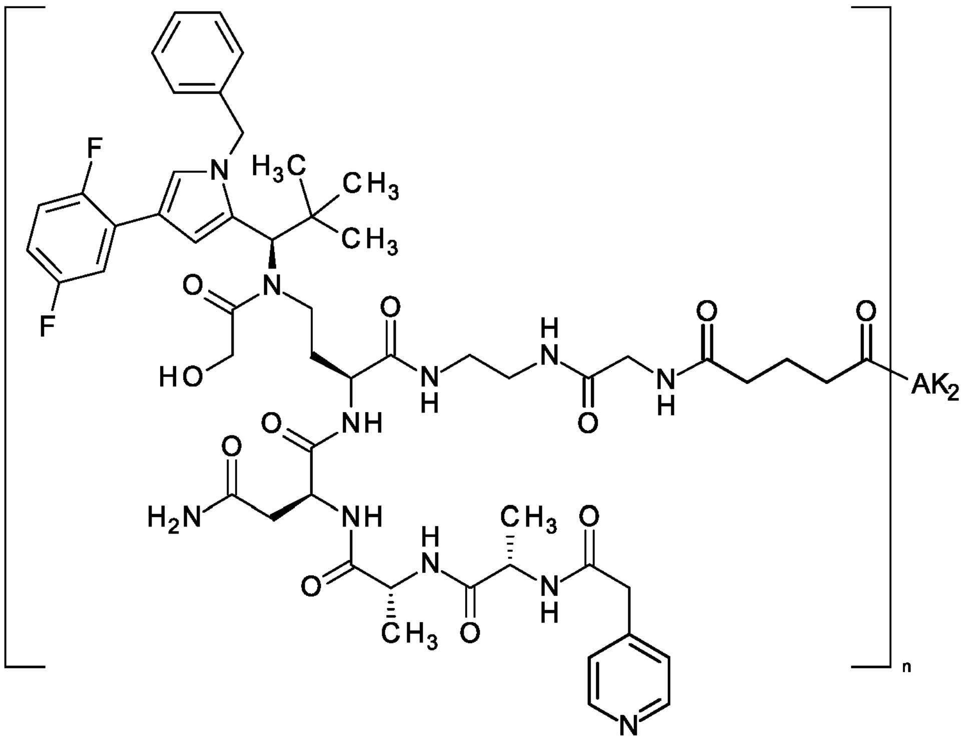

5 mg Cetuximab in 0.5 ml PBS (c=10 mg/mL) wurden unter Argon mit einer Lösung aus 5 Eq (0.2 mg) von Intermediat R8 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 1.57 mg/mL
Drug/mAb Ratio: 3.6

**Beispiel 8e**

In analoger Weise wurde Intermediat R8 mit 5 mg anti-HER2 Antikörper TPP-1015 gekuppelt. Der erhaltene ADC Batch wurde folgendermaßen charakterisiert:

Protein Konzentration: 1.25 mg/mL
Drug/mAb Ratio: 4.8

**Beispiel 8k**

In analoger Weise wurde Intermediat R8 mit 5 mg anti-TWEAKR Antikörper TPP-2658 gekuppelt. Der erhaltene ADC Batch wurde folgendermaßen charakterisiert:

Protein Konzentration: 1.15 mg/mL
Drug/mAb Ratio: 7.0

**Beispiel 9a**

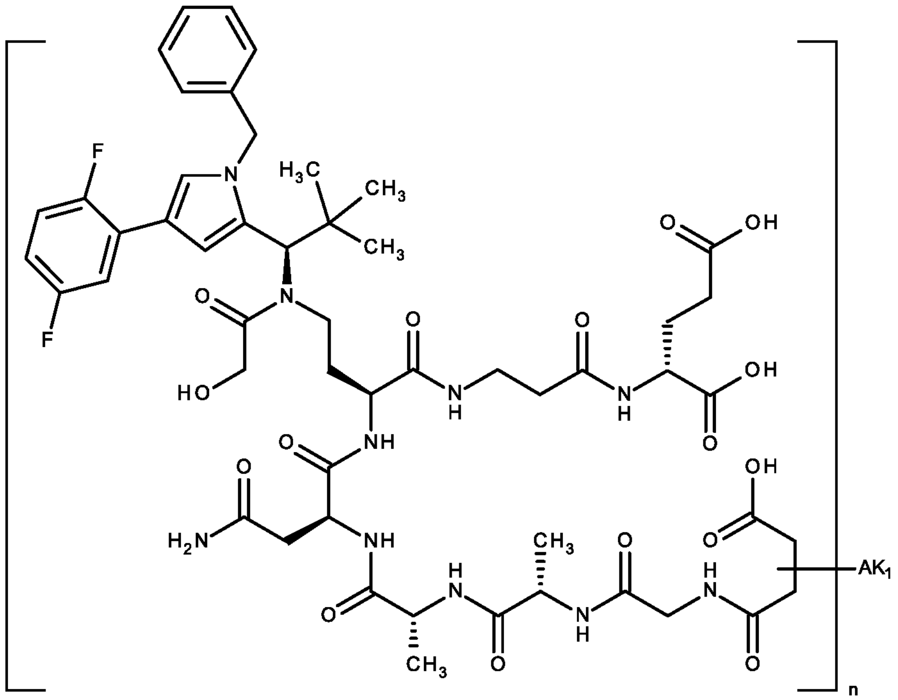

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R9 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 1.95 mg/mL
Drug/mAb Ratio: 2.7

**Beispiel 9e**

In analoger Weise wurde Intermediat R9 mit 5 mg anti-HER2 Antikörper TPP-1015 gekuppelt. Der erhaltene ADC Batch wurde folgendermaßen charakterisiert:

Protein Konzentration: 1.88 mg/mL
Drug/mAb Ratio: 3.2

**Beispiel 9h1**

30 mg vom anti-B7H3 Antikörper TPP-8382 in 2.55 ml PBS (c=15.69 mg/mL) wurden unter Argon mit einer Lösung aus 0.23 mg TCEP in 0.5 ml PBS-Puffer (pH 7.2) versetzt. Der Ansatz wurde 30min bei RT gerührt und anschließend wurden 2.07 mg (0.00187 mmol) von Intermediat R9 gelöst in 250 µl DMSO zugegeben. Nach weiteren 90 min Rühren bei RT wurde der Ansatz mit PBS-Puffer, der zuvor auf pH 8 eingestellt wurde, auf 5 ml verdünnt, geteilt und dann jeweils über eine mit PBS-Puffer pH 8 equillibrierte PD 10-Säule (Sephadex® G-25, GE Healthcare) gegeben und mit PBS-Puffer pH 8 eluiert. Die Eluate wurden wieder vereinigt, mit PBS-Puffer pH 8 auf 7.5 ml verdünnt und über Nacht bei RT unter Argon gerührt. Diese Lösung wurde dann über mit PBS-Puffer pH7.2 equillibrierte PD 10-Säulen (Sephadex® G-25, GE Healthcare) gegeben und mit PBS-Puffer pH7.2 eluiert. Anschließend wurde durch Ultrazentrifugation aufkonzentriert und rückverdünnt mit PBS-Puffer (pH 7.2) und erneut aufkonzentriert und steril filtriert. Der erhaltene ADC Batch wurde folgendermaßen charakterisiert:

Protein Konzentration: 8.46 mg/mL
Drug/mAb Ratio: 2.8

**Beispiel 9h2**

In analoger Weise wurde Intermediat R9 mit 30 mg anti-B7H3 Antikörper TPP-8567 in 3 ml PBS (c=10 mg/mL) gekuppelt. Der erhaltene ADC Batch wurde folgendermaßen charakterisiert:

Protein Konzentration: 8.08 mg/mL
Drug/mAb Ratio: 3.9

**Beispiel 9k**

In analoger Weise wurde Intermediat R9 mit 5 mg anti-TWEAKR Antikörper TPP-2658 gekuppelt. Der erhaltene ADC Batch wurde folgendermaßen charakterisiert:

Protein Konzentration: 1.81 mg/mL
Drug/mAb Ratio: 3.2

**Beispiel 10k**

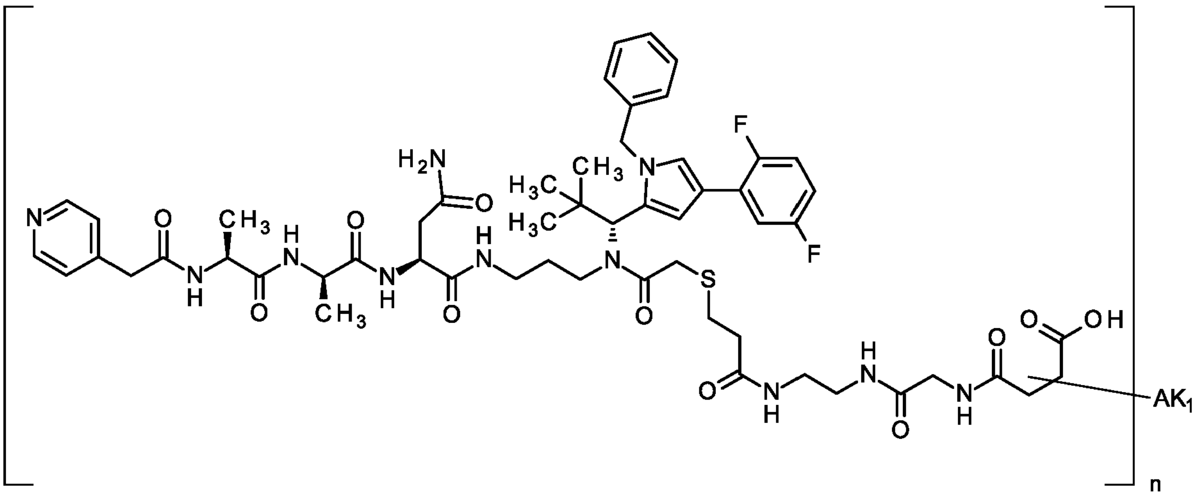

5 mg anti-TWEAKR Antikörper TPP-2658 in 0.24 ml PBS (c = 20.9 mg/mL) wurden unter Argon mit einer Lösung aus 0.028 mg TCEP in 0.05 ml PBS-Puffer versetzt. Der Ansatz wurde mit 2.11 mL PBS-Puffer verdünnt und 30min bei RT gerührt. Anschließend wurden 0.25 mg (0.00023 mmol) von Intermediat R10 gelöst in 100 µl DMSO zugegeben. Nach weiteren 90 min Rühren bei RT wurde der Ansatz über eine mit PBS-Puffer pH 8 equillibrierte PD 10-Säule (Sephadex® G-25, GE Healthcare) gegeben und mit PBS-Puffer pH 8 eluiert. Der Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 1.0
Drug/mAb Ratio: 0

**Beispiel 10h1**

In Analogie zu Beispiel 9h1 wurde Intermediat R10 mit 30 mg anti-B7H3 Antikörper TPP-8382 gekuppelt. Der erhaltene ADC Batch wurde folgendermaßen charakterisiert:

Protein Konzentration: 10.95 mg/mL
Drug/mAb Ratio: 4.4

**Beispiel 11a**

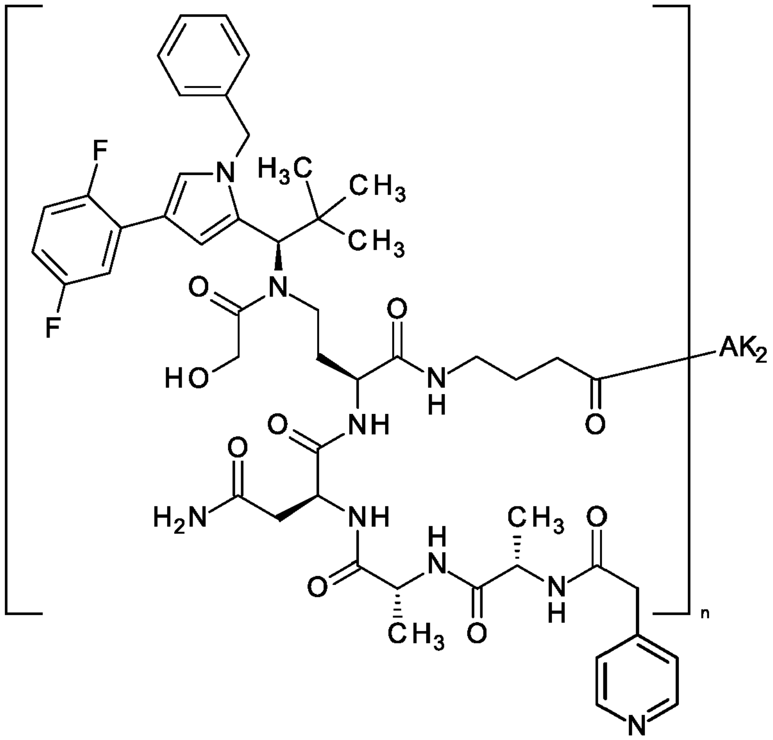

5 mg Cetuximab in 0.5 ml PBS (c=10 mg/mL) wurden unter Argon mit einer Lösung aus 5 Eq (0.2 mg) von Intermediat R11 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.2 mg/mL
Drug/mAb Ratio: 4.0

**Beispiel 11e**

In analoger Weise wurde Intermediat R11 mit 5 mg anti-HER2 Antikörper TPP-1015 gekuppelt. Der erhaltene ADC Batch wurde folgendermaßen charakterisiert:

Protein Konzentration: 1.13 mg/mL
Drug/mAb Ratio: 4.4

**Beispiel 11k**

In analoger Weise wurde Intermediat R11 mit 5 mg anti-TWEAKR Antikörper TPP-2658 gekuppelt. Der erhaltene ADC Batch wurde folgendermaßen charakterisiert:

Protein Konzentration: 0.98 mg/mL
Drug/mAb Ratio: 6.0

**Beispiel 12a**

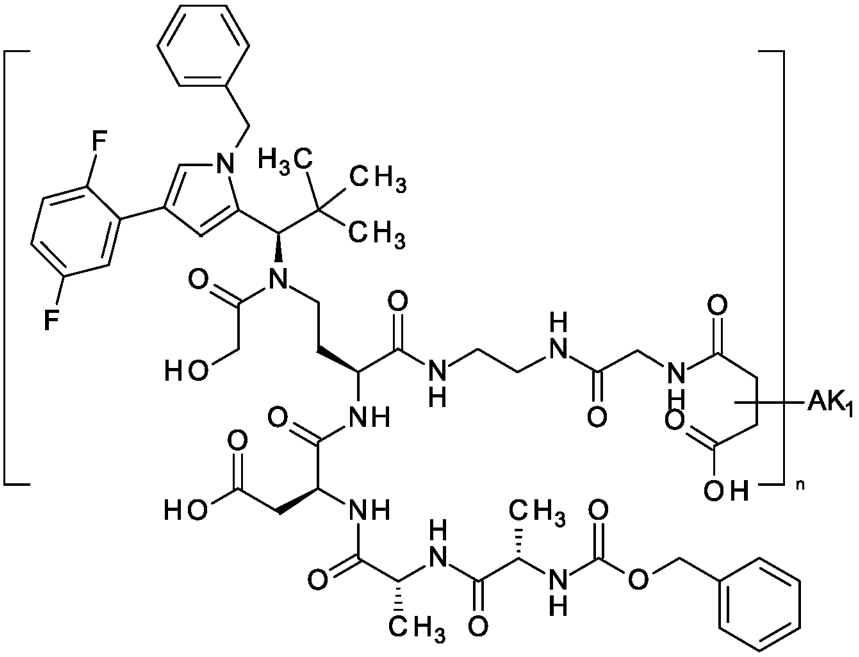

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat R12 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 2.0 mg/mL
Drug/mAb Ratio: 3.3

**Beispiel 12e**

In analoger Weise wurde Intermediat R12 mit 5 mg anti-HER2 Antikörper TPP-1015 gekuppelt. Der erhaltene ADC Batch wurde folgendermaßen charakterisiert:

Protein Konzentration: 1.88 mg/mL
Drug/mAb Ratio: 3.2

**Beispiel 12k**

In analoger Weise wurde Intermediat R12 mit 5 mg anti-TWEAKR Antikörper TPP-2658 gekuppelt. Der erhaltene ADC Batch wurde folgendermaßen charakterisiert:

Protein Konzentration: 1.98 mg/mL
Drug/mAb Ratio: 4.2

**Beispiel 13a**

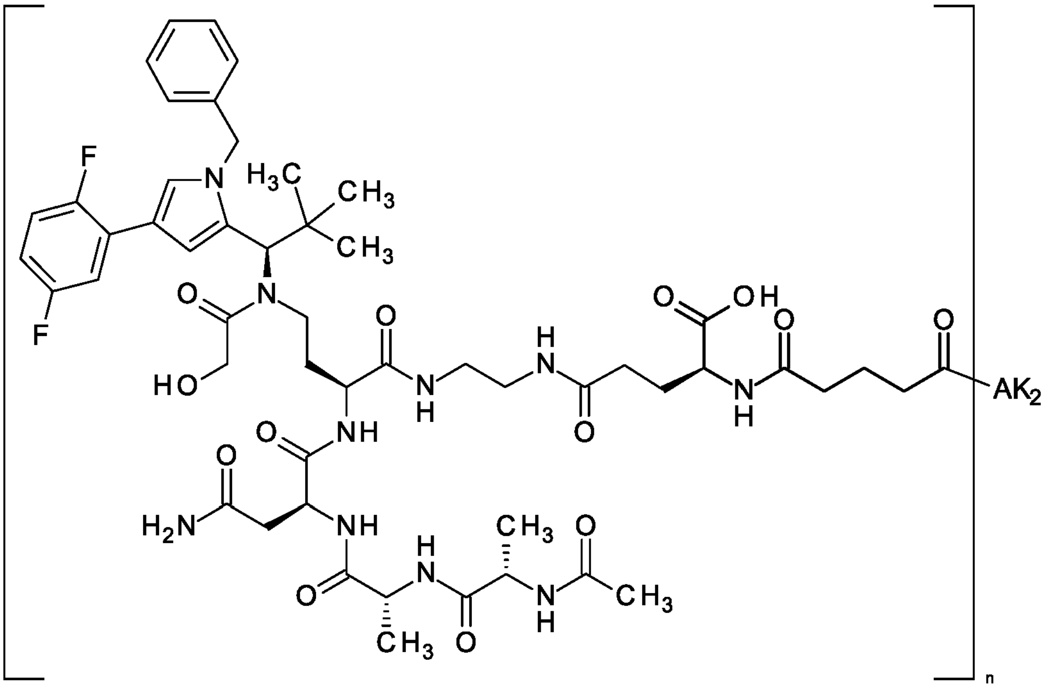

5 mg Cetuximab in 0.55 ml PBS (c=9 mg/mL) wurden unter Argon mit einer Lösung aus 5 Eq (0.21 mg) von Intermediat R13 gelöst in 50μL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.1 mg/mL
Drug/mAb Ratio: 3.5

**Beispiel 13e**

In analoger Weise wurde Intermediat R13 mit 5 mg anti-HER2 Antikörper TPP-1015 gekuppelt. Der erhaltene ADC Batch wurde folgendermaßen charakterisiert:

Protein Konzentration: 2.01 mg/mL
Drug/mAb Ratio: 4.1

**Beispiel 13k**

In analoger Weise wurde Intermediat R13 mit 5 mg anti-TWEAKR Antikörper TPP-2658 gekuppelt. Der erhaltene ADC Batch wurde folgendermaßen charakterisiert:

Protein Konzentration: 2.07 mg/mL
Drug/mAb Ratio: 4.5

**Beispiel 14a**

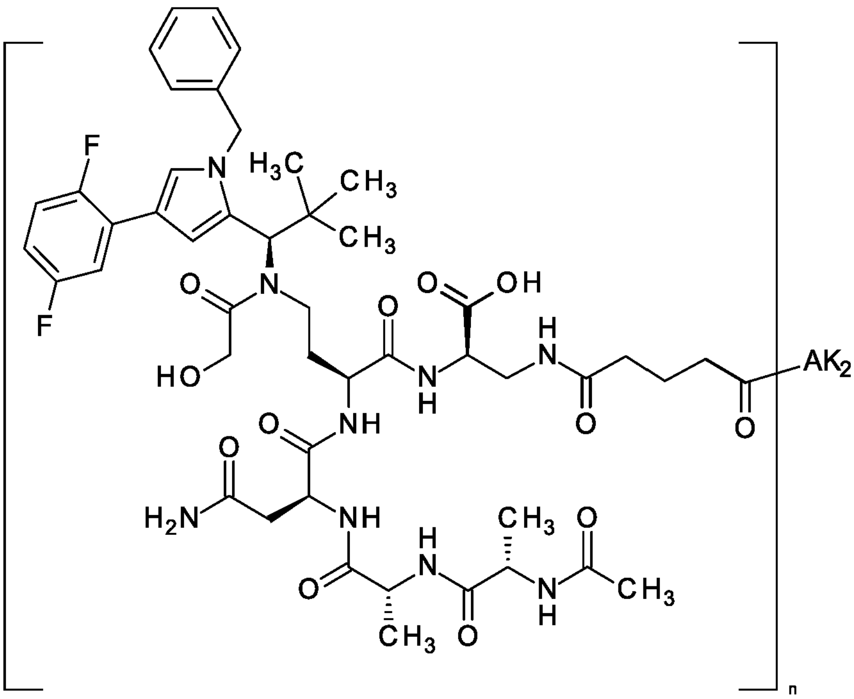

5 mg Cetuximab in 0.5 ml PBS (c=10 mg/mL) wurden unter Argon mit einer Lösung aus 5 Eq (0.19 mg) von Intermediat R14 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.04 mg/mL
Drug/mAb Ratio: 3.9

**Beispiel 14e**

In analoger Weise wurde Intermediat R14 mit 5 mg anti-HER2 Antikörper TPP-1015 gekuppelt. Der erhaltene ADC Batch wurde folgendermaßen charakterisiert:

Protein Konzentration: 1.79 mg/mL
Drug/mAb Ratio: 5.9

**Beispiel 14k**

In analoger Weise wurde Intermediat R14 mit 5 mg anti-TWEAKR Antikörper TPP-2658 gekuppelt. Der erhaltene ADC Batch wurde folgendermaßen charakterisiert:

Protein Konzentration: 2.07 mg/mL
Drug/mAb Ratio: 5.4

**Beispiel 15a**

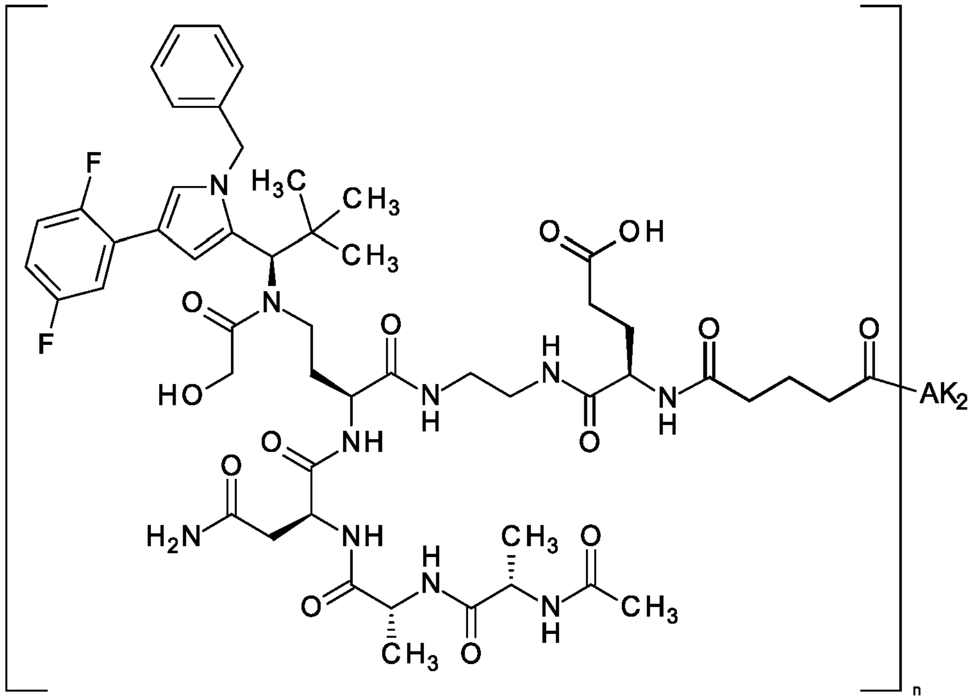

5 mg Cetuximab in 0.458 ml PBS (c=10.9 mg/mL) wurden unter Argon mit einer Lösung aus 5 Eq (0.2 mg) von Intermediat R15 gelöst in 50µL DMSO versetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.31 mg/mL
Drug/mAb Ratio: 5.5

**Beispiel 15e**

In analoger Weise wurde Intermediat R15 mit 5 mg anti-HER2 Antikörper TPP-1015 gekuppelt. Der erhaltene ADC Batch wurde folgendermaßen charakterisiert:

Protein Konzentration: 1.89 mg/mL
Drug/mAb Ratio: 6.8

**Beispiel 15h1**

30 mg vom B7H3 Antikörper TPP-8382 in 1.91 ml PBS (c=15.7 mg/mL) wurden unter Argon mit einer Lösung aus 5 Eq (1.2 mg) von Intermediat R15 gelöst in 200µL DMSO versetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt und über eine Sephadex-Säule gereinigt. Das Eluat wurde anschließend durch Ultrazentrifugation aufkonzentriert, rückverdünnt mit PBS (pH7.2) und erneut aufkonzentriert und steril filtriert .

Protein Konzentration: 10.39 mg/mL
Drug/mAb Ratio: 5.6

**Beispiel 15k**

In analoger Weise wurde Intermediat R15 mit 5 mg anti-TWEAKR Antikörper TPP-2658 gekuppelt. Der erhaltene ADC Batch wurde folgendermaßen charakterisiert:

Protein Konzentration: 1.92 mg/mL

Drug/mAb Ratio: 8.1

**Beispiel 15l1**

In analoger Weise wurde Intermediat R15 mit 5 mg anti-TWEAKR Antikörper TPP-7007 gekuppelt. Dabei wurde der Überschuß von R15 von 10 Equivalenten auf 5 Equivalente reduziert. Der erhaltene ADC Batch wurde folgendermaßen charakterisiert:

Protein Konzentration: 1.64 mg/mL
Drug/mAb Ratio: 2.2

**Beispiel 16a**

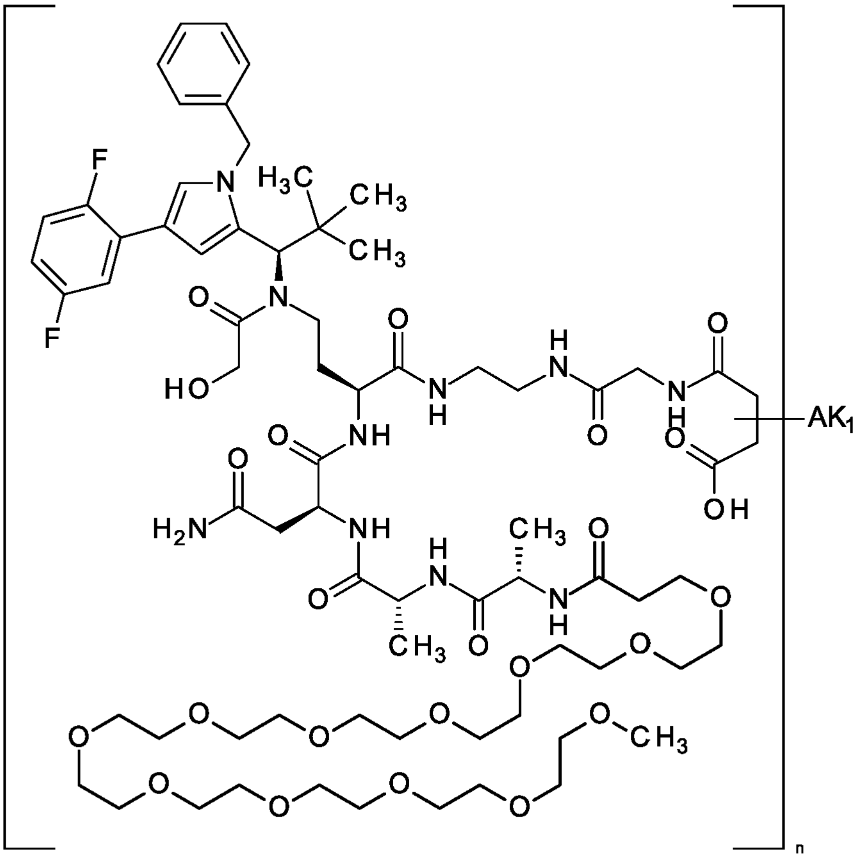

5 mg Cetuximab in 0.458 ml PBS (c=10.92 mg/mL) wurden unter Argon mit einer Lösung aus 0.076 mg TCEP in 0.05 ml PBS-Puffer versetzt. Der Ansatz wurde 150 min bei RT gerührt und anschließend wurden 0.811 mg (0.000533 mmol) von Intermediat R16 gelöst in 50 µl DMSO zugegeben. Nach weiteren 120 min Rühren bei RT wurde der Ansatz mit 1.95 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 2.20 mg/mL
Drug/mAb Ratio: 7.3

**Beispiel 16e**

In analoger Weise wurde Intermediat R16 mit 5 mg anti-HER2 Antikörper TPP-1015 gekuppelt. Der erhaltene

ADC Batch wurde folgendermaßen charakterisiert:

Protein Konzentration: 2,25 mg/mL
Drug/mAb Ratio: 7.3

**Beispiel 16h1**

50 mg vom B7H3 Antikörper TPP-8382 in 3.19 ml PBS (c=15.7 mg/mL) wurden unter Argon mit einer Lösung aus 0.77 mg TCEP in 0.4 ml PBS-Puffer versetzt. Der Ansatz wurde 150 min bei RT gerührt. Dann wurden 16 Eq (8.1 mg) von Intermediat R16 gelöst in 400μL DMSO hinzugefügt und der Ansatz 2h bei RT gerührt. Anschließend wurde der Ansatz mit PBS Puffer (pH8) auf 5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde mit 0.5 mL PBS-Puffer pH8 verdünnt und über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 9.56 mg/mL
Drug/mAb Ratio: 7.2

**Beispiel 16h2**

40 mg vom B7H3 Antikörper TPP-8567 in 2.79 ml PBS (c=14.4 mg/mL) wurden in analoger Weise mit Intermediat R16 gekuppelt.

Protein Konzentration: 9.93 mg/mL
Drug/mAb Ratio: 7.9

**Beispiel 16k**

In analoger Weise wurde Intermediat R16 mit 5 mg anti-TWEAKR Antikörper TPP-2658 gekuppelt. Der erhaltene ADC Batch wurde folgendermaßen charakterisiert:

Protein Konzentration: 2,36 mg/mL
Drug/mAb Ratio: 7.4

**Beispiel 16l2**

25 mg vom anti-TWEAKR Antikörper TPP-7006 in 1.514 ml PBS (c=16.5 mg/mL) wurden wie unter 16h1 beschrieben mit Intermediat R16 gekuppelt.

Protein Konzentration: 11.54 mg/mL
Drug/mAb Ratio: 8.0

**Beispiel 17a**

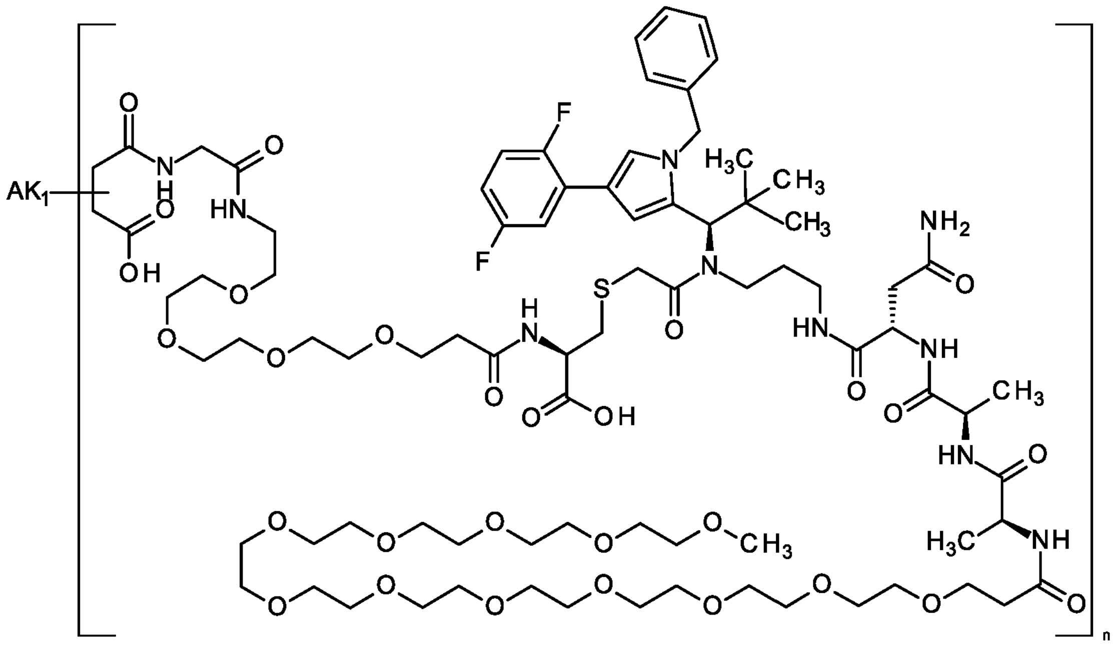

5 mg Cetuximab in 0.458 ml PBS (c=10.92 mg/mL) wurden unter Argon mit einer Lösung aus 0.086 mg TCEP in 0.05 ml PBS-Puffer versetzt. Der Ansatz wurde 150 min bei RT gerührt und anschließend wurden 1,07 mg (0.00060 mmol) von Intermediat R17 gelöst in 50 µl DMSO zugegeben. Nach weiteren 120 min Rühren bei RT wurde der Ansatz mit 1.95 ml PBS-Puffer, der zuvor auf pH 8 eingestellt wurde, verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:

Protein Konzentration: 2.2 mg/mL
Drug/mAb Ratio: 5.5

**Beispiel 17e**

In analoger Weise wurde Intermediat R17 mit 5 mg anti-HER2 Antikörper TPP-1015gekuppelt. Der erhaltene ADC Batch wurde folgendermaßen charakterisiert:

Protein Konzentration: 1,71 mg/mL
Drug/mAb Ratio: 4.3

**Beispiel 17h1**

In analoger Weise wurde Intermediat R17 mit 5 mg anti-B7H3 TPP-8382 gekuppelt. Der erhaltene ADC Batch wurde folgendermaßen charakterisiert:

Protein Konzentration: 1.52 mg/mL
Drug/mAb Ratio: 5.1

**Beispiel 17k**

In analoger Weise wurde Intermediat R17 mit 5 mg anti-TWEAKR Antikörper TPP-2658 gekuppelt. Der erhaltene ADC Batch wurde folgendermaßen charakterisiert:

Protein Konzentration: 1,65 mg/mL
Drug/mAb Ratio: 5.4

**Beispiel 18a**

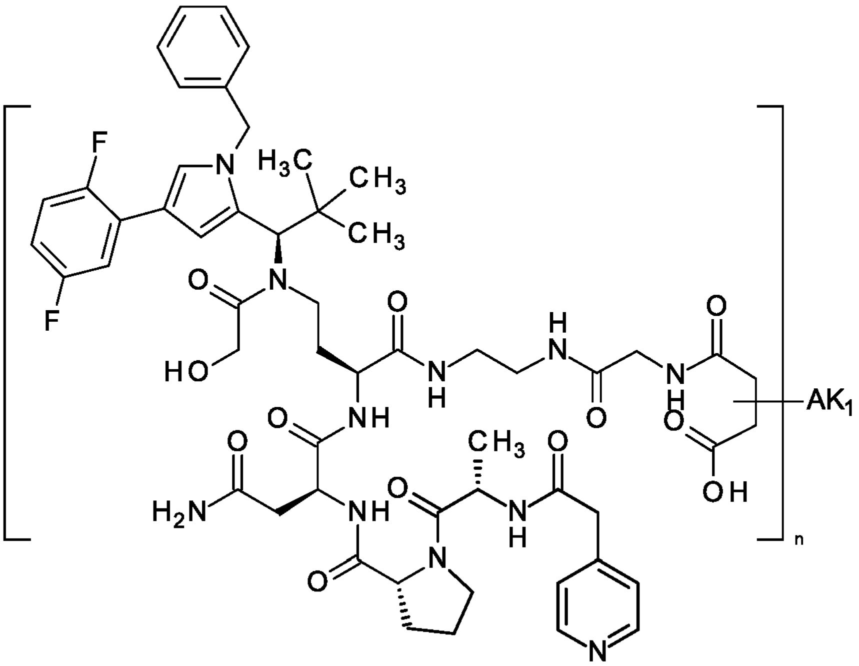

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R18 gelöst in 50 μl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.82 mg/mL
Drug/mAb Ratio: 3.0

**Beispiel 18e**

In analoger Weise wurde Intermediat R18 mit 5 mg anti-HER2 Antikörper TPP-1015 gekuppelt. Der erhaltene ADC Batch wurde folgendermaßen charakterisiert:

Protein Konzentration: 1.7 mg/mL
Drug/mAb Ratio: 4.1

**Beispiel 18h1**

In analoger Weise wurde Intermediat R18 mit 5 mg anti-B7H3 TPP-8382 gekuppelt. Der erhaltene ADC Batch wurde folgendermaßen charakterisiert:

Protein Konzentration: 1.71 mg/mL
Drug/mAb Ratio: 4.5

**Beispiel 18k**

In analoger Weise wurde Intermediat R18 mit 5 mg anti-TWEAKR Antikörper TPP-2658 gekuppelt. Der erhaltene

ADC Batch wurde folgendermaßen charakterisiert:

Protein Konzentration: 1.69 mg/mL
Drug/mAb Ratio: 3.6

**Beispiel 19a**

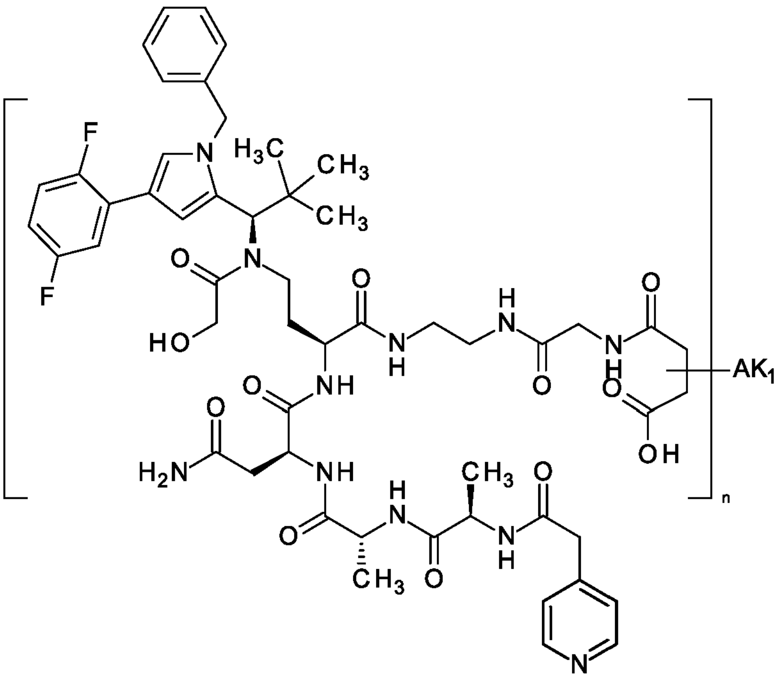

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat R19 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.88 mg/mL
Drug/mAb Ratio: 2.8

**Beispiel 19e**

In analoger Weise wurde Intermediat R19 mit 5 mg anti-HER2 Antikörper TPP-1015 gekuppelt. Der erhaltene ADC Batch wurde folgendermaßen charakterisiert:

Protein Konzentration: 1.7 mg/mL
Drug/mAb Ratio: 3.3

**Beispiel 19k**

In analoger Weise wurde Intermediat R19 mit 5 mg anti-TWEAKR Antikörper TPP-2658 gekuppelt. Der erhaltene ADC Batch wurde folgendermaßen charakterisiert:

Protein Konzentration: 2.35 mg/mL

Drug/mAb Ratio: 3.2

**Beispiel 20a**

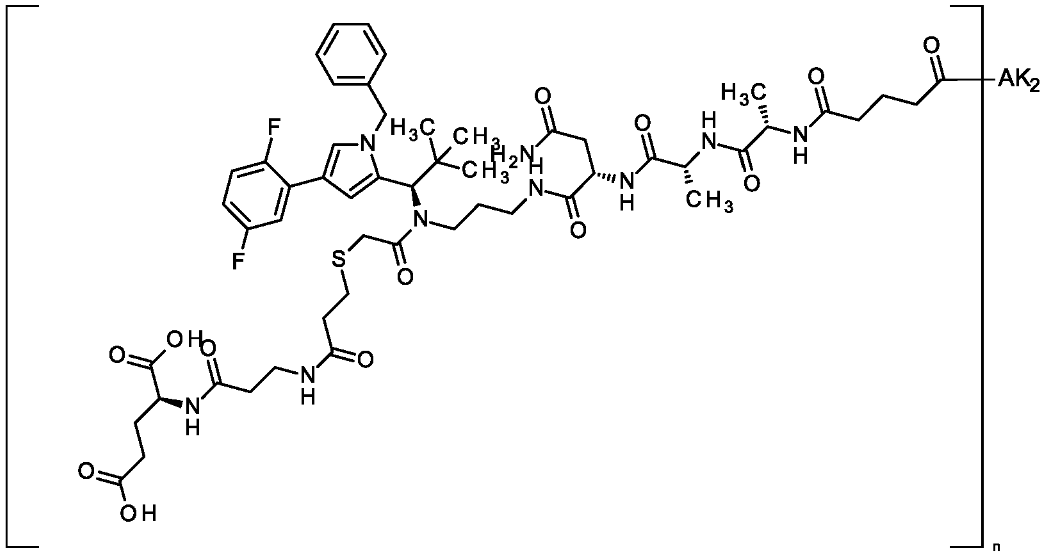

3 mg Cetuximab in 0.3 ml PBS (c=10 mg/mL) wurden unter Argon mit einer Lösung aus 5 Eq (0.268 mg) von Intermediat R20 gelöst in 30µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und mit PBS (pH7.2) rückverdünnt.

Protein Konzentration: 2.35 mg/mL
Drug/mAb Ratio: 5.3

**Beispiel 20e**

In analoger Weise wurde Intermediat R20 mit 3 mg anti-HER2 Antikörper TPP-1015 gekuppelt. Der erhaltene ADC Batch wurde folgendermaßen charakterisiert:

Protein Konzentration: 1.94 mg/mL
Drug/mAb Ratio: 7.1

**Beispiel 20h1**

In analoger Weise wurde Intermediat R20 mit 3 mg anti-B7H3 Antikörper TPP-8382 gekuppelt. Der erhaltene ADC Batch wurde folgendermaßen charakterisiert:

Protein Konzentration: 2.12 mg/mL
Drug/mAb Ratio: 5.9

**Beispiel 20k**

In analoger Weise wurde Intermediat R20 mit 3 mg anti-B7H3 TPP-8382 gekuppelt. Der erhaltene ADC Batch wurde folgendermaßen charakterisiert:

Protein Konzentration: 2.21 mg/mL
Drug/mAb Ratio: 6.8

**Beispiel 21a**

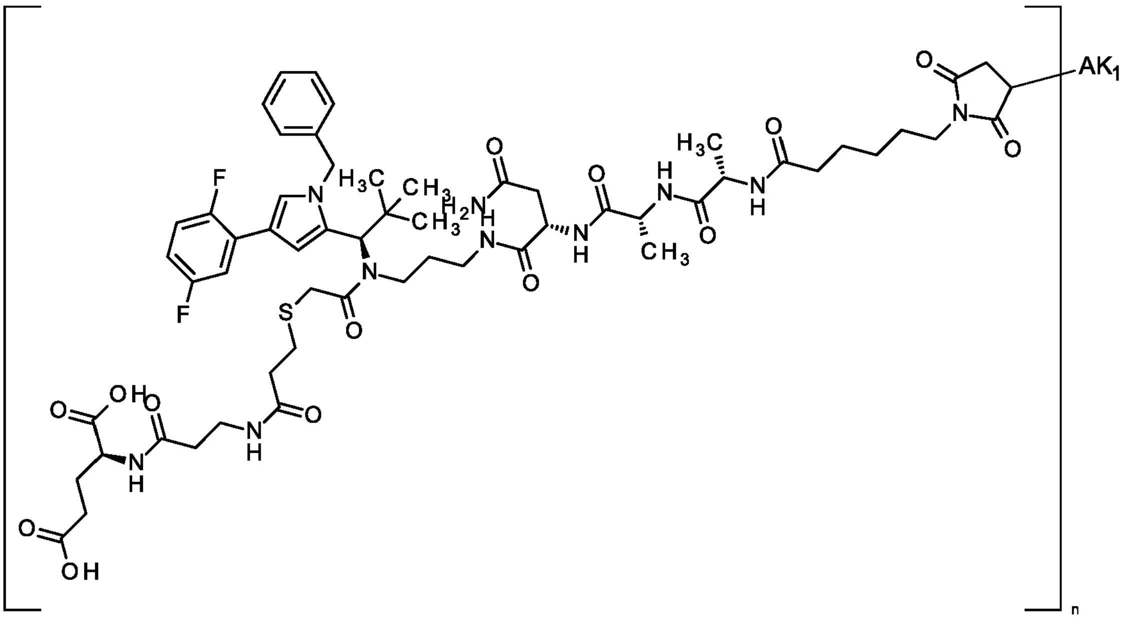

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.31 mg (0.00023 mmol) von Intermediat R21 gelöst in 50 µl DMSO zugegeben. Nach weiteren 90 min Rühren bei RT wurde der Ansatz mit PBS-Puffer auf ein Volumen von 2.5 mL verdünnt, über eine Sephadex-Säule gereinigt und dann durch Ultrazentrifugation aufkonzentriert und mit PBS (pH7.2) rückverdünnt. Der erhaltene ADC Batch wurde folgendermaßen charakterisiert:

Protein Konzentration: 2.17 mg/mL
Drug/mAb Ratio: 5.6

**Beispiel 21e**

In analoger Weise wurde Intermediat R21 mit 5 mg anti-HER2 Antikörper TPP-1015 gekuppelt. Der erhaltene ADC Batch wurde folgendermaßen charakterisiert:

Protein Konzentration: 1.99 mg/mL
Drug/mAb Ratio: 3.8

**Beispiel 21h1**

In analoger Weise wurde Intermediat R21 mit 5 mg anti-B7H3 TPP-8382 gekuppelt. Der erhaltene ADC Batch wurde folgendermaßen charakterisiert:

Protein Konzentration: 2.08 mg/mL
Drug/mAb Ratio: 4.6

**Beispiel 21k**

In analoger Weise wurde Intermediat R21 mit 5 mg anti-TWEAKR Antikörper TPP-2658 gekuppelt. Der erhaltene ADC Batch wurde folgendermaßen charakterisiert:

Protein Konzentration: 2.13 mg/mL
Drug/mAb Ratio: 4.4

**Beispiel 22a**

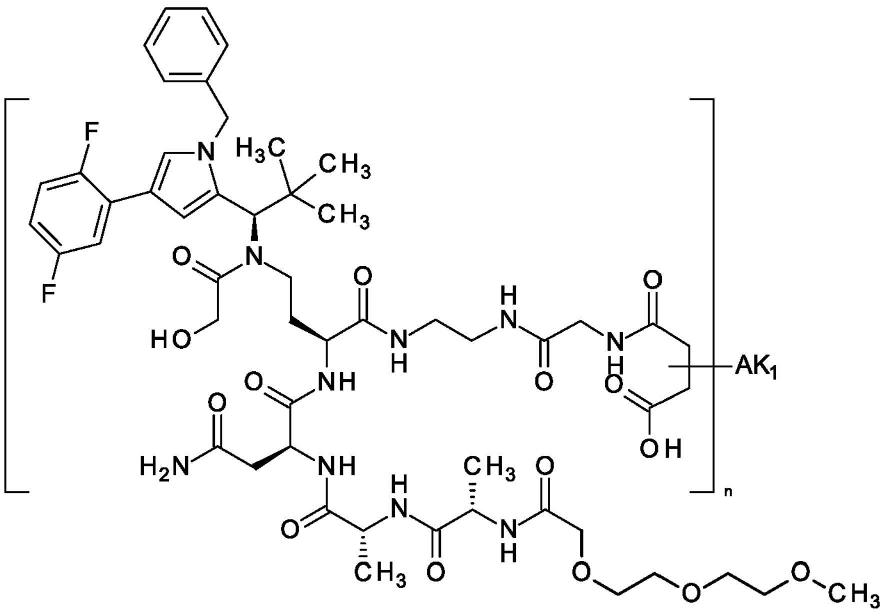

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R22 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.99 mg/mL
Drug/mAb Ratio: 2.8

**Beispiel 22e**

In analoger Weise wurde Intermediat R22 mit 5 mg anti-HER2 Antikörper TPP-1015gekuppelt. Der erhaltene ADC Batch wurde folgendermaßen charakterisiert:

Protein Konzentration: 1.9 mg/mL
Drug/mAb Ratio: 3.3

**Beispiel 22h1**

In Analogie zu Beispiel 9h1 wurde Intermediat R22 mit 30 mg anti-B7H3 Antikörper TPP-8382 in 3 ml PBS gekuppelt. Der erhaltene ADC Batch wurde folgendermaßen charakterisiert:

Protein Konzentration: 8.74 mg/mL
Drug/mAb Ratio: 3.9

**Beispiel 22h2**

In analoger Weise wurde Intermediat R22 mit 5 mg anti-B7H3 Antikörper TPP-8567 gekuppelt. Der erhaltene ADC Batch wurde folgendermaßen charakterisiert:

Protein Konzentration: 2.11 mg/mL
Drug/mAb Ratio: 4.7

**Beispiel 22k**

In analoger Weise wurde Intermediat R22 mit 5 mg anti-TWEAKR Antikörper TPP-2658 gekuppelt. Der erhaltene ADC Batch wurde folgendermaßen charakterisiert:

Protein Konzentration: 1.79 mg/mL
Drug/mAb Ratio: 3.3

**Beispiel 23a**

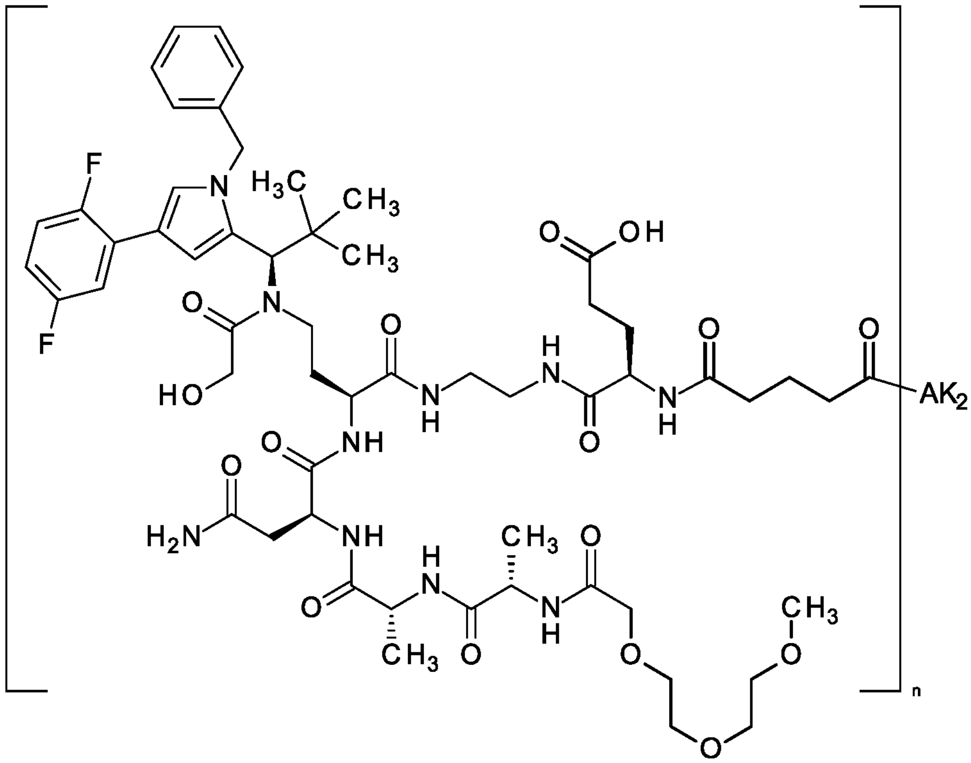

5 mg Cetuximab in 0.55 ml PBS (c=9.1 mg/mL) wurden unter Argon mit einer Lösung aus 5 Eq (0.24 mg) von Intermediat R23 gelöst in 50µL DMSO versetzt. Nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.13 mg/mL
Drug/mAb Ratio: 6.1

**Beispiel 23e**

In analoger Weise wurde Intermediat R23 mit 5 mg anti-HER2 Antikörper TPP-1015 gekuppelt. Der erhaltene ADC Batch wurde folgendermaßen charakterisiert:

Protein Konzentration: 2.02 mg/mL
Drug/mAb Ratio: 7.6

**Beispiel 23h1**

In Analogie zu Beispiel 15h1 wurde Intermediat R23 mit 30 mg anti-B7H3 Antikörper TPP-8382 gekuppelt. Der erhaltene ADC Batch wurde folgendermaßen charakterisiert:

Protein Konzentration: 9.97 mg/mL
Drug/mAb Ratio: 6.4

**Beispiel 23k**

In analoger Weise wurde Intermediat R23 mit 5 mg anti-TWEAKR Antikörper TPP-2658 gekuppelt. Der erhaltene ADC Batch wurde folgendermaßen charakterisiert:

Protein Konzentration: 2.24 mg/mL
Drug/mAb Ratio: 7.3

**Beispiel 24a**

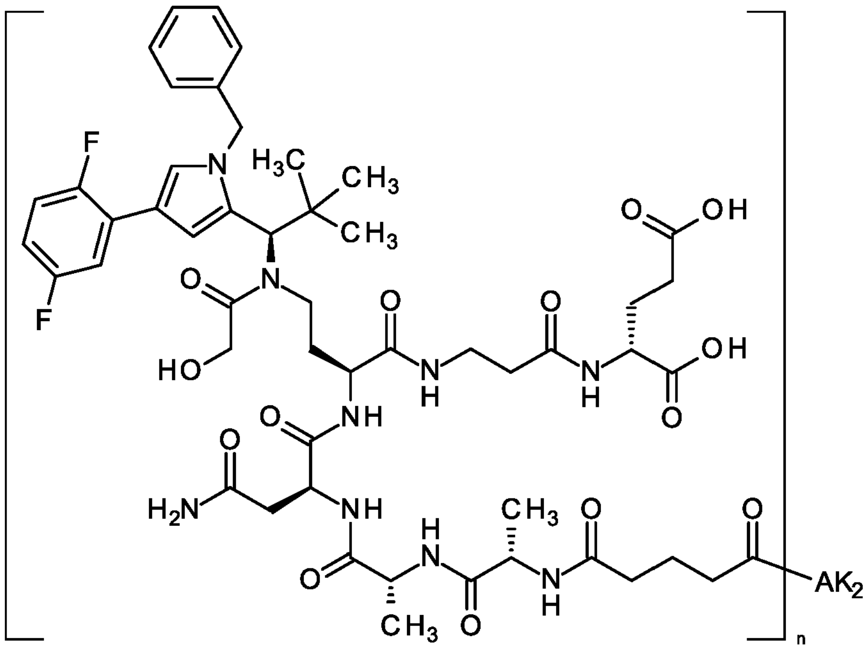

5 mg Cetuximab in 0.458 ml PBS (c=10.9 mg/mL) wurden unter Argon mit einer Lösung aus 5 Eq (0.24 mg) von Intermediat R24 gelöst in 50µL DMSO versetzt. Nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.2 mg/mL
Drug/mAb Ratio: 4.7

**Beispiel 24e**

In analoger Weise wurde Intermediat R24 mit 5 mg anti-HER2 Antikörper TPP-1015 gekuppelt. Der erhaltene ADC Batch wurde folgendermaßen charakterisiert:

Protein Konzentration: 1.89 mg/mL
Drug/mAb Ratio: 5.8

**Beispiel 24k**

In analoger Weise wurde Intermediat R24 mit 5 mg anti-TWEAKR Antikörper TPP-2658 gekuppelt. Der erhaltene ADC Batch wurde folgendermaßen charakterisiert:

Protein Konzentration: 2.21 mg/mL
Drug/mAb Ratio: 5.7

**Beispiel 24l1**

Zu einer Lösung von 30 mg vom anti-TWEAKR Antikörper TPP-7007 in 2.52 ml PBS (c=11.9 mg/mL) wurden unter Argon 1.2 mg (0.001 mmol) von Intermediat R24 gelöst in 100 µl DMSO zugegeben. Nach 60 min Rühren bei RT wurde nochmals die gleiche Menge hinzugefügt und der Ansatz eine weitere Stunde bei RT gerührt. Anschließend wurde der Ansatz mit PBS Puffer (pH7.2) auf 5 mL verdünnt, über eine Sephadex-Säule gereinigt und dann durch Ultrazentrifugation aufkonzentriert, rückverdünnt mit PBS (pH7.2) und erneut aufkonzentriert und steril filtriert. Der erhaltene ADC Batch wurde folgendermaßen charakterisiert:

Protein Konzentration: 10.5 mg/mL
Drug/mAb Ratio: 3.3

**Beispiel 24l3**

In analoger Weise wurde Intermediat R24 mit 30 mg anti-TWEAKR Antikörper TPP-10336 in 2.61 ml PBS (c=11.5 mg/mL) gekuppelt. Der erhaltene ADC Batch wurde folgendermaßen charakterisiert:

Protein Konzentration: 10.46 mg/mL
Drug/mAb Ratio: 5.0

**Beispiel 24l4**

In analoger Weise wurde Intermediat R24 mit 30 mg anti-TWEAKR Antikörper TPP-10337 in 2.78 ml PBS (c=10.8 mg/mL) gekuppelt. Der erhaltene ADC Batch wurde folgendermaßen charakterisiert:

Protein Konzentration: 7.85 mg/mL
Drug/mAb Ratio: 3.7

**Beispiel 25a**

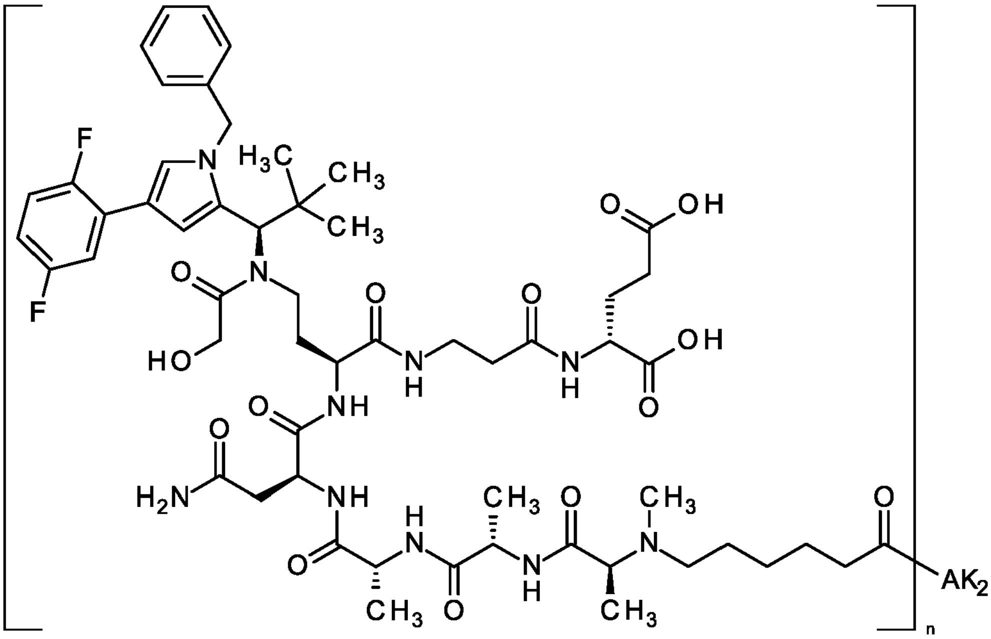

5 mg Cetuximab in 0.55 ml PBS (c=9.1 mg/mL) wurden unter Argon mit einer Lösung aus 5 Eq (0.23 mg) von Intermediat R25 gelöst in 50μL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).

Protein Konzentration: 2.07 mg/mL
Drug/mAb Ratio: 4.5

**Beispiel 25e**

In analoger Weise wurde Intermediat R25 mit 5 mg anti-HER2 Antikörper TPP-1015 gekuppelt. Der erhaltene ADC Batch wurde folgendermaßen charakterisiert:

Protein Konzentration: 1.42 mg/mL
Drug/mAb Ratio: 5.5

**Beispiel 25h1**

In analoger Weise wurde Intermediat R25 mit 5 mg anti-B7H3 Antikörper TPP-8382 gekuppelt. Der erhaltene ADC Batch wurde folgendermaßen charakterisiert:

Protein Konzentration: 1.82 mg/mL
Drug/mAb Ratio: 5.7

**Beispiel 25k**

In analoger Weise wurde Intermediat R25 mit 5 mg anti-TWEAKR Antikörper TPP-2658 gekuppelt. Der erhaltene ADC Batch wurde folgendermaßen charakterisiert:

Protein Konzentration: 2.16 mg/mL
Drug/mAb Ratio: 5.6

**Beispiel 26a**

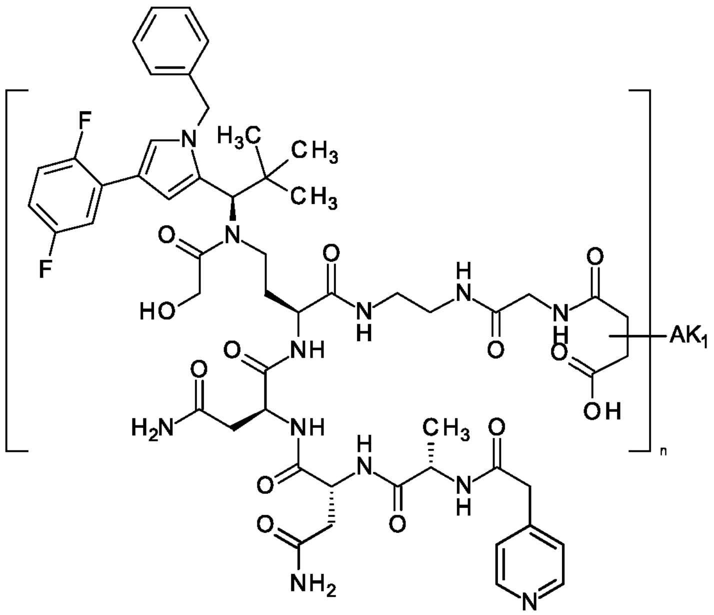

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R26 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.94 mg/mL
Drug/mAb Ratio: 2.8

**Beispiel 26e**

In analoger Weise wurde Intermediat R26 mit 5 mg anti-HER2 Antikörper TPP-1015 gekuppelt. Der erhaltene ADC Batch wurde folgendermaßen charakterisiert:

Protein Konzentration: 1.86 mg/mL
Drug/mAb Ratio: 3.5

**Beispiel 26h1**

In analoger Weise wurde Intermediat R26 mit 5 mg anti-B7H3 Antikörper TPP-8382 gekuppelt. Der erhaltene ADC Batch wurde folgendermaßen charakterisiert:

Protein Konzentration: 2.0 mg/mL
Drug/mAb Ratio: 4.0

**Beispiel 26k**

In analoger Weise wurde Intermediat R26 mit 5 mg anti-TWEAKR Antikörper TPP-2658 gekuppelt. Der erhaltene ADC Batch wurde folgendermaßen charakterisiert:

Protein Konzentration: 1.75 mg/mL
Drug/mAb Ratio: 3.7

**Beispiel 27a**

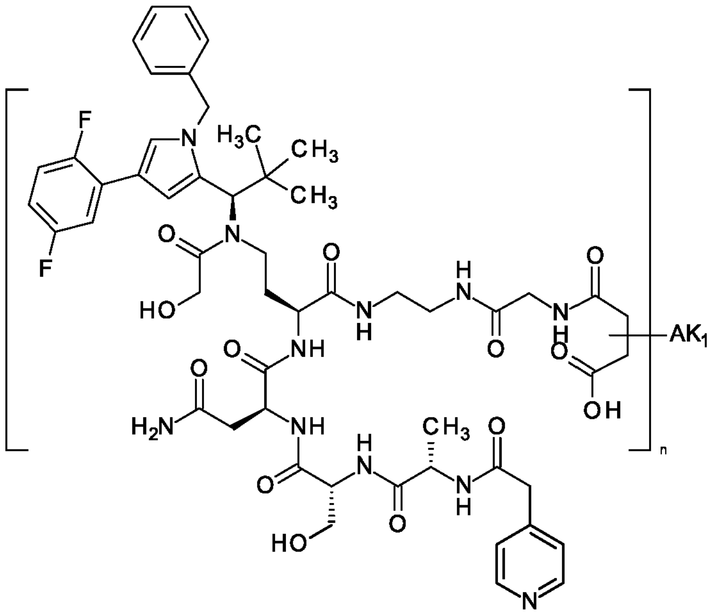

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat R27 gelöst in 50 μl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.65 mg/mL
Drug/mAb Ratio: 3.1

**Beispiel 27e**

In analoger Weise wurde Intermediat R27 mit 5 mg anti-HER2 Antikörper TPP-1015 gekuppelt. Der erhaltene ADC Batch wurde folgendermaßen charakterisiert:

Protein Konzentration: 1.67 mg/mL
Drug/mAb Ratio: 4.0

**Beispiel 27h1**

In analoger Weise wurde Intermediat R27 mit 5 mg anti-B7H3 Antikörper TPP-8382 gekuppelt. Der erhaltene ADC Batch wurde folgendermaßen charakterisiert:

Protein Konzentration: 1.86 mg/mL
Drug/mAb Ratio: 3.9

**Beispiel 27k**

In analoger Weise wurde Intermediat R27 mit 5 mg anti-TWEAKR Antikörper TPP-2658 gekuppelt. Der erhaltene ADC Batch wurde folgendermaßen charakterisiert:

Protein Konzentration: 1.8 mg/mL
Drug/mAb Ratio: 4.4

**Beispiel 28a**

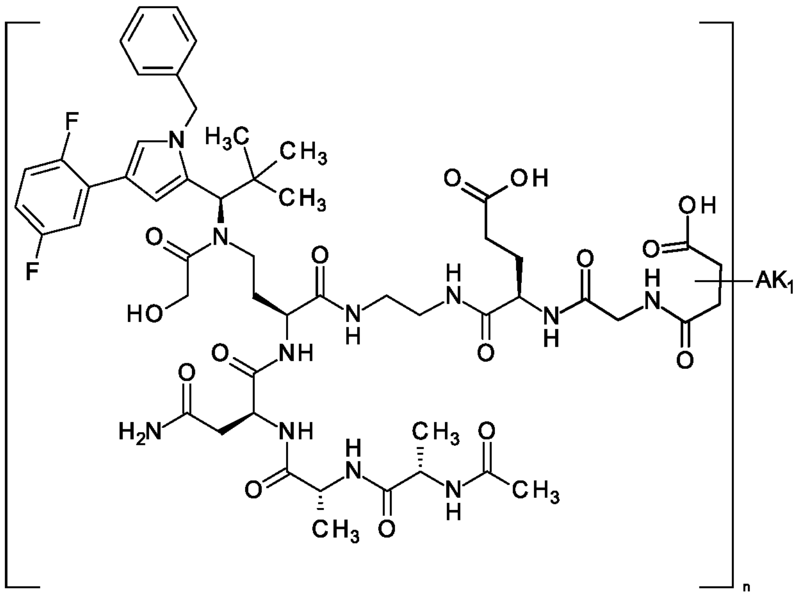

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R28 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.68 mg/mL
Drug/mAb Ratio: 3.0

**Beispiel 28e**

In analoger Weise wurde Intermediat R28 mit 5 mg anti-HER2 Antikörper TPP-1015 gekuppelt. Der erhaltene ADC Batch wurde folgendermaßen charakterisiert:

Protein Konzentration: 1.55 mg/mL
Drug/mAb Ratio: 3.6

**Beispiel 28h1**

In analoger Weise wurde Intermediat R26 mit 5 mg anti-B7H3 Antikörper TPP-8382 gekuppelt. Der erhaltene ADC Batch wurde folgendermaßen charakterisiert:

Protein Konzentration: 1.95 mg/mL
Drug/mAb Ratio: 3.4

**Beispiel 28l1**

30 mg vom anti-TWEAKR Antikörper TPP-7007 in 2.5 ml PBS (c=12 mg/mL) wurden unter Argon mit einer

Lösung aus 0.17 mg TCEP in 0.25 ml PBS-Puffer (pH 7.2) versetzt. Der Ansatz wurde 30min bei RT gerührt und anschließend wurden 1.57 mg (0.0014 mmol) von Intermediat R28 gelöst in 250 µl DMSO zugegeben. Nach weiteren 90 min Rühren bei RT wurde der Ansatz mit PBS-Puffer, der zuvor auf pH 8 eingestellt wurde, auf 5 ml verdünnt, geteilt und dann jeweils über eine mit PBS-Puffer pH 8 equillibrierte PD 10-Säule (Sephadex® G-25, GE Healthcare) gegeben und mit PBS-Puffer pH 8 eluiert. Die Eluate wurden wieder vereinigt, mit PBS-Puffer pH 8 auf 7.5 ml verdünnt und über Nacht bei RT unter Argon gerührt. Diese Lösung wurde dann über mit PBS-Puffer pH7.2 equillibrierte PD 10-Säulen (Sephadex® G-25, GE Healthcare) gegeben und mit PBS-Puffer pH7.2 eluiert. Anschließend wurde durch Ultrazentrifugation aufkonzentriert und rückverdünnt mit PBS-Puffer (pH 7.2) und erneut aufkonzentriert und steril filtriert. Der erhaltene ADC Batch wurde folgendermaßen charakterisiert:

Protein Konzentration: 8.2 mg/mL
Drug/mAb Ratio: 3.1

**Beispiel 29a**

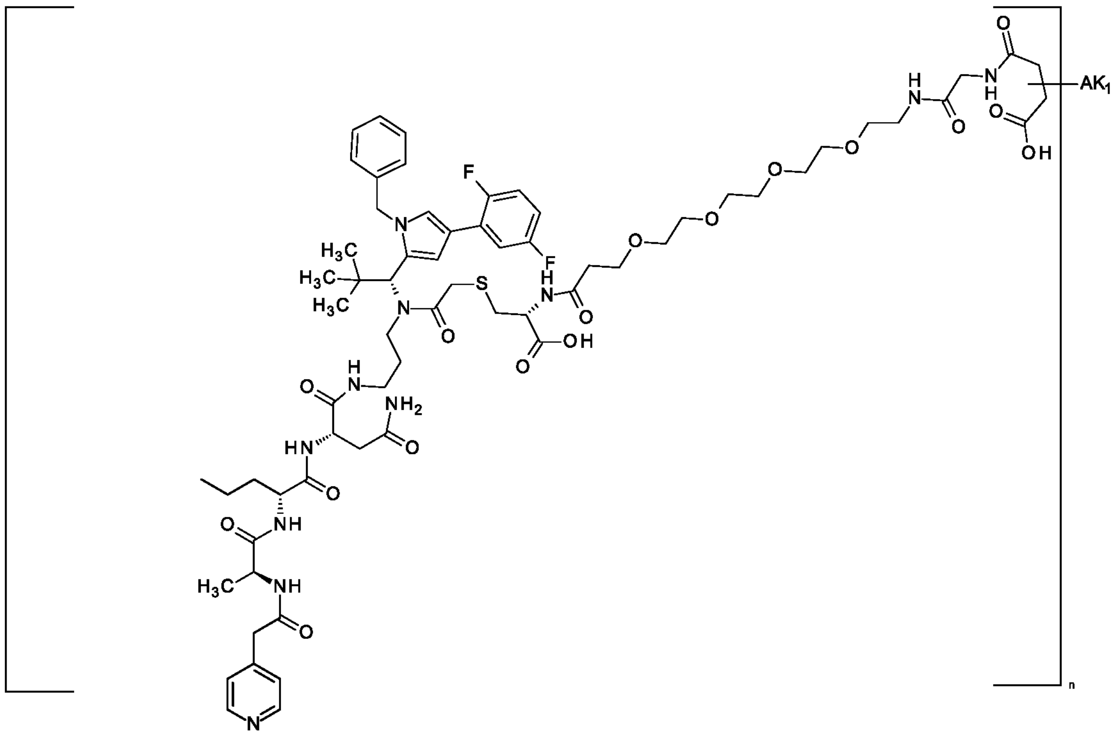

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.34 mg (0.00023 mmol) von Intermediat R29 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und mit PBS-Puffer (pH 7.2) rückverdünnt. Der erhaltene ADC Batch wurde folgendermaßen charakterisiert:

Protein Konzentration: 1.66 mg/mL
Drug/mAb Ratio: 3.6

**Beispiel 29e**

In analoger Weise wurde Intermediat R29 mit 5 mg anti-HER2 Antikörper TPP-1015gekuppelt. Der erhaltene ADC Batch wurde folgendermaßen charakterisiert:

Protein Konzentration: 1.24 mg/mL

Drug/mAb Ratio: 3.8

**Beispiel 29h1**

In analoger Weise wurde Intermediat R29 mit 5 mg anti-B7H3 Antikörper TPP-8382 gekuppelt. Der erhaltene ADC Batch wurde folgendermaßen charakterisiert:

Protein Konzentration: 1.54 mg/mL
Drug/mAb Ratio: 2.6

**Beispiel 29k**

In analoger Weise wurde Intermediat R29 mit 5 mg anti-TWEAKR Antikörper TPP-2658 gekuppelt. Der erhaltene ADC Batch wurde folgendermaßen charakterisiert:

Protein Konzentration: 1.63 mg/mL
Drug/mAb Ratio: 3.8

**Beispiel 30a**

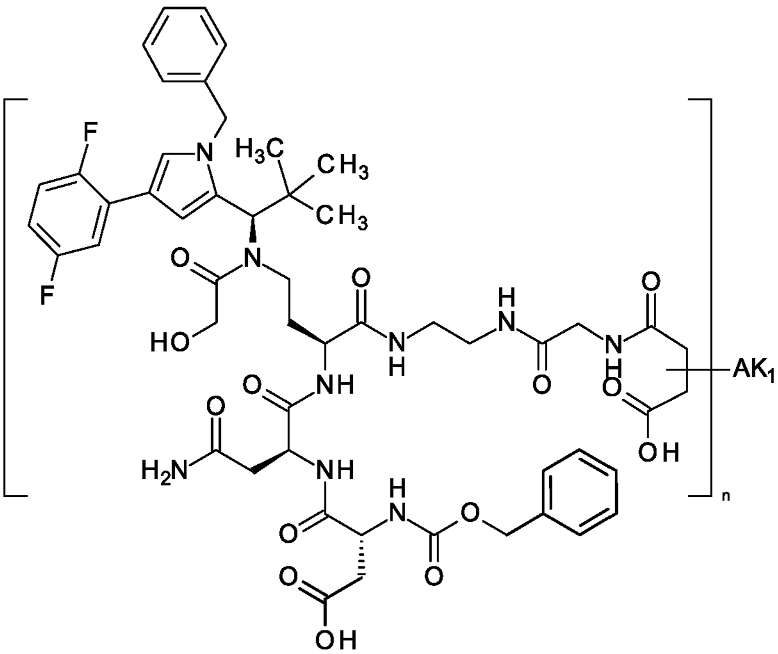

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R30 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.74 mg/mL
Drug/mAb Ratio: 3.0

**Beispiel 30e**

In analoger Weise wurde Intermediat R30 mit 5 mg anti-HER2 Antikörper TPP-1015 gekuppelt. Der erhaltene ADC Batch wurde folgendermaßen charakterisiert:

Protein Konzentration: 1.82 mg/mL
Drug/mAb Ratio: 3.3

**Beispiel 30k**

In analoger Weise wurde Intermediat R30 mit 5 mg anti-TWEAKR Antikörper TPP-2658 gekuppelt. Der erhaltene ADC Batch wurde folgendermaßen charakterisiert:

Protein Konzentration: 1.7 mg/mL
Drug/mAb Ratio: 3.0

**Beispiel 31a**

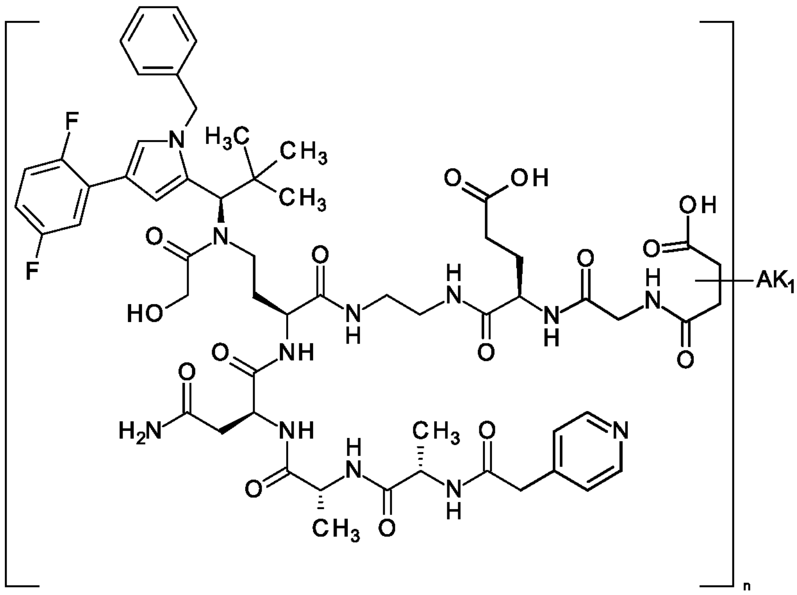

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat R31 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 2.4 mg/mL
Drug/mAb Ratio: 2.8

**Beispiel 31e**

In analoger Weise wurde Intermediat R31 mit 5 mg anti-HER2 Antikörper TPP-1015 gekuppelt. Der erhaltene ADC Batch wurde folgendermaßen charakterisiert:

Protein Konzentration: 1.76 mg/mL

Drug/mAb Ratio: 3.1

**Beispiel 31h1**

In Analogie zu Beispiel 9h1 wurde Intermediat R31 mit 20 mg anti-B7H3 Antikörper TPP-8382 in 2 ml PBS gekuppelt. Der erhaltene ADC Batch wurde folgendermaßen charakterisiert:

Protein Konzentration: 9.45 mg/mL
Drug/mAb Ratio: 3.3

**Beispiel 31l1**

30 mg vom anti-TWEAKR Antikörper TPP-7007 in 3.4 ml PBS (c=8.8 mg/mL) wurden unter Argon mit einer Lösung aus 0.17 mg TCEP in 0.3 ml PBS-Puffer (pH 7.2) versetzt. Der Ansatz wurde 30min bei RT gerührt und anschließend wurden 1.68 mg (0.0014 mmol) von Intermediat R31 gelöst in 250 µl DMSO zugegeben. Nach weiteren 90 min Rühren bei RT wurde der Ansatz mit PBS-Puffer, der zuvor auf pH 8 eingestellt wurde, auf 5 ml verdünnt, geteilt und dann jeweils über eine mit PBS-Puffer pH 8 equillibrierte PD 10-Säule (Sephadex® G-25, GE Healthcare) gegeben und mit PBS-Puffer pH 8 eluiert. Die Eluate wurden wieder vereinigt, mit PBS-Puffer pH 8 auf 7.5 ml verdünnt und über Nacht bei RT unter Argon gerührt. Diese Lösung wurde dann über mit PBS-Puffer pH7.2 equillibrierte PD 10-Säulen (Sephadex® G-25, GE Healthcare) gegeben und mit PBS-Puffer pH7.2 eluiert. Anschließend wurde durch Ultrazentrifugation aufkonzentriert und rückverdünnt mit PBS-Puffer (pH 7.2) und erneut aufkonzentriert und steril filtriert. Der erhaltene ADC Batch wurde folgendermaßen charakterisiert:

Protein Konzentration: 8.06 mg/mL
Drug/mAb Ratio: 4.1

**Beispiel 32a**

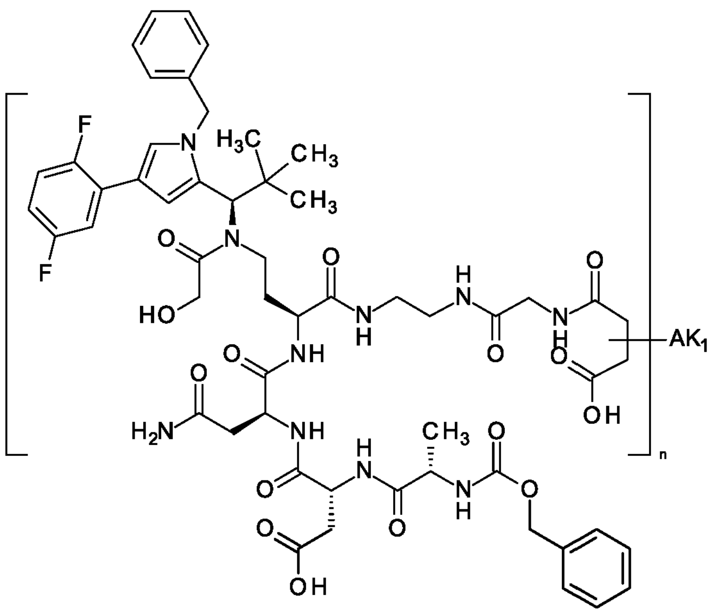

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.29 mg (0.00023 mmol) von Intermediat R32 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2).

Der erhaltene ADC Batch wurde folgendermaßen charakterisiert:

Protein Konzentration: 1.84 mg/mL
Drug/mAb Ratio: 3.5

**Beispiel 32e**

In analoger Weise wurde Intermediat R32 mit 5 mg anti-HER2 Antikörper TPP-1015 gekuppelt. Der erhaltene ADC Batch wurde folgendermaßen charakterisiert:

Protein Konzentration: 1.82 mg/mL
Drug/mAb Ratio: 3.9

**Beispiel 32k**

In analoger Weise wurde Intermediat R32 mit 5 mg anti-TWEAKR Antikörper TPP-2658 gekuppelt. Der erhaltene ADC Batch wurde folgendermaßen charakterisiert:

Protein Konzentration: 1.88 mg/mL
Drug/mAb Ratio: 3.7

**Beispiel 33a**

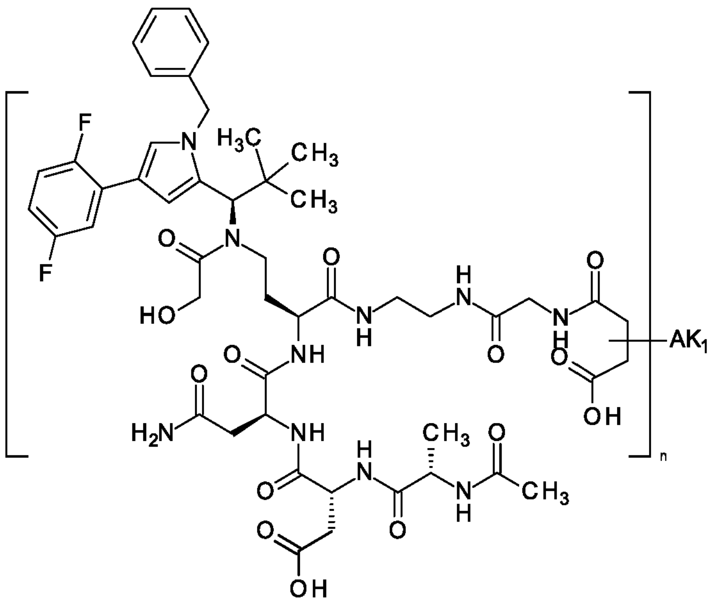

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.27 mg (0.00023 mmol) von Intermediat R33 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 2.13 mg/mL
Drug/mAb Ratio: 3.6

**Beispiel 33e**

In analoger Weise wurde Intermediat R33 mit 5 mg anti-HER2 Antikörper TPP-1015 gekuppelt. Der erhaltene ADC Batch wurde folgendermaßen charakterisiert:

Protein Konzentration: 1.93 mg/mL
Drug/mAb Ratio: 3.5

**Beispiel 33h1**

In analoger Weise wurde Intermediat R33 mit 5 mg anti-B7H3 Antikörper TPP-8382 gekuppelt. Der erhaltene ADC Batch wurde folgendermaßen charakterisiert:

Protein Konzentration: 1.81 mg/mL
Drug/mAb Ratio: 4.5

**Beispiel 33l1**

In analoger Weise wurde Intermediat R33 mit 5 mg anti-TWEAKR Antikörper TPP-7007 gekuppelt. Der erhaltene ADC Batch wurde folgendermaßen charakterisiert:

Protein Konzentration: 1.86 mg/mL
Drug/mAb Ratio: 3.8

**Beispiel 34a**

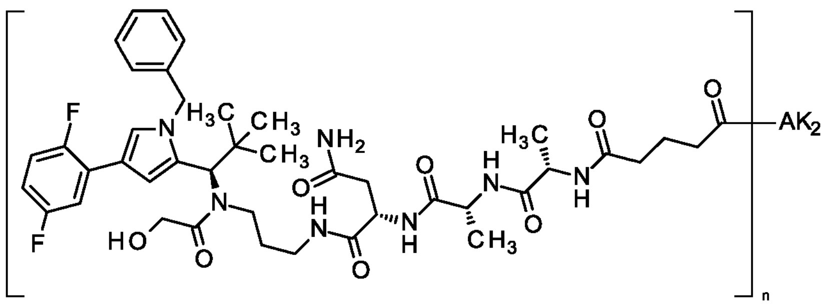

5 mg Cetuximab in 0.5 ml PBS (c=10 mg/mL) wurden unter Argon mit einer Lösung aus 5 Eq (0.15 mg) von Intermediat R34 gelöst in 50μL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und mit PBS (pH7.2) rückverdünnt.

Protein Konzentration: 1.84 mg/mL
Drug/mAb Ratio: 3.4

**Beispiel 34e**

In analoger Weise wurde Intermediat R34 mit 5 mg anti-HER2 Antikörper TPP-1015 gekuppelt. Der erhaltene ADC Batch wurde folgendermaßen charakterisiert:

Protein Konzentration: 1.75 mg/mL
Drug/mAb Ratio: 4.3

**Beispiel 34l1**

In analoger Weise wurde Intermediat R34 mit 5 mg anti-TWEAKR Antikörper TPP-7007 gekuppelt. Der erhaltene ADC Batch wurde folgendermaßen charakterisiert:

Protein Konzentration: 1.76 mg/mL
Drug/mAb Ratio: 3.3

**Beispiel 35a**

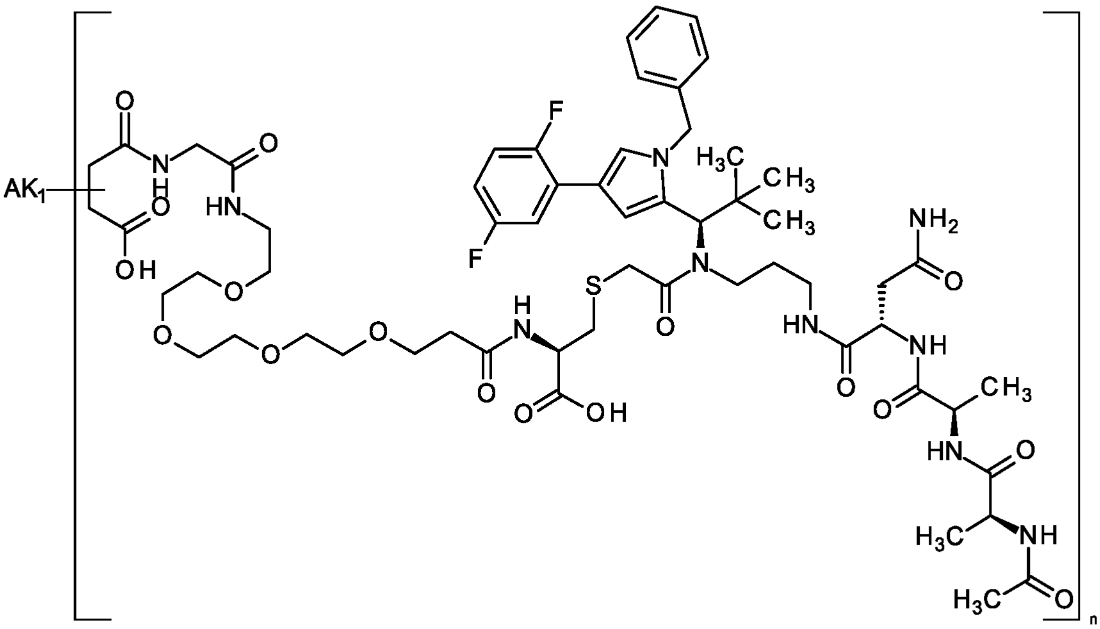

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30 min bei RT gerührt und anschließend wurden 0.32 mg (0.00023 mmol) von Intermediat R35 gelöst in 50 μl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.
Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.72 mg/mL
Drug/mAb Ratio: 4.3

**Beispiel 35e**

In analoger Weise wurde Intermediat R35 mit 5 mg anti-HER2 Antikörper TPP-1015 gekuppelt. Der erhaltene ADC Batch wurde folgendermaßen charakterisiert:

Protein Konzentration: 1.57 mg/mL
Drug/mAb Ratio: 4.4

**Beispiel 35k**

In analoger Weise wurde Intermediat R35 mit 5 mg anti-TWEAKR Antikörper TPP-2658 gekuppelt. Der erhaltene ADC Batch wurde folgendermaßen charakterisiert:

Protein Konzentration: 1.73 mg/mL
Drug/mAb Ratio: 4.3

**Beispiel 35l1**

Intermediat R35 wurde mit 25 mg anti-TWEAKR Antikörper TPP-7007 in 2.5 mL PBS in Analogie zu Beispiel **31l1** gekuppelt. Der erhaltene ADC Batch wurde folgendermaßen charakterisiert:

Protein Konzentration: 11.55 mg/mL
Drug/mAb Ratio: 3.5

**Beispiel 36a**

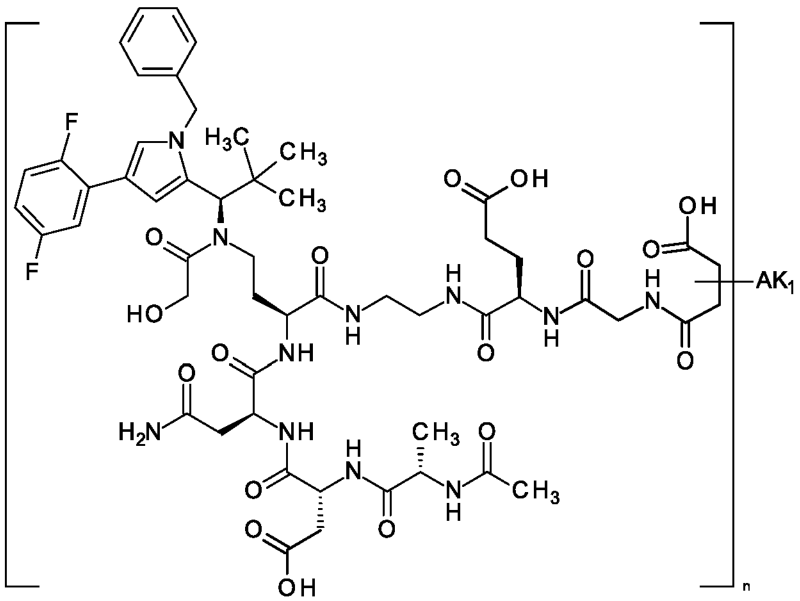

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.3 mg (0.00023 mmol) von Intermediat R36 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.95 mg/mL
Drug/mAb Ratio: 2.8

**Beispiel 36e**

In analoger Weise wurde Intermediat R36 mit 5 mg anti-HER2 Antikörper TPP-1015 gekuppelt. Der erhaltene ADC Batch wurde folgendermaßen charakterisiert:

Protein Konzentration: 1.42 mg/mL
Drug/mAb Ratio: 3.2

**Beispiel 36l1**

In analoger Weise wurde Intermediat R36 mit 5 mg anti-TWEAKR Antikörper TPP-7007 gekuppelt. Der erhaltene ADC Batch wurde folgendermaßen charakterisiert:

Protein Konzentration: 1.94 mg/mL
Drug/mAb Ratio: 2.7

**Beispiel 37a**

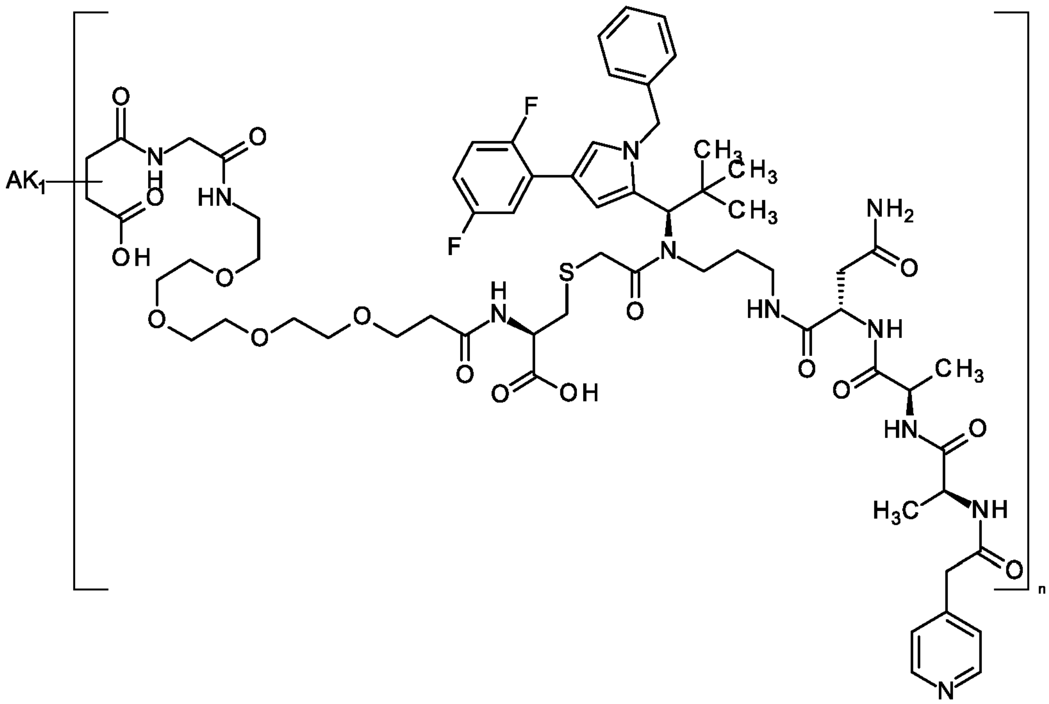

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30 min bei RT gerührt und anschließend wurden 0.32 mg (0.00023 mmol) von Intermediat R37 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.77 mg/mL
Drug/mAb Ratio: 4.2

**Beispiel 37e**

In analoger Weise wurde Intermediat R37 mit 5 mg anti-HER2 Antikörper TPP-1015 gekuppelt. Der erhaltene ADC Batch wurde folgendermaßen charakterisiert:

Protein Konzentration: 1.44 mg/mL
Drug/mAb Ratio: 4.6

**Beispiel 37k**

In analoger Weise wurde Intermediat R37 mit 5 mg anti-TWEAKR Antikörper TPP-2658 gekuppelt. Der erhaltene ADC Batch wurde folgendermaßen charakterisiert:

Protein Konzentration: 1.53 mg/mL
Drug/mAb Ratio: 4.0

**Beispiel 37l1**

Intermediat R37 wurde mit 25 mg anti-TWEAKR Antikörper TPP-7007 in 2.5 mL PBS in Analogie zu Beispiel **31l1** gekuppelt. Der erhaltene ADC Batch wurde folgendermaßen charakterisiert:

Protein Konzentration: 11.46 mg/mL
Drug/mAb Ratio: 3.3

**Beispiel 38a**

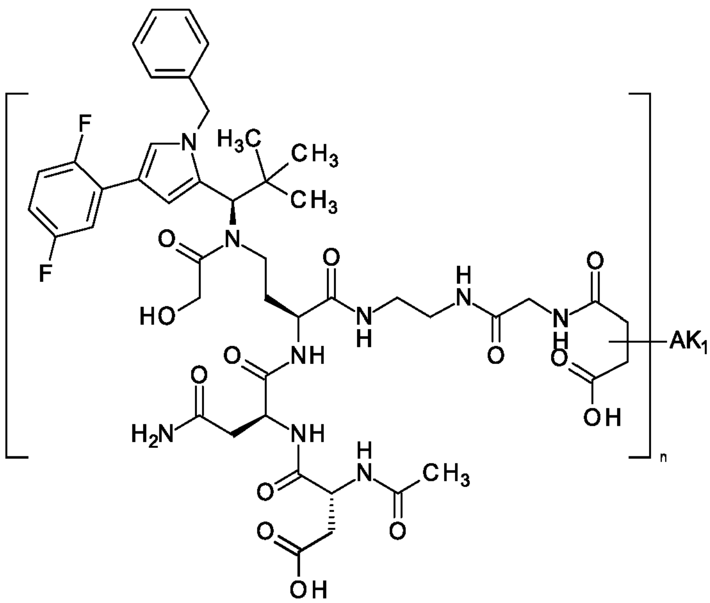

5 mg Cetuximab in 0.4 ml PBS (c=12.5 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat R38 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 2.02 mg/mL
Drug/mAb Ratio: 3.0

**Beispiel 38e**

In analoger Weise wurde Intermediat R38 mit 5 mg anti-HER2 Antikörper TPP-1015 gekuppelt. Der erhaltene ADC Batch wurde folgendermaßen charakterisiert:

Protein Konzentration: 1.77 mg/mL
Drug/mAb Ratio: 3.3

**Beispiel 38l1**

In analoger Weise wurde Intermediat R38 mit 5 mg anti-TWEAKR Antikörper TPP-7007 gekuppelt. Der erhaltene ADC Batch wurde folgendermaßen charakterisiert:

Protein Konzentration: 1.06 mg/mL
Drug/mAb Ratio: 4.0

**Beispiel 39a**

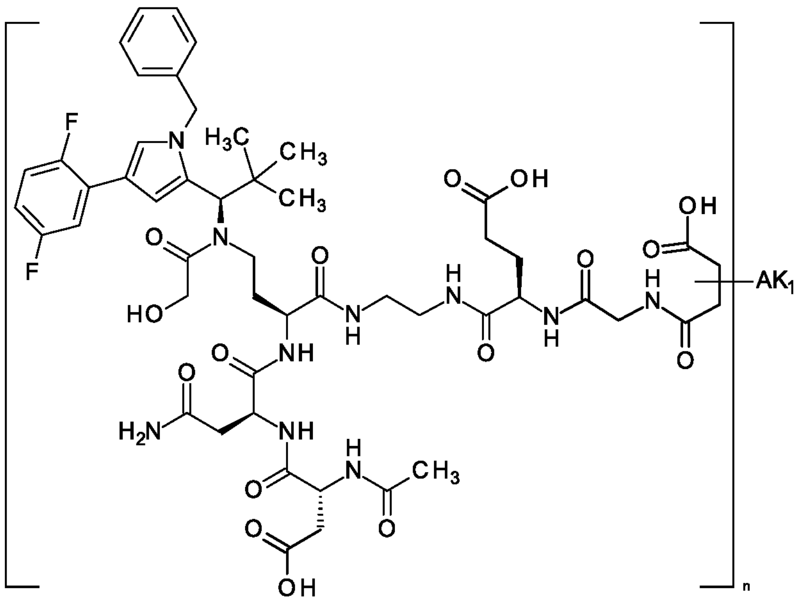

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.3 mg (0.00023 mmol) von Intermediat R39 gelöst in 50 µl DMSO zugegeben. Nach weiteren 90 min Rühren bei RT wurde der Ansatz PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 2.01 mg/mL
Drug/mAb Ratio: 3.6

**Beispiel 39e**

In analoger Weise wurde Intermediat R39 mit 5 mg anti-HER2 Antikörper TPP-1015 gekuppelt. Der erhaltene ADC Batch wurde folgendermaßen charakterisiert:

Protein Konzentration: 1.85 mg/mL
Drug/mAb Ratio: 3.5

**Beispiel 39l1**

In analoger Weise wurde Intermediat R39 mit 5 mg anti-TWEAKR Antikörper TPP-7007 gekuppelt. Der erhaltene ADC Batch wurde folgendermaßen charakterisiert:

Protein Konzentration: 1.84 mg/mL
Drug/mAb Ratio: 3.4

**Beispiel 40a**

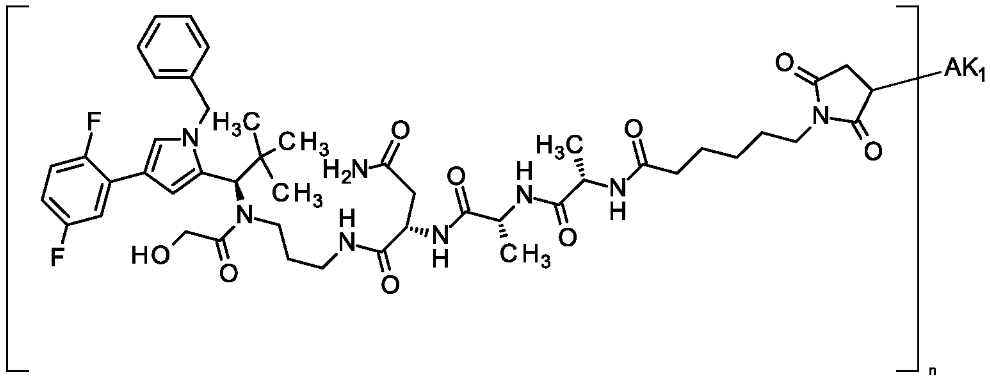

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.31 mg (0.00023 mmol) von Intermediat R40 gelöst in 50 µl DMSO zugegeben. Nach weiteren 90 min Rühren bei RT wurde der Ansatz mit PBS-Puffer auf ein Volumen von 2.5 mL verdünnt, über eine Sephadex-Säule gereinigt und dann durch Ultrazentrifugation aufkonzentriert und mit PBS (pH7.2) rückverdünnt. Der erhaltene ADC Batch wurde folgendermaßen charakterisiert:

Protein Konzentration: 1.85 mg/mL
Drug/mAb Ratio: 3.3

**Beispiel 40e**

In analoger Weise wurde Intermediat R40 mit 5 mg anti-HER2 Antikörper TPP-1015 gekuppelt. Der erhaltene ADC Batch wurde folgendermaßen charakterisiert:

Protein Konzentration: 2.05 mg/mL
Drug/mAb Ratio: 3.9

**Beispiel 40l1**

In analoger Weise wurde Intermediat R40 mit 5 mg anti-TWEAKR Antikörper TPP-7007 gekuppelt. Der erhaltene ADC Batch wurde folgendermaßen charakterisiert:

Protein Konzentration: 1.98 mg/mL
Drug/mAb Ratio: 3.9

**Beispiel 41a**

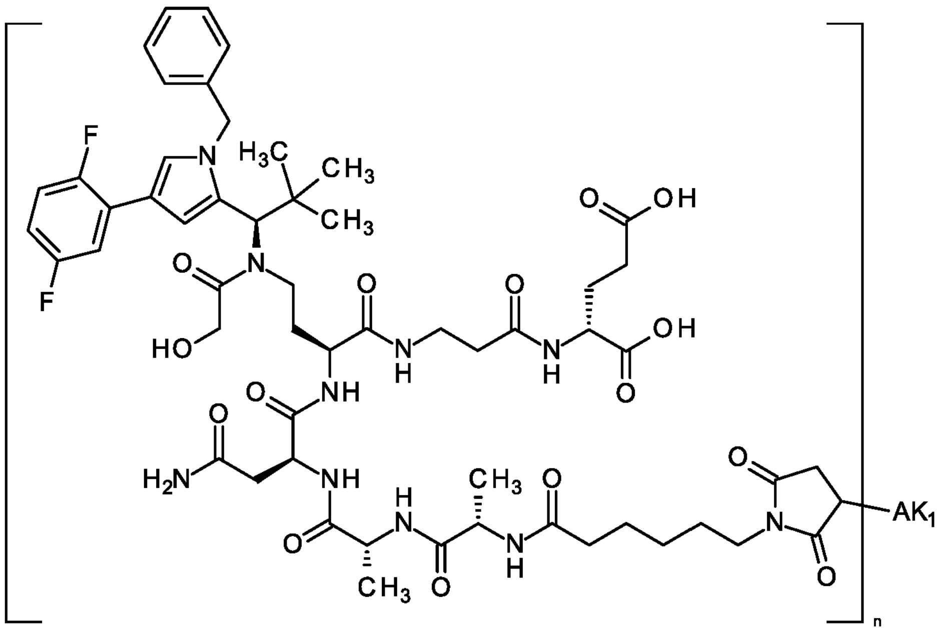

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.27 mg (0.00023 mmol) von Intermediat R41 gelöst in 50 µl DMSO zugegeben. Nach weiteren 90 min Rühren bei RT wurde der Ansatz mit PBS-Puffer auf ein Volumen von 2.5 mL verdünnt, über eine Sephadex-Säule gereinigt und dann durch Ultrazentrifugation aufkonzentriert und mit PBS (pH7.2) rückverdünnt. Der erhaltene ADC Batch wurde folgendermaßen charakterisiert:

Protein Konzentration: 2.02 mg/mL
Drug/mAb Ratio: 3.5

**Beispiel 41e**

In analoger Weise wurde Intermediat R41 mit 5 mg anti-HER2 Antikörper TPP-1015 gekuppelt. Der erhaltene ADC Batch wurde folgendermaßen charakterisiert:

Protein Konzentration: 2.08 mg/mL
Drug/mAb Ratio: 3.5

**Beispiel 41l1**

In analoger Weise wurde Intermediat R41 mit 5 mg anti-TWEAKR Antikörper TPP-7007 gekuppelt. Der erhaltene ADC Batch wurde folgendermaßen charakterisiert:

Protein Konzentration: 1.94 mg/mL
Drug/mAb Ratio: 3.9

**Beispiel 42a**

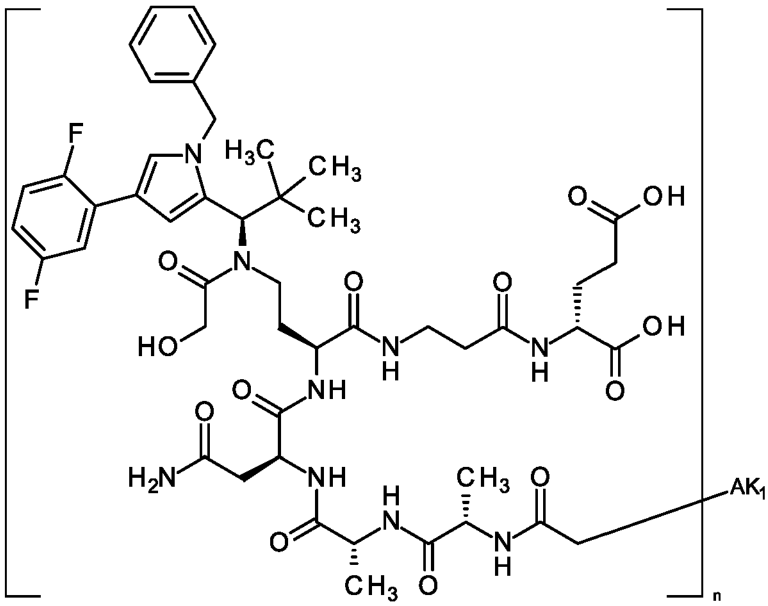

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.29 mg (0.00027 mmol) von Intermediat R42gelöst in 50 µl DMSO zugegeben. Nach weiteren 4 h Rühren bei RT wurde der Ansatz mit PBS-Puffer auf ein Volumen von 2.5 mL verdünnt, über eine Sephadex-Säule gereinigt und dann durch Ultrazentrifugation aufkonzentriert und mit PBS (pH7.2) rückverdünnt. Der erhaltene ADC Batch wurde folgendermaßen charakterisiert:

Protein Konzentration: 1.68 mg/mL
Drug/mAb Ratio: 2.6

**Beispiel 42e**

In analoger Weise wurde Intermediat R42 mit 5 mg anti-HER2 Antikörper TPP-1015 gekuppelt. Der erhaltene ADC Batch wurde folgendermaßen charakterisiert:

Protein Konzentration: 2.09 mg/mL
Drug/mAb Ratio: 3.0

**Beispiel 42l1**

In analoger Weise wurde Intermediat R42 mit 5 mg anti-TWEAKR Antikörper TPP-7007 gekuppelt. Der erhaltene ADC Batch wurde folgendermaßen charakterisiert:

Protein Konzentration: 1.7 mg/mL
Drug/mAb Ratio: 3.4

**Beispiel 43a**

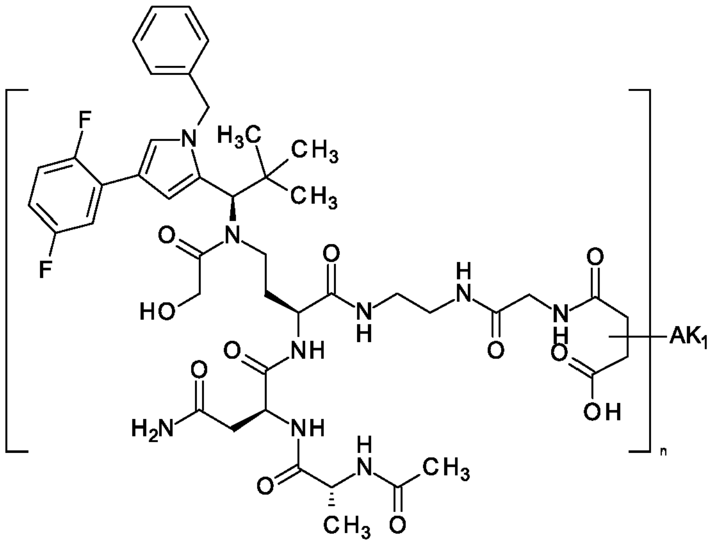

5 mg Cetuximab in 0.4 ml PBS (c=12.5 mg/mL) wurden unter Argon mit einer Lösung aus 0.029 mg TCEP in 0.05 ml PBS-Puffer versetzt. Der Ansatz wurde 30min bei RT gerührt und anschließend wurden 0.22 mg (0.00023 mmol) von Intermediat R43 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 2.08 mg/mL
Drug/mAb Ratio: 3.5

**Beispiel 43e**

In analoger Weise wurde Intermediat R43 mit 5 mg anti-HER2 Antikörper TPP-1015 gekuppelt. Der erhaltene ADC Batch wurde folgendermaßen charakterisiert:

Protein Konzentration: 1.9 mg/mL
Drug/mAb Ratio: 3.6

**Beispiel 43l1**

In analoger Weise wurde Intermediat R38 mit 5 mg anti-TWEAKR Antikörper TPP-7007 gekuppelt. Der erhaltene ADC Batch wurde folgendermaßen charakterisiert:

Protein Konzentration: 1.57 mg/mL
Drug/mAb Ratio: 3.1

**Beispiel 44e**

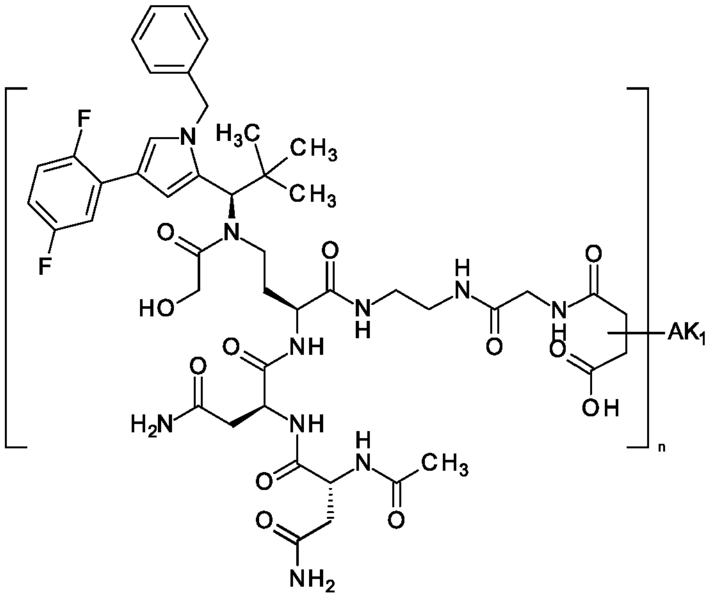

4 mg anti-HER2 Antikörper TPP-1015 in 0.4 ml PBS (c=10 mg/mL) wurden unter Argon mit einer Lösung aus 0.023 mg TCEP in 0.05 ml PBS-Puffer versetzt. Der Ansatz wurde 30min bei RT gerührt und anschließend wurden 0.18 mg (0.00019 mmol) von Intermediat R44 gelöst in 50 μl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.59 mg/mL
Drug/mAb Ratio: 3.4

**Beispiel 44l1**

In analoger Weise wurde Intermediat R45 mit 5 mg anti-TWEAKR Antikörper TPP-7007 gekuppelt. Der erhaltene ADC Batch wurde folgendermaßen charakterisiert:

Protein Konzentration: 1.57 mg/mL
Drug/mAb Ratio: 3.9

**Beispiel 45a**

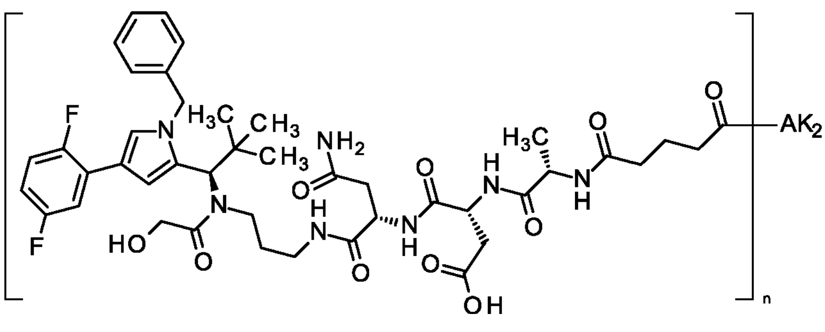

5 mg Cetuximab in 0.5 ml PBS (c=10 mg/mL) wurden unter Argon mit einer Lösung aus 5 Eq (0.16 mg) von Intermediat R45 gelöst in 50μL DMSO versetzt. Nach 1h Rühren bei RT wurde nochmals die gleiche Menge hinzugefügt

und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und mit PBS (pH7.2) rückverdünnt.

Protein Konzentration: 2.14 mg/mL
Drug/mAb Ratio: 4.6

**Beispiel 45e**

In analoger Weise wurde Intermediat R45 mit 5 mg anti-HER2 Antikörper TPP-1015 gekuppelt. Der erhaltene ADC Batch wurde folgendermaßen charakterisiert:

Protein Konzentration: 1.84 mg/mL
Drug/mAb Ratio: 4.9

**Beispiel 45l1**

In analoger Weise wurde Intermediat R45 mit 5 mg anti-TWEAKR Antikörper TPP-7007 gekuppelt. Der erhaltene ADC Batch wurde folgendermaßen charakterisiert:

Protein Konzentration: 2.01 mg/mL
Drug/mAb Ratio: 4.3

**Beispiel 46a**

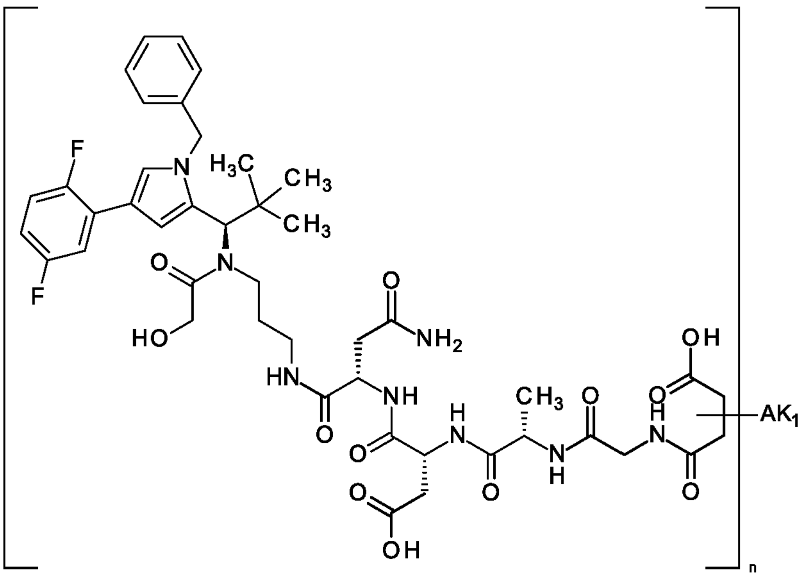

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.21 mg (0.00023 mmol) von Intermediat R46 gelöst in 50 μl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.83 mg/mL
Drug/mAb Ratio: 3.1

**Beispiel 46e**

In analoger Weise wurde Intermediat R46 mit 5 mg anti-HER2 Antikörper TPP-1015 gekuppelt. Der erhaltene ADC Batch wurde folgendermaßen charakterisiert:

Protein Konzentration: 1.71 mg/mL
Drug/mAb Ratio: 2.9

**Beispiel 46l1**

In analoger Weise wurde Intermediat R46 mit 5 mg anti-TWEAKR Antikörper TPP-7007 gekuppelt. Der erhaltene ADC Batch wurde folgendermaßen charakterisiert:

Protein Konzentration: 1.78 mg/mL
Drug/mAb Ratio: 3.4

**Beispiel 47a**

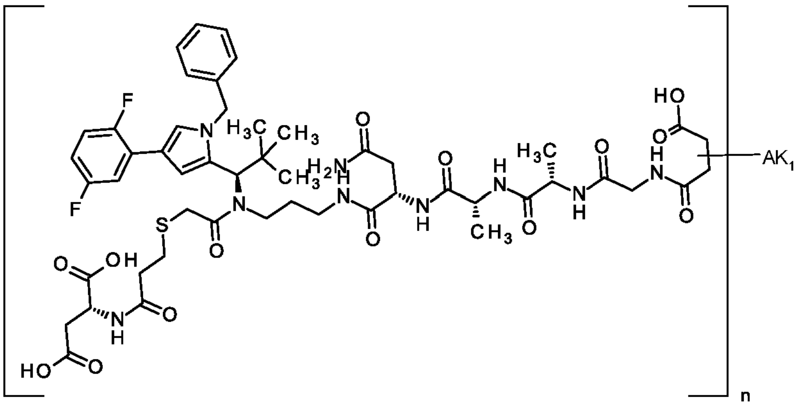

5 mg Cetuximab in 0.45 ml PBS (c=11 mg/mL) wurden unter Argon mit einer Lösung aus 0.029 mg TCEP in 0.05 ml PBS-Puffer versetzt. Der Ansatz wurde 30min bei RT gerührt und anschließend wurden 0.275 mg (0.00023 mmol) von Intermediat R47 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.
Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.77 mg/mL
Drug/mAb Ratio: 3.9

**Beispiel 47e**

In analoger Weise wurde Intermediat R47 mit 5 mg anti-HER2 Antikörper TPP-1015 gekuppelt. Der erhaltene ADC Batch wurde folgendermaßen charakterisiert:

Protein Konzentration: 1.43 mg/mL
Drug/mAb Ratio: 4.0

**Beispiel 47l1**

In analoger Weise wurde Intermediat R47 mit 5 mg anti-TWEAKR Antikörper TPP-7007 gekuppelt. Der erhaltene ADC Batch wurde folgendermaßen charakterisiert:

Protein Konzentration: 1.60 mg/mL
Drug/mAb Ratio: 4.1

**Beispiel 48a**

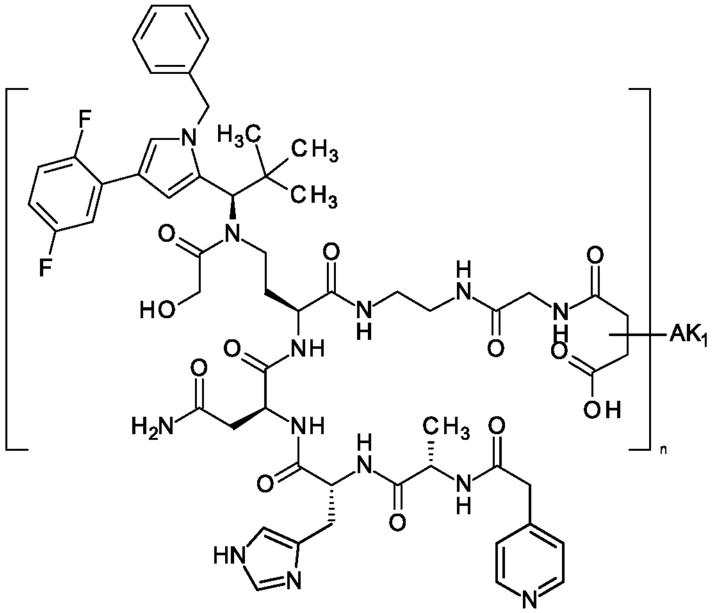

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.29 mg (0.00023 mmol) von Intermediat R48 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex® G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:

Protein Konzentration: 1.75 mg/mL
Drug/mAb Ratio: 4.1

**Beispiel 48e**

In analoger Weise wurde Intermediat R48 mit 5 mg anti-HER2 Antikörper TPP-1015 gekuppelt. Der erhaltene ADC Batch wurde folgendermaßen charakterisiert:

Protein Konzentration: 1.72 mg/mL
Drug/mAb Ratio: 4.7

**Beispiel 48l1**

In analoger Weise wurde Intermediat R48 mit 5 mg anti-TWEAKR Antikörper TPP-7007 gekuppelt. Der erhaltene ADC Batch wurde folgendermaßen charakterisiert:

Protein Konzentration: 1.71 mg/mL
Drug/mAb Ratio: 2.4

**Beispiel 49a**

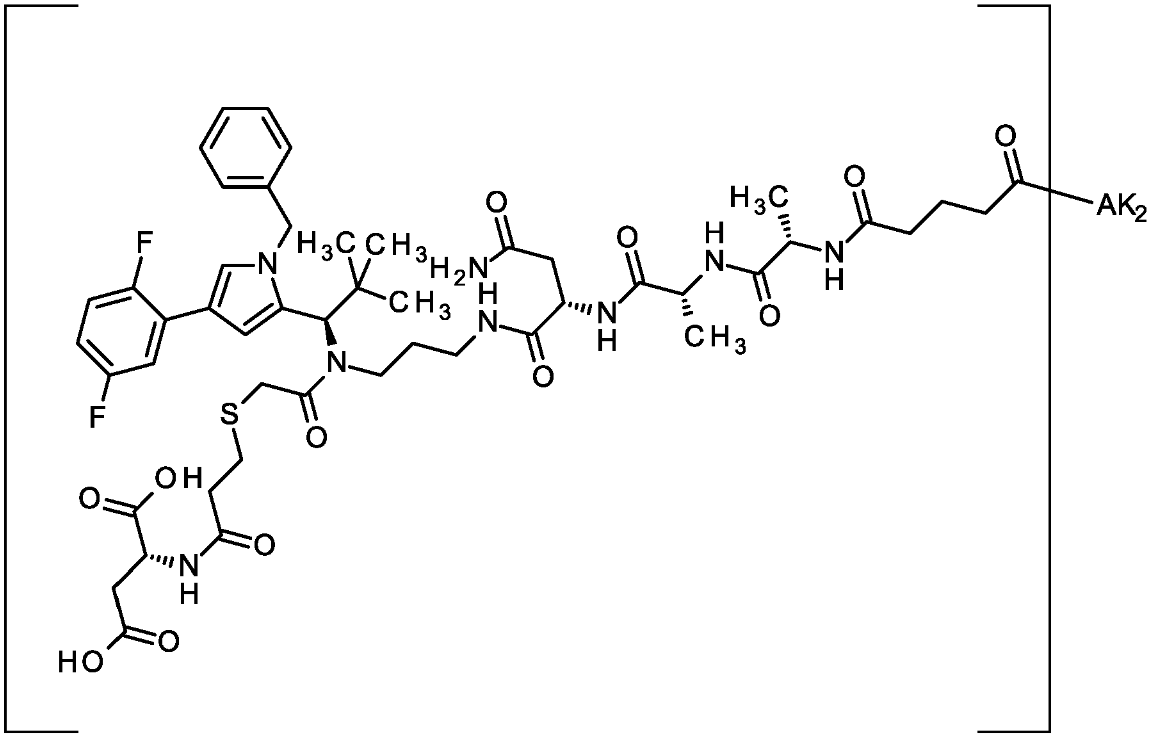

5 mg Cetuximab in 0.5 ml PBS (c=10 mg/mL) wurden unter Argon mit einer Lösung aus 5 eq (0.47 mg) von Intermediat R34 gelöst in 50μL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und mit PBS (pH7.2) auf 2.5 mL rückverdünnt.

Der erhaltene ADC Batch wurde folgendermaßen charakterisiert:

Protein Konzentration: 2.31 mg/mL

Drug/mAb Ratio: 5.6

**Beispiel 49e**

In analoger Weise wurde Intermediat R34 mit 5 mg anti-HER2 Antikörper TPP-1015 gekuppelt. Der erhaltene ADC Batch wurde folgendermaßen charakterisiert:

Protein Konzentration: 2.12 mg/mL
Drug/mAb Ratio: 7.1

**Beispiel 49l1**

In analoger Weise wurde Intermediat R34 mit 5 mg anti-TWEAKR Antikörper TPP-7007 gekuppelt. Der erhaltene ADC Batch wurde folgendermaßen charakterisiert:

Protein Konzentration: 2.25 mg/mL

Drug/mAb Ratio: 5.8

**Beispiel 50dt-2**

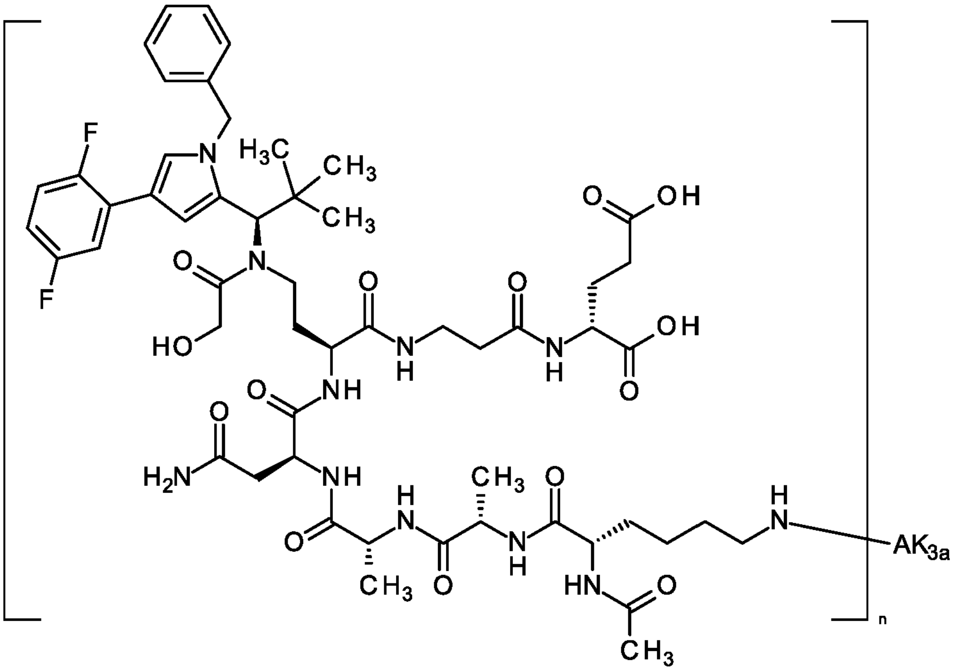

Zu einer Lösung von 5 mg des anti-TWEAKR Antikörpers TPP-2658 (entspricht TPP-2090-HC-N297A) in 540 μl DPBS pH 7.2 (c~10mg/mL) wurden 20 μL einer 10 mMol Lösung von Intermediat R50 in DMSO hinzugegeben. Nach 5 min Inkubation bei 37°C wurden 40 μL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.2 auf ein Volumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS. Schließlich wurden noch 0.005 μmol des b-Transglutaminase Blockers Zedira C100 in 12.5 μL DPBS zu der Lösung hinzugefügt. Die erhaltene ADC Lösung wurde folgendermaßen charakterisiert:

Protein Konzentration: 1.71 mg/mL
Drug/mAb Ratio: 1.9

**Beispiel 50dt-4**

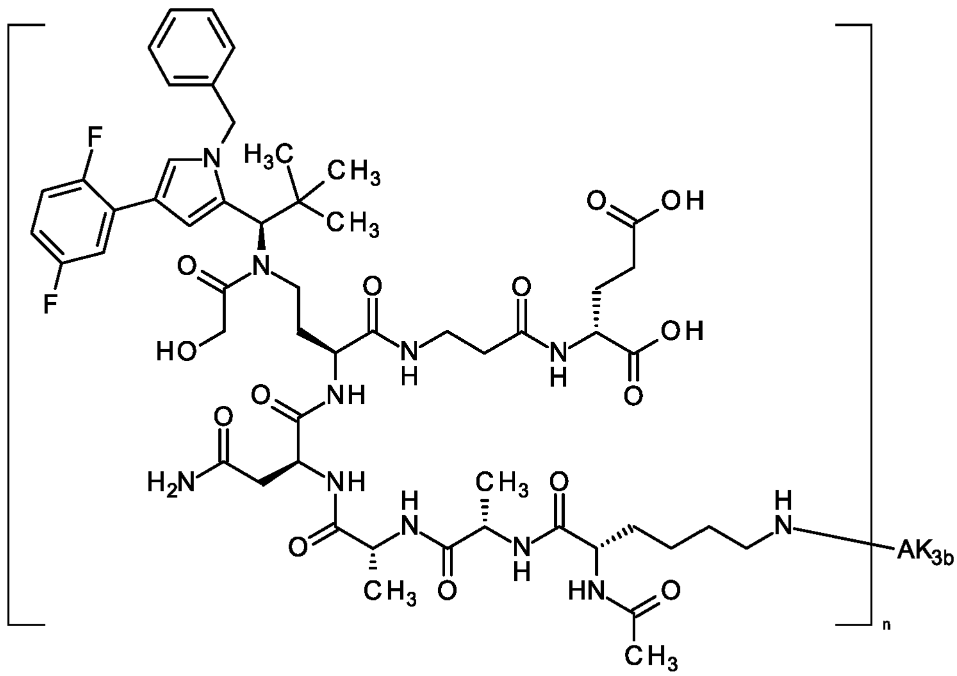

Zu einer Lösung von 5 mg des anti-TWEAKR Antikörpers TPP-5442 (entspricht TPP-2090-HC-N297Q) in DPBS pH 7.2 (c=7.4 mg/mL) wurden 80 μL einer 10 mMol Lösung von Intermediat R50 in DMSO hinzugegeben. Nach 5 min Inkubation bei 37°C wurden 400 μL einer Lösung von rekombinanter bakterieller Transglutaminase Lösung in Wasser (Produkt Nummer T001 von Zedira GmbH, Darmstadt, Germany) (25 U/mL) hinzugegeben und 24 h bei 37°C inkubiert. Dann wurde die Reaktionsmischung mit DPBS pH 7.2 auf ein Gesamtvolumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.1 μmol des b-Transglutaminase Blockers Zedira C100 in 200 μL DPBS zu der Lösung hinzugefügt. Die erhaltene ADC Lösung wurde folgendermaßen charakterisiert:

Protein Konzentration: 1.69 mg/mL
Drug/mAb Ratio: 3.7

**Beispiel 50et-4**

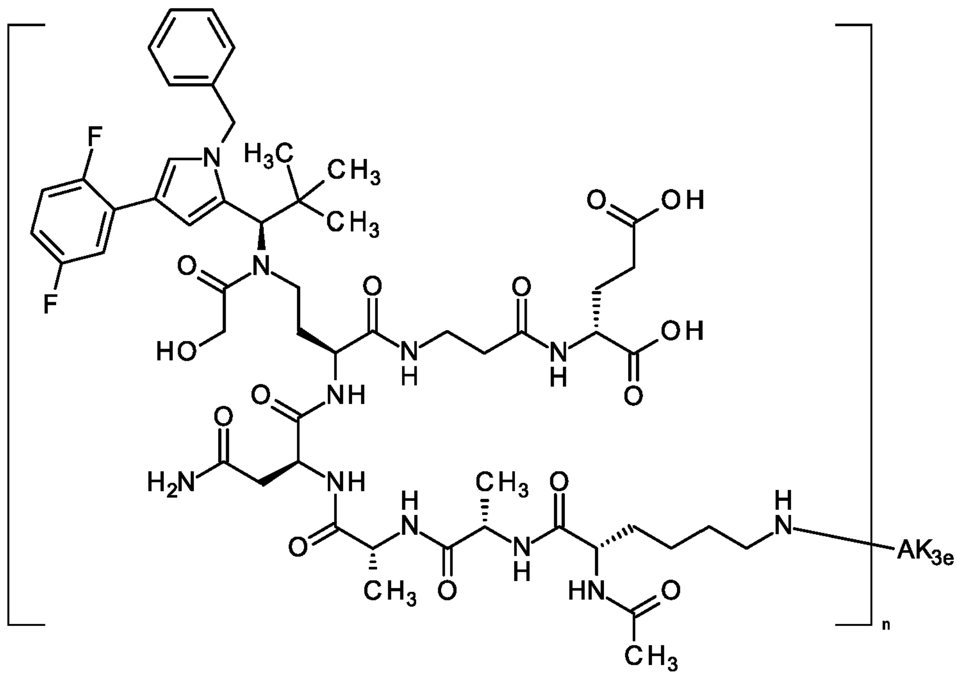

Zu einer Lösung von 5 mg des anti-HER2 Antikörpers TPP-7511 (entspricht anti-HER2 Antikörper-HC-N297Q) in DPBS pH 7.2 (c=10 mg/mL) wurden 80 μL einer 10 mMol Lösung von Intermediat R50 in DMSO hinzugegeben. Nach 5 min Inkubation bei 37°C wurden 400 μL einer Lösung von rekombinanter bakterieller Transglutaminase Lösung in Wasser (Produkt Nummer T001 von Zedira GmbH, Darmstadt, Germany) (25 U/mL) hinzugegeben und 24 h bei 37°C inkubiert. Dann wurde die Reaktionsmischung mit DPBS pH 7.2 auf ein Gesamtvolumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.1 μmol des b-Transglutaminase Blockers Zedira C100 in 200 μL DPBS zu der Lösung hinzugefügt. Die erhaltene ADC Lösung wurde folgendermaßen charakterisiert:

Protein Konzentration: 1.64 mg/mL
Drug/mAb Ratio: 3.8

**Beispiel 51dt-2**

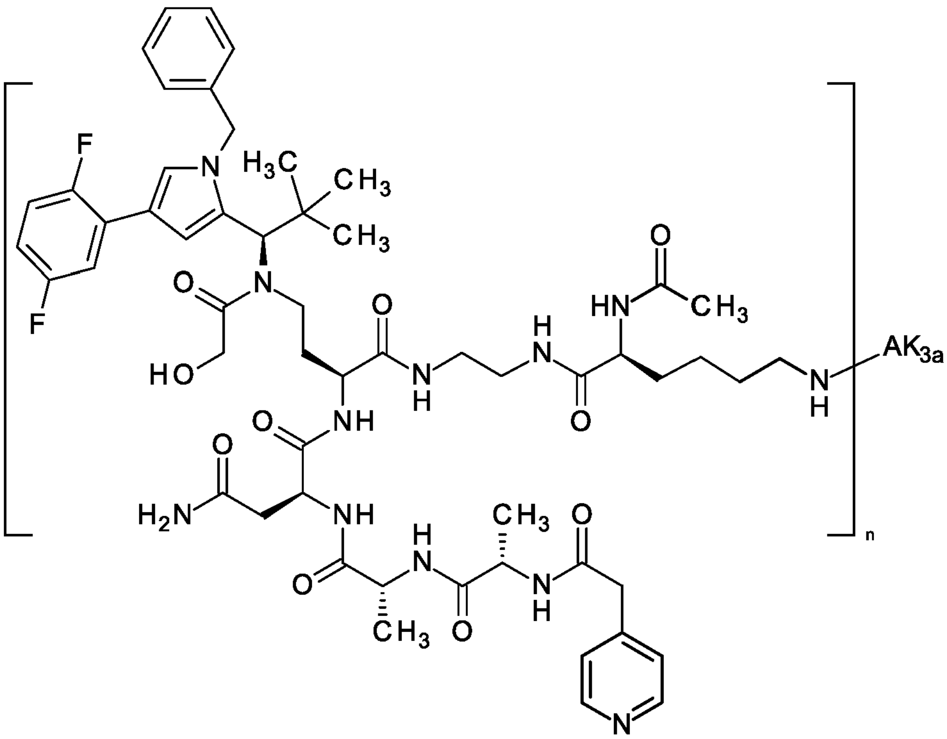

Zu einer Lösung von 5 mg des anti-TWEAKR Antikörpers TPP-2658 (entspricht TPP-2090-HC-N297A) in 530 µl DPBS pH 7.2 (c~10mg/mL) wurden 20 µL einer 10 mMol Lösung von Intermediat R51 in DMSO hinzugegeben. Nach 5 min Inkubation bei 37°C wurden 50 µL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.2 auf ein Volumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS. Schließlich wurden noch 0.005 µmol des b-Transglutaminase Blockers Zedira C100 in 12.5 µL DPBS zu der Lösung hinzugefügt. Die erhaltene ADC Lösung wurde folgendermaßen charakterisiert:

Protein Konzentration: 2.08 mg/mL
Drug/mAb Ratio: 1.9

**Beispiel 51dt-4**

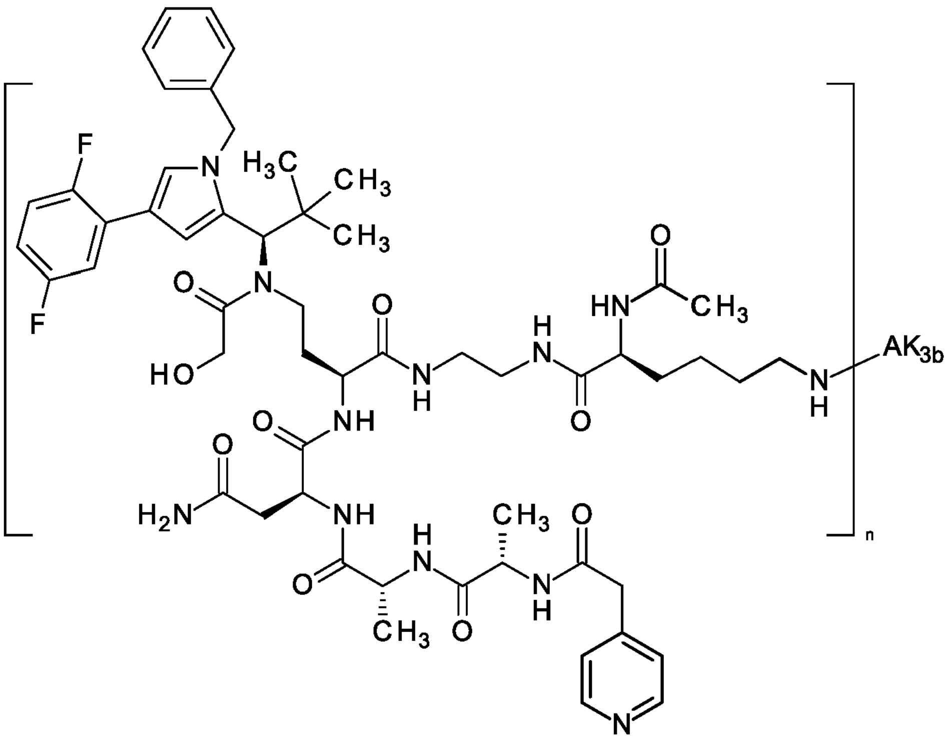

Zu einer Lösung von 5 mg des anti-TWEAKR Antikörpers TPP-5442 (entspricht TPP-2090-HC-N297Q) in DPBS pH 7.2 (c=10 mg/mL) wurden 53 μL einer 10 mMol Lösung von Intermediat R51 in DMSO hinzugegeben. Nach 5 min Inkubation bei 37°C wurden 400 μL einer Lösung von rekombinanter bakterieller Transglutaminase Lösung in Wasser (Produkt Nummer T001 von Zedira GmbH, Darmstadt, Germany) (25 U/mL) hinzugegeben und 24 h bei 37°C inkubiert. Dann wurde die Reaktionsmischung mit DPBS pH 7.2 auf ein Gesamtvolumen von 2.5 mL verdünnt und durch Gelfiltration über mit DPBS equillibierte PD 10-Säulen (Sephadex® G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.05 μmol des b-Transglutaminase Blockers Zedira C100 in 200 μL DPBS zu der Lösung hinzugefügt. Die erhaltene ADC Lösung wurde folgendermaßen charakterisiert:

Protein Konzentration: 1.56 mg/mL
Drug/mAb Ratio: 3.3

## Referenzbeispiele: Kleine Moleküle, APDCs und ADCs

Zunächst wurden zur Untersuchung der Legumain-vermittelten Spaltung und der Stabilität unter verschiedenen Bedingungen die Legumain-spaltbaren Prodrugs R3r-LLL und R3r-LDL hergestellt.

Darüber hinaus wurden als repräsentative Referenzverbindungen die Epimere der erfindungsgemäßen Legumain-spaltbaren APDCs und ADCs hergestellt, bei denen im Legumain-spaltbaren Linker alle Aminsosäuren in der natürlichen L-Konfiguration vorliegen (Referenzbeispiele R3a, e, k; R4a, e, k; R5a, e, k und R9a, e, k)

Schließlich wurde zur Bestimmung der Konzentrationen der aktiven Metaboliten in Tumor und anderen Geweben (-> Kapitel C5b) zum Vergleich mit Beispiel 3k (mit Legumainspaltbarer Gruppe) die Referenz-ADCs R10k und R10h1 (ohne Legumain-spaltbare Gruppe) hergestellt. Da beide ADCs den gleichen aktiven Metaboliten bilden, kann so der Einfluß der erfindungsgemäßen Legumain-spaltbaren Gruppe auf die Organvetreilung desselben untersucht werden.

## Referenzbeispiel R3r-LLL

N-(Pyridin-4-ylacetyl)-L-alanyl-L-alanyl-N$^1$-[(2S)-4-[{(1R)-1-[1-benzyl-4-(2,5-difluorphenyl)-1H-pyrrol-2-yl]-2,2-dimethylpropyl}(glycoloyl)amino]-1-(methylamino)-1-oxobutan-2-yl]-L-aspartamid

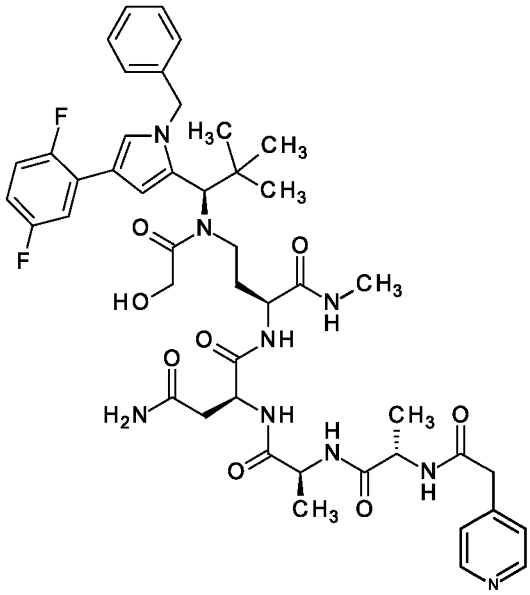

Zunächst wurde Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid (1:1), wie in WO 2015096982 A1 beschrieben, hergestellt. Anschließend wurde aus diesem Intermediat durch Kupplung mit Intermediat L103 in DMF in Gegenwart von HATU und von N,N-Diisopropyl-ethylamin die Titelverbindung hergestellt.

LC-MS (Methode 1): $R_t$ = 0.86 min; MS (ESIpos): m/z = 902 $[M+H]^+$.

## Referenzbeispiel R3r-LDL

N-(Pyridin-4-ylacetyl)-L-alanyl-D-alanyl-$N^1$-[(2S)-4-[{(1R)-1-[1-benzyl-4-(2,5-difluorphenyl)-1H-pyrrol-2-yl]-2,2-dimethylpropyl}(glycoloyl)amino]-1-(methylamino)-1-oxobutan-2-yl]-L-aspartamid

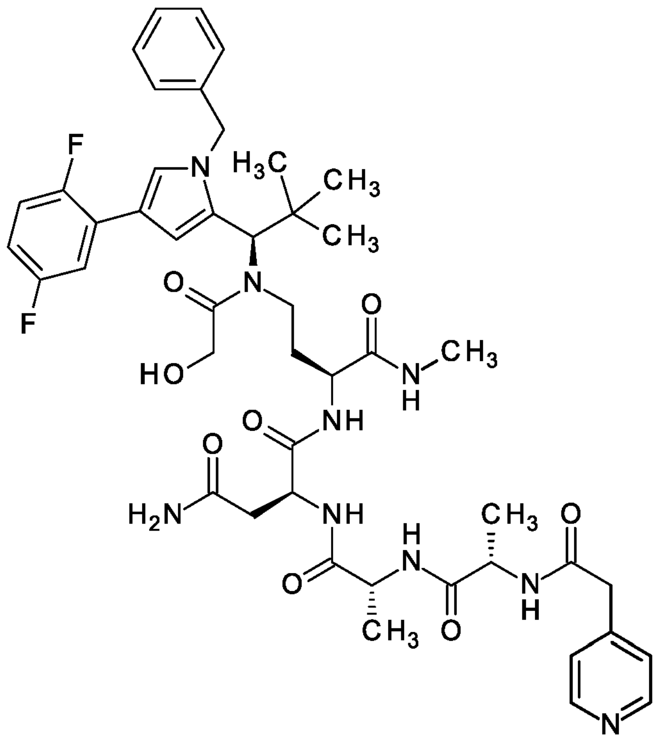

Zunächst wurde Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1), wie in WO 2015096982 A1 beschrieben, hergestellt. Anschließend wurde aus diesem Intermediat durch Kupplung mit Intermediat L110 in DMF in Gegenwart von HATU und von N,N-Diisopropylethylamin die Titelverbindung hergestellt.

LC-MS (Methode 1): $R_t$ = 0.88 min; MS (ESIpos): m/z = 902 $[M+H]^+$.

## Referenzbeispiel R3a

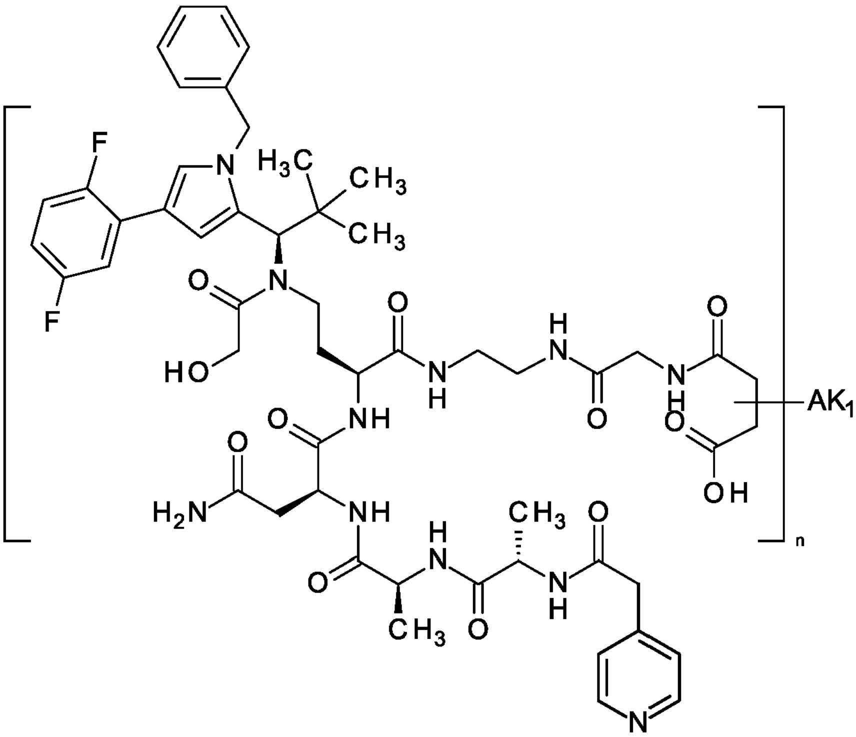

Die Herstellung erfolgte analog Beispiel 3a.

Protein Konzentration: 1.99 mg/mL
Drug/mAb Ratio: 2.6

Ebenso wurde in analoger Weise das Referenzbeispiel R3e mit dem anti-HER2 Antikörper TPP-1015 sowie das Referenzbeispiel R3k mit dem anti-TWEAKR Antikörper TPP-2658 hergestellt:

## Referenzbeispiel R3e

Protein Konzentration: 1.70 mg/mL
Drug/mAb Ratio: 3.3

## Referenzbeispiel R3k

Protein Konzentration: 1.71 mg/mL
Drug/mAb Ratio: 3.1

## Referenzbeispiel R4a

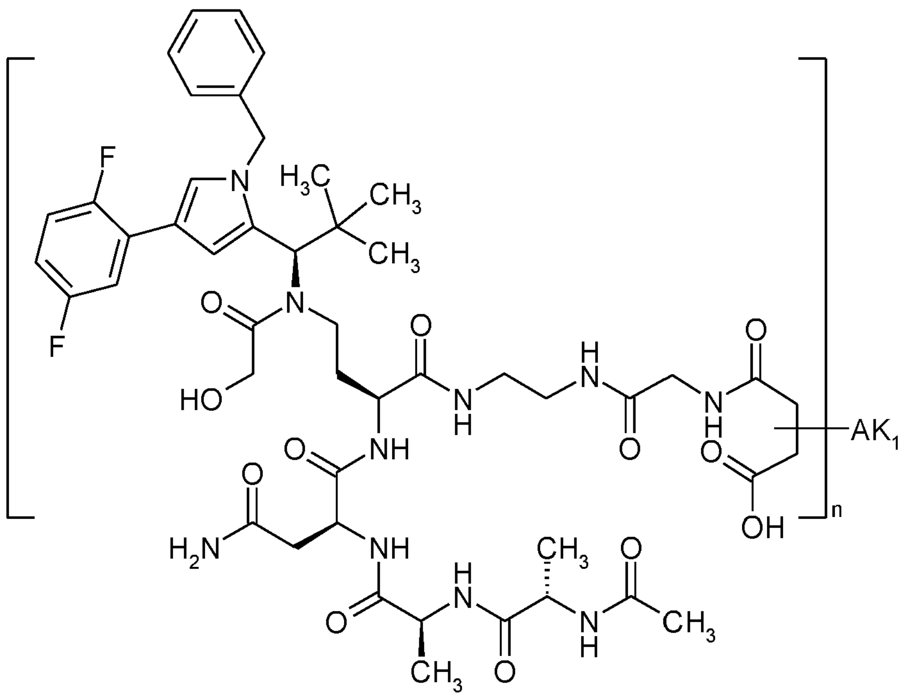

Die Herstellung erfolgte analog Beispiel 4a.

Protein Konzentration: 1.87 mg/mL
Drug/mAb Ratio: 3.2

Ebenso wurde in analoger Weise das Referenzbeispiel R4e mit dem anti-HER2 Antikörper TPP-1015 sowie das Referenzbeispiel R4k mit dem anti-TWEAKR Antikörper TPP-2658 hergestellt:

**Referenzbeispiel R4e**

Protein Konzentration: 1.70 mg/mL
Drug/mAb Ratio: 3.3

**Referenzbeispiel R4k**

Protein Konzentration: 1.79 mg/mL
Drug/mAb Ratio: 3.3

**Referenzbeispiel R5a**

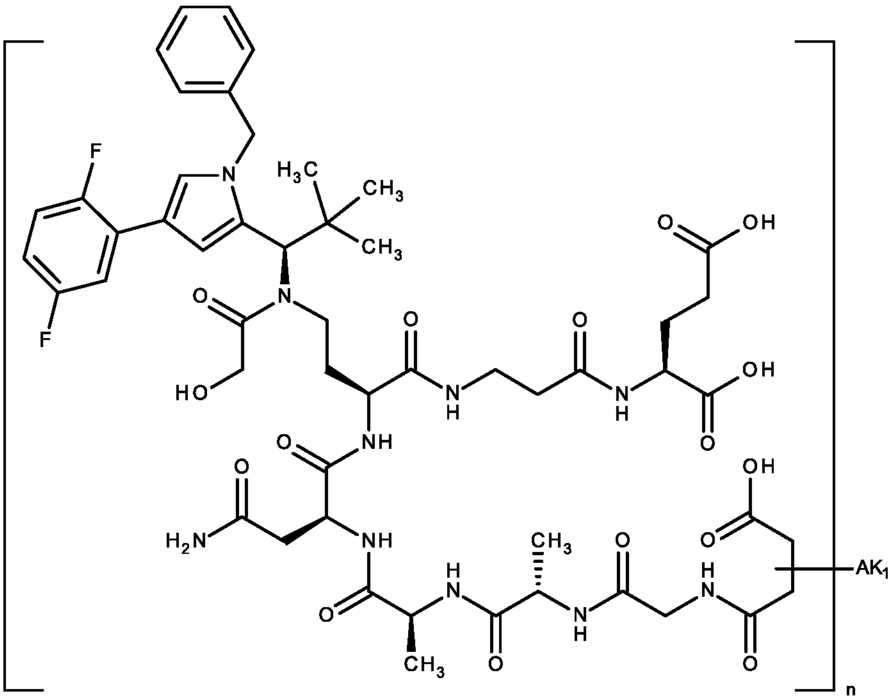

Die Herstellung erfolgte analog Beispiel 5a.

Protein Konzentration: 2.02 mg/mL
Drug/mAb Ratio: 3.5

Ebenso wurde in analoger Weise das Referenzbeispiel R5e mit dem anti-HER2 Antikörper TPP-1015 sowie das Referenzbeispiel R5k mit dem anti-TWEAKR Antikörper TPP-2658 hergestellt:

**Referenzbeispiel R5e**

Protein Konzentration: 1.72 mg/mL
Drug/mAb Ratio: 4.2

**Referenzbeispiel R5k**

Protein Konzentration: 1.79 mg/mL
Drug/mAb Ratio: 3.1

**Referenzbeispiel R9a**

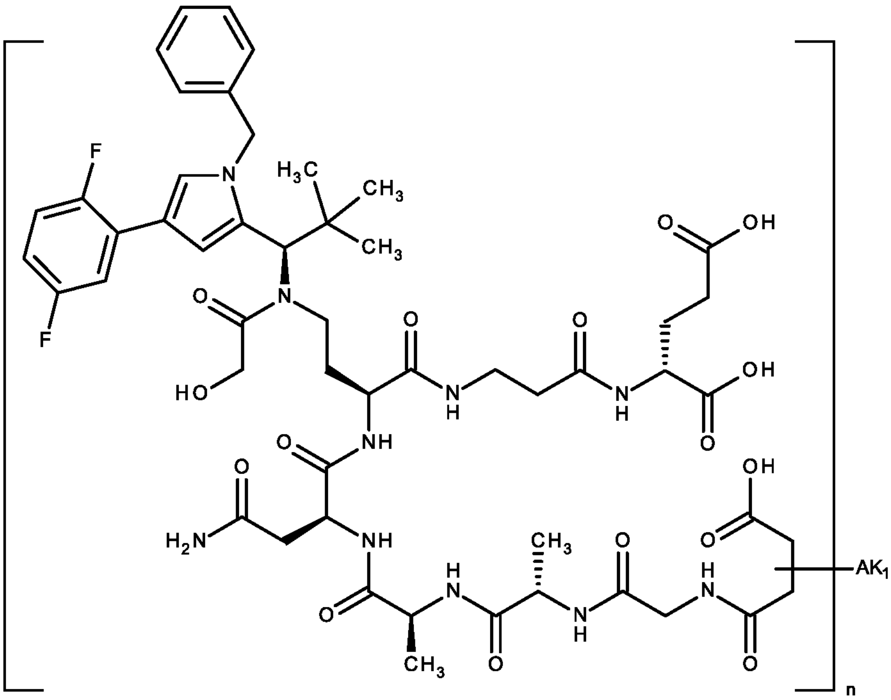

Die Herstellung erfolgte analog Beispiel 9a.

Protein Konzentration: 1.57 mg/mL
Drug/mAb Ratio: 2.4

Ebenso wurde in analoger Weise das Referenzbeispiel R9e mit dem anti-HER2 Antikörper TPP-1015 sowie das Referenzbeispiel R9k mit dem anti-TWEAKR Antikörper TPP-2658 hergestellt:

**Referenzbeispiel R9e**

Protein Konzentration: 1.79 mg/mL
Drug/mAb Ratio: 3.1

**Referenzbeispiel R9k**

Protein Konzentration: 0.68 mg/mL
Drug/mAb Ratio: 2.8

**Referenzbeispiel R10k**

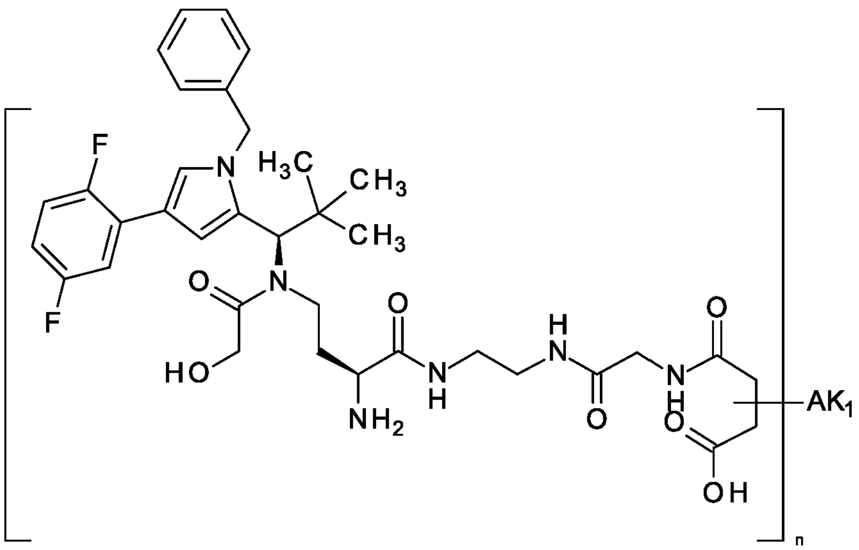

150 mg anti-TWEAKR Antikörper TPP-2658 in 10.5 mL ml PBS (c=14.28 mg/mL) wurden unter Argon mit einer Lösung aus 0.86 mg TCEP in 2 ml PBS-Puffer versetzt. Der Ansatzwurde 30min bei RT gerührt und anschließend wurden 6.63 mg (0.008 mmol) von Intermediat F104 gelöst in 1250 µl DMSO zugegeben. Nach weiteren 90 min Rühren bei RT wurde der Ansatz mit 1250 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf ein Gesamtvolumen von 22.5 ml verdünnt. Diese Lösung wurde über Nacht bei RT unter Argon gerührt und anschließend wieder mittel PD-10 Säulen auf pH 7.2 umgepuffert. Anschließend wurde durch Ultrazentrifugation aufkonzentriert, rückverdünnt mit PBS-Puffer (pH 7.2) und erneut aufkonzentriert. Der erhaltene ADC Batch wurde folgendermaßen charakterisiert:

Protein Konzentration: 14.06 mg/mL
Drug/mAb Ratio: 3.4

**Referenzbeispiel R10h1**

In analoger Weise wurde Intermediat F104 mit 100 mg anti-B7H3 Antikörper TPP-8382 gekuppelt. Der erhaltene ADC Batch wurde folgendermaßen charakterisiert:

Protein Konzentration: 14.12 mg/mL
Drug/mAb Ratio: 3.2

## C: Bewertung der biologischen Wirksamkeit

Die biologische Wirkung der erfindungsgemäßen Verbindungen kann durch die nachstehend beschriebenen Assays gezeigt werden:

### C-1a Bestimmung der cytotoxischen Wirkung der ADCs

Die Analyse der cytototoxischen Wirkung der ADCs erfolgt auf verschiedenen Zelllinien:

NCI-H292: humane mukoepidermoide Lungenkarzinomzellen, ATCC-CRL-1848, Standardmedium: RPMI 1640 (Biochrom; #FG1215, stab. Glutamin) + 10% FCS (Sigma #F2442), TWEAKR-positiv; EGFR-positiv.

BxPC3: humane Bauchspeicheldrüsenkrebszellen, ATCC-CRL-1687, Standardmedium: RPMI 1640 (Biochrom; #FG1215, stab. Glutamin) + 10% FCS (Sigma #F2442), TWEAKR-positiv

LoVo humane kolorektale Krebszellen, ATCC No. CCL-229, Kultivierung für MTT-Assay: Standardmedium: Kaighn's + L-Glutamin (Invitrogen 21127) + 10% heat inactivated FCS (Fa. Gibco, No. 10500-064). Kultivierung für CTG-Assay: RPMI 1640 (Biochrom; #FG1215, stab. Glutamin) + 10% FCS (Sigma #F2442). TWEAKR-positiv.

KPL4: humane Brustkrebszelllinie, Bayer Pharma AG (identity checked and confirmed on 19.7.2012 at DSMZ), Standardmedium: RPMI 1640 (Fa. Gibco; #21875- 059, stab. L-Glutamin) + 10% heat inactivated FCS (Fa. Gibco, No. 10500-064); HER2-positiv.

SK-HEP-1: humane Leberkrebszelllinie, ATCC No. HTB-52, Standardmedium: MEM mit Earle's Salzen + Glutamax I (Invitrogen 41090) + 10% heat inactivated FCS (Fa. Gibco, No. 10500-064); EGFR-positiv, TWEAKR-positiv

KU-19-19: humane Blasenkarzinomzellen, DMSZ, Standardmedium: RPMI 1640 (Biochrom; #FG1215, stab. Glutamin) + 10% FCS (Sigma #F2442), TWEAKR-positiv

SCC4: humanes Zungengrundkarzinom, Standardmedium: DMEM / Ham's F12; (Biochrom; # FG 4815, mit stabilem Glutamin), ATCC-CRL-1624+ 10% FCS (Sigma #F2442), TWEAKR-positiv

Die Kultivierung der Zellen erfolgt nach Standard-Methode, wie bei der American Tissue Culture Collection (ATCC) oder des Leibniz-Institut DSMZ-Deutsche Sammlung von Mikroorganismen und Zellkulturen GmbH (DSMZ) für die jeweiligen Zelllinien angegeben.

**CTG-Assay**

Die Kultivierung der Zellen erfolgte nach Standard-Methode, mit den unter C-1a angegebenen Wachstumsmedien. Zur Durchführung wurden die Zellen mit einer Lösung von Trypsin (0.05%) und EDTA (0.02%) in PBS (Biochrom AG #L2143) abgelöst, pelletiert, in Kulturmedium resuspendiert, gezählt und in eine 96-Loch Kulturplatte mit weissem Boden (Costar #3610) ausgesät (in 75μl/Loch, folgende Zellzahlen je Loch: NCI-H292: 2500 Zellen/ Loch, BxPC3 2500 Zellen / Loch, LoVo 3000 Zellen / Loch) und im Brutschrank bei 37°C und 5% Kohlendioxid inkubiert. Nach 24h wurden die Antikörper-Wirkstoffkonjugate in 25μl Kulturmedium (vierfach konzentriert) auf die Zellen gegeben, so dass finale Konzentrationen der Antikörper-Wirkstoffkonjugate von $3 \times 10^{-7}$ M bis $3 \times 10^{-11}$ M auf den Zellen erreicht wurden (Triplikate). Anschließend wurden die Zellen im Brutschrank bei 37°C und 5% Kohlendioxid inkubiert. In einer parallelen Platte wurde die Zellvitalität zu Beginn der Wirkstoffbehandlung (Tag 0) mit dem Cell Titer Glow (CTG) Luminescent Cell Viability Assay (Promega #G7573 und #G7571) bestimmt. Dazu wurden pro Zellansatz 100μl des Substrats hinzugefügt, die Platten anschließend mit Alufolie abgedeckt, für 2 Minuten mit dem Plattenschüttler bei 180 rpm geschüttelt, für 8 Minuten auf der Laborbank stehen gelassen und dann mit einem Luminometer (Victor X2, Perkin Elmer) gemessen. Das Substrat detektiert den ATP-Gehalt in den lebenden Zellen, wobei ein Lumineszenz-Signal erzeugt wird, dessen Höhe direkt proportional zur Vitalität der Zellen ist. Nach 72h Inkubation mit den Antikörper-Wirkstoffkonjugaten wurde nun auch in diesen Zellen die Vitalität mit dem Cell Titer Glow Luminescent Cell Viability Assay wie oben beschrieben bestimmt. Aus den gemessenen Daten wurde die $IC_{50}$ der Wachstumshemmung im Vergleich zum Tag 0 unter Verwendung des DRC (Dose Response Curve) Analysis Spreadsheets anhand einer 4-Parameter Anpassung berechnet. Das DRC Analysis Spreadsheet ist ein von Bayer Pharma AG und Bayer Business Services auf der Plattform IDBS E-WorkBook Suite entwickeltes Biobook Spreadsheet (IDBS: ID Business Solutions Ltd., Guildford, UK).

In der folgenden Tabelle 1a sind die $IC_{50}$-Werte repräsentativer Ausführungsbeispiele aus diesem Assay aufgeführt:

**Tabelle 1a**

| Beispiel | BxPC3 $IC_{50}$ [M] CTG | NCI-H292 $IC_{50}$ [M] CTG | LoVo $IC_{50}$ [M] CTG |
|---|---|---|---|
| 1k | 1.76E-09 | 1.13E-09 | 4.36E-10 |
| 2k | 1.68E-09 | 1.58E-09 | 8.70E-10 |
| 3k | 1.42E-09 | 1.34E-09 | 7.90E-10 |
| 3l1 | 3.00E-10 | 1.59E-10 | 3.10E-11 |
| 3l2 | 6.68E-10 | 4.27E-10 | 1.84E-10 |
| 3l3 | 1.22E-09 | 6.45E-10 | 5.34E-11 |
| 3l4 | 8.91E-10 | 4.15E-10 | 3.97E-11 |
| 4k | 2.66E-09 | 1.39E-09 | 7.89E-10 |
| 5k | 7.24E-10 | 1.05E-09 | 3.80E-10 |
| 6k | 1.34E-09 | 2.04E-09 | 8.95E-10 |

(fortgesetzt)

| Beispiel | BxPC3 $IC_{50}$ [M] CTG | NCI-H292 $IC_{50}$ [M] CTG | LoVo $IC_{50}$ [M] CTG |
|---|---|---|---|
| 7k | 2.14E-09 | 3.09E-09 | >6.00E-07 |
| 8k | 1.48E-09 | 2.11E-09 | 4.62E-08 |
| 9k | 6.25E-10 | 7.39E-10 | 3.24E-10 |
| 11k | 1.14E-08 | 4.00E-08 | >6.00E-07 |
| 12k | 5.96E-09 | >6.00E-07 | >6.00E-07 |
| 13k | 4.02E-09 | 6.77E-09 | 2.16E-09 |
| 14k | 4.19E-08 | 1.86E-07 | >6.00E-07 |
| 15k | 1.35E-09 | 2.22E-09 | 4.49E-10 |
| 15l1 | 1.71E-09 | 8.61E-10 | 7.76E-10 |
| 16k | 1.16E-09 | 1.45E-09 | 3.35E-10 |
| 16l2 | 1.95E-10 | 1.70E-10 | 5.58E-11 |
| 17k | 5.53E-10 | 5.36E-10 | 9.49E-11 |
| 18k | 4.34E-08 | 1.53E-08 | 1.31E-09 |
| 19k | 1.05E-08 | 9.02E-09 | 1.39E-09 |
| 20k | 8.39E-10 | 2.94E-09 | 7.51E-10 |
| 21k | 1.81E-09 | 2.74E-09 | 4.52E-09 |
| 22k | 1.74E-09 | 4.96E-09 | 5.74E-10 |
| 23k | 1.90E-09 | 3.01E-09 | 6.11E-10 |
| 24k | 8.38E-08 | 1.09E-09 | 4.81E-10 |
| 24l1 | 1.70E-10 | 1.80E-10 | 1.50E-11 |
| 24l3 | 7.26E-11 | 5.83E-11 | 3.00E-11 |
| 24l4 | 1.44E-10 | 1.02E-10 | 3.00E-11 |
| 25k | 7.23E-10 | 1.59E-09 | 4.41E-10 |
| 26k | 2.02E-09 | 2.17E-09 | 8,13E-10 |
| 27k | 1.01E-09 | 8.72E-10 | 1.95E-10 |
| 28l1 | 3.64E-10 | 1.42E-10 | 1.50E-11 |
| 29k | 9.46E-10 | 1.16E-09 | 8.86E-11 |
| 30k | 8.73E-10 | 1.09E-09 | 1.50E-10 |
| 31l1 | 3.87E-10 | 2.05E-10 | 1.50E-11 |
| 32k | 6.19E-10 | 7.65E-10 | 1.13E-10 |
| 33l1 | 2.96E-10 | 1.36E-10 | 1.50E-11 |
| 34l1 | 5.22E-10 | 3.98E-10 | 6.99E-11 |
| 35k | 2.59E-10 | 3.15E-10 | 1.93E-10 |
| 35l1 | 2.00E-10 | 2.08E-10 | 9.23E-11 |
| 36l1 | 2.80E-10 | 1.68E-10 | 3.44E-11 |
| 37k | 3.77E-10 | 6.11E-10 | 2.98E-10 |
| 37l1 | 2.12E-10 | 2.31E-10 | 9.21E-11 |
| 38l1 | 1.75E-10 | 1.29E-10 | 1.50E-11 |

(fortgesetzt)

| Beispiel | BxPC3 $IC_{50}$ [M] CTG | NCI-H292 $IC_{50}$ [M] CTG | LoVo $IC_{50}$ [M] CTG |
|---|---|---|---|
| 39I1 | 1.36E-10 | 9.60E-11 | 1.15E-11 |
| 40I1 | 1.42E-09 | 5.00E-09 | 6.00E-07 |
| 41I1 | 1.77E-10 | 1.33E-10 | 1.50E-11 |
| 42I1 | 1.98E-10 | 2.00E-10 | 1.50E-11 |
| 43I1 | 3.66E-10 | 1.60E-10 | 1.50E-11 |
| 44I1 | 3.15E-10 | 9,57E-11 | 3.15E-11 |
| 45I1 | 3.00E-10 | 1.81E-10 | 6.96E-08 |
| 46I1 | 5.54E-10 | 2.72E-10 | 6.00E-07 |
| 47I1 | 3.44E-10 | 1.67E-10 | 5.79E-11 |
| 48I1 | 3.83E-10 | 3.54E-10 | 1.50E-11 |
| 49I1 | 2.06E-10 | 1.50E-10 | 1.50E-11 |

In der folgenden Tabelle 1b sind Vergleichdaten repräsentativer Beispiele der erfindungsgemäßen ADCs und APDCs mit den entsprechenden epimeren ADCs und APDCs, bei denen alle Aminsosäuren der Legumain-spaltbaren Gruppe in der L-Konfiguration vorliegen, (Referenzbeispielserie R) aufgeführt. Es ergeben sich keine signifikanten Unterschiede:

**Tabelle 1b)**

| Beispiel | BxPC3 $IC_{50}$ [M] CTG | NCI-H292 $IC_{50}$ [M] CTG | LoVo $IC_{50}$ [M] CTG |
|---|---|---|---|
| 3k | 1.42E-09 | 1.34E-09 | 7.90E-10 |
| R3k (L-L-L) | 1.36E-09 | 8.51E-10 | 2.28E-10 |
| | | | |
| 4k | 2.66E-09 | 1.39E-09 | 7.89E-10 |
| R4k (L-L-L) | 2.94E-09 | 5.84E-10 | 2.04E-10 |
| | | | |
| 5k | 7.24E-10 | 1.05E-09 | 3.80E-10 |
| R5k (L-L-L) | 5.34E-10 | 8.61E-10 | 3.46E-10 |
| | | | |
| 9k | 6.25E-10 | 7.39E-10 | 3.24E-10 |
| R9k (L-L-L) | 2.56E-10 | 3.91E-10 | 1.09E-10 |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Antikörper-Wirkstoffkonjugate war selektiv versus der jeweiligen Isotypkontrolle, die den jeweils entsprechenden Linker und Toxophor enthielt.

**MTT-Assay**

Die Kultivierung der Zellen erfolgte nach Standardmethode, mit den unter C-1a angegebenen Wachstumsmedien. Zur Durchführung wurden die Zellen mit einer Lösung von Accutase in PBS (Fa. Biochrom AG #L2143) abgelöst, pelletiert, in Kulturmedium resuspendiert, gezählt und in eine 96-Loch Kulturplatte mit weißem Boden (Fa. Costar #3610) ausgesät (NCI-H292: 2500 Zellen/well; SK-HEP-1: 1000 Zellen/well; KPL4: 1200 Zellen/well; in 100µL Gesamtvolumen). Anschließend wurden die Zellen im Brutschrank bei 37°C und 5% Kohlendioxid inkubiert. Nach 48h wurde ein Mediumwechsel durchgeführt. Dann wurden die Antikörper-Wirkstoff-Konjugate in 10μl Kulturmedium in Konzentrationen von $10^{-5}$M bis $10^{-13}$M zu den Zellen (Triplikate) pipettiert, bevor der Ansatz im Brutschrank bei 37°C und 5% Kohlendioxid inkubierte. Nach 96h erfolgte die Detektion der Zellproliferation mit Hilfe des MTT-Assays (ATCC, Manassas, Virginia, USA, Katalog-Nr. 30-1010K). Hierzu wurde das MTT Reagens für 4h mit den Zellen inkubiert, bevor durch Zugabe des Detergenzes die Lyse der Zellen über Nacht erfolgte. Die Detektion des gebildeten Farbstoffs erfolgte bei 570nm (Infinite M1000 pro, Fa. Tecan). Aus den gemessenen Daten wurde die $IC_{50}$ der Wachstumshemmung unter Verwendung der DRC (Dose Response Curve) berechnet. Die Proliferation ohne Testsubstanz, aber ansonsten identisch behandelten Zellen, wird als 100% Wert definiert.

In den folgenden Tabellen 1c-f sind die $IC_{50}$-Werte repräsentativer Ausführungsbeispiele aus diesem Assay aufgeführt:

**Tabelle 1c**

| Beispiel | NCI-H292 $IC_{50}$ [M] MTT Assay | SK-HEP-1 $IC_{50}$ [M] MTT Assay |
|---|---|---|
| 1a | 6.87E-11 | 4.96E-08 |
| 2a | 1.62E-10 | 8.31E-08 |
| 3a | 5.06E-10 | 1.13E-07 |
| 4a | 1.82E-10 | 9.52E-08 |
| 5a | 2.55E-11 | 3,30E-08 |
| 6a | 3.45E-12 | 9.93E-08 |
| 7a | 5.33E-10 | 7,52E-08 |
| 8a | 3,01E-11 | 6.83E-10 |
| 9a | 2.53E-11 | 2.47E-07 |
| 11a | 4.05E-10 | 4.35E-07 |
| 12a | 1.86E-10 | 5.00E.07 |
| 13a | 6.04E-10 | 8.27E-08 |
| 14a | 1.30E-10 | 1.13E-07 |
| 15a | 1.99E-10 | 1.03E-08 |
| 16a | 1.67E-11 | 1.33E-11 |
| 17a | 2.65E-12 | 2.94E-11 |
| 18a | 7.18E-11 | 6.62E-08 |
| 19a | 7.63E-11 | 7.14E-08 |
| 20a | 1.00E-12 | 1.57E-11 |
| 21a | 1.20E-11 | 2.59E-07 |
| 22a | 1.89E-10 | 7.32E-08 |
| 23a | 1.00E-12 | 1.00E-11 |
| 24a | 1.00E-12 | 1.04E-10 |
| 25a | 2.92E-12 | 2.16E-08 |
| 26a | 3.02E-09 | 6.01E-10 |
| 27a | 2.55E-12 | 6.02E-09 |
| 28a | 5.00E-07 | 5.00E-07 |
| 29a | 3.59E-11 | 1.04E-07 |
| 30a | 5.72E-11 | 6.03E-08 |
| 31a | 2.65E-10 | 2.35E-08 |

(fortgesetzt)

| Beispiel | NCI-H292 $IC_{50}$ [M] MTT Assay | SK-HEP-1 $IC_{50}$ [M] MTT Assay |
|---|---|---|
| 32a | 3.00E-11 | 6.39E-09 |
| 33a | 3.44E-12 | 1.49E-09 |
| 34a | 8.96E-10 | 1.71E-09 |
| 35a | 1.23E-12 | 2.89E-10 |
| 36a | 9.98E-12 | 1.02E-07 |
| 37a | 1.90E-12 | 3.16E-10 |
| 38a | 7.62E-12 | 4.28E-09 |
| 39a | 8.68E-12 | 1.35E-08 |
| 40a | 5.93E-10 | 5.00E-07 |
| 41a | 2.46E-12 | 5.00E-07 |
| 42a | 3.28E-10 | 5.00E-07 |
| 43a | 4.58E-12 | 9.18E-08 |
| 45a | 7.63E-12 | 5.40E-09 |
| 46a | 5.37E-11 | 9.15E-08 |
| 47a | 8.41E-12 | 4.50E-08 |
| 48a | 2.15E-12 | 2.07E-10 |
| 49a | 3.15E-13 | 2.51E-12 |
| 50dt-2 | 4.29E-10 | 3.10E-10 |
| 50dt-4 | 1.49E-10 | 2.77E-10 |
| 51dt-2 | 1.67E-08 | 8.79E-08 |
| 51dt-4 | 2.84E-09 | 1.21E-07 |

In der folgenden Tabelle 1d sind Vergleichsdaten repräsentativer Beispiele mit den erfindungsgemäßen ADCs und entsprechenden APDCs, bei denen alle Aminsosäuren der Legumain-spaltbaren Gruppe in der L-Konfiguration vorliegen, (Referenzbeispielserie R) aufgeführt. Es wurden an der NCI-H292 Zelllinie keine deutlich veränderten $IC_{50}$-Werte gemessen.

**Tabelle 1d:**

| Beispiel | NCI-H292 $IC_{50}$ [M] MTT Assay | SK-HEP-1 $IC_{50}$ [M] MTT Assay |
|---|---|---|
| 3a | 5.06E-10 | 1.13E-07 |
| R3a (L-L-L) | 2.80E-12 | 3.86E-1015 |
| 4a | 1.82E-10 | 9.52E-08 |
| R4a (L-L-L) | 4.65E-11 | 1.25E-10 |
| 5a | 2.55E-11 | 3,30E-08 |
| R5a (L-L-L) | 3.17E-12 | 4.83E-09 |
| 9a | 2.53E-11 | 2.47E-07 |
| R9a (L-L-L) | 1.69E-11 | 2.56E-09 |

**Tabelle 1e**

| Beispiel | KPL4 $IC_{50}$ [M] MTT Assay | |
|---|---|---|
| 1e | 8.00E-10 | |
| 2e | 2.58E-10 | |
| 3e | 1.47E-10 | |
| 4e | 2.10E-10 | |
| 5e | 1.43E-10 | |
| 6e | 1.16E-10 | |
| 7e | 1.17E-10 | |
| 8e | 2.42E-11 | |
| 9e | 8.45E-11 | |
| 12e | 4.53E-08 | |
| 13e | 1.16E-10 | |
| 14e | 2.01E-09 | |
| 15e | 4.37E-11 | |
| 16e | 1.91E-11 | |
| 17e | 1.16E-11 | |
| 18e | 1.83E-10 | |
| 19e | 1.25E-10 | |
| 20e | 2.77E-12 | |
| 21e | 1.56E-11 | |
| 22e | 7.11E-11 | |
| 23e | 2.30E-11 | |
| 24e | 9.73E-11 | |
| 25e | 1.30E-10 | |
| 28e | 1.48E-10 | |
| 29e | 2.82E-10 | |
| 30e | 1.77E-10 | |
| 31e | 3.77E-10 | |
| 32e | 1.75E-10 | |
| 33e | 6.10E-12 | |
| 34e | 6.44E-10 | |
| 35e | 4.06E-12 | |
| 36e | 4.31E-11 | |
| 37e | 7.31E-12 | |
| 38e | 6.22E-12 | |
| 39e | 3.75E-11 | |
| 40e | 5,95E-08 | |
| 41e | 2.69E-10 | |

(fortgesetzt)

| Beispiel | KPL4 $IC_{50}$ [M] MTT Assay | |
|---|---|---|
| 42e | 3.89E-10 | |
| 43e | 1.19E-10 | |
| 44e | 6.79E-11 | |
| 45e | 7.36E-10 | |
| 46e | 5.37E-09 | |
| 47e | 1.17E-10 | |
| 48e | 7.50E-11 | |
| 49e | 3.00E-11 | |
| 50et-4 | 3.77E-10 | |

In der folgenden Tabelle 1f sind Vergleichsdaten repräsentativer Beispiele mit den erfindungsgemäßen ADCs und entsprechenden APDCs, bei denen alle Aminsosäuren der Legumain-spaltbaren Gruppe in der L-Konfiguration vorliegen, (Referenzbeispielserie R) aufgeführt. Es wurden keine signifikanten veränderte $IC_{50}$- Werte gemessen.

**Tabelle 1f:**

| Beispiel | KPL4 $IC_{50}$ [M] MTT Assay |
|---|---|
| 3e | 1.47E-10 |
| R3e (L-L-L) | 9.37E-11 |
| 4e | 2.10E-10 |
| R4e (L-L-L) | 4.15E-09 |
| 5e | 1.43E-10 |
| R5e (L-L-L) | 4.81E-10 |
| 9e | 8.45E-11 |
| R9e (L-L-L) | 8.25E-11 |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Antikörper-Wirkstoffkonjugate war selektiv versus der jeweiligen Isotypkontrolle, die den jeweils entsprechenden Linker und Toxophor enthielt.

**C-1b Bestimmung der Inhibition des Kinesinspindelproteins KSP/ Eg5 durch ausgewählte Beispiele**

Die Motordomäne des humanen Kinesinspindelproteins KSP /Eg5 (Fa. tebu-bio/ Cytoskeleton Inc, No. 027EG01-XL) wurde in einer Konzentration von 10nM mit 50µg/ml Taxol (Fa. Sigma No. T7191-5MG) stabilisierten Microtubuli (bovine oder porcine, Fa. tebu-bio/ Cytoskeleton Inc) für 5 min bei RT in 15mM PIPES, pH 6,8 (5mM MgCl2 und 10mM DTT, Fa. Sigma) inkubiert. Die frisch hergestellte Mischung wurde in eine 384 MTP (Fa. Corning) aliquotiert. Es folgte die Zugabe der zu untersuchenden Inhibitoren in Konzentration von 1.0 x10-6M bis 1.0x 10-13M und ATP (finale Konzentration 500µM; Fa. Sigma). Die Inkubation erfolgte über 2h bei RT. Die ATPase-Aktivität wurde durch den Nachweis des entstehenden anorganischen Phosphats mit Malachit-Grün detektiert (Fa. Biomol). Nach der Zugabe des Reagenz erfolgte eine 50min Inkubation bei RT, bevor die Detektion der Absorption bei einer Wellenlänge von 620nm erfolgt. Als Positivkontrolle wurden Monastrol (Fa. Sigma, M8515-1mg) und Ispinesib (Fa. AdooQ Bioscience A10486) verwendet. Die Einzeldaten der Dosis-Wirkungskurve stellen achtfach Bestimmungen dar. Bei den $IC_{50}$-Werten handelt es sich um Mittelwerte aus zwei unabhängigen Experimenten. Als 100% Kontrolle diente die nicht mit Inhibitoren behandelte Probe.

In der folgenden Tabelle 2 sind die $IC_{50}$-Werte repräsentativer Ausführungsbeispiele aus dem beschriebenen Assay und den korrespondierenden zytotoxischen Daten (MTT-Assay) zusammengefasst:

**Tabelle 2**

| Beispiele | KSP-Assay $IC_{50}$ [M] | NCI-H292 $IC_{50}$ [M] MTT Assay | SK-HEP-1 $IC_{50}$ [M] MTT Assay | KPL4 $IC_{50}$ [M] MTT Assay |
|---|---|---|---|---|
| M01 | 2.01E-09 | 5.00E-07 | | 5.00E-07 |
| M02 | 2.45E-09 | 1.62E-07 | 1.40E-07 | 1.63E-07 |
| M03 | 1.52E-09 | 2.34E-08 | 1.18E-07 | 9.00E-08 |
| M04 | 2.71E-10 | 4.43E-08 | 3.45E-08 | 1.76E-07 |
| M05 | 4.57E-10 | 7.94E-08 | 2.19E-07 | 2.22E-07 |
| M06 | 1.78E-09 | 4.63E-08 | 1.77E-07 | 1.93E-07 |
| M07 | 6.21E-10 | 2.12E-08 | | 9.23E-08 |
| M08 | 1.64E-08 | | | 4.87E-07 |
| M09 | 1.09E-09 | 2.70E-10 | 7.62E-09 | 5.14E-10 |
| M10 | 4.70E-10 | 3.03E-07 | 1.37E-07 | 2.26E-07 |
| M11 | 1.11E-09 | 4.32E-11 | 1.89E-10 | |
| M12 | 4.46E-10 | 3.3E-08 | 2.11E-08 | |
| M13 | 1.50E-09 | 1.52E-07 | 6.81E-08 | 1.69E-07 |
| M14 | 2.16E-09 | 1.74E-07 | 9.43E-08 | 1.77E-07 |
| M15 | 9.64E-10 | 1.33E-07 | 4.98E-08 | 1.69E-07 |
| M16 | 1.48E-09 | 1.43E-07 | 4.40E-08 | 1.95E-07 |
| M17 | 4.17E-09 | 7.35E-09 | | |
| M18 | 5.17E-09 | 3.55E-08 | | |
| M19 | 2.58E-09 | 1.21E-07 | | |
| M20 | 1.50E-09 | 1.49E-07 | 1.81E-07 | 2.13E-07 |
| M21 | 2.31E-09 | | | |
| M22 | 8.27E-10 | 2.89E-08 | 2.03E-08 | 1.82E-07 |
| M23 | 1.26E-09 | 5.00E-07 | 5.00E-07 | 5.00E-07 |
| M24 | 2.57E-09 | 1.67E-07 | 5.73E-08 | 5.00E-07 |
| M25 | 2.91E-09 | 5.00E-07 | 5.00E-07 | 5.00E-07 |
| M26 | 9.441E-10 | 6.38E-08 | 1.90E-08 | |
| M27 | 2.03E-09 | 2.76E-07 | 8.90E-08 | |
| M28 | 1.41E-09 | 1.55E-07 | 1.87E-07 | 2.10E-07 |
| M29 | 2.03E-09 | 1.87E-07 | 2.07E-07 | |
| R3r-LDL | 2.64E-07 | 1.90E-07 | 2.83E-07 | |
| R3r- LLL | 1.62E-07 | 2.04E-07 | 2.32E-07 | |

Die angegebenen Wirkdaten beziehen sich auf die im vorliegenden experimentellen Teil beschriebenen Ausführungsbeispiele.

## C-1c Enzymatische Assays und Stabilitätsuntersuchungen

### a: Cathepsin B-Assay

Für jede zu untersuchende Cathepsin B-spaltbare Prodrug wurde ein Ansatz in einem Mikroreaktionsgefäße (0.5ml, Fa. Eppendorf) angesetzt. Das hier verwendete Enzym wurde aus humanem Lebergewebe gewonnen. Es wurden 2µg Cathepsin B (Sigma C8571 25 µg) vorgelegt und mit 50mM Na-Phosphat Puffer, pH6.0, 2mM DTT auf ein Gesamtvolumen von 200µL aufgefüllt. Dann wurden 50 µL der zu untersuchenden Substratlösung-Lösung zupipettiert. Die Inkubation des Ansatzes erfolgte im Thermoblock (Fa. Thermo Fisher Scientific) bei 40°C unter ständigem Schütteln bei 300rpm. Die enzymatische Reaktion wurde kinetisch kontrolliert. Hierzu wurde zu unterschiedlichen Zeitpunkten eine 10µL Probe entnommen. Die entnommene Probe wurde sofort mit 20µL eiskaltem Methanol versetzt, um die enzymatische Reaktion zu stoppen und dann bei - 20°C eingefroren. Die gewählten Zeitpunkte zur Probenentnahme waren nach 10min, 2h, 4h und 24h. Die Proben wurden dann anschließend mittels RP-HPLC-Analyse untersucht (reverse phase HPLC, Fa. Agilent Technologies 1200er Serie). Die Bestimmung des freigesetzten Toxophors ermöglichte die Bestimmung der Halbwertszeit $t_{1/2}$ der enzymatischen Reaktion.

### b: Legumain Assay

Der Legumain Assay wurde mit rekombinantem humanen Enzym durchgeführt. Die rhLegumain Enzymlösung (Catalog # 2199-CY, R&D Systems) wurde in 50mM Na-Acetat Puffer/ 100mM NaCl, pH4.0 auf die gewünschte Konzentration verdünnt und 2h bei 37°C vorinkubiert. rhLegumain wurde dann in 50mM MES Puffer, 250mM NaCl, pH 5.0 auf eine finale Konzentration von 1ng/µL eingestellt. Für jede zu untersuchende Legumain-spaltbare Prodrug wurde ein Ansatz in einem Mikroreaktionsgefäße (0.5m1, Fa. Eppendorf) angesetzt. Hierzu wurde die Substratlösung mit 50mM MES Puffer, 250mM NaCl, pH 5.0 auf die gewünschte Konzentration (2-fach konzentriert) eingestellt. Für die kinetische Messung der enzymatischen Reaktion wurden zunächst 250µL der Legumainlösung vorgelegt und durch Zugabe von 250µL der Substratlösung (finale Konzentration einfach konzentriert) wurde die Enzymreaktion gestartet. Zu verschiedenen Zeitpunkten wurden je 50µL Proben entnommen Diese Probe wurde sofort mit 100µL eiskaltem Methanol versetzt, um die enzymatische Reaktion zu stoppen und dann bei - 20°C eingefroren. Die gewählten Zeitpunkte zur Probenentnahme waren nach 0,5h, 1h, 3h und 24h. Die Proben wurden dann anschließend mittels RP-HPLC-Analyse und durch LC-MS Analytik untersucht. Die Bestimmung des freigesetzten Toxophors ermöglichte die Bestimmung der Halbwertszeit $t_{1/2}$ der enzymatischen Reaktion (Abbildung 1).

Um die Legumain-vermittelte Spaltung an repräsentativen Beispielen zu zeigen, wurden als Substrate im Legumain-Assay die stereoisomeren Modellverbindungen A (=Referenzbeispiel R3r-LLL) und B (=Referenzbeispiel R3r-LDL) mit S bzw. R Konfiguration am zentralen Alanin-Baustein hergestellt. Verbindung A wurde unter den oben beschriebenen Bedingungen mit einer Halbwertszeit von 1.1h zur Zielverbindung gespalten. Verbindung B wurde innerhalb von 24 h zu ca. 20% zur Zielverbindung gespalten.

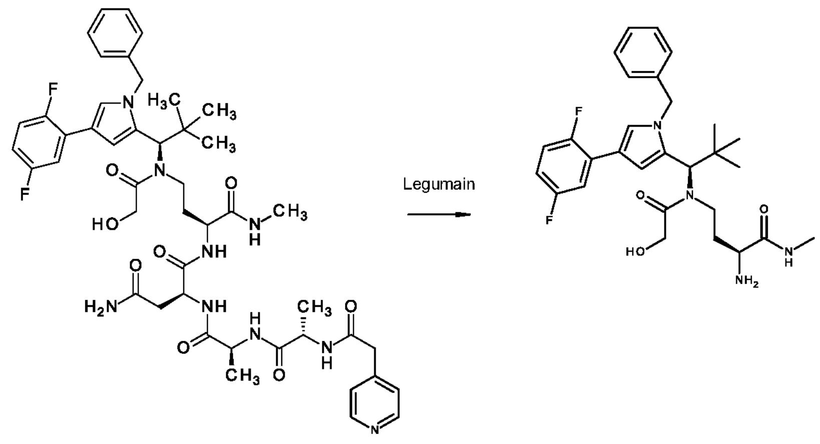


Modellverbindung A

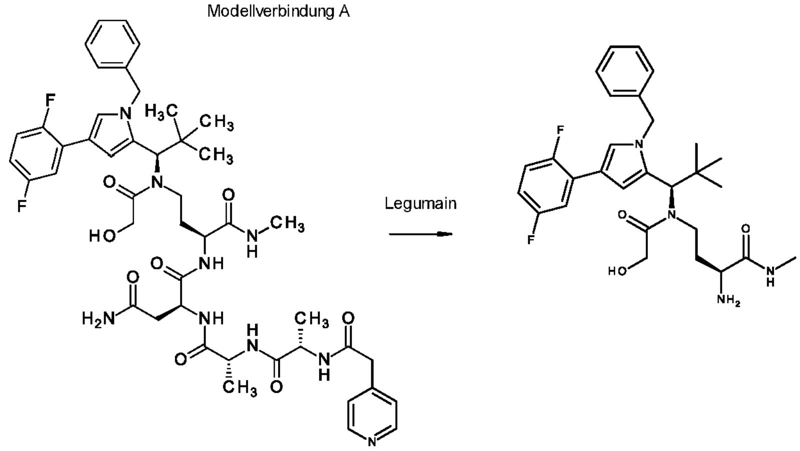


Modellverbindung B

Abbildung 1: Legumain-vermittelte Spaltung der Modellverbindungen A (Referenzbeispiel R3r-LLL) und B (Referenzbeispiel R3r-LDL)

**c: Assay zur Ermittlung der Stabilität in Rattenplasma**

Zur Untersuchung der Stabilität von Verbindung A und B wurden jeweils 1 mL Rattenplasma in 1.5 mL Eppendorftubes auf einem Eppendorfschüttler auf 37°C temperiert. Es wurde eine Stammlösung (9 Aectonitril/ 1 DMSO) mit einer Konzentration von 100 μg/mL für Verbindung A und Verbindung B hergestellt. Jeweils 10 μL der Stammlösung wurden in 1 mL temperiertes Rattenplasma pipettiert, sodass sich eine Konzentration von 1μg/mL ergab.

Die Proben wurden bei 450 rpm über 24 h bei 37°C gehalten. Bei den Entnahmezeitpunkten 0, 0.25h, 0.5h, 1h, 2h, 4h, 6h und 24h wurden jeweils 50 μL entnommen und zu 150 μL Methanol pipettiert. Interner Standard wurde mit einer Konzentration von 0.05 μg/mL im Methanol vorgelegt. Nach kurzem vortexen wurden 300 μL 10 mM Ammoniumacetat Puffer (pH 6.8) zu gegeben und bei 1881g 10 min zentrifugiert. Die Proben wurden dann anschließend mittels RP-HPLC-Analyse und durch LC-MS Analytik untersucht.

In diesem Assay waren sowohl Verbindung A (Referenzbeispiel R3r-LLL) als auch Verbindung B (Referenzbeispiel R3r-LDL) über 24h stabil.

**d: Assay zur Ermittlung der Stabilität in Rattenleberlysosomen**

Zur lysosomalen Stabilitätsuntersuchung der Verbindungen A und B wurden lysosomale Enzyme aus Rattenleberzellen isoliert. Zu diesem lysosomalen Extrakt wurde jeweils Verbindung A und B zugegeben, um die Stabilität unter lysosomalen Bedingungen zu untersuchen. Das proteolytische Enzym Legumain ist in Rattenleberlysosomen nicht oder nur in sehr geringem Maße expremiert (Chen, J-M. *et al* 1997). Zur Kontrolle der enzymatischen Aktivität der lysosomalen Enzyme wurde ein Cathepsin spezifisches Substrat zugegeben.

Zunächst wurde eine frische Rattenleber entnommen, gewogen und sofort auf Eis in Homogenisierungs-Medium (0.25 M Saccharose, 1 mM EDTA, 10 mM HEPES, pH7) gelegt. Die Leber wurde zerkleinert und ein Medienwechsel vorgenommen. Die Homogenisierung der Rattenleber erfolgte mit der 4-fachen Menge des Rattenlebergewichts bei 750 rpm in einem Potter (B. Braun). Das Homogenat wurde 10 min bei 1000g zentrifugiert und der Überstand filtriert. Im nächsten Schritt wurde mithilfe einer Ultrazentrifuge die "leichte mitochondriale Fraktion" (LMF) aus dem Überstand bei 26500g über 20 min abzentrifugiert. Im Pellet sind außer Mitochondrien auch die Lysosomen enthalten. Der Überstand wurde verworfen und das Pellet mit 0.8 mL/g Homogenisierungs-Medium resuspendiert.

Um die lysosomale Fraktion von den übrigen Zellbestandteilen der LMF abzutrennen wurden 6 Optiprep Dichtegradienten mit 8, 12, 16, 19, 22.5 und 27% Sucroseanteil im Optiprep Puffer (100 mM MOPS, 20mM EDTA, 0.5% EtOH, pH 7.6) hergestellt. Die Sucrose wurde aus einer 2.3M Stammlsöung in der jeweiligen Prozentigkeit zugegben. In die Dichtestufe mit 19% Sucroseanteil wurden zusätzlich 2.5 mL der isolierten LMF gegeben. Anschließend wurden die Dichtestufen in 10 mL Zentrifugenröhrchen übereinandergeschichtet und bei 48500g 17h zentrifugiert. Die Fraktionen 1-8 liegen in den oberen 5.6 mL des Gradienten und wurden verworfen. Die Fraktionen 9 und 10 befinden sich in den darunter liegenden 1.6 mL und wurden aus dem Gradient entnommen und mit 1.6 mL Lysepuffer (25 mM HEPES, 150 mM NACl, 0.1% Triton X100, pH 5) 5 min auf Eis lysiert. In Fraktion 9 und 10 sind die Lysosomen enthalten. Zur Lysekontrolle wurde der Proteingehalt der lysierten lysosomalen Fraktion mit Hilfe eines BCA assays (Pierce BCA Protein Assay Kit) kontrolliert.

Zur Untersuchung der lysosomalen Stabilität der Verbindungen A und B wurden nun 6 μL einer 100 μg/mL Stammlösung (9 Aectonitril/ 1 DMSO) zu 290 μL 90 mM Citratpuffer und 300 μL lysosmalem Extrakt gegeben und bei 37°C auf dem Eppendorfschüttler inkubiert. Es wurden nach 0h, 1h, 2h, 6h, 24h und 48h je 50 μL aus der Inkubationslösung entnommen und zu 150 μL MeOH pipettiert. Interner Standard wurde mit 0.05 μg/mL vorgelegt. Die Proben wurden final zur RP-HPLC LCMS Analyse mit 300 μL 10 mM Amoniumacetatpuffer (pH 6.8) verdünnt und gemessen.

Unter den Bedingungen des lysosomalen Stabilitätsassays wurde Verbindung A (Referenzbeispiel R3r-LLL) mit einer Halbwertszeit von etwa 6 h innerhalb von 24h zu etwa 80% gespalten, während Verbindung B (Referenzbeispiel R3r-LDL) im gleichen Zeitraum stabil war.

## C-2 Internalisierungsassay

Internalisierung ist der Schüsselprozess, um eine spezifische und effiziente Bereitstellung der zytotoxischen Payload in Antigen-exprimierenden Krebszellen durch Antikörper-Drug-Konjugate (ADC) zu ermöglichen. Dieser Prozess wird über Fluoreszenzmarkierung von spezifischen Antikörpern und einem Isotyp-Kontrollantikörper verfolgt. Hierzu wurde zunächst die Konjugation des Fluoreszenzfarbstoffes an Lysine des Antikörpers durchgeführt. Die Konjugation erfolgte mit zweifach molarem Überschuss von CypHer 5E mono NHS ester (Batch 357392, GE Healthcare) bei pH 8, 3. Nach erfolgter Kupplung wurde die Reaktionsmischung gelchromatographisch aufgereinigt (Zeba Spin Desalting Columns, 40K, Fa. Thermo Scientific, No. 87768; Elutionspuffer: DULBECCO'S PBS, Fa. Sima-Aldrich, No. D8537), um überschüssigen Farbstoff zu eliminieren und den pH-Wert zu adjustieren. Die Ankonzentrierung der Proteinlösung erfolgte mittels VIVASPIN 500 Säulen (Fa Sartorius stedim biotec). Die Bestimmung der dye load des Antikörpers erfolgte mittels spektrophotometrischer Analyse (Fa. NanoDrop) und anschließender Berechnung

$$(D: P = A_{dye}\,\varepsilon_{protein}:(A_{280}-0,16A_{dye})\varepsilon_{dye}).$$

Die dye load der hier untersuchten Antikörpern sowie der Isotyp-Kontrolle lagen in vergleichbarer Größenordnung. In Zellbindungs-Assays wurde getestet, dass die Kupplung zu keiner Affinitätsänderung der Antikörper führte.

Die markierten Antikörper wurden im Internalisierungs-Assays eingesetzt. Vor dem Behandlungsstart wurden Zellen ($2\times10^4$/well) in 100μL Medium in einer 96-MTP ausgesät (fat, black, clear bottom No 4308776, Fa. Applied Biosystems). Nach 18h Inkubation bei 37°C/5%$CO_2$ wurde das Medium gewechselt und markierte Antikörper in variierender Konzentration hinzugefügt (10, 5, 2.5, 1, 0.1μg/mL). Das gleiche Behandlungsschema erfolgte mit der markierten Isotyp-Kontrolle (negative Kontrolle). Die gewählten Inkubationszeiten waren 0h, 0,25h, 0,5h, 1h, 1,5h 2h, 3h, 6h and 24h. Die Fluoreszenz-Messung wurde mit Hilfe des InCellAnalyzer 1000 (Fa. GE Healthcare) durchgeführt. Es erfolgte eine kinetische Evaluierung über die Messung der Parameter granule counts/cell und totale granule intensity/cell.

Antikörper wurden nach Bindung an den Rezeptor auf ihre Internalisierungsfähigkeit hin untersucht. Hierzu wurden Zellen mit verschiedenen Expressionsleveln des Rezeptors gewählt. Es konnte Target-vermittelte spezifische Internalisierung mit den im Rahmen der Erfindung genutzten Antikörpern beobachtet werden, wohingegen die Isotyp-Kontrolle keine Internalisierung zeigte.

## C-3 In vitro Tests zur Bestimmung der Zell-Permeabilität

Die Zell-Permeabilität einer Substanz kann mittels *in vitro*-Testung in einem Flux-Assay unter Verwendung von

Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (AB SCIEX Deutschland GmbH, Darmstadt, Deutschland) gekoppelt. Die Permeabilität wurde anhand eines $P_{app}$-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von $P_{app}$ (B-A) zu $P_{app}$ (A-B) (efflux ratio) >2 oder <0.5 war.

Von entscheidender Bedeutung für Toxophore, die intrazellulär freigesetzt werden, sind die Permeabilität von B nach A [$P_{app}$ (B-A)] und das Verhältnis von $P_{app}$ (B-A) zu $P_{app}$ (A-B) (efflux ratio): Je niedriger diese Permeabilität ist, desto langsamer sind die aktiven und passiven Transportvorgänge der Substanz durch die Monoschicht von Caco-2-Zellen. Gibt das efflux ratio zudem keine Hinweise auf aktiven Transport, kann die Substanz nach intrazellulärer Freisetzung länger in der Zelle verweilen. Damit steigt auch die Zeit, die für eine Interaktion mit dem biochemischen Target (hier: Kinesin-Spindelprotein, KSP / Eg5) zur Verfügung steht.

In der folgenden Tabelle 3 sind Permeabilitätsdaten repräsentativer Ausführungsbeispiele aus diesem Assay aufgeführt:

**Tabelle 3**

| Ausführungsbeispiel | $P_{app}$ (B-A) [nm/s] | Efflux ratio |
|---|---|---|
| M1 | 7.8 | 4 |
| M2 | 4.8 | 6.4 |
| M3 | 1.4 | 1.3 |
| M4 | 21.3 | 18.7 |
| M5 | 20.3 | 26.5 |
| M6 | 1.7 | 0.7 |
| M7 | 5.6 | 2.2 |
| M9 | 213 | 16 |
| M10 | 2.0 | 0.4 |
| M11 | 24.3 | 27.7 |
| M12 | 3.3 | 1.8 |
| M13 | 7.1 | 3.6 |
| M14 | 12.7 | 6.6 |
| M15 | 6.4 | 4.4 |
| M16 | 9.0 | 7.0 |
| M17 | 93.6 | 81.5 |
| M18 | 1.6 | 2.9 |
| M19 | 1.9 | 2.9 |
| M20 | 0.9 | 0.2 |
| M21 | 0.5 | 1.5 |
| M22 | 0.9 | 0.9 |
| M23 | 2.8 | 2.0 |
| M24 | 3.9 | 1.0 |
| M25 | 8.1 | 3.6 |
| M26 | 13.0 | 9.6 |

(fortgesetzt)

| Ausführungsbeispiel | $P_{app}$ (B-A) [nm/s] | Efflux ratio |
|---|---|---|
| M27 | 13.2 | 11.9 |
| M28 | 1.9 | 1.0 |
| M29 | 4.1 | 5.3 |

## C-4 In vitro Tests zur Bestimmung der Substrateigenschaften für P-Glycoprotein (P-qp)

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines $P_{app}$-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis $P_{app}$ (B-A) zu $P_{app}$ (A-B) >2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

## C-5 Pharmakokinetik

### C5a: Identifizierung der ADC-Metabolite nach Internalisierung in vitro

**Methodenbeschreibung**:

Internalisierungsuntersuchungen mit Immunkonjugaten werden durchgeführt, um intrazellulär entstandene Metaboliten zu analysieren. Hierzu werden humane Lungentumorzellen NCI H292 ($3\times10^5$/well) in 6-well Platten ausgesät und über Nacht inkubiert (37 °C, 5% $CO_2$). Es erfolgt eine Behandlung mit 10 μg/mL (66 nM) des zu untersuchenden ADCs. Die Internalisierung wurde bei 37 °C und 5% $CO_2$ durchgeführt. Zu verschiedenen Zeitpunkten (0, 4, 24, 48, 72h) werden Zellproben zur weiteren Analyse genommen. Zunächst werden die Überstände (ca. 5 mL) geerntet und nach erfolgter Zentrifugation (2 min, RT, 1000 rpm Heraeus Variofuge 3.0R) bei -80 °C gelagert. Die Zellen werden mit PBS gewaschen, mit Accutase abgelöst und die Zellzahl bestimmt. Nach erneutem Waschen wird eine definierte Zellzahl ($2\times10^5$) mit 100 mL Lysis Puffer (Mammalian Cell Lysis Kit (Sigma MCL1) versetzt und unter ständigem Schütteln (Thermomixer, 15min, 4°C, 650 rpm) in Protein LoBind tubes (eppendorf Cat.No. 0030 108.116) inkubiert. Nach der Inkubation wird das Lysat zentrifugiert (10min, 4°C, 12000g, eppendorf 5415R) und der Überstand geerntet. Der gewonnene Überstand wird bei -80 °C gelagert. Alle Proben werden anschließend wie folgt analysiert.

Die Messung der Verbindungen im Kulturüberstand bzw. Zellysat erfolgt nach Fällung der Proteine mit Methanol oder Acetonitril durch eine Hochdruck-Flüssigkeits-Chromatographie (HPLC) gekoppelt an ein Triple-Quadrupol-Massenspektrometer (MS).

Zur Aufarbeitung von 50 μL Kulturüberstand/Zelllysat werden diese mit 150 μL Fällungsreagenz (Methanol) versetzt und für 10 Sekunden geschüttelt. Das Fällungsreagenz enthält einen internen Standard (ISTD) in geeigneter Konzentration (in der Regel im Bereich 20-100 μg/L). Nach dem 10minütigen Zentrifugieren bei 1881g wird der Überstand in ein Autosampler-Vial überführt, mit 300 μL eines auf das Laufmittel abgestimmten Puffers aufgefüllt und erneut geschüttelt und 10 min bei 1881g zentrifugiert. Die Messung der Zelllysat- und Überstandsproben erfolgt schließlich an dem mit einer HPLC gekoppelten Triple-Quadrupol-Massenspektrometer API6500 der Firma AB SCIEX Deutschland GmbH.

Zur Kalibrierung wird Leerlysat bzw. Leerüberstand mit entsprechenden Konzentrationen (0.1 - 1000 μg/L)

versetzt. Die Nachweisgrenze (LLOQ) liegt bei ca. 0.2 μg/L.

Qualitätskontrollen zur Gültigkeitsprüfung enthalten 4 und 40 μg/L.

**C5b: Identifizierung der ADC-Metabolite in vivo**

Nach i.v. Applikation von 10 mg/kg verschiedener erfindungsgemäßer Konjugate in Xenograft Mäuse können 24h nach Applikation dieser Konjugate, die Plasma-, Tumor-, Leber- und Nierenkonzentrationen des Antikörpers sowie potentiell auftretender Metabolite gemessen werden. Unter C-6 ist eine genauere Methodenbeschreibung bezüglich der Xenograft Modelle zu finden. Hier soll nur auf die Metabolitenkonzentrationen der erfindungsgemäßen Konjugate eingegangen werden. Die Messwerte der Metabolite in den genannten Matrizes geben darüber Aufschluss, wie stark die Belastung mit Metabolit in Plasma, Niere und Leber ausgeprägt ist im Vergleich zur Belastung im Tumor.

**Analytik zur Quantifizierung der potentiell auftretenden Metabolite**

Die Messung der Verbindungen in Plasma, Tumor, Leber und Niere erfolgt nach Fällung der Proteine mit in der Regel Methanol durch eine Hochdruck-Flüssigkeits-Chromatographie (HPLC) gekoppelt an ein Triple-Quadrupol-Massenspektrometer (MS).

Zur Aufarbeitung von 50 μL Plasma werden diese mit 150 μL Fällungsreagenz (in der Regel Methanol) versetzt und für 10 sec geschüttelt. Das Fällungsreagenz enthält einen internen Standard (ISTD) in geeigneter Konzentration (in der Regel im Bereich 20-100 μg/ L). Nach dem 10minütigen Zentrifugieren bei 1881g wird der Überstand in ein Autosampler-Vial überführt, mit 300 μL eines auf das Laufmittel abgestimmten Puffers aufgefüllt und erneut geschüttelt.

Bei der Aufarbeitung von Tumor- oder Organmaterial wird das jeweilige Material mit der 3-20 fachen Menge an Extraktionspuffer versetzt. Der Extraktionspuffer enthält 50 mL Tissue Protein Extraction Reagent (Pierce, Rockford, IL), zwei Pellets Complete-Protease-Inhibitor-Cocktail (Roche Diagnostics GmbH, Mannheim, Deutschland) und Phenylmethylsulfonylfluorid (Sigma, St. Louis, MO) in einer finaler Konzentration von 1 mM. Je nach Gewebetyp (hart: Tumor; weich: Leber, Niere) wird das Lyse und Homogeniserungsprogramm des Prescellys 24 Lysis and Homogenization Gerätes (Bertin Technologies) ausgewählt (www.prescellys.com). Die homogenisierten Proben werden über Nacht bei 4°C stehen gelassen. 50 μL des Homogenats werden in ein Autosampler-Vial überführt und mit 150 μL Methanol inklusive ISTD aufgefüllt und 10 sec geschüttelt und darauf 5 min stehen gelassen. Nach Zugabe von 300 μL Ammoniumacetat Puffer (pH6.8) und kurzem Schütteln wird die Probe 10min bei 1881g zentrifugiert.

Zur Kalibrierung wird für Plasmaproben Plasma und für Gewebeproben entsprechende Leermatrix mit Konzentrationen von 0.6 - 1000 μg/L versetzt. Die Nachweisgrenze (LOQ) liegt je nach Probentyp bzw. Gewebetyp zwischen 1 und 20 μg/L.

Die Messung der Plasma und Matrixproben erfolgt schließlich an dem mit einer HPLC gekoppelten Triple-Quadrupol-Massenspektrometer API6500 der Firma AB SCIEX Deutschland GmbH.

Qualitätskontrollen zur Gültigkeitsprüfung enthalten 4, 40 und 400 μg/L.

Tabelle 4: Metaboliten Konzentrationen (MW:Mittelwert, SD:Standardabweichung) in Tumor, Plasma, Leber und Niere von Xenograft Mäusen (n=3) 24 h nach Applikation erfindungsgemäßer Konjugate in Ku-19-19 Xenograft Mäuse. LLOQ Tumor: 3-20 μg/L; LLOQ Plasma: 1μg/L; LLOQ Leber und Niere: 5μg/L.

Tabelle 4:

| | | M26 MW (μg/L) | SD (μg/L) |
|---|---|---|---|
| Tumor | R10k | 151.7 | 11.8 |
| | Beispiel 3k | 76.3 | 2.7 |
| Plasma | R10k | 3.2 | 0.05 |
| | Beispiel 3k | <LLOQ | <LLOQ |
| Leber | R10k | 209.4 | 134.0 |
| | Beispiel 3k | 26.0 | 6.3 |
| Niere | R10k | 107.6 | 10.2 |
| | Beispiel 3k | 49.3 | 10.2 |

Die ADCs aus Beispiel 3k und Referenzbeispiel R10k (mit und ohne Legumain-spaltbare Gruppe) zeigen eine vergleichbare *in vivo* Wirkung im Ku-19-19 Modell (vgl. Kapitel C-6b). Ebenso zeigt das ADCs aus Beispiel 3h1 eine

mindestens vergleichbare *in vivo* Wirkung im A498 Modell (vgl. Kapitel C-6c) als die Referenzbeispiel R10h1 (mit und ohne Legumain-spaltbare Gruppe).

Nach Applikation des ADCs aus Beispiel 3k werden jedoch deutlich niedrigere Konzentrationen des aktiven Metaboliten insbesondere in der Leber gemessen als nach Applikation des Vergleichs-ADCs R10k. Die niedrigen Konzentrationen des aktiven Metaboliten M26 in der Leber nach Applikation des ADCs aus Beispiel 3k (26μg/L] gegenüber dem Referenz-ADC ohne enzymatisch spaltbare Gruppe aus Beispiel R10k (209.4 μg/L) stehen in Einklang mit der in Kapitel C1c beschriebenen hohen Stabilität der Modellverbindung B (Referenzbeispiel R3r-LDL) in Rattenleberlysosomen.

## C-6 Wirksamkeitstest in vivo

Die Wirksamkeit der erfindungsgemäßen Konjugate wurde in vivo beispielsweise mittels Xenograft-Modellen getestet. Der Fachmann kennt Methoden im Stand der Technik, anhand derer die Wirksamkeit der erfindungsgemäßen Verbindungen getestet werden kann (siehe z.B. WO 2005/081711; Polson et al., Cancer Res. 2009 Mar 15;69(6):2358-64). Beispielsweise wurde hierzu Nagern (z.B. Mäusen) eine Tumorzelllinie, welche das Zielmolekül des Binders exprimiert, implantiert. Anschließend wurde den Implantat-Tieren entweder ein erfindungsgemäßes Konjugat, ein Isotyp-Antikörper-Kontrollkonjugate oder ein Kontrollantikörper oder isotonische Salzlösung appliziert. Die Applikation erfolgte einmalig oder öfter. Nach einer Inkubationszeit von mehreren Tagen wurde die Tumorgröße im Vergleich von Konjugat-behandelten Tieren und der Kontrollgruppe bestimmt. Die Konjugat-behandelten Tiere zeigten eine geringere Tumorgröße.

### C-6a. Wachstumshemmung / Regression von experimentellen Tumoren in der Maus

Humane Tumorzellen, die das Antigen für das Antikörper-Wirkstoffkonjugat exprimieren, werden subkutan in die Flanke von immunsupprimierten Mäusen inokuliert, beispielsweise NMRi Nude- oder SCID-Mäuse. 1-10 Millionen Zellen werden aus der Zellkultur abgelöst, zentrifugiert und mit Medium oder Medium / Matrigel resuspendiert. Die Zellsuspension wird unter die Haut der Maus gespritzt.

Innerhalb von einigen Tagen wächst ein Tumor heran. Die Behandlung beginnt nach Etablierung des Tumors, ungefähr bei einer Tumorgröße von 40 $mm^2$. Um die Wirkung auf größere Tumoren zu untersuchen, kann die Behandlung auch erst bei einer Tumorgröße von 50-100 $mm^2$ begonnen werden.

Die Behandlung mit APDCs und ADCs erfolgt über die intravenöse (i.v.) Route in die Schwanzvene der Maus. Das ADC wird mit einem Volumen von 5 mL / kg appliziert.

Das Behandlungsschema richtet sich nach der Pharmakokinetik des Antikörpers. Als Standard wird drei Mal in Folge jeden vierten Tag behandelt. Bei langsam wachsenden Tumoren bietet sich eine wöchentliche Behandlung an. Für eine zeitnahe Beurteilung kann sich auch ein Schema mit einer Einmalbehandlung eignen. Die Behandlung kann aber auch weiter fortgesetzt werden oder es kann sich zu einem späteren Zeitpunkt ein zweiter Zyklus mit drei Behandlungstagen anschließen.

Standardmäßig werden 8 Tiere pro Behandlungsgruppe eingesetzt. Neben den Gruppen, die die Wirksubstanzen bekommen, wird eine Gruppe als Kontrollgruppe nur mit dem Puffer nach dem gleichen Schema behandelt.

Im Verlauf des Experiments wird die Tumorfläche regelmäßig mit einer Schieblehre in zwei Dimensionen (Länge / Breite) gemessen. Die Tumorfläche wird mittels Länge x Breite bestimmt. Der Vergleich der mittleren Tumorfläche der Behandlungsgruppe mit der Kontrollgruppe wird als T/C area angegeben.

Werden alle Gruppen des Experimentes nach Behandlungsende gleichzeitig beendet, können die Tumore entnommen und gewogen werden. Der Vergleich der mittleren Tumorgewichte der Behandlungsgruppe mit der Kontrollgruppe wird als T/C weight angegeben.

### C-6b. Wirksamkeit der anti-TWEAKR-APDCs in verschiedenen Xenograftmodellen

Die Tumorzellen (z.B. KU-19-19, NCI-H292, SCC4) werden subkutan in die Flanke von weiblichen NMRI-nude oder NOD.SCID Mäusen (Janvier) inokuliert. Bei einer Tumorgröße von ~40 $mm^2$ wird intravenös mit dem Antikörper-Wirkstoffkonjugat behandelt. Im Anschluss an die Behandlung wird das Tumorwachstum ggf. weiterverfolgt.

Die Behandlung mit anti-TWEAKR Antikörper-Prodrug Konjugaten (APDCs) führt zu einer deutlichen und lang anhaltenden Wachstumshemmung der Tumore im Vergleich zur Kontrollgruppe und dem Isotyp-Wirkstoffkonjugat und ist mit der Wachstumshemmung des entsprechenden ADCs ohne Legumain-spaltbare Gruppe (R10k) vergleichbar. Die Tabelle 5 gibt die T/C Werte an, ermittelt über die Tumorfläche am Tag mit dem letzten Messwert der Kontrollgruppe gerechnet nach Behandlungsstart. Auch legumain-spaltbare anti-TWEAKR ADCs (e.g. Beispiel 24l1) zeigten eine potente und spezifische anti-Tumorwirkung.

**Tabelle 5:**

| Beispiel | Tumor Modell | Dosis | Dosissche ma | T/C area |
|---|---|---|---|---|
| 3k | KU-19-19 (humanes Blasenkarzinom) | 5 mg/kg | Q7dx3 | 0.39 (Tag 15) |
| R10k | | 5mg/kg | Q7dx3 | 0.32 (Tag 15) |
| Isotyp-Wirkstoffkonjugat | | 5 mg/kg | Q7dx3 | 0.81 (Tag 15) |
| 3k | NCI-H292 (humanes Lungenkarzinom) | 5 mg/kg | SD (single dose) | 0.63 (Tag 20) |
| 3L1 | NCI-H292 | 5 mg/kg | Q7dx3 | 0.09 (Tag 25) |
| 3L1 | KU-19-19<br>KU-19-19<br>KU-19-19 | 5 mg/kg<br>2.5 mg/kg<br>1 mg/kg | Q7dx3<br>Q7dx3<br>Q7dx3 | 0.30 (Tag 25)<br>0.67 (Tag 25)<br>1.01 (Tag 25) |
| 3L2 | NCI-H292 | 10 mg/kg | SD | 0.39 (Tag 29) |
| 16l2 | NCI-H292 | 10 mg/kg | SD | 0.18 (Tag 29) |
| 24l1 | NCI-H292 | 5 mg/kg | SD | 0.38 (Tag 21) |
| 28l1 | KU-19-19 | 5 mg/kg | Q7dx3 | 0.25 (Tag 25) |
| 28l1 | NCI-H292 | 5 mg/kg | SD | 0.31 (Tag 21) |
| 28l1 | SCC4 (humanes Zungengrundkarzi nom) | 5 mg/kg | Q7dx3 | 0.30 (Tag 39) |
| 31l1 | KU-19-19<br>KU-19-19<br>KU-19-19 | 5 mg/kg<br>2.5 mg/kg<br>1 mg/kg | Q7dx3<br>Q7dx3<br>Q7dx3 | 0.44 (Tag 25)<br>0.68 (Tag 25)<br>0.97 (Tag 25) |
| 31l1 | NCI-H292<br>NCI-H292 | 5 mg/kg<br>2.5 mg/kg | SD<br>SD | 0.29 (Tag 21)<br>0.64 (Tag 21) |
| 35l1 | NCI-H292 | 5 mg/kg | SD | 0.27 (Tag 21) |
| 37l1 | NCI-H292 | 5 mg/kg | SD | 0.30 (Tag 21) |

### C-6c. Wirksamkeit der anti-B7H3-APDCs in verschiedenen Xenograftmodellen

Die Tumorzellen (z.B. U251-MG, NCI-H292, SCC4, NCI-H1703) werden subkutan in die Flanke von weiblichen NMRI-nude oder NOD.SCID Mäusen (Janvier) inokuliert. Bei einer Tumorgröße von ~40 $mm^2$ wird intravenös mit dem Antikörper-Wirkstoffkonjugat behandelt. Im Anschluss an die Behandlung wird das Tumorwachstum ggf. weiterverfolgt.

Die Behandlung mit anti-B7H3 Antikörper-Prodrug Konjugaten (APDC) führt zu einer deutlichen und lang anhaltenden Wachstumshemmung der Tumore im Vergleich zur Kontrollgruppe und dem Isotyp-Wirkstoffkonjugat. In Tabelle 6 sind die T/C Werte von verschiedenen anti-B7H3 APDCs sowie der T/C Wert der Referenzverbindung R10h1, ermittelt über die Tumorfläche am Tag mit dem letzten Messwert der Kontrollgruppe gerechnet nach Behandlungsstart in verschiedenen Xenograft-Modellen angegeben.

**Tabelle 6:**

| Beispiel | Tumor Modell | Dosis | Dosissche ma | T/C area |
|---|---|---|---|---|
| 3h1 | A498 (humanes Nierenzellkarzino m) | 10 mg/kg | Q7dx3 | 0.33 (Tag 52) |
| R10h1 | A498 | 10 mg/kg | Q7dx3 | 0.43 (Tag 52) |
| 3h1 | U251 MG (humanes Glioblastom) | 5 mg/kg | Q7dx3 | 0.28 (Tag 34) |
| 3h1 | SCC4 | 10 mg/kg | Q7dx3 | 0.28 (Tag 31) |
| 3h2 | NCI-H1703 (humanes Lungenkarzinom) | 10 mg/kg | Q7dx3 | 0.10 (Tag 33) |
| 3h2 | SCC4 | 10 mg/kg | Q7dx3 | 0.001 (Tag 51) |
| 16h1 | U251-MG | 5 mg/kg | Q7dx3 | 0.17 (Tag 47) |

(fortgesetzt)

| Beispiel | Tumor Modell | Dosis | Dosissche ma | T/C area |
|---|---|---|---|---|
| 16h1 | NCI-H292 | 10 mg/kg | SD | 0.29 (Tag 22) |
| 22h1 | U251-MG | 5 mg/kg | Q7dx3 | 0.38 (Tag 47) |
| 22h1 | NCI-H292 | 10 mg/kg | SD | 0.83 (Tag 22) |

**Patentansprüche**

**1.** Verbindung der folgenden Formel Ia:

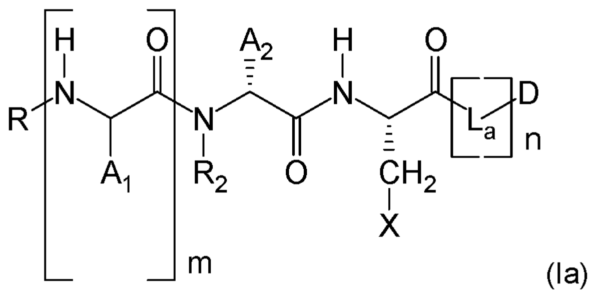

(Ia),

in der

m 0 bis 2 ist;
n 0 oder 1 ist;
X für $-C(=O)-NH_2$ oder -COOH steht,
$L_a$ für einen *self-immolative linker* steht,
$A_1$ für einen Rest einer der Aminosäuren Gly, Pro, Ala, Val, Nva, Leu, Ile, Met, Phe, Tyr, Trp, Ser, Thr, Cys, Asn, Gin, Asp, Glu, Lys, Arg, Citrullin oder His, oder einer der jeweiligen N-Alkyl-Aminosäuren steht,
$A_2$ für einen Rest einer der Aminosäuren D-Ala, D-Pro, D-Ser, D-Asn, D-Asp oder D-His steht,
$R_2$ für -H- oder $C_1$-$C_3$-Alkyl steht, oder
$R_2$ für die Bindung zur Methylengruppe des Prolinringes steht, falls $A_2$ für einen Rest von D-Pro steht,
D für $-D_1-(L_b)_o-(LIG)_p$ steht,
$D_1$ für einen cytotoxischen Wirkstoff steht,
LIG für einen Binder steht, der nach Bindung an ein Zielmolekül einer Tumorzelle von der Tumorzelle internalisiert und intrazellulär, vorzugsweise lysosomal, prozessiert wird,
$L_b$ für einen Linker steht,
o und p jeweils unabhängig voneinander 0 oder 1 sind,

R für $Z_1$-(C=O)q- steht,
q 0 oder 1 ist,
$Z_1$ für eine $C_{1-10}$-Alkyl-, $C_{5-10}$-Aryl- oder $C_{6-10}$)-Aralkyl-, $C_{5-10}$)-Heteroalkyl-, $C_{1-10}$-Alkyl-O-$C_{6-10}$-Aryl-, $C_{5-10}$-Heterocycloalkyl-, Heteroaryl-, Heteroaryl-alkyl-, $C_{5-10}$-Heteroaryl-alkoxy-, $C_{1-10}$-Alkoxy-, $C_{6-10}$-Aryl-$C_{1-10}$-alkyloxy-, $C_{6-10}$-Aryloxy- oder $C_{6-10}$)-Aralkoxy-, $C_{5-10}$)-Heteroalkoxy-, $C_{1-10}$)-Alkyl-O-$C_{6-10}$)-Aryloxy- oder $C_{5-10}$-Heterocyclo-alkoxy-Gruppe steht, die ein- oder mehrfach mit $-NH_2$, -C(=O)-, -NH-Alkyl, $-N(Alkyl)_2$, -NH-C(=O)-Alkyl, -N(Alkyl)-C(=O)-Alkyl, $-S(=O)_3-H$, $-S(=O)_2-NH_2$, $-S(=O)_2-N(Alkyl)_2$, -COOH, $-C(=O)NH_2$, $-C(=O)-N(Alkyl)_2$ oder -OH substituiert sein kann, oder für -H oder eine Gruppe $-(CH_2)_{0-1}-O_x-(CH_2CH_2O)_v-R^1$ steht,
x 0 oder 1 ist
v eine Zahl von 1 bis 20 ist,
$R^1$ für -H, -Alkyl, $-CH_2-COOH$, $-CH_2-CH_2-COOH$ oder $-CH_2-CH_2-NH_2$ steht,

oder

R für LIG-$(L_c)_r$- steht,
LIG für einen Binder steht, der nach Bindung an ein Zielmolekül auf einer Tumorzelle von der Tumorzelle internalisiert und intrazellulär, vorzugsweise lysosomal, prozessiert wird,
$L_c$ für einen Linker steht und
r 0 oder 1 ist.

**2.** Verbindung nach Anspruch 1, wobei $R_2$ -H oder eine Methylgrupe, vorzugsweise -H ist.

**3.** Verbindung nach einem oder mehreren der vorhergehenden Ansprüche, wobei m 1 ist.

**4.** Verbindung nach nach einem oder mehreren der vorhergehenden Ansprüche, wobei $A_1$ in der L-Konfiguration oder D-Konfiguration vorliegt.

**5.** Verbindung nach einem oder mehreren der vorhergehenden Ansprüche, wobei $A_1$ für einen Rest der Aminosäure Ala oder Val steht.

**6.** Verbindung nach einem oder mehreren der vorhergehenden Ansprüche, wobei q 1 ist, und $Z_1$ eine $C_{1-10}$-Alkyl-, die einfach oder mehrfach durch ein Sauerstoffatom unterbrochen sein kann (wie z.B, eine Methylgruppe oder eine ggf. alkylierte Oligoalkylenoxidkette), eine $C_{6-10}$)-Aralkyl-, $C_{5-10}$)-Heteroaryl-alkyl-, $C_{6-10}$)-Aralkoxy-, oder $C_{5-10}$)-Heteroaryl-alkoxy-Gruppe darstellt.

**7.** Verbindung nach einem oder mehreren der vorhergehenden Ansprüche, wobei $L_a$ ausgewählt wird aus den folgenden Gruppen:

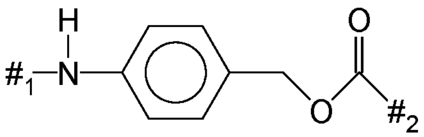

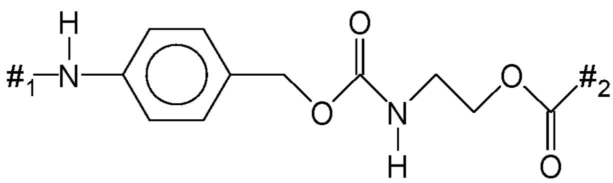

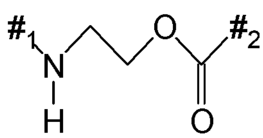

wobei $\#_1$ die Bindung zur Carbonylgruppe und $\#_2$ die Bindung zur Hydroxyl- bzw. Aminogruppe von $D_1$ darstellt.

**8.** Verbindung nach einem oder mehreren der vorhergehenden Ansprüche, wobei $D_1$ einen Wirkstoff darstellt, ausgewählt aus Mitomycin, Doxorubicin, Aminopterin, Actinomycin, Bleomycin, 9-Amino-Camptothecin, n8-Acetyl-spermidin, 1-(2-Chloroethyl)-1,2-dimethansulfonyl-hydrazid, Tallysomycin, Cytarabin, Etoposid, Camptothecin, Taxol, Esperamicin, Podophyllotoxin, Anguidin, Vincristin, Vinblastin, Morpholin-doxorubicin, n-(5,5-diacetoxy-pentyl)-doxorubicin, Duocarmycin, Auristatin, Pyrrolobenzodiazepin-Derivate, Calicheamicin, Daunorubicin, Camptophecin DX8951 (Exatecan) oder einen Kinesin Spindel Protein-Inhibitor (KSP-Inhibitor) verwendet, wobei der Wirkstoff über seine Hydroxyl- oder Aminogruppe an $L_a$ (bei n=1) oder die Carbonylgruppe (bei n=0) gemäß Formel I gebunden ist.

**9.** Verbindung nach Anspruch 8, wobei der KSP-Inhibitor eine Verbindung der folgenden Formel (IIa)

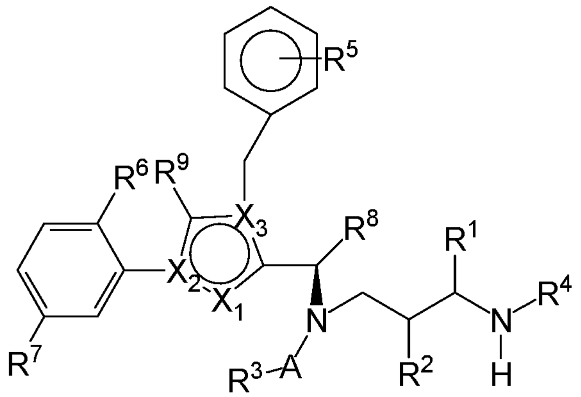

(IIa)

ist, in der

$X_1$ für N,
$X_2$ für N und
$X_3$ für C steht,

oder

$X_1$ für N,
$X_2$ für C und
$X_3$ für N steht,

oder

$X_1$ für CH oder CF,
$X_2$ für C und
$X_3$ für N steht,

oder

$X_1$ für NH,
$X_2$ für C und
$X_3$ für C steht,

oder

$X_1$ für CH,
$X_2$ für N und
$X_3$ für C steht.
A für -C(=O)-, -S(=O)-, $-S(=O)_2-$, oder $-S(=O)_2-NH-$ steht
$R^1$ für -H, -L-#1, -MOD oder $-(CH_2)_{0-3}Z$ steht,
Z für -H, $-NHY^3$, $-OY^3$, $-SY^3$, Halogen, $-C(=O)-NY^1Y^2$, oder $-C(=O)-OY^3$ steht,
$Y^1$ und $Y^2$ unabhängig voneinander für -H, $-NH_2$, $-(CH_2CH_2O)_{0-3}-(CH_2)_{0-3}Z'$ oder $-CH(CH_2W)Z'$ stehen,

$Y^3$ für -H oder $-(CH_2)_{0-3}Z'$ steht,
Z' für -H, $-NH_2$, $-S(=O)_3H$, -COOH, $-NH-C(=O)-CH_2-CH_2-CH(NH_2)C(=O)-$ oder $-(C(=O)-NH-CHY^4)_{1-3}COOH$ steht,
W für -H oder -OH steht,
$Y^4$ für lineares oder verzweigtes $C_{1-6}$ Alkyl steht, das gegebenenfalls mit $-NH-C(=O)-NH_2$ substituiert ist, oder für Aryl oder Benzyl steht, das gegebenenfalls mit $-NH_2$ substituiert ist,
$R^2$ für -H, -L-#1 , -MOD, $-C(=O)-CHY^4-NHY^5$ oder $-(CH_2)_{0-3}Z$ steht,
Z für -H, Halogen, $-OY^3$, $-SY^3$, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z' für -H, $-S(=O)_3H$, $-NH_2$ oder -COOH steht,
$Y^4$ für lineares oder verzweigtes $C_{1-6}$ Alkyl- steht, das gegebenenfalls mit $-NHC(=O)-NH_2$ substituiert ist, oder für Aryl oder Benzyl steht, das gegebenenfalls mit $-NH_2$ substituiertes ist,
$Y^5$ für-H oder $-C(=O)-CHY^6-NH_2$ steht,
$Y^6$ für lineares oder verzweigtes $C_{1-6}$ Alkyl- steht,
$R^3$ für -MOD, -L-#1, oder eine gegebenenfalls substituierte Alkyl-, Cycloalkyl-, Aryl-, Heteroaryl-, Heteroalkyl-, Heterocycloalkyl-Gruppe steht, die mit ein bis drei OH-Gruppen, ein bis drei Halogenatomen, ein bis drei mono-, di- oder tri-halogenierten Alkylgruppen, ein bis drei -O-Alkyl-Gruppen, ein bis drei -SH-Gruppen, ein bis drei -S-Alkyl-Gruppen, ein bis drei -O-C(=O)-Alkyl-Gruppen, ein bis drei -O-C(=O)-NH-Alkyl-Gruppen, ein bis drei -NH-C(=O)-Alkyl-Gruppen, ein bis drei -NH-C(=O)-NH-Alkyl-Gruppen, ein bis drei $-S(=O)_n$-Alkyl-Gruppen, ein bis drei $-S(=O)_2$-NH-Alkyl-Gruppen, 1-3 -NH-Alkyl-Gruppen, ein bis drei $-N(Alkyl)_2$-Gruppen, ein bis drei $NH_2$-Gruppen oder ein bis drei $-(CH_2)_{0-3}Z$-Gruppen substituiert sein können,
n 0, 1 oder 2 ist,
Z für -H, Halogen, $-OY^3$, $-SY^3$, $-NHY^3$, $-C(=O)-NY^1Y^2$ oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H, $-(CH_2)_{0-3}-CH(NHC(=O)CH_3)Z'$,$-(CH_2)_{0-3}-CH(NH_2)Z'$ oder $-(CH_2)_{0-3}Z'$ steht,
Z' für -H, $-S(=O)_3H$, $-NH_2$ oder -C(=O)-OH steht,
$R^4$ für die durch Legumain spaltbare Gruppe der Formeln Ia′, Ia″ und Ia‴ steht,
$R^5$ für -H, $-NH_2$, $-NO_2$, Halogen, -CN, CF3, -OCF3, -CH2F, -CH2F, -SH oder $-(CH_2)_{0-3}Z$ steht,
Z für -H, $-OY^3$, $-SY^3$, Halogen, $-NHY^3$, $-C(=O)-NY^1Y^2$, oder $-C(=O)-OY^3$ steht,
$Y^1$ und $Y^2$ unabhängig voneinander für -H, $-NH_2$, oder $-(CH_2)_{0-3}Z'$ stehen,
$Y^3$ für -H oder $-(CH_2)_{0-3}Z'$ steht,
Z' für -H, $-S(=O)_3H$, $-NH_2$ oder -COOH steht,
$R^6$ und $R^7$ unabhängig voneinander für -H, -CN, $C_{1-10}$-Alkyl, Fluor- $C_{1-10}$-Alkyl, $C_{2-10}$-Alkenyl-, Fluor- $C_{2-10}$-Alkenyl-, $C_{2-10}$-Alkinyl-, Fluor- $C_{2-10}$-Alkinyl-, Hydroxy-, $-NO_2$, $-NH_2$, -COOH oder Halogen stehen,
$R^8$ für $C_{1-10}$-Alkyl, Fluor- $C_{1-10}$-Alkyl, $C_{2-10}$-Alkenyl-, Fluor- $C_{2-10}$-Alkenyl-, $C_{2-10)}$-Alkinyl-, Fluor- $C_{2-10}$-Alkinyl-, $C_{4-10}$-Cycloalkyl-, Fluor-$C_{4-10}$-Cycloalkyl- oder $-(CH_2)_{0-2}-(HZ^2)$ steht,
$HZ^2$ für einen 4 bis 7-gliedrigen Heterocyclus mit bis zu zwei Heteroatome, ausgewählt aus N, O und S steht, der mit-OH, -COOH, $-NH_2$ oder-L-#1 substituiert sein kann,
$R^9$ für -H, -F, $-CH_3$, $-CF_3$, $-CH_2F$ oder $-CHF_2$ steht,
-L-#1 für $-(L_b)_b-(LIG)_b$ steht,
LIG für einen Binder steht, der nach Bindung an ein Zielmolekül einer Tumorzelle von der Tumorzelle internalisiert und intrazellulär und vorzugsweise lysosomal, prozessiert wird,
$L_b$ für einen Linker steht,
o und p unabhängig voneinander für 0 oder 1 stehen,
-MOD für $-(NR^{10})_n-(G1)_o$-G2-G3 darstellt,
$R^{10}$ für -H oder $C_1$-$C_3$-Alkyl- steht,
G1 für -NH-C-(=O)- , -C(=O)-NH- oder

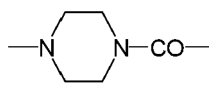

steht,
n 0 oder 1 ist;
o 0 oder 1 ist und
G2 für eine geradkettige oder verzweigte Kohlenwasserstoffkette mit 1 bis 20 Kohlenstoffatomen steht, die einfach oder mehrfach durch -O-, -S-, -S(=O)-, $-S(=O)_2$-, -NRY-, $-NR^yC(=O)-$, $-C(=O)NR^y-$, -NRYNRY-,

-$S(=O)_2$-$NR^yNR^y$-, -C(=O)-$NR^yNR^y$-, -C(=O)-, -$CR^x$=N-O- unterbrochen sein kann und die geradkettige oder verzweigte Kohlenwasserstoffkette mit -NH-C(=O)-$NH_2$, -COOH, -OH, -$NH_2$, Sulfonamid, Sulfon, Sulfoxid, oder Sulfonsäure substituiert sein kann,

$R^y$ für -H, Phenyl-, $C_1$-$C_{10}$-Alkyl-, $C_2$-$C_{10}$-Alkenyl- oder $C_2$-$C_{10}$-Alkinyl- steht, die jeweils mit -NH-C(=O)-$NH_2$, -COOH, -OH, -$NH_2$, Sulfonamid, Sulfon, Sulfoxid, oder Sulfonsäure substituiert sein können,

Rx für -H, $C_1$-$C_3$-Alkyl- oder Phenyl- steht,

G3 für -H oder -COOH steht und

-MOD mindestens eine COOH-Gruppe aufweist, sowie ihre Salze, Solvate und Salze der Solvate.

**10.** Verbindung nach Anspruch 9, wobei $R^1$ oder $R^3$ für -L-#1 stehen.

**11.** Verbindung nach einem oder mehreren der Ansprüche 9 und 10, wobei $X_1$ für CH steht, $X_2$ für C steht und $X_3$ für N steht.

**12.** Verbindung nach einem oder mehreren der Ansprüche 9 bis 11, wobei $R^6$ und $R^7$ unabhängig voneinander füer -H, $C_{1-3}$-Alkyl oder Halogen stehen.

**13.** Verbindung nach Anspruch 12, wobei $R^6$ und $R^7$ für F steht.

**14.** Verbindung nach einem oder mehreren der Ansprüche 9 bis 13, wobei $R^8$ für $C_{1-4}$-Alkyl (vorzugsweise tert-butyl) oder Cylcohexyl steht.

**15.** Verbindung nach einem oder mehreren der Ansprüche 9 bis 14, wobei $R^9$ für -H steht.

**16.** Verbindung nach einem oder mehreren der vorhergehenden Ansprüche, wobei entweder D oder R den Binder LIG enthalten.

**17.** Verbindung nach Anspruch 16, wobei LIG ein Peptid, Protein oder Derivat hiervon ist, ausgewählt aus Octreotid, GnRH-III, [D-$Tyr^6$, β-$Ala^{11}$, $Phe^{13}$, $Nle^{14}$]BN(6-14), NT(8-13), c(RGDfK), HSDAVFTDNYTRLRKQMAVKKYLNSILN-$NH_2$ (SEQ ID NO:
161), NAPamide, [$Phe^7$, $Pro^{34}$]NPY, HER2-targeting peptide, ATEPRKQYATPRVFWTDAPG (SEQ ID NO: 162), LQWRRDDNVHNFGVWARYRL (SEQ ID NO: 163), oder einen Antikörper bzw. ein Fragment oder Derivat hiervon darstellt, der bzw. das an ein extrazelluläres Zielmolekül einer Tumorzelle bindet und der bzw. das ggf. mit weiteren cytotoxischen Wirkstoffen konjugiert ist.

**18.** Verbindungen nach einem oder mehreren der vorhergehenden Ansprüche, wobei die Verbindungen die folgende Formel III'

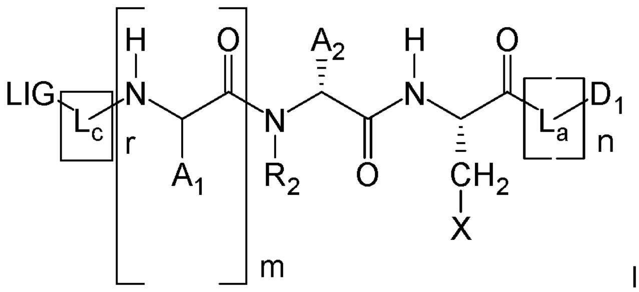

III'

aufweisen, in der

m, n, r, LIG, $L_a$, $L_c$, $D_1$, X, $A_2$, $R_2$ und $A_1$ die unter Anspruch 1 angegebenen Bedeutungen haben.

**19.** Verbindungen der Formel IIIa'

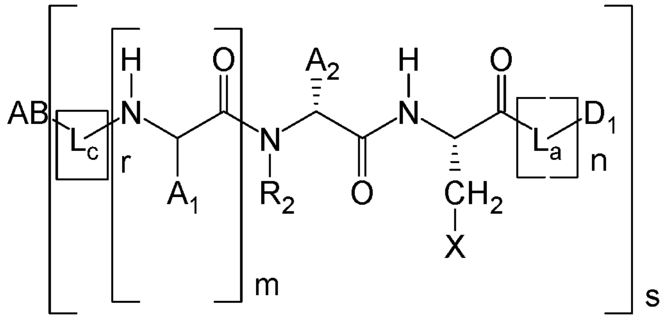

IIIa'

in der

m,n,r,$L_a$, $L_c$, $D_1$, X, $R_2$, $A_2$ dieselbe Bedeutung haben wie in Anspruch 1,
AB für einen Antikörper oder ein Antigen-bindendes Antikörperfragment steht und
s 1 bis 20 ist vorzugsgweise 2 bis 8 ist, besonders bevorzugt 2 bis 6 ist.

**20.** Verbindungen nach einem oder mehreren der Ansprüche 1 bis 17, wobei die Verbindungen die folgende Formel IV'

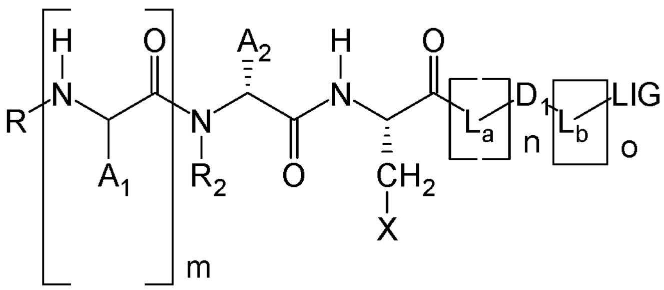

IV'

aufweist, in der

m, n, o, R, LIG, $L_a$, $L_b$,
$D_1$, X, $R_2$, $A_2$ und $A_1$ dieselbe Bedeutung haben wie in Anspruch 1.

**21.** Verbindungen der Formel IVa'

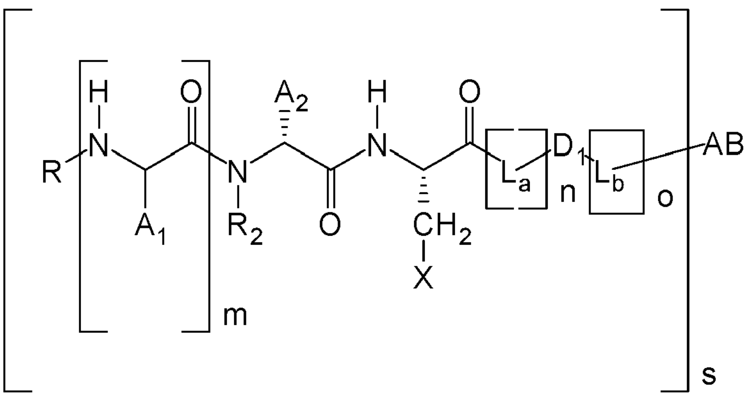

IVa'

in der

m, n, o, R, $L_a$, $L_b$, $D_1$, X, $R_2$, $A_2$ und $A_1$ dieselbe Bedeutung haben wie in Anspruch 1,
AB für einen Antikörper oder ein Antigen-bindendes Antikörperfragment steht und

s 1 bis 20 ist, vorzugsgweise 2 bis 8 ist, besonders bevorzugt 2 bis 6 ist.

**22.** Verbindungen nach einem oder mehreren der vorhergehenden Ansprüche, wobei der Linker $L_b$ bzw. $L_c$ an eine Cystein-Seitenkette bzw. einen Cysteinrest eines Binder wie z.B. eines Antikörpers oder eines Antigen-bindenden Antikörperfragmentes gebunden ist und die folgende Formel aufweist:

§-(C=O)m-L1-L2-§§

wobei

m 0 oder 1 ist;
§ für die Bindung an das Wirkstoffmolekül bzw. die Legumain spaltbare Gruppe steht und
§§ für die Bindung an den Binder steht,
-L2- für die gruppe

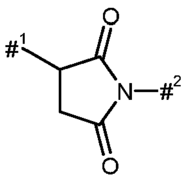 , 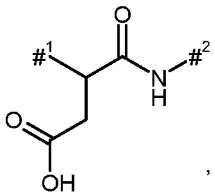 , 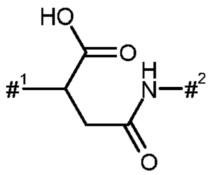 , 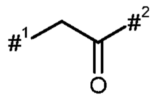 , 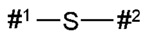 ,

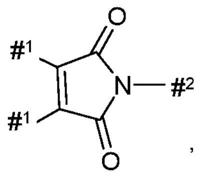 , 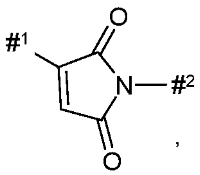 , 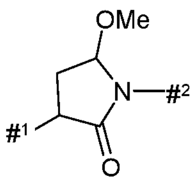 oder 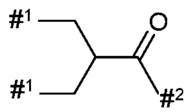

steht, wobei

$\#^1$ die Verknüpfungsstelle mit dem Schwefelatom des Binders kennzeichnet,
$\#^2$ die Verknüpfungsstelle mit der Gruppe $L^1$ kennzeichnet,
L1 für $-(NR^{10})_n-(G1)_o-G2-$ steht,
$R^{10}$ für -H, $-NH_2$ oder $C_1$-$C_3$-Alkyl steht,
G1 für -NHC(=O)- oder

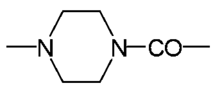

steht,
n 0 oder 1 ist,
o 0 oder 1 ist,
G2 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen steht, die einfach oder mehrfach durch eine oder mehrere der Gruppen -O-, -S-, , -S(=O)-, $-S(=O)_2$, -NH-, -C(=O)-, -Nme-, -NHNH-, $-S(=O)_2$-NHNH-, -NH-C(=O)-, -C(=O)-NH-, -C(=O)-NHNH- und einen 5 bis 10gliedriger, aromatischen oder nicht aromatischen Heterozyklus mit bis zu 4 Heteroatomen, ausgewählt aus N, O und S, -S(=O)-oder $-S(=O)_2$- unterbrochen sein kann (vorzugsweise

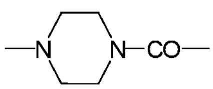

), und wobei die Seitenketten, sofern vorhanden, mit $-NH-C(=O)-NH_2$, -COOH, -OH, $-NH_2$, NH-CNNH$_2$, Sulfo-

namid, Sulfon, Sulfoxid oder Sulfonsäure substituiert sein können, oder eine der folgenden Gruppen

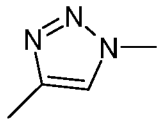,
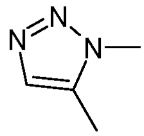,
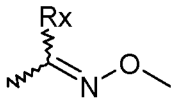

oder

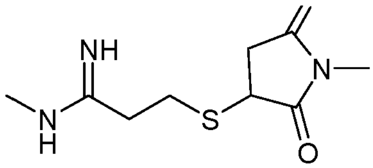

darstellt, wobei
Rx für -H, $C_1$-$C_3$-Alkyl oder Phenyl steht.

**23.** Verbindungen nach Anspruch 22, wobei L2 für eine oder beide der folgenden Formeln

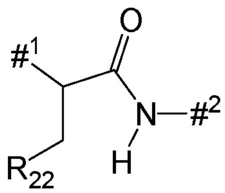

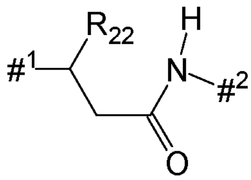

steht, wobei

#[1] für die Verknüpfungsstelle mit dem Schwefelatom des Binders steht,
#[2] für die Verknüpfungsstelle mit der Gruppe $L^1$ steht,
$R^{22}$ für -COOH steht und

die Bindungen an das Schwefelatom des Bindesr zu über 80 %, (bezogen auf die Gesamtanzahl von Bindungen des Linkers an den Binder) in einer dieser beiden Formeln vorliegen.

**24.** Verbindungen nach Anspruch 22 oder 23, wobei die Kohlenwasserstoffkette durch eine der folgenden Gruppen

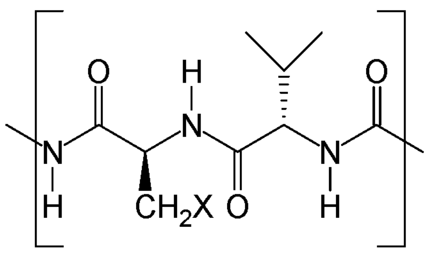

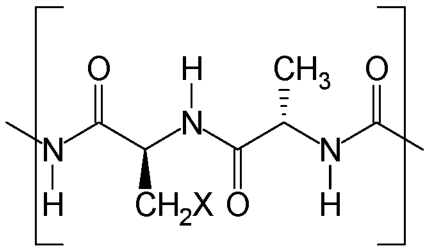

unterbrochen ist, wobei

X für -H oder eine $C_{1-10}$-Alkylgruppe steht, die gegebenenfalls mit -NH-C(=O)-$NH_2$, -COOH, -OH, -$NH_2$, -NH-$CNNH_2$, Sulfon, Sulfoxid oder Sulfonsäure substituiert sein kann.

**25.** Verbindungen nach einem der Ansprüche 22 bis 24, wobei der Linker die folgende Formel

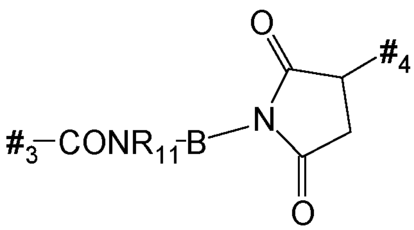

aufweist, in der

#3 für die Bindung an das Wirkstoffmolekül steht,
#4 für die Bindung an den Binder steht,
$R^{11}$ für -H oder -$NH_2$ steht,
B für die Gruppe -$[(CH_2)_x-(X^4)_y]_w-(CH_2)_z$- steht,
w 0 bis 20 ist;
x 0 bis 5 ist;
x 0 bis 5 ist;
y 0 oder 1 ist;
z 0 bis 5 ist; und
$X^4$ für -O-, -C(=O)-NH- , -NH-C(=O)- oder

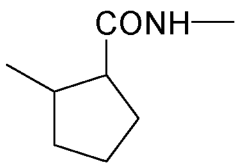

steht.

**26.** Verbindungen nach einem oder mehreren der vorhergehenden Ansprüche, wobei der Linker -$L_b$- und/oder -$L_c$- an eine Cystein-Seitenkette bzw. einen Cysteinrest gebunden ist und die folgende Formel

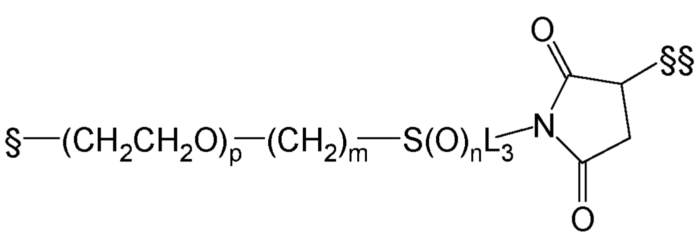

aufweist, in der

§ für die Bindung an das Wirkstoffmolekül bzw. die durch Legumain spaltbare Gruppe steht,
§§ für die Bindung an den Binder steht,
m 0, 1, 2 oder 3 ist,
n 0, 1 oder 2 ist,
p 0 bis 20 ist,
L3 für die Gruppe

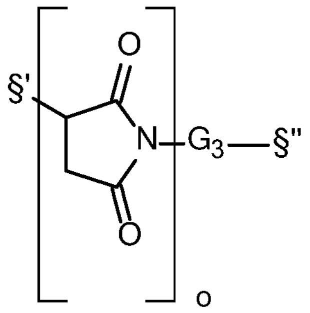

steht, in der
o 0 oder 1 ist,
§' für die Bindung an die -S(O)n-Gruppe und
§" für die Bindung an das N-Atom im Ring steht.
G3 für eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen steht, die einfach oder mehrfach durch eine oder mehrere der Gruppen -O-, -S-, -S(=O)-, $S(=O)_2$-, -NH-, -C(=O)-, -NH-C(=O)-, -C(=O)-NH- und einen 5 bis 10gliedriger, aromatischen oder nicht aromatischen Heterozyklus mit bis zu 4 Heteroatomen ausgewählt aus N, O und S, -Nme-, -NHNH-, -$S(=O)_2$-NHNH-, -C(=O)-NHNH-, -S(=O)- oder -$S(=O)_2$- unterbrochen sein kann (vorzugsweise

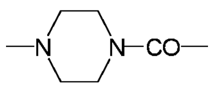

), wobei die Seitenketten, sofern vorhanden, mit -NHC(=O)-$NH_2$, -COOH, -OH, Sulfon, Sulfoxid oder Sulfonsäure substituiert sein können.

**27.** Verbindungen nach Anspruch 26, wobei der Linker -$L_b$- bzw. -$L_c$- an eine Cystein-Seitenkette bzw. Cysteinrest gebunden ist und die folgende Formel

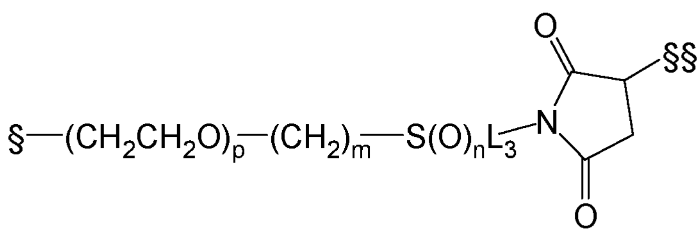

aufweist, wobei

§ für die Bindung an das Wirkstoffmolekül bzw die durch Legumain spaltbare Gruppe steht,
§§ für die Bindung an den Binder steht,
m 1 ist;
p 0 ist;
n 0 ist,
L3 für die Gruppe

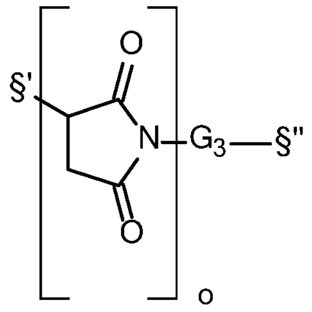

steht, in der
o 0 oder 1 ist,
$G_3$ für $-(CH_2CH_2O)_s - (CH_2)_t - (C(=O)-NH)_u - CH_2CH_2O)_v-(CH_2)_w-$ steht,
s, t, v und w unabhängig voneinander für 0 bis 20 stehen,
u 0 oder 1 ist,
§' für die Bindung an die -S(O)n-Gruppe und
§" für die Bindung an das N-Atom im Ring steht.

**28.** Verbindungen nach Anspruch 25 oder 26, wobei $R^2$ oder $R^3$ für -L-#1 steht.

**29.** Konjugate nach einem oder mehreren der vorhergehenden Ansprüche der folgenden Strukturen, in denen

AK für den Binder, vorzugsweise für einen Antikörper oder ein Antigenbindendes Antikörperfragment ($AK_1$, $AK_2$, $AK_3$) steht und
$AK_1$ vorzugsweise für einen Antikörper, der über einen Cysteinrest an den KSP-Inhibitor gebunden ist, steht,
$AK_2$ vorzugsweise für einen Antikörper, der über einen Lysinrest an den KSP-Inhibitor gebunden ist, steht und
$AK_3$ vorzugsweise für einen Antikörper, der über einen Glutaminrest an den KSP-Inhibitor gebunden ist, steht,

und

n für eine Zahl von 1 bis 50, vorzugsweise 1 bis 20, besonders bevorzugt 2 bis 8 und insbesondere 2 bis 6 steht:

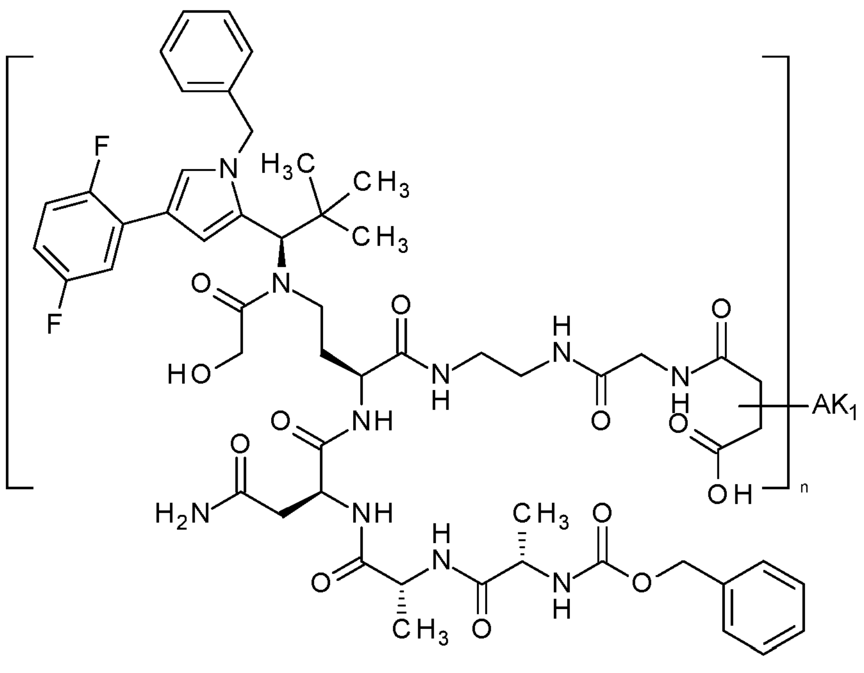

,

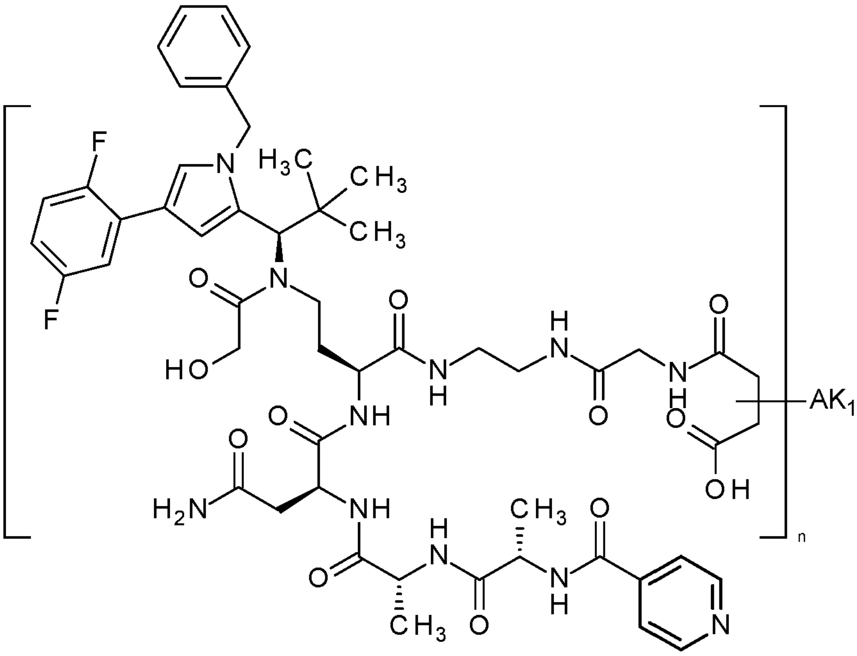

,

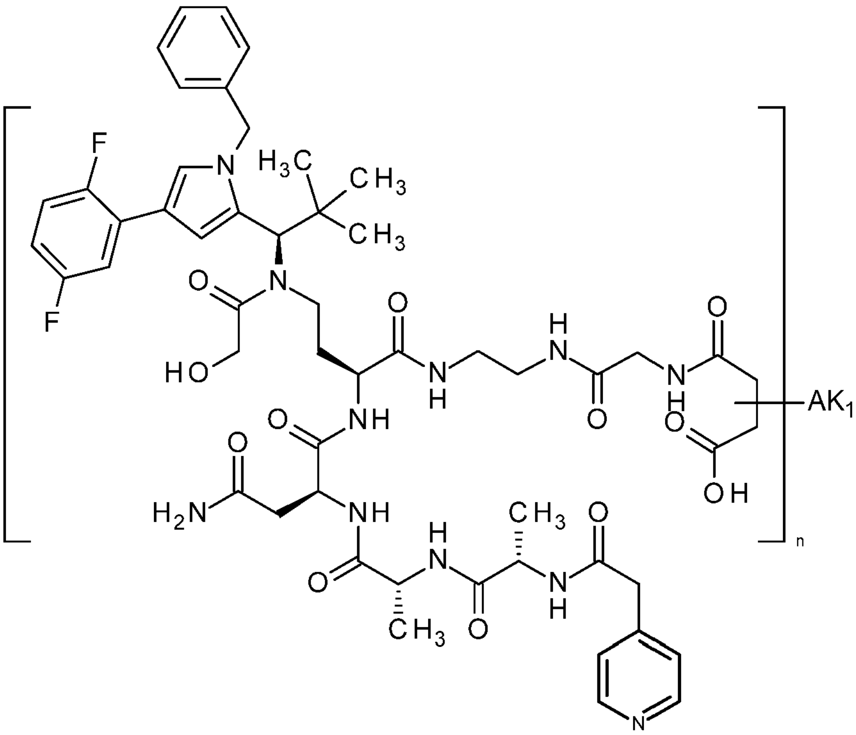

,

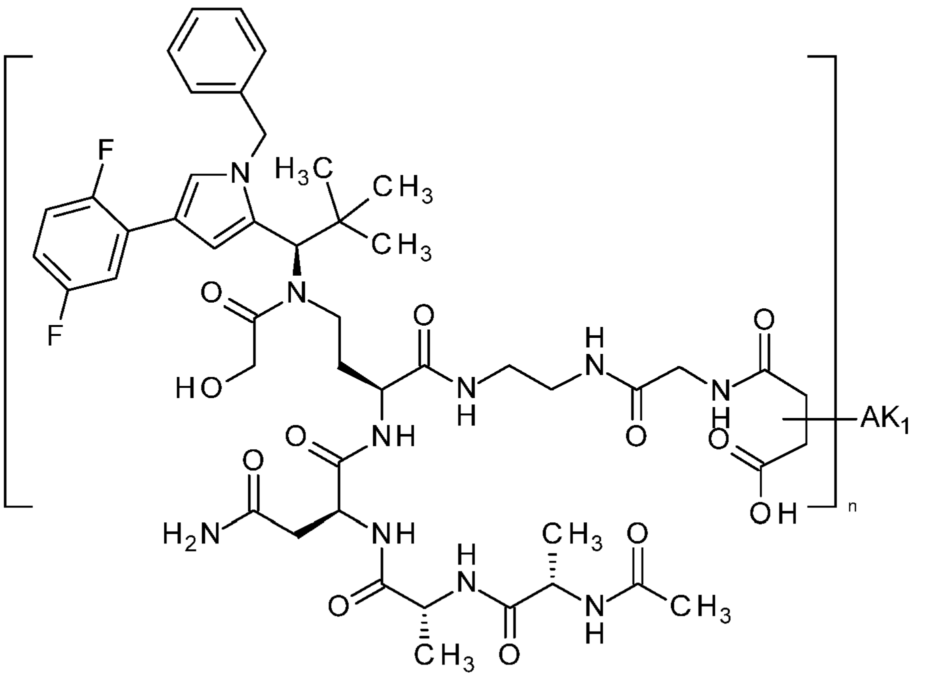

,

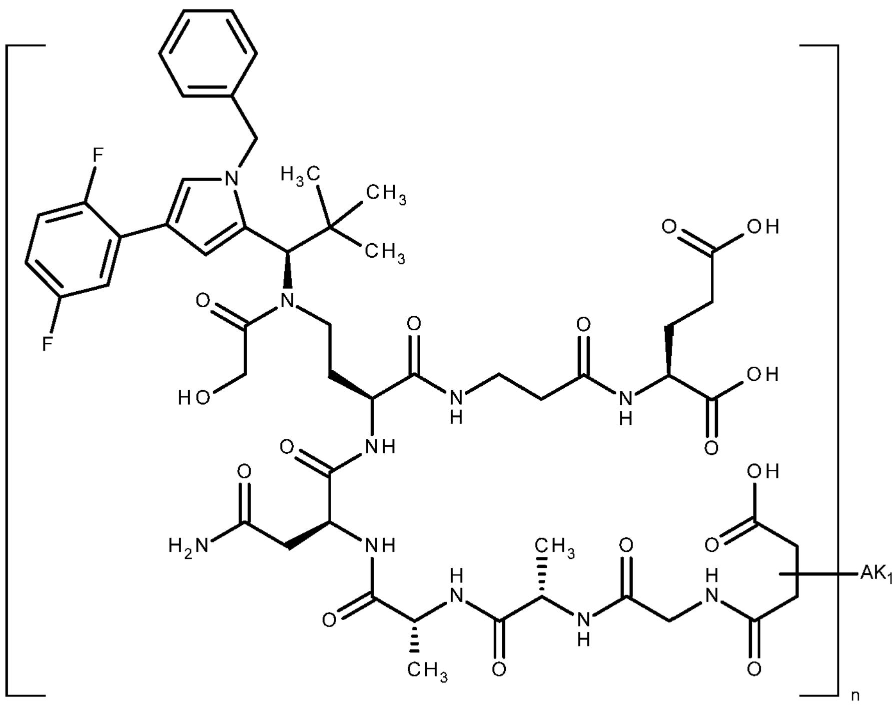

,

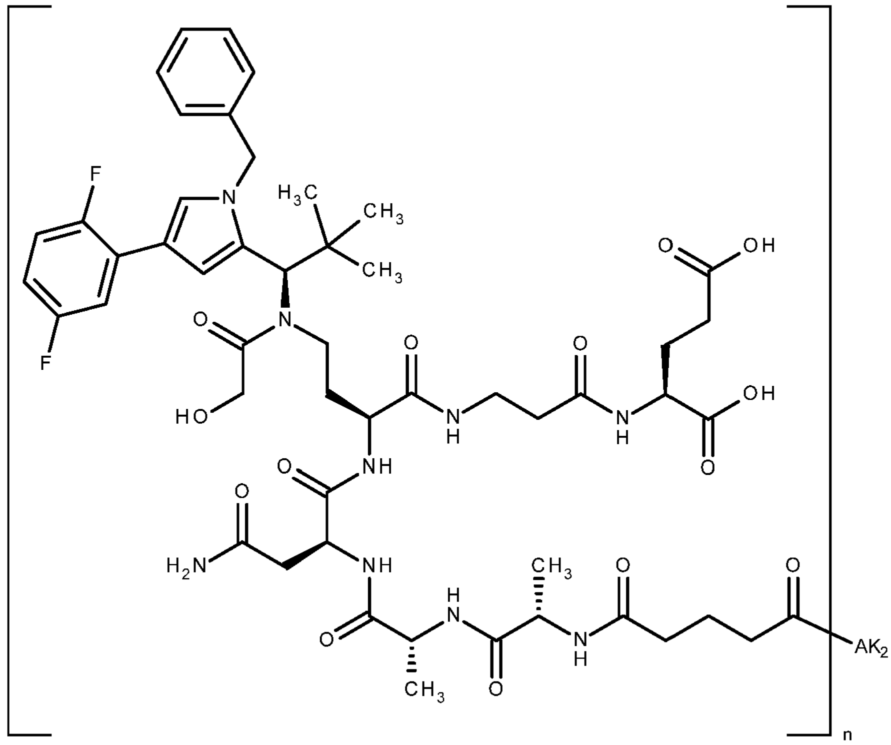

,

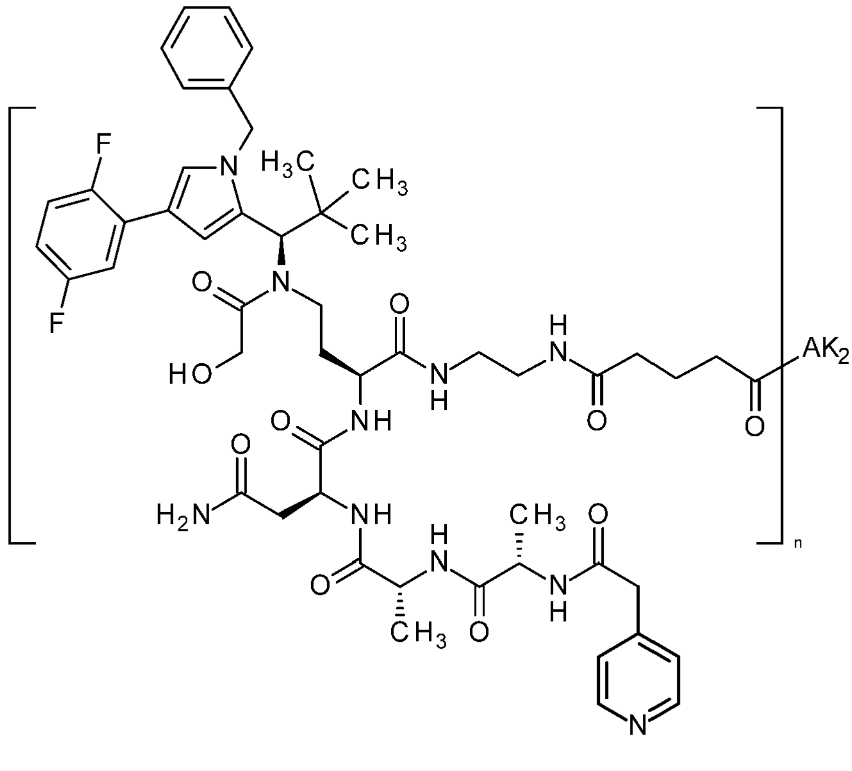

,

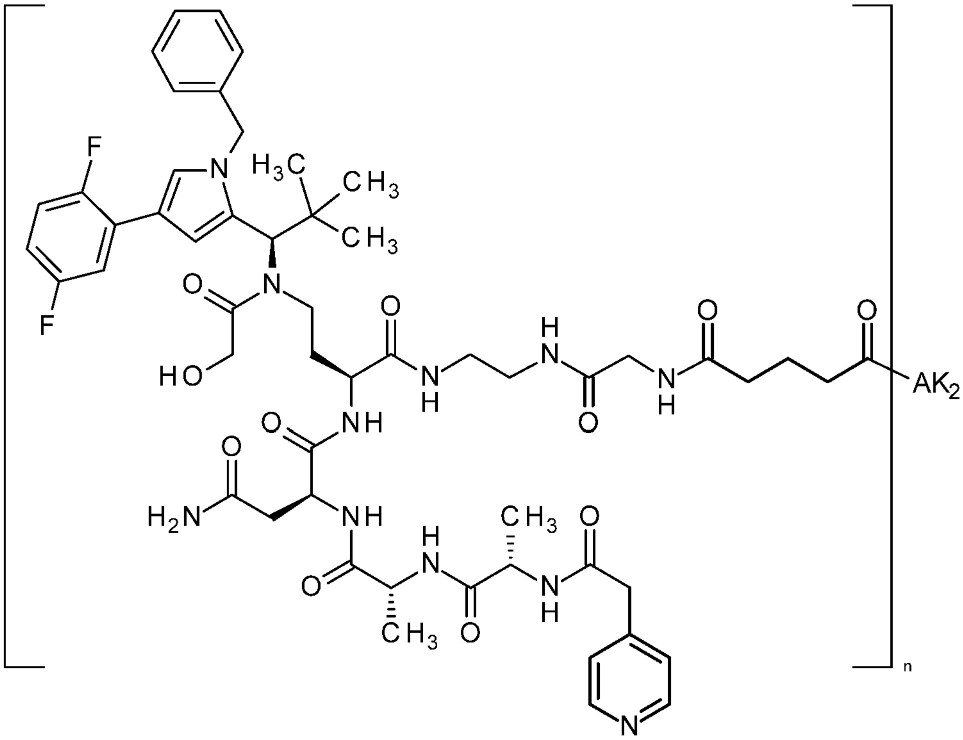

,

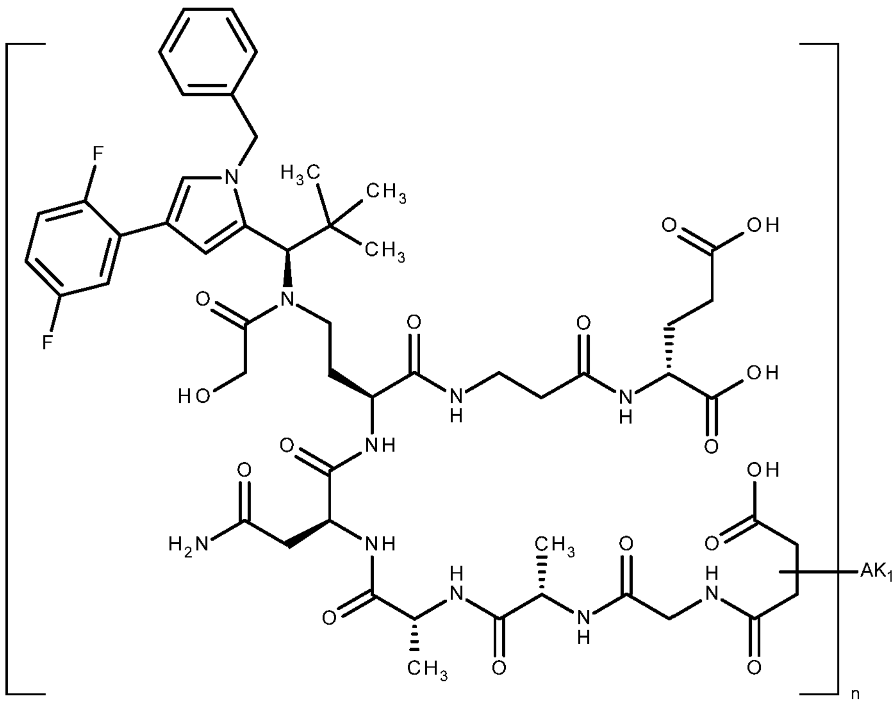

,

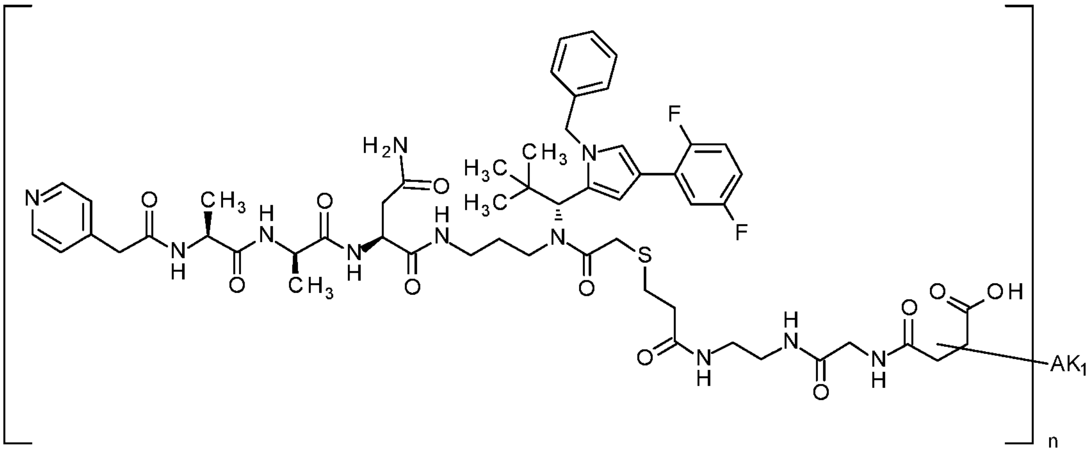

,

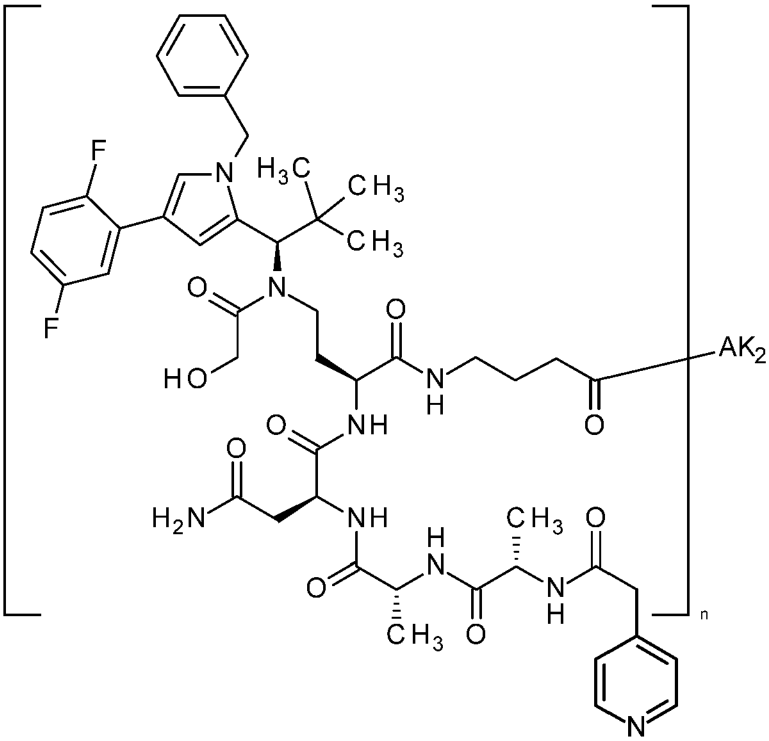

,

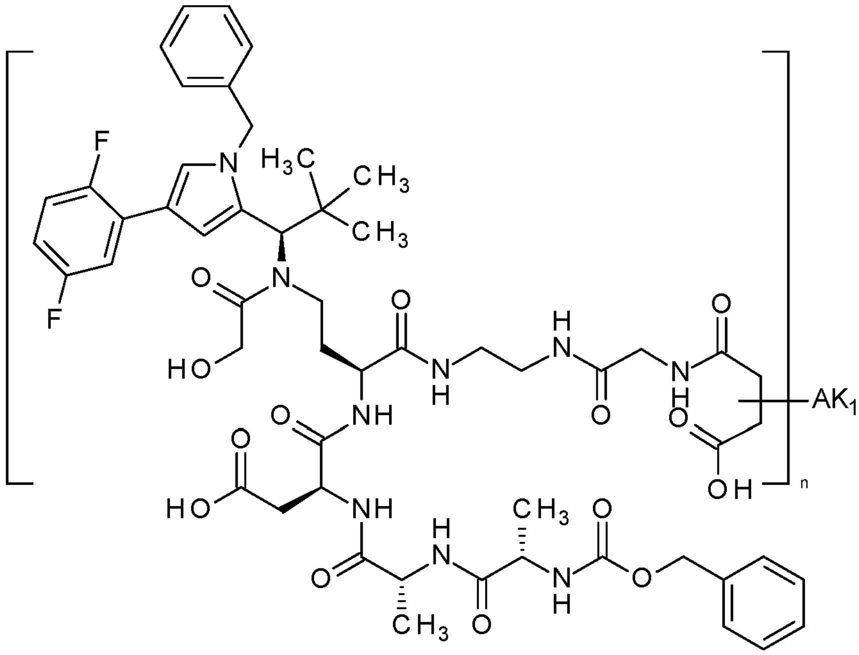

,

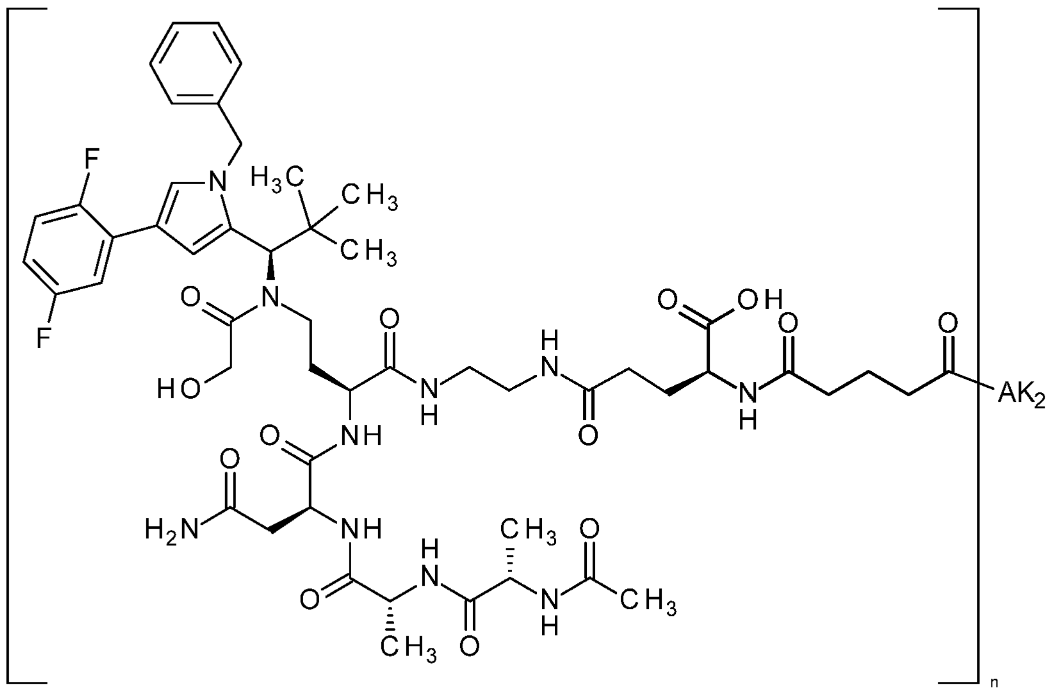

,

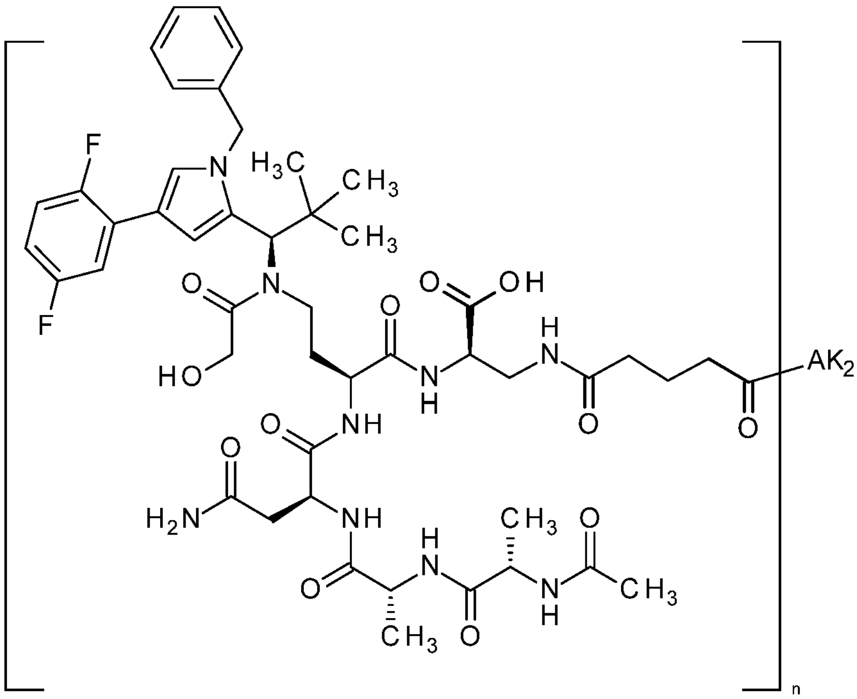

,

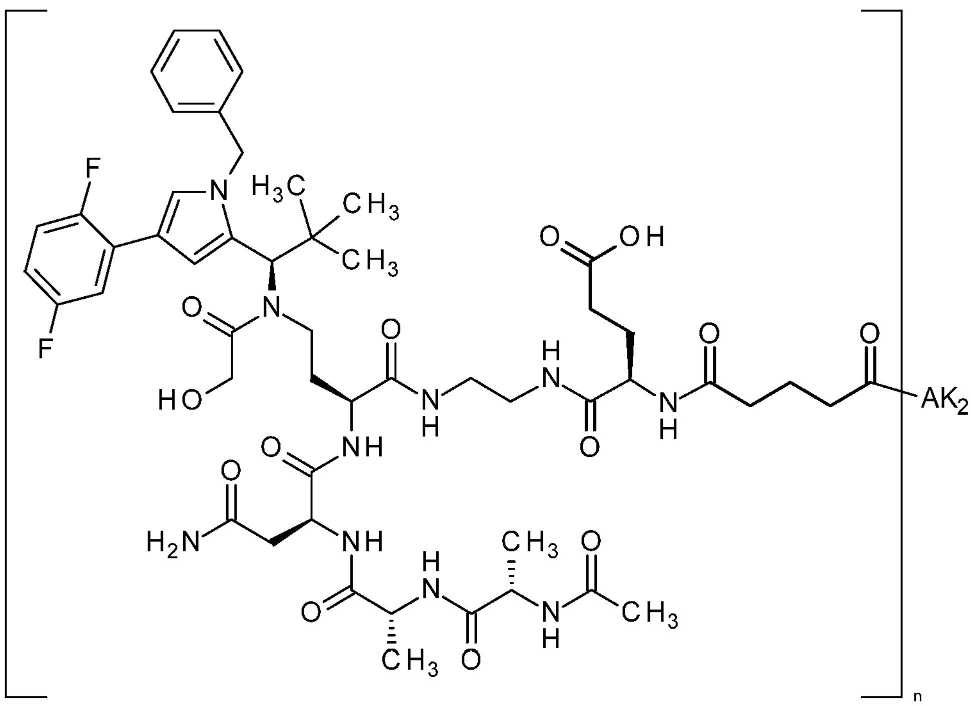

,

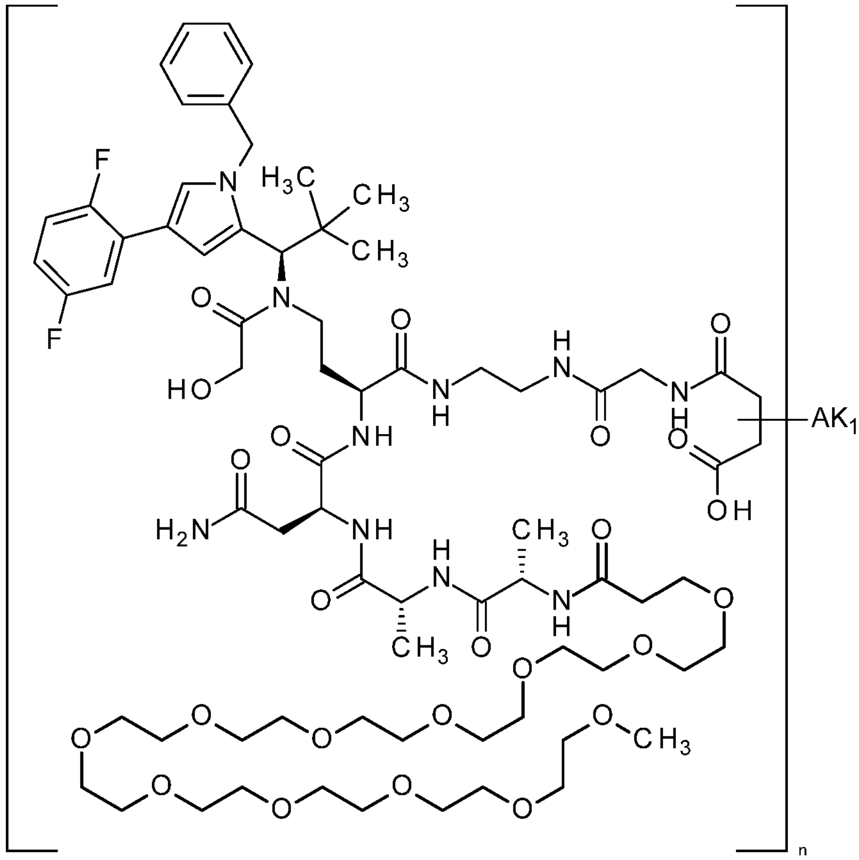

,

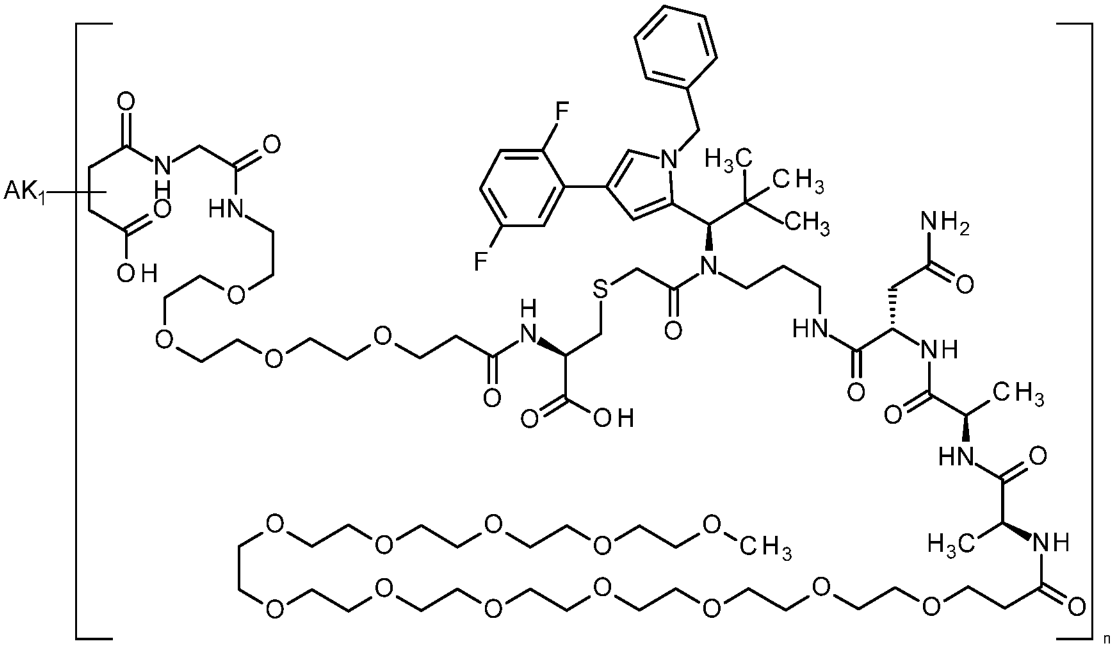

,

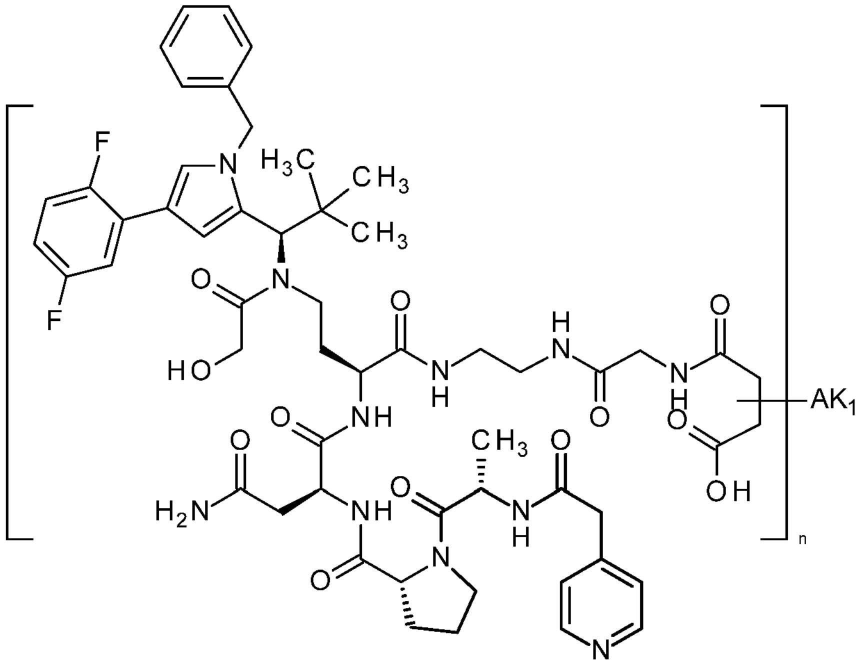

,

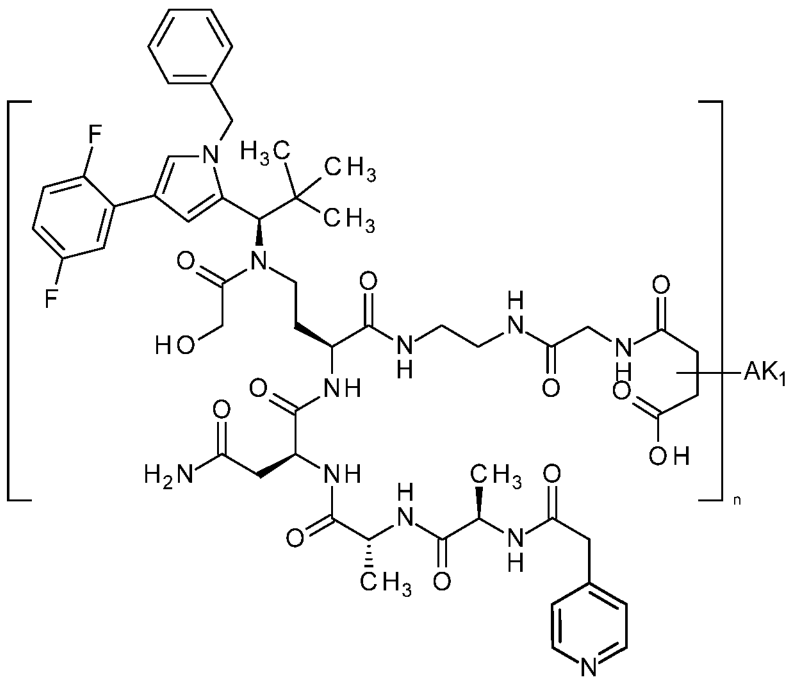

,

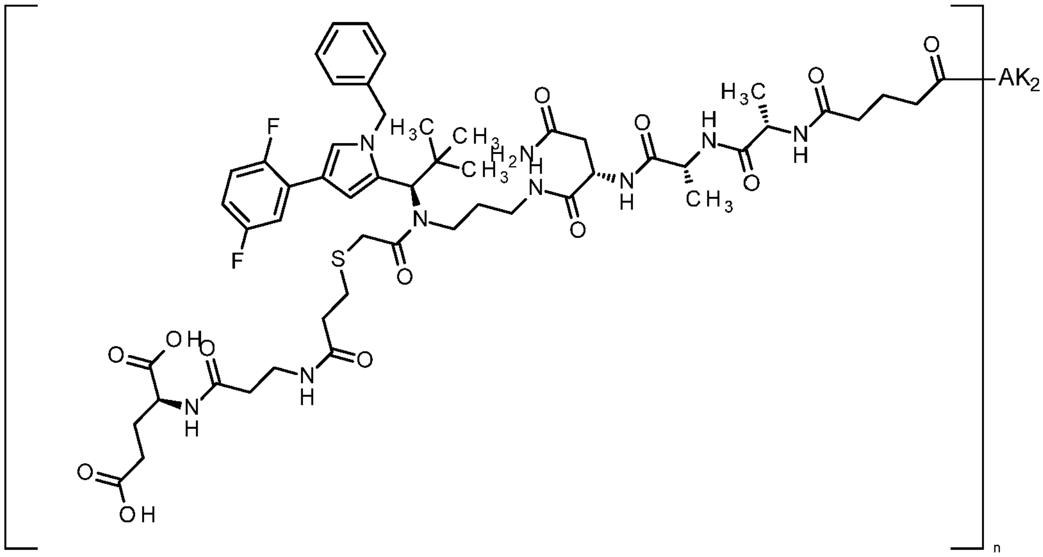

,

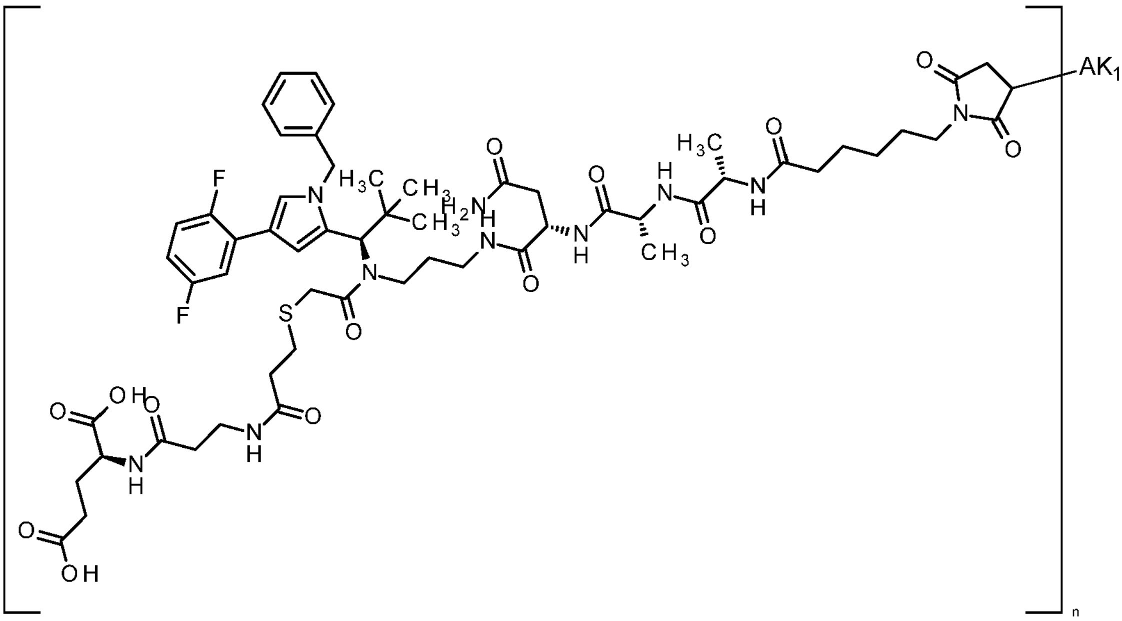

,

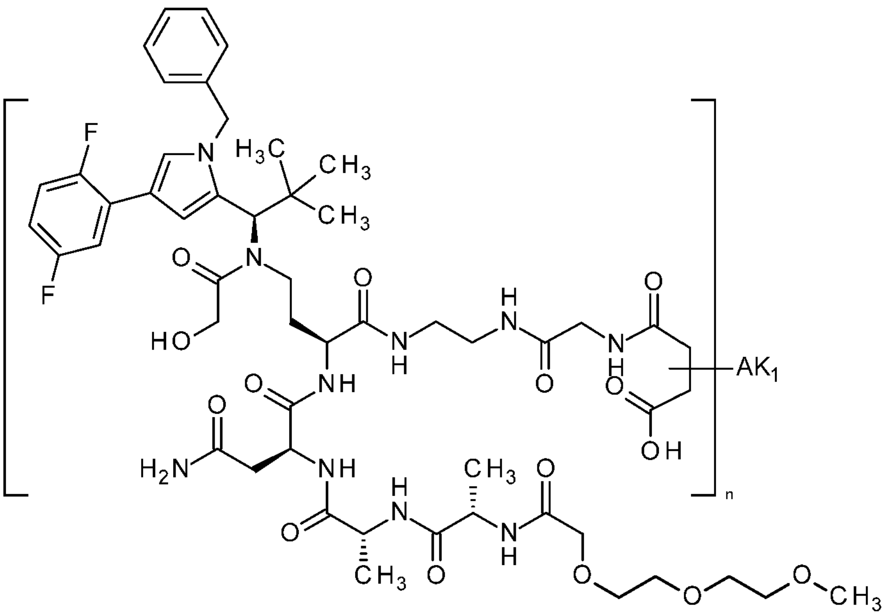

,

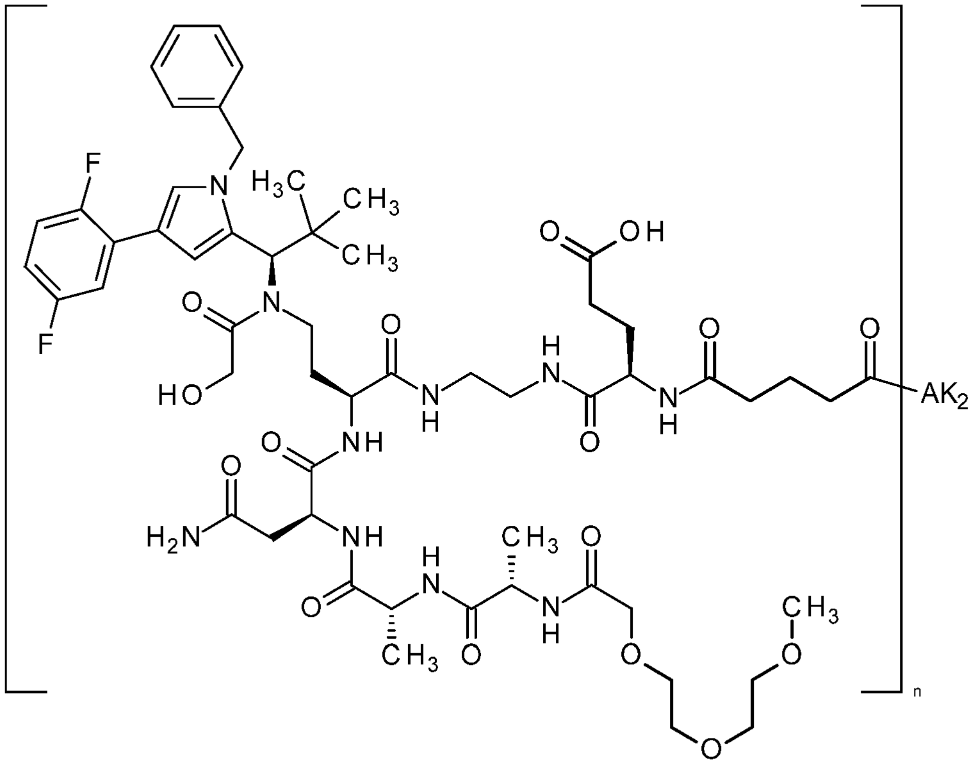

,

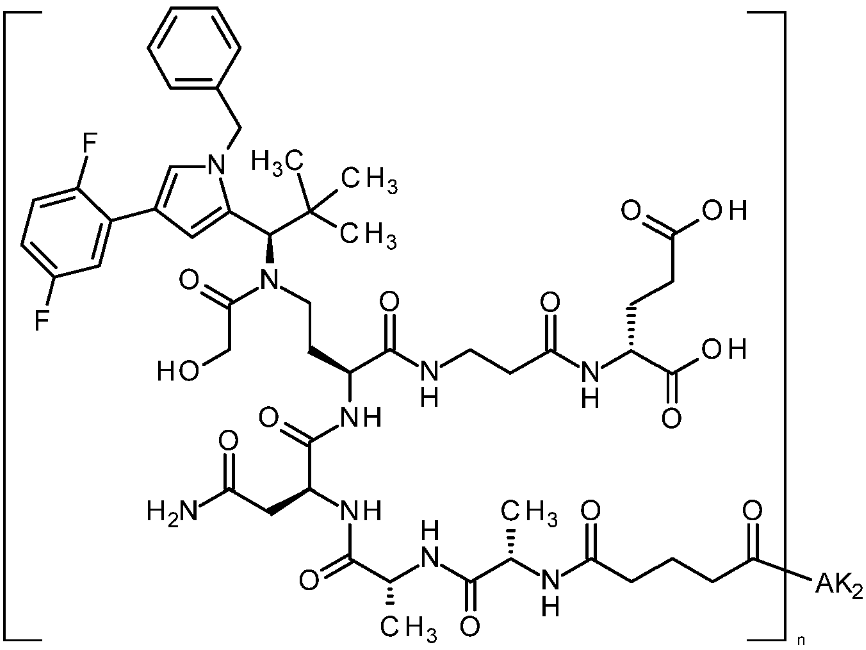

,

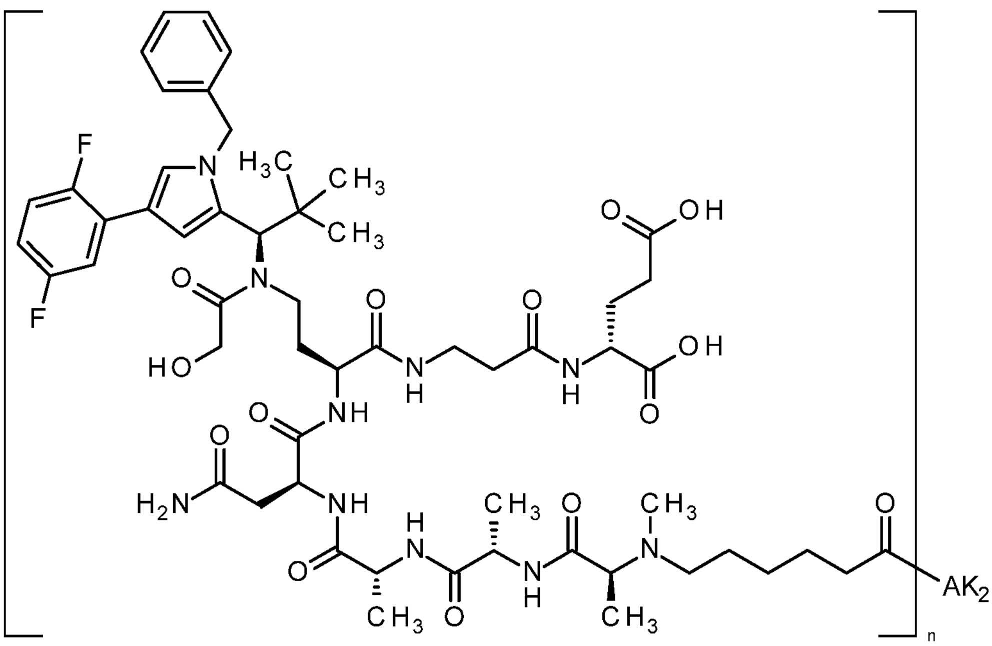

,

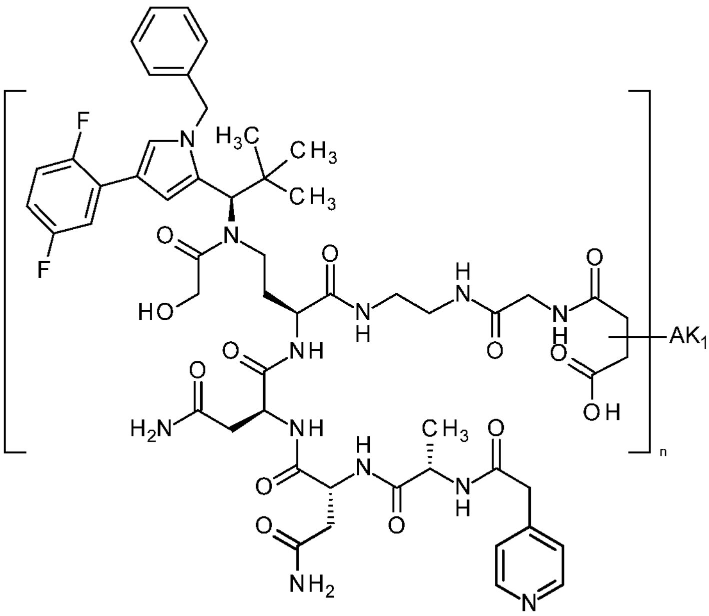

,

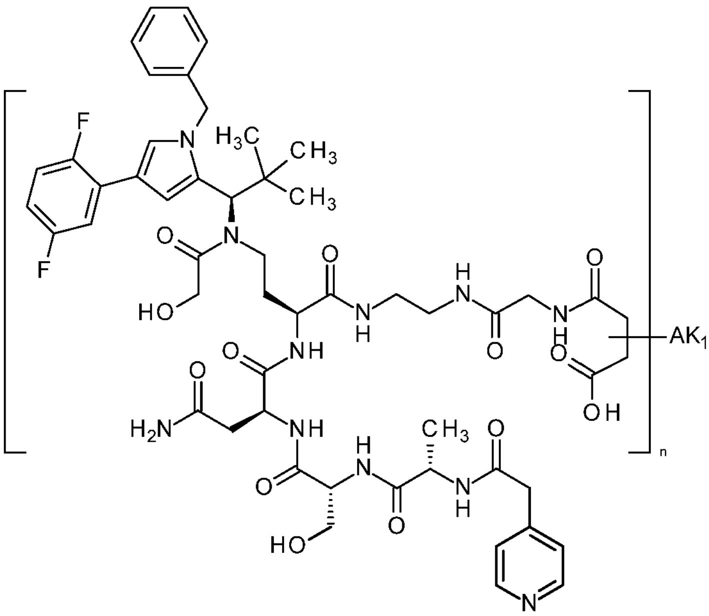

,

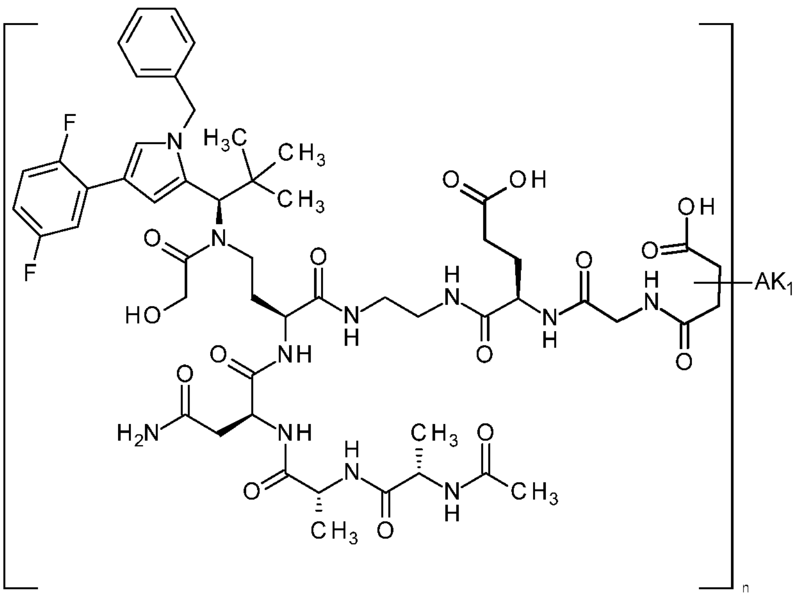

,

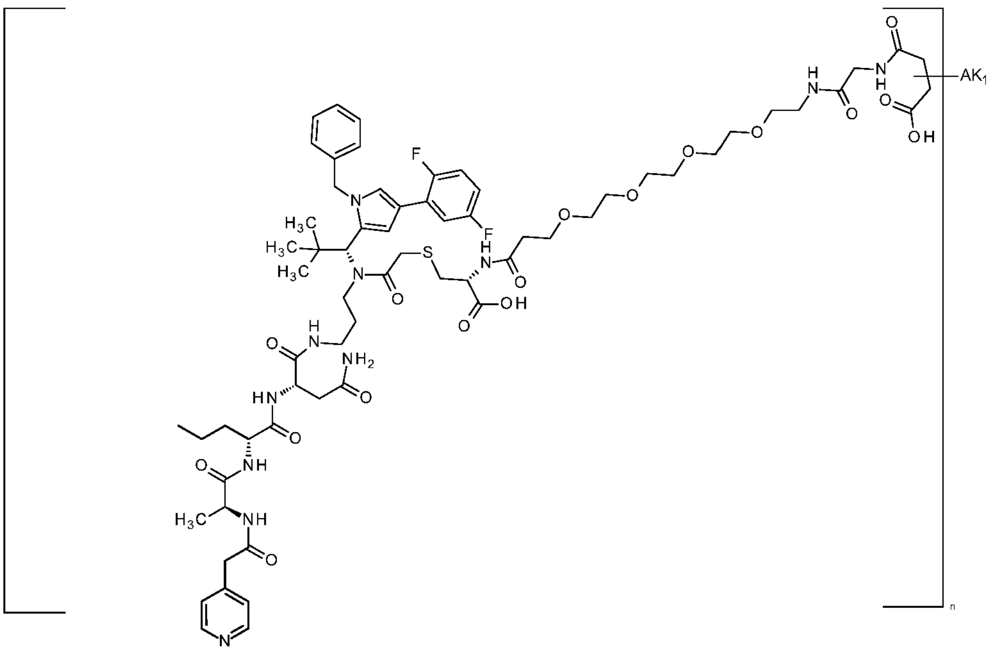

,

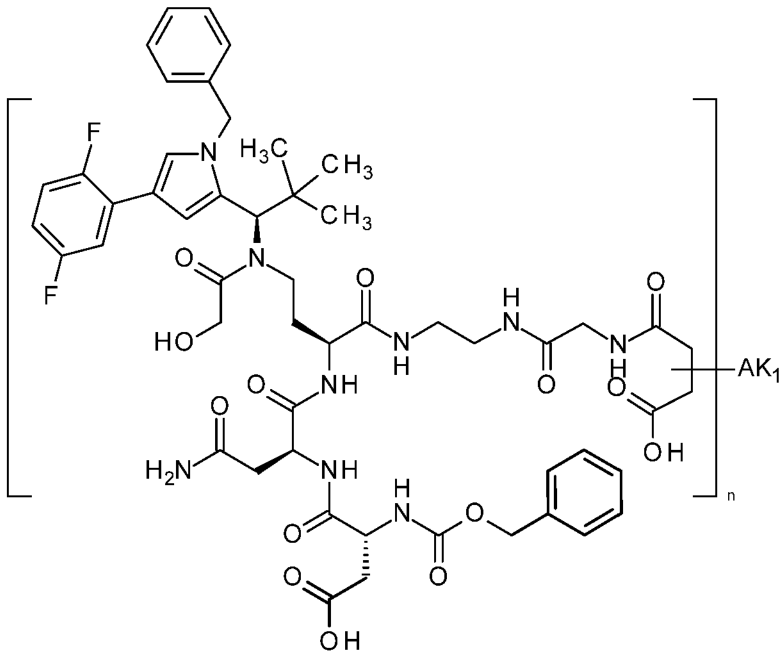

,

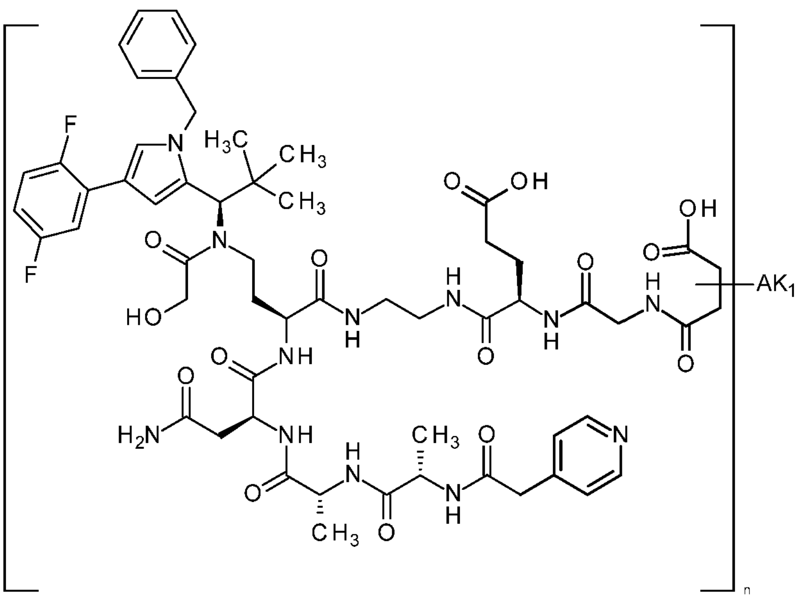

,

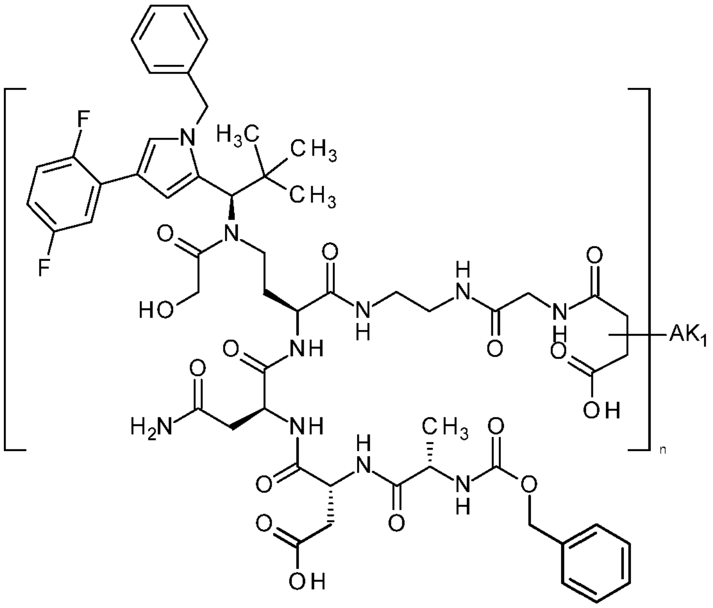

,

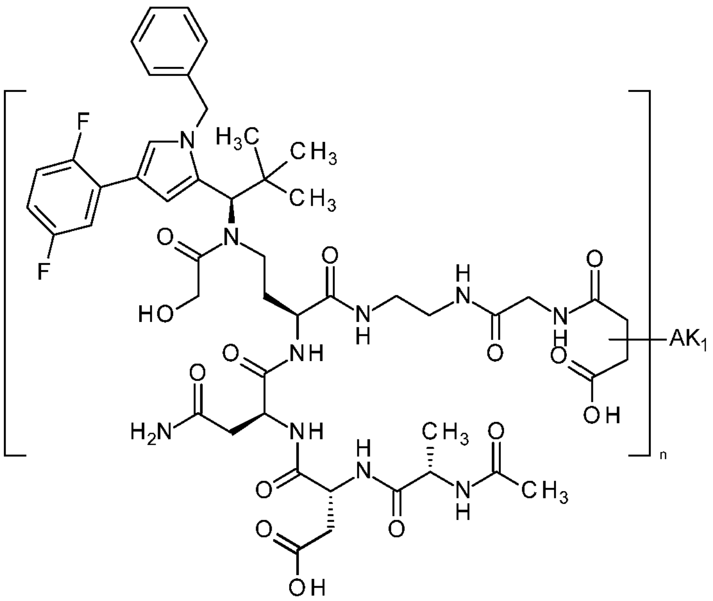

,

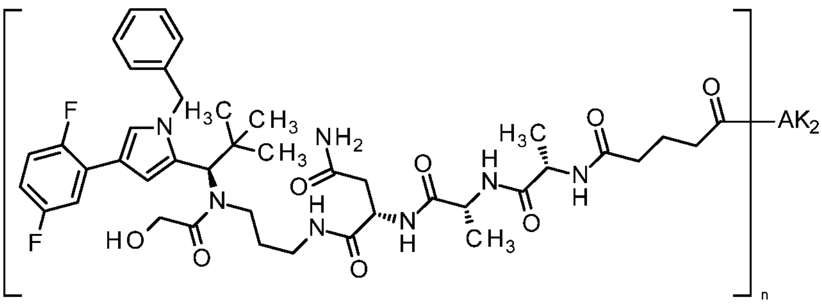

,

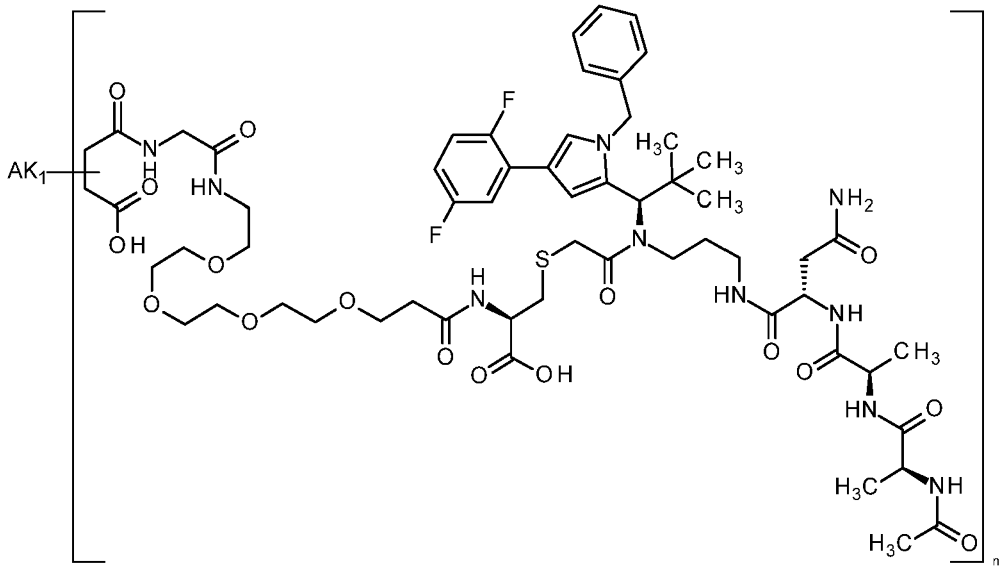

,

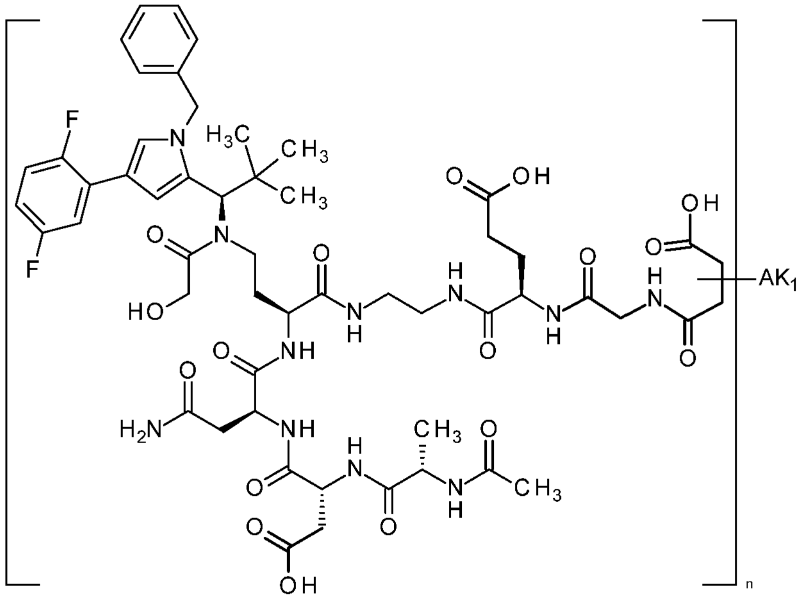

,

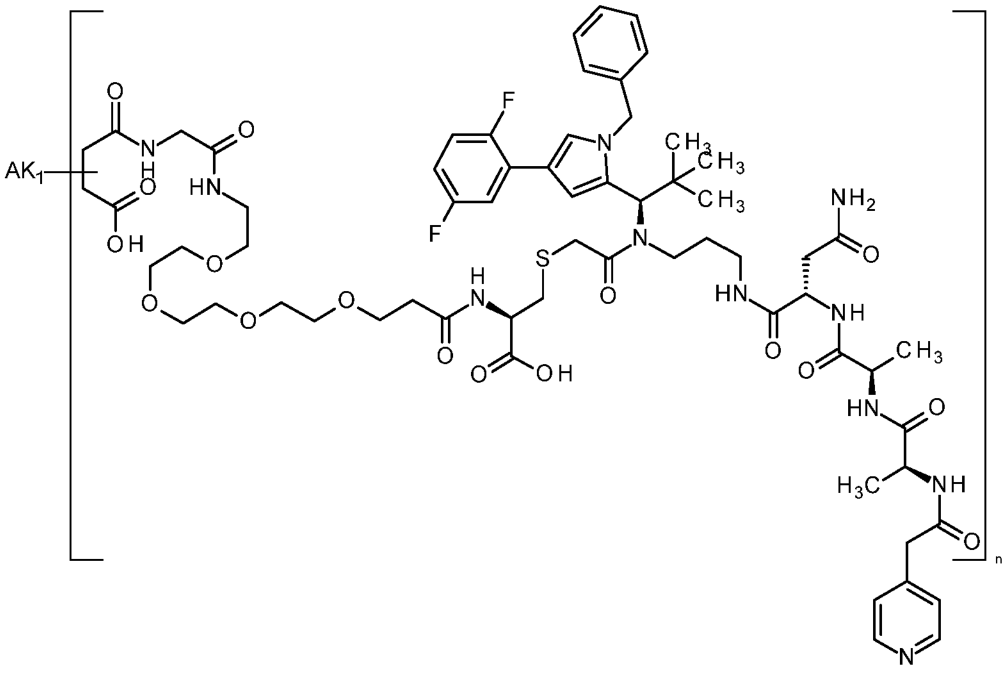

,

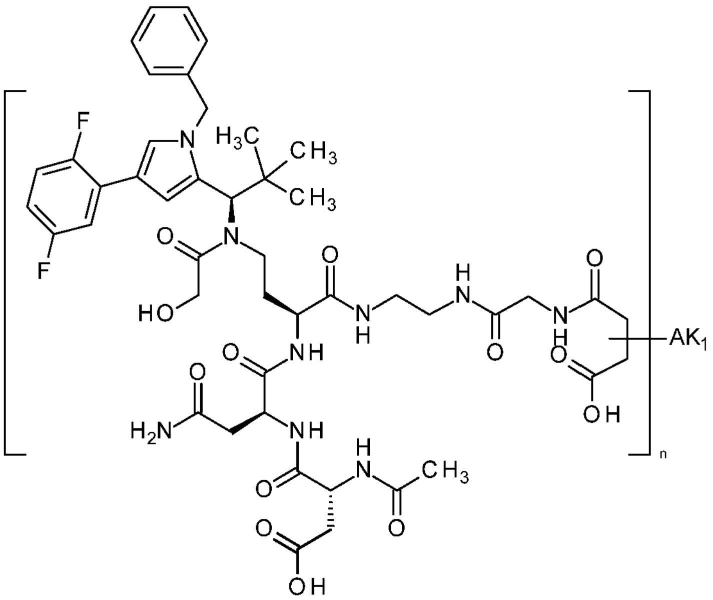

,

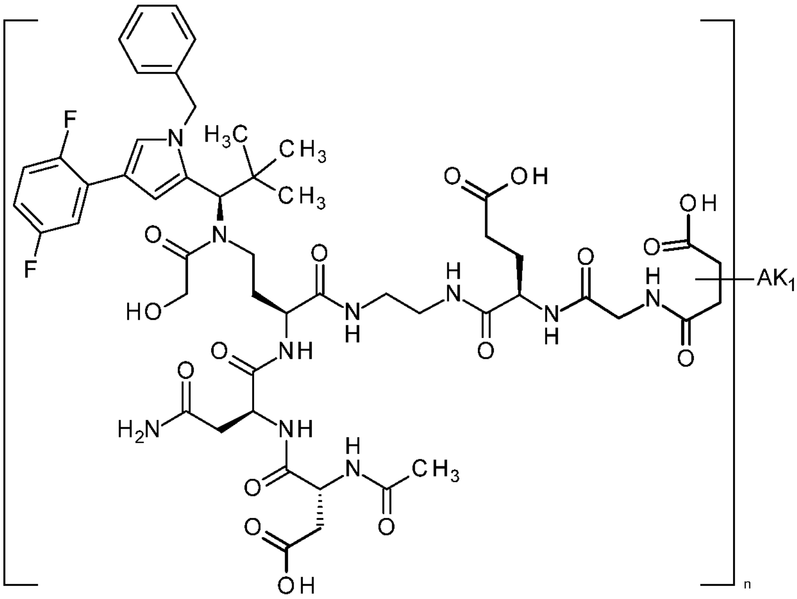

,

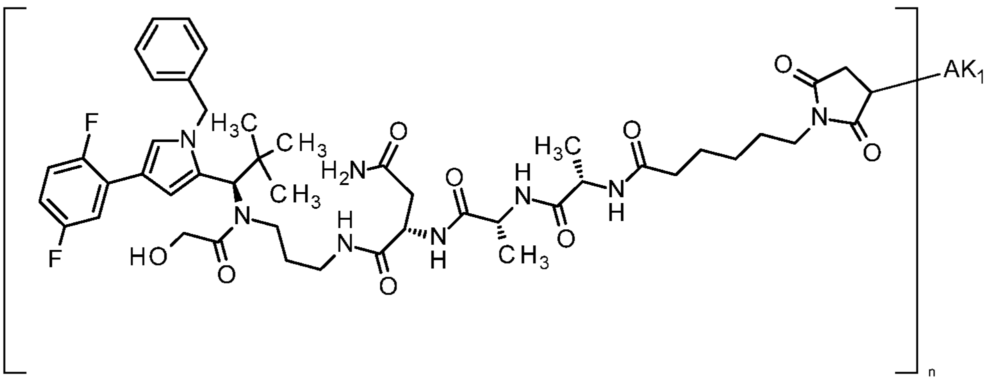

.

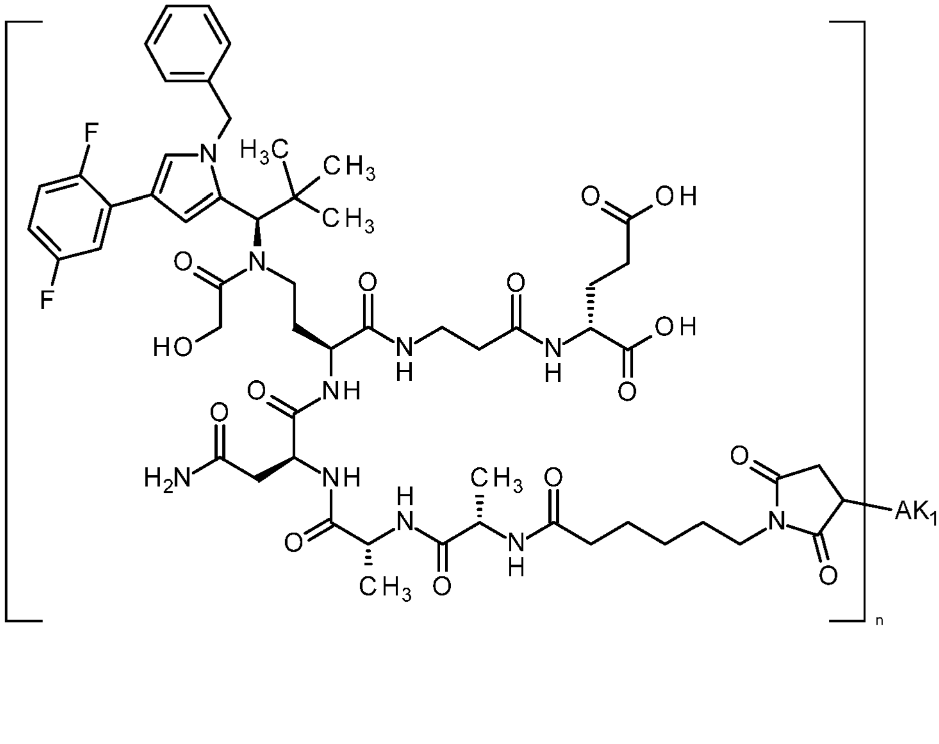

,

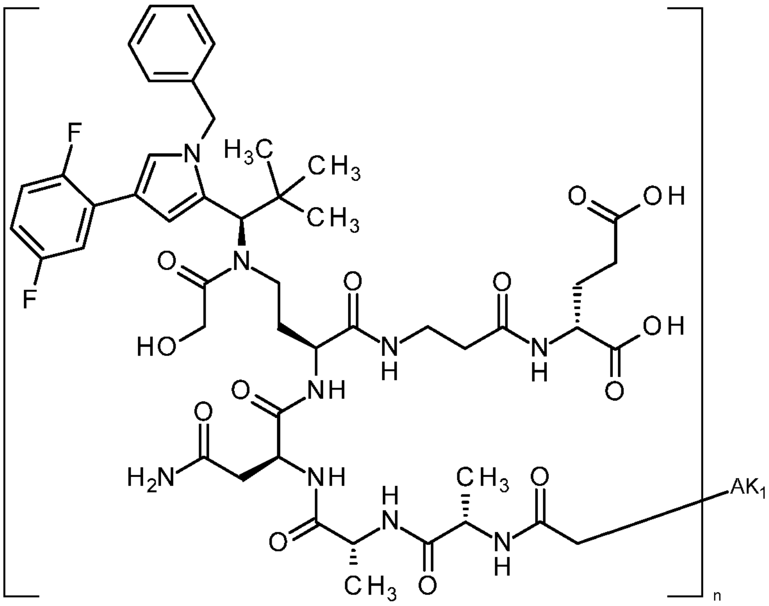

,

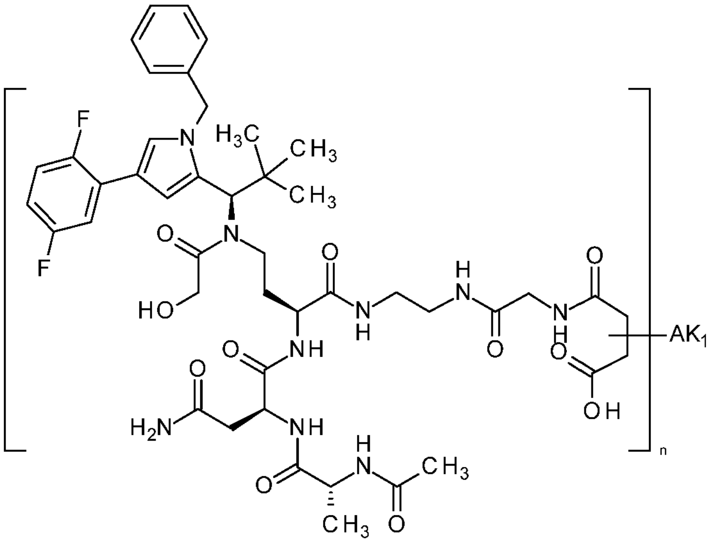

,

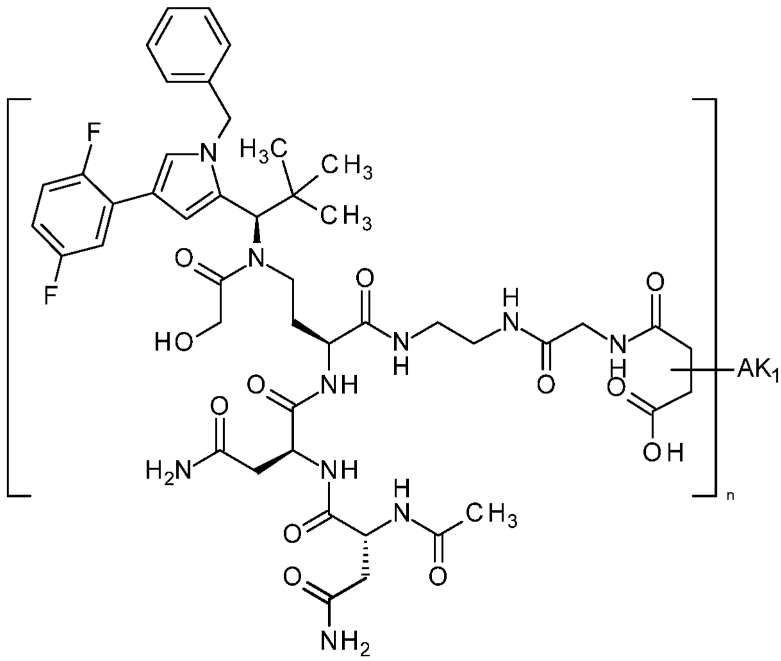

,

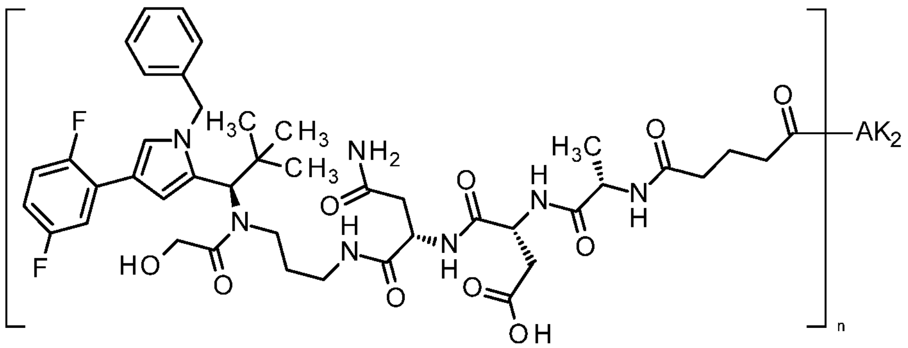

,

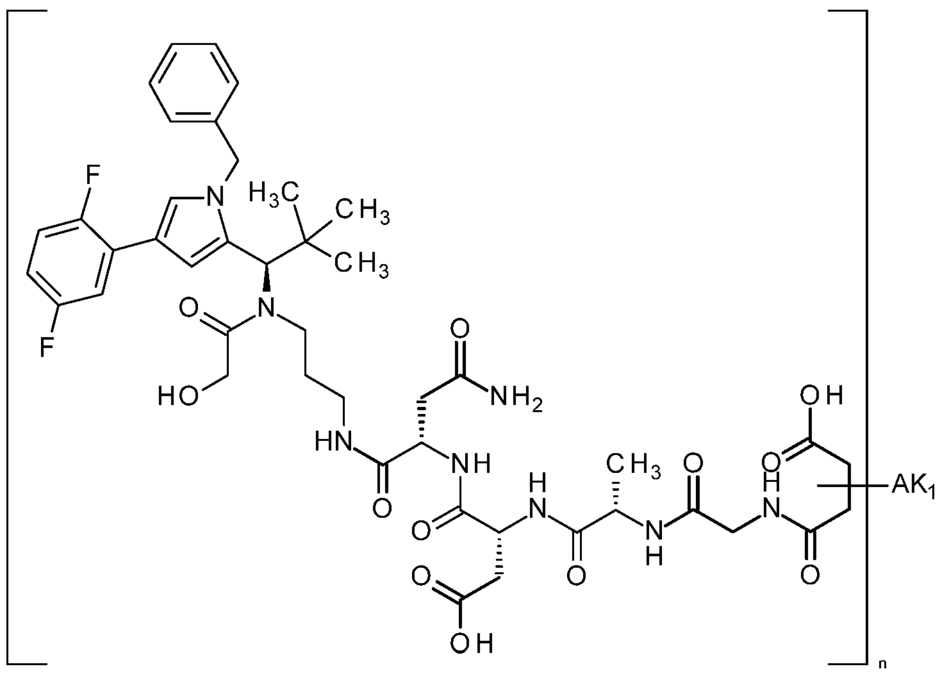

,

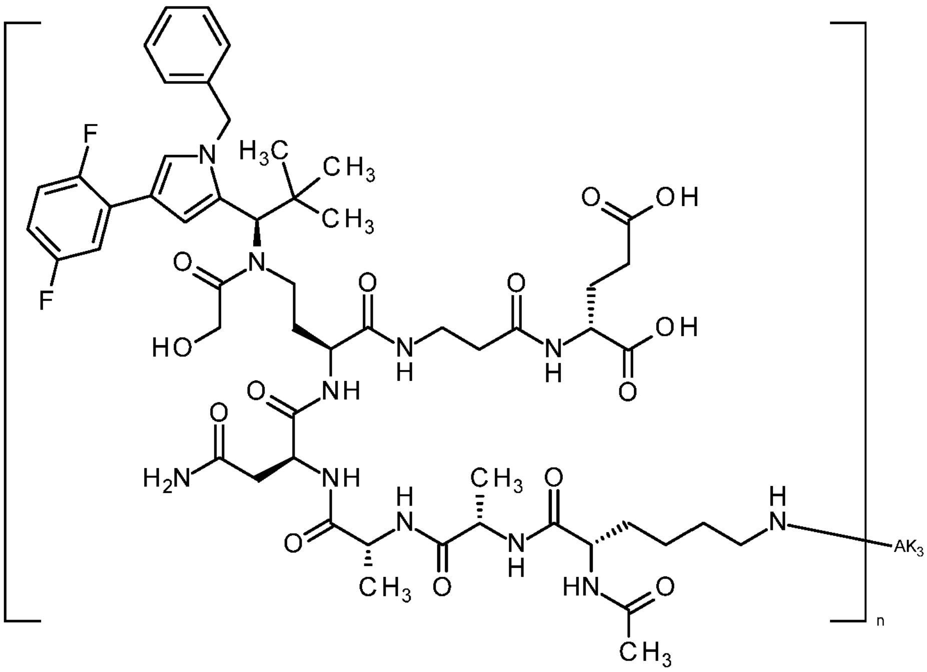

,

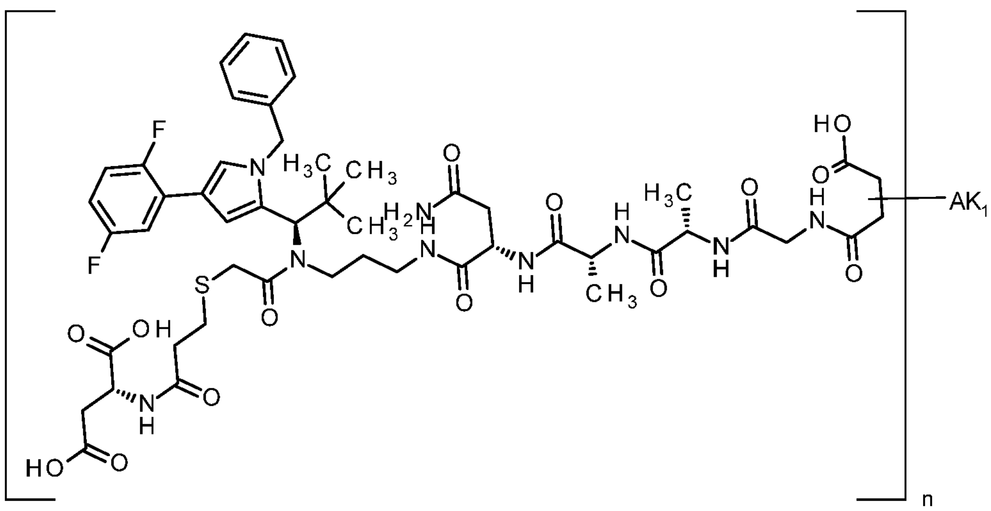

,

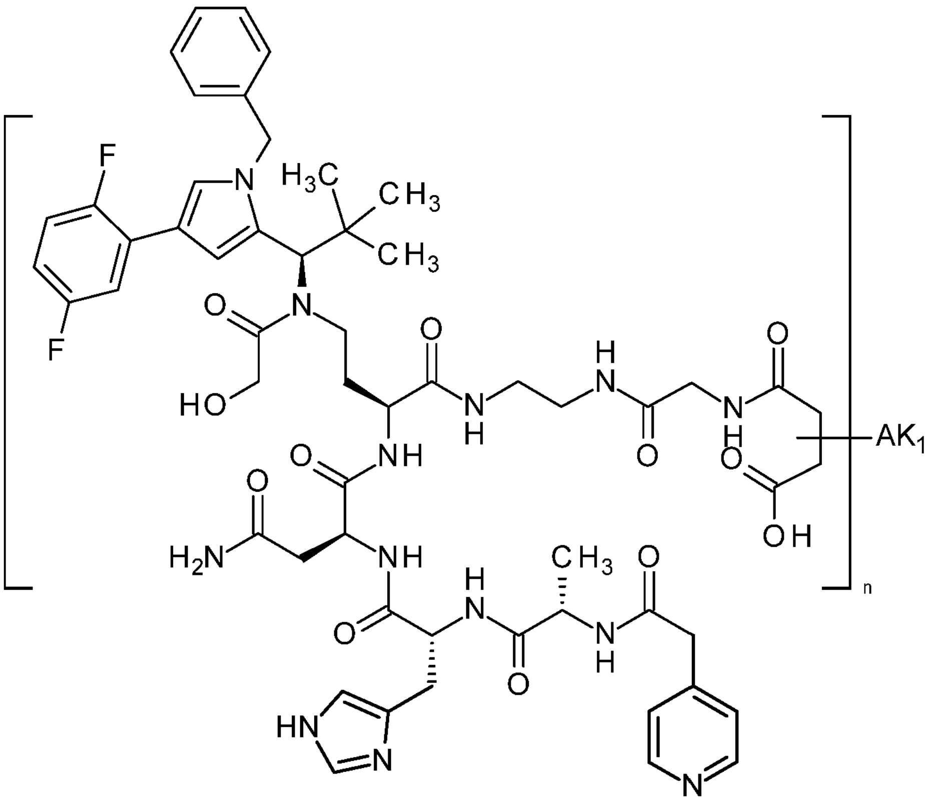

,

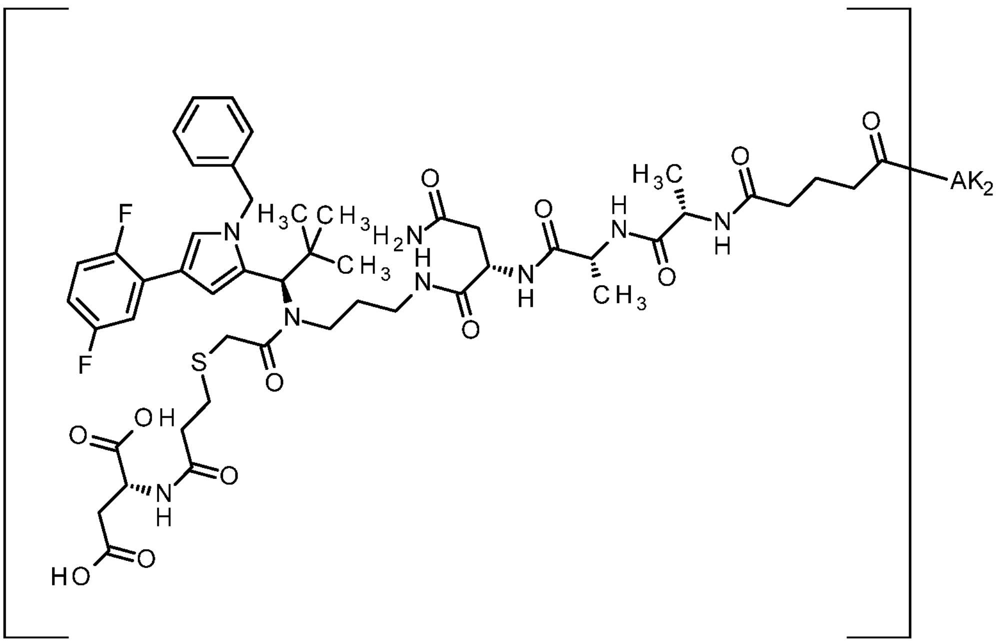

und

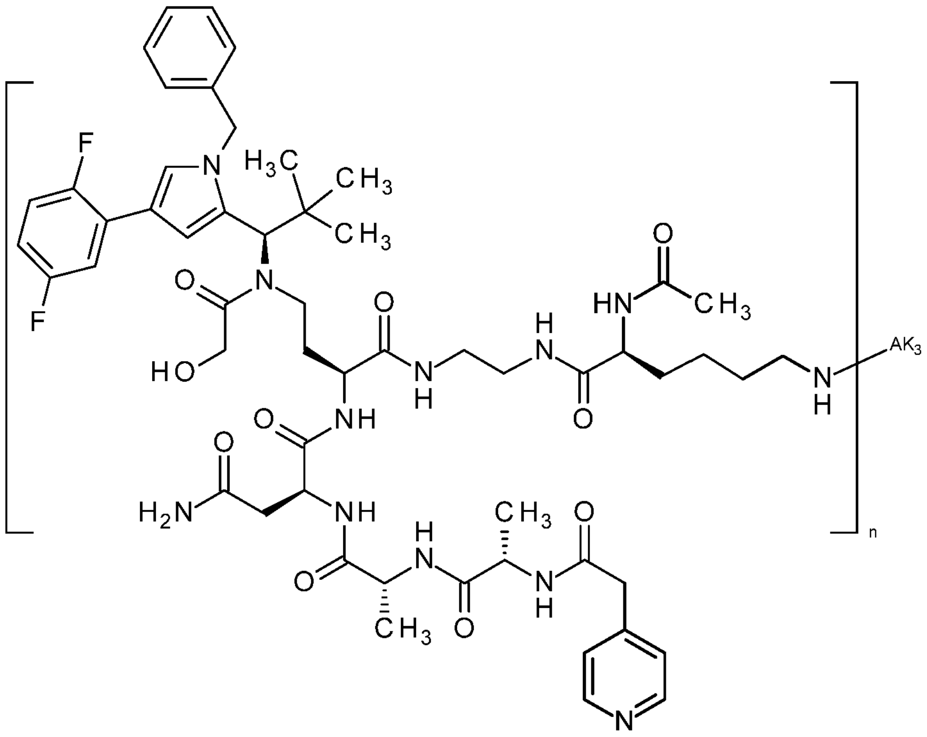

30. Verbindung oder Konjugat nach einem oder mehreren der vorhergehenden Ansprüche, wobei der Antikörper oder das Antigen-bindendes Antikörperfragment an ein eextrazelluläres Krebs-Zielmolekül bindet.

31. Verbindung oder Konjugat nach einem oder mehreren der vorhergehenden Ansprüche, wobei der Antikörper oder das Antigen-bindendes Antikörperfragment nach Bindung an sein extrazelluläres Zielmolekül auf der Zielzelle durch die Bindung von der Zielzelle internalisiert.

32. Verbindung oder Konjugat nach einem oder mehreren der vorhergehenden Ansprüche, wobei der Antikörper ein anti-HER2-Antikörper, ein anti-EGFR-Antikörper, anti-B7H3-Antikörper, ein anti-TWEAKR-Antikörper, oder ein Antigen-bindendes Antikörperfragment von diesen ist.

33. Verbindung oder Konjugat nach Anspruch 32 wobei der anti-TWEAKR-Antikörper ausgewählt aus der Gruppe bestehend aus TPP-7006, TPP-7007, TPP-10336 und TPp10337, der anti-B7H3-Antikörper Antikörper ausgewählt aus der Gruppe bestehend aus TPP-8382 und TPP-8567, der anti-EGFR-Antikörper Cetuximab (TPp-981) und der anti-HER2-Antikörper ausgewählt aus der Gruppe bestehend aus Trastuzumab und TPP-1015 ist.

34. Verbindung oder Konjugat nach einem oder mehreren der vorhergehenden Ansprüche, wobei der Antikörper (AK, AB, AK1, AK2, AK3)

(i) für einen anti-EGFR Antikörper umfassend eine variable Region der schwere Kette (VH) umfassend die variable CDR1-Sequenz der schweren Kette (H-CDR1), wie dargestellt durch SEQ ID NO: 2, die variable CDR2-Sequenz der schweren Kette (H-CDR2), wie dargestellt durch SEQ ID NO: 3 und die variable CDR3-Sequenz der schweren Kette (H-CDR3), wie dargestellt durch SEQ ID NO: 4, sowie eine variable Region der leichte Kette (VL) umfassend die variable CDR1-Sequenz der leichten Kette (L-CDR1), wie dargestellt durch SEQ ID NO: 6, die variable CDR2-Sequenz der leichten Kette (L-CDR2), wie dargestellt durch SEQ ID NO: 7 und die variable CDR3-Sequenz der leichten Kette (L-CDR3), wie dargestellt durch SEQ ID NO: 8,
(ii) für einen anti-HER2 Antikörper umfassend eine variable Region der schwere Kette (VH) umfassend die variable CDR1-Sequenz der schweren Kette (H-CDR1), wie dargestellt durch SEQ ID NO: 12, die variable CDR2-Sequenz der schweren Kette (H-CDR2), wie dargestellt durch SEQ ID NO: 13 und die variable CDR3-Sequenz der schweren Kette (H-CDR3), wie dargestellt durch SEQ ID NO: 14, sowie eine variable Region der leichte Kette (VL) umfassend die variable CDR1-Sequenz der leichten Kette (L-CDR1), wie dargestellt durch SEQ ID NO: 16, die variable CDR2-Sequenz der leichten Kette (L-CDR2), wie dargestellt durch SEQ ID NO:

17 und die variable CDR3-Sequenz der leichten Kette (L-CDR3), wie dargestellt durch SEQ ID NO: 18,

(iii) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 22, die variable CDR2-Sequenz der schweren Kette (H-CDR2), wie dargestellt durch SEQ ID NO: 23 und die variable CDR3-Sequenz der schweren Kette (H-CDR3), wie dargestellt durch SEQ ID NO: 24, sowie eine variable Region der leichte Kette (VL) umfassend die variable CDR1-Sequenz der leichten Kette (L-CDR1), wie dargestellt durch SEQ ID NO: 26, die variable CDR2-Sequenz der leichten Kette (L-CDR2), wie dargestellt durch SEQ ID NO: 27 und die variable CDR3-Sequenz der leichten Kette (L-CDR3), wie dargestellt durch SEQ ID NO: 28,

(iv) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 32, die variable CDR2-Sequenz der schweren Kette (H-CDR2), wie dargestellt durch SEQ ID NO: 33 und die variable CDR3-Sequenz der schweren Kette (H-CDR3), wie dargestellt durch SEQ ID NO: 34, sowie eine variable Region der leichte Kette (VL) umfassend die variable CDR1-Sequenz der leichten Kette (L-CDR1), wie dargestellt durch SEQ ID NO: 36, die variable CDR2-Sequenz der leichten Kette (L-CDR2), wie dargestellt durch SEQ ID NO: 37 und die variable CDR3-Sequenz der leichten Kette (L-CDR3), wie dargestellt durch SEQ ID NO: 38,

(v) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 42, die variable CDR2-Sequenz der schweren Kette (H-CDR2), wie dargestellt durch SEQ ID NO: 43 und die variable CDR3-Sequenz der schweren Kette (H-CDR3), wie dargestellt durch SEQ ID NO: 44, sowie eine variable Region der leichte Kette (VL) umfassend die variable CDR1-Sequenz der leichten Kette (L-CDR1), wie dargestellt durch SEQ ID NO: 46, die variable CDR2-Sequenz der leichten Kette (L-CDR2), wie dargestellt durch SEQ ID NO: 47 und die variable CDR3-Sequenz der leichten Kette (L-CDR3), wie dargestellt durch SEQ ID NO: 48,

(vi) für einen ein anti-TWEAKR Antikörper umfassend eine variable Region der schwere Kette (VH) umfassend die variable CDR1-Sequenz der schweren Kette (H-CDR1), wie dargestellt durch SEQ ID NO: 52, die variable CDR2-Sequenz der schweren Kette (H-CDR2), wie dargestellt durch SEQ ID NO: 53 und die variable CDR3-Sequenz der schweren Kette (H-CDR3), wie dargestellt durch SEQ ID NO: 54, sowie eine variable Region der leichte Kette (VL) umfassend die variable CDR1-Sequenz der leichten Kette (L-CDR1), wie dargestellt durch SEQ ID NO: 56, die variable CDR2-Sequenz der leichten Kette (L-CDR2), wie dargestellt durch SEQ ID NO: 57 und die variable CDR3-Sequenz der leichten Kette (L-CDR3), wie dargestellt durch SEQ ID NO: 58,

(vii) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 62, die variable CDR2-Sequenz der schweren Kette (H-CDR2), wie dargestellt durch SEQ ID NO: 63 und die variable CDR3-Sequenz der schweren Kette (H-CDR3), wie dargestellt durch SEQ ID NO: 64, sowie eine variable Region der leichte Kette (VL) umfassend die variable CDR1-Sequenz der leichten Kette (L-CDR1), wie dargestellt durch SEQ ID NO: 66, die variable CDR2-Sequenz der leichten Kette (L-CDR2), wie dargestellt durch SEQ ID NO: 67 und die variable CDR3-Sequenz der leichten Kette (L-CDR3), wie dargestellt durch SEQ ID NO: 68,

(viii) für einen anti-HER2 Antikörper umfassend eine variable Region der schwere Kette (VH) umfassend die variable CDR1-Sequenz der schweren Kette (H-CDR1), wie dargestellt durch SEQ ID NO: 72, die variable CDR2-Sequenz der schweren Kette (H-CDR2), wie dargestellt durch SEQ ID NO: 73 und die variable CDR3-Sequenz der schweren Kette (H-CDR3), wie dargestellt durch SEQ ID NO: 74, sowie eine variable Region der leichte Kette (VL) umfassend die variable CDR1-Sequenz der leichten Kette (L-CDR1), wie dargestellt durch SEQ ID NO: 76, die variable CDR2-Sequenz der leichten Kette (L-CDR2), wie dargestellt durch SEQ ID NO: 77 und die variable CDR3-Sequenz der leichten Kette (L-CDR3), wie dargestellt durch SEQ ID NO: 78,

(ix) für einen anti-HER2 Antikörper umfassend eine variable Region der schwere Kette (VH) umfassend die variable CDR1-Sequenz der schweren Kette (H-CDR1), wie dargestellt durch SEQ ID NO: 82, die variable CDR2-Sequenz der schweren Kette (H-CDR2), wie dargestellt durch SEQ ID NO: 83 und die variable CDR3-Sequenz der schweren Kette (H-CDR3), wie dargestellt durch SEQ ID NO: 84, sowie eine variable Region der leichte Kette (VL) umfassend die variable CDR1-Sequenz der leichten Kette (L-CDR1), wie dargestellt durch SEQ ID NO: 86, die variable CDR2-Sequenz der leichten Kette (L-CDR2), wie dargestellt durch SEQ ID NO: 87 und die variable CDR3-Sequenz der leichten Kette (L-CDR3), wie dargestellt durch SEQ ID NO: 88,

(x) für einen anti-B7H3 Antikörper umfassend eine variable Region der schwere Kette (VH) umfassend die variable CDR1-Sequenz der schweren Kette (H-CDR1), wie dargestellt durch SEQ ID NO: 92, die variable CDR2-Sequenz der schweren Kette (H-CDR2), wie dargestellt durch SEQ ID NO: 93 und die variable CDR3-Sequenz der schweren Kette (H-CDR3), wie dargestellt durch SEQ ID NO: 94, sowie eine variable Region der leichte Kette (VL) umfassend die variable CDR1-Sequenz der leichten Kette (L-CDR1), wie dargestellt durch SEQ ID NO: 96, die variable CDR2-Sequenz der leichten Kette (L-CDR2), wie dargestellt durch SEQ ID NO: 97 und die variable CDR3-Sequenz der leichten Kette (L-CDR3), wie dargestellt durch SEQ ID NO: 98,

(xi) für einen anti-B7H3 Antikörper umfassend eine variable Region der schwere Kette (VH) umfassend die

variable CDR1-Sequenz der schweren Kette (H-CDR1), wie dargestellt durch SEQ ID NO: 102, die variable CDR2-Sequenz der schweren Kette (H-CDR2), wie dargestellt durch SEQ ID NO: 103 und die variable CDR3-Sequenz der schweren Kette (H-CDR3), wie dargestellt durch SEQ ID NO: 104, sowie eine variable Region der leichte Kette (VL) umfassend die variable CDR1-Sequenz der leichten Kette (L-CDR1), wie dargestellt durch SEQ ID NO: 106, die variable CDR2-Sequenz der leichten Kette (L-CDR2), wie dargestellt durch SEQ ID NO: 107 und die variable CDR3-Sequenz der leichten Kette (L-CDR3), wie dargestellt durch SEQ ID NO: 108,
(xii) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 112, die variable CDR2-Sequenz der schweren Kette (H-CDR2), wie dargestellt durch SEQ ID NO: 113 und die variable CDR3-Sequenz der schweren Kette (H-CDR3), wie dargestellt durch SEQ ID NO: 114, sowie eine variable Region der leichte Kette (VL) umfassend die variable CDR1-Sequenz der leichten Kette (L-CDR1), wie dargestellt durch SEQ ID NO: 116, die variable CDR2-Sequenz der leichten Kette (L-CDR2), wie dargestellt durch SEQ ID NO: 117 und die variable CDR3-Sequenz der leichten Kette (L-CDR3), wie dargestellt durch SEQ ID NO: 118,
(xiii) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 122, die variable CDR2-Sequenz der schweren Kette (H-CDR2), wie dargestellt durch SEQ ID NO: 123 und die variable CDR3-Sequenz der schweren Kette (H-CDR3), wie dargestellt durch SEQ ID NO: 124, sowie eine variable Region der leichte Kette (VL) umfassend die variable CDR1-Sequenz der leichten Kette (L-CDR1), wie dargestellt durch SEQ ID NO: 126, die variable CDR2-Sequenz der leichten Kette (L-CDR2), wie dargestellt durch SEQ ID NO: 127 und die variable CDR3-Sequenz der leichten Kette (L-CDR3), wie dargestellt durch SEQ ID NO: 128,
(xiv) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 132, die variable CDR2-Sequenz der schweren Kette (H-CDR2), wie dargestellt durch SEQ ID NO: 133 und die variable CDR3-Sequenz der schweren Kette (H-CDR3), wie dargestellt durch SEQ ID NO: 134, sowie eine variable Region der leichte Kette (VL) umfassend die variable CDR1-Sequenz der leichten Kette (L-CDR1), wie dargestellt durch SEQ ID NO: 136, die variable CDR2-Sequenz der leichten Kette (L-CDR2), wie dargestellt durch SEQ ID NO: 137 und die variable CDR3-Sequenz der leichten Kette (L-CDR3), wie dargestellt durch SEQ ID NO: 138,
(xv) für einen ein anti-TWEAKR Antikörper umfassend eine variable Region der schwere Kette (VH) umfassend die variable CDR1-Sequenz der schweren Kette (H-CDR1), wie dargestellt durch SEQ ID NO: 142, die variable CDR2-Sequenz der schweren Kette (H-CDR2), wie dargestellt durch SEQ ID NO: 143 und die variable CDR3-Sequenz der schweren Kette (H-CDR3), wie dargestellt durch SEQ ID NO: 144, sowie eine variable Region der leichte Kette (VL) umfassend die variable CDR1-Sequenz der leichten Kette (L-CDR1), wie dargestellt durch SEQ ID NO: 146, die variable CDR2-Sequenz der leichten Kette (L-CDR2), wie dargestellt durch SEQ ID NO: 147 und die variable CDR3-Sequenz der leichten Kette (L-CDR3), wie dargestellt durch SEQ ID NO: 148, oder
(xvi) für einen anti-TWEAKR Antikörper umfassend eine variable Region der schwere Kette (VH) umfassend die variable CDR1-Sequenz der schweren Kette (H-CDR1), wie dargestellt durch SEQ ID NO: 152, die variable CDR2-Sequenz der schweren Kette (H-CDR2), wie dargestellt durch SEQ ID NO: 153 und die variable CDR3-Sequenz der schweren Kette (H-CDR3), wie dargestellt durch SEQ ID NO: 154, sowie eine variable Region der leichte Kette (VL) umfassend die variable CDR1-Sequenz der leichten Kette (L-CDR1), wie dargestellt durch SEQ ID NO: 156, die variable CDR2-Sequenz der leichten Kette (L-CDR2), wie dargestellt durch SEQ ID NO: 157 und die variable CDR3-Sequenz der leichten Kette (L-CDR3), wie dargestellt durch SEQ ID NO: 158 steht,

oder für ein Antigen-bindendes Fragment von diesen Antikörpern steht.

**35.** Verbindung oder Konjugat nach einem oder mehreren der vorhergehenden Ansprüche, wobei der Antikörper (AK, AB, AK1, AK2, AK3)

(i) für einen anti-EGFR Antikörper umfassend eine variable Region der schweren Kette (VH) entsprechend SEQ ID NO: 1 sowie eine variable Region der leichten Kette (VL) entsprechend SEQ ID NO: 5,
(ii) für einen anti-HER2 Antikörper umfassend eine variable Region der schweren Kette (VH) entsprechend SEQ ID NO: 11 sowie eine variable Region der leichten Kette (VL) entsprechend SEQ ID NO: 15,
(iii) für einen anti-TWEAKR Antikörper umfassend eine variable Region der schweren Kette (VH) entsprechend SEQ ID NO: 21 sowie eine variable Region der leichten Kette (VL) entsprechend SEQ ID NO: 25,
(iv) für einen anti-TWEAKR Antikörper umfassend eine variable Region der schweren Kette (VH) entsprechend SEQ ID NO: 31 sowie eine variable Region der leichten Kette (VL) entsprechend SEQ ID NO: 35,
(v) für einen anti-TWEAKR Antikörper umfassend eine variable Region der schweren Kette (VH) entsprechend SEQ ID NO: 41 sowie eine variable Region der leichten Kette (VL) entsprechend SEQ ID NO: 45,
(vi) für einen anti-TWEAKR Antikörper umfassend eine variable Region der schweren Kette (VH) entsprechend

SEQ ID NO: 51 sowie eine variable Region der leichten Kette (VL) entsprechend SEQ ID NO: 55,
(vii) für einen anti-TWEAKR Antikörper umfassend eine variable Region der schweren Kette (VH) entsprechend SEQ ID NO: 61 sowie eine variable Region der leichten Kette (VL) entsprechend SEQ ID NO: 65,
(viii) für einen anti-HER2 Antikörper umfassend eine variable Region der schweren Kette (VH) entsprechend SEQ ID NO: 71 sowie eine variable Region der leichten Kette (VL) entsprechend SEQ ID NO: 75,
(ix) für einen anti-HER2 Antikörper umfassend eine variable Region der schweren Kette (VH) entsprechend SEQ ID NO: 81 sowie eine variable Region der leichten Kette (VL) entsprechend SEQ ID NO: 85,
(x) für einen anti-B7H3 Antikörper umfassend eine variable Region der schweren Kette (VH) entsprechend SEQ ID NO: 91 sowie eine variable Region der leichten Kette (VL) entsprechend SEQ ID NO: 95,
(xi) für einen anti-B7H3 Antikörper umfassend eine variable Region der schweren Kette (VH) entsprechend SEQ ID NO: 101 sowie eine variable Region der leichten Kette (VL) entsprechend SEQ ID NO: 105,
(xii) für einen anti-TWEAKR Antikörper umfassend eine variable Region der schweren Kette (VH) entsprechend SEQ ID NO: 111 sowie eine variable Region der leichten Kette (VL) entsprechend SEQ ID NO: 115,
(xiii) für einen anti-TWEAKR Antikörper umfassend eine variable Region der schweren Kette (VH) entsprechend SEQ ID NO: 121 sowie eine variable Region der leichten Kette (VL) entsprechend SEQ ID NO: 125,
(xiv) für einen anti-TWEAKR Antikörper umfassend eine variable Region der schweren Kette (VH) entsprechend SEQ ID NO: 131 sowie eine variable Region der leichten Kette (VL) entsprechend SEQ ID NO: 135,
(xv) für einen anti-TWEAKR Antikörper umfassend eine variable Region der schweren Kette (VH) entsprechend SEQ ID NO: 141 sowie eine variable Region der leichten Kette (VL) entsprechend SEQ ID NO: 145, oder
(xvi) für einen anti-TWEAKR Antikörper umfassend eine variable Region der schweren Kette (VH) entsprechend SEQ ID NO: 151 sowie eine variable Region der leichten Kette (VL) entsprechend SEQ ID NO: 155 steht,

oder für ein Antigen-bindendes Fragment von diesen Antikörpern steht.

**36.** Verbindung oder Konjugat nach einem oder mehreren der vorhergehenden Ansprüche, wobei der Antikörper (AK, AB, AK1, AK2, AK3)

(i) für einen anti-EGFR Antikörper umfassend eine Region der schweren Kette entsprechend SEQ ID NO: 9 sowie eine Region der leichten Kette entsprechend SEQ ID NO: 10,
(ii) für einen anti-HER2 Antikörper umfassend eine Region der schweren Kette entsprechend SEQ ID NO: 19 sowie eine Region der leichten Kette entsprechend SEQ ID NO: 20,
(iii) für einen anti-TWEAKR Antikörper umfassend eine Region der schweren Kette entsprechend SEQ ID NO: 29 sowie eine Region der leichten Kette entsprechend SEQ ID NO: 30,
(iv) für einen anti-TWEAKR Antikörper umfassend eine Region der schweren Kette entsprechend SEQ ID NO: 39 sowie eine Region der leichten Kette entsprechend SEQ ID NO: 40,
(v) für einen anti-TWEAKR Antikörper umfassend eine Region der schweren Kette entsprechend SEQ ID NO: 49 sowie eine Region der leichten Kette entsprechend SEQ ID NO: 50,
(vi) für einen anti-TWEAKR Antikörper umfassend eine Region der schweren Kette entsprechend SEQ ID NO: 59 sowie eine Region der leichten Kette entsprechend SEQ ID NO: 60,
(vii) für einen anti-TWEAKR Antikörper umfassend eine Region der schweren Kette entsprechend SEQ ID NO: 69 sowie eine Region der leichten Kette entsprechend SEQ ID NO: 70,
(viii) für einen anti-HER2 Antikörper umfassend eine Region der schweren Kette entsprechend SEQ ID NO: 79 sowie eine Region der leichten Kette entsprechend SEQ ID NO: 80,
(ix) für einen anti-HER2 Antikörper umfassend eine Region der schweren Kette entsprechend SEQ ID NO: 89 sowie eine Region der leichten Kette entsprechend SEQ ID NO: 90,
(x) für einen anti-B7H3 Antikörper umfassend eine Region der schweren Kette entsprechend SEQ ID NO: 99 sowie eine Region der leichten Kette entsprechend SEQ ID NO: 100,
(xi) für einen anti-B7H3 Antikörper umfassend eine Region der schweren Kette entsprechend SEQ ID NO: 109 sowie eine Region der leichten Kette entsprechend SEQ ID NO: 110,
(xii) für einen anti-TWEAKR Antikörper umfassend eine Region der schweren Kette entsprechend SEQ ID NO: 119 sowie eine Region der leichten Kette entsprechend SEQ ID NO: 120,
(xiii) für einen anti-TWEAKR Antikörper umfassend eine Region der schweren Kette entsprechend SEQ ID NO: 129 sowie eine Region der leichten Kette entsprechend SEQ ID NO: 130,
(xiv) für einen anti-TWEAKR Antikörper umfassend eine Region der schweren Kette entsprechend SEQ ID NO: 139 sowie eine Region der leichten Kette entsprechend SEQ ID NO: 140,
(xv) für einen anti-TWEAKR Antikörper umfassend eine Region der schweren Kette entsprechend SEQ ID NO: 149 sowie eine Region der leichten Kette entsprechend SEQ ID NO: 150, oder
(xvi) für einen anti-TWEAKR Antikörper umfassend eine Region der schweren Kette entsprechend SEQ ID NO:

159 sowie eine Region der leichten Kette entsprechend SEQ ID NO: 160 steht,

oder für ein Antigen-bindendes Fragment von diesen Antikörpern steht.

**37.** Pharmazeutische Zusammensetzung umfassend eine Verbindung nach einem oder mehreren der vorhergehenden Ansprüche in Kombination mit einem inerten, nicht-toxischen, pharmazeutisch geeigneten Hilfsstoff.

**38.** Verbindung nach einem oder mehreren der vorhergehenden Ansprüche zur Verwendung in einem Verfahren zur Behandlung und/oder Prophylaxe von Krankheiten.

**39.** Verbindung nach einem oder mehreren der vorhergehenden Ansprüche zur Verwendung zur Verwendung in einem Verfahren zur Behandlung von hyperproliferativen und/oder angiogenen Erkrankungen.

## Claims

**1.** A compound of the following formula Ia:

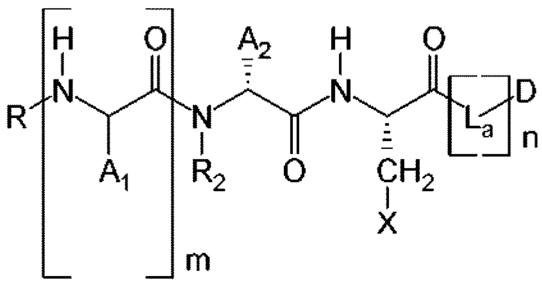

(Ia),

where

m is 0 to 2;
n is 0 or 1;
X stands for $-C(=O)-NH_2$ or -COOH,
$L_a$ stands for a self-immolative linker,
$A_1$ stands for a residue of one of the amino acids Gly, Pro, Ala, Val, Nva, Leu, Ile, Met, Phe, Tyr, Trp, Ser, Thr, Cys, Asn, Gln, Asp, Glu, Lys, Arg, citrulline or His, or one of the respective N-alkyl amino acids,
$A_2$ stands for a residue of one of the amino acids D-Ala, D-Pro, D-Ser, D-Asn, D-Asp or D-His,
$R_2$ stands for -H or $C_1$-$C_3$ alkyl,
or $R_2$ stands for the binding to the methylene group of the proline ring, in the event of $A_2$ standing for a residue of D-Pro,
D stands for $-D_1-(L_b)_o-(LIG)_p$,
$D_1$ stands for a cytotoxic agent,
LIG stands for a binder, which, after binding to a target molecule of a tumour cell, is internalised by the tumour cell and processed intracellularly, preferably lysosomally,
$L_b$ stands for a linker,
o and p are 0 or 1, in each case independently of one another,
R stands for $Z_1$-(C=O)q-,
q is 0 or 1,
$Z_1$ stands for a $C_{1-10}$ alkyl-, $C_{5-10}$ aryl- or $C_{6-10}$ aralkyl-, $C_{5-10}$heteroalkyl-, $C_{1-10}$ alkyl-O-$C_{6-10}$ aryl-, $C_{5-10}$ heterocycloalkyl-, heteroaryl-, heteroaryl-alkyl-, $C_{5-10}$ heteroaryl-alkoxy-, $C_{1-10}$ alkoxy-, $C_{6-10}$ aryl-, $C_{1-10}$alkyloxy-, $C_{6-10}$ aryloxy- or $C_{6-10}$ aralkoxy-, $C_{5-10}$ heteroalkoxy-, $C_{1-10}$ alkyl-O-$C_{6-10}$ aryloxy- or $C_{5-10}$ heterocyclo-alkoxy group, which can be substituted once or more by $-NH_2$,-C(=O), -NH-alkyl, $-N(alkyl)_2$, -NH-C(=O)-alkyl, -N(alkyl)-C(=O)-alkyl,$-S(=O)_3-H$, $-S(=O)_2-NH_2$, $-S(=O)_2-N(alkyl)_2$, -COOH, $-C(=O)NH_2$, $-C(=O)-N(Alkyl)_2$ or -OH, or for -H or a group $-(CH_2)_{0-1}-O_x-(CH_2CH_2O)_v-R^1$,
x is 0 or 1
v is a number from 1 to 20,
$R^1$ stands for -H, -alkyl, $-CH_2-COOH$, $-CH_2-CH_2-COOH$ or $-CH_2-CH_2-NH_2$, or

R stands for LIG-$(L_c)_r$,
LIG stands for a binder, which, after binding to a target molecule of a tumour cell, is internalised by the tumour cell and processed intracellularly, preferably lysosomally,
$L_c$ stands for a linker; and
r is 0 or 1.

**2.** The compound in accordance with Claim 1, wherein $R_2$ is -H or a methyl group, preferably -H.

**3.** The compound in accordance with one or more of the foregoing claims, wherein m is1.

**4.** The compound in accordance with one or more of the foregoing claims, wherein $A_1$ exists in the "L" configuration or "D" configuration.

**5.** The compound in accordance with one or more of the foregoing claims, wherein $A_1$ stands for a residue of the amino acids Ala or Val.

**6.** The compound in accordance with one or more of the foregoing claims, wherein q is 1, and $Z_1$ constitutes a $C_{1-10}$-alkyl, which can be interrupted once or more by an oxygen atom (such as a methyl group or, if applicable, an alkylated oligoalkylene oxide chain), a $C_{6-10}$-aralkyl-, $C_{5-10}$-heteroaryl-alkyl-, $C_{6-10}$-aralkoxy-, or $C_{5-10}$-heteroaryl-alkoxy group.

**7.** The compound in accordance with one or more of the foregoing claims, wherein $L_a$ is selected from the following groups:

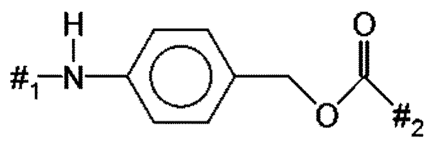

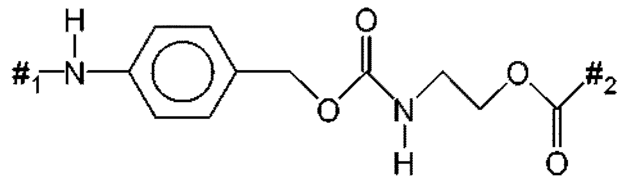

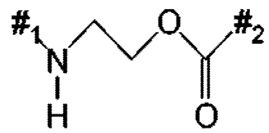

wherein $\#_1$ constitutes the binding to the carbonyl group and $\#_2$ the binding to the hydroxyl or amino group of $D_1$.

**8.** The compound in accordance with one or more of the foregoing claims, wherein $D_1$ constitutes an agent selected from mitomycin, doxorubicin, aminopterin, actinomycin, bleomycin, 9-amino-camptothecin, n8-acetyl-spermidine, 1-(2-chloroethyl)-1,2-dimethane sulfonyl hydrazide, tallysomycin, cytarabine, etoposide, camptothecin, taxol, esperamicin, podophyllotoxin, anguidine, vincristine, vinblastine, morpholine-doxorubicin, n-(5,5-diacetoxy-pentyl)-doxorubicin, duocarmycin, auristatin, pyrrolobenzodiazepine derivatives, calicheamicin, daunorubicin, camptophecin DX8951 (exatecan) or uses a kinesin spindle protein inhibitor (KSP inhibitor), wherein the agent is bound to $L_a$ (when n=1) or the carbonyl group (when n=0) via its hydroxyl or amino group, in accordance with Formula I.

**9.** The compound in accordance with Claim 8, wherein the KSP inhibitor is a compound of the following formula (IIa),

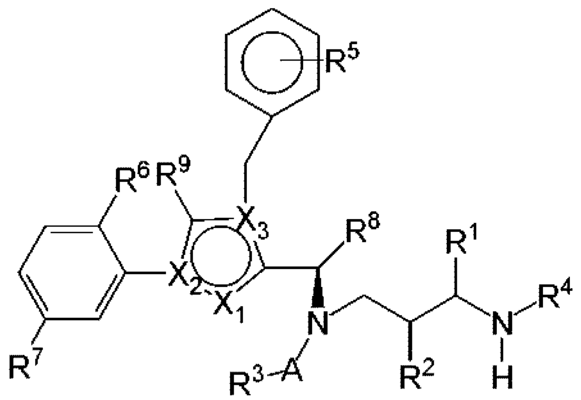

(IIa)

where

$X_1$ stands for N,
$X_2$ for N and
$X_3$ for C,

or

$X_1$ stands for N,
$X_2$ for C and
$X_3$ for N,

or

$X_1$ stands for CH or CF,
$X_2$ for C and
$X_3$ for N,

or

$X_1$ stands for NH,
$X_2$ for C and
$X_3$ for C,

of

$X_1$ stands for CH,
$X_2$ for N and
$X_3$ for C
A stands for -C(=O)-, -S(=O)-, $-S(=O)_2-$, or $-S(=O)_2-NH-$
$R^1$ stands for -H, -L-#1, -MOD or $-(CH_2)_{0-3}Z$,
Z stands for -H, $-NHY^3$, $-OY^3$, $-SY^3$, halogen, $-C(=O)-NY^1Y^2$, or $-C(=O)-OY^3$,
$Y^1$ and $Y^2$ independently from one another, stand for -H, $-NH_2$, $-(CH_2CH_2O)_{0-3}-(CH_2)_{0-3}Z'$ or $-CH(CH_2W)Z'$,
$Y^3$ stands for -H or $-(CH_2)_{0-3}Z'$,
Z' stands for -H, $-NH_2$, $-S(=O)_3H$, -COOH, $-NH-C(=O)-CH_2-CH_2-CH(NH_2)C(=O)-$ or $-(C(=O)-NH-CHY^4)_{1-3}COOH$,
W stands for -H- or- OH,
$Y^4$ stands for linear or branched $C_{1-6}$ alkyl, which may be substituted by $-NH-C(=O)-NH_2$, or stands for aryl or benzyl, which may be substituted by $-NH_2$,
$R_2$ stands for -H, -L-#1, -MOD, $-C(=O)-CHY^4-NHY^5$ or $-(CH_2)_{0-3}Z$,
Z stands for -H, halogen, $-OY^3$, $-SY^3$, $-NHY^3$, $-C(=O)-NY^1Y^2$, or $-C(=O)-OY^3$,
$Y^1$ and $Y^2$ independently from one another, stand for -H, $-NH_2$, or $-(CH_2)_{0-3}Z'$,

$Y^3$ stands for -H or $-(CH_2)_{0-3}Z^1$,
Z' stands for -H, $-S(=O)_3H$, $-NH_2$ or -COOH,
$Y^4$ stands for linear or branched $C_{1-6}$ alkyl, which may be substituted by-NHC(=O)-$NH_2$, or for aryl or benzyl, which may be substituted by-NH2,
$Y^5$ stands for -H or -C(=O)-$CHY^6$-$NH_2$,
$Y^6$ stands for linear or branched $C_{1-6}$ alkyl,
$R^3$ stands for -MOD, -L-#1, or any alkyl, cycloalkyl, aryl, heteroaryl, heteroalkyl or heterocycloalkyl group which may be substituted, which can be substituted by one to three OH groups, one to three halogen atoms, one to three mono-, di- or tri-halogenated alkyl groups, one to three -O-alkyl groups, one to three -SH groups, one to three -S-alkyl groups, one to three -O-C(=O)-alkyl groups, one to three -O-C(=O)-NH-alkyl groups, one to three -NH-C(=O)-alkyl groups, one to three -NH-C(=O)-NH-alkyl groups, one to three $-S(=O)_n$-alkyl groups, one to three $-S(=O)_2$-NH alkyl groups, 1-3 -NH alkyl groups, one to three $-N(alkyl)_2$ groups, one to three $NH_2$ groups or one to three $-(CH_2)_{0-3}Z$ groups,
n is 0, 1 or 2,
Z stands for -H, halogen, $-OY^3$, $-SY^3$, $-NHY^3$, $-C(=O)-NY^1Y^2$ or $-C(=O)-OY^3$,
$Y^1$ and $Y^2$ independently from one another, stand for -H, $-NH_2$, or $-(CH_2)_{0-3}Z'$,
$Y^3$ stands for -H, $-(CH_2)_{0-3}$-CH(NHC(=O)$CH_3$)Z', $-(CH_2)_{0-3}$-CH($NH_2$)Z' or-$(CH_2)_{0-3}Z'$,
Z' stands for -H, $-S(=O)_3H$, $-NH_2$ or -C(=O)-OH,
$R^4$ stands for the group cleavable by legumain, having the formulae Ia', Ia" and Ia''',
$R^5$ stands for -H, $-NH_2$, $-NO_2$, halogen, -CN, $CF_3$, $-OCF_3$, $-CH_2F$, $-CH_2F$, -SH or-$(CH_2)_{0-3}Z$,
Z stands for -H, $-OY^3$, $-SY^3$, halogen, $-NHY^3$, $-C(=O)-NY^1Y^2$ , or $-C(=O)-OY^3$,
$Y^1$ and $Y^2$ independently from one another, stand for -H, $-NH_2$, or $-(CH_2)_{0-3}Z'$,
$Y^3$ stands for -H or $-(CH_2)_{0-3}Z'$,
Z' stands for -H, $-S(=O)_3H$, $-NH_2$ or -COOH,
$R^6$ and $R^7$ independently from one another, stand for -H, -CN, $C_{1-10}$-alkyl, fluorine-$C_{1-10}$-alkyl, $C_{2-10}$-alkenyl, fluorine-$C_{2-10}$-alkenyl, $C_{2-10}$-alkinyl, fluorine-$C_{2-10}$-alkinyl, hydroxy, $-NO_2$, $-NH_2$, -COOH or halogen,
$R^8$ stands for $C_{1-10}$-alkyl, fluorine-($C_{1-10}$-alkyl, $C_{2-10}$-alkenyl, fluorine-$C_{2-10}$-alkenyl, $C_{2-10}$-alkinyl, fluorine-$C_{2-10}$-alkinyl, $C_{4-10}$-cycloalkyl, fluorine-$C_{4-10}$-cycloalkyl or $-(CH_2)_{0-2}-(HZ^2)$,
$HZ^2$ stands for a 4 to 7-membered heterocycle with up to two heteroatoms, selected from N, O and S, which may be substituted by-OH, -COOH, $-NH_2$ or-L-#1,
$R^9$ stands for -H, -F, $-CH_3$, $-CF_3$, $-CH_2F$ or $-CHF_2$,
-L-#1 stands for-$(L_b)_o$-$(LIG)_p$,
LIG stands for a binder, which, after binding to a target molecule of a tumour cell, is internalised by the tumour cell and processed intracellularly, and preferably lysosomally,
$L_b$ stands for a linker,
o and p stand for 0 or 1, independently of one another,
-MOD constitutes $-(NR^{10})_n-(G1)_o$-G2-G3,
$R^{10}$ stands for -H or $C_1$-$C_3$ alkyl,
G1 stands for -NH-C-(=O)-, -C(=O)-NH- or

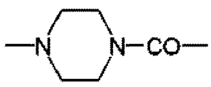
,

n is 0 or 1;
o is 0 or 1 and
G2 stands for a straight chain or branched hydrocarbon chain with 1 to 20 carbon atoms, which may be interrupted once or multiple times by -O-, -S-, -S(=O)-,$-S(=O)_2$-, $-NR^y$-, $-NR^yC(=O)$-, $-C(=O)NR^y$-, $-NR^yNR^y$-, $-S(=O)_2-NR^yNR^y$-,$-C(=O)-NR^yNR^y$-, -C(=O)-, $-CR^x$=N-O- and the straight chain or branched hydrocarbon chain can be substituted by -NH-C(=O)-$NH_2$, -COOH, -OH,$-NH_2$, sulphonamide, sulphone, sulphoxide, or sulphonic acid,
$R^y$ stands for -H, phenyl-, $C_1$-$C_{10}$-alkyl-, $C_2$-$C_{10}$-alkenyl or $C_2$-$C_{10}$-alkinyl, which can be substituted respectively by -NH-C(=O)-$NH_2$, -COOH, -OH, $-NH_2$, sulphonamide, sulphone, sulphoxide, or sulphonic acid,
Rx stands for -H, $C_1$-$C_3$ alkyl or phenyl
G3 stands for -H or -COOH, and
-MOD contains at least one COOH group,

as well as its salts, solvates and salts of the solvates.

**10.** The compound in accordance with Claim 9, wherein $R^1$ or $R^3$ stands for -L-#1.

**11.** The compound in accordance with one or more of Claims 9 and 10, wherein $X_1$ stands for CH, $X_2$ stands for C and $X_3$ stands for N.

**12.** The compound in accordance with one or more of Claims 9 to 11, wherein $R^6$ and $R^7$ independently from one another stand for -H, $C_{1-3}$-alkyl or halogen.

**13.** The compound in accordance with Claim 12, wherein $R^6$ and $R^7$ stand for F.

**14.** The compound in accordance with one or more of Claims 9 to 13, wherein $R^8$ stands for $C_{1-4}$-alkyl (preferably tert-butyl) or cyclohexyl.

**15.** The compound in accordance with one or more of Claims 9 to 14, wherein $R^9$ stands for -H.

**16.** The compound in accordance with one or more of the foregoing claims, wherein either D or R contains the binder LIG.

**17.** The compound in accordance with Claim 16, wherein LIG is a peptide, protein or derivative of it, selected from octreotide, GnRH-III, [D-Tyr$^6$, P-Ala$^{11}$, Phe$^{13}$, Nle$^{14}$]BN(6-14), NT(8-13), c(RGDfK), **HSDAVFTDNYTRLRKQ-MAVKKYLNSILN-NH$_2$** (SEQ ID NO: 161), NAPamide, [Phe$^7$, Pro$^{34}$]NPY, HER2-targeting peptide, ATEPRKQYATPRVFWTDAPG (SEQ ID NO: 162), LQWRRDDNVHNFGVWARYRL (SEQ ID NO: 163), or constitutes an antibody or a fragment or derivative of it, which binds to an extracellular target molecule of a tumour cell and may be conjugated with other cytotoxic agents.

**18.** Compounds in accordance with one or more of the foregoing claims, wherein said compounds possess the following formula III',

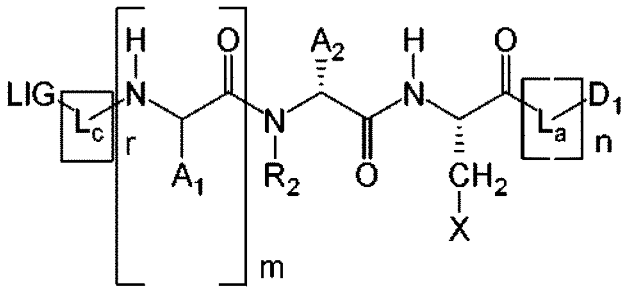

III'

where m, n, r, LIG, $L_a$, $L_c$, $D_1$,

X, $A_2$, $R_2$ and $A_1$ have the meanings specified in Claim 1.

**19.** Compound of the formula IIIa',

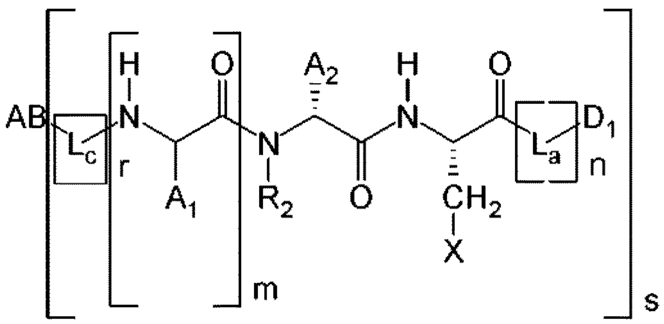

IIIa'

where $L_a$, $L_c$, Di, X,

$R_2$, $A_2$ and $A_1$ have the same meaning as in Claim 1,
AB stands for an antibody or an antigen-binding antibody fragment, and
s is 1 to 20, preferably 2 to 8; especially preferably 2 to 6.

**20.** Compounds in accordance with one or more of Claims 1 to 17, wherein the compounds have the following formula IV'

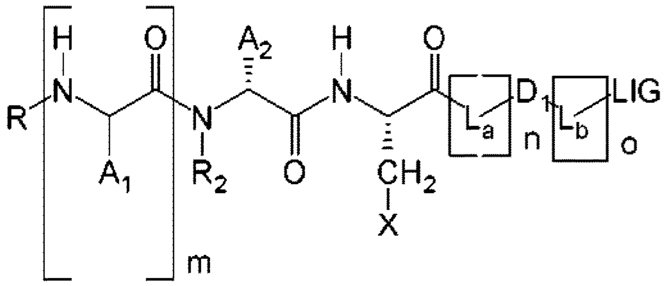

IV'

where

m, n, o, R, LIG, $L_a$, $L_b$,
$D_1$, X, $R_2$, $A_2$ and $A_1$ have the same meaning as in Claim 1.

**21.** Compound of the formula IVa'

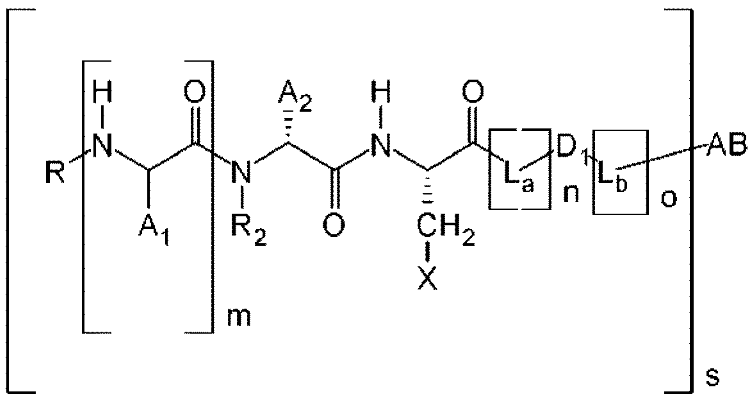

IVa'

where

m, n, o, R, $L_a$, $L_b$, $D_1$, X, $R_2$, $A_2$ and $A_1$ have the same meaning as in Claim 1,
AB stands for an antibody or an antigen-binding antibody fragment, and
s is 1 to 20, preferably 2 to 8;

**22.** Compounds in accordance with one or more of the foregoing claims, wherein the linker $L_b$ or $L_c$ is bound to a cysteine side chain or a cysteine residue of a binder, such as an antibody or an antigen-binding antibody fragment, and has the following formula:

§-(C=O)m-L1-L2-§§

wherein

m is 0 or 1;
§ stands for the binding to the drug molecule or the cleavable legumain group; and
§§ stands for the binding to the binder
-L2- stands for the group

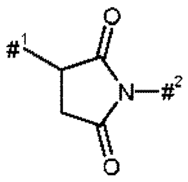, 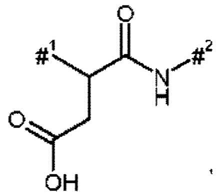, 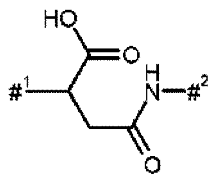, 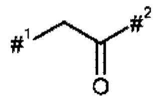, 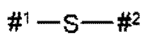,

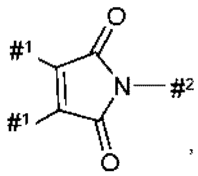, 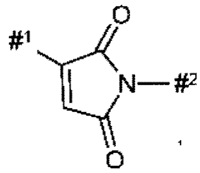, 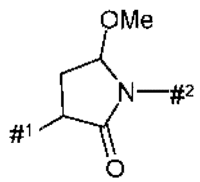 or 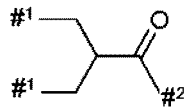,

wherein

#[1] designates the point of attachment with the sulphur atom of the binder,
#[2] designates the point of attachment with the group $L^1$,
L1 stands for-$(NR^{10})_n$-$(G1)_o$-G2-,
$R^{10}$ stands for -H, $-NH_2$ or $C_1$-$C_3$-alkyl,
G1 stands for -NHC(=O)- or

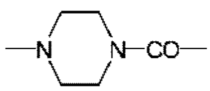,

n is 0 or 1,
o is 0 or 1,

G2 stands for a straight chain or branched hydrocarbon chain with 1 to 100 carbon atoms from arylene groups and/or straight chain and/or branched and/or cyclic alkylene groups, which can be interrupted once or multiple times by one or more of the groups -O-, -S-, -S(=O)-, $-S(=O)_2$, -NH-, -C(=O)-, -Nme-, -NHNH-, $-S(=O)_2$-NHNH-, -NH-C(=O)-, -C(=O)-NH-, - C(=O)-NHNH- and a 5 to 10-membered, aromatic or non-aromatic heterocycle with up to 4 heteroatoms, selected from N, O and S, -S(=O)- or $-S(=O)_2$ (preferably

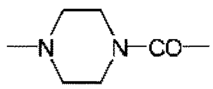

), and wherein the side chains, if they are present, can be substituted by-NH-C(=O)-$NH_2$, -COOH, - OH, $-NH_2$, NH-$CNNH_2$, sulphonamide, sulphone, sulphoxide or sulphonic acid, or constitutes one of the following groups

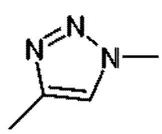, 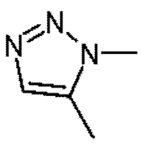, 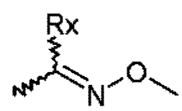

or

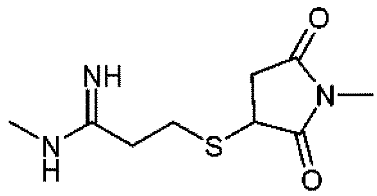

wherein

Rx stands for -H, $C_1$-$C_3$-alkyl or -phenyl.

**23.** Compounds in accordance with Claim 22, wherein L2 stands for one or both of the following formulae

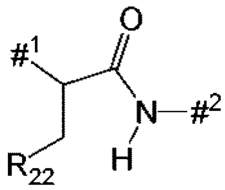

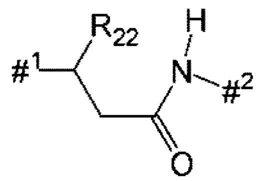

,

wherein

#[1] stands for the point of attachment to the sulphur atom of the binder,
#[2] stands for the point of attachment to the group $L^1$,
$R^{22}$ stands for -COOH and

the bonds with the sulphur atom of the binder exist to the extent of over 80% (in regard to the total number of bonds of the linker to the binder) in one of these two formulae.

**24.** Compounds in accordance with Claim 22 or 23, wherein the hydrocarbon chain is interrupted by one of the following groups,

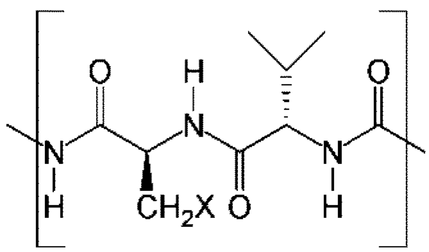

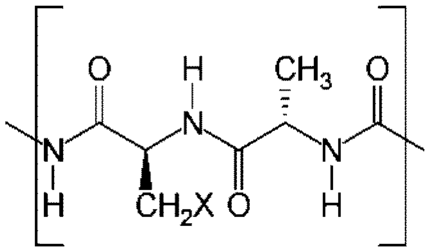

wherein,
X stands for -H or a $C_{1-10}$-alkyl group, which may be substituted by -NH-C(=O)-$NH_2$, -COOH, -OH, -$NH_2$, -NH-$CNNH_2$, sulphone, sulphoxide or sulphonic acid.

**25.** Compounds in accordance with one of Claims 22 to 24, wherein the linker possesses the following formula

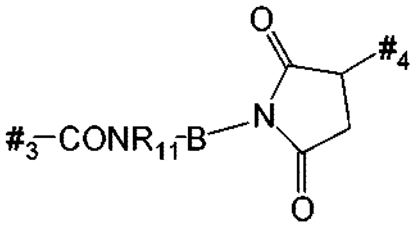

, where

#3 stands for the binding to the drug molecule,
#4 stands for the binding to the binder
$R^{11}$ stands for -H or -$NH_2$,
B stands for the group -[$(CH_2)_x$-$(X^4)_y$]$_w$-$(CH_2)_z$-,
w is 0 to 20;
x is 0 to 5;
x is 0 to 5;
y is 0 or 1;
z is 0 to 5; and

$X^4$ stands for -O-, -C(=O)-NH-, -NH-C'(=O)- or

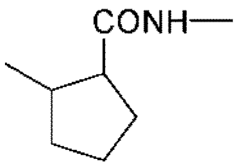

.

**26.** Compounds in accordance with one or more of the foregoing claims, wherein the linker-$L_b$- and/or-$L_c$- is bound to a cysteine side chain or a cysteine residue and has the following formula,

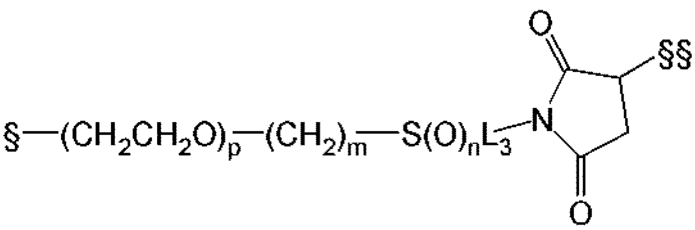

where

§ stands for the binding to the drug molecule or the group cleavable through legumain,
§§ stands for the binding to the binder,
m is 0, 1, 2 or 3,
n is 0, 1 or 2,
p is 0 to 20,
L3 stands for the group

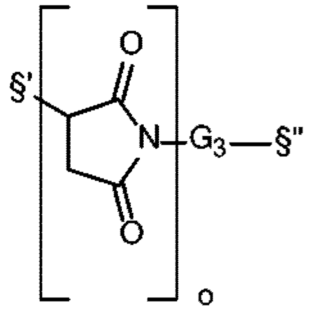

,

where

o is 0 or 1,
§' stands for the binding to the -S(O)n group and
§" for the binding to the "N" atom in the ring
G3 stands for a straight chain or branched hydrocarbon chain with 1 to 100 carbon atoms from arylene groups and/or straight chain and/or branched and/or cyclic alkylene groups, which can be interrupted once or multiple times by one or more of the groups -O-, -S-, -S(=O)-, $S(=O)_2$-, -NH-, -C(=O)-, -NH-C(=O)-,-C(=O)-NH- and a 5 to 10-membered, aromatic or non-aromatic heterocycle with up to 4 heteroatoms selected from N, O and S, -Nme-, -NHNH-, -$S(=O)_2$-NHNH-, -C(=O)-NHNH-, -S(=O)- or-$S(=O)_2$- (preferably

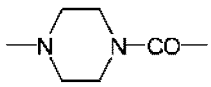

), wherein the side chains, in so far as they exist, can be substituted by-NHC(=O)-$NH_2$, -COOH, -OH, sulphone, sulphoxide or sulphonic acid.

**27.** Compounds in accordance with Claim 26, wherein the linker -Lb- or -$L_c$- is bound to a cysteine side chain and has the following formula

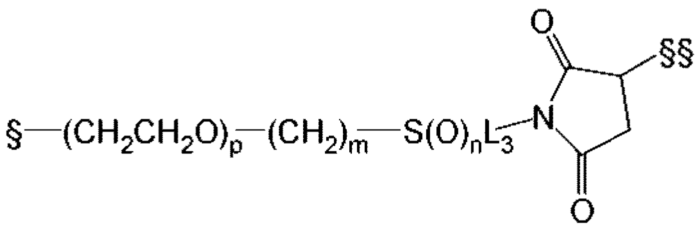

,

wherein

§ stands for the binding to the drug molecule or the group cleavable through legumain,
§§ stands for the binding to the binder,
m is 1;
p is 0;

n is 0;
L3 stands for the group

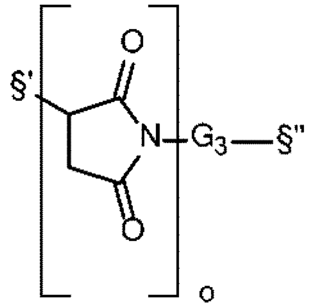

,

where

o is 0 or 1,
$G_3$ stands for $-(CH_2CH_2O)_s-(CH_2)_t-(C(=O)-NH)_u-CH_2CH_2O)_v-(CH_2)_w-$,
s, t, v and w stand for 0 to 20, independently of one another,
u is 0 or 1,
§' stands for the binding to the -S(O)n group and
§" for the binding to the "N" atom in the ring.

**28.** Compounds in accordance with Claim 25 or 26, wherein $R^2$ or $R^3$ stands for -L-#1.

**29.** Conjugates in accordance with one or more of the foregoing claims of the following structures, where

AK stands for the binder, preferably for an antibody or an antigen-binding antibody fragment ($AK_1$, $AK_2$, $AK_3$), and
$AK_1$ preferably stands for an antibody which is bound to the KSP inhibitor via a cysteine residue,
$AK_2$ preferably stands for an antibody which is bound to the KSP inhibitor via a lysine residue, and
$AK_3$ preferably stands for an antibody which is bound to the KSP inhibitor via a glutamine residue,
and
n stands for a number from 1 to 50, preferably 1 to 20, especially preferably 2 to 8 and in particular 2 to 6:

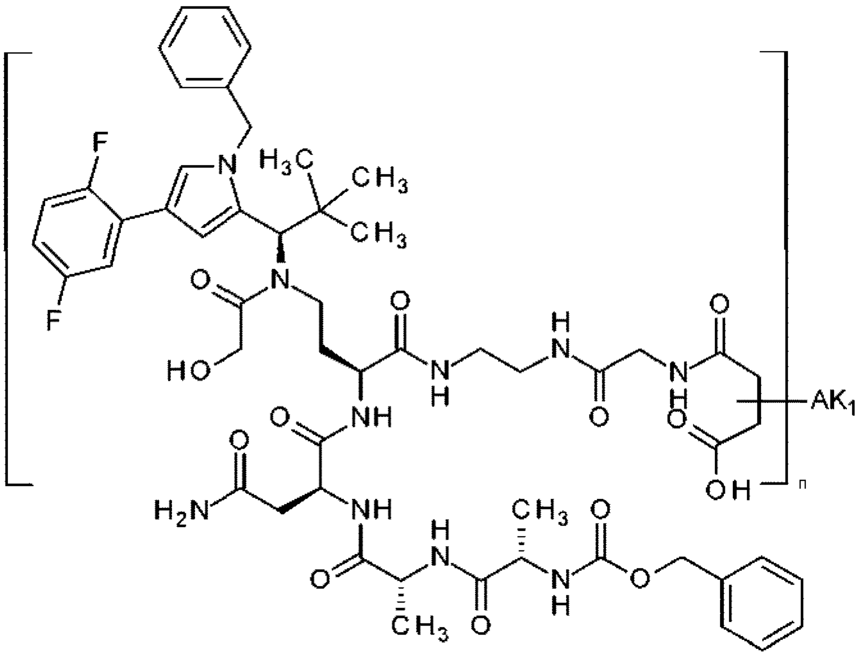

,

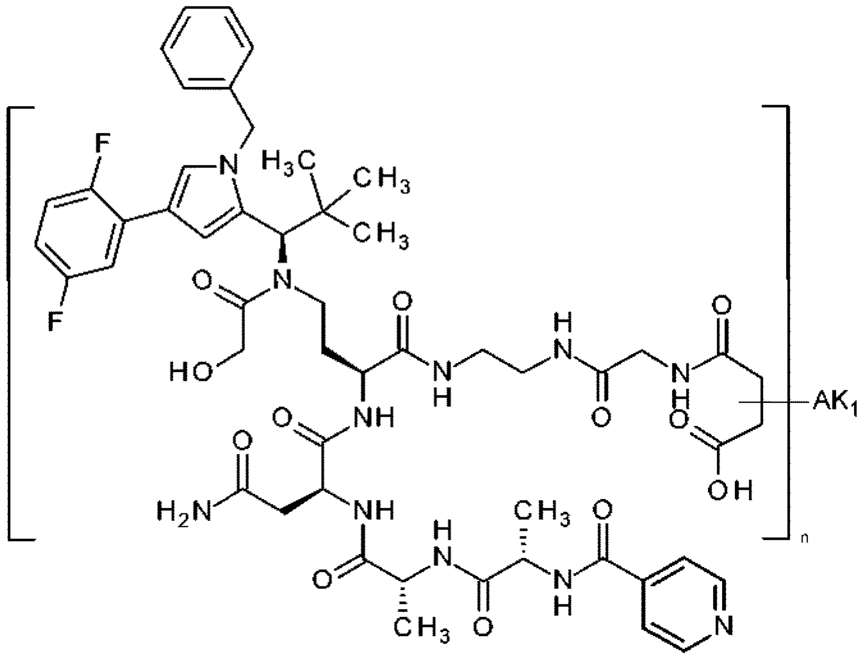

,

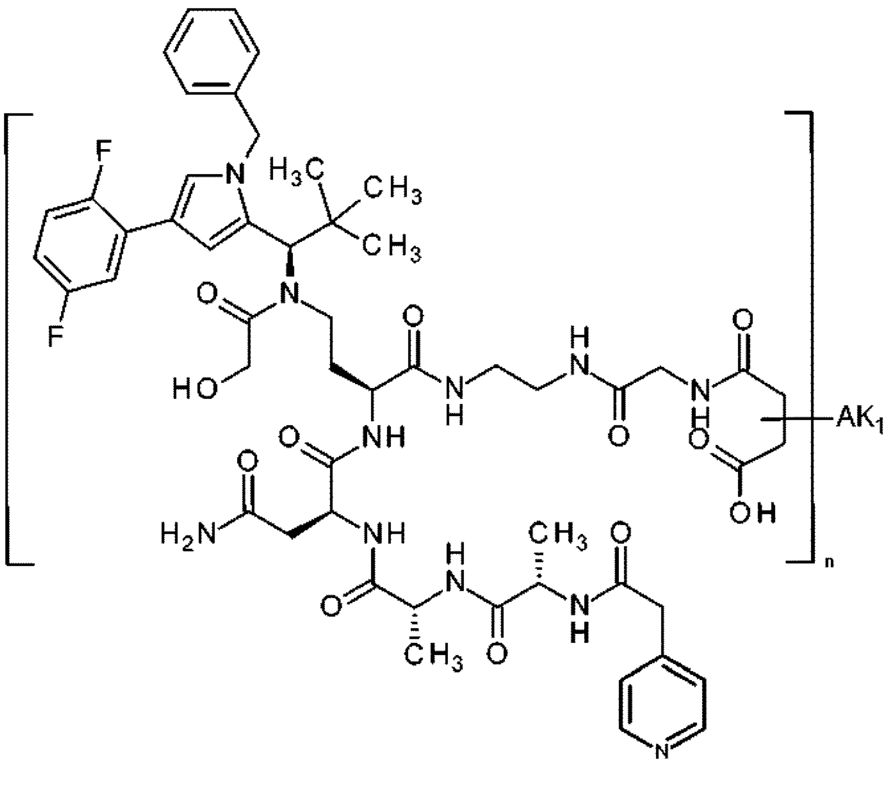

,

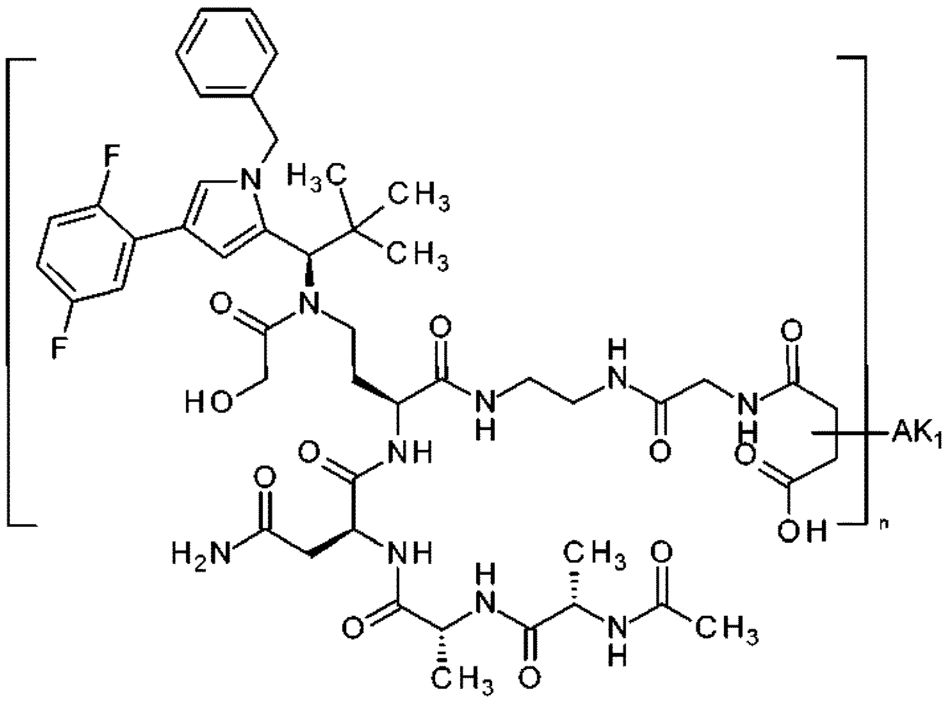

,

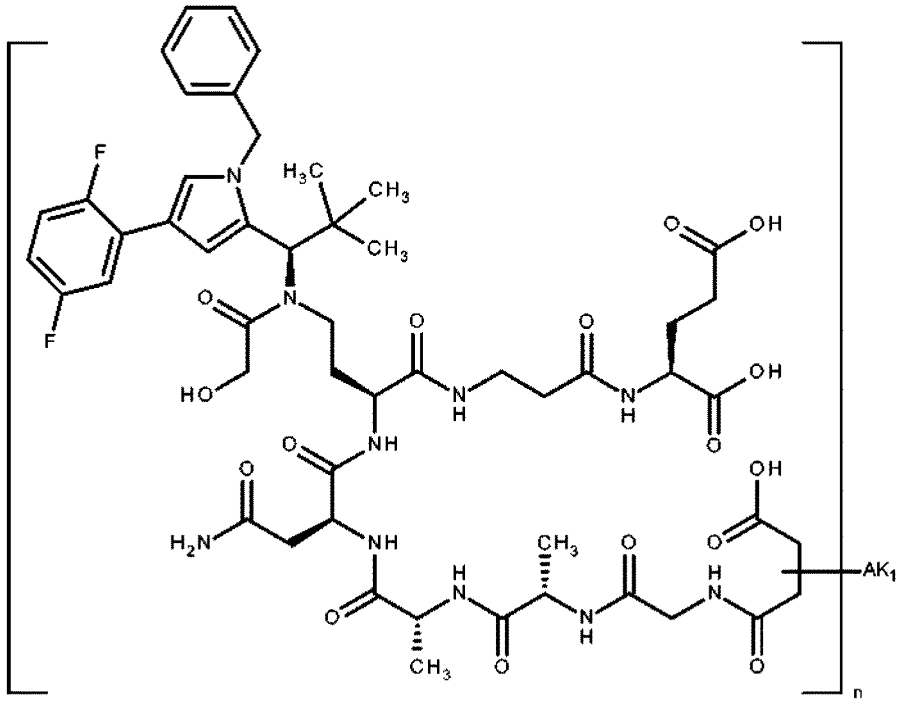

,

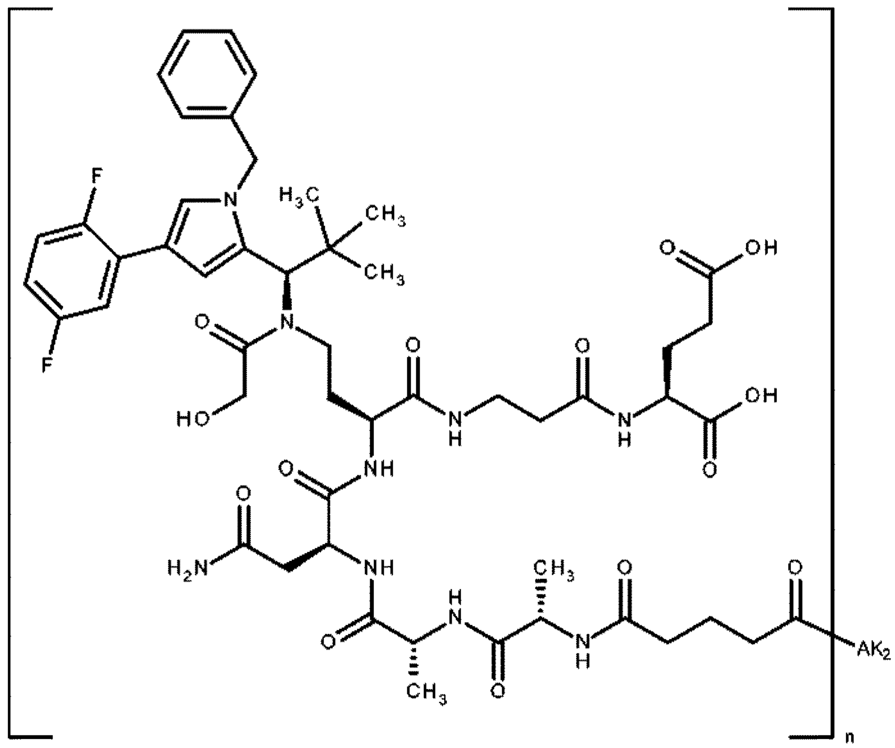

,

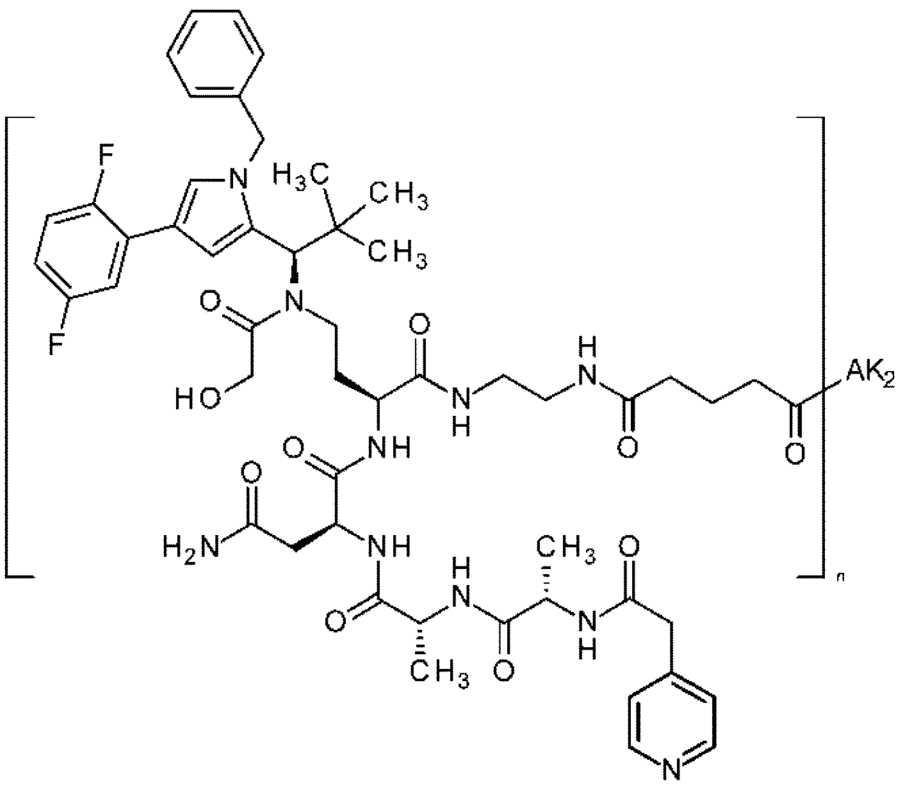

,

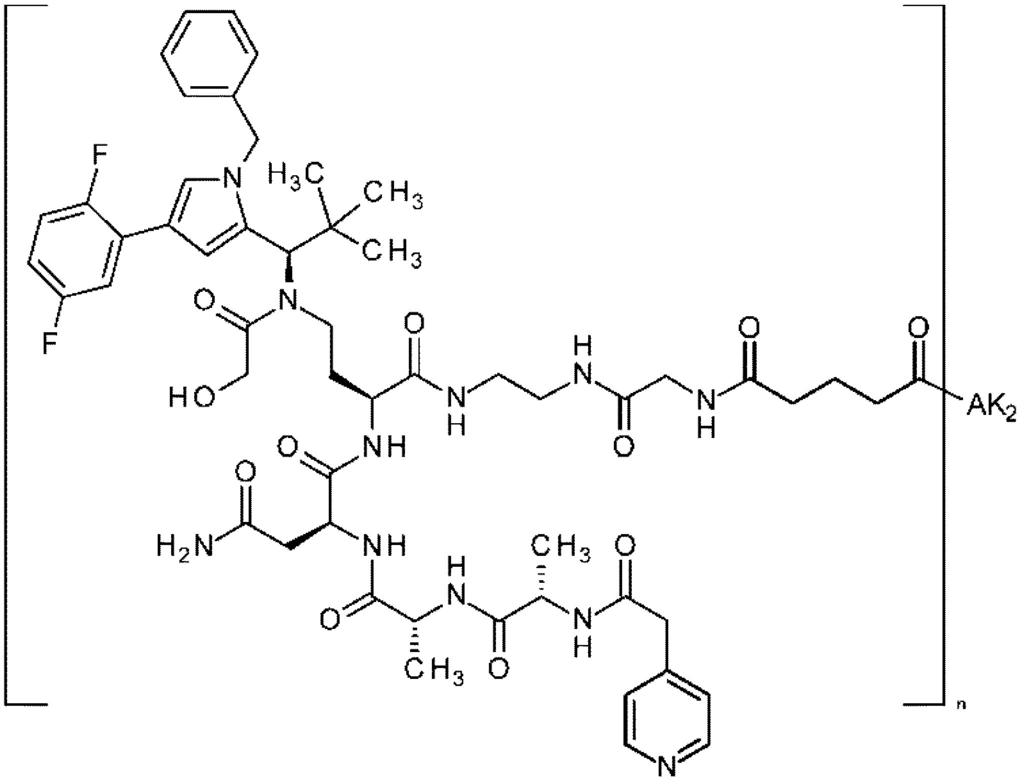

,

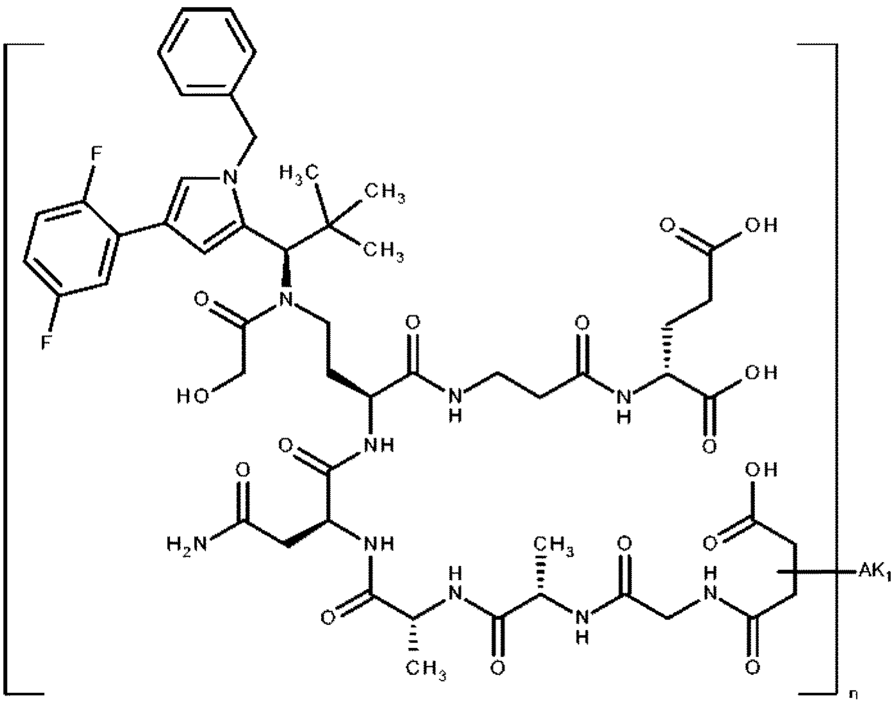

,

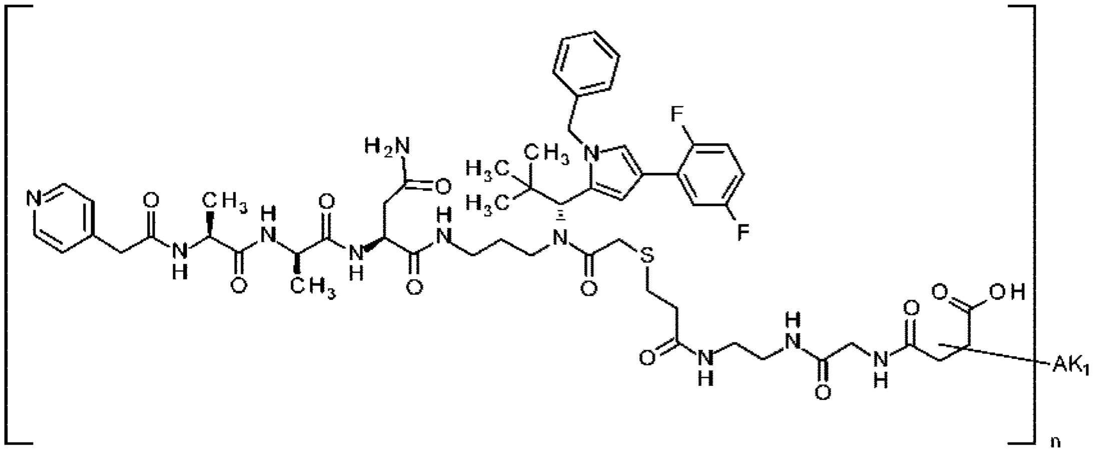

,

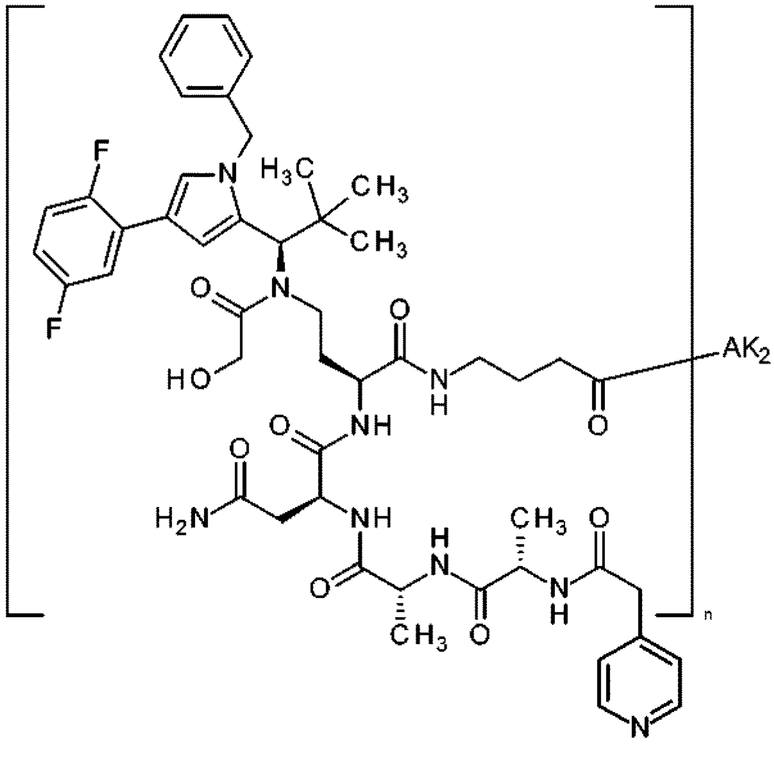

,

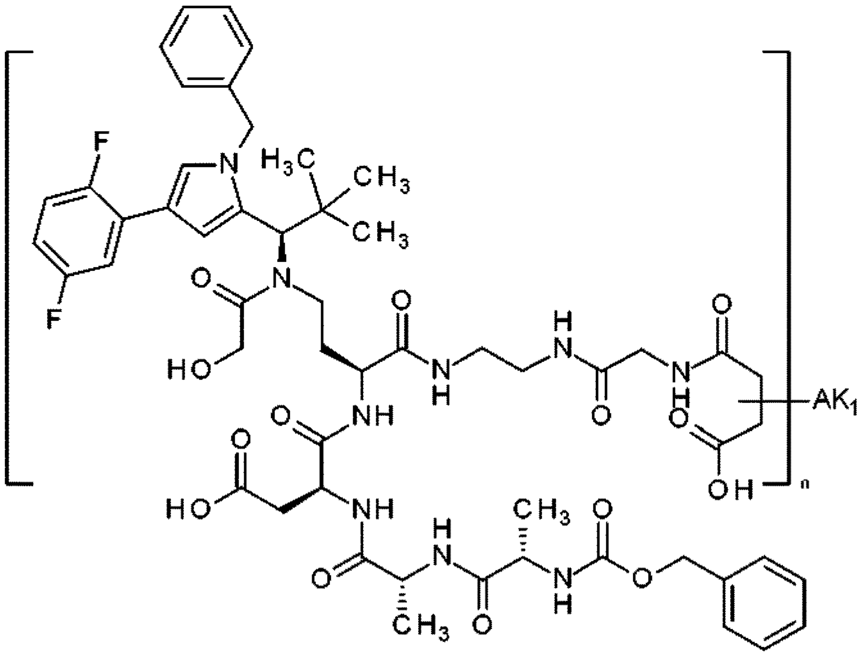

,

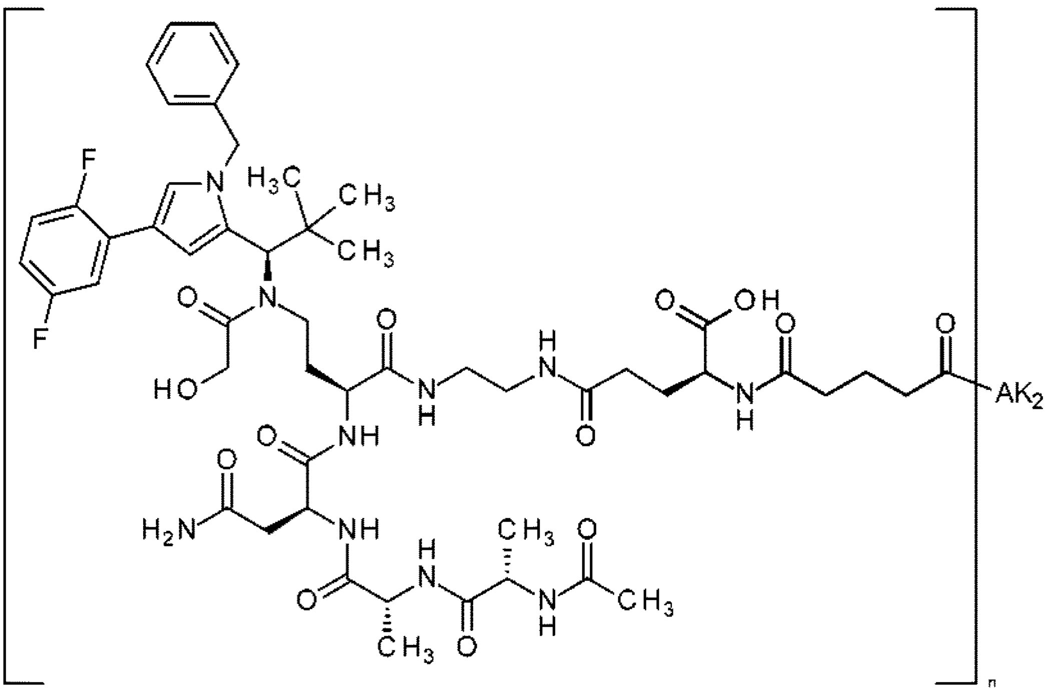

,

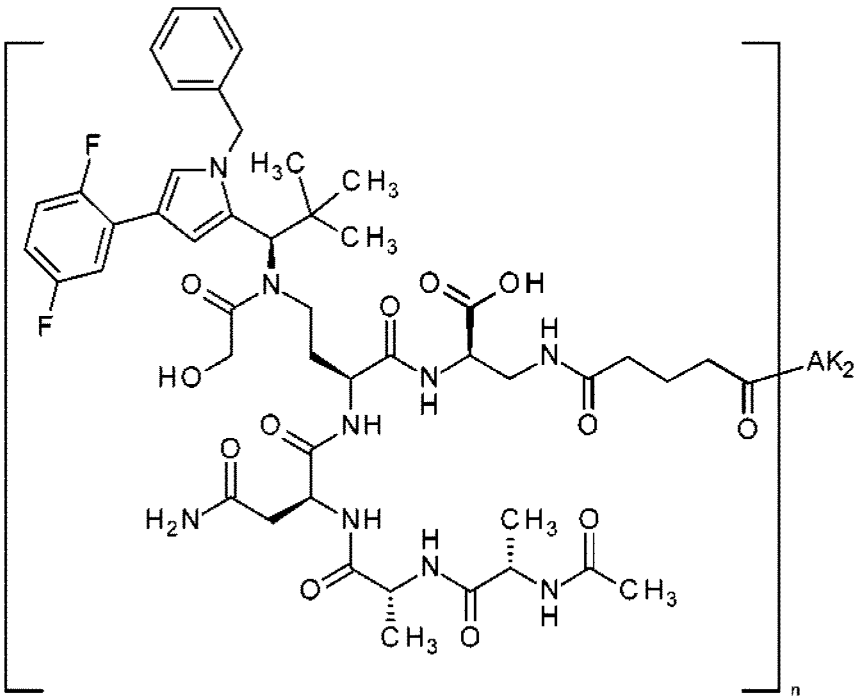

,

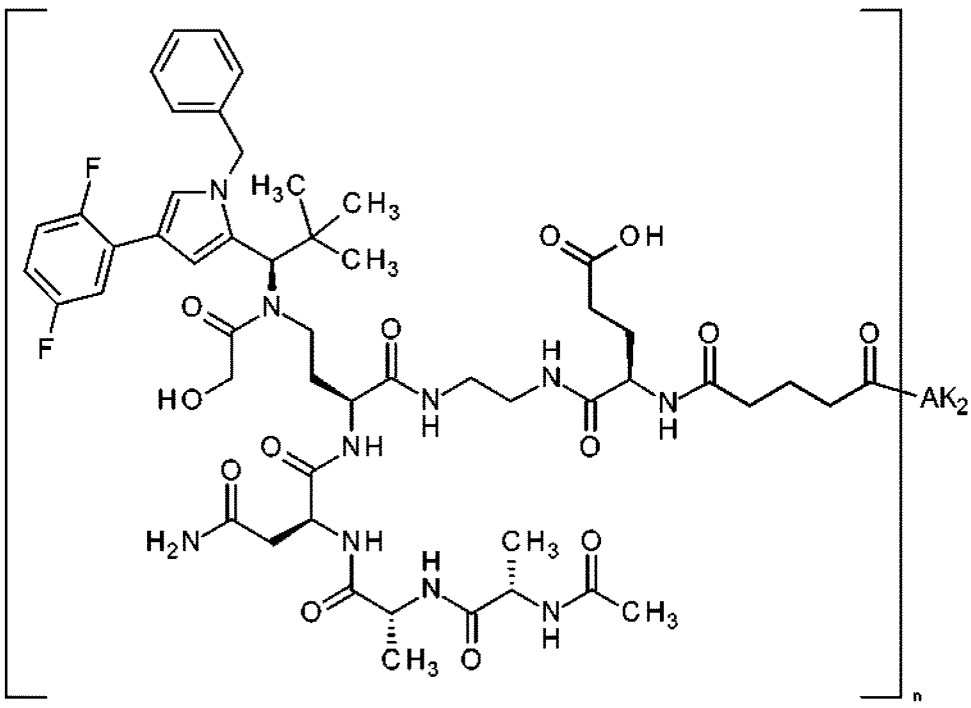

,

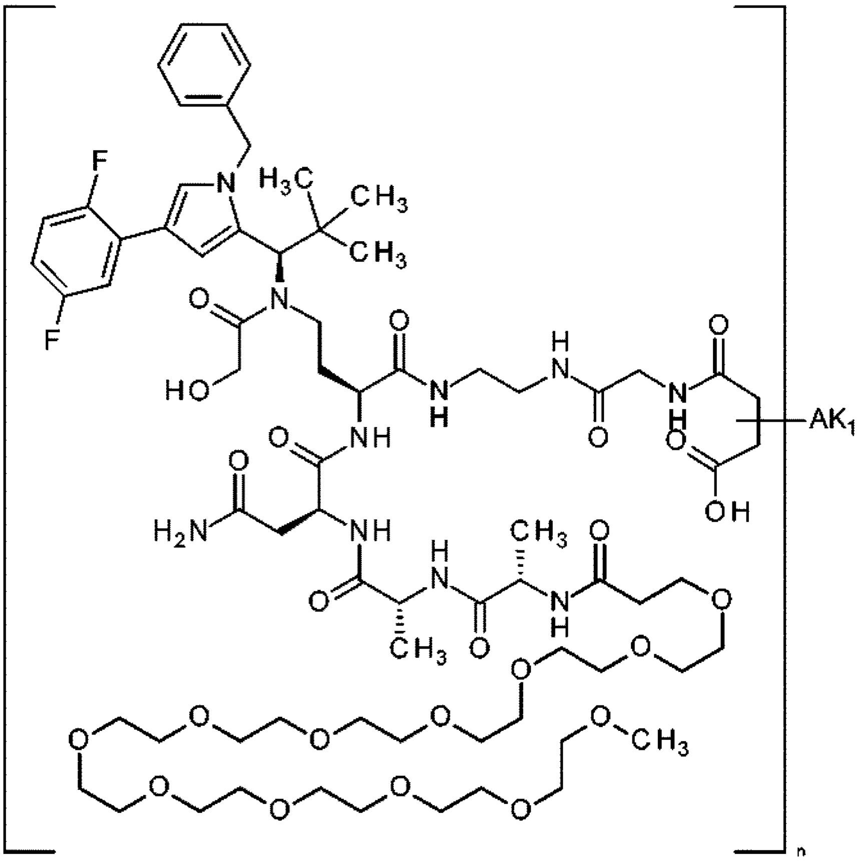

,

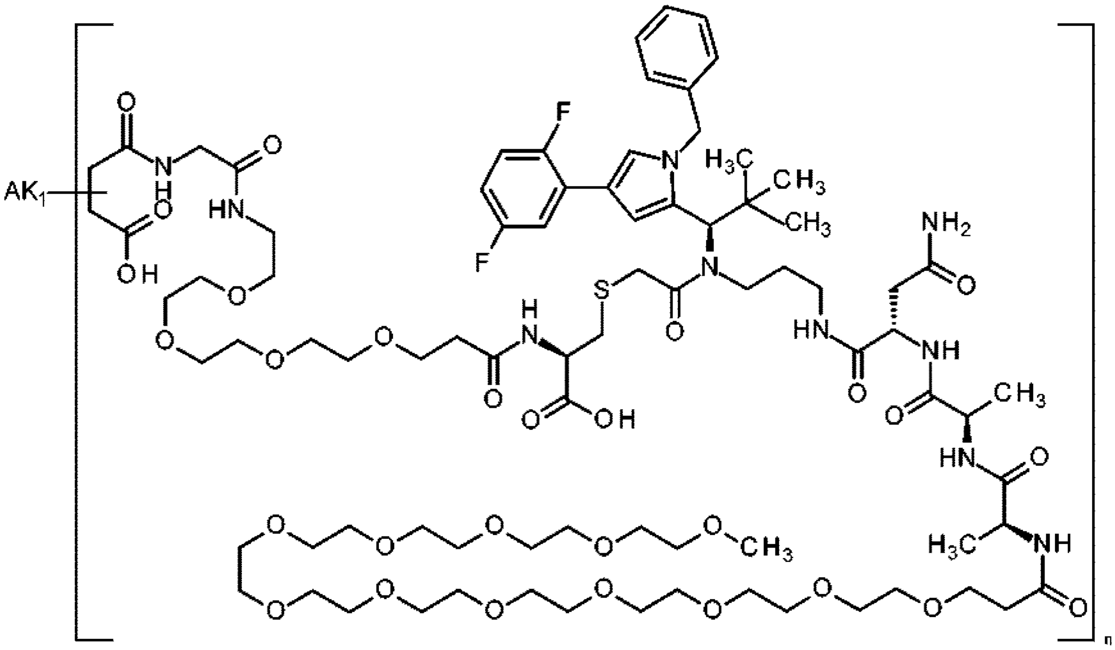

,

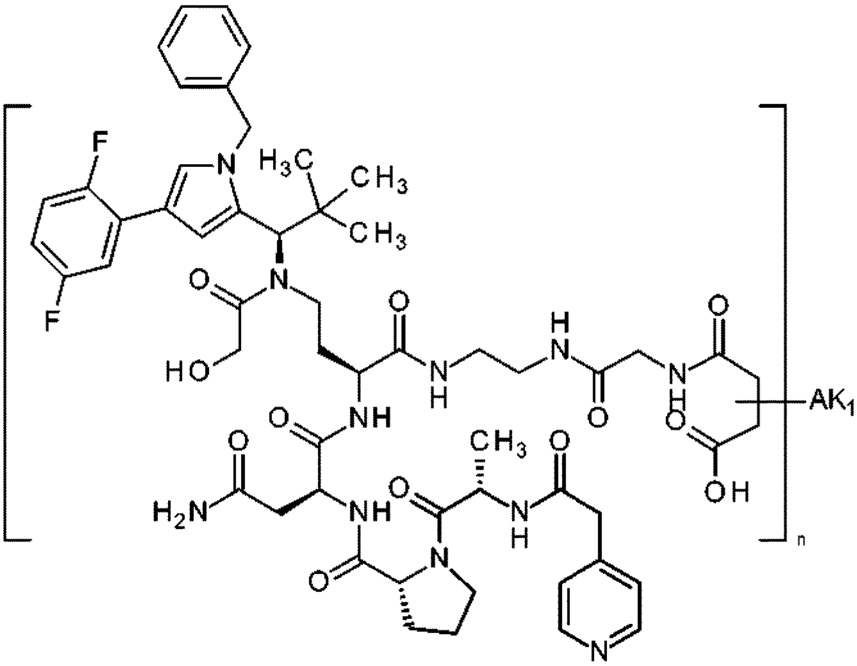

,

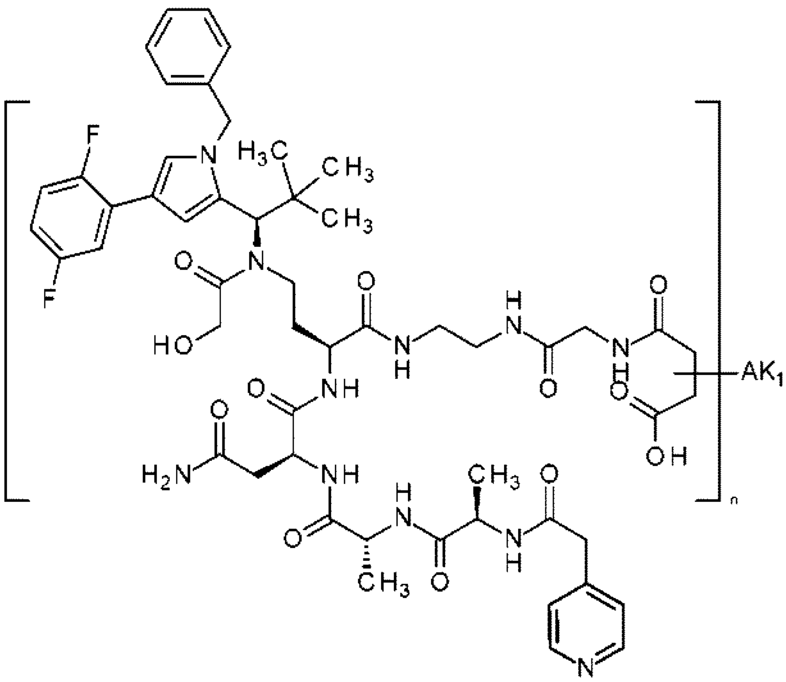

,

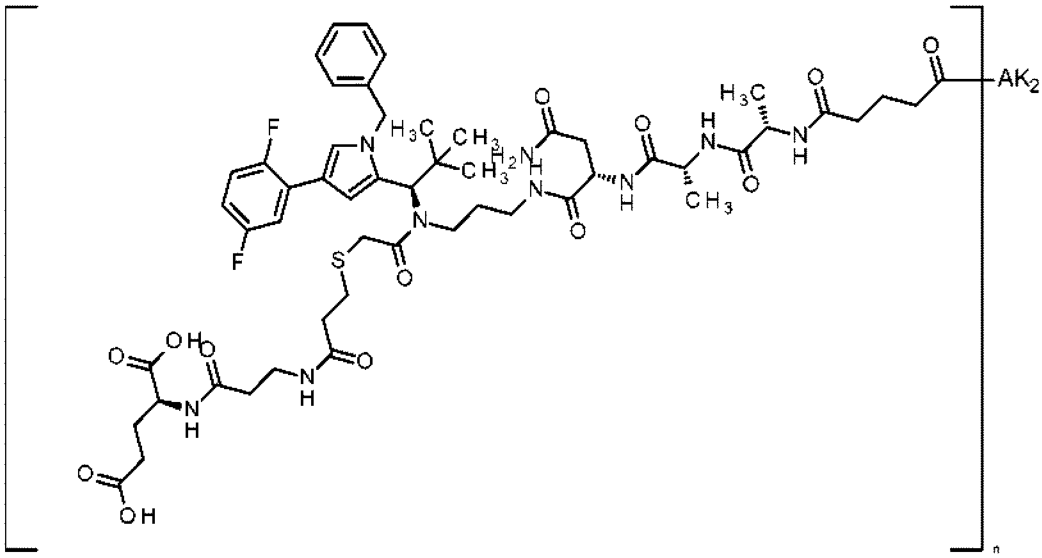

,

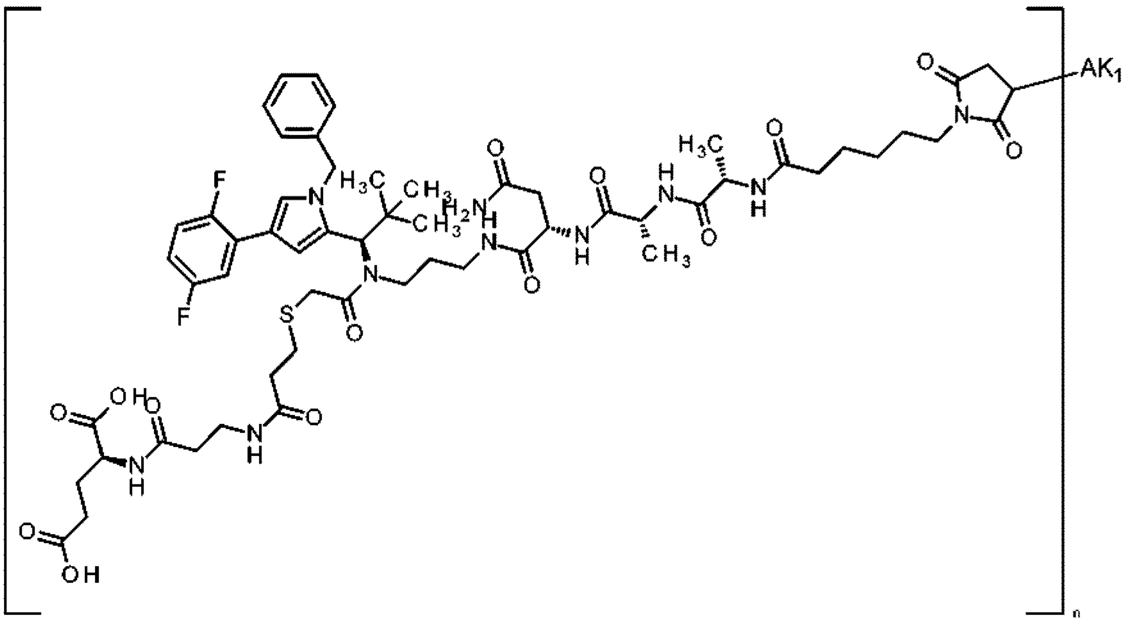

,

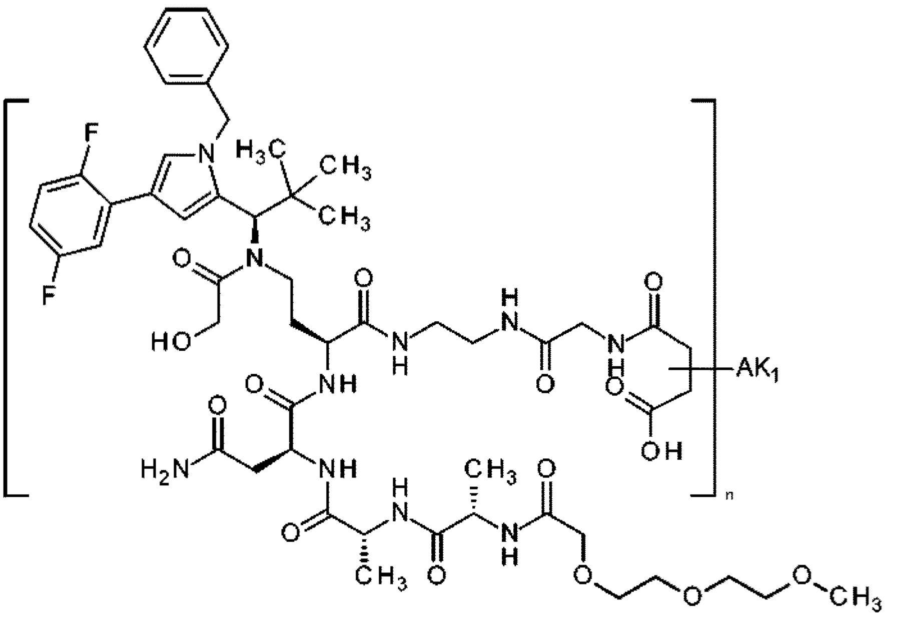

,

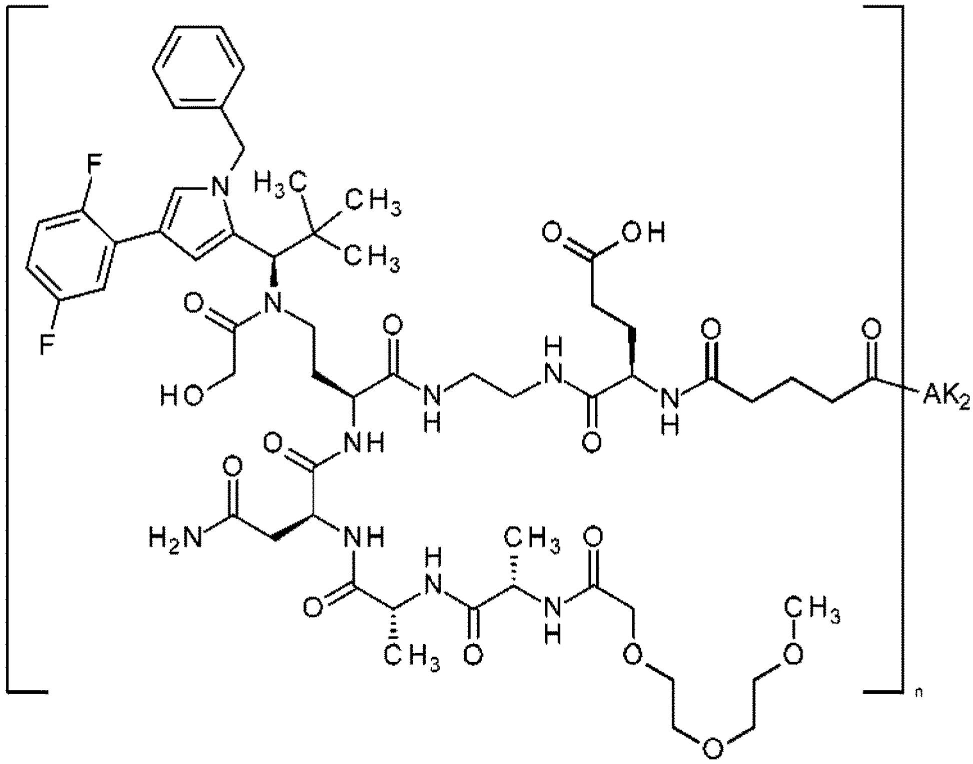

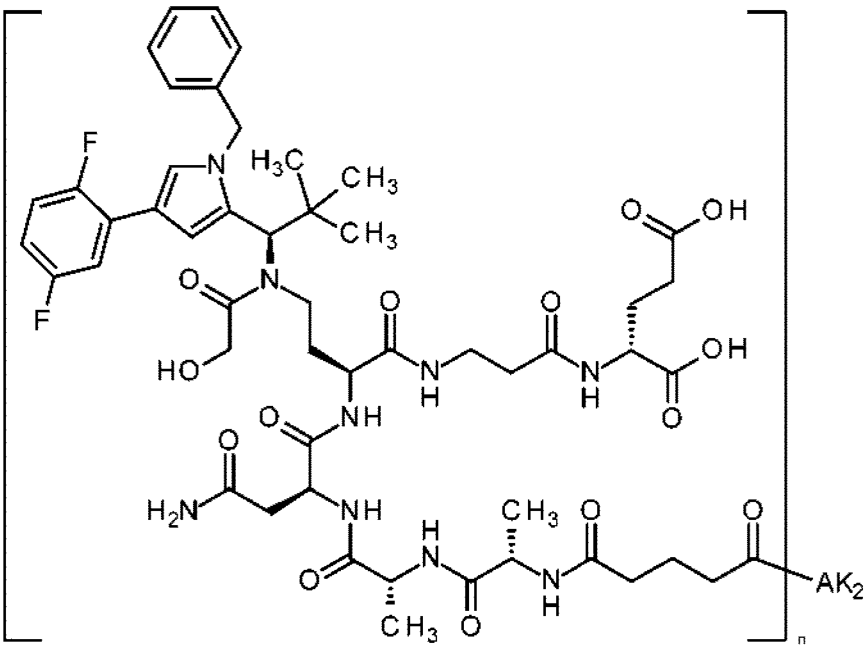

,

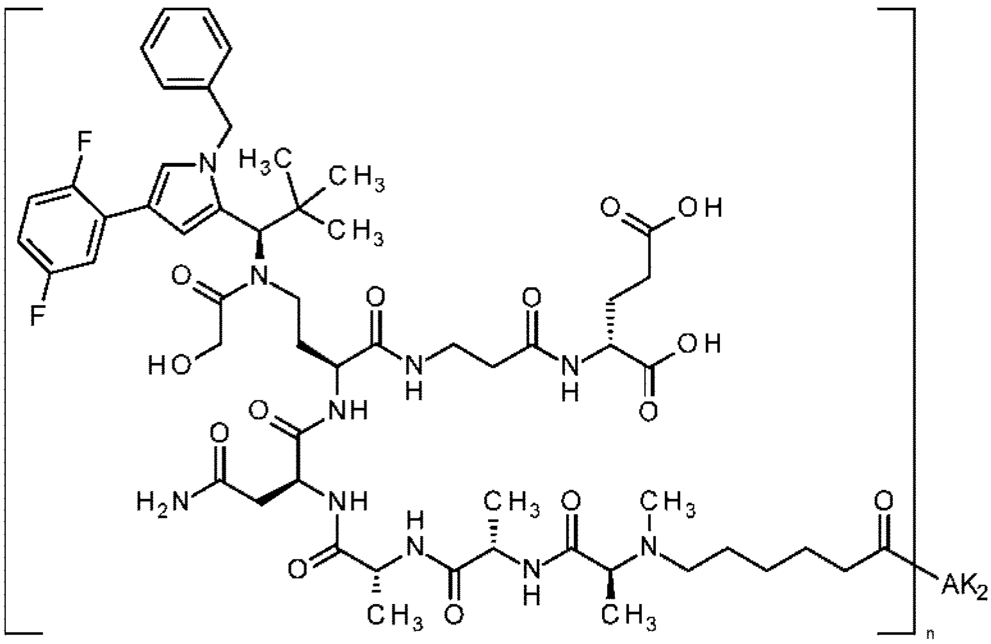

,

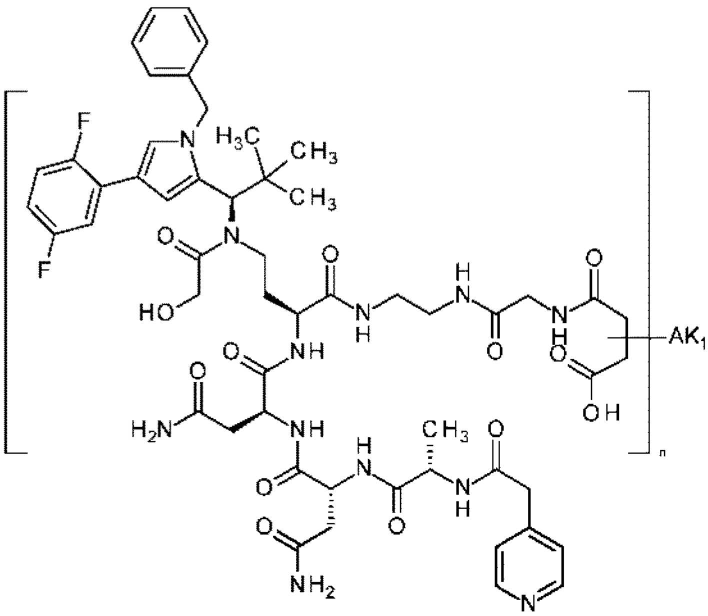

,

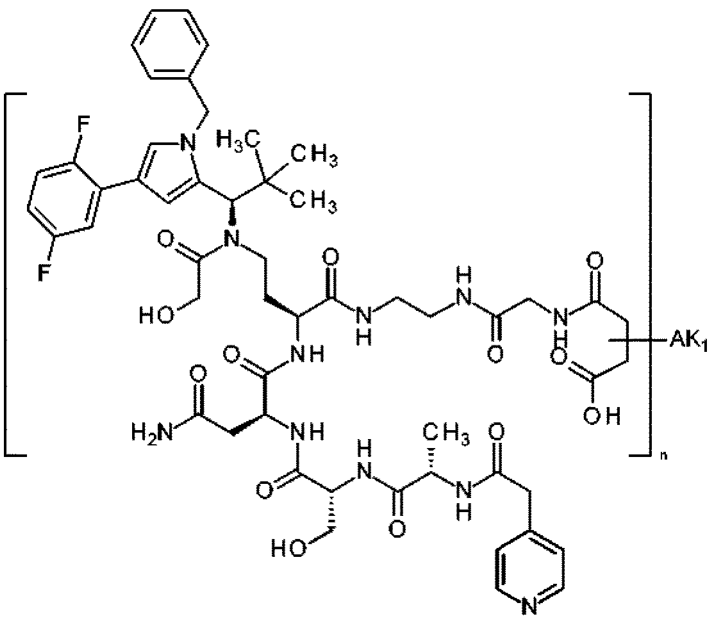

,

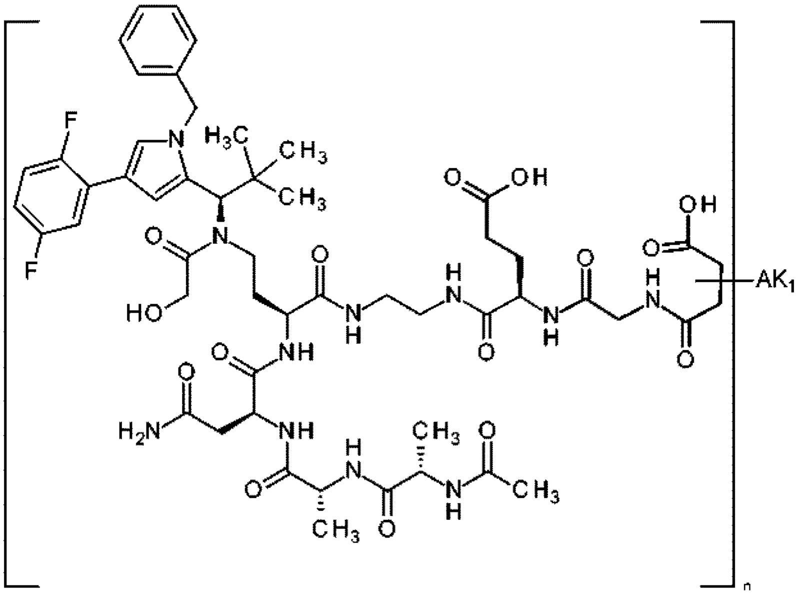

,

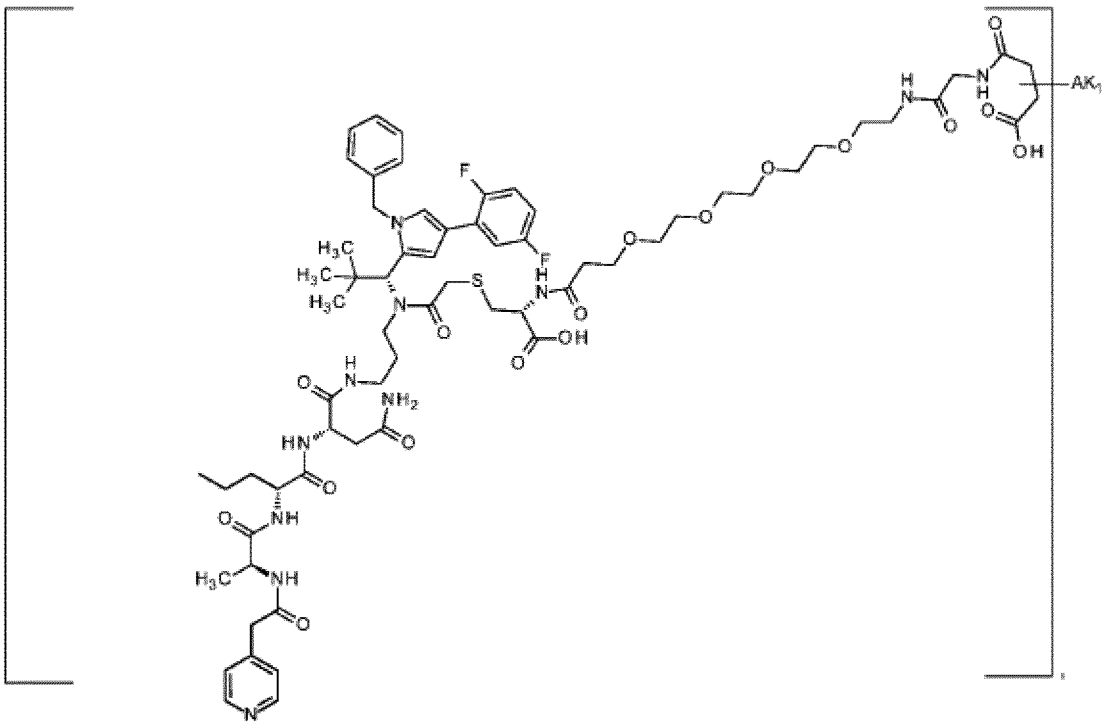

.

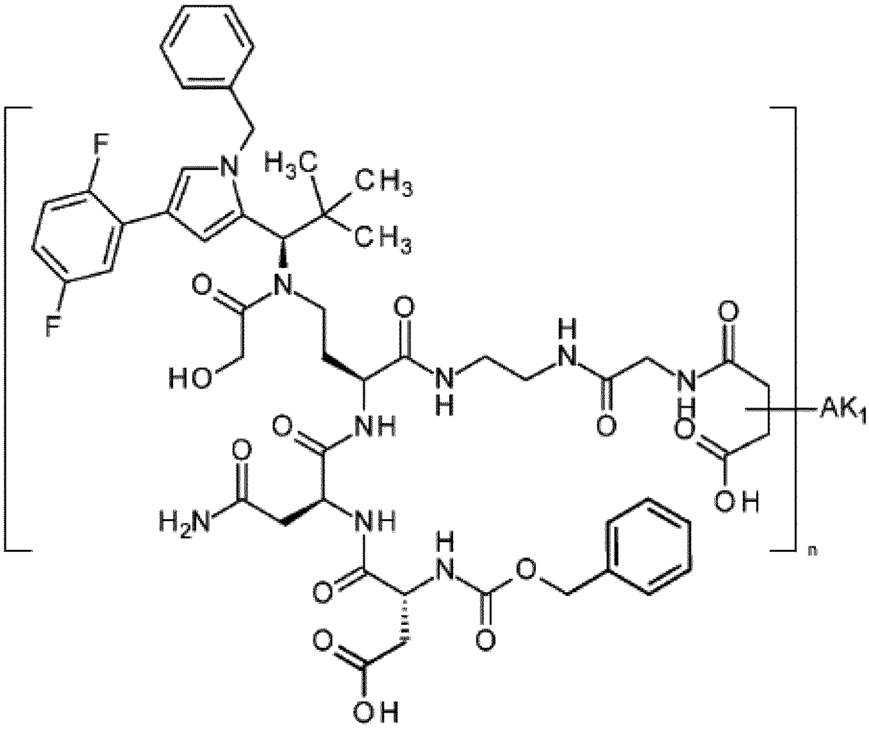

,

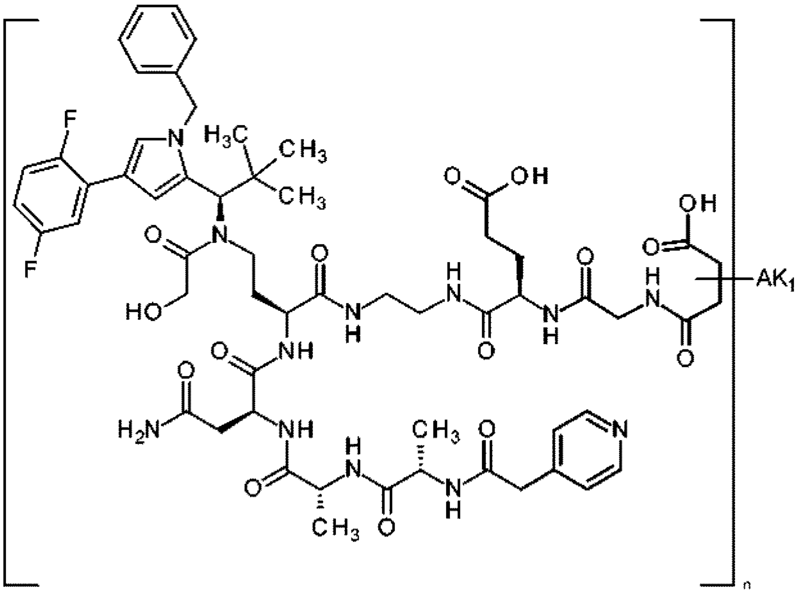

,

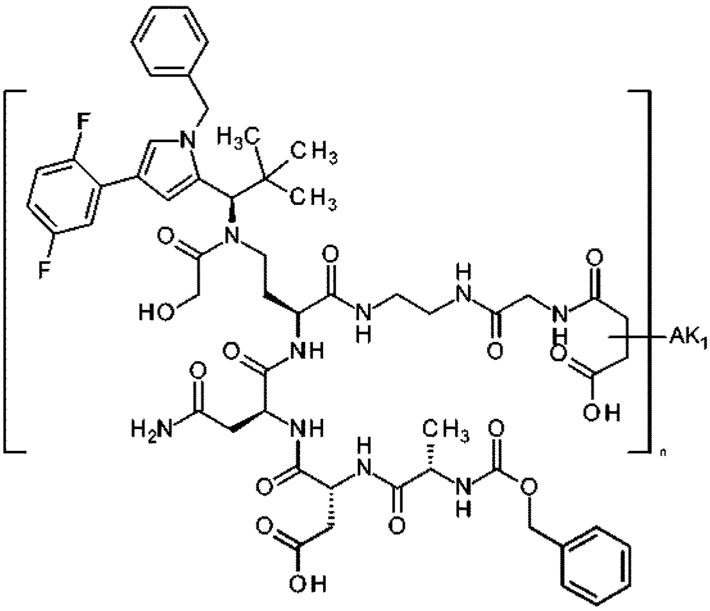

,

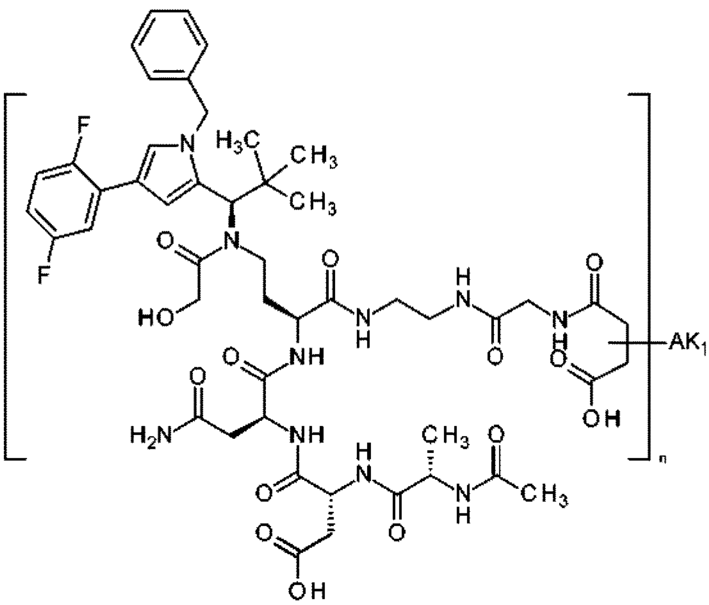

,

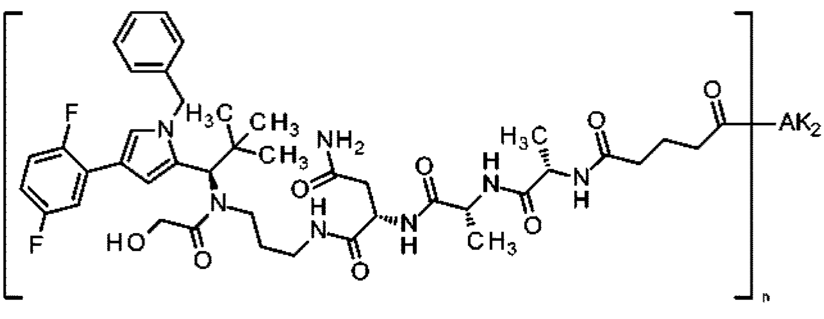

,

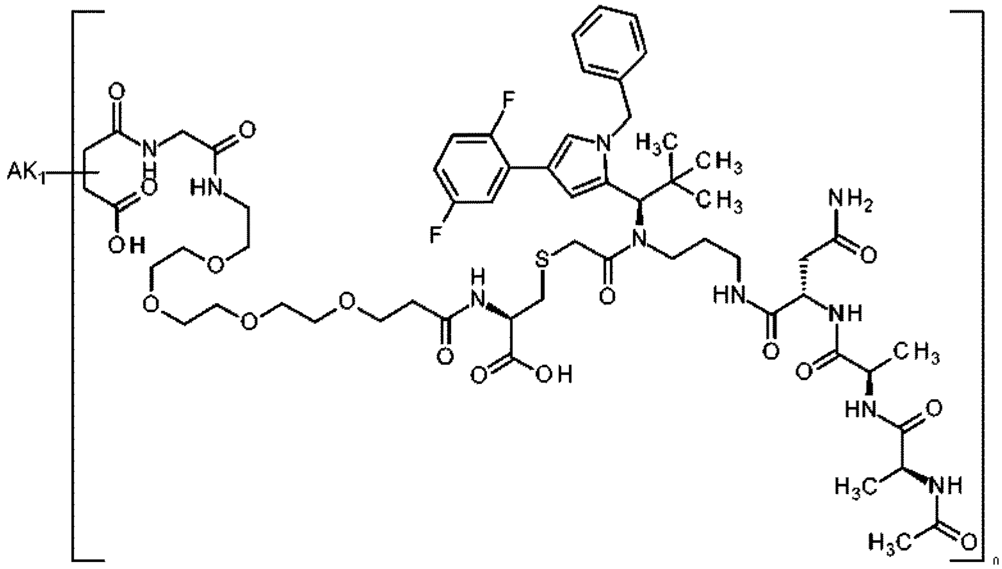

,

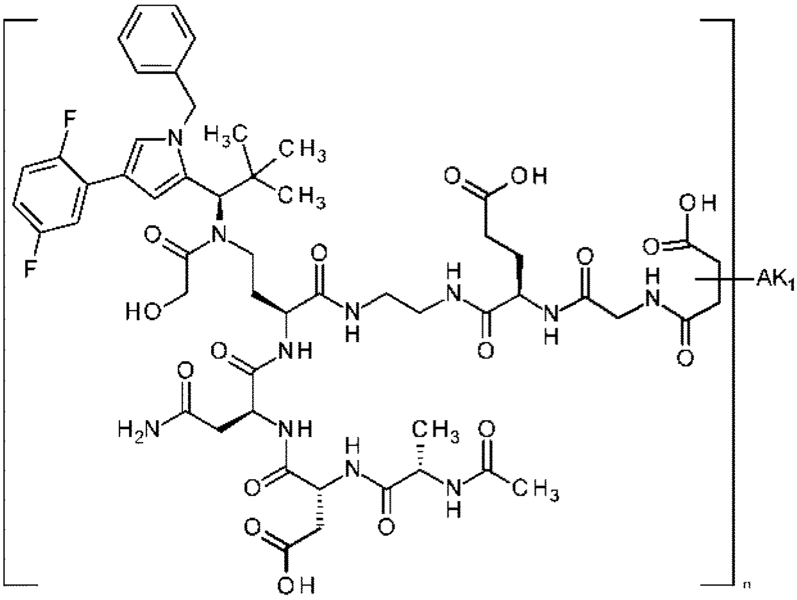

,

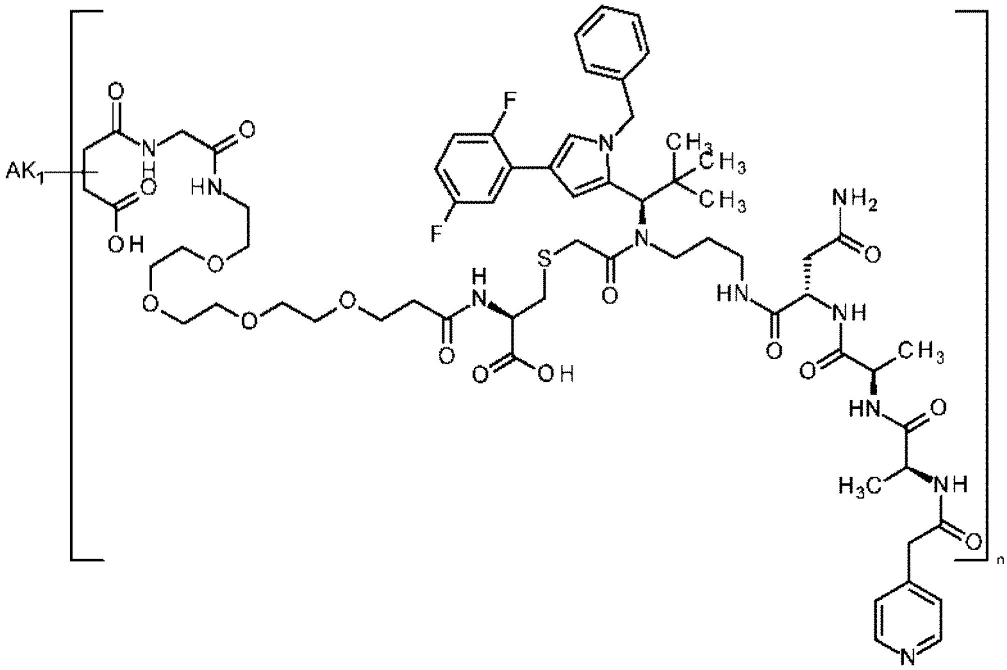

,

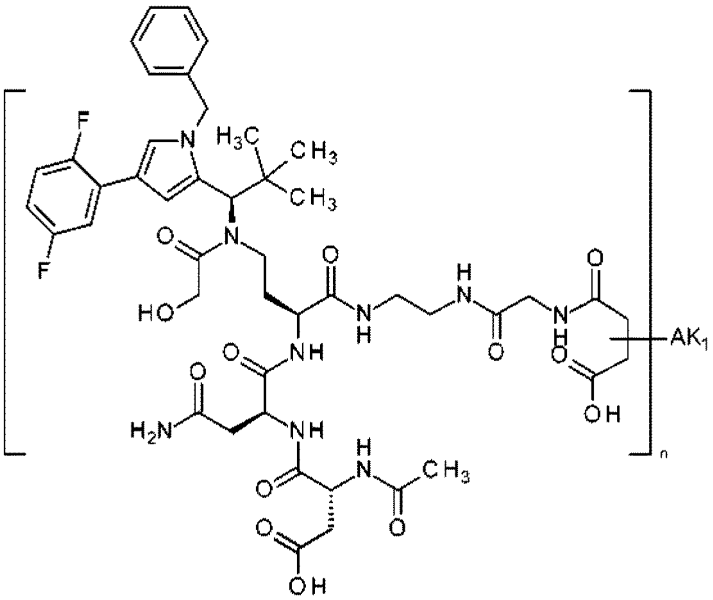

,

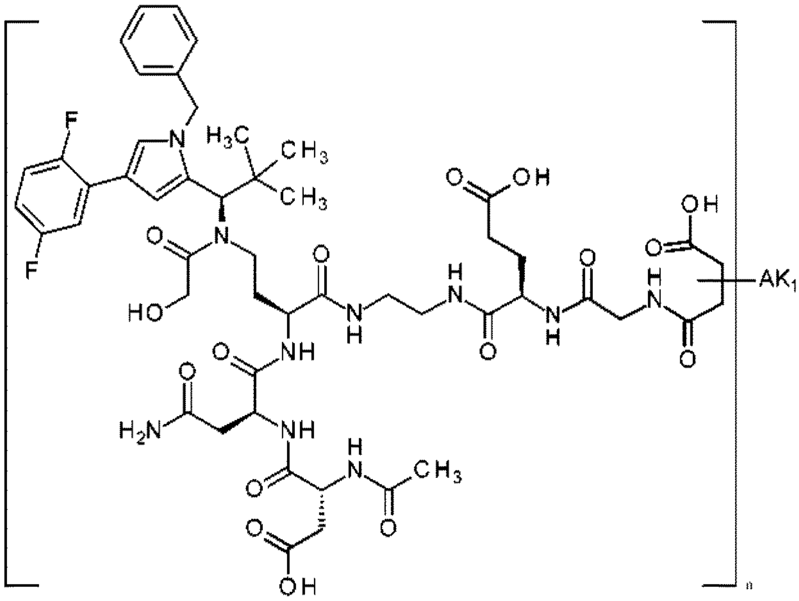

,

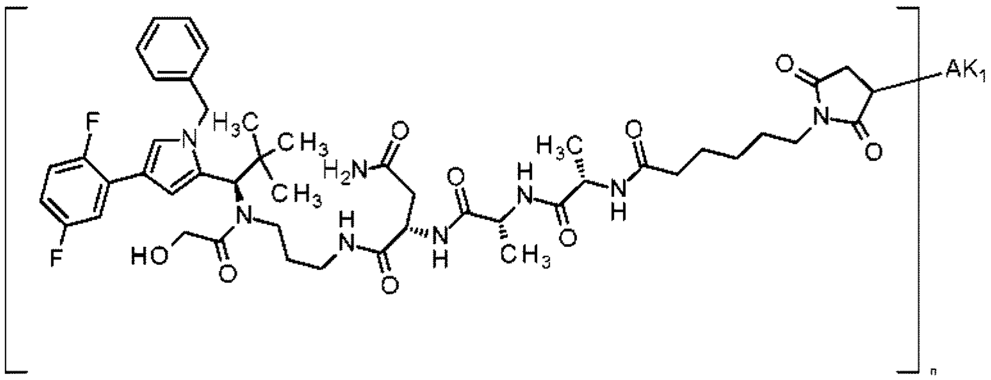

,

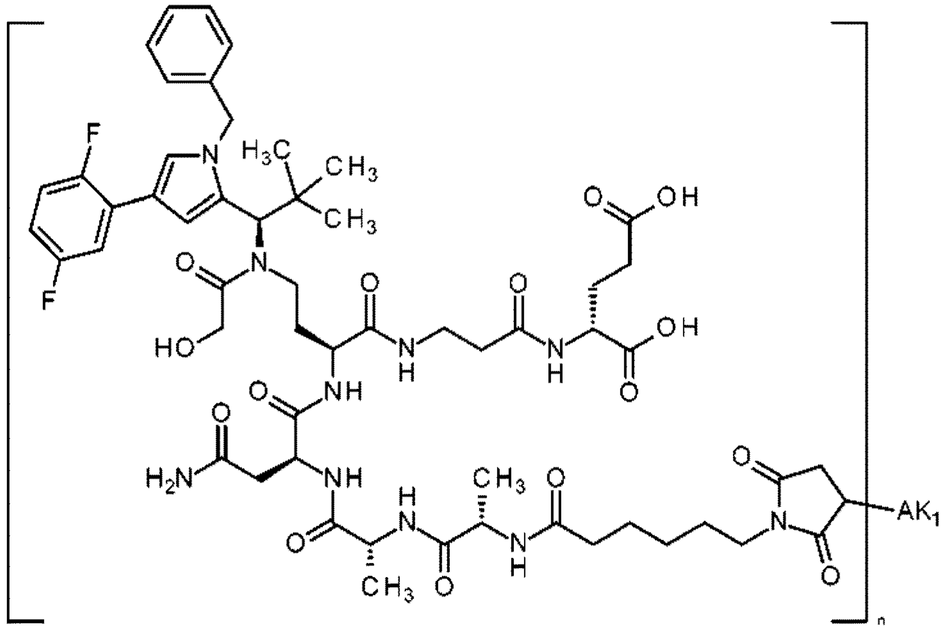

,

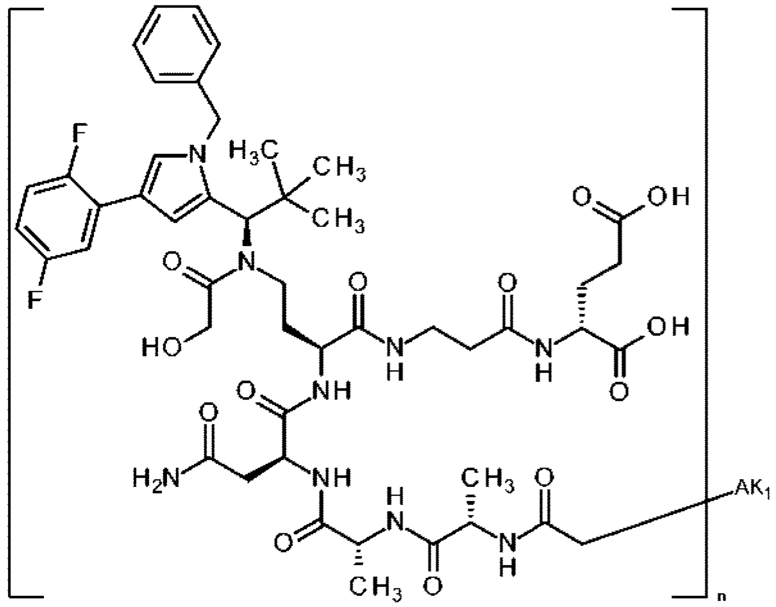

,

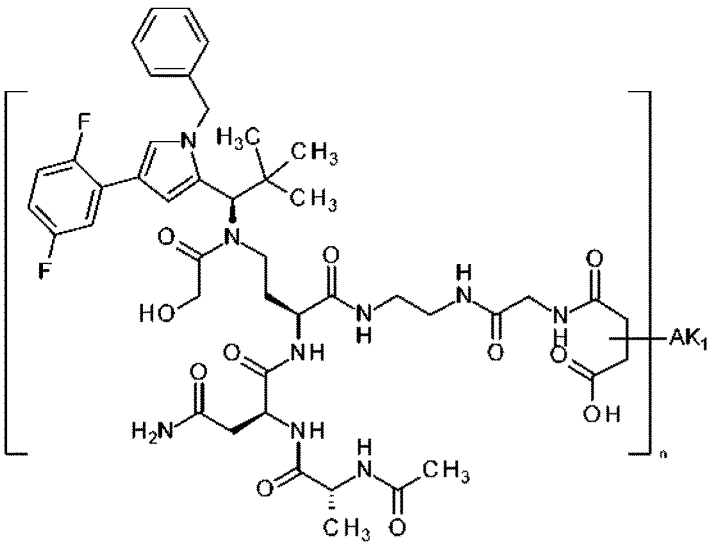

,

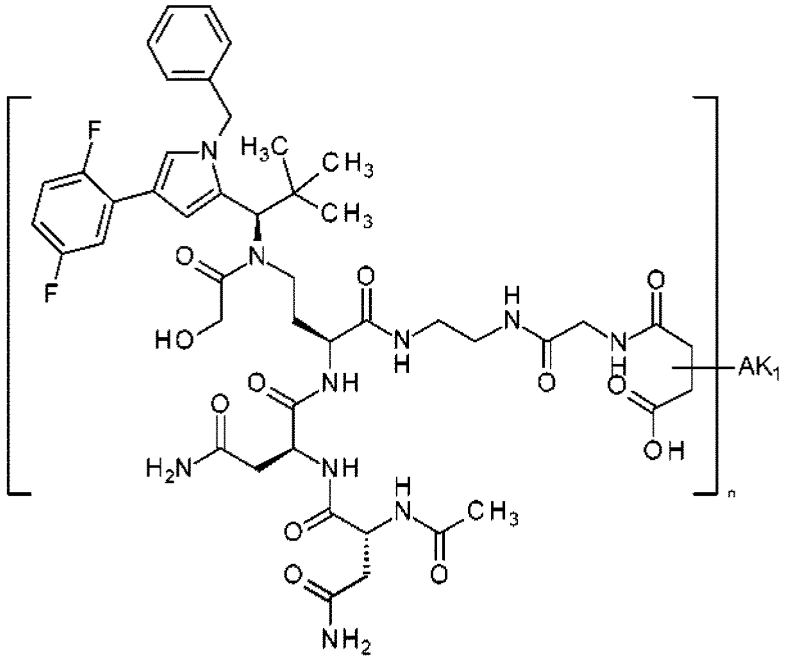

,

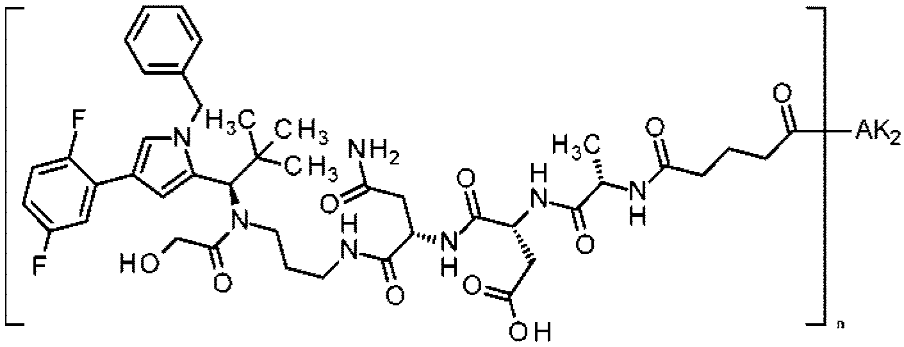

,

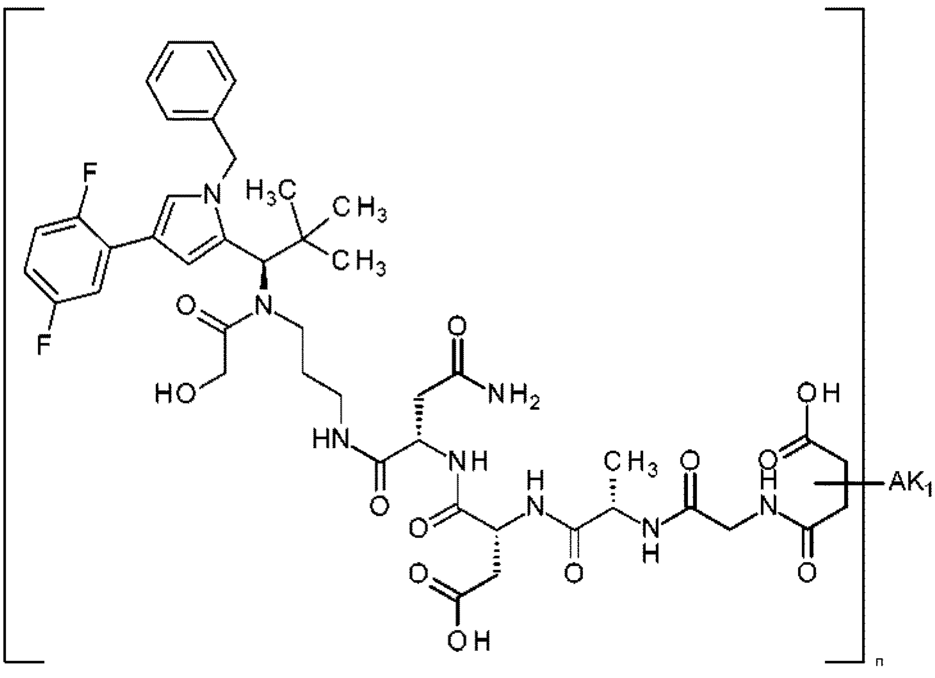

,

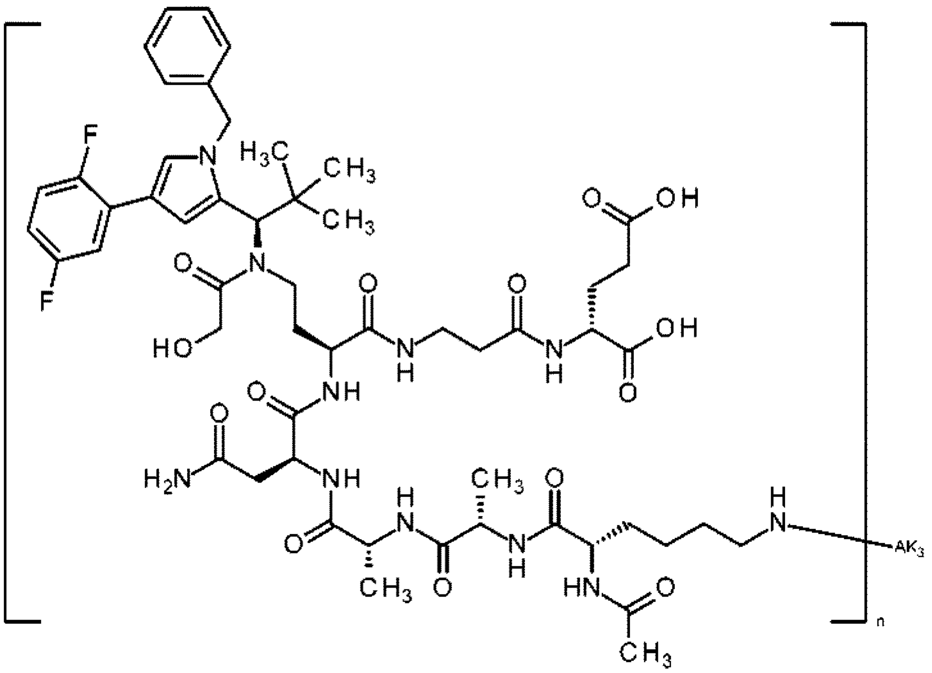

,

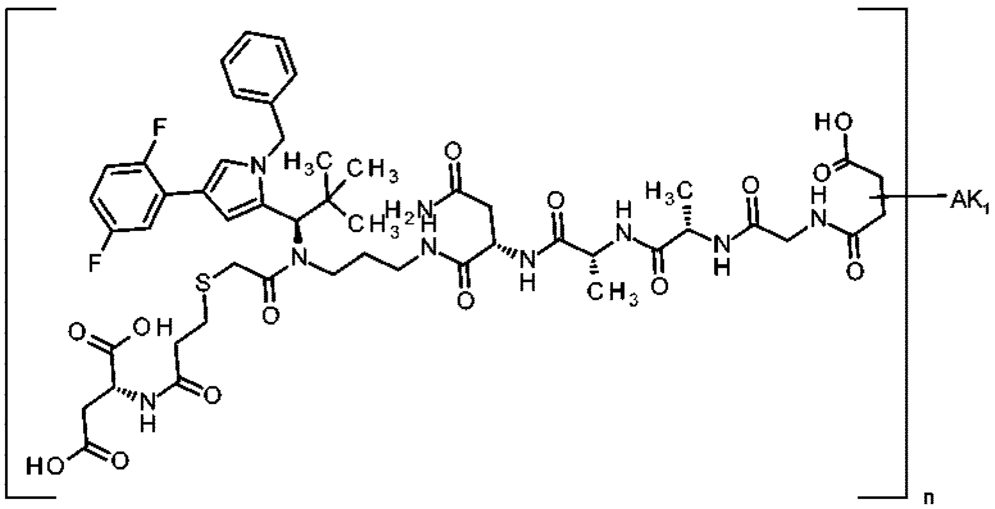

,

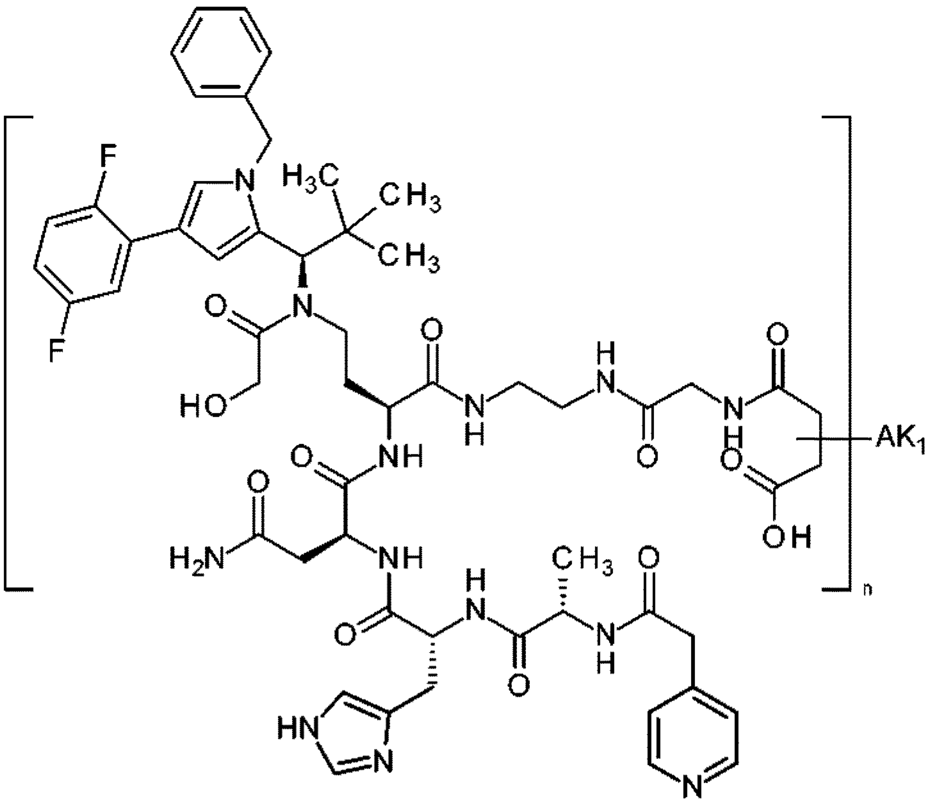

,

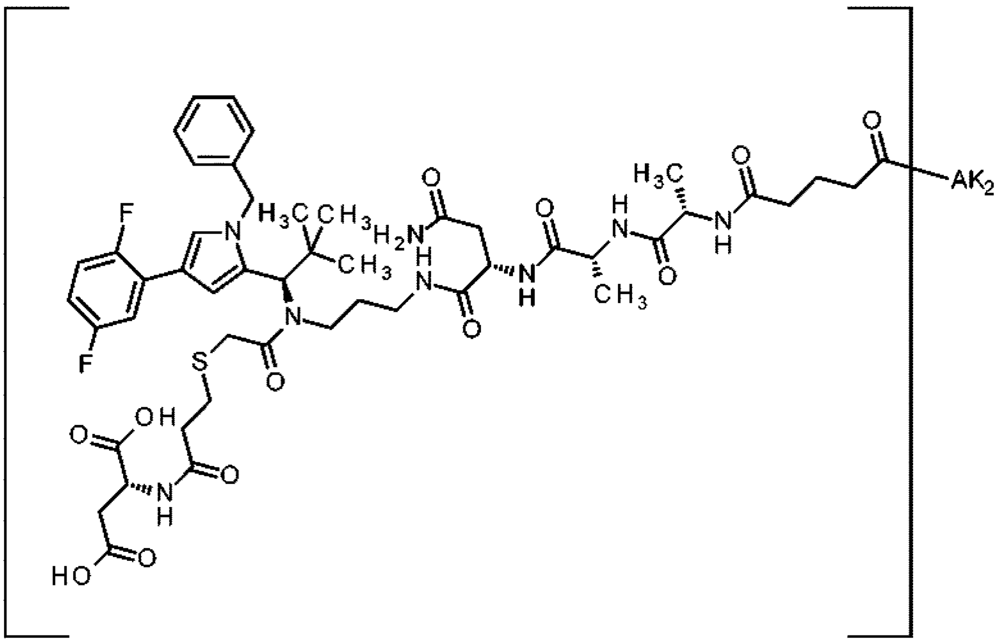

and

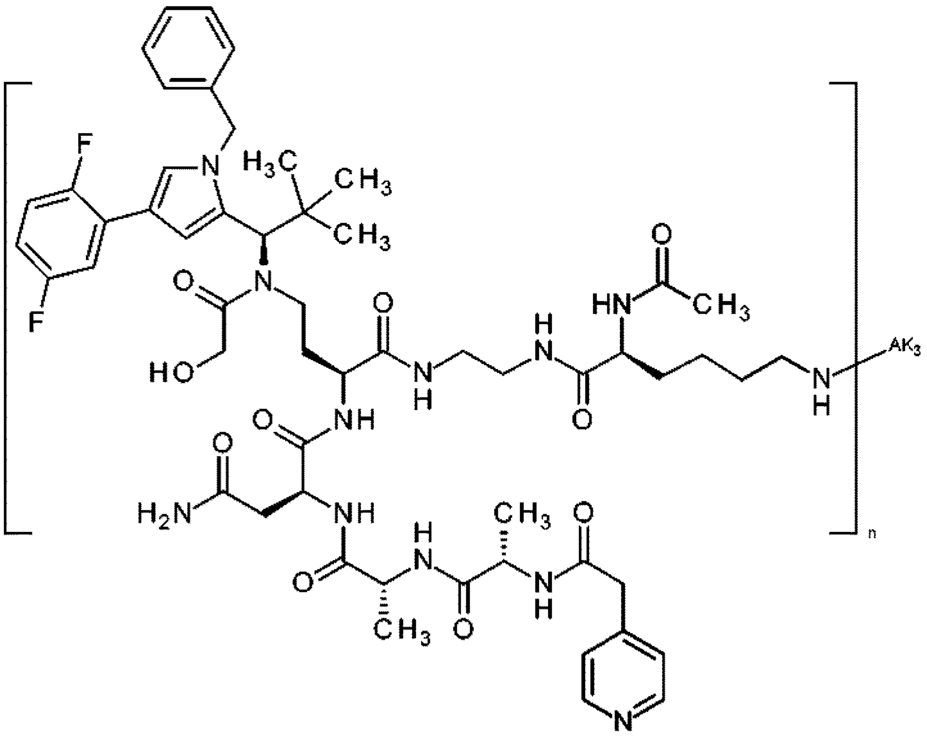

30. The compound or conjugate in accordance with one or more of the foregoing claims, wherein the antibody or the antigen-binding antibody fragment binds to an extracellular target cancer molecule.

31. The compound or conjugate in accordance with one or more of the foregoing claims, wherein, after binding to its extracellular target molecule on the target cell, the antibody or the antigen-binding fragment is internalised through the binding of the target cell.

32. The compound or conjugate in accordance with one or more of the foregoing claims, wherein the antibody is an anti-HER2 antibody, an anti-EGFR antibody, an anti-B7H3 antibody, an anti-TWEAKR antibody or an antigen-binding antibody fragment.

33. The compound or conjugate in accordance with Claim 32, wherein the anti-TWEAKR antibody is selected from the group consisting of TPP-7006, TPP-7007, TPP-10336 and TPp10337, the anti-B7H3 antibody is selected from the group consisting of TPP-8382 and TPP-8567, the anti-EGFR antibody is cetuximab (TPp-981) and the anti-HER2 antibody is selected from the group consisting of trastuzumab and TPP-1015.

34. The compound or conjugate in accordance with one or more the foregoing claims, wherein the antibody (AK, AB, AK1, AK2, AK3)

(i) stands for an anti-EGFR antibody comprising a variable region of the heavy chain (VH), comprising the variable CDR1 sequence of the heavy chain (H-CDR1), as represented by SEQ ID NO. 2, the variable CDR2 sequence of the heavy chain (H-CDR2), as represented by SEQ ID NO. 3 and the variable CDR3 sequence of the heavy chain (H-CDR3), as represented by SEQ ID NO. 4, as well as a variable region of the light chain (VL), comprising the variable CDR1 sequence of the light chain (L-CDR1), as represented by SEQ ID NO. 6, the variable CDR2 sequence of the light chain (L-CDR2), as represented by SEQ ID NO. 7 and the variable CDR3 sequence of the light chain (L-CDR3), as represented by SEQ ID NO. 8.
(ii) for an anti-HER2 antibody comprising a variable region of the heavy chain (VH), comprising the variable CDR1 sequence of the heavy chain (H-CDR1), as represented by SEQ ID NO. 12, the variable CDR2 sequence of the heavy chain (H-CDR2), as represented by SEQ ID NO. 13 and the variable CDR3 sequence of the heavy chain (H-CDR3), as represented by SEQ ID NO. 14, as well as a variable region of the light chain (VL), comprising the variable CDR1 sequence of the light chain (L-CDR1), as represented by SEQ ID NO. 16, the variable CDR2 sequence of the light chain (L-CDR2), as represented by SEQ ID NO. 17 and the variable CDR3 sequence of

the light chain (L-CDR3), as represented by SEQ ID NO. 18,

(iii) for an anti-TWEAKR antibody comprising a variable region of the heavy chain (VH), comprising the variable CDR1 sequence of the heavy chain (H-CDR1), as represented by SEQ ID NO. 22, the variable CDR2 sequence of the heavy chain (H-CDR2), as represented by SEQ ID NO. 23 and the variable CDR3 sequence of the heavy chain (H-CDR3), as represented by SEQ ID NO. 24, as well as a variable region of the light chain (VL), comprising the variable CDR1 sequence of the light chain (L-CDR1), as represented by SEQ ID NO. 26, the variable CDR2 sequence of the light chain (L-CDR2), as represented by SEQ ID NO. 27 and the variable CDR3 sequence of the light chain (L-CDR3), as represented by SEQ ID NO. 28,

(iv) for an anti-TWEAKR antibody comprising a variable region of the heavy chain (VH), comprising the variable CDR1 sequence of the heavy chain (H-CDR1), as represented by SEQ ID NO. 32, the variable CDR2 sequence of the heavy chain (H-CDR2), as represented by SEQ ID NO. 33 and the variable CDR3 sequence of the heavy chain (H-CDR3), as represented by SEQ ID NO. 34, as well as a variable region of the light chain (VL), comprising the variable CDR1 sequence of the light chain (L-CDR1), as represented by SEQ ID NO. 36, the variable CDR2 sequence of the light chain (L-CDR2), as represented by SEQ ID NO. 37 and the variable CDR3 sequence of the light chain (L-CDR3), as represented by SEQ ID NO. 38,

(v) for an anti-TWEAKR antibody comprising a variable region of the heavy chain (VH), comprising the variable CDR1 sequence of the heavy chain (H-CDR1), as represented by SEQ ID NO. 42, the variable CDR2 sequence of the heavy chain (H-CDR2), as represented by SEQ ID NO. 43 and the variable CDR3 sequence of the heavy chain (H-CDR3), as represented by SEQ ID NO. 44, as well as a variable region of the light chain (VL), comprising the variable CDR1 sequence of the light chain (L-CDR1), as represented by SEQ ID NO. 46, the variable CDR2 sequence of the light chain, (L-CDR2), as represented by SEQ ID NO. 47 and the variable CDR3 sequence of the light chain (L-CDR3), as represented by SEQ ID NO. 48,

(vi) for an anti-TWEAKR antibody comprising a variable region of the heavy chain (VH), comprising the variable CDR1 sequence of the heavy chain (H-CDR1), as represented by SEQ ID NO. 52, the variable CDR2 sequence of the heavy chain (H-CDR2), as represented by SEQ ID NO. 53 and the variable CDR3 sequence of the heavy chain (H-CDR3), as represented by SEQ ID NO. 54, as well as a variable region of the light chain (VL), comprising the variable CDR1 sequence of the light chain (L-CDR1), as represented by SEQ ID NO. 56, the variable CDR2 sequence of the light chain (L-CDR2), as represented by SEQ ID NO. 57 and the variable CDR3 sequence of the light chain (L-CDR3), as represented by SEQ ID NO. 58,

(vii) for an anti-TWEAKR antibody comprising a variable region of the heavy chain (VH), comprising the variable CDR1 sequence of the heavy chain (H-CDR1), as represented by SEQ ID NO. 62, the variable CDR2 sequence of the heavy chain (H-CDR2), as represented by SEQ ID NO. 63 and the variable CDR3 sequence of the heavy chain (H-CDR3), as represented by SEQ ID NO. 64, as well as a variable region of the light chain (VL), comprising the variable CDR1 sequence of the light chain (L-CDR1), as represented by SEQ ID NO. 66, the variable CDR2 sequence of the light chain (L-CDR2), as represented by SEQ ID NO. 67 and the variable CDR3 sequence of the light chain (L-CDR3), as represented by SEQ ID NO. 68,

(viii) for an anti-HER2 antibody comprising a variable region of the heavy chain (VH), comprising the variable CDR1 sequence of the heavy chain (H-CDR1), as represented by SEQ ID NO. 72, the variable CDR2 sequence of the heavy chain (H-CDR2), as represented by SEQ ID NO. 73 and the variable CDR3 sequence of the heavy chain (H-CDR3), as represented by SEQ ID NO. 74, as well as a variable region of the light chain (VL), comprising the variable CDR1 sequence of the light chain (L-CDR1), as represented by SEQ ID NO. 76, the variable CDR2 sequence of the light chain (L-CDR2), as represented by SEQ ID NO. 77 and the variable CDR3 sequence of the light chain (L-CDR3), as represented by SEQ ID NO. 78,

(ix) for an anti-HER2 antibody comprising a variable region of the heavy chain (VH), comprising the variable CDR1 sequence of the heavy chain (H-CDR1), as represented by SEQ ID NO. 82, the variable CDR2 sequence of the heavy chain (H-CDR2), as represented by SEQ ID NO. 83 and the variable CDR3 sequence of the heavy chain (H-CDR3), as represented by SEQ ID NO. 84, as well as a variable region of the light chain (VL), comprising the variable CDR1 sequence of the light chain (L-CDR1), as represented by SEQ ID NO. 86, the variable CDR2 sequence of the light chain (L-CDR2), as represented by SEQ ID NO. 87 and the variable CDR3 sequence of the light chain (L-CDR3), as represented by SEQ ID NO. 88,

(x) for an anti-B7H3 antibody comprising a variable region of the heavy chain (VH), comprising the variable CDR1 sequence of the heavy chain (H-CDR1), as represented by SEQ ID NO. 92, the variable CDR2 sequence of the heavy chain (H-CDR2), as represented by SEQ ID NO. 93 and the variable CDR3 sequence of the heavy chain (H-CDR3), as represented by SEQ ID NO. 94, as well as a variable region of the light chain (VL), comprising the variable CDR1 sequence of the light chain (L-CDR1), as represented by SEQ ID NO. 96, the variable CDR2 sequence of the light chain (L-CDR2), as represented by SEQ ID NO. 97 and the variable CDR3 sequence of the light chain (L-CDR3), as represented by SEQ ID NO. 98,

(xi) for an anti-B7H3 antibody comprising a variable region of the heavy chain (VH), comprising the variable

CDR1 sequence of the heavy chain (H-CDR1), as represented by SEQ ID NO. 102, the variable CDR2 sequence of the heavy chain (H-CDR2), as represented by SEQ ID NO. 103 and the variable CDR3 sequence of the heavy chain (H-CDR3), as represented by SEQ ID NO. 104, as well as a variable region of the light chain (VL), comprising the variable CDR1 sequence of the light chain (L-CDR1), as represented by SEQ ID NO. 106, the variable CDR2 sequence of the light chain (L-CDR2), as represented by SEQ ID NO. 107 and the variable CDR3 sequence of the light chain (L-CDR3), as represented by SEQ ID NO. 108,

(xii) for an anti-TWEAKR antibody comprising a variable region of the heavy chain (VH), comprising the variable CDR1 sequence of the heavy chain (H-CDR1), as represented by SEQ ID NO. 112, the variable CDR2 sequence of the heavy chain (H-CDR2), as represented by SEQ ID NO. 113 and the variable CDR3 sequence of the heavy chain (H-CDR3), as represented by SEQ ID NO. 114, as well as a variable region of the light chain (VL), comprising the variable CDR1 sequence of the light chain (L-CDR1), as represented by SEQ ID NO. 116, the variable CDR2 sequence of the light chain (L-CDR2), as represented by SEQ ID NO. 117 and the variable CDR3 sequence of the light chain (L-CDR3), as represented by SEQ ID NO. 118,

(xiii) for an anti-TWEAKR antibody comprising a variable region of the heavy chain (VH), comprising the variable CDR1 sequence of the heavy chain (H-CDR1), as represented by SEQ ID NO. 122, the variable CDR2 sequence of the heavy chain (H-CDR2), as represented by SEQ ID NO. 123 and the variable CDR3 sequence of the heavy chain (H-CDR3), as represented by SEQ ID NO. 124, as well as a variable region of the light chain (VL), comprising the variable CDR1 sequence of the light chain (L-CDR1), as represented by SEQ ID NO. 126, the variable CDR2 sequence of the light chain (L-CDR2), as represented by SEQ ID NO. 127 and the variable CDR3 sequence of the light chain (L-CDR3), as represented by SEQ ID NO. 128,

(xiv) for an anti-TWEAKR antibody comprising a variable region of the heavy chain (VH), comprising the variable CDR1 sequence of the heavy chain (H-CDR1), as represented by SEQ ID NO. 132, the variable CDR2 sequence of the heavy chain (H-CDR2), as represented by SEQ ID NO. 133 and the variable CDR3 sequence of the heavy chain (H-CDR3), as represented by SEQ ID NO. 134, as well as a variable region of the light chain (VL), comprising the variable CDR1 sequence of the light chain (L-CDR1), as represented by SEQ ID NO. 136, the variable CDR2 sequence of the light chain (L-CDR2), as represented by SEQ ID NO. 137 and the variable CDR3 sequence of the light chain (L-CDR3), as represented by SEQ ID NO. 138,

(xv) for an anti-TWEAKR antibody comprising a variable region of the heavy chain (VH), comprising the variable CDR1 sequence of the heavy chain (H-CDR1), as represented by SEQ ID NO. 142, the variable CDR2 sequence of the heavy chain (H-CDR2), as represented by SEQ ID NO. 143 and the variable CDR3 sequence of the heavy chain (H-CDR3), as represented by SEQ ID NO. 144, as well as a variable region of the light chain (VL), comprising the variable CDR1 sequence of the light chain (L-CDR1), as represented by SEQ ID NO. 146, the variable CDR2 sequence of the light chain (L-CDR2), as represented by SEQ ID NO. 147 and the variable CDR3 sequence of the light chain (L-CDR3), as represented by SEQ ID NO. 148; or

(xvi) for an anti-TWEAKR antibody comprising a variable region of the heavy chain (VH), comprising the variable CDR1 sequence of the heavy chain (H-CDR1), as represented by SEQ ID NO. 152, the variable CDR2 sequence of the heavy chain (H-CDR2), as represented by SEQ ID NO. 153 and the variable CDR3 sequence of the heavy chain (H-CDR3), as represented by SEQ ID NO. 154, as well as a variable region of the light chain (VL), comprising the variable CDR1 sequence of the light chain (L-CDR1), as represented by SEQ ID NO. 156, the variable CDR2 sequence of the light chain (L-CDR2), as represented by SEQ ID NO. 157 and the variable CDR3 sequence of the light chain (L-CDR3), as represented by SEQ ID NO. 158,

or for an antigen-binding fragment of said antibodies.

**35.** The compound or conjugate in accordance with one or more the foregoing claims, wherein the antibody (AK, AB, AK1, AK2, AK3)

(i) stands for an anti-EGFR antibody comprising a variable region of the heavy chain (VH) corresponding to SEQ ID NO. 1, as well as a variable region of the light chain (VL), corresponding to SEQ ID NO. 5,

(ii) for an anti- HER2 antibody comprising a variable region of the heavy chain (VH), corresponding to SEQ ID NO. 11, as well as a variable region of the light chain (VL), corresponding to SEQ ID NO. 15,

(iii) for an anti-TWEAKR antibody comprising a variable region of the heavy chain (VH), corresponding to SEQ ID NO. 21, as well as a variable region of the light chain (VL), corresponding to SEQ ID NO. 25,

(iv) for an anti-TWEAKR antibody comprising a variable region of the heavy chain (VH), corresponding to SEQ ID NO. 31, as well as a variable region of the light chain (VL), corresponding to SEQ ID NO. 35,

(v) for an anti-TWEAKR antibody comprising a variable region of the heavy chain (VH), corresponding to SEQ ID NO. 41, as well as a variable region of the light chain (VL), corresponding to SEQ ID NO. 45,

(vi) for an anti-TWEAKR antibody comprising a variable region of the heavy chain (VH), corresponding to SEQ

ID NO. 51, as well as a variable region of the light chain (VL), corresponding to SEQ ID NO. 55,
(vii) for an anti-TWEAKR antibody comprising a variable region of the heavy chain (VH), corresponding to SEQ ID NO. 61, as well as a variable region of the light chain (VL), corresponding to SEQ ID NO. 65,
(viii) for an anti-HER2 antibody comprising a variable region of the heavy chain (VH), corresponding to SEQ ID NO. 71, as well as a variable region of the light chain (VL), corresponding to SEQ ID NO. 75,
(ix) for an anti-HER2 antibody comprising a variable region of the heavy chain (VH), corresponding to SEQ ID NO. 81, as well as a variable region of the light chain (VL), corresponding to SEQ ID NO. 85,
(x) for an anti- B7H3 antibody comprising a variable region of the heavy chain (VH), corresponding to SEQ ID NO. 91, as well as a variable region of the light chain (VL), corresponding to SEQ ID NO. 95,
(xi) for an anti- B7H3 antibody comprising a variable region of the heavy chain (VH), corresponding to SEQ ID NO. 101, as well as a variable region of the light chain (VL), corresponding to SEQ ID NO. 105,
(xii) for an anti-TWEAKR antibody comprising a variable region of the heavy chain (VH), corresponding to SEQ ID NO. 111, as well as a variable region of the light chain (VL), corresponding to SEQ ID NO. 115,
(xiii) for an anti-TWEAKR antibody comprising a variable region of the heavy chain (VH), corresponding to SEQ ID NO. 121, as well as a variable region of the light chain (VL), corresponding to SEQ ID NO. 125,
(xiv) for an anti-TWEAKR antibody comprising a variable region of the heavy chain (VH), corresponding to SEQ ID NO. 131, as well as a variable region of the light chain (VL), corresponding to SEQ ID NO. 135,
(xv) for an anti-TWEAKR antibody comprising a variable region of the heavy chain (VH), corresponding to SEQ ID NO. 141, as well as a variable region of the light chain (VL), corresponding to SEQ ID NO. 145, or
(xvi) for an anti-TWEAKR antibody comprising a variable region of the heavy chain (VH), corresponding to SEQ ID NO. 151, as well as a variable region of the light chain (VL), corresponding to SEQ ID NO. 155,

or for an antigen-binding fragment of said antibodies.

**36.** The compound or conjugate in accordance with one or more the foregoing claims, wherein the antibody (AK, AB, AK1, AK2, AK3)

(i) stands for an anti-EGFR antibody comprising a region of the heavy chain, corresponding to SEQ ID NO. 9, as well as a region of the light chain, corresponding to SEQ ID NO. 10,
(ii) for an anti-HER2 antibody comprising a region of the heavy chain, corresponding to SEQ ID NO. 19, as well as a variable region of the light chain, corresponding to SEQ ID NO. 20,
(iii) for an anti-TWEAKR antibody comprising a region of the heavy chain, corresponding to SEQ ID NO. 29, as well as a region of the light chain, corresponding to SEQ ID NO. 30,
(iv) for an anti-TWEAKR antibody comprising a region of the heavy chain, corresponding to SEQ ID NO. 39, as well as a region of the light chain, corresponding to SEQ ID NO. 40,
(v) for an anti-TWEAKR antibody comprising a region of the heavy chain, corresponding to SEQ ID NO. 49, as well as a region of the light chain, corresponding to SEQ ID NO. 50,
(vi) for an anti-TWEAKR antibody comprising a region of the heavy chain, corresponding to SEQ ID NO. 59, as well as a region of the light chain, corresponding to SEQ ID NO. 60,
(vii) for an anti-TWEAKR antibody comprising a region of the heavy chain, corresponding to SEQ ID NO. 69, as well as a region of the light chain, corresponding to SEQ ID NO. 70,
(viii) for an anti-HER2 antibody comprising a region of the heavy chain, corresponding to SEQ ID NO. 79, as well as a region of the light chain, corresponding to SEQ ID NO. 80,
(ix) for an anti-HER2 antibody comprising a region of the heavy chain, corresponding to SEQ ID NO. 89, as well as a region of the light chain, corresponding to SEQ ID NO. 90,
(x) for an anti-B7H3 antibody comprising a region of the heavy chain, corresponding to SEQ ID NO. 99, as well as a region of the light chain, corresponding to SEQ ID NO. 100,
(xi) for an anti-B7H3 antibody comprising a region of the heavy chain, corresponding to SEQ ID NO. 109, as well as a region of the light chain, corresponding to SEQ ID NO. 110,
(xii) for an anti-TWEAKR antibody comprising a region of the heavy chain, corresponding to SEQ ID NO. 119, as well as a region of the light chain, corresponding to SEQ ID NO. 120,
(xiii) for an anti-TWEAKR antibody comprising a region of the heavy chain, corresponding to SEQ ID NO. 129, as well as a region of the light chain, corresponding to SEQ ID NO. 130,
(xiv) for an anti-TWEAKR antibody comprising a region of the heavy chain, corresponding to SEQ ID NO. 139, as well as a region of the light chain, corresponding to SEQ ID NO. 140,
(xv) for an anti-TWEAKR antibody comprising a region of the heavy chain, corresponding to SEQ ID NO. 149, as well as a region of the light chain, corresponding to SEQ ID NO. 150, or
(xvi) for an anti-TWEAKR antibody comprising a region of the heavy chain, corresponding to SEQ ID NO. 159,

as well as a region of the light chain, corresponding to SEQ ID NO. 160,

or for an antigen-binding fragment of said antibodies.

**37.** A pharmaceutical composition comprising a compound in accordance with one or more of the foregoing claims in combination with an inert, non-toxic, pharmaceutically suitable auxiliary substance.

**38.** The compound in accordance with one or more of the foregoing claims, for use in a procedure for treating and/or preventing diseases.

**39.** The compound in accordance with one or more of the foregoing claims for use in a procedure to treat hyperproliferative and/or angiogenic diseases.

## Revendications

**1.** Composé de la formule suivante la :

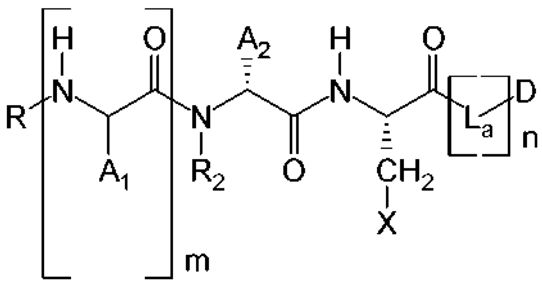

(Ia),

dans laquelle

m va de 0 à 2 ;
n est 0 ou 1 ;
X représente -C(=O)-$NH_2$ ou -COOH,
$L_a$ représente une *séquence de liaison auto-immolable,*
$A_1$ représente un des résidus acides aminés Gly, Pro, Ala, Val, Nva, Leu, Ile, Met, Phe, Tyr, Trp, Ser, Thr, Cys, Asn, Gln, Asp, Glu, Lys, Arg, citrulline ou His, ou un de leurs N-alkyl-acides aminés,
$A_2$ représente un des résidus acides aminés D-Ala, D-Pro, D-Ser, D-Asn, D-Asp ou D-His,
$R_2$ représente -H- ou un alkyle en $C_1$-$C_3$, ou
$R_2$ représente la liaison au groupe méthylène du cycle proline, dans le cas où $A_2$ représente un résidu D-Pro,
D représente -$D_1$-$(L_b)_o$-$(LIG)_p$,
$D_1$ représente un agent actif cytotoxique ,
LIG représente un lieur, qui après liaison à une molécule cible d'une cellule tumorale est internalisé par la cellule tumorale et subit une transformation intracellulaire, de préférence lysosomale,
$L_b$ représente une séquence de liaison,
o et p sont chacun indépendamment l'un de l'autre 0 ou 1,
R représente $Z_1$-(C=O)q-,
q est 0 ou 1,
$Z_1$ représente un groupe alkyle en $C_{1-10}$, aryle en $C_{5-10}$ ou aralkyle en $C_{6-10}$, hétéroalkyle en $C_{5-10}$, alkyle en $C_{1-10}$-O-aryle en $C_{6-10}$, hétérocycloalkyle, hétéroaryle, hétéroaryl-alkyle en $C_{5-10}$, hétéroaryl-alkoxy en $C_{5-10}$, alcoxy en $C_{1-10}$, aryle en $C_{6-10}$-alkyloxy en $C_{1-10}$, aryloxy en $C_{6-10}$ ou aralcoxy en $C_{6-10}$, hétéroalcoxy en $C_{5-10}$, alkyle en $C_{1-10}$-O-aryloxy en $C_{6-10}$ ou hétérocycloalcoxy en $C_{5-10}$, qui peut être mono ou polysubstitué par -$NH_2$, -C(=O)-, -NH-alkyle, -N(alkyle)$_2$, -NH-C(=O)-alkyle,-N(alkyle)-C(=O)-alkyle, -$S(=O)_3$-H, -$S(=O)_2$-$NH_2$, -$S(=O)_2$-N(alkyle)$_2$, -COOH,-C(=O)$NH_2$, -C(=O)-N(alkyle)$_2$ ou -OH, ou représente -H ou un groupe -$(CH_2)_{0-1}$-$O_x$-$(CH_2CH_2O)_v$-$R^1$,
x est 0 ou 1
v est un nombre de 1 à 20,
$R^1$ représente -H, -alkyle, -$CH_2$-COOH, -$CH_2$-$CH_2$-COOH ou -$CH_2$-$CH_2$-$NH_2$, ou

R représente LIG-$(L_c)_r$-,
LIG représente un lieur, qui après la liaison à une molécule cible sur une cellule tumorale est internalisé par la cellule tumorale et subit une transformation intracellulaire, de préférence lysosomale,
$L_c$ représente une séquence de liaison et
r est 0 ou 1.

**2.** Composé selon la revendication 1, dans lequel $R_2$ est -H ou un groupe méthyle, de préférence -H.

**3.** Composé selon une ou plusieurs des revendications précédentes dans lequel m est 1.

**4.** Composé selon une ou plusieurs des revendications précédentes dans lequel $A_1$ est dans la configuration L ou dans la configuration D.

**5.** Composé selon une ou plusieurs des revendications précédentes dans lequel $A_1$ représente un résidu acide aminé Ala ou Val.

**6.** Composé selon une ou plusieurs des revendications précédentes dans lequel q est 1, et Z1 représente un alkyle en $C_{1-10}$, qui peut être interrompu une ou plusieurs fois par un atome d'oxygène (comme p. ex. un groupe méthyle ou une chaîne oligoalkylènoxyde éventuellement alkylée), un groupe aralkyle en $C_{6-10}$, hétéroaryl-alkyle en $C_{5-10}$, araloxy en $C_{6-10}$ ou hétéroaryl-alcoxy en $C_{5-10}$.

**7.** Composé selon une ou plusieurs des revendications précédentes dans lequel $L_a$ est choisi parmi les groupes suivants :

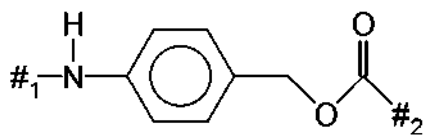

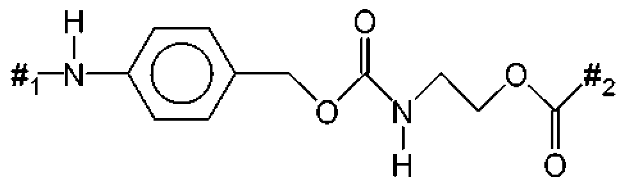

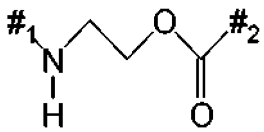

dans lesquels $\#_1$ représente la liaison à un groupe carbonyle et $\#_2$ représente la liaison à un groupe hydroxyle- ou amino de $D_1$.

**8.** Composé selon une ou plusieurs des revendications précédentes dans lequel $D_1$ représente un agent actif choisi parmi la mitomycine, la doxorubicine, l'aminoptérine, l'actinomycine, la bléomycine, la 9-amino-camptothécine, la n8-acétyl-spermidine, le 1-(2-chloroéthyl)-1,2-diméthansulfonyl-hydrazide, la tallysomycine, la cytarabine, l'étoposide, la camptothécine, le taxol, l'espéramicine, la podophyllotoxine, l'anguidine, la vincristine, la vinblastine, la morpholin-doxorubicine, la n-(5,5-diacétoxy-pentyl)-doxorubicine, la duocarmycine, l'auristatine, des dérivés pyrrolobenzodiazépine, la calichéamicine, la daunorubicine, la camptophécine DX8951 (Exatécan) ou un inhibiteur de la protéine de faisceau kinésine (inhibiteur de KSP) est utilisé, dans lequel l'agent actif est lié par son groupe hydroxyle ou amino à $L_a$ (lorsque n=1) ou le groupe carbonyle (lorsque n=0) selon la formule I.

**9.** Composé selon la revendication 8, dans lequel l'inhibiteur de KSP est un composé de formule suivante (IIa)

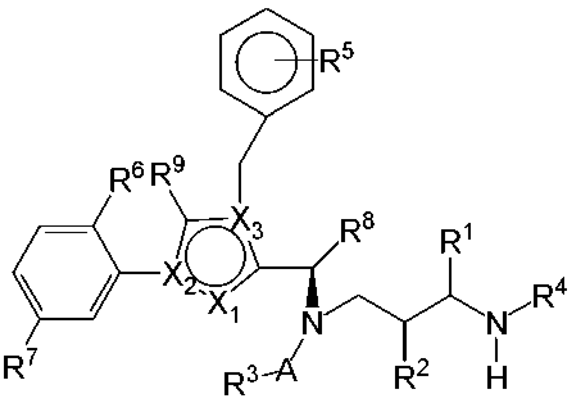

(IIa)

dans laquelle

$X_1$ représente N,
$X_2$ représente N et
$X_3$ représente C,

ou

$X_1$ représente N,
$X_2$ représente C et
$X_3$ représente N,

ou

$X_1$ représente CH ou CF,
$X_2$ représente C et
$X_3$ représente N,

ou

$X_1$ représente NH,
$X_2$ représente C et
$X_3$ représente C,
ou $X_1$ représente CH,
$X_2$ représente N et
$X_3$ représente C
A représente -C(=O)-, -S(=O)-, $-S(=O)_2-$, ou $-S(=O)_2-NH-$
$R^1$ représente -H, -L-#1, -MOD ou $-(CH_2)_{0-3}Z$,
Z représente -H, $-NHY^3$, $-OY^3$, $-SY^3$, un halogène, $-C(=O)-NY^1Y^2$, ou $-C(=O)-OY^3$,
$Y^1$ et $Y^2$ représentent indépendamment l'un de l'autre -H, $-NH_2$, $-(CH_2CH_2O)_{0-3}-(CH_2)_{0-3}Z'$ ou $-CH(CH_2W)Z'$,
$Y^3$ représente -H ou $-(CH_2)_{0-3}Z'$,
Z' représente -H, $-NH_2$, $-S(=O)_3H$, -COOH, $-NH-C(=O)-CH_2-CH_2-CH(NH_2)C(=O)-$ou $-(C(=O)-NH-CHY^4)_{1-3}COOH$,
W représente -H ou -OH,
$Y^4$ représente un alkyle en $C_{1-6}$ linéaire ou ramifié, qui est éventuellement substitué par $-NH-C(=O)-NH_2$, ou représente un aryle ou un benzyle, qui est éventuellement substitué par $-NH_2$,
$R_2$ représente -H, -L-#1, -MOD, $-C(=O)-CHY^4-NHY^5$ ou $-(CH_2)_{0-3}Z$,
Z représente -H, un halogène, $-OY^3$, $-SY^3$, $-NHY^3$, $-C(=O)-NY^1Y^2$, ou $-C(=O)-OY^3$,
$Y^1$ et $Y^2$ représentent indépendamment l'un de l'autre -H, $-NH_2$, ou $-(CH_2)_{0-3}Z'$,
$Y^3$ représente -H ou $-(CH_2)_{0-3}Z'$,
Z' représente -H, $-S(=O)_3H$, $-NH_2$ ou -COOH,
$Y^4$ représente un alkyle en $C_{1-6}$ linéaire ou ramifié, qui est éventuellement substitué par $-NH-C(=O)-NH_2$, ou

représente un aryle ou un benzyle, qui est éventuellement substitué par $-NH_2$,
$Y^5$ représente -H ou $-C(=O)-CHY^6-NH_2$,
$Y^6$ représente un alkyle en $C_{1-6}$ linéaire ou ramifié,
$R^3$ représente -MOD, -L-#1, ou un groupe alkyle-, cycloalkyle-, aryle-, hétéroaryle-, hétéroroalkyle-, hétérocycloalkyle- éventuellement substitué, qui peuvent être substitués par un à trois groupes OH, un à trois atomes d'halogènes, un à trois groupes alkyle mono-, di- ou tri-halogénés, un à trois groupes -O-alkyle, un à trois groupes -SH-, un à trois groupes -S-alkyle, un à trois groupes -O-C(=O)-alkyle, un à trois groupes -O-C(=O)-NH-alkyle, un à trois groupes -NH-C(=O)-alkyle, un à trois groupes -NH-C(=O)-NH-alkyle, un à trois groupes $-S(=O)_n$-alkyle, un à trois groupes $-S(=O)_2$-NH-alkyle, 1 à 3 groupes -NH-alkyle, un à trois groupes$-N(alkyl)_2$-, un à trois groupes $NH_2$- ou un à trois groupes $-(CH_2)_{0-3}$ groupes Z,
n est 0,1 ou 2,
Z représente -H, un halogène, $-OY^3$, $-SY^3$, $-NHY^3$, $-C(=O)-NY^1Y^2$, ou $-C(=O)-OY^3$,
$Y^1$ et $Y^2$ représentent indépendamment l'un de l'autre -H, $-NH_2$, ou $-(CH_2)_{0-3}Z'$ stehen,
$Y^3$ représente -H, $-(CH_2)_{0-3}-CH(NHC(=O)CH_3)Z'$,$-(CH_2)_{0-3}-CH(NH_2)Z'$ ou $-(CH_2)_{0-3}Z'$,
Z' représente -H, $-S(=O)_3H$, $-NH_2$ ou -COOH,
$R^4$ représente le groupe clivable par la legumaïne de formule Ia', Ia" et Ia‴,
$R^5$ représente -H, $-NH_2$, $-NO_2$, un halogène, -CN, $CF_3$, $-OCF_3$, $-CH_2F$, $-CH_2F$, -SH ou $-(CH_2)_{0-3}Z$,
Z représente -H, $-OY^3$, $-SY^3$, un halogène, $-NHY^3$, $-C(=O)-NY^1Y^2$, ou $-C(=O)-OY^3$,
$Y^1$ et $Y^2$ représentent indépendamment l'un de l'autre -H, $-NH_2$, ou $-(CH_2)_{0-3}Z'$,
$Y^3$ représente -H ou $-(CH_2)_{0-3}Z'$,
Z' représente -H, $-S(=O)_3H$, $-NH_2$ ou -COOH,
$R^6$ et $R^7$ représentent indépendamment l'un de l'autre -H, -CN, un alkyle en $C_{1-10}$-, un fluoro-alkyle en $C_{1-10}$, un alcényle en $C_{2-10}$, un fluoro alcényle en $C_{2-10}$, un alcynyle en $C_{2-10}$, un fluoro-alcynyle en $C_{2-10}$, un hydroxy-, $-NO_2$, $-NH_2$, -COOH ou un halogène,
$R^8$ représente un alkyle en $C_{1-10}$, un fluoro-alkyle en $C_{1-10}$-, un alcényle en $C_{2-10}$, un fluoro-alcényle en $C_{2-10}$, un alcynyle en $C_{2-10}$, un fluoro-alcényle en $C_{2-10}$, un cycloalkyle en $C_{4-10}$, un fluoro-cycloalkyle en $C_{4-10}$ ou $-(CH_2)_{0-2}-(HZ^2)$,
$HZ^2$ représente un hétérocyle à 4 à 7 chaînons avec un à deux hétéroatomes, choisis parmi N, O et S, qui peut être substitué par -OH, -COOH, $-NH_2$ ou -L-#1,
$R^9$ représente -H, -F, -CH3, $-CF_3$, $-CH_2F$ ou $-CHF_2$,
-L-#1 représente $-(L_b)_O-(LIG)_p$,
LIG représente un lieur, qui après liaison à une molécule cible d'une cellule tumorale est internalisé par la cellule tumorale et subit une transformation intracellulaire, de préférence lysosomale,
$L_b$ représente une séquence de liaison,
o et p représentent indépendamment l'un de l'autre 0 ou 1,
-MOD représente $-(NR^{10})_n-(G1)_O-G2-G3$,
$R^{10}$ représente -H ou un alkyle en $C_1-C_3$,
G1 représente -NH-C-(=O)-, -C(=O)-NH- ou

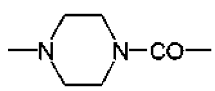
;

n est 0 ou 1 ;
o est 0 ou 1 et
G2 représente une chaîne hydrocarbonée linéaire ou ramifiée avec 1 à 20 atomes de carbone, qui peut être interrompue un ou plusieurs fois par -O-, -S-, -S(=O)-,$-S(=O)_2$-, $-NR^y$-, $-NR^yC(=O)$-, $-C(=O)NR^y$-, $-NR^yNR^y$-, $-S(=O)_2-NR^yNR^y$-, $-C(=O)-NR^yNR^y$-, -C(=O)-, $-CR^x=N-O$- et la chaîne hydrocarbonée linéaire ou ramifiée peut être substituée par $-NH-C(=O)-NH_2$, -COOH, -OH, $-NH_2$, un sulfonamide, une sulfone, un sulfoxyde, ou un acide sulfonique,
$R^y$ représente -H, un phényle, un alkyle en $C_1-C_{10}$, un alcényle en $C_2-C_{10}$ ou un alcynyle en $C_2-C_{10}$, qui peuvent être chacun substitués par $-NH-C(=O)-NH_2$,-COOH, -OH, $-NH_2$, un sulfonamide, une sulfone, un sulfoxyde, ou un acide sulfonique,
Rx représente -H, un alkyle en $C_1-C_3$ ou un phényle,
G3 représente -H ou -COOH et
-MOD présente au moins un groupe COOH,

ainsi que leurs sels, solvates et sels de solvates.

**10.** Composé selon la revendication 9, dans lequel $R^1$ ou $R^3$ représentent -L-#1.

**11.** Composé selon une ou plusieurs des revendications 9 et 10, dans lequel $X_1$ représente CH, $X_2$ représente C et $X_3$ représente N.

**12.** Composé selon une ou plusieurs des revendications 9 à 11, dans lequel $R^6$ et $R^7$ représentent indépendamment l'un de l'autre -H, un alkyle en $C_{1-3}$ ou un halogène.

**13.** Composé selon la revendication 12, dans lequel $R^6$ et $R^7$ représentent F.

**14.** Composé selon une ou plusieurs des revendications 9 à 13, dans lequel $R^8$ représente un alkyle en $C_{1-4}$ (de préférence le tert-butyle) ou un cyclohexyle.

**15.** Composé selon une ou plusieurs des revendications 9 à 14, dans lequel $R^9$ représente -H.

**16.** Composé de l'une quelconque ou de plusieurs des revendications précédentes, dans lequel soit D soit R comprend le lieur LIG.

**17.** Composé selon la revendication 16, dans lequel LIG est un peptide, une protéine ou un dérivé de celui-ci, choisi parmi octréotide, GnRH-III, [D-Tyr$^6$, β-Ala$^{11}$, Phe$^{13}$, Nle$^{14}$]BN(6-14), NT(8-13), c(RGDfK), HSDAVFTDNYTRLRKOMAVKKYLNSILN-NH2 (SEQ ID NO: 161), NAPamide, [Phe$^7$, Pro$^{34}$]NPY, HER2-targeting peptide, ATEPRKQYATPRVFWTDAPG (SEQ ID NO: 162), LQWRRDDNVHNFGVWARYRL (SEQ ID NO: 163), ou représente un anticorps ou un fragment ou dérivé de celui-ci, qui le cas échéant se lie à une molécule cible extracellulaire d'une cellule tumorale et qui est le cas échéant conjugué à d'autres agents actifs cytotoxiques.

**18.** Composés selon une ou plusieurs des revendications précédentes, les composés présentant la formule suivante III'

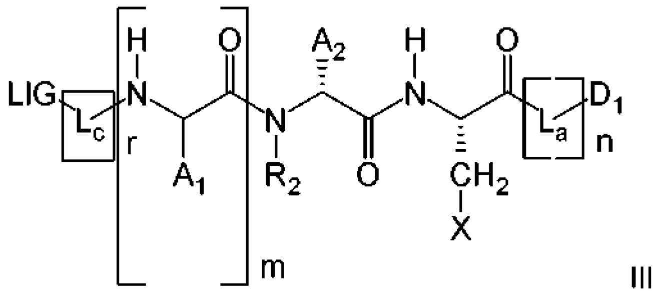

III'

dans laquelle

m, n, r, LIG, $L_a$, $L_c$, $D_1$,
X, $A_2$, $R_2$ et $A_1$ ont les significations indiquées dans la revendication 1.

**19.** Composés de formule IIIa'

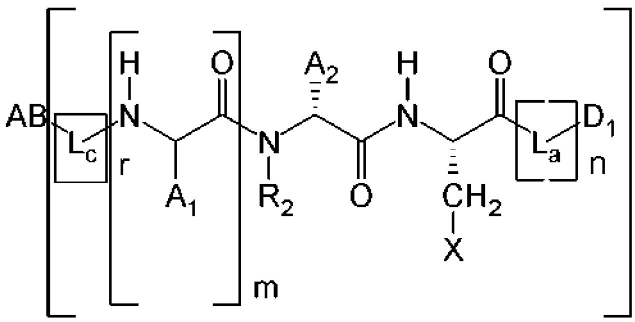

IIIa'

dans laquelle

m, n, r, $L_a$, $L_c$, $D_1$, X, $R_2$, $A_2$ et $A_1$ ont la même signification que dans la revendication 1,
AB représente un anticorps ou un fragment d'anticorps liant l'antigène et
s va de 1 à 20 de préférence de 2 à 8, de manière particulièrement préférée de 2 à 6.

**20.** Composés selon une ou plusieurs des revendications 1 à 17, les composés présentant la formule IV'

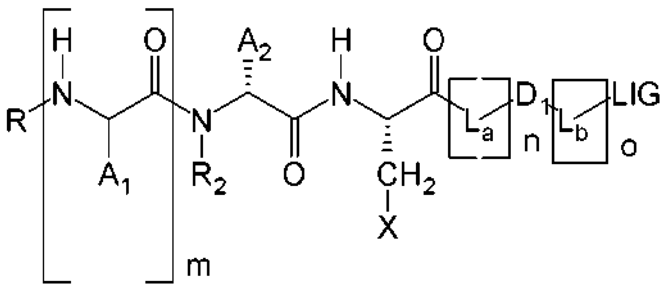

IV'

dans laquelle

m, n, o, R, LIG, $L_a$, $L_b$,
Di, X, $R_2$, $A_2$ et $A_1$ ont la même signification que dans la revendication 1.

**21.** Composés de formule IVa'

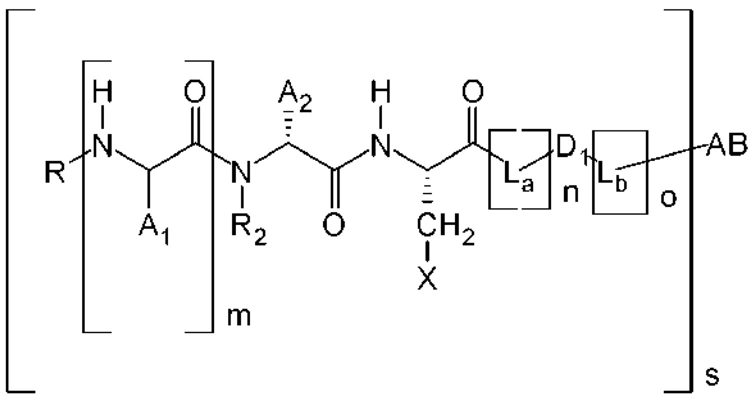

IVa'

dans laquelle

m, n, o, R, $L_a$, $L_b$, $D_1$, X, $R_2$, $A_2$ et $A_1$ ont la même signification que dans la revendication 1,
AB représente un anticorps ou un fragment d'anticorps liant l'antigène et
s va de 1 à 20, de préférence de 2 à 8, de manière particulièrement préférée de 2 à 6.

**22.** Composés selon une ou plusieurs des revendications précédentes, dans lesquels la séquence de liaison $L_b$ ou $L_c$ est liée à une chaîne latérale de cystéine ou à un résidu cystéine, d'un lieur tel qu'un anticorps ou un fragment d'anticorps se liant à un antigène et a la formule suivante :

§-(C=O)m-L1-L2-§§

dans laquelle

m est 0 ou 1 ;
§ représente un groupe pour la liaison à la molécule d'agent actif ou clivable par la legumaïne et

§§ est pour la liaison au lieur,

-L2- représente le groupe

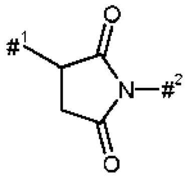,
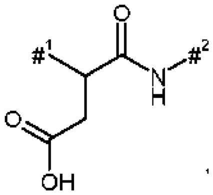,
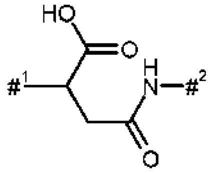,
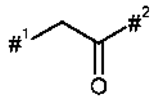,
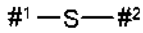,
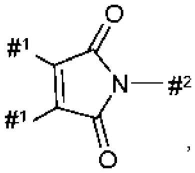,
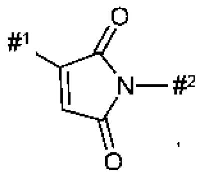,
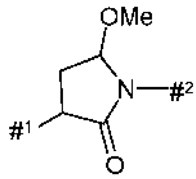
ou
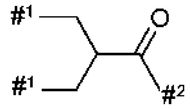

dans lesquels

$\#^1$ indique le site de liaison avec l'atome de soufre du lieur,

$\#^2$ indique le site de liaison avec le groupe $L^1$,

L1 représente-$(NR^{10})_n$-$(G1)_o$-G2-,

$R^{10}$ représente -H, $-NH_2$ ou un alkyle en $C_1$-$C_3$,

G1 représente -NHC(=O)- ou

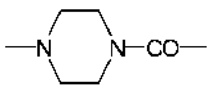

n est 0 ou 1,

o est 0 ou 1,

G2 représente une chaîne hydrocarbonée linéaire ou ramifiée contenant de 1 à 100 atomes de carbone composée de groupes arylène et/ou de groupes alkylène linéaires et/ou ramifiés et/ou cycliques, qui peut être interrompue un ou plusieurs fois par un ou plusieurs des groupes -O-, -S-, , -S(=O)-, $-S(=O)_2$, -NH-, -C(=O)-, -Nme-, -NHNH-, $-S(=O)_2$-NHNH-, - NH-C(=O)-, -C(=O)-NH-, -C(=O)-NHNH- et un hétérocycle aromatique ou non aromatique à 5 à 10 chaînons avec jusqu'à 4 hétéroatomes, choisis parmi N, O et S, -S(=O)- ou $-S(=O)_2$- (de préférence

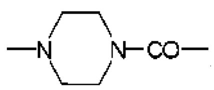

), et dans laquelle les chaînes latérales, s'il en est, peuvent être substituées par -NH-C(=O)-$NH_2$, -COOH, -OH, $-NH_2$, NH-$CNNH_2$, un sulfonamide, une sulfone, un sulfoxyde ou un acide sulfonique, ou un des groupes suivants

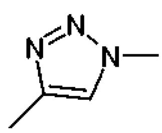,
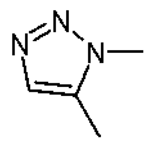,
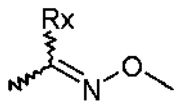
ou
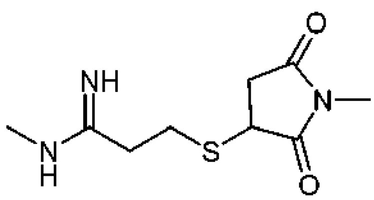

dans lesquels

Rx représente -H, un alkyle en $C_1$-$C_3$ ou un phényle.

**23.** Composés selon la revendication 22, dans lesquels L2 représente une ou les deux formules suivantes

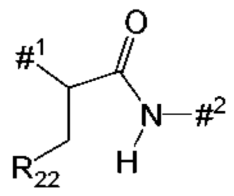

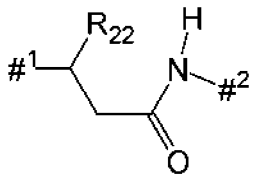

dans lesquels

$\#^1$ représente le site de liaison à l'atome de soufre du lieur,
$\#^2$ représente le site de liaison avec le groupe $L^1$,
$R^{22}$ représente -COOH et

plus de 80 % des liaisons à l'atome de soufre du liant (par rapport au nombre total de liaisons du lieur au liant) sont présentes dans l'une de ces deux formules.

**24.** Composés selon la revendication 22 ou 23, dans lesquels la chaîne hydrocarbonée est interrompue par un des

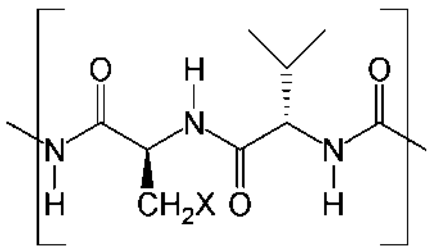

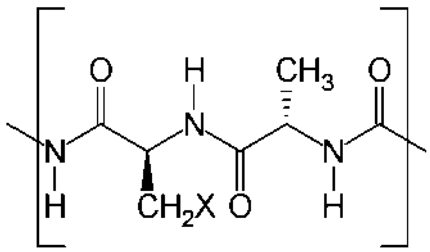

groupes suivants, dans lesquels
X représente -H ou un groupe alkyle en $C_{1-10}$, qui peut le cas échéant être substitué par - NH-C(=O)-$NH_2$, -COOH, -OH, -$NH_2$, -NH-CNN$H_2$, une sulfone, un sulfoxyde ou un acide sulfonique.

**25.** Composés selon une des revendications 22 à 24, dans lesquels le lieur présente la formule suivante

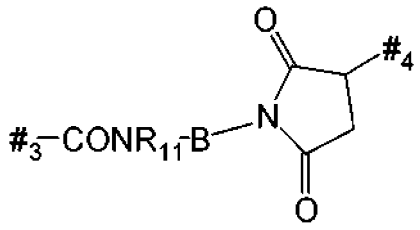

dans laquelle

#3 représente la liaison à la molécule d'agent actif,
#4 représente la liaison au lieur,
$R^{11}$ représente -H ou $-NH_2$,
B représente le groupe $-[(CH_2)_x-(X^4)_y]_w-(CH_2)_z-$,
w va de 0 à 20 ;
x va de 0 à 5 ;
x va de 0 à 5 ;
y est 0 ou 1 ;
z va de 0 à 5 ; et
$X^4$ représente -O-, -C(=O)-NH-, -NH-C(=O)- ou

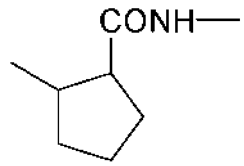

.

**26.** Composés selon une ou plusieurs des revendications précédentes, dans lesquels le lieur - $L_b$ et/ou $-L_c$ est lié à une chaîne latérale cystéine ou à un résidu cystéine et présente la formule suivante

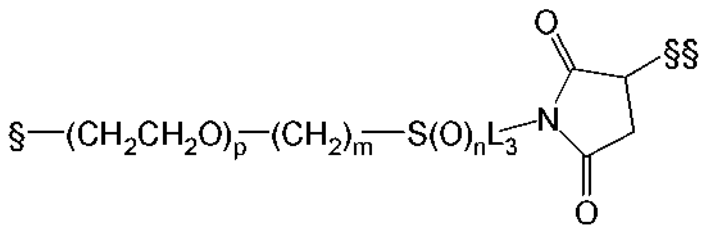

dans laquelle

§ représente la liaison à la molécule de l'agent actif ou le groupe clivable par la légumaïne,
§§ représente la liaison au lieur,
m est 0, 1, 2 ou 3,
n est 0, 1 ou 2,
p va de 0 à 20,
L3 représente le groupe

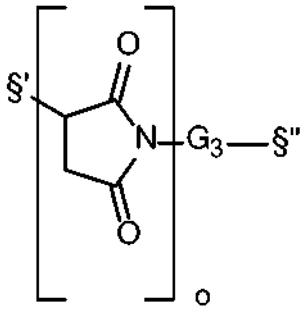

dans lequel

o est 0 ou 1,
§' représente la liaison au groupe -S(O)n et
§" représente la liaison à l'atome N dans le cycle

G3 représente une chaîne hydrocarbonée linéaire ou ramifiée contenant 1 à 100 atomes de carbone, constituée de groupes arylène et/ou de groupes alkylène linéaires et/ou ramifiés et/ou cycliques, qui peut être interrompue par un ou plusieurs des groupes -O-, -S-, -S(=O)-, $S(=O)_2$-, - NH-, -C(=O)-, -NH-C(=O)-, -C(=O)-NH- et un hétérocycle aromatique ou non aromatique à 5 à 10 chaînons avec jusqu'à 4 hétéroatomes, choisis parmi N, O et S, -Nme-, -NHNH-, -$S(=O)_2$-NHNH-, -C(=O)-NHNH-, -S(=O)- ou -$S(=O)_2$ (de préférence

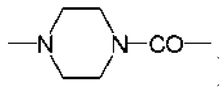

), dans laquelle les chaînes latérales, s'il en est, peuvent être substituées par -NHC(=O)-$NH_2$, -COOH, -OH, une sulfone, un sulfoxyde ou un acide sulfonique.

**27.** Composés selon la revendication 26, dans lesquels le lieur -$L_b$ ou -$L_c$ est lié à une chaîne latérale cystéine ou à un résidu cystéine et présente la formule suivante

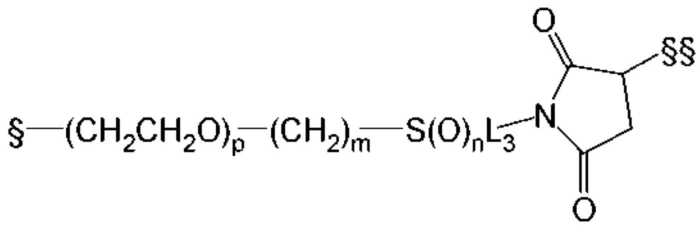

dans laquelle

§ représente le groupe pour la liaison à la molécule d'agent actif ou clivable par la legumaïne,
§§ est pour la liaison au lieur,
m est 1
p est 0 ;
n est 0,
L3 représente le groupe

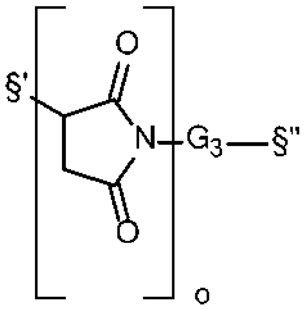

dans lequel

o est 0 ou 1,
G3 représente $-(CH_2CH_{20})_s$ - $(CH_2)_t$ - $(C(=O)-NH)_u$ - $CH_2CH_2O)_v$ - $(CH_2)_w$,
s, t, v et w représentent indépendamment les uns des autres 0 à 20,
u est 0 ou 1,
§' représente la liaison au groupe -S(O)n et
§" représente la liaison à l'atome de N dans le cycle.

**28.** Composés selon la revendication 25 ou 26, dans lesquels $R^2$ ou $R^3$ représentent -L-#1.

**29.** Conjugués selon une ou plusieurs des revendications précédentes de structures suivantes, dans lesquelles

AK représente le lieur, de préférence un anticorps ou un fragment d'anticorps liant l'antigène ($AK_1$, $AK_2$, $AK_3$) et
$AK_1$ représente de préférence un anticorps, qui est lié par un résidu cystéine à l'inhibiteur de KSP,
$AK_2$ représente de préférence un anticorps, qui est lié par un résidu lysine à l'inhibiteur de KSP, et
$AK_3$ représente de préférence un anticorps, qui est lié par un résidu glutamine à l'inhibiteur de KSP,
et
n représente un nombre de 1 à 50, de préférence 1 à 20, de manière particulièrement préférée 2 à 8 et en particulier 2 à 6 :

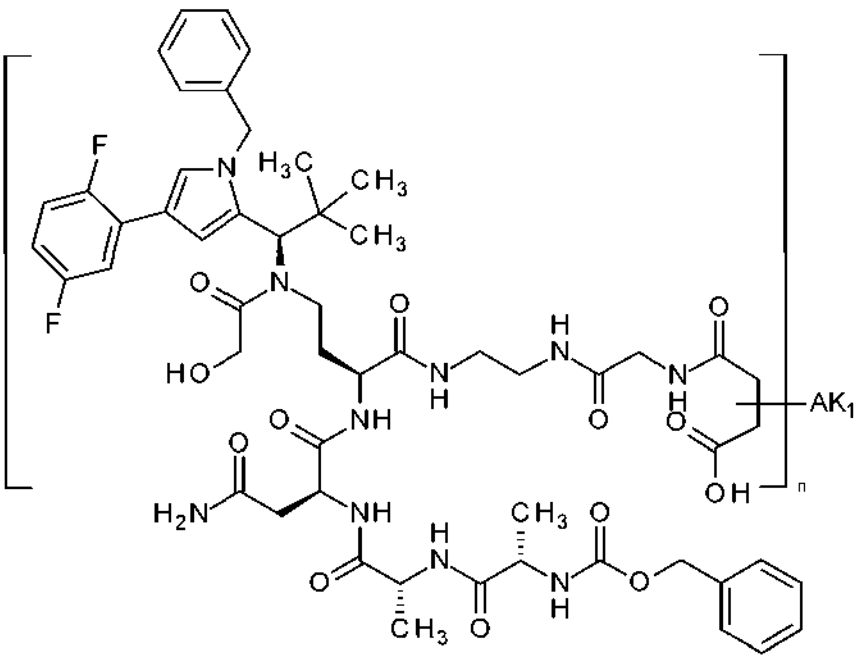

,

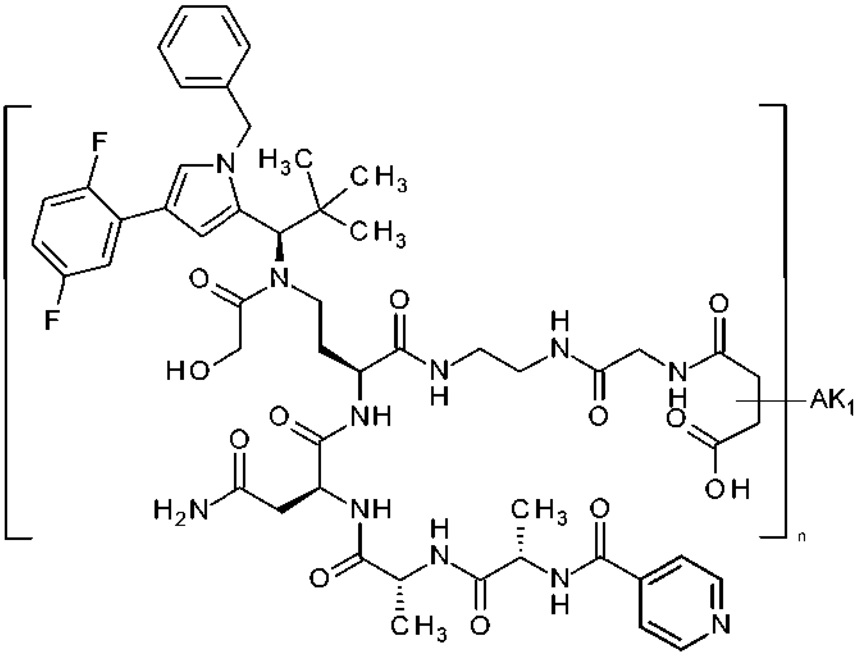

,

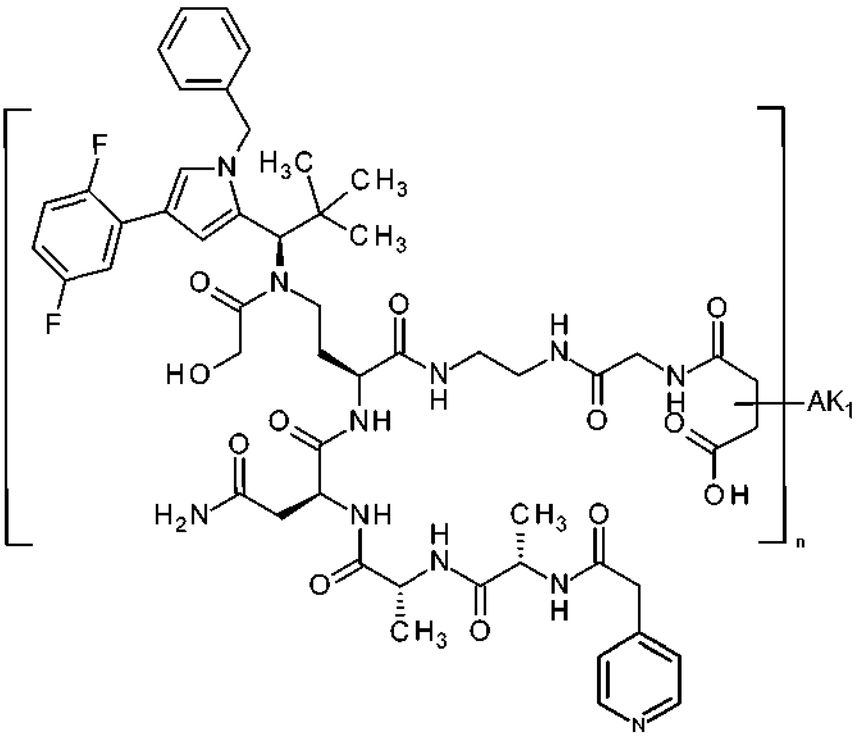

,

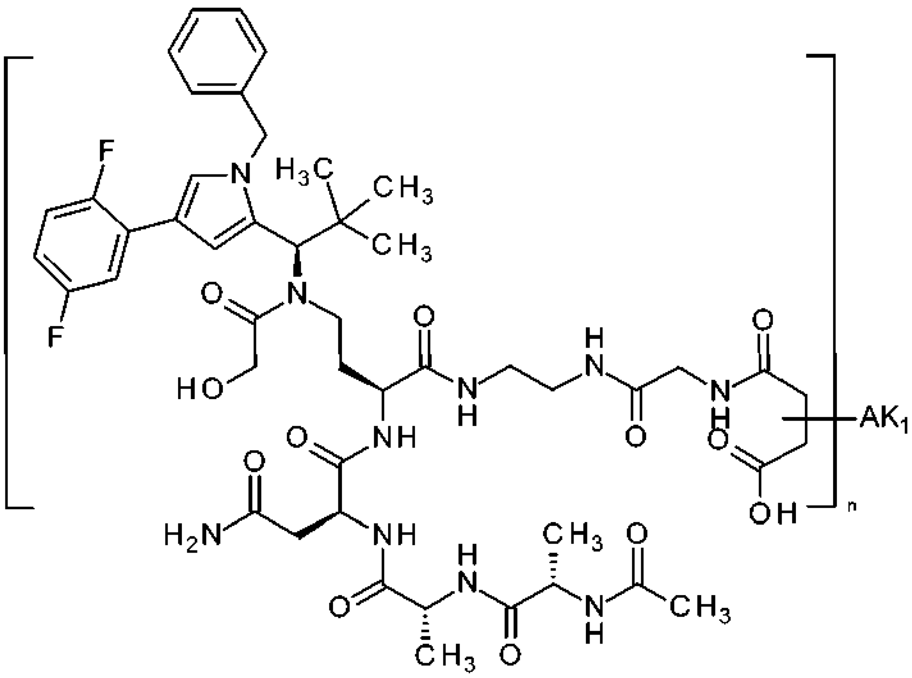

,

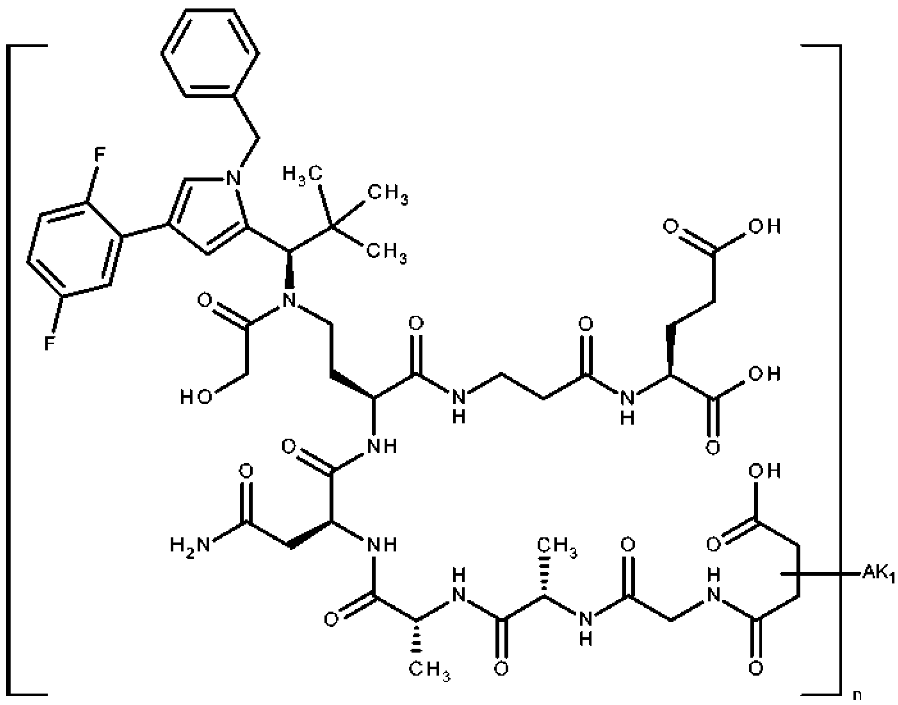

,

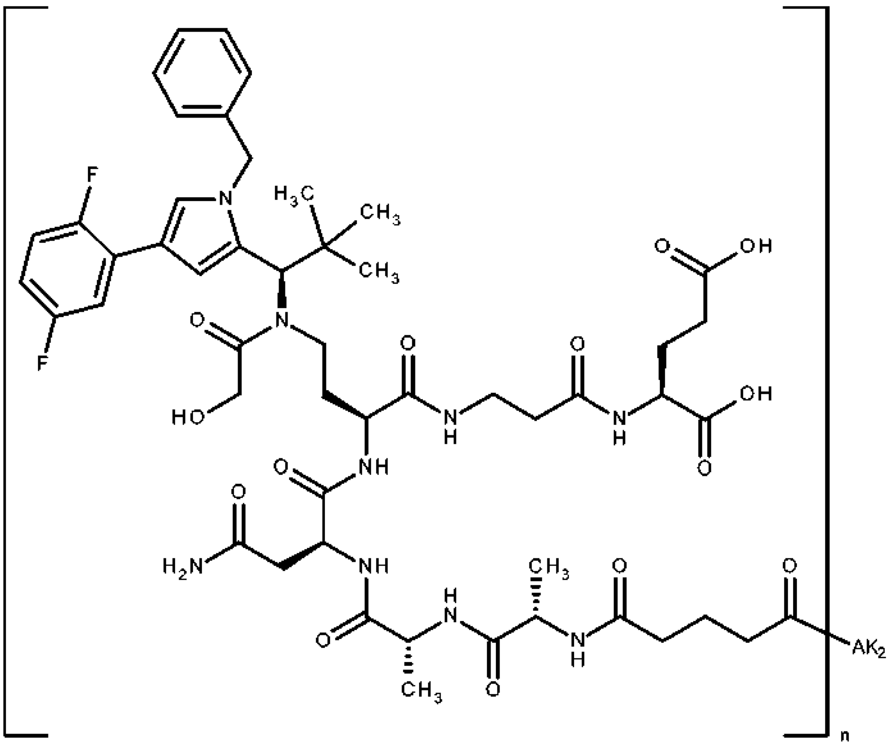

,

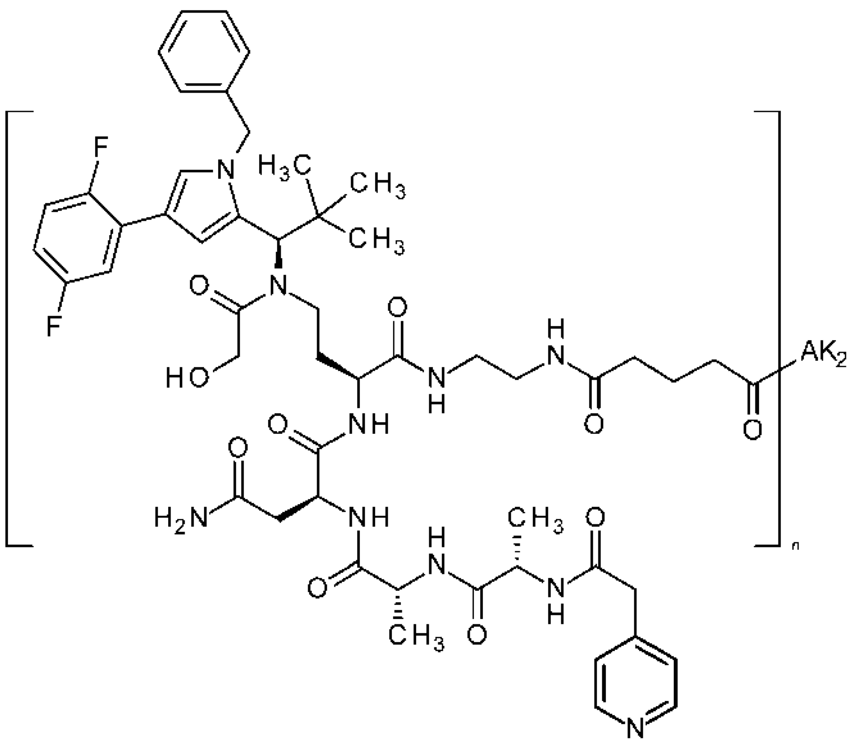

,

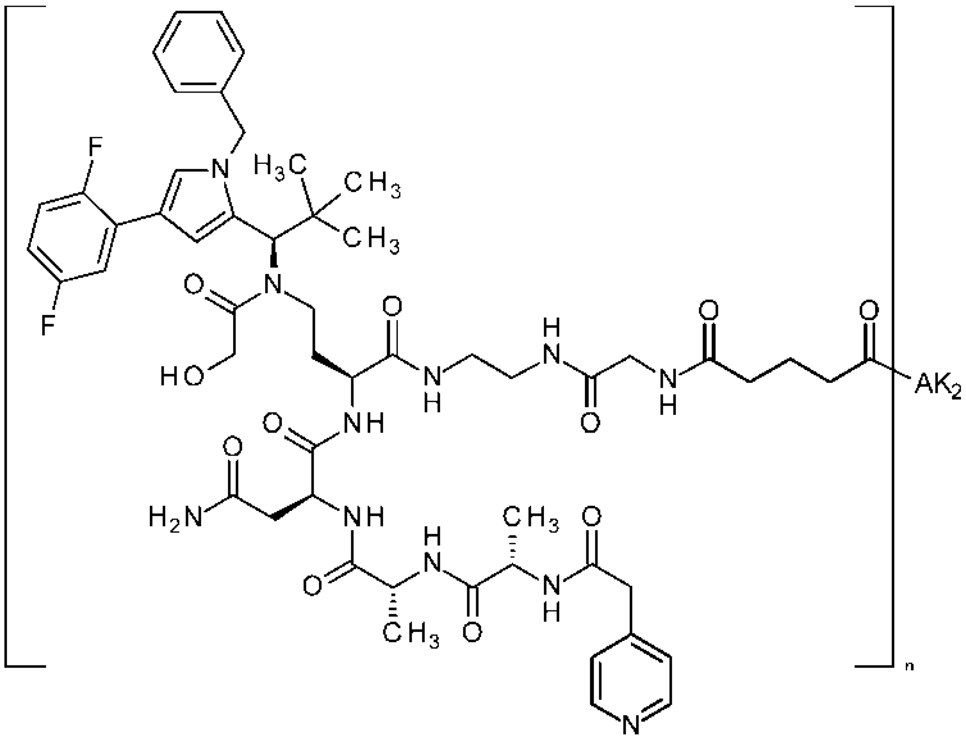

,

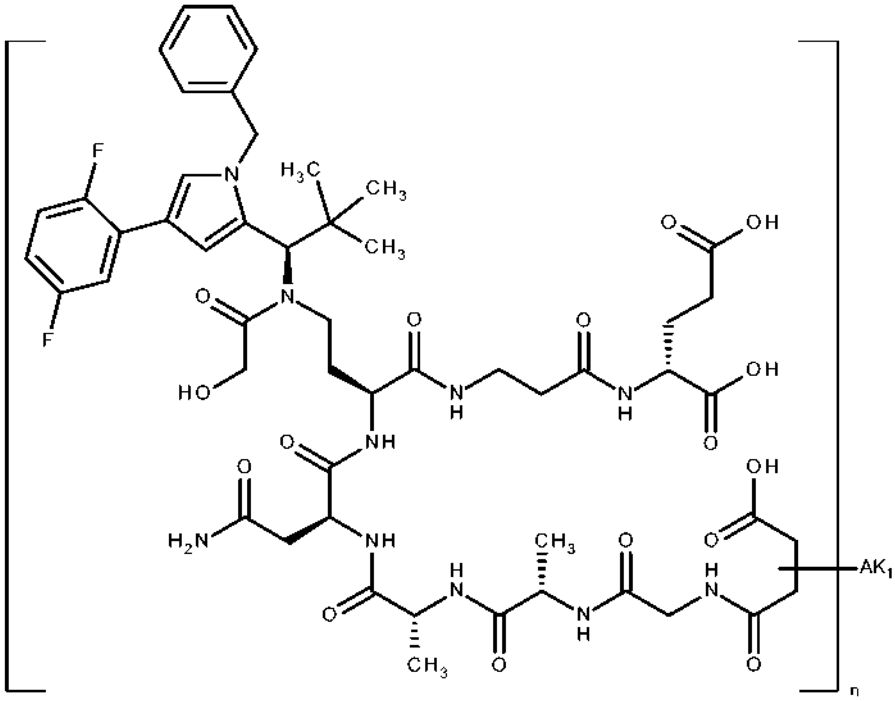

,

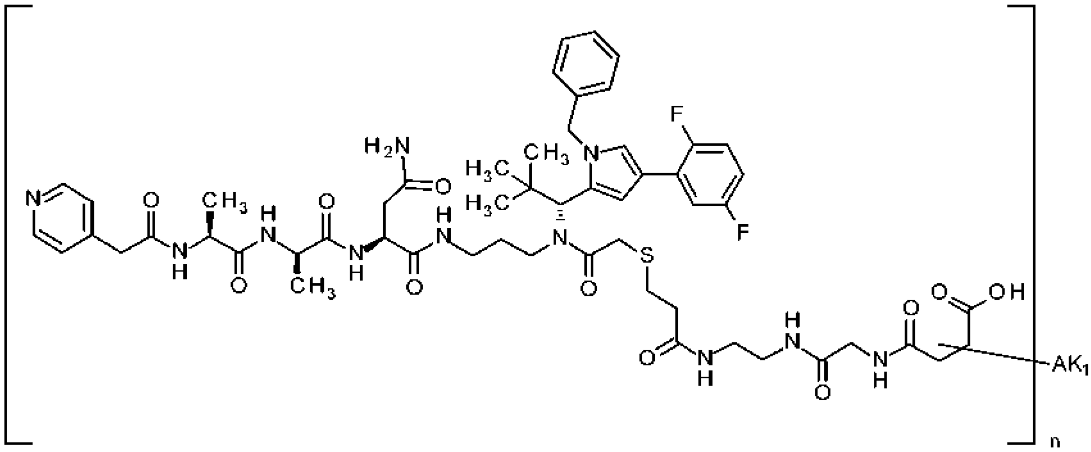

,

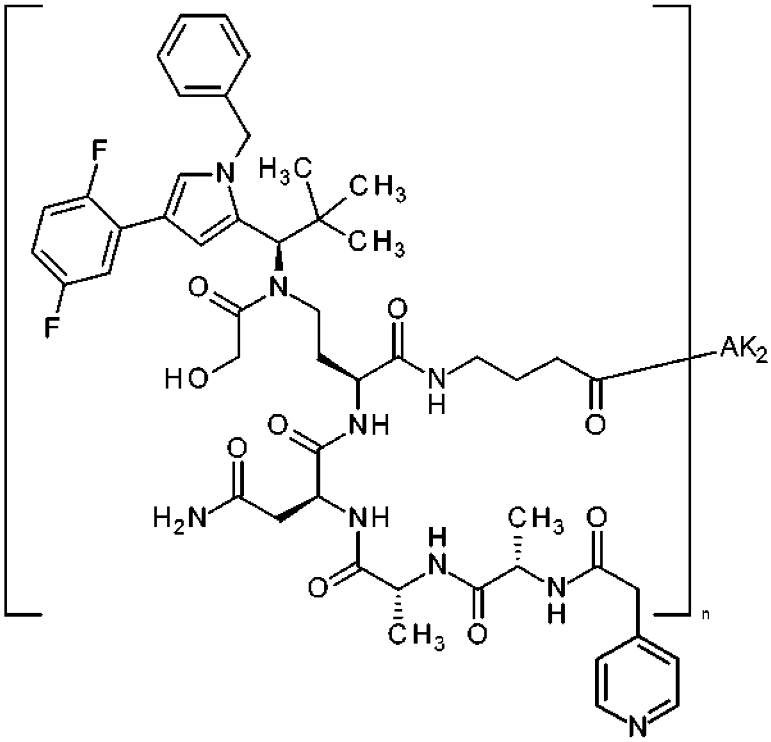

,

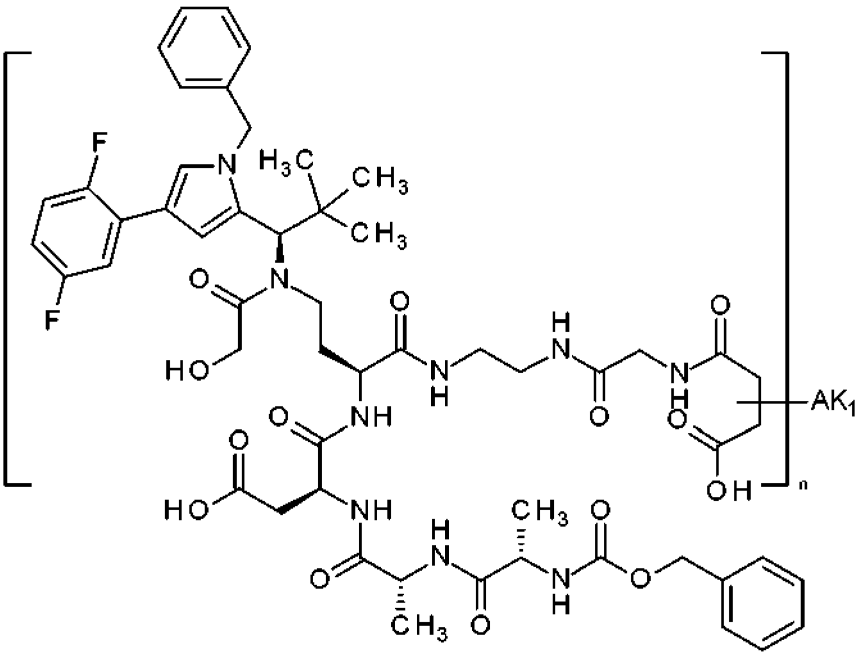

,

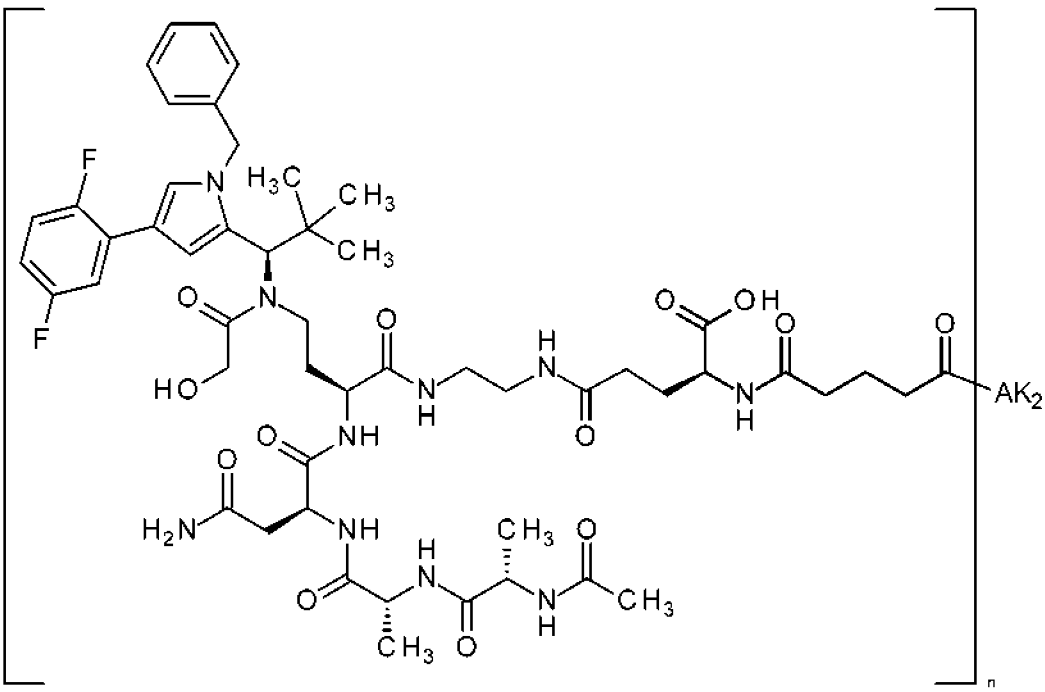

,

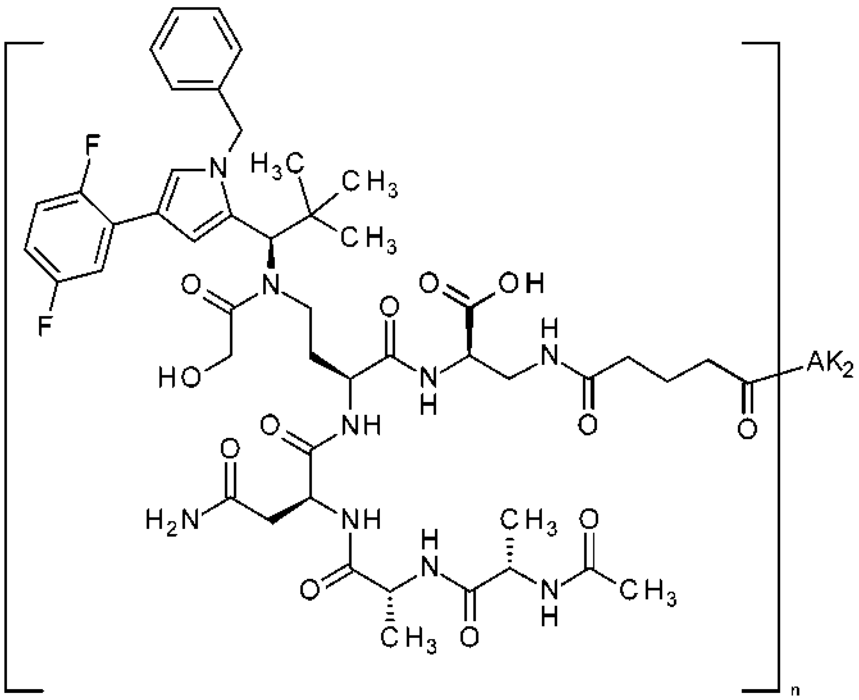

,

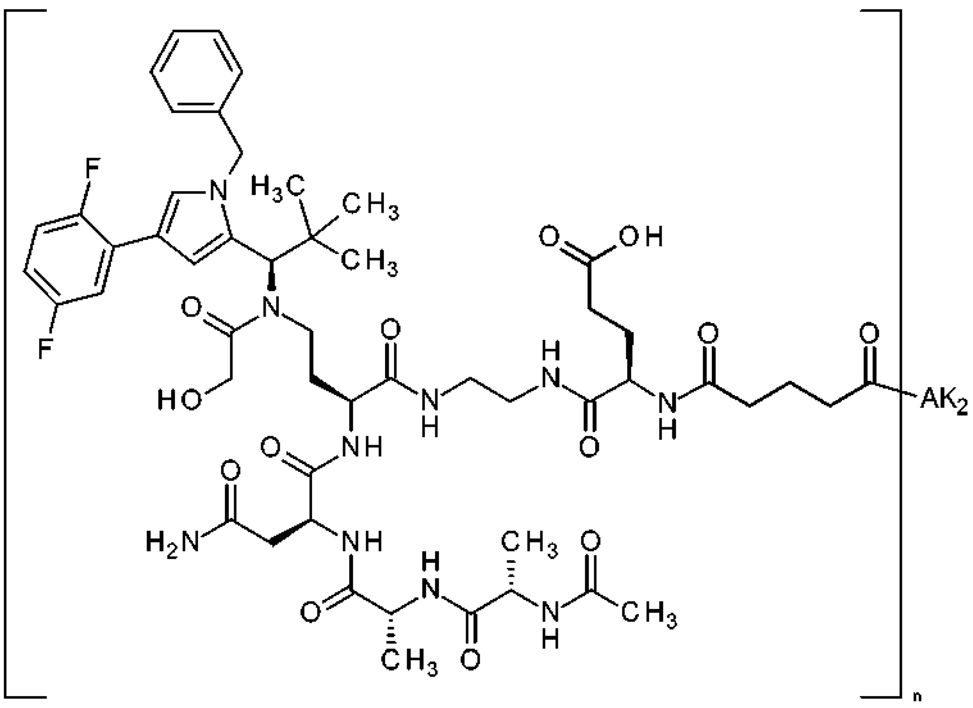

,

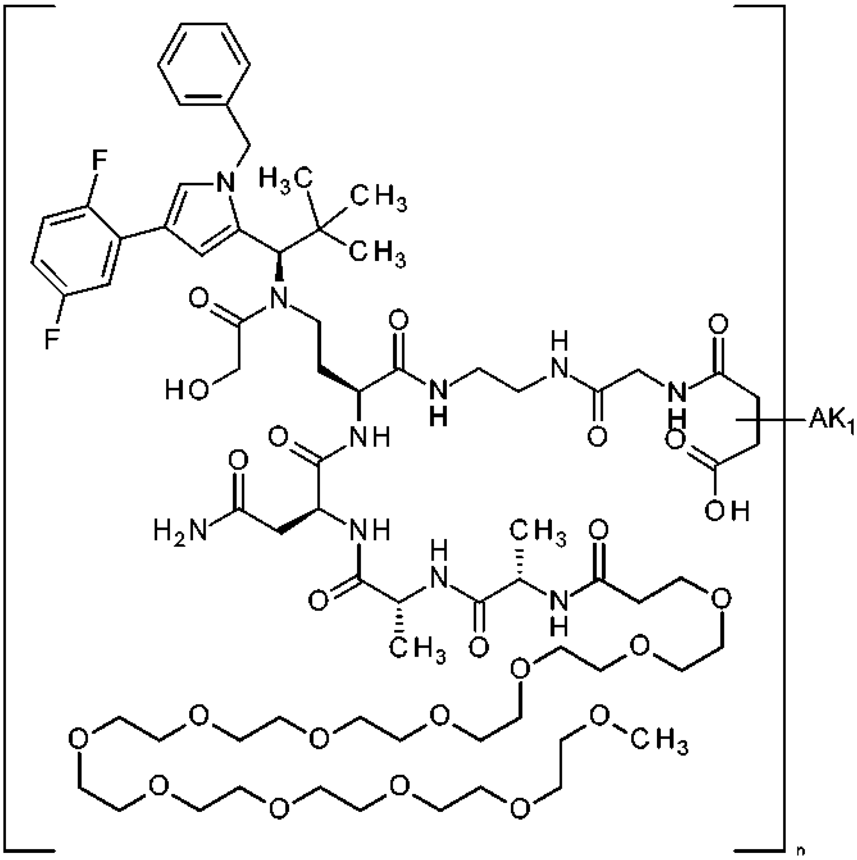

,

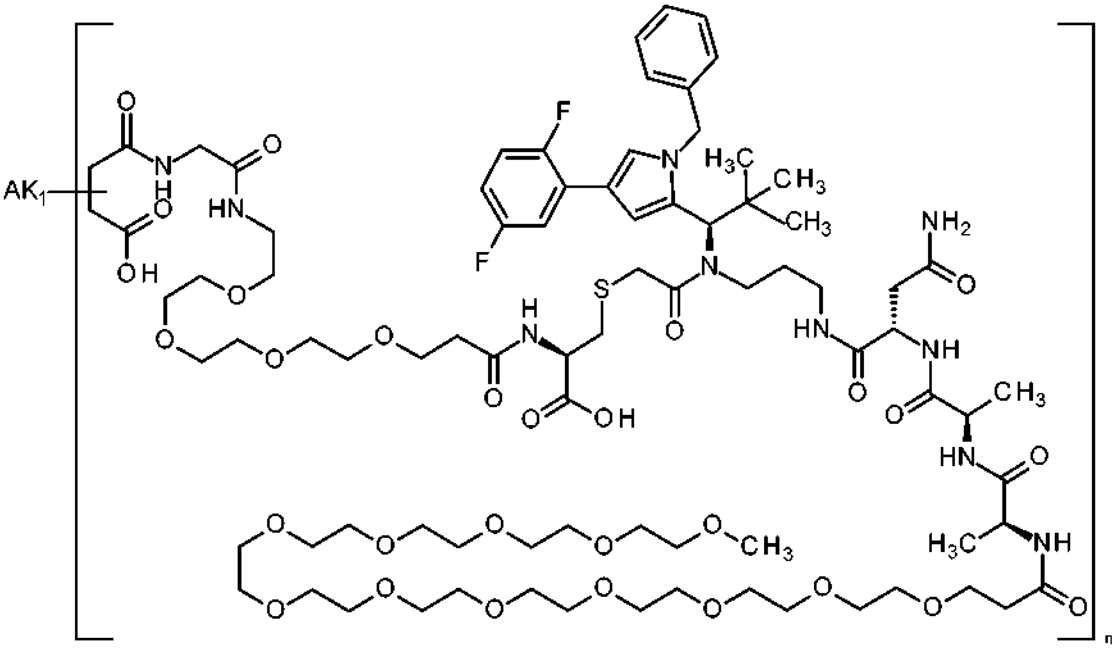

,

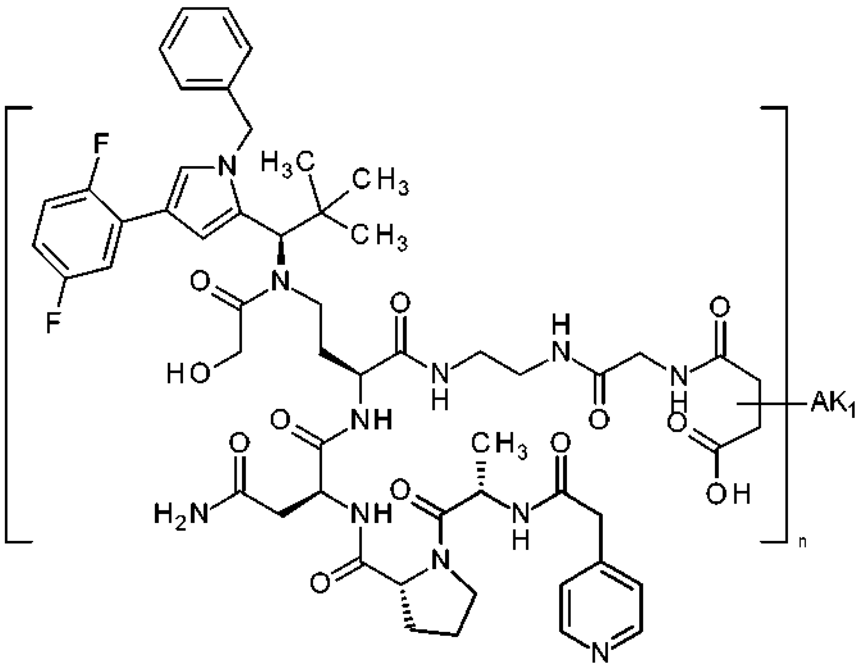

,

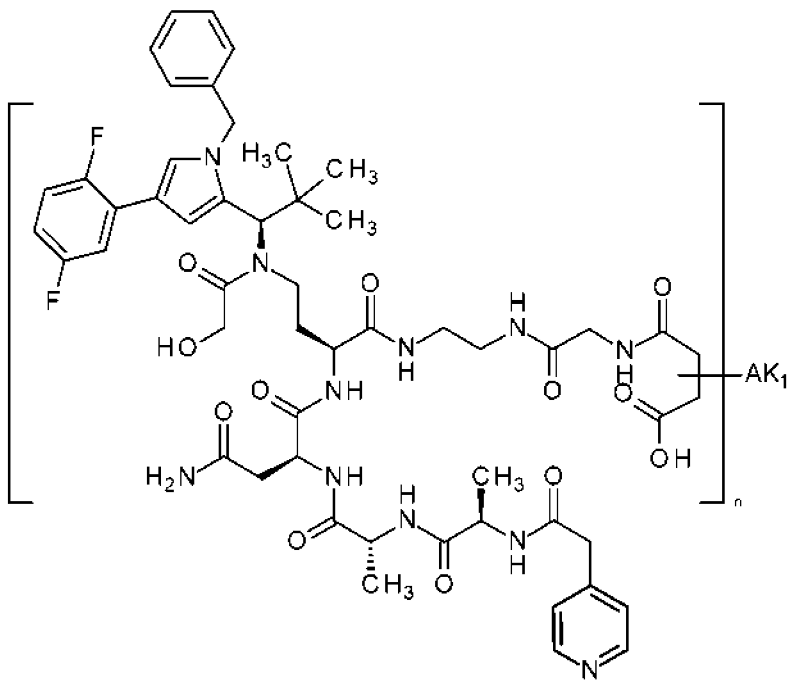

,

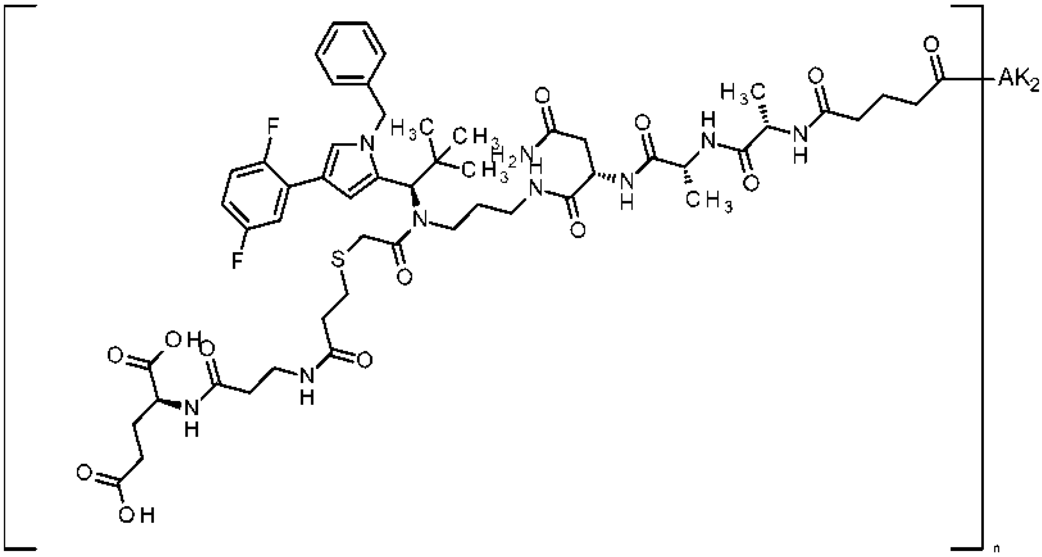

,

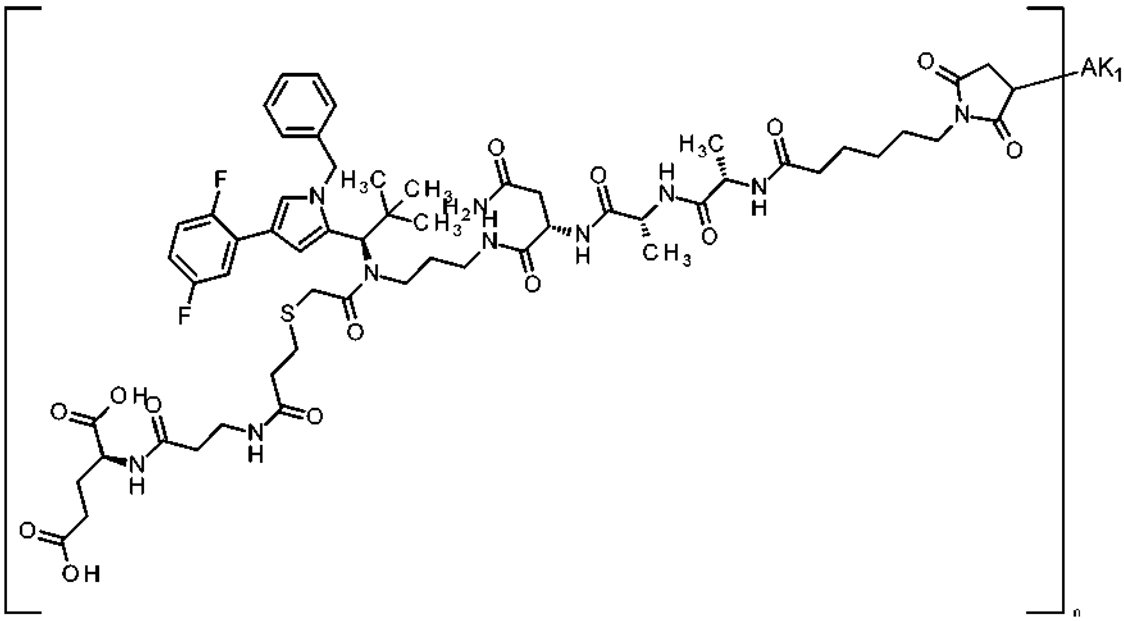

,

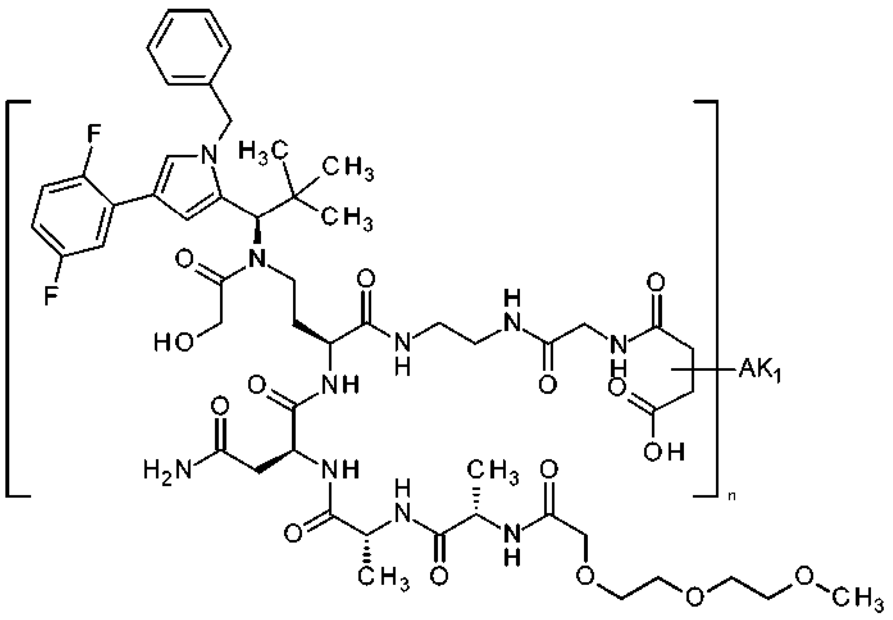

,

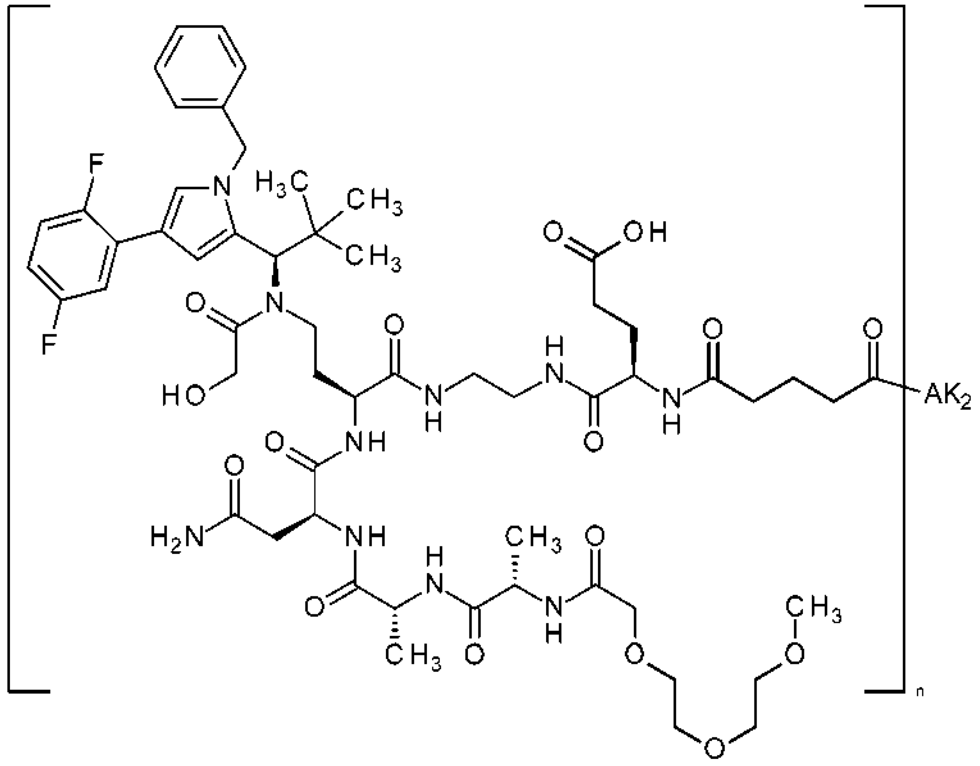

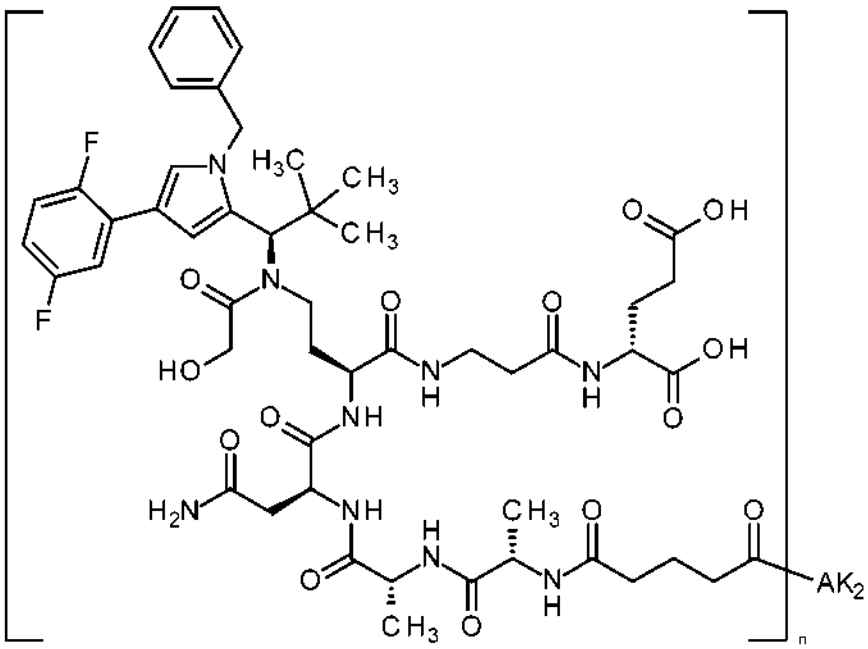

,

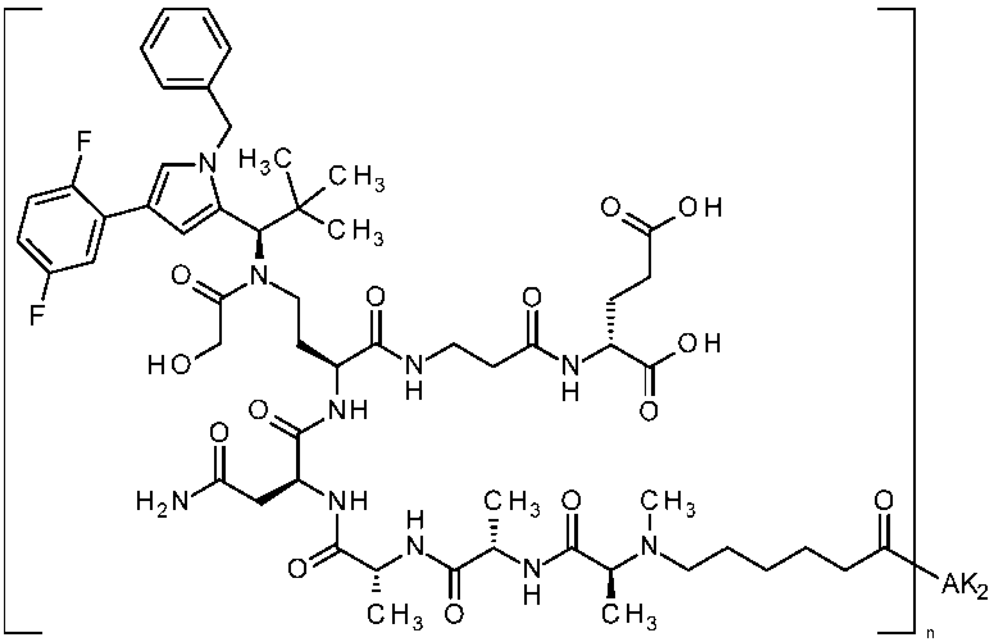

,

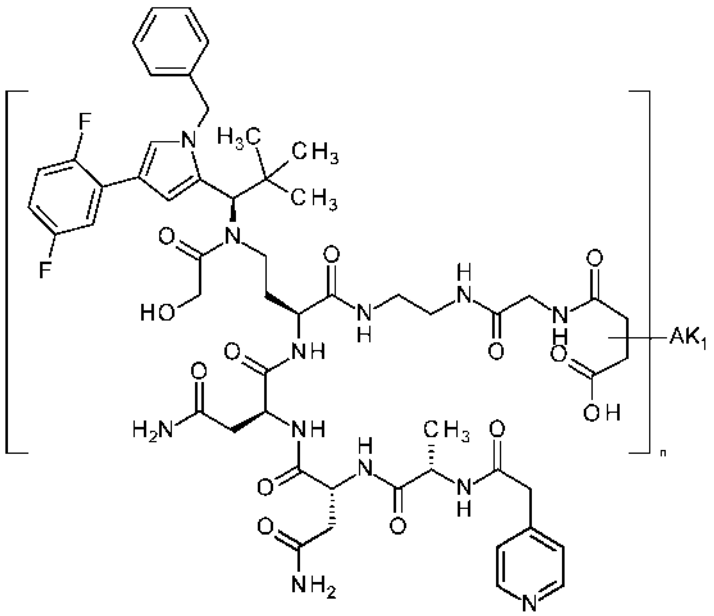

,

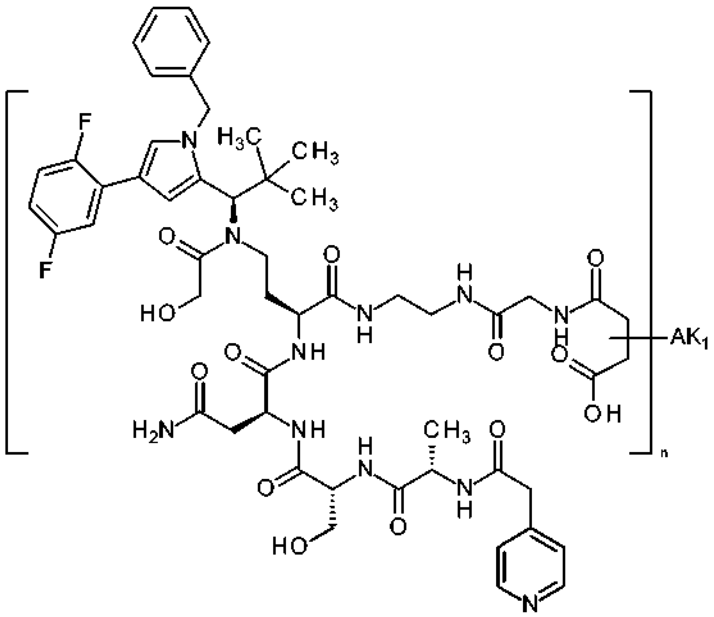

,

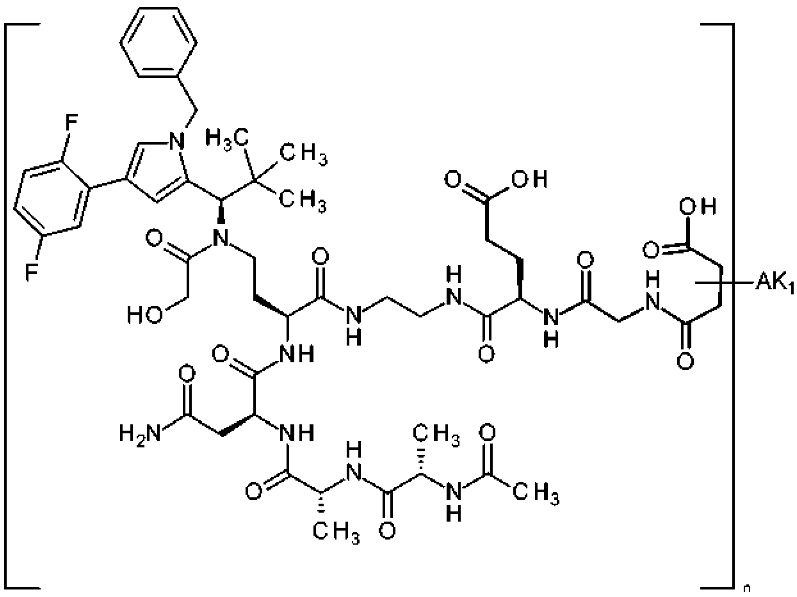

,

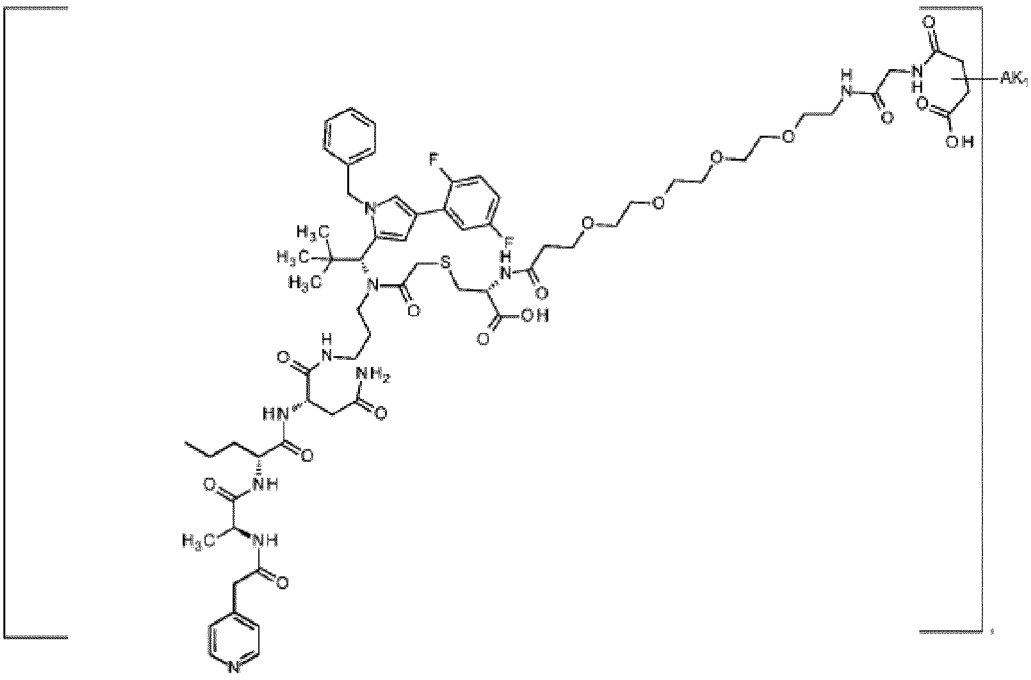

,

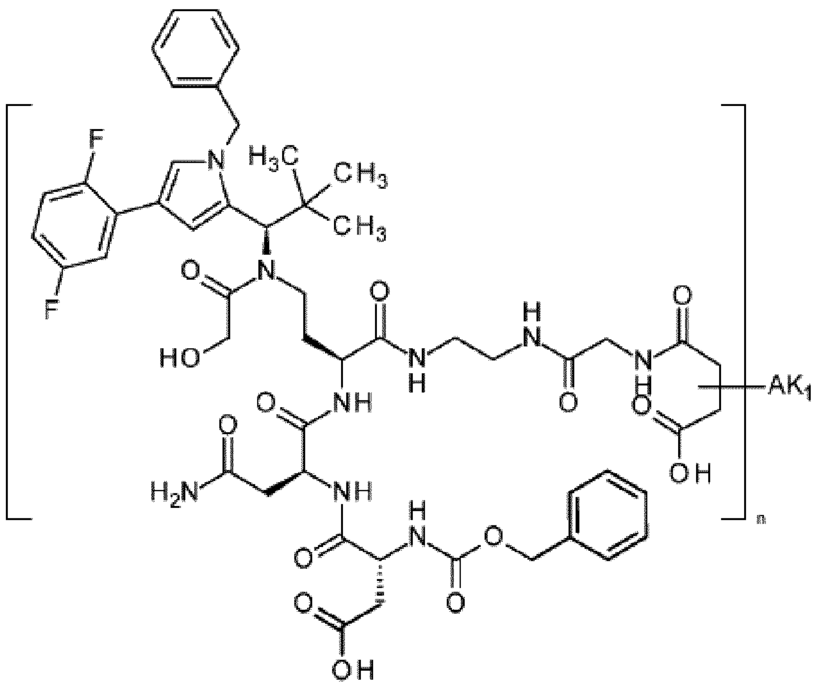

,

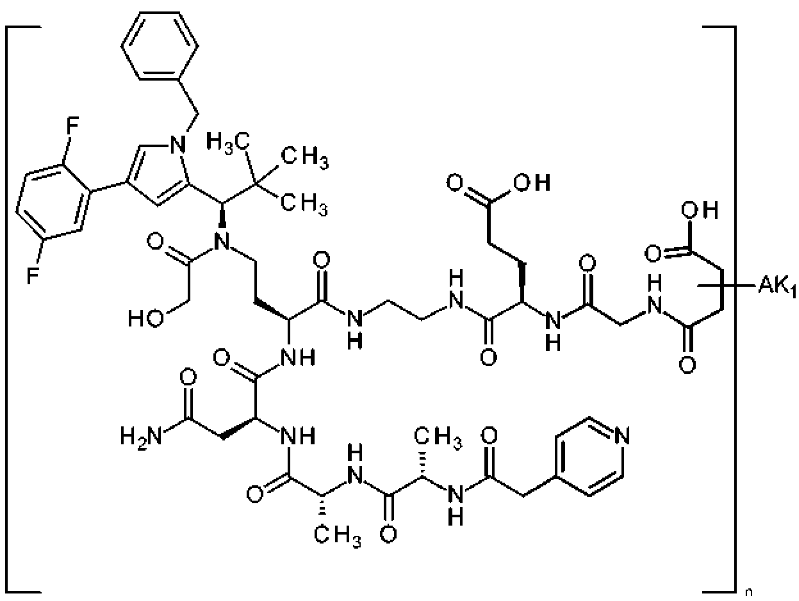

,

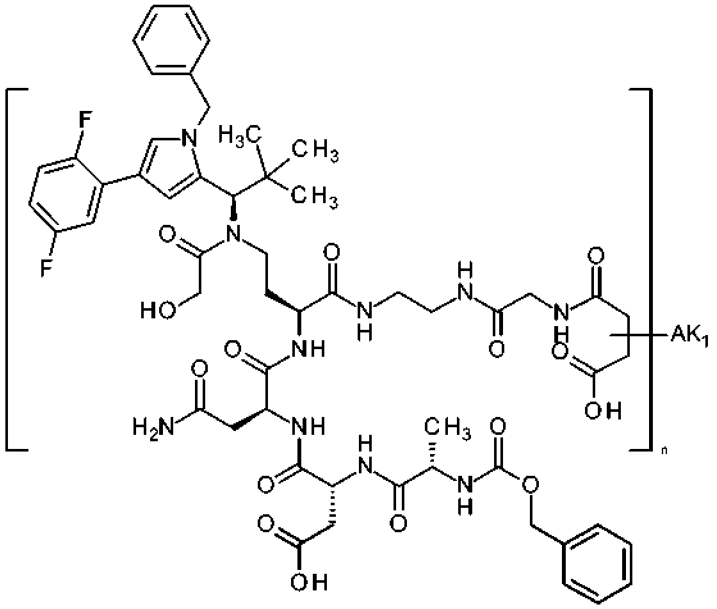

,

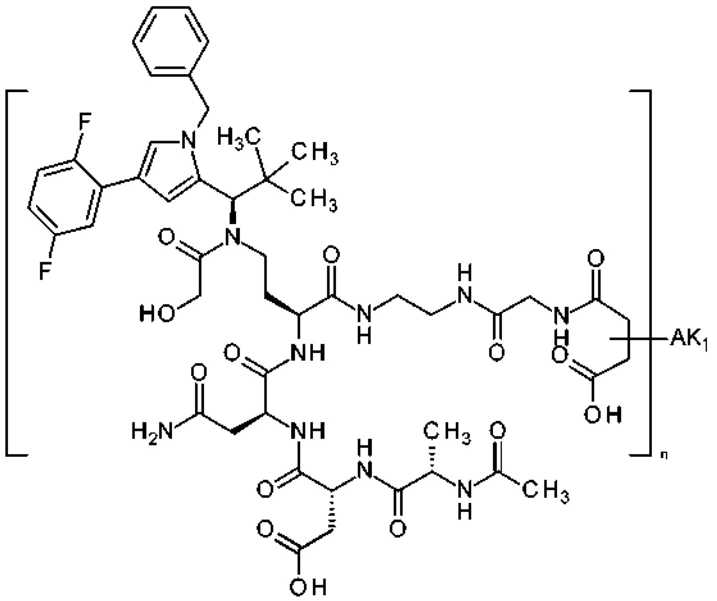

,

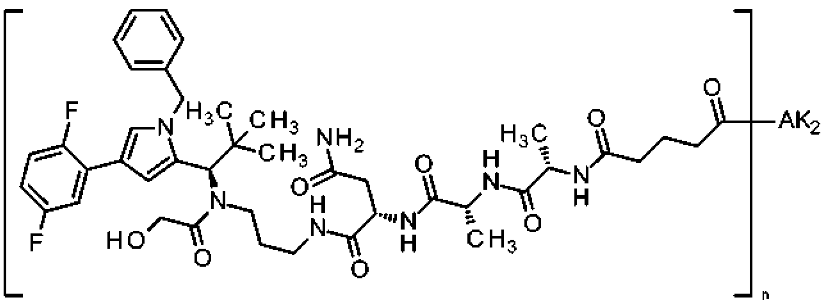

,

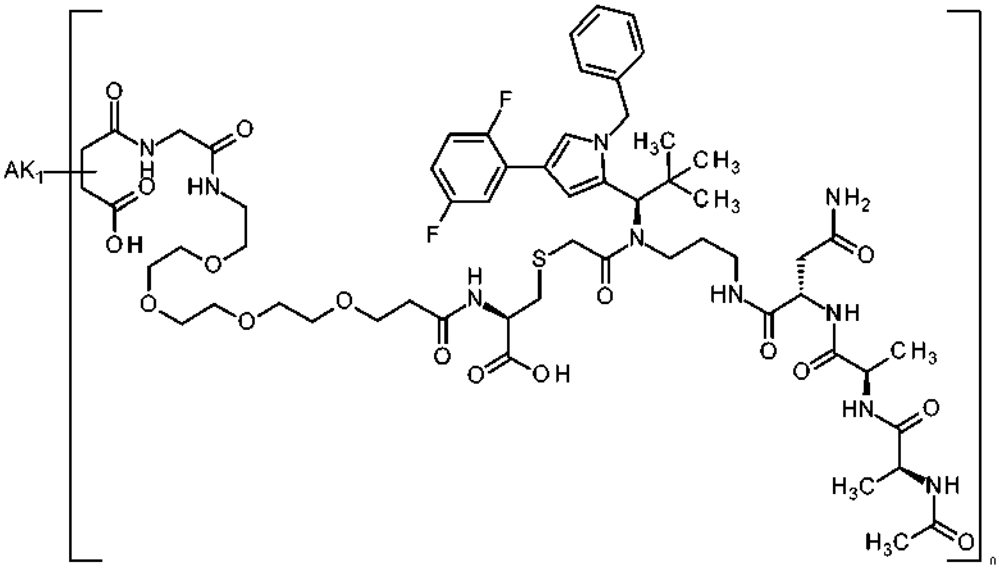

,

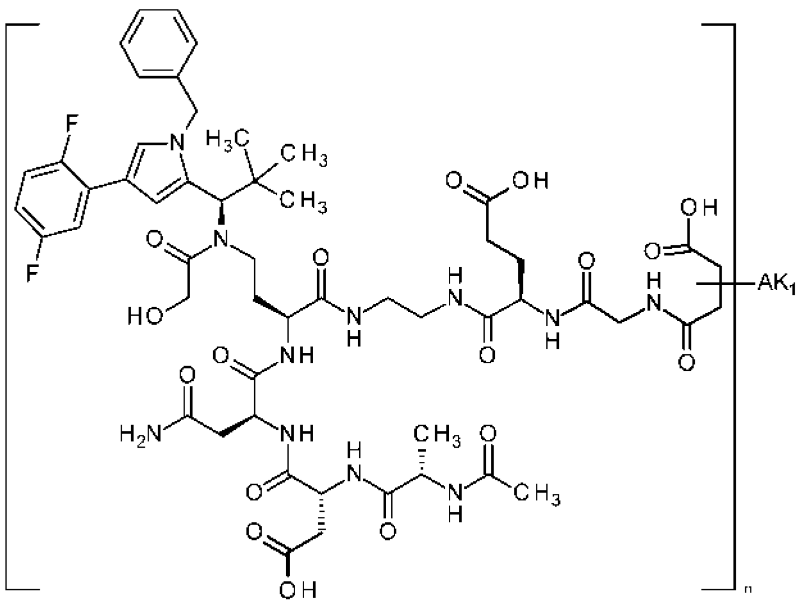

,

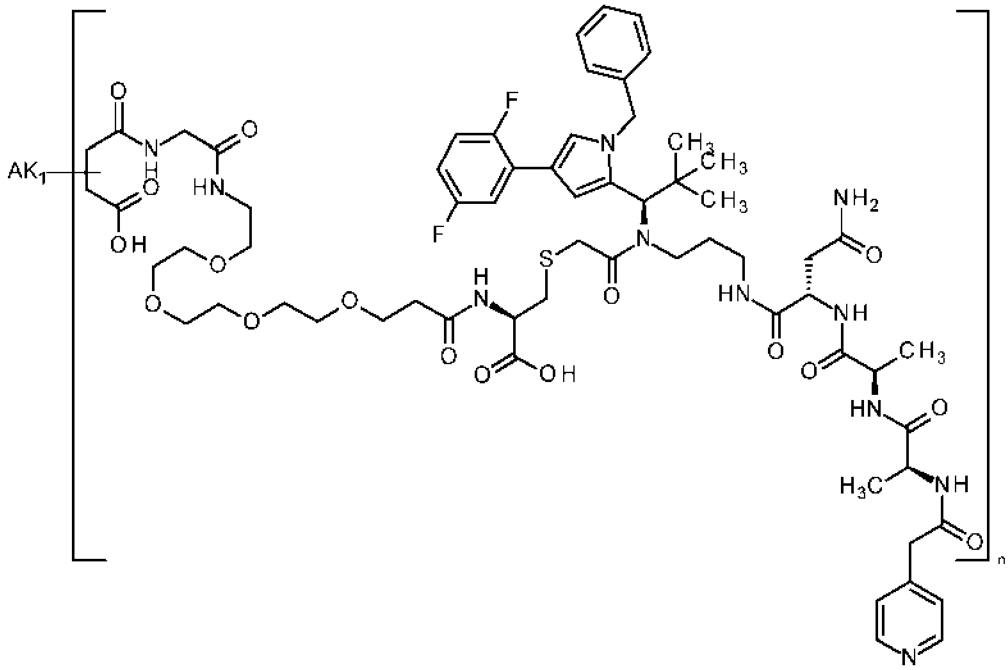

,

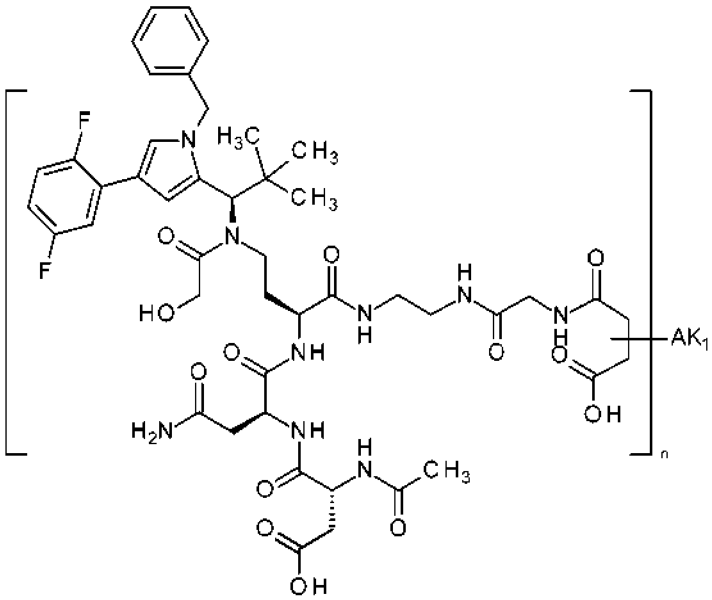

,

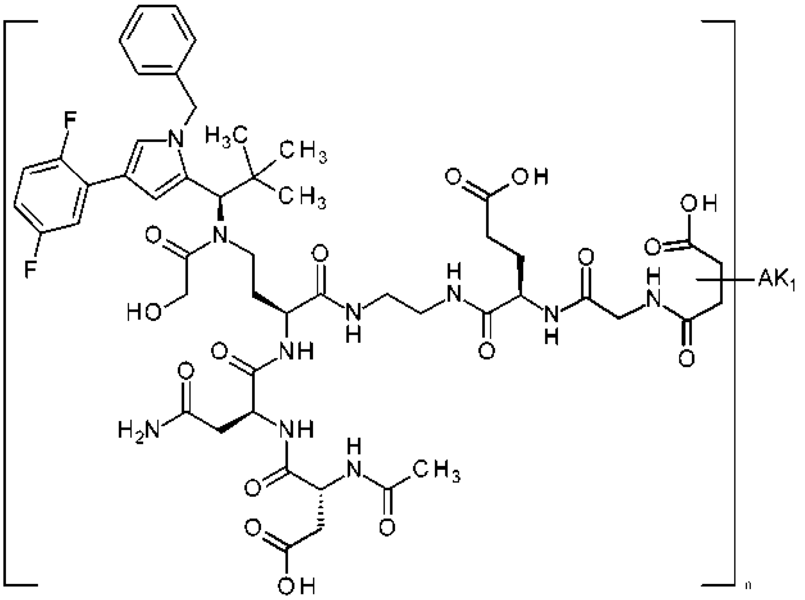

,

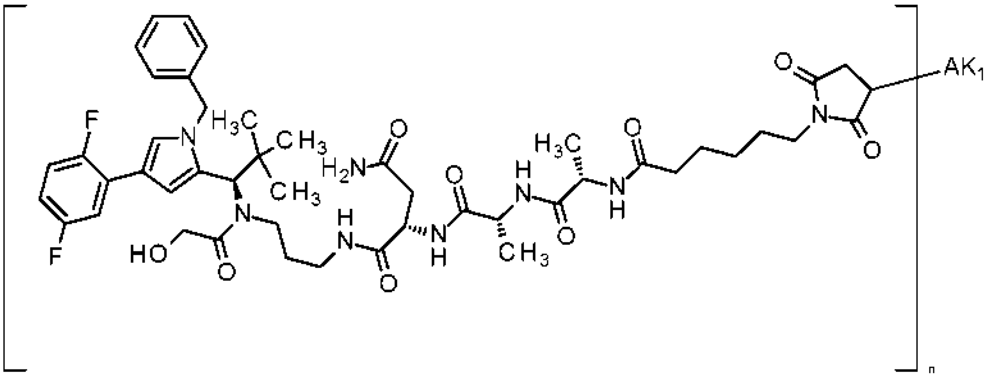

,

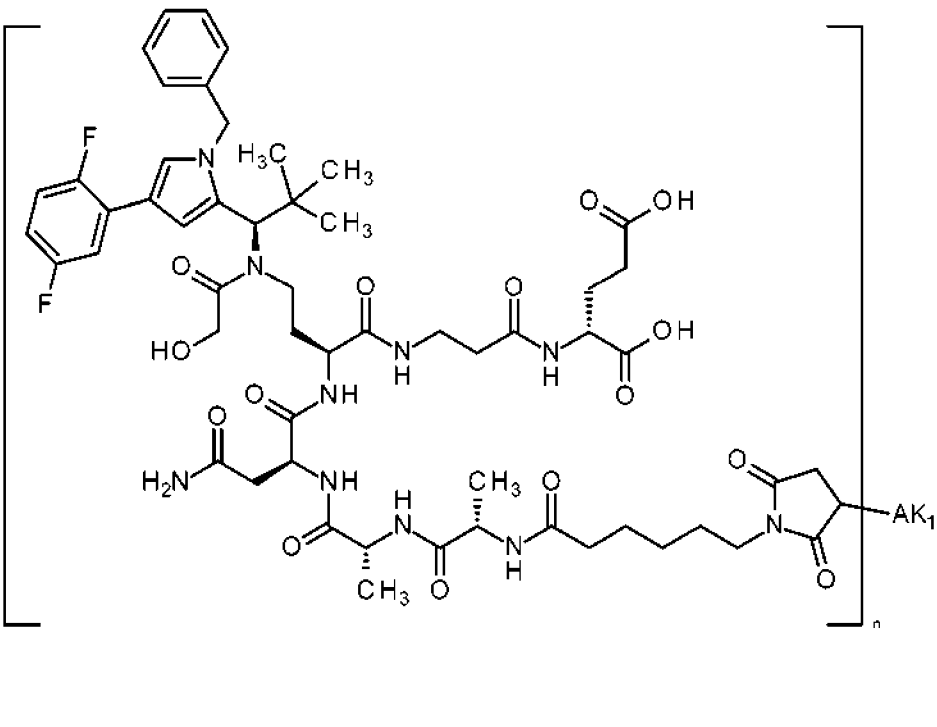

,

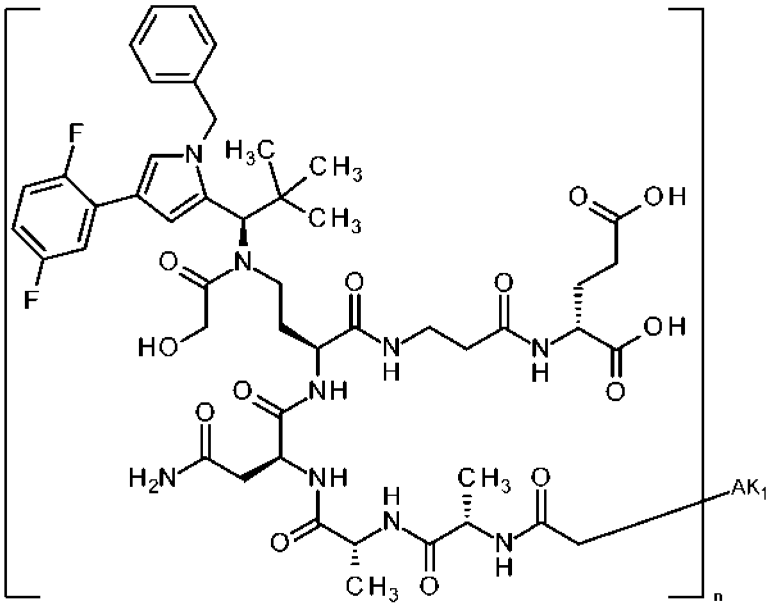

,

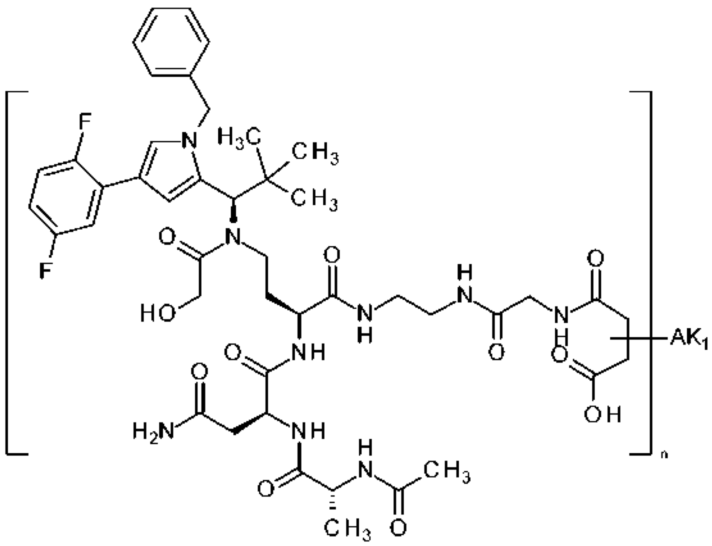

,

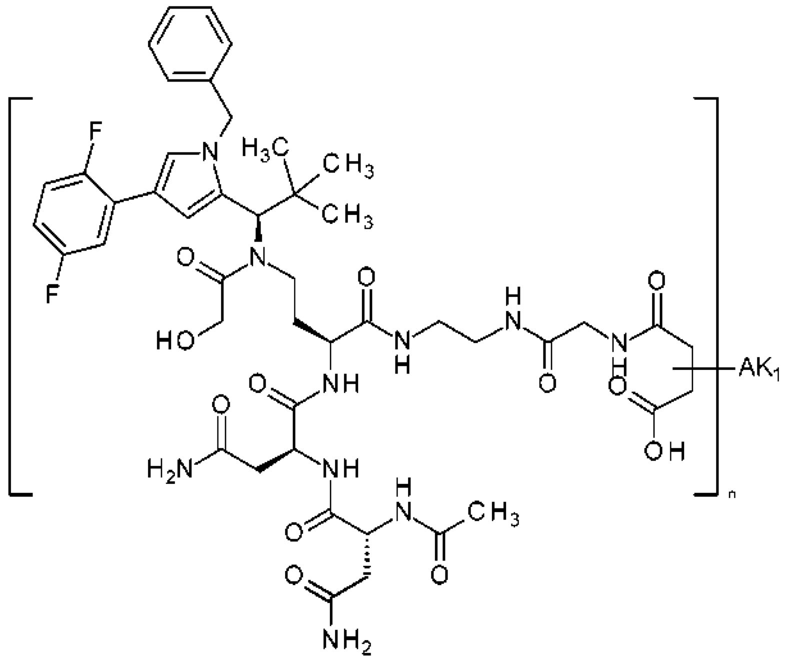

,

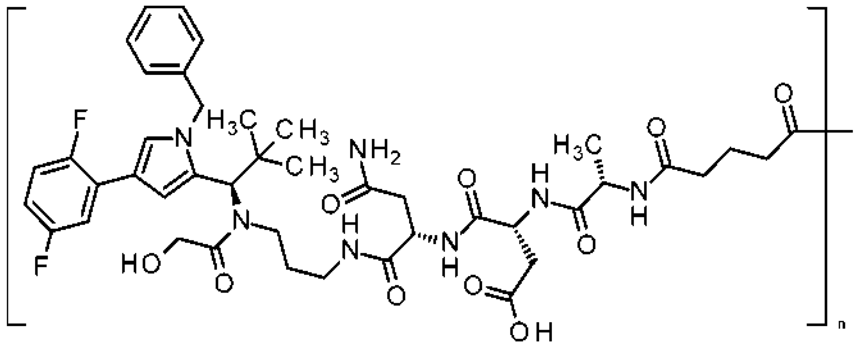

,

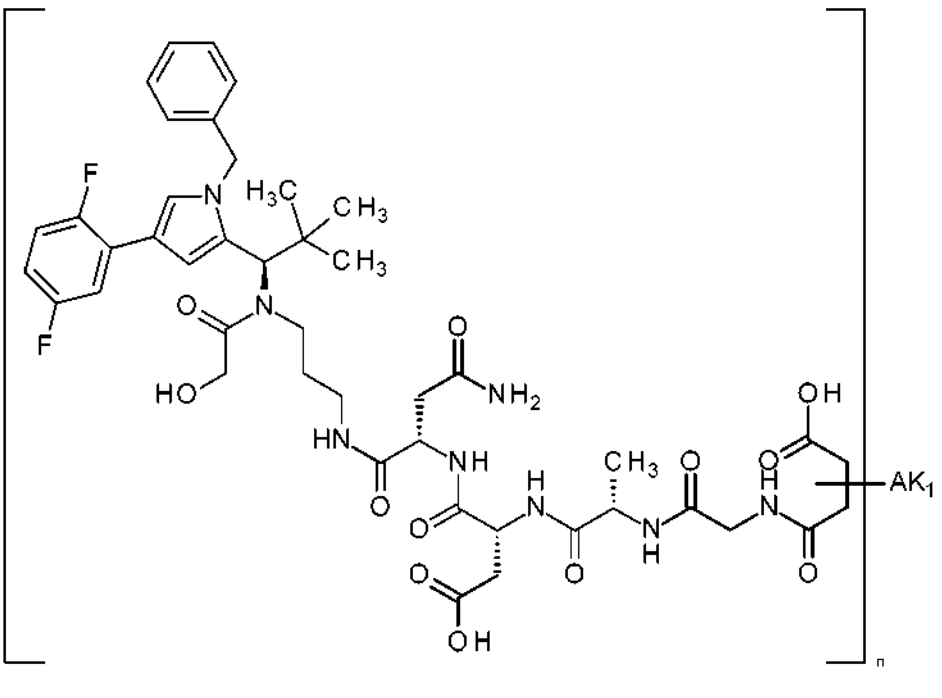

,

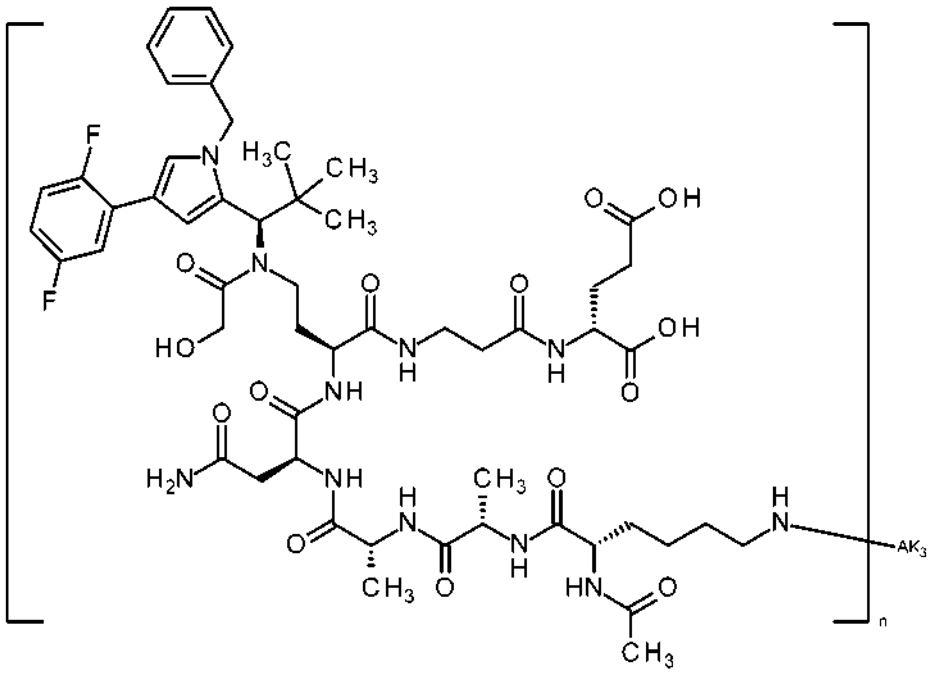

,

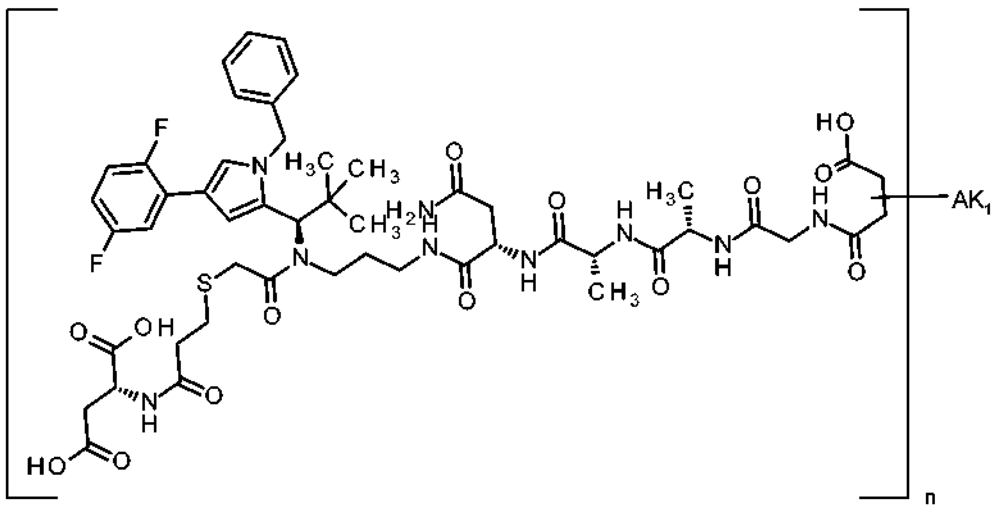

,

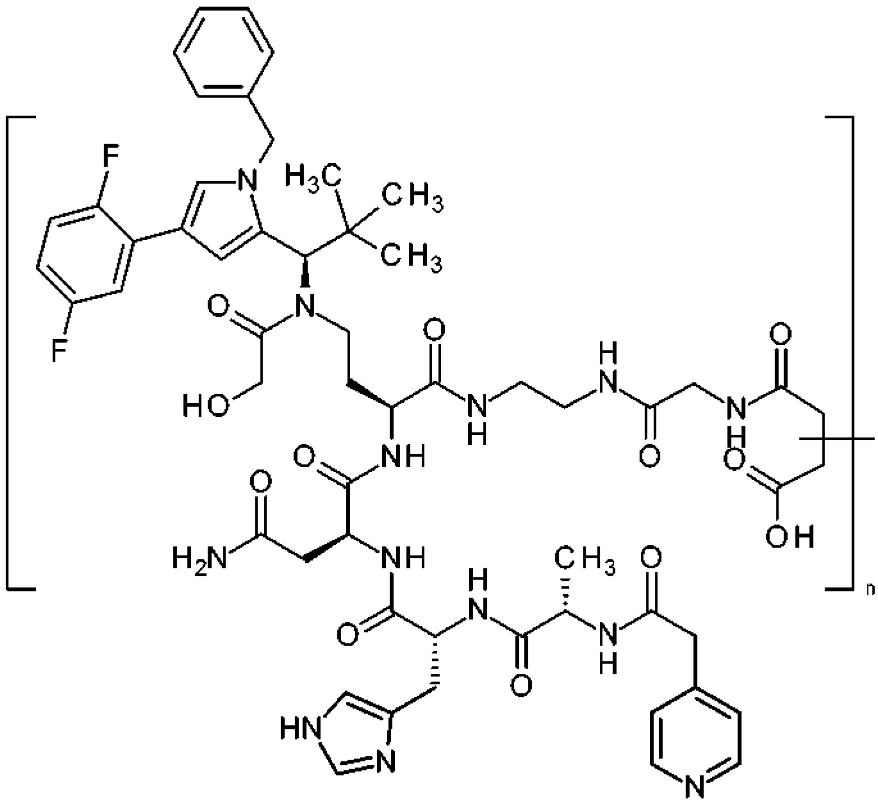

,

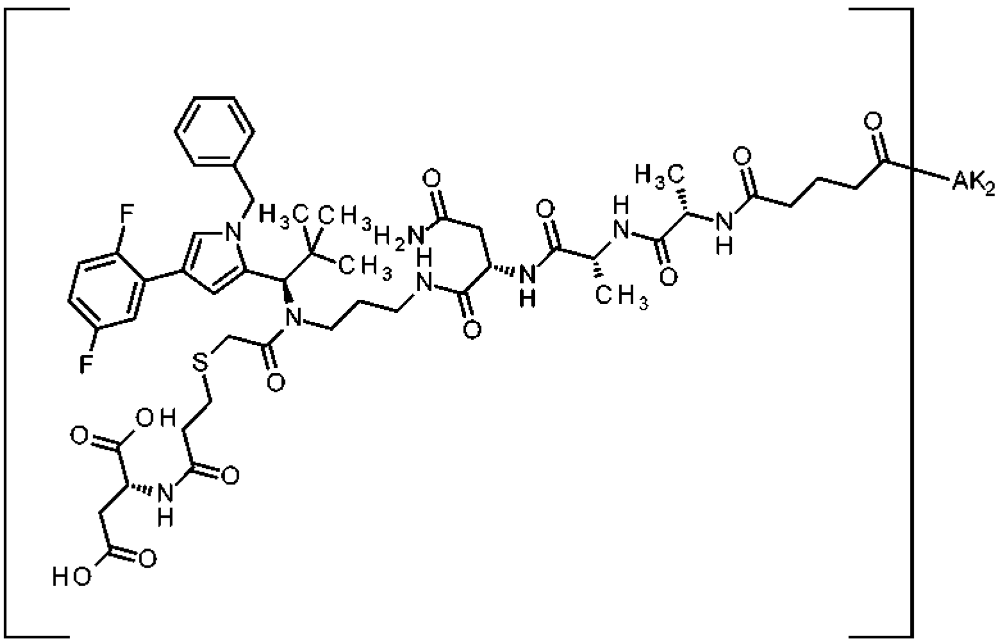

et

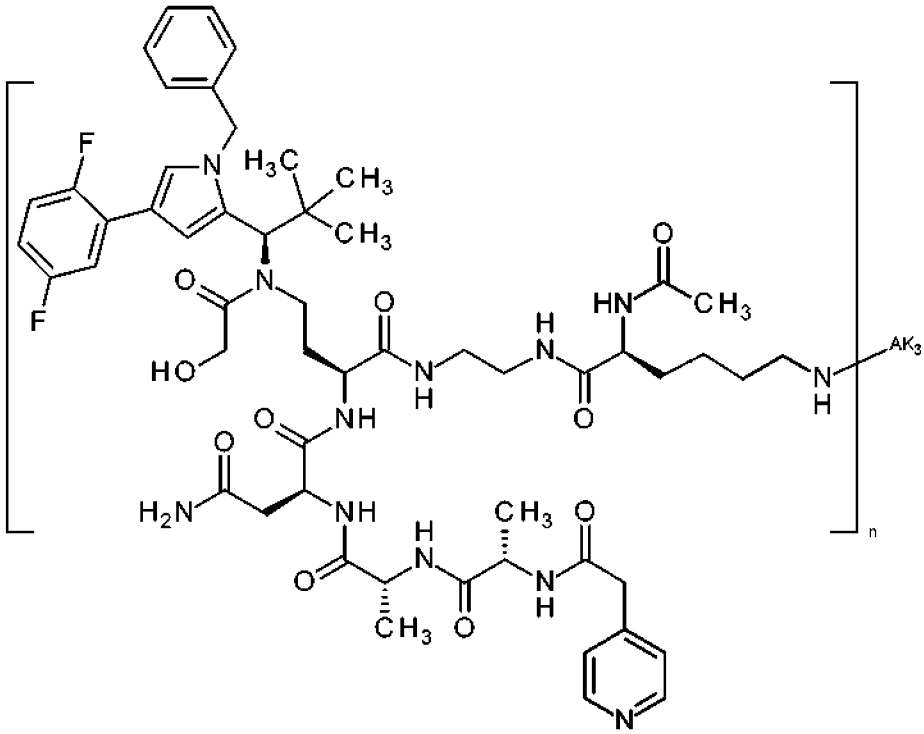

30. Composé ou conjugué selon une ou plusieurs des revendications précédentes, l'anticorps ou le fragment d'anticorps se liant à un antigène se lie à une molécule cible extracellulaire du cancer.

31. Composé ou conjugué selon une ou plusieurs des revendications précédentes, dans lequel l'anticorps ou le fragment d'anticorps se liant à un antigène, après s'être lié à sa molécule cible extracellulaire sur la cellule cible, s'internalise par la liaison de la cellule cible.

32. Composé ou conjugué selon une ou plusieurs des revendications précédentes, dans lequel l'anticorps est un anticorps anti-HER2, un anticorps anti-EGFR, un anticorps anti-B7H3, un anticorps anti-TWEAKR, ou un fragment d'anticorps de celui-ci liant l'antigène.

33. Composé ou conjugué selon la revendication 32, dans lequel l'anticorps anti-TWEAKR est choisi dans le groupe constitué de TPP-7006, TPP-7007, TPP-10336 et TPP-10337, l'anticorps anti-B7H3 est un anticorps choisi dans le groupe constitué de TPP-8382 et TPP-8567, l'anticorps anti-EGFR est le cetuximab (TPP-981), et l'anticorps anti-HER2 est choisi dans le groupe constitué par le trastuzumab et le TPP-1015.

34. Composé ou conjugué selon une ou plusieurs des revendications précédentes, dans lequel l'anticorps (AK, AB, AK1, AK2, AK3)

(i) représente un anticorps anti-EGFR comprenant une région variable de chaîne lourde (VH) comprenant la séquence CDR1 variable de chaîne lourde (H-CDR1), telle que représentée par SEQ ID NO : 2, la séquence CDR2 variable de chaîne lourde (H-CDR2) telle que représentée par SEQ ID NO : 3 et la séquence CDR3 variable de chaîne lourde (H-CDR3) telle que représentée par SEQ ID NO : 4, ainsi qu'une région variable de chaîne légère (VL) comprenant la séquence CDR1 variable de chaîne légère (L-CDR1), telle que représentée par SEQ ID NO : 6, la séquence CDR2 variable de chaîne légère (L-CDR2) telle que représentée par SEQ ID NO : 7, et la séquence CDR3 variable de chaîne légère (L-CDR3) telle que représentée par SEQ ID NO : 8,
(ii) représente un anticorps anti-HER2 comprenant une région variable de chaîne lourde (VH) comprenant la séquence CDR1 variable de chaîne lourde (H-CDR1), telle que représentée par SEQ ID NO : 12, la séquence CDR2 variable de chaîne lourde (H-CDR2) telle que représentée par SEQ ID NO : 13 et la séquence CDR3 variable de chaîne lourde (H-CDR3) telle que représentée par SEQ ID NO : 14, ainsi qu'une région variable de chaîne légère (VL) comprenant la séquence CDR1 variable de chaîne légère (L-CDR1), telle que représentée par SEQ ID NO : 16, la séquence CDR2 variable de chaîne légère (L-CDR2) telle que représentée par SEQ

ID NO : 17, et la séquence CDR3 variable de chaîne légère (L-CDR3) telle que représentée par SEQ ID NO : 18, (iii) représente un anticorps anti-TWEAKR comprenant une région variable de chaîne lourde (VH) comprenant la séquence CDR1 variable de chaîne lourde (H-CDR1), telle que représentée par SEQ ID NO : 22, la séquence CDR2 variable de chaîne lourde (H-CDR2) telle que représentée par SEQ ID NO : 23 et la séquence CDR3 variable de chaîne lourde (H-CDR3) telle que représentée par SEQ ID NO : 24, ainsi qu'une région variable de chaîne légère (VL) comprenant la séquence CDR1 variable de chaîne légère (L-CDR1), telle que représentée par SEQ ID NO : 26, la séquence CDR2 variable de chaîne légère (L-CDR2) telle que représentée par SEQ ID NO : 27, et la séquence CDR3 variable de chaîne légère (L-CDR3) telle que représentée par SEQ ID NO : 28, (iv) représente un anticorps anti-TWEAKR comprenant une région variable de chaîne lourde (VH) comprenant la séquence CDR1 variable de chaîne lourde (H-CDR1), telle que représentée par SEQ ID NO : 32, la séquence CDR2 variable de chaîne lourde (H-CDR2) telle que représentée par SEQ ID NO : 33 et la séquence CDR3 variable de chaîne lourde (H-CDR3) telle que représentée par SEQ ID NO : 34, ainsi qu'une région variable de chaîne légère (VL) comprenant la séquence CDR1 variable de chaîne légère (L-CDR1), telle que représentée par SEQ ID NO : 36, la séquence CDR2 variable de chaîne légère (L-CDR2) telle que représentée par SEQ ID NO : 37, et la séquence CDR3 variable de chaîne légère (L-CDR3) telle que représentée par SEQ ID NO : 38, (v) représente un anticorps anti-TWEAKR comprenant une région variable de chaîne lourde (VH) comprenant la séquence CDR1 variable de chaîne lourde (H-CDR1), telle que représentée par SEQ ID NO : 42, la séquence CDR2 variable de chaîne lourde (H-CDR2) telle que représentée par SEQ ID NO : 43 et la séquence CDR3 variable de chaîne lourde (H-CDR3) telle que représentée par SEQ ID NO : 44, ainsi qu'une région variable de chaîne légère (VL) comprenant la séquence CDR1 variable de chaîne légère (L-CDR1), telle que représentée par SEQ ID NO : 46, la séquence CDR2 variable de chaîne légère (L-CDR2) telle que représentée par SEQ ID NO : 47, et la séquence CDR3 variable de chaîne légère (L-CDR3) telle que représentée par SEQ ID NO : 48, (vi) représente un anticorps anti-TWEAKR comprenant une région variable de chaîne lourde (VH) comprenant la séquence CDR1 variable de chaîne lourde (H-CDR1), telle que représentée par SEQ ID NO : 52, la séquence CDR2 variable de chaîne lourde (H-CDR2) telle que représentée par SEQ ID NO : 53 et la séquence CDR3 variable de chaîne lourde (H-CDR3) telle que représentée par SEQ ID NO : 54, ainsi qu'une région variable de chaîne légère (VL) comprenant la séquence CDR1 variable de chaîne légère (L-CDR1), telle que représentée par SEQ ID NO : 56, la séquence CDR2 variable de chaîne légère (L-CDR2) telle que représentée par SEQ ID NO : 57, et la séquence CDR3 variable de chaîne légère (L-CDR3) telle que représentée par SEQ ID NO : 58, (vii) représente un anticorps anti-TWEAKR comprenant une région variable de chaîne lourde (VH) comprenant la séquence CDR1 variable de chaîne lourde (H-CDR1), telle que représentée par SEQ ID NO : 62, la séquence CDR2 variable de chaîne lourde (H-CDR2) telle que représentée par SEQ ID NO : 63 et la séquence CDR3 variable de chaîne lourde (H-CDR3) telle que représentée par SEQ ID NO : 64, ainsi qu'une région variable de chaîne légère (VL) comprenant la séquence CDR1 variable de chaîne légère (L-CDR1), telle que représentée par SEQ ID NO : 66, la séquence CDR2 variable de chaîne légère (L-CDR2) telle que représentée par SEQ ID NO : 67, et la séquence CDR3 variable de chaîne légère (L-CDR3) telle que représentée par SEQ ID NO : 68, (viii) représente un anticorps anti-HER2 comprenant une région variable de chaîne lourde (VH) comprenant la séquence CDR1 variable de chaîne lourde (H-CDR1), telle que représentée par SEQ ID NO : 72, la séquence CDR2 variable de chaîne lourde (H-CDR2) telle que représentée par SEQ ID NO : 73 et la séquence CDR3 variable de chaîne lourde (H-CDR3) telle que représentée par SEQ ID NO : 74, ainsi qu'une région variable de chaîne légère (VL) comprenant la séquence CDR1 variable de chaîne légère (L-CDR1), telle que représentée par SEQ ID NO : 76, la séquence CDR2 variable de chaîne légère (L-CDR2) telle que représentée par SEQ ID NO : 77, et la séquence CDR3 variable de chaîne légère (L-CDR3) telle que représentée par SEQ ID NO : 78, (ix) représente un anticorps anti-HER2 comprenant une région variable de chaîne lourde (VH) comprenant la séquence CDR1 variable de chaîne lourde (H-CDR1), telle que représentée par SEQ ID NO : 82, la séquence CDR2 variable de chaîne lourde (H-CDR2) telle que représentée par SEQ ID NO : 83 et la séquence CDR3 variable de chaîne lourde (H-CDR3) telle que représentée par SEQ ID NO : 84, ainsi qu'une région variable de chaîne légère (VL) comprenant la séquence CDR1 variable de chaîne légère (L-CDR1), telle que représentée par SEQ ID NO : 86, la séquence CDR2 variable de chaîne légère (L-CDR2) telle que représentée par SEQ ID NO : 87, et la séquence CDR3 variable de chaîne légère (L-CDR3) telle que représentée par SEQ ID NO : 88, (x) représente un anticorps anti-B7H3 comprenant une région variable de chaîne lourde (VH) comprenant la séquence CDR1 variable de chaîne lourde (H-CDR1), telle que représentée par SEQ ID NO : 92, la séquence CDR2 variable de chaîne lourde (H-CDR2) telle que représentée par SEQ ID NO : 93 et la séquence CDR3 variable de chaîne lourde (H-CDR3) telle que représentée par SEQ ID NO : 94, ainsi qu'une région variable de chaîne légère (VL) comprenant la séquence CDR1 variable de chaîne légère (L-CDR1), telle que représentée par SEQ ID NO : 96, la séquence CDR2 variable de chaîne légère (L-CDR2) telle que représentée par SEQ ID NO : 97, et la séquence CDR3 variable de chaîne légère (L-CDR3) telle que représentée par SEQ ID NO : 98, (xi) représente un anticorps anti-B7H3 comprenant une région variable de chaîne lourde (VH) comprenant la

séquence CDR1 variable de chaîne lourde (H-CDR1), telle que représentée par SEQ ID NO : 102, la séquence CDR2 variable de chaîne lourde (H-CDR2) telle que représentée par SEQ ID NO : 103 et la séquence CDR3 variable de chaîne lourde (H-CDR3) telle que représentée par SEQ ID NO : 104, ainsi qu'une région variable de chaîne légère (VL) comprenant la séquence CDR1 variable de chaîne légère (L-CDR1), telle que représentée par SEQ ID NO : 106, la séquence CDR2 variable de chaîne légère (L-CDR2) telle que représentée par SEQ ID NO : 107, et la séquence CDR3 variable de chaîne légère (L-CDR3) telle que représentée par SEQ ID NO : 108,

(xii) représente un anticorps anti-TWEAKR comprenant une région variable de chaîne lourde (VH) comprenant la séquence CDR1 variable de chaîne lourde (H-CDR1), telle que représentée par SEQ ID NO : 112, la séquence CDR2 variable de chaîne lourde (H-CDR2) telle que représentée par SEQ ID NO : 113 et la séquence CDR3 variable de chaîne lourde (H-CDR3) telle que représentée par SEQ ID NO : 114, ainsi qu'une région variable de chaîne légère (VL) comprenant la séquence CDR1 variable de chaîne légère (L-CDR1), telle que représentée par SEQ ID NO : 116, la séquence CDR2 variable de chaîne légère (L-CDR2) telle que représentée par SEQ ID NO : 117, et la séquence CDR3 variable de chaîne légère (L-CDR3) telle que représentée par SEQ ID NO : 118,

(xiii) représente un anticorps anti-TWEAKR comprenant une région variable de chaîne lourde (VH) comprenant la séquence CDR1 variable de chaîne lourde (H-CDR1), telle que représentée par SEQ ID NO : 122, la séquence CDR2 variable de chaîne lourde (H-CDR2) telle que représentée par SEQ ID NO : 123 et la séquence CDR3 variable de chaîne lourde (H-CDR3) telle que représentée par SEQ ID NO : 124, ainsi qu'une région variable de chaîne légère (VL) comprenant la séquence CDR1 variable de chaîne légère (L-CDR1), telle que représentée par SEQ ID NO : 126, la séquence CDR2 variable de chaîne légère (L-CDR2) telle que représentée par SEQ ID NO : 127, et la séquence CDR3 variable de chaîne légère (L-CDR3) telle que représentée par SEQ ID NO : 128,

(xiv) représente un anticorps anti-TWEAKR comprenant une région variable de chaîne lourde (VH) comprenant la séquence CDR1 variable de chaîne lourde (H-CDR1), telle que représentée par SEQ ID NO : 132, la séquence CDR2 variable de chaîne lourde (H-CDR2) telle que représentée par SEQ ID NO : 133 et la séquence CDR3 variable de chaîne lourde (H-CDR3) telle que représentée par SEQ ID NO : 134, ainsi qu'une région variable de chaîne légère (VL) comprenant la séquence CDR1 variable de chaîne légère (L-CDR1), telle que représentée par SEQ ID NO : 136, la séquence CDR2 variable de chaîne légère (L-CDR2) telle que représentée par SEQ ID NO : 137, et la séquence CDR3 variable de chaîne légère (L-CDR3) telle que représentée par SEQ ID NO : 138,

(xv) représente un anticorps anti-TWEAKR comprenant une région variable de chaîne lourde (VH) comprenant la séquence CDR1 variable de chaîne lourde (H-CDR1), telle que représentée par SEQ ID NO : 142, la séquence CDR2 variable de chaîne lourde (H-CDR2) telle que représentée par SEQ ID NO : 143 et la séquence CDR3 variable de chaîne lourde (H-CDR3) telle que représentée par SEQ ID NO : 144, ainsi qu'une région variable de chaîne légère (VL) comprenant la séquence CDR1 variable de chaîne légère (L-CDR1), telle que représentée par SEQ ID NO : 146, la séquence CDR2 variable de chaîne légère (L-CDR2) telle que représentée par SEQ ID NO : 147, et la séquence CDR3 variable de chaîne légère (L-CDR3) telle que représentée par SEQ ID NO : 148,

(xvi) représente un anticorps anti-TWEAKR comprenant une région variable de chaîne lourde (VH) comprenant la séquence CDR1 variable de chaîne lourde (H-CDR1), telle que représentée par SEQ ID NO : 152, la séquence CDR2 variable de chaîne lourde (H-CDR2) telle que représentée par SEQ ID NO : 153 et la séquence CDR3 variable de chaîne lourde (H-CDR3) telle que représentée par SEQ ID NO : 154, ainsi qu'une région variable de chaîne légère (VL) comprenant la séquence CDR1 variable de chaîne légère (L-CDR1), telle que représentée par SEQ ID NO : 156, la séquence CDR2 variable de chaîne légère (L-CDR2) telle que représentée par SEQ ID NO : 157, et la séquence CDR3 variable de chaîne légère (L-CDR3) telle que représentée par SEQ ID NO : 158,

ou représente un fragment liant l'antigène de cet anticorps.

**35.** Composé ou conjugué selon une ou plusieurs des revendications précédentes, (AK, AB, AK1, AK2, AK3)

(i) représente un anticorps anti-EGFR comprenant une région variable de chaîne lourde (VH) correspondant à SEQ ID NO: 1 ainsi qu'une région variable de chaîne légère (VL) correspondant à SEQ ID NO: 5,

(ii) représente un anticorps anti-HER2 comprenant une région variable de chaîne lourde (VH) correspondant à SEQ ID NO: 11 ainsi qu'une région variable de chaîne légère (VL) correspondant à SEQ ID NO: 15,

(iii) représente un anticorps anti-TWEAKR comprenant une région variable de chaîne lourde (VH) correspondant à SEQ ID NO: 21 ainsi qu'une région variable de chaîne légère (VL) correspondant à SEQ ID NO: 25,

(iv) représente un anticorps anti-TWEAKR comprenant une région variable de chaîne lourde (VH) correspondant à SEQ ID NO: 31 ainsi qu'une région variable de chaîne légère (VL) correspondant à SEQ ID NO: 35,
(v) représente un anticorps anti-TWEAKR comprenant une région variable de chaîne lourde (VH) correspondant à SEQ ID NO: 41 ainsi qu'une région variable de chaîne légère (VL) correspondant à SEQ ID NO: 45,
(vi) représente un anticorps anti-TWEAKR comprenant une région variable de chaîne lourde (VH) correspondant à SEQ ID NO: 51 ainsi qu'une région variable de chaîne légère (VL) correspondant à SEQ ID NO: 55,
(vii) représente un anticorps anti-TWEAKR comprenant une région variable de chaîne lourde (VH) correspondant à SEQ ID NO: 61 ainsi qu'une région variable de chaîne légère (VL) correspondant à SEQ ID NO: 65,
(viii) représente un anticorps anti-HER2 comprenant une région variable de chaîne lourde (VH) correspondant à SEQ ID NO: 71 ainsi qu'une région variable de chaîne légère (VL) correspondant à SEQ ID NO: 75,
(ix) représente un anticorps anti-HER2 comprenant une région variable de chaîne lourde (VH) correspondant à SEQ ID NO: 81 ainsi qu'une région variable de chaîne légère (VL) correspondant à SEQ ID NO: 85,
(x) représente un anticorps anti-B7H3 comprenant une région variable de chaîne lourde (VH) correspondant à SEQ ID NO: 91 ainsi qu'une région variable de chaîne légère (VL) correspondant à SEQ ID NO: 95,
(xi) représente un anticorps anti-B7H3 comprenant une région variable de chaîne lourde (VH) correspondant à SEQ ID NO: 101 ainsi qu'une région variable de chaîne légère (VL) correspondant à SEQ ID NO: 105,
(xii) représente un anticorps anti-TWEAKR comprenant une région variable de chaîne lourde (VH) correspondant à SEQ ID NO: 111 ainsi qu'une région variable de chaîne légère (VL) correspondant à SEQ ID NO: 115,
(xiii) représente un anticorps anti-TWEAKR comprenant une région variable de chaîne lourde (VH) correspondant à SEQ ID NO: 121 ainsi qu'une région variable de chaîne légère (VL) correspondant à SEQ ID NO: 125,
(xiv) représente un anticorps anti-TWEAKR comprenant une région variable de chaîne lourde (VH) correspondant à SEQ ID NO: 131 ainsi qu'une région variable de chaîne légère (VL) correspondant à SEQ ID NO: 135,
(xv) représente un anticorps anti-TWEAKR comprenant une région variable de chaîne lourde (VH) correspondant à SEQ ID NO: 141 ainsi qu'une région variable de chaîne légère (VL) correspondant à SEQ ID NO: 145, ou
(xvi) représente un anticorps anti-TWEAKR comprenant une région variable de chaîne lourde (VH) correspondant à SEQ ID NO: 151 ainsi qu'une région variable de chaîne légère (VL) correspondant à SEQ ID NO: 155,

ou représente un fragment de cet anticorps liant l'antigène.

**36.** Composé ou conjugué selon une ou plusieurs des revendications précédentes, dans lequel l'anticorps (AK, AB, AK1, AK2, AK3)

(i) représente un anticorps anti-EGFR comprenant une région de chaîne lourde correspondant à SEQ ID NO: 9 ainsi qu'une région de chaîne légère correspondant à SEQ ID NO: 10,
(ii) représente un anticorps anti-HER2 comprenant une région de chaîne lourde correspondant à SEQ ID NO: 19 ainsi qu'une région de chaîne légère correspondant à SEQ ID NO: 20,
(iii) représente un anticorps anti-TWEAKR comprenant une région de chaîne lourde correspondant à SEQ ID NO: 29 ainsi qu'une région de chaîne légère correspondant à SEQ ID NO: 30,
(iv) représente un anticorps anti-TWEAKR comprenant une région de chaîne lourde correspondant à SEQ ID NO: 39 ainsi qu'une région de chaîne légère correspondant à SEQ ID NO: 40,
(v) représente un anticorps anti-TWEAKR comprenant une région de chaîne lourde correspondant à SEQ ID NO: 49 ainsi qu'une région de chaîne légère correspondant à SEQ ID NO: 50,
(vi) représente un anticorps anti-TWEAKR comprenant une région de chaîne lourde correspondant à SEQ ID NO: 59 ainsi qu'une région de chaîne légère correspondant à SEQ ID NO: 60,
(vii) représente un anticorps anti-TWEAKR comprenant une région de chaîne lourde correspondant à SEQ ID NO: 69 ainsi qu'une région de chaîne légère correspondant à SEQ ID NO: 70,
(viii) représente un anticorps anti-TWEAKR comprenant une région de chaîne lourde correspondant à SEQ ID NO: 79 ainsi qu'une région de chaîne légère correspondant à SEQ ID NO: 80,
(ix) représente un anticorps anti-HER2 comprenant une région de chaîne lourde correspondant à SEQ ID NO: 89 ainsi qu'une région de chaîne légère correspondant à SEQ ID NO: 90,
(x) représente un anticorps anti-B7H3 comprenant une région de chaîne lourde correspondant à SEQ ID NO: 99 ainsi qu'une région de chaîne légère correspondant à SEQ ID NO: 100,
(xi) représente un anticorps anti-B7H3 comprenant une région de chaîne lourde correspondant à SEQ ID NO: 109 ainsi qu'une région de chaîne légère correspondant à SEQ ID NO: 110,
(xii) représente un anticorps anti-TWEAKR comprenant une région de chaîne lourde correspondant à SEQ ID NO: 119 ainsi qu'une région de chaîne légère correspondant à SEQ ID NO: 120,
(xiii) représente un anticorps anti-TWEAKR comprenant une région de chaîne lourde correspondant à SEQ ID NO: 129 ainsi qu'une région de chaîne légère correspondant à SEQ ID NO: 130,

(xiv) représente un anticorps anti-TWEAKR comprenant une région de chaîne lourde correspondant à SEQ ID NO: 139 ainsi qu'une région de chaîne légère correspondant à SEQ ID NO: 140,
(xv) représente un anticorps anti-TWEAKR comprenant une région de chaîne lourde correspondant à SEQ ID NO: 149 ainsi qu'une région de chaîne légère correspondant à SEQ ID NO: 150, ou
(xvi) représente un anticorps anti-TWEAKR comprenant une région de chaîne lourde correspondant à SEQ ID NO: 159 ainsi qu'une région de chaîne légère correspondant à SEQ ID NO: 160,

ou représente un fragment de cet anticorps liant l'antigène.

**37.** Composition pharmaceutique comprenant un composé selon une ou plusieurs des revendications précédentes en combinaison avec un excipient inerte, non toxique, pharmaceutiquement acceptable.

**38.** Composé selon une ou plusieurs des revendications précédentes pour une utilisation dans une méthode de traitement et/ou de prophylaxie d'une maladie.

**39.** Composé selon une ou plusieurs des revendications précédentes pour une utilisation dans une méthode de traitement des maladies hyperprolifératives et/ou angiogéniques.

Fig. 1/4

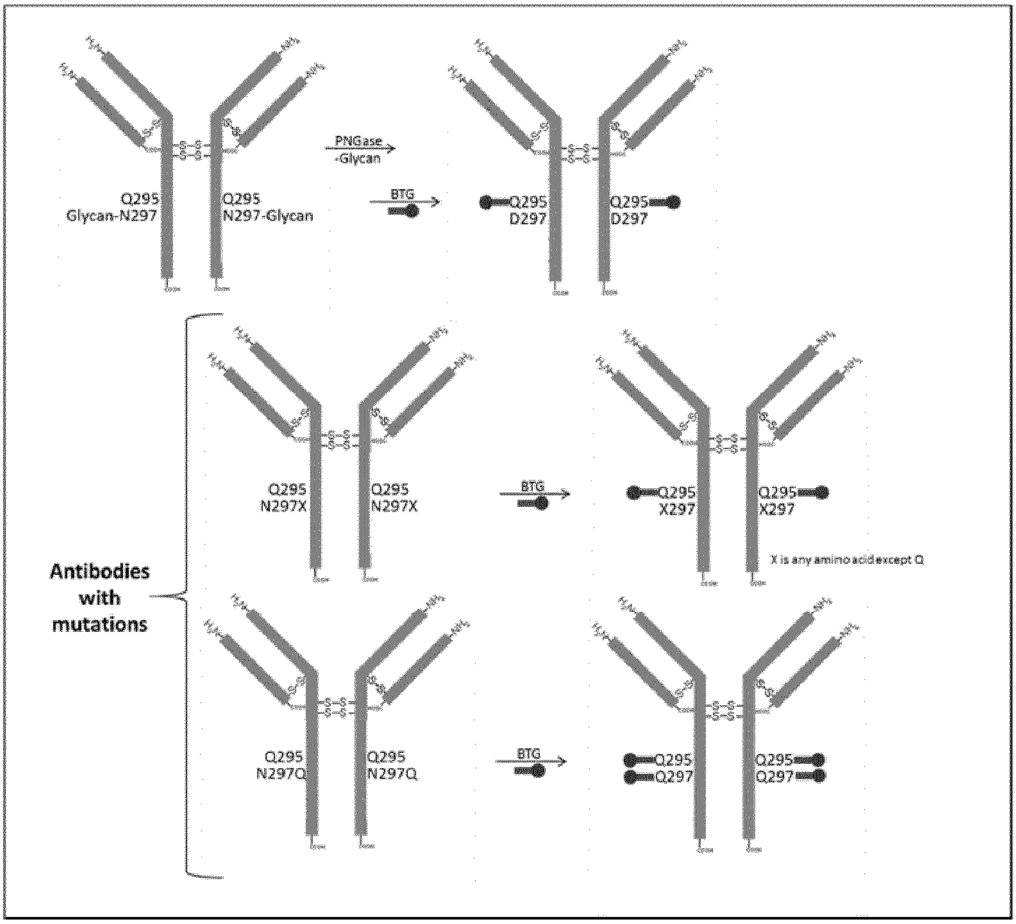
PNGase
-Glycan
BTG
Q295
Glycan-N297
Q295
N297-Glycan
Q295
D297
Q295
D297
Antibodies
with
mutations
Q295
N297X
Q295
N297X
Q295
X297
Q295
X297
X is any amino acid except Q
Q295
N297Q
Q295
N297Q
Q295
Q297
Q295
Q297

**Fig. 2/4**

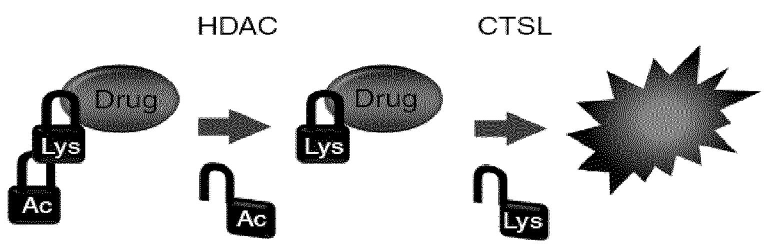

## Fig. 3/4

```
>TPP-981 VH (PRT)
QVQLKQSGPGLVQPSQSLSITCTVSGFSLTNYGVHWVRQSPGKGLEWLGVIWSGGNTDYNTPFTSRLSINKDNSKSQVFF
                              |---|              |----HCDR2-----|
                              HCDR1

KMNSLQSNDTAIYYCARALTYYDYEFAYWGQGTLVTVSA
                 |--HCDR3--|


>TPP-981 VL (PRT)
DILLTQSPVILSVSPGERVSFSCRASQSIGTNIHWYQQRTNGSPRLLIKYASESISGIPSRFSGSGSGTDFTLSINSVES
                       |--LCDR1--|               |-----|
                                                 LCDR2

EDIADYYCQQNNNWPTTFGAGTKLELK
        |-LCDR3-|


>TPP-981 Heavy Chain (PRT)
QVQLKQSGPGLVQPSQSLSITCTVSGFSLTNYGVHWVRQSPGKGLEWLGVIWSGGNTDYNTPFTSRLSINKDNSKSQVFF
|---------------------------------------------------------VH--------------------
                              |---|              |----HCDR2-----|
                              HCDR1

KMNSLQSNDTAIYYCARALTYYDYEFAYWGQGTLVTVSAASTKGPSVFPLAPSSKSTSGGTAALGCLVKDYFPEPVTVSW
--------------------------------------|
                 |--HCDR3--|


NSGALTSGVHTFPAVLQSSGLYSLSSVVTVPSSSLGTQTYICNVNHKPSNTKVDKRVEPKSCDKTHTCPPCPAPELLGGP


SVFLFPPKPKDTLMISRTPEVTCVVVDVSHEDPEVKFNWYVDGVEVHNAKTKPREEQYNSTYRVVSVLTVLHQDWLNGKE


YKCKVSNKALPAPIEKTISKAKGQPREPQVYTLPPSREEMTKNQVSLTCLVKGFYPSDIAVEWESNGQPENNYKTTPPVL


DSDGSFFLYSKLTVDKSRWQQGNVFSCSVMHEALHNHYTQKSLSLSPGK


>TPP-981 Light Chain (PRT)
DILLTQSPVILSVSPGERVSFSCRASQSIGTNIHWYQQRTNGSPRLLIKYASESISGIPSRFSGSGSGTDFTLSINSVES
|---------------------------------------------------VL--------------------------
                       |--LCDR1--|               |-----|
                                                 LCDR2

EDIADYYCQQNNNWPTTFGAGTKLELKRTVAAPSVFIFPPSDEQLKSGTASVVCLLNNFYPREAKVQWKVDNALQSGNSQ
--------------------------|
        |-LCDR3-|


ESVTEQDSKDSTYSLSSTLTLSKADYEKHKVYACEVTHQGLSSPVTKSFNRGEC
```

```
>TPP-1015 VH (PRT)
EVQLVESGGGLVQPGGSLRLSCAASGFNIKDTYIHWVRQAPGKGLEWVARIYPTNGYTRYADSVKGRFTISADTSKNTAY
                              |---|              |-----HCDR2-----|
                              HCDR1

LQMNSLRAEDTAVYYCSRWGGDGFYAMDYWGQGTLVTVSS
                  |--HCDR3--|


>TPP-1015 VL (PRT)
DIQMTQSPSSLSASVGDRVTITCRASQDVNTAVAWYQQKPGKAPKLLIYSASFLYSGVPSRFSGSRSGTDFTLTISSLQP
                       |--LCDR1--|               |-----|
                                                 LCDR2

EDFATYYCQQHYTTPPTFGQGTKVEIK
        |-LCDR3-|


>TPP-1015 Heavy Chain (PRT)
EVQLVESGGGLVQPGGSLRLSCAASGFNIKDTYIHWVRQAPGKGLEWVARIYPTNGYTRYADSVKGRFTISADTSKNTAY
|----------------------------------------------------------VH-------------------
                              |---|              |-----HCDR2-----|
                              HCDR1

LQMNSLRAEDTAVYYCSRWGGDGFYAMDYWGQGTLVTVSSASTKGPSVFPLAPSSKSTSGGTAALGCLVKDYFPEPVTVS
---------------------------------------|
                  |--HCDR3--|


WNSGALTSGVHTFPAVLQSSGLYSLSSVVTVPSSSLGTQTYICNVNHKPSNTKVDKKVEPKSCDKTHTCPPCPAPELLGG


PSVFLFPPKPKDTLMISRTPEVTCVVVDVSHEDPEVKFNWYVDGVEVHNAKTKPREEQYNSTYRVVSVLTVLHQDWLNGK


EYKCKVSNKALPAPIEKTISKAKGQPREPQVYTLPPSREEMTKNQVSLTCLVKGFYPSDIAVEWESNGQPENNYKTTPPV


LDSDGSFFLYSKLTVDKSRWQQGNVFSCSVMHEALHNHYTQKSLSLSPGK


>TPP-1015 Light Chain (PRT)
DIQMTQSPSSLSASVGDRVTITCRASQDVNTAVAWYQQKPGKAPKLLIYSASFLYSGVPSRFSGSRSGTDFTLTISSLQP
|---------------------------------------------------VL--------------------------
                       |--LCDR1--|               |-----|
                                                 LCDR2

EDFATYYCQQHYTTPPTFGQGTKVEIKRTVAAPSVFIFPPSDEQLKSGTASVVCLLNNFYPREAKVQWKVDNALQSGNSQ
--------------------------|
        |-LCDR3-|


ESVTEQDSKDSTYSLSSTLTLSKADYEKHKVYACEVTHQGLSSPVTKSFNRGEC


>TPP-2090 VH (PRT)
EVQLLESGGGLVQPGGSLRLSCAASGFTFSPYPMIWVRQAPGKGLEWVSYISPSGGSTHYADSVKGRFTISRDNSKNTLY
                              |---|              |-----HCDR2-----|
                              HCDR1

LQMNSLRAEDTAVYYCARGGDTYFDYFDYWGQGTLVTVSS
                  |--HCDR3--|
```

```
>TPP-2090 VL (PRT)
DIQMTQSPSSLSASVGDRVTITCRASQSISGYLNWYQQKPGKAPKLLIYQASSLQSGVPSRFSGSGSGTDFTLTISSLQP
                        |--LCDR1--|                |-----|
                                                   LCDR2

EDFATYYCQQSYTSPFITFGQGTKVEIK
        |-LCDR3--|

>TPP-2090 Heavy Chain (PRT)
EVQLLESGGGLVQPGGSLRLSCAASGFTFSPYPMIWVRQAPGKGLEWVSYISPSGGSTHYADSVKGRFTISRDNSKNTLY
|-------------------------------------------------------------VH-----------------
                               |---|               |-----HCDR2-----|
                               HCDR1

LQMNSLRAEDTAVYYCARGGDTYFDYFDYWGQGTLVTVSSASTKGPSVFPLAPSSKSTSGGTAALGCLVKDYFPEPVTVS
----------------------------------------|
                   |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPGK

>TPP-2090 Light Chain (PRT)
DIQMTQSPSSLSASVGDRVTITCRASQSISGYLNWYQQKPGKAPKLLIYQASSLQSGVPSRFSGSGSGTDFTLTISSLQP
|-------------------------------------------------------VL-----------------------
                        |--LCDR1--|                |-----|
                                                   LCDR2

EDFATYYCQQSYTSPFITFGQGTKVEIKRTVAAPSVFIFPPSDEQLKSGTASVVCLLNNFYPREAKVQWKVDNALQSGNS
----------------------------|
        |-LCDR3--|

QESVTEQDSKDSTYSLSSTLTLSKADYEKHKVYACEVTHQGLSSPVTKSFNRGEC

>TPP-2658 VH (PRT)
EVQLLESGGGLVQPGGSLRLSCAASGFTFSPYPMIWVRQAPGKGLEWVSYISPSGGSTHYADSVKGRFTISRDNSKNTLY
                               |---|               |-----HCDR2-----|
                               HCDR1

LQMNSLRAEDTAVYYCARGGDTYFDYFDYWGQGTLVTVSS
                   |--HCDR3--|
```

```
>TPP-2658 VL (PRT)
DIQMTQSPSSLSASVGDRVTITCRASQSISGYLNWYQQKPGKAPKLLIYQASSLQSGVPSRFSGSGSGTDFTLTISSLQP
                       |--LCDR1--|               |-----|
                                                 LCDR2

EDFATYYCQQSYTSPFITFGQGTKVEIK
        |-LCDR3--|


>TPP-2658 Heavy Chain (PRT)
EVQLLESGGGLVQPGGSLRLSCAASGFTFSPYPMIWVRQAPGKGLEWVSYISPSGGSTHYADSVKGRFTISRDNSKNTLY
|----------------------------------------------------------VH-------------------
                              |---|              |-----HCDR2-----|
                              HCDR1

LQMNSLRAEDTAVYYCARGGDTYFDYFDYWGQGTLVTVSSASTKGPSVFPLAPSSKSTSGGTAALGCLVKDYFPEPVTVS
---------------------------------------|
                  |--HCDR3--|


WNSGALTSGVHTFPAVLQSSGLYSLSSVVTVPSSSLGTQTYICNVNHKPSNTKVDKKVEPKSCDKTHTCPPCPAPELLGG


PSVFLFPPKPKDTLMISRTPEVTCVVVDVSHEDPEVKFNWYVDGVEVHNAKTKPREEQYASTYRVVSVLTVLHQDWLNGK


EYKCKVSNKALPAPIEKTISKAKGQPREPQVYTLPPSRDELTKNQVSLTCLVKGFYPSDIAVEWESNGQPENNYKTTPPV


LDSDGSFFLYSKLTVDKSRWQQGNVFSCSVMHEALHNHYTQKSLSLSPG


>TPP-2658 Light Chain (PRT)
DIQMTQSPSSLSASVGDRVTITCRASQSISGYLNWYQQKPGKAPKLLIYQASSLQSGVPSRFSGSGSGTDFTLTISSLQP
|----------------------------------------------------VL-------------------------
                       |--LCDR1--|               |-----|
                                                 LCDR2

EDFATYYCQQSYTSPFITFGQGTKVEIKRTVAAPSVFIFPPSDEQLKSGTASVVCLLNNFYPREAKVQWKVDNALQSGNS
---------------------------|
        |-LCDR3--|


QESVTEQDSKDSTYSLSSTLTLSKADYEKHKVYACEVTHQGLSSPVTKSFNRGEC


>TPP-5442 VH (PRT)
EVQLLESGGGLVQPGGSLRLSCAASGFTFSPYPMIWVRQAPGKGLEWVSYISPSGGSTHYADSVKGRFTISRDNSKNTLY
                              |---|              |-----HCDR2-----|
                              HCDR1

LQMNSLRAEDTAVYYCARGGDTYFDYFDYWGQGTLVTVSS
                  |--HCDR3--|


>TPP-5442 VL (PRT)
DIQMTQSPSSLSASVGDRVTITCRASQSISGYLNWYQQKPGKAPKLLIYQASSLQSGVPSRFSGSGSGTDFTLTISSLQP
                       |--LCDR1--|               |-----|
                                                 LCDR2

EDFATYYCQQSYTSPFITFGQGTKVEIK
        |-LCDR3--|
```

```
>TPP-5442 Heavy Chain (PRT)
EVQLLESGGGLVQPGGSLRLSCAASGFTFSPYPMIWVRQAPGKGLEWVSYISPSGGSTHYADSVKGRFTISRDNSKNTLY
|----------------------------------------------------------VH-------------------
                              |---|             |-----HCDR2-----|
                              HCDR1

LQMNSLRAEDTAVYYCARGGDTYFDYFDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYQSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG

>TPP-5442 Light Chain (PRT)
DIQMTQSPSSLSASVGDRVTITCRASQSISGYLNWYQQKPGKAPKLLIYQASSLQSGVPSRFSGSGSGTDFTLTISSLQP
|----------------------------------------------------VL-------------------------
                       |--LCDR1--|              |-----|
                                                LCDR2

EDFATYYCQQSYTSPFITFGQGTKVEIKRTVAAPSVFIFPPSDEQLKSGTASVVCLLNNFYPREAKVQWKVDNALQSGNS
---------------------------|
        |-LCDR3--|

QESVTEQDSKDSTYSLSSTLTLSKADYEKHKVYACEVTHQGLSSPVTKSFNRGEC

>TPP-7006 VH (PRT)
QVQLQQSGPELVKPGASVKMSCKASGYTFTDFIIAWVKQRTGQGLEWIGEIYPGTGRTYYSEKFRGKATLTADKSSNTAY
                              |---|             |-----HCDR2-----|
                              HCDR1

MQLSSLTSVDSAVYFCARRTIYYDYDGDYWGQGTTLTVSS
                  |--HCDR3--|

>TPP-7006 VL (PRT)
DIVMTQAAFSNPVTLGTSASISCRSSKSLLHSNGITYLYWYLQKPGQSPQLLIYQMSNLASGVPDRFSSSGSGTDFTLRI
                       |----LCDR1-----|               |-----|
                                                      LCDR2

SRVEAEDVGVYYCAHNLELPWTFGGGTKLELK
             |-LCDR3-|
```

```
>TPP-7006 Heavy Chain (PRT)
QVQLQQSGPELVKPGASVKMSCKASGYTFTDFIIAWVKQRTGQGLEWIGEIYPGTGRTYYSEKFRGKATLTADKSSNTAY
|----------------------------------------------------------VH-------------------
                              |---|               |-----HCDR2-----|
                              HCDR1

MQLSSLTSVDSAVYFCARRTIYYDYDGDYWGQGTTLTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG


>TPP-7006 Light Chain (PRT)
DIVMTQAAFSNPVTLGTSASISCRSSKSLLHSNGITYLYWYLQKPGQSPQLLIYQMSNLASGVPDRFSSSGSGTDFTLRI
|------------------------------------------------------VL-----------------------
                       |----LCDR1-----|               |-----|
                                                      LCDR2

SRVEAEDVGVYYCAHNLELPWTFGGGTKLELKRTVAAPSVFIFPPSDEQLKSGTASVVCLLNNFYPREAKVQWKVDNALQ
-------------------------------|
             |-LCDR3-|

SGNSQESVTEQDSKDSTYSLSSTLTLSKADYEKHKVYACEVTHQGLSSPVTKSFNRGEC


>TPP-7007 VH (PRT)
QVQLVQSGAEVKKPGASVKVSCKASGYTFTDFIIAWVKQAPGQGLEWIGEIYPGTGRTYYSEKFRGKATLTADTSTSTAY
                              |---|               |-----HCDR2-----|
                              HCDR1

MELSSLRSEDTAVYFCARRTIYYDYDGDYWGQGTLVTVSS
                  |--HCDR3--|

>TPP-7007 VL (PRT)
DIVMTQSPLSLPVTPGEPASISCRSSKSLLHSNGITYLYWYLQKPGQSPQLLIYQMSNLASGVPDRFSGSGSGTDFTLKI
                       |----LCDR1-----|               |-----|
                                                      LCDR2

SRVEAEDVGVYYCAHNLELPWTFGQGTKVEIK
             |-LCDR3-|
```

```
>TPP-7007 Heavy Chain (PRT)
QVQLVQSGAEVKKPGASVKVSCKASGYTFTDFIIAWVKQAPGQGLEWIGEIYPGTGRTYYSEKFRGKATLTADTSTSTAY
|----------------------------------------------------------VH-------------------
                              |---|              |-----HCDR2-----|
                              HCDR1

MELSSLRSEDTAVYFCARRTIYYDYDGDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG


>TPP-7007 Light Chain (PRT)
DIVMTQSPLSLPVTPGEPASISCRSSKSLLHSNGITYLYWYLQKPGQSPQLLIYQMSNLASGVPDRFSGSGSGTDFTLKI
|------------------------------------------------------VL-----------------------
                       |----LCDR1-----|               |-----|
                                                      LCDR2

SRVEAEDVGVYYCAHNLELPWTFGQGTKVEIKRTVAAPSVFIFPPSDEQLKSGTASVVCLLNNFYPREAKVQWKVDNALQ
-------------------------------|
             |-LCDR3-|

SGNSQESVTEQDSKDSTYSLSSTLTLSKADYEKHKVYACEVTHQGLSSPVTKSFNRGEC


>TPP-7510 VH (PRT)
EVQLVESGGGLVQPGGSLRLSCAASGFNIKDTYIHWVRQAPGKGLEWVARIYPTNGYTRYADSVKGRFTISADTSKNTAY
                              |---|              |-----HCDR2-----|
                              HCDR1

LQMNSLRAEDTAVYYCSRWGGDGFYAMDYWGQGTLVTVSS
                  |--HCDR3--|

>TPP-7510 VL (PRT)
DIQMTQSPSSLSASVGDRVTITCRASQDVNTAVAWYQQKPGKAPKLLIYSASFLYSGVPSRFSGSRSGTDFTLTISSLQP
                       |--LCDR1--|               |-----|
                                                 LCDR2

EDFATYYCQQHYTTPPTFGQGTKVEIK
        |-LCDR3-|
```

```
>TPP-7510 Heavy Chain (PRT)
EVQLVESGGGLVQPGGSLRLSCAASGFNIKDTYIHWVRQAPGKGLEWVARIYPTNGYTRYADSVKGRFTISADTSKNTAY
|----------------------------------------------------------VH-------------------
                              |---|              |-----HCDR2-----|
                              HCDR1

LQMNSLRAEDTAVYYCSRWGGDGFYAMDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYASTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG


>TPP-7510 Light Chain (PRT)
DIQMTQSPSSLSASVGDRVTITCRASQDVNTAVAWYQQKPGKAPKLLIYSASFLYSGVPSRFSGSRSGTDFTLTISSLQP
|---------------------------------------------------VL--------------------------
                       |--LCDR1--|               |-----|
                                                 LCDR2

EDFATYYCQQHYTTPPTFGQGTKVEIKRTVAAPSVFIFPPSDEQLKSGTASVVCLLNNFYPREAKVQWKVDNALQSGNSQ
--------------------------|
        |-LCDR3-|

ESVTEQDSKDSTYSLSSTLTLSKADYEKHKVYACEVTHQGLSSPVTKSFNRGEC


>TPP-7511 VH (PRT)
EVQLVESGGGLVQPGGSLRLSCAASGFNIKDTYIHWVRQAPGKGLEWVARIYPTNGYTRYADSVKGRFTISADTSKNTAY
                              |---|              |-----HCDR2-----|
                              HCDR1

LQMNSLRAEDTAVYYCSRWGGDGFYAMDYWGQGTLVTVSS
                  |--HCDR3--|

>TPP-7511 VL (PRT)
DIQMTQSPSSLSASVGDRVTITCRASQDVNTAVAWYQQKPGKAPKLLIYSASFLYSGVPSRFSGSRSGTDFTLTISSLQP
                       |--LCDR1--|               |-----|
                                                 LCDR2

EDFATYYCQQHYTTPPTFGQGTKVEIK
        |-LCDR3-|
```

```
>TPP-7511 Heavy Chain (PRT)
EVQLVESGGGLVQPGGSLRLSCAASGFNIKDTYIHWVRQAPGKGLEWVARIYPTNGYTRYADSVKGRFTISADTSKNTAY
|----------------------------------------------------------VH-------------------
                              |---|               |-----HCDR2-----|
                              HCDR1

LQMNSLRAEDTAVYYCSRWGGDGFYAMDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYQSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG

>TPP-7511 (PRT)
DIQMTQSPSSLSASVGDRVTITCRASQDVNTAVAWYQQKPGKAPKLLIYSASFLYSGVPSRFSGSRSGTDFTLTISSLQP
|---------------------------------------------------VL--------------------------
                       |--LCDR1--|                |-----|
                                                  LCDR2

EDFATYYCQQHYTTPPTFGQGTKVEIKRTVAAPSVFIFPPSDEQLKSGTASVVCLLNNFYPREAKVQWKVDNALQSGNSQ
--------------------------|
        |-LCDR3-|

ESVTEQDSKDSTYSLSSTLTLSKADYEKHKVYACEVTHQGLSSPVTKSFNRGEC

>TPP-8382 VH (PRT)
EVQLLESGGGLVQPGGSLRLSCAASGFTFSSYAMSWVRQAPGKGLEWVSSISGSGGSTLYADSVKGRFTISRDNSKNTLY
                              |---|               |-----HCDR2-----|
                              HCDR1

LQMNSLRAEDTAVYYCAKLTGTSFDYWGQGTLVTVSS
                  |------|
                  HCDR3

>TPP-8382 VL (PRT)
QSVLTQPPSASGTPGQRVTISCSGSSSNIGSNPVNWYQQLPGTAPKLLIYSNNQRPSGVPDRFSGSKSGTSASLAITGLQ
                      |---LCDR1---|                |-----|
                                                   LCDR2

SEDEADYYCQSFDSSLKKVFGGGTKLTVL
         |-LCDR3--|
```

```
>TPP-8382 Heavy Chain (PRT)
EVQLLESGGGLVQPGGSLRLSCAASGFTFSSYAMSWVRQAPGKGLEWVSSISGSGGSTLYADSVKGRFTISRDNSKNTLY
|---------------------------------------------------------VH---------------------
                              |---|              |-----HCDR2-----|
                              HCDR1

LQMNSLRAEDTAVYYCAKLTGTSFDYWGQGTLVTVSSASTKGPSVFPLAPSSKSTSGGTAALGCLVKDYFPEPVTVSWNS
-------------------------------------|
                  |------|
                  HCDR3

GALTSGVHTFPAVLQSSGLYSLSSVVTVPSSSLGTQTYICNVNHKPSNTKVDKKVEPKSCDKTHTCPPCPAPELLGGPSV

FLFPPKPKDTLMISRTPEVTCVVVDVSHEDPEVKFNWYVDGVEVHNAKTKPREEQYNSTYRVVSVLTVLHQDWLNGKEYK

CKVSNKALPAPIEKTISKAKGQPREPQVYTLPPSRDELTKNQVSLTCLVKGFYPSDIAVEWESNGQPENNYKTTPPVLDS

DGSFFLYSKLTVDKSRWQQGNVFSCSVMHEALHNHYTQKSLSLSPG

>TPP-8382 Light Chain (PRT)
QSVLTQPPSASGTPGQRVTISCSGSSSNIGSNPVNWYQQLPGTAPKLLIYSNNQRPSGVPDRFSGSKSGTSASLAITGLQ
|-----------------------------------------------------VL-------------------------
                      |---LCDR1---|                |-----|
                                                   LCDR2

SEDEADYYCQSFDSSLKKVFGGGTKLTVLGQPKAAPSVTLFPPSSEELQANKATLVCLISDFYPGAVTVAWKADSSPVKA
----------------------------|
         |-LCDR3--|

GVETTTPSKQSNNKYAASSYLSLTPEQWKSHRSYSCQVTHEGSTVEKTVAPTECS

>TPP-8567 VH (PRT)
EVQLLESGGGLVQPGGSLRLSCAASGFTFSSYAMSWVRQAPGKGLEWVSSISGSGGSTLYADSVKGRFTISRDNSKNTLY
                              |---|              |-----HCDR2-----|
                              HCDR1

LQMNSLRAEDTAVYYCAKLTGTSFDYWGQGTLVTVSS
                  |------|
                  HCDR3

>TPP-8567 VL (PRT)
QSVLTQPPSASGTPGQRVTISCSGSSSNIGSNPVNWYQQLPGTAPKLLIYSNNQRPSGVPDRFSGSKSGTSASLAITGLQ
                      |---LCDR1---|                |-----|
                                                   LCDR2

SEDEADYYCQSFDSSLKKVFGGGTKLTVL
         |-LCDR3--|
```

```
>TPP-8567 Heavy Chain (PRT)
EVQLLESGGGLVQPGGSLRLSCAASGFTFSSYAMSWVRQAPGKGLEWVSSISGSGGSTLYADSVKGRFTISRDNSKNTLY
|--------------------------------------------------------VH---------------------
                              |---|              |-----HCDR2-----|
                              HCDR1

LQMNSLRAEDTAVYYCAKLTGTSFDYWGQGTLVTVSSASTKGPSVFPLAPSSKSTSGGTAALGCLVKDYFPEPVTVSWNS
------------------------------------|
                  |------|
                  HCDR3

GALTSGVHTFPAVLQSSGLYSLSSVVTVPSSSLGTQTYICNVNHKPSNTKVDKKVEPKSCDKTHTCPPCPAPELLGGPSV

FLFPPKPKDTLMISRTPEVTCVVVDVSHEDPEVKFNWYVDGVEVHNAKTKPREEQYQSTYRVVSVLTVLHQDWLNGKEYK

CKVSNKALPAPIEKTISKAKGQPREPQVYTLPPSRDELTKNQVSLTCLVKGFYPSDIAVEWESNGQPENNYKTTPPVLDS

DGSFFLYSKLTVDKSRWQQGNVFSCSVMHEALHNHYTQKSLSLSPG


>TPP-8567 Light Chain (PRT)
QSVLTQPPSASGTPGQRVTISCSGSSSNIGSNPVNWYQQLPGTAPKLLIYSNNQRPSGVPDRFSGSKSGTSASLAITGLQ
|----------------------------------------------------VL-------------------------
                      |---LCDR1---|                 |-----|
                                                    LCDR2

SEDEADYYCQSFDSSLKKVFGGGTKLTVLGQPKAAPSVTLFPPSSEELQANKATLVCLISDFYPGAVTVAWKADSSPVKA
----------------------------|
         |-LCDR3--|

GVETTTPSKQSNNKYAASSYLSLTPEQWKSHRSYSCQVTHEGSTVEKTVAPTECS


>TPP-8825 VH (PRT)
EVQLLESGGGLVQPGGSLRLSCAASGFTFSPYPMIWVRQAPGKGLEWVSYISPSGGSTHYADSVKGRFTISRDNSKNTLY
                              |---|              |-----HCDR2-----|
                              HCDR1

LQMNSLRAEDTAVYYCARGGDTYFDYFDYWGQGTLVTVSS
                  |--HCDR3--|

>TPP-8825 VL (PRT)
DIQMTQSPSSLSASVGDRVTITCRASQSISGYLNWYQQKPGKAPKLLIYQASSLQSGVPSRFSGSGSGTDFTLTISSLQP
                       |--LCDR1--|              |-----|
                                                LCDR2

EDFATYYCQQSYTSPFITFGQGTKVEIK
        |-LCDR3--|
```

```
>TPP-8825 Heavy Chain (PRT)
EVQLLESGGGLVQPGGSLRLSCAASGFTFSPYPMIWVRQAPGKGLEWVSYISPSGGSTHYADSVKGRFTISRDNSKNTLY
|-------------------------------------------------------------------------VH------------------------
                                      |---|                   |-----HCDR2-----|
                                      HCDR1

LQMNSLRAEDTAVYYCARGGDTYFDYFDYWGQGTLVTVSSASTKGPSVFPLAPSSKSTSGGTAALGCLVKDYFPEPVTVS
-------------------------------------------------|
                       |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREENYQSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG

>TPP-8825 Light Chain (PRT)
DIQMTQSPSSLSASVGDRVTITCRASQSISGYLNWYQQKPGKAPKLLIYQASSLQSGVPSRFSGSGSGTDFTLTISSLQP
|-----------------------------------------------------------------VL--------------------------------
                             |--LCDR1--|                    |-----|
                                                            LCDR2

EDFATYYCQQSYTSPFITFGQGTKVEIKRTVAAPSVFIFPPSDEQLKSGTASVVCLLNNFYPREAKVQWKVDNALQSGNS
----------------------------------|
          |-LCDR3--|

QESVTEQDSKDSTYSLSSTLTLSKADYEKHKVYACEVTHQGLSSPVTKSFNRGEC

>TPP-10334 VH (PRT)
QVQLVQSGAEVKKPGASVKVSCKASGYTFTDFIIAWVKQAPGQGLEWIGEIYPGTGRTYYSEKFRGKATLTADTSTSTAY
                                      |---|                   |-----HCDR2-----|
                                      HCDR1

MELSSLRSEDTAVYFCARRTIYYDYDGDYWGQGTLVTVSS
                       |--HCDR3--|

>TPP-10334 VL (PRT)
DIVMTQSPLSLPVTPGEPASISCRSSQSLLHSNGITYLYWYLQKPGQSPQLLIYQMSNRASGVPDRFSGSGSGTDFTLKI
                             |----LCDR1-----|                      |-----|
                                                                   LCDR2

SRVEAEDVGVYYCAHNLELPWTFGQGTKVEIK
                |-LCDR3-|
```

```
>TPP-10334 Heavy Chain (PRT)
QVQLVQSGAEVKKPGASVKVSCKASGYTFTDFIIAWVKQAPGQGLEWIGEIYPGTGRTYYSEKFRGKATLTADTSTSTAY
|----------------------------------------------------------VH-------------------
                              |---|              |-----HCDR2-----|
                              HCDR1

MELSSLRSEDTAVYFCARRTIYYDYDGDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG

>TPP-10334 Light Chain (PRT)
DIVMTQSPLSLPVTPGEPASISCRSSQSLLHSNGITYLYWYLQKPGQSPQLLIYQMSNRASGVPDRFSGSGSGTDFTLKI
|------------------------------------------------------VL-----------------------
                       |----LCDR1-----|               |-----|
                                                      LCDR2

SRVEAEDVGVYYCAHNLELPWTFGQGTKVEIKRTVAAPSVFIFPPSDEQLKSGTASVVCLLNNFYPREAKVQWKVDNALQ
-------------------------------|
             |-LCDR3-|

SGNSQESVTEQDSKDSTYSLSSTLTLSKADYEKHKVYACEVTHQGLSSPVTKSFNRGEC

>TPP-10335 VH (PRT)
QVQLVQSGAEVKKPGASVKVSCKASGYTFTDFIIAWVKQAPGQGLEWIGEIYPGTGRTYYSEKFRGKATLTADTSTSTAY
                              |---|              |-----HCDR2-----|
                              HCDR1

MELSSLRSEDTAVYFCARRTIYYDYDGDYWGQGTLVTVSS
                  |--HCDR3--|

>TPP-10335 VL (PRT)
DIVMTQSPLSLPVTPGEPASISCRSSQSLLHSNGINYLYWYLQKPGQSPQLLIYQMSNRASGVPDRFSGSGSGTDFTLKI
                       |----LCDR1-----|               |-----|
                                                      LCDR2

SRVEAEDVGVYYCAHNLELPWTFGQGTKVEIK
             |-LCDR3-|
```

```
>TPP-10335 Heavy Chain (PRT)
QVQLVQSGAEVKKPGASVKVSCKASGYTFTDFIIAWVKQAPGQGLEWIGEIYPGTGRTYYSEKFRGKATLTADTSTSTAY
|----------------------------------------------------------VH-------------------
                              |---|             |-----HCDR2-----|
                              HCDR1

MELSSLRSEDTAVYFCARRTIYYDYDGDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG

>TPP-10335 Light Chain (PRT)
DIVMTQSPLSLPVTPGEPASISCRSSQSLLHSNGINYLYWYLQKPGQSPQLLIYQMSNRASGVPDRFSGSGSGTDFTLKI
|------------------------------------------------------VL-----------------------
                       |----LCDR1-----|               |-----|
                                                      LCDR2

SRVEAEDVGVYYCAHNLELPWTFGQGTKVEIKRTVAAPSVFIFPPSDEQLKSGTASVVCLLNNFYPREAKVQWKVDNALQ
-------------------------------|
             |-LCDR3-|

SGNSQESVTEQDSKDSTYSLSSTLTLSKADYEKHKVYACEVTHQGLSSPVTKSFNRGEC

>TPP-10336 VH (PRT)
QVQLVQSGAEVKKPGASVKVSCKASGYTFTDFIIAWVKQAPGQGLEWIGEIYPGTGRTYYSEKFRGKATLTADTSTSTAY
                              |---|             |-----HCDR2-----|
                              HCDR1

MELSSLRSEDTAVYFCARRTIYYDYDGDYWGQGTLVTVSS
                  |--HCDR3--|

>TPP-10336 VL (PRT)
DIVMTQSPLSLPVTPGEPASISCRSSQSLLHSNWINYLYWYLQKPGQSPQLLIYQMSNRASGVPDRFSGSGSGTDFTLKI
                       |----LCDR1-----|               |-----|
                                                      LCDR2

SRVEAEDVGVYYCAHNLELPWTFGQGTKVEIK
             |-LCDR3-|
```

```
>TPP-10336 Heavy Chain (PRT)
QVQLVQSGAEVKKPGASVKVSCKASGYTFTDFIIAWVKQAPGQGLEWIGEIYPGTGRTYYSEKFRGKATLTADTSTSTAY
|----------------------------------------------------------VH-------------------
                              |---|              |-----HCDR2-----|
                              HCDR1

MELSSLRSEDTAVYFCARRTIYYDYDGDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG

>TPP-10336 Light Chain (PRT)
DIVMTQSPLSLPVTPGEPASISCRSSQSLLHSNWINYLYWYLQKPGQSPQLLIYQMSNRASGVPDRFSGSGSGTDFTLKI
|------------------------------------------------------VL-----------------------
                       |----LCDR1-----|               |-----|
                                                      LCDR2

SRVEAEDVGVYYCAHNLELPWTFGQGTKVEIKRTVAAPSVFIFPPSDEQLKSGTASVVCLLNNFYPREAKVQWKVDNALQ
-------------------------------|
             |-LCDR3-|

SGNSQESVTEQDSKDSTYSLSSTLTLSKADYEKHKVYACEVTHQGLSSPVTKSFNRGEC

>TPP-10337 VH (PRT)
QVQLVQSGAEVKKPGASVKVSCKASGYTFTDFIIAWVKQAPGQGLEWIGEIYPGTGRTYYSEKFRGKATLTADTSTSTAY
                              |---|              |-----HCDR2-----|
                              HCDR1

MELSSLRSEDTAVYFCARRTIYYDYDGDYWGQGTLVTVSS
                  |--HCDR3--|

>TPP-10337 VL (PRT)
DIVMTQSPLSLPVTPGEPASISCRSSQSLLHSNWINYLYWYLQKPGQSPQLLIYQGSNRASGVPDRFSGSGSGTDFTLKI
                       |----LCDR1-----|               |-----|
                                                      LCDR2

SRVEAEDVGVYYCAHNLELPWTFGQGTKVEIK
             |-LCDR3-|
```

```
>TPP-10337 Heavy Chain (PRT)
QVQLVQSGAEVKKPGASVKVSCKASGYTFTDFIIAWVKQAPGQGLEWIGEIYPGTGRTYYSEKFRGKATLTADTSTSTAY
|----------------------------------------------------------VH-------------------
                              |---|              |-----HCDR2-----|
                              HCDR1

MELSSLRSEDTAVYFCARRTIYYDYDGDYWGQGTLVTVSSASTKGPSVFPLAPSSKSTSGGTAALGCLVKDYFPEPVTVS
---------------------------------------|
                  |--HCDR3--|

WNSGALTSGVHTFPAVLQSSGLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQYNSTYRVVSVLTVLHQDWLNGK

EYKCKVSNKALPAPIEKTISKAKGQPREPQVYTLPPSRDELTKNQVSLTCLVKGFYPSDIAVEWESNGQPENNYKTTPPV

LDSDGSFFLYSKLTVDKSRWQQGNVFSCSVMHEALHNHYTQKSLSLSPG


>TPP-10337 Light Chain (PRT)
DIVMTQSPLSLPVTPGEPASISCRSSQSLLHSNWINYLYWYLQKPGQSPQLLIYQGSNRASGVPDRFSGSGSGTDFTLKI
|------------------------------------------------------VL-----------------------
                       |----LCDR1-----|               |-----|
                                                      LCDR2

SRVEAEDVGVYYCAHNLELPWTFGQGTKVEIKRTVAAPSVFIFPPSDEQLKSGTASVVCLLNNFYPREAKVQWKVDNALQ
-------------------------------|
             |-LCDR3-|

SGNSQESVTEQDSKDSTYSLSSTLTLSKADYEKHKVYACEVTHQGLSSPVTKSFNRGEC
```

## Fig. 4/4

<SEQ ID NO:1>
QVQLKQSGPGLVQPSQSLSITCTVSGFSLTNYGVHWVRQSPGKGLEWLGVIWSGGNTDYNTPFTSRLSINKDN
SKSQVFFKMNSLQSNDTAIYYCARALTYYDYEFAYWGQGTLVTVSA

<SEQ ID NO:2>
NYGVH

<SEQ ID NO:3>
VIWSGGNTDYNTPFTS

<SEQ ID NO:4>
ALTYYDYEFAY

<SEQ ID NO:5>
DILLTQSPVILSVSPGERVSFSCRASQSIGTNIHWYQQRTNGSPRLLIKYASESISGIPSRFSGSGSGTDFTL
SINSVESEDIADYYCQQNNNWPTTFGAGTKLELK

<SEQ ID NO:6>
RASQSIGTNIH

<SEQ ID NO:7>
YASESIS

<SEQ ID NO:8>
QQNNNWPTT

<SEQ ID NO:9>
QVQLKQSGPGLVQPSQSLSITCTVSGFSLTNYGVHWVRQSPGKGLEWLGVIWSGGNTDYNTPFTSRLSINKDN
SKSQVFFKMNSLQSNDTAIYYCARALTYYDYEFAYWGQGTLVTVSAASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKRVEP
KSCDKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSREEMTKNQV
SLTCLVKGFYPSDIAVEWESNGQPENNYKTTPPVLDSDGSFFLYSKLTVDKSRWQQGNVFSCSVMHEALHNHY
TQKSLSLSPGK

<SEQ ID NO:10>
DILLTQSPVILSVSPGERVSFSCRASQSIGTNIHWYQQRTNGSPRLLIKYASESISGIPSRFSGSGSGTDFTL
SINSVESEDIADYYCQQNNNWPTTFGAGTKLELKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC

<SEQ ID NO:11>
EVQLVESGGGLVQPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSS

<SEQ ID NO:12>
DTYIH

<SEQ ID NO:13>
RIYPTNGYTRYADSVKG

<SEQ ID NO:14>
WGGDGFYAMDY

<SEQ ID NO:15>
DIQMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIK

<SEQ ID NO:16>
RASQDVNTAVA

<SEQ ID NO:17>
SASFLYS

<SEQ ID NO:18>
QQHYTTPPT

<SEQ ID NO:19>
EVQLVESGGGLVQPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVFSCSVMHEALHNH
YTQKSLSLSPGK

<SEQ ID NO:20>
DIQMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC

<SEQ ID NO:21>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSS

<SEQ ID NO:22>
PYPMI

<SEQ ID NO:23>
YISPSGGSTHYADSVKG

<SEQ ID NO:24>
GGDTYFDYFDY

<SEQ ID NO:25>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIK

<SEQ ID NO:26>
RASQSISGYLN

<SEQ ID NO:27>
QASSLQS

<SEQ ID NO:28>
QQSYTSPFIT

<SEQ ID NO:29>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPGK

<SEQ ID NO:30>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

<SEQ ID NO:31>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSS

<SEQ ID NO:32>
PYPMI

<SEQ ID NO:33>
YISPSGGSTHYADSVKG

<SEQ ID NO:34>
GGDTYFDYFDY

<SEQ ID NO:35>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIK

<SEQ ID NO:36>
RASQSISGYLN

<SEQ ID NO:37>
QASSLQS

<SEQ ID NO:38>
QQSYTSPFIT

<SEQ ID NO:39>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYASTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:40>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

<SEQ ID NO:41>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSS

<SEQ ID NO:42>
PYPMI

<SEQ ID NO:43>
YISPSGGSTHYADSVKG

<SEQ ID NO:44>
GGDTYFDYFDY

<SEQ ID NO:45>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIK

<SEQ ID NO:46>
RASQSISGYLN

<SEQ ID NO:47>
QASSLQS

<SEQ ID NO:48>
QQSYTSPFIT

<SEQ ID NO:49>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYQSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:50>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

<SEQ ID NO:51>
QVQLQQSGPELVKPGASVKMSCKASGYTFTDFIIAWVKQRTGQGLEWIGEIYPGTGRTYYSEKFRGKATLTAD
KSSNTAYMQLSSLTSVDSAVYFCARRTIYYDYDGDYWGQGTTLTVSS

<SEQ ID NO:52>
DFIIA

<SEQ ID NO:53>
EIYPGTGRTYYSEKFRG

<SEQ ID NO:54>
RTIYYDYDGDY

<SEQ ID NO:55>
DIVMTQAAFSNPVTLGTSASISCRSSKSLLHSNGITYLYWYLQKPGQSPQLLIYQMSNLASGVPDRFSSSGSG
TDFTLRISRVEAEDVGVYYCAHNLELPWTFGGGTKLELK

<SEQ ID NO:56>
RSSKSLLHSNGITYLY

<SEQ ID NO:57>
QMSNLAS

<SEQ ID NO:58>
AHNLELPWT

<SEQ ID NO:59>
QVQLQQSGPELVKPGASVKMSCKASGYTFTDFIIAWVKQRTGQGLEWIGEIYPGTGRTYYSEKFRGKATLTAD
KSSNTAYMQLSSLTSVDSAVYFCARRTIYYDYDGDYWGQGTTLTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE

PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:60>
DIVMTQAAFSNPVTLGTSASISCRSSKSLLHSNGITYLYWYLQKPGQSPQLLIYQMSNLASGVPDRFSSSGSG
TDFTLRISRVEAEDVGVYYCAHNLELPWTFGGGTKLELKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFNRGEC

<SEQ ID NO:61>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSS

<SEQ ID NO:62>
DFIIA

<SEQ ID NO:63>
EIYPGTGRTYYSEKFRG

<SEQ ID NO:64>
RTIYYDYDGDY

<SEQ ID NO:65>
DIVMTQSPLSLPVTPGEPASISCRSSKSLLHSNGITYLYWYLQKPGQSPQLLIYQMSNLASGVPDRFSGSGSG
TDFTLKISRVEAEDVGVYYCAHNLELPWTFGQGTKVEIK

<SEQ ID NO:66>
RSSKSLLHSNGITYLY

<SEQ ID NO:67>
QMSNLAS

<SEQ ID NO:68>
AHNLELPWT

<SEQ ID NO:69>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:70>
DIVMTQSPLSLPVTPGEPASISCRSSKSLLHSNGITYLYWYLQKPGQSPQLLIYQMSNLASGVPDRFSGSGSG
TDFTLKISRVEAEDVGVYYCAHNLELPWTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFNRGEC

<SEQ ID NO:71>
EVQLVESGGGLVQPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSS

<SEQ ID NO:72>
DTYIH

<SEQ ID NO:73>

RIYPTNGYTRYADSVKG

<SEQ ID NO:74>
WGGDGFYAMDY

<SEQ ID NO:75>
DIQMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIK

<SEQ ID NO:76>
RASQDVNTAVA

<SEQ ID NO:77>
SASFLYS

<SEQ ID NO:78>
QQHYTTPPT

<SEQ ID NO:79>
EVQLVESGGGLVQPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYASTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:80>
DIQMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC

<SEQ ID NO:81>
EVQLVESGGGLVQPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSS

<SEQ ID NO:82>
DTYIH

<SEQ ID NO:83>
RIYPTNGYTRYADSVKG

<SEQ ID NO:84>
WGGDGFYAMDY

<SEQ ID NO:85>
DIQMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIK

<SEQ ID NO:86>
RASQDVNTAVA

<SEQ ID NO:87>
SASFLYS

<SEQ ID NO:88>
QQHYTTPPT

<SEQ ID NO:89>
EVQLVESGGGLVQPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYQSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:90>
DIQMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC

<SEQ ID NO:91>
EVQLLESGGGLVQPGGSLRLSCAASGFTFSSYAMSWVRQAPGKGLEWVSSISGSGGSTLYADSVKGRFTISRD
NSKNTLYLQMNSLRAEDTAVYYCAKLTGTSFDYWGQGTLVTVSS

<SEQ ID NO:92>
SYAMS

<SEQ ID NO:93>
SISGSGGSTLYADSVKG

<SEQ ID NO:94>
LTGTSFDY

<SEQ ID NO:95>
QSVLTQPPSASGTPGQRVTISCSGSSSNIGSNPVNWYQQLPGTAPKLLIYSNNQRPSGVPDRFSGSKSGTSAS
LAITGLQSEDEADYYCQSFDSSLKKVFGGGTKLTVL

<SEQ ID NO:96>
SGSSSNIGSNPVN

<SEQ ID NO:97>
SNNQRPS

<SEQ ID NO:98>
QSFDSSLKKV

<SEQ ID NO:99>
EVQLLESGGGLVQPGGSLRLSCAASGFTFSSYAMSWVRQAPGKGLEWVSSISGSGGSTLYADSVKGRFTISRD
NSKNTLYLQMNSLRAEDTAVYYCAKLTGTSFDYWGQGTLVTVSSASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQ
KSLSLSPG

<SEQ ID NO:100>
QSVLTQPPSASGTPGQRVTISCSGSSSNIGSNPVNWYQQLPGTAPKLLIYSNNQRPSGVPDRFSGSKSGTSAS
LAITGLQSEDEADYYCQSFDSSLKKVFGGGTKLTVLGQPKAAPSVTLFPPSSEELQANKATLVCLISDFYPGA
VTVAWKADSSPVKAGVETTTPSKQSNNKYAASSYLSLTPEQWKSHRSYSCQVTHEGSTVEKTVAPTECS

<SEQ ID NO:101>
EVQLLESGGGLVQPGGSLRLSCAASGFTFSSYAMSWVRQAPGKGLEWVSSISGSGGSTLYADSVKGRFTISRD
NSKNTLYLQMNSLRAEDTAVYYCAKLTGTSFDYWGQGTLVTVSS

<SEQ ID NO:102>
SYAMS

<SEQ ID NO:103>
SISGSGGSTLYADSVKG

<SEQ ID NO:104>
LTGTSFDY

<SEQ ID NO:105>
QSVLTQPPSASGTPGQRVTISCSGSSSNIGSNPVNWYQQLPGTAPKLLIYSNNQRPSGVPDRFSGSKSGTSAS
LAITGLQSEDEADYYCQSFDSSLKKVFGGGTKLTVL

<SEQ ID NO:106>
SGSSSNIGSNPVN

<SEQ ID NO:107>
SNNQRPS

<SEQ ID NO:108>
QSFDSSLKKV

<SEQ ID NO:109>
EVQLLESGGGLVQPGGSLRLSCAASGFTFSSYAMSWVRQAPGKGLEWVSSISGSGGSTLYADSVKGRFTISRD
NSKNTLYLQMNSLRAEDTAVYYCAKLTGTSFDYWGQGTLVTVSSASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVKFNWYVDGVEVHNAKTKPR
EEQYQSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQ
KSLSLSPG

<SEQ ID NO:110>
QSVLTQPPSASGTPGQRVTISCSGSSSNIGSNPVNWYQQLPGTAPKLLIYSNNQRPSGVPDRFSGSKSGTSAS
LAITGLQSEDEADYYCQSFDSSLKKVFGGGTKLTVLGQPKAAPSVTLFPPSSEELQANKATLVCLISDFYPGA
VTVAWKADSSPVKAGVETTTPSKQSNNKYAASSYLSLTPEQWKSHRSYSCQVTHEGSTVEKTVAPTECS

<SEQ ID NO:111>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSS

<SEQ ID NO:112>
PYPMI

<SEQ ID NO:113>
YISPSGGSTHYADSVKG

<SEQ ID NO:114>
GGDTYFDYFDY

<SEQ ID NO:115>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIK

<SEQ ID NO:116>
RASQSISGYLN

<SEQ ID NO:117>
QASSLQS

<SEQ ID NO:118>
QQSYTSPFIT

<SEQ ID NO:119>
EVQLLESGGGLVQPGGSLRLSCAASGFTFSPYPMIWVRQAPGKGLEWVSYISPSGGSTHYADSVKGRFTISRD
NSKNTLYLQMNSLRAEDTAVYYCARGGDTYFDYFDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREENYQSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:120>
DIQMTQSPSSLSASVGDRVTITCRASQSISGYLNWYQQKPGKAPKLLIYQASSLQSGVPSRFSGSGSGTDFTL
TISSLQPEDFATYYCQQSYTSPFITFGQGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

<SEQ ID NO:121>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSS

<SEQ ID NO:122>
DFIIA

<SEQ ID NO:123>
EIYPGTGRTYYSEKFRG

<SEQ ID NO:124>
RTIYYDYDGDY

<SEQ ID NO:125>
DIVMTQSPLSLPVTPGEPASISCRSSQSLLHSNGITYLYWYLQKPGQSPQLLIYQMSNRASGVPDRFSGSGSG
TDFTLKISRVEAEDVGVYYCAHNLELPWTFGQGTKVEIK

<SEQ ID NO:126>
RSSQSLLHSNGITYLY

<SEQ ID NO:127>
QMSNRAS

<SEQ ID NO:128>
AHNLELPWT

<SEQ ID NO:129>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:130>
DIVMTQSPLSLPVTPGEPASISCRSSQSLLHSNGITYLYWYLQKPGQSPQLLIYQMSNRASGVPDRFSGSGSG
TDFTLKISRVEAEDVGVYYCAHNLELPWTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFNRGEC

<SEQ ID NO:131>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSS

<SEQ ID NO:132>
DFIIA

<SEQ ID NO:133>
EIYPGTGRTYYSEKFRG

<SEQ ID NO:134>
RTIYYDYDGDY

<SEQ ID NO:135>
DIVMTQSPLSLPVTPGEPASISCRSSQSLLHSNGINYLYWYLQKPGQSPQLLIYQMSNRASGVPDRFSGSGSG
TDFTLKISRVEAEDVGVYYCAHNLELPWTFGQGTKVEIK

<SEQ ID NO:136>
RSSQSLLHSNGINYLY

<SEQ ID NO:137>
QMSNRAS

<SEQ ID NO:138>
AHNLELPWT

<SEQ ID NO:139>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:140>
DIVMTQSPLSLPVTPGEPASISCRSSQSLLHSNGINYLYWYLQKPGQSPQLLIYQMSNRASGVPDRFSGSGSG
TDFTLKISRVEAEDVGVYYCAHNLELPWTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFNRGEC

<SEQ ID NO:141>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSS

<SEQ ID NO:142>
DFIIA

<SEQ ID NO:143>
EIYPGTGRTYYSEKFRG

<SEQ ID NO:144>
RTIYYDYDGDY

<SEQ ID NO:145>
DIVMTQSPLSLPVTPGEPASISCRSSQSLLHSNWINYLYWYLQKPGQSPQLLIYQMSNRASGVPDRFSGSGSG
TDFTLKISRVEAEDVGVYYCAHNLELPWTFGQGTKVEIK

<SEQ ID NO:146>

RSSQSLLHSNWINYLY

<SEQ ID NO:147>
QMSNRAS

<SEQ ID NO:148>
AHNLELPWT

<SEQ ID NO:149>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:150>
DIVMTQSPLSLPVTPGEPASISCRSSQSLLHSNWINYLYWYLQKPGQSPQLLIYQMSNRASGVPDRFSGSGSG
TDFTLKISRVEAEDVGVYYCAHNLELPWTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFNRGEC

<SEQ ID NO:151>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSS

<SEQ ID NO:152>
DFIIA

<SEQ ID NO:153>
EIYPGTGRTYYSEKFRG

<SEQ ID NO:154>
RTIYYDYDGDY

<SEQ ID NO:155>
DIVMTQSPLSLPVTPGEPASISCRSSQSLLHSNWINYLYWYLQKPGQSPQLLIYQGSNRASGVPDRFSGSGSG
TDFTLKISRVEAEDVGVYYCAHNLELPWTFGQGTKVEIK

<SEQ ID NO:156>
RSSQSLLHSNWINYLY

<SEQ ID NO:157>
QGSNRAS

<SEQ ID NO:158>
AHNLELPWT

<SEQ ID NO:159>
QVQLVQSGAEVKKPGASVKVSCKASGYTFTDFIIAWVKQAPGQGLEWIGEIYPGTGRTYYSEKFRGKATLTAD
TSTSTAYMELSSLRSEDTAVYFCARRTIYYDYDGDYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHNH
YTQKSLSLSPG

<SEQ ID NO:160>

DIVMTQSPLSLPVTPGEPASISCRSSQSLLHSNWINYLYWYLQKPGQSPQLLIYQGSNRASGVPDRFSGSGSG
TDFTLKISRVEAEDVGVYYCAHNLELPWTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFNRGEC

<SEQ ID NO:161>
HSDAVFTDNYTRLRKQMAVKKYLNSILN

<SEQ ID NO:162>
ATEPRKQYATPRVFWTDAPG

<SEQ ID NO:163>
LQWRRDDNVHNFGVWARYRL

<SEQ ID NO:164>
MARGSLRRLLRLLVLGLWLALLRSVAGEQAPGTAPCSRGSSWSADLDKCMDCASCRARPHSDFCLGCAAAPPA
PFRLLWPILGGALSLTFVLGLLSGFLVWRRCRRREKFTTPIEETGGEGCPAVALIQ

<SEQ ID NO:165>
MLRRRGSPGMGVHVGAALGALWFCLTGALEVQVPEDPVVALVGTDATLCCSFSPEPGFSLAQLNLIWQLTDTK
QLVHSFAEGQDQGSAYANRTALFPDLLAQGNASLRLQRVRVADEGSFTCFVSIRDFGSAAVSLQVAAPYSKPS
MTLEPNKDLRPGDTVTITCSSYQGYPEAEVFWQDGQGVPLTGNVTTSQMANEQGLFDVHSILRVVLGANGTYS
CLVRNPVLQQDAHSSVTITPQRSPTGAVEVQVPEDPVVALVGTDATLRCSFSPEPGFSLAQLNLIWQLTDTKQ
LVHSFTEGRDQGSAYANRTALFPDLLAQGNASLRLQRVRVADEGSFTCFVSIRDFGSAAVSLQVAAPYSKPSM
TLEPNKDLRPGDTVTITCSSYRGYPEAEVFWQDGQGVPLTGNVTTSQMANEQGLFDVHSVLRVVLGANGTYSC
LVRNPVLQQDAHGSVTITGQPMTFPPEALWVTVGLSVCLIALLVALAFVCWRKIKQSCEEENAGAEDQDGEGE
GSKTALQPLKHSDSKEDDGQEIA

<SEQ ID NO:166>
MELAALCRWGLLLALLPPGAASTQVCTGTDMKLRLPASPETHLDMLRHLYQGCQVVQGNLELTYLPTNASLSF
LQDIQEVQGYVLIAHNQVRQVPLQRLRIVRGTQLFEDNYALAVLDNGDPLNNTTPVTGASPGGLRELQLRSLT
EILKGGVLIQRNPQLCYQDTILWKDIFHKNNQLALTLIDTNRSRACHPCSPMCKGSRCWGESSEDCQSLTRTV
CAGGCARCKGPLPTDCCHEQCAAGCTGPKHSDCLACLHFNHSGICELHCPALVTYNTDTFESMPNPEGRYTFG
ASCVTACPYNYLSTDVGSCTLVCPLHNQEVTAEDGTQRCEKCSKPCARVCYGLGMEHLREVRAVTSANIQEFA
GCKKIFGSLAFLPESFDGDPASNTAPLQPEQLQVFETLEEITGYLYISAWPDSLPDLSVFQNLQVIRGRILHN
GAYSLTLQGLGISWLGLRSLRELGSGLALIHHNTHLCFVHTVPWDQLFRNPHQALLHTANRPEDECVGEGLAC
HQLCARGHCWGPGPTQCVNCSQFLRGQECVEECRVLQGLPREYVNARHCLPCHPECQPQNGSVTCFGPEADQC
VACAHYKDPPFCVARCPSGVKPDLSYMPIWKFPDEEGACQPCPINCTHSCVDLDDKGCPAEQRASPLTSIISA
VVGILLVVVLGVVFGILIKRRQQKIRKYTMRRLLQETELVEPLTPSGAMPNQAQMRILKETELRKVKVLGSGA
FGTVYKGIWIPDGENVKIPVAIKVLRENTSPKANKEILDEAYVMAGVGSPYVSRLLGICLTSTVQLVTQLMPY
GCLLDHVRENRGRLGSQDLLNWCMQIAKGMSYLEDVRLVHRDLAARNVLVKSPNHVKITDFGLARLLDIDETE
YHADGGKVPIKWMALESILRRRFTHQSDVWSYGVTVWELMTFGAKPYDGIPAREIPDLLEKGERLPQPPICTI
DVYMIMVKCWMIDSECRPRFRELVSEFSRMARDPQRFVVIQNEDLGPASPLDSTFYRSLLEDDDMGDLVDAEE
YLVPQQGFFCPDPAPGAGGMVHHRHRSSSTRSGGGDLTLGLEPSEEEAPRSPLAPSEGAGSDVFDGDLGMGAA
KGLQSLPTHDPSPLQRYSEDPTVPLPSETDGYVAPLTCSPQPEYVNQPDVRPQPPSPREGPLPAARPAGATLE
RPKTLSPGKNGVVKDVFAFGGAVENPEYLTPQGGAAPQPHPPPAFSPAFDNLYYWDQDPPERGAPPSTFKGTP
TAENPEYLGLDVPV

<SEQ ID NO:167>
MRPSGTAGAALLALLAALCPASRALEEKKVCQGTSNKLTQLGTFEDHFLSLQRMFNNCEVVLGNLEITYVQRN
YDLSFLKTIQEVAGYVLIALNTVERIPLENLQIIRGNMYYENSYALAVLSNYDANKTGLKELPMRNLQEILHG
AVRFSNNPALCNVESIQWRDIVSSDFLSNMSMDFQNHLGSCQKCDPSCPNGSCWGAGEENCQKLTKIICAQQC
SGRCRGKSPSDCCHNQCAAGCTGPRESDCLVCRKFRDEATCKDTCPPLMLYNPTTYQMDVNPEGKYSFGATCV
KKCPRNYVVTDHGSCVRACGADSYEMEEDGVRKCKKCEGPCRKVCNGIGIGEFKDSLSINATNIKHFKNCTSI
SGDLHILPVAFRGDSFTHTPPLDPQELDILKTVKEITGFLLIQAWPENRTDLHAFENLEIIRGRTKQHGQFSL
AVVSLNITSLGLRSLKEISDGDVIISGNKNLCYANTINWKKLFGTSGQKTKIISNRGENSCKATGQVCHALCS
PEGCWGPEPRDCVSCRNVSRGRECVDKCNLLEGEPREFVENSECIQCHPECLPQAMNITCTGRGPDNCIQCAH
YIDGPHCVKTCPAGVMGENNTLVWKYADAGHVCHLCHPNCTYGCTGPGLEGCPTNGPKIPSIATGMVGALLLL
LVVALGIGLFMRRRHIVRKRTLRRLLQERELVEPLTPSGEAPNQALLRILKETEFKKIKVLGSGAFGTVYKGL

WIPEGEKVKIPVAIKELREATSPKANKEILDEAYVMASVDNPHVCRLLGICLTSTVQLITQLMPFGCLLDYVR
EHKDNIGSQYLLNWCVQIAKGMNYLEDRRLVHRDLAARNVLVKTPQHVKITDFGLAKLLGAEEKEYHAEGGKV
PIKWMALESILHRIYTHQSDVWSYGVTVWELMTFGSKPYDGIPASEISSILEKGERLPQPPICTIDVYMIMVK
CWMIDADSRPKFRELIIEFSKMARDPQRYLVIQGDERMHLPSPTDSNFYRALMDEEDMDDVVDADEYLIPQQG
FFSSPSTSRTPLLSSLSATSNNSTVACIDRNGLQSCPIKEDSFLQRYSSDPTGALTEDSIDDTFLPVPEYINQ
SVPKRPAGSVQNPVYHNQPLNPAPSRDPHYQDPHSTAVGNPEYLNTVQPTCVNSTFDSPAHWAQKGSHQISLD
NPDYQQDFFPKEAKPNGIFKGSTAENAEYLRVAPQSSEFIGA

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20150343083 A1
- WO 2014062697 A2
- WO 2015096982 A
- US 5270163 A
- WO 9708320 A
- WO 9317715 A
- WO 9208802 A
- WO 9100360 A
- WO 9205793 A
- US 4474893 A
- US 4714681 A
- US 4925648 A
- US 5573920 A
- US 5601819 A
- US 4816567 A, Cabilly
- US 4816397 A, Boss
- WO 2007070538 A
- WO 900786 A
- US 5168062 A, Stinski
- US 4510245 A, Bell
- US 4968615 A, Schaffner
- US 4399216 A
- US 4634665 A
- US 5179017 A
- WO 2009020933 A2
- WO 2009140177 A2
- WO 2014198817 A1
- WO 2015189143 A1
- WO 2009020933 A
- WO 201109400 A
- EP 1773884 A
- WO 2014061277 A
- EP 2121008 A
- WO 2009123894 A2
- WO 2008140603 A2
- WO 2011044368 A2
- WO 02100348 A
- WO 2004056847 A
- WO 2005090407 A
- EP 01735348 A1
- US 20070264253 A1
- US 7598350 B
- WO 2005090407 A1
- WO 9215683 A
- WO 2010112413 A1
- WO 2010115554 A
- WO 2008028686 A2
- EP 01900750 A1
- EP 01911766 A1
- EP 02073842 A2
- US 20100028947 A1
- WO 2008004834 A1
- WO 02092771 A
- WO 2005081854 A
- WO 2009023265 A
- WO 2010022736 A2
- WO 2001062931 A2
- WO 2005010151 A
- US 7628986 B
- WO 01088138 A1
- WO 9105871 A
- WO 2005056606 A
- PH 048638
- WO 2007070538 A2
- US 6177078 B, Lopez
- WO 2009070844 A
- WO 2012021934 A
- EP 2426148 A
- WO 2013173820 A
- EP 2195023 A
- WO 2012143499 A2
- WO 2009068204 A
- WO 2008092117 A
- WO 2008036688 A
- WO 2006089232 A
- WO 2007038637 A2
- WO 2008070593 A2
- WO 2008031056 A2
- WO 2008047242 A2
- WO 2003106495 A2
- WO 2005009369 A2
- WO 2009080829 A1
- WO 2009080830 A1
- US 7465449 B
- EP 719859 A1
- WO 2002012501 A1
- WO 2006062779 A2
- WO 2007024536 A2
- WO 02077033 A1
- US 7318924 B
- WO 2003083041 A2
- WO 2002088170 A2
- WO 9735616 A1
- WO 9947554 A1
- WO 01009192 A1
- WO 2003034903 A
- WO 2009026274 A1
- WO 2007002222 A

- WO 2004091375 A2
- WO 2009033094 A2
- US 20090175796 A1
- WO 2005081711 A2
- WO 2006074418 A2
- WO 2013076186 A
- WO 2013092998 A1
- WO 2013092983 A2
- WO 2012112363 A, K. Kassahun
- WO 201596982 A1
- WO 2014151030 A1
- WO 2015096982 A1
- WO 2005081711 A


**In der Beschreibung aufgeführte Nicht-Patentliteratur**


- **S. ISHII.** *Methods Enzymol,* 1994, vol. 244, 604
- **J. M. CHEN et al.** *J. Biol. Chem.,* 1997, vol. 272, 8090
- **W. WU et al.** *Cancer Res,* 2006, vol. 66, 970
- **L.STERN et al.** *Bioconjugate Chem,* 2009, vol. 20, 500
- **K.M. BAJJURI et al.** *ChemMedChem,* 2011, vol. 6, 54
- **MARY A. MATHIEU et al.** *Molecular and Biochemical Parasitology,* 04. Marz 2002, vol. 121 (1,4), 99-105
- *Histone Deacetylase und Cathepsin L gemäß Nat. Commun.,* 2013, vol. 4, 2735
- *Anticancer Agents in Medicinal Chemistry,* 2008, vol. 8, 618-637
- *Bioorg. Med. Chem.,* 1999, vol. 7, 1597
- *J. Med. Chem.,* 2002, vol. 45, 937
- *Bioorg. Med. Chem.,* 2002, vol. 10, 71
- *Bioorg. Med. Chem.,* 2003, vol. 11, 2277
- *Bioorg. Med. Chem.,* 2007, vol. 15, 4973
- *Bioorg. Med. Chem. Lett.,* 2007, vol. 17, 2241
- *Angew. Chem. Inter. Ed.,* 2005, vol. 44, 4378
- 1505 bzgl. Duocarmycin. *Nat. Struct. Biol.,* 2002, vol. 9, 337
- *Journal of Med. Chem.,* 2010, vol. 53 (3
- 1043 bzgl. Camptothecin. *ChemMedChem,* 2011, vol. 6 (1
- 54 bzgl. Auristatin. *Mol. Cancer. Ther.,* 2005, vol. 4
- 751 bzgl. Doxorubicin und. *J. Biol. Chem,* 2008, vol. 283
- 9318 bzgl. Pyrrolobenzodiazepin-Derivate (PBD-Derivate). *J.Med.Chem,* 2013, vol. 56, 7564
- *J.Med.Chem.,* 2001, vol. 44, 1341
- *Oncology Reports,* 2011, vol. 26, 629
- **KEEFE AD. et al.** *Nat. Rev. Drug Discov.,* 2010, vol. 9, 537-550
- **GEBAUER M. et al.** *Curr. Opinion in Chem. Biol.,* 2009, vol. 13, 245-255
- **NUTTALL S.D. et al.** *Curr. Opinion in Pharmacology,* 2008, vol. 8, 608-617
- **D.BÖHME ; A. BECK-SICKINGER.** *J.Pept.Sci.,* 2015, vol. 21, 186
- **B.FORNER et al.** *Specialty Chemicals Magazine,* Mai 2012
- **V. AHRENS et al.** *Future Med. Chem.,* 2012, vol. 4, 1567
- **W.TAI et al.** *Mol. Pharmaceutics,* 2011, vol. 8, 901
- **R.SOUDY et al.** *J.Med.Chem.,* 2013, vol. 56, 7564
- **R.SOUDY ; M.LANGER et al.** *J.Med.Chem.,* 2001, vol. 44, 1341
- **C.GRUENDKER et al.** *Oncology Reports,* 2011, vol. 26, 629
- **UMLAUF et al.** *Bioconj.Chem.,* 15. Oktober 2014, vol. 25 (10), 1829-37
- **JUNUTULA et al.** *Nat Biotechnol,* August 2008, vol. 26 (8), 925-32
- **SÖDERLIND et al.** *Nature Biotech,* 2000, vol. 18, 853-856
- **KABAT et al.** Sequences of Proteins of Immunological Interest. Public Health Service, National Institutes of Health, 1991
- **KETTE CHOTHIA ; LESK.** *J Mol Biol,* 1987, vol. 196, 901-917
- Antibody Engineering (Breakthroughs in Molecular Biology). Oxford University Press, 1995
- Antibody Engineering (Springer Laboratory Manual). Springer Verlag, 2001
- **TUTT et al.** *J. Immunol.,* 1991, vol. 147, 60-69
- **KOSTELNY et al.** *J. Immunol.,* 1992, vol. 148, 1547-1553
- **KÖHLER ; MILSTEIN.** *Nature,* 1975, vol. 256, 495-497
- **OLSSON et al.** *Meth Enzymol,* vol. 92, 3-16
- **N LONBERG ; D HUSZAR.** *Int Rev Immunol,* 1995, vol. 13 (1), 65-93
- **CLACKSON et al.** *Nature,* 15. August 1991, vol. 352 (6336), 624-8
- **QUEEN et al.** *Pros. Natl. Acad. Sci. USA,* 1989, vol. 86, 10029-10033
- Guide to Molecular Cloning Techniques. **BERGER ; KIMRNEL.** Methods in Enzymology. Academic Press, Inc, vol. 1, 152
- **SAMBROOK et al.** Molecular Cloning: A Laboratory Manual. Cold Spring Harbor Laboratory Press, 1989, vol. 1-3

- Current Protocols. Current Protocols in Molecular Biolony. Green Publishing Associates, Inc. / John Wiley & Sons, Inc,
- **HARLOW et al.** Monoclonal Antibodies: A Laboratory Manual. Cold Spring Harbor Laboratory Press, 19881
- Fundamental Immunology. Lippincott Williams & Wilkins, 1998
- **HARLOW et al.** Using Antibodies: A Laboratory Manual. Cold Spring Harbor Laboratory Press, 1998
- **BEBBINGTON et al.** *Biotechnology (N Y),* Februar 1992, vol. 10 (2), 169-75
- **URLAUB ; CHASIN.** *Proc. Natl. Acad. Sci. USA,* 1980, vol. 77, 4216-4220
- **URLAUB et al.** *Cell,* Juni 1983, vol. 33 (2), 405-12
- **R. J. KAUFMAN ; P. A. SHARP.** *Mol. Biol.,* 1982, vol. 159, 601-621
- **FAN et al.** *Biotechnol Bioeng,* April 2012, vol. 109 (4), 1007-15
- **DUROCHER et al.** *Nucleic Acids Res,* 15. Januar 2002, vol. 30 (2), E9
- **COLLIGAN.** Current Protocols in Immunology, or Current Protocols in Protein Science. John Wiley & Sons, 1997
- **NAKAYAMA et al.** *Biochem Biophy Res Comm,* 2003, vol. 306, 819-825
- **ZHOU et al.** *J Invest Dermatol,* 2013, vol. 133 (4), 1052-62
- *CHEMICAL ABSTRACTS,* 180288-69-1
- *CHEMICAL ABSTRACTS,* 380610-27-5
- **SUN et al.** *Blood,* 1996, vol. 87 (1), 83-92
- **KUO.** *Bioconjug Chem,* 2009, vol. 20 (10), 1975-82
- *CHEMICAL ABSTRACTS,* 174722-31-7
- *CHEMICAL ABSTRACTS,* 216503-57-0
- *CHEMICAL ABSTRACTS,* 139504-50-0 bzw. 474645-27-7
- *CHEMICAL ABSTRACTS,* 474645-27-7
- *CHEMICAL ABSTRACTS,* 139504-50-0
- *CHEMICAL ABSTRACTS,* 23214-92-8
- *CHEMICAL ABSTRACTS,* 725735-28-4
- *CHEMICAL ABSTRACTS,* 143653-53-6
- *CHEMICAL ABSTRACTS,* 892553-42-3
- *CHEMICAL ABSTRACTS,* 301344-99-0
- **K. LANG ; J. W. CHIN.** *Chem. Rev.,* 2014, vol. 114, 4764-4806
- **M. RASHIDIAN et al.** *Bioconjugate Chem,* 2013, vol. 24, 1277-1294
- **AGARWAL et al.** *Bioconjug. Chem.,* 2015, vol. 26, 176-192
- **CAL et al.** *Angew. Chem. Int. Ed. Engl.,* 2014, vol. 53, 10585-10587
- **BEHRENS et al.** *MAbs,* 2014, vol. 6, 46-53
- **PANOWSKI et al.** *MAbs,* 2014, vol. 6, 34-45
- **SOCHAJ et al.** *Biotechnology Advances,* 2015, vol. 33, 775-784
- **KABAT et al.** Sequences of Proteins of Immulological Interest. Public Health Service, National Institutes of Health, 1991
- **JEGER et al.** *Angewandte Chemie Int. Ed. Engl,* 2010, vol. 49, 9995-9997
- **JOSTEN et al.** *J. Immunol. Methods,* 2000, vol. 240, 47-54
- **MINDT et al.** *Bioconjugate Chem,* 2008, vol. 19, 271-278
- **DENNLER et al.** Antibody Drug Conjuagtes. Humana Press, 2013, 205-215
- **STROP et al.** *Chem. Biol.,* 2013, vol. 20, 161-167
- **DENNLER et al.** *Bioconjugate Chemistry,* 2014, vol. 19, 569-578
- **LHOSPICE et al.** *Molecular Pharmaceutics,* 2015, vol. 12, 1863-1871
- **L. DUCRY ; B. STUMP.** *Bioconjugate Chem,* 2010, vol. 21, 5-13
- *Bioconjugate Chem,* 2002, vol. 13, 855-869
- *Bioorganic & Medicinal Chemistry Letters,* 1998, vol. 8, 3341-3346
- *Bioconjugate Chem,* 1998, vol. 9, 618-626
- *Nat. Commun.,* 2013, vol. 4, 2735
- **LAN ; CHIN.** Cellular Incorporation of Unnatural Amino Acids and Bioorthogonal Labeling of Proteins. *Chem.Rev.,* 2014, vol. 114, 4764-4806
- Isotopic Compositions of the Elements 1997. *Pure Appl. Chem.,* 1998, vol. 70 (1), 217-235
- **C. L. PERRIN et al.** *J. Am. Chem. Soc.,* 2007, vol. 129, 4490
- **C. L. PERRIN et al.** *J. Am. Chem. Soc.,* 2005, vol. 127, 9641
- **B. TESTA et al.** *Int. J. Pharm.,* 1984, vol. 19 (3), 271
- **A. E. MUTLIB et al.** *Toxicol. Appl. Pharmacol.,* 2000, vol. 169, 102
- **A. M. SHARMA et al.** *Chem. Res. Toxicol.,* 2013, vol. 26, 410
- **C. J. WENTHUR et al.** *J. Med. Chem.,* 2013, vol. 56, 5208
- **F. SCHNEIDER et al.** *Arzneim. Forsch. / Drug. Res.,* 2006, vol. 56, 295
- **F. MALTAIS et al.** *J. Med. Chem.,* 2009, vol. 52, 7993
- *J.Org.Chem.,* 1993, vol. 58, 2196
- **TOM et al.** Methods Express: Expression Systems herausgegeben. Micheal R. Dyson and Yves Durocher, Scion Publishing Ltd, 2007
- **DORONINA et al.** *Nature Biotechnol,* 2003, vol. 21, 778-784
- **POLSON et al.** *Blood,* 2007, vol. 1102, 616-623
- **M.D. TROUTMAN ; D.R. THAKKER.** *Pharm. Res.,* 2003, vol. 20 (8), 1210-1224

- **D. SCHWAB et al.** *J. Med. Chem.,* 2003, vol. 46, 1716-1725
- **A.H. SCHINKEL et al.** *J. Clin. Invest.,* 1995, vol. 96, 1698-1705
- **POLSON et al.** *Cancer Res,* 15. Marz 2009, vol. 69 (6), 2358-64

SEQUENCE LISTING

<110> Bayer Pharma Aktiengesellschaft

<120> Prodrugs of cytotoxic drugs having enzymatically cleavable groups

<130> BHC 16 3 005

<160> 167

<170> PatentIn version 3.5

<210> 1
<211> 119
<212> PRT
<213> Artificial Sequence

<220>
<223> Antibody sequence

<400> 1

```
Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ala
        115
```

<210> 2
<211> 5
<212> PRT
<213> Artificial Sequence

<220>
<223> Antibody sequence

<400> 2

```
Asn Tyr Gly Val His
1               5
```

<210> 3
<211> 16
<212> PRT
<213> Artificial Sequence

<220>
<223> Antibody sequence

<400> 3

```
Val Ile Trp Ser Gly Gly Asn Thr Asp Tyr Asn Thr Pro Phe Thr Ser
1               5                   10                  15
```

<210> 4
<211> 11

<212> PRT
<213> Artificial Sequence

<220>
<223> Antibody sequence

<400> 4

```
Ala Leu Thr Tyr Tyr Asp Tyr Glu Phe Ala Tyr
1               5                   10
```

<210> 5
<211> 107
<212> PRT
<213> Artificial Sequence

<220>
<223> Antibody sequence

<400> 5

```
Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys
            100                 105
```

<210> 6
<211> 11
<212> PRT
<213> Artificial Sequence

<220>
<223> Antibody sequence

<400> 6

```
Arg Ala Ser Gln Ser Ile Gly Thr Asn Ile His
1               5                   10
```

<210> 7
<211> 7
<212> PRT
<213> Artificial Sequence

<220>
<223> Antibody sequence

<400> 7

```
Tyr Ala Ser Glu Ser Ile Ser
1               5
```

<210> 8
<211> 9
<212> PRT
<213> Artificial Sequence

<220>
<223> Antibody sequence

<400> 8

```
Gln Gln Asn Asn Asn Trp Pro Thr Thr
1               5
```

<210> 9
<211> 449
<212> PRT
<213> Artificial Sequence

<220>
<223> Antibody sequence

<400> 9

```
Gln Val Gln Leu Lys Gln Ser Gly Pro Gly Leu Val Gln Pro Ser Gln
1               5                   10                  15
```

Ser Leu Ser Ile Thr Cys Thr Val Ser Gly Phe Ser Leu Thr Asn Tyr
20 25 30

Gly Val His Trp Val Arg Gln Ser Pro Gly Lys Gly Leu Glu Trp Leu
35 40 45

Gly Val Ile Trp Ser Gly Gly Asn Thr Asp Tyr Asn Thr Pro Phe Thr
50 55 60

Ser Arg Leu Ser Ile Asn Lys Asp Asn Ser Lys Ser Gln Val Phe Phe
65 70 75 80

Lys Met Asn Ser Leu Gln Ser Asn Asp Thr Ala Ile Tyr Tyr Cys Ala
85 90 95

Arg Ala Leu Thr Tyr Tyr Asp Tyr Glu Phe Ala Tyr Trp Gly Gln Gly
100 105 110

Thr Leu Val Thr Val Ser Ala Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Arg Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Glu Glu Met Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 10
<211> 214
<212> PRT
<213> Artificial Sequence

<220>
<223> Antibody sequence

<400> 10

Asp Ile Leu Leu Thr Gln Ser Pro Val Ile Leu Ser Val Ser Pro Gly
1 5 10 15

```
Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 11
<211> 120
<212> PRT
<213> Artificial Sequence

<220>
<223> Antibody sequence

<400> 11

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120
```

<210> 12
<211> 5
<212> PRT
<213> Artificial Sequence

<220>
<223> Antibody sequence

<400> 12

```
Asp Thr Tyr Ile His
1               5
```

<210> 13
<211> 17
<212> PRT
<213> Artificial Sequence

<220>
<223> Antibody sequence

<400> 13

```
Arg Ile Tyr Pro Thr Asn Gly Tyr Thr Arg Tyr Ala Asp Ser Val Lys
1               5                   10                  15

Gly
```

<210> 14
<211> 11
<212> PRT
<213> Artificial Sequence

<220>
<223> Antibody sequence

<400> 14

```
Trp Gly Gly Asp Gly Phe Tyr Ala Met Asp Tyr
1               5                   10
```

<210> 15
<211> 107
<212> PRT
<213> Artificial Sequence

<220>
<223> Antibody sequence

<400> 15

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105
```

<210> 16
<211> 11
<212> PRT
<213> Artificial Sequence

<220>
<223> Antibody sequence

<400> 16

Arg Ala Ser Gln Asp Val Asn Thr Ala Val Ala
1 5 10

<210> 17
<211> 7
<212> PRT
<213> Artificial Sequence

<220>
<223> Antibody sequence

<400> 17

Ser Ala Ser Phe Leu Tyr Ser
1 5

<210> 18
<211> 9
<212> PRT
<213> Artificial Sequence

<220>
<223> Antibody sequence

<400> 18

Gln Gln His Tyr Thr Thr Pro Pro Thr
1 5

<210> 19
<211> 450
<212> PRT
<213> Artificial Sequence

<220>
<223> Antibody sequence

<400> 19

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
```

85 90 95

Ser Arg Trp Gly Gly Asp Gly Phe Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 20
<211> 214
<212> PRT
<213> Artificial Sequence

<220>
<223> Antibody sequence

<400> 20

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro

                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 21
<211> 120
<212> PRT
<213> Artificial Sequence

<220>
<223> Antibody sequence

<400> 21

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45

Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser
        115                 120
```

<210> 22
<211> 5
<212> PRT
<213> Artificial Sequence

<220>
<223> Antibody sequence

<400> 22

```
Pro Tyr Pro Met Ile
1               5
```

<210> 23
<211> 17
<212> PRT
<213> Artificial Sequence

<220>
<223> Antibody sequence

<400> 23

Tyr Ile Ser Pro Ser Gly Gly Ser Thr His Tyr Ala Asp Ser Val Lys
1               5                   10                  15

Gly

<210> 24
<211> 11
<212> PRT
<213> Artificial Sequence

<220>
<223> Antibody sequence

<400> 24

Gly Gly Asp Thr Tyr Phe Asp Tyr Phe Asp Tyr
1               5                   10

<210> 25
<211> 108
<212> PRT
<213> Artificial Sequence

<220>
<223> Antibody sequence

<400> 25

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Gly Tyr
            20                  25                  30

Leu Asn Trp Tyr Gln Gln Lys Pro Gly Lys Ala Pro Lys Leu Leu Ile
        35                  40                  45

Tyr Gln Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Ser Pro Phe
                85                  90                  95

Ile Thr Phe Gly Gln Gly Thr Lys Val Glu Ile Lys
            100                 105

<210> 26
<211> 11
<212> PRT

<213> Artificial Sequence

<220>
<223> Antibody sequence

<400> 26

Arg Ala Ser Gln Ser Ile Ser Gly Tyr Leu Asn
1 5 10

<210> 27
<211> 7
<212> PRT
<213> Artificial Sequence

<220>
<223> Antibody sequence

<400> 27

Gln Ala Ser Ser Leu Gln Ser
1 5

<210> 28
<211> 10
<212> PRT
<213> Artificial Sequence

<220>
<223> Antibody sequence

<400> 28

Gln Gln Ser Tyr Thr Ser Pro Phe Ile Thr
1 5 10

<210> 29
<211> 450
<212> PRT
<213> Artificial Sequence

<220>
<223> Antibody sequence

<400> 29

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45

Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160
```

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

```
Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

<210> 30
<211> 215
<212> PRT
<213> Artificial Sequence

<220>
<223> Antibody sequence

<400> 30

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Gly Tyr
            20                  25                  30

Leu Asn Trp Tyr Gln Gln Lys Pro Gly Lys Ala Pro Lys Leu Leu Ile
        35                  40                  45

Tyr Gln Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Ser Pro Phe
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160
```

Gln Glu Ser Val Thr Glu Gln Asp Ser Lys Asp Ser Thr Tyr Ser Leu
165 170 175

Ser Ser Thr Leu Thr Leu Ser Lys Ala Asp Tyr Glu Lys His Lys Val
180 185 190

Tyr Ala Cys Glu Val Thr His Gln Gly Leu Ser Ser Pro Val Thr Lys
195 200 205

Ser Phe Asn Arg Gly Glu Cys
210 215

<210> 31
<211> 120
<212> PRT
<213> Artificial Sequence

<220>
<223> Antibody sequence

<400> 31

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Pro Tyr
20 25 30

Pro Met Ile Trp Val Arg Gln Ala Pro Gly Lys Gly Leu Glu Trp Val
35 40 45

Ser Tyr Ile Ser Pro Ser Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Gly Gly Asp Thr Tyr Phe Asp Tyr Phe Asp Tyr Trp Gly Gln
100 105 110

Gly Thr Leu Val Thr Val Ser Ser
115 120

<210> 32
<211> 5
<212> PRT

<213> Artificial Sequence

<220>
<223> Antibody sequence

<400> 32

Pro Tyr Pro Met Ile
1 5

<210> 33
<211> 17
<212> PRT
<213> Artificial Sequence

<220>
<223> Antibody sequence

<400> 33

Tyr Ile Ser Pro Ser Gly Gly Ser Thr His Tyr Ala Asp Ser Val Lys
1 5 10 15

Gly

<210> 34
<211> 11
<212> PRT
<213> Artificial Sequence

<220>
<223> Antibody sequence

<400> 34

Gly Gly Asp Thr Tyr Phe Asp Tyr Phe Asp Tyr
1 5 10

<210> 35
<211> 108
<212> PRT
<213> Artificial Sequence

<220>
<223> Antibody sequence

<400> 35

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Gly Tyr
20 25 30

Leu Asn Trp Tyr Gln Gln Lys Pro Gly Lys Ala Pro Lys Leu Leu Ile
35 40 45

Tyr Gln Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Thr Ser Pro Phe
85 90 95

Ile Thr Phe Gly Gln Gly Thr Lys Val Glu Ile Lys
100 105

<210> 36
<211> 11
<212> PRT
<213> Artificial Sequence

<220>
<223> Antibody sequence

<400> 36

Arg Ala Ser Gln Ser Ile Ser Gly Tyr Leu Asn
1 5 10

<210> 37
<211> 7
<212> PRT
<213> Artificial Sequence

<220>
<223> Antibody sequence

<400> 37

Gln Ala Ser Ser Leu Gln Ser
1 5

<210> 38
<211> 10
<212> PRT
<213> Artificial Sequence

<220>

<223> Antibody sequence

<400> 38

Gln Gln Ser Tyr Thr Ser Pro Phe Ile Thr
1 5 10

<210> 39
<211> 449
<212> PRT
<213> Artificial Sequence

<220>
<223> Antibody sequence

<400> 39

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45

Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
```

225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Ala Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 40
<211> 215
<212> PRT
<213> Artificial Sequence

<220>
<223> Antibody sequence

<400> 40

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Gly Tyr
            20                  25                  30

Leu Asn Trp Tyr Gln Gln Lys Pro Gly Lys Ala Pro Lys Leu Leu Ile
        35                  40                  45

Tyr Gln Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Ser Pro Phe
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 41

<211> 120
<212> PRT
<213> Artificial Sequence

<220>
<223> Antibody sequence

<400> 41

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45

Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser
        115                 120
```

<210> 42
<211> 5
<212> PRT
<213> Artificial Sequence

<220>
<223> Antibody sequence

<400> 42

```
Pro Tyr Pro Met Ile
1               5
```

<210> 43
<211> 17
<212> PRT
<213> Artificial Sequence

<220>

<223> Antibody sequence

<400> 43

```
Tyr Ile Ser Pro Ser Gly Gly Ser Thr His Tyr Ala Asp Ser Val Lys
1               5                   10                  15

Gly
```

<210> 44
<211> 11
<212> PRT
<213> Artificial Sequence

<220>
<223> Antibody sequence

<400> 44

```
Gly Gly Asp Thr Tyr Phe Asp Tyr Phe Asp Tyr
1               5                   10
```

<210> 45
<211> 108
<212> PRT
<213> Artificial Sequence

<220>
<223> Antibody sequence

<400> 45

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Gly Tyr
            20                  25                  30

Leu Asn Trp Tyr Gln Gln Lys Pro Gly Lys Ala Pro Lys Leu Leu Ile
        35                  40                  45

Tyr Gln Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Ser Pro Phe
                85                  90                  95

Ile Thr Phe Gly Gln Gly Thr Lys Val Glu Ile Lys
            100                 105
```

<210> 46
<211> 11
<212> PRT
<213> Artificial Sequence

<220>
<223> Antibody sequence

<400> 46

```
Arg Ala Ser Gln Ser Ile Ser Gly Tyr Leu Asn
1               5                   10
```

<210> 47
<211> 7
<212> PRT
<213> Artificial Sequence

<220>
<223> Antibody sequence

<400> 47

```
Gln Ala Ser Ser Leu Gln Ser
1               5
```

<210> 48
<211> 10
<212> PRT
<213> Artificial Sequence

<220>
<223> Antibody sequence

<400> 48

```
Gln Gln Ser Tyr Thr Ser Pro Phe Ile Thr
1               5                   10
```

<210> 49
<211> 449
<212> PRT
<213> Artificial Sequence

<220>
<223> Antibody sequence

<400> 49

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45
```

```
Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300
```

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 50
<211> 215
<212> PRT
<213> Artificial Sequence

<220>
<223> Antibody sequence

<400> 50

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Gly Tyr
20 25 30

Leu Asn Trp Tyr Gln Gln Lys Pro Gly Lys Ala Pro Lys Leu Leu Ile
35 40 45

```
Tyr Gln Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Ser Pro Phe
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 51
<211> 120
<212> PRT
<213> Artificial Sequence

<220>
<223> Antibody sequence

<400> 51

```
Gln Val Gln Leu Gln Gln Ser Gly Pro Glu Leu Val Lys Pro Gly Ala
1               5                   10                  15

Ser Val Lys Met Ser Cys Lys Ala Ser Gly Tyr Thr Phe Thr Asp Phe
            20                  25                  30

Ile Ile Ala Trp Val Lys Gln Arg Thr Gly Gln Gly Leu Glu Trp Ile

        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Lys Ser Ser Asn Thr Ala Tyr
65                  70                  75                  80

Met Gln Leu Ser Ser Leu Thr Ser Val Asp Ser Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Thr Leu Thr Val Ser Ser
        115                 120
```

<210> 52
<211> 5
<212> PRT
<213> Artificial Sequence

<220>
<223> Antibody sequence

<400> 52

```
Asp Phe Ile Ile Ala
1               5
```

<210> 53
<211> 17
<212> PRT
<213> Artificial Sequence

<220>
<223> Antibody sequence

<400> 53

Glu Ile Tyr Pro Gly Thr Gly Arg Thr Tyr Tyr Ser Glu Lys Phe Arg
1               5                   10                  15

Gly

<210> 54
<211> 11
<212> PRT
<213> Artificial Sequence

<220>
<223> Antibody sequence

<400> 54

Arg Thr Ile Tyr Tyr Asp Tyr Asp Gly Asp Tyr
1               5                   10

<210> 55
<211> 112
<212> PRT
<213> Artificial Sequence

<220>
<223> Antibody sequence

<400> 55

Asp Ile Val Met Thr Gln Ala Ala Phe Ser Asn Pro Val Thr Leu Gly
1               5                   10                  15

Thr Ser Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Ser Ser Gly Ser Gly Thr Asp Phe Thr Leu Arg Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gly Gly Thr Lys Leu Glu Leu Lys
            100                 105                 110

<210> 56
<211> 16
<212> PRT

<213> Artificial Sequence

<220>
<223> Antibody sequence

<400> 56

Arg Ser Ser Lys Ser Leu Leu His Ser Asn Gly Ile Thr Tyr Leu Tyr
1               5                   10                  15

<210> 57
<211> 7
<212> PRT
<213> Artificial Sequence

<220>
<223> Antibody sequence

<400> 57

Gln Met Ser Asn Leu Ala Ser
1               5

<210> 58
<211> 9
<212> PRT
<213> Artificial Sequence

<220>
<223> Antibody sequence

<400> 58

Ala His Asn Leu Glu Leu Pro Trp Thr
1               5

<210> 59
<211> 449
<212> PRT
<213> Artificial Sequence

<220>
<223> Antibody sequence

<400> 59

```
Gln Val Gln Leu Gln Gln Ser Gly Pro Glu Leu Val Lys Pro Gly Ala
1               5                   10                  15

Ser Val Lys Met Ser Cys Lys Ala Ser Gly Tyr Thr Phe Thr Asp Phe
            20                  25                  30

Ile Ile Ala Trp Val Lys Gln Arg Thr Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Lys Ser Ser Asn Thr Ala Tyr
65                  70                  75                  80

Met Gln Leu Ser Ser Leu Thr Ser Val Asp Ser Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Thr Leu Thr Val Ser Ser Ala Ser Thr Lys Gly Pro Ser Val
        115                 120                 125
```

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp

```
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly
```

<210> 60
<211> 219
<212> PRT
<213> Artificial Sequence

<220>
<223> Antibody sequence

<400> 60

Asp Ile Val Met Thr Gln Ala Ala Phe Ser Asn Pro Val Thr Leu Gly
1 5 10 15

Thr Ser Ala Ser Ile Ser Cys Arg Ser Ser Lys Ser Leu Leu His Ser
20 25 30

Asn Gly Ile Thr Tyr Leu Tyr Trp Tyr Leu Gln Lys Pro Gly Gln Ser
35 40 45

Pro Gln Leu Leu Ile Tyr Gln Met Ser Asn Leu Ala Ser Gly Val Pro
50 55 60

Asp Arg Phe Ser Ser Ser Gly Ser Gly Thr Asp Phe Thr Leu Arg Ile
65 70 75 80

Ser Arg Val Glu Ala Glu Asp Val Gly Val Tyr Tyr Cys Ala His Asn
85 90 95

Leu Glu Leu Pro Trp Thr Phe Gly Gly Gly Thr Lys Leu Glu Leu Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 61
<211> 120
<212> PRT
<213> Artificial Sequence

<220>

<223> Antibody sequence

<400> 61

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser
        115                 120
```

<210> 62
<211> 5
<212> PRT
<213> Artificial Sequence

<220>
<223> Antibody sequence

<400> 62

```
Asp Phe Ile Ile Ala
1               5
```

<210> 63
<211> 17
<212> PRT
<213> Artificial Sequence

<220>
<223> Antibody sequence

<400> 63

Glu Ile Tyr Pro Gly Thr Gly Arg Thr Tyr Tyr Ser Glu Lys Phe Arg
1               5                   10                  15

Gly

<210> 64
<211> 11
<212> PRT
<213> Artificial Sequence

<220>
<223> Antibody sequence

<400> 64

Arg Thr Ile Tyr Tyr Asp Tyr Asp Gly Asp Tyr
1               5                   10

<210> 65
<211> 112
<212> PRT
<213> Artificial Sequence

<220>
<223> Antibody sequence

<400> 65

Asp Ile Val Met Thr Gln Ser Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

<210> 66
<211> 16
<212> PRT
<213> Artificial Sequence

<220>
<223> Antibody sequence

<400> 66

Arg Ser Ser Lys Ser Leu Leu His Ser Asn Gly Ile Thr Tyr Leu Tyr
1 5 10 15

<210> 67
<211> 7
<212> PRT
<213> Artificial Sequence

<220>
<223> Antibody sequence

<400> 67

Gln Met Ser Asn Leu Ala Ser
1 5

<210> 68
<211> 9
<212> PRT
<213> Artificial Sequence

<220>
<223> Antibody sequence

<400> 68

Ala His Asn Leu Glu Leu Pro Trp Thr
1 5

<210> 69
<211> 449
<212> PRT
<213> Artificial Sequence

<220>
<223> Antibody sequence

<400> 69

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190
```

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 70
<211> 219
<212> PRT
<213> Artificial Sequence

<220>
<223> Antibody sequence

<400> 70

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190
```

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 71
<211> 120
<212> PRT
<213> Artificial Sequence

<220>
<223> Antibody sequence

<400> 71

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser
115 120

<210> 72
<211> 5
<212> PRT
<213> Artificial Sequence

<220>
<223> Antibody sequence

<400> 72

Asp Thr Tyr Ile His
1 5

<210> 73
<211> 17
<212> PRT
<213> Artificial Sequence

<220>
<223> Antibody sequence

<400> 73

Arg Ile Tyr Pro Thr Asn Gly Tyr Thr Arg Tyr Ala Asp Ser Val Lys
1 5 10 15

Gly

<210> 74
<211> 11
<212> PRT
<213> Artificial Sequence

<220>
<223> Antibody sequence

<400> 74

Trp Gly Gly Asp Gly Phe Tyr Ala Met Asp Tyr
1 5 10

<210> 75
<211> 107
<212> PRT
<213> Artificial Sequence

<220>
<223> Antibody sequence

<400> 75

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105
```

<210> 76
<211> 11
<212> PRT
<213> Artificial Sequence

<220>
<223> Antibody sequence

<400> 76

```
Arg Ala Ser Gln Asp Val Asn Thr Ala Val Ala
1               5                   10
```

<210> 77
<211> 7
<212> PRT
<213> Artificial Sequence

<220>
<223> Antibody sequence

<400> 77

```
Ser Ala Ser Phe Leu Tyr Ser
1               5
```

<210> 78
<211> 9
<212> PRT
<213> Artificial Sequence

<220>
<223> Antibody sequence

<400> 78

Gln Gln His Tyr Thr Thr Pro Pro Thr
1 5

<210> 79
<211> 449
<212> PRT
<213> Artificial Sequence

<220>
<223> Antibody sequence

<400> 79

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Ala Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 80
<211> 214
<212> PRT
<213> Artificial Sequence

<220>
<223> Antibody sequence

<400> 80

Asp Ile Gln Met Thr Gln Ser Pro Ser Ser Leu Ser Ala Ser Val Gly
1 5 10 15

```
Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 81
<211> 120
<212> PRT
<213> Artificial Sequence

<220>
<223> Antibody sequence

<400> 81

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser
115 120

<210> 82
<211> 5
<212> PRT
<213> Artificial Sequence

<220>
<223> Antibody sequence

<400> 82

Asp Thr Tyr Ile His
1 5

<210> 83
<211> 17
<212> PRT
<213> Artificial Sequence

<220>
<223> Antibody sequence

<400> 83

Arg Ile Tyr Pro Thr Asn Gly Tyr Thr Arg Tyr Ala Asp Ser Val Lys
1 5 10 15

Gly

<210> 84
<211> 11
<212> PRT
<213> Artificial Sequence

<220>
<223> Antibody sequence

<400> 84

```
Trp Gly Gly Asp Gly Phe Tyr Ala Met Asp Tyr
1               5                   10
```

<210> 85
<211> 107
<212> PRT
<213> Artificial Sequence

<220>
<223> Antibody sequence

<400> 85

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105
```

<210> 86
<211> 11
<212> PRT
<213> Artificial Sequence

<220>
<223> Antibody sequence

<400> 86

Arg Ala Ser Gln Asp Val Asn Thr Ala Val Ala
1 5 10

<210> 87
<211> 7
<212> PRT
<213> Artificial Sequence

<220>
<223> Antibody sequence

<400> 87

Ser Ala Ser Phe Leu Tyr Ser
1 5

<210> 88
<211> 9
<212> PRT
<213> Artificial Sequence

<220>
<223> Antibody sequence

<400> 88

Gln Gln His Tyr Thr Thr Pro Pro Thr
1 5

<210> 89
<211> 449
<212> PRT
<213> Artificial Sequence

<220>
<223> Antibody sequence

<400> 89

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
```

85 90 95

Ser Arg Trp Gly Gly Asp Gly Phe Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Gln Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 90
<211> 214
<212> PRT
<213> Artificial Sequence

<220>
<223> Antibody sequence

<400> 90

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro

                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 91
<211> 117
<212> PRT
<213> Artificial Sequence

<220>
<223> Antibody sequence

<400> 91

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ser Ile Ser Gly Ser Gly Gly Ser Thr Leu Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Thr Gly Thr Ser Phe Asp Tyr Trp Gly Gln Gly Thr Leu
            100                 105                 110

Val Thr Val Ser Ser
        115
```

<210> 92
<211> 5
<212> PRT
<213> Artificial Sequence

<220>
<223> Antibody sequence

<400> 92

```
Ser Tyr Ala Met Ser
1               5
```

<210> 93
<211> 17
<212> PRT
<213> Artificial Sequence

<220>
<223> Antibody sequence

<400> 93

Ser Ile Ser Gly Ser Gly Gly Ser Thr Leu Tyr Ala Asp Ser Val Lys
1               5                   10                  15

Gly

<210> 94
<211> 8
<212> PRT
<213> Artificial Sequence

<220>
<223> Antibody sequence

<400> 94

Leu Thr Gly Thr Ser Phe Asp Tyr
1               5

<210> 95
<211> 109
<212> PRT
<213> Artificial Sequence

<220>
<223> Antibody sequence

<400> 95

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Phe Asp Ser Ser Leu
                85                  90                  95

Lys Lys Val Phe Gly Gly Gly Thr Lys Leu Thr Val Leu
            100                 105

<210> 96
<211> 13
<212> PRT

<213> Artificial Sequence

<220>
<223> Antibody sequence

<400> 96

Ser Gly Ser Ser Ser Asn Ile Gly Ser Asn Pro Val Asn
1               5                   10

<210> 97
<211> 7
<212> PRT
<213> Artificial Sequence

<220>
<223> Antibody sequence

<400> 97

Ser Asn Asn Gln Arg Pro Ser
1               5

<210> 98
<211> 10
<212> PRT
<213> Artificial Sequence

<220>
<223> Antibody sequence

<400> 98

Gln Ser Phe Asp Ser Ser Leu Lys Lys Val
1               5                   10

<210> 99
<211> 446
<212> PRT
<213> Artificial Sequence

<220>
<223> Antibody sequence

<400> 99

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ser Ile Ser Gly Ser Gly Gly Ser Thr Leu Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Thr Gly Thr Ser Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160
```

```
Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415
```

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly
435 440 445

<210> 100
<211> 215
<212> PRT
<213> Artificial Sequence

<220>
<223> Antibody sequence

<400> 100

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Thr Gly Leu Gln
65 70 75 80

Ser Glu Asp Glu Ala Asp Tyr Tyr Cys Gln Ser Phe Asp Ser Ser Leu
85 90 95

Lys Lys Val Phe Gly Gly Gly Thr Lys Leu Thr Val Leu Gly Gln Pro
100 105 110

Lys Ala Ala Pro Ser Val Thr Leu Phe Pro Pro Ser Ser Glu Glu Leu
115 120 125

Gln Ala Asn Lys Ala Thr Leu Val Cys Leu Ile Ser Asp Phe Tyr Pro
130 135 140

Gly Ala Val Thr Val Ala Trp Lys Ala Asp Ser Ser Pro Val Lys Ala
145 150 155 160

Gly Val Glu Thr Thr Thr Pro Ser Lys Gln Ser Asn Asn Lys Tyr Ala
165 170 175

Ala Ser Ser Tyr Leu Ser Leu Thr Pro Glu Gln Trp Lys Ser His Arg
180 185 190

Ser Tyr Ser Cys Gln Val Thr His Glu Gly Ser Thr Val Glu Lys Thr
195 200 205

Val Ala Pro Thr Glu Cys Ser
210 215

<210> 101
<211> 117
<212> PRT
<213> Artificial Sequence

<220>
<223> Antibody sequence

<400> 101

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ser Ile Ser Gly Ser Gly Gly Ser Thr Leu Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Leu Thr Gly Thr Ser Phe Asp Tyr Trp Gly Gln Gly Thr Leu
100 105 110

Val Thr Val Ser Ser
115

<210> 102
<211> 5
<212> PRT
<213> Artificial Sequence

<220>
<223> Antibody sequence

<400> 102

Ser Tyr Ala Met Ser
1               5

<210> 103
<211> 17
<212> PRT
<213> Artificial Sequence

<220>
<223> Antibody sequence

<400> 103

Ser Ile Ser Gly Ser Gly Gly Ser Thr Leu Tyr Ala Asp Ser Val Lys
1               5                   10                  15

Gly

<210> 104
<211> 8
<212> PRT
<213> Artificial Sequence

<220>
<223> Antibody sequence

<400> 104

Leu Thr Gly Thr Ser Phe Asp Tyr
1               5

<210> 105
<211> 109
<212> PRT
<213> Artificial Sequence

<220>
<223> Antibody sequence

<400> 105

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65 70 75 80

Ser Glu Asp Glu Ala Asp Tyr Tyr Cys Gln Ser Phe Asp Ser Ser Leu
85 90 95

Lys Lys Val Phe Gly Gly Gly Thr Lys Leu Thr Val Leu
100 105

<210> 106
<211> 13
<212> PRT
<213> Artificial Sequence

<220>
<223> Antibody sequence

<400> 106

Ser Gly Ser Ser Ser Asn Ile Gly Ser Asn Pro Val Asn
1 5 10

<210> 107
<211> 7
<212> PRT
<213> Artificial Sequence

<220>
<223> Antibody sequence

<400> 107

Ser Asn Asn Gln Arg Pro Ser
1 5

<210> 108
<211> 10
<212> PRT
<213> Artificial Sequence

<220>
<223> Antibody sequence

<400> 108

Gln Ser Phe Asp Ser Ser Leu Lys Lys Val
1 5 10

<210> 109
<211> 446
<212> PRT
<213> Artificial Sequence

<220>
<223> Antibody sequence

<400> 109

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ser Ile Ser Gly Ser Gly Gly Ser Thr Leu Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Thr Gly Thr Ser Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr

245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Gln Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Pro Ala Pro Ile Glu Lys Thr Ile
325 330 335

Ser Lys Ala Lys Gly Gln Pro Arg Glu Pro Gln Val Tyr Thr Leu Pro
340 345 350

Pro Ser Arg Asp Glu Leu Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly
435 440 445

<210> 110
<211> 215
<212> PRT
<213> Artificial Sequence

<220>
<223> Antibody sequence

<400> 110

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Phe Asp Ser Ser Leu
                85                  90                  95

Lys Lys Val Phe Gly Gly Gly Thr Lys Leu Thr Val Leu Gly Gln Pro
            100                 105                 110

Lys Ala Ala Pro Ser Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
    130                 135                 140

Gly Ala Val Thr Val Ala Trp Lys Ala Asp Ser Ser Pro Val Lys Ala
145                 150                 155                 160

Gly Val Glu Thr Thr Thr Pro Ser Lys Gln Ser Asn Asn Lys Tyr Ala
                165                 170                 175

Ala Ser Ser Tyr Leu Ser Leu Thr Pro Glu Gln Trp Lys Ser His Arg
            180                 185                 190

Ser Tyr Ser Cys Gln Val Thr His Glu Gly Ser Thr Val Glu Lys Thr
        195                 200                 205

Val Ala Pro Thr Glu Cys Ser
    210                 215

<210> 111
<211> 120
<212> PRT
<213> Artificial Sequence

<220>
<223> Antibody sequence

<400> 111

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45

Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser
        115                 120
```

<210> 112
<211> 5
<212> PRT
<213> Artificial Sequence

<220>
<223> Antibody sequence

<400> 112

```
Pro Tyr Pro Met Ile
1               5
```

<210> 113
<211> 17
<212> PRT
<213> Artificial Sequence

<220>
<223> Antibody sequence

<400> 113

Tyr Ile Ser Pro Ser Gly Gly Ser Thr His Tyr Ala Asp Ser Val Lys
1               5                   10                  15

Gly

<210> 114
<211> 11
<212> PRT
<213> Artificial Sequence

<220>
<223> Antibody sequence

<400> 114

Gly Gly Asp Thr Tyr Phe Asp Tyr Phe Asp Tyr
1               5                   10

<210> 115
<211> 108
<212> PRT
<213> Artificial Sequence

<220>
<223> Antibody sequence

<400> 115

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Gly Tyr
            20                  25                  30

Leu Asn Trp Tyr Gln Gln Lys Pro Gly Lys Ala Pro Lys Leu Leu Ile
        35                  40                  45

Tyr Gln Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Ser Pro Phe
                85                  90                  95

Ile Thr Phe Gly Gln Gly Thr Lys Val Glu Ile Lys
            100                 105

<210> 116
<211> 11
<212> PRT

<213> Artificial Sequence

<220>
<223> Antibody sequence

<400> 116

```
Arg Ala Ser Gln Ser Ile Ser Gly Tyr Leu Asn
1               5                   10
```

<210> 117
<211> 7
<212> PRT
<213> Artificial Sequence

<220>
<223> Antibody sequence

<400> 117

```
Gln Ala Ser Ser Leu Gln Ser
1               5
```

<210> 118
<211> 10
<212> PRT
<213> Artificial Sequence

<220>
<223> Antibody sequence

<400> 118

```
Gln Gln Ser Tyr Thr Ser Pro Phe Ile Thr
1               5                   10
```

<210> 119
<211> 449
<212> PRT
<213> Artificial Sequence

<220>
<223> Antibody sequence

<400> 119

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Pro Met Ile Trp Val Arg Gln Ala Pro Gly Lys Gly Leu Glu Trp Val
        35                  40                  45

Ser Tyr Ile Ser Pro Ser Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80
```

```
Leu Gln Met Asn Ser Leu Arg Ala Glu Asp Thr Ala Val Tyr Tyr Cys
                85                  90                  95

Ala Arg Gly Gly Asp Thr Tyr Phe Asp Tyr Phe Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Asn Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335
```

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 120
<211> 215
<212> PRT
<213> Artificial Sequence

<220>
<223> Antibody sequence

<400> 120

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Gly Tyr
20 25 30

Leu Asn Trp Tyr Gln Gln Lys Pro Gly Lys Ala Pro Lys Leu Leu Ile
35 40 45

Tyr Gln Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Thr Ser Pro Phe
85 90 95

Ile Thr Phe Gly Gln Gly Thr Lys Val Glu Ile Lys Arg Thr Val Ala
100 105 110

Ala Pro Ser Val Phe Ile Phe Pro Pro Ser Asp Glu Gln Leu Lys Ser
115 120 125

Gly Thr Ala Ser Val Val Cys Leu Leu Asn Asn Phe Tyr Pro Arg Glu
130 135 140

Ala Lys Val Gln Trp Lys Val Asp Asn Ala Leu Gln Ser Gly Asn Ser
145 150 155 160

Gln Glu Ser Val Thr Glu Gln Asp Ser Lys Asp Ser Thr Tyr Ser Leu
165 170 175

Ser Ser Thr Leu Thr Leu Ser Lys Ala Asp Tyr Glu Lys His Lys Val
180 185 190

Tyr Ala Cys Glu Val Thr His Gln Gly Leu Ser Ser Pro Val Thr Lys
195 200 205

Ser Phe Asn Arg Gly Glu Cys
210 215

<210> 121
<211> 120
<212> PRT
<213> Artificial Sequence

<220>
<223> Antibody sequence

<400> 121

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr

65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser
        115                 120
```

<210> 122
<211> 5
<212> PRT
<213> Artificial Sequence

<220>
<223> Antibody sequence

<400> 122

```
Asp Phe Ile Ile Ala
1               5
```

<210> 123
<211> 17
<212> PRT
<213> Artificial Sequence

<220>
<223> Antibody sequence

<400> 123

Glu Ile Tyr Pro Gly Thr Gly Arg Thr Tyr Tyr Ser Glu Lys Phe Arg
1               5                   10                  15

Gly

<210> 124
<211> 11
<212> PRT
<213> Artificial Sequence

<220>
<223> Antibody sequence

<400> 124

Arg Thr Ile Tyr Tyr Asp Tyr Asp Gly Asp Tyr
1               5                   10

<210> 125
<211> 112
<212> PRT
<213> Artificial Sequence

<220>
<223> Antibody sequence

<400> 125

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

<210> 126
<211> 16
<212> PRT

<213> Artificial Sequence

<220>
<223> Antibody sequence

<400> 126

Arg Ser Ser Gln Ser Leu Leu His Ser Asn Gly Ile Thr Tyr Leu Tyr
1 5 10 15

<210> 127
<211> 7
<212> PRT
<213> Artificial Sequence

<220>
<223> Antibody sequence

<400> 127

Gln Met Ser Asn Arg Ala Ser
1 5

<210> 128
<211> 9
<212> PRT
<213> **Artificial** Sequence

<220>
<223> Antibody sequence

<400> 128

Ala His Asn Leu Glu Leu Pro Trp Thr
1 5

<210> 129
<211> 449
<212> **PRT**
<213> **Artificial** Sequence

<220>
<223> Antibody sequence

<400> 129

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Phe
20 25 30

Ile Ile Ala Trp Val Lys Gln Ala Pro Gly Gln Gly Leu Glu Trp Ile
35 40 45

Gly Glu Ile Tyr Pro Gly Thr Gly Arg Thr Tyr Tyr Ser Glu Lys Phe
50 55 60

Arg Gly Lys Ala Thr Leu Thr Ala Asp Thr Ser Thr Ser Thr Ala Tyr
65 70 75 80

Met Glu Leu Ser Ser Leu Arg Ser Glu Asp Thr Ala Val Tyr Phe Cys
85 90 95

Ala Arg Arg Thr Ile Tyr Tyr Asp Tyr Asp Gly Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp

405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 130
<211> 219
<212> PRT
<213> Artificial Sequence

<220>
<223> Antibody sequence

<400> 130

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210> 131
<211> 120
<212> PRT
<213> Artificial Sequence

<220>

<223> Antibody sequence

<400> 131

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Phe
20 25 30

Ile Ile Ala Trp Val Lys Gln Ala Pro Gly Gln Gly Leu Glu Trp Ile
35 40 45

Gly Glu Ile Tyr Pro Gly Thr Gly Arg Thr Tyr Tyr Ser Glu Lys Phe
50 55 60

Arg Gly Lys Ala Thr Leu Thr Ala Asp Thr Ser Thr Ser Thr Ala Tyr
65 70 75 80

Met Glu Leu Ser Ser Leu Arg Ser Glu Asp Thr Ala Val Tyr Phe Cys
85 90 95

Ala Arg Arg Thr Ile Tyr Tyr Asp Tyr Asp Gly Asp Tyr Trp Gly Gln
100 105 110

Gly Thr Leu Val Thr Val Ser Ser
115 120

<210> 132
<211> 5
<212> PRT
<213> Artificial Sequence

<220>
<223> Antibody sequence

<400> 132

Asp Phe Ile Ile Ala
1 5

<210> 133
<211> 17
<212> PRT
<213> Artificial Sequence

<220>
<223> Antibody sequence

<400> 133

Glu Ile Tyr Pro Gly Thr Gly Arg Thr Tyr Tyr Ser Glu Lys Phe Arg
1 5 10 15

Gly

<210> 134
<211> 11
<212> **PRT**
<213> Artificial Sequence

<220>
<223> Antibody sequence

<400> 134

Arg Thr Ile Tyr Tyr Asp Tyr Asp Gly Asp Tyr
1 5 10

<210> 135
<211> 112
<212> PRT
<213> Artificial Sequence

<220>
<223> Antibody sequence

<400> 135

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Leu His Ser
20 25 30

Asn Gly Ile Asn Tyr Leu Tyr Trp Tyr Leu Gln Lys Pro Gly Gln Ser
35 40 45

Pro Gln Leu Leu Ile Tyr Gln Met Ser Asn Arg Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ala His Asn
85 90 95

Leu Glu Leu Pro Trp Thr Phe Gly Gln Gly Thr Lys Val Glu Ile Lys
100 105 110

<210> 136
<211> 16
<212> PRT

<213> Artificial Sequence

<220>
<223> Antibody sequence

<400> 136

Arg Ser Ser Gln Ser Leu Leu His Ser Asn Gly Ile Asn Tyr Leu Tyr
1 5 10 15

<210> 137
<211> 7
<212> PRT
<213> Artificial Sequence

<220>
<223> Antibody sequence

<400> 137

Gln Met Ser Asn Arg Ala Ser
1 5

<210> 138
<211> 9
<212> PRT
<213> Artificial Sequence

<220>
<223> Antibody sequence

<400> 138

Ala His Asn Leu Glu Leu Pro Trp Thr
1 5

<210> 139
<211> 449
<212> PRT
<213> Artificial Sequence

<220>
<223> Antibody sequence

<400> 139

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

```
Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly
```

<210> 140
<211> 219
<212> PRT

<213> Artificial Sequence

<220>
<223> Antibody sequence

<400> 140

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Ile Asn Tyr Leu Tyr Trp Tyr Leu Gln Lys Pro Gly Gln Ser
        35                  40                  45

Pro Gln Leu Leu Ile Tyr Gln Met Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 141
<211> 120
<212> PRT
<213> Artificial Sequence

<220>
<223> Antibody sequence

<400> 141

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser
        115                 120

<210> 142
<211> 5
<212> PRT
<213> Artificial Sequence

<220>
<223> Antibody sequence

<400> 142

Asp Phe Ile Ile Ala
1               5

<210> 143
<211> 17
<212> PRT
<213> Artificial Sequence

<220>
<223> Antibody sequence

<400> 143

Glu Ile Tyr Pro Gly Thr Gly Arg Thr Tyr Tyr Ser Glu Lys Phe Arg
1 5 10 15

Gly

<210> 144
<211> 11
<212> PRT
<213> Artificial Sequence

<220>
<223> Antibody sequence

<400> 144

Arg Thr Ile Tyr Tyr Asp Tyr Asp Gly Asp Tyr
1 5 10

<210> 145
<211> 112
<212> PRT
<213> Artificial Sequence

<220>
<223> Antibody sequence

<400> 145

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Trp Ile Asn Tyr Leu Tyr Trp Tyr Leu Gln Lys Pro Gly Gln Ser
        35                  40                  45

Pro Gln Leu Leu Ile Tyr Gln Met Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

<210> 146
<211> 16
<212> PRT
<213> Artificial Sequence

<220>
<223> Antibody sequence

<400> 146

Arg Ser Ser Gln Ser Leu Leu His Ser Asn Trp Ile Asn Tyr Leu Tyr
1               5                   10                  15

<210> 147
<211> 7
<212> PRT
<213> Artificial Sequence

<220>
<223> Antibody sequence

<400> 147

Gln Met Ser Asn Arg Ala Ser
1               5

<210> 148
<211> 9
<212> PRT
<213> Artificial Sequence

<220>

<223> Antibody sequence

<400> 148

```
Ala His Asn Leu Glu Leu Pro Trp Thr
1               5
```

<210> 149
<211> 449
<212> PRT
<213> Artificial Sequence

<220>
<223> Antibody sequence

<400> 149

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45
```

```
Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300
```

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 150
<211> 219
<212> PRT
<213> Artificial Sequence

<220>
<223> Antibody sequence

<400> 150

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Leu His Ser
20 25 30

Asn Trp Ile Asn Tyr Leu Tyr Trp Tyr Leu Gln Lys Pro Gly Gln Ser
35 40 45

```
Pro Gln Leu Leu Ile Tyr Gln Met Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 151
<211> 120
<212> **PRT**
<213> **Artificial** Sequence

<220>
<223> Antibody sequence

<400> 151

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30
```

```
Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser
        115                 120
```

<210> 152
<211> 5
<212> **PRT**
<213> **Artificial** Sequence

<220>
<223> Antibody sequence

<400> 152

```
Asp Phe Ile Ile Ala
1               5
```

<210> 153
<211> 17
<212> PRT
<213> Artificial Sequence

<220>
<223> Antibody sequence

<400> 153

```
Glu Ile Tyr Pro Gly Thr Gly Arg Thr Tyr Tyr Ser Glu Lys Phe Arg
1               5                   10                  15

Gly
```

<210> 154
<211> 11
<212> **PRT**
<213> **Artificial** Sequence

<220>
<223> Antibody sequence

<400> 154

```
Arg Thr Ile Tyr Tyr Asp Tyr Asp Gly Asp Tyr
1               5                   10
```

<210> 155
<211> 112
<212> PRT
<213> Artificial Sequence

<220>
<223> Antibody sequence

<400> 155

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Trp Ile Asn Tyr Leu Tyr Trp Tyr Leu Gln Lys Pro Gly Gln Ser
        35                  40                  45

Pro Gln Leu Leu Ile Tyr Gln Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110
```

<210> 156
<211> 16
<212> **PRT**
<213> **Artificial** Sequence

<220>
<223> Antibody sequence

<400> 156

```
Arg Ser Ser Gln Ser Leu Leu His Ser Asn Trp Ile Asn Tyr Leu Tyr
1               5                   10                  15
```

<210> 157
<211> 7
<212> PRT
<213> Artificial Sequence

<220>
<223> Antibody sequence

<400> 157

```
Gln Gly Ser Asn Arg Ala Ser
1               5
```

<210> 158
<211> 9
<212> **PRT**
<213> **Artificial** Sequence

<220>
<223> Antibody sequence

<400> 158

```
Ala His Asn Leu Glu Leu Pro Trp Thr
1               5
```

<210> 159
<211> 449
<212> PRT
<213> **Artificial** Sequence

<220>
<223> Antibody sequence

<400> 159

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30

Ile Ile Ala Trp Val Lys Gln Ala Pro Gly Gln Gly Leu Glu Trp Ile
        35                  40                  45

Gly Glu Ile Tyr Pro Gly Thr Gly Arg Thr Tyr Tyr Ser Glu Lys Phe
    50                  55                  60

Arg Gly Lys Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Thr Ile Tyr Tyr Asp Tyr Asp Gly Asp Tyr Trp Gly Gln
            100                 105                 110

Gly Thr Leu Val Thr Val Ser Ser Ala Ser Thr Lys Gly Pro Ser Val
```

```
            115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365
```

```
Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly
```

<210> 160
<211> 219
<212> **PRT**
<213> **Artificial** Sequence

<220>
<223> Antibody sequence

<400> 160

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Trp Ile Asn Tyr Leu Tyr Trp Tyr Leu Gln Lys Pro Gly Gln Ser
        35                  40                  45

Pro Gln Leu Leu Ile Tyr Gln Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala His Asn
                85                  90                  95

Leu Glu Leu Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu


        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 161
<211> 28
<212> PRT
<213> Artificial Sequence

<220>
<223> c(RGDfK)

<400> 161

```
His Ser Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25
```

<210> 162
<211> 20
<212> PRT
<213> Artificial Sequence

<220>
<223> HER2-targeting peptide

<400> 162

```
Ala Thr Glu Pro Arg Lys Gln Tyr Ala Thr Pro Arg Val Phe Trp Thr
1               5                   10                  15

Asp Ala Pro Gly
            20
```

<210> 163
<211> 20
<212> PRT
<213> Artificial Sequence

<220>
<223> HER2-targeting peptide

<400> 163

```
Leu Gln Trp Arg Arg Asp Asp Asn Val His Asn Phe Gly Val Trp Ala
1               5                   10                  15

Arg Tyr Arg Leu
            20
```

<210> 164
<211> 129
<212> PRT
<213> Homo sapiens

<400> 164

```
Met Ala Arg Gly Ser Leu Arg Arg Leu Leu Arg Leu Leu Val Leu Gly
1               5                   10                  15

Leu Trp Leu Ala Leu Leu Arg Ser Val Ala Gly Glu Gln Ala Pro Gly
            20                  25                  30

Thr Ala Pro Cys Ser Arg Gly Ser Ser Trp Ser Ala Asp Leu Asp Lys
        35                  40                  45

Cys Met Asp Cys Ala Ser Cys Arg Ala Arg Pro His Ser Asp Phe Cys
    50                  55                  60

Leu Gly Cys Ala Ala Ala Pro Pro Ala Pro Phe Arg Leu Leu Trp Pro
65                  70                  75                  80

Ile Leu Gly Gly Ala Leu Ser Leu Thr Phe Val Leu Gly Leu Leu Ser
                85                  90                  95

Gly Phe Leu Val Trp Arg Arg Cys Arg Arg Arg Glu Lys Phe Thr Thr
            100                 105                 110

Pro Ile Glu Glu Thr Gly Gly Glu Gly Cys Pro Ala Val Ala Leu Ile
        115                 120                 125

Gln
```

<210> 165
<211> 534
<212> **PRT**
<213> Homo sapiens

<400> 165

```
Met Leu Arg Arg Arg Gly Ser Pro Gly Met Gly Val His Val Gly Ala
1               5                   10                  15

Ala Leu Gly Ala Leu Trp Phe Cys Leu Thr Gly Ala Leu Glu Val Gln
            20                  25                  30

Val Pro Glu Asp Pro Val Val Ala Leu Val Gly Thr Asp Ala Thr Leu
        35                  40                  45

Cys Cys Ser Phe Ser Pro Glu Pro Gly Phe Ser Leu Ala Gln Leu Asn
    50                  55                  60

Leu Ile Trp Gln Leu Thr Asp Thr Lys Gln Leu Val His Ser Phe Ala
65                  70                  75                  80

Glu Gly Gln Asp Gln Gly Ser Ala Tyr Ala Asn Arg Thr Ala Leu Phe
                85                  90                  95

Pro Asp Leu Leu Ala Gln Gly Asn Ala Ser Leu Arg Leu Gln Arg Val
            100                 105                 110

Arg Val Ala Asp Glu Gly Ser Phe Thr Cys Phe Val Ser Ile Arg Asp
        115                 120                 125

Phe Gly Ser Ala Ala Val Ser Leu Gln Val Ala Ala Pro Tyr Ser Lys
    130                 135                 140

Pro Ser Met Thr Leu Glu Pro Asn Lys Asp Leu Arg Pro Gly Asp Thr
145                 150                 155                 160

Val Thr Ile Thr Cys Ser Ser Tyr Gln Gly Tyr Pro Glu Ala Glu Val
                165                 170                 175

Phe Trp Gln Asp Gly Gln Gly Val Pro Leu Thr Gly Asn Val Thr Thr
            180                 185                 190

Ser Gln Met Ala Asn Glu Gln Gly Leu Phe Asp Val His Ser Ile Leu
        195                 200                 205

Arg Val Val Leu Gly Ala Asn Gly Thr Tyr Ser Cys Leu Val Arg Asn
    210                 215                 220

Pro Val Leu Gln Gln Asp Ala His Ser Ser Val Thr Ile Thr Pro Gln
225                 230                 235                 240
```

Arg Ser Pro Thr Gly Ala Val Glu Val Gln Val Pro Glu Asp Pro Val
245 250 255

Val Ala Leu Val Gly Thr Asp Ala Thr Leu Arg Cys Ser Phe Ser Pro
260 265 270

Glu Pro Gly Phe Ser Leu Ala Gln Leu Asn Leu Ile Trp Gln Leu Thr
275 280 285

Asp Thr Lys Gln Leu Val His Ser Phe Thr Glu Gly Arg Asp Gln Gly
290 295 300

Ser Ala Tyr Ala Asn Arg Thr Ala Leu Phe Pro Asp Leu Leu Ala Gln
305 310 315 320

Gly Asn Ala Ser Leu Arg Leu Gln Arg Val Arg Val Ala Asp Glu Gly
325 330 335

Ser Phe Thr Cys Phe Val Ser Ile Arg Asp Phe Gly Ser Ala Ala Val
340 345 350

Ser Leu Gln Val Ala Ala Pro Tyr Ser Lys Pro Ser Met Thr Leu Glu
355 360 365

Pro Asn Lys Asp Leu Arg Pro Gly Asp Thr Val Thr Ile Thr Cys Ser
370 375 380

Ser Tyr Arg Gly Tyr Pro Glu Ala Glu Val Phe Trp Gln Asp Gly Gln
385 390 395 400

Gly Val Pro Leu Thr Gly Asn Val Thr Thr Ser Gln Met Ala Asn Glu
405 410 415

Gln Gly Leu Phe Asp Val His Ser Val Leu Arg Val Val Leu Gly Ala
420 425 430

Asn Gly Thr Tyr Ser Cys Leu Val Arg Asn Pro Val Leu Gln Gln Asp
435 440 445

Ala His Gly Ser Val Thr Ile Thr Gly Gln Pro Met Thr Phe Pro Pro
450 455 460

Glu Ala Leu Trp Val Thr Val Gly Leu Ser Val Cys Leu Ile Ala Leu
465 470 475 480

Leu Val Ala Leu Ala Phe Val Cys Trp Arg Lys Ile Lys Gln Ser Cys

485 490 495

Glu Glu Glu Asn Ala Gly Ala Glu Asp Gln Asp Gly Glu Gly Glu Gly
500 505 510

Ser Lys Thr Ala Leu Gln Pro Leu Lys His Ser Asp Ser Lys Glu Asp
515 520 525

Asp Gly Gln Glu Ile Ala
530

<210> 166
<211> 1255
<212> **PRT**
<213> Homo sapiens

<400> 166

Met Glu Leu Ala Ala Leu Cys Arg Trp Gly Leu Leu Leu Ala Leu Leu
1 5 10 15

Pro Pro Gly Ala Ala Ser Thr Gln Val Cys Thr Gly Thr Asp Met Lys
20 25 30

Leu Arg Leu Pro Ala Ser Pro Glu Thr His Leu Asp Met Leu Arg His
35 40 45

Leu Tyr Gln Gly Cys Gln Val Val Gln Gly Asn Leu Glu Leu Thr Tyr
50 55 60

Leu Pro Thr Asn Ala Ser Leu Ser Phe Leu Gln Asp Ile Gln Glu Val
65 70 75 80

Gln Gly Tyr Val Leu Ile Ala His Asn Gln Val Arg Gln Val Pro Leu
85 90 95

Gln Arg Leu Arg Ile Val Arg Gly Thr Gln Leu Phe Glu Asp Asn Tyr
100 105 110

Ala Leu Ala Val Leu Asp Asn Gly Asp Pro Leu Asn Asn Thr Thr Pro
115 120 125

Val Thr Gly Ala Ser Pro Gly Gly Leu Arg Glu Leu Gln Leu Arg Ser
130 135 140

Leu Thr Glu Ile Leu Lys Gly Gly Val Leu Ile Gln Arg Asn Pro Gln
145 150 155 160

Leu Cys Tyr Gln Asp Thr Ile Leu Trp Lys Asp Ile Phe His Lys Asn

165 170 175

Asn Gln Leu Ala Leu Thr Leu Ile Asp Thr Asn Arg Ser Arg Ala Cys
180 185 190

His Pro Cys Ser Pro Met Cys Lys Gly Ser Arg Cys Trp Gly Glu Ser
195 200 205

Ser Glu Asp Cys Gln Ser Leu Thr Arg Thr Val Cys Ala Gly Gly Cys
210 215 220

Ala Arg Cys Lys Gly Pro Leu Pro Thr Asp Cys Cys His Glu Gln Cys
225 230 235 240

Ala Ala Gly Cys Thr Gly Pro Lys His Ser Asp Cys Leu Ala Cys Leu
245 250 255

His Phe Asn His Ser Gly Ile Cys Glu Leu His Cys Pro Ala Leu Val
260 265 270

Thr Tyr Asn Thr Asp Thr Phe Glu Ser Met Pro Asn Pro Glu Gly Arg
275 280 285

Tyr Thr Phe Gly Ala Ser Cys Val Thr Ala Cys Pro Tyr Asn Tyr Leu
290 295 300

Ser Thr Asp Val Gly Ser Cys Thr Leu Val Cys Pro Leu His Asn Gln
305 310 315 320

Glu Val Thr Ala Glu Asp Gly Thr Gln Arg Cys Glu Lys Cys Ser Lys
325 330 335

Pro Cys Ala Arg Val Cys Tyr Gly Leu Gly Met Glu His Leu Arg Glu
340 345 350

Val Arg Ala Val Thr Ser Ala Asn Ile Gln Glu Phe Ala Gly Cys Lys
355 360 365

Lys Ile Phe Gly Ser Leu Ala Phe Leu Pro Glu Ser Phe Asp Gly Asp
370 375 380

Pro Ala Ser Asn Thr Ala Pro Leu Gln Pro Glu Gln Leu Gln Val Phe
385 390 395 400

Glu Thr Leu Glu Glu Ile Thr Gly Tyr Leu Tyr Ile Ser Ala Trp Pro
405 410 415

Asp Ser Leu Pro Asp Leu Ser Val Phe Gln Asn Leu Gln Val Ile Arg
420 425 430

Gly Arg Ile Leu His Asn Gly Ala Tyr Ser Leu Thr Leu Gln Gly Leu
435 440 445

Gly Ile Ser Trp Leu Gly Leu Arg Ser Leu Arg Glu Leu Gly Ser Gly
450 455 460

Leu Ala Leu Ile His His Asn Thr His Leu Cys Phe Val His Thr Val
465 470 475 480

Pro Trp Asp Gln Leu Phe Arg Asn Pro His Gln Ala Leu Leu His Thr
485 490 495

Ala Asn Arg Pro Glu Asp Glu Cys Val Gly Glu Gly Leu Ala Cys His
500 505 510

Gln Leu Cys Ala Arg Gly His Cys Trp Gly Pro Gly Pro Thr Gln Cys
515 520 525

Val Asn Cys Ser Gln Phe Leu Arg Gly Gln Glu Cys Val Glu Glu Cys
530 535 540

Arg Val Leu Gln Gly Leu Pro Arg Glu Tyr Val Asn Ala Arg His Cys
545 550 555 560

Leu Pro Cys His Pro Glu Cys Gln Pro Gln Asn Gly Ser Val Thr Cys
565 570 575

Phe Gly Pro Glu Ala Asp Gln Cys Val Ala Cys Ala His Tyr Lys Asp
580 585 590

Pro Pro Phe Cys Val Ala Arg Cys Pro Ser Gly Val Lys Pro Asp Leu
595 600 605

Ser Tyr Met Pro Ile Trp Lys Phe Pro Asp Glu Glu Gly Ala Cys Gln
610 615 620

Pro Cys Pro Ile Asn Cys Thr His Ser Cys Val Asp Leu Asp Asp Lys
625 630 635 640

Gly Cys Pro Ala Glu Gln Arg Ala Ser Pro Leu Thr Ser Ile Ile Ser
645 650 655

Ala Val Val Gly Ile Leu Leu Val Val Val Leu Gly Val Val Phe Gly
660 665 670

Ile Leu Ile Lys Arg Arg Gln Gln Lys Ile Arg Lys Tyr Thr Met Arg
675 680 685

Arg Leu Leu Gln Glu Thr Glu Leu Val Glu Pro Leu Thr Pro Ser Gly
690 695 700

Ala Met Pro Asn Gln Ala Gln Met Arg Ile Leu Lys Glu Thr Glu Leu
705 710 715 720

Arg Lys Val Lys Val Leu Gly Ser Gly Ala Phe Gly Thr Val Tyr Lys
725 730 735

Gly Ile Trp Ile Pro Asp Gly Glu Asn Val Lys Ile Pro Val Ala Ile
740 745 750

Lys Val Leu Arg Glu Asn Thr Ser Pro Lys Ala Asn Lys Glu Ile Leu
755 760 765

Asp Glu Ala Tyr Val Met Ala Gly Val Gly Ser Pro Tyr Val Ser Arg
770 775 780

Leu Leu Gly Ile Cys Leu Thr Ser Thr Val Gln Leu Val Thr Gln Leu
785 790 795 800

Met Pro Tyr Gly Cys Leu Leu Asp His Val Arg Glu Asn Arg Gly Arg
805 810 815

Leu Gly Ser Gln Asp Leu Leu Asn Trp Cys Met Gln Ile Ala Lys Gly
820 825 830

Met Ser Tyr Leu Glu Asp Val Arg Leu Val His Arg Asp Leu Ala Ala
835 840 845

Arg Asn Val Leu Val Lys Ser Pro Asn His Val Lys Ile Thr Asp Phe
850 855 860

Gly Leu Ala Arg Leu Leu Asp Ile Asp Glu Thr Glu Tyr His Ala Asp
865 870 875 880

Gly Gly Lys Val Pro Ile Lys Trp Met Ala Leu Glu Ser Ile Leu Arg
885 890 895

Arg Arg Phe Thr His Gln Ser Asp Val Trp Ser Tyr Gly Val Thr Val
900 905 910

Trp Glu Leu Met Thr Phe Gly Ala Lys Pro Tyr Asp Gly Ile Pro Ala
915 920 925

Arg Glu Ile Pro Asp Leu Leu Glu Lys Gly Glu Arg Leu Pro Gln Pro
930 935 940

Pro Ile Cys Thr Ile Asp Val Tyr Met Ile Met Val Lys Cys Trp Met
945 950 955 960

Ile Asp Ser Glu Cys Arg Pro Arg Phe Arg Glu Leu Val Ser Glu Phe
965 970 975

Ser Arg Met Ala Arg Asp Pro Gln Arg Phe Val Val Ile Gln Asn Glu
980 985 990

Asp Leu Gly Pro Ala Ser Pro Leu Asp Ser Thr Phe Tyr Arg Ser Leu
995 1000 1005

Leu Glu Asp Asp Asp Met Gly Asp Leu Val Asp Ala Glu Glu Tyr
1010 1015 1020

Leu Val Pro Gln Gln Gly Phe Phe Cys Pro Asp Pro Ala Pro Gly
1025 1030 1035

Ala Gly Gly Met Val His His Arg His Arg Ser Ser Ser Thr Arg
1040 1045 1050

Ser Gly Gly Gly Asp Leu Thr Leu Gly Leu Glu Pro Ser Glu Glu
1055 1060 1065

Glu Ala Pro Arg Ser Pro Leu Ala Pro Ser Glu Gly Ala Gly Ser
1070 1075 1080

Asp Val Phe Asp Gly Asp Leu Gly Met Gly Ala Ala Lys Gly Leu
1085 1090 1095

Gln Ser Leu Pro Thr His Asp Pro Ser Pro Leu Gln Arg Tyr Ser
1100 1105 1110

Glu Asp Pro Thr Val Pro Leu Pro Ser Glu Thr Asp Gly Tyr Val
1115 1120 1125

Ala Pro Leu Thr Cys Ser Pro Gln Pro Glu Tyr Val Asn Gln Pro
1130 1135 1140

Asp Val Arg Pro Gln Pro Pro Ser Pro Arg Glu Gly Pro Leu Pro
1145 1150 1155

Ala Ala Arg Pro Ala Gly Ala Thr Leu Glu Arg Pro Lys Thr Leu

1160 1165 1170

Ser Pro Gly Lys Asn Gly Val Val Lys Asp Val Phe Ala Phe Gly
1175 1180 1185

Gly Ala Val Glu Asn Pro Glu Tyr Leu Thr Pro Gln Gly Gly Ala
1190 1195 1200

Ala Pro Gln Pro His Pro Pro Pro Ala Phe Ser Pro Ala Phe Asp
1205 1210 1215

Asn Leu Tyr Tyr Trp Asp Gln Asp Pro Pro Glu Arg Gly Ala Pro
1220 1225 1230

Pro Ser Thr Phe Lys Gly Thr Pro Thr Ala Glu Asn Pro Glu Tyr
1235 1240 1245

Leu Gly Leu Asp Val Pro Val
1250 1255

<210> 167
<211> 1210
<212> **PRT**
<213> Homo sapiens

<400> 167

```
Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
```

115 120 125

Lys Thr Gly Leu Lys Glu Leu Pro Met Arg Asn Leu Gln Glu Ile Leu
130 135 140

His Gly Ala Val Arg Phe Ser Asn Asn Pro Ala Leu Cys Asn Val Glu
145 150 155 160

Ser Ile Gln Trp Arg Asp Ile Val Ser Ser Asp Phe Leu Ser Asn Met
165 170 175

Ser Met Asp Phe Gln Asn His Leu Gly Ser Cys Gln Lys Cys Asp Pro
180 185 190

Ser Cys Pro Asn Gly Ser Cys Trp Gly Ala Gly Glu Glu Asn Cys Gln
195 200 205

Lys Leu Thr Lys Ile Ile Cys Ala Gln Gln Cys Ser Gly Arg Cys Arg
210 215 220

Gly Lys Ser Pro Ser Asp Cys Cys His Asn Gln Cys Ala Ala Gly Cys
225 230 235 240

Thr Gly Pro Arg Glu Ser Asp Cys Leu Val Cys Arg Lys Phe Arg Asp
245 250 255

Glu Ala Thr Cys Lys Asp Thr Cys Pro Pro Leu Met Leu Tyr Asn Pro
260 265 270

Thr Thr Tyr Gln Met Asp Val Asn Pro Glu Gly Lys Tyr Ser Phe Gly
275 280 285

Ala Thr Cys Val Lys Lys Cys Pro Arg Asn Tyr Val Val Thr Asp His
290 295 300

Gly Ser Cys Val Arg Ala Cys Gly Ala Asp Ser Tyr Glu Met Glu Glu
305 310 315 320

Asp Gly Val Arg Lys Cys Lys Lys Cys Glu Gly Pro Cys Arg Lys Val
325 330 335

Cys Asn Gly Ile Gly Ile Gly Glu Phe Lys Asp Ser Leu Ser Ile Asn
340 345 350

Ala Thr Asn Ile Lys His Phe Lys Asn Cys Thr Ser Ile Ser Gly Asp
355 360 365

```
Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620
```

Thr Tyr Gly Cys Thr Gly Pro Gly Leu Glu Gly Cys Pro Thr Asn Gly
625 630 635 640

Pro Lys Ile Pro Ser Ile Ala Thr Gly Met Val Gly Ala Leu Leu Leu
645 650 655

Leu Leu Val Val Ala Leu Gly Ile Gly Leu Phe Met Arg Arg Arg His
660 665 670

Ile Val Arg Lys Arg Thr Leu Arg Arg Leu Leu Gln Glu Arg Glu Leu
675 680 685

Val Glu Pro Leu Thr Pro Ser Gly Glu Ala Pro Asn Gln Ala Leu Leu
690 695 700

Arg Ile Leu Lys Glu Thr Glu Phe Lys Lys Ile Lys Val Leu Gly Ser
705 710 715 720

Gly Ala Phe Gly Thr Val Tyr Lys Gly Leu Trp Ile Pro Glu Gly Glu
725 730 735

Lys Val Lys Ile Pro Val Ala Ile Lys Glu Leu Arg Glu Ala Thr Ser
740 745 750

Pro Lys Ala Asn Lys Glu Ile Leu Asp Glu Ala Tyr Val Met Ala Ser
755 760 765

Val Asp Asn Pro His Val Cys Arg Leu Leu Gly Ile Cys Leu Thr Ser
770 775 780

Thr Val Gln Leu Ile Thr Gln Leu Met Pro Phe Gly Cys Leu Leu Asp
785 790 795 800

Tyr Val Arg Glu His Lys Asp Asn Ile Gly Ser Gln Tyr Leu Leu Asn
805 810 815

Trp Cys Val Gln Ile Ala Lys Gly Met Asn Tyr Leu Glu Asp Arg Arg
820 825 830

Leu Val His Arg Asp Leu Ala Ala Arg Asn Val Leu Val Lys Thr Pro
835 840 845

Gln His Val Lys Ile Thr Asp Phe Gly Leu Ala Lys Leu Leu Gly Ala
850 855 860

Glu Glu Lys Glu Tyr His Ala Glu Gly Gly Lys Val Pro Ile Lys Trp
865 870 875 880

```
Met Ala Leu Glu Ser Ile Leu His Arg Ile Tyr Thr His Gln Ser Asp
                885                 890                 895

Val Trp Ser Tyr Gly Val Thr Val Trp Glu Leu Met Thr Phe Gly Ser
            900                 905                 910

Lys Pro Tyr Asp Gly Ile Pro Ala Ser Glu Ile Ser Ser Ile Leu Glu
        915                 920                 925

Lys Gly Glu Arg Leu Pro Gln Pro Pro Ile Cys Thr Ile Asp Val Tyr
    930                 935                 940

Met Ile Met Val Lys Cys Trp Met Ile Asp Ala Asp Ser Arg Pro Lys
945                 950                 955                 960

Phe Arg Glu Leu Ile Ile Glu Phe Ser Lys Met Ala Arg Asp Pro Gln
                965                 970                 975

Arg Tyr Leu Val Ile Gln Gly Asp Glu Arg Met His Leu Pro Ser Pro
            980                 985                 990

Thr Asp Ser Asn Phe Tyr Arg Ala  Leu Met Asp Glu Glu  Asp Met Asp
        995                 1000                1005

Asp Val  Val Asp Ala Asp Glu  Tyr Leu Ile Pro Gln  Gln Gly Phe
    1010                 1015                 1020

Phe Ser  Ser Pro Ser Thr Ser  Arg Thr Pro Leu Leu  Ser Ser Leu
    1025                 1030                 1035

Ser Ala  Thr Ser Asn Asn Ser  Thr Val Ala Cys Ile  Asp Arg Asn
    1040                 1045                 1050

Gly Leu  Gln Ser Cys Pro Ile  Lys Glu Asp Ser Phe  Leu Gln Arg
    1055                 1060                 1065

Tyr Ser  Ser Asp Pro Thr Gly  Ala Leu Thr Glu Asp  Ser Ile Asp
    1070                 1075                 1080

Asp Thr  Phe Leu Pro Val Pro  Glu Tyr Ile Asn Gln  Ser Val Pro
    1085                 1090                 1095

Lys Arg  Pro Ala Gly Ser Val  Gln Asn Pro Val Tyr  His Asn Gln
    1100                 1105                 1110

Pro Leu  Asn Pro Ala Pro Ser  Arg Asp Pro His Tyr  Gln Asp Pro
```

```
        1115                1120                1125

His Ser Thr Ala Val Gly Asn Pro Glu Tyr Leu Asn Thr Val Gln
        1130                1135                1140

Pro Thr Cys Val Asn Ser Thr Phe Asp Ser Pro Ala His Trp Ala
        1145                1150                1155

Gln Lys Gly Ser His Gln Ile Ser Leu Asp Asn Pro Asp Tyr Gln
        1160                1165                1170

Gln Asp Phe Phe Pro Lys Glu Ala Lys Pro Asn Gly Ile Phe Lys
        1175                1180                1185

Gly Ser Thr Ala Glu Asn Ala Glu Tyr Leu Arg Val Ala Pro Gln
        1190                1195                1200

Ser Ser Glu Phe Ile Gly Ala
        1205                1210
```